# EUROPEAN PATENT APPLICATION

(11) **EP 4 755 385 A2**
(43) Date of publication of application: **10.06.2026**
(21) Application number: 26173001.4
(22) Date of filing: 02.12.2019
(51) Int. Cl.: A61K 31/5386

(54) **IRAK DEGRADERS AND USES THEREOF**

(30) Priority: 30.11.2018 US 201862774051 P; 04.01.2019 US 201962788460 P; 18.01.2019 US 201962793992 P; 29.03.2019 US 201962826743 P; 08.04.2019 US 201962831007 P; 22.05.2019 US 201962851427 P; 28.06.2019 US 201962868574 P; 17.07.2019 US 201962875347 P; 26.07.2019 US 201962879117 P
(62) Divisional of application: 19891087.9
(71) Applicant: Kymera Therapeutics, Inc., Watertown, MA 02472 (US)
(72) Inventor: MAINOLFI, Nello, Belmont, 02478 (US); JI, Nan, Arlington, 02476 (US); KLUGE, Arthur F., Concord, 01742 (US); WEISS, Matthew M., Boston, 02114 (US); ZHANG, Yi, Belmont, 02478 (US); ZHENG, Xiaozhang, Lexington, 02421 (US)
(74) Representative: Casalonga

(57) **Abstract**

The present invention provides compounds, compositions thereof, and methods of using the same.

## Description

### CROSS-REFERENCE TO RELATED APPLICATIONS

This application claims the benefit of U.S. Provisional App. No. 62/774,051, filed on November 30, 2018, U.S. Provisional App. No. 62/788,460, filed on January 4, 2019, U.S. Provisional App. No. 62/793,992, filed on January 18, 2019, U.S. Provisional App. No. 62/826,743, filed on March 29, 2019, U.S. Provisional App. No. 62/831,007, filed on April 8, 2019, U.S. Provisional App. No. 62/851,427, filed on May 22, 2019, U.S. Provisional App. No. 62/868,574, filed on June 28, 2019, U.S. Provisional App. No. 62/875,347, filed on July 17, 2019, and U.S. Provisional App. No. 62/879,117, filed on July 26, 2019, the content of each of which is hereby incorporated by reference.

### TECHNICAL FIELD OF THE INVENTION

The present invention relates to compounds and methods useful for the modulation of one or more interleukin-1 receptor-associated kinases ("IRAK") via ubiquitination and/or degradation by compounds according to the present invention. The invention also provides pharmaceutically acceptable compositions comprising compounds of the present invention and methods of using said compositions in the treatment of various disorders.

### BACKGROUND OF THE INVENTION

Ubiquitin-Proteasome Pathway (UPP) is a critical pathway that regulates key regulator proteins and degrades misfolded or abnormal proteins. UPP is central to multiple cellular processes, and if defective or imbalanced, it leads to pathogenesis of a variety of diseases. The covalent attachment of ubiquitin to specific protein substrates is achieved through the action of E3 ubiquitin ligases.

There are over 600 E3 ubiquitin ligases which facilitate the ubiquitination of different proteins in vivo, which can be divided into four families: HECT-domain E3s, U-box E3s, monomeric RING E3s and multi-subunit E3s. See generally Li et al. (PLOS One, 2008, 3, 1487) titled "Genome-wide and functional annotation of human E3 ubiquitin ligases identifies MULAN, a mitochondrial E3 that regulates the organelle's dynamics and signaling."; Bemdsen et al. (Nat. Struct. Mol. Biol., 2014, 21, 301-307) titled "New insights into ubiquitin E3 ligase mechanism"; Deshaies et al. (Ann. Rev. Biochem., 2009, 78, 399-434) titled "RING domain E3 ubiquitin ligases."; Spratt et al. (Biochem. 2014, 458, 421-437) titled "RBR E3 ubiquitin ligases: new structures, new insights, new questions."; and Wang et al. (Nat. Rev. Cancer., 2014, 14, 233-347) titled "Roles of F-box proteins in cancer."

UPP plays a key role in the degradation of short-lived and regulatory proteins important in a variety of basic cellular processes, including regulation of the cell cycle, modulation of cell surface receptors and ion channels, and antigen presentation. The pathway has been implicated in several forms of malignancy, in the pathogenesis of several genetic diseases (including cystic fibrosis, Angelman's syndrome, and Liddle syndrome), in immune surveillance/viral pathogenesis, and in the pathology of muscle wasting. Many diseases are associated with an abnormal UPP and negatively affect cell cycle and division, the cellular response to stress and to extracellular modulators, morphogenesis of neuronal networks, modulation of cell surface receptors, ion channels, the secretory pathway, DNA repair and biogenesis of organelles.

Aberrations in the process have recently been implicated in the pathogenesis of several diseases, both inherited and acquired. These diseases fall into two major groups: (a) those that result from loss of function with the resultant stabilization of certain proteins, and (b) those that result from gain of function, i.e. abnormal or accelerated degradation of the protein target.

The UPP is used to induce selective protein degradation, including use of fusion proteins to artificially ubiquitinate target proteins and synthetic small-molecule probes to induce proteasome-dependent degradation. Bifunctional compounds composed of a target protein-binding ligand and an E3 ubiquitin ligase ligand, induced proteasome-mediated degradation of selected proteins via their recruitment to E3 ubiquitin ligase and subsequent ubiquitination. These drug-like molecules offer the possibility of temporal control over protein expression. Such compounds are capable of inducing the inactivation of a protein of interest upon addition to cells or administration to an animal or human, and could be useful as biochemical reagents and lead to a new paradigm for the treatment of diseases by removing pathogenic or oncogenic proteins (Crews C, Chemistry & Biology, 2010, 17(6):551-555; Schnnekloth JS Jr., Chembiochem, 2005, 6(l):40-46).

An ongoing need exists in the art for effective treatments for disease, especially hyperplasias and cancers, such as multiple myeloma. However, non-specific effects, and the inability to target and modulate certain classes of proteins altogether, such as transcription factors, remain as obstacles to the development of effective anti-cancer agents. As such, small molecule therapeutic agents that leverage E3 ligase mediated protein degradation to target cancer-associated proteins such as interleukin-1 receptor-associated kinases ("IRAK") hold promise as therapeutic agents. Accordingly, there remains a need to find compounds that are IRAK degraders useful as therapeutic agents.

### SUMMARY OF THE INVENTION

The present application relates novel bifunctional compounds, which function to recruit IRAK kinases to E3 Ubiquitin Ligase for degradation, and methods of preparation and uses thereof. In particular, the present disclosure provides bifunctional compounds, which find utility as modulators of targeted ubiquitination of IRAK kinases, which are then degraded and/or otherwise inhibited by the bifunctional compounds as described herein. Also provided are monovalent compounds, which find utility as inducers of targeted ubiquitination of IRAK kinases, which are then degraded and/or otherwise inhibited by the monovalent compounds as described herein. An advantage of the compounds provided herein is that a broad range of pharmacological activities is possible, consistent with the degradation/inhibition of IRAK kinases. In addition, the description provides methods of using an effective amount of the compounds as described herein for the treatment or amelioration of a disease condition, such as cancer, e.g., multiple myeloma.

The present application further relates to targeted degradation of IRAK kinases through the use of bifunctional molecules, including bifunctional molecules that link a cereblon-binding moiety to a ligand that binds IRAK kinasses.

It has now been found that compounds of this invention, and pharmaceutically acceptable compositions thereof, are effective as degraders of IRAK kinases. Such compounds have the general formula **I**: 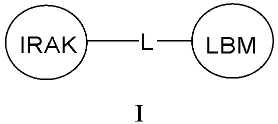 or a pharmaceutically acceptable salt thereof, wherein each variable is as defined and described herein.

It has also now been found that compounds of this invention, and pharmaceutically acceptable compositions thereof, are effective as degraders of IRAK kinases. Such compounds have the general formula **V**: 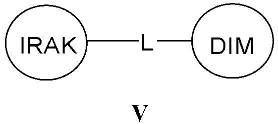 or a pharmaceutically acceptable salt thereof, wherein each variable is as defined and described herein.

Compounds of the present invention, and pharmaceutically acceptable compositions thereof, are useful for treating a variety of diseases, disorders or conditions, associated with regulation of signaling pathways implicating IRAK kinases. Such diseases, disorders, or conditions include those described herein.

Compounds provided by this invention are also useful for the study of IRAK enzymes in biological and pathological phenomena; the study of intracellular signal transduction pathways occurring in bodily tissues; and the comparative evaluation of new IRAK inhibitors or IRAK degraders or other regulators of kinases, signaling pathways, and cytokine levels in vitro or in vivo.

### BRIEF DESCRIPTION OF THE FIGURES

FIG. 1 is an image showing a western blot of degrader **I-30** in OCI-LY10 at 24 h.
FIG. 2 is an image of a dose response curve for IKAK4 (% control)(y-axis) versus degrader **I-30** concentration (µM) (x-axis) for OCI-LY10 and TMD8 cell lines and in vitro degradation results (DC₉₀, µM).
FIG. 3 is an image of a deep TMT proteomics scatterplot in OCI-LY10 at 8h showing Log2 FC 10 nM degrader **I-30** in DMSO (y-axis) and Log2 FC 10 nM degrader in DMSO Rep1 (x-axis).

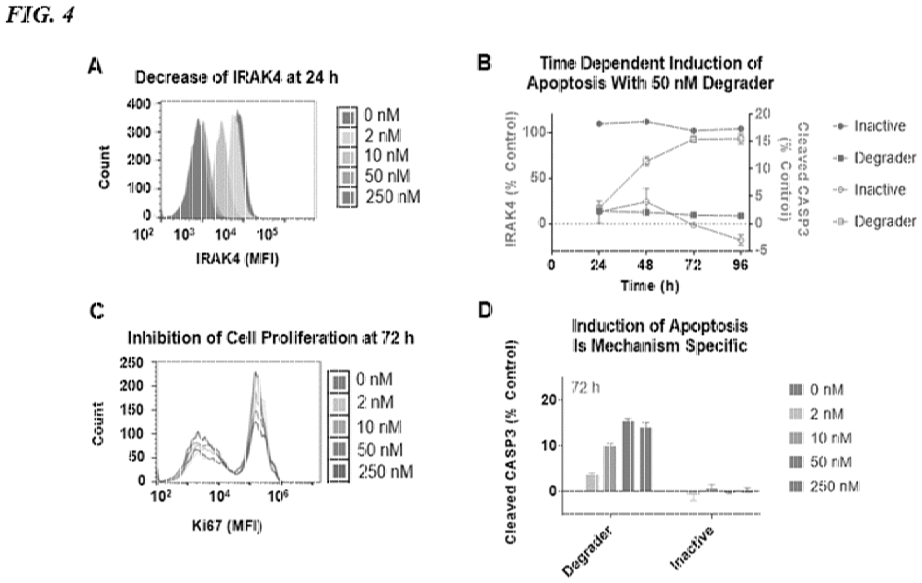

FIG. 4 includes graphical images showing the decrease of IRAK4 at 24h with count (y-axis) versus IRAK (MFI) (x-axis) using several concentrations of degrader **I-30** (A); the time dependent induction of apoptosis with 50 nM degrader **I-30** showing IRAK4 (% control) (left y-axis) and cleaved CASP3 (% control) (right y-axis) versus time (h) (x-axis) (B); the inhibition of cell proliferation at 72 h showing count (y-axis) versus Ki67 (MFI) (x-axis) at several concentrations of degrader **I-30** (C); and the induction of apoptosis is mechanism specific showing cleaved CASP3 (% control) (y-axis) for degrader **I-30** and inactive (x-axis) at several concentrations respectively (D).
FIG. 5 shows the results (CTG; IC₅₀ in µM) of the cellular viability assay using degrader **I-30** with OCI-LY10 and Daudi.

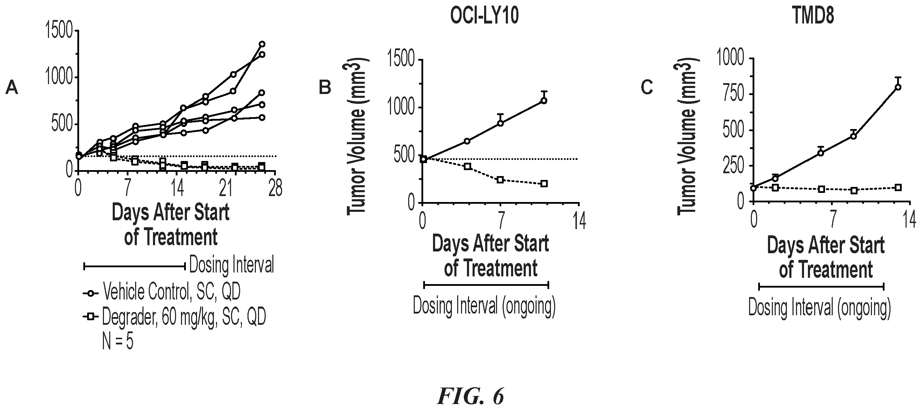

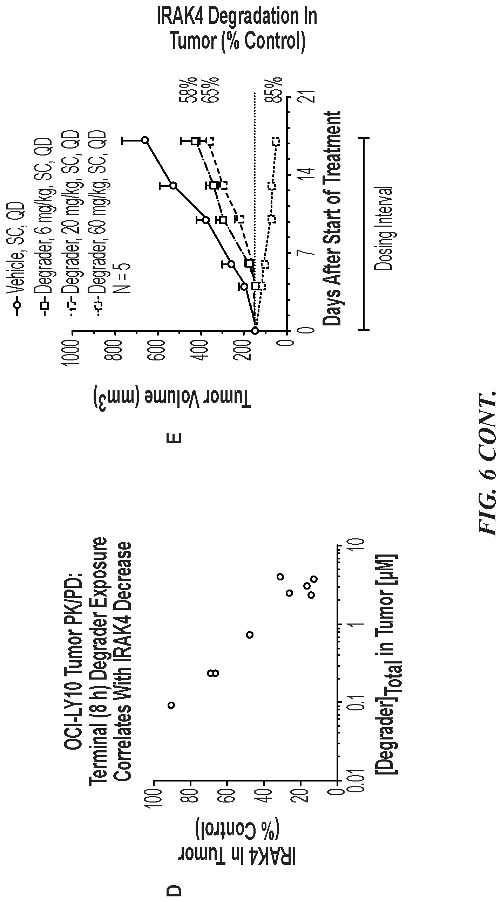

FIG. 6 includes graphical images of xenograft results for control (SC, QD) and degrader **I-30** (60 mg/kg SC, QD, N=5) showing tumor volume (mm³) (y-axis) versus dosing interval (days after start of treatment) (x-axis) for OCI-LY10 (A and B) and TMD8 cells (C); data showing terminal (8 h) degrader **I-30** exposure correlates with IRAK4 decrease in OCI-LY10 tumor PK/PD with IRAK4 in tumor (% control) (y-axis) versus total degrader concentration in tumor (µM) (D); and data showing tumor volume (mm³) (left y-axis) and IRAK4 **I-30** degradation in tumor (% control) (right y-axis) versus dosing interval (days after start of treatment) (E).
FIG. 7 is a graphical image of xenograph results for vehicle (PO, BID) and degrader **I-30** (PO, BID: 10 mg/kg; 30 mg/kg; and 100 mg/kg) showing tumor volume (mm³) (y-axis) versus dosing interval (days after start of treatment) (x-axis) for OCI-LY10 (MYD88 mutant) cells.
FIG. 8 is a graphical image showing in vivo degradation of IRAK4 in BALB/c mouse spleen following three PO doses of degrader **I-30** depicted as IRAK4 (% control) (y-axis) and PO doses (10 mg/kg; 30 mg/kg; and 100 mg/kg) (x-axis).
FIG. 9 is a graphical image depicting neotrophils in exudate collected from air pouch in mouse following MSU crystal challenge using showing neutrophiles count (% cells in extrudate) (y-axis) and (MSU; colchicine, 1 mg/kg SC; anakinra, 100 mg/kg SC; and PO **I-30** doses: 10 mg/kg; 30 mg/kg; and 100 mg/kg) (x-axis).
FIG. 10 is an image of a dose response curve for IKAK4 (% control) (y-axis) versus degrader **I-30** concentration (uM) (x-axis) for OCI-LY10 cell lines and in vitro degradation results (DC₉₀, µM).
FIG. 11 is a graphical image showing oral degrader **I-75** exposure in mouse with degrader concentration in plasma (ng/mL) (y-axis) over time (hours) (x-axis) at doses of 30 mg/kg, 100 mg/kg, and 300 mg/kg (A), PK characteristics in CD1 mice (B), and oral bioavailability across species (C).
FIG. 12 is are graphical image showing apoptosis in OCI-LY10 at 72h with Apoptotic Cells (CC3⁺/CPARP⁺, % Control) (y-axis) over several concentrations of 2 nM ibrutinib, degrader **I-30,** and the combination of 2 nM ibrutinib and degrader **I-30** (µM) (x-axis) (A); and the graphical results of a Cell Titer Glo assay showing synergism (B).
FIG. 13 is a graphical image of in vivo xenograph results for vehicle (PO, QD), degrader **I-30** (PO, QD, 25 mg/kg), ibrutinib (PO, QD, 25 mg/kg), and degrader + ibrutinib (PO, QD: 25 + 25 mg/kg), showing tumor volume (mm³) (y-axis) versus dosing interval (days after start of treatment) (x-axis) for OCI-LY10 (MYD88 L265P, CD79 mutant) cells.
FIG. 14 is a graphical image of in vivo xenograph results for vehicle (10% TPGS in water, PO, QD) and degrader **I-75** (PO, QD: 25 mg/kg and 50 mg/kg) showing tumor volume (mm³)(y-axis) versus dosing interval (days after start of treatment) (x-axis) for OCI-LY10 (MYD88 mutant) cells.
FIG. 15 is a graphical image of in vivo IL-1β testing following MSU crystal challenge showing IL-1β in plasma (pg/mL) (y-axis) and (Naive, Vehicle PO; and degrader **I-30** PO TID: 10 mg/kg; 30 mg/kg; and 100 mg/kg) (x-axis).
FIG. 16 includes graphical images of comparative TLR-stimulated pro-inflammatory cytokine inhibition results in vitro using a degrader **I-75** and IRAK4 inhibitor PF-06550833 for LPS (TLR4)-induced IL-6 (A), LPS (TLR4)-induced TNFα (B), R848 (TLR7/8)-induced IL-8 (C), and LPS (TLR4)-induced IL-1β (D) showing DMSO control (%) (y-axis) versus concentration (log µM) (x-axis) in human whole blood.
FIG. 17 includes graphical images of MSU induced gouty arthritis anti-inflammatory and analgesic results showing knee swelling (% control) (x-axis) and (Vehicle PO; colchicine SC; anakinra IP; and PO doses of degrader **I-75**: 10 mg/kg; 30 mg/kg; and 100 mg/kg) (x-axis) (A); and pain sensitivity (% control) (x-axis) and (Vehicle PO; colchicine SC; anakinra IP; and PO doses of degrader: 10 mg/kg; 30 mg/kg; and 100 mg/kg) (x-axis) (B).
FIG. 18 is a graphical image of IRAK4 degradation in OCI-LY10 tumor xenograft without regression for vehicle (PO, BID) and degrader **I-257** (PO, BID, 150 mg/kg), showing tumor volume (mm³) (y-axis) versus dosing interval (days after start of treatment) (x-axis).
FIG. 19 is a graphical image of viability results using PF-06650833, pomalidoamide, 1:1 PF-06650833:pomalidomide, and degrader **I-387** showing viability (% control) (y-axis) versus concentration (µM) (x-axis) for OCI-LY10 (MYD88 L265P, CD79 mutant) cells.
FIG. 20 includes transcriptome scatterplots of IFN signaling results showing -log 10 p-values of **I-257,** pomalidomide, and **I-208** in DMSO (y-axis).
FIG. 21 is a graphical image of IRAK4 and Ikaros degradation in OCI-LY10 tumor xenograft with regression for vehicle (PO, QD) and degrader **I-208** (PO, QD; 24, 72, and 240 mg/kg), showing tumor volume (mm³) (y-axis) versus dosing interval (days after start of treatment) (x-axis).
FIG. 22 shows pharmacokinetic results of **I-75** and **I-241.**
FIG. 23 is a graphical image of xenograph results for vehicle (SC, QD) and degrader **I-38** and **I-73** (SC, QD: 60 mg/kg) showing tumor volume (mm³) (y-axis) versus dosing interval (days after start of treatment) (x-axis) for OCI-LY10 (MYD88 mutant) cells.
FIG. 24 is a graphical image of xenograph results for vehicle (SC, 5 µL/g, QD) and degrader **I-3, I-11, I-62**, **1-10, I-113,** and **I-117** at various dosing regimens showing tumor volume (mm³) (y-axis) versus dosing interval (days after start of treatment) (x-axis) for OCI-LY10 (MYD88 mutant) cells.
FIG. 25 is a graphical image of xenograph results for vehicle (SC, 5 µL/g, QD) and degrader **I-41, I-61, 1-114, I-123, I-125, I-127,** and **I-241** at various dosing regimens showing tumor volume (mm³) (y-axis) versus dosing interval (days after start of treatment) (x-axis) for OCI-LY10 (MYD88 mutant) cells.
FIG. 26 is a graphical image of xenograph results for vehicle (PO, BID) and degrader **I-125** and **I-239** at various dosing regimens showing tumor volume (mm³) (y-axis) versus dosing interval (days after start of treatment) (x-axis) for OCI-LY10 (MYD88 mutant) cells.
FIG. 27 is a graphical image of xenograph results for vehicle (PO, QD) and degrader **I-167** and **I-259** at various dosing regimens showing tumor volume (mm³) (y-axis) versus dosing interval (days after start of treatment) (x-axis) for OCI-LY10 (MYD88 mutant) cells.
FIG. 28 is a graphical image of xenograph results for vehicle (PO, BID) and degrader **I-168** at various dosing regimens showing tumor volume (mm³) (y-axis) versus dosing interval (days after start of treatment) (x-axis) for OCI-LY10 (MYD88 mutant) cells.
FIG. 29 is a graphical image of xenograph results for vehicle (PO, QD) and degrader **I-221** (30 mg/kg, PO, QD) showing tumor volume (mm³) (y-axis) versus dosing interval (days after start of treatment) (x-axis) for OCI-LY10 (MYD88 mutant) cells.
FIG. 30 is a graphical image of xenograph results for vehicle (SC, 5 µL/g, QD) and degrader **I-168** at various dosing regimens showing tumor volume (mm³) (y-axis) versus dosing interval (days after start of treatment) (x-axis) for OCI-LY10 (MYD88 mutant) cells.
FIG. 31 is a graphical image of xenograph results for vehicle (PO, BID) and degrader **I-168** and **I-208** at various dosing regimens showing tumor volume (mm³) (y-axis) versus dosing interval (days post-randomization) (x-axis) for SUDHL-2 cells.
FIG. 32 is a graphical image depicting WBCs in exudate collected from air pouch in mouse following MSU crystal challenge using showing total cell count (% cells in extrudate) (y-axis) and (MSU; colchicine; 30, 100, and 300 mg/kg PO BID **I-417**; and 50 mg/kg PO BID **I-257**) (x-axis).
FIG. 33 is a graphical image depicting neutrophils in exudate collected from air pouch in mouse following MSU crystal challenge using showing total cell count (% cells in extrudate) (y-axis) and (MSU; colchicine; 30, 100, and 300 mg/kg PO BID **I-417**; and 50 mg/kg PO BID **I-257**) (x-axis).
FIG. 34 is a graphical image depicting IL-1b in exudate collected from air pouch in mouse following MSU crystal challenge using showing total cell count (% cells in extrudate) (y-axis) and (MSU; colchicine; 30, 100, and 300 mg/kg PO BID **I-417**; and 50 mg/kg PO BID **I-257**) (x-axis).
FIG. 35 includes graphical images depicting the effect of pretreatment with **I-417** on exudate TNFα content showing average TNFα (pg) (y-axes) and MSU, colchicine, and 30, 100, and 300 mg/kg PO **I-417** (x-axes).
FIG. 36 is a graphical image depicting mouse imiquimod (IMQ) induced skin inflammation showing change in ear thicknesss (µm) (y-axis) and vehicle, clobetasol, and I-417 (30, 100, and 300 mg/kg, PO, BID) (x-axis).
FIG. 37 includes graphical images depicting the results of the mouse intra peritoneal MSU induced peritonitis model showing lavage fluid IL-1b levels (left) and plasma IL-6 levels (right) (pg/mL) (y-axis) and naive, vehicle (PO), and 10, 30, and 100 mg/kg **I**-30 (PO TID) (x-axis).
FIG. 38 includes graphical images depicting the results of a PD study using **I-417** in wild-type mice showing IRAK4 level (y-axis) over time (hr) (x-axis) in skin (left) and spleen (right).

### DETAILED DESCRIPTION OF CERTAIN EMBODIMENTS

### 1. General Description of Certain Embodiments of the Invention:

Compounds of the present invention, and compositions thereof, are useful as degraders and/or inhibitors of one or more IRAK protein kinases. In some embodiments, a provided compound degrades and/or inhibits IRAK-1/2/3/4.

In certain embodiments, the present invention provides a compound of formula **I**: 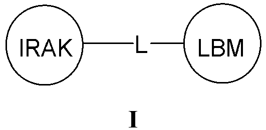 or a pharmaceutically acceptable salt thereof, wherein:
IRAK is an IRAK binding moiety capable of binding to one or more of IRAK-1, -2, -3, or -4;
L is a bivalent moiety that connects IRAK to LBM; and
LBM is a ligase binding moiety.

In certain embodiments, the present invention provides a compound of Formula **V**: 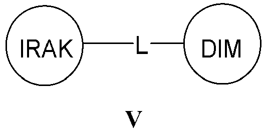 or a pharmaceutically acceptable salt thereof, wherein:
IRAK is an IRAK binding moiety capable of binding to one or more of IRAK-1, -2, -3, or -4;
L is a bivalent moiety that connects IRAK to DIM; and
DIM is a degradation inducing moiety.

### 2. Compounds and Definitions:

Compounds of the present invention include those described generally herein, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle," "cycloaliphatic" or "cycloalkyl"), that has a single point of attachment to the rest of the molecule. Unless otherwise specified, aliphatic groups contain 1-6 aliphatic carbon atoms. In some embodiments, aliphatic groups contain 1-5 aliphatic carbon atoms. In other embodiments, aliphatic groups contain 1-4 aliphatic carbon atoms. In still other embodiments, aliphatic groups contain 1-3 aliphatic carbon atoms, and in yet other embodiments, aliphatic groups contain 1-2 aliphatic carbon atoms. In some embodiments, "cycloaliphatic" (or "carbocycle" or "cycloalkyl") refers to a monocyclic C₃-C₆ hydrocarbon that is completely saturated or that contains one or more units of unsaturation, but which is not aromatic, that has a single point of attachment to the rest of the molecule. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

As used herein, the term "bridged bicyclic" refers to any bicyclic ring system, i.e. carbocyclic or heterocyclic, saturated or partially unsaturated, having at least one bridge. As defined by IUPAC, a "bridge" is an unbranched chain of atoms or an atom or a valence bond connecting two bridgeheads, where a "bridgehead" is any skeletal atom of the ring system which is bonded to three or more skeletal atoms (excluding hydrogen). In some embodiments, a bridged bicyclic group has 7-12 ring members and 0-4 heteroatoms independently selected from nitrogen, oxygen, or sulfur. Such bridged bicyclic groups are well known in the art and include those groups set forth below where each group is attached to the rest of the molecule at any substitutable carbon or nitrogen atom. Unless otherwise specified, a bridged bicyclic group is optionally substituted with one or more substituents as set forth for aliphatic groups. Additionally or alternatively, any substitutable nitrogen of a bridged bicyclic group is optionally substituted. Exemplary bridged bicyclics include:

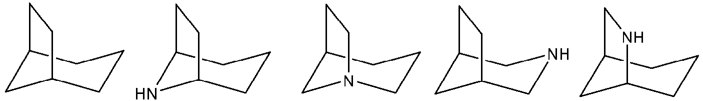

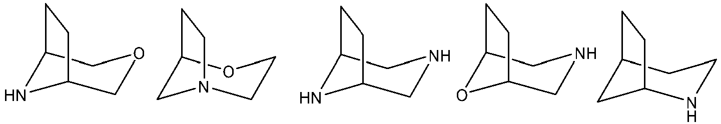

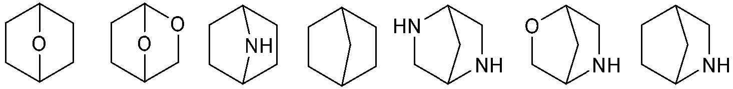

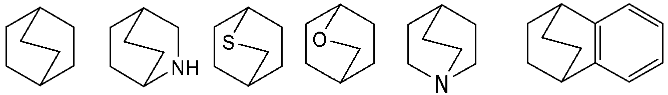

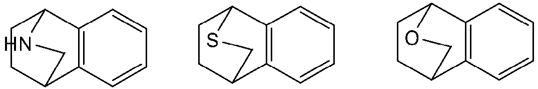

The term "lower alkyl" refers to a C₁₋₄ straight or branched alkyl group. Exemplary lower alkyl groups are methyl, ethyl, propyl, isopropyl, butyl, isobutyl, and tert-butyl.

The term "lower haloalkyl" refers to a C₁₋₄ straight or branched alkyl group that is substituted with one or more halogen atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or NR⁺ (as in N-substituted pyrrolidinyl)).

The term "unsaturated," as used herein, means that a moiety has one or more units of unsaturation.

As used herein, the term "bivalent C₁₋₈ (or C₁₋₆) saturated or unsaturated, straight or branched, hydrocarbon chain", refers to bivalent alkylene, alkenylene, and alkynylene chains that are straight or branched as defined herein.

The term "alkylene" refers to a bivalent alkyl group. An "alkylene chain" is a polymethylene group, i.e., -(CH₂)ₙ-, wherein n is a positive integer, preferably from 1 to 6, from 1 to 4, from 1 to 3, from 1 to 2, or from 2 to 3. A substituted alkylene chain is a polymethylene group in which one or more methylene hydrogen atoms are replaced with a substituent. Suitable substituents include those described below for a substituted aliphatic group.

The term "alkenylene" refers to a bivalent alkenyl group. A substituted alkenylene chain is a polymethylene group containing at least one double bond in which one or more hydrogen atoms are replaced with a substituent. Suitable substituents include those described below for a substituted aliphatic group.

As used herein, the term "cyclopropylenyl" refers to a bivalent cyclopropyl group of the following structure:

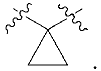

The term "halogen" means F, Cl, Br, or I.

The term "aryl" used alone or as part of a larger moiety as in "aralkyl," "aralkoxy," or "aryloxyalkyl," refers to monocyclic or bicyclic ring systems having a total of five to fourteen ring members, wherein at least one ring in the system is aromatic and wherein each ring in the system contains 3 to 7 ring members. The term "aryl" may be used interchangeably with the term "aryl ring." In certain embodiments of the present invention, "aryl" refers to an aromatic ring system which includes, but not limited to, phenyl, biphenyl, naphthyl, anthracyl and the like, which may bear one or more substituents. Also included within the scope of the term "aryl," as it is used herein, is a group in which an aromatic ring is fused to one or more non-aromatic rings, such as indanyl, phthalimidyl, naphthimidyl, phenanthridinyl, or tetrahydronaphthyl, and the like.

The terms "heteroaryl" and "heteroar-," used alone or as part of a larger moiety, e.g., "heteroaralkyl," or "heteroaralkoxy," refer to groups having 5 to 10 ring atoms, preferably 5, 6, or 9 ring atoms; having 6, 10, or 14 π electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, and pteridinyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring. Nonlimiting examples include indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, and pyrido[2,3-b]-1,4-oxazin-3(4H)-one. A heteroaryl group may be mono- or bicyclic. The term "heteroaryl" may be used interchangeably with the terms "heteroaryl ring," "heteroaryl group," or "heteroaromatic," any of which terms include rings that are optionally substituted. The term "heteroaralkyl" refers to an alkyl group substituted by a heteroaryl, wherein the alkyl and heteroaryl portions independently are optionally substituted.

As used herein, the terms "heterocycle," "heterocyclyl," "heterocyclic radical," and "heterocyclic ring" are used interchangeably and refer to a stable 5- to 7-membered monocyclic or 7-10-membered bicyclic heterocyclic moiety that is either saturated or partially unsaturated, and having, in addition to carbon atoms, one or more, preferably one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2*H*-pyrrolyl), NH (as in pyrrolidinyl), or ⁺NR (as in N-substituted pyrrolidinyl).

A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, tetrahydrofuranyl, tetrahydrothiophenyl pyrrolidinyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, and quinuclidinyl. The terms "heterocycle," "heterocyclyl," "heterocyclyl ring," "heterocyclic group," "heterocyclic moiety," and "heterocyclic radical," are used interchangeably herein, and also include groups in which a heterocyclyl ring is fused to one or more aryl, heteroaryl, or cycloaliphatic rings, such as indolinyl, 3*H*-indolyl, chromanyl, phenanthridinyl, or tetrahydroquinolinyl. A heterocyclyl group may be mono- or bicyclic. The term "heterocyclylalkyl" refers to an alkyl group substituted by a heterocyclyl, wherein the alkyl and heterocyclyl portions independently are optionally substituted.

As used herein, the term "partially unsaturated" refers to a ring moiety that includes at least one double or triple bond. The term "partially unsaturated" is intended to encompass rings having multiple sites of unsaturation, but is not intended to include aryl or heteroaryl moieties, as herein defined.

As described herein, compounds of the invention may contain "optionally substituted" moieties. In general, the term "substituted," whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group are independently halogen; -(CH₂)₀₋₄R°; -(CH₂)₀₋₄OR°; -O(CH₂)₀₋₄R°, -O-(CH₂)₀₋₄C(O)OR°; -(CH₂)₀₋₄CH(OR°)₂; -(CH₂)₀₋₄SR°; -(CH₂)₀₋₄Ph, which may be substituted with R°; -(CH₂)₀₋₄O(CH₂)₀₋₁Ph which may be substituted with R°; -CH=CHPh, which may be substituted with R°; -(CH₂)₀₋₄O(CH₂)₀₋₁-pyridyl which may be substituted with R°; -NO₂; -CN; -N₃; -(CH₂)₀₋₄N(R°)₂; -(CH₂)₀₋₄N(R°)C(O)R°; -N(R°)C(S)R°; -(CH₂)₀₋₄N(R°)C(O)NR°₂; -N(R°)C(S)NR°₂; -(CH₂)₀₋₄N(R°)C(O)OR°; - N(R°)N(R°)C(O)R°; -N(R°)N(R°)C(O)NR°₂; -N(R°)N(R°)C(O)OR°; -(CH₂)₀₋₄C(O)R°; - C(S)R°; -(CH₂)₀₋₄C(O)OR°; -(CH₂)₀₋₄C(O)SR°; -(CH₂)₀₋₄C(O)OSiR°₃; -(CH₂)₀₋₄OC(O)R°; - OC(O)(CH₂)₀₋₄SR-, SC(S)SR°; -(CH₂)₀₋₄SC(O)R°; -(CH₂)₀₋₄C(O)NR°₂; -C(S)NR°₂; -C(S)SR°; -SC(S)SR°, -(CH₂)₀₋₄OC(O)NR°₂; -C(O)N(OR°)R°; -C(O)C(O)R°; -C(O)CH₂C(O)R°; - C(NOR°)R°; -(CH₂)₀₋₄SSR°; -(CH₂)₀₋₄S(O)₂R°; -(CH₂)₀₋₄S(O)₂OR°; -(CH₂)₀₋₄OS(O)₂R°; - S(O)₂NR°₂; -(CH₂)₀₋₄S(O)R°; -N(R°)S(O)₂NR°₂; -N(R°)S(O)₂R°; -N(OR°)R°; -C(NH)NR°₂; - P(O)₂R°; -P(O)R°₂; -OP(O)R°₂; -OP(O)(OR°)₂; SiR°₃; -(C₁₋₄ straight or branched alkylene)ON(R°)₂; or -(C₁₋₄ straight or branched alkylene)C(O)O-N(R°)₂, wherein each R° may be substituted as defined below and is independently hydrogen, C₁₋₆ aliphatic, -CH₂Ph, -O(CH₂)₀₋₁Ph, -CH₂-(5-6 membered heteroaryl ring), or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, -(CH₂)₀₋₂R^{•}, -(haloR*), -(CH₂)₀₋₂OH, -(CH₂)₀₋₂OR^{•}, -(CH₂)₀₋₂CH(OR^{•})₂; -O(haloR^{•}), -CN, -N₃, -(CH₂)₀₋₂C(O)R^{•}, -(CH₂)₀₋₂C(O)OH, -(CH₂)₀₋₂C(O)OR^{•}, -(CH₂)₀₋₂SR^{•}, -(CH₂)₀₋₂SH, -(CH₂)₀₋₂NH₂, - (CH₂)₀₋₂NHR^{•}, -(CH₂)₀₋₂NR^{•}₂, -NO₂, -SiR^{•}₃, -OSiR^{•}₃, -C(O)SR^{•}, -(C₁₋₄ straight or branched alkylene)C(O)OR^{•}, or -SSR^{•} wherein each R^{•} is unsubstituted or where preceded by "halo" is substituted only with one or more halogens, and is independently selected from C₁₋₄ aliphatic, - CH₂Ph, -O(CH₂)₀₋₁Ph, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, =NNR^{*}₂, =NNHC(O)R^{*}, =NNHC(O)OR^{*}, =NNHS(O)₂R^{*}, =NR^{*}, =NOR*, -O(C(R^{*}₂))₂₋₃O-, or -S(C(R^{*}₂))₂₋₃S-, wherein each independent occurrence of R* is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR^{*}₂)₂₋₃O-, wherein each independent occurrence of R* is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R^{*} include halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, - C(O)CH₂C(O)R^{†}, -S(O)₂R^{†}, -S(O)₂NR^{†}₂, -C(S)NR^{†}₂, -C(NH)NR^{†}₂, or -N(R^{†})S(O)₂R^{†}; wherein each R^{†} is independently hydrogen, C₁₋₆ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3-12-membered saturated, partially unsaturated, or aryl mono- or bicyclic ring having 0-4 heteroatoms independently selected from nitrogen, oxygen, or sulfur.

Suitable substituents on the aliphatic group of R^{†} are independently halogen, - R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur.

As used herein, the term "provided compound" refers to any genus, subgenus, and/or species set forth herein.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention

As used herein, the term "inhibitor" is defined as a compound that binds to and /or inhibits an IRAK kinase with measurable affinity. In certain embodiments, an inhibitor has an IC₅₀ and/or binding constant of less than about 50 µM, less than about 1 µM, less than about 500 nM, less than about 100 nM, less than about 10 nM, or less than about 1 nM.

As used herein, the term "degrader" is defined as a heterobifunctional compound that binds to and /or inhibits both an IRAK kinase and an E3 ligase with measurable affinity resulting in the ubiqitination and subsequent degradation of the IRAK kinase. In certain embodiments, a degrader has an DC₅₀ of less than about 50 µM, less than about 1 µM, less than about 500 nM, less than about 100 nM, less than about 10 nM, or less than about 1 nM. As used herein, the term "monovalent" refers to a degrader compound without an appended E3 ligase binding moiety.

A compound of the present invention may be tethered to a detectable moiety. It will be appreciated that such compounds are useful as imaging agents. One of ordinary skill in the art will recognize that a detectable moiety may be attached to a provided compound *via* a suitable substituent. As used herein, the term "suitable substituent" refers to a moiety that is capable of covalent attachment to a detectable moiety. Such moieties are well known to one of ordinary skill in the art and include groups containing, e.g., a carboxylate moiety, an amino moiety, a thiol moiety, or a hydroxyl moiety, to name but a few. It will be appreciated that such moieties may be directly attached to a provided compound or *via* a tethering group, such as a bivalent saturated or unsaturated hydrocarbon chain. In some embodiments, such moieties may be attached via click chemistry. In some embodiments, such moieties may be attached via a 1,3-cycloaddition of an azide with an alkyne, optionally in the presence of a copper catalyst. Methods of using click chemistry are known in the art and include those described by Rostovtsev et al., Angew. Chem. Int. Ed. 2002, 41, 2596-99 and Sun et al., Bioconjugate Chem., 2006, 17, 52-57.

As used herein, the term "detectable moiety" is used interchangeably with the term "label" and relates to any moiety capable of being detected, e.g., primary labels and secondary labels. Primary labels, such as radioisotopes (e.g., tritium, ³²P, ³³P, ³⁵S, or ¹⁴C), mass-tags, and fluorescent labels are signal generating reporter groups which can be detected without further modifications. Detectable moieties also include luminescent and phosphorescent groups.

The term "secondary label" as used herein refers to moieties such as biotin and various protein antigens that require the presence of a second intermediate for production of a detectable signal. For biotin, the secondary intermediate may include streptavidin-enzyme conjugates. For antigen labels, secondary intermediates may include antibody-enzyme conjugates. Some fluorescent groups act as secondary labels because they transfer energy to another group in the process of nonradiative fluorescent resonance energy transfer (FRET), and the second group produces the detected signal.

The terms "fluorescent label", "fluorescent dye", and "fluorophore" as used herein refer to moieties that absorb light energy at a defined excitation wavelength and emit light energy at a different wavelength. Examples of fluorescent labels include, but are not limited to: Alexa Fluor dyes (Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), AMCA, AMCA-S, BODIPY dyes (BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), Carboxyrhodamine 6G, carboxy-X-rhodamine (ROX), Cascade Blue, Cascade Yellow, Coumarin 343, Cyanine dyes (Cy3, Cy5, Cy3.5, Cy5.5), Dansyl, Dapoxyl, Dialkylaminocoumarin, 4',5'-Dichloro-2',7'-dimethoxy-fluorescein, DM-NERF, Eosin, Erythrosin, Fluorescein, FAM, Hydroxycoumarin, IRDyes (IRD40, IRD 700, IRD 800), JOE, Lissamine rhodamine B, Marina Blue, Methoxycoumarin, Naphthofluorescein, Oregon Green 488, Oregon Green 500, Oregon Green 514, Pacific Blue, PyMPO, Pyrene, Rhodamine B, Rhodamine 6G, Rhodamine Green, Rhodamine Red, Rhodol Green, 2',4',5',7'-Tetra-bromosulfone-fluorescein, Tetramethylrhodamine (TMR), Carboxytetramethylrhodamine (TAMRA), Texas Red, Texas Red-X.

The term "mass-tag" as used herein refers to any moiety that is capable of being uniquely detected by virtue of its mass using mass spectrometry (MS) detection techniques. Examples of mass-tags include electrophore release tags such as N-[3-[4'-[(p-Methoxytetrafluorobenzyl)oxy]phenyl]-3-methylglyceronyl]isonipecotic Acid, 4'-[2,3,5,6-Tetrafluoro-4-(pentafluorophenoxyl)]methyl acetophenone, and their derivatives. The synthesis and utility of these mass-tags is described in United States Patents 4,650,750, 4,709,016, 5,360,8191, 5,516,931, 5,602,273, 5,604,104, 5,610,020, and 5,650,270. Other examples of mass-tags include, but are not limited to, nucleotides, dideoxynucleotides, oligonucleotides of varying length and base composition, oligopeptides, oligosaccharides, and other synthetic polymers of varying length and monomer composition. A large variety of organic molecules, both neutral and charged (biomolecules or synthetic compounds) of an appropriate mass range (100-2000 Daltons) may also be used as mass-tags.

The terms "measurable affinity" and "measurably inhibit," as used herein, means a measurable change in an IRAK protein kinase activity between a sample comprising a compound of the present invention, or composition thereof, and an IRAK protein kinase, and an equivalent sample comprising an IRAK protein kinase, in the absence of said compound, or composition thereof.

### 3. Description of Exemplary Embodiments:

As described above, in certain embodiments, the present invention provides a compound of formula **I**: 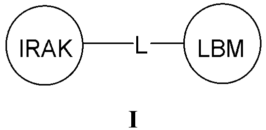 or a pharmaceutically acceptable salt thereof, wherein:
IRAK is an IRAK binding moiety capable of binding to one or more of IRAK-1, -2, -3, or -4;
L is a bivalent moiety that connects IRAK to LBM; and
LBM is a ligase binding moiety.

In some embodiments, the present invention provides a compound of formula **I**: 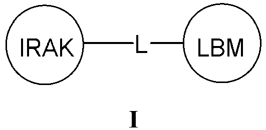 or a pharmaceutically acceptable salt thereof, wherein:
IRAK is an IRAK-4 binding moiety;
L is a bivalent moiety that connects IRAK to LBM; and
LBM is a cereblon ligase binding moiety.

As described above, in certain embodiments, the present invention provides a compound of formula **V**: 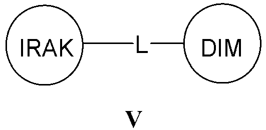 or a pharmaceutically acceptable salt thereof, wherein:
IRAK is an IRAK binding moiety capable of binding to one or more of IRAK-1, -2, -3, or -4;
L is a bivalent moiety that connects IRAK to DIM; and
DIM is a degradation inducing moiety.

In some embodiments, the present invention provides a compound of formula **V**: 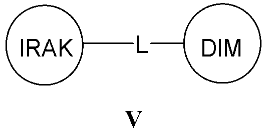 or a pharmaceutically acceptable salt thereof, wherein:
IRAK is an IRAK-4 binding moiety;
L is a bivalent moiety that connects IRAK to DIM; and
DIM is LBM, a lysine mimetic, or a hydrogen atom.

### IRAK Binding Moeity (IRAK)

In certain embodiments, the present invention provides a compound of formula **I,** where IRAK is a IRAK-4 binding moiety thereby forming a compound of formula **II**: 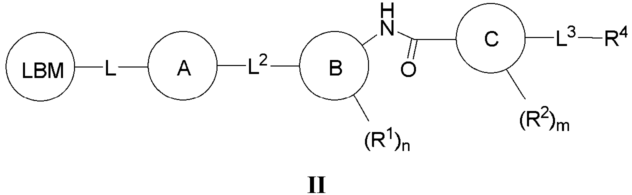 or a pharmaceutically acceptable salt thereof, wherein L and LBM are as defined above and described in embodiments herein, and wherein:
Ring A is a 4-10 membered saturated mono- or bicyclic carbocyclic or hetereocyclic ring having 0-2 heteroatoms independently selected from nitrogen, oxygen, and sulfur;
Ring B is phenyl, a 4-10 membered saturated or partially unsaturated mono- or bicyclic carbocyclic or heterocyclic ring having 1-3 heteroatoms independently selected from nitrogen, oxygen, and sulfur, or a 5-9 membered mono- or bicyclic heteroaryl ring having 1-4 heteroatoms independently selected from nitrogen, oxygen, and sulfur;
Ring C is phenyl or a 5-10 membered mono- or bicyclic heteroaryl ring having 1-5 heteroatoms independently selected from nitrogen, oxygen, and sulfur;
each of L² and L³ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
R⁴ is selected from 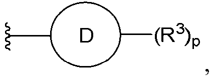 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur;
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4; and
each p is 0, 1, 2, 3 or 4;
wherein the compound of formula **II** is not compound **I-99** or **I-100** in **Table 1A.**

In certain embodiments, the present invention provides a compound of Formula **II'**: 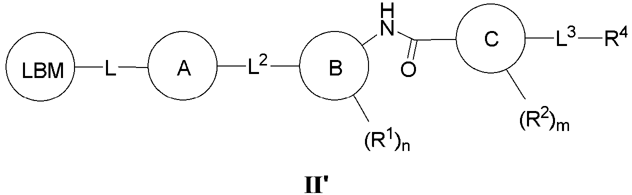 or a pharmaceutically acceptable salt thereof, wherein:
Ring A is an optionally substituted 4-10 membered saturated mono- or bicyclic carbocyclic or heterocyclic ring having 0-2 heteroatoms independently selected from nitrogen, oxygen, and sulfur;
Ring B is phenyl, a 4-7 membered saturated or partially unsaturated carbocyclic ring or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
Ring C is phenyl, a 4-7 membered saturated or partially unsaturated carbocyclic ring or heterocyclic ring having 1-3 heteroatoms independently selected from nitrogen, oxygen, and sulfur, or a 5-6 membered heteroaryl ring having 1-5 heteroatoms independently selected from nitrogen, oxygen, and sulfur;
each of L² and L³ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂, or two R¹ on the same carbon together form =O or =S;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R, or two R² on the same carbon together form =O or =S;
R⁴ is selected from hydrogen or 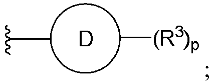
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R, or two R³ on the same carbon together form =O or =S;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4;
each p is 0, 1, 2, 3 or 4;

In certain embodiments, the present invention provides a compound of formula **V,** where IRAK is a IRAK-4 binding moiety thereby forming a compound of formula **V-a**: 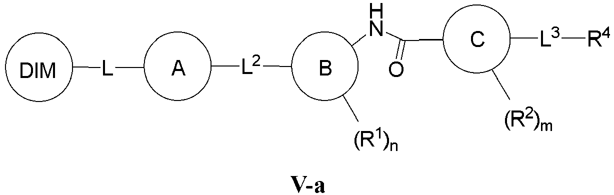 or a pharmaceutically acceptable salt thereof, wherein L and DIM are as defined above and described in embodiments herein, and wherein:
Ring A is a 4-10 membered saturated mono- or bicyclic carbocyclic or hetereocyclic ring having 0-2 heteroatoms independently selected from nitrogen, oxygen, and sulfur;
Ring B is phenyl, a 4-10 membered saturated or partially unsaturated mono- or bicyclic carbocyclic or heterocyclic ring having 1-3 heteroatoms independently selected from nitrogen, oxygen, and sulfur, or a 5-9 membered mono- or bicyclic heteroaryl ring having 1-4 heteroatoms independently selected from nitrogen, oxygen, and sulfur;
Ring C is phenyl or a 5-10 membered mono- or bicyclic heteroaryl ring having 1-5 heteroatoms independently selected from nitrogen, oxygen, and sulfur;
each of L² and L³ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
R⁴ is selected from 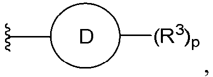 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur;
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R⁵ is independently an optionally substituted group selected from C₁₋₆ aliphatic, phenyl, a 3-7 membered saturated or partially unsaturated carboyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4; and
each p is 0, 1, 2, 3 or 4;
wherein the compound of formula **V-a** is not compound **I-99** or **I-100** in **Table 1A.**

The below embodiments found in paragraphs [0084] to [00140] are to compounds of formula **II, II'** and **V-a.**

As defined generally above, Ring A is a 4-10 membered saturated mono- or bicyclic carbocyclic or hetereocyclic ring having 0-2 heteroatoms independently selected from nitrogen, oxygen, and sulfur.

In some embodiments, Ring A is cyclobutyl. In some embodiments, Ring A is cyclopentyl. In some embodiments, Ring A is cyclohexyl. In some embodiments, Ring A is cycloheptyl. In some embodiments, Ring A is 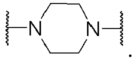 In some embodiments, Ring A is

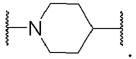

In some embodiments, Ring A is selected from those depicted in **Table 1,** below.

As generally defined above, Ring B is phenyl, a 4-10 membered saturated or partially unsaturated mono- or bicyclic carbocyclic or heterocyclic ring having 1-3 heteroatoms independently selected from nitrogen, oxygen, and sulfur, or a 5-9 membered mono- or bicyclic heteroaryl ring having 1-4 heteroatoms independently selected from nitrogen, oxygen, and sulfur.

In some embodiments, Ring B is phenyl. In some embodiments, Ring B is a 4-10 membered saturated or partially unsaturated mono- or bicyclic carbocyclic or heterocyclic ring having 1-3 heteroatoms independently selected from nitrogen, oxygen, and sulfur. In some embodiments, Ring B is a 5-9 membered mono- or bicyclic heteroaryl ring having 1-4 heteroatoms independently selected from nitrogen, oxygen, and sulfur.

In some embodiments, Ring B is 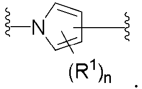 In some embodiments, Ring B is 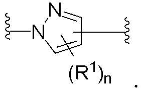 In some embodiments, Ring B is 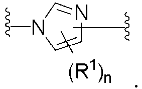 In some embodiments, Ring B is 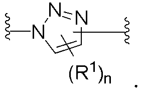 In some embodiments, Ring B is 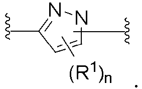 In some embodiments, Ring B is

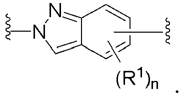

As defined generally above, Ring C is phenyl or a 5-10 membered mono- or bicyclic heteroaryl ring having 1-5 heteroatoms independently selected from nitrogen, oxygen, and sulfur.

In some embodiments, Ring C is phenyl. In some embodiments, Ring C is a 5-10 membered mono- or bicyclic heteroaryl ring having 1-5 heteroatoms independently selected from nitrogen, oxygen, and sulfur.

In some embodiments, Ring C is 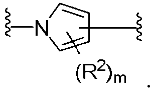 In some embodiments, Ring C is 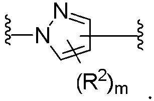 In some embodiments, Ring C is 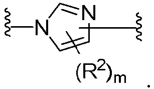 In some embodiments, Ring C is 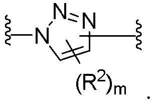 In some embodiments, Ring C is 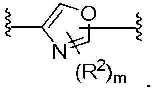 In some embodiments, Ring C is 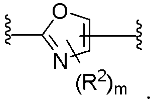 In some embodiments, Ring C is 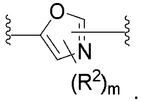 In some embodiments, Ring C is 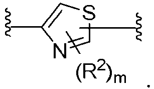 In some embodiments, Ring C is 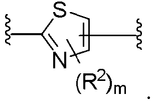 In some embodiments, Ring C is

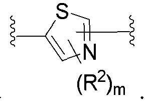

In some embodiments, Ring C is 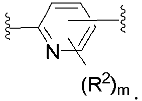 In some embodiments, Ring C is 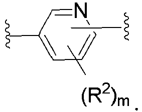 In some embodiments, Ring C is 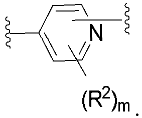 In some embodiments, Ring C is 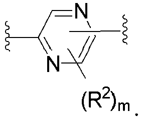 In some embodiments, Ring C is 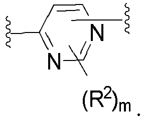 In some embodiments, Ring C is 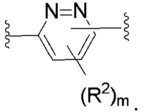 In some embodiments, Ring C is 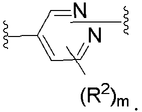 In some embodiments, Ring C is

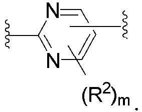

In some embodiments, Ring C is 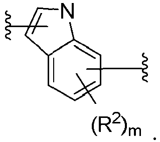 In some embodiments, Ring C is 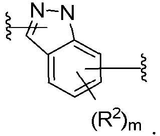 In some embodiments, Ring C is 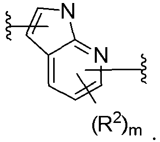 In some embodiments, Ring C is 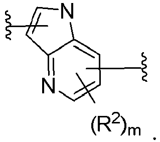 In some embodiments, Ring C is 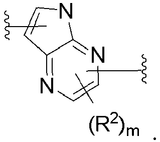 In some embodiments, Ring C is 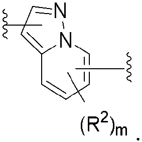 In some embodiments, Ring C is

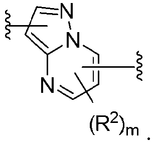

In some embodiments, Ring C is selected from those depicted in **Table 1,** below.

As generally defined above, L² is a bivalent moiety selected from a covalent bond or a C₁₋₃ bivalent straight or branched saturated or unsaturated hydrocarbon chain wherein 1-3 methylene units of the chain are independently and optionally replaced with -O-, -C(O)-, -C(S)-, - C(R)₂-, -CH(R)-, -C(F)₂-, -N(R)-, -S-, -S(O)₂- or -CR=CR-.

In some embodiments, L² a covalent bond. In some embodiments, L² is a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, - C(F)₂-, -N(R)-, -S-, -S(O)₂- or -CR=CR-. In some embodiments, L² is a C₁₋₃ aliphatic. In some embodiments, L² is -CH₂-. In some embodiments, L² is -C(D)(H)-. In some embodiments, L² is -C(D)₂-. In some embodiments, L² is -CH₂CH₂-. In some embodiments, L² is -NR-. In some embodiments, L² is -CH₂NR-. In some embodiments, L² is or -O-. In some embodiments, L² is -CH₂O-. In some embodiments, L² is -S-. In some embodiments, L² is -OC(O)-. In some embodiments, L² is -C(O)O-. In some embodiments, L² is -C(O)-. In some embodiments, L² is - S(O)-. In some embodiments, L² is -S(O)₂-,. In some embodiments, L² is -NRS(O)₂-. In some embodiments, L² is -S(O)₂NR-. In some embodiments, L² is -NRC(O)-. In some embodiments, L² is -C(O)NR-. In some embodiments, L² is -OC(O)NR-. In some embodiments, L² is - NRC(O)O-.

As generally defined above, L³ is a bivalent moiety selected from a covalent bond or a C₁₋₃ bivalent straight or branched saturated or unsaturated hydrocarbon chain wherein 1-3 methylene units of the chain are independently and optionally replaced with -O-, -C(O)-, -C(S)-, - C(R)₂-, -CH(R)-, -C(F)₂-, -N(R)-, -S-, -S(O)₂- or -CR=CR-.

In some embodiments, L³ is a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-. In some embodiments, L³ is a C₁₋₃ aliphatic. In some embodiments, L³ is -CH₂-. In some embodiments, L³ is -C(D)(H)-. In some embodiments, L³ is -C(D)₂-. In some embodiments, L³ is -CH₂CH₂-. In some embodiments, L³ is -NR-. In some embodiments, L³ is -CH₂NR-. In some embodiments, L³ is or -O-. In some embodiments, L³ is -CH₂O-. In some embodiments, L³ is -S-. In some embodiments, L³ is -OC(O)-. In some embodiments, L³ is -C(O)O-. In some embodiments, L³ is -C(O)-. In some embodiments, L³ is -S(O)-. In some embodiments, L³ is - S(O)₂-,. In some embodiments, L³ is -NRS(O)₂-. In some embodiments, L³ is -S(O)₂NR-. In some embodiments, L³ is -NRC(O)-. In some embodiments, L³ is -C(O)NR-. In some embodiments, L³ is -OC(O)NR-. In some embodiments, L³ is -NRC(O)O-.

In some embodiments, L² and L³ are selected from those depicted in **Table 1,** below.

As defined generally above, each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), -CFR₂, - CF₃, -CR₂(OR), -CR₂(NR₂), -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, -N(R)S(O)₂R, -N⁺(O⁻)R₂, -OP(O)R₂, -OP(O)(OR)₂, -OP(O)(OR)NR₂, -OP(O)(NR₂)₂, -P(O)R₂, - SiR₃, -Si(OR)R₂, -SF₅, or

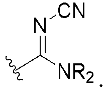

In some embodiments, each R¹ is independently hydrogen. In some embodiments, R¹ is deuterium. In some embodiments, each R¹ is independently -R⁵. In some embodiments, each R¹ is independently halogen. In some embodiments, each R¹ is independently -CN. In some embodiments, each R¹ is independently -NO₂. In some embodiments, each R¹ is independently - OR. In some embodiments, each R¹ is independently -SR. In some embodiments, each R¹ is independently -NR₂. In some embodiments, each R¹ is independently -S(O)₂R. In some embodiments, each R¹ is independently -S(O)₂NR₂. In some embodiments, each R¹ is independently -S(O)R. In some embodiments, each R¹ is independently -S(O)(NR)R. In some embodiments, each R¹ is independently -P(O)(OR)₂. In some embodiments, each R¹ is independently -P(O)(NR₂)₂. In some embodiments, each R¹ is independently -CF₂(R). In some embodiments, each R¹ is independently -CFR₂. In some embodiments, each R¹ is independently - CF₃. In some embodiments, each R¹ is independently -CR₂(OR). In some embodiments, each R¹ is independently -CR₂(NR₂). In some embodiments, each R¹ is independently -C(O)R. In some embodiments each R¹ is independently -C(O)OR. In some embodiments, each R¹ is independently -C(O)NR₂. In some embodiments, each R¹ is independently -C(O)N(R)OR. In some embodiments, each R¹ is independently -OC(O)R. In some embodiments, each R¹ is independently -OC(O)NR₂. In some embodiments, each R¹ is independently -N(R)C(O)OR. In some embodiments, each R¹ is independently -N(R)C(O)R. In some embodiments, each R¹ is independently -N(R)C(O)NR₂. In some embodiments, each R¹ is independently -N(R)S(O)₂R. In some embodiments, each R¹ is independently -N⁺(O⁻)R₂. In some embodiments, each R¹ is independently -OP(O)R₂. In some embodiments, each R¹ is independently -OP(O)(OR)₂. In some embodiments, each R¹ is independently -OP(O)(OR)NR₂. In some embodiments, each R¹ is independently -OP(O)(NR₂)₂. In some embodiments, each R¹ is independently -P(O)R₂. In some embodiments, each R¹ is independently -SiR₃. In some embodiments, each R¹ is independently - Si(OR)R₂. In some embodiments, each R¹ is independently -SF₅. In some embodiments, each R¹ is independently

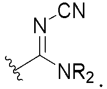

In some embodiments, R¹ is -CHF₂. In some embodiments, R¹ is -C(OH)(CH₃)₂. In some embodiments, R¹ is -C(O)NH₂. In some embodiments, R¹ is -CF₃. In some embodiments, R¹ is -iPr. In some embodiments, R¹ is isoprene. In some embodiments, R¹ is 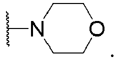 In some embodiments, R¹ is

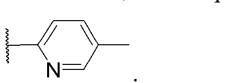

As defined generally above, each R² and R³ are independently hydrogen, deuterium, - R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), -CFR₂, - CF₃, -CR₂(OR), -CR₂(NR₂), -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, - N(R)S(O)₂R, -N⁺(O⁻)R₂, -OP(O)R₂, -OP(O)(OR)₂, -OP(O)(OR)NR₂, -OP(O)(NR₂)₂, -P(O)R₂, - SiR₃, -Si(OR)R₂, -SF₅, or

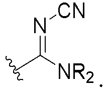

In some embodiments, each R² and R³ are independently hydrogen. In some embodiments, each R² and R³ are independently deuterium. In some embodiments, each R² and R³ are independently -R⁵. In some embodiments, each R² and R³ are independently halogen. In some embodiments, each R² and R³ are independently -CN. In some embodiments, each R² and R³ are independently -NO₂. In some embodiments, each R² and R³ are independently -OR. In some embodiments, each R² and R³ are independently -SR. In some embodiments, each R² and R³ are independently -NR₂. In some embodiments, each R² and R³ are independently -S(O)₂R. In some embodiments, each R² and R³ are independently -S(O)₂NR₂. In some embodiments, each R² and R³ are independently -S(O)R. In some embodiments, each R² and R³ are independently -S(O)(NR)R. In some embodiments, each R² and R³ are independently -P(O)(OR)₂. In some embodiments, each R² and R³ are independently -P(O)(NR₂)₂. In some embodiments, each R² and R³ are independently -CF₂(R). In some embodiments, each R² and R³ are independently -CFR₂. In some embodiments, each R² and R³ are independently -CF₃. In some embodiments, each R² and R³ are independently -CR₂(OR). In some embodiments, each R² and R³ are independently -CR₂(NR₂). In some embodiments, each R² and R³ are independently -C(O)R. In some embodiments, each R² and R³ are independently -C(O)OR. In some embodiments, each R² and R³ are independently -C(O)NR₂. In some embodiments, each R² and R³ are independently -C(O)N(R)OR. In some embodiments, each R² and R³ are independently -OC(O)R. In some embodiments, each R² and R³ are independently -OC(O)NR₂. In some embodiments, each R² and R³ are independently -N(R)C(O)OR. In some embodiments, each R² and R³ are independently -N(R)C(O)R. In some embodiments, each R² and R³ are independently -N(R)C(O)NR₂. In some embodiments, each R¹ and R² are independently - N(R)S(O)₂R. In some embodiments, each R² and R³ are independently -N⁺(O⁻)R₂. In some embodiments, each R² and R³ are independently -OP(O)R₂. In some embodiments, each R² and R³ are independently -OP(O)(OR)₂. In some embodiments, each R² and R³ are independently - OP(O)(OR)NR₂. In some embodiments, each R² and R³ are independently -OP(O)(NR₂)₂. In some embodiments, each R² and R³ are independently -P(O)R₂. In some embodiments, each R² and R³ are independently -SiR₃. In some embodiments, each R² and R³ are independently - Si(OR)R₂. In some embodiments, each R² and R³ are independently -SF₅. In some embodiments, each R² and R³ are independently

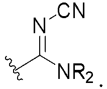

In some embodiments, R² is -CF₃. In some embodiments, R² is 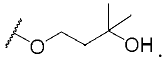 In some embodiments, R² is 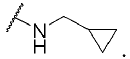 In some embodiments, R² is 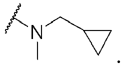 In some embodiments, R² is -C(OH)(CH₃)₂. In some embodiments, R² is

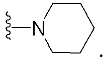

In some embodiments, R³ is -NHCH₃. In some embodiments, R³ is -CH₃. In some embodiments, R³ is 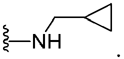 In some embodiments, R³ is 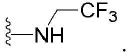 In some embodiments, R³ is -C(OH)(CH₃)₂. In some embodiments, R³ is

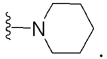

In some embodiments, each R¹, R², and R³ are independently selected from those depicted in **Table 1,** below.

As generally defined above, R⁴ is selected from 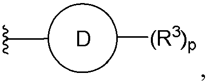 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur.

In some embodiments, R⁴ is 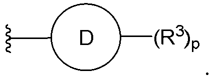 In some embodiments, R⁴ is hydrogen. In some embodiments, R⁴ is an optionally substituted group selected from C₁₋₆ aliphatic. In some embodiments, R⁴ is an optionally substituted 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur.

In some embodiments, R⁴ is 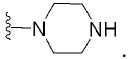 In some embodiments, R⁴ is 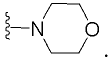 In some embodiments, R⁴ is 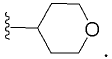 In some embodiments, R⁴ is 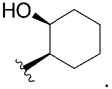 In some embodiments, R⁴ is 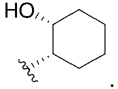 In some embodiments, R⁴ is 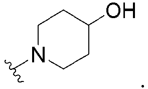 In some embodiments, R⁴ is 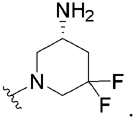 In some embodiments, R⁴ is 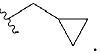 In some embodiments, R⁴ is 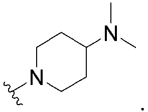 In some embodiments, R⁴ is 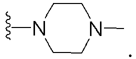 In some embodiments, R⁴ is 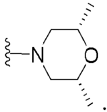 In some embodiments, R⁴ is 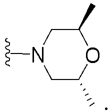 In some embodiments, R⁴ is 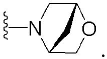 In some embodiments, R⁴ is 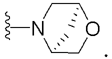 In some embodiments, R⁴ is 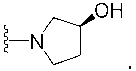 In some embodiments, R⁴ is 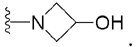 In some embodiments R⁴ is 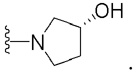 In some embodiments, R⁴ is 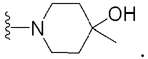 In some embodiments, R⁴ is 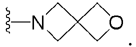 In some embodiments, R⁴ is 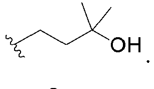 In some embodiments, R⁴ is 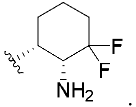 In some embodiments, R⁴ is 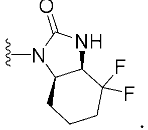 In some embodiments, R⁴ is 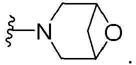 In some embodiments, R⁴ is 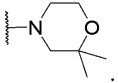 In some embodiments, R⁴ is 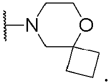 In some embodiments, R⁴ is 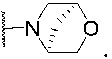 In some embodiments, R⁴ is 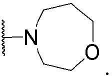 In some embodiments, R⁴ is

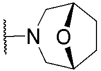

As defined generally above, Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur.

In some embodiments, Ring D is phenyl. In some embodiments, Ring D is a 4-10 membered saturated or partially unsaturated mono- or bicyclic carbocyclic or heterocyclic ring having 1-3 heteroatoms independently selected from nitrogen, oxygen, and sulfur. In some embodiments, Ring D is a 5-6 membered heteroaryl ring having 1-4 heteroatoms independently selected from nitrogen, oxygen, and sulfur.

In some embodiments, Ring D is 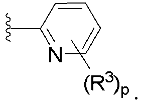 In some embodiments, Ring D is 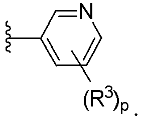 In some embodiments, Ring D is 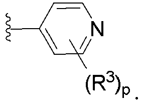 In some embodiments, Ring D is 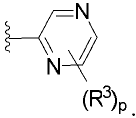 In some embodiments, Ring D is 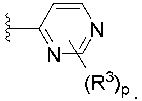 In some embodiments, Ring D is 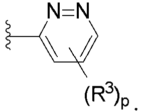 In some embodiments, Ring D is 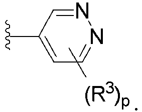 In some embodiments, Ring D is

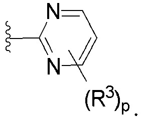

In some embodiments, Ring D is selected from those depicted in **Table 1,** below.

As generally defined above, each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur.

In some embodiments, each R is independently hydrogen. In some embodiments, each R is an optionally substituted group selected from C₁₋₆ aliphatic. In some embodiments, each R is an optionally substituted phenyl. In some embodiments, each R is an optionally substituted 4-7 membered saturated or partially unsaturated heterocyclic having 1-2 heteroatoms independently selected from nitrogen, oxygen, and sulfur. In some embodiments, each R is an optionally substituted 5-6 membered heteroaryl ring having 1-4 heteroatoms independently selected from nitrogen, oxygen, and sulfur. In some embodiments, two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur.

In some embodiments, each R is selected from those depicted in **Table 1,** below.

As generally defined above, each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur.

In some embodiments, each R⁵ is independently an optionally substituted group selected from C₁₋₆ aliphatic. In some embodiments, each R⁵ is independently an optionally substituted phenyl. In some embodiments, each R⁵ is independently an optionally substituted 3-7 membered saturated or partially unsaturated carbocyclic or heterocyclic ring having 1-2 heteroatoms independently selected from nitrogen, oxygen, and sulfur. In some embodiments, each R⁵ is independently an optionally substituted 5-6 membered heteroaryl ring having 1-4 heteroatoms independently selected from nitrogen, oxygen, and sulfur.

In some embodiments, R⁵ is 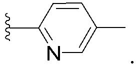 In some embodiments, R⁵ is optionally substituted

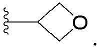

In some embodiments, each R⁵ is selected from those depicted in **Table 1,** below.

As generally defined above, each n is independently 0, 1, or 2.

In some embodiments, each n is independently 0. In some embodiments, each n is independently 1. In some embodiments, each n is independently 2.

As generally defined above, each m and p are independently 0, 1, 2, 3 or 4.

In some embodiments, each m and p are independently 0. In some embodiments, each m and p are independently 1. In some embodiments, each m and p are independently 2. In some embodiments, each m and p are independently 3. In some embodiments, each m and p are independently 4.

In some embodiments, each m and p are selected from those depicted in **Table 1,** below.

In some embodiments, the present invention provides the compound of formula **II,** wherein Ring A is cyclohexyl, Ring B is pyrazolyl, and L³ is a covalent bond thereby forming a compound of formula **II-a**: 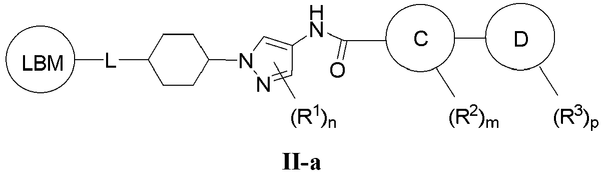 or a pharmaceutically acceptable salt thereof, wherein each of LBM, L, R¹, R², R³, Ring C, Ring D, n, m, and p is as defined above and described in embodiments herein, both singly and in combination.

In some embodiments, the present invention provides the compound of formula **II,** wherein Ring A is cyclohexyl, Ring B is pyrazolyl, Ring C is oxazolyl, and L³ is a covalent bond thereby forming a compound of formula **II-b**: 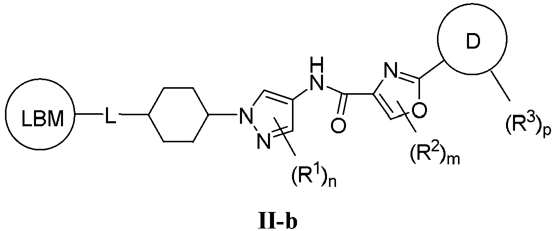 or a pharmaceutically acceptable salt thereof, wherein each of LBM, L, R¹, R², R³, Ring D, n, m, and p is as defined above and described in embodiments herein, both singly and in combination.

In some embodiments, the present invention provides the compound of formula **II,** wherein Ring A is cyclohexyl, Ring B is pyrazolyl, Ring D is pyridyl, and L³ is a covalent bond thereby forming a compound of formula **II-c**: 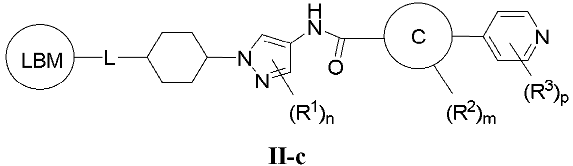 or a pharmaceutically acceptable salt thereof, wherein each of LBM, L, R¹, R², R³, Ring C, n, m, and p is as defined above and described in embodiments herein, both singly and in combination.

In some embodiments, the present invention provides the compound of formula **II,** wherein Ring A is cyclohexyl, Ring B is pyrazolyl, thereby forming a compound of formula **II-d**: 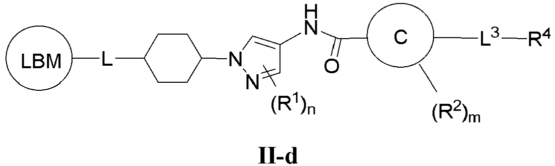 or a pharmaceutically acceptable salt thereof, wherein each of LBM, L, L³, R¹, R², R⁴, Ring C, n, and m is as defined above and described in embodiments herein, both singly and in combination.

In certain embodiments, the present invention provides a compound of formula **II-d**: 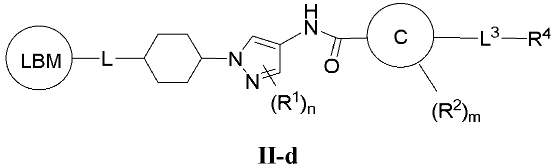 or a pharmaceutically acceptable salt thereof, wherein L and LBM are as defined above and described in embodiments herein, and wherein:
Ring C is phenyl or a 5-10 membered mono- or bicyclic heteroaryl ring having 1-5 heteroatoms independently selected from nitrogen, oxygen, and sulfur;
L³ a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR,-SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
R⁴ is selected from 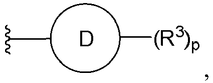 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur.
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4; and
each p is 0, 1, 2, 3 or 4.

In some embodiments, the present invention provides the compound of formula **II,** wherein Ring A is cyclohexyl, Ring B is pyrazolyl, Ring C is pyrazolo[1,5-a]pyrimidyl, thereby forming a compound of formula **II-e**: 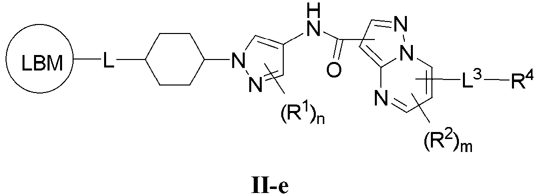 or a pharmaceutically acceptable salt thereof, wherein each of LBM, L, L³, R1, R², R⁴, n, and m is as defined above and described in embodiments herein, both singly and in combination.

In certain embodiments, the present invention provides a compound of formula **II-e**: 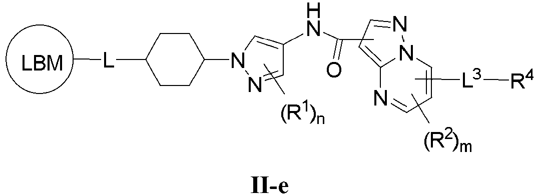 or a pharmaceutically acceptable salt thereof, wherein L and LBM are as defined above and described in embodiments herein, and wherein:
L³ a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
R⁴ is selected from 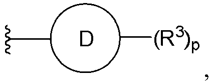 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur.
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)_{2,} -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4; and
each p is 0, 1, 2, 3 or 4.

In some embodiments, the present invention provides the compound of formula **V-a,** wherein Ring A is cyclohexyl, Ring B is pyrazolyl, and L³ is a covalent bond thereby forming a compound of formula **V-b:** 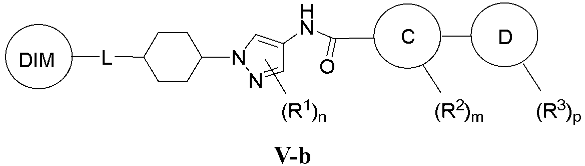 or a pharmaceutically acceptable salt thereof, wherein each of DIM, L, R¹, R², R³, Ring C, Ring D, n, m, and p is as defined above and described in embodiments herein, both singly and in combination.

In some embodiments, the present invention provides the compound of formula **V-a,** wherein Ring A is cyclohexyl, Ring B is pyrazolyl, Ring C is oxazolyl, and L³ is a covalent bond thereby forming a compound of formula **V-c:** 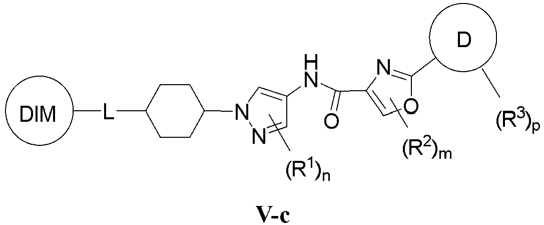 or a pharmaceutically acceptable salt thereof, wherein each of DIM, L, R¹, R², R³, Ring D, n, m, and p is as defined above and described in embodiments herein, both singly and in combination.

In some embodiments, the present invention provides the compound of formula V-a, wherein Ring A is cyclohexyl, Ring B is pyrazolyl, Ring D is pyridyl, and L³ is a covalent bond thereby forming a compound of formula **V-d:** 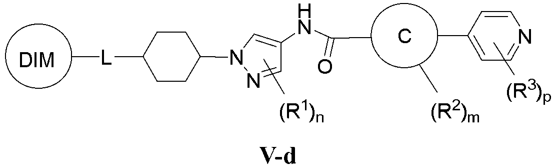 or a pharmaceutically acceptable salt thereof, wherein each of DIM, L, R¹, R², R³, Ring C, n, m, and p is as defined above and described in embodiments herein, both singly and in combination.

In some embodiments, the present invention provides the compound of formula **V-a,** wherein Ring A is cyclohexyl, Ring B is pyrazolyl, L³ is a covalent bond, and R⁴ is hydrogen thereby forming a compound of formula **V-e:** 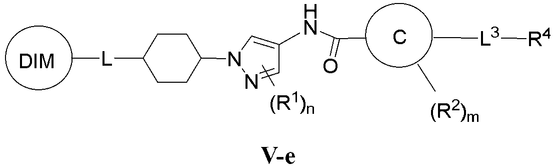 or a pharmaceutically acceptable salt thereof, wherein each of DIM, L, L³, R¹, R², R⁴, Ring C, n, and m is as defined above and described in embodiments herein, both singly and in combination.

In some embodiments, the present invention provides the compound of formula **V-a,** wherein Ring A is cyclohexyl, Ring B is pyrazolyl, Ring C is pyrazolo[1,5-a]pyrimidyl, L³ is a covalent bond, and R⁴ is hydrogen thereby forming a compound of formula **V-f:** 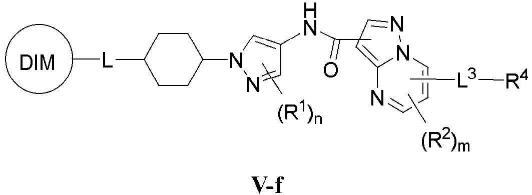 or a pharmaceutically acceptable salt thereof, wherein each of DIM, L, L³, R¹, R², R⁴, n, and m is as defined above and described in embodiments herein, both singly and in combination.

In some embodiments, IRAK is 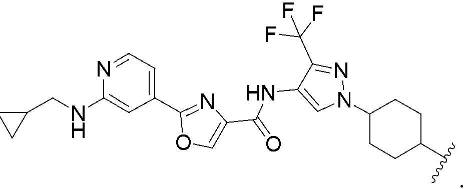 In some embodiments, IRAK is 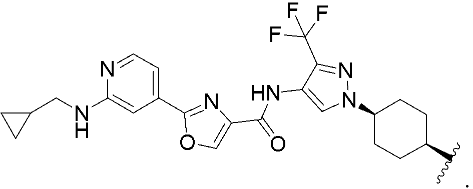 In some embodiments, IRAk is 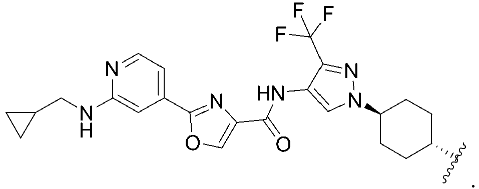 In some embodiments, IRAK is 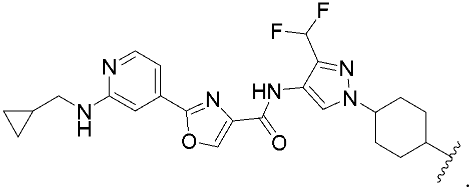 In some embodiments, IRAK is 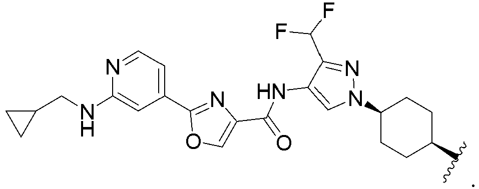 In some embodiments, IRAK is 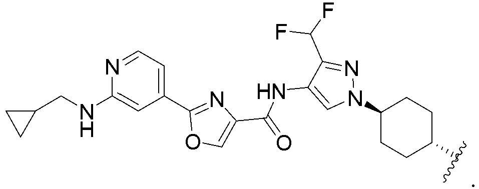 In some embodiments, IRAK is 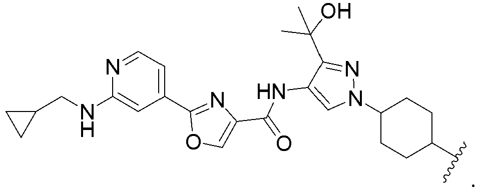 In some embodiments, IRAK is 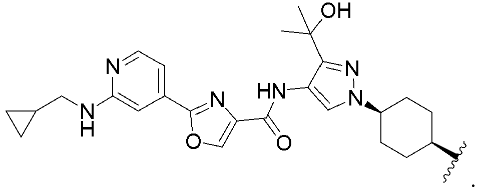 In some embodiments, IRAK is 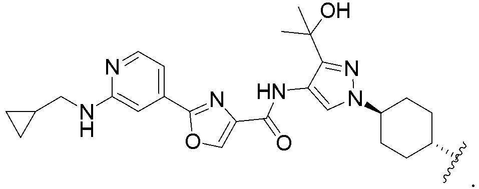 In some embodiments, IRAK is 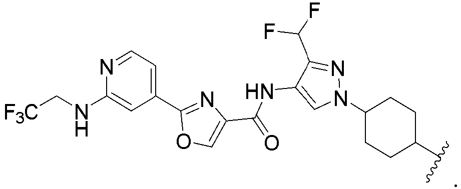 In some embodiments, IRAK is 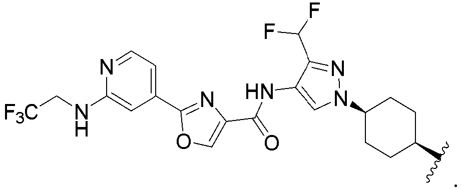 In some embodiments, IRAK is 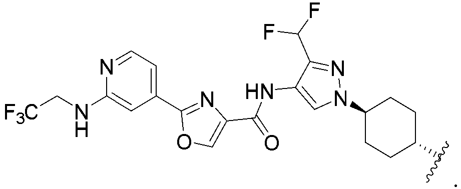 In some embodiments, IRAK is 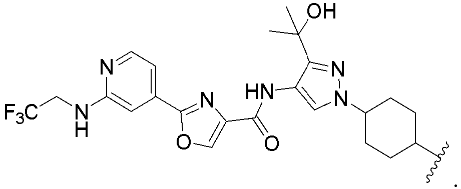 In some embodiments, IRAK is 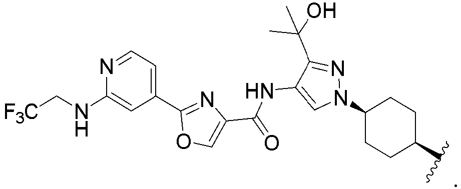 In some embodiments, IRAK is 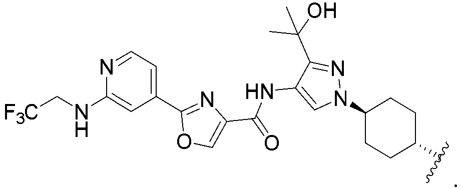 In some embodiments, IRAK is 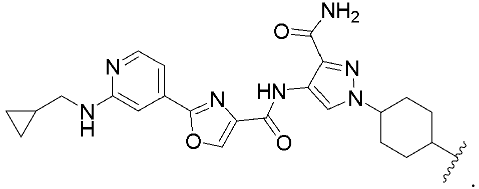 In some embodiments, IRAK is 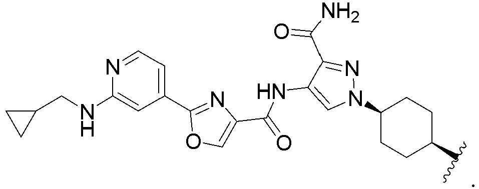 In some embodiments, IRAK is 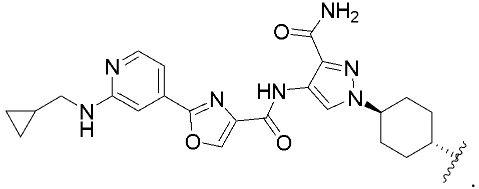 In some embodiments, IRAK is 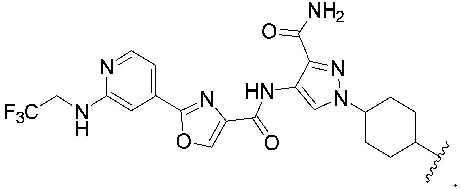 In some embodiments, IRAK is 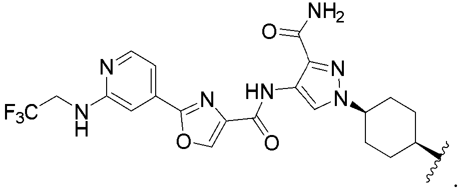 In some embodiments, IRAK is 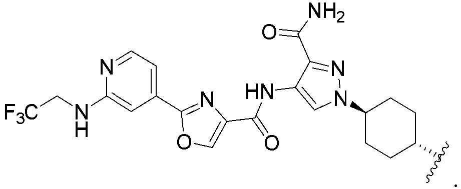 In some embodiments, IRAK is 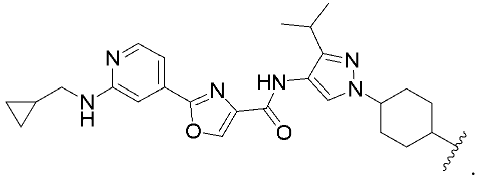 In some embodiments, IRAK is 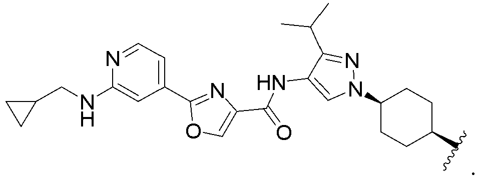 In some embodiments, IRAK is 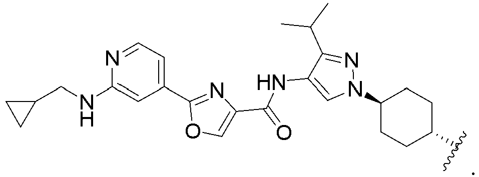 In some embodiments, IRAK is 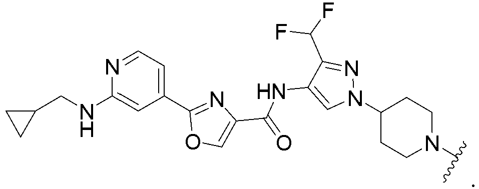 In some embodiments, IRAK is 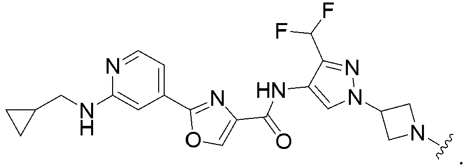 In some embodiments, IRAK is 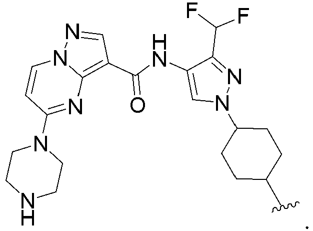 In some embodiments, IRAK is 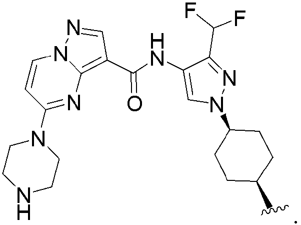 In some embodiments, IRAK is 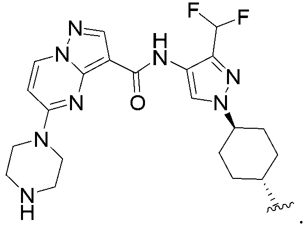 In some embodiments, IRAK is 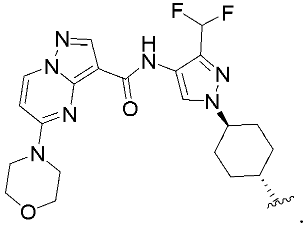 In some embodiments, IRAK is 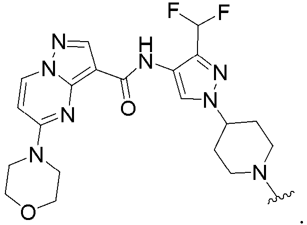 In some embodiments, IRAK is 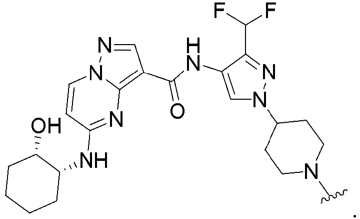 In some embodiments, IRAK is 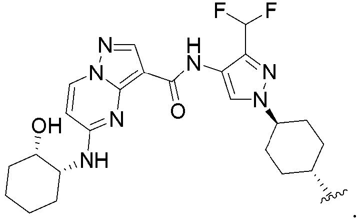 In some embodiments, IRAK is 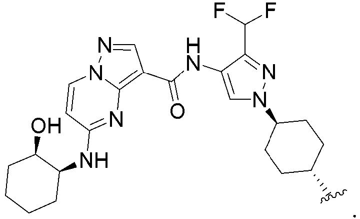 In some embodiments, IRAK is 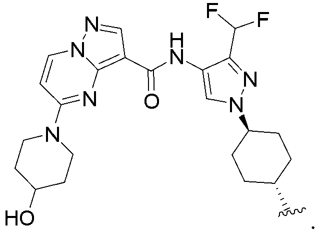 In some embodiments, IRAK is 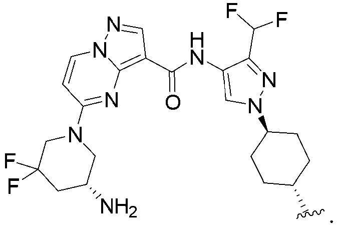 In some embodiments, IRAK is 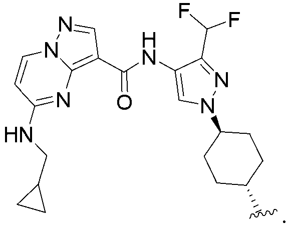 In some embodiments, IRAK is 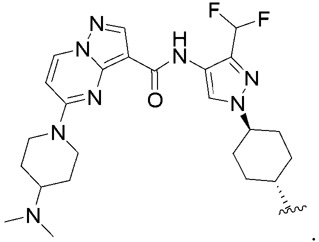 In some embodiments, IRAK is 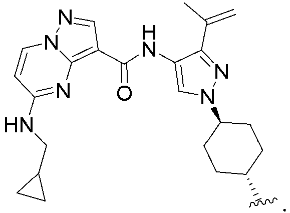 In some embodiments, IRAK is 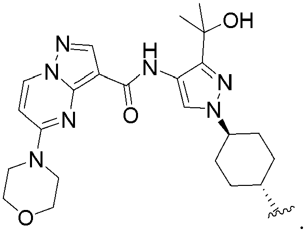 In some embodiments, IRAK is 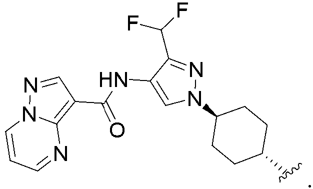 In some embodiments, IRAK is 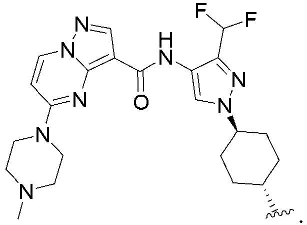 In some embodiments, IRAK is 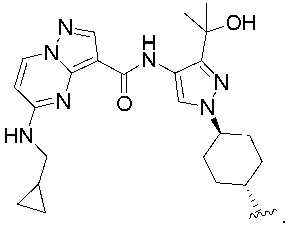 In some embodiments, IRAK is 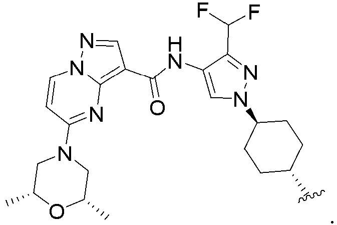 In some embodiments, IRAK is 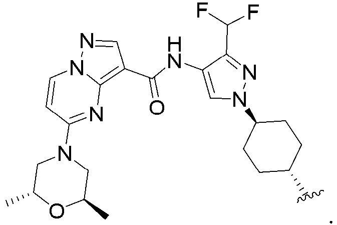 In some embodiments, IRAK is 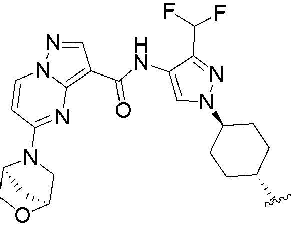 . In some embodiments, IRAK is 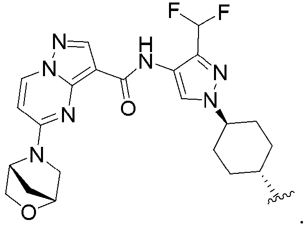 In some embodiments, IRAK is 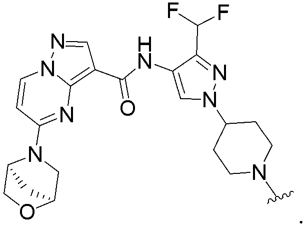 In some embodiments, IRAK is 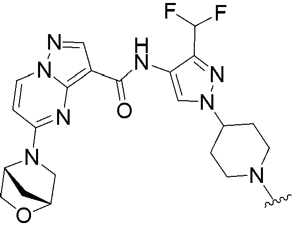 . In some embodiments, IRAK is 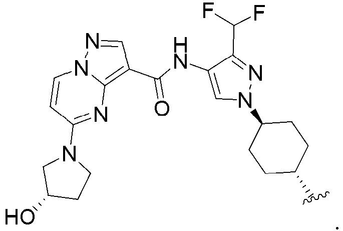 In some embodiments, IRAK is 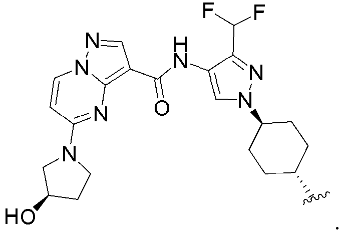 In some embodiments, IRAK is 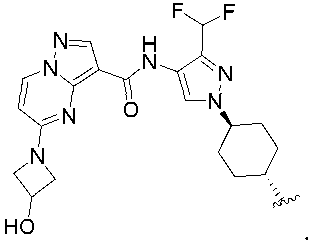 In some embodiments, IRAK is 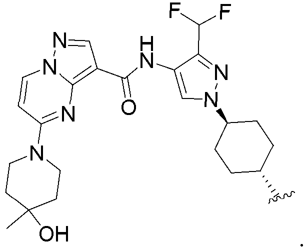 In some embodiments, IRAK is 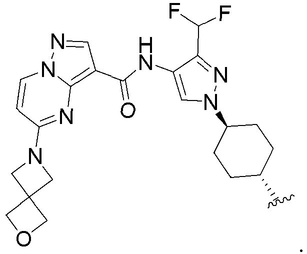 In some embodiments, IRAK is 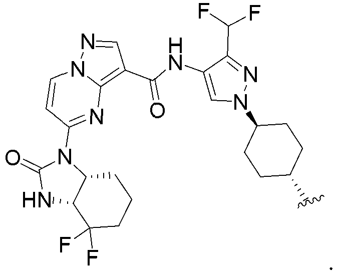 In some embodiments, IRAK is 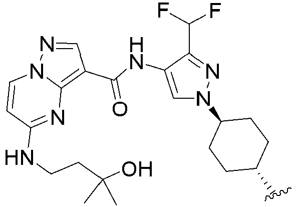 . In some embodiments, IRAK is 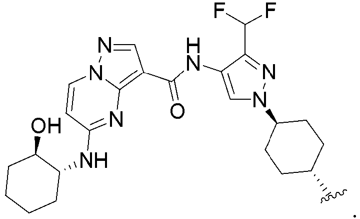 In some embodiments, IRAK is 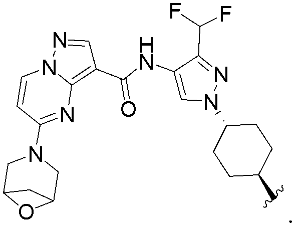 In some embodiments, IRAK is 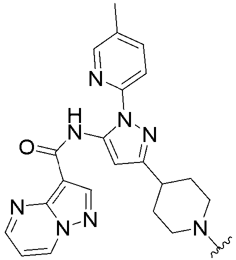 . In some embodiments, IRAK is 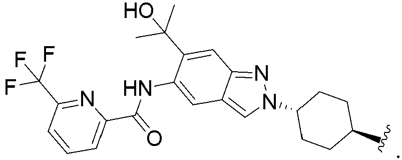 In some embodiments, IRAK is 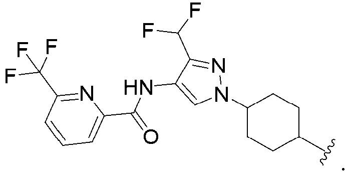 In some embodiments, IRAK is 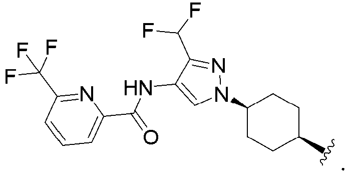 In some embodiments, IRAK is 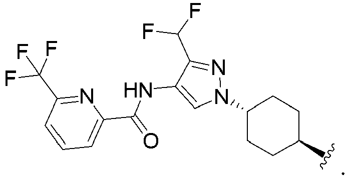 In some embodiments, IRAK is 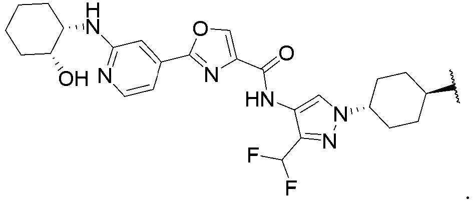 In some embodiments, IRAK is 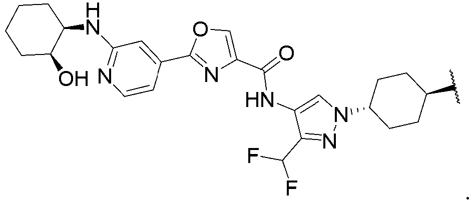 In some embodiments, IRAK is 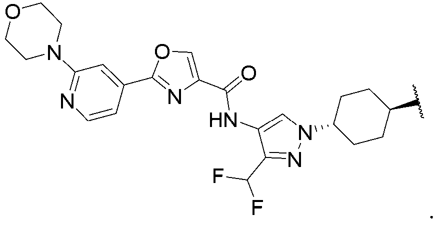 In some embodiments, IRAK is 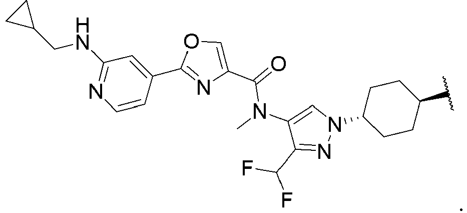 In some embodiments, IRAK is 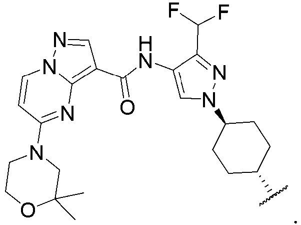 In some embodiments, IRAK is 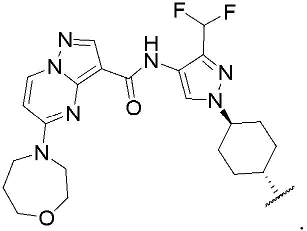 In some embodiments, IRAK is 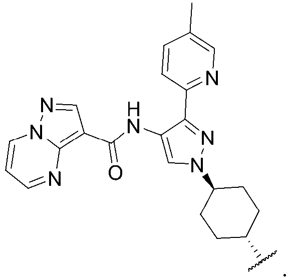 In some embodiments, IRAK is 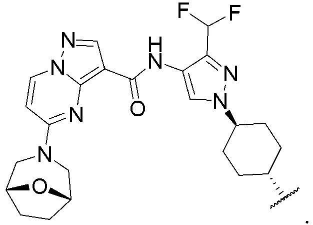 In some embodiments, IRAK is 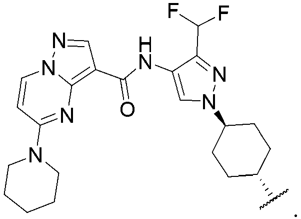 In some embodiments, IRAK is 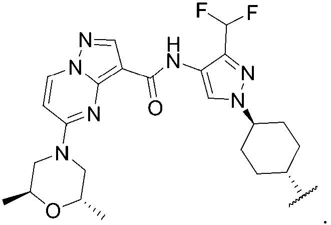 In some embodiments, IRAK 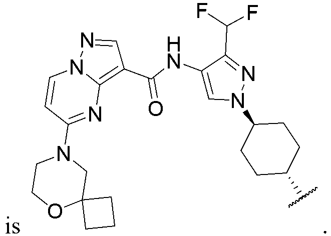 In some embodiments, IRAK is 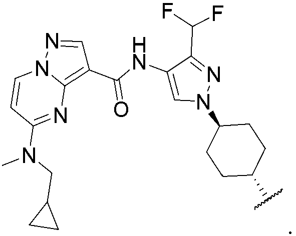 In some embodiments, IRAK is

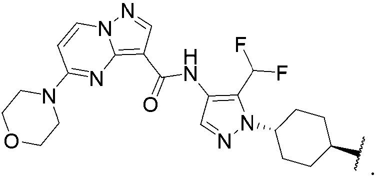

In certain embodiments, the present invention provides a compound of formula **I,** where IRAK is a IRAK-4 binding moiety thereby forming a compound of formula **III:** 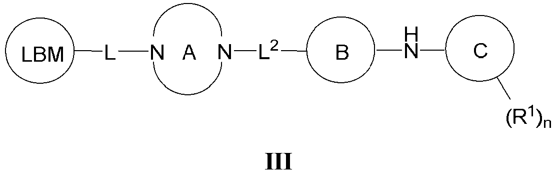 or a pharmaceutically acceptable salt thereof, wherein L and LBM are as defined above and described in embodiments herein, and wherein:
Ring A is a 4-7 membered saturated monocyclic ring having two ring nitrogen atoms;
Ring B is a 4-10 membered saturated mono- or bicyclic carbocyclic or hetereocyclic ring having 0-2 heteroatoms independently selected from nitrogen, oxygen, and sulfur;
Ring C is phenyl, a 4-10 membered saturated or partially unsaturated mono- or bicyclic carbocyclic or heterocyclic ring having 1-3 heteroatoms independently selected from nitrogen, oxygen, and sulfur, or a 5-10 membered mono- or bicyclic heteroaryl ring having 1-4 heteroatoms independently selected from nitrogen, oxygen, and sulfur;
L² is a bivalent moiety selected from a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁴, halogen, -CN, -NO₂, -OR, - SR, -NR₂, -S(O)₂R, -S(O)₂NR₂, -S(O)R, -CF₂(R), -CF₃, -CR₂(OR), - CR₂(NR₂), -C(O)R, -C(O)OR, -C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R⁴ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur; and
each n is 0, 1, 2, 3 or 4;
wherein the compound of formula **III** is not compound **I-101, I-102, I-103, I-104,** or **I-105** in Table 1A.

In certain embodiments, the present invention provides a compound of formula V, where IRAK is a IRAK-4 binding moiety thereby forming a compound of formula V-g: 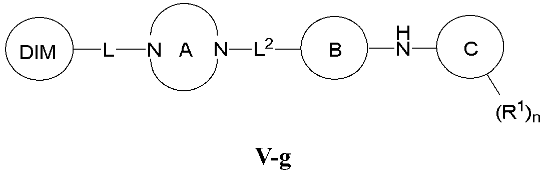 or a pharmaceutically acceptable salt thereof, wherein L and DIM are as defined above and described in embodiments herein, and wherein:
Ring A is a 4-7 membered saturated monocyclic ring having two ring nitrogen atoms;
Ring B is a 4-10 membered saturated mono- or bicyclic carbocyclic or hetereocyclic ring having 0-2 heteroatoms independently selected from nitrogen, oxygen, and sulfur;
Ring C is phenyl, a 4-10 membered saturated or partially unsaturated mono- or bicyclic carbocyclic or heterocyclic ring having 1-3 heteroatoms independently selected from nitrogen, oxygen, and sulfur, or a 5-10 membered mono- or bicyclic heteroaryl ring having 1-4 heteroatoms independently selected from nitrogen, oxygen, and sulfur;
L² is a bivalent moiety selected from a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁴, halogen, -CN, -NO₂, -OR, - SR, -NR₂, -S(O)₂R, -S(O)₂NR₂, -S(O)R, -CF₂(R), -CF₃, -CR₂(OR), - CR₂(NR₂), -C(O)R, -C(O)OR, -C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R⁴ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur; and
each n is 0, 1, 2, 3 or 4;
wherein the compound of formula **V-g** is not compound **I-101, I-102, I-103, I-104, or I-105 in Table 1A.**

The below embodiments found in paragraphs [00144] to [00179] are to compounds of formula **III** and **V-g.**

As defined generally above, Ring A is a 4-7 membered saturated monocyclic ring having two ring nitrogen atoms.

In some embodiments, Ring A is piperazine. In some embodiments, Ring A is 1,4-diazepane.

In some embodiments, Ring A is selected from those depicted in **Table 1,** below.

As defined generally above, Ring B a 4-10 membered saturated mono- or bicyclic carbocyclic or hetereocyclic ring having 0-2 heteroatoms independently selected from nitrogen, oxygen, and sulfur.

In some embodiments, Ring B is cyclobutyl. In some embodiments, Ring B is cyclopentyl. In some embodiments, Ring B is cyclohexyl. In some embodiments, Ring B is cycloheptyl.

In some embodiments, Ring B is selected from those depicted in **Table 1,** below.

As defined generally above, Ring C is a 9 membered bicyclic heteroaryl ring having 1-3 nitrogen atoms.

In some embodiments, Ring C is phenyl, a 4-10 membered saturated or partially unsaturated mono- or bicyclic carbocyclic or heterocyclic ring having 1-3 heteroatoms independently selected from nitrogen, oxygen, and sulfur, or a 5-10 membered mono- or bicyclic heteroaryl ring having 1-4 heteroatoms independently selected from nitrogen, oxygen, and sulfur.

In some embodiments, Ring B is phenyl. In some embodiments, Ring is a 4-10 membered saturated or partially unsaturated mono- or bicyclic carbocyclic or heterocyclic ring having 1-3 heteroatoms independently selected from nitrogen, oxygen, and sulfur. In some embodiments, Ring B is a 5-6 membered heteroaryl ring having 1-4 heteroatoms independently selected from nitrogen, oxygen, and sulfur.

In some embodiments, Ring C is 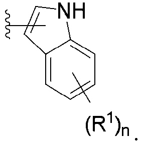 In some embodiments, Ring C is 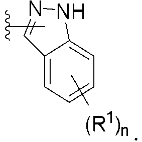 In some embodiments, Ring C is 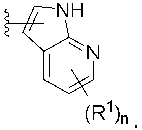 In some embodiments, Ring C is 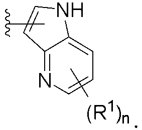 In some embodiments, Ring C is 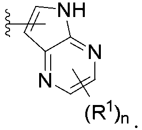 In some embodiments, Ring C is 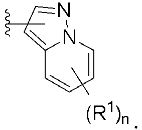 In some embodiments, Ring C is

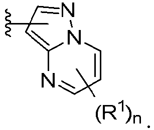

In some embodiments, Ring C is 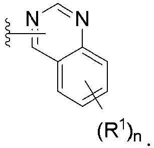 In some embodiments, Ring C is

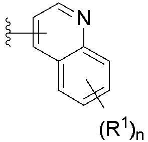

In some embodiments, Ring C is selected from those depicted in **Table 1,** below.

As generally defined above, L² is a bivalent moiety selected from a covalent bond or a C₁₋₃ bivalent straight or branched saturated or unsaturated hydrocarbon chain wherein 1-3 methylene units of the chain are independently and optionally replaced with -O-, -C(O)-, -C(S)-, - C(R)₂-, -CH(R)-, -C(F)₂-, -N(R)-, -S-, -S(O)₂- or -CR=CR-.

In some embodiments, L² a covalent bond. In some embodiments, L² is a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, - C(F)₂-, -N(R)-, -S-, -S(O)₂- or -CR=CR-. In some embodiments, L² is a C₁₋₃ aliphatic. In some embodiments, L² is -CH₂-. In some embodiments, L² is -C(D)(H)-. In some embodiments, L² is -C(D)₂-. In some embodiments, L² is -CH₂CH₂-. In some embodiments, L² is -NR-. In some embodiments, L² is -CH₂NR-. In some embodiments, L² is or -O-. In some embodiments, L² is -CH₂O-. In some embodiments, L² is -S-. In some embodiments, L² is -OC(O)-. In some embodiments, L² is -C(O)O-. In some embodiments, L² is -C(O)-. In some embodiments, L² is - S(O)-. In some embodiments, L² is -S(O)₂-,. In some embodiments, L² is -NRS(O)₂-. In some embodiments, L² is -S(O)₂NR-. In some embodiments, L² is -NRC(O)-. In some embodiments, L² is -C(O)NR-. In some embodiments, L² is -OC(O)NR-. In some embodiments, L² is - NRC(O)O-.

In some embodiments, L² is selected from those depicted in **Table 1,** below.

As defined generally above, each R¹ is independently hydrogen, -R⁴, halogen, -CN, - NO₂, -OR, -SR, -NR₂, -S(O)₂R, -S(O)₂NR₂, -S(O)R, -CF₂(R), -CR₂(CN), -CF₃, -CR₂(OR), - CR₂(NR₂), -C(O)R, -C(O)OR, -C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R.

In some embodiments, each R¹ is independently hydrogen. In some embodiments, each R¹ is independently -R⁴. In some embodiments, each R¹ is independently halogen. In some embodiments, each R¹ is independently -CN. In some embodiments, each R¹ is independently - NO₂. In some embodiments, each R¹ is independently -OR. In some embodiments, each R¹ is independently -SR. In some embodiments, each R¹ is independently -NR₂. In some embodiments, each R¹ is independently -S(O)₂R. In some embodiments, each R¹ is independently -S(O)₂NR₂. In some embodiments, each R¹ is independently -S(O)R. In some embodiments, each R¹ is independently -CF₂(R). In some embodiments, each R¹ is independently -CR₂(CN). In some embodiments, each R¹ is independently -CF₃. In some embodiments, each R¹ is independently - CR₂(OR). In some embodiments, each R¹ is independently -CR₂(NR₂). In some embodiments, each R¹ is independently -C(O)R. In some embodiments, each R¹ is independently -C(O)OR. In some embodiments, each R¹ is independently -C(O)NR₂. In some embodiments, each R¹ is independently -C(O)NR₂. In some embodiments, each R¹ is independently -C(O)N(R)OR. In some embodiments, each R¹ is independently -OC(O)R. In some embodiments, each R¹ is independently -OC(O)NR₂. In some embodiments, each R¹ is independently -N(R)C(O)OR. In some embodiments, each R¹ is independently -N(R)C(O)R. In some embodiments, each R¹ is independently -N(R)C(O)NR₂. In some embodiments, each R¹ is independently -N(R)S(O)₂R.

In some embodiments, R¹ is 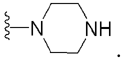 In some embodiments, R¹ is 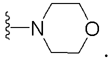 In some embodiments, R¹ is 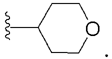 In some embodiment, R¹ is methyl. In some embodiments, R¹ is -CH₂(CN). In some embodiments, R¹ is -CN.

In some embodiments, each R¹ is independently selected from those depicted in **Table 1,** below.

As generally defined above, each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, each R is independently hydrogen. In some embodiments, each R is an optionally substituted group selected from C₁₋₆ aliphatic. In some embodiments, each R is a phenyl. In some embodiments, each R is a 4-7 membered saturated or partially unsaturated heterocyclic having 1-2 heteroatoms independently selected from nitrogen, oxygen, and sulfur. In some embodiments, each R is a 5-6 membered heteroaryl ring having 1-4 heteroatoms independently selected from nitrogen, oxygen, and sulfur. In some embodiments, two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, each R is selected from those depicted in **Table 1,** below.

As generally defined above, each R⁴ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur.

In some embodiments, each R⁴ is independently an optionally substituted group selected from C₁₋₆ aliphatic. In some embodiments, each R⁴ is independently an optionally substituted phenyl. In some embodiments, each R⁴ is independently an optionally substituted 4-7 membered saturated or partially unsaturated heterocyclic ring having 1-2 heteroatoms independently selected from nitrogen, oxygen, and sulfur. In some embodiments, each R⁴ is independently an optionally substituted 5-6 membered heteroaryl ring having 1-4 heteroatoms independently selected from nitrogen, oxygen, and sulfur.

In some embodiments, each R⁴ is selected from those depicted in **Table 1,** below.

A As generally defined above, each n is independently 0, 1, or 2.

In some embodiments, each n is independently 0. In some embodiments, each n is independently 1. In some embodiments, each n is independently 2.

In some embodiments, each n is selected from those depicted in **Table 1,** below.

In some embodiments, the present invention provides the compound of formula III, wherein Ring B is cyclohexyl thereby forming a compound of formula **III-a:** 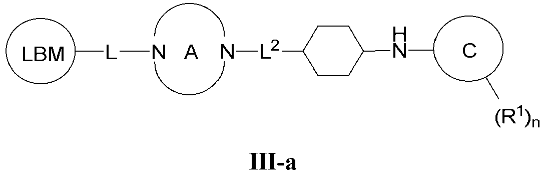 or a pharmaceutically acceptable salt thereof, wherein each of LBM, L, L², Ring A, R¹, Ring C, and n is as defined above and described in embodiments herein, both singly and in combination.

In some embodiments, the invention provides the compound of formula **III,** wherein L² is a covalent bond, Ring B is cyclohexyl, and Ring C is pyrrolo[2,1-f][1,2,4]triazinyl, 7H-pyrrolo[2,3-d]pyrimidinyl, or quinazolinyl thereby forming a compound of formula **III-b, III-c,** or **III-d** respectively: 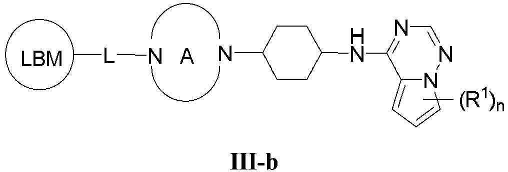 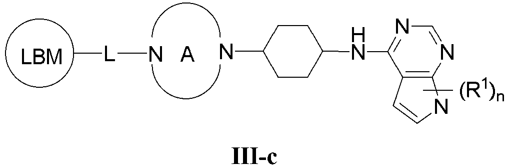 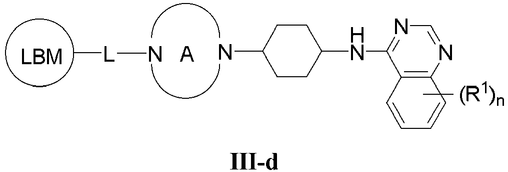 or a pharmaceutically acceptable salt thereof, wherein each of LBM, L, Ring A, R¹, and n is as defined above and described in embodiments herein, both singly and in combination.

In some embodiments, the present invention provides the compound of formula **V-g,** wherein Ring B is cyclohexyl thereby a compound of formula **V-h:** 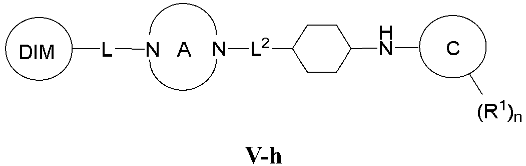 or a pharmaceutically acceptable salt thereof, wherein each of DIM, L, L², Ring A, R¹, Ring C, and n is as defined above and described in embodiments herein, both singly and in combination.

In some embodiments, the invention provides the compound of formula **V-g,** wherein L² is a covalent bond, Ring B is cyclohexyl, and Ring C is pyrrolo[2,1-f][1,2,4]triazinyl, 7H-pyrrolo[2,3-d]pyrimidinyl, or quinazolinyl thereby forming a compound of formula **V-i-1, V-i-2,** or **V-i-3** respectively: 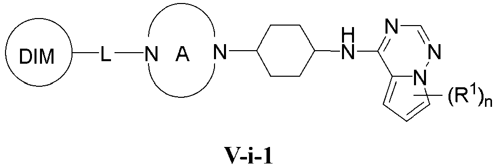 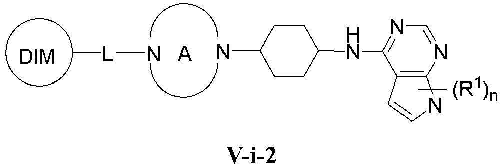 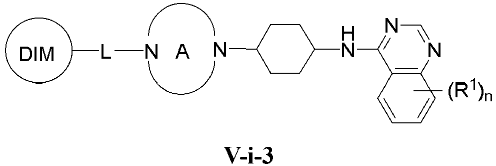 or a pharmaceutically acceptable salt thereof, wherein each of DIM, L, Ring A, R¹, and n is as defined above and described in embodiments herein, both singly and in combination.

In some embodiments, IRAK is 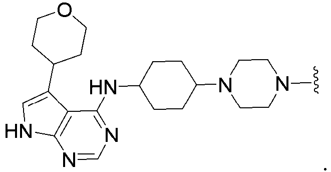 In some embodiments, IRAK is 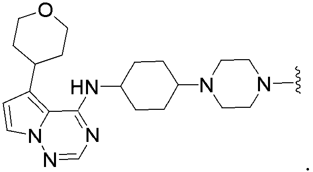 In some embodiments, IRAK is 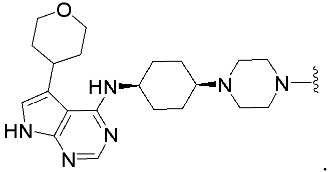 In some embodiments, IRAK is 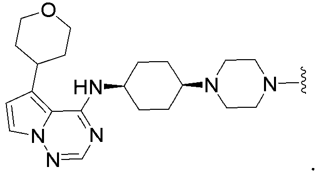 In some embodiments, IRAK is 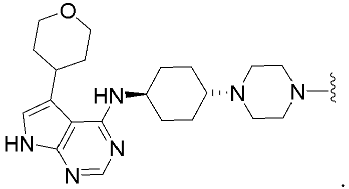 In some embodiments, IRAK is 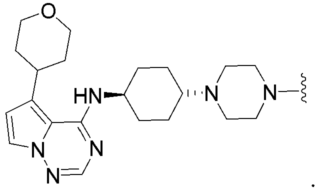 In some embodiments, IRAK is 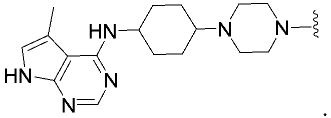 In some embodiments, IRAK is 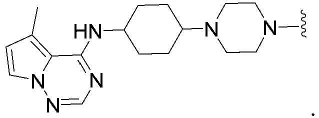 In some embodiments, IRAK is 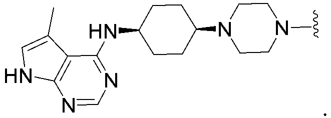 In some embodiments, IRAK is 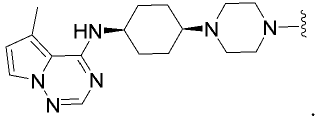 In some embodiments, IRAK is 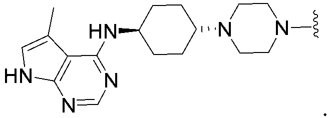 In some embodiments, IRAK is 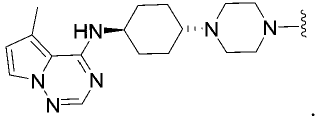 In some embodiments, IRAK is 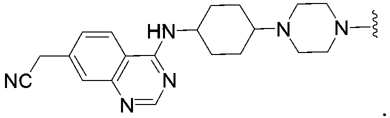 In some embodiments, IRAK is 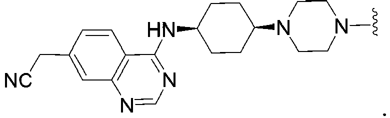 In some embodiments, IRAK is 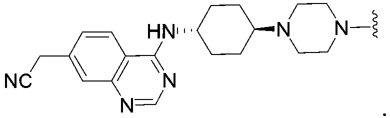 In some embodiments, IRAK is

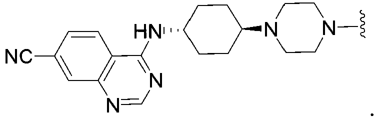

In some embodiments, IRAK is selected from those in **Table 1,** below.

In some embodiments, the present invention provides a compound of Formula **I,** wherein IRAK is an IRAK-4 inhibitor 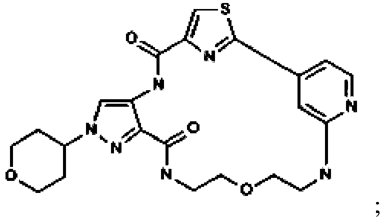 thereby forming a compound of formula IV: 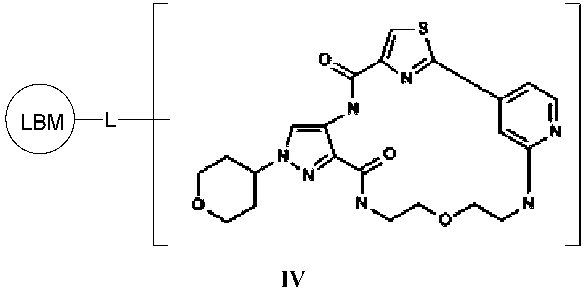 or a pharmaceutically acceptable salt thereof, wherein 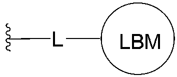 is attached to a modifiable carbon, oxygen, or nitrogen, and wherein L and LBM are as defined above and described in embodiments herein.

In some embodiments, IRAK is 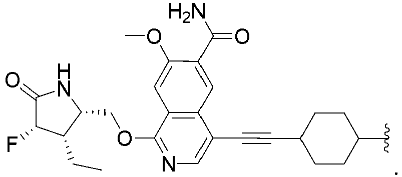 In some embodiments, IRAK is 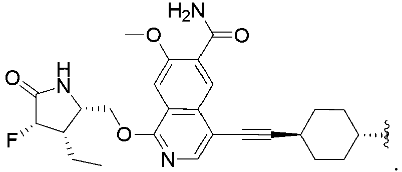 In some embodiments, IRAK is 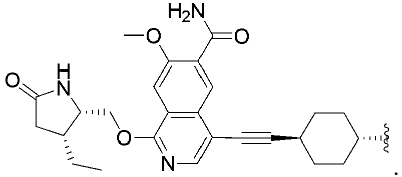 In some embodiments, IRAK is In some embodiments, IRAK is 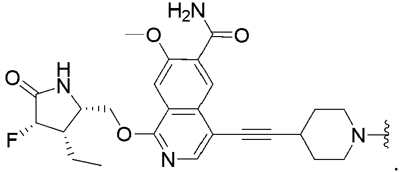 In some embodiments, IRAK is 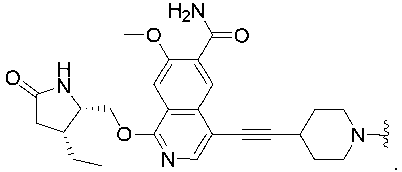 In some embodiments, IRAK is 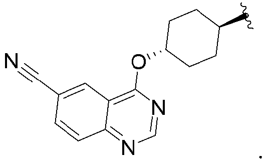 In some embodiments, IRAK is 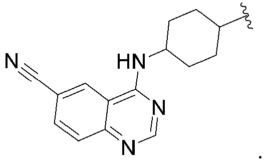 In some embodiments, IRAK is 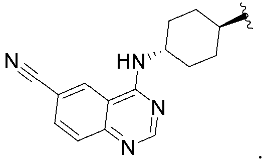 In some embodiments, IRAK is 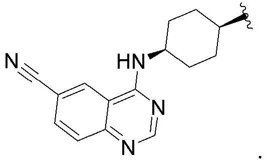 In some embodiments, IRAK is 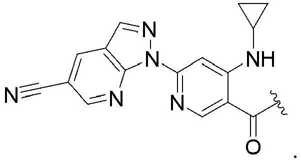 In some embodiments, IRAK is

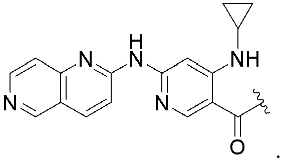

In some embodiments, IRAK is selected from those in Table 1, below.

### Ligase Binding Moiety (LBM)

In some embodiments, LBM is an E3 ligase ligand. Such E3 ligase ligands are well known to one of ordinary skill in the art and include those described in M. Toure, C. M. Crews, Angew. Chem. Int. Ed. 2016, 55, 1966, T. Uehara et al. Nature Chemical Biology 2017, 13, 675*,* WO 2017/176708, US 2017/0281784, WO 2017/161119, WO 2017/176957, WO 2017/176958, WO 2015/160845, US 2015/0291562, WO 2016/197032, WO 2016/105518, US 2018/0009779, WO 2017/007612, 2018/0134684, WO 2013/106643, US 2014/0356322, WO 2002/020740, US 2002/0068063, WO 2012/078559, US 2014/0302523, WO 2012/003281, US 2013/0190340, US 2016/0022642, WO 2014/063061, US 2015/0274738, WO 2016/118666, US 2016/0214972, WO 2016/149668, US 2016/0272639, WO 2016/169989, US 2018/0118733, WO 2016/197114, US 2018/0147202, WO 2017/011371, US 2017/0008904, WO 2017/011590, US 2017/0037004, WO 2017/079267, US 2017/0121321, WO 2017/117473, WO 2017/117474, WO 2013/106646, WO 2014/108452, WO 2017/197036, US 2019/0076540, WO 2017/197046, US 2019/0076542, WO 2017/197051, US 2019/0076539, WO 2017/197055, US 2019/0076541, and WO 2017/197056, the entirety of each of which is herein incorporated by reference.

In certain embodiments, the present invention provides a compound of formula **I,** wherein LBM is an E3 ubiquitin ligase (cereblon) binding moiety 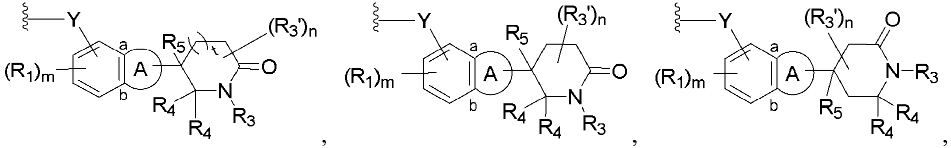 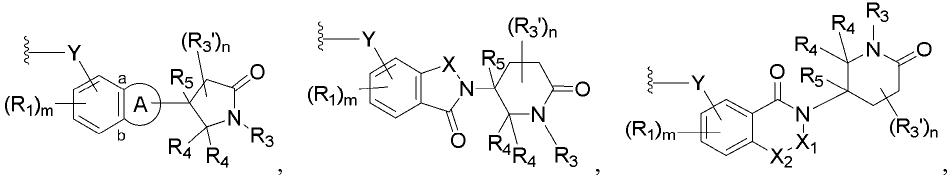 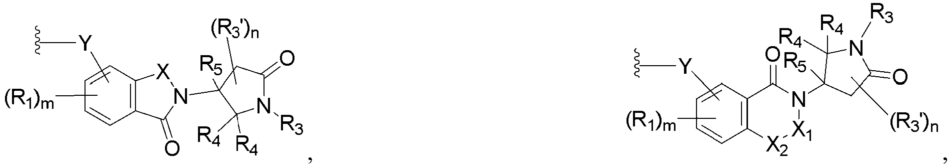 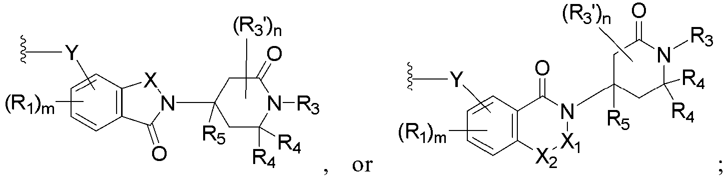 thereby forming a compound of formula I-a-1, I-a-2, I-a-3, I-a-4, I-a-5, I-a-6, I-a-7, I-a-8, I-a-9, or I-a-10 respectively: 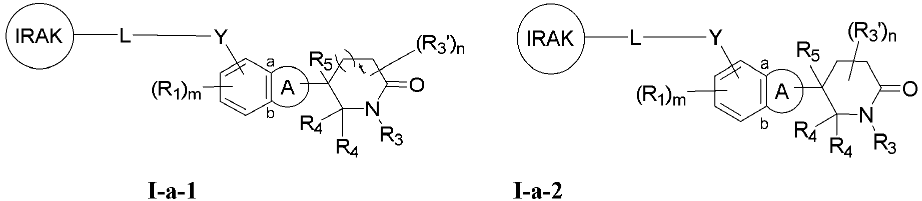 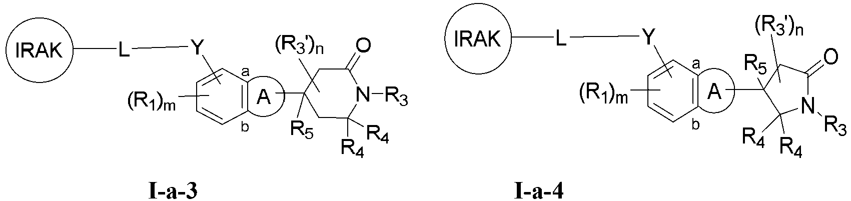 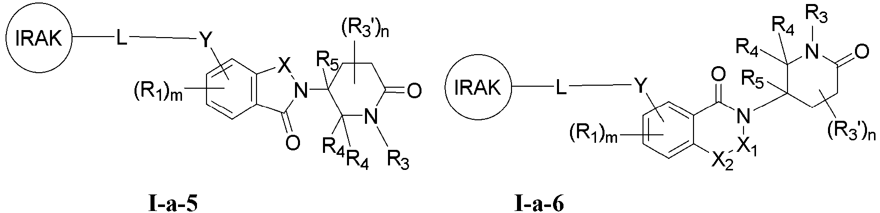 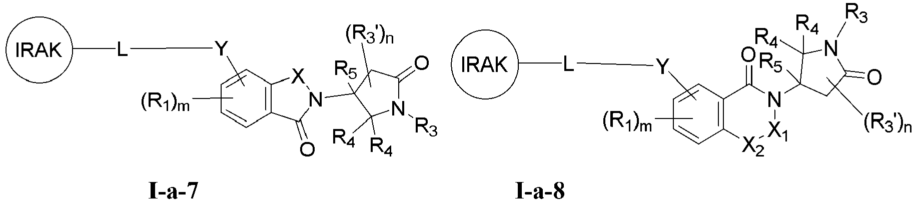 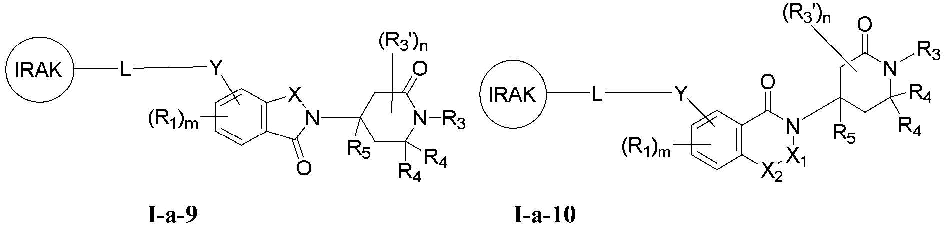
or a pharmaceutically acceptable salt thereof, wherein L and IRAK are as defined above and described in embodiments herein, and wherein: 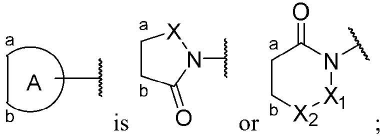
Y is a bond, Yₗ, O, NH, NR₂, C(O)O, OC(O), C(O)NR₂', NR₂'C(O), Y₁-O, Y₁-NH, Y₁-NR₂, Y₁-C(O), Y₁-C(O)O, Y₁-OC(O), Y₁-C(O)NR₂', or Y₁-NR₂'C(O), wherein Y₁ is C₁₋₆ alkylene, C₂-₆ alkenylene, or C₂-C₆ alkynylene;
X is C(O) or C(R₃)₂;
X₁-X₂ is C(R₃)=N or C(R₃)₂-C(R₃)₂;
each R¹ is independently halogen, nitro, NH₂, OH, C(O)OH, C₁₋₆ alkyl, or C₁₋₆ alkoxy;
R₂ is C₁₋₆ alkyl, C₂-₆ alkenyl, C₃-₈ cycloalkyl, 3- to 8-membered heterocycloalkyl, C(O)-C₁-₆ alkyl, C(O)-C₂-₆ alkenyl, C(O)-C₃-₈ cycloalkyl, or C(O)-3- to 8-membered heterocycloalkyl, and R₂ is optionally substituted with one or more of halogen, N(Rₐ)₂, NHC(O)Rₐ, NHC(O)ORₐ, OR_{b}, C₃-₈ cycloalkyl, 3- to 8-membered heterocycloalkyl, C₆-₁₀ aryl, or 5- to 10-membered heteroaryl, wherein each of the C₃-₈ cycloalkyl, 3- to 8-membered heterocycloalkyl, C₆-₁₀ aryl or 5- to 10-membered heteroaryl is optionally further substituted with one or more of halogen, NH₂, CN, nitro, OH, C(O)OH, C₁₋₆ alkyl, C₁₋₆ haloalkyl, C₁₋₆ alkoxy, or C₁-₆ haloalkoxy;
R₂' is H, C₁₋₆ alkyl, C₂-₆ alkenyl, C₃-₈ cycloalkyl, or 3- to 8-membered heterocycloalkyl, and R₂', when not being H, is optionally substituted with one or more of halogen, N(Rₐ)₂, NHC(O)Rₐ, NHC(O)ORₐ, OR_{b}, C₃-₈ cycloalkyl, 3- to 8-membered heterocycloalkyl, C₆-₁₀ aryl, or 5- to 10-membered heteroaryl, wherein each of the C₃-₈ cycloalkyl, 3- to 8-membered heterocycloalkyl, C₆-₁₀ aryl or 5- to 10-membered heteroaryl is optionally further substituted with one or more of halogen, NH₂, CN, nitro, OH, C(O)OH, C₁₋₆ alkyl, C₁₋₆ haloalkyl, C₁₋₆ alkoxy, or C₁₋₆ haloalkoxy;
each R₃ is independently H or C₁₋₃ alkyl optionally substituted with C₆-₁₀ aryl or 5- to 10-membered heteroaryl;
each R₃' is independently C₁₋₃ alkyl;
each R⁴ is independently H or C₁₋₃ alkyl; or two R⁴, together with the carbon atom to which they are attached, form C(O), a C₃-₆ carbocycle, or a 4-, 5-, or 6-membered heterocycle comprising 1 or 2 heteroatoms selected from N and O;
R₅ is H, C₁₋₃ alkyl, F, or Cl;
each Rₐ independently is H or C₁₋₆ alkyl;
R_{b} is H or tosyl;
t is 0 or 1;
m is 0, 1, 2 or 3; and
n is 0, 1 or 2,
as defined and described in WO 2017/007612 and US 2018/0134684, the entirety of each of which is herein incorporated by reference.

In certain embodiments, the present invention provides a compound of formula **I,** wherein LBM is an E3 ubiquitin ligase (cereblon) binding moiety 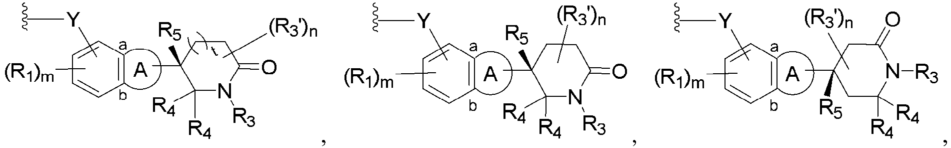 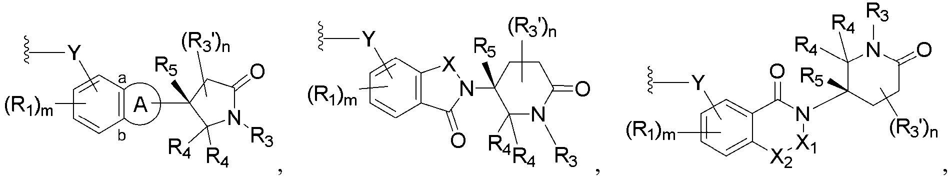 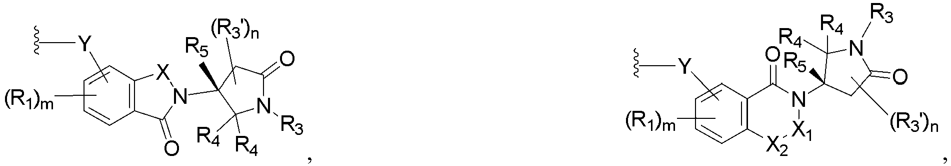 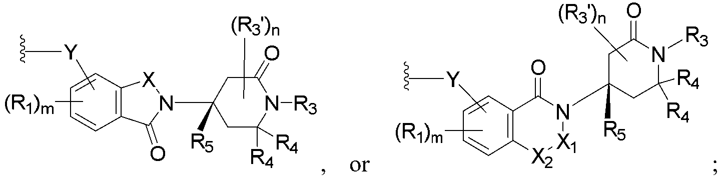 thereby forming a compound of formula **I-a'-1, I-a'-2, I-a'-3, I-a'-4, I-a'-5, I-a'-6, I-a'-7, I-a'-8, I-a'-9,** or **I-a'-10** respectively: 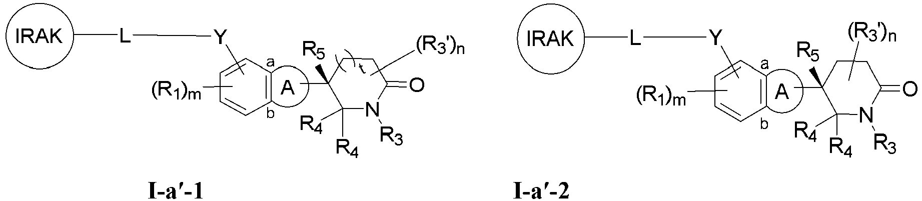 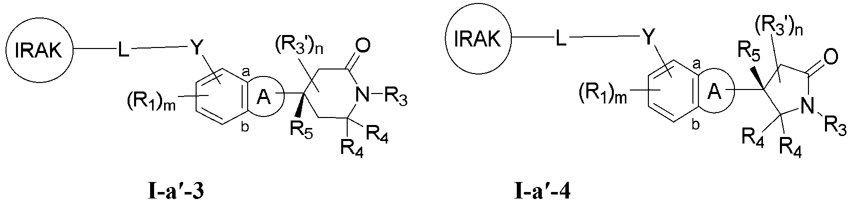 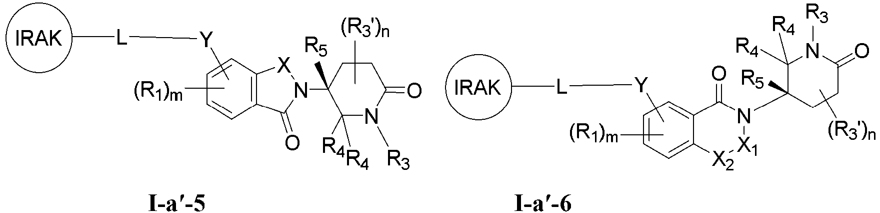 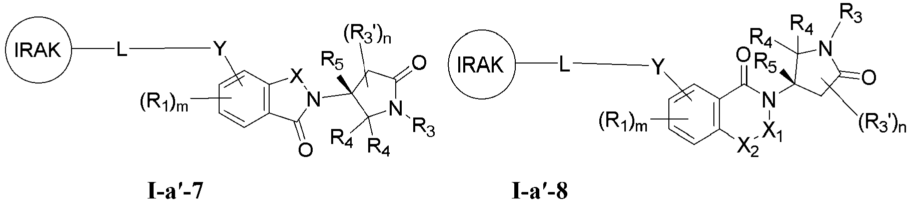 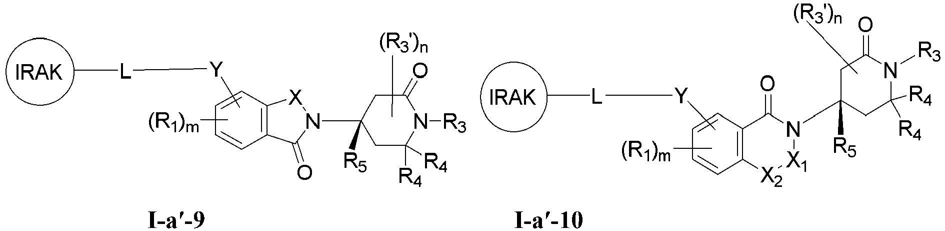 or a pharmaceutically acceptable salt thereof, wherein L and IRAK are as defined above and described in embodiments herein, and wherein each of the variables 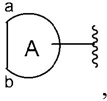 X, X₁, X₂, Y, R₁, R₃, R₃', R₄, R₅, t, m and n is as defined and described in WO 2017/007612 and US 2018/0134684, the entirety of each of which is herein incorporated by reference.

In certain embodiments, the present invention provides a compound of formula **I,** wherein LBM is an E3 ubiquitin ligase (cereblon) binding moiety 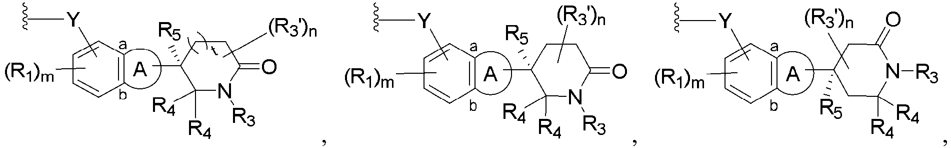 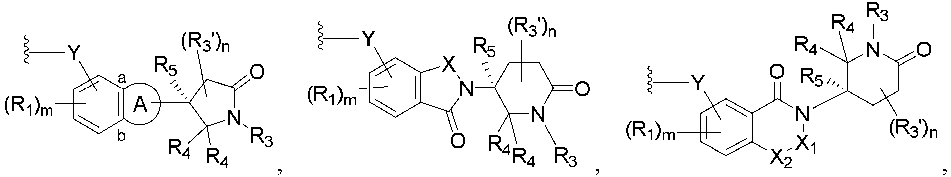 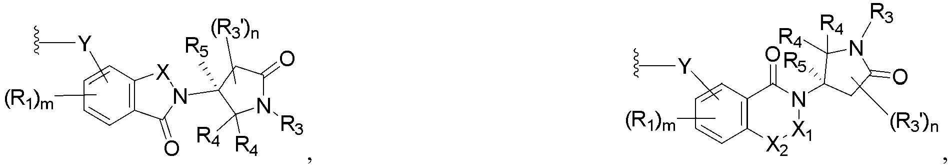 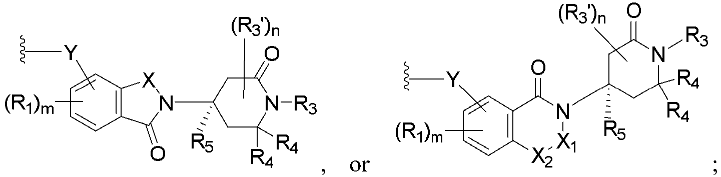 thereby forming a compound of formula I-a"-1, I-a"-2, I-a"-3, I-a"-4, I-a"-5, I-a"-6, I-a"-7, I-a"-8, I-a"-9, or - a'-10 respectively: 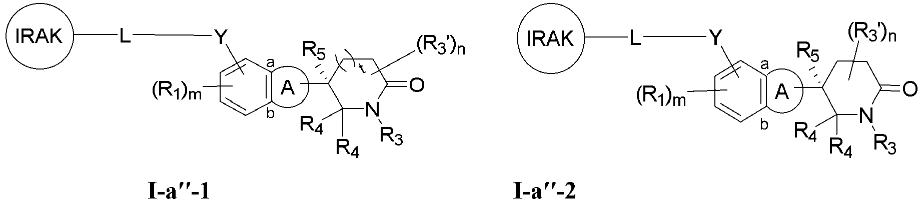 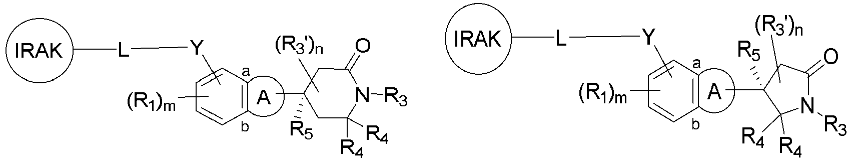 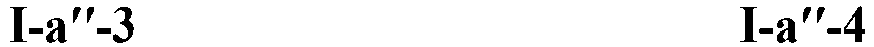 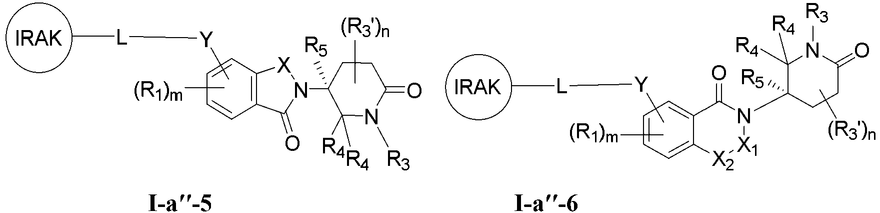 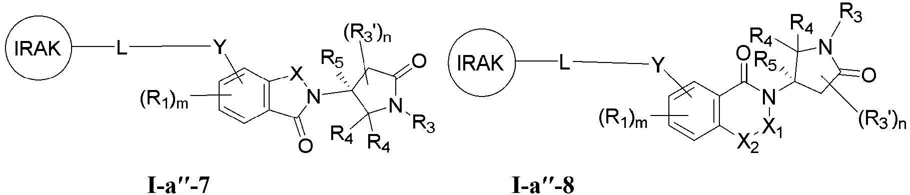 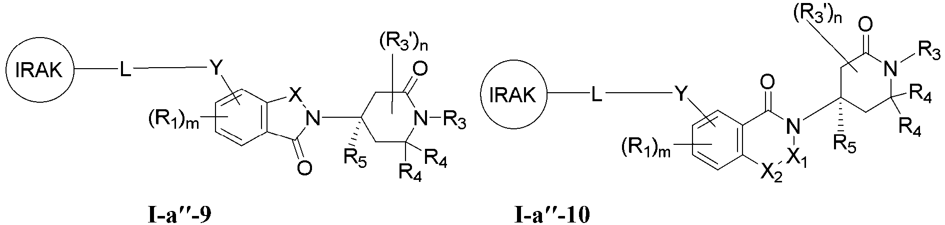 or a pharmaceutically acceptable salt thereof, wherein L and IRAK are as defined above and described in embodiments herein, and wherein each of the variables 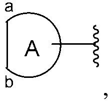 X, IX₁, X₂, Y, R₁, R₃, R₃', R₄, R₅, t, m and n is as defined and described in WO 2017/007612 and US 2018/0134684, the entirety of each of which is herein incorporated by reference.

In certain embodiments, the present invention provides a compound of formula **I,** wherein LBM is a VHL E3 ubiquitin ligase binding moiety 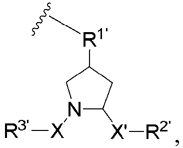 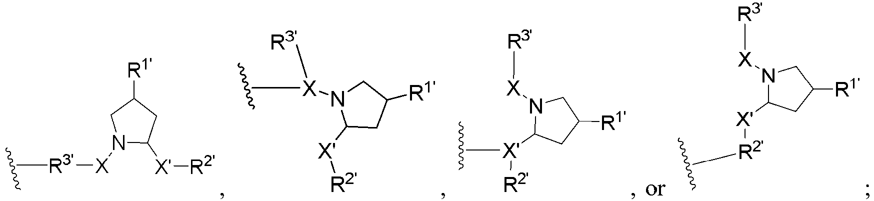 thereby forming a compound of formula I-b-1, I-b-2, I-b-3, I-b-4, or I-b-5 respectively: 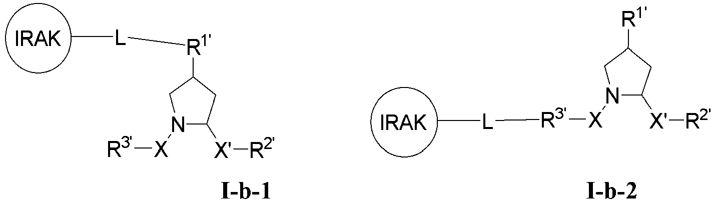 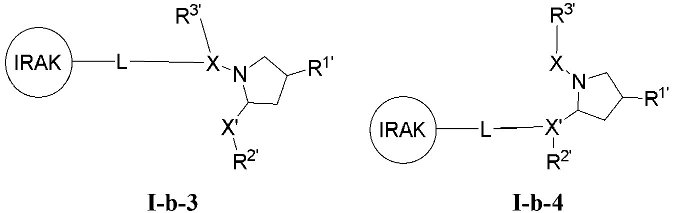 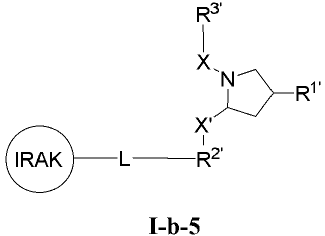 or a pharmaceutically acceptable salt thereof, wherein L and IRAK are as defined above and described in embodiments herein, and wherein each of the variables R^{1'}, R^{2'}, R₃', X, and X' is as defined and described in WO 2013/106643 and US 2014/0356322, the entirety of each of which is herein incorporated by reference.

In certain embodiments, the present invention provides a compound of formula **I,** wherein LBM is an E3 ubiquitin ligase (cereblon) binding moiety 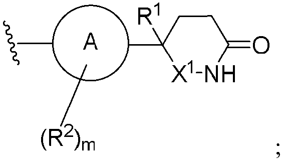 thereby forming a compound of formula I-c: 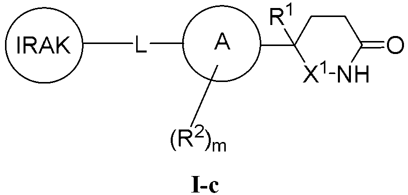 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 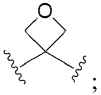
R¹ is hydrogen, deuterium, halogen, -CN, -OR, -SR, -S(O)R, -S(O)₂R, -NR₂, or an optionally substituted C₁₋₄ aliphatic;
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 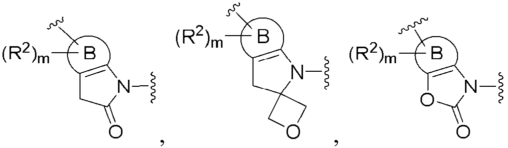 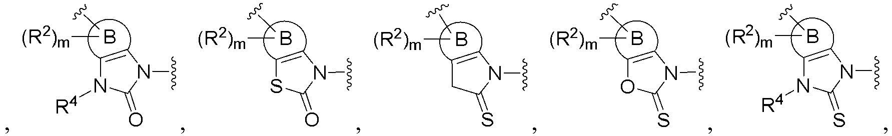 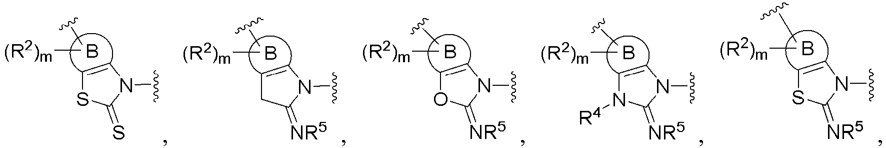 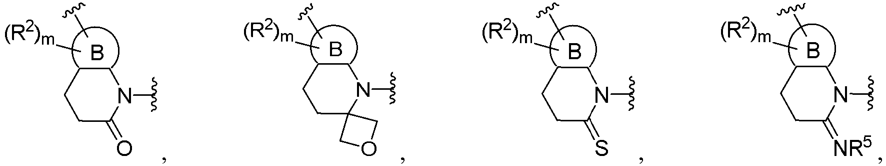 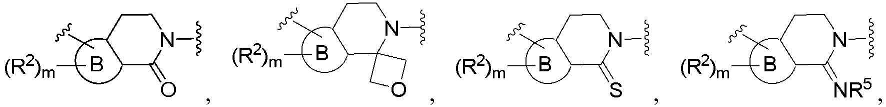 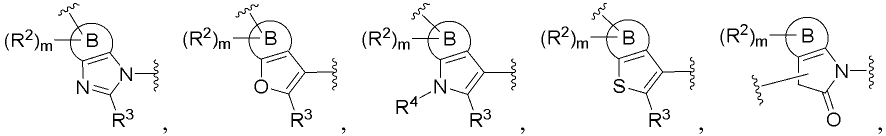 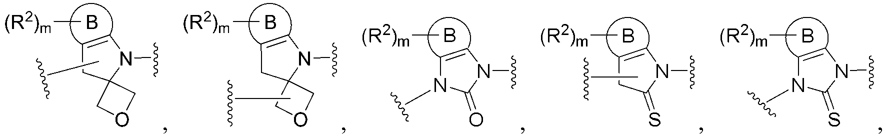 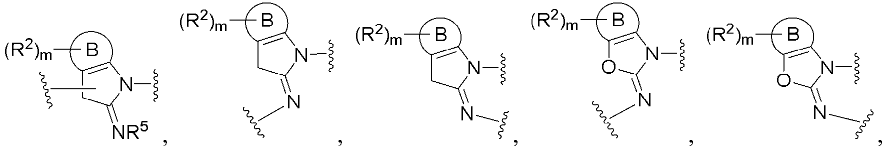 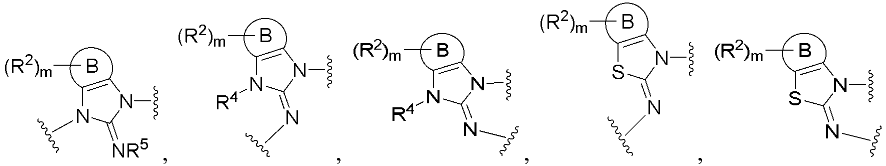 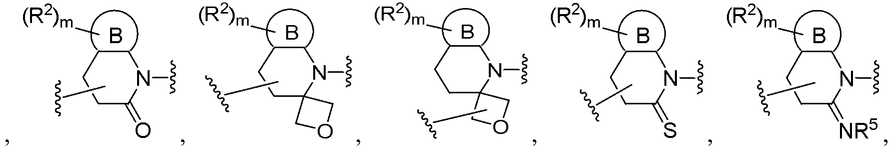 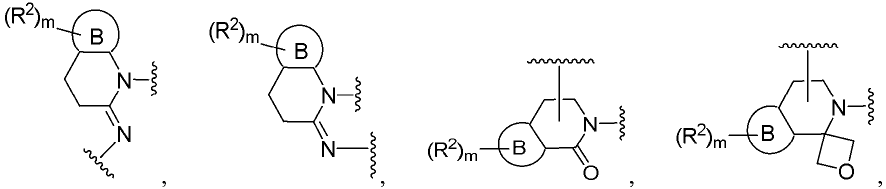 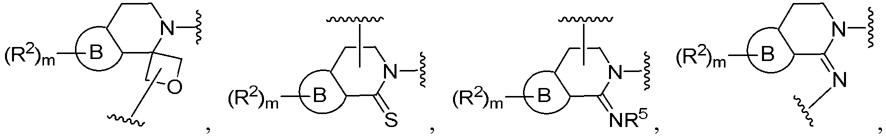 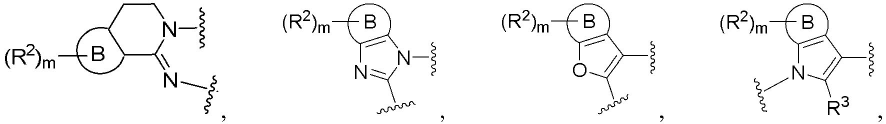 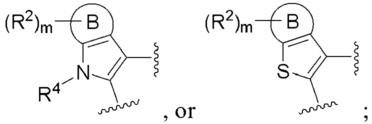
Ring B is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 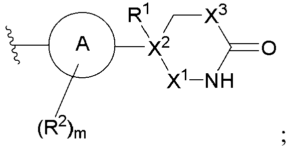 thereby forming a compound of formula I-c': 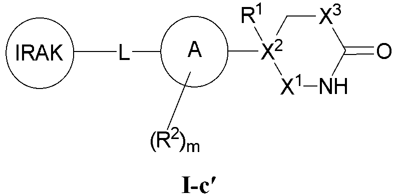 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 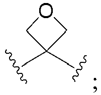
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R₂ is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 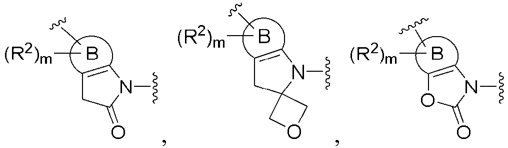 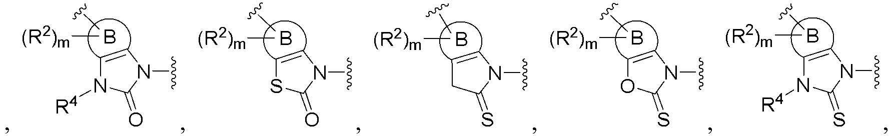 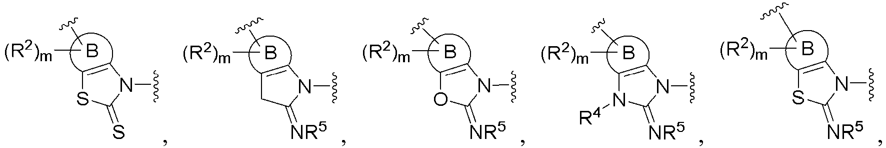 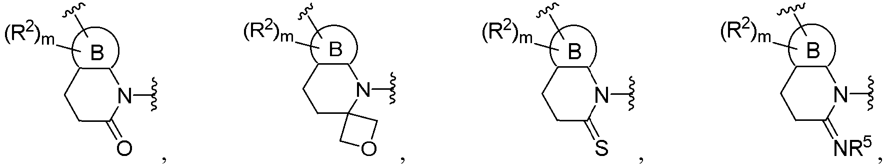 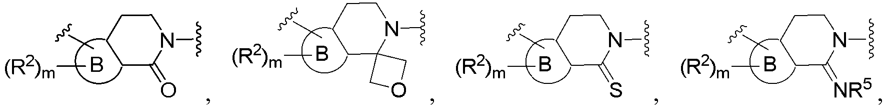 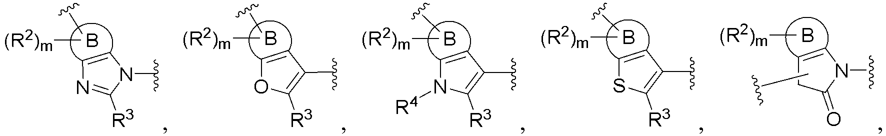 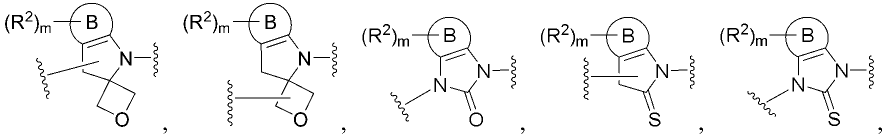 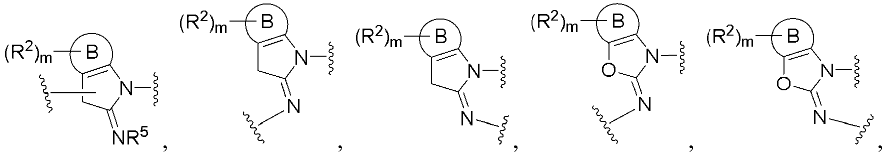 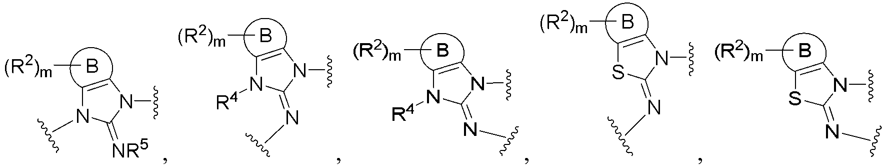 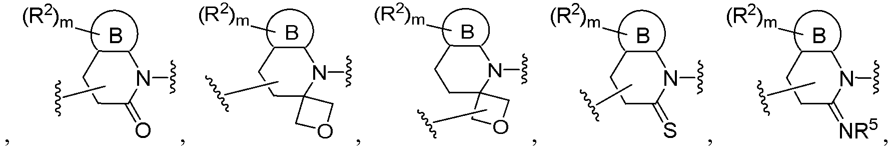 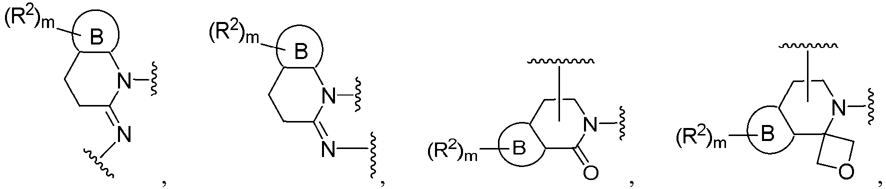 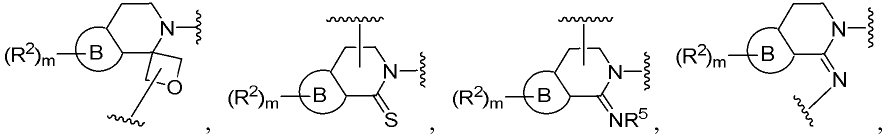 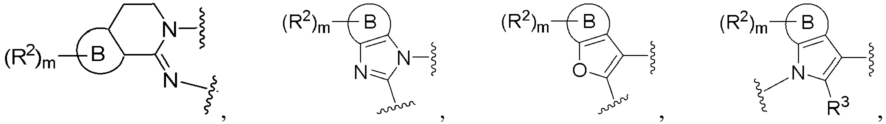 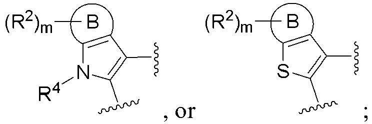
Ring B is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from boron, nitrogen, oxygen, silicon, or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala I-c' above is provided as a compound of formula I-c" or formula I-c‴: 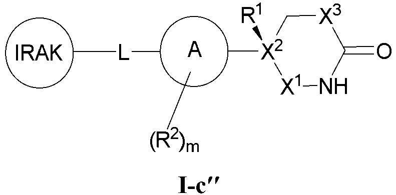 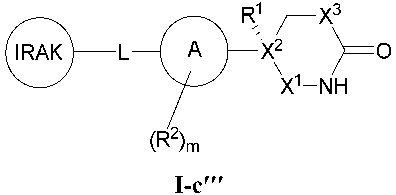 or a pharmaceutically acceptable salt thereof, wherein:
each of IRAK, Ring A, L, R¹, R², IX₁, X², X³, and m is as defined above.

In certain embodiments, the present invention provides a compound of formula **I,** wherein LBM is an E3 ubiquitin ligase (cereblon) binding moiety 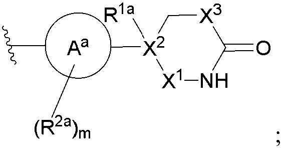 thereby forming a compound of formula I-c-1: 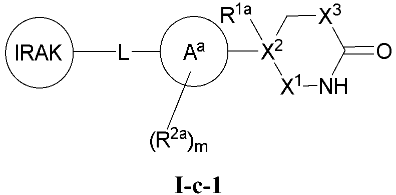 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 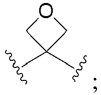
X² is a carbon atom or silicon atom;
X³ is a bivalent moiety selected from -CH₂- or -Si(R₂)-;
R^{1a} is hydrogen, deuterium, halogen, -CN, -OR, -SR, -S(O)R, -S(O)₂R, -N(R)₂, -Si(R)₃, or an optionally substituted C₁₋₄ aliphatic;
each R^{2a} is independently hydrogen, deuterium, -R^{6a}, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A^{a} is a bi- or tricyclic ring selected from 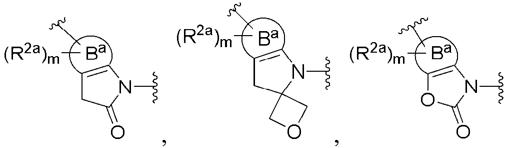 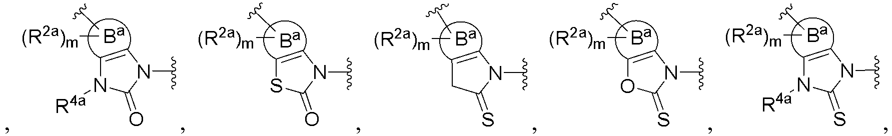 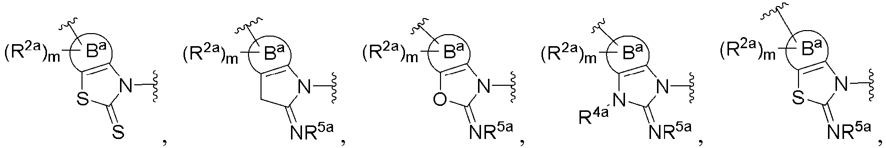 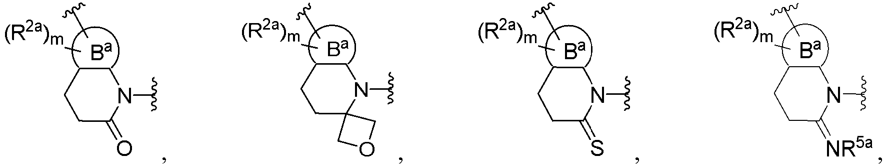 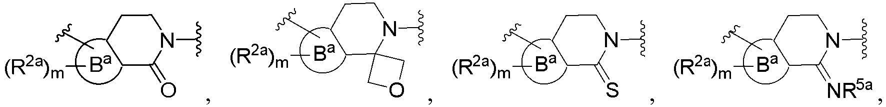 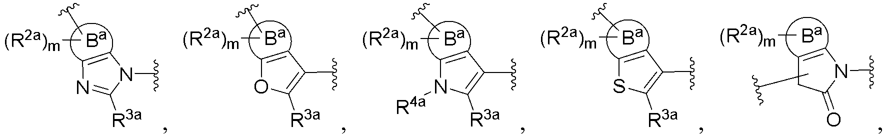 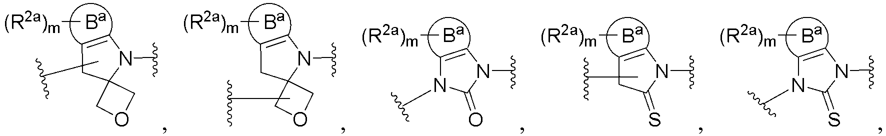 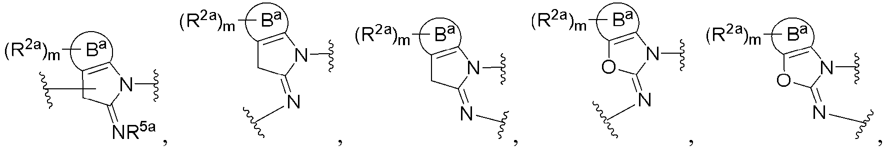 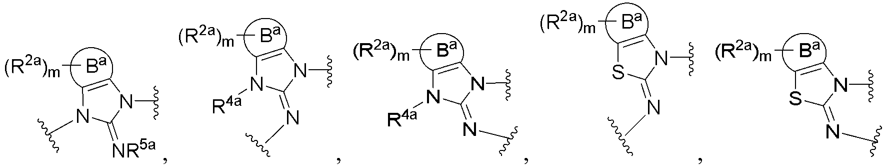 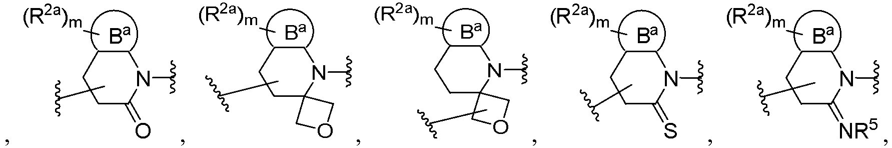 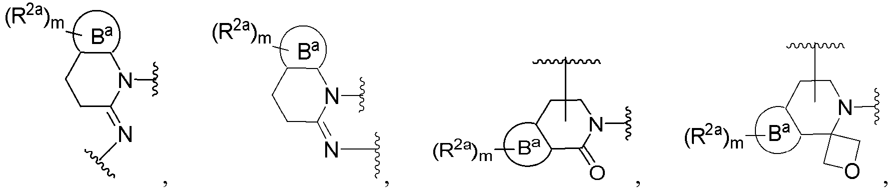 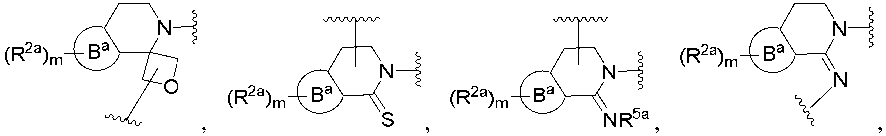 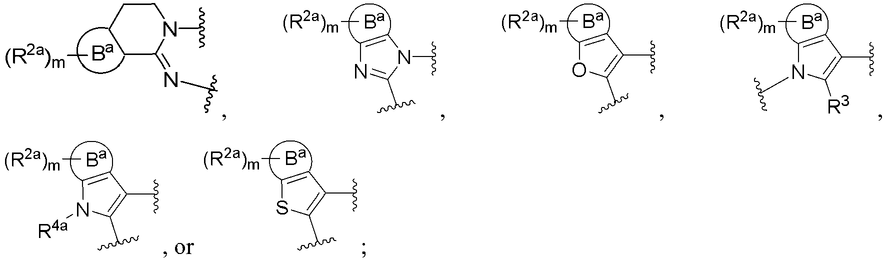
Ring B^{a} is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from boron, nitrogen, oxygen, silicon, or sulfur, or 5-membered heteroaryl with 1-3 heteroatoms independently selected from nitrogen, oxygen or sulfur;
R^{3a} is selected from hydrogen, halogen, -OR, -N(R)₂, or -SR;
each R^{4a} is independently hydrogen, -R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5a} is hydrogen, C₁₋₄ aliphatic, or -CN;
each R^{6a} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-c-1** above is provided as a compound of formula **I-c-1'** or formula **I-c-1":** 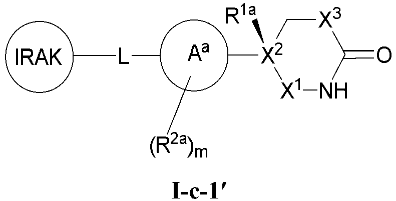 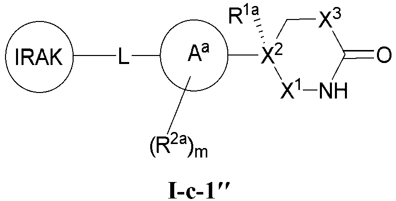 or a pharmaceutically acceptable salt thereof, wherein:
each of IRAK, Ring A^{a}, L, R^{1a}, R^{2a}, X¹, X², X³, and m is as defined above.

In certain embodiments, the present invention provides a compound of formula **I,** wherein LBM is an E3 ubiquitin ligase (cereblon) binding moiety 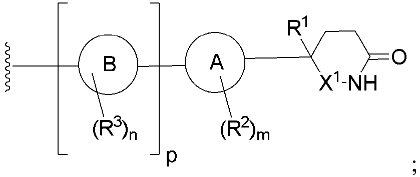 thereby forming a compound of formula **I-d:** 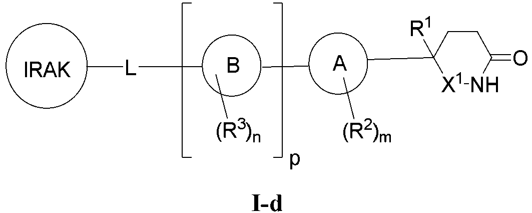 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 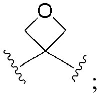
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 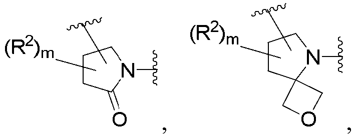 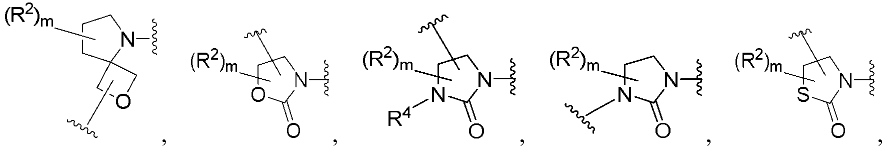 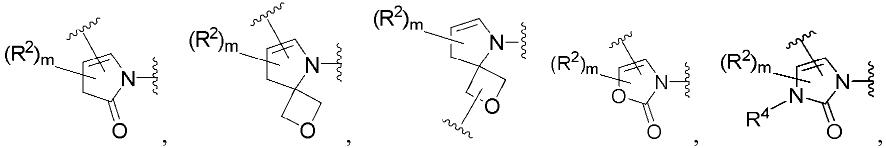 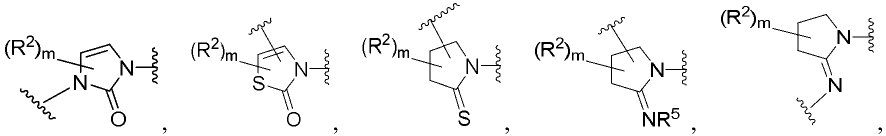 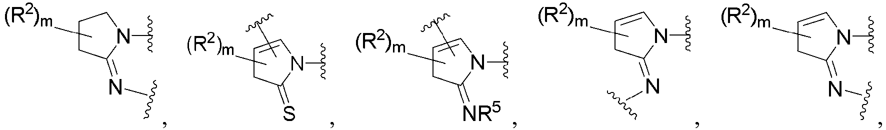 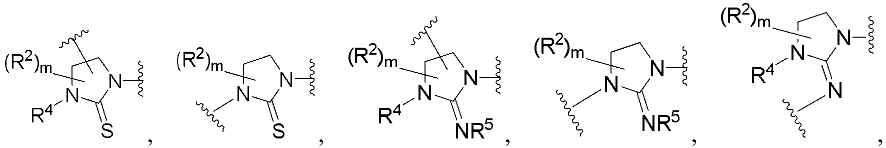 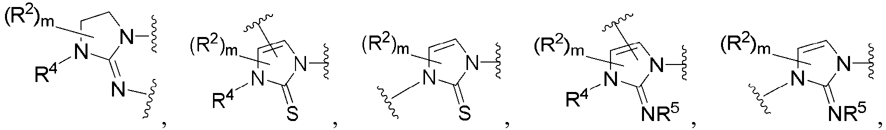 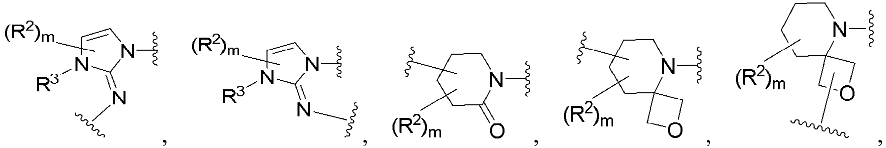 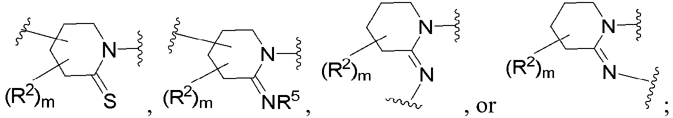
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1, wherein when p is 0, the bond connecting Ring A and Ring B is connected to 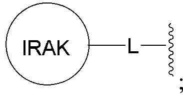 and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or: two R groups on the same nitrogen are optionally taken together with their intervening
   atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 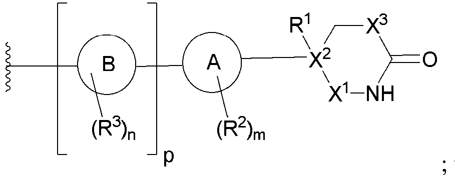 thereby forming a compound of formula **I-d':** 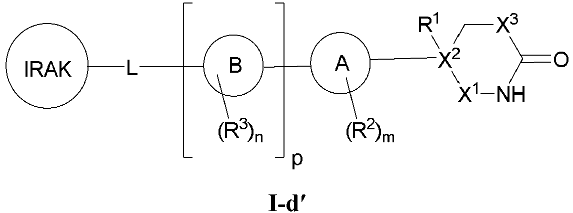 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 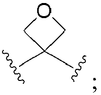
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 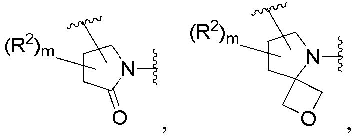 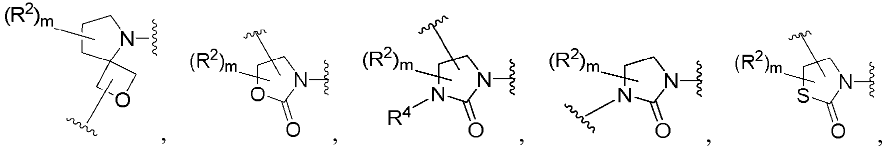 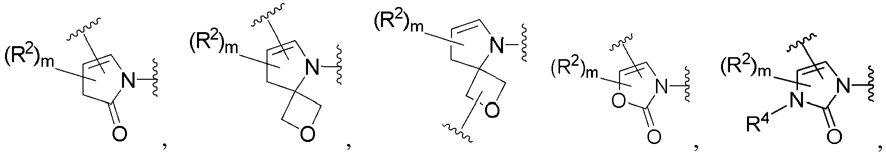 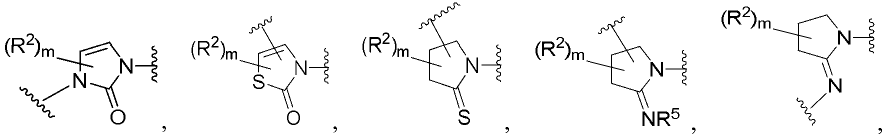 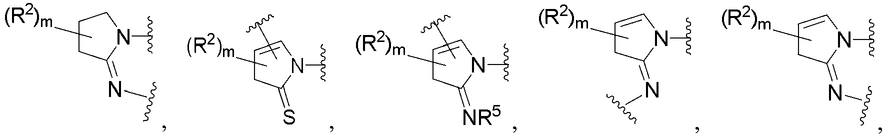 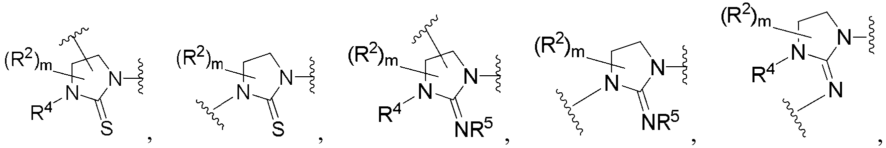 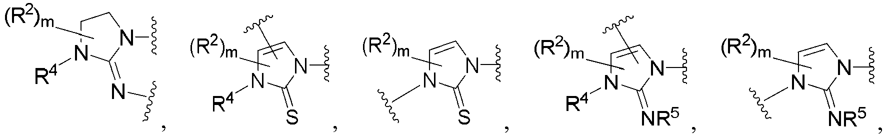 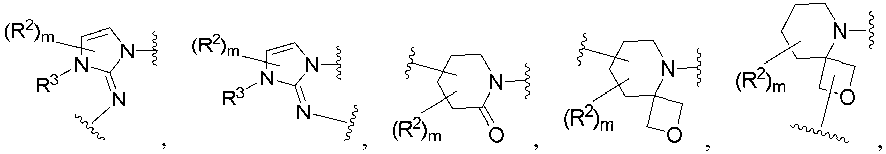 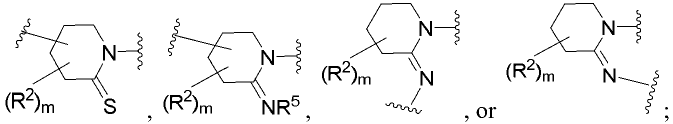
each R² is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1, wherein when p is 0, the bond connecting Ring A and Ring B is connected to 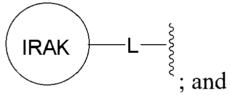
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-d'** above is provided as a compound of formula **I-d"** or formula **I-d‴:** 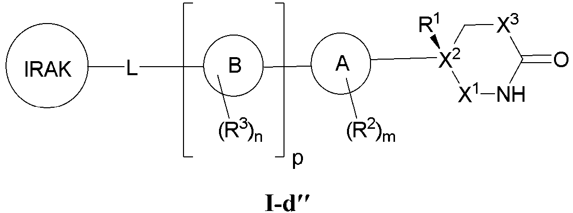 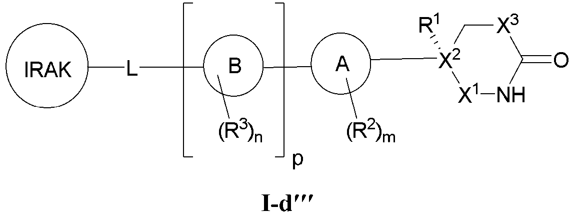 or a pharmaceutically acceptable salt thereof, wherein:
each of IRAK, Ring A, Ring B, L, R¹, R², R³, IX₁, X², X³, m, and p is as defined above.

In certain embodiments, the present invention provides a compound of formula **I,** wherein LBM is an E3 ubiquitin ligase (cereblon) binding moiety 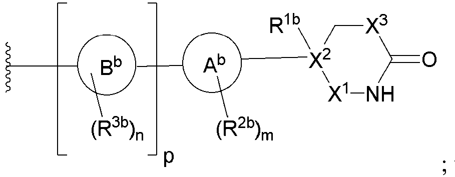 thereby forming a compound of formula **I-d-1:** 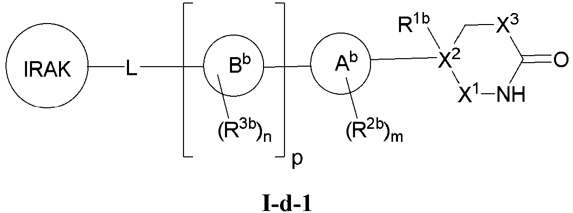 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 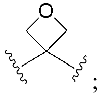
X² is a carbon atom or silicon atom;
X³ is a bivalent moiety selected from -CH₂- or -Si(R₂)-;
R^{1b} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{b} is a mono- or bicyclic ring selected from 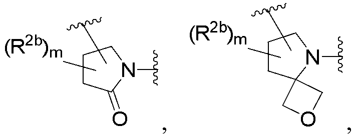 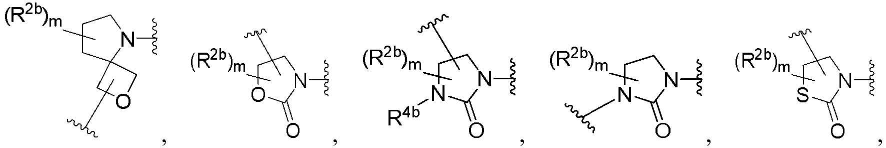 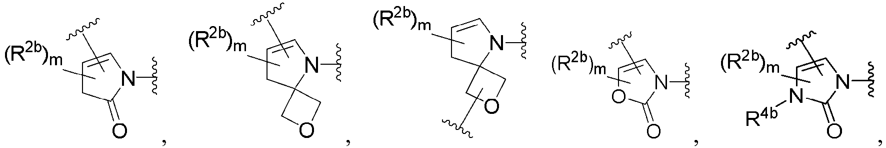 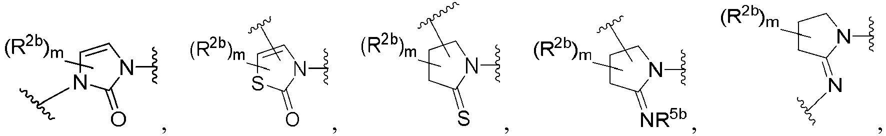 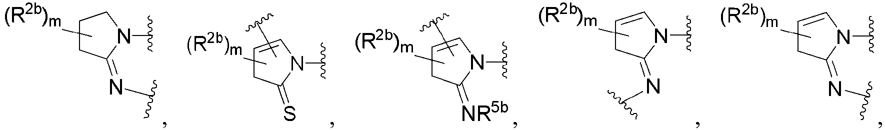 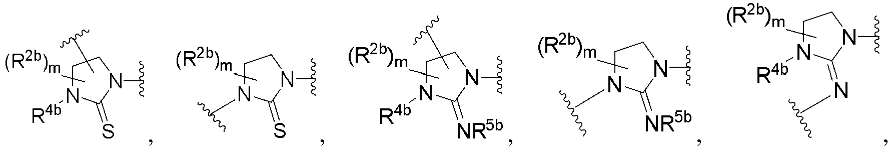 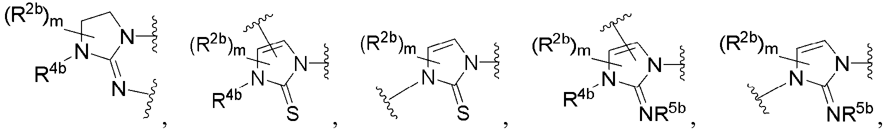 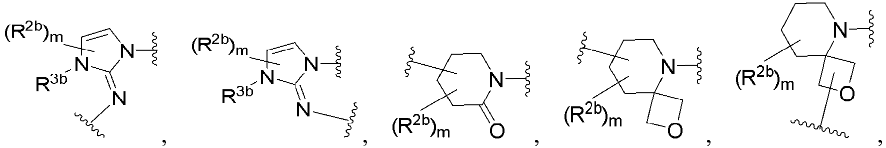 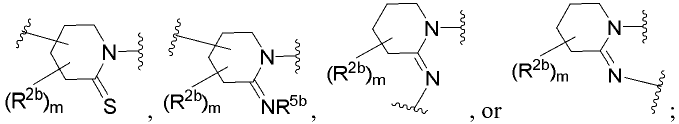
each R^{2b} is independently hydrogen, deuterium, -R^{6b}, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{b} is selected from a 6-membered aryl containing 0-2 nitrogen atoms or a 5-membered heteroaryl with 1-3 heteroatoms independently selected from nitrogen, oxygen or sulfur;
each of R^{3b} and R^{4b} is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5b} is hydrogen, C₁₋₄ aliphatic, or -CN;
each R^{6b} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1, wherein when p is 0, the bond connecting Ring A^{b} and Ring B^{b} is connected to 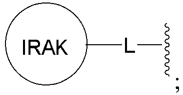 and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-d-1** above is provided as a compound of formula **I-d-1'** or formula **I-d-1":** 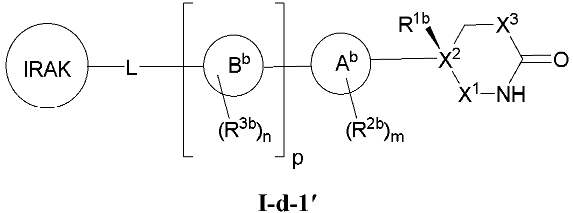 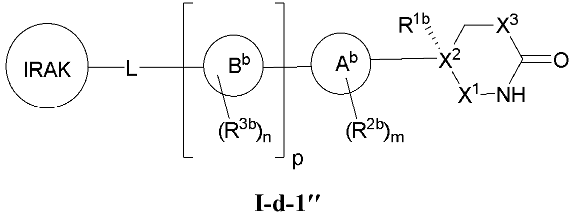 or a pharmaceutically acceptable salt thereof, wherein:
each of IRAK, Ring A^{b}, Ring B^{b}, L, R^{1b}, R^{2b}, R^{3b}, X¹, X², X³, m, and p is as defined above.

In certain embodiments, the present invention provides a compound of formula **I,** wherein LBM is an E3 ubiquitin ligase (cereblon) binding moiety 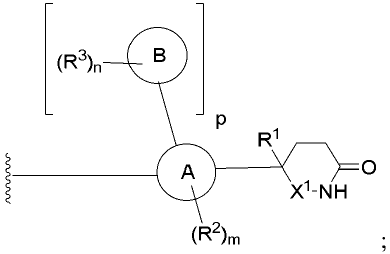 thereby forming a compound of formula **I-e:** 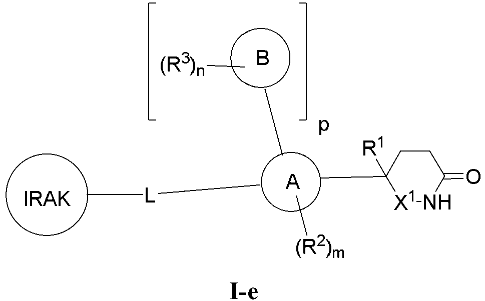 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 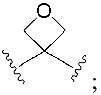
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 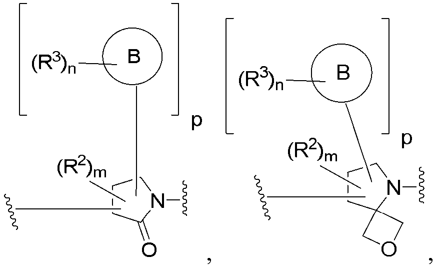 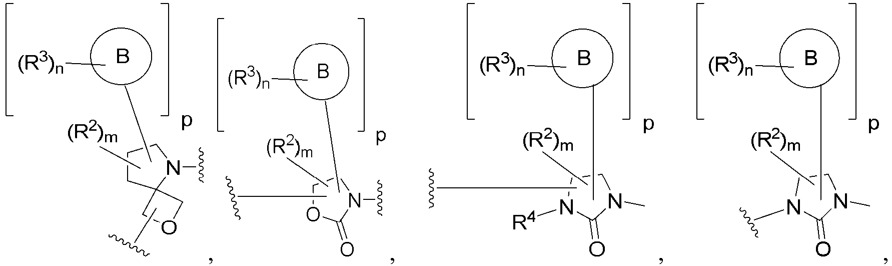 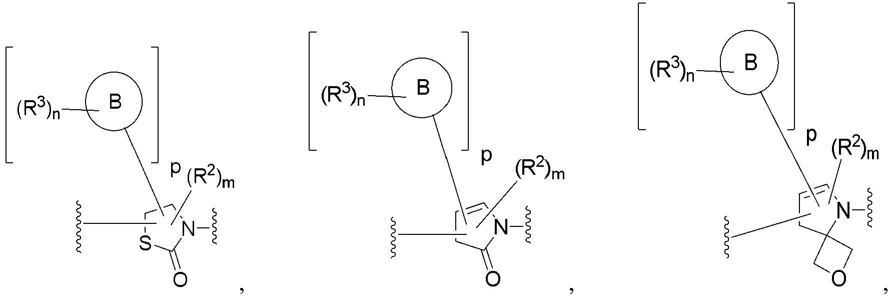 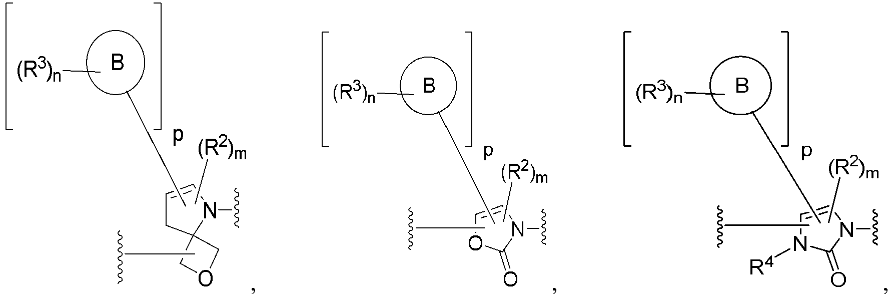 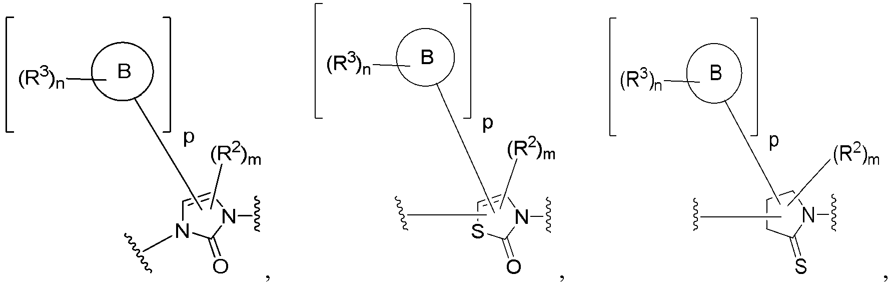 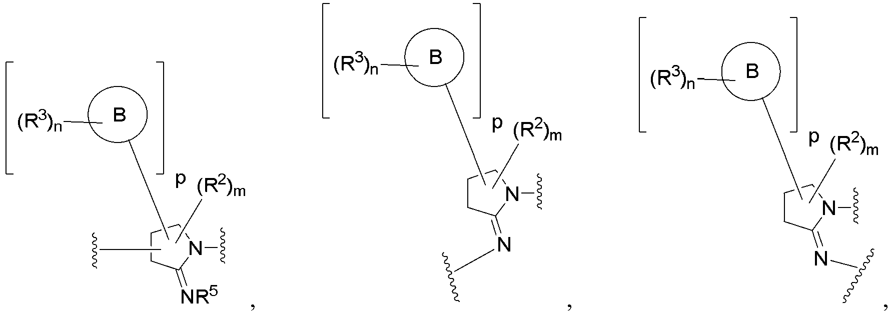 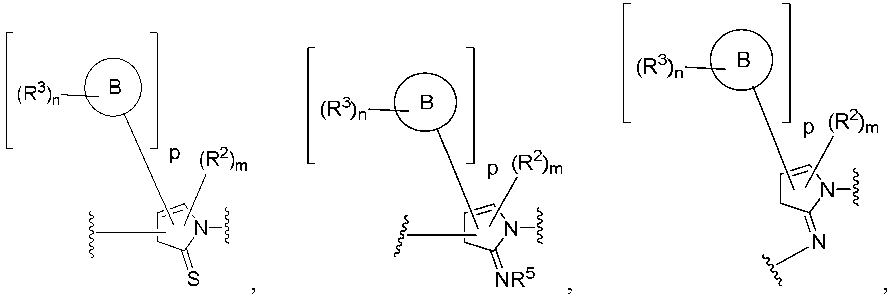 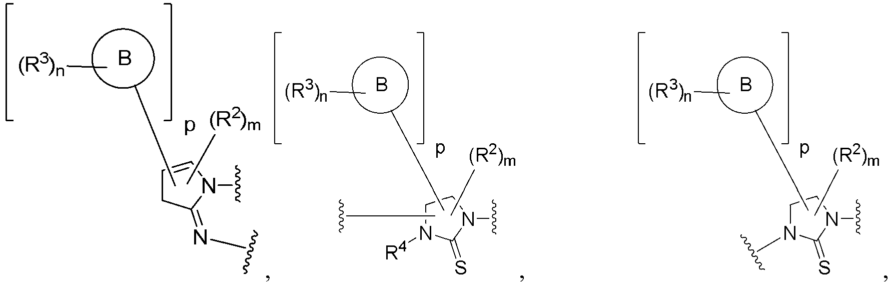 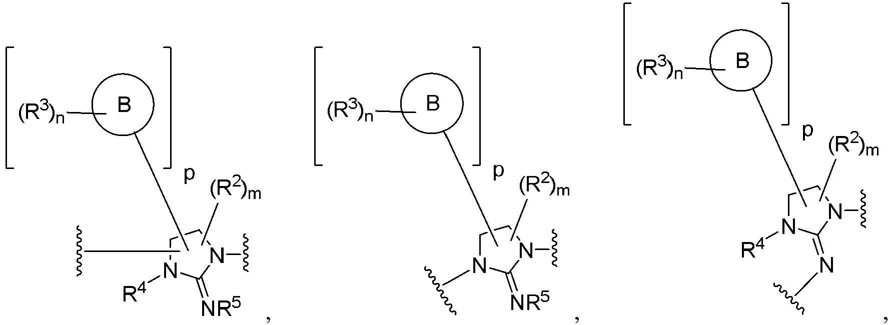 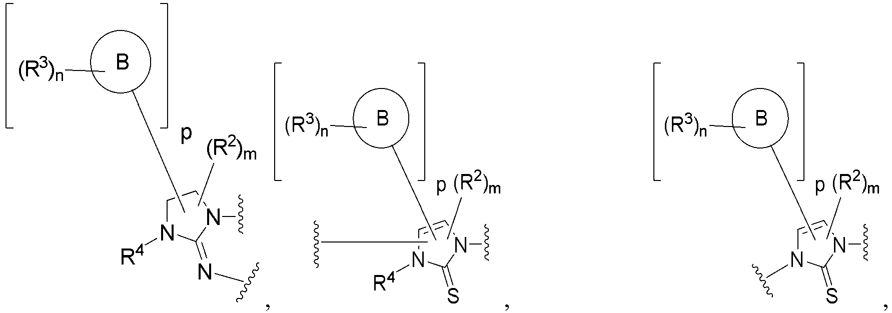 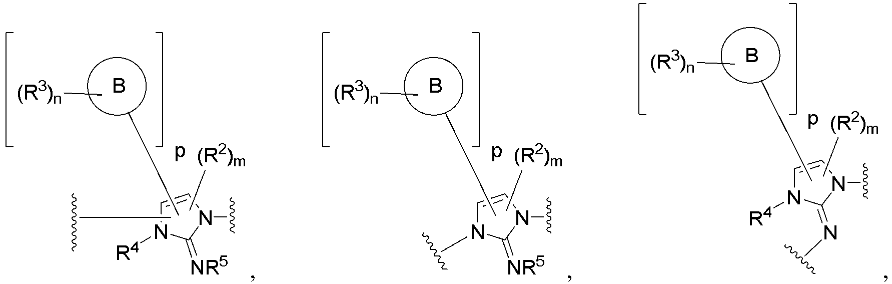 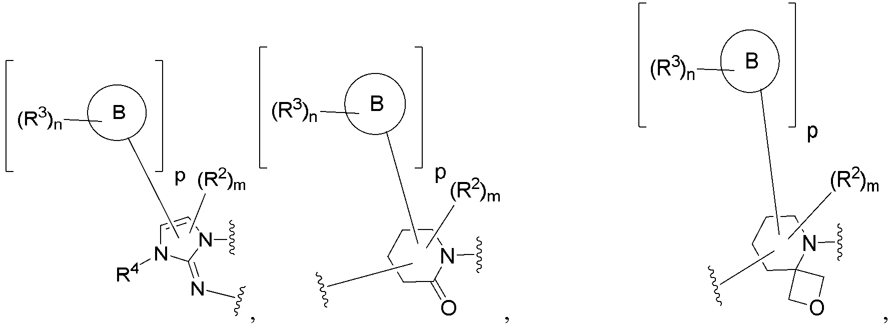 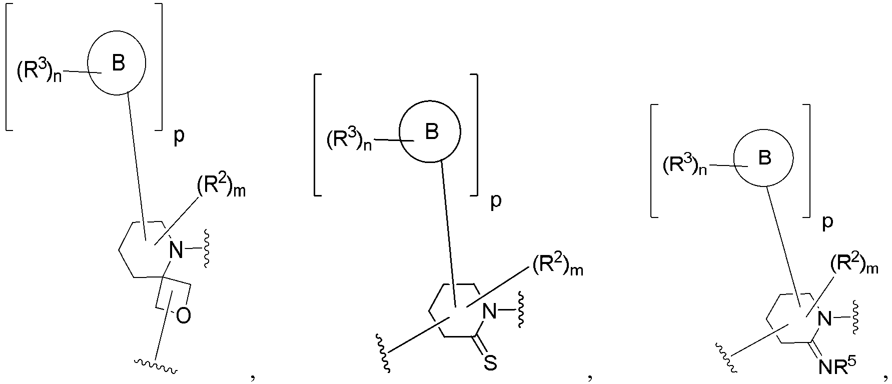 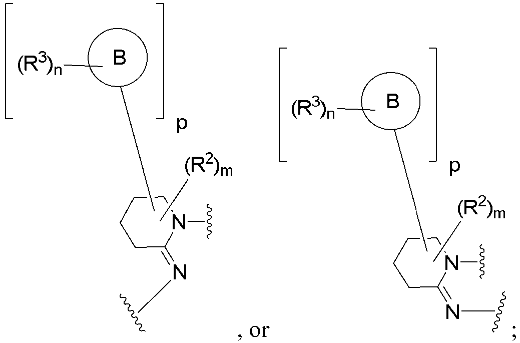
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 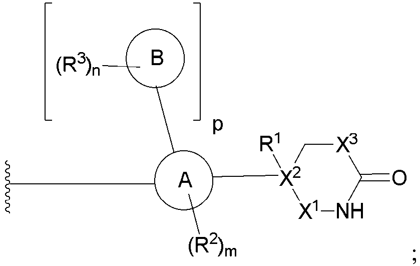 thereby forming a compound of formula I-e': 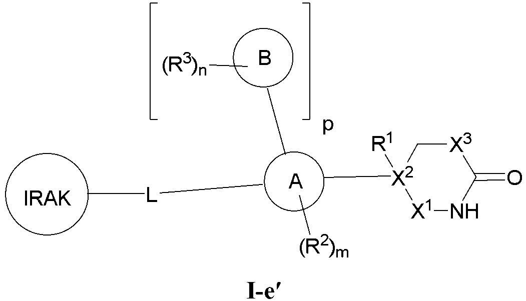 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 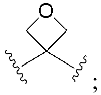
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 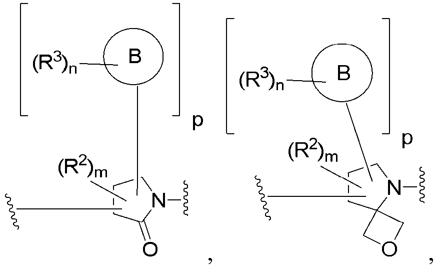 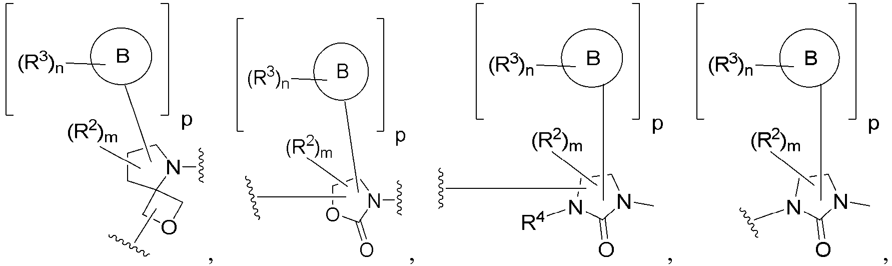 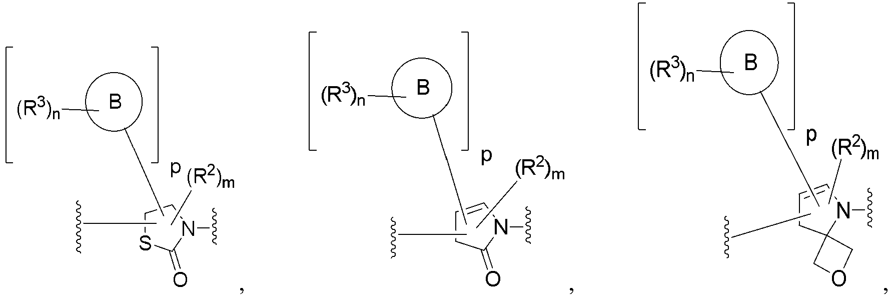 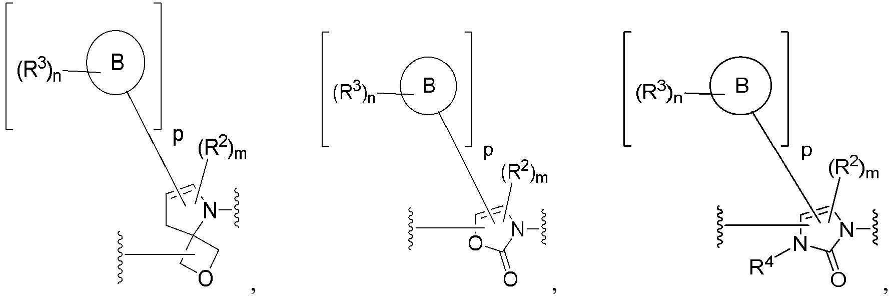 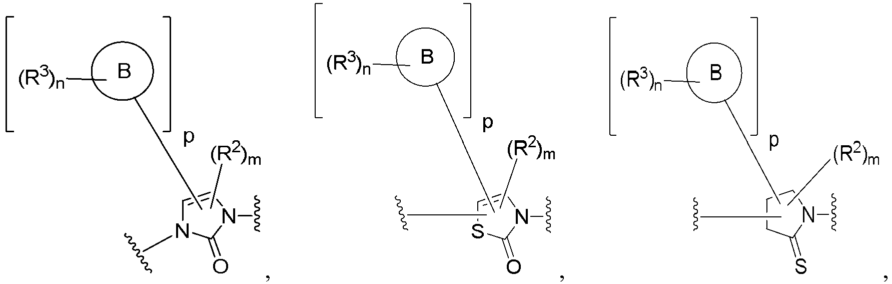 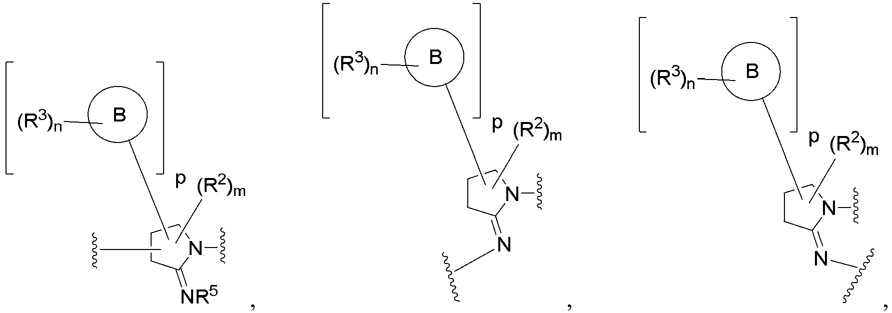 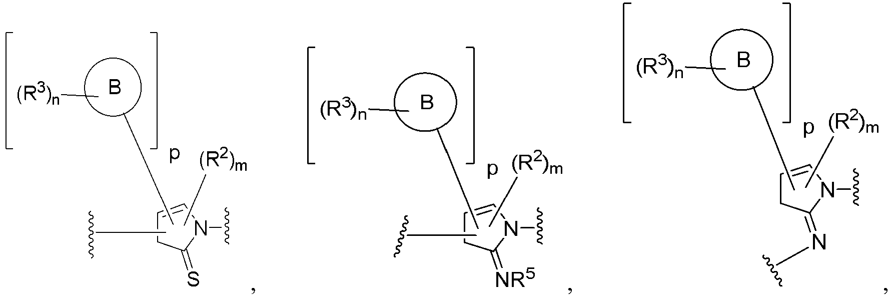 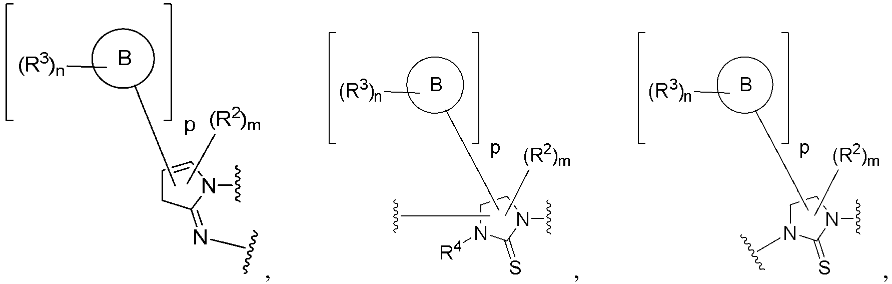 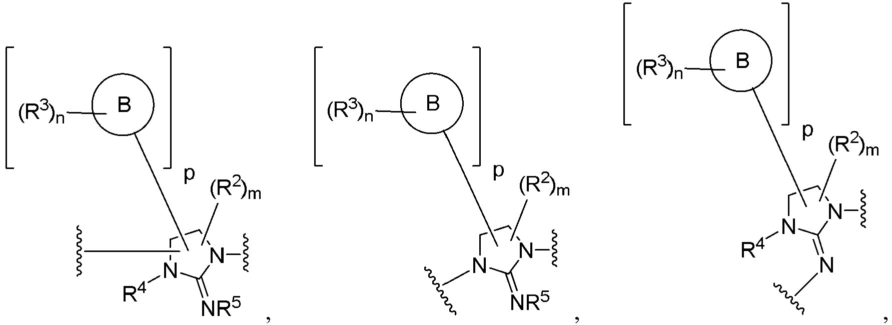 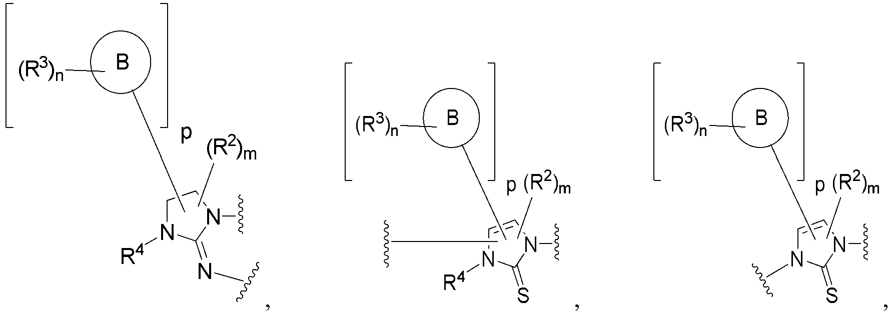 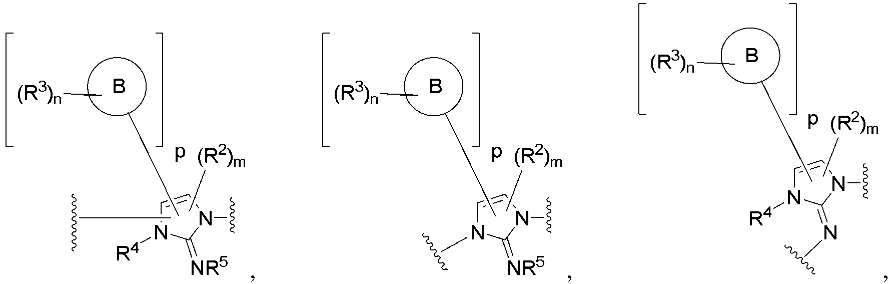 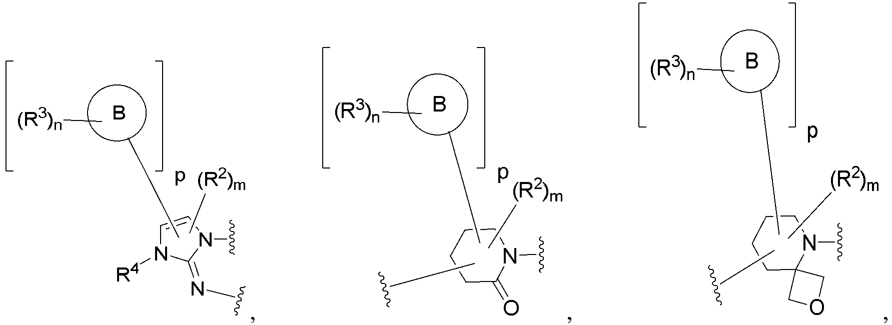 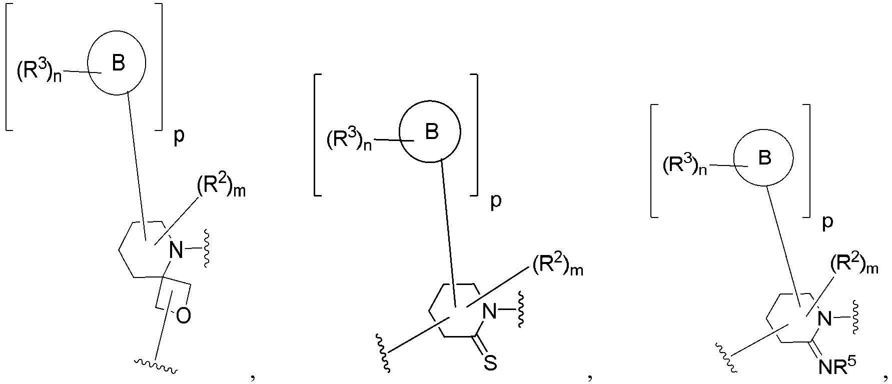 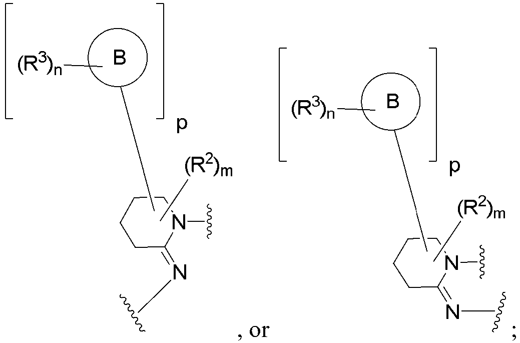
each R² is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-e'** above is provided as a compound of formula I-e" or formula **I-e‴**: 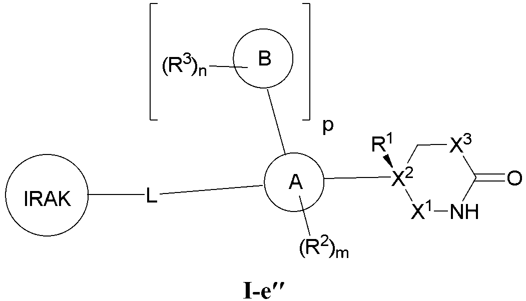 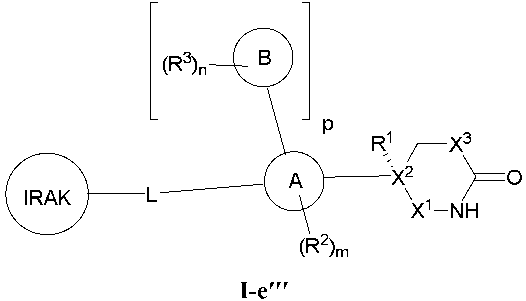 or a pharmaceutically acceptable salt thereof, wherein: each of IRAK, Ring A, Ring B, L, R¹, R², R³, X¹, X², X³, p, and m is as defined above.

In certain embodiments, the present invention provides a compound of formula **I,** wherein LBM is an E3 ubiquitin ligase (cereblon) binding moiety 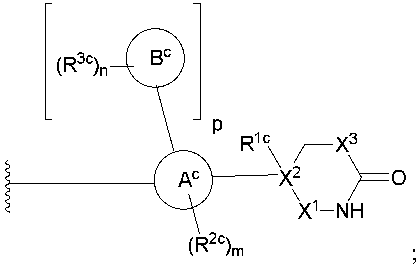 thereby forming a compound of formula I-e-1: 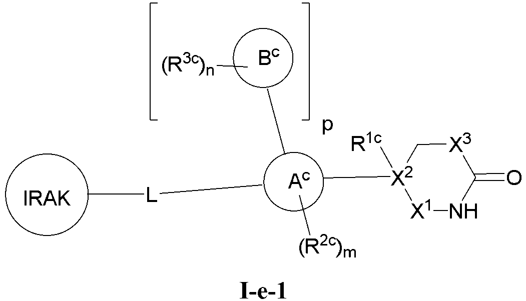 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 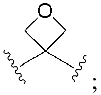
X² is a carbon atom or silicon atom;
X³ is a bivalent moiety selected from -CH₂- or -Si(R₂)-;
R^{1c} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{c} is a mono- or bicyclic ring selected from 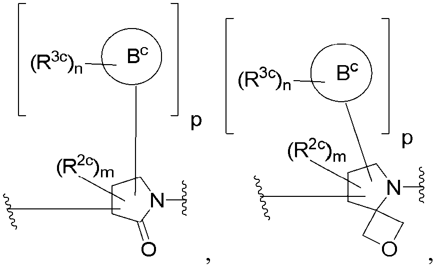 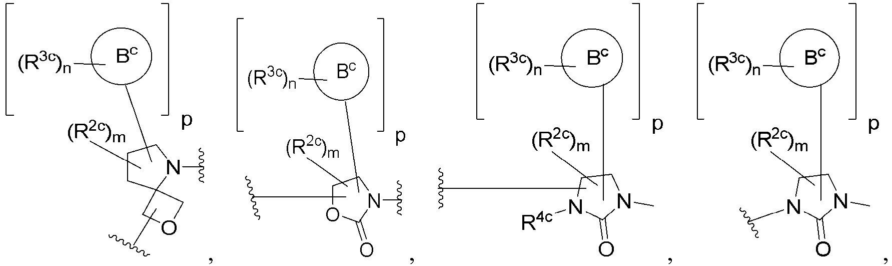 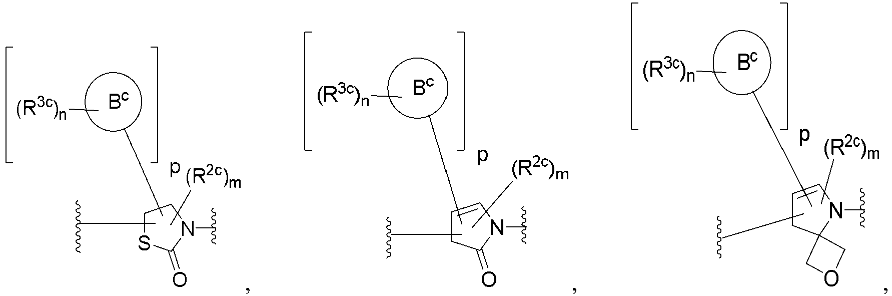 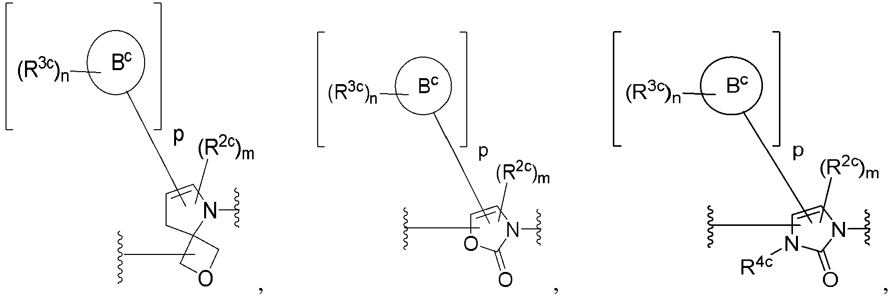 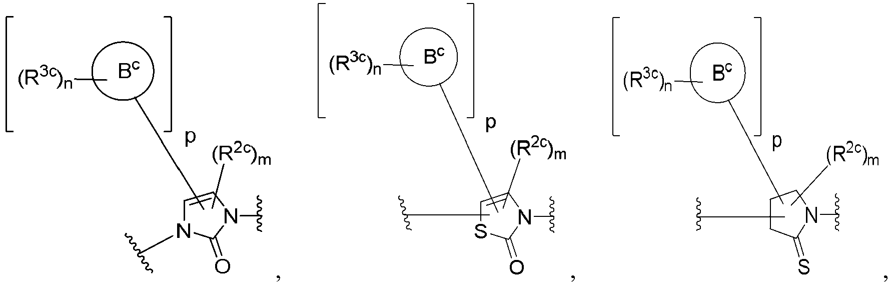 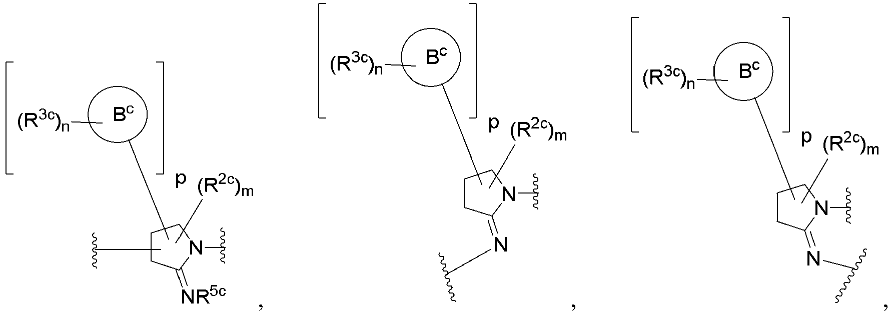 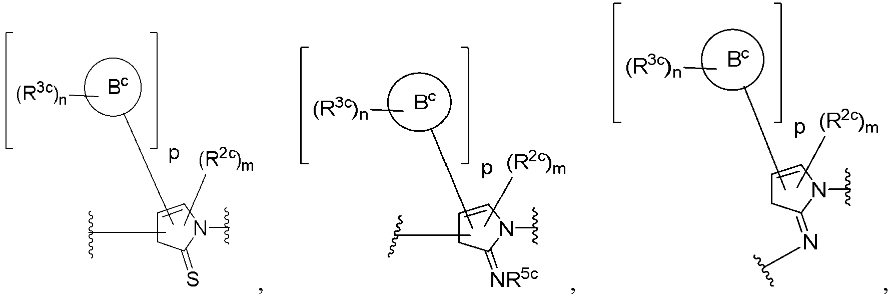 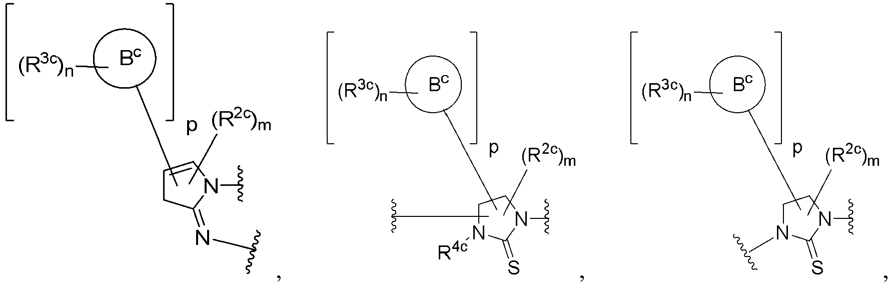 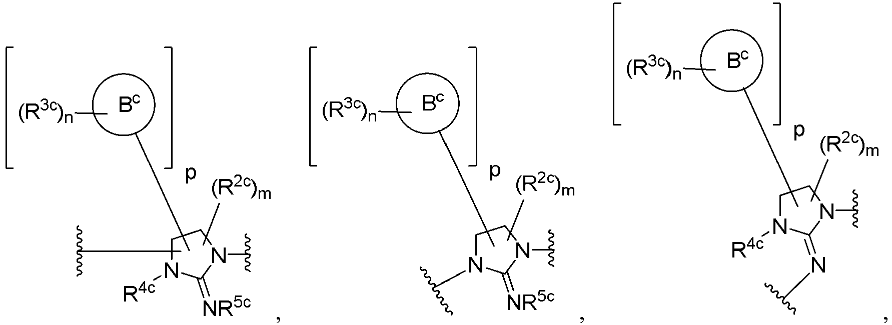 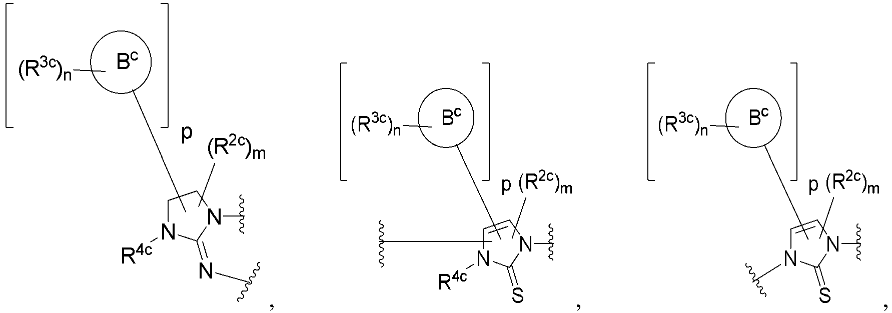 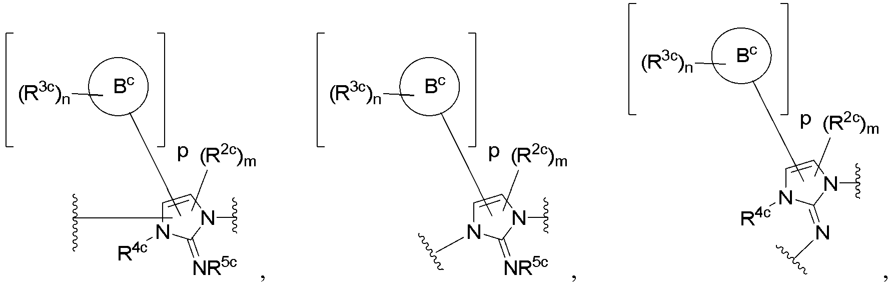 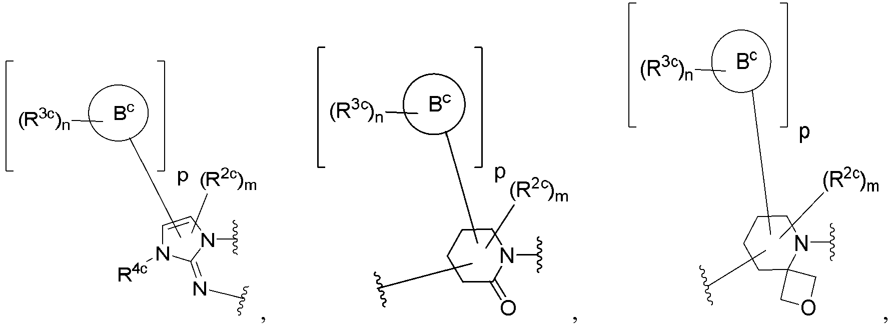 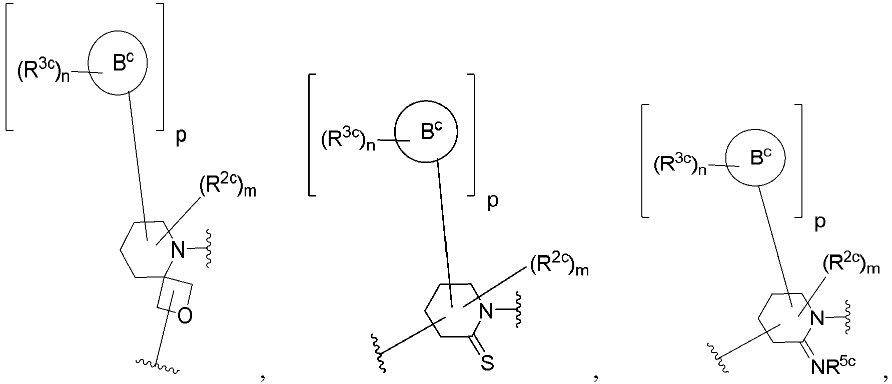 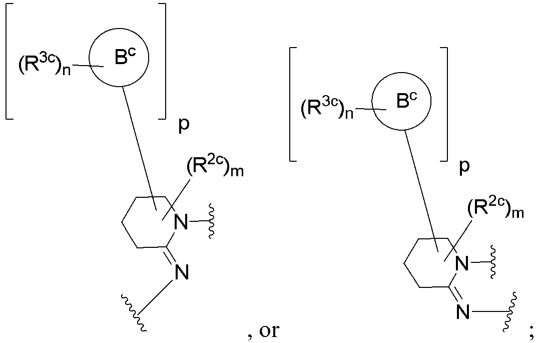
each R^{2c} is independently hydrogen, deuterium, -R^{6c}, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{c} is selected from a 6-membered aryl containing 0-2 nitrogen atoms or a 5-membered heteroaryl with 1-3 heteroatoms independently selected from nitrogen, oxygen or sulfur;
each of R^{3c} and R^{4c} is independently hydrogen, -R^{6c}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5c} is hydrogen, C₁₋₄ aliphatic, or -CN;
each R^{6c} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-e-1** above is provided as a compound of formula **I-e-1'** or formula **I-e-1":** 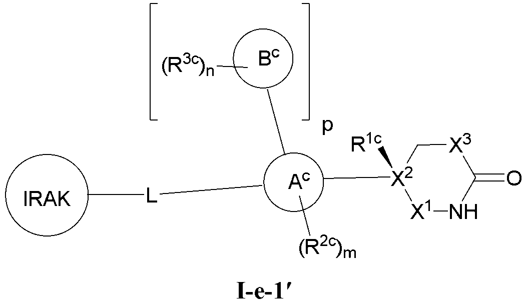 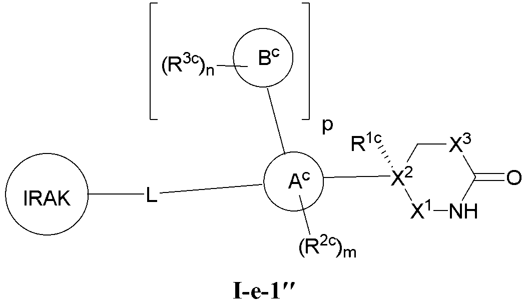 or a pharmaceutically acceptable salt thereof, wherein:
each of IRAK, Ring A^{c}, Ring B^{c}, L, R^{1c}, R^{2c}, R^{3c}, X¹, X², X³, p, and m is as defined above.

In certain embodiments, the present invention provides a compound of formula **I,** wherein LBM is a VHL E3 ubiquitin ligase binding moiety 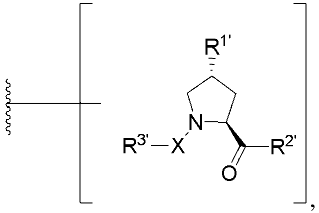 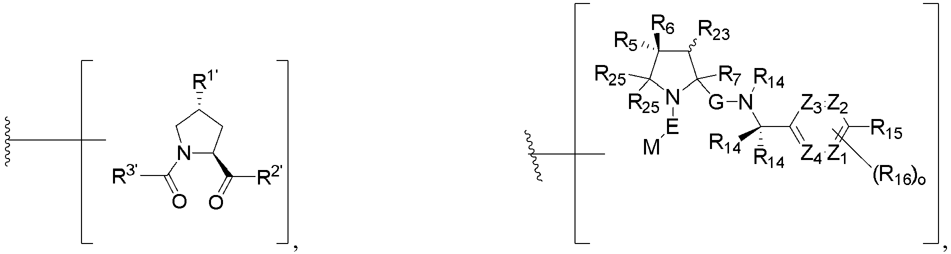 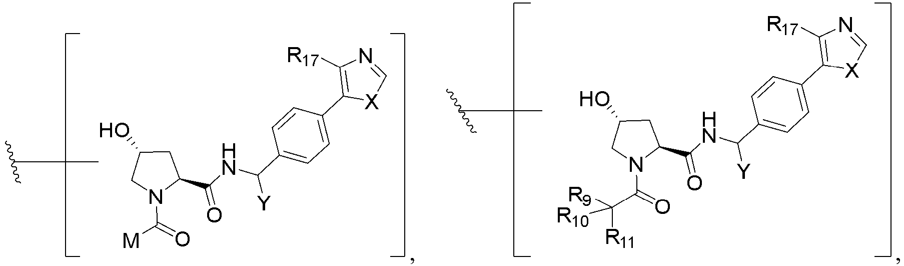 or 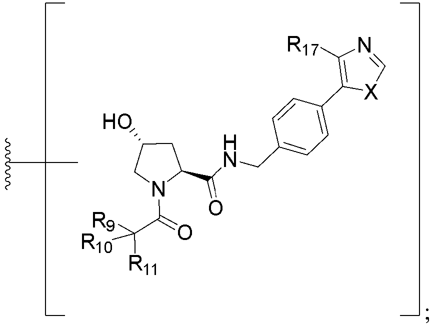 thereby forming a compound of formula I-f-1, I-f-2, **I-f-3, I-f-4, I-f-5** or **I-f-6** respectively: 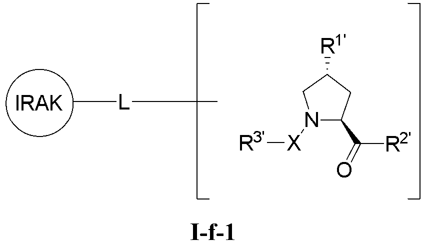 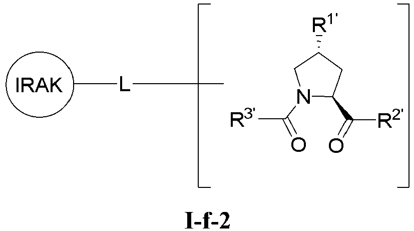 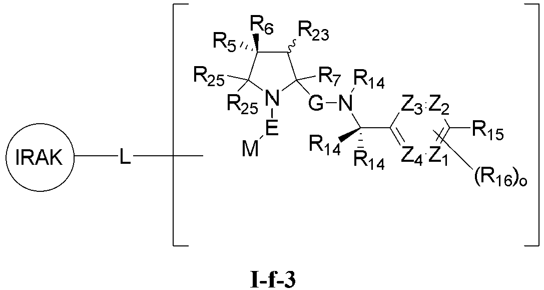 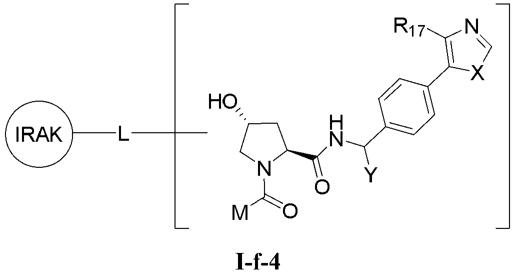 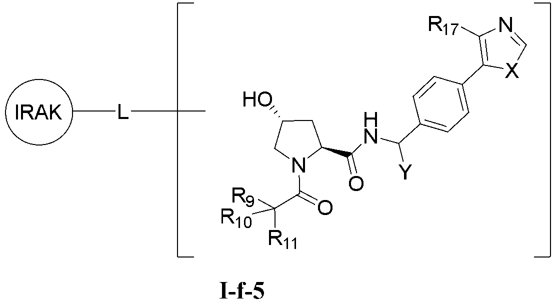 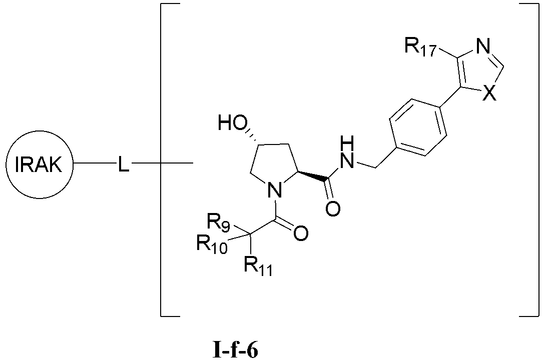 or a pharmaceutically acceptable salt thereof, wherein L and IRAK are as defined above and described in embodiments herein, and wherein each of the variables R^{1'}, R^{2'}, R^{3'}, R₅, R₆, R₇, R₉, R₁₀, R₁₁, R₁₄, R₁₅, R₁₆, R₁₇, R₂₃, R₂₅, E, G, M, X, X', Y, Z₁, Z₂, Z₃, Z₄, and o is as defined and described in WO 2016/149668 and US 2016/0272639, the entirety of each of which is herein incorporated by reference.

In certain embodiments, LBM is VHL E3 ubiquitin ligase binding moiety 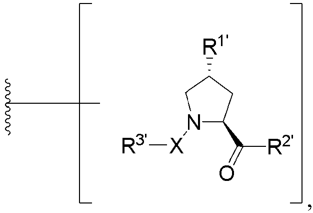 or a pharmaceutically acceptable salt thereof, wherein:
R^{1'} is an optionally substituted C₁₋₆ alkyl group, an optionally substituted -(CH₂)ₙOH, an optionally substituted -(CH₂)ₙSH, an optionally substituted (CH₂)ₙ-O-(C₁-C₆)alkyl group, an optionally substituted (CH₂)ₙ-WCOCW-(C₀-₆) alkyl group containing an epoxide moiety WCOCW where each W is independently H or a C₁-₃ alkyl group, an optionally substituted -(CH₂)ₙCOOH, an optionally substituted -(CH₂)ₙC(O)-(C₁-₆ alkyl), an optionally substituted -(CH₂)ₙNHC(O)-R₁, an optionally substituted -(CH₂)ₙC(O)-NR₁R₂, an optionally substituted -(CH₂)ₙOC(O)-NR₁R₂, -(CH₂O)ₙH, an optionally substituted - (CH₂)ₙOC(O)-(C₁-₆ alkyl), an optionally substituted -(CH₂)ₙC(O)-O-(C₁-₆ alkyl), an optionally substituted -(CH₂O)ₙCOOH, an optionally substituted -(OCH₂)ₙO-(C₁-C₆ alkyl), an optionally substituted -(CH₂O)ₙC(O)-(C₁-₆ alkyl), an optionally substituted - (OCH₂)ₙNHC(O)-R₁, an optionally substituted -(CH₂O)ₙC(O)-NR₁R₂, -(CH₂CH₂O)ₙH, an optionally substituted -(CH₂CH₂O)ₙCOOH, an optionally substituted -(OCH₂CH₂)ₙO-(C₁-₆ alkyl), an optionally substituted -(CH₂CH₂O)ₙC(O)-(C₁-C₆ alkyl), an optionally substituted -(OCH₂CH₂)ₙNHC(O)-R₁, an optionally substituted -(CH₂CH₂O)ₙC(O)-NR₁R₂, an optionally substituted -SO₂R_{S}, an optionally substituted S(O)R_{S}, NO₂, CN, or halogen;
R₁ and R₂ are each independently H or a C₁₋₆ alkyl group which may be optionally substituted with one or two hydroxyl groups or up to three halogen groups;
R_{S} is a C₁₋₆ alkyl group, an optionally substituted aryl, heteroaryl or heterocycle group or a - (CH₂)ₘNR₁R₂ group;
X and X' are each independently C=O, C=S, -S(O), S(O)₂;
R is an optionally substituted -(CH₂)ₙ-(C=O)ᵤ(NR₁)ᵥ(SO₂)_{w}alkyl group, an optionally substituted -(CH₂)ₙ-(C=O)ᵤ(NR₁)ᵥ(SO₂)_{w}NR_{1N}R_{2N} group, an optionally substituted -(CH₂)ₙ-(C=O)ᵤ(NR₁)ᵥ(SO₂)_{w}-Aryl, an optionally substituted -(CH₂)ₙ-(C=O)ᵤ(NR₁)ᵥ(SO₂)_{w}-heteroaryl, an optionally substituted -(CH₂)ₙ-(C=O)ᵥNR₁(SO₂)_{w}-heterocycle, an optionally substituted -NR¹-(CH₂)ₙ-C(O)ᵤ(NR₁)ᵥ(SO₂)_{w}-alkyl, an optionally substituted -NR¹-(CH₂)ₙ-C(O)ᵤ(NR₁)ᵥ(SO₂)_{w}-NR_{1N}R_{2N}, an optionally substituted -NR¹-(CH₂)ₙ-C(O)ᵤ(NR₁)ᵥ(SO₂)_{w}-NR₁C(O)R_{1N}, an optionally substituted -NR¹-(CH₂)ₙ-(C=O)ᵤ(NR₁)ᵥ(SO₂)_{w}-aryl, an optionally substituted -NR¹-(CH₂)ₙ-(C=O)ᵤ(NR₁)ᵥ(SO₂)_{w}-heteroaryl or an optionally substituted -NR¹-(CH₂)ₙ-(C=O)ᵥNR₁(SO₂)_{w}-heterocycle, an optionally substituted -X^{R2'}-alkyl group; an optionally substituted -X^{R2'}-aryl group; an optionally substituted -X^{R2'}-heteroaryl group; an optionally substituted -X^{R2'}-heterocycle group;
R^{3'} is an optionally substituted alkyl, an optionally substituted -(CH₂)ₙ-(O)ᵤ(NR₁)ᵥ(SO₂)_{w}-alkyl, an optionally substituted -(CH₂)ₙ-C(O)ᵤ(NR₁)ᵥ(SO₂)_{w}-NR_{1N}R_{2N}, an optionally substituted - (CH₂)ₙ-C(O)ᵤ(NR₁)ᵥ(SO₂)_{w}-NR₁C(O)R_{1N}, an optionally substituted -(CH₂)ₙ-C(O)ᵤ(NR₁)ᵥ(SO₂)_{w}-C(O)NR₁R₂, an optionally substituted -(CH₂)ₙ-C(O)ᵤ(NR₁)ᵥ(SO₂)_{w}-Aryl, an optionally substituted -(CH₂)ₙ-C(O)ᵤ(NR₁)ᵥ(SO₂)_{w}-heteroaryl, an optionally substituted -(CH₂)ₙ-C(O)ᵤ(NR₁)ᵥ(SO₂)_{w}-heterocycle, an optionally substituted -NR¹-(CH₂)ₙ-C(O)ᵤ(NR₁)ᵥ(SO₂)_{w}-alkyl, an optionally substituted -NR¹-(CH₂)ₙ-C(O)ᵤ(NR₁)ᵥ(SO₂)_{w}-NR_{1N}R_{2N}, an optionally substituted -NR¹-(CH₂)ₙ-C(O)ᵤ(NR₁)ᵥ(SO₂)_{w}-NR₁C(O)R_{1N}, an optionally substituted -NR¹-(CH₂)ₙ-C(O)ᵤ(NR₁)ᵥ(SO₂)_{w}-Aryl, an optionally substituted -NR¹-(CH₂)ₙ-C(O)ᵤ(NR₁)ᵥ(SO₂)_{w}-heteroaryl, an optionally substituted -NR¹-(CH₂)ₙ-C(O)ᵤ(NR₁)ᵥ(SO₂)_{w}-heterocycle, an optionally substituted -O-(CH₂)n-(C=O)ᵤ(NR₁)ᵥ(SO₂)_{w}-alkyl, an optionally substituted -O-(CH₂)n-(C=O)ᵤ(NR₁)ᵥ(SO₂)_{w}-NR_{1N}R_{2N}, an optionally substituted -O-(CH₂)n-(C=O)ᵤ(NR₁)ᵥ(SO₂)_{w}-NR₁C(O)R_{1N}, an optionally substituted -O-(CH₂)n-(C=O)ᵤ(NR₁)ᵥ(SO₂)_{w}-Aryl, an optionally substituted -O-(CH₂)ₙ-(C=O)ᵤ(NR₁)ᵥ(SO₂)_{w}-heteroaryl or an optionally substituted -O-(CH₂)ₙ-(C=O)ᵤ(NR₁)ᵥ(SO₂)_{w}-heterocycle; - (CH₂)ₙ-(V)_{n'}-(CH₂)ₙ-(V)_{n'}-alkyl group, an optionally substituted -(CH₂)ₙ-(V)_{n'}-(CH₂)ₙ-(V)_{n'}-aryl group, an optionally substituted -(CH₂)ₙ-(V)_{n'}-(CH₂)ₙ-(V)_{n'}-heteroaryl group, an optionally substituted -(CH₂)ₙ-(V)_{n'}-(CH₂)ₙ-(V)_{n'}-heterocycle group, an optionally substituted -(CH₂)ₙ-N(R_{1'})(C=O)_{m'}-(V)_{n'}-alkyl group, an optionally substituted -(CH₂)-N(R_{1'})(C=O)_{m'}-(V)_{n'}-aryl group, an optionally substituted -(CH₂)ₙ-N(R_{1'})(C=O)_{m'}-(V)_{n'}-heteroaryl group, an optionally substituted -(CH₂)ₙ-N(R_{1'})(C=O)_{m'}-(V)_{n'}-heterocycle group, an optionally substituted -X^{R3'}-alkyl group, an optionally substituted -X^{R3'}-aryl group, an optionally substituted -X^{R3'}-heteroaryl group, an optionally substituted -X^{R3'}-heterocycle group, wherein R_{1N} and R_{2N} are each independently H, C₁-₆ alkyl which is optionally substituted with one or two hydroxyl groups and up to three halogen groups or an optionally substituted -CH₂)ₙ-aryl, -(CH₂)ₙ-heteroaryl or -(CH₂)ₙ-heterocycle group;
V is O, S or NR₁;
R₁ is the same as above;
R¹ and R_{1'} are each independently H or a C₁-C₃ alkyl group;
X^{R2'} and X^{R3'} are each independently an optionally substituted -CH₂)ₙ-, -CH₂)ₙ-CH(Xᵥ)=CH(Xᵥ)-, -CH₂)ₙ-CH≡CH-, -(CH₂CH₂O)ₙ- or a C₃-C₆ cycloalkyl group, where Xᵥ is H, a halo or a C₁-C₃ alkyl group which is optionally substituted;
each m is independently 0, 1, 2, 3, 4, 5, 6;
each m' is independently 0 or 1;
each n is independently 0, 1, 2, 3, 4, 5, 6;
each n' is independently 0 or 1;
each u is independently 0 or 1;
each v is independently 0 or 1; and
each w is independently 0 or 1.

As used herein, depiction of brackets around any LBM 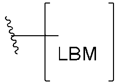 means that the 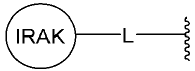 moiety is covalently attached to said LBM at any available modifiable carbon, nitrogen, oxygen, or sulfur atom. For purposes of clarity and by way of example, such available modifiable carbon, nitrogen, oxygen, or sulfur atoms in the following LBM compound structure are depicted below, wherein each wavy bond defines the point of attachment to said

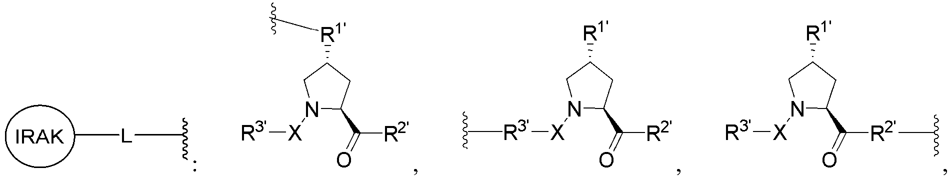

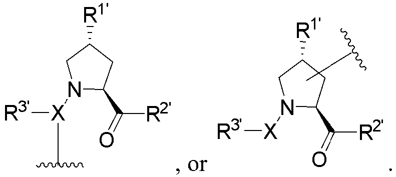

In certain embodiments, the present invention provides a compound of formula **I,** wherein LBM is a VHL E3 ubiquitin ligase binding moiety 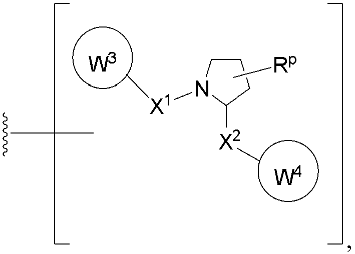 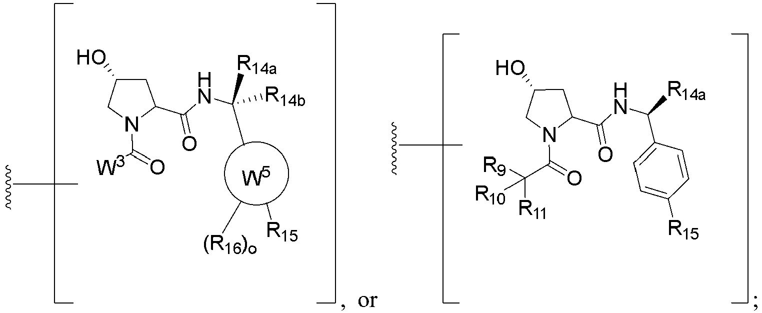 thereby forming a compound of formula **I-g-1, I-g-2,** or **I-g-3** respectively: 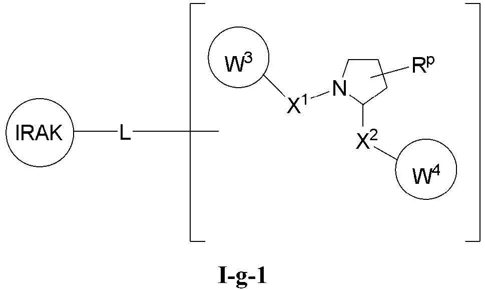 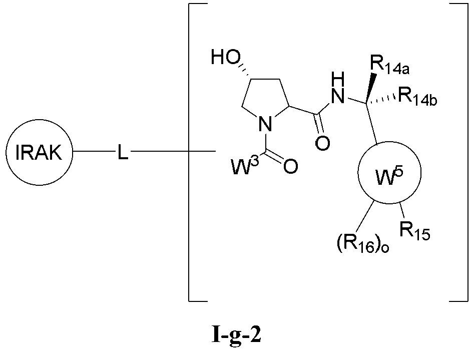 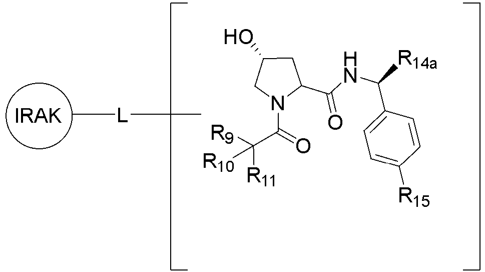 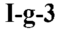 or a pharmaceutically acceptable salt thereof, wherein L and IRAK are as defined above and described in embodiments herein, and wherein each of the variables R^{p}, R₉, R₁₀, R₁₁, R₁₄ₐ, R_{14b}, R₁₅, R₁₆, W³, W⁴, W⁵, X¹, X², and o is as defined and described in WO 2017/030814, WO 2016/118666 and US 2016/0214972, the entirety of each of which is herein incorporated by reference.

In certain embodiments, the present invention provides a compound of formula **I,** wherein LBM is an E3 ubiquitin ligase (cereblon) binding moiety 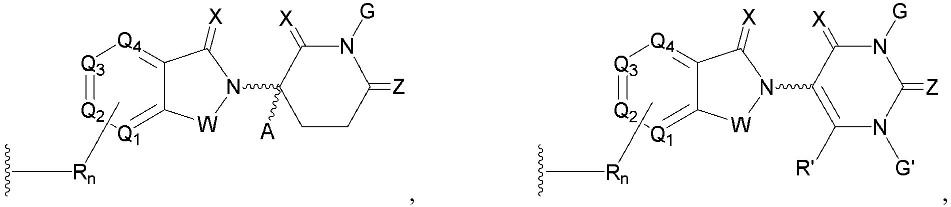 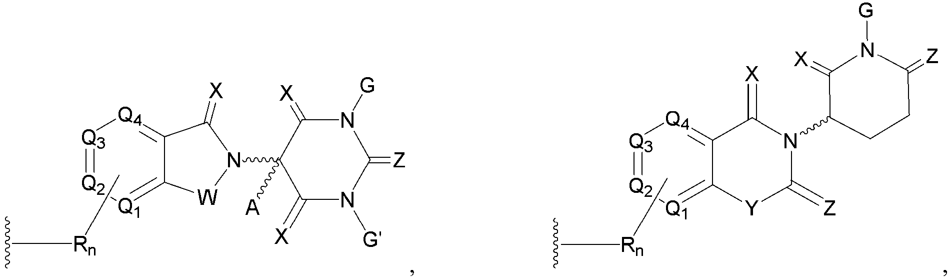 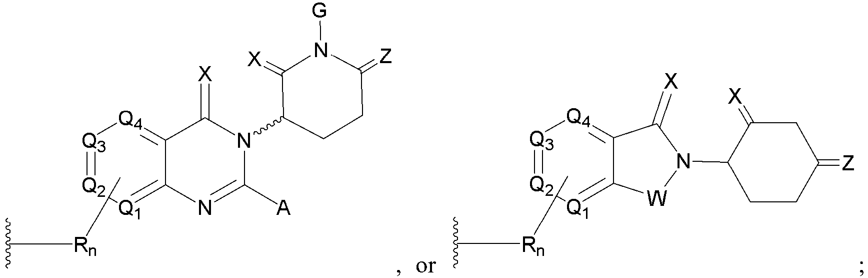 thereby forming a compound of formula **I-h-1, I-h-2, I-h-3, I-h-4, I-h-5,** or **I-h-6** respectively: 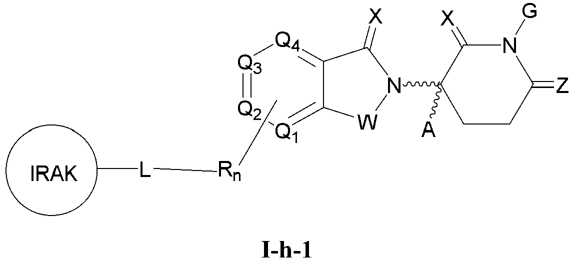 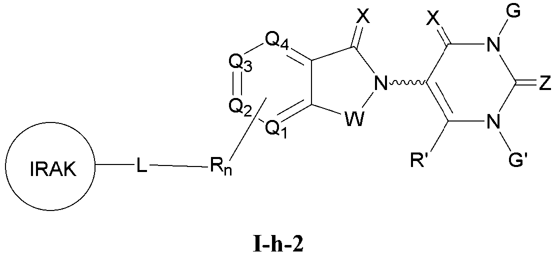 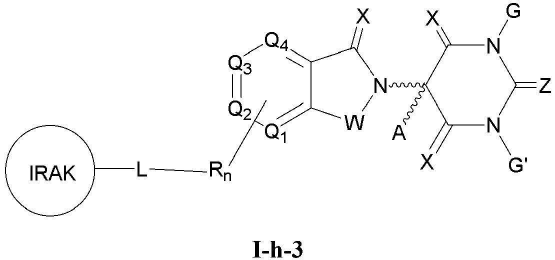 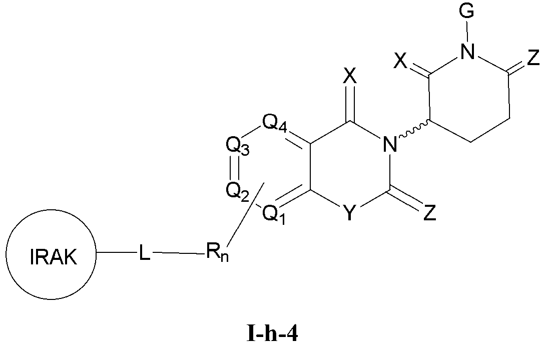 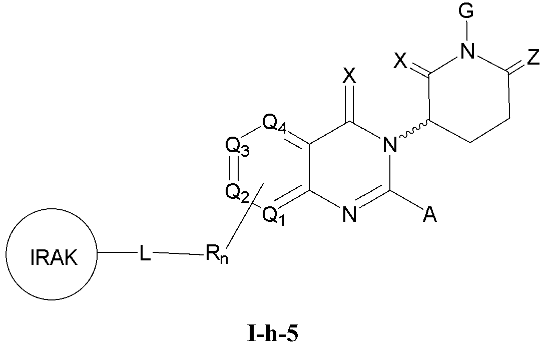 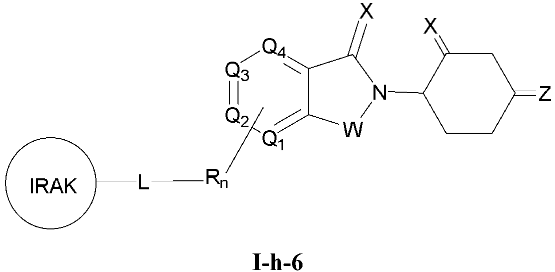 or a pharmaceutically acceptable salt thereof, wherein L and IRAK are as defined above and described in embodiments herein, and wherein each of the variables A, G, G', Q₁, Q₂, Q₃, Q₄, R, R', W, X, Z, and n is as defined and described in WO 2016/197114 and US 2018/0147202, the entirety of each of which is herein incorporated by reference.

In certain embodiments, the present invention provides a compound of formula **I,** wherein LBM is a MDM2 (i.e. human double minute 2 or HDM2) E3 ligase binding moiety 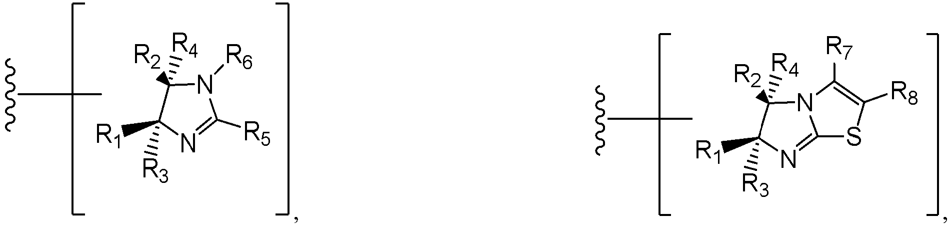 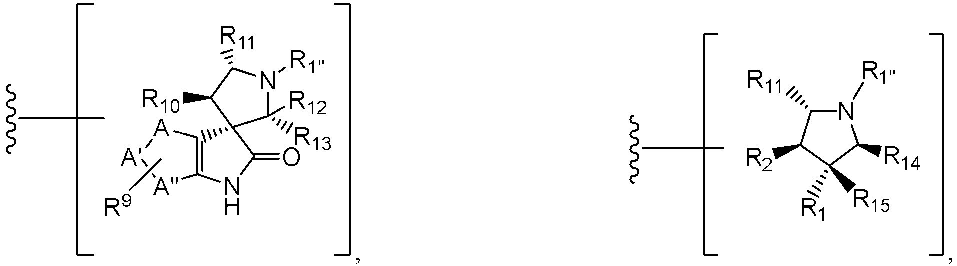 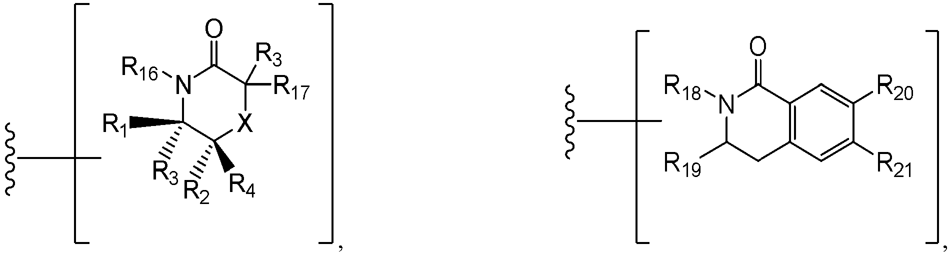 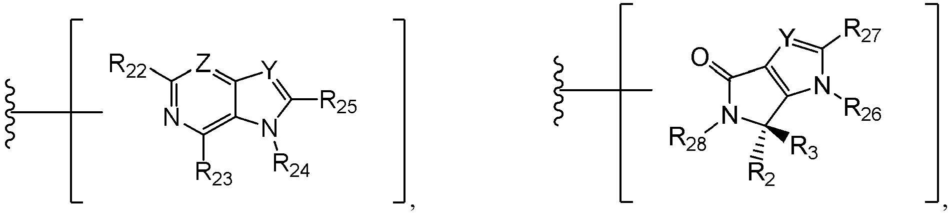 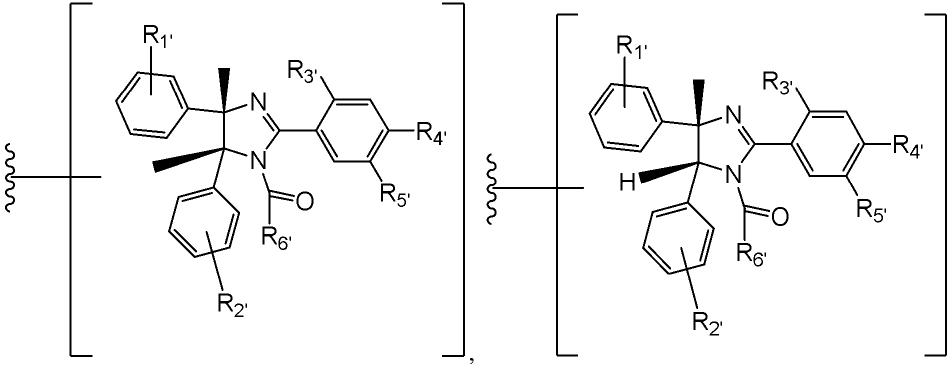 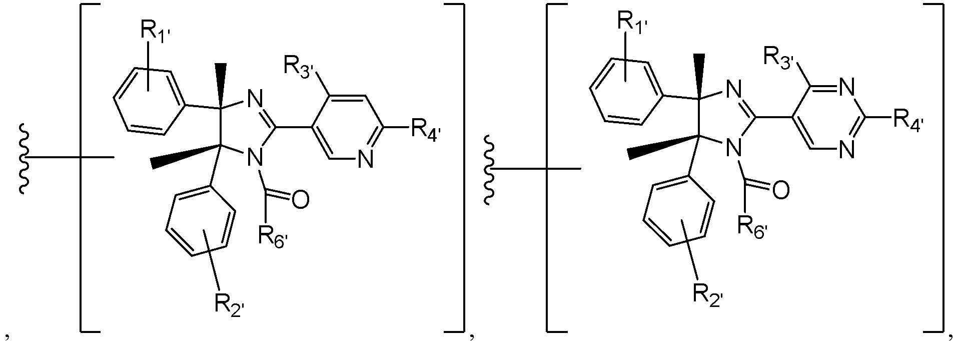 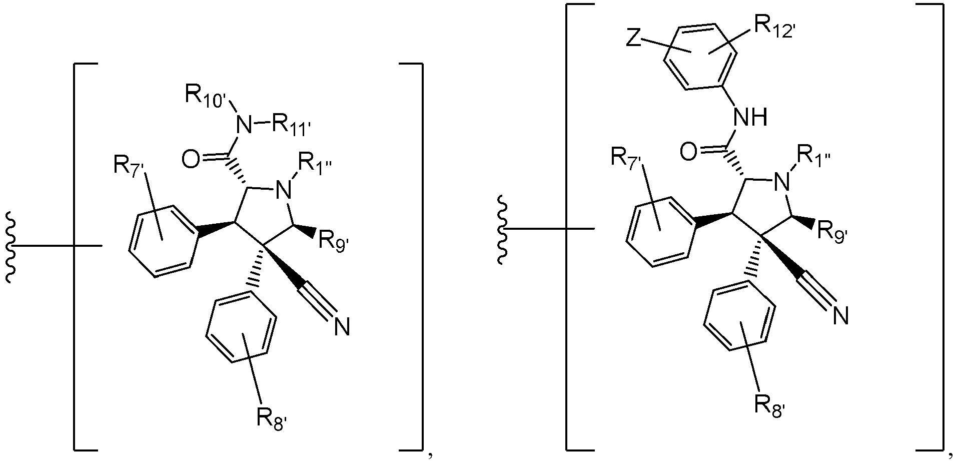 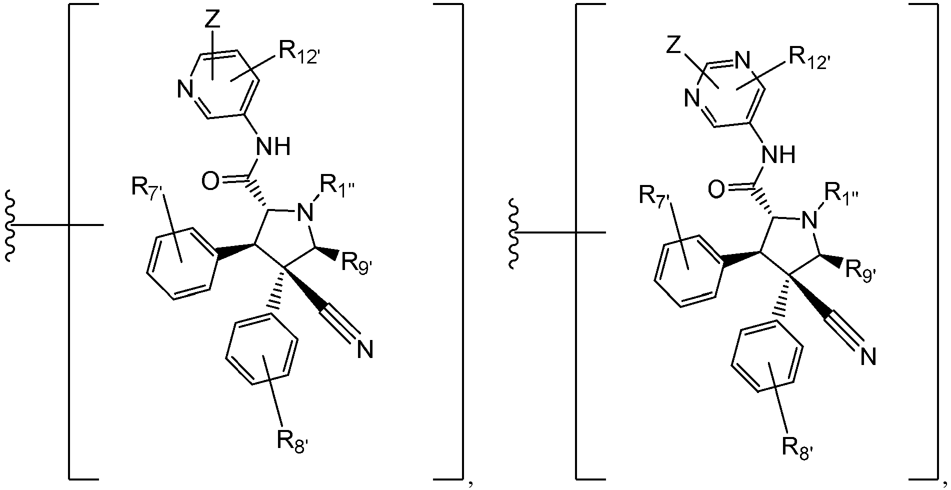 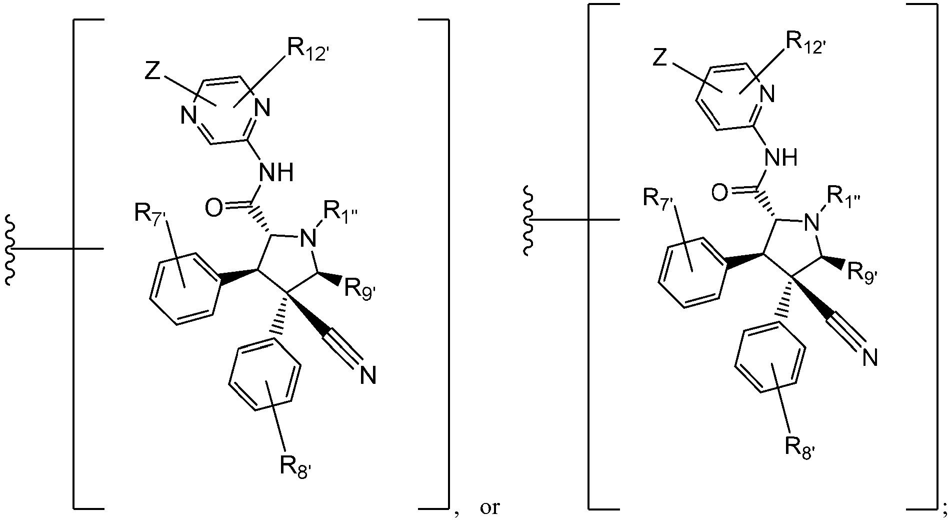 thereby forming a compound of formula **I-i-1, I-i-2, I-i-3, I-i-4, I-i-5, I-i-6, I-i-7, I-i-8, I-i-9, I-i-10, I-i-11, I-i-12, I-i-13, I-i-14, I-i-15, I-i-16, I-i-17,** or **I-i-18** respectively: 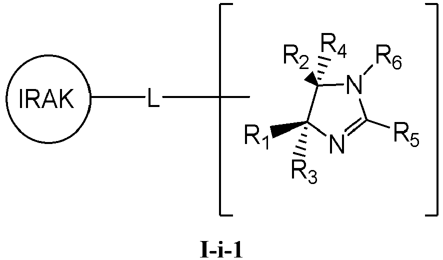 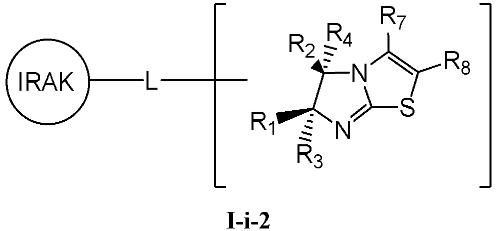 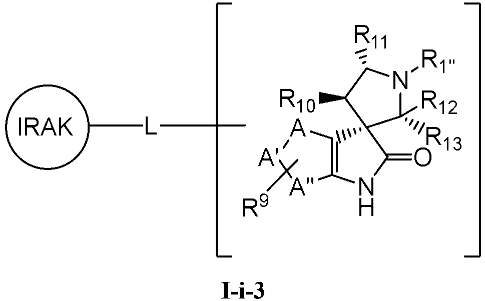 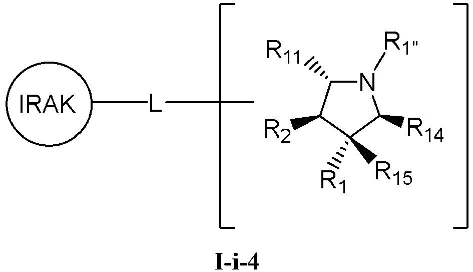 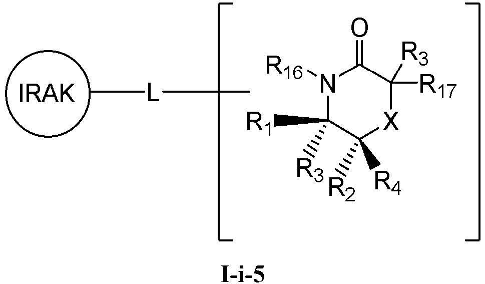 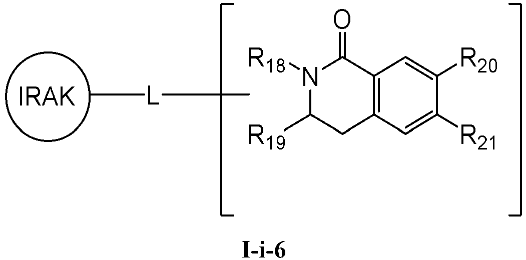 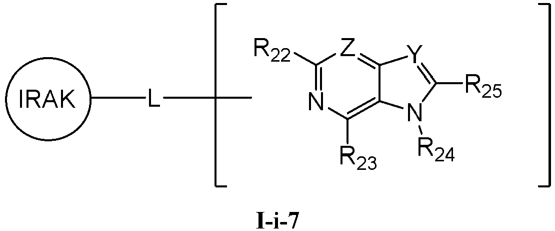 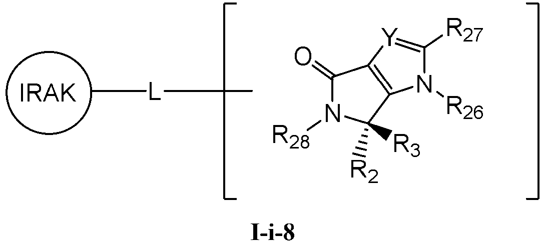 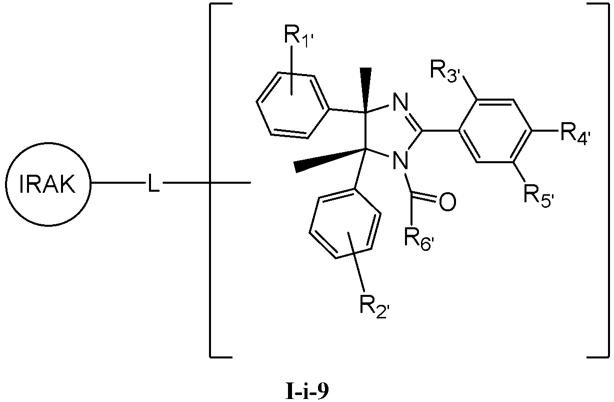 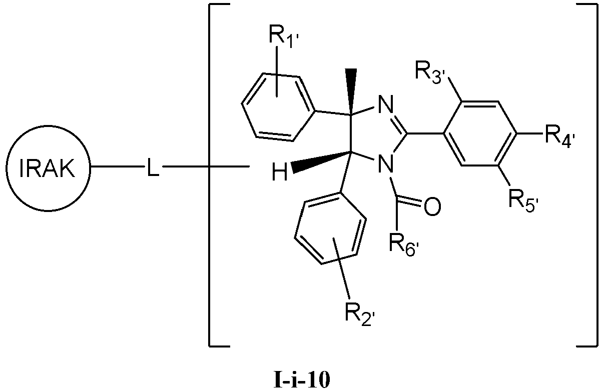 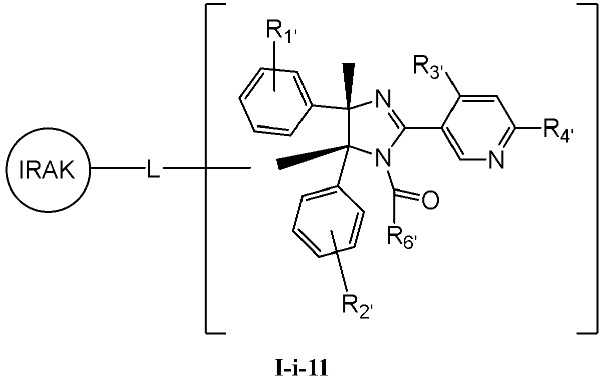 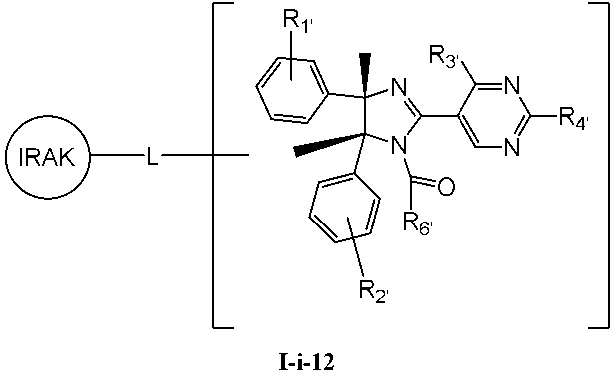 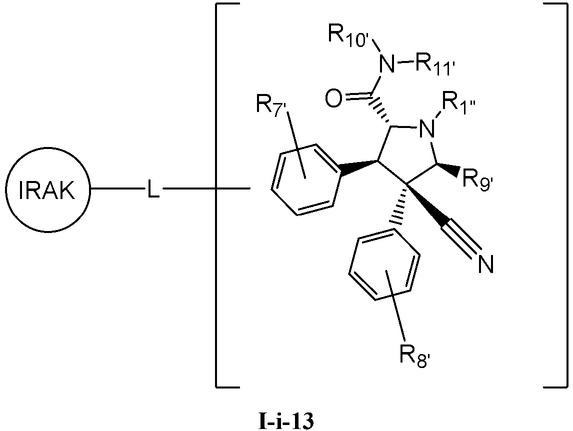 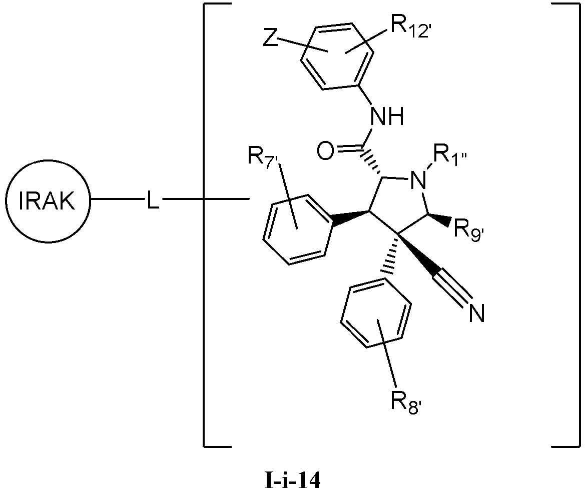 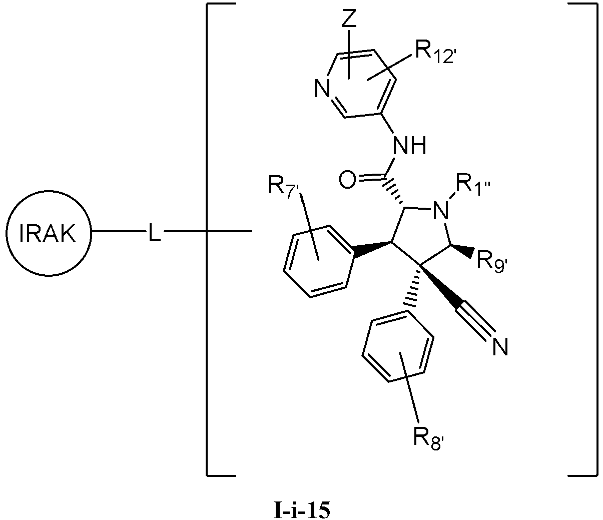 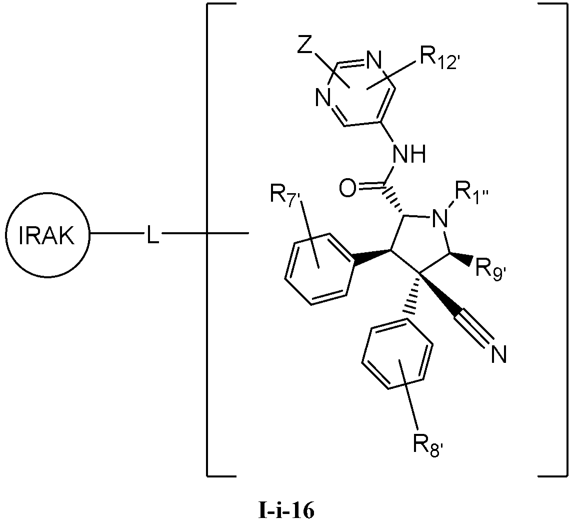 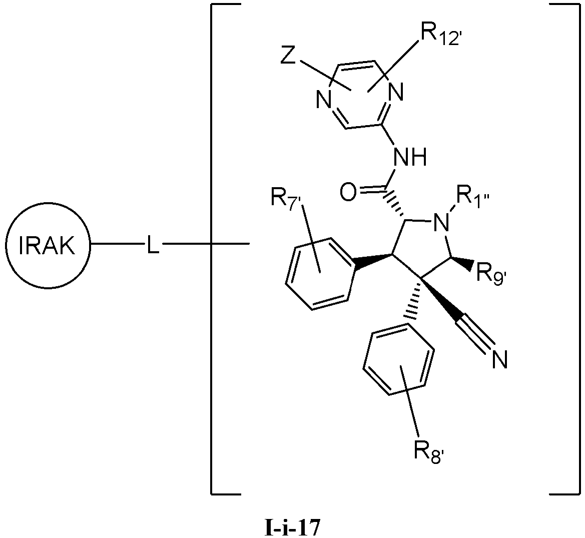 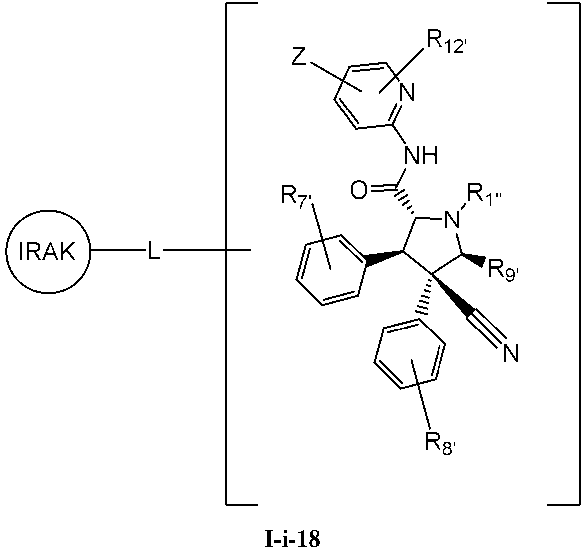 or a pharmaceutically acceptable salt thereof, wherein L and IRAK are as defined above and described in embodiments herein, and wherein each of the variables R₁, R₂, R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, R₂₄, R₂₅, R₂₆, R₂₇, R₂₈, R_{1'}, R_{2'}, R_{3'}, R_{4'}, R_{5'}, R_{6'}, R_{7'}, R_{8'}, R_{9'}, R_{10'}, R_{11'}, R_{12'}, R_{1"}, A, A', A", X, Y, and Z is as defined and described in WO 2017/011371 and US 2017/0008904, the entirety of each of which is herein incorporated by reference.

In certain embodiments, LBM is a MDM2 E3 ligase binding moiety 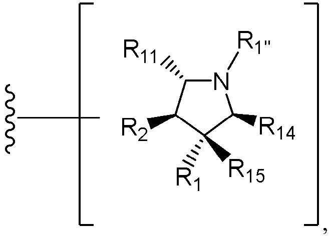 or a pharmaceutically acceptable salt thereof, wherein:
R₁ and R₂ are independently selected from the group consisting of an aryl or heteroaryl group, a heteroaryl group having one or two heteroatoms independently selected from sulfur or nitrogen, wherein the aryl or heteroaryl group can be mono-cyclic or bi-cyclic, or unsubstituted or substituted with one to three substituents independently selected from the group consisting of: halogen, -CN, C₁₋₆ alkyl group, C₃₋₆ cycloalkyl, -OH, alkoxy with 1 to 6 carbons, fluorine substituted alkoxy with 1-6 carbons, sulfoxide with 1-6 carbons, sulfone with 1-6 carbons, ketone with 2-6 carbons, amides with 2-6 carbons, and dialkyl amine with 2-6 carbons;
R₁₁ is -C(O)-N(R^{h})(Rⁱ), wherein R^{h} and Rⁱ are selected from groups consisting of the following: H, C1 to C6 alkyl, alkoxy substituted alkyl, sulfone substituted alkyl, aryl, heterol aryl, mono-, bis- or tri-substituted aryl or hetero aryl, alkyl carboxylic acid, heteroaryl carboxylic acid, alkyl carboxylic acid, fluorine substituted alkyl carboxylic acid, aryl substituted cycloalkyl, hetero aryl substituted cycloalkyl; wherein R^{h} and Rⁱ are independently selected from the group consisting of H, connected to form a ring, 4-hydroxycyclohehexane; mono- and di-hydroxy substituted alkyl (C₃₋₆); 3-hydroxycyclobutane; phenyl-4-carboxylic acid, and substituted phenyl-4-carboxylic acid;
R₁₄ is selected from the group consisting of alkyl, substituted alkyl, alkenyl, substituted alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₁₅ is CN;
R_{1"} is selected from the group consisting of alkyl, aryl substituted alkyl, alkoxy substituted alkyl, cycloalkyl, aryl-substituted cycloalkyl, and alkoxy substituted cycloalkyl,
as defined and described in WO 2017/011371 and US 2017/0008904, the entirety of each of which is herein incorporated by reference.

In certain embodiments, the present invention provides a compound of formula **I,** wherein LBM is a CRBN or VHL E3 ubiquitin ligase binding moiety selected from the group consisting of 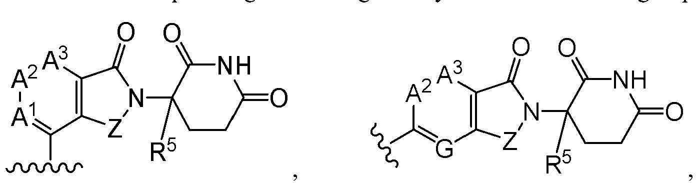 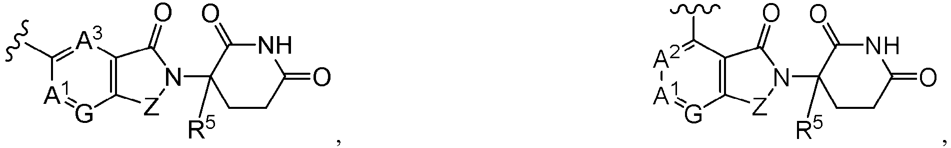 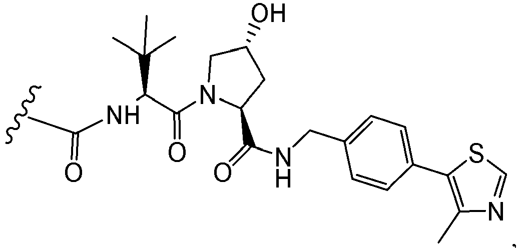 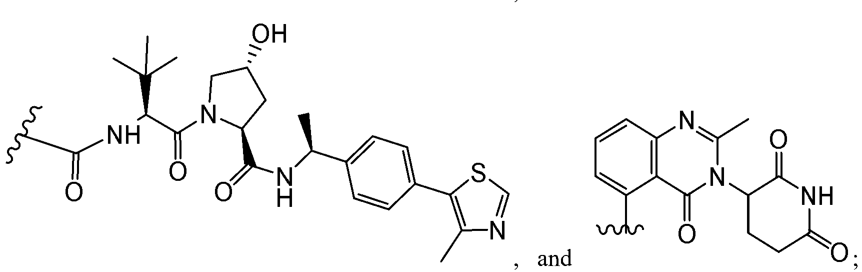 thereby forming a compound of formula **I-j-1, I-j-2, I-j-3, I-j-4, I-j-5, I-j-6,** or **I-j-7** respectively: 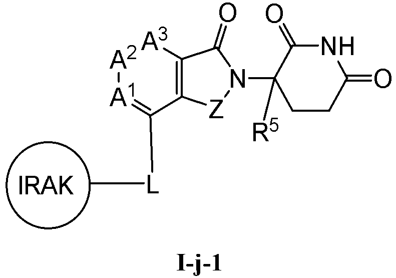 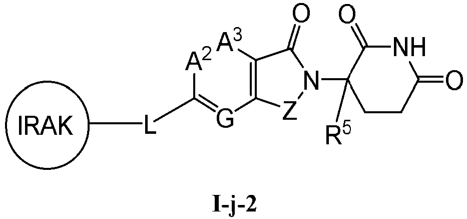 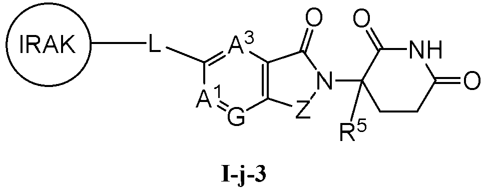 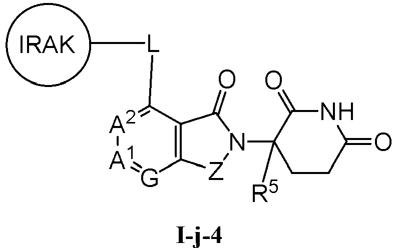 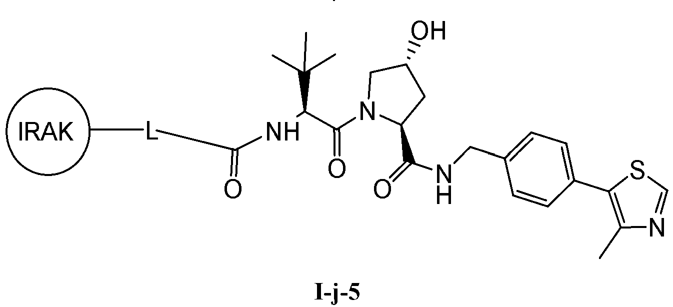 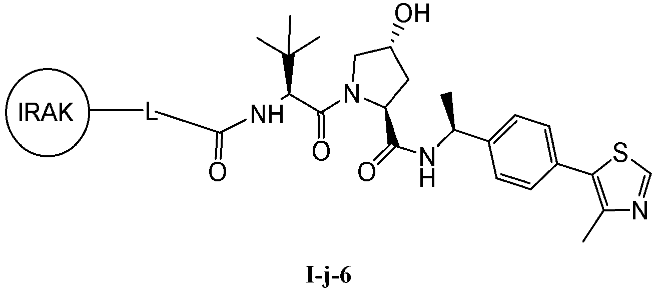 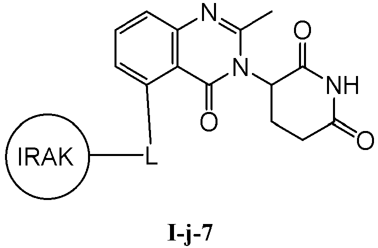 or a pharmaceutically acceptable salt thereof, wherein L and IRAK are as defined above and described in embodiments herein, and wherein each of the variables A¹, A², A³, R⁵, G and Z is as defined and described in WO 2017/176958 the entirety of each of which is herein incorporated by reference.

In certain embodiments, the present invention provides a compound of formula **I,** wherein LBM is a CRBN or VHL E3 ubiquitin ligase binding moiety selected from the group consisting of 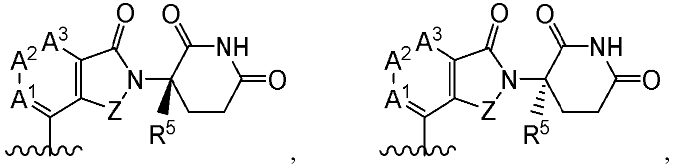 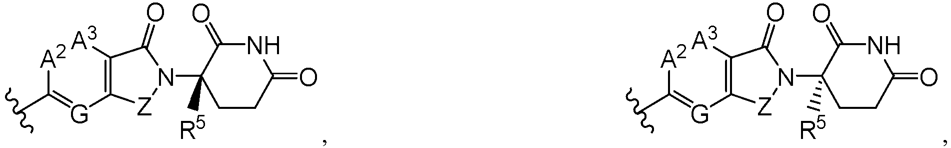 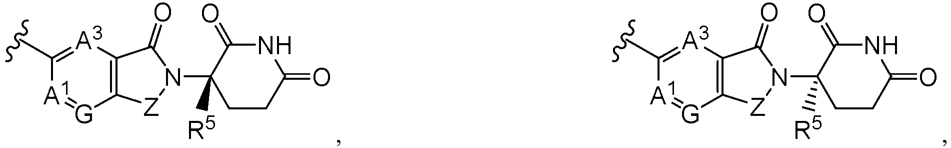 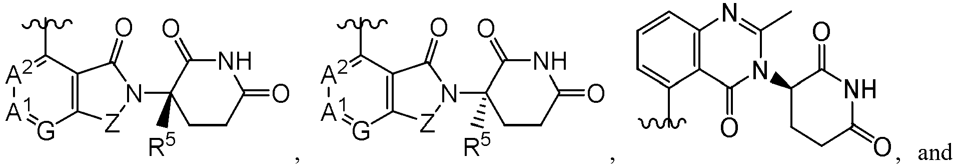 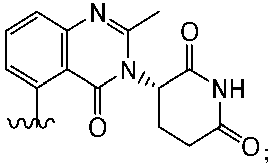 thereby forming a compound of formula **I-j'-1, I-j"-1, I-j'-2, I-j"-2, I-j'-3, I-j"-3, I-j'-4, I-j"-4, I-j'-7** or **I-j"-7** respectively: 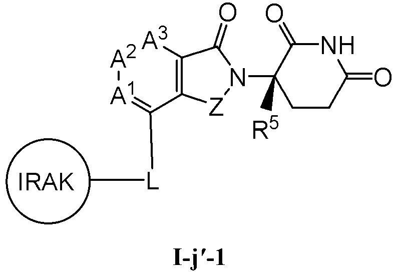 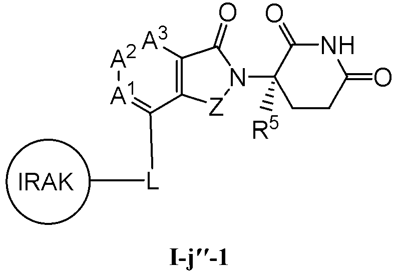 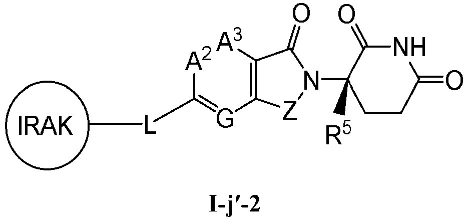 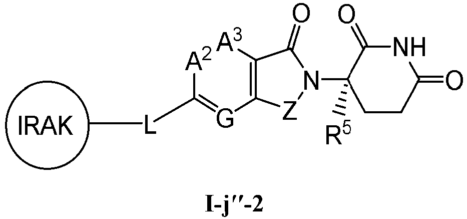 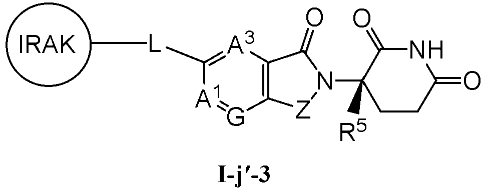 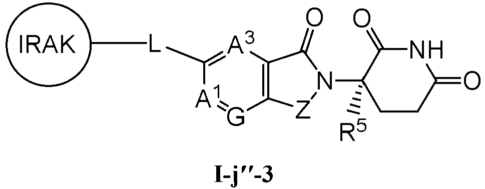 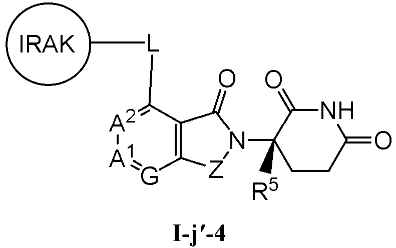 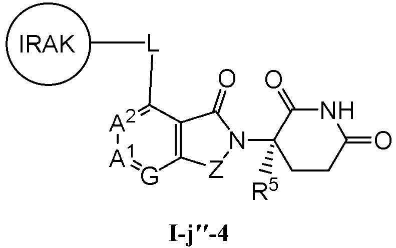 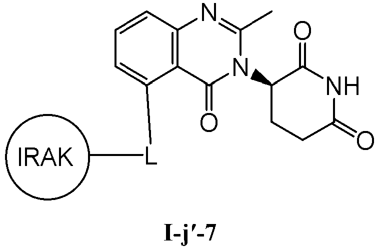 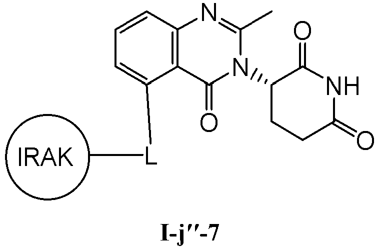 or a pharmaceutically acceptable salt thereof, wherein L and IRAK are as defined above and described in embodiments herein, and wherein each of the variables A¹, A², A³, R⁵, G and Z is as defined and described in WO 2017/176958, the entirety of each of which is herein incorporated by reference.

In certain embodiments, the present invention provides a compound of formula **I,** wherein LBM is an IAP E3 ubiquitin ligase binding moiety 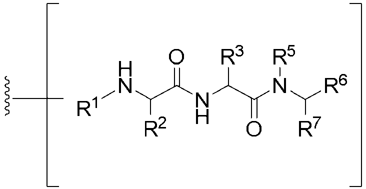 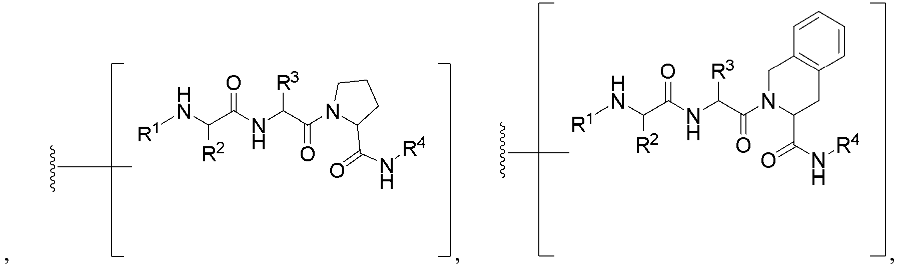 or 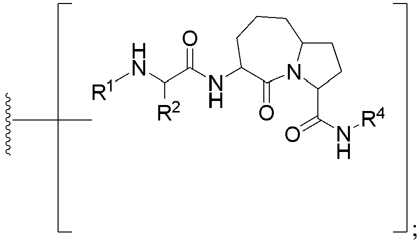 thereby forming a compound of formula **I-k-1, I-k-2, I-k-3,** or **I-k-4** respectively: 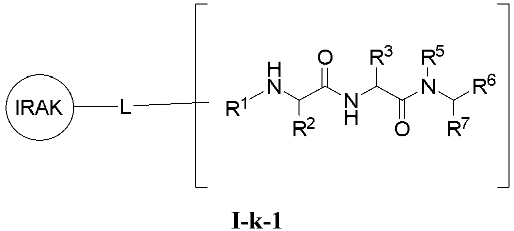 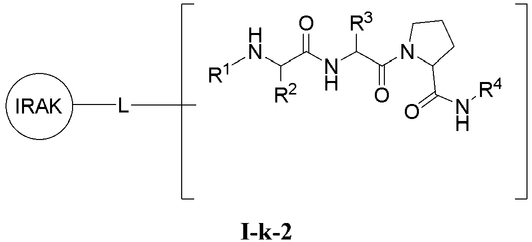 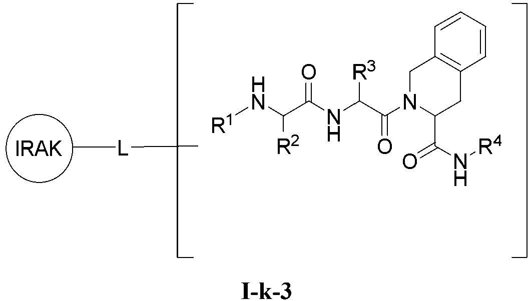 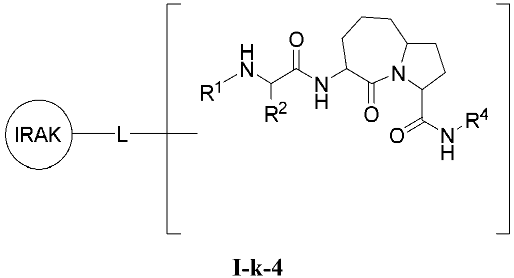 or a pharmaceutically acceptable salt thereof, wherein L and IRAK are as defined above and described in embodiments herein, and wherein:
R¹ is selected from hydrogen or alkyl;
R² is selected from hydrogen or alkyl;
R³ is selected from hydrogen, alkyl, cycloalkyl and heterocycloalkyl;
R⁵ and R⁶ are independently selected from hydrogen, alkyl, cycloalkyl, heterocycloalkyl, or
   R⁵ and R⁶ are taken together to form a pyrrolidine or a piperidine ring further optionally fused to 1-2 cycloalkyl, heterocycloalkyl, aryl or heteroaryl rings, each of which can then be further fused to another cycloalkyl, heterocycloalkyl, aryl or heteroaryl ring, or
   R³ and R⁵ are taken together to form a 5-8-membered ring further optionally fused to 1-2 cycloalkyl, heterocycloalkyl, aryl or heteroaryl ring;
R⁷ is selected from cycloalkyl, cycloalkylalkyl, heterocycloalkyl, heterocycloalkylalkyl, aryl, arylalkyl, heteroaryl or heteroarylalkyl, each one further optionally substituted with 1-3 substituents selected from halogen, alkyl, haloalkyl, hydroxyl, alkoxy, cyano, (hetero)cycloalkyl or (hetero)aryl, or R⁷ is C(O)NHR⁴; and
R⁴ is selected from alkyl, cycloalkyl, heterocycloalkyl, cycloalkylalkyl, heterocycloalkylalkyl, aryl, arylalkyl, heteroaryl, heteroarylalkyl, further optionally substituted with 1-3 substituents selected from halogen, alkyl, haloalkyl, hydroxyl, alkoxy, cyano, (hetero)cycloalkyl or (hetero)aryl, or R⁷ is C(O)NHR⁴,
as defined and described in WO 2017/011590 and US 2017/0037004, the entirety of each of which is herein incorporated by reference.

In certain embodiments, the present invention provides a compound of formula **I,** wherein LBM is an IAP E3 ubiquitin ligase binding moiety 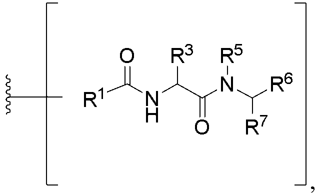 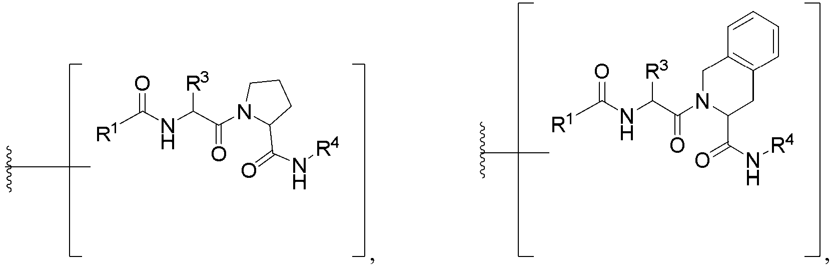 or 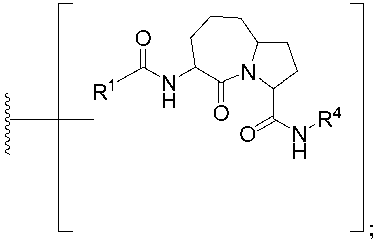 thereby forming a compound of formula **I-k'-1, I-k'-2, I-k'-3**, or **l-k'-4** respectively: 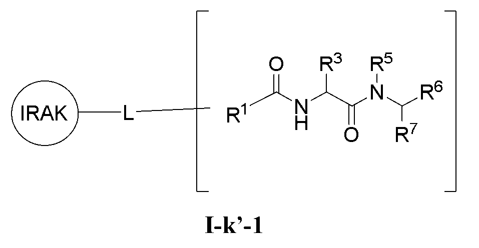 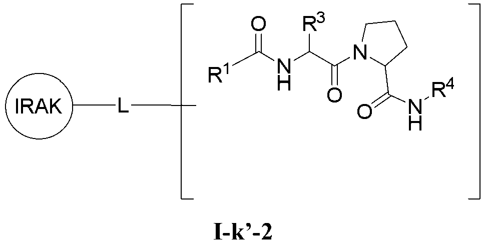 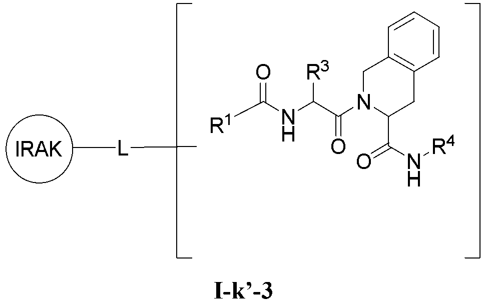 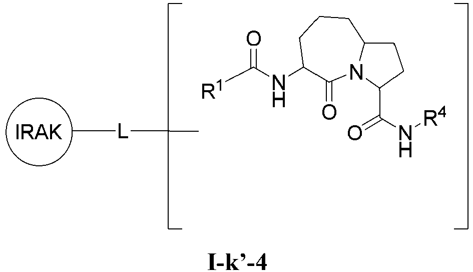 or a pharmaceutically acceptable salt thereof, wherein L and IRAK are as defined above and described in embodiments herein, and wherein each of the variables R¹, R³, R⁴, R⁵, R⁶, and R⁷, is as defined and described in WO 2017/011590 and US 2017/0037004, the entirety of each of which is herein incorporated by reference.

In certain embodiments, the present invention provides a compound of formula **I,** wherein LBM is an E3 ubiquitin ligase (cereblon) binding moiety 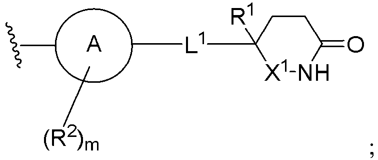 thereby forming a compound of formula **I-l**: 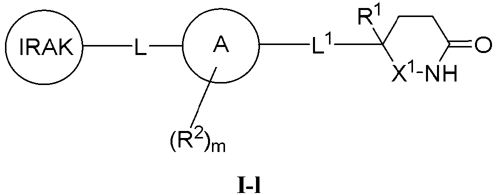 or a pharmaceutically acceptable salt thereof, wherein L and IRAK are as defined above and described herein, and wherein:
X¹ is a bivalent moiety selected from a covalent bond, -CH₂-, -C(O)-, -C(S)-, or 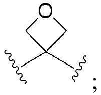
R¹ is hydrogen, deuterium, halogen, -CN, -OR, -SR, -S(O)R, -S(O)₂R, -NR₂, or an optionally substituted C₁₋₄ aliphatic;
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 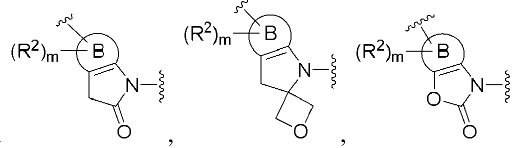 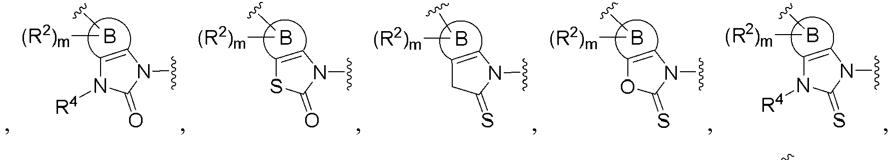 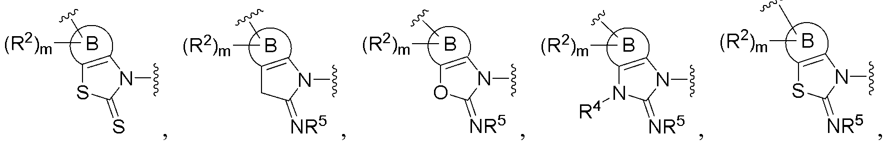 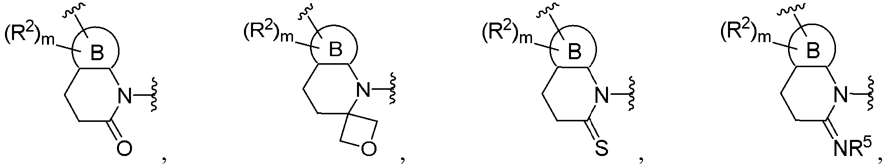 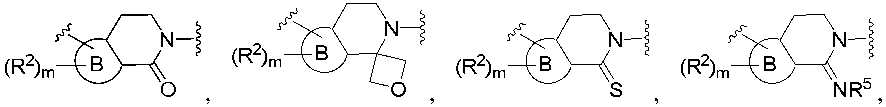 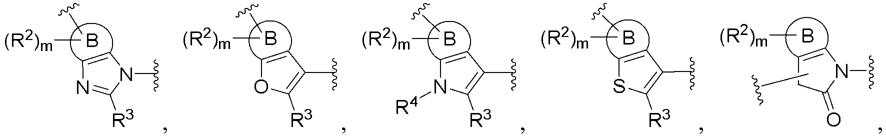 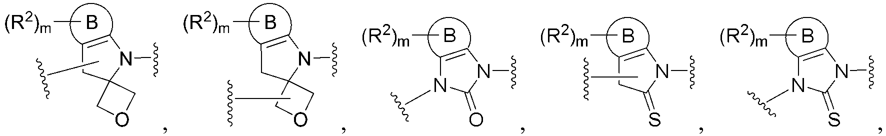 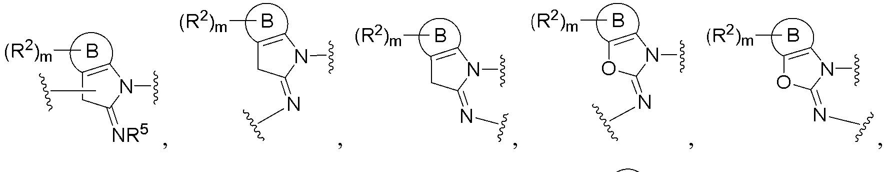 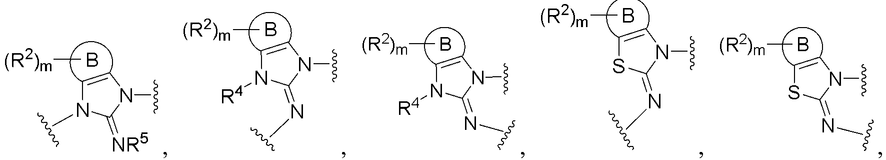 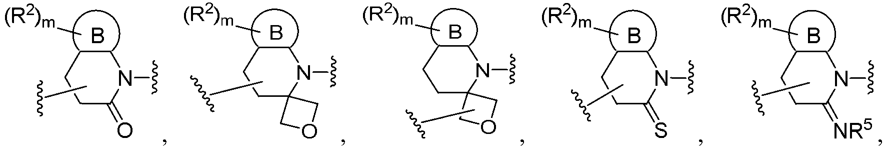 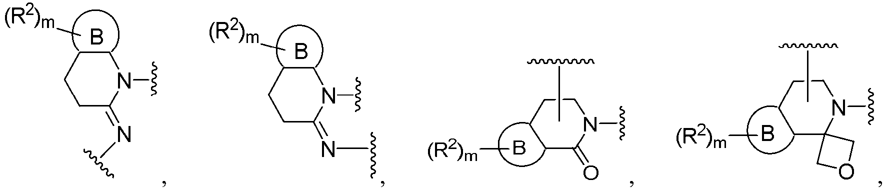 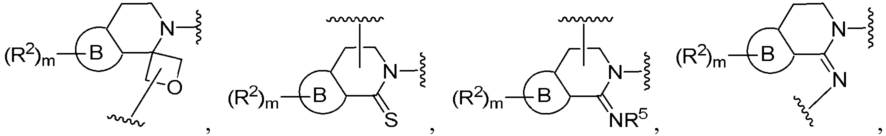 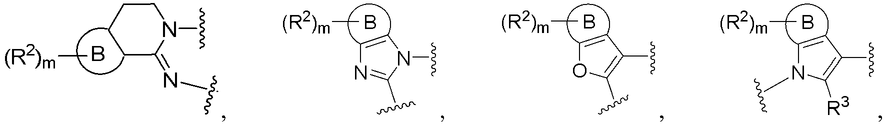 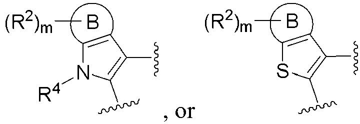 wherein
Ring B is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 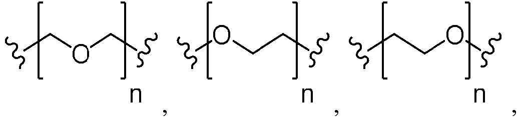 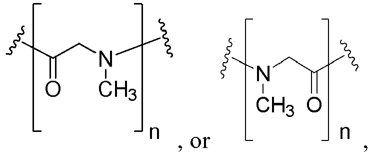 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, 2, 3 or 4;
   each of n is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

Where a point of attachment of -(R²)ₙ is depicted on Ring B, it is intended, and one of ordinary skill in the art would appreciate, that the point of attachment of -(R²)ₙ may be on Ring A and may also be at any available carbon or nitrogen atom on Ring A including the ring to which Ring B is fused. Where -R² is attached to a nitrogen atom bound to R⁴ or R⁵, R⁴ or R⁵ is absent and -R² takes the place of the R⁴ or R⁵ group. Where -R² is attached to a carbon atom bound to R³, R³ is absent and -R² takes the place of the R³ group.

In certain embodiments, the present invention provides a compound of formula **I,** wherein LBM is an E3 ubiquitin ligase (cereblon) binding moiety 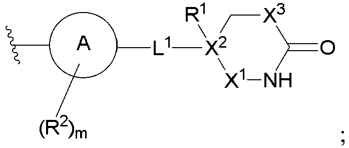 thereby forming a compound of formula **I-l':** 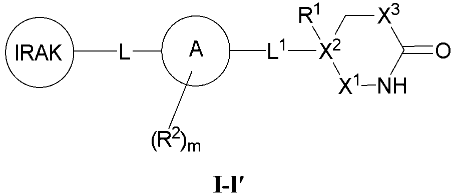 or a pharmaceutically acceptable salt thereof, wherein L and IRAK are as defined above and described herein, and wherein:
X¹ is a bivalent moiety selected from a covalent bond, -CH₂-, -C(O)-, -C(S)-, or 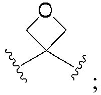
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 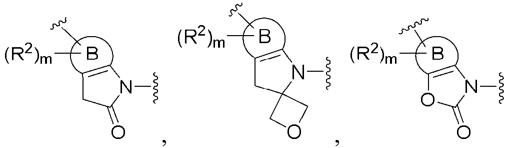 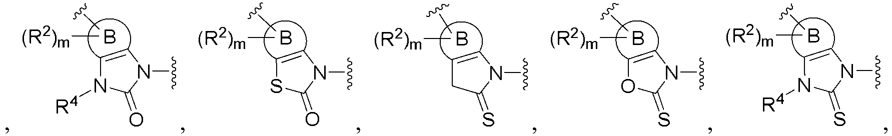 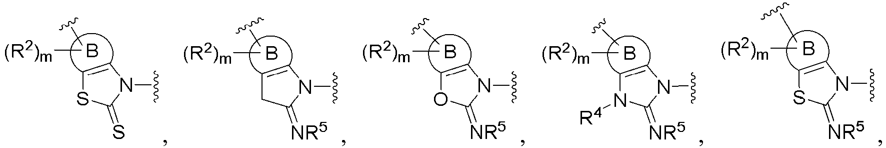 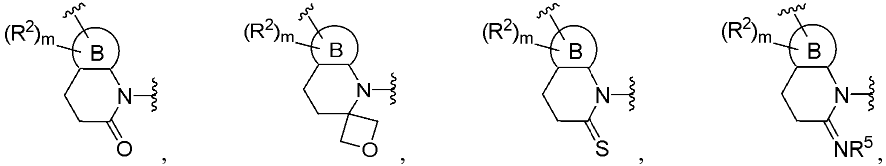 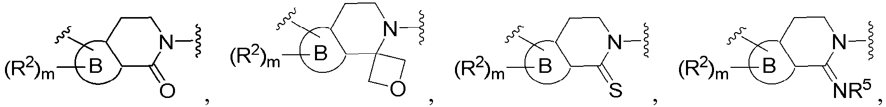 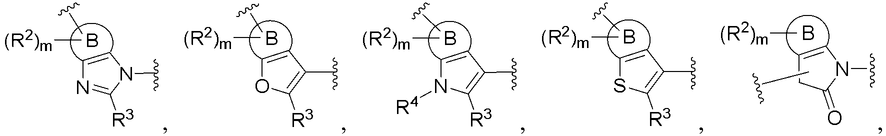 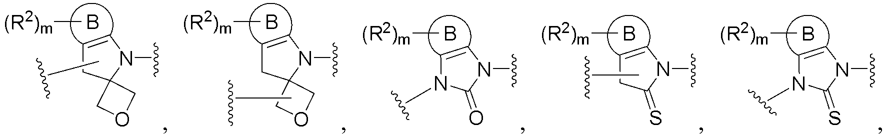 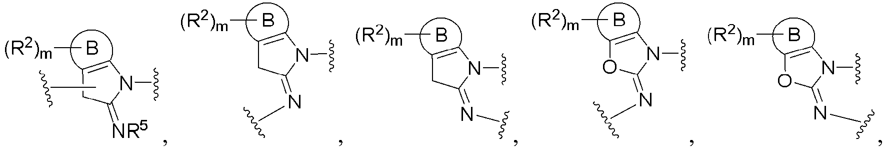 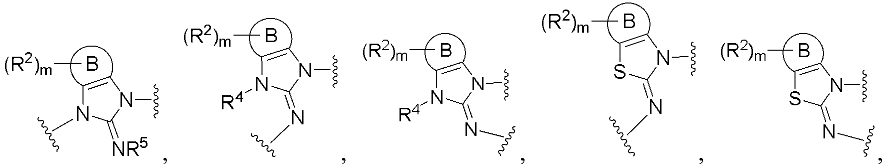 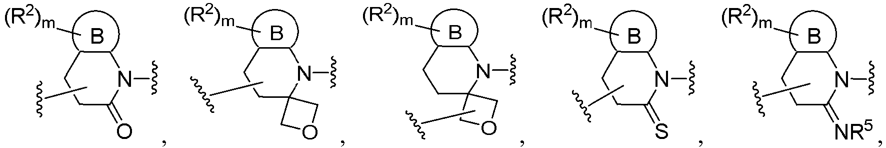 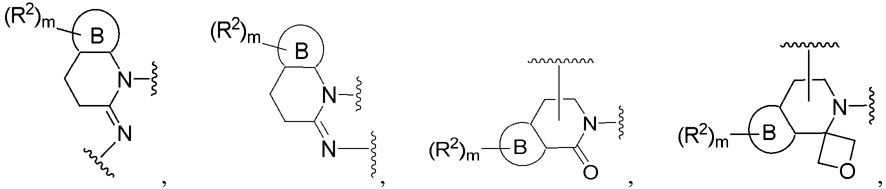 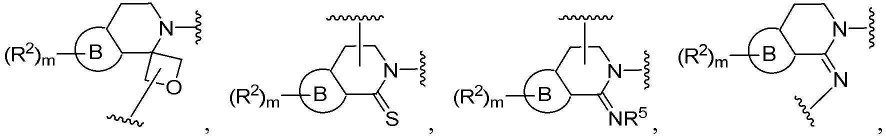 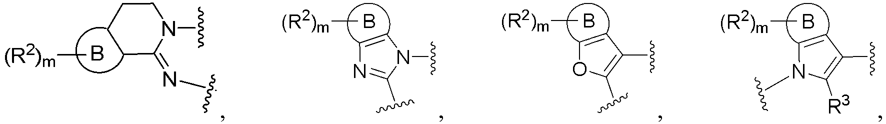 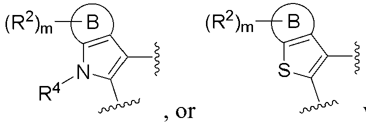 wherein
Ring B is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 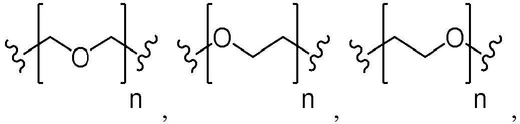 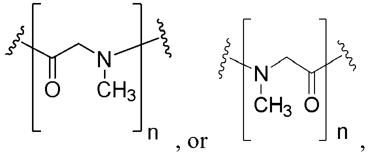 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, 2, 3 or 4;
   each of n is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

Where a point of attachment of -(R²)ₙ is depicted on Ring B, it is intended, and one of ordinary skill in the art would appreciate, that the point of attachment of -(R²)ₙ may be on Ring A and may also be at any available carbon or nitrogen atom on Ring A including the ring to which Ring B is fused. Where -R² is attached to a nitrogen atom bound to R⁴ or R⁵, R⁴ or R⁵ is absent and -R² takes the place of the R⁴ or R⁵ group. Where -R² is attached to a carbon atom bound to R³, R³ is absent and -R² takes the place of the R³ group.

In some embodiments, a compound of formala **I-l'** above is provided as a compound of formula **I-l"** or formula **I-l‴:** 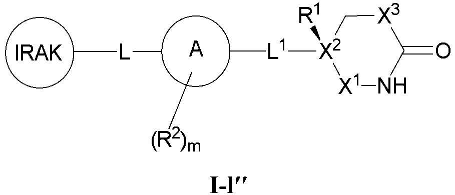 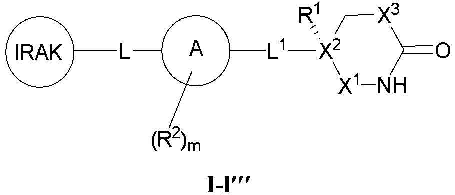 or a pharmaceutically acceptable salt thereof, wherein:
each of IRAK, Ring A, L, L¹, R¹, R², X¹, X², X³, and m is as defined above.

In certain embodiments, the present invention provides a compound of formula **I,** wherein LBM is an E3 ubiquitin ligase (cereblon) binding moiety 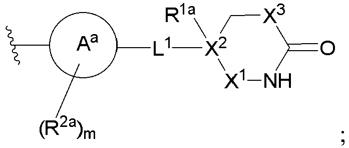 thereby forming a compound of formula **I-l-1:** 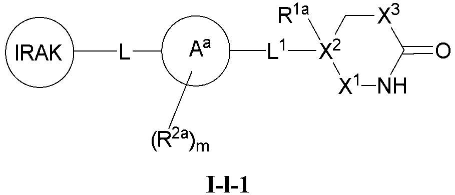 or a pharmaceutically acceptable salt thereof, wherein L and IRAK are as defined above and described herein, and wherein:
X¹ is a bivalent moiety selected from a covalent bond, -CH₂-, -C(O)-, -C(S)-, or 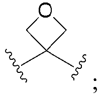
X² is a carbon atom or silicon atom;
X³ is a bivalent moiety selected from -CH₂- or -Si(R₂)-;
R^{1a} is hydrogen, deuterium, halogen, -CN, -OR, -SR, -S(O)R, -S(O)₂R, -N(R)₂, -Si(R)₃, or an optionally substituted C₁₋₄ aliphatic;
each R^{2a} is independently hydrogen, -R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A^{a} is a bi- or tricyclic ring selected from 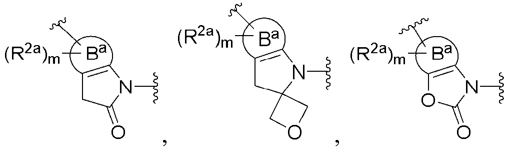 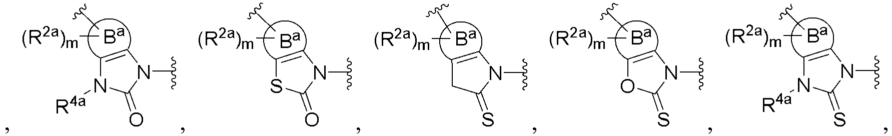 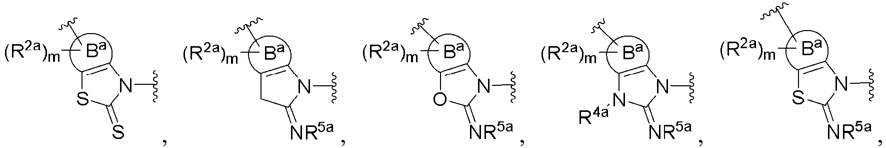 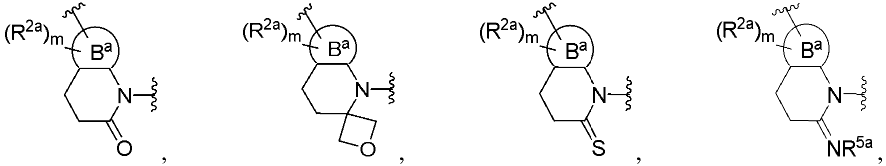 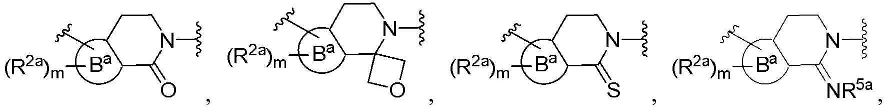 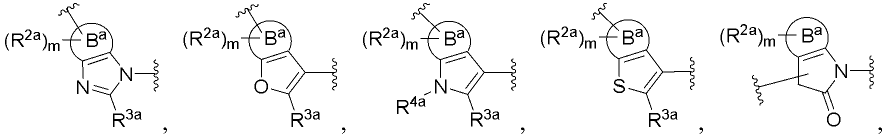 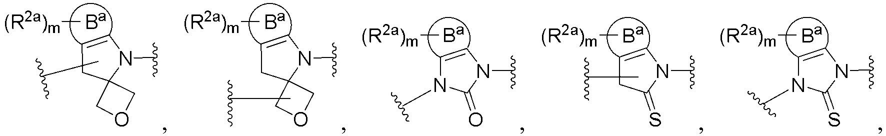 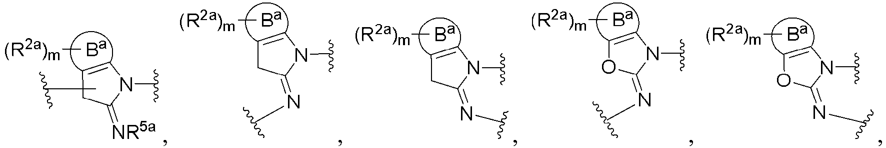 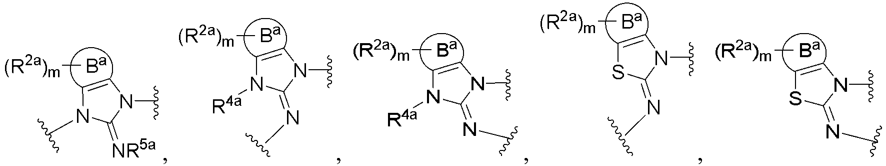 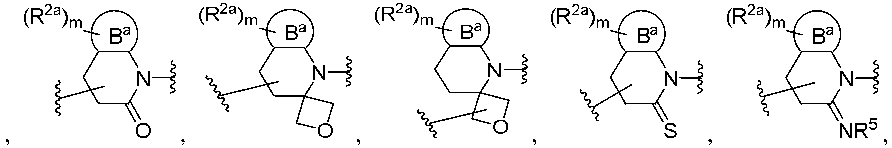 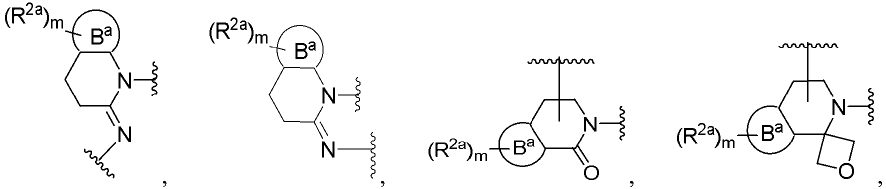 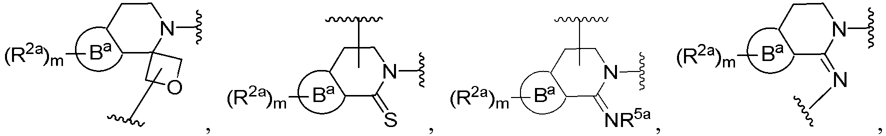 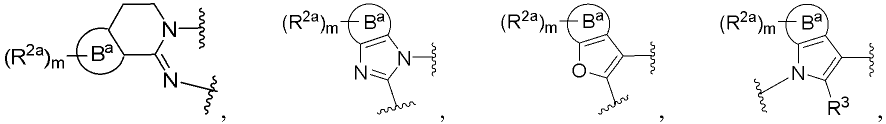 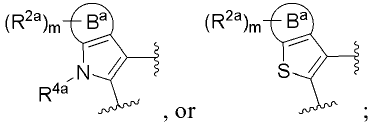
Ring B^{a} is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R^{3a} is selected from hydrogen, halogen, -OR, -N(R)₂, or -SR;
each R^{4a} is independently hydrogen, -R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5a} is hydrogen, C₁₋₄ aliphatic, or -CN;
each R^{6a} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 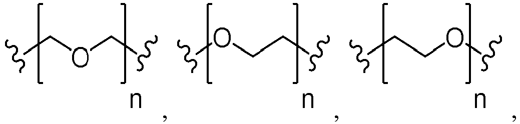 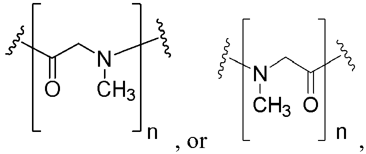 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, 2, 3 or 4;
   each of n is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

Where a point of attachment of -(R^{2a})ₙ is depicted on Ring B, it is intended, and one of ordinary skill in the art would appreciate, that the point of attachment of -(R^{2a})ₙ may be on Ring A and may also be at any available carbon or nitrogen atom on Ring A including the ring to which Ring B is fused. Where -R^{2a} is attached to a nitrogen atom bound to R^{4a} or R^{5a}, R^{4a} or R^{5a} is absent and -R^{2a} takes the place of the R^{4a} or R^{5a} group. Where -R^{2a} is attached to a carbon atom bound to R^{3a}, R^{3a} is absent and -R^{2a} takes the place of the R^{3a} group.

In some embodiments, a compound of formala **I-l-1** above is provided as a compound of formula **I-l-1'** or formula **I-l**-**1":** 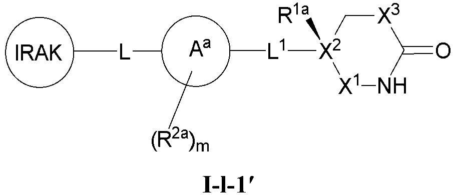 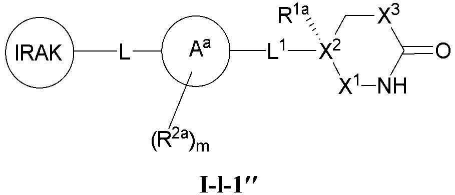 or a pharmaceutically acceptable salt thereof, wherein: each of IRAK, Ring A^{a}, L, L¹, R^{1a}, R^{2a}, X¹, X², X³, and m is as defined above.

In certain embodiments, the present invention provides a compound of formula **I,** wherein LBM is an E3 ubiquitin ligase (cereblon) binding moiety 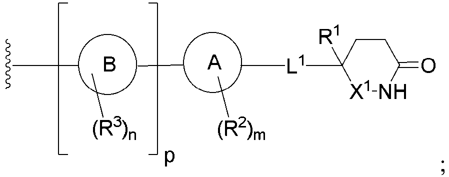 thereby forming a compound of formula I-m: 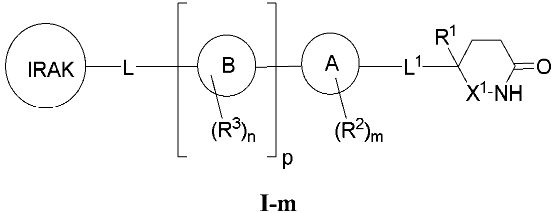 or a pharmaceutically acceptable salt thereof, wherein, L and IRAK are as defined above and described in embodiments herein, and wherein:
X¹ is a bivalent moiety selected from a covalent bond, -CH₂-, -C(O)-, -C(S)-, or 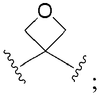
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 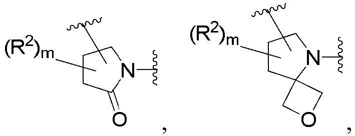 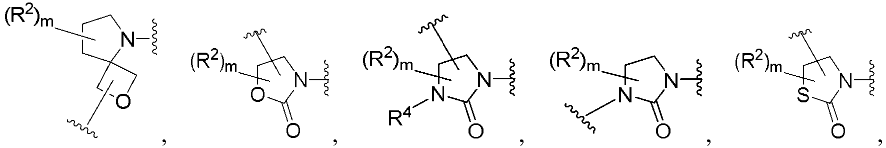 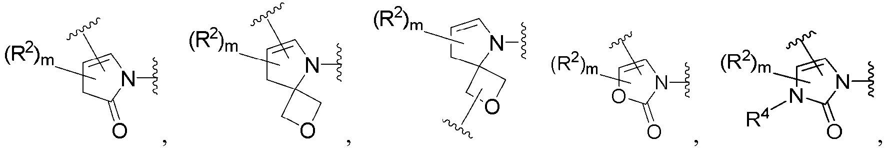 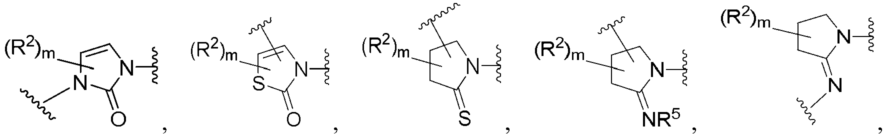 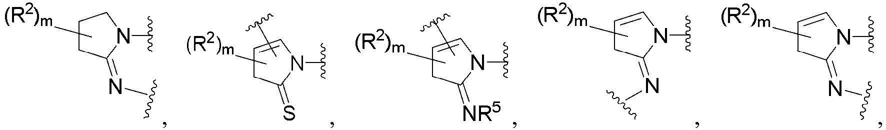 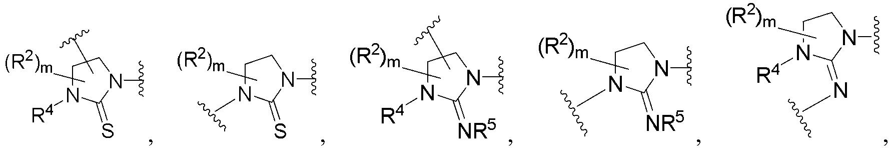 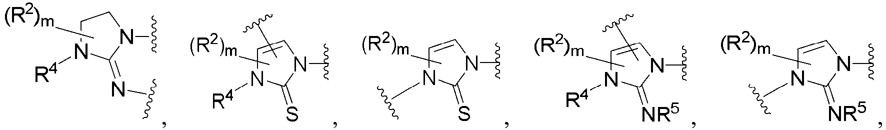 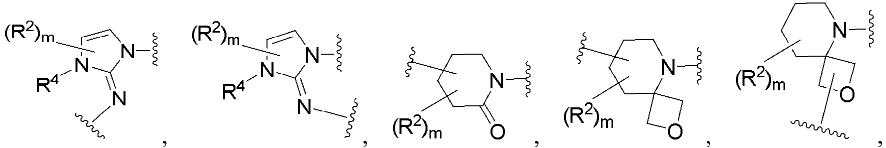 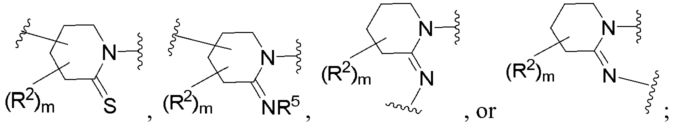
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 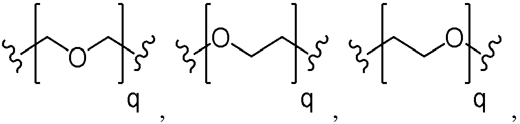 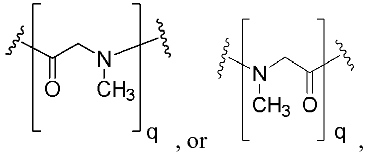 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1, wherein when p is 0, the bond connecting Ring A and Ring B is connected to 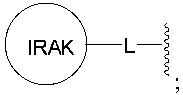
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 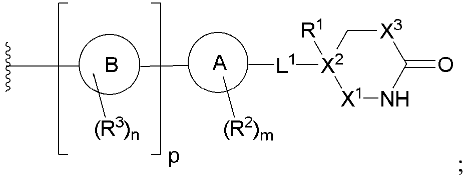 thereby forming a compound of formula I-m': 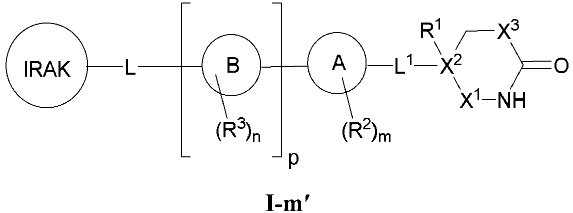 or a pharmaceutically acceptable salt thereof, wherein, L and IRAK are as defined above and described in embodiments herein, and wherein:
X¹ is a bivalent moiety selected from a covalent bond, -CH₂-, -C(O)-, -C(S)-, or 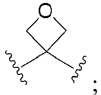
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 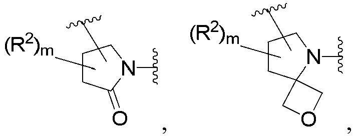 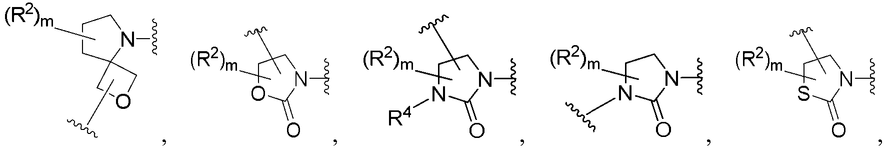 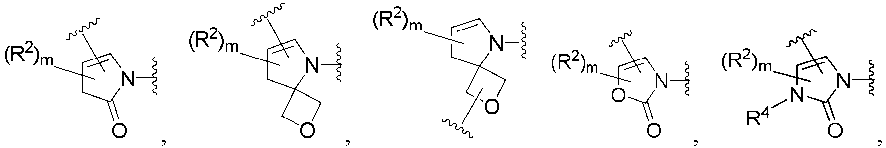 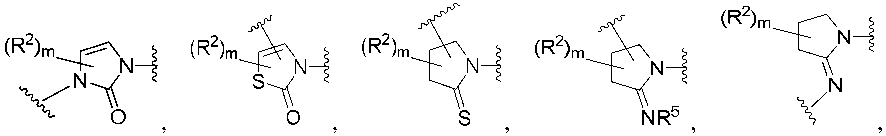 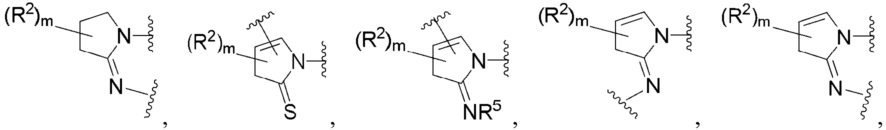 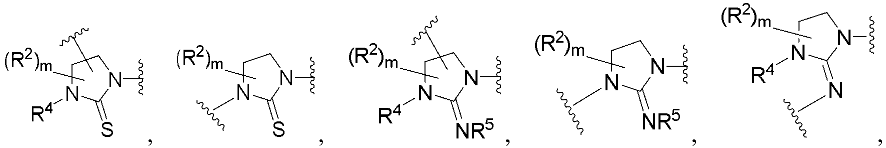 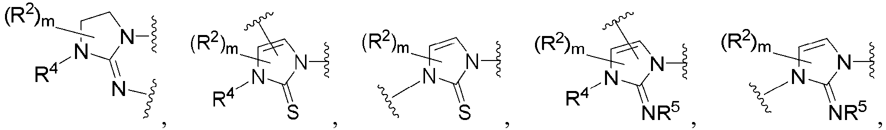 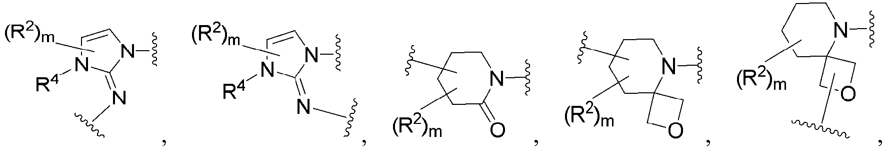 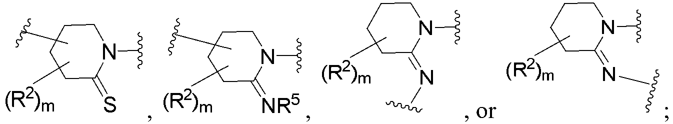
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 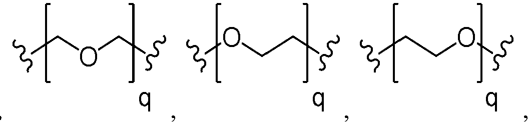 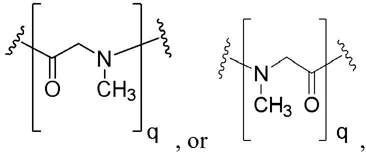 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1, wherein when p is 0, the bond connecting Ring A and Ring B is connected to 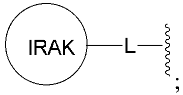
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, a compound of formala I-m' above is provided as a compound of formula I-m" or formula **I-m‴**: 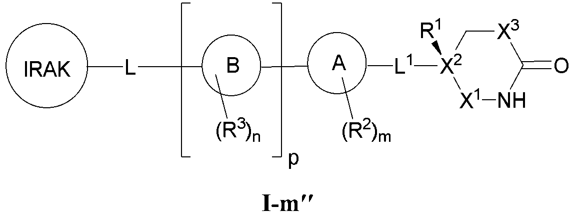 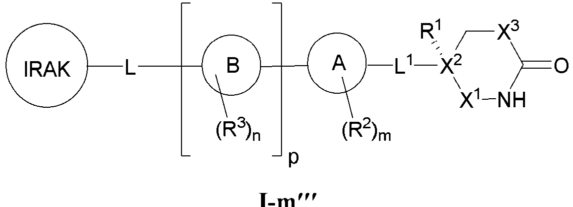 or a pharmaceutically acceptable salt thereof, wherein:
each of IRAK, Ring A, Ring B, L, L¹, R¹, R², R³, X¹, X², X³, n, p, and m is as defined above.

In certain embodiments, the present invention provides a compound of formula **I,** wherein LBM is an E3 ubiquitin ligase (cereblon) binding moiety 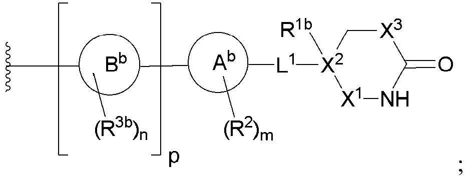 thereby forming a compound of formula I-m-1: 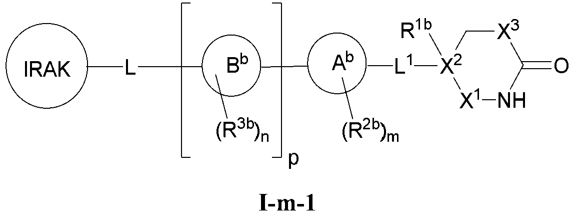 or a pharmaceutically acceptable salt thereof, wherein, L and IRAK are as defined above and described in embodiments herein, and wherein:
X¹ is a bivalent moiety selected from a covalent bond, -CH₂-, -C(O)-, -C(S)-, or 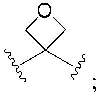
X² is a carbon atom or silicon atom;
X³ is a bivalent moiety selected from -CH₂- or -Si(R₂)-;
R^{1b} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{b} is a mono- or bicyclic ring selected from 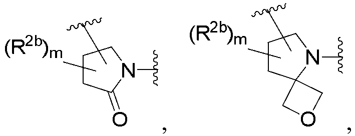 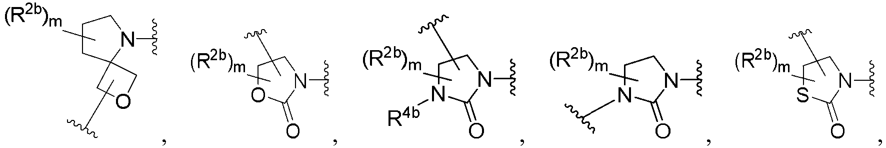 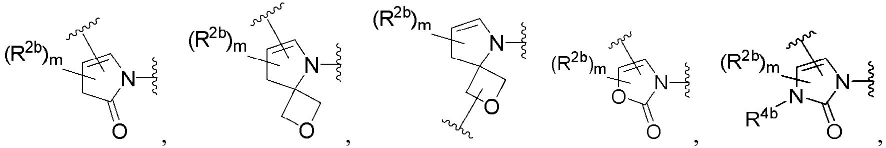 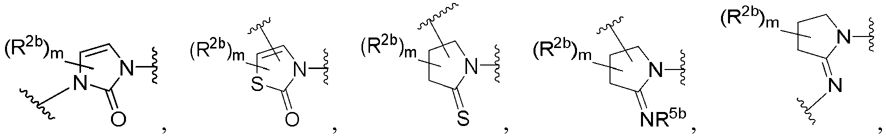 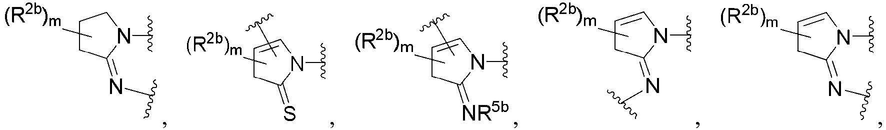 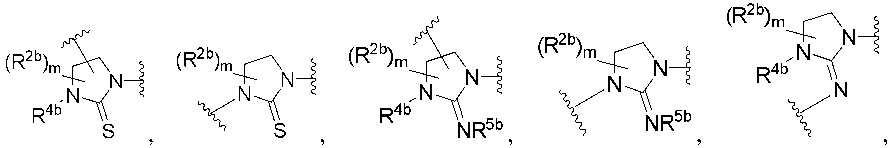 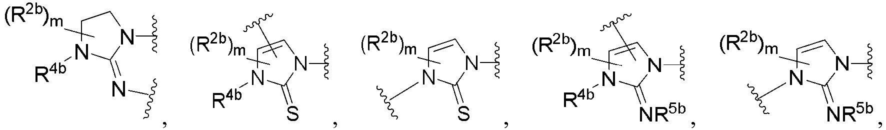 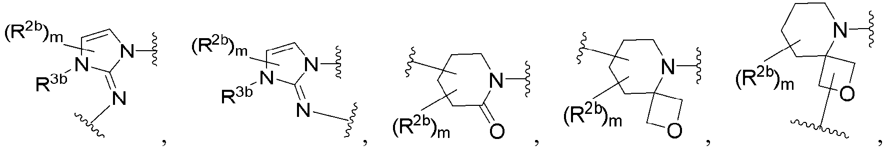 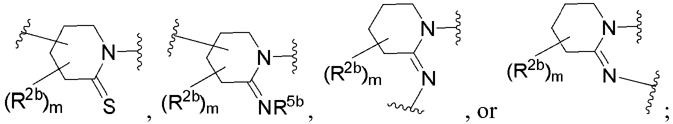
each R^{2b} is independently hydrogen, -R^{6b}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{b} is selected from a 6-membered aryl containing 0-2 nitrogen atoms or a 5-membered heteroaryl with 1-3 heteroatoms independently selected from nitrogen, oxygen or sulfur;
each of R^{3b} and R^{4b} is independently hydrogen, -R^{6b}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5b} is hydrogen, C₁₋₄ aliphatic, or -CN;
each R^{6b} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 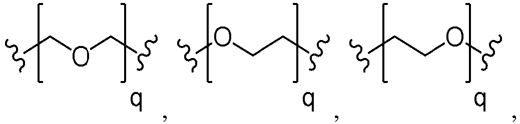 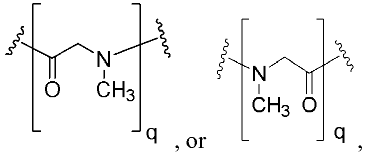 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1, wherein when p is 0, the bond connecting Ring A and Ring B is connected to 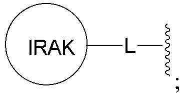
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, a compound of formala **I-m'** above is provided as a compound of formula **I-m"** or formula **I-m‴**: 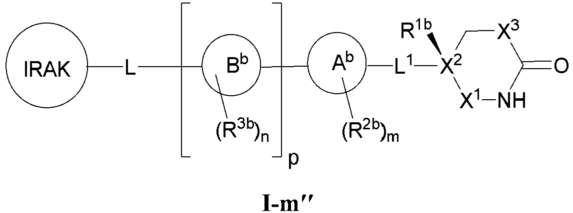 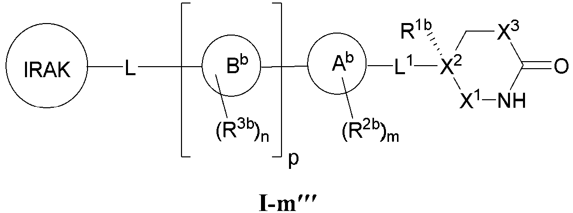 or a pharmaceutically acceptable salt thereof, wherein:
each of IRAK, Ring A^{b}, Ring B^{b}, L, L¹, R^{1b}, R^{2b}, R^{3b}, X¹, X², X³, n, p, and m is as defined above.

In certain embodiments, the present invention provides a compound of formula **I,** wherein LBM is an E3 ubiquitin ligase (cereblon) binding moiety 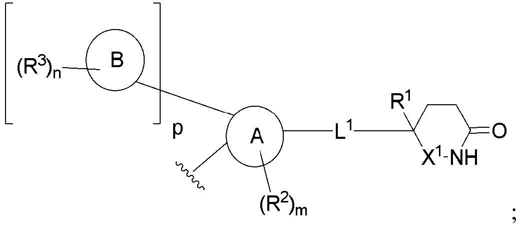 thereby forming a compound of formula **I-n**: 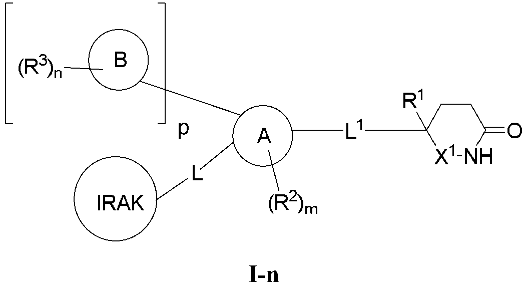 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 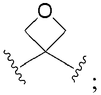
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 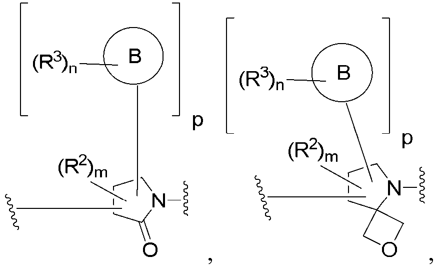 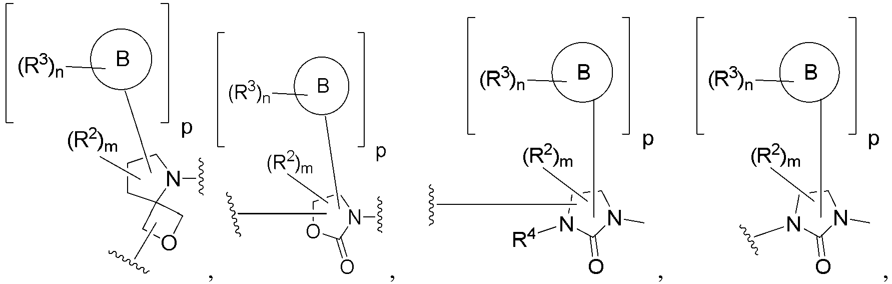 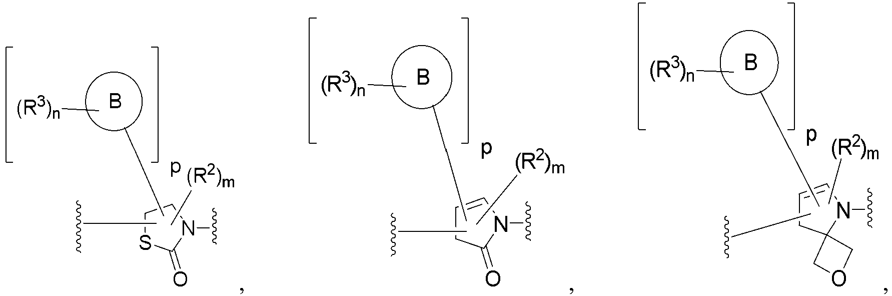 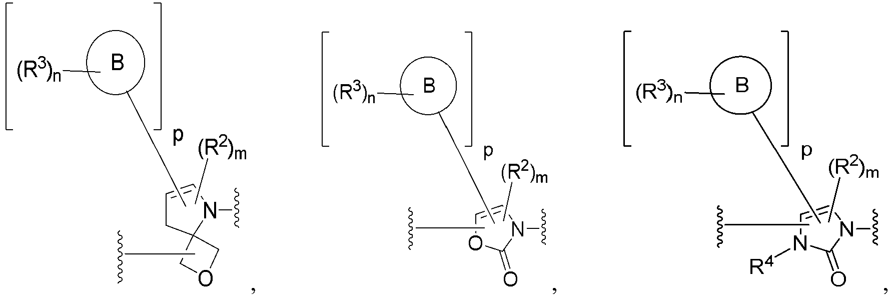 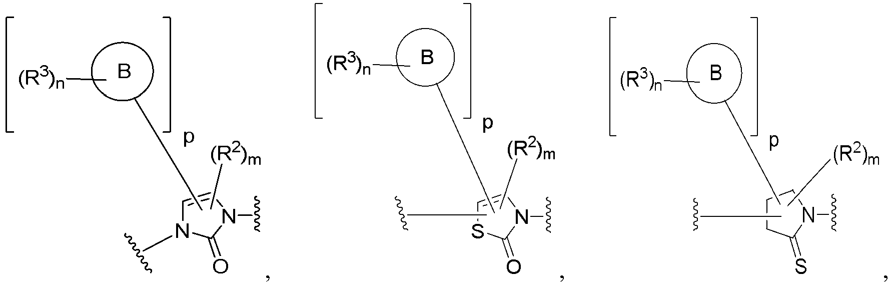 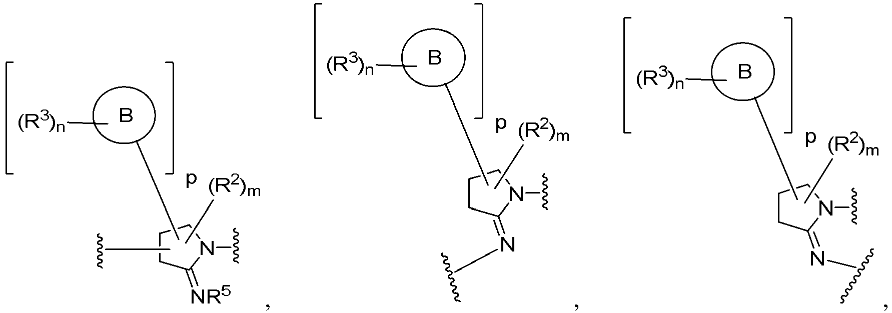 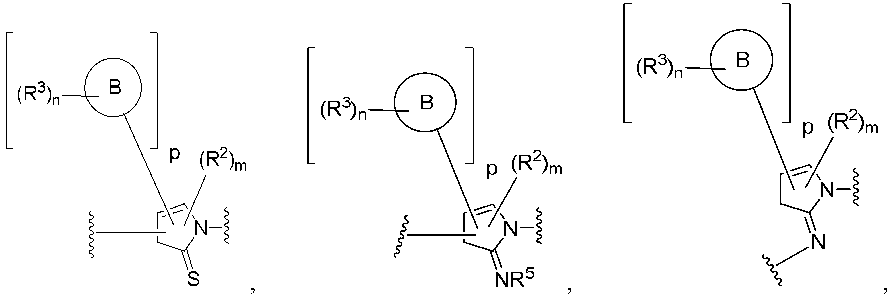 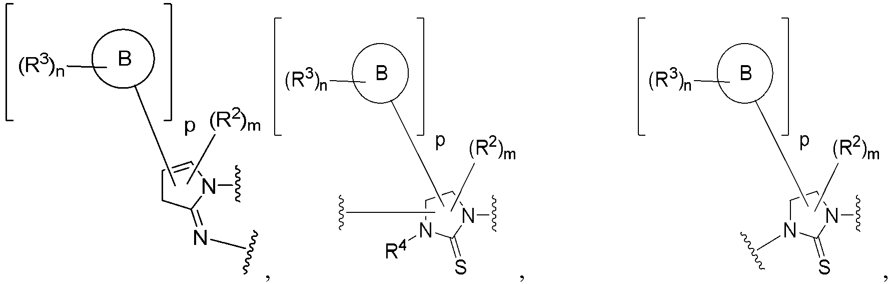 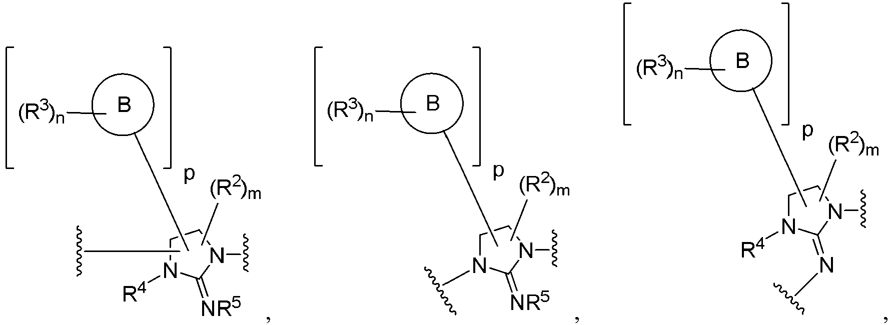 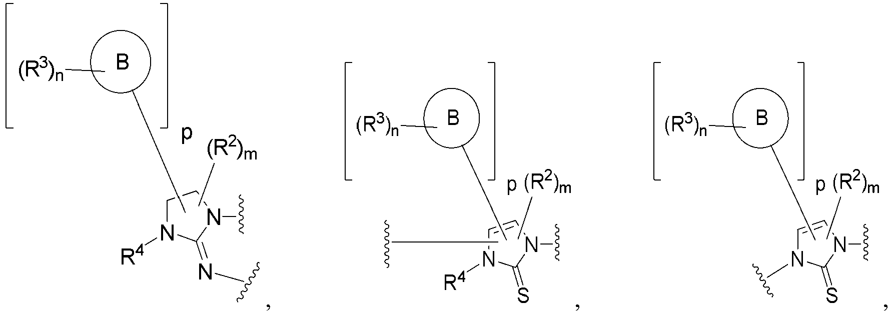 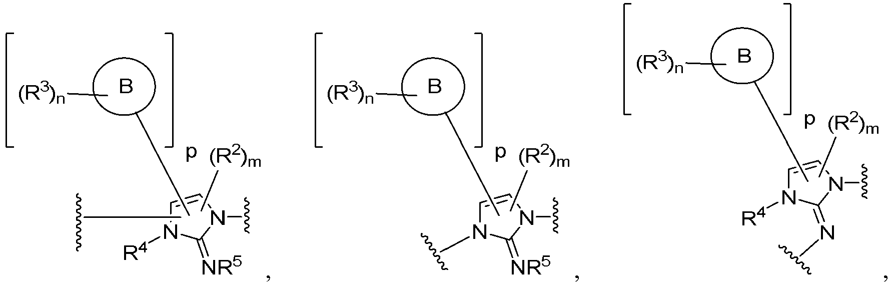 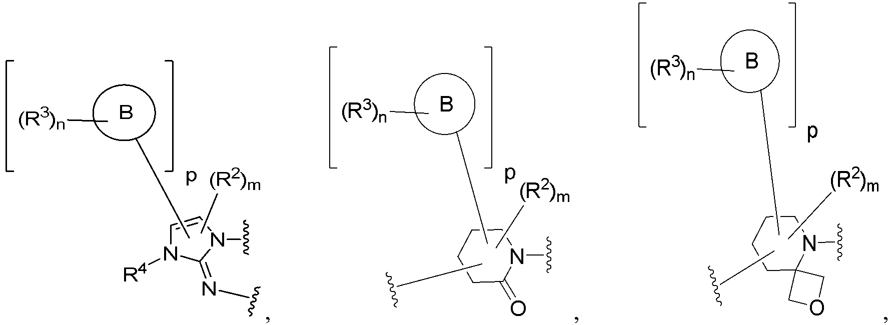 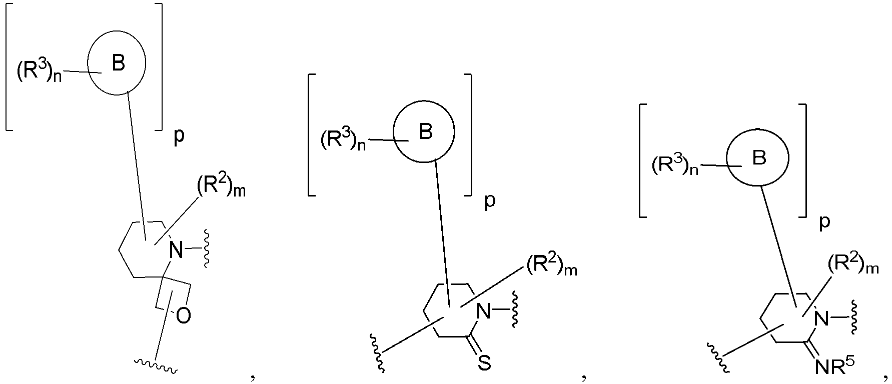 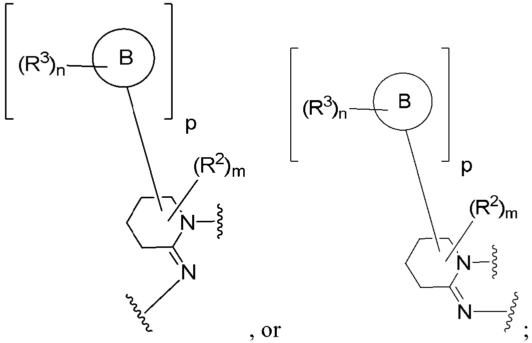
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 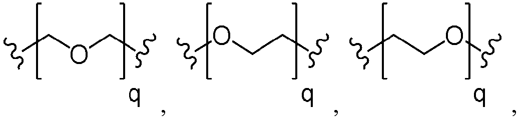 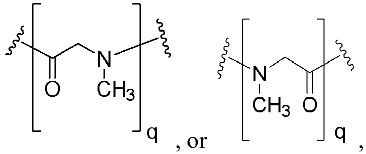 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1;
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 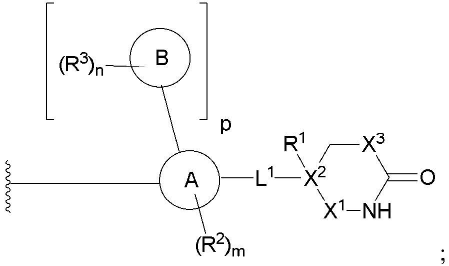 thereby forming a compound of formula I-n': 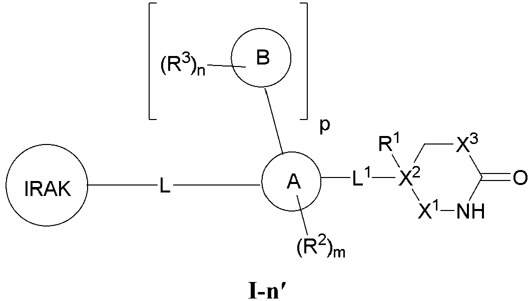 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 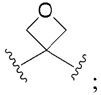
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 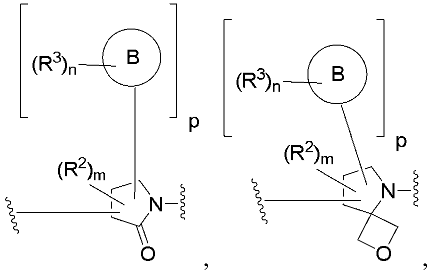 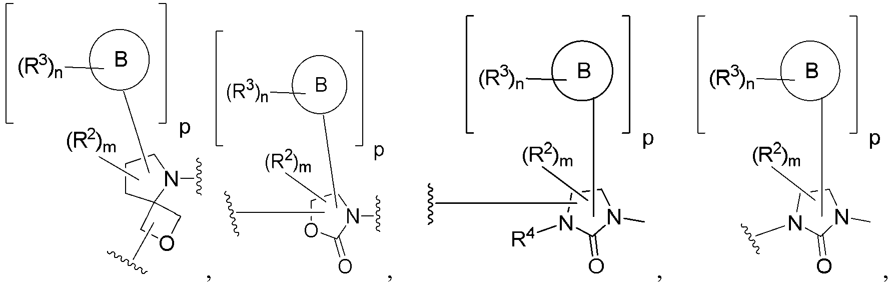 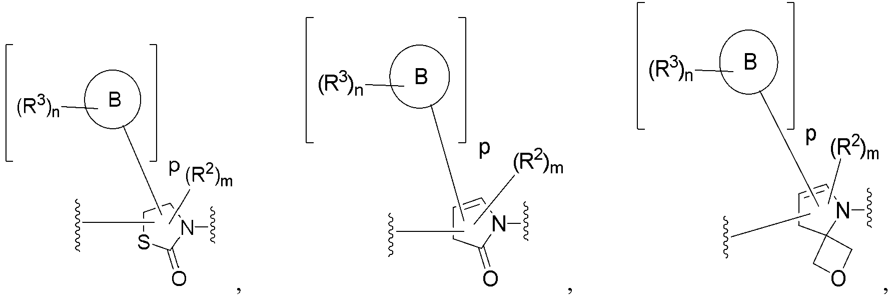 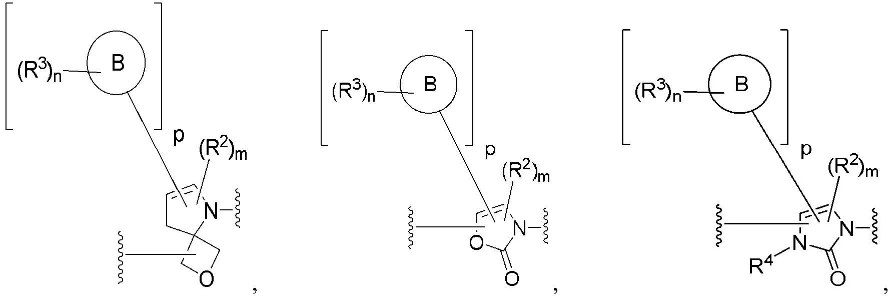 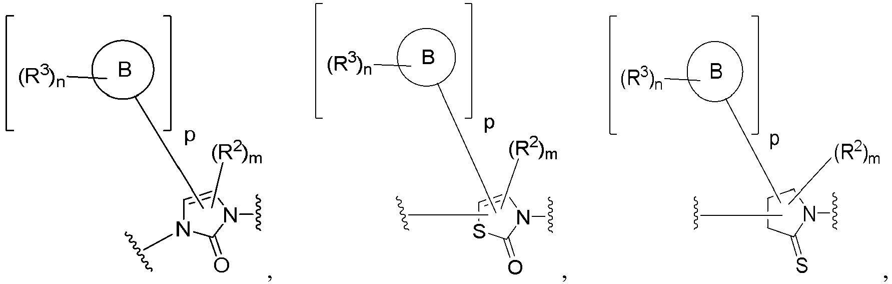 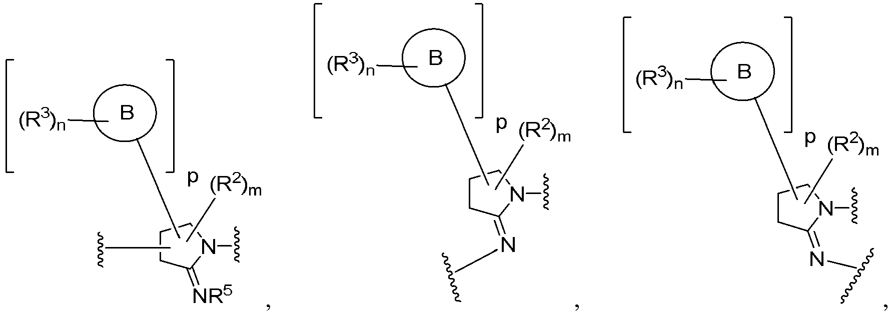 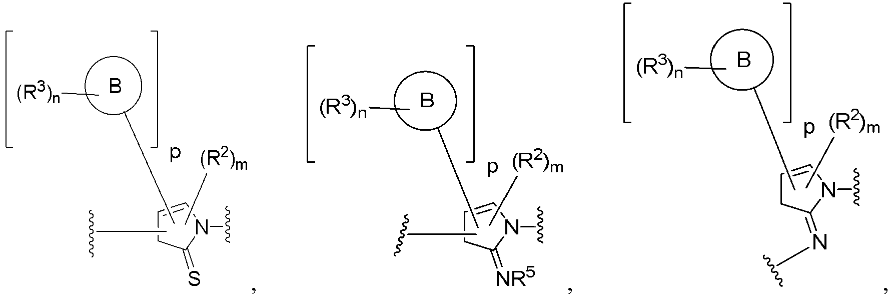 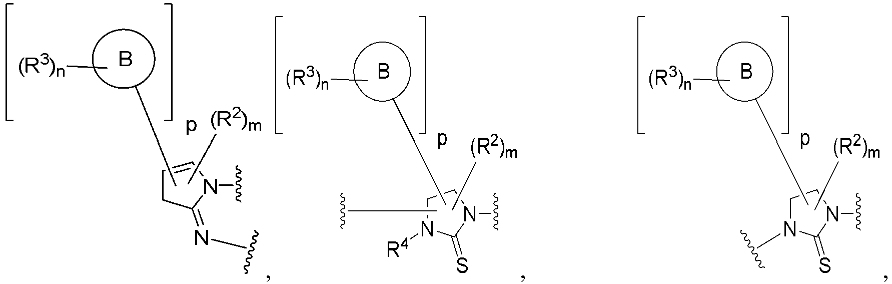 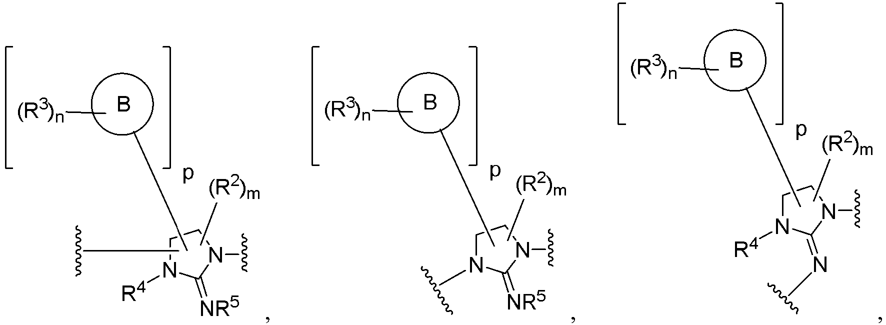 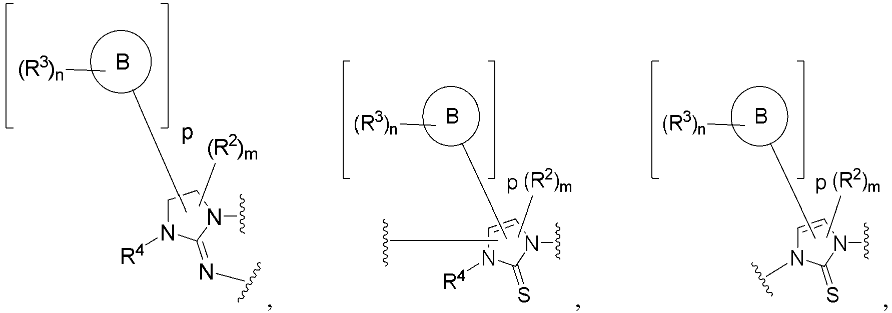 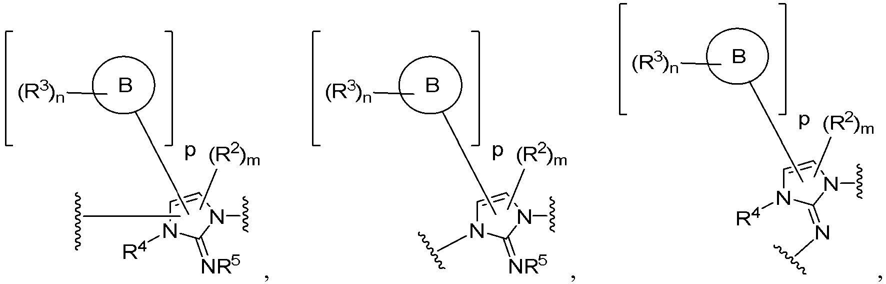 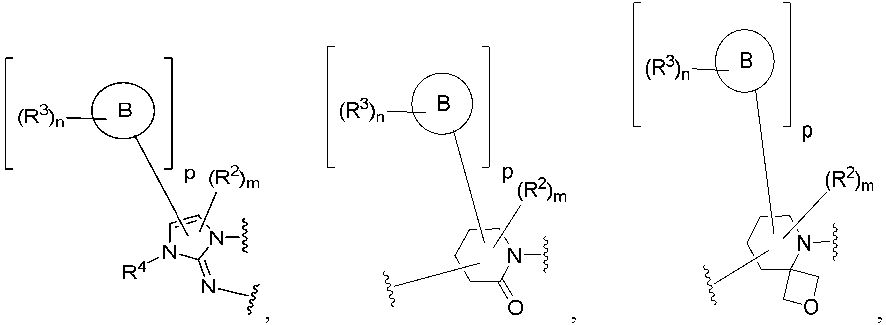 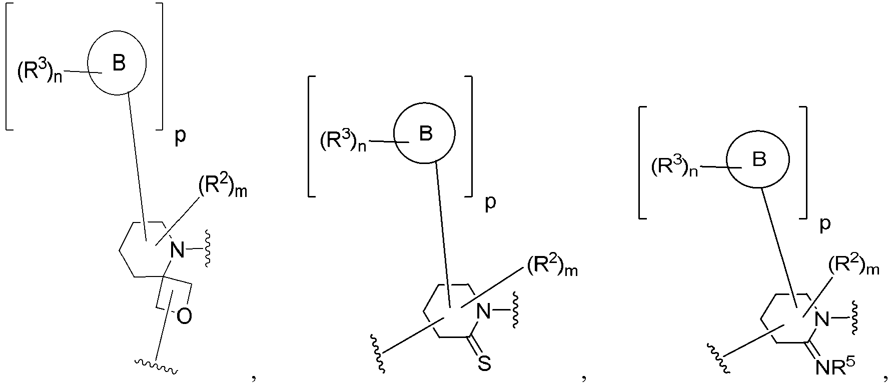 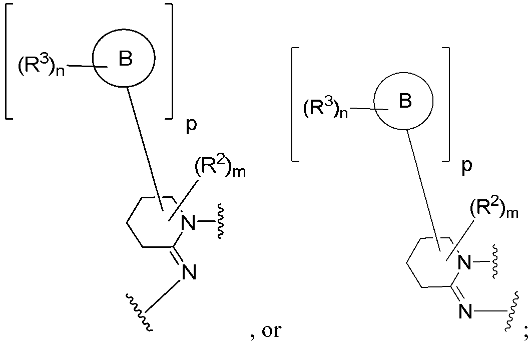
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 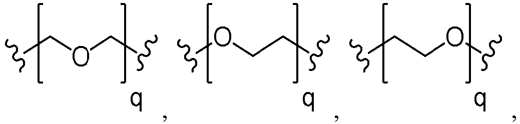 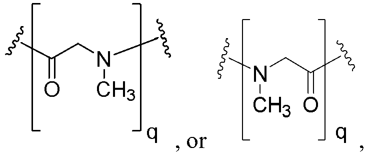 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1;
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:

two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, a compound of formala **I-n'** above is provided as a compound of formula **I-n"** or formula **I-n‴**: 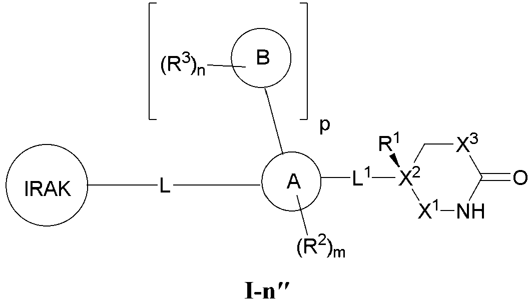 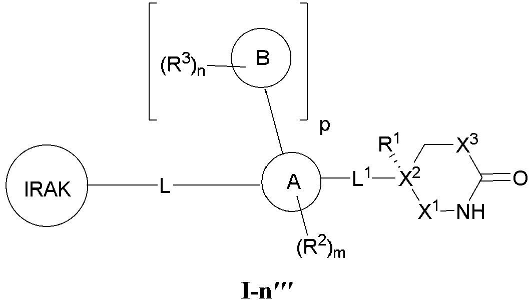 or a pharmaceutically acceptable salt thereof, wherein:
each of IRAK, Ring A, Ring B, L, L¹, R¹, R², R³, X¹, X², X³, n, p, and m is as defined above.

In certain embodiments, the present invention provides a compound of formula **I,** wherein LBM is an E3 ubiquitin ligase (cereblon) binding moiety 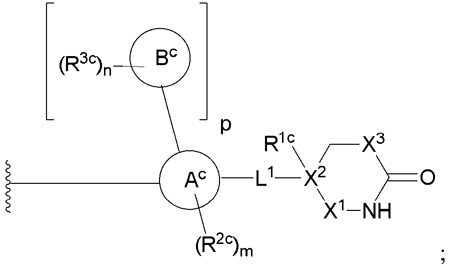 thereby forming a compound of formula **I-n-1**: 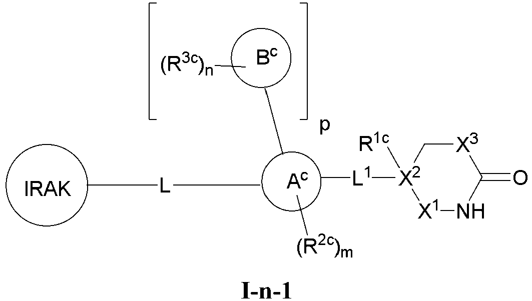 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 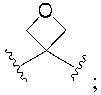
X² is a carbon atom or silicon atom;
X³ is a bivalent moiety selected from -CH₂- or -Si(R₂)-;
R^{1c} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{c} is a mono- or bicyclic ring selected from 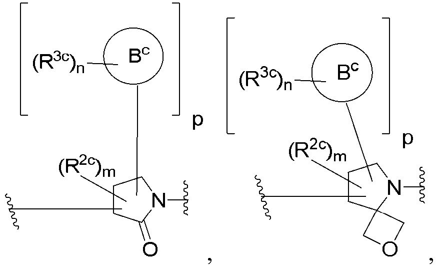 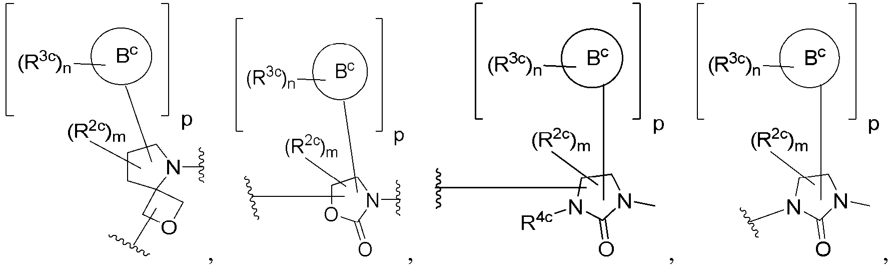 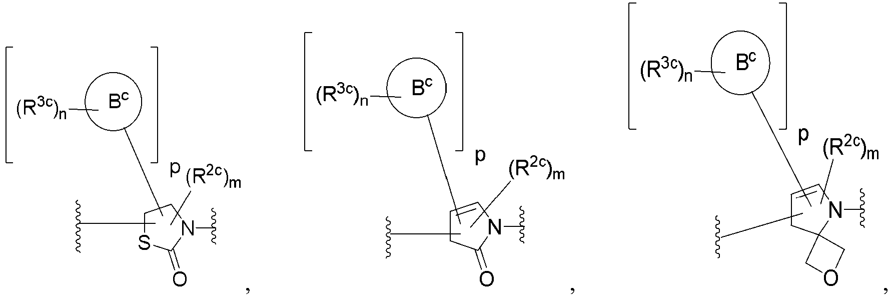 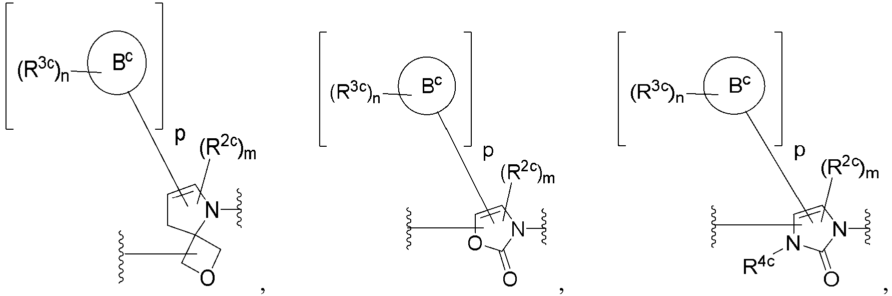 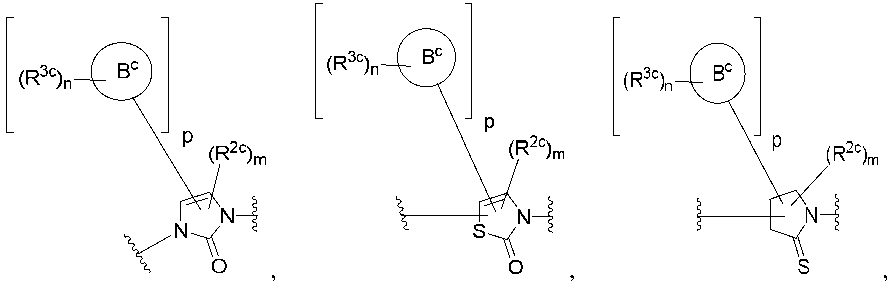 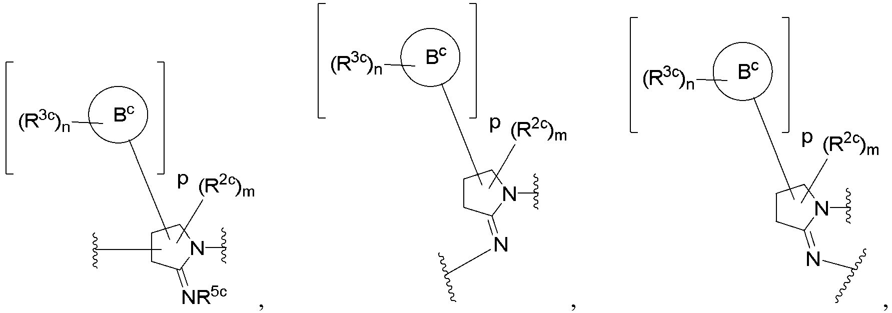 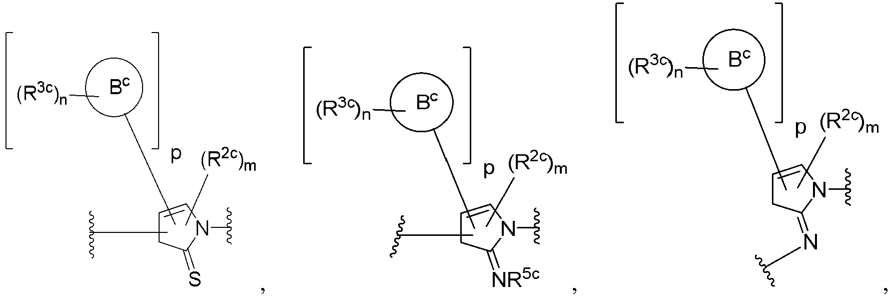 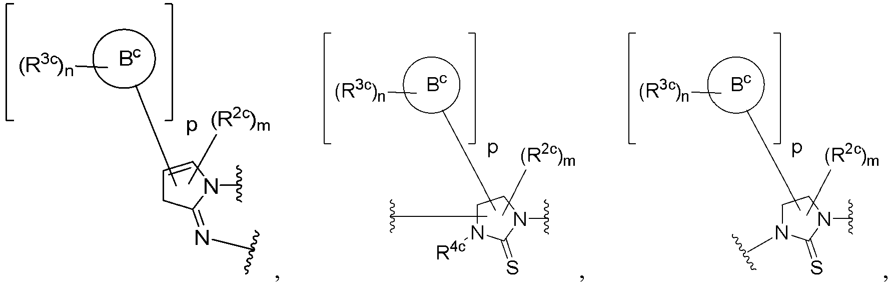 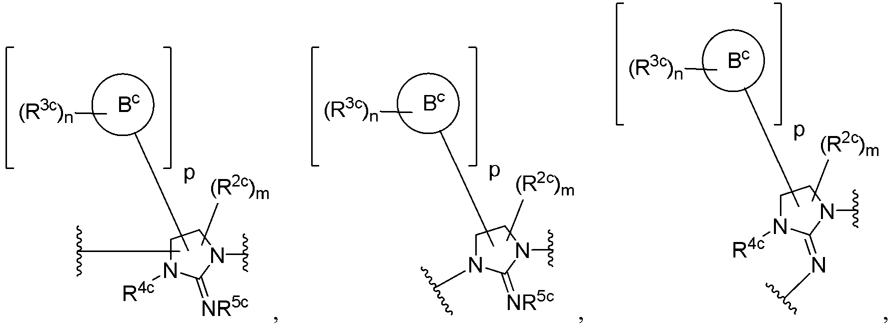 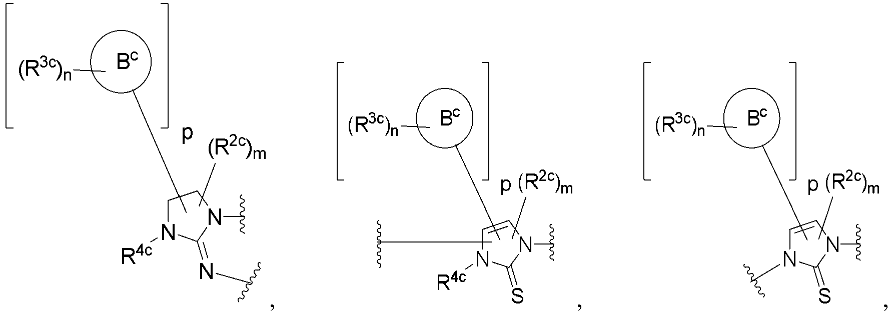 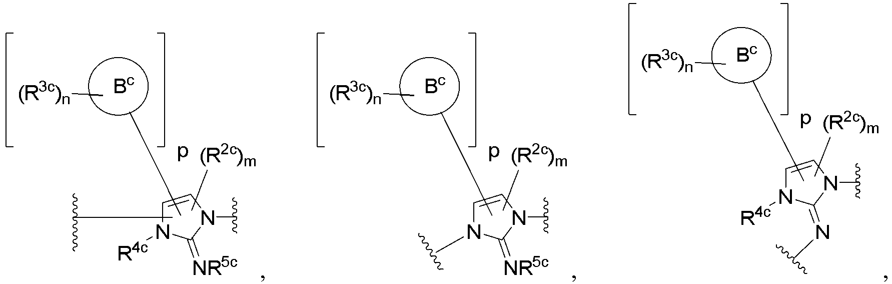 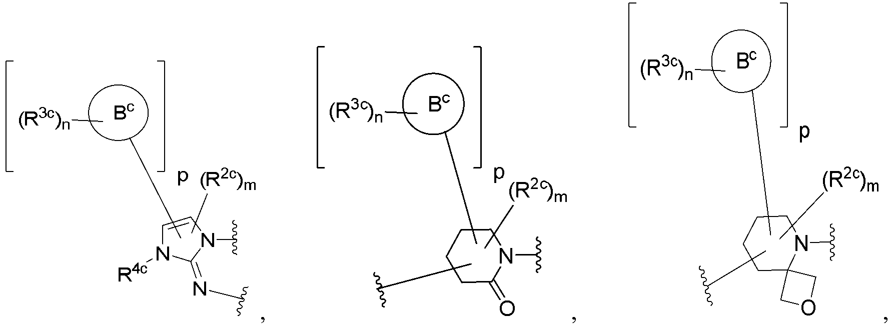 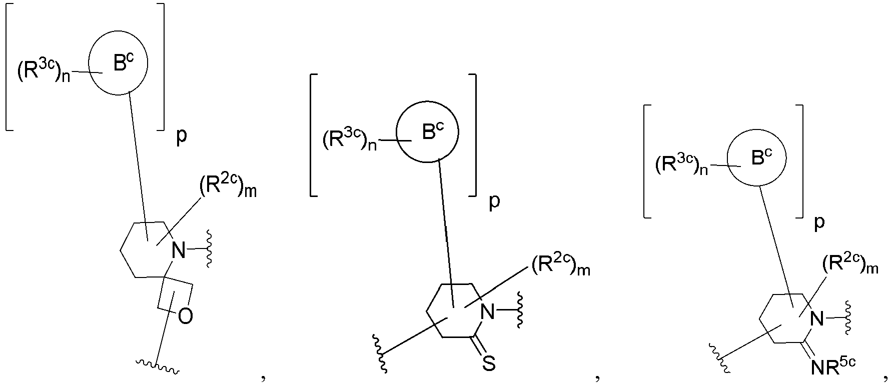 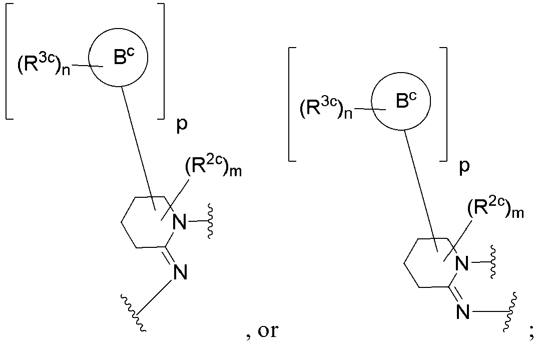
each R^{2c} is independently hydrogen, -R^{6c}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{c} is selected from a 6-membered aryl containing 0-2 nitrogen atoms or a 5-membered heteroaryl with 1-3 heteroatoms independently selected from nitrogen, oxygen or sulfur;
each of R^{3c} and R^{4c} is independently hydrogen, -R^{6c}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5c} is hydrogen, C₁₋₄ aliphatic, or -CN;
each R^{6c} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 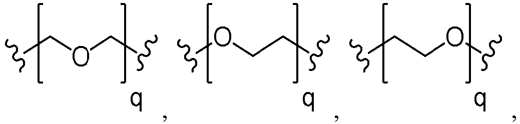 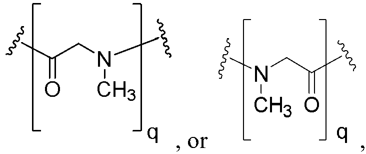 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, or 2;
   n is 0, 1, 2, 3, or 4;
   p is 0 or 1;
   each of q is independently 1, 2, 3, 4, 5, 6, 7, 8, 9, or 10; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, a compound of formala **I-n-1** above is provided as a compound of formula **I-n-1'** or formula **I-n-1"**: 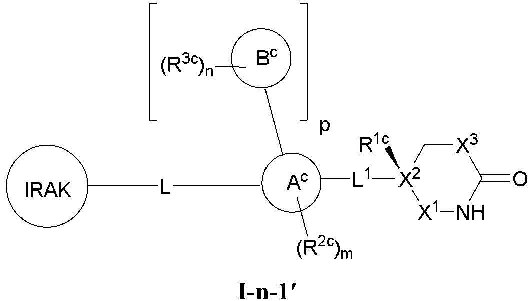 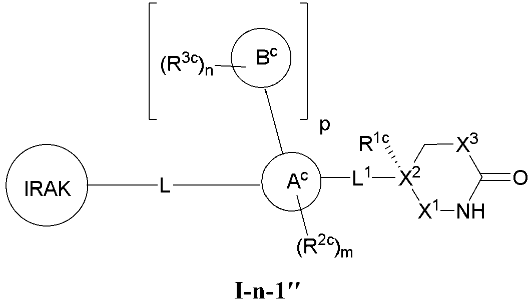 or a pharmaceutically acceptable salt thereof, wherein:
each of IRAK, Ring A^{c}, Ring B^{c}, L, L¹, R^{1c}, R^{2c}, R^{3c}, X¹, X², X³, n, p, and m is as defined above.

In some embodiments, LBM is 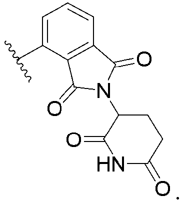 In some embodiments, LBM is 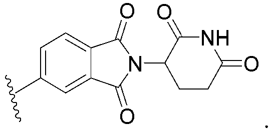 In some embodiments, LBM is

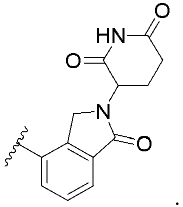

In some embodiments, LBM is 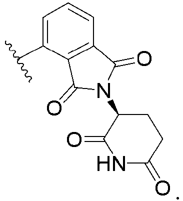 In some embodiments, LBM is 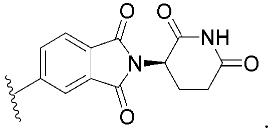 In some embodiments, LBM is

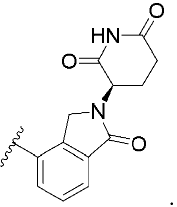

In some embodiments, LBM is 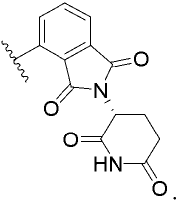 In some embodiments, LBM is 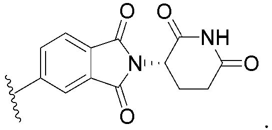 In some embodiments, LBM is

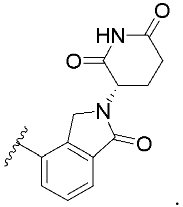

In some embodiments, LBM is 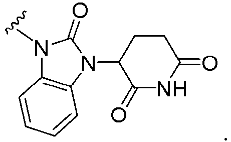 In some embodiments, LBM is 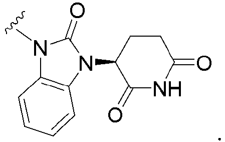 In some embodiments, LBM is 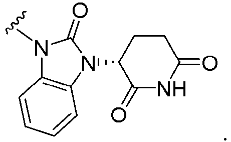 In some embodiments, LBM is 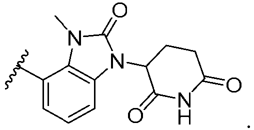 In some embodiments, LBM is 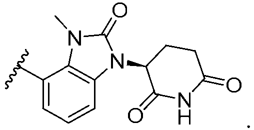 In some embodiments, LBM is 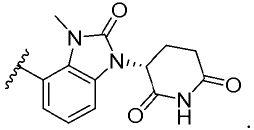 In some embodiments, LBM is 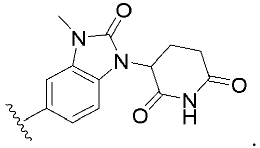 In some embodiments, LBM is 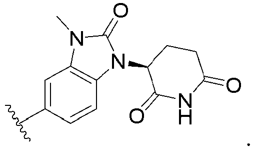 In some embodiments, LBM is

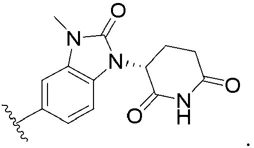

In some embodiments, LBM is 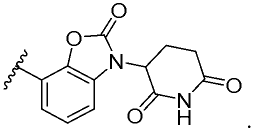 In some embodiments, LBM is 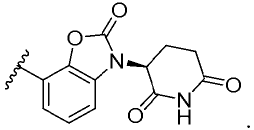 In some embodiments, LBM is 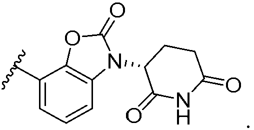 In some embodiments, LBM is 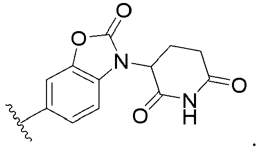 In some embodiments, LBM is 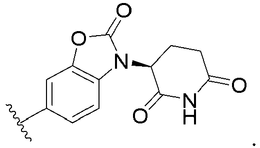 In some embodiments, LBM is

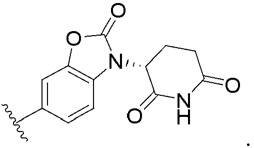

In some embodiments, LBM is 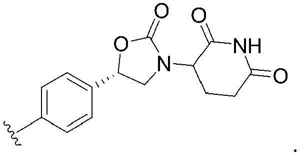 In some embodiments, LBM is 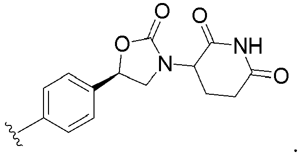 In some embodiments, LBM is 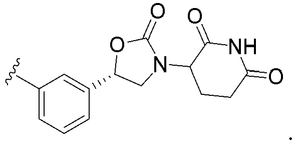 In some embodiments, LBM is

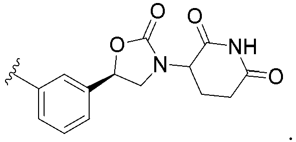

In some embodiments, LBM is selected from those depicted in **Table 1,** below.

In some embodiments, the present invention provides the compound of formula **II** as a compound of formula **II-f**: 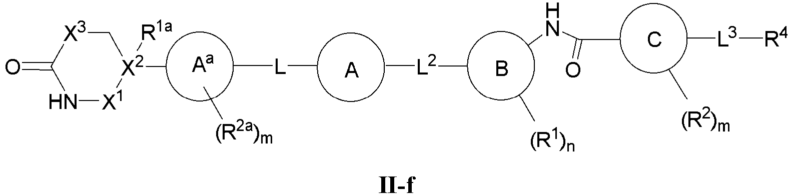 or a pharmaceutically acceptable salt thereof, wherein each of X¹, X², X³, R^{1a}, R^{2a}, Ring A^{a}, and m of the LBM moiety, L, and L², L³, Ring A, Ring B, Ring C, R¹, R², R⁴, n, and m of the IRAK moiety is as defined above and described in embodiments herein, both singly and in combination.

In certain embodiments, the present invention provides a compound of formula **II-f**: 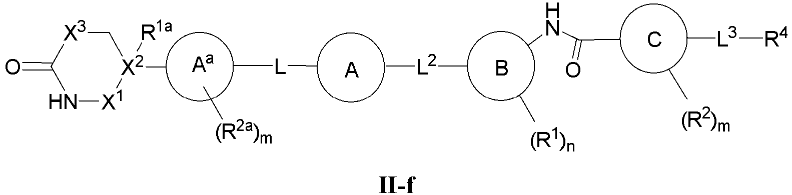 or a pharmaceutically acceptable salt thereof, wherein:
L is a bivalent moiety that connects Ring A^{a} to Ring A;
X¹ is a bivalent moiety selected from a covalent bond, -CH₂-, -C(O)-, -C(S)-, or 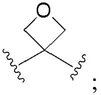
X² is a carbon atom or silicon atom;
X³ is a bivalent moiety selected from -CH₂- or -Si(R₂)-;
R^{1a} is hydrogen, deuterium, halogen, -CN, -OR, -SR, -S(O)R, -S(O)₂R, -N(R)₂, -Si(R)₃, or an optionally substituted C₁₋₄ aliphatic;
each R^{2a} is independently hydrogen, R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A^{a} is a bi- or tricyclic ring selected from 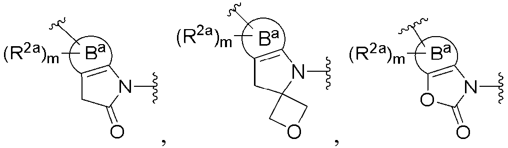 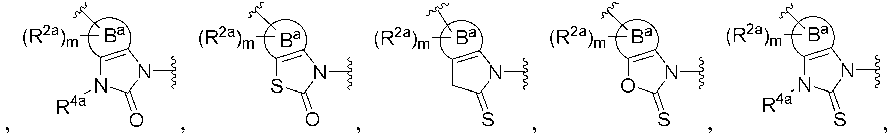 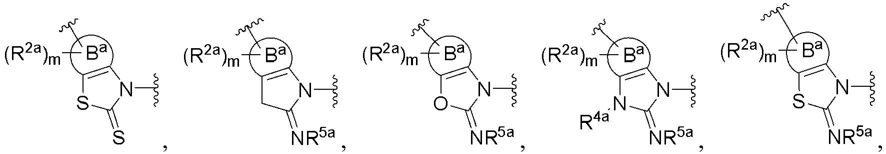 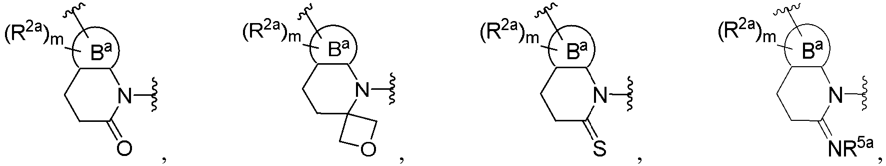 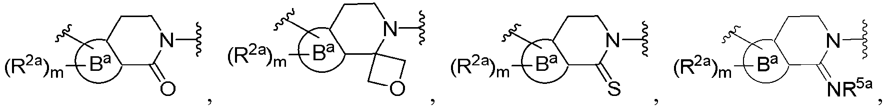 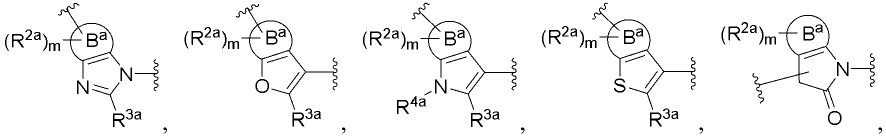 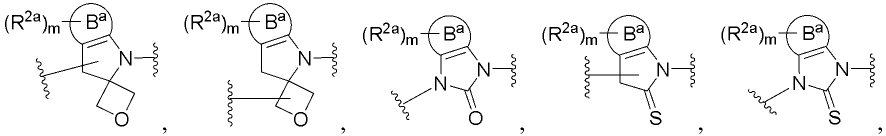 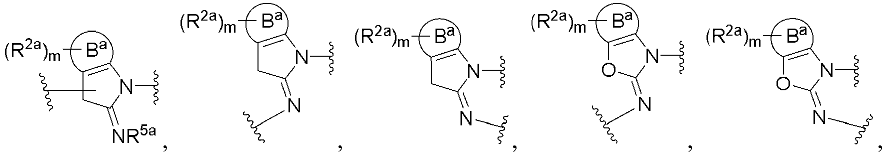 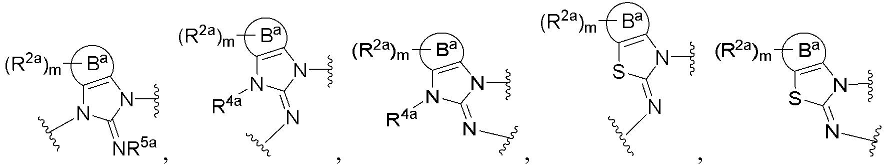 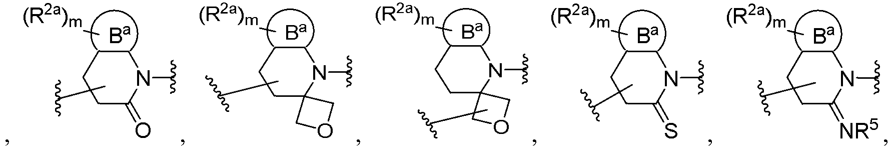 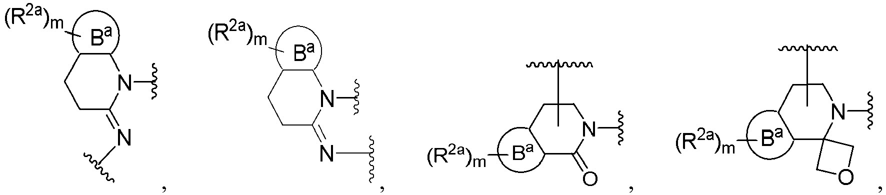 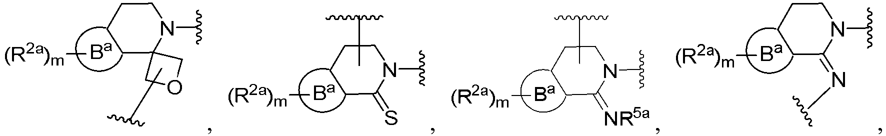 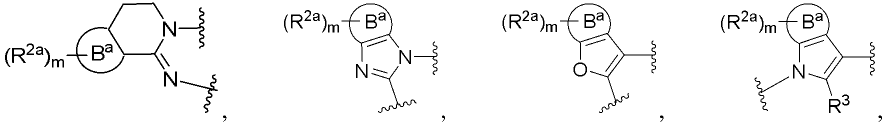 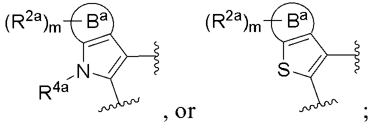
Ring B^{a} is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R^{3a} is selected from hydrogen, halogen, -OR, -N(R)₂, or -SR;
each R^{4a} is independently hydrogen, R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5a} is hydrogen, C₁₋₄ aliphatic, or -CN;
each R^{6a} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a 4-10 membered saturated mono- or bicyclic carbocyclic or heterocyclic ring having 0-2 heteroatoms independently selected from nitrogen, oxygen, and sulfur;
Ring B is phenyl, a 4-10 membered saturated or partially unsaturated mono- or bicyclic carbocyclic or heterocyclic ring having 1-3 heteroatoms independently selected from nitrogen, oxygen, and sulfur, or a 5-9 membered mono- or bicyclic heteroaryl ring having 1-4 heteroatoms independently selected from nitrogen, oxygen, and sulfur;
Ring C is phenyl or a 5-10 membered mono- or bicyclic heteroaryl ring having 1-5 heteroatoms independently selected from nitrogen, oxygen, and sulfur;
each of L² and L³ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
R⁴ is selected from 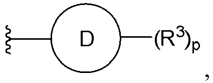 hydrogen, or an optionally substituted group selected from C₁₋₆ aliphatic or a 4-11 membered saturated or partially unsaturated carbocyclic or heterocyclic monocyclic, bicyclic, bridged bicyclic, or spiro ring having 1-3 heteroatoms independently selected from nitrogen, oxygen, and sulfur;
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
n is 0, 1, or 2;
each m is independently 0, 1, 2, 3 or 4; and
p is 0, 1, 2, 3 or 4;

In some embodiments, the present invention provides the compound of formula **II-f,** wherein X² is a carbon atom, X³ is -CH₂-, and Ring B is pyrazolyl as shown, thereby providing a compound of formula **II-f-1**: 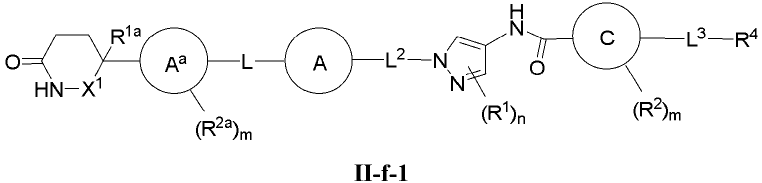 or a pharmaceutically acceptable salt thereof, wherein each of X¹, R^{1a}, R^{2a}, Ring A^{a}, and m of the LBM moiety, L, and L², L³, Ring A, Ring C, R¹, R², R⁴, n, and m of the IRAK moiety is as defined above and described in embodiments herein, both singly and in combination.

In some embodiments, the present invention provides the compound of formula **II-f,** wherein X² is a carbon atom, X³ is -CH₂-, Ring A^{a} is 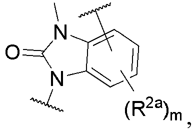 and Ring B is pyrazolyl as shown, thereby providing a compound of formula **II-f-2**: 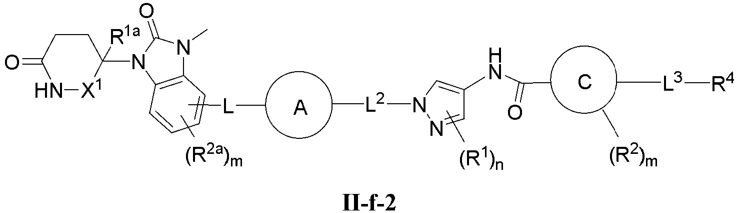 or a pharmaceutically acceptable salt thereof, wherein each of X¹, R^{1a}, R^{2a}, and m of the LBM moiety, L, and L², L³, Ring A, Ring C, R¹, R², R⁴, n, and m of the IRAK moiety is as defined above and described in embodiments herein, both singly and in combination.

In some embodiments, the present invention provides the compound of formula **II-f,** wherein X² is a carbon atom, X³ is -CH₂-, Ring A is cyclohexyl, L² is a covalent bond, and Ring B is pyrazolyl as shown, thereby providing a compound of formula **II-f-3**: 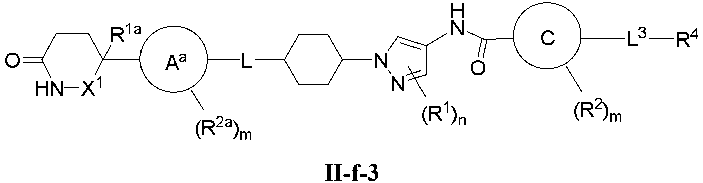 or a pharmaceutically acceptable salt thereof, wherein each of X¹, R^{1a}, R^{2a}, Ring A^{a}, and m of the LBM moiety, L, and L³, Ring C, R¹, R², R⁴, n, and m of the IRAK moiety is as defined above and described in embodiments herein, both singly and in combination.

In some embodiments, the present invention provides the compound of formula **II-f,** wherein X² is a carbon atom, X³ is -CH₂-, Ring C is pyrazolo[1,5-a]pyrimidine, and Ring B is pyrazolyl as shown, thereby providing a compound of formula **II-f-4**: 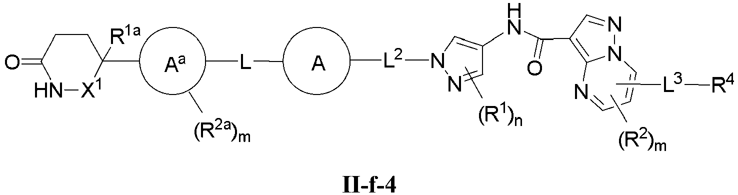 or a pharmaceutically acceptable salt thereof, wherein each of X¹, R^{1a}, R^{2a}, Ring A^{a}, and m of the LBM moiety, L, and L², L³, Ring A, Ring C, R¹, R², R⁴, n, and m of the IRAK moiety is as defined above and described in embodiments herein, both singly and in combination.

In some embodiments, the present invention provides the compound of formula **II-f,** wherein X² is a carbon atom, X³ is -CH₂-, L is 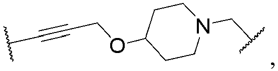 and Ring B is pyrazolyl as shown, thereby providing a compound of formula **II-f-5**: 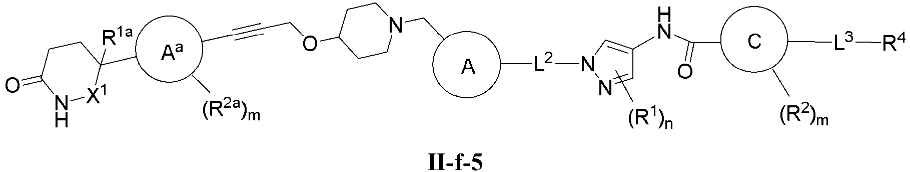 or a pharmaceutically acceptable salt thereof, wherein each of X¹, R^{1a}, R^{2a}, Ring A^{a}, and m of the LBM moiety, and L², L³, Ring A, Ring C, R¹, R², R⁴, n, and m of the IRAK moiety is as defined above and described in embodiments herein, both singly and in combination.

In some embodiments, the present invention provides the compound of formula **II,** wherein L² is a covalent bond, Ring A is cyclohexyl, Ring B is pyrazolyl, Ring C is oxazolyl, and Ring D is pyridyl thereby forming a compound of formula **II-g**: 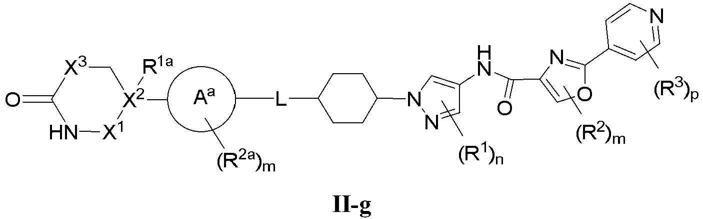 or a pharmaceutically acceptable salt thereof, wherein each of X¹, X², X³, R^{1a}, R^{2a}, Ring A^{a}, and m of the LBM moiety, L, and R¹, R², R³, n, m, and p of the IRAK moiety is as defined above and described in embodiments herein, both singly and in combination.

In some embodiments, the present invention provides the compound of formula **II,** wherein LBM is 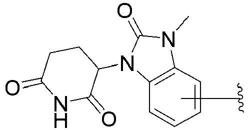 thereby forming a compound of formula **II-h**: 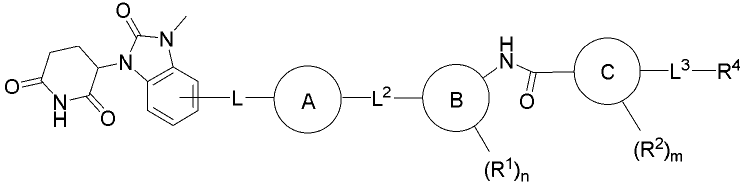 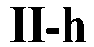 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is 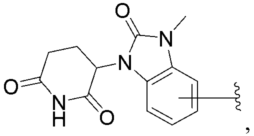 L² is a covalent bond, Ring A is cyclohexyl, Ring B is pyrazolyl, Ring C is oxazolyl, and Ring D is pyridyl thereby forming a compound of formula **II-i**: 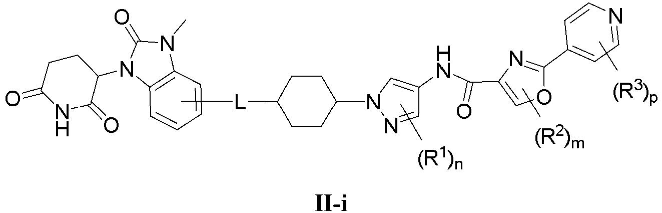 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 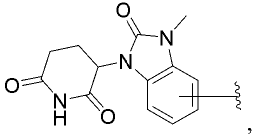 L² is a covalent bond, Ring A is cyclohexyl, and Ring B is pyrazolyl as shown, thereby forming a compound of formula **II-d-1**: 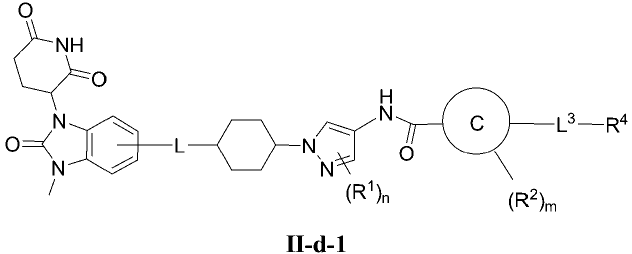 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 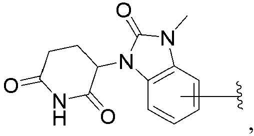 L² is a covalent bond, Ring A is cyclohexyl, Ring C is pyrazolo[1,5-a]pyrimidine, and Ring B is pyrazolyl as shown, thereby forming a compound of formula **II-e-1**: 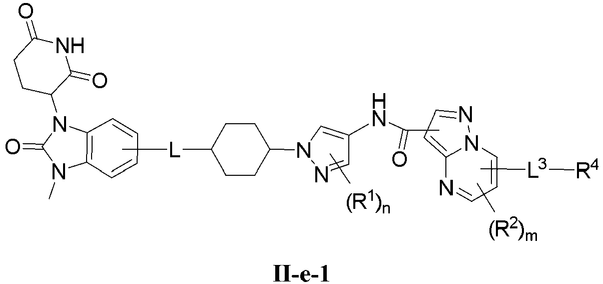 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is 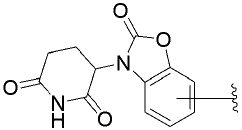 thereby forming a compound of formula **II-j**: 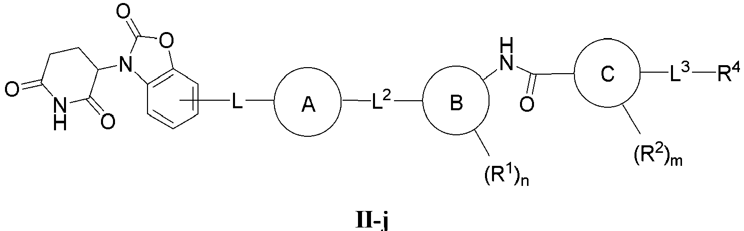 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is 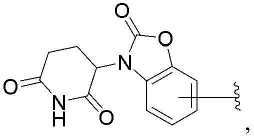 L ² is a covalent bond, Ring A is cyclohexyl, Ring B is pyrazolyl, Ring C is oxazolyl, and Ring D is pyridyl thereby forming a compound of formula **II-k**: 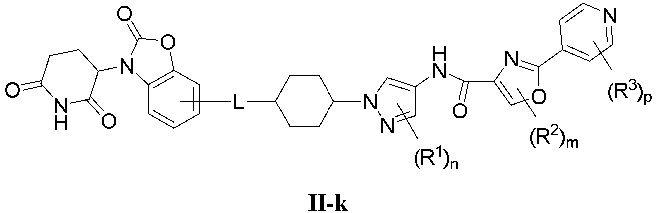 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 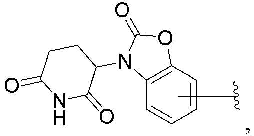 L² is a covalent bond, Ring A is cyclohexyl, and Ring B is pyrazolyl as shown, thereby forming a compound of formula **II-d-2**: 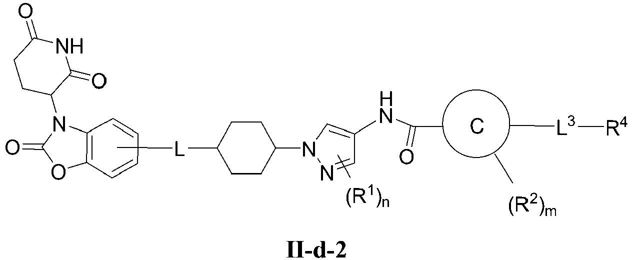 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 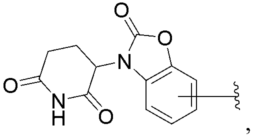 L² is a covalent bond, Ring A is cyclohexyl, Ring C is pyrazolo[1,5-a]pyrimidine, and Ring B is pyrazolyl as shown, thereby forming a compound of formula **II-e-2**: 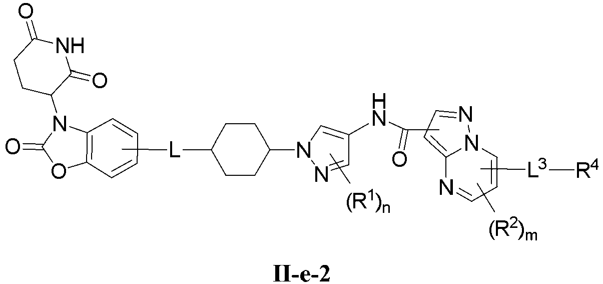 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is 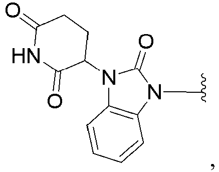 L² is a covalent bond, Ring A is cyclohexyl, Ring B is pyrazolyl, Ring C is oxazolyl, and Ring D is pyridyl thereby forming a compound of formula **II-l**: 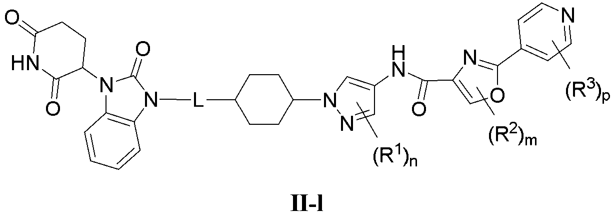 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 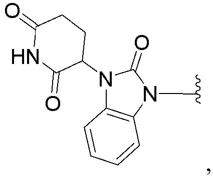 L² is a covalent bond, Ring A is cyclohexyl, and Ring B is pyrazolyl as shown, thereby forming a compound of formula **II-d-3**: 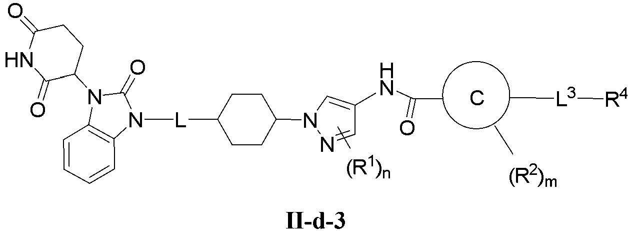 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 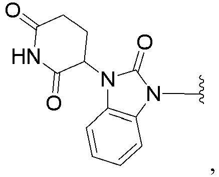 L² is a covalent bond, Ring A is cyclohexyl, Ring C is pyrazolo[1,5-a]pyrimidine, and Ring B is pyrazolyl as shown, thereby forming a compound of formula **II-e-3**: 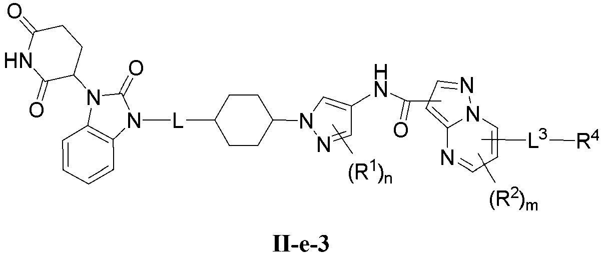 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is 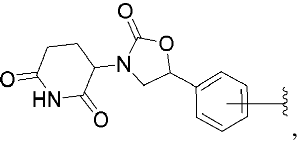 Ring A is cyclohexyl, Ring B is pyrazolyl, Ring C is oxazolyl, and Ring D is pyridyl thereby forming a compound of formula **II-m**: 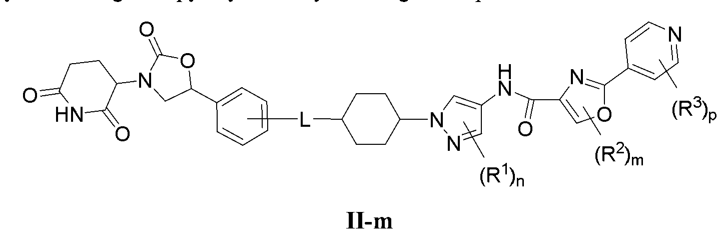 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 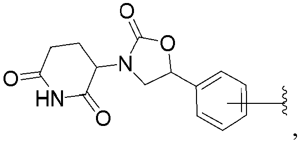 thereby forming a compound of formula **II-d-4**: 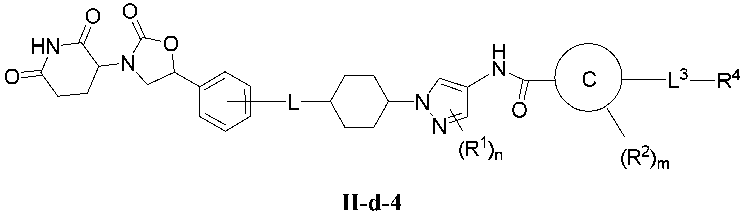 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 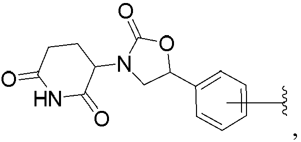 thereby forming a compound of formula **II-e-4**: 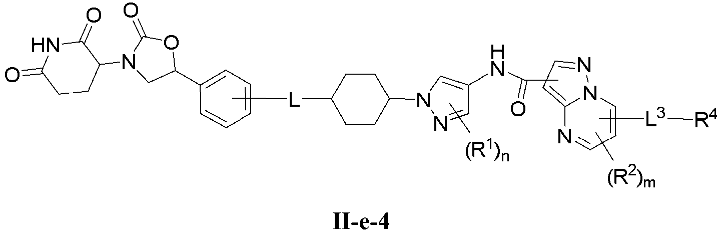 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is 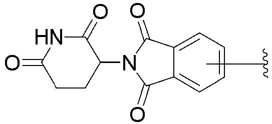 thereby forming a compound of formula **II-n**: 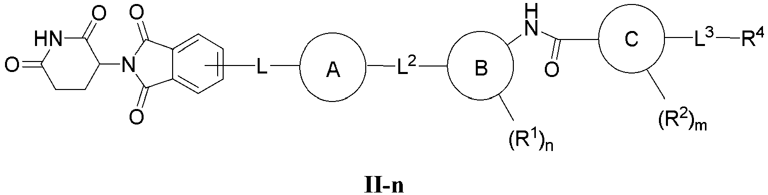 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is 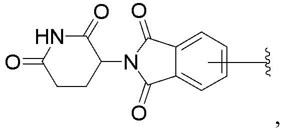 Ring A is cyclohexyl, Ring B is pyrazolyl, Ring C is oxazolyl, and Ring D is pyridyl thereby forming a compound of formula **II-o**: 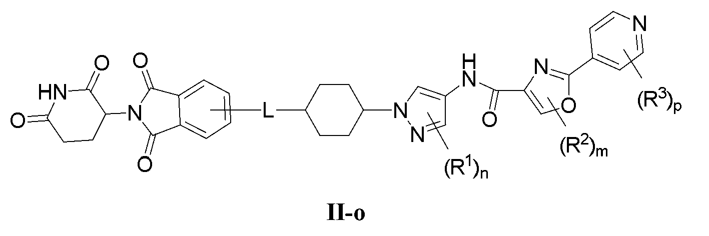 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 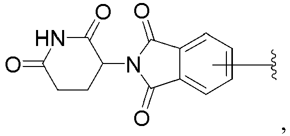 thereby forming a compound of formula **II-d-5**: 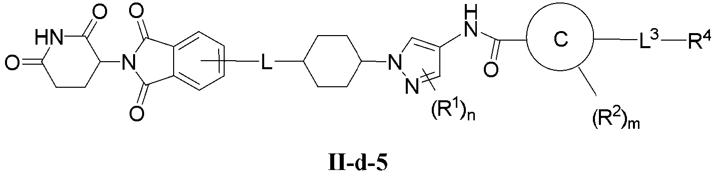 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 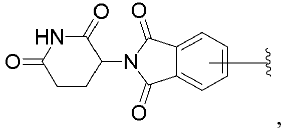 thereby forming a compound of formula **II-e-5**: 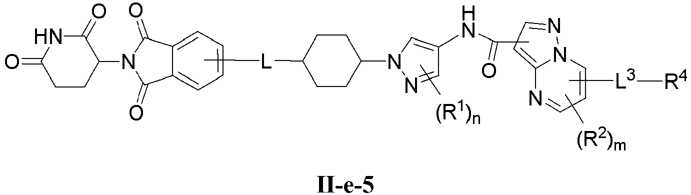 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is **II-p**: 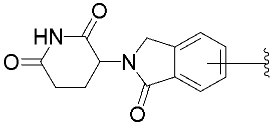 thereby forming a compound a compound of formula 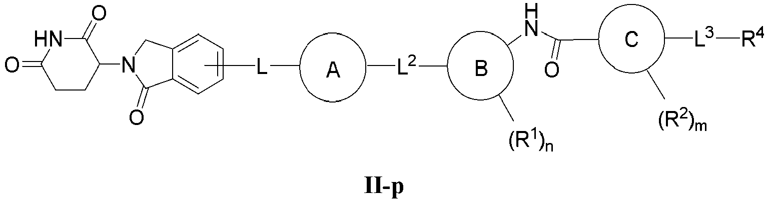 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is 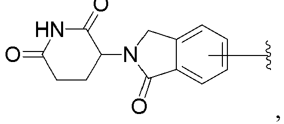 Ring A is cyclohexyl, Ring B is pyrazolyl, Ring C is oxazolyl, and Ring D is pyridyl thereby forming a compound of formula **II-q**: 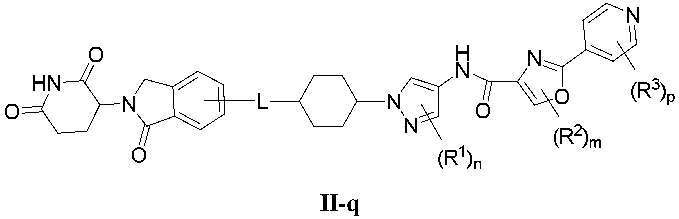 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is 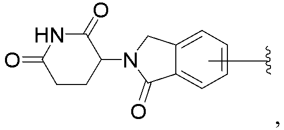 thereby forming a compound of formula **II-d-6**: 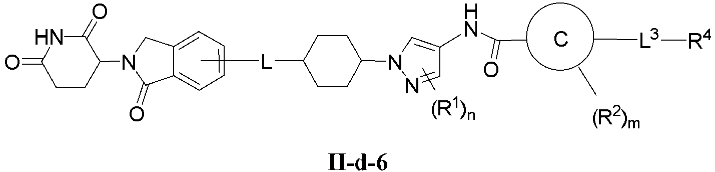 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is 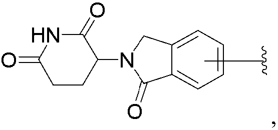 thereby forming a compound of formula **II-e-6**: 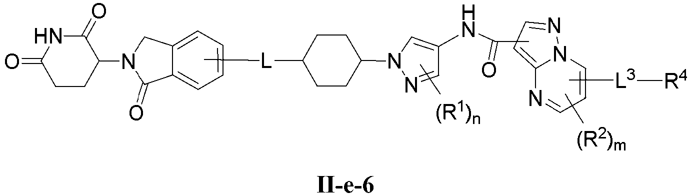 or a pharmaceutically acceptable salt thereof, wherein each of L, R¹, R², R³, n, m, and p is as defined above and described in embodiments herein, both singly and in combination.

In certain embodiments, the present invention provides a compound of formula **I,** wherein LBM is an E3 ubiquitin ligase (cereblon) binding moiety 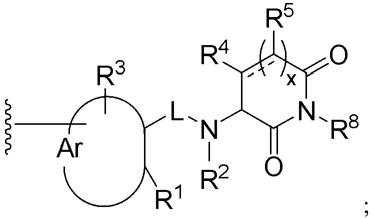 thereby forming a compound of formula **I-o**: 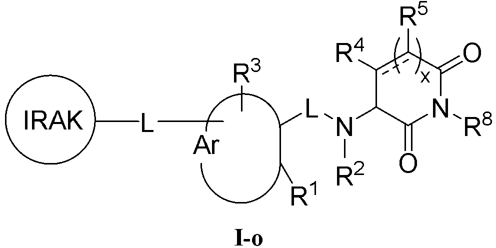 or a pharmaceutically acceptable salt thereof, wherein L and IRAK are as defined above and described in embodiments herein, and wherein each of the variables Ar, R¹, R², R³, R⁴, R⁵, R⁸, L, x, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 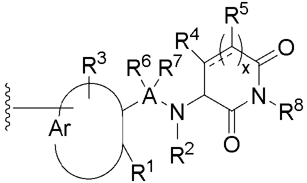 thereby forming a compound of formula **I-p**: 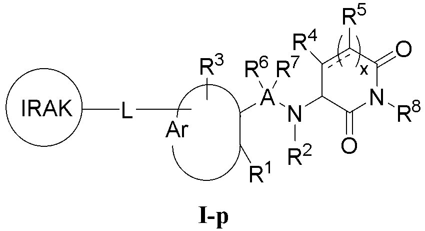 or a pharmaceutically acceptable salt thereof, wherein L and IRAK are as defined above and described in embodiments herein, and wherein each of the variables Ar, R¹, R², R³, R⁴, R⁵, R⁶, R⁷, R⁸, A, x, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 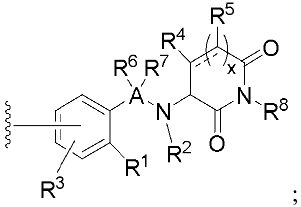 thereby forming a compound of formula **I-q**: 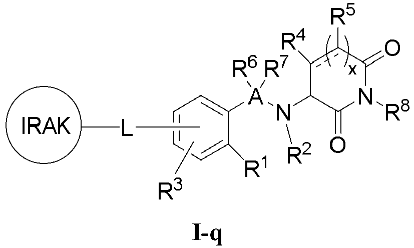 or a pharmaceutically acceptable salt thereof, wherein L and IRAK are as defined above and described in embodiments herein, and wherein each of the variables R¹, R², R³, R⁴, R⁵, R⁶, R⁷, R⁸, A, x, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 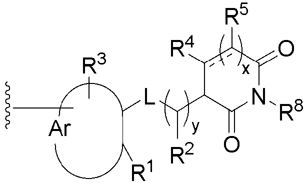 thereby forming a compound of formula **I-r**: 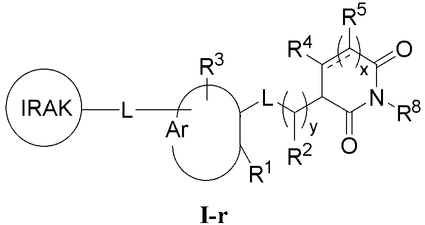 or a pharmaceutically acceptable salt thereof, wherein L and IRAK are as defined above and described in embodiments herein, and wherein each of the variables Ar, R¹, R², R³, R⁴, R⁵, R⁸, L, x, y, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 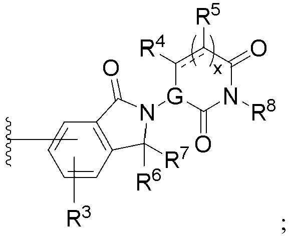 thereby forming a compound of formula **I-s**: 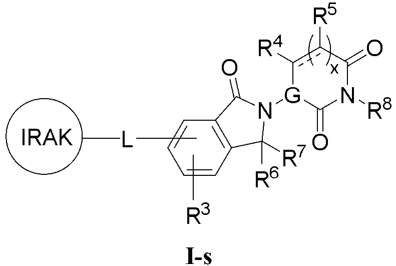 or a pharmaceutically acceptable salt thereof, wherein L and IRAK are as defined above and described in embodiments herein, and wherein each of the variables G, R³, R⁴, R⁵, R⁶, R⁷, R⁸, x, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 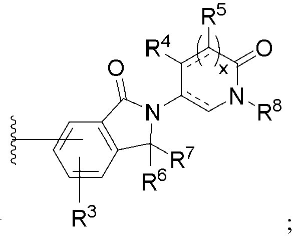 thereby forming a compound of formula **I-t**: 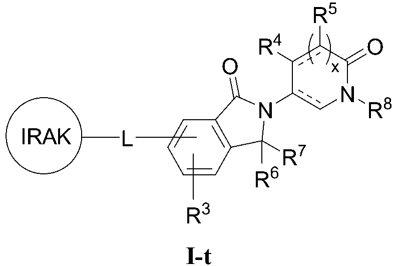 or a pharmaceutically acceptable salt thereof, wherein L and IRAK are as defined above and described in embodiments herein, and wherein each of the variables R³, R⁴, R⁵, R⁶, R⁷, R⁸, x, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 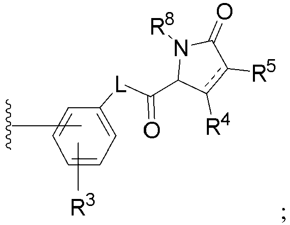 thereby forming a compound of formula **I-u**: 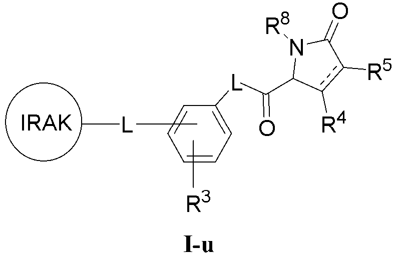 or a pharmaceutically acceptable salt thereof, wherein L and IRAK are as defined above and described in embodiments herein, and wherein each of the variables R³, R⁴, R⁵, R⁸, L, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 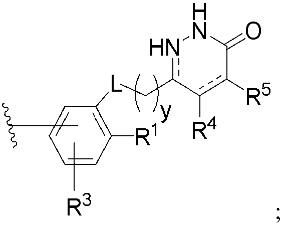 thereby forming a compound of formula **I-v**: 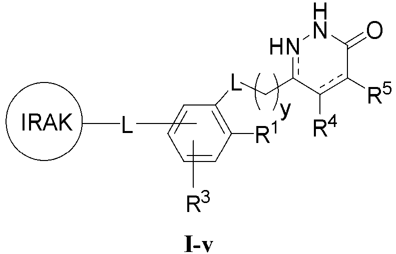 or a pharmaceutically acceptable salt thereof, wherein L and IRAK are as defined above and described in embodiments herein, and wherein each of the variables R¹, R³, R⁴, R⁵, L, y, and the bond --- is as described and defined in WO 2017/161119, the entirety of each of which is herein incorporated by reference.

In certain embodiments, the present invention provides a compound of formula **I,** wherein LBM is an E3 ubiquitin ligase (cereblon) binding moiety 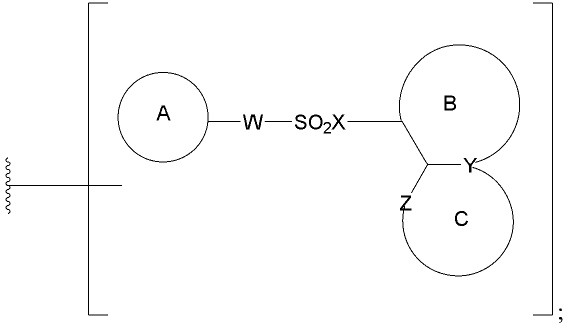 thereby forming a compound of formula I-x: 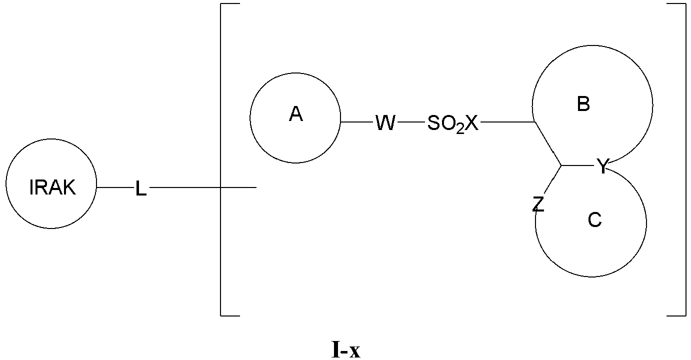 or a pharmaceutically acceptable salt thereof, wherein L and IRAK are as defined above and described in embodiments herein, and wherein each of the variables A, B, C, W, X, Y, and Z is as described and defined in US 5,721,246, the entirety of each of which is herein incorporated by reference.

In certain embodiments, the present invention provides a compound of Formula **I,** wherein LBM is an E3 ubiquitin ligase (cereblon) binding moiety 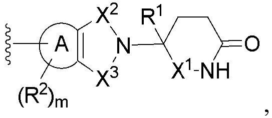 a DCAF15 E3 ubiquitin ligase binding moiety 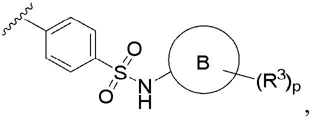 or a VHL E3 ubiquitin ligase binding moiety 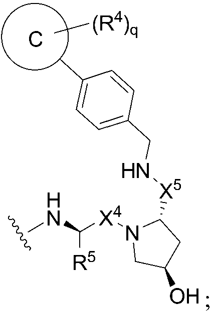 thereby forming a compound of formula **I-y-1, I-y-2,** or **I-y-3**: 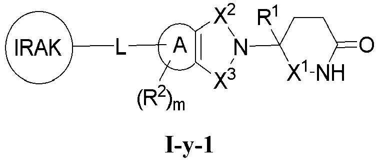 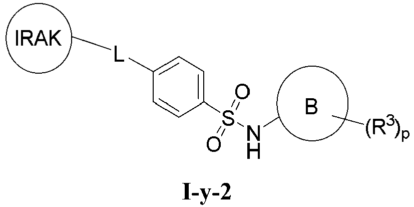 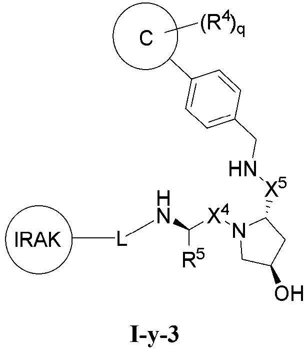 or a pharmaceutically acceptable salt thereof, wherein IRAK is as defined above and described in embodiments herein, and wherein:
each of X¹, X², and X³ is independently a bivalent moiety selected from a covalent bond, -CH₂-, -C(O)-, -C(S)-, or 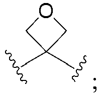
each of X⁴ and X⁵ is independently a bivalent moiety selected from -CH₂-, -C(O)-, -C(S)-, or 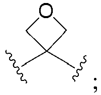
R¹ is hydrogen, deuterium, halogen, -CN, -OR, -SR, -S(O)R, -S(O)₂R, -NR₂, or an optionally substituted C₁₋₄ aliphatic;
each of R², R³, and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen or C₁₋₆ aliphatic;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
Ring B is selected from 6-membered aryl containing 0-2 nitrogen atoms or a 8-10 membered bicyclic heteroaryl having 1-5 heteroatoms independently selected from nitrogen, oxygen, or sulfur;
Ring C is a selected from 6-membered aryl containing 0-2 nitrogen atoms or a 5-membered heteroaryl with 1-3 heteroatoms independently selected from nitrogen, oxygen or sulfur;
L is a covalent bond or a bivalent, saturated or unsaturated, straight or branched C₁₋₅₀ hydrocarbon chain, wherein 0-6 methylene units of L are independently replaced by -Cy-, -O-, -NR-, - S-, -OC(O)-, -C(O)O-, -C(O)-, -S(O)-, -S(O)₂-, -NRS(O)₂-, -S(O)₂NR-, -NRC(O)-, - C(O)NR-, -OC(O)NR-, -NRC(O)O-, 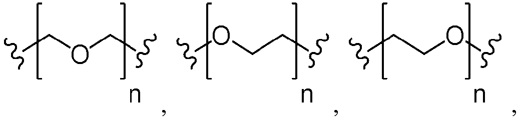 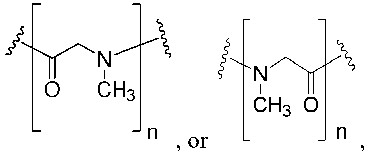 wherein:
   each -Cy- is independently an optionally substituted bivalent ring selected from phenylenyl, an 8-10 membered bicyclic arylenyl, a 4-7 membered saturated or partially unsaturated carbocyclylenyl, a 4-7 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-7 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur;
   m is 0, 1, 2, 3 or 4;
   each of n is independently 1, 2, 3, 4, 5, 6, 7, 8, 9, or 10;
   p is 0, 1, 2, 3 or 4;
   q is 0, 1, 2, 3 or 4; and
   each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
      two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 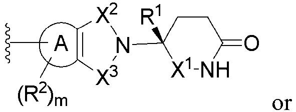 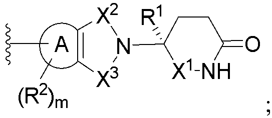 thereby forming a compound of formula **I-y'-1** or **I-y''-1**: 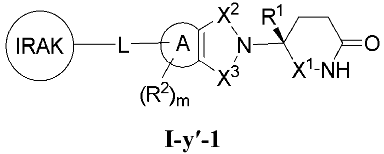 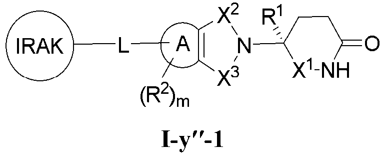 wherein IRAK, L, Ring A, X¹, X², X³, R¹, R² and m are as defined above.

In certain embodiments, the present invention provides a compound of formula **I,** wherein LBM is an E3 ubiquitin ligase (cereblon) binding moiety 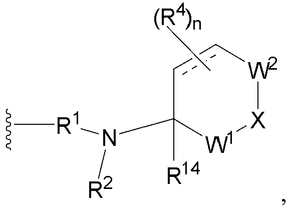 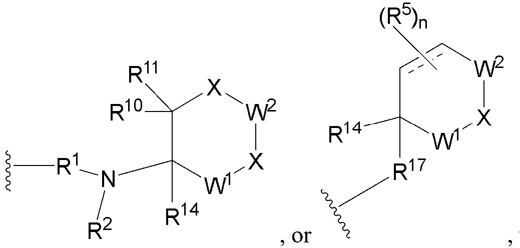 thereby forming a compound of formula **I-z-1, I-z-2,** or **I-z-3** respectively: 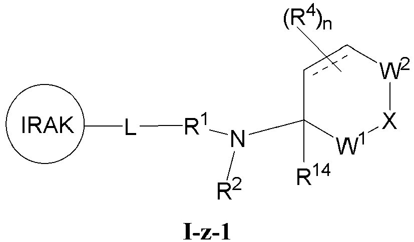 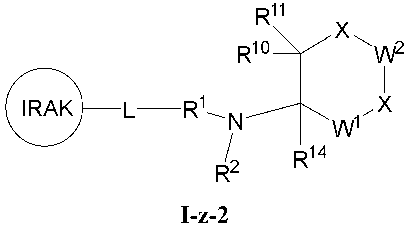 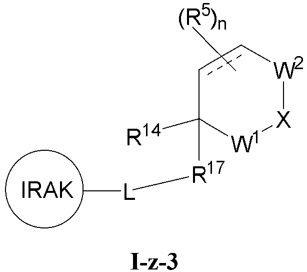 or a pharmaceutically acceptable salt thereof, wherein L and IRAK are as defined above and described herein, and wherein each of the variables R¹, R², R⁴, R⁵, R¹⁰, R¹¹, R¹⁴, R¹⁷, W¹, W², X and n is as defined in WO 2017/197051, the entirety of each of which is herein incorporated by reference, and wherein 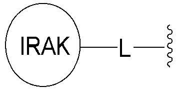 is attached to R¹, the ring formed by combining R¹ and R², or R¹⁷ at the site of attachment of R¹² as defined in WO 2017/197051 such that 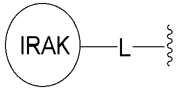 takes the place of the R¹² substituent.

In certain embodiments, the present invention provides a compound of formula **I,** wherein LBM is an E3 ubiquitin ligase (cereblon) binding moiety 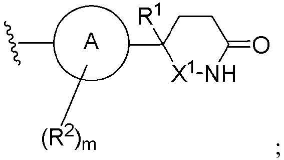 thereby forming a compound of formula **I-aa**: 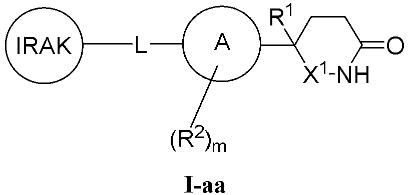 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 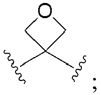
R¹ is hydrogen, deuterium, halogen, -CN, -OR, -SR, -S(O)R, -S(O)₂R, -NR₂, or an optionally substituted C₁₋₄ aliphatic;
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 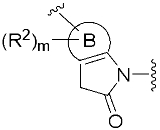 wherein Ring B is other than imidazo or benzo, 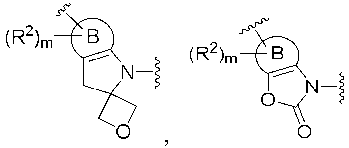 wherein Ring B is other than benzo, 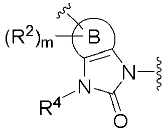 wherein Ring B is other than benzo, 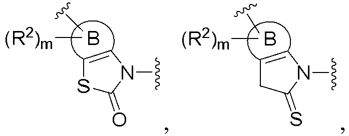 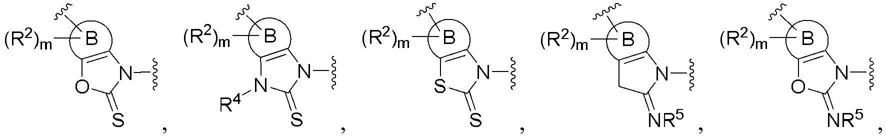 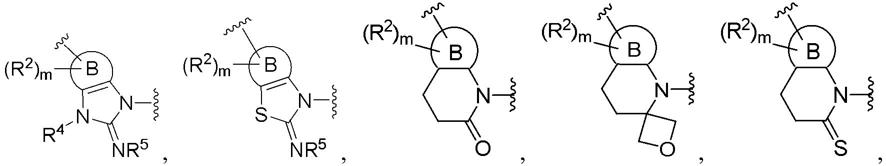 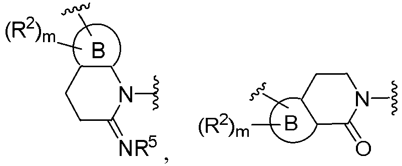 wherein Ring B is other than benzo, 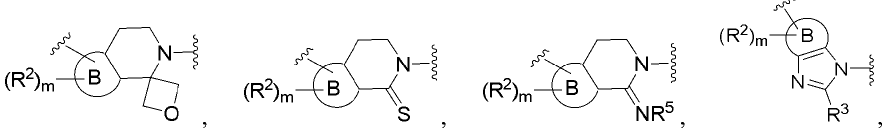 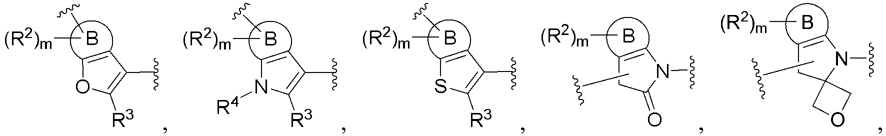 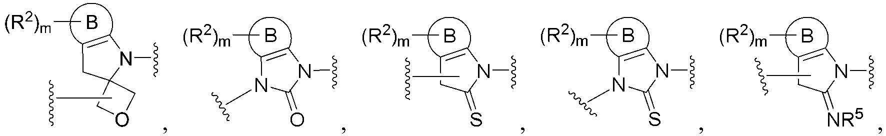 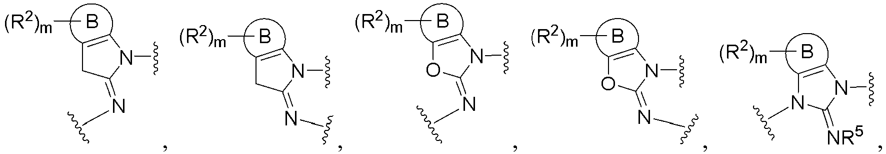 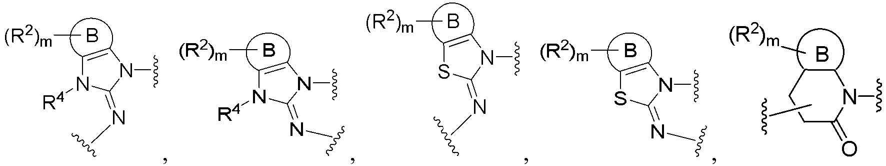 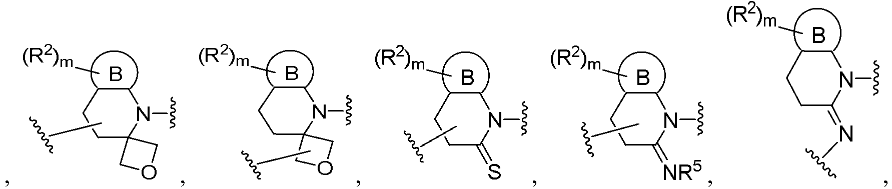 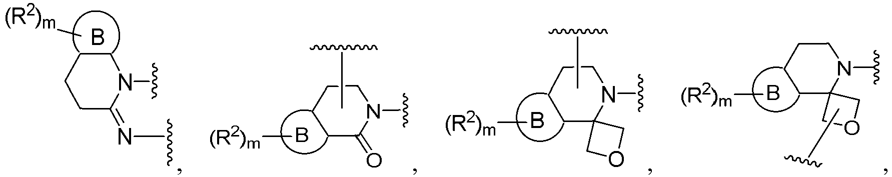 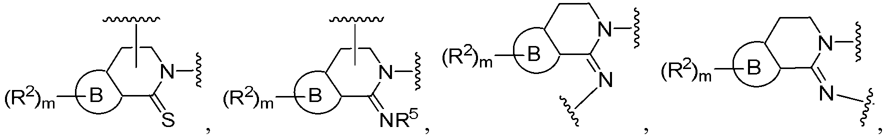 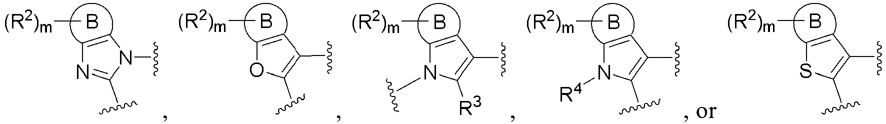 wherein
Ring B is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 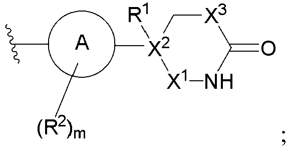 thereby forming a compound of formula **I-aa'**: 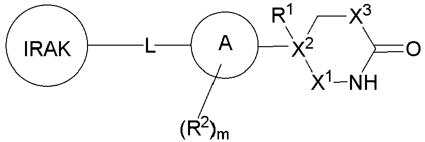 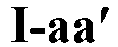 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 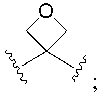
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R² is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A is a bi- or tricyclic ring selected from 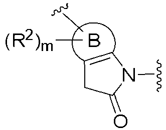 wherein Ring B is other than imidazo or benzo, 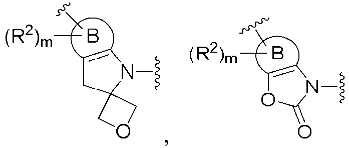 wherein Ring B is other than benzo, 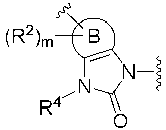 wherein Ring B is other than benzo, 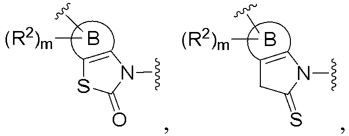 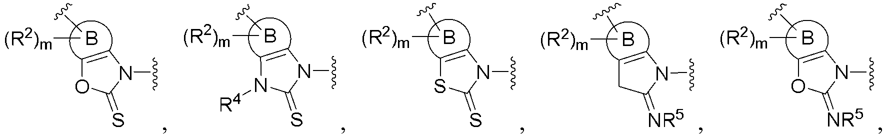 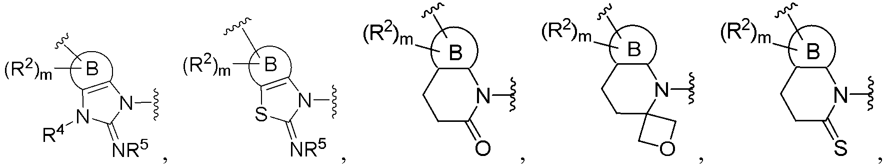 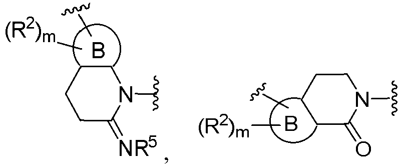 wherein Ring B is other than benzo, 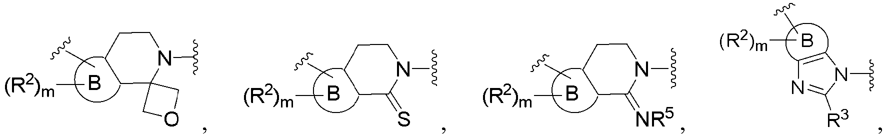 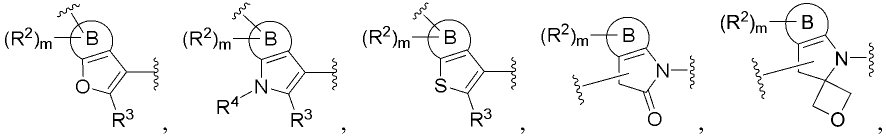 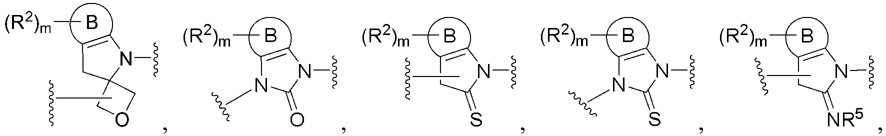 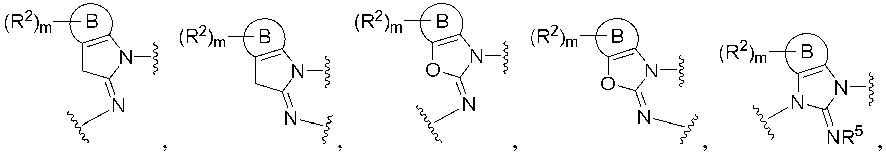 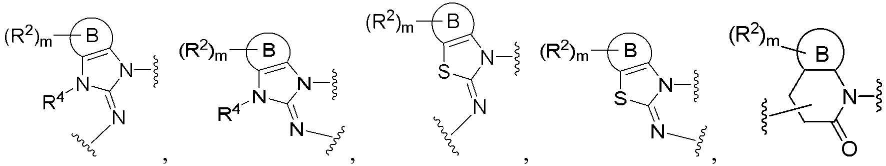 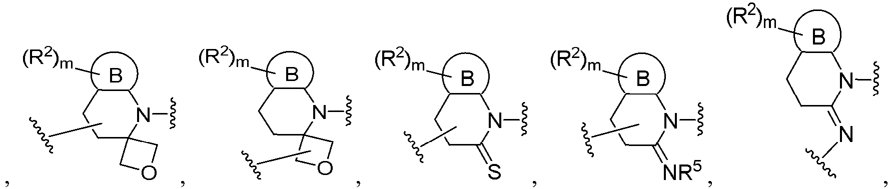 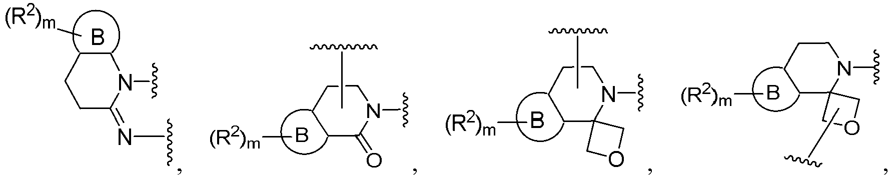 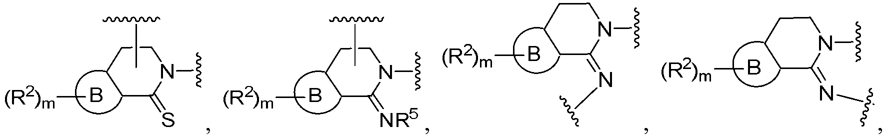 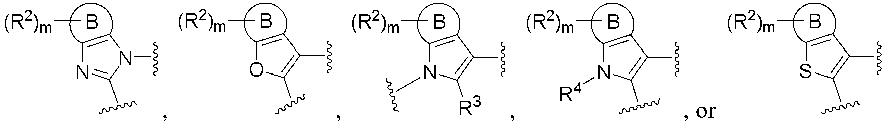 wherein
Ring B is a fused ring selected from 6-membered aryl containing 0-2 nitrogen atoms, 5 to 7-membered partially saturated carbocyclyl, 5 to 7-membered partially saturated heterocyclyl with 1-3 heteroatoms independently selected from boron, nitrogen, oxygen, silicon, or sulfur, or 5-membered heteroaryl with 1-3 heteroatoms independently selected from nitrogen, oxygen or sulfur;
R³ is selected from hydrogen, halogen, -OR, -N(R)₂, or -SR;
each R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala I-aa' above is provided as a compound of formula **I-aa"** or formula **I-aa‴**: 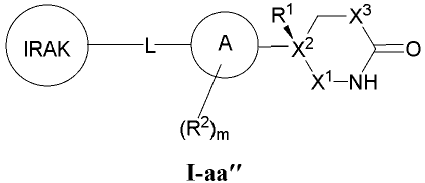 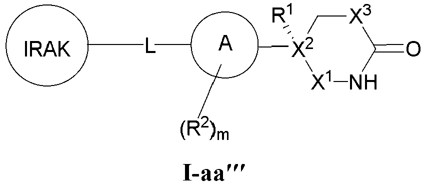 or a pharmaceutically acceptable salt thereof, wherein:
each of IRAK, Ring A, L, R¹, R², X¹, X², X³, and m is as defined above.

In certain embodiments, the present invention provides a compound of formula **I,** wherein LBM is an E3 ubiquitin ligase (cereblon) binding moiety 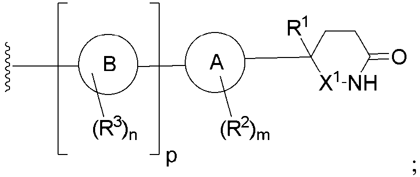 thereby forming a compound of formula **I-bb:** 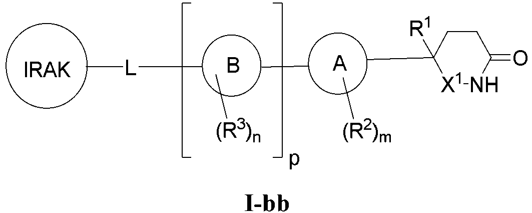 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 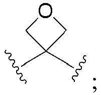
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 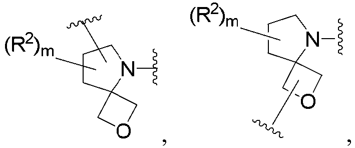 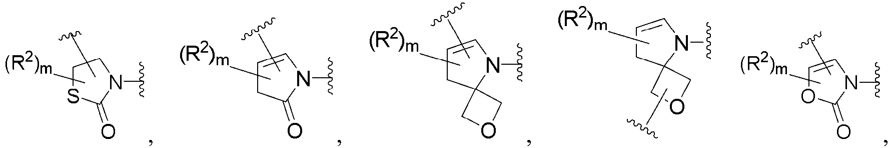 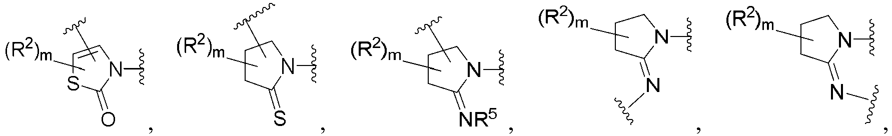 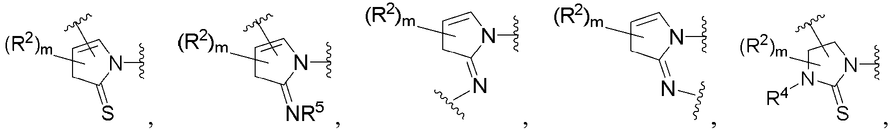 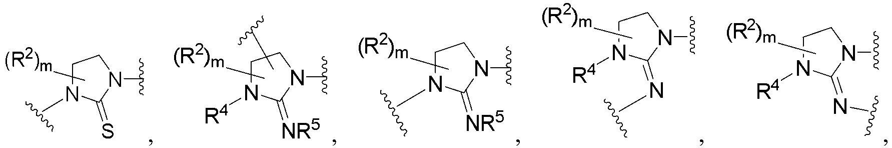 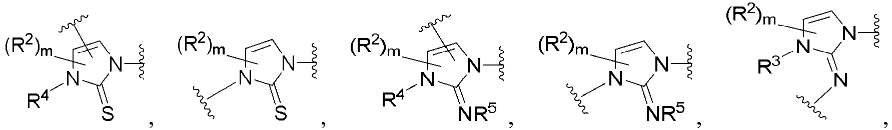 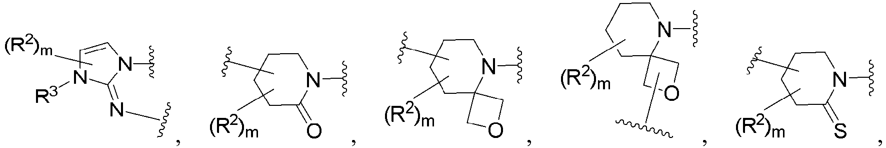 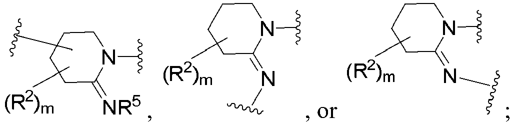
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1, wherein when p is 0, the bond connecting Ring A and Ring B is connected to 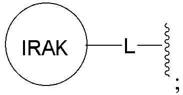 and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 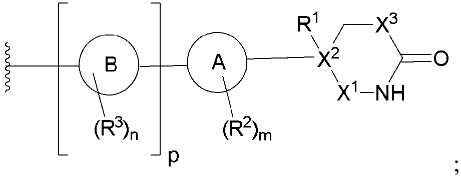 thereby forming a compound of formula **I-bb':** 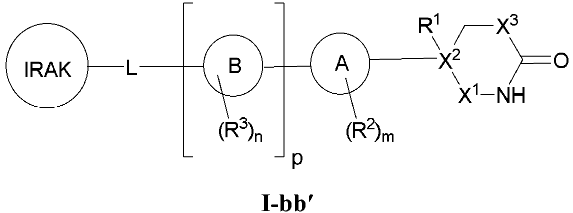 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 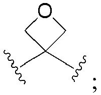
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 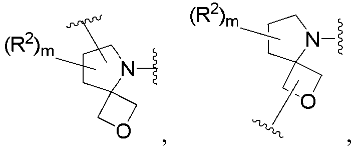 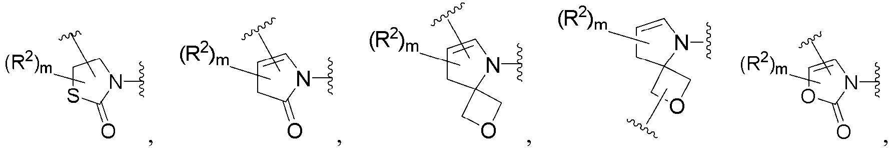 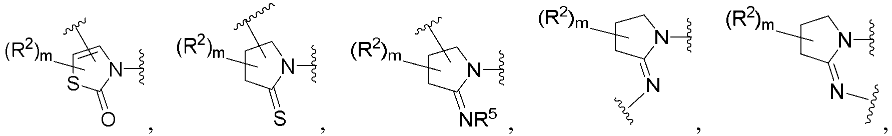 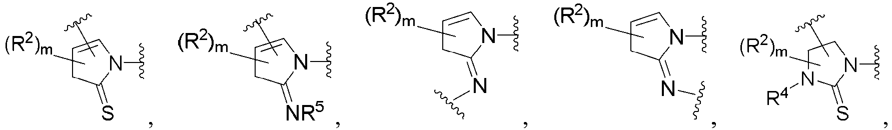 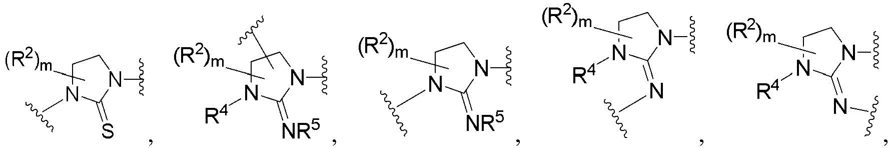 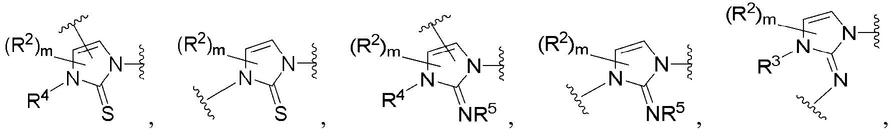 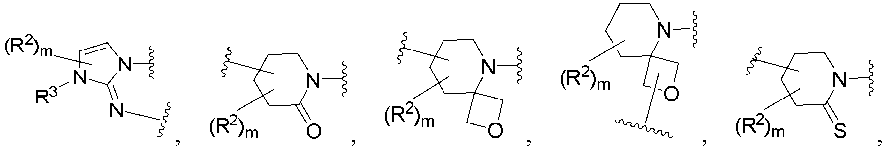 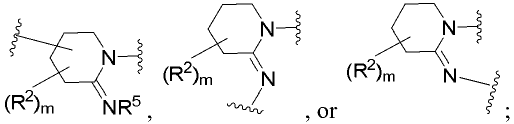
each R² is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1, wherein when p is 0, the bond connecting Ring A and Ring B is connected to 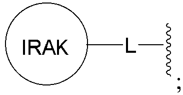 and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, the compound of formala **I-bb'** above is provided as a compound of formula **I-bb"** or formula **I-bb‴:** 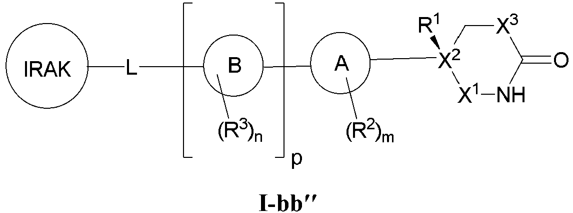 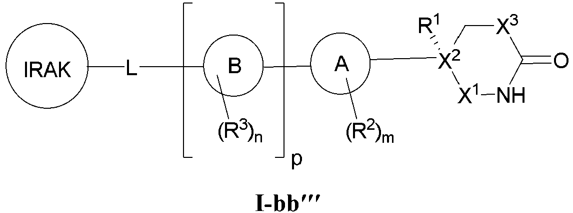 or a pharmaceutically acceptable salt thereof, wherein:
each of IRAK, Ring A, Ring B, L, R¹, R², R³, X¹, X², X³, p, and m is as defined above.

In certain embodiments, the present invention provides a compound of formula **I,** wherein LBM is an E3 ubiquitin ligase (cereblon) binding moiety 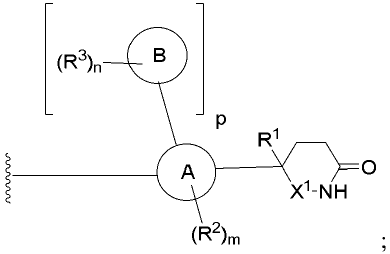 thereby forming a compound of formula I-cc: 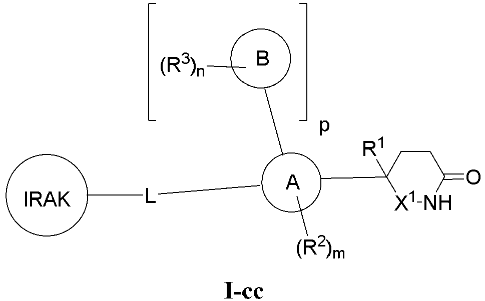 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 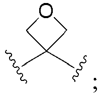
R¹ is hydrogen, deuterium, halogen, -CN, -OR, -SR, -S(O)R, -S(O)₂R, -NR₂, or an optionally substituted C₁₋₄ aliphatic;
Ring A is a mono- or bicyclic ring selected from 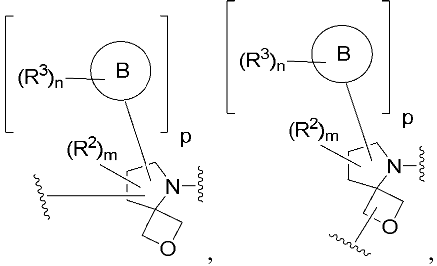 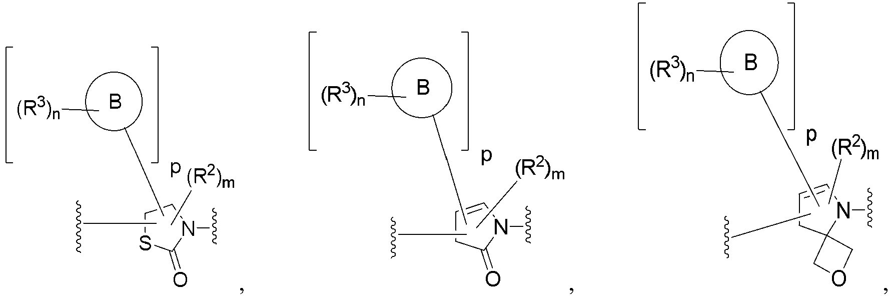 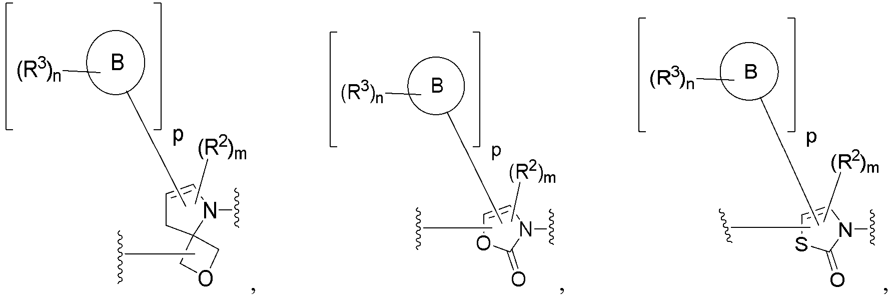 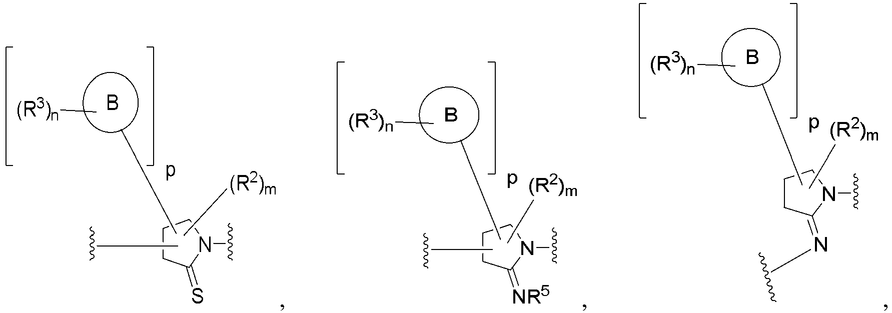 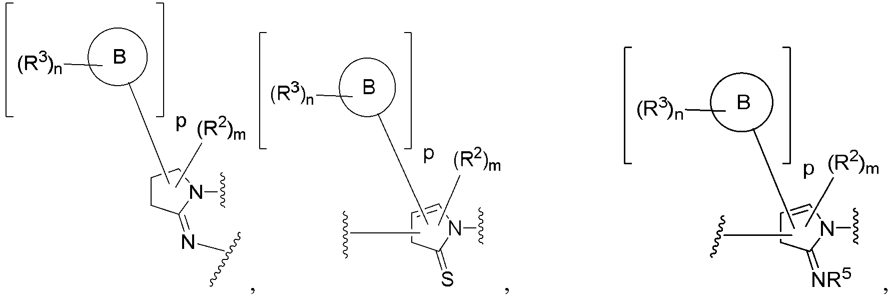 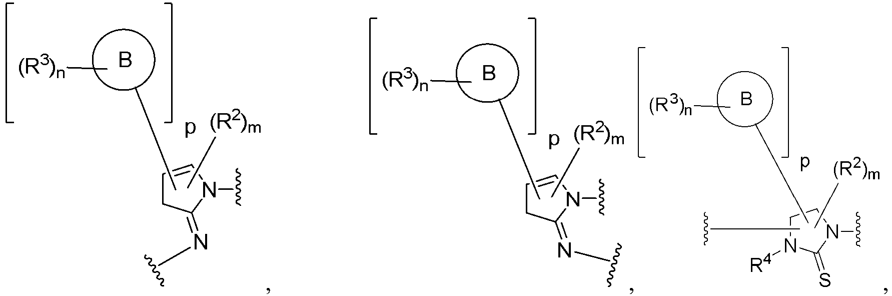 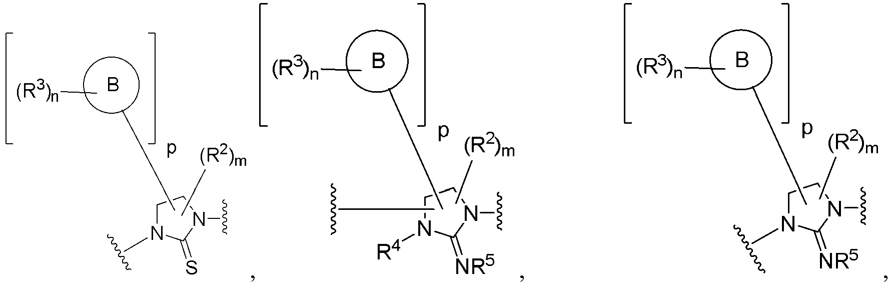 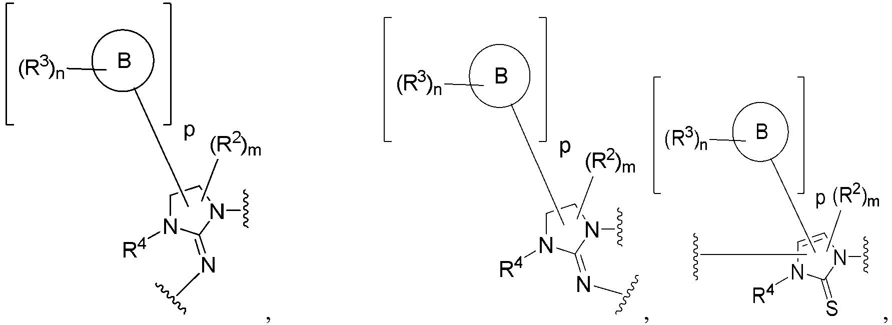 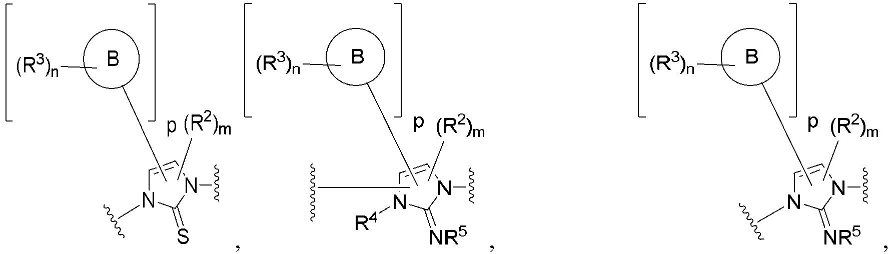 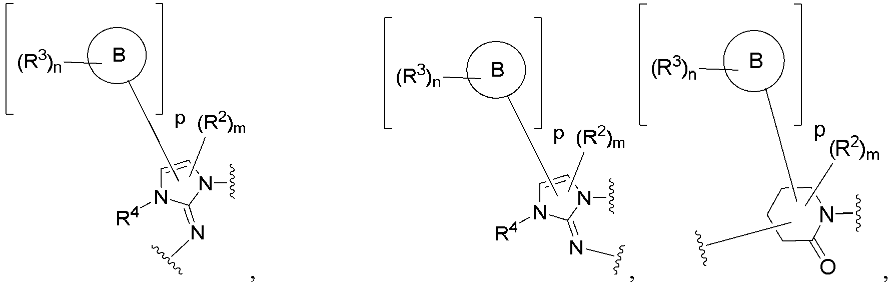 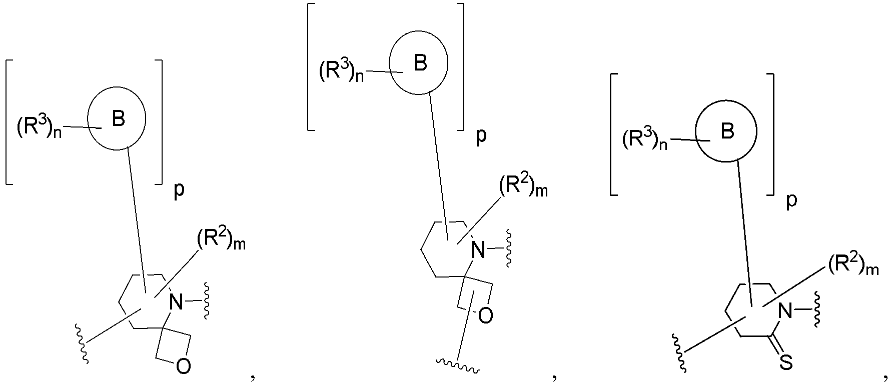 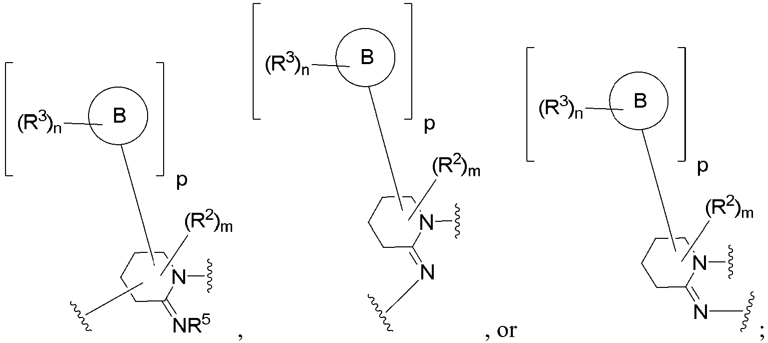
each R² is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In certain embodiments, the present invention provides a compound of formula **I,** wherein LBM is an E3 ubiquitin ligase (cereblon) binding moiety 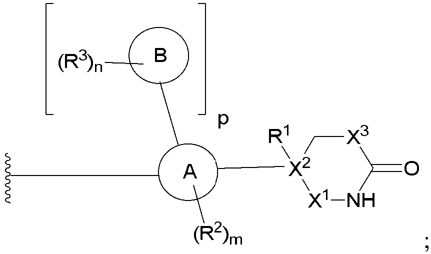 thereby forming a compound of formula I-cc': 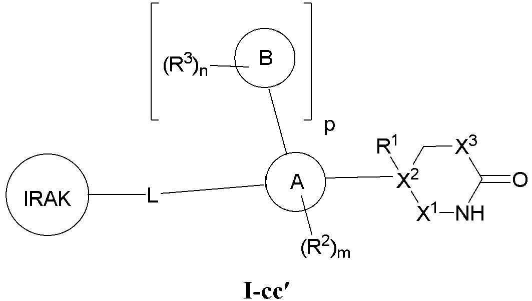 or a pharmaceutically acceptable salt thereof, wherein L and IRAK are as defined above and described in embodiments herein, and wherein:
X¹ is a bivalent moiety selected from a covalent bond, -CH₂-, -C(O)-, -C(S)-, or 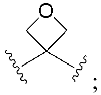
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
Ring A is a mono- or bicyclic ring selected from 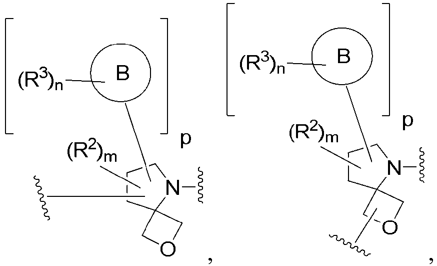 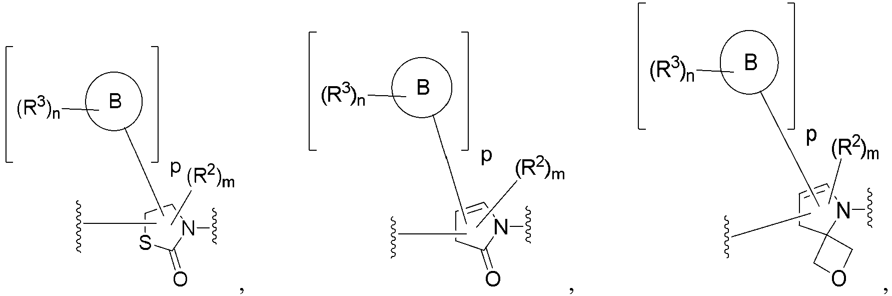 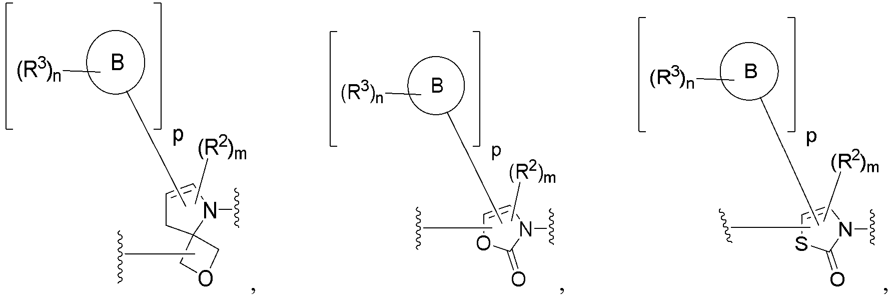 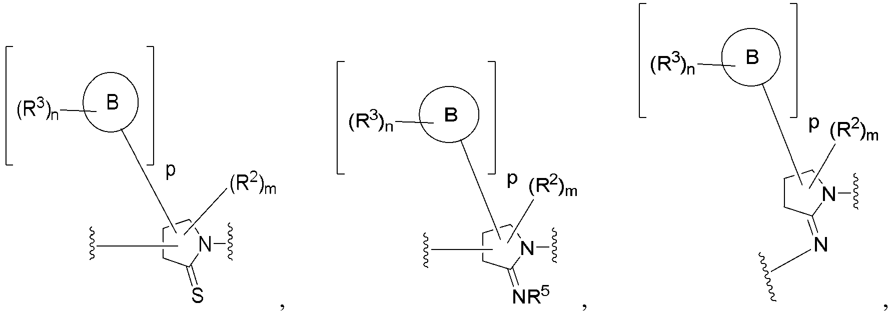 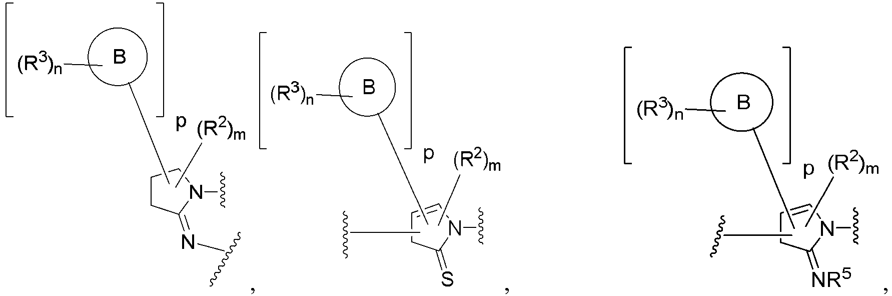 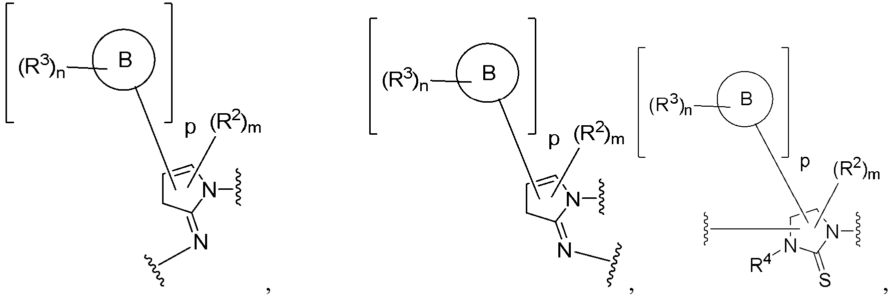 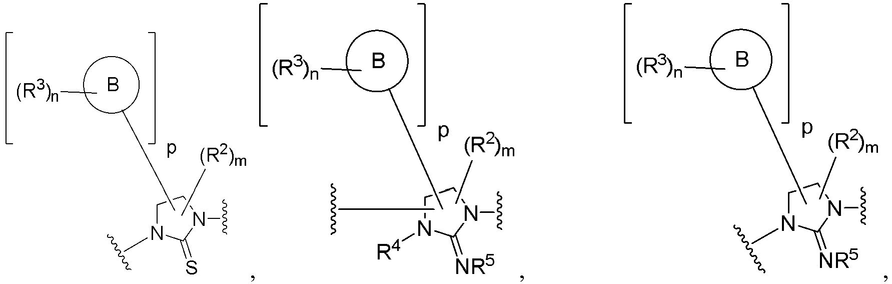 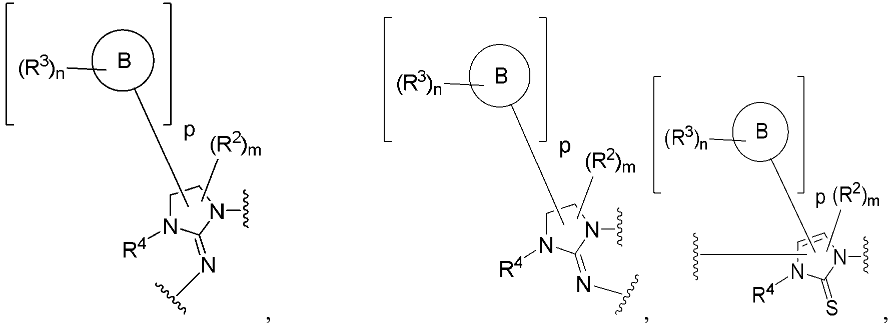 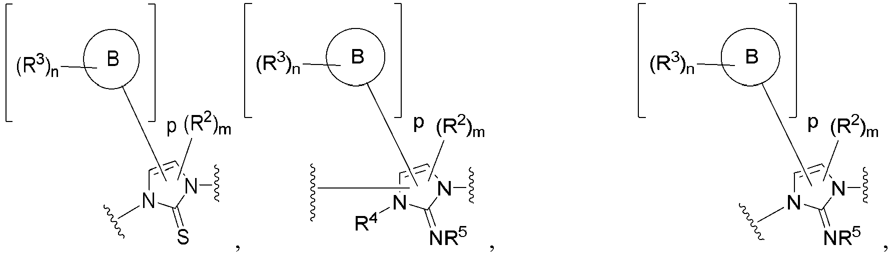 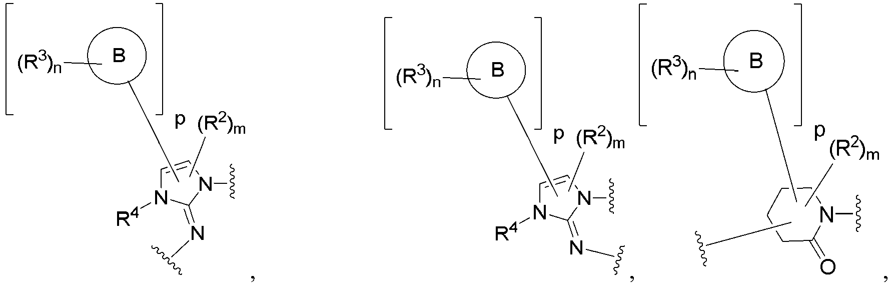 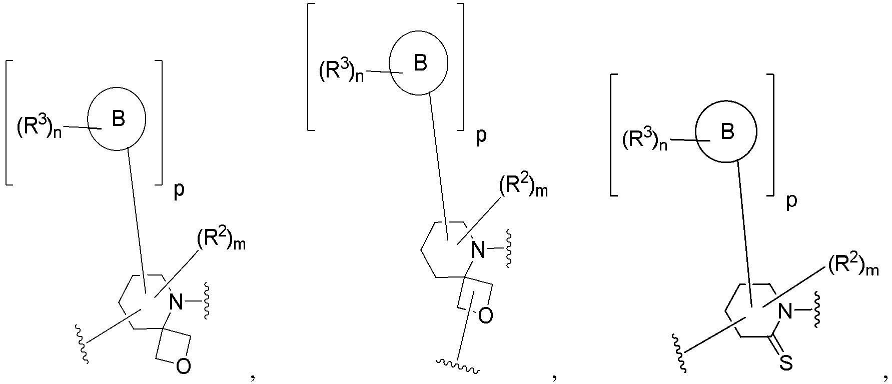 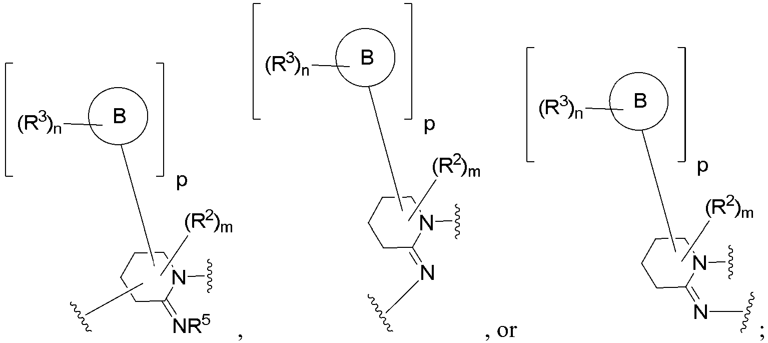
each R² is independently hydrogen, deuterium, -R⁶, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B is selected from a 6-membered aryl containing 0-2 nitrogen atoms or a 5-membered heteroaryl with 1-3 heteroatoms independently selected from nitrogen, oxygen or sulfur;
each of R³ and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
m is 0, 1, or 2;
n is 0, 1, 2, 3, or 4;
p is 0 or 1; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, a compound of formala **I-cc'** above is provided as a compound of formula **I-cc"** or formula **I-cc‴**: 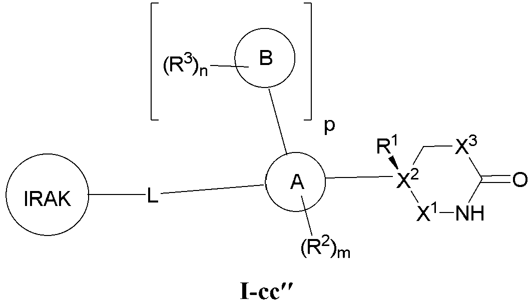 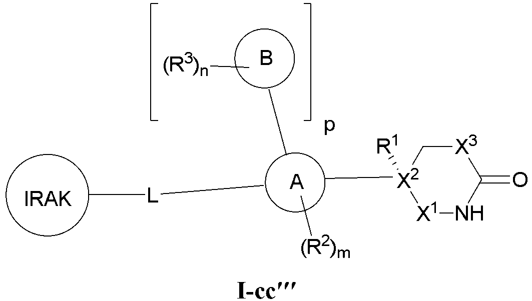 or a pharmaceutically acceptable salt thereof, wherein:
each of IRAK, Ring A, Ring B, L, R¹, R², R³, X¹, X², X³, p, n, and m is as defined above.

As defined above and described herein, LBM is a ligase binding moiety.

In some embodiments, LBM is an E3 ubiquitin ligase (cereblon) binding moiety 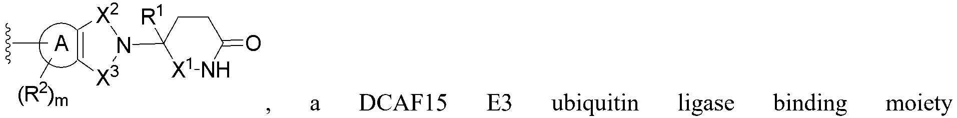 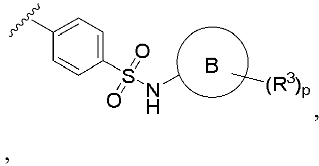 or a VHL E3 ubiquitin ligase binding moiety 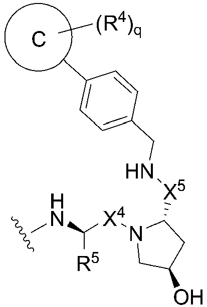
wherein each of X¹, X², and X³ is independently a bivalent moiety selected from a covalent bond, -CH₂-, -C(O)-, -C(S)-, or 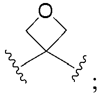
each of X⁴ and X⁵ is independently a bivalent moiety selected from -CH₂-, -C(O)-, -C(S)-, or 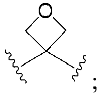
R¹ is hydrogen, deuterium, halogen, -CN, -OR, -SR, -S(O)R, -S(O)₂R, -NR₂, or an optionally substituted C₁₋₄ aliphatic;
each of R², R³, and R⁴ is independently hydrogen, -R⁶, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen or C₁₋₆ aliphatic;
each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur;
Ring B is selected from 6-membered aryl containing 0-2 nitrogen atoms or a 8-10 membered bicyclic heteroaryl having 1-5 heteroatoms independently selected from nitrogen, oxygen, or sulfur;
Ring C is a selected from 6-membered aryl containing 0-2 nitrogen atoms or a 5-membered heteroaryl with 1-3 heteroatoms independently selected from nitrogen, oxygen or sulfur;
m is 0, 1, 2, 3 or 4;
each of n is independently 1, 2, 3, 4, 5, 6, 7, 8, 9, or 10;
p is 0, 1, 2, 3 or 4;
q is 0, 1, 2, 3 or 4; and
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, LBM is selected from those depicted in **Table 1,** below.

As defined above and described herein, each of X¹, X², and X³ is independently a bivalent moiety selected from a covalent bond, -CH₂-, -C(O)-, -C(S)-, or

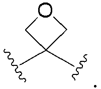

In some embodiments, X¹ is a covalent bond, -CH₂-, -C(O)-, -C(S)-, or

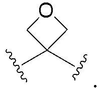

In some embodiments, X¹ is selected from those depicted in **Table 1,** below.

In some embodiments, X² is a covalent bond, -CH₂-, -C(O)-, -C(S)-, or

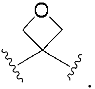

In some embodiments, X² is selected from those depicted in **Table 1,** below.

In some embodiments, X³ is a covalent bond, -CH₂-, -C(O)-, -C(S)-, or

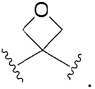

In some embodiments, X³ is selected from those depicted in **Table 1,** below.

As defined above and described herein, each of X⁴ and X⁵ is independently a bivalent moiety selected from -CH₂-, -C(O)-, -C(S)-, or

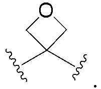

In some embodiments, X⁴ is -CH₂-, -C(O)-, -C(S)-, or

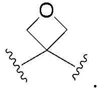

In some embodiments, X⁴ is selected from those depicted in **Table 1,** below.

In some embodiments, X⁵ is -CH₂-, -C(O)-, -C(S)-, or

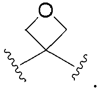

In some embodiments, X⁵ is selected from those depicted in **Table 1,** below.

As defined above and described herein, R¹ is hydrogen, deuterium, halogen, -CN, - OR, -SR, -S(O)R, -S(O)₂R, -NR₂, or an optionally substituted C₁₋₄ aliphatic.

In some embodiments, R¹ is hydrogen, deuterium, halogen, -CN, -OR, -SR, -S(O)R, -S(O)₂R, -NR₂, or an optionally substituted C₁₋₄ aliphatic.

In some embodiments, R¹ is selected from those depicted in **Table 1,** below.

As defined above and described herein, each of R², R³, and R⁴ is independently hydrogen, -R⁶, halogen, -CN, -NO₂, -OR, - SR, -NR₂, -S(O)₂R, -S(O)₂NR₂, -S(O)R, -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R.

In some embodiments, R² is hydrogen, -R⁶, halogen, -CN, -NO₂, -OR, - SR, -NR₂, -S(O)₂R, -S(O)₂NR₂, -S(O)R, -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R.

In some embodiments, R² is selected from those depicted in **Table 1,** below.

In some embodiments, R³ is hydrogen, -R⁶, halogen, -CN, -NO₂, -OR, - SR, -NR₂, -S(O)₂R, -S(O)₂NR₂, -S(O)R, -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R.

In some embodiments, R³ is methyl.

In some embodiments, R³ is selected from those depicted in **Table 1,** below.

In some embodiments, R⁴ is hydrogen, -R⁶, halogen, -CN, -NO₂, -OR, - SR, -NR₂, -S(O)₂R, -S(O)₂NR₂, -S(O)R, -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R.

In some embodiments, R⁴ is methyl.

In some embodiments, R⁴ is selected from those depicted in **Table 1,** below.

As defined above and described herein, R⁵ is hydrogen or C₁₋₆ aliphatic.

In some embodiments, R⁵ is t-butyl.

In some embodiments, R⁵ is selected from those depicted in **Table 1,** below.

As defined above and described herein, each R⁶ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur.

In some embodiments, R⁶ is an optionally substituted C₁₋₆ aliphatic group. In some embodiments, R⁶ is an optionally substituted phenyl. In some embodiments, R⁶ is an optionally substituted 4-7 membered saturated or partially unsaturated heterocyclic ring having 1-2 heteroatoms independently selected from nitrogen, oxygen, and sulfur. In some embodiments, R⁶ is an optionally substituted 5-6 membered heteroaryl ring having 1-4 heteroatoms independently selected from nitrogen, oxygen, and sulfur.

In some embodiments, R⁶ is selected from those depicted in **Table 1,** below.

As defined above and described herein, Ring A is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from nitrogen, oxygen or sulfur, or 5-membered heteroaryl with 1-3 heteroatoms independently selected from nitrogen, oxygen or sulfur.

In some embodiments Ring A is a fused 6-membered aryl containing 0-2 nitrogen atoms. In some embodiments Ring A is a fused 5 to 7-membered partially saturated carbocyclyl. In some embodiments Ring A is a fused 5 to 7-membered partially saturated heterocyclyl with 1-2 heteroatoms independently selected from nitrogen, oxygen or sulfur. In some embodiments Ring A is a fused 5-membered heteroaryl with 1-3 heteroatoms independently selected from nitrogen, oxygen or sulfur.

In some embodiments, Ring A is a fused phenyl.

In some embodiments, Ring A is selected from those depicted in **Table 1,** below.

As defined above and described herein, Ring B is selected from 6-membered aryl containing 0-2 nitrogen atoms or a 8-10 membered bicyclic heteroaryl having 1-5 heteroatoms independently selected from nitrogen, oxygen, or sulfur.

In some embodiments, Ring B is a 6-membered aryl containing 0-2 nitrogen atoms. In some embodiments, Ring B is a 8-10 membered bicyclic heteroaryl having 1-5 heteroatoms independently selected from nitrogen, oxygen, or sulfur.

In some embodiments, Ring B is

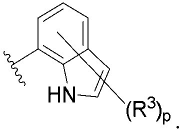

In some embodiments, Ring B is selected from those depicted in **Table 1,** below.

As defined above and described herein, Ring C is selected from 6-membered aryl containing 0-2 nitrogen atoms or a 5-membered heteroaryl with 1-3 heteroatoms independently selected from nitrogen, oxygen or sulfur.

In some embodiments, Ring C is a 6-membered aryl containing 0-2 nitrogen atoms. In some embodiments, Ring C is a 5-membered heteroaryl with 1-3 heteroatoms independently selected from nitrogen, oxygen or sulfur.

In some embodiments, Ring C is

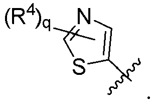

In some embodiments, Ring C is selected from those depicted in **Table 1,** below.

As defined above and described herein, m is 0, 1, 2, 3 or 4.

In some embodiments, m is 0. In some embodiments, m is 1. In some embodiments, m is 2. In some embodiments, m is 3. In some embodiments, m is 4.

In some embodiments, m is selected from those depicted in **Table 1,** below.

As defined above and described herein, each of n is independently 1, 2, 3, 4, 5, 6, 7, 8, 9, or 10.

In some embodiments, n is 0. In some embodiments, n is 1. In some embodiments, n is 2. In some embodiments, n is 3. In some embodiments, n is 4. In some embodiments, n is 5. In some embodiments, n is 6. In some embodiments, n is 7. In some embodiments, n is 8. In some embodiments, n is 9. In some embodiments, n is 10.

In some embodiments, n is selected from those depicted in **Table 1,** below.

As defined above and described herein, p is 0, 1, 2, 3 or 4.

In some embodiments, p is 0. In some embodiments, p is 1. In some embodiments, p is 2. In some embodiments, p is 3. In some embodiments, p is 4.

In some embodiments, p is selected from those depicted in **Table 1,** below.

As defined above and described herein, q is 0, 1, 2, 3 or 4.

In some embodiments, q is 0. In some embodiments, q is 1. In some embodiments, q is 2. In some embodiments, q is 3. In some embodiments, q is 4.

In some embodiments, q is selected from those depicted in **Table 1,** below.

As defined above and described herein, each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or: two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, R is hydrogen. In some embodiments, R is phenyl. In some embodiments, R is a 4-7 membered saturated or partially unsaturated heterocyclic having 1-2 heteroatoms independently selected from nitrogen, oxygen, and sulfur. In some embodiments, R is a 5-6 membered heteroaryl ring having 1-4 heteroatoms independently selected from nitrogen, oxygen, and sulfur. In some embodiments, two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, R is selected from those depicted in **Table 1,** below.

In some embodiments, LBM is a E3 Ubiquitin ligase (cereblon) binding moiety recited in Varfolomeev, E. et al., IAP Antagonists Induce Autoubiquitination of c-IAPs, NF-κB activation, and TNFα-Dependent Apoptosis, Cell, 2007, 131(4): 669-81, such as, for example: 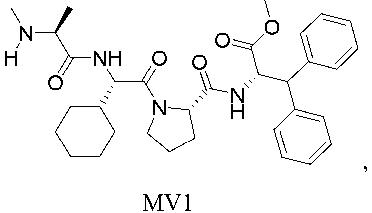 and 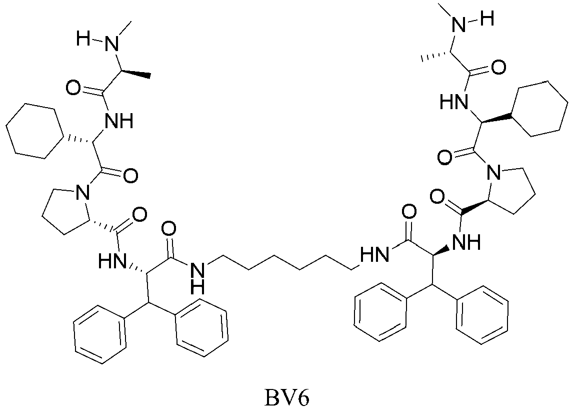 wherein 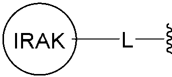 is attached to a modifiable carbon, oxygen, nitrogen or sulfur atom.

In some embodiments, R is selected from those depicted in **Table 1,** below.

In certain embodiments, the present invention provides a compound of formula **I,** wherein LBM is an E3 ubiquitin ligase (cereblon) binding moiety thereby forming a compound of formula **I-dd:** 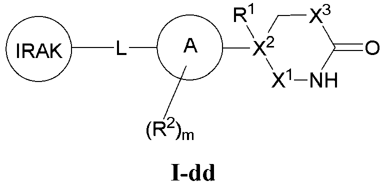 or a pharmaceutically acceptable salt thereof, wherein:
X¹ is a bivalent moiety selected from a covalent bond, -CH₂-, -C(O)-, -C(S)-, or 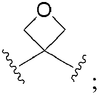
X² is a carbon atom or silicon atom;
X³ is a bivalent moiety selected from -CH₂- or -Si(R₂)-;
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R² is independently hydrogen, -R³, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R;
each R³ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a tricyclic ring selected from 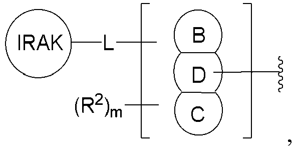 wherein
each of Ring B, Ring C, and Ring D is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur; and
m is 0, 1, 2, 3, 4, 5, 6, 7, 8, 9, 10, 11, 12, 13, 14, 15, or 16;
wherein L and IRAK are as described in embodiments herein.

In some embodiments, a compound of formala **I-dd** above is provided as a compound of formula **I-dd'** or formula **I-dd":** 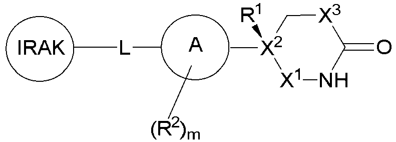 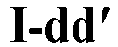 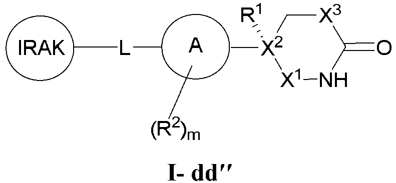 or a pharmaceutically acceptable salt thereof, wherein:
each of IRAK, Ring A, L, R¹, R², X¹, X², X³, and m is as defined above.

In certain embodiments, the present invention provides a compound of formula **I,** wherein LBM is an E3 ubiquitin ligase (cereblon) binding moiety 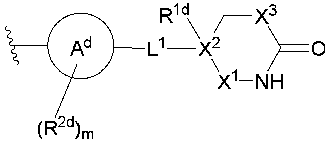 thereby forming a compound of formula **I-dd-1:** 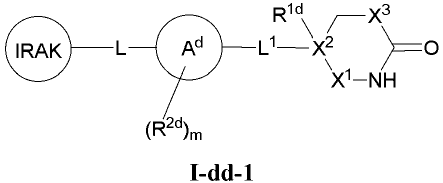 or a pharmaceutically acceptable salt thereof, wherein:
X¹ is a bivalent moiety selected from a covalent bond, -CH₂-, -C(O)-, -C(S)-, or 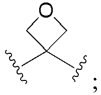
X² is a carbon atom or silicon atom;
X³ is a bivalent moiety selected from -CH₂- or -Si(R₂)-;
R^{1d} is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R^{2d} is independently hydrogen, -R^{3d}, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R;
each R^{3d} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A^{d} is a tricyclic ring selected from 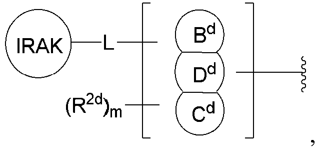 wherein
each of Ring B^{d}, Ring C^{d}, and Ring D^{d} is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur; and
m is 0, 1, 2, 3, 4, 5, 6, 7, 8, 9, 10, 11, 12, 13, 14, 15, or 16;
wherein L and IRAK are as described in embodiments herein.

In some embodiments, a compound of formala **I-dd-1** above is provided as a compound of formula **I-dd-1'** or formula **I-dd-1":** 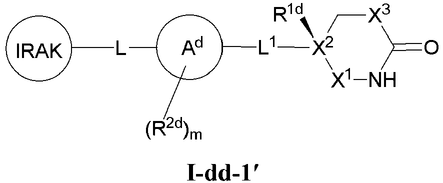 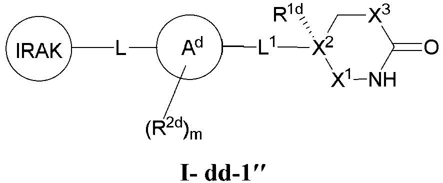 or a pharmaceutically acceptable salt thereof, wherein:
each of IRAK, Ring A^{d}, L, R^{1d}, R^{2d}, X¹, X², X³, and m is as defined above.

In certain embodiments, the present invention provides a compound of Formula **I,** wherein LBM is an E3 ubiquitin ligase (cereblon) binding moiety thereby forming a compound of formula **I-dd-2** or **I-dd-3:** 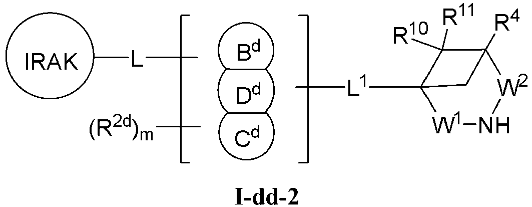 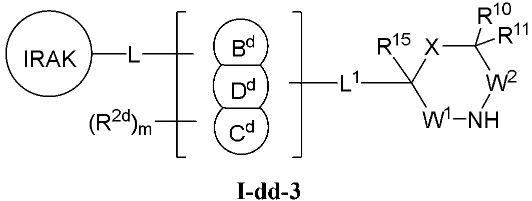 or a pharmaceutically acceptable salt thereof, wherein L and SMARCA are as defined above and described in embodiments herein, and wherein:
each R^{2d} is independently hydrogen, deuterium, -R^{3d}, halogen, -CN, -NO₂, -OR, -SR, -NR₂, - SiR₃, -S(O)₂R, -S(O)₂NR₂, -S(O)R, -C(O)R, -C(O)OR, -C(O)NR₂, -C(O)N(R)OR, - C(R)₂N(R)C(O)R, -C(R)₂N(R)C(O)N(R)₂, -OC(O)R, -OC(O)N(R)₂, -OP(O)R₂, - OP(O)(OR)₂, -OP(O)(OR)NR₂, -OP(O)(NR₂)₂-, - N(R)C(O)OR, -N(R)C(O)R, -N(R)C(O)NR₂, -N(R)S(O)₂R, -NP(O)R₂, -N(R)P(O)(OR)₂, - N(R)P(O)(OR)NR₂, -N(R)P(O)(NR₂)₂, or -N(R)S(O)₂R;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R^{3d} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each of Ring B^{d}, Ring C^{d}, and Ring D^{d} is independently a fused ring selected from 6-membered aryl, 6-membered heteroaryl containing 1-4 heteroatoms independently selected from nitrogen, oxygen, or sulfur, 5 to 7-membered saturated or partially unsaturated carbocyclyl, 5 to 7-membered saturated or partially unsaturated heterocyclyl with 1-3 heteroatoms independently selected from boron, nitrogen, oxygen, silicon, or sulfur, or 5-membered heteroaryl with 1-4 heteroatoms independently selected from nitrogen, oxygen or sulfur;
L¹ is a covalent bond or a C₁₋₃ bivalent straight or branched saturated or unsaturated hydrocarbon chain wherein 1-2 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
m is 0, 1, 2, 3, 4, 5, 6, 7, 8, 9, 10, 11, 12, 13, 14, 15, or 16; and
R⁴, R¹⁰, R¹¹, R¹⁵, W¹, W², and X is as defined in WO 2019/099868, the entirety of each of which is herein incorporated by reference.

Where a point of attachment of 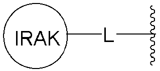 is depicted on Ring B^{d}, Ring C^{d}, or Ring D^{d}, it is intended, and one of ordinary skill in the art would appreciate, that the point of attachment of 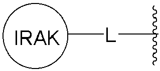 may be on any available carbon or nitrogen atom on Ring B^{d}, Ring C^{d}, or Ring D^{d}, including the ring to which Ring B^{d} or Ring D^{d} is fused to Ring C^{d}.

Where a point of attachment of -(R^{2d})ₘ is depicted on Ring B^{d}, Ring C^{d}, or Ring D^{d}, it is intended, and one of ordinary skill in the art would appreciate, that the point of attachment of - (R²)ₘ may be at any available carbon or nitrogen atom on Ring B^{d}, Ring C^{d}, or Ring D^{d} including the carbon atom to which Ring B^{d} or Ring D^{d} are fused to Ring C^{d}.

Where a point of attachment of 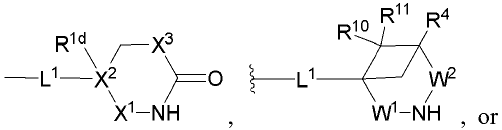 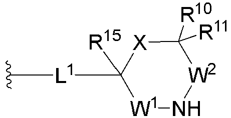 is depicted on Ring B^{d}, Ring C^{d}, or Ring D^{d}, it is intended, and one of ordinary skill in the art would appreciate, that the point of attachment of 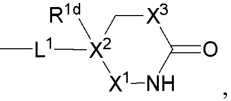 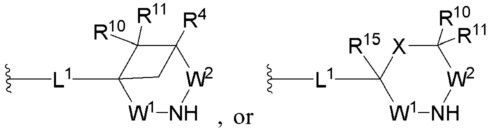 may be on any available carbon or nitrogen atom on Ring B^{d}, Ring C^{d}, or Ring D^{d}, including the carbon atom to which Ring B^{d} or Ring D^{d} are fused to Ring C^{d}.

In certain embodiments, the present invention provides a compound of formula **I,** wherein LBM is an E3 ubiquitin ligase (cereblon) binding moiety thereby forming a compound of formula I-ee: 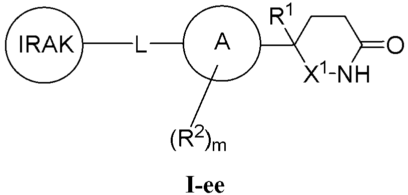 or a pharmaceutically acceptable salt thereof, wherein:
X¹ is a bivalent moiety selected from a covalent bond, -CH₂-, -C(O)-, -C(S)-, or 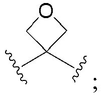
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R² is independently hydrogen, -R³, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R;
each R³ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a tricyclic ring selected from 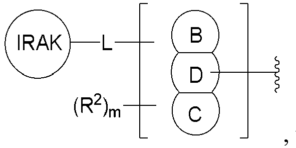 wherein
each of Ring B, Ring C, and Ring D is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur; and
m is 0, 1, 2, 3, 4, 5, 6, 7, 8, 9, 10, 11, 12, 13, 14, 15, or 16;
wherein L and IRAK are as described in embodiments herein.

In some embodiments, a compound of formala **I-ee** above is provided as a compound of formula **I-ee'** or formula **I-ee"**: 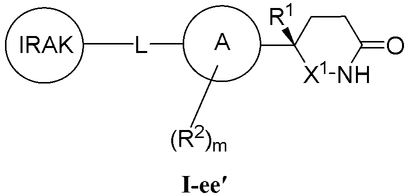 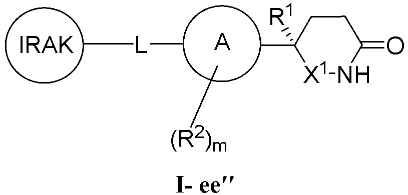 or a pharmaceutically acceptable salt thereof, wherein:
each of IRAK, Ring A, L, R¹, R², X¹, and m is as defined above.

In certain embodiments, the present invention provides a compound of formula **I,** wherein LBM is an E3 ubiquitin ligase (cereblon) binding moiety thereby forming a compound of formula **I-ff:** 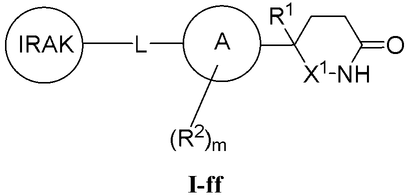 or a pharmaceutically acceptable salt thereof, wherein:
X¹ is a bivalent moiety selected from a covalent bond, -CH₂-, -C(O)-, -C(S)-, or 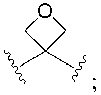
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R² is independently hydrogen, -R³, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R;
each R³ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a tricyclic ring selected from 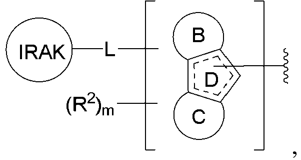 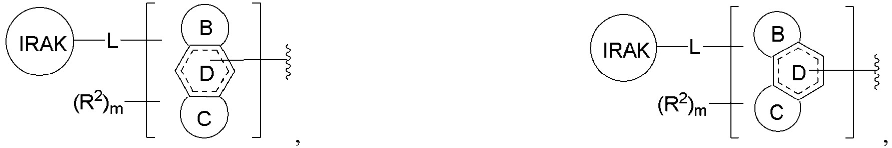 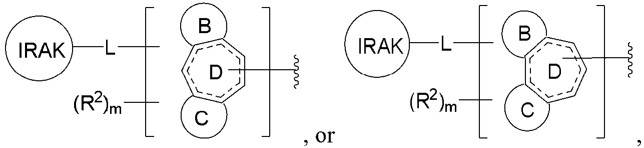 wherein
each of Ring B and Ring C is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen or sulfur, or 5-membered heteroaryl with 1-3 heteroatoms independently selected from nitrogen, oxygen or sulfur;
Ring D is a fused ring selected from aryl containing 0-3 nitrogens, saturated or partially unsaturated carbocyclyl, saturated or partially unsaturated heterocyclyl ring with 1-2 heteroatoms independently selected from nitrogen, oxygen, silicon, or sulfur, or heteroaryl with 1-3 heteroatoms independently selected from nitrogen, oxygen or sulfur;
- - - is a single or double bond;
m is 0, 1, 2, 3, 4, 5, 6, 7, 8, 9, 10, 11, 12, 13, 14, 15, or 16;
wherein L and IRAK are as described in embodiments herein.

In some embodiments, a compound of formala **I-ff** above is provided as a compound of formula **I-ff'** or formula **I-ff"**: 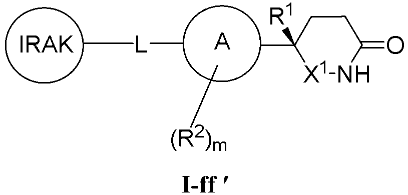 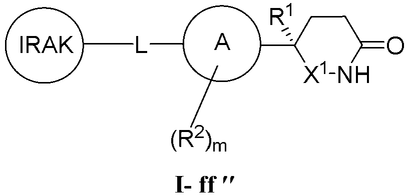 or a pharmaceutically acceptable salt thereof, wherein:
each of IRAK, Ring A, L, R¹, R², X¹, and m is as defined above.

In certain embodiments, the present invention provides a compound of formula **I,** wherein LBM is an E3 ubiquitin ligase (cereblon) binding moiety thereby forming a compound of formula **I-gg:** 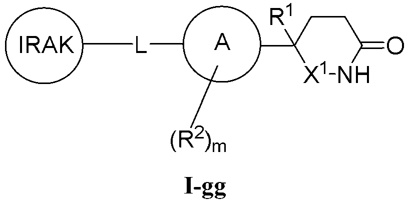 or a pharmaceutically acceptable salt thereof, wherein:
X¹ is a bivalent moiety selected from a covalent bond, -CH₂-, -C(O)-, -C(S)-, or 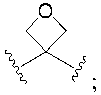
R¹ is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R² is independently hydrogen, -R³, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R;
each R³ is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A is a tricyclic ring selected from 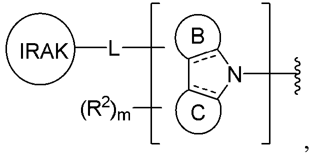 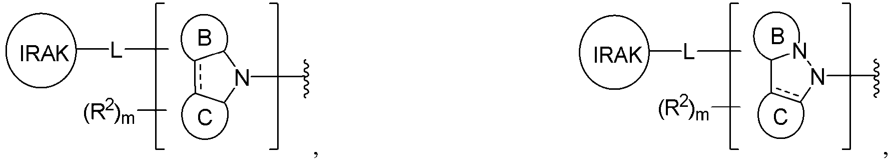 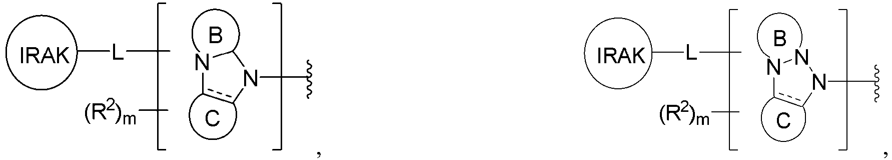 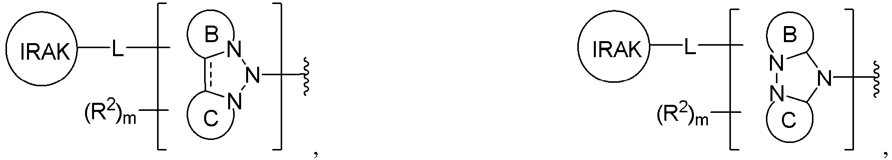 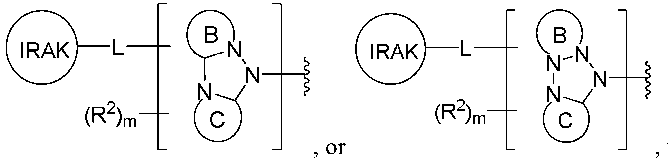 wherein
each of Ring B and Ring C is independently a fused ring selected from 6-membered aryl containing 0-2 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur;
- - - is a single or double bond;
m is 0, 1, 2, 3, 4, 5, 6, 7, or 8;
wherein L and IRAK are as described in embodiments herein.

In some embodiments, a compound of formala I-gg above is provided as a compound of formula **I-gg'** or formula **I-gg"**: 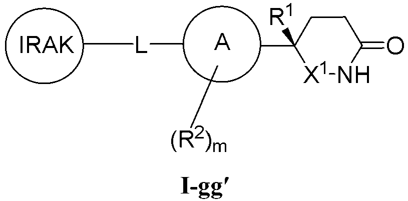 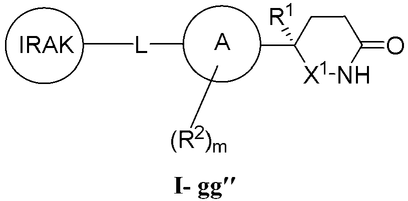 or a pharmaceutically acceptable salt thereof, wherein:
each of IRAK, Ring A, L, R¹, R², X¹, and m is as defined above.

As defined above and described herein, X¹ is a bivalent moiety selected from a covalent bond, -CH₂-, -C(O)-, -C(S)-, or

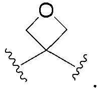

In some embodiments, X¹ is a covalent bond. In some embodiments, X¹ is -CH₂-. In some embodiments, X¹ is -C(O)-. In some embodiments, X¹ is -C(S)-. In some embodiments, X¹ is

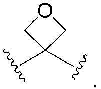

In some embodiments, X¹ is selected from those depicted in **Table 1,** below.

As defined above and described herein, X² is a carbon atom or silicon atom.

In some embodiments, X² is a carbon atom. In some embodiments, X² is a silicon atom.

In some embodiments, X² is selected from those depicted in **Table 1,** below.

As defined above and described herein, X³ is a bivalent moiety selected from -CH₂- or -Si(R₂)-.

In some embodiments, X³ is -CH₂-. In some embodiments, X² is -Si(R₂)-.

In some embodiments, X³ is selected from those depicted in **Table 1,** below.

As defined above and described herein, R¹ or R^{1d} is hydrogen, deuterium, halogen, - CN, -OR, -SR, -S(O)R, -S(O)₂R, -NR₂, -Si(R₃), or an optionally substituted C₁₋₄ aliphatic.

In some embodiments, R¹ or R^{1d} is hydrogen. In some embodiments, R¹ or R^{1d} is deuterium. In some embodiments, R¹ or R^{1d} is halogen. In some embodiments, R¹ or R^{1d} is -CN. In some embodiments, R¹ or R^{1d} is -OR. In some embodiments, R¹ or R^{1d} is -SR. In some embodiments, R¹ or R^{1d} is -S(O)R. In some embodiments, R¹ or R^{1d} is -S(O)₂R. In some embodiments, R¹ or R^{1d} is -NR₂. In some embodiments, R¹ or R^{1d} is -Si(R₃). In some embodiments, R¹ or R^{1d} is an optionally substituted C₁₋₄ aliphatic.

In some embodiments, R¹ or R^{1d} is selected from those depicted in **Table 1,** below.

As defined above and described herein, each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or: two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, R is hydrogen. In some embodiments, R is optionally substituted C₁₋₆ aliphatic. In some embodiments, R is optionally substituted phenyl. In some embodiments, R is optionally substituted 4-7 membered saturated or partially unsaturated heterocyclic having 1-2 heteroatoms independently selected from nitrogen, oxygen, and sulfur. In some embodiments, R is optionally substituted 5-6 membered heteroaryl ring having 1-4 heteroatoms independently selected from nitrogen, oxygen, and sulfur. In some embodiments, two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur.

In some embodiments, R is selected from those depicted in **Table 1,** below.

As defined above and described herein, each R² or R^{2d} is independently hydrogen, -R³, -R^{3d}, halogen, -CN, -NO₂, -OR, -SR, -N(R)₂, - Si(R₃), -S(O)₂R, -S(O)₂N(R)₂, -S(O)R, -C(O)R, -C(O)OR, -C(O)N(R)₂, -C(O)N(R)OR, - C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R.

In some embodiments, R² or R^{2d} is hydrogen. In some embodiments, R² is -R³. In some embodiments, R^{2d} is -R^{3d}. In some embodiments, R² or R^{2d} is halogen. In some embodiments, R² or R^{2d} is -CN. In some embodiments, R² or R^{2d} is -NO₂. In some embodiments, R² or R^{2d} is -OR. In some embodiments, R² or R^{2d} is -SR. In some embodiments, R² is -NR₂. In some embodiments, R² or R^{2d} is -Si(R₃). In some embodiments, R² or R^{2d} is -S(O)₂R. In some embodiments, R² or R^{2d} is -S(O)₂NR₂. In some embodiments, R² or R^{2d} is -S(O)R. In some embodiments, R² or R^{2d} is -C(O)R. In some embodiments, R² or R^{2d} is -C(O)OR. In some embodiments, R² or R^{2d} is -C(O)NR₂. In some embodiments, R² or R^{2d} is -C(O)N(R)OR. In some embodiments, R² or R^{2d} is -C(R)₂N(R)C(O)R. In some embodiments, R² or R^{2d} is - C(R)₂N(R)C(O)N(R)₂. In some embodiments, R² or R^{2d} is -OC(O)R. In some embodiments, R² or R^{2d} is -OC(O)NR₂. In some embodiments, R² or R^{2d} is -N(R)C(O)OR. In some embodiments, R² or R^{2d} is -N(R)C(O)R. In some embodiments, R² or R^{2d} is -N(R)C(O)NR₂. In some embodiments, R² or R^{2d} is -N(R)S(O)₂R.

In some embodiments, R² is selected from those depicted in **Table 1,** below.

As defined above and described herein, each R³ or R^{3d} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur.

In some embodiments, R³ or R^{3d} is an optionally substituted C₁₋₆ aliphatic. In some embodiments, R³ or R^{3d} is an optionally substituted phenyl. In some embodiments, R³ or R^{3d} is an optionally substituted 4-7 membered saturated or partially unsaturated heterocyclic ring having 1-2 heteroatoms independently selected from nitrogen, oxygen, and sulfur. In some embodiments, R³ or R^{3d} is an optionally substituted 5-6 membered heteroaryl ring having 1-4 heteroatoms independently selected from nitrogen, oxygen, and sulfur.

In some embodiments, R³ or R^{3d} is selected from those depicted in **Table 1,** below.

As defined above and described herein, Ring A is a tricyclic ring selected from

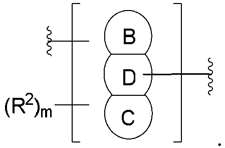

In some embodiments, Ring A is

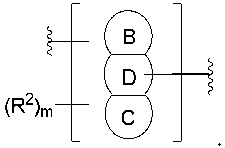

In some embodiments, Ring A is selected from those depicted in **Table 1**, below.

As defined above and described herein, Ring A^{d} is a tricyclic ring selected from

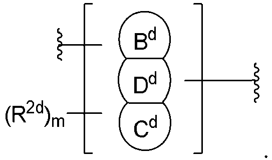

In some embodiments, Ring A^{d} is

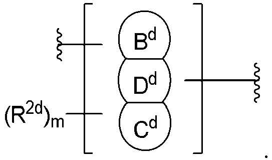

In some embodiments, Ring A^{d} is selected from those depicted in **Table 1,** below.

As defined above and described herein, each of Ring B, Ring C, and Ring D or Ring B^{d}, Ring C^{d}, and Ring D^{d} is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur.

In some embodiments, each Ring B, Ring C, and Ring D or Ring B^{d}, Ring C^{d}, and Ring D^{d} is independently a 6-membered aryl containing 0-2 nitrogen atoms. In some embodiments, each Ring B, Ring C, and Ring D or Ring B^{d}, Ring C^{d}, and Ring D^{d} is independently a 5 to 7-membered saturated or partially unsaturated carbocyclyl. In some embodiments, each Ring B, Ring C, and Ring D or Ring B^{d}, Ring C^{d}, and Ring D^{d} is independently a 5 to 7-membered saturated or partially unsaturated heterocyclyl with 1-2 heteroatoms independently selected from boron, nitrogen, oxygen, silicon, or sulfur. In some embodiments, each Ring B, Ring C, and Ring D or Ring B^{d}, Ring C^{d}, and Ring D^{d} is independently a 5-membered heteroaryl with 1-3 heteroatoms independently selected from nitrogen, oxygen or sulfur.

In some embodiments, Ring B, Ring C, and Ring D or Ring B^{d}, Ring C^{d}, and Ring D^{d} is selected from those depicted in **Table 1,** below.

As defined above and described herein, Ring A is a tricyclic ring selected from

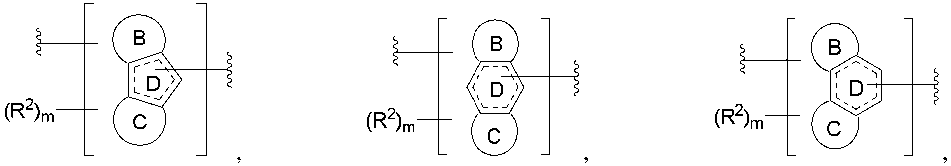

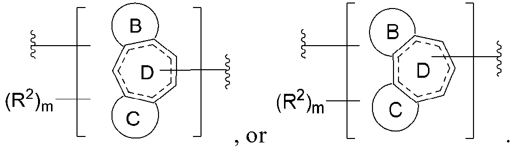

In some embodiments, Ring A is 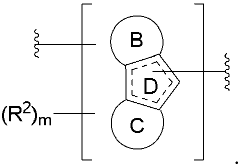 In some embodiments, Ring A is 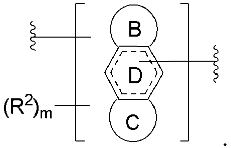 In some embodiment, Ring A is 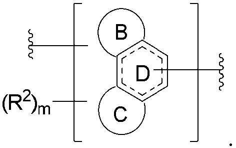 In some embodiments, Ring A is 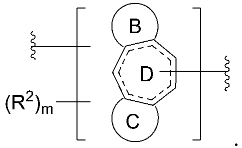 In some embodiments, Ring A is

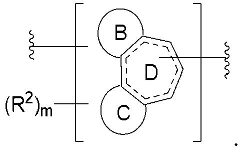

In some embodiments, Ring A is selected from those depicted in **Table 1,** below.

As defined above and described herein, Ring D or Ring D^{d} is a fused ring selected from aryl containing 0-3 nitrogens, saturated or partially unsaturated carbocyclyl, saturated or partially unsaturated heterocyclyl ring with 1-2 heteroatoms independently selected from nitrogen, oxygen, silicon, or sulfur, or heteroaryl with 1-3 heteroatoms independently selected from nitrogen, oxygen or sulfur.

In some embodiments, Ring D or Ring D^{d} is an aryl containing 0-2 nitrogen atoms. In some embodiments, Ring D or Ring D^{d} is a saturated or partially unsaturated carbocyclyl. In some embodiments, each Ring D or Ring D^{d} is a saturated or partially unsaturated heterocyclyl with 1-2 heteroatoms independently selected from nitrogen, oxygen, silicon, or sulfur. In some embodiments, Ring D or Ring D^{d} is a heteroaryl with 1-3 heteroatoms independently selected from nitrogen, oxygen or sulfur.

In some embodiments, Ring D is 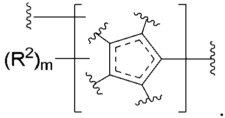 In some embodiments, Ring D is 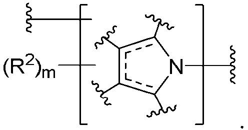 In some embodiments, Ring D is 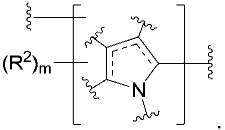 In some embodiments, Ring D is 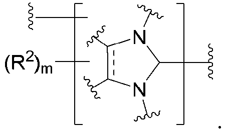 In some embodiments, Ring D is 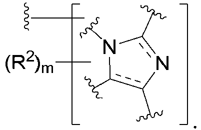 In some embodiments, Ring D is 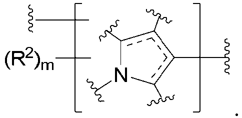 In some embodiments, Ring D is 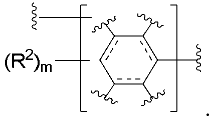 In some embodiments, Ring D is 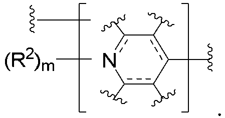 In some embodiments, Ring D is 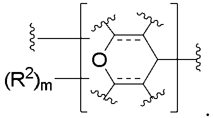 In some embodiments, Ring D is 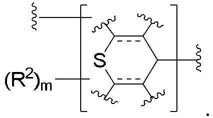 In some embodiments, Ring D is 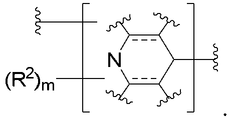 In some embodiments, Ring D is 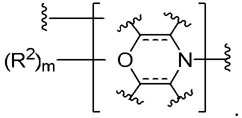 In some embodiments, Ring D is 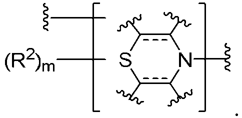 In some embodiments, Ring D is 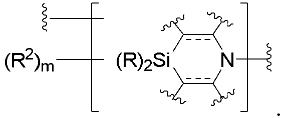 In some embodiments, Ring D is 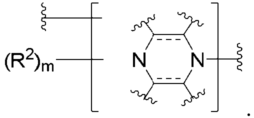 In some embodiments, Ring D is 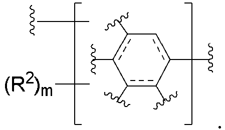 In some embodiments, Ring D is 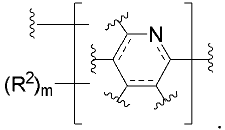 In some embodiments, Ring D is 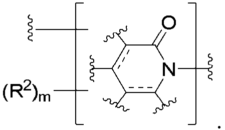 In some embodiments, Ring D is 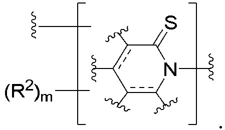 In some embodiments, Ring D is

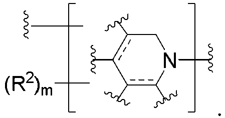

In some embodiments, Ring D is 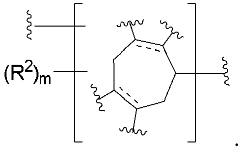 In some embodiments, Ring D is 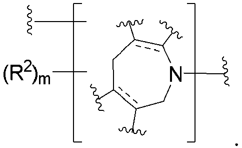 In some embodiments, Ring D is 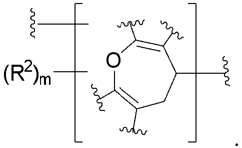 In some embodiments, Ring D is 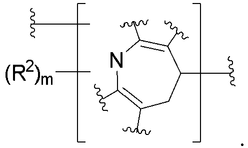 In some embodiments, Ring D is 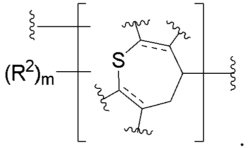 In some embodiments, Ring D is 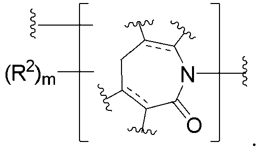 In some embodiments, Ring D is 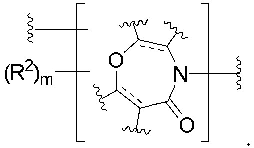 In some embodiments, Ring D is 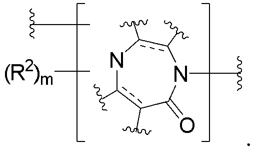 In some embodiments, Ring D is 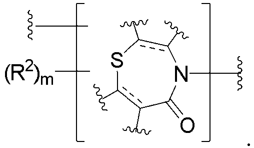 In some embodiments, Ring D is 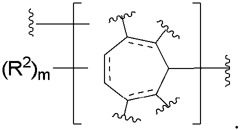 In some embodiments, Ring D is 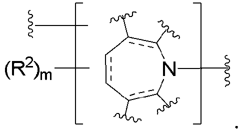 In some embodiments, Ring D is 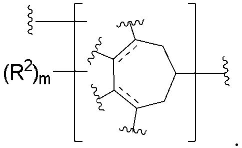 In some embodiments, Ring D is 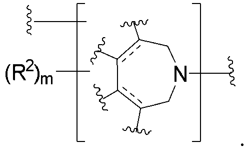 In some embodiments, Ring D is 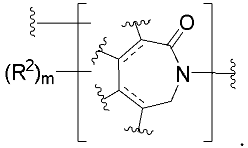 In some embodiments, Ring D is 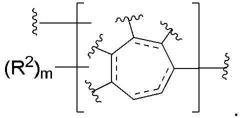 In some embodiments, Ring D is 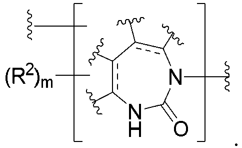 In some embodiments, Ring D is

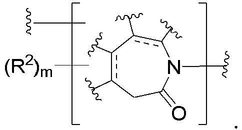

In some embodiments, Ring D is selected from those depicted in **Table 1,** below.

As defined above and described herein, m is 0, 1, 2, 3, 4, 5, 6, 7, 8, 9, 10, 11, 12, 13, 14, 15, or 16.

In some embodiments, m is 0. In some embodiments, m is 1. In some embodiments, m is 2. In some embodiments, m is 3. In some embodiments, m is 4. In some embodiments, m is 5. In some embodiments, m is 6. In some embodiments, m is 7. In some embodiments, m is 8. In some embodiments, m is 9. In some embodiments, m is 10. In some embodiments, m is 11. In some embodiments, m is 12. In some embodiments, m is 13. In some embodiments, m is 14. In some embodiments, m is 15. In some embodiments, m is 16.

In some embodiments, m is selected from those depicted in **Table 1,** below.

As defined above and described herein, Ring A is a tricyclic ring selected from 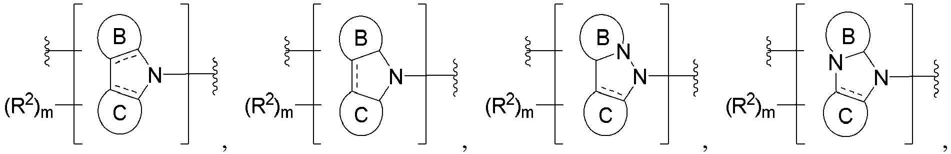 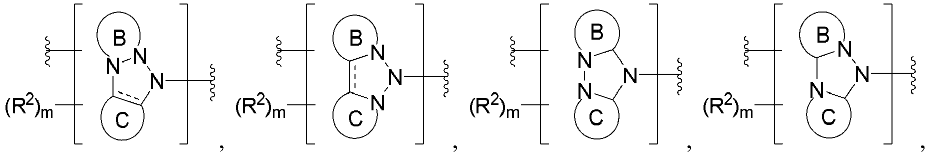 or

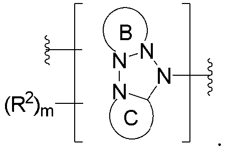

In some embodiments, Ring A is 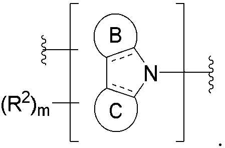 In some embodiments, Ring A is 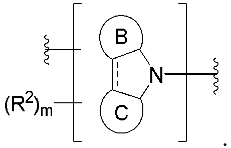 In some embodiment, Ring A is 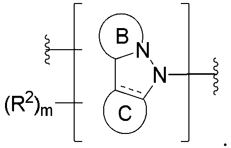 In some embodiments, Ring A is 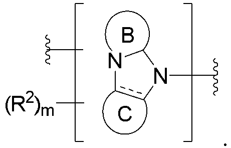 In some embodiments, Ring A is 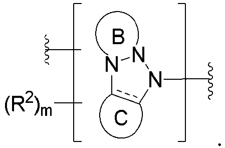 In some embodiments, Ring A is 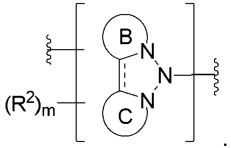 In some embodiments, Ring A is 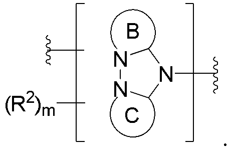 In some embodiments, Ring A is 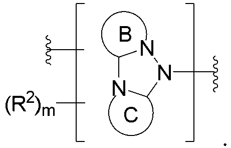 In some embodiments, Ring A is

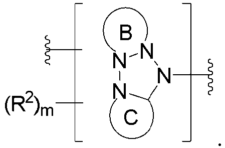

In some embodiments, Ring A is selected from those depicted in **Table 1,** below.

As defined above and described herein, each Ring B and Ring C or Ring B^{d} and Ring C^{d} is independently a fused ring selected from 6-membered aryl containing 0-2 nitrogen atoms, 5 to 7-membered saturated or partially unsaturated carbocyclyl, 5 to 7-membered saturated or partially unsaturated heterocyclyl with 1-3 heteroatoms independently selected from boron, nitrogen, oxygen, silicon, or sulfur, or 5-membered heteroaryl with 1-3 heteroatoms independently selected from nitrogen, oxygen or sulfur.

In some embodiments, each Ring B and Ring C or Ring B^{d} and Ring C^{d} is independently a 6-membered aryl containing 0-2 nitrogen atoms. In some embodiments, each Ring B and Ring C or Ring B^{d} and Ring C^{d} is independently a 5 to 7-membered saturated or partially unsaturated carbocyclyl. In some embodiments, each Ring B and Ring C or Ring B^{d} and Ring C^{d} is independently a 5 to 7-membered saturated or partially unsaturated heterocyclyl with 1-3 heteroatoms independently selected from boron, nitrogen, oxygen, silicon, or sulfur. In some embodiments, each Ring B and Ring C or Ring B^{d} and Ring C^{d} is independently a 5-membered heteroaryl with 1-3 heteroatoms independently selected from nitrogen, oxygen or sulfur.

In some embodiments, each Ring B and Ring C is independently 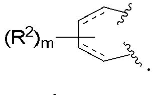 In some embodiments, each Ring B and Ring C is independently 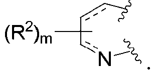 In some embodiments, each Ring B and Ring C is independently 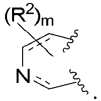 In some embodiments, each Ring B and Ring C is independently 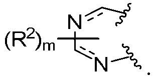 In some embodiments, Ring B and Ring C is independently

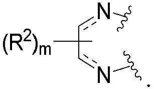

In some embodiments, Ring B and Ring C is independently is 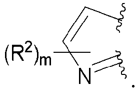 In some embodiments, Ring B and Ring C is independently 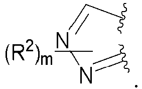 In some embodiments, Ring B and Ring C is independently 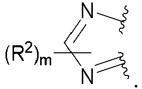 In some embodiments, Ring B and Ring C is independently 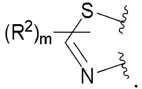 In some embodiments, Ring B and Ring C is independently 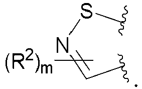 In some embodiments, Ring B and Ring C is independently 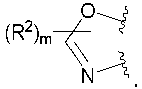 In some embodiments, Ring B and Ring C is independently

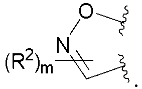

In some embodiments, Ring B and Ring C is independently 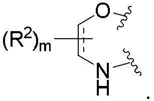 In some embodiments, Ring B and Ring C is independently 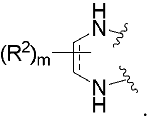 In some embodiments, B and Ring C is independently 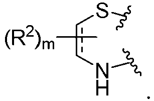 In some embodiments, Ring B and Ring C is independently 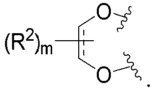 In some embodiments, Ring B and Ring C is independently

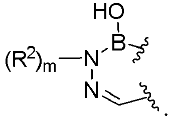

In some embodiments, Ring B and Ring C is independently selected from those depicted in **Table 1,** below.

As defined above and described herein, - - - is a single or double bond

In some embodiments, - - - is a single bond. In some embodiments, - - - is a double bond.

As defined above and described herein, m is 0, 1, 2, 3, 4, 5, 6, 7, or 8.

In some embodiments, m is 0. In some embodiments, m is 1. In some embodiments, m is 2. In some embodiments, m is 3. In some embodiments, m is 4. In some embodiments, m is 5. In some embodiments, m is 6. In some embodiments, m is 7. In some embodiments, m is 8.

In some embodiments, m is selected from those depicted in **Table 1,** below.

In some embodiments, In some embodiments, LBM is 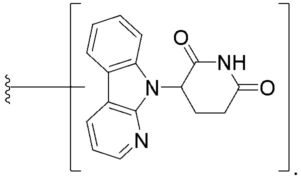 In some embodiments, LBM is 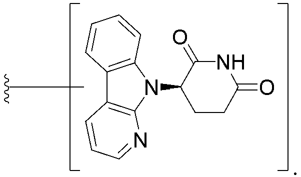 In some embodiments, LBM is 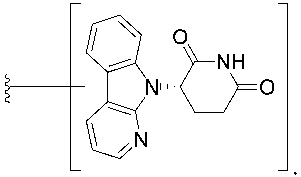 In some embodiments, In some embodiments, LBM is 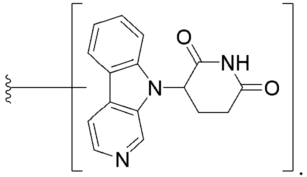 In some embodiments, LBM is 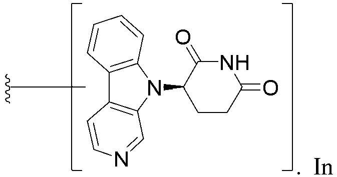 some embodiments, LBM is

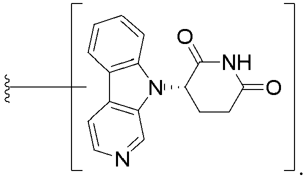

In some embodiments, LBM is 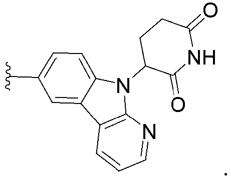 In some embodiments, LBM is 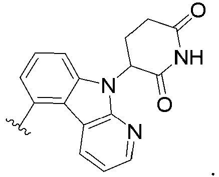 In some embodiments, LBM is 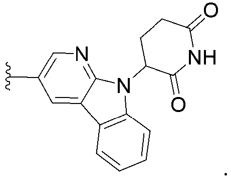 In some embodiments, LBM is 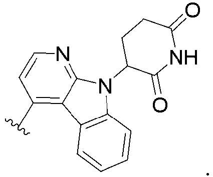 In some embodiments, LBM is 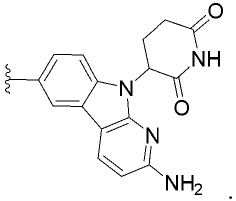 In some embodiments, LBM is 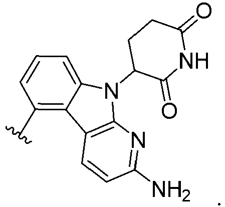 In some embodiments, LBM is 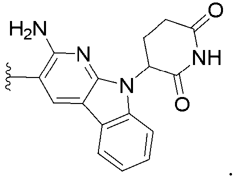 In some embodiments, LBM is

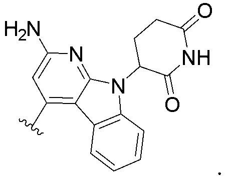

In some embodiments, LBM is selected from those in **Table 1** below.

In some embodiments, the present invention provides the compound of formula **II**, wherein LBM is 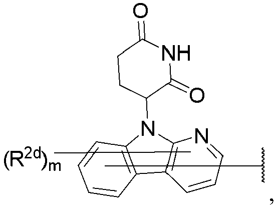 thereby forming a compound of formula **II-r:** 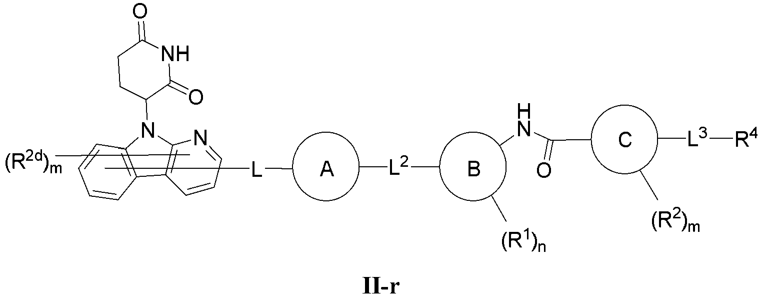 or a pharmaceutically acceptable salt thereof, wherein each of L, L², L³, Ring A, Ring B, Ring C, R¹, R², R^{2d}, R⁴, n, and each m is as defined above and described in embodiments herein, both singly and in combination.

In some embodiments, the present invention provides the compound of formula **II,** wherein LBM is 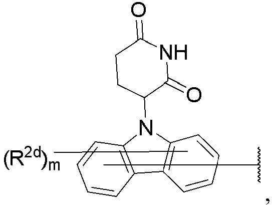 L² is a covalent bond, Ring A is cyclohexyl, Ring B is pyrazolyl, Ring C is oxazolyl, and Ring D is pyridyl thereby forming a compound of formula **II-s:** 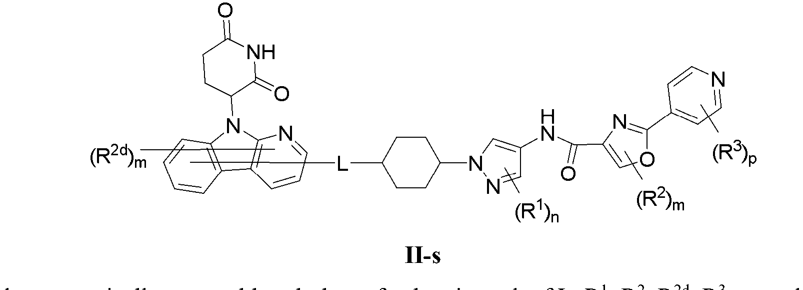 or a pharmaceutically acceptable salt thereot, wherein each of L, R¹, R², R^{2d}, R³, n, each m, and p is as defined above and described in embodiments herein, both singly and in combination.

In certain embodiments, the present invention provides the compound of formula **II-d,** wherein LBM is 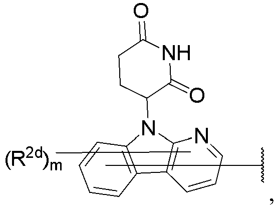 L² is a covalent bond, Ring A is cyclohexyl, and Ring B is pyrazolyl as shown, thereby forming a compound of formula **II-d-7:** 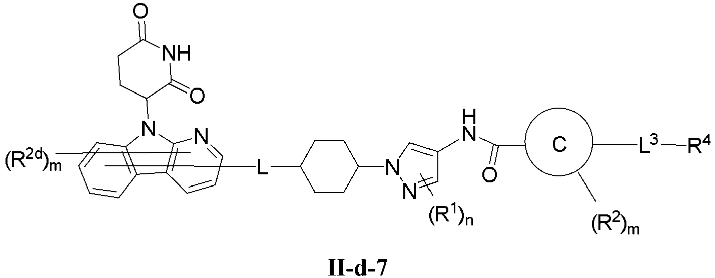 or a pharmaceutically acceptable salt thereof, wherein each of L, L³, Ring C, R¹, R², R^{2d}, R⁴, n, and each m is as defined above and described in embodiments herein, both singly and in combination.

In certain embodiments, the present invention provides the compound of formula **II-e,** wherein LBM is 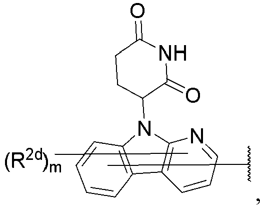 L² is a covalent bond, Ring A is cyclohexyl, Ring C is pyrazolo[1,5-a]pyrimidine, and Ring B is pyrazolyl as shown, thereby forming a compound of formula **II-e-7:** 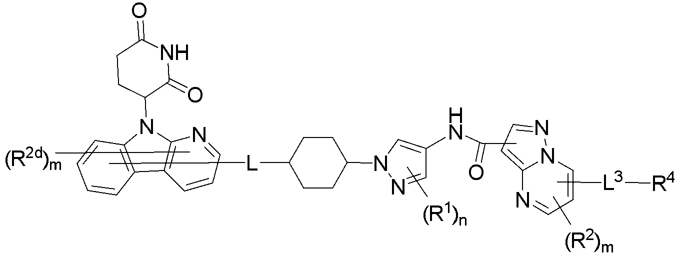 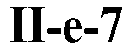 or a pharmaceutically acceptable salt thereof, wherein each of L, L³, R¹, R², R^{2d}, R⁴, n, and each m is as defined above and described in embodiments herein, both singly and in combination.

In certain embodiments, the present invention provides a compound of formula **I,** wherein LBM is VHL binding moiety 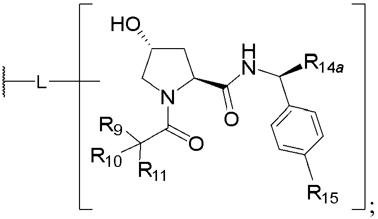 thereby forming a compound of formula **I-hh:** 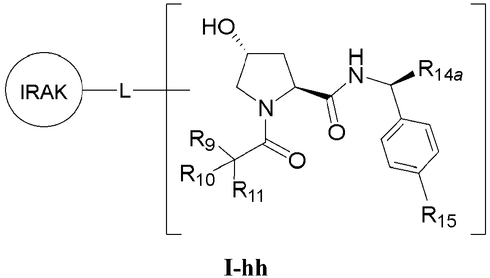 or a pharmaceutically acceptable salt thereof, wherein L and IRAK are as defined above and described in embodiments herein, and wherein each of the variables R₉, R₁₀, R₁₁, R₁₄ₐ, and R₁₅ is as described and defined in WO 2017/030814, WO 2016/118666, and US 2017/0327469, the entirety of each of which is herein incorporated by reference.

In certain embodiments, the present invention provides a compound of formula **I,** wherein LBM is VHL binding moiety 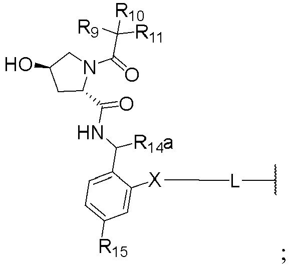 thereby forming a compound of formula **I-ii:** 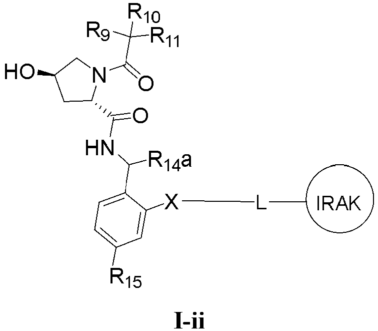 or a pharmaceutically acceptable salt thereof, wherein L and IRAK are as defined above and described in embodiments herein, and wherein each of the variables X, R₉, R₁₀, R₁₁, R₁₄ₐ, and R₁₅ is as described and defined in WO 2017/030814, WO 2016/118666, and US 2017/0327469, the entirety of each of which is herein incorporated by reference.

In certain embodiments, the present invention provides a compound of formula **I,** wherein LBM is an IAP binding moiety 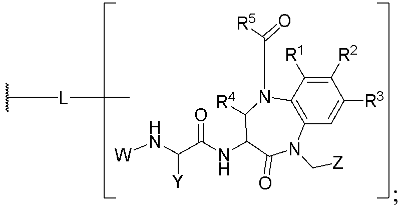 thereby forming a compound of formula I-jj: 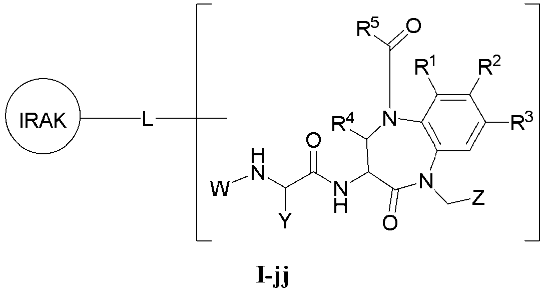 or a pharmaceutically acceptable salt thereof, wherein L and IRAK are as defined above and described in embodiments herein, and wherein each of the variables W, Y, Z, R¹, R², R³, R⁴, and R⁵ is as described and defined in WO 2014/044622, US 2015/0225449, WO 2015/071393, and US 2016/0272596, the entirety of each of which is herein incorporated by reference.

In certain embodiments, the present invention provides a compound of formula **I,** wherein LBM is IAP binding moiety 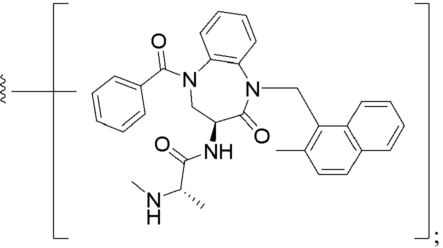 thereby forming a compound of formula **I-kk**: 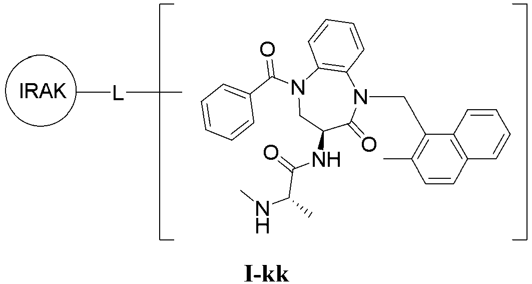 or a pharmaceutically acceptable salt thereof, wherein L and IRAK are as defined above and described in embodiments herein, as described and defined in Kester R.F., et al., J. Med. Chem. 2013, 56(20), 7788-7803, the entirety of which is herein incorporated by reference.

In certain embodiments, the present invention provides a compound of formula **I,** wherein LBM is MDM2 binding moiety 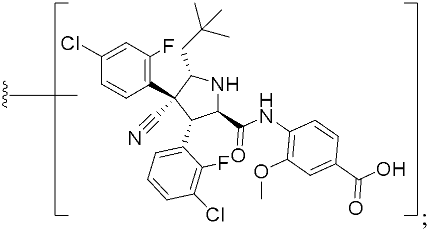 thereby forming a compound of formula **I-ll**: 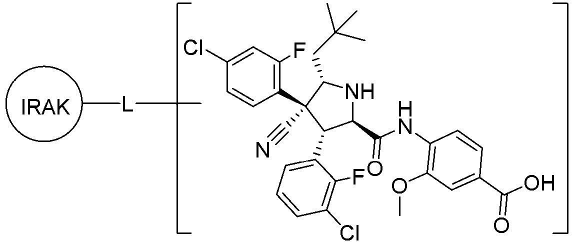 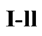 or a pharmaceutically acceptable salt thereof, wherein L and IRAK are as defined above and described in embodiments herein, as described and defined in Hines, J. et al., Cancer Res. (DOI: 10.1158/0008-5472.CAN-18-2918), the entirety of which is herein incorporated by reference.

In certain embodiments, the present invention provides a compound of formula **I,** wherein LBM is DCAF16 binding moiety 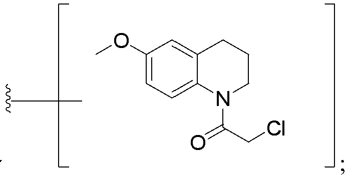 thereby forming a compound of formula **I-mm**: 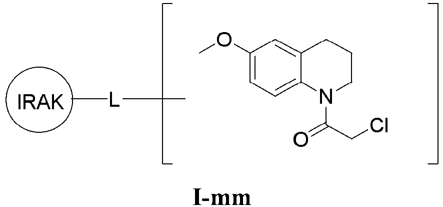 or a pharmaceutically acceptable salt thereof, wherein L and IRAK are as defined above and described in embodiments herein, as described and defined in Zhang, X. et al., bioRxiv (doi: https://doi.org/10.1101/443804), the entirety of which is herein incorporated by reference.

In certain embodiments, the present invention provides a compound of formula **I,** wherein LBM is RNF114 binding moiety 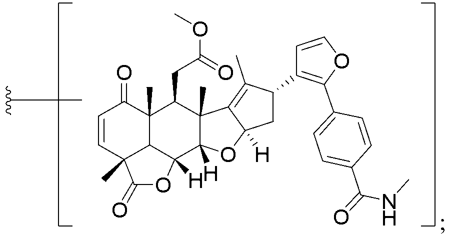 thereby forming a compound of formula **I-nn**: 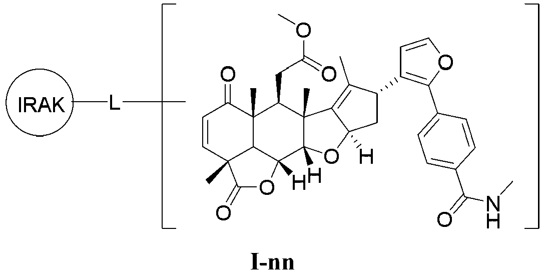 or a pharmaceutically acceptable salt thereof, wherein L and IRAK are as defined above and described in embodiments herein, as described and defined in Spradin, J.N. et al., bioRxiv (doi: https://doi.org/10.1101/436998), the entirety of which is herein incorporated by reference.

In certain embodiments, the present invention provides a compound of formula **I,** wherein LBM is a RNF4 binding moiety 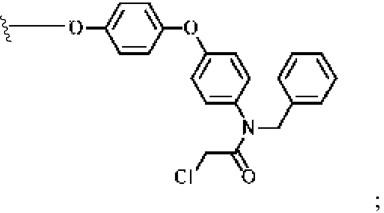 thereby forming a compound of formula **I-oo**: 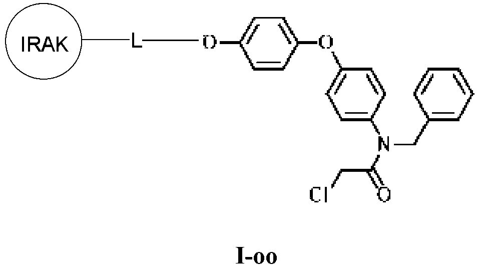 or a pharmaceutically acceptable salt thereof, wherein L and IRAK are as defined above and described in embodiments herein, as described and defined in Ward, C.C., et al., bioRxiv (doi: https://doi.org/10.1101/439125), the entirety of which is herein incorporated by reference.

In some embodiments, the present invention provides a compound of formula **I,** wherein LBM is an E3 ubiquitin ligase (cereblon) binding moiety 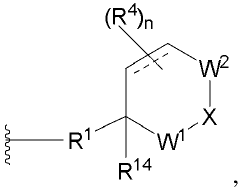 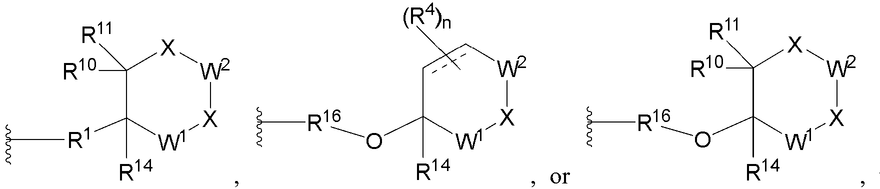 thereby forming a compound of formula **I-pp-1, I-pp-2, I-pp-3,** or **I-pp-4,** respectively: 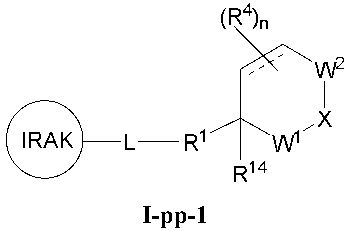 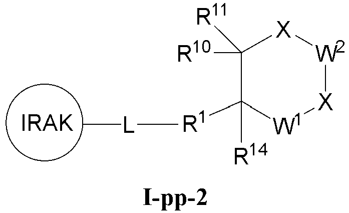 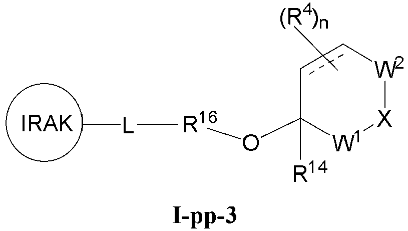 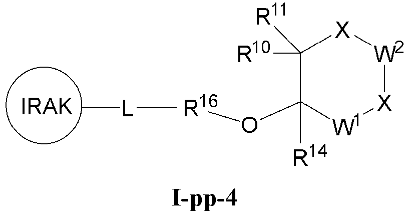 or a pharmaceutically acceptable salt thereof, wherein L and IRAK are as defined above and described herein, and wherein each of the variables R¹, R⁴, R¹⁰, R¹¹, R¹⁴, R¹⁶, W¹, W², X and n is as defined in WO 2018/237026, the entirety of each of which is herein incorporated by reference, and wherein 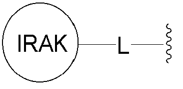 is attached to R¹ or R¹⁶ at the site of attachment of R¹² as defined in WO 2018/237026, such that 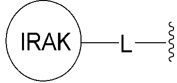 takes the place of the R¹² substituent.

In some embodiments, the present invention provides a compound of formula **I,** wherein LBM is an E3 ubiquitin ligase (cereblon) binding moiety 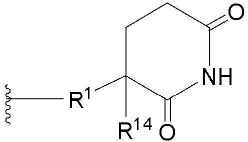 or 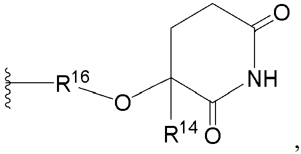 thereby forming a compound of formula **I-pp'-1** or **I-pp'-3,** respectively: 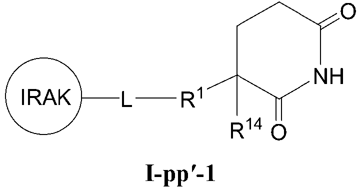 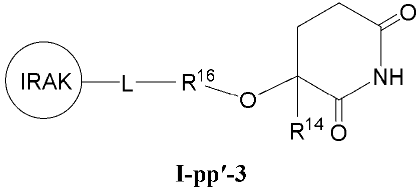 or a pharmaceutically acceptable salt thereof, wherein L and IRAK are as defined above and described herein, and wherein each of the variables R¹, R¹⁴, and R¹⁶ is as defined in WO 2018/237026, the entirety of each of which is herein incorporated by reference, and wherein 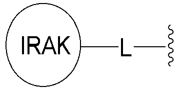 is attached to R¹ or R¹⁶ at the site of attachment of R¹² as defined in WO 2018/237026, such that 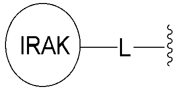 takes the place of the R¹² substituent.

In certain embodiments, the present invention provides a compound of formula **I,** 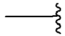 wherein LBM is an E3 ubiquitin ligase (cereblon) binding moiety
thereby forming a compound of formula **I-qq**: 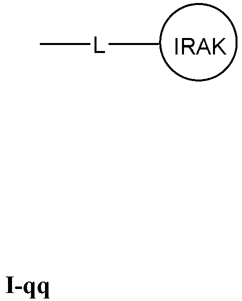 or a pharmaceutically acceptable salt thereof, wherein L and IRAK are as defined above and described in embodiments herein, and wherein each of the variables R₁, R₂, and n is as described and defined in WO 2019/043214, the entirety of each of which is herein incorporated by reference.

In certain embodiments, the present invention provides a compound of formula **I,** wherein LBM is a VHL binding moiety thereby forming a compound of formula **I-rr-1** or **I-rr-2**: 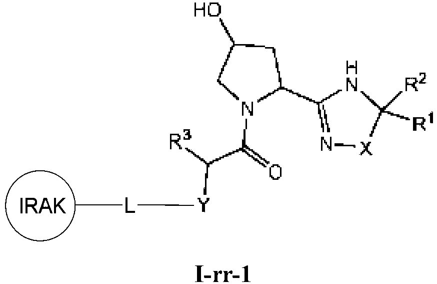 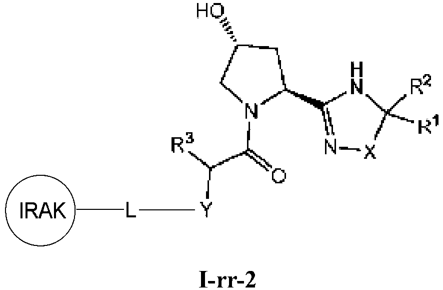 or a pharmaceutically acceptable salt thereof, wherein L and IRAK are as defined above and described in embodiments herein, and wherein each of the variables R¹, R², R³, X, and Y is as defined and described in WO 2019/084026, the entirety of each of which is herein incorporated by reference.

In certain embodiments, the present invention provides a compound of formula **I,** wherein LBM is a VHL binding moiety thereby forming a compound of formula **I-ss-1** or **I-ss-2**: 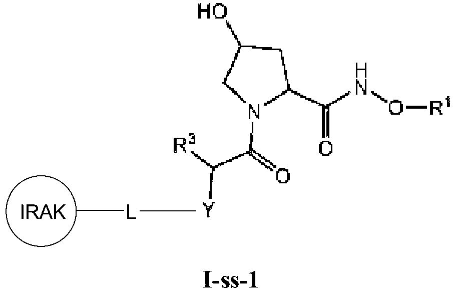 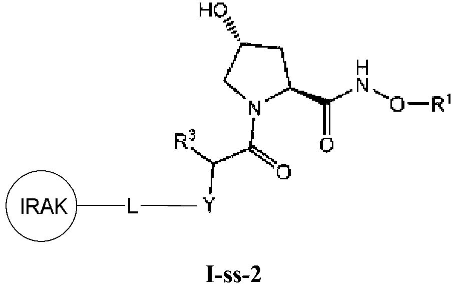 or a pharmaceutically acceptable salt thereof, wherein L and IRAK are as defined above and described in embodiments herein, and wherein each of the variables R¹, R³, and Y is as defined and described in WO 2019/084030, the entirety of each of which is herein incorporated by reference.

In certain embodiments, the present invention provides a compound of formula **I,** wherein LBM is a E3 ubiquitin ligase (cereblon) binding moiety thereby forming a compound of formula **I-tt-1, I-tt-2, I-tt-3,** or **I-tt-4b:** 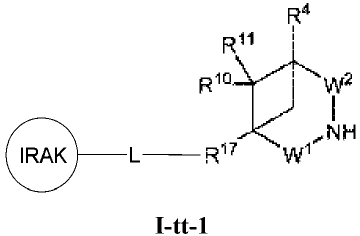 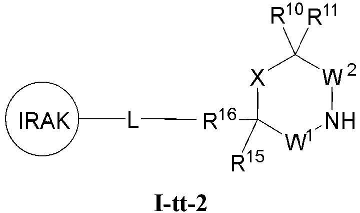 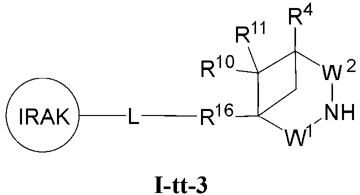 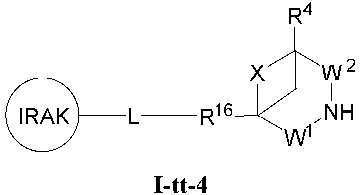 or a pharmaceutically acceptable salt thereof, wherein L and IRAK are as defined above and described herein, and wherein each of the variables R⁴, R¹⁰, R¹¹, R¹⁵, R¹⁶, R¹⁷, W¹, W², and X is as defined in WO 2019/099868 which is herein incorporated by reference in its entirety, and wherein 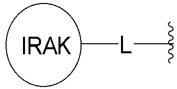 is attached to R¹⁷ or R¹⁶ at the site of attachment of R¹² as defined in WO 2018/237026, such that 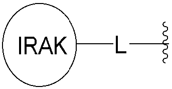 takes the place of the R¹² substituent.

In certain embodiments, the present invention provides a compound of formula **I,** wherein LBM is a E3 ubiquitin ligase (cereblon) binding moiety thereby forming a compound of formula **I-uu**: 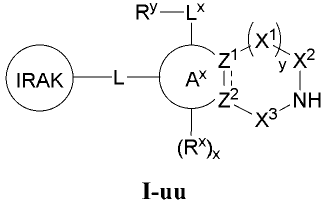 or a pharmaceutically acceptable salt thereof, wherein L and IRAK are as defined above and described in embodiments herein, wherein:
each X¹ is indenpendly -CH₂-, -O-, -NR-, -CF₂-, 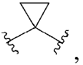 -C(O)-, -C(S)-, or 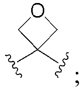
X² and X³ are independently -CH₂-, -C(O)-, -C(S)-, or 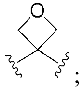
Z¹ and Z² are independently a carbon atom or a nitrogen atom;
Ring A^{x} is a fused ring selected from benzo or a 5-6 membered heteroaryl ring having 1-4 heteroatoms independently selected from nitrogen, oxygen, and sulfur;
L^{x} is a covalent bond or a C₁₋₃ bivalent straight or branched saturated or unsaturated hydrocarbon chain wherein 1-2 methylene units of the chain are independently and optionally replaced with -O-, -S-, -C(O)-, -C(S)-, -CR₂-, -CRF-, -CF₂-, -NR-, or -S(O)₂-;
each R^{x} is independently selected from hydrogen, deuterium, R^{z}, halogen, -CN, -NO₂, -OR, - SR, -NR₂, -S(O)₂R, -S(O)₂NR₂, -S(O)R, -CF₂R, -CF₃, -CR₂(OR), - CR₂(NR₂), -C(O)R, -C(O)OR, -C(O)NR₂, -C(O)N(R)OR, -OC(O)R, -OC(O)NR₂, - C(S)NR₂, -N(R)C(O)OR, -N(R)C(O)R, -N(R)C(O)NR₂, -N(R)S(O)₂R, -OP(O)R₂, - OP(O)(OR)₂, -OP(O)(OR)NR₂, -OP(O)(NR₂)₂, -Si(OR)R₂, and -SiR₃; or
   two R^{x} groups are optionally taken together to form an optionally substituted 5-8 membered partially unsaturated or aryl fused ring having 0-2 heteroatoms independently selected from nitrogen, oxygen, or sulfur;
each R is independently selected from hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
   two R groups on the same carbon or nitrogen are optionally taken together with their intervening atoms to form an optionally substituted 4-7 membered saturated, partially unsaturated, or heteroaryl ring having 0-3 heteroatoms, in addition to the carbon or nitrogen, independently selected from nitrogen, oxygen, and sulfur;
R^{y} is selected from 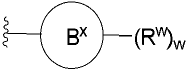 or hydrogen;
Ring B^{x} is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur, wherein Ring B^{x} is further optionally substituted with 1-2 oxo groups;
each R^{w} is independently selected from hydrogen, deuterium, R^{z}, halogen, -CN, -NO₂, -OR, - SR, -NR₂, -S(O)₂R, -S(O)₂NR₂, -S(O)R, -CF₂R, -CF₃, -CR₂(OR), - CR₂(NR₂), -C(O)R, -C(O)OR, -C(O)NR₂, -C(O)N(R)OR, -OC(O)R, -OC(O)NR₂, - N(R)C(O)OR, -N(R)C(O)R, -N(R)C(O)NR₂, -N(R)S(O)₂R, -OP(O)R₂, -OP(O)(OR)₂, - OP(O)(OR)NR₂, -OP(O)(NR₂)₂, and -SiR₃;
each R^{z} is independently selected from an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
- - - - is a single or double bond;
x is 0, 1, 2, 3 or 4;
y is 0, 1 or 2; and
w is 0, 1, 2, 3 or 4.

As defined above and described herein, each X¹ is independently -CH₂-, -O-, -NR-, - CF₂-, 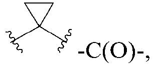 -C(S)-, or

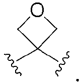

In some embodiments, X¹ is a covalent bond. In some embodiments, X¹ is -CH₂-. In some embodimens, X¹ is -O-. In some embodiments, X¹ is -NR-. In some embodiments, X¹ is - CF₂-. In some embodiments, X¹ is 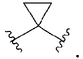 In some embodiments, X¹ is -C(O)- . In some embodiments, X¹ is -C(S)- . In some embodiments, X¹ is

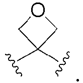

In certain embodiments, X¹ is selected from those shown in the compounds of **Table 1.**

As defined above and described herein, X² and X³ are independently -CH₂-, -C(O)-, - C(S)-, or

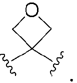

In some embodiments, X² and X³ are independently -CH₂-. In some embodiments, X² and X³ are independently -C(O)-. In some embodiments, X² and X³ are independently -C(S)-. In some embodiments, X² and X³ are independently

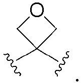

In certain embodiments, X² and X³ are independently selected from those shown in the compounds of **Table 1.**

As define above and described herein, Z¹ and Z² are independently a carbon atom or a nitrogen atom.

In some embodiments, Z¹ and Z² are independently a carbon atom. In some embodiments, Z¹ and Z² are independently a carbon atom.

In certain embodiments, Z¹ and Z² are independently selected from those shown in the compounds of **Table 1.**

As defined above and described herein, Ring A^{x} is fused ring selected from benzo or a 5-6 membered heteroaryl ring having 1-4 heteroatoms independently selected from nitrogen, oxygen, and sulfur.

In some embodiments, Ring A^{x} is benzo. In some embodiments, Ring A^{x} is a 5-6 membered heteroaryl ring having 1-4 heteroatoms independently selected from nitrogen, oxygen, and sulfur.

In some embodiments, Ring A^{x} is 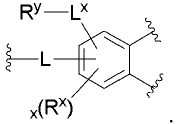 In some embodiments, Ring A^{x} is 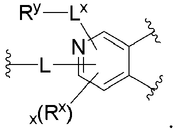 In some embodiments, Ring A^{x} is 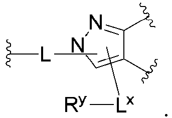 In some embodiments, Ring A^{x} is

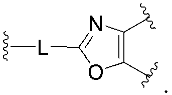

In certain embodiments, Ring A^{x} is selected from those shown in the compounds of **Table 1.**

As defined above and described herein, L^{x} is a covalent bond or a C₁₋₃ bivalent straight or branched saturated or unsaturated hydrocarbon chain wherein 1-2 methylene units of the chain are independently and optionally replaced with -O-, -S-, -C(O)-, -C(S)-, -CR₂-, -CRF-, -CF₂-, - NR-, or -S(O)₂-.

In some embodiments, L^{x} is a covalent bond. In some embodiments, L^{x} is a C₁₋₃ bivalent straight or branched saturated or unsaturated hydrocarbon chain wherein 1-2 methylene units of the chain are independently and optionally replaced with -O-, -S-, -C(O)-, -C(S)-, -CR₂-, -CRF-, -CF₂-, -NR-, or -S(O)₂-.

In some embodiments, L^{x} is -C(O)-.

In certain embodiments, L^{x} is selected from those shown in the compounds of Table 1.

As defined above and described herein, each R^{x} is independently selected from hydrogen, deuterium, R^{z}, halogen, -CN, -NO₂, -OR, -SR, -NR₂, -S(O)₂R, -S(O)₂NR₂, -S(O)R, - CF₂R, -CF₃, -CR₂(OR), -CR₂(NR₂), -C(O)R, -C(O)OR, - C(O)NR₂, -C(O)N(R)OR, -OC(O)R, -OC(O)NR₂, -C(S)NR₂, - N(R)C(O)OR, -N(R)C(O)R, -N(R)C(O)NR₂, -N(R)S(O)₂R, -OP(O)R₂, -OP(O)(OR)₂, - OP(O)(OR)NR₂, -OP(O)(NR₂)₂, -Si(OR)R₂, -SF₅, and -SiR₃, or two R^{x} groups are optionally taken together to form an optionally substituted 5-8 membered partially unsaturated or aryl fused ring having 0-2 heteroatoms independently selected from nitrogen, oxygen, or sulfur.

In some embodiments, R^{x} is hydrogen. In some embodiments, R^{x} is deuterium. In some embodiments, R^{x} is R^{z}. In some embodiments, R^{x} is halogen. In some embodiments, R^{x} is -CN. In some embodiments, R^{x} is -NO₂. In some embodiments, R^{x} is -OR. In some embodiments, R^{x} is -SR. In some embodiments, R^{x} is -NR₂. In some embodiments, R^{x} is -S(O)₂R. In some embodiments, R^{x} is -S(O)₂NR₂. In some embodiments, R^{x} is -S(O)R. In some embodiments, R^{x} is -CF₂R. In some embodiments, R^{x} is -CF₃. In some embodiments, R^{x} is - CR₂(OR). In some embodiments, R^{x} is -CR₂(NR₂). In some embodiments, R^{x} is -C(O)R. In some embodiments, R^{x} is -C(O)OR. In some embodiments, R^{x} is -C(O)NR₂. In some embodiments, R^{x} is -C(O)N(R)OR. In some embodiments, R^{x} is -OC(O)R. In some embodiments, R^{x} is -OC(O)NR₂. In some embodiments, R^{x} is -C(S)NR₂. In some embodiments, R^{x} is - N(R)C(O)OR. In some embodiments, R^{x} is -N(R)C(O)R. In some embodiments, R^{x} is -N(R)C(O)NR₂. In some embodiments, R^{x} is -N(R)S(O)₂R. In some embodiments, R^{x} is - OP(O)R₂. In some embodiments, R^{x} is -OP(O)(OR)₂,. In some embodiments, R^{x} is - OP(O)(OR)NR₂. In some embodiments, R^{x} is -OP(O)(NR₂)₂. In some embodiments, R^{x} is - Si(OR)R₂. In some embodiments, R^{x} is -SF₅. In some embodiments, R^{x} is -SiR₃. In some embodiments, two R^{x} groups are optionally taken together to form an optionally substituted 5-8 membered partially unsaturated or aryl fused ring having 0-2 heteroatoms independently selected from nitrogen, oxygen, or sulfur.

In some embodiments, R^{x} is fluoro. In some embodiments, R^{x} is bromo. In some embodiments, R^{x} is methyl. In some embodiments, R^{x} is -OH. In some embodiments, R^{x} is -NH₂. In some embodiments, R^{x} is -NHCH₃. In some embodiments, R^{x} is -N(CH₃)₂. In some embodiments, R^{x} is -NHCH(CH₃)₂. In some embodiments, R^{x} is -NHSO₂CH₃. In some embodiments, R^{x} is -CH₂OH. In some embodiments, R^{x} is -CH₂NH₂. In some embodiments, R^{x} is -C(O)NH₂. In some embodiments, R^{x} is -C(O)NHCH₃. In some embodiments, R^{x} is 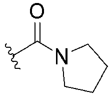 In some embodiments, R^{x} is 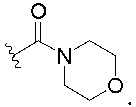 In some embodiments, R^{x} is 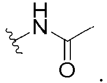 In some embodiments, R^{x} is 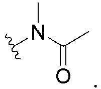 In some embodiments, R^{x} is 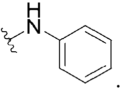 In some embodiments, R^{x} is 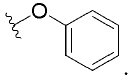 In some embodiments, R^{x} is 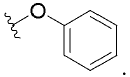 In some embodiments, R^{x} is 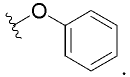 In some embodiments, R^{x} is 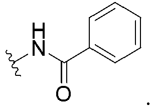 In some embodiments, R^{x} is

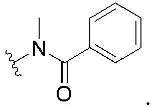

In certain embodiments, each R^{x} is independently selected from those shown in the compounds of **Table 1.**

As defined above and described here, each R is independently selected from hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or two R groups on the same carbon or nitrogen are optionally taken together with their intervening atoms to form an optionally substituted 4-7 membered saturated, partially unsaturated, or heteroaryl ring having 0-3 heteroatoms, in addition to the carbon or nitrogen, independently selected from nitrogen, oxygen, and sulfur.

In some embodiments, R is hydrogen. In some embodiments, R is an optionally substituted C₁₋₆ aliphatic. In some embodiments, R is an optionally substituted phenyl. In some embodiments, R is an optionally substituted 4-7 membered saturated or partially unsaturated heterocyclic having 1-2 heteroatoms independently selected from nitrogen, oxygen, and sulfur. In some embodiments, R is an optionally substituted a 5-6 membered heteroaryl ring having 1-4 heteroatoms independently selected from nitrogen, oxygen, and sulfur. In some embodiments, two R groups on the same carbon or nitrogen are optionally taken together with their intervening atoms to form an optionally substituted 4-7 membered saturated, partially unsaturated, or heteroaryl ring having 0-3 heteroatoms, in addition to the carbon or nitrogen, independently selected from nitrogen, oxygen, and sulfur.

As defined above and described herein, R^{y} is selected from 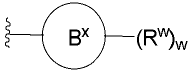 or hydrogen.

In some embodiment R^{y} is 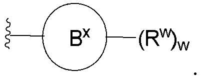 In some embodiments, R^{y} is hydrogen.

In certain embodiments, R^{y} is selected from those shown in the compounds of Table 1.

As defined above and described herein, Ring B^{x} is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur, wherein Ring B^{x} is further optionally substituted with 1-2 oxo groups.

In some embodiments, Ring B^{x} is phenyl. In some embodiments, Ring B^{x} is a 4-10 membered saturated or partially unsaturated mono- or bicyclic carbocyclic or heterocyclic ring having 1-3 heteroatoms independently selected from nitrogen, oxygen, and sulfur In some embodiments, Ring B^{x} is a 5-6 membered heteroaryl ring having 1-4 heteroatoms independently selected from nitrogen, oxygen, and sulfur. In some embodiments, Ring B^{x} is further optionally substituted with 1-2 oxo groups.

In some embodiments, Ring B^{x} is 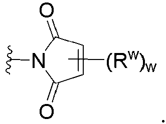 In some embodiments, Ring B^{x} is 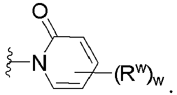 In some embodiments, Ring B^{x} is 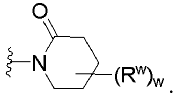 In some embodiments Ring B^{x} is 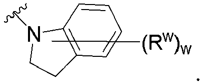 In some embodiments Ring B^{x} is

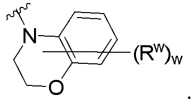

In certain embodiments, Ring B^{x} is selected from those shown in the compounds of **Table 1.**

As defined above and described herein, each R^{w} is independently selected from hydrogen, deuterium, R^{z}, halogen, -CN, -NO₂, -OR, -SR, -NR₂, -S(O)₂R, -S(O)₂NR₂, -S(O)R, - CF₂R, -CF₃, -CR₂(OR), -CR₂(NR₂), -C(O)R, -C(O)OR, - C(O)NR₂, -C(O)N(R)OR, -OC(O)R, -OC(O)NR₂, -N(R)C(O)OR, -N(R)C(O)R, -N(R)C(O)NR₂, - N(R)S(O)₂R, -OP(O)R₂, -OP(O)(OR)₂, -OP(O)(OR)NR₂, -OP(O)(NR₂)₂, -SF₅, and -SiR₃.

In some embodiments, R^{w} is hydrogen. In some embodiments, R^{w} is deuterium. In some embodiments, R^{w} is R^{z}. In some embodiments, R^{w} is halogen. In some embodiments, R^{w} is -CN. In some embodiments, R^{w} is -NO₂. In some embodiments, R^{w} is -OR. In some embodiments, R^{w} is -SR. In some embodiments, R^{w} is -NR₂. In some embodiments, R^{w} is -S(O)₂R. In some embodiments, R^{w} is -S(O)₂NR₂. In some embodiments, R^{w} is -S(O)R. In some embodiments, R^{w} is -CF₂R. In some embodiments, R^{w} is -CF₃. In some embodiments, R^{w} is -CR₂(OR). In some embodiments, R^{w} is -CR₂(NR₂). In some embodiments, R^{w} is -C(O)R. In some embodiments, R^{w} is -C(O)OR. In some embodiments, R^{w} is -C(O)NR₂. In some embodiments, R^{w} is -C(O)N(R)OR. In some embodiments, R^{w} is -OC(O)R. In some embodiments, R^{w} is -OC(O)NR₂. In some embodiments, R^{w} is -N(R)C(O)OR. In some embodiments, R^{w} is -N(R)C(O)R. In some embodiments, R^{w} is -N(R)C(O)NR₂. In some embodiments, R^{w} is -N(R)S(O)₂R. In some embodiments, R^{w} is -OP(O)R₂. In some embodiments, R^{w} is -OP(O)(OR)₂. In some embodiments, R^{w} is -OP(O)(OR)NR₂. In some embodiments, R^{w} is -OP(O)(NR₂)₂. In some embodiments, R^{w} is -SF₅. In some embodiments, R^{w} is -SiR₃.

In certain embodiments, R^{w} is selected from those shown in the compounds of **Table 1.**

As defined above and described herein, each R^{z} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur.

In some embodiments, R^{z} is an optionally substituted C₁₋₆ aliphatic. In some embodiments, R^{z} is an optionally substituted phenyl. In some embodiments, R^{z} is an optionally substituted 4-7 membered saturated or partially unsaturated heterocyclic ring having 1-2 heteroatoms independently selected from nitrogen, oxygen, and sulfur. In some embodiments, R^{z} is an optionally substituted 5-6 membered heteroaryl ring having 1-4 heteroatoms independently selected from nitrogen, oxygen, and sulfur.

In some embodiments, R^{z} is 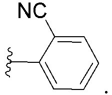 In some embodiments, R^{z} is 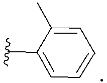 In some embodiments, R^{z} is 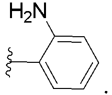 In some embodiments, R^{z} is 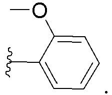 In some embodiments, R^{z} is 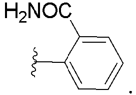 In some embodiments, R^{z} is 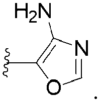 In some embodiments, R^{z} is

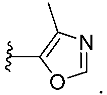

In certain embodiments, R^{z} is selected from those shown in the compounds of **Table 1.**

As defined above and described herein, - - - - is a single or double bond.

In some embodiments, - - - - is a single bond. In some embodiments, - - - - is a double bond.

In certain embodiments, - - - - is selected from those shown in the compounds of **Table 1.**

As defined above and described herein, w is 0, 1, 2, 3 or 4.

In some embodiments, w is 0. In some embodiments, w is 1. In some embodiments, w is 2. In some embodiments, w is 3. In some embodiments, w is 4.

In certain embodiments, w is selected from those shown in the compounds of **Table 1.**

As defined above and described herein, x is 0, 1, 2, 3 or 4.

In some embodiments, x is 0. In some embodiments, x is 1. In some embodiments, m is 2. In some embodiments, x is 3. In some embodiments, x is 4.

In certain embodiments, x is selected from those shown in the compounds of **Table 1.**

As defined above and described herein, y is 0, 1 or 2.

In some embodiments, y is 0. In some embodiments, y is 1. In some embodiments, y is 2.

In certain embodiments, y is selected from those shown in the compounds of **Table 1.**

In some embodiments, the present invention provides a compound of formula **I-uu,** wherein Ring A^{x} is benzo, y is 1, X¹ is -CH₂-, X² and X³ are -C(O)-, and Z¹ and Z² are carbon atoms as shown, to provide a compound of formula **I-uu-1:** 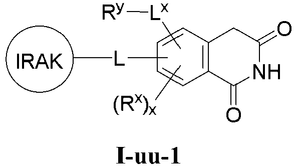 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is imidazolyl, y is 1, X¹ is -CH₂-, X² and X³ are -C(O)-, and Z¹ and Z² are carbon atoms as shown, to provide a compound of formula **I-uu-2:** 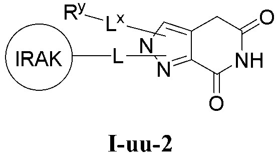 or a pharmaceutically acceptable salt thereof, wherein each of IRAK, L, L^{x}, and R^{y} is as defined above and described in embodiments herein, both singly and in combination.

In some embodiments, the present invention provides a compound of formula **I-uu,** wherein Ring A^{x} is imidazolyl, y is 1, X¹ is -CH₂-, X² and X³ are -C(O)-, and Z¹ and Z² are carbon atoms as shown, to provide a compound of formula **I-uu-3:** 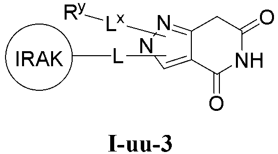 or a pharmaceutically acceptable salt thereof, wherein each of IRAK, L, L^{x}, and R^{y} is as defined above and described in embodiments herein, both singly and in combination.

In some embodiments, the present invention provides a compound of formula **I-uu,** wherein Ring A^{x} is oxazolyl, y is 1, X¹ is -CH₂-, X² and X³ are -C(O)-, and Z¹ and Z² are carbon atoms as shown, to provide a compound of formula **I-uu-4**: 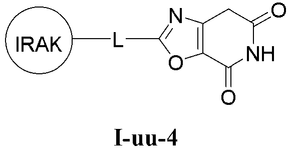 or a pharmaceutically acceptable salt thereof, wherein each of IRAK and L is as defined above and described in embodiments herein, both singly and in combination.

In some embodiments, the present invention provides a compound of formula **I-uu,** wherein Ring A^{x} is benzo, y is 0, X² and X³ are -C(O)-, and Z¹ and Z² are carbon atoms as shown, to provide a compound of formula **I-uu-5**: 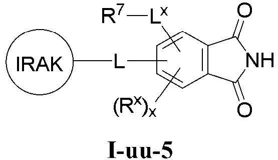 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is benzo, y is 1, X¹ is -O-, X² and X³ are -C(O)-, and Z¹ and Z² are carbon atoms as shown, to provide a compound of formula **I-uu-6**: 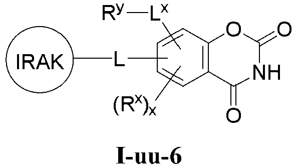 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is benzo, y is 1, X¹ is -NR-, X² and X³ are -C(O)-, and Z¹ and Z² are carbon atoms as shown, to provide a compound of formula **I-uu-7**: 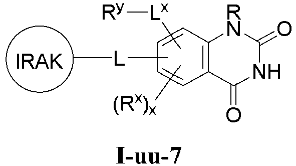 or a pharmaceutically acceptable salt thereof, wherein each of IRAK, L, L^{x}, R, R^{x}, R^{y}, and x is as defined above and described in embodiments herein, both singly and in combination.

In some embodiments, the present invention provides a compound of formula **I-uu,** wherein Ring A^{x} is benzo, y is 1, X¹ is -CF₂-, X² and X³ are -C(O)-, and Z¹ and Z² are carbon atoms as shown, to provide a compound of formula **I-uu-8**: 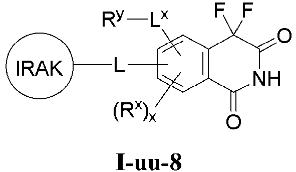 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is benzo, y is 1, X¹ is 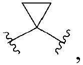 X² and X³ are -C(O)-, and Z¹ and Z² are carbon atoms as shown, to provide a compound of formula **I-uu-9**: 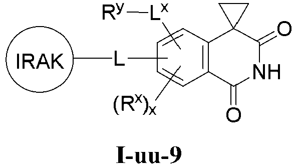 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is pyridyl, y is 1, X¹ is -CH₂-, X² and X³ are -C(O)-, and Z¹ and Z² are carbon atoms as shown, to provide a compound of formula **I-uu-10**: 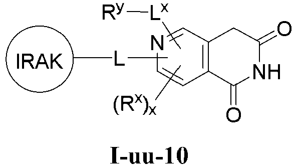 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A^{x} is pyridyl, y is 1, X¹ is -CH₂-, X² and X³ are -C(O)-, and Z¹ and Z² are carbon atoms as shown, to provide a compound of formula **I-uu-11**: 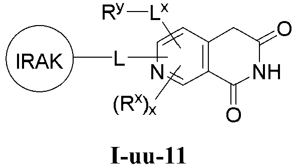 or a pharmaceutically acceptable salt thereof, wherein each of IRAK, L, L^{x}, R^{x}, R^{y}, and x is as defined above and described in embodiments herein, both singly and in combination.

In some embodiments, the present invention provides a compound of formula **I-uu,** wherein Ring A is benzo, y is 1, X¹, X² and X³ are -C(O)-, and Z¹ and Z² are carbon atoms as shown, to provide a compound of formula **I-uu-12**: 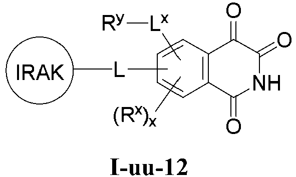 or a pharmaceutically acceptable salt thereof, wherein each of IRAK, L, L^{x}, R^{x}, R^{y}, and x is as defined above and described in embodiments herein, both singly and in combination.

In some embodiments, LBM is 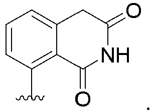 In some embodiments, LBM is 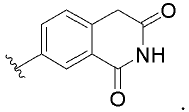 In some embodiments, LBM is 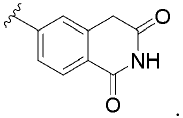 In some embodiments, LBM is 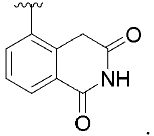 In some embodiments, LBM is 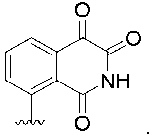 In some embodiments, LBM is 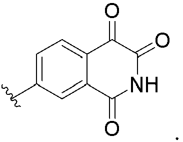 In some embodiments, LBM is 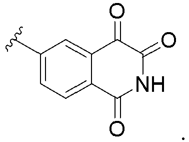 In some embodiments, LBM is

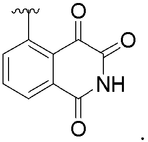

In some embodiments, LBM is 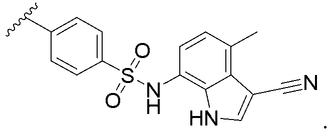 In some embodiments, LBM is 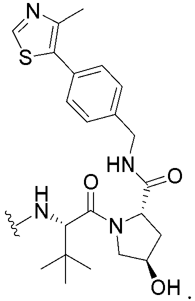 In some embodiments, LBM is 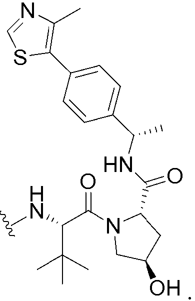 In some embodiments, LBM is 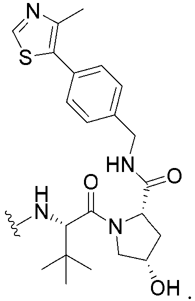 In some embodiments, LBM is 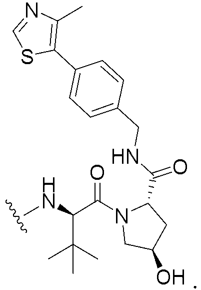 In some embodiments, LBM is 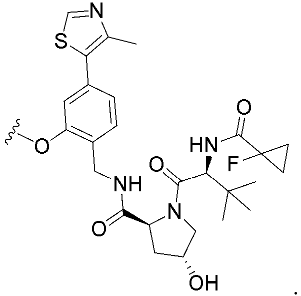 In some embodiments, LBM is 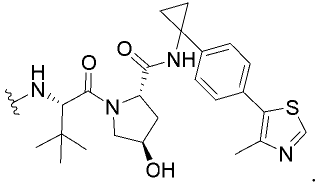 In some embodiments, LBM is 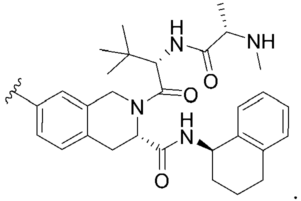 In some embodiments, LBM is 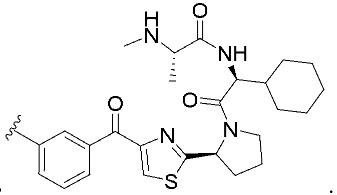 In some embodiments, LBM is 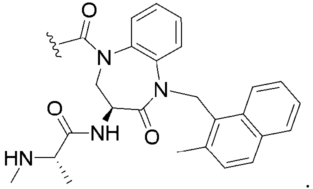 In some embodiments, LBM is 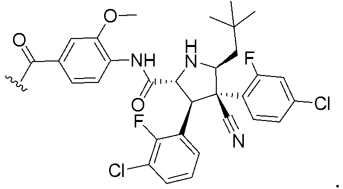 In some embodiments, LBM is 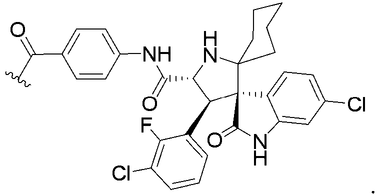 In some embodiments, LBM is 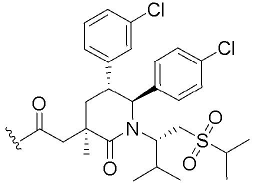 In some embodiments, LBM is 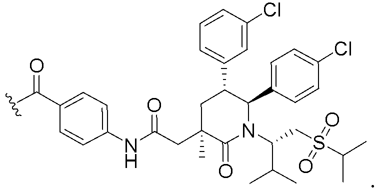 In some embodiments, LBM is 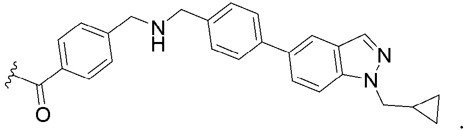 In some embodiments, LBM is

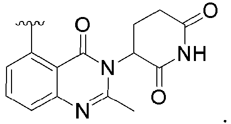

In some embodiments, LBM is selected from those in **Table 1,** below.

In some embodiments, the present invention provides the compound of formula **II,** wherein LBM is IAP binding moeity 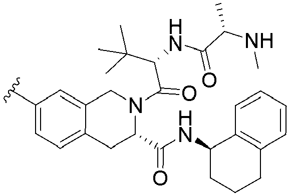 thereby forming a compound of formula **II-vv**: 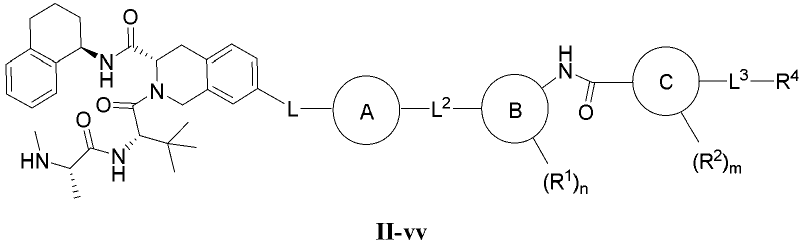 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is IAP binding moeity 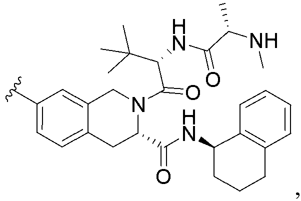 L² is a covalent bond, Ring A is cyclohexyl, Ring B is pyrazolyl, Ring C is oxazolyl, and Ring D is pyridyl thereby forming a compound of formula **II-ww**: 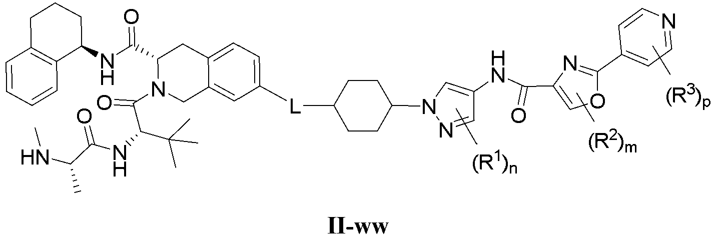 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is IAP binding moeity 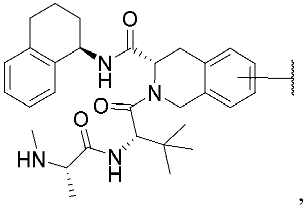 L² is a covalent bond, Ring A is cyclohexyl, and Ring B is pyrazolyl as shown, thereby forming a compound of formula **II-d-8**: 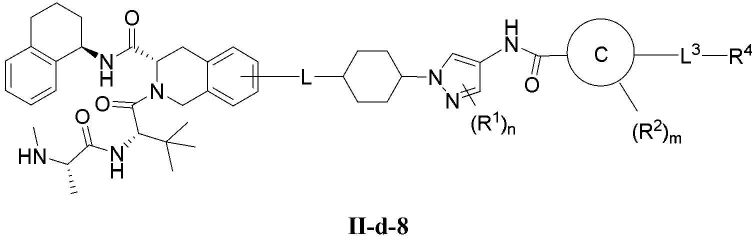 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is IAP binding moeity 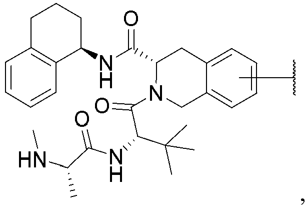 L² is a covalent bond, Ring A is cyclohexyl, Ring C is pyrazolo[1,5-a]pyrimidine, and Ring B is pyrazolyl as shown, thereby forming a compound of formula **II-e-8**: 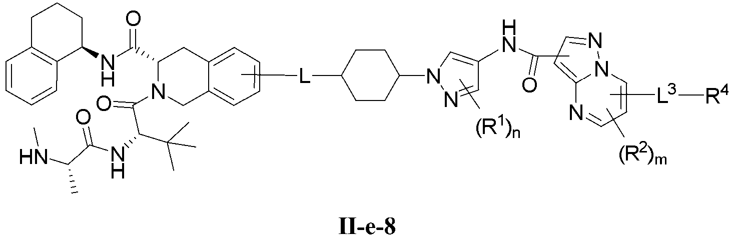 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is MDM2 binding moeity 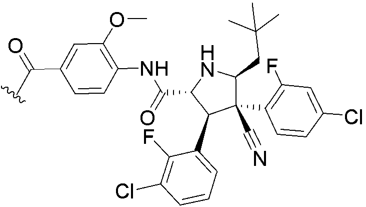 thereby forming a compound of formula II-xx: 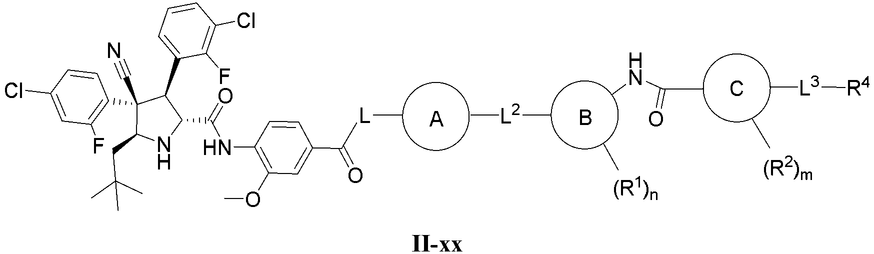 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is MDM2 binding moeity 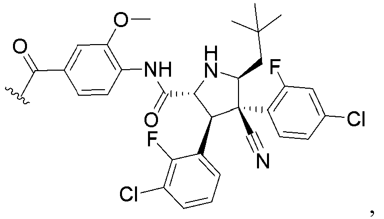 L² is a covalent bond, Ring A is cyclohexyl, Ring B is pyrazolyl, Ring C is oxazolyl, and Ring D is pyridyl thereby forming a compound of formula **II-yy**: 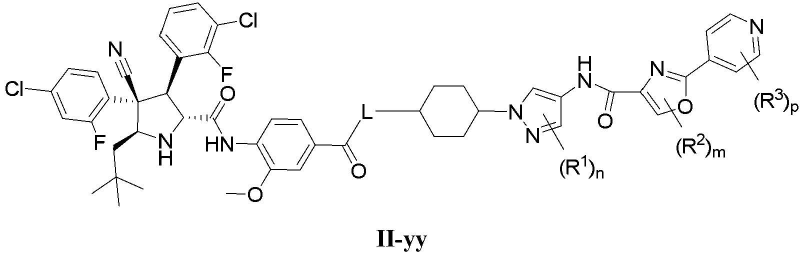 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is MDM2 binding moeity 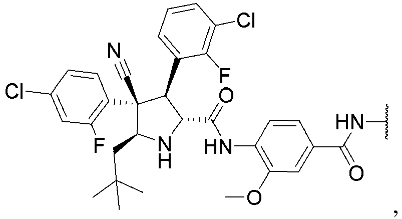 L² is a covalent bond, Ring A is cyclohexyl, and Ring B is pyrazolyl as shown, thereby forming a compound of formula **II-d-9**: 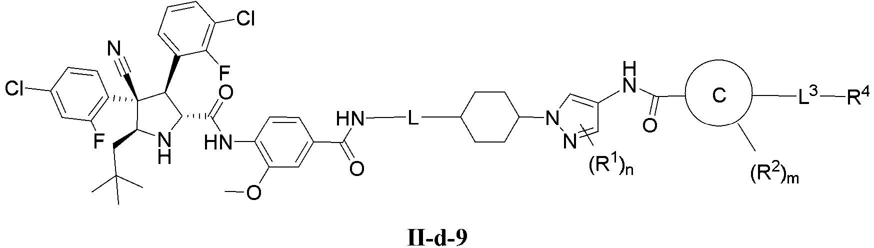 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is MDM2 binding moeity 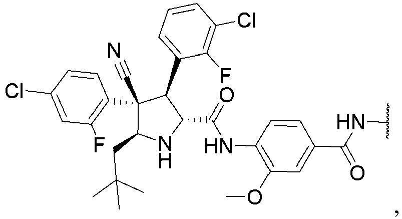 L² is a covalent bond, Ring A is cyclohexyl, Ring C is pyrazolo[1,5-a]pyrimidine, and Ring B is pyrazolyl as shown, thereby forming a compound of formula **II-e-9**: 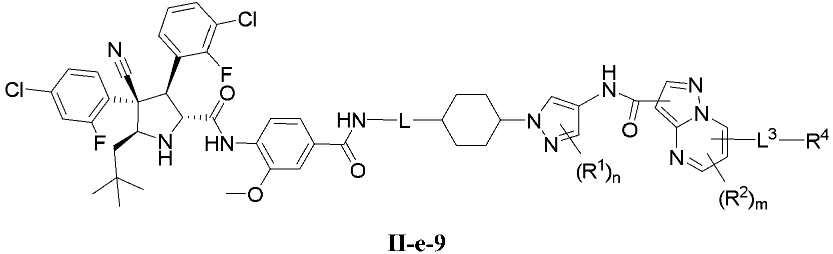 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is IAP binding moeity 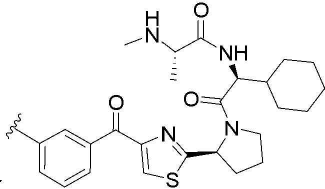 thereby forming a compound of formula **II-zz**: 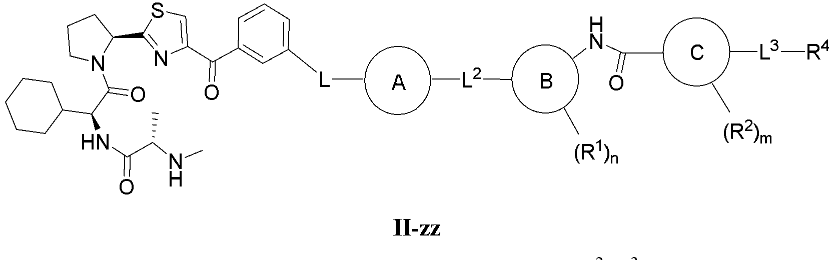 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is IAP binding moeity 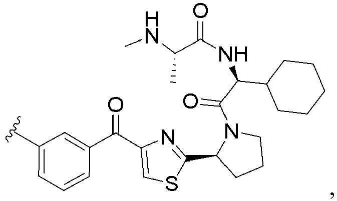 L² is a covalent bond, Ring A is cyclohexyl, Ring B is pyrazolyl, Ring C is oxazolyl, and Ring D is pyridyl thereby forming a compound of formula **II-aaa**: 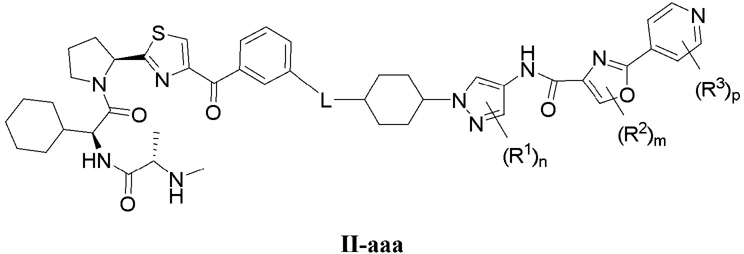 or a pharmaceutically acceptable salt thereof, wherein each of L, R¹, R², R³, n, m, and p is as defined above and described in embodiments herein, both singly and in combination.

In certain embodiments, the present invention provides the compound of formula **II-d,** wherein LBM is IAP binding moeity 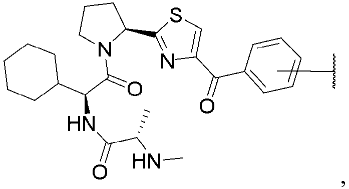 L² is a covalent bond, Ring A is cyclohexyl, and Ring B is pyrazolyl as shown, thereby forming a compound of formula **II-d-10**: 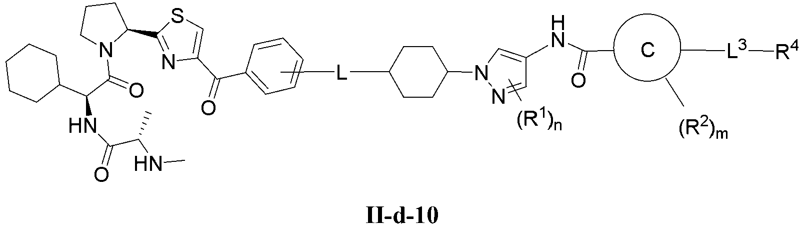 or a pharmaceutically acceptable salt thereof, wherein each of L, L³, Ring C, R¹, R², R⁴, n, and m is as defined above and described in embodiments herein, both singly and in combination.

In certain embodiments, the present invention provides the compound of formula **II-e,** wherein LBM is IAP binding moeity 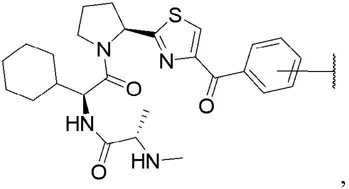 L² is a covalent bond, Ring A is cyclohexyl, Ring C is pyrazolo[1,5-a]pyrimidine, and Ring B is pyrazolyl as shown, thereby forming a compound of formula **II-e-10**: 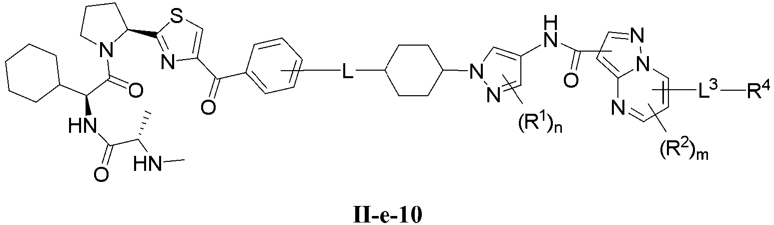 or a pharmaceutically acceptable salt thereof, wherein each of L, R¹, R², R³, n, m, and p is as defined above and described in embodiments herein, both singly and in combination.

In some embodiments, the present invention provides the compound of formula **II,** wherein LBM is VHL binding moeity 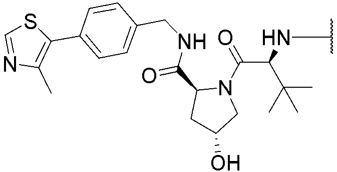 thereby forming a compound of formula **II-bbb**: 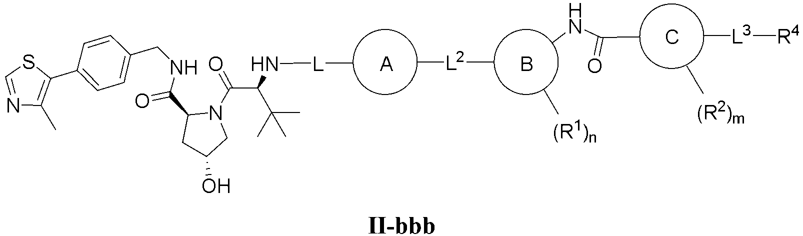 or a pharmaceutically acceptable salt thereof, wherein each of L, L², L³, Ring A, Ring B, Ring C, R¹, R², R⁴, n, and m is as defined above and described in embodiments herein, both singly and in combination.

In some embodiments, the present invention provides the compound of formula **II,** wherein LBM is VHL binding moeity 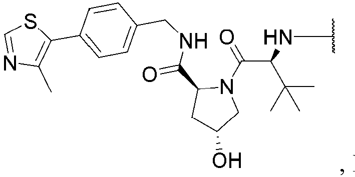 L² is a covalent bond, Ring A is cyclohexyl, Ring B is pyrazolyl, Ring C is oxazolyl, and Ring D is pyridyl thereby forming a compound of formula **II-ccc**: 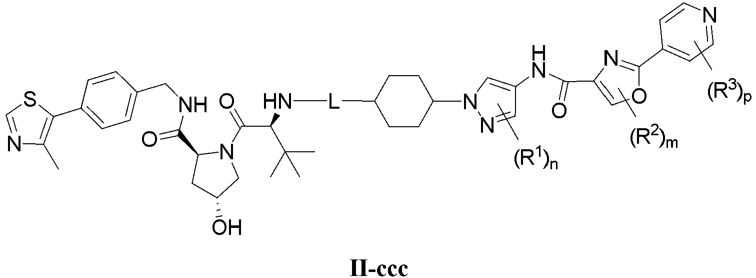 or a pharmaceutically acceptable salt thereof, wherein each of L, R¹, R², R³, n, m, and p is as defined above and described in embodiments herein, both singly and in combination.

In certain embodiments, the present invention provides the compound of formula **II-e,** wherein LBM is VHL binding moeity 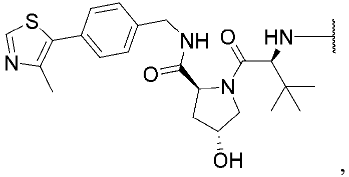 L² is a covalent bond, Ring A is cyclohexyl, and Ring B is pyrazolyl as shown, thereby forming a compound of formula **II-d-11**: 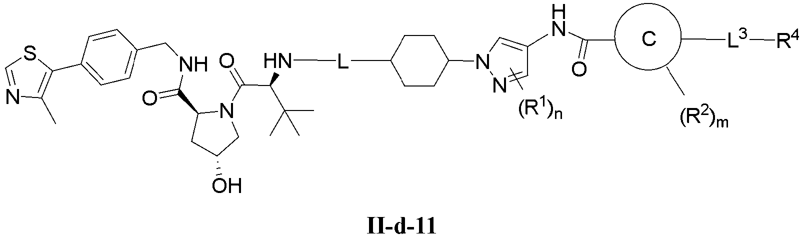 or a pharmaceutically acceptable salt thereof, wherein each of L, L², L³, Ring A, Ring B, Ring C, R¹, R², R⁴, n, and m is as defined above and described in embodiments herein, both singly and in combination.

In certain embodiments, the present invention provides the compound of formula **II-e,** wherein LBM is VHL binding moeity 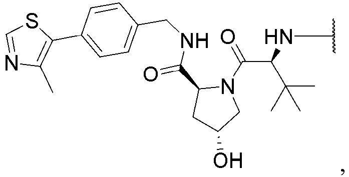 L² is a covalent bond, Ring A is cyclohexyl, Ring C is pyrazolo[1,5-a]pyrimidine, and Ring B is pyrazolyl as shown, thereby forming a compound of formula **II-e-11**: 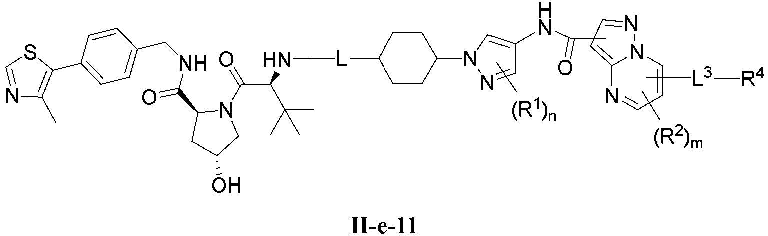 or a pharmaceutically acceptable salt thereof, wherein each of L, L³, R¹, R², R⁴, n, and each m is as defined above and described in embodiments herein, both singly and in combination.

In certain embodiments, the present invention provides a compound of formula **II-e-12**: 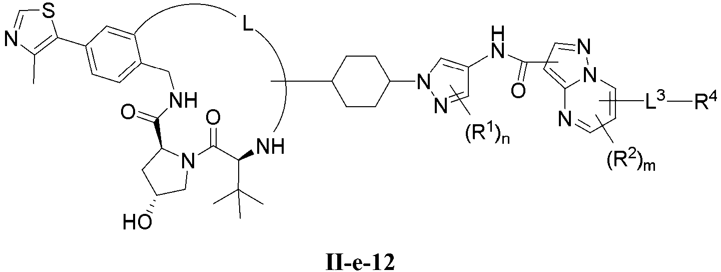 or a pharmaceutically acceptable salt thereof, wherein the VHL binding moeity forms a macrocycle with L as shown and the IRAK binding moeity attaches to any modifiable carbon, oxygen, or nitrogen atom of L, wherein each of L, L³, R¹, R², R⁴, n, and each m is as defined above and described in embodiments herein, both singly and in combination.

In certain embodiments, the present invention provides a compound of formula **II-e-13**: 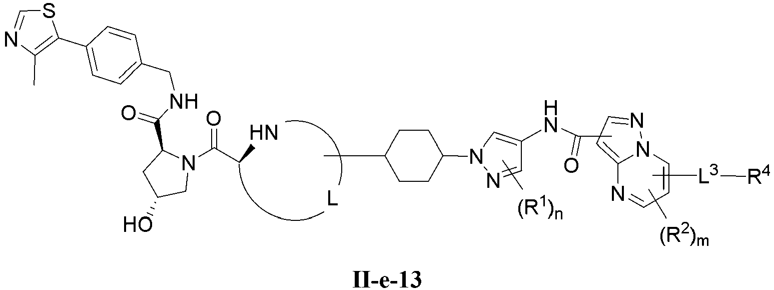 or a pharmaceutically acceptable salt thereof, wherein the VHL binding moeity forms a macrocycle with L as shown and the IRAK binding moeity attaches to any modifiable carbon, oxygen, or nitrogen atom of L, wherein each of L, L³, R¹, R², R⁴, n, and each m is as defined above and described in embodiments herein, both singly and in combination.

In certain embodiments, the present invention provides the compound of formula **II-e,** wherein LBM is VHL binding moeity 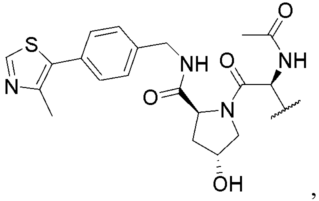 L² is a covalent bond, Ring A is cyclohexyl, Ring C is pyrazolo[1,5-a]pyrimidine, and Ring B is pyrazolyl as shown, thereby forming a compound of formula **II-e-14**: 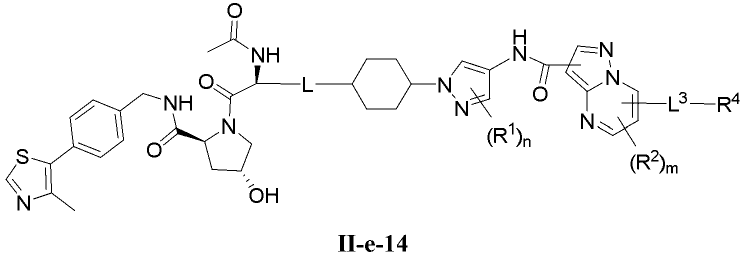 or a pharmaceutically acceptable salt thereof, wherein each of L, L³, R¹, R², R⁴, n, and each m is as defined above and described in embodiments herein, both singly and in combination.

In certain embodiments, the present invention provides the compound of formula **II-e,** wherein LBM is VHL binding moeity 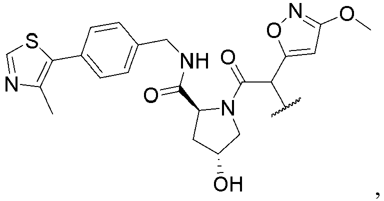 L² is a covalent bond, Ring A is cyclohexyl, Ring C is pyrazolo[1,5-a]pyrimidine, and Ring B is pyrazolyl as shown, thereby forming a compound of formula **II-e-15**: 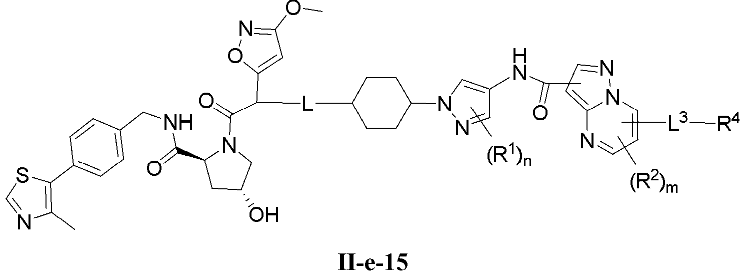 or a pharmaceutically acceptable salt thereof, wherein each of L, L³, R¹, R², R⁴, n, and each m is as defined above and described in embodiments herein, both singly and in combination.

In certain embodiments, the present invention provides the compound of formula **II-e,** wherein LBM is VHL binding moeity 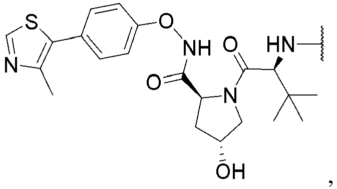 L² is a covalent bond, Ring A is cyclohexyl, Ring C is pyrazolo[1,5-a]pyrimidine, and Ring B is pyrazolyl as shown, thereby forming a compound of formula **II-e-16**: 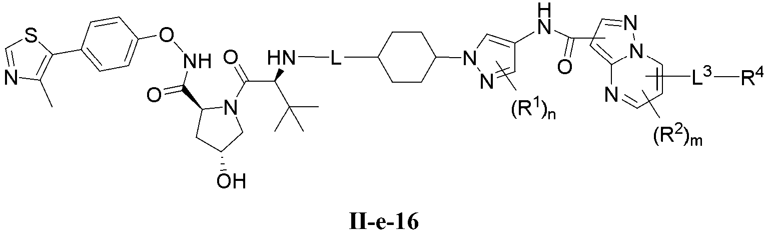 or a pharmaceutically acceptable salt thereof, wherein each of L, L³, R¹, R², R⁴, n, and each m is as defined above and described in embodiments herein, both singly and in combination.

In certain embodiments, the present invention provides the compound of formula **II-e,** wherein LBM is VHL binding moeity 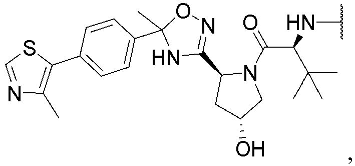 L² is a covalent bond, Ring A is cyclohexyl, Ring C is pyrazolo[1,5-a]pyrimidine, and Ring B is pyrazolyl as shown, thereby forming a compound of formula **II-e-17**: 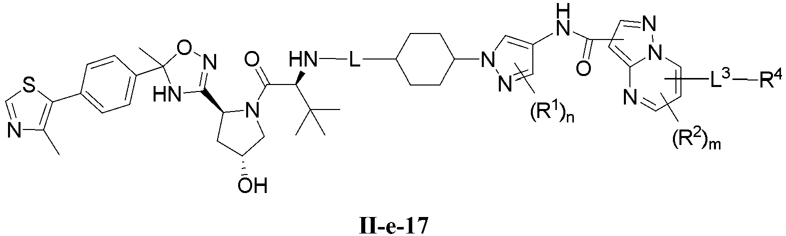 or a pharmaceutically acceptable salt thereof, wherein each of L, L³, R¹, R², R⁴, n, and each m is as defined above and described in embodiments herein, both singly and in combination.

In certain embodiments, the present invention provides the compound of formula **II-e,** wherein LBM is IAP binding moeity 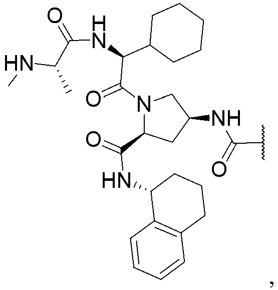 L² is a covalent bond, Ring A is cyclohexyl, Ring C is pyrazolo[1,5-a]pyrimidine, and Ring B is pyrazolyl as shown, thereby forming a compound of formula **II-e-18**: 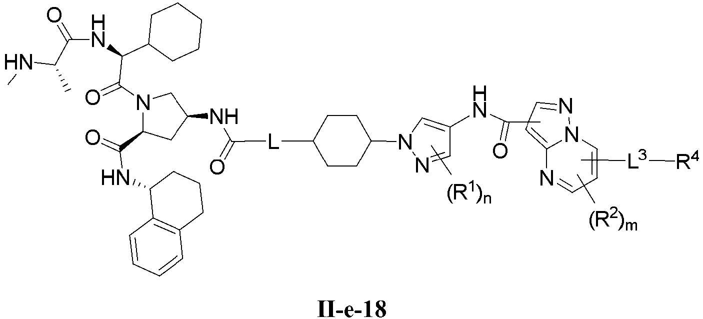 or a pharmaceutically acceptable salt thereof, wherein each of L, R¹, R², R³, n, m, and p is as defined above and described in embodiments herein, both singly and in combination.

In certain embodiments, the present invention provides the compound of formula **II-e,** wherein LBM is MDM2 binding moeity 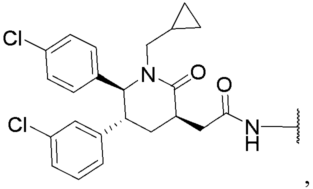 L² is a covalent bond, Ring A is cyclohexyl, Ring C is pyrazolo[1,5-a]pyrimidine, and Ring B is pyrazolyl as shown, thereby forming a compound of formula **II-e-19:** 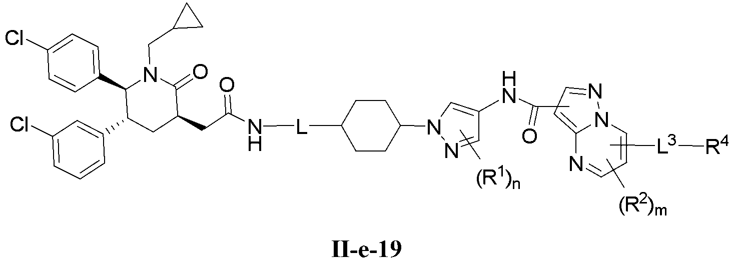 or a pharmaceutically acceptable salt thereof, wherein each of L, R¹, R², R³, n, m, and p is as defined above and described in embodiments herein, both singly and in combination.

In certain embodiments, the present invention provides the compound of formula **II-e,** wherein LBM is MDM2 binding moeity 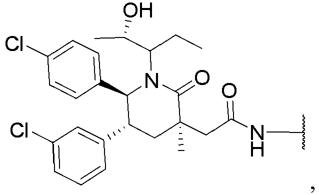 L² is a covalent bond, Ring A is cyclohexyl, Ring C is pyrazolo[1,5-a]pyrimidine, and Ring B is pyrazolyl as shown, thereby forming a compound of formula **II-e-20**: 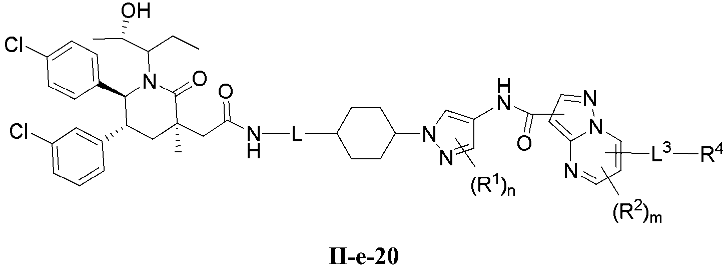 or a pharmaceutically acceptable salt thereof, wherein each of L, R¹, R², R³, n, m, and p is as defined above and described in embodiments herein, both singly and in combination.

In certain embodiments, the present invention provides the compound of formula **II-e,** wherein LBM is MDM2 binding moeity 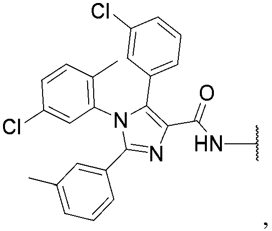 L² is a covalent bond, Ring A is cyclohexyl, Ring C is pyrazolo[1,5-a]pyrimidine, and Ring B is pyrazolyl as shown, thereby forming a compound of formula **II-e-21**: 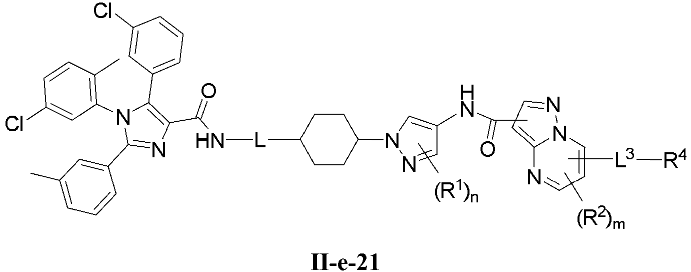 or a pharmaceutically acceptable salt thereof, wherein each of L, R¹, R², R³, n, m, and p is as defined above and described in embodiments herein, both singly and in combination.

In certain embodiments, the present invention provides the compound of formula **II-e,** wherein LBM is MDM2 binding moeity 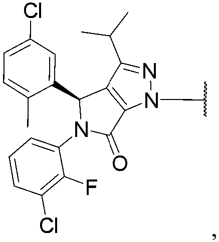 L² is a covalent bond, Ring A is cyclohexyl, Ring C is pyrazolo[1,5-a]pyrimidine, and Ring B is pyrazolyl as shown, thereby forming a compound of formula **II-e-22**: 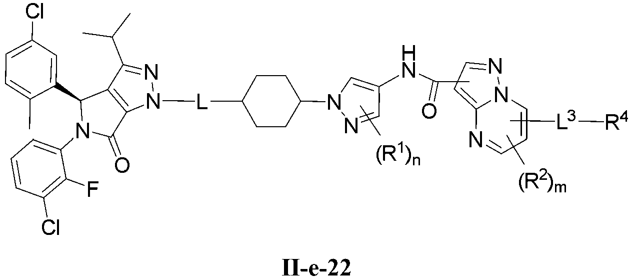 or a pharmaceutically acceptable salt thereof, wherein each of L, R¹, R², R³, n, m, and p is as defined above and described in embodiments herein, both singly and in combination.

In certain embodiments, the present invention provides a compound of formula **I,** wherein LBM is a RPN13 binding moiety thereby forming a compound of formula **I-ddd**: 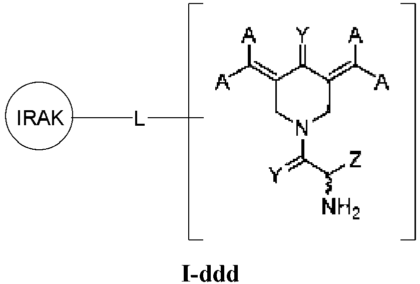 or a pharmaceutically acceptable salt thereof, wherein L and IRAK are as defined above and described in embodiments herein, and wherein each of the variables A, Y, and Z is as described and defined in WO 2019/165229, the entirety of each of which is herein incorporated by reference.

In certain embodiments, the present invention provides a compound of formula **I,** wherein LBM is a Ubr1 binding moiety as described in Shanmugasundaram, K. et al, J. Bio. Chem. 2019, doi: 10.1074/jbc.AC 119.010790, the entirety of each of which is herein incorporated by reference, thereby forming a compound of formula **I-eee-1** or **I-eee-2**: 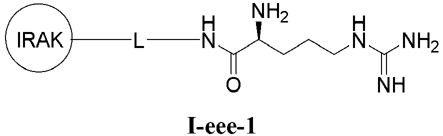 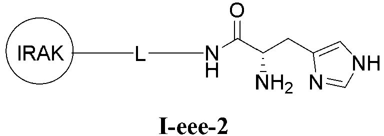 or a pharmaceutically acceptable salt thereof, wherein L and IRAK are as defined above and described in embodiments herein.

In certain embodiments, the present invention provides a compound of formula **I,** wherein LBM is a CRBN binding moiety thereby forming a compound of formula **I-fff:** 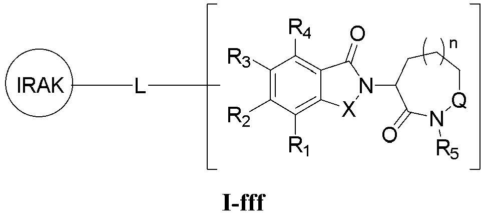 or a pharmaceutically acceptable salt thereof, wherein L and IRAK are as defined above and described in embodiments herein, and wherein each of the variables R₁, R₂, R₃, R₄, R₅, Q, X, and n is as described and defined in US 2019/276474, the entirety of each of which is herein incorporated by reference.

### Lysine Mimetic

In some embodiments, DIM is LBM as described above and herein. In some embodiments, DIM is lysine mimetic. In some embodiments, the covalent attachment of ubiquitin to a member of the IRAK kinase family (*i.e.*, IRAK-1, -2, -3, or -4) is achieved through the action of a lysine mimetic. In some embodiments, upon the binding of a compound of formula **V** to IRAK-1, the moiety that mimics a lysine undergoes ubiquitination thereby marking IRAK-1 for degradation via the Ubiquitin-Proteasome Pathway (UPP). In some embodiments, upon the binding of a compound of formula **V** to IRAK-2, the moiety that mimics a lysine undergoes ubiquitination thereby marking IRAK-2 for degradation via the Ubiquitin-Proteasome Pathway (UPP). In some embodiments, upon the binding of a compound of formula **V** to IRAK-3, the moiety that mimics a lysine undergoes ubiquitination thereby marking IRAK-3 for degradation via the Ubiquitin-Proteasome Pathway (UPP). In some embodiments, upon the binding of a compound of formula **V** to IRAK-4, the moiety that mimics a lysine undergoes ubiquitination thereby marking IRAK-4 for degradation via the Ubiquitin-Proteasome Pathway (UPP).

In some embodiments, DIM is 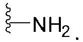 In some embodiments, DIM is 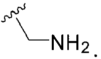 In some embodiments, DIM is

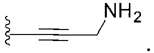

In some embodiments, DIM is selected from those depicted in **Table 1,** below.

In certain embodiments, the present invention provides a compound of Formula **V,** wherein DIM is a lysine mimetic 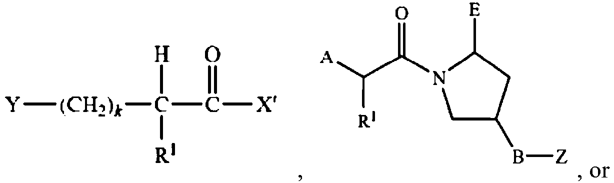 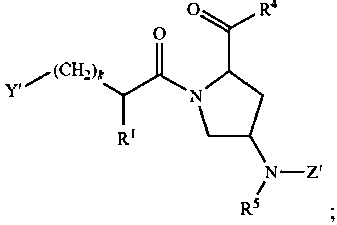 thereby forming a compound of Formulae **V-j-1, V-j-2,** or **V-j-3,** respectively: 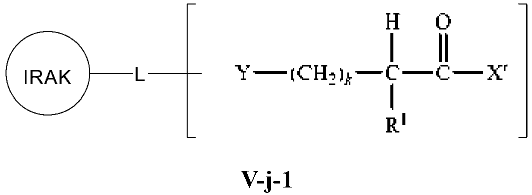 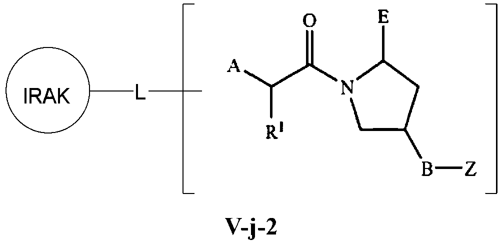 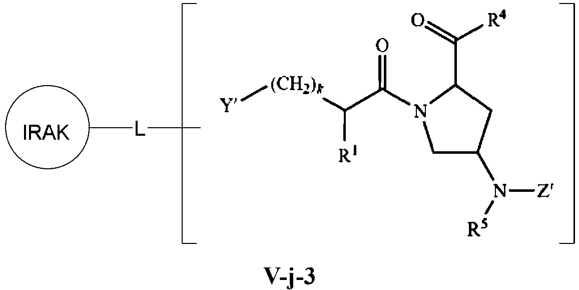 or a pharmaceutically acceptable salt thereof, wherein L and IRAK are as defined above and described in embodiments herein, and wherein each of the variables R¹, R⁴, R⁵, A, B, E, Y, Y', Z, Z', and k are as defined and described in U.S. Pat. No. 7,622,496, the entirety of each of which is herein incorporated by reference.

In some embodiments, the present invention provides the compound of formula **V** wherein DIM is 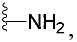 thereby forming a compound of formula **V-k**: 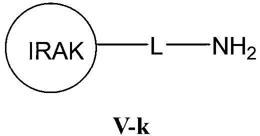 or a pharmaceutically acceptable salt thereof, wherein each of IRAK and L is as defined above and described in embodiments herein, both singly and in combination.

In some embodiments, the present invention provides the compound of formula **V** wherein DIM is 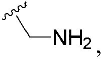 thereby forming a compound of formula **V-1:** 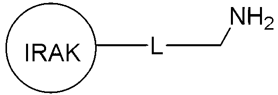 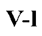 or a pharmaceutically acceptable salt thereof, wherein each of IRAK and L is as defined above and described in embodiments herein, both singly and in combination.

In some embodiments, the present invention provides the compound of formula V wherein DIM is 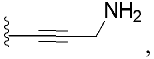 thereby forming a compound of formula **V-m**: 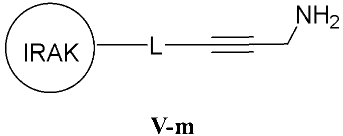 or a pharmaceutically acceptable salt thereof, wherein each of IRAK and L is as defined above and described in embodiments herein, both singly and in combination.

### Hydrogen Atom

In some embodiments, DIM is a hydrogen atom. In some embodiments, the covalent attachment of ubiquitin to one or more members of the IRAK kinase family (*i.e.*, IRAK-1, -2, -3, or -4) is achieved through a provided compound wherein DIM is a hydrogen atom. In some embodiments, upon the binding of a compound of formula **V** to IRAK-1, the DIM moiety being hydrogen effectuates ubiquitination thereby marking IRAK-1 for degradation via the Ubiquitin-Proteasome Pathway (UPP). In some embodiments, upon the binding of a compound of formula **V** to IRAK-2, the DIM moiety being hydrogen effectuates ubiquitination thereby marking IRAK-2 for degradation via the Ubiquitin-Proteasome Pathway (UPP). In some embodiments, upon the binding of a compound of formula **V** to IRAK-3, the DIM moiety being hydrogen effectuates ubiquitination thereby marking IRAK-3 for degradation via the Ubiquitin-Proteasome Pathway (UPP). In some embodiments, upon the binding of a compound of formula **V** to IRAK-4, the DIM moiety being hydrogen effectuates ubiquitination thereby marking IRAK-4 for degradation via the Ubiquitin-Proteasome Pathway (UPP).

In some embodiments, DIM is selected from those depicted in **Table 1,** below.

In some embodiments, the present invention provides the compound of formula **V** wherein DIM is a hydrogen atom, thereby forming a compound of formula **V-n**: 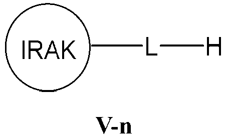 or a pharmaceutically acceptable salt thereof, wherein each of IRAK and L is as defined above and described in embodiments herein, both singly and in combination.

### Linker (L)

As defined above and described herein, L is a bivalent moiety that connects IRAK to LBM or IRAK to DIM.

In some embodiments, L is a bivalent moiety that connects IRAK to LBM. In some embodiments, L is a bivalent moiety that connects IRAK to DIM. In some embodiments, L is a bivalent moiety that connects IRAK to a lysine mimetic. In some embodiments, L is a bivalent moiety that connects IRAK to a hydrogen atom.

In some embodiments, L is a covalent bond or a bivalent, saturated or unsaturated, straight or branched C₁₋₅₀ hydrocarbon chain, wherein 0-6 methylene units of L are independently replaced by -Cy-, -O-, -NR-, -S-, -OC(O)-, -C(O)O-, -C(O)-, -S(O)-, -S(O)₂-, -NRS(O)₂-, - S(O)₂NR-, -NRC(O)-, -C(O)NR-, -OC(O)NR-, -NRC(O)O-, 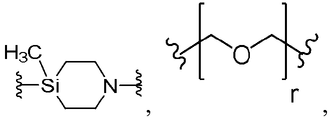 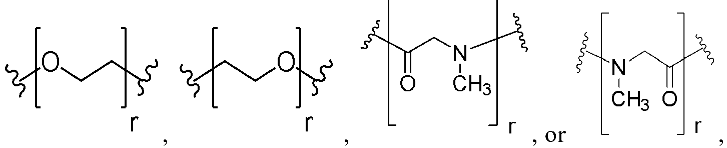 wherein: each -Cy- is independently an optionally substituted bivalent ring selected from phenylenyl, an 8-10 membered bicyclic arylenyl, a 4-7 membered saturated or partially unsaturated carbocyclylenyl, a 4-11 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-11 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur, and wherein r is 0, 1, 2, 3, 4, 5, 6, 7, 8, 9, or 10.

In some embodiments, each -Cy- is independently an optionally substituted bivalent phenylenyl. In some embodiments, each -Cy- is independently an optionally substituted 8-10 membered bicyclic arylenyl. In some embodiments, each -Cy- is independently an optionally substituted 4-11 membered saturated or partially unsaturated carbocyclylenyl. In some embodiments, each -Cy- is independently an optionally substituted 4-7 membered saturated or partially unsaturated spiro carbocyclylenyl. In some embodiments, each -Cy- is independently an optionally substituted 8-10 membered bicyclic saturated or partially unsaturated carbocyclylenyl. In some embodiments, each -Cy- is independently an optionally substituted 4-7 membered saturated or partially unsaturated heterocyclylenyl having 1-2 heteroatoms independently selected from nitrogen, oxygen, and sulfur. In some embodiments, each -Cy- is independently an optionally substituted 4-11 membered saturated or partially unsaturated spiro heterocyclylenyl having 1-2 heteroatoms independently selected from nitrogen, oxygen, and sulfur. In some embodiments, each -Cy- is independently an optionally substituted 8-10 membered bicyclic saturated or partially unsaturated heterocyclylenyl having 1-2 heteroatoms independently selected from nitrogen, oxygen, and sulfur. In some embodiments, each -Cy- is independently an optionally substituted 5-6 membered heteroarylenyl having 1-4 heteroatoms independently selected from nitrogen, oxygen, and sulfur. In some embodiments, each -Cy- is independently an optionally substituted 8-10 membered bicyclic heteroarylenyl having 1-5 heteroatoms independently selected from nitrogen, oxygen, or sulfur.

In some embodiments, -Cy- is 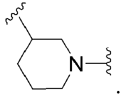 In some embodiments, -Cy- is 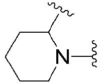 In some embodiments, -Cy- is 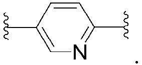 In some embodiments, -Cy- is 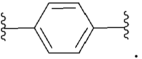 In some embodiments, -Cy- is 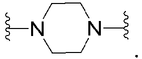 In some embodiments, -Cy- is 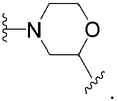 In some embodiments, -Cy- is 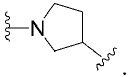 In some embodiments, -Cy- is 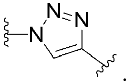 In some embodiments, -Cy- is 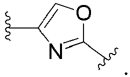 In some embodiments, -Cy- is 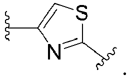 In some embodiments, -Cy- is 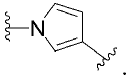 In some embodiments, -Cy- is 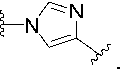 In some embodiments, -Cy- is 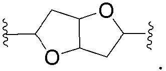 In some embodiments, -Cy- is 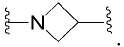 In some embodiments, -Cy- is 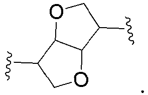 In some embodiments, -Cy- is 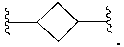 In some embodiments, -Cy- is 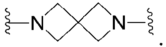 In some embodiments, -Cy- is 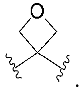 In some embodiments, -Cy- is 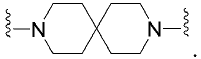 In some embodiments, -Cy- is 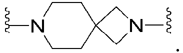 In some embodiments, -Cy- is 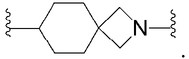 In some embodiments, -Cy- is 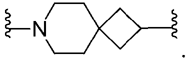 In some embodiments, -Cy- is 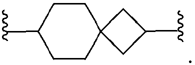 In some embodiments, -Cy- is 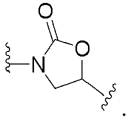 In some embodiments, -Cy- is 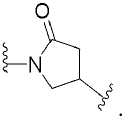 In some embodiments, - Cy- is 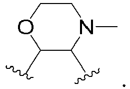 In some embodiments, -Cy- is 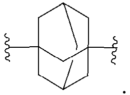 In some embodiments, -Cy- is 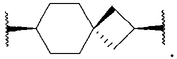 In some embodiments, -Cy- is 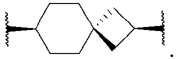 In some embodiments, - 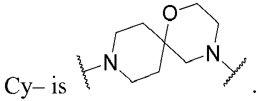 In some embodiments, -Cy- is 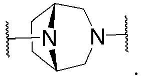 **In** some embodiments, -Cy- is 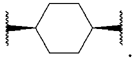 In some embodiments, -Cy- is 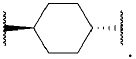 In some embodiments, - Cy- is 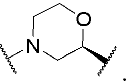 In some embodiments, -Cy- is 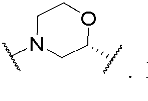 In some embodiments, -Cy-is 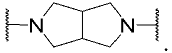 In some embodiments, -Cy- is 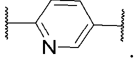 In some embodiments, -Cy-is 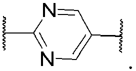 In some embodiments, -Cy- is

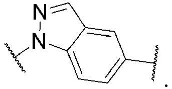

In some embodiments, -Cy- is selected from those depicted in **Table 1,** below.

In some embodiments, L is selected from those depicted in **Table** 1, below.

In some embodiments, r is 0. In some embodiments, r is 1. In some embodiments, r is 2. In some embodiments, r is 3. In some embodiments, r is 4. In some embodiments, r is 5. In some embodiments, r is 6. In some embodiments, r is 7. In some embodiments, r is 8. In some embodiments, r is 9. In some embodiments, r is 10.

In some embodiments, r is selected from those depicted in **Table 1,** below.

In some embodiments, L is 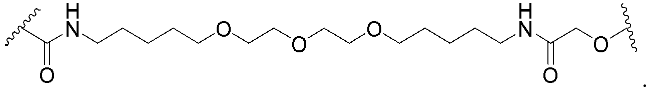 In some embodiments, L is 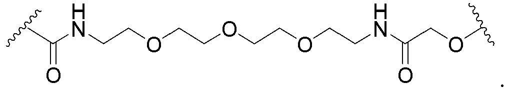 In some embodimentsn, L is 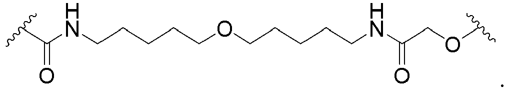 In some embodiments, L is 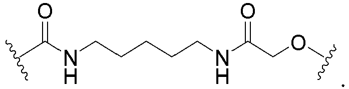 In some embodiments, L is 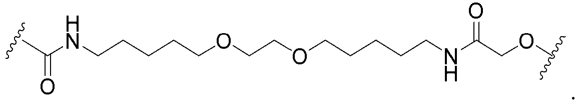 In some embodiments, L is 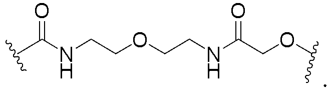 In some embodiments, L is 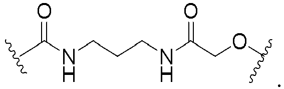 In some embodiments, L is 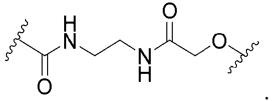 In some embodiments, L is 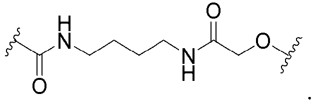 In some embodiments, L is 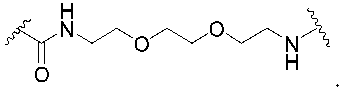 In some embodiments, L is 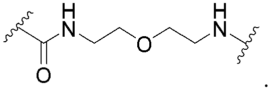 In some embodiments, L is 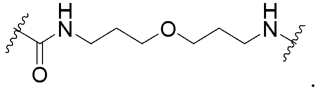 In some embodiments, L is 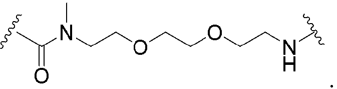 In some embodiments, L is 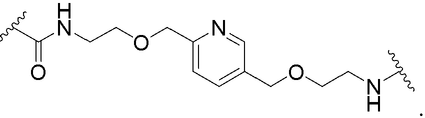 In some embodiments, L 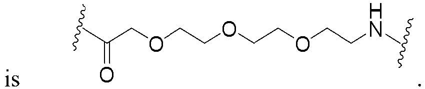 In some embodiments, L is 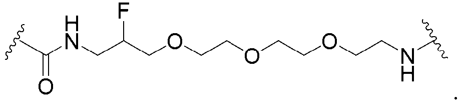 In some embodiments, L is 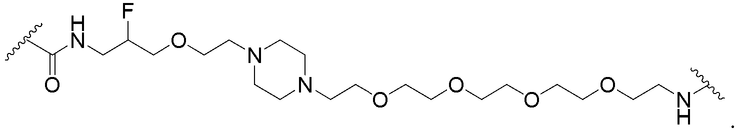 In some embodiments, L is 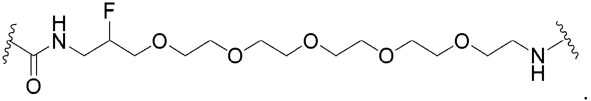 In some embodiments, L is 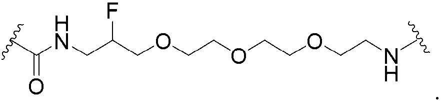 In some embodiments, L is 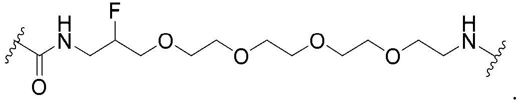 In some embodiments, L is 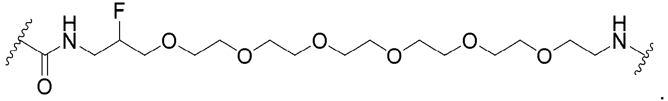 In some embodiments, L is 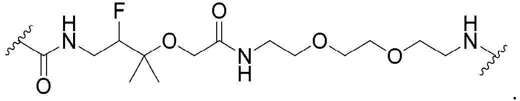 In some embodiments, L is 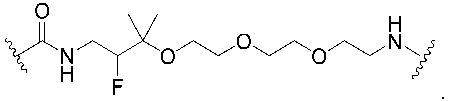 In some embodiments, L is 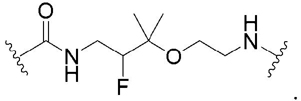 In some embodiments, L is 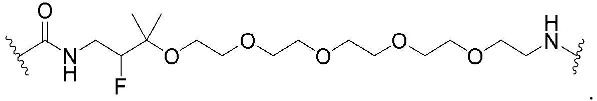 In some embodiments, L is 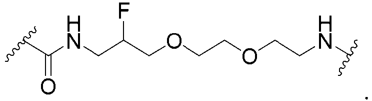 In some embodiments, L is 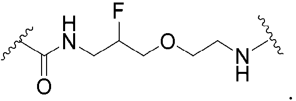 In some embodiments, L is 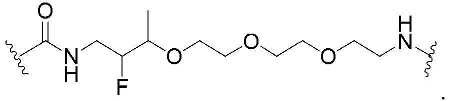 In some embodiments, L is 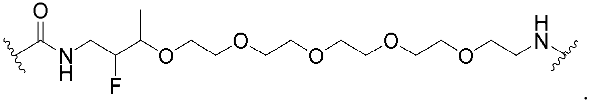 In some embodiments, L is 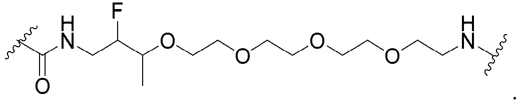 In some embodiments, L is 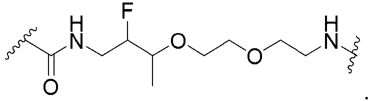 In some embodiments, L is 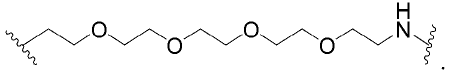 In some embodiments, L is 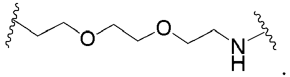 In some embodiments, L is 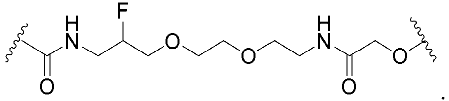 In some embodiments, L is 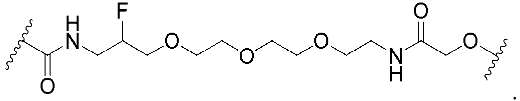 In some embodiments, L is 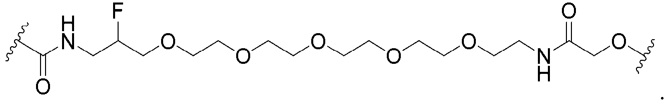 In some embodiments, L is 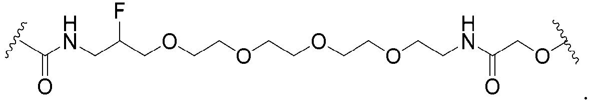 In some embodiments, L is 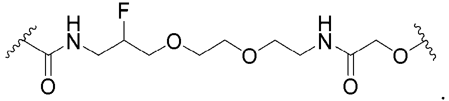 In some embodiments, L is 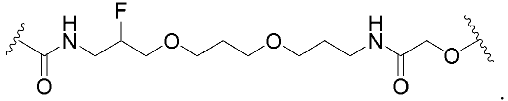 In some embodiments, L is 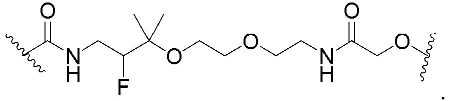 In some embodiments, L is 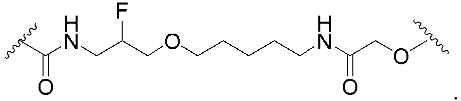 In some embodiments, L is 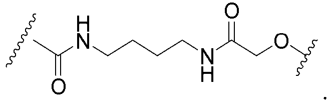 In some embodiments, L is 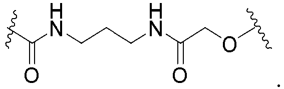 In some embodiments, L is 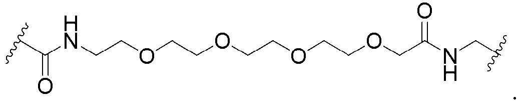 In some embodiments, L is 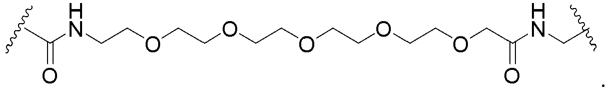 In some embodiments, L is 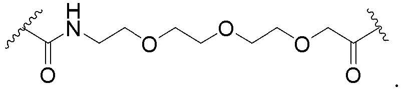 In some embodiments, L is 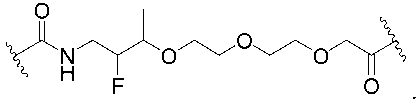 In some embodiments, L is 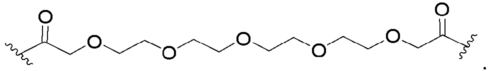 In some embodiments, L is 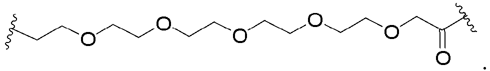 In some embodiments, L is 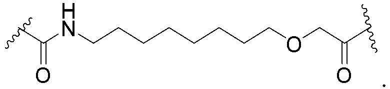 In some embodiments, L is 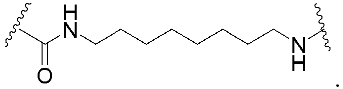 In some embodiments, L is 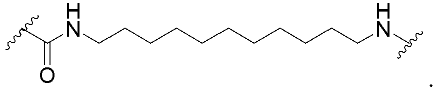 In some embodiments, L is 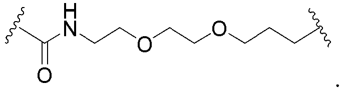 In some embodiments, L is 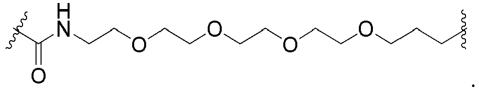 In some embodiments, L is 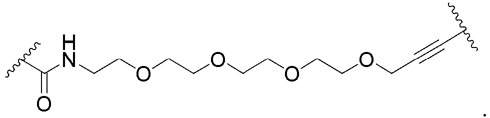 In some embodiments, L is 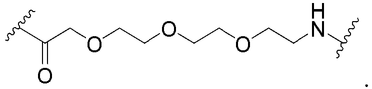 In some embodiments, L is 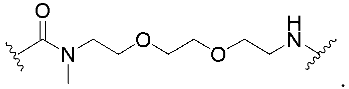 In some embodiments, L is 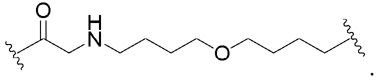 In some embodiments, L is 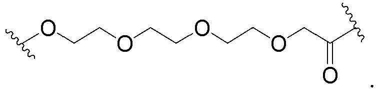 In some embodiments, L is 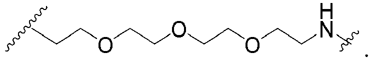 In some embodiments, L is 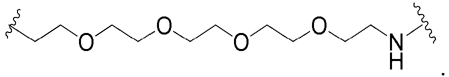 In some embodiments, L is 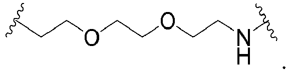 In some embodiments, L is 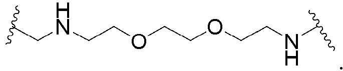 In some embodiments, L is 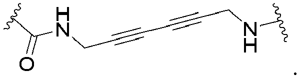 In some embodiments, L is 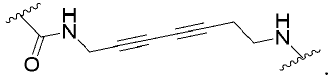 In some embodiments, L is 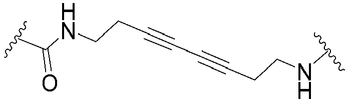 . In some embodiments, L is 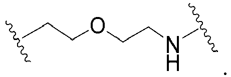 In some embodiments, L is 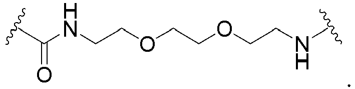 In some embodiments, L is 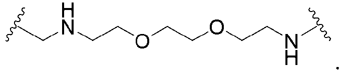 In some embodiments, L is 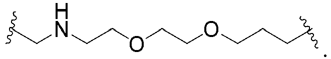 In some embodiments, L is 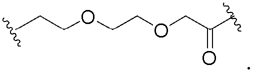 In some embodiments, L is 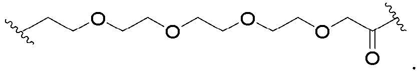 In some embodiments, L is 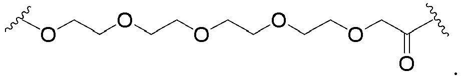 In some embodiments, L is 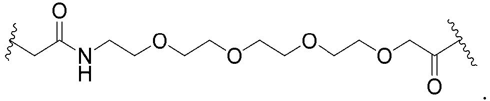 In some embodiments, L is 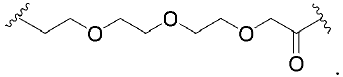 In some embodiments, L is 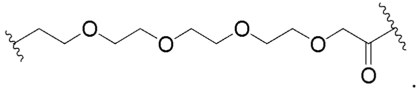 In some embodiments, L is 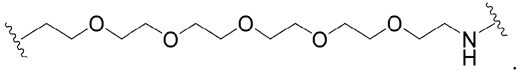 In some embodiments, L is 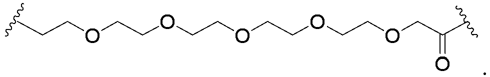 In some embodiments, L is 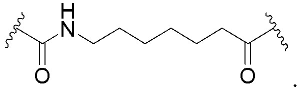 In some embodiments, L is 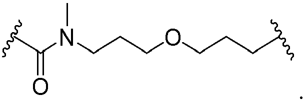 In some embodiments, L is 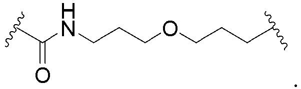 In some embodiments, L is 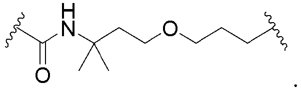 In some embodiments, L is 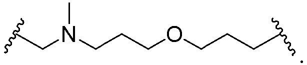 In some embodiments, L is 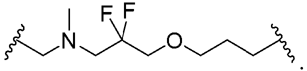 In some embodiments, L is 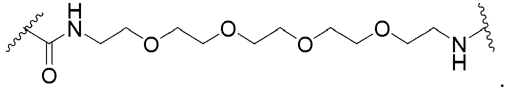 In some embodiments, L is 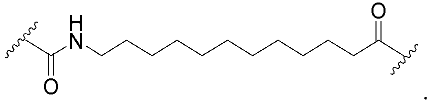 In some embodiments, L is 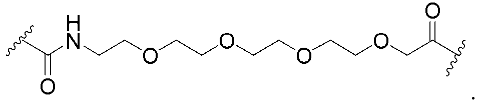 In some embodiments, L is 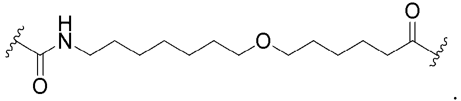 In some embodiments, L is 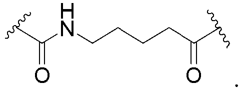 In some embodiments, L is 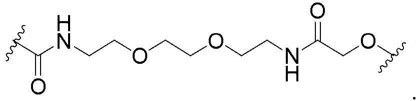 In some embodiments, L is 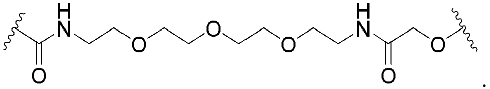 In some embodiments, L is 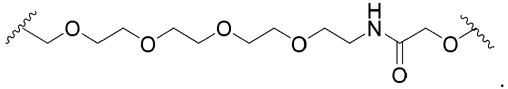 In some embodiments, L 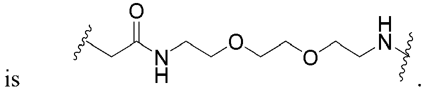 In some embodiments, L is 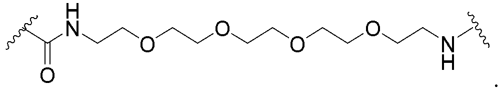 In some embodiments, L is 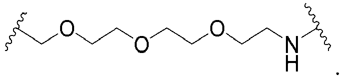 In some embodiments, L is 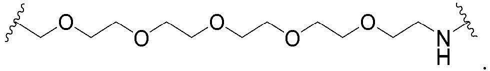 In some embodiments, L is 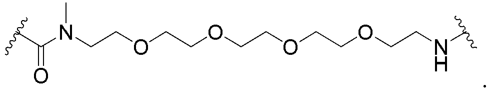 In some embodiments, L is 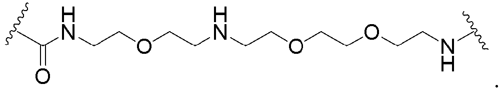 In some embodiments, L is 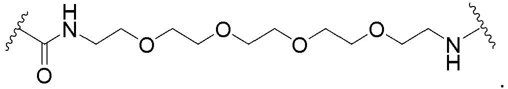 In some embodiments, L is 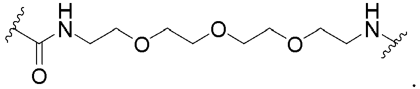 In some embodiments, L is 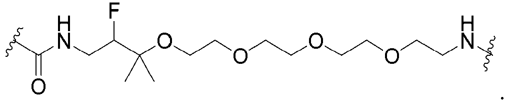 In some embodiments, L is 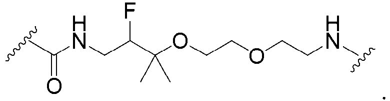 In some embodiments, L is 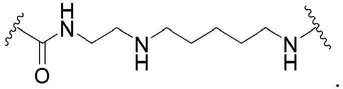 In some embodiments, L is 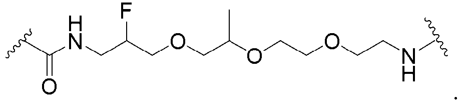 In some embodiments, L is 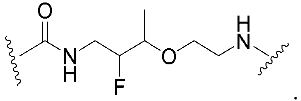 In some embodiments, L is 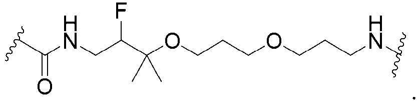 In some embodiments, L is 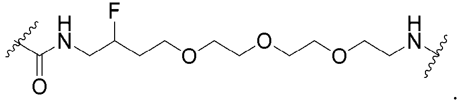 In some embodiments, L is 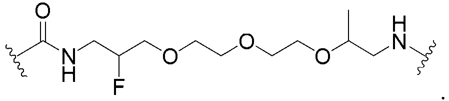 In some embodiments, L is 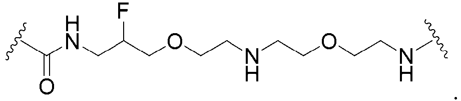 In some embodiments, L is 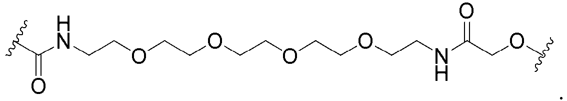 In some embodiments, L is 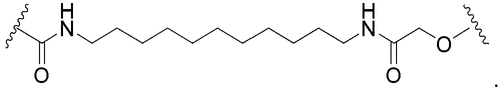 In some embodiments, L is 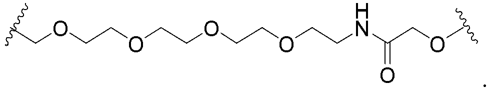 In some embodiments, L is 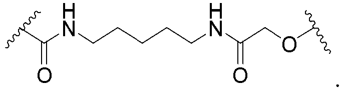 In some embodiments, L is 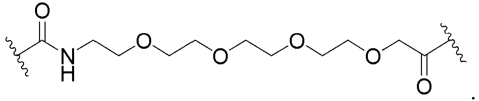 In some embodiments, L is 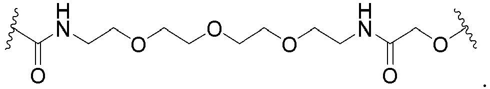 In some embodiments, L is 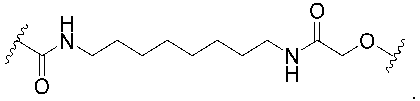 In some embodiments, L is 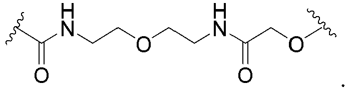 In some embodiments, L is 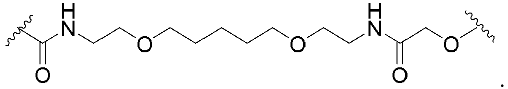 In some embodiments, L is 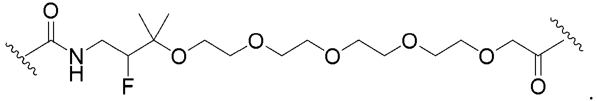 In some embodiments, L is 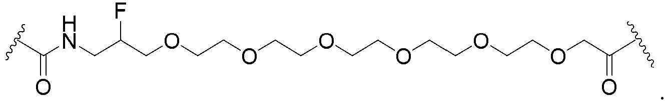 In some embodiments, L is 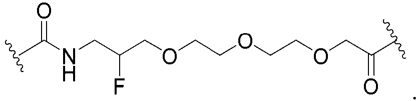 In some embodiments, L is 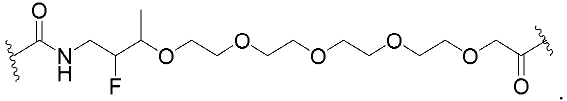 In some embodiments, L is 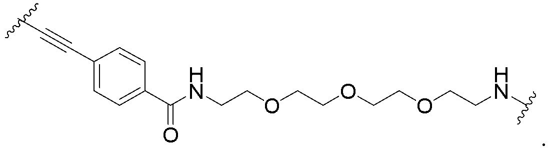 In some embodiments, L is 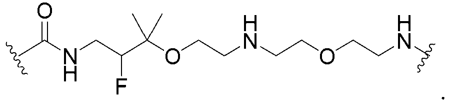 In some embodiments, L is 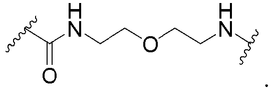 In some embodiments, L is 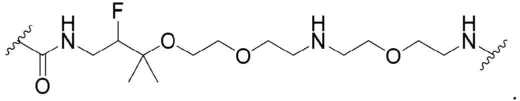 In some embodiments, L is 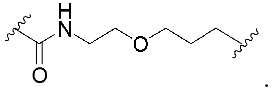 In some embodiments, L is 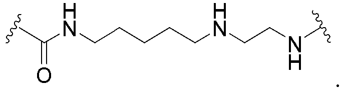 In some embodiments, L is 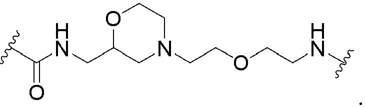 In some embodiments, L is 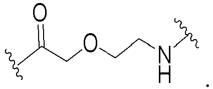 In some embodiments, L is 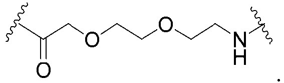 In some embodiments, L is 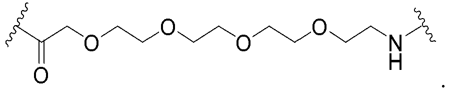 In some embodiments, L is 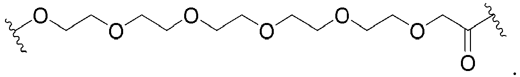 In some embodiments, L is 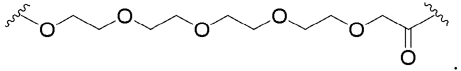 In some embodiments, L is 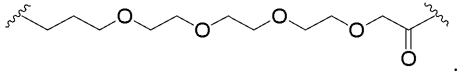 In some embodiments, L is 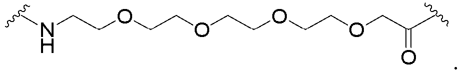 In some embodiments, L is 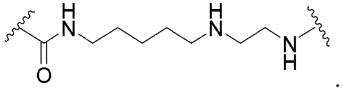 In some embodiments, L is 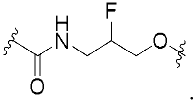 In some embodiments, L is 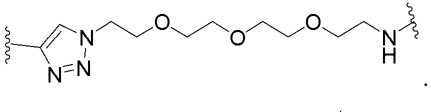 In some embodiments, L is 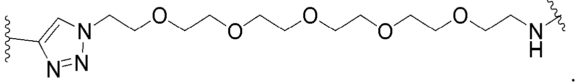 In some embodiments, L is 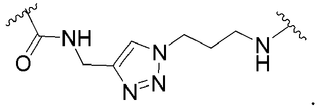 In some embodiments, L is 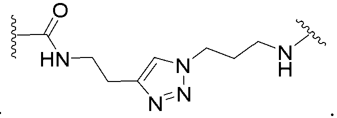 In some embodiments, L is 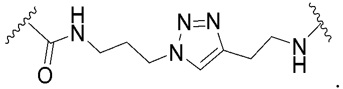 In some embodiments, L is 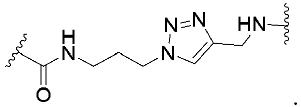 In some embodiments, L is 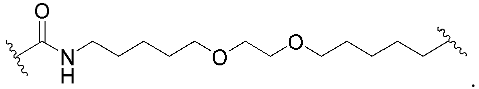 In some embodiments, L is 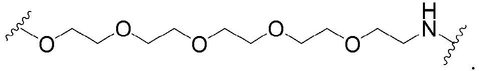 In some embodiments, L is 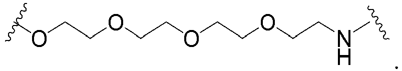 In some embodiments, L is 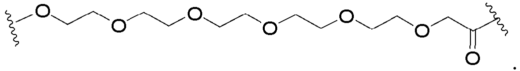 In some embodiments, L is 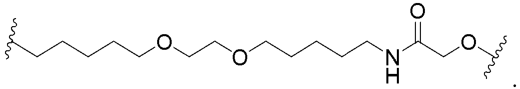 In some embodiments, L is 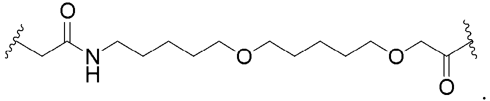 In some embodiments, L is 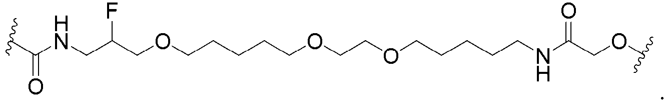 In some embodiments, L is 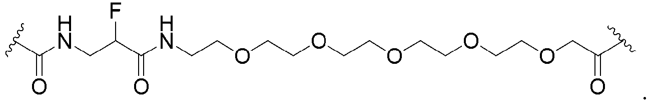 In some embodiments, L is 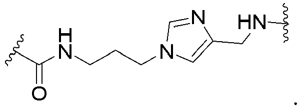 In some embodiments, L is 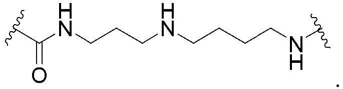 In some embodiments, L is 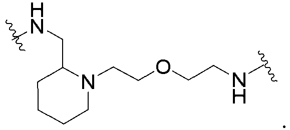 In some embodiments, L is 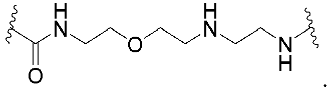 In some embodiments, L is 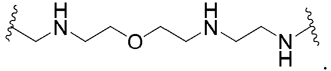 In some embodiment, L is 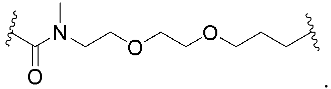 In some embodiment, L is 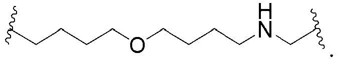 In some embodiment, L is 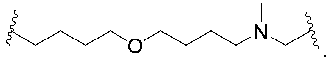 In some embodiments, L is 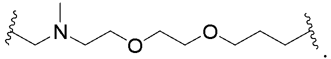 In some embodiments, L is 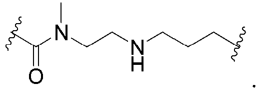 In some embodiments, L is 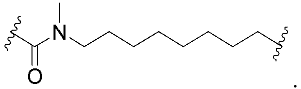 In some embodiments, L is 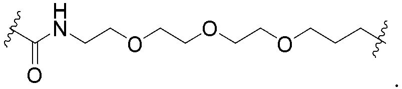 In some embodiments, L is 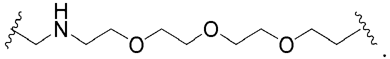 In some embodiments, L is 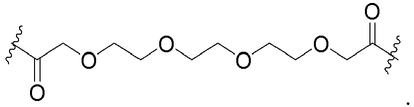 In some embodiments, L is 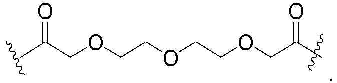 In In some embodiments, L is 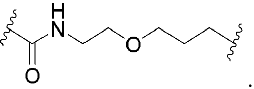 In some embodiments, L is 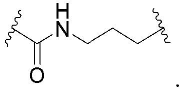 In some embodiments, L is 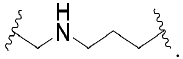 In some embodiments, L is 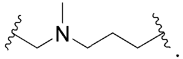 In some embodiments, L is 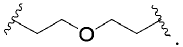 In some embodiments, L is 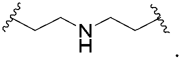 In some embodiments, L is 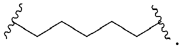 In some embodiments, L is 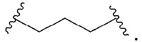 **In** some embodiments, L is 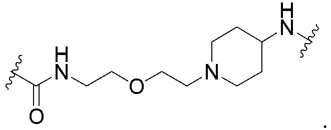 In some embodiments, L is 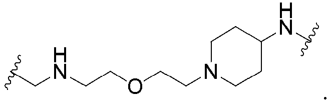 In some embodiments, L is 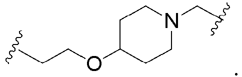 In some embodiments, L is 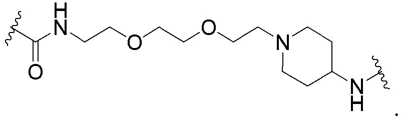 In some embodiments, L is 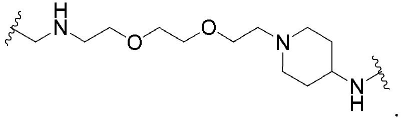 In some embodiments, L is 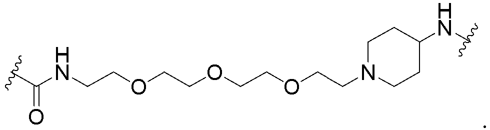 In some embodiments, L is 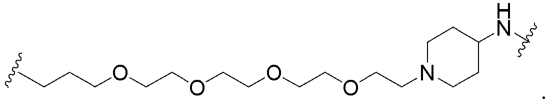 In some embodiments, L is 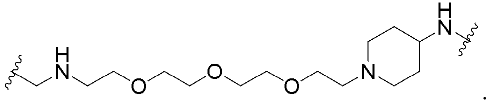 In some embodiments, L is 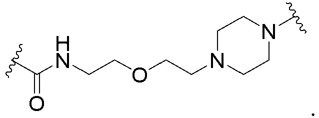 In some embodiments, L is 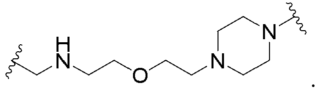 In some embodiments, L is 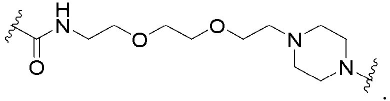 In some embodiments, L is 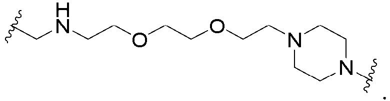 In some embodiments, L is 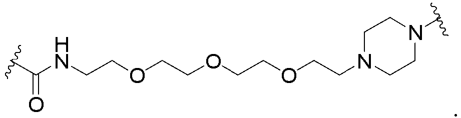 In some embodiments, L is 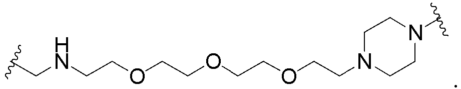 In some embodiments, L is 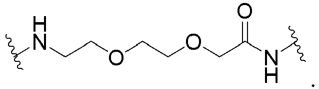 In some embodiments, L is 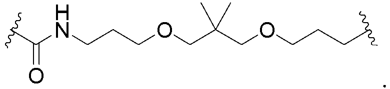 In some embodiments, L is 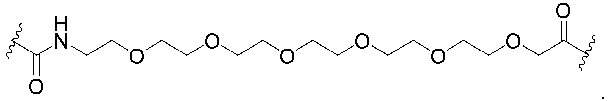 In some embodiments, L is 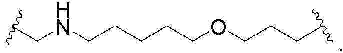 In some embodiments, L is 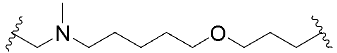 . In some embodiments, L is 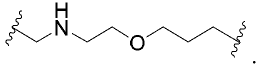 In some embodiments, L is 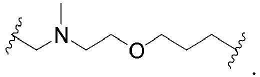 In some embodiments, L is 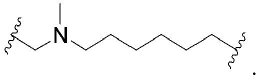 In some embodiments, L is 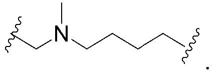 In some embodiments, L is 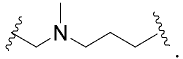 In some embodiments, L is 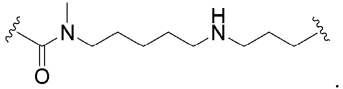 In some embodiments, L is 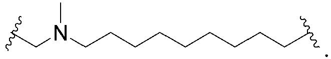 In some embodiments, L is 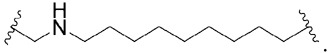 In some embodiments, L is 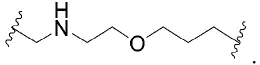 In some embodiments, L is 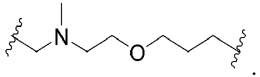 In some embodiments, L is 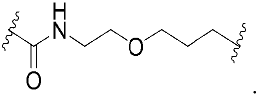 In some embodiments, L is 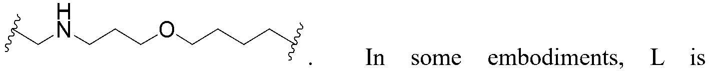 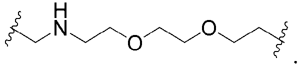 In some embodiments, L is 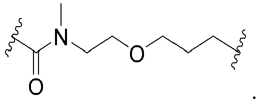 In some embodiments, L is 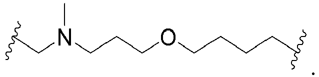 In some embodiments, L is 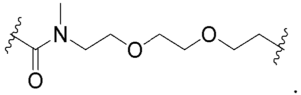 In some embodiments, L is 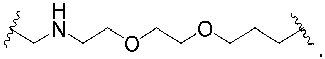 In some embodiments, L is 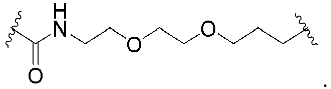 In some embodiments, L is 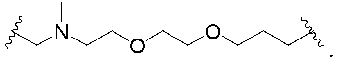 In some embodiments, L is 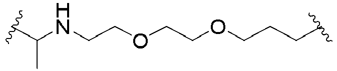 . In some embodiments, L is 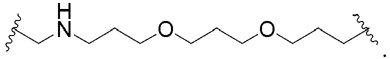 In some embodiment, L is 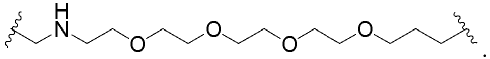 In some embodiment, L is 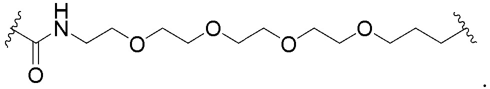 In some embodiments, L is 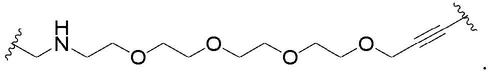 In some embodiments, L is 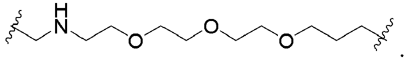 In some embodiments, L is 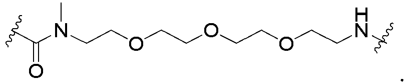 In some embodiments, L is 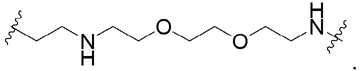 **In** some embodiments, L is 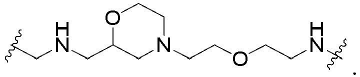 In some embodiments, L is 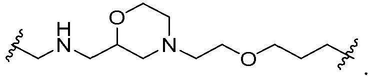 In some embodiments, L is 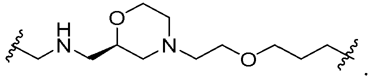 In some embodiments, L is 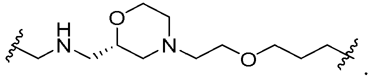 In some embodiments, L is 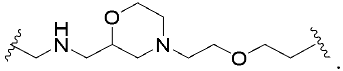 In some embodiments, L is 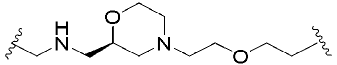 . In some embodiments, L is 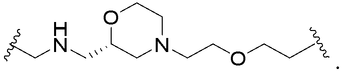 In some embodiments, L is 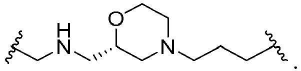 In some embodiments, L is 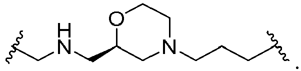 In some embodiments, L is 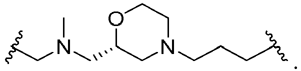 In some embodiments, L is 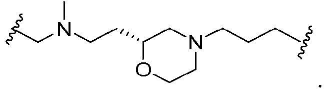 In some embodiments, L is 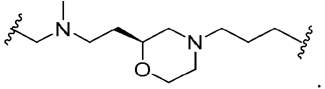 In some embodiments, L is 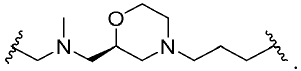 In some embodiments, L is 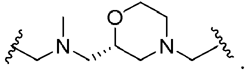 In some embodiments, L is 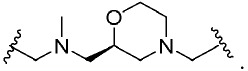 In some embodiments, L is 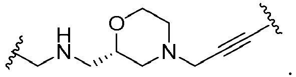 In some embodiments, L is 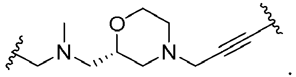 In some embodiments, L is 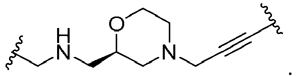 **In** some embodiments, L is 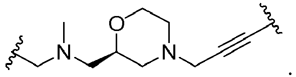 In some embodiments, L is 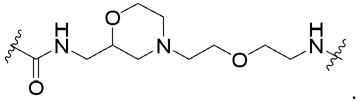 In some embodiments, L is 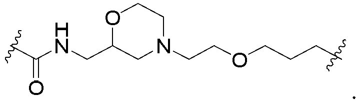 In some embodiments, L is 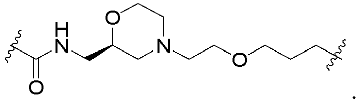 In some embodiments, L is 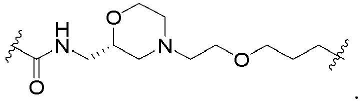 In some embodiments, L is 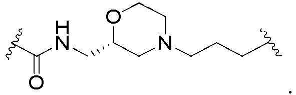 In some embodiments, L is 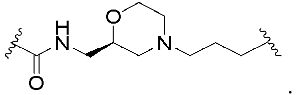 In some embodiments, L is 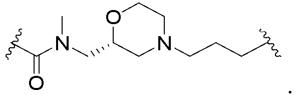 In some embodiments, L is 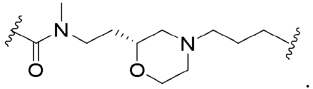 In some embodiments, L is 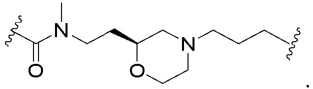 In some embodiments, L is 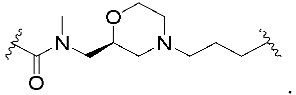 In some embodiments, L is 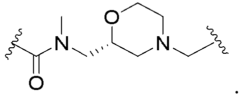 In some embodiments, L is 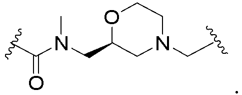 In some embodiments, L is 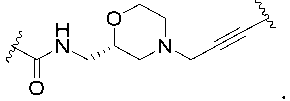 In some embodiments, L is 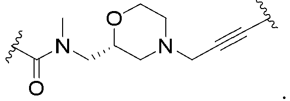 In some embodiments, L is 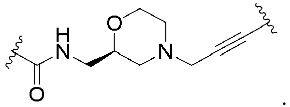 In some embodiments, L is 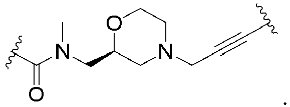 In some embodiments, L is 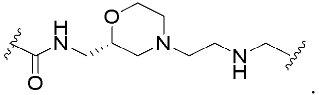 In some embodiments, L is 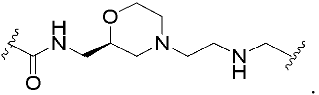 In some embodiments, L is 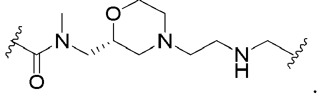 In some embodiments, L is 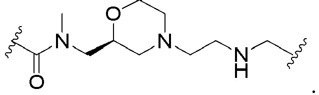 In some embodiments, L is 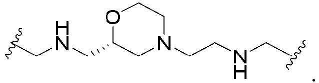 In some embodiments, L is 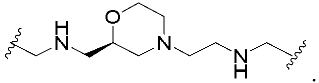 In some embodiments, L is 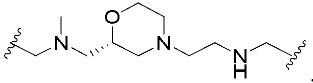 . In some embodiments, L is 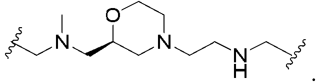 In some embodiments, L is 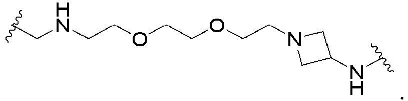 In some embodiments, L is 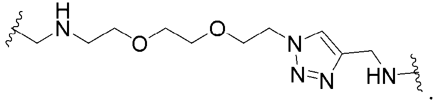 In some embodiments, L is 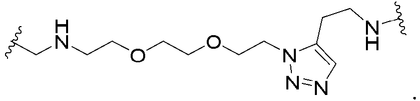 In some embodiments, L is 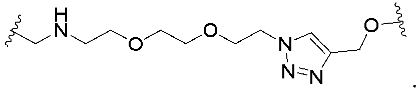 In some embodiments, L is 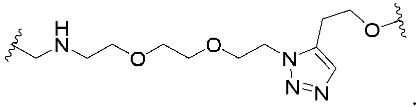 In some embodiments, L is 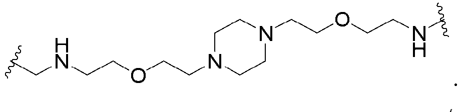 In some embodiments, L is 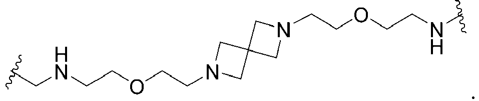 In some embodiments, L is 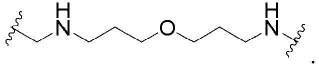 In some embodiments, L is 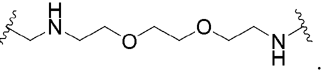 In some embodiments, L is 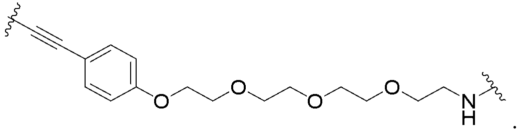 In some embodiments, L is 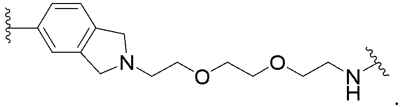 In some embodiments, L is 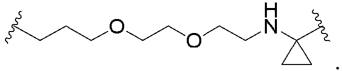 In some embodiments, L is 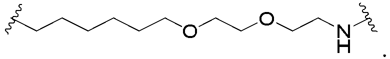 In some embodiments, L is 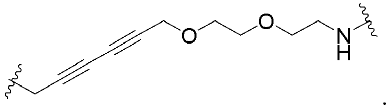 In some embodiments, L is 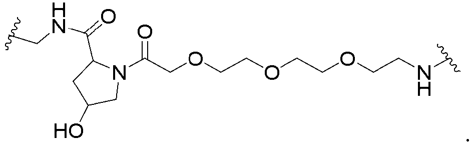 In some embodiments, L is 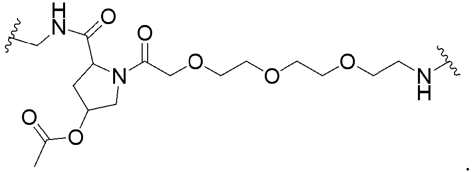 In some enbodiments, L is 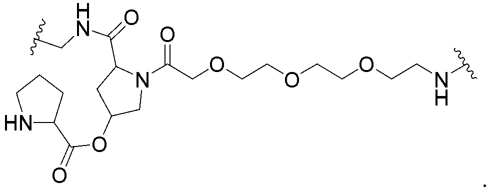 In some embodiments, L is 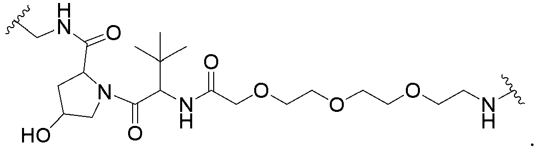 In some embodiments, L is 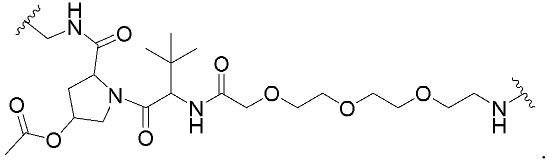 In some embodiments, L is 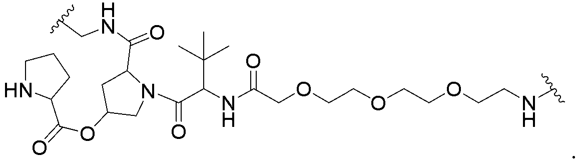 In some embodiments, L is 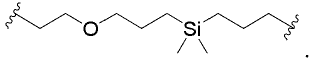 In some embodiments, L is 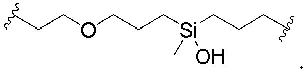 In some embodiments, L is 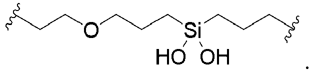 **In** some embodiments, L is 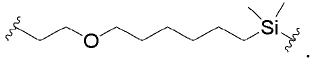 In some embodiments, L is 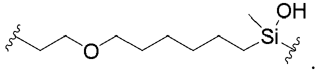 In some embodiments, L is 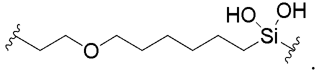 In some embodiments, L is 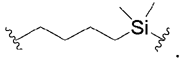 In some embodiments, L is 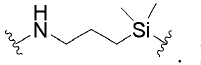 In some embodiments, L is 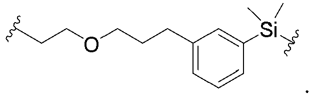 In some embodiments, L is 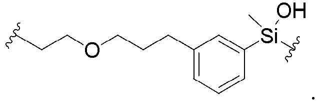 In some embodiments, L is 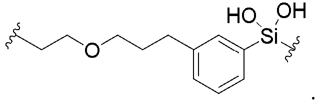 In some embodiments, L is 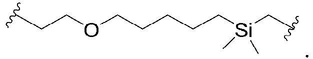 In some embodiments, L is 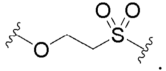 In some embodiments, L is 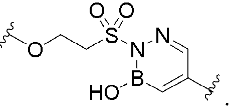 In some embodiments, L is 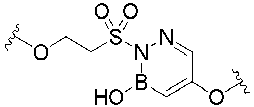 . In some embodiments, L is 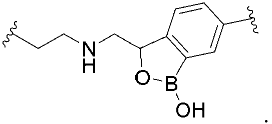 **In** some embodiments, L is 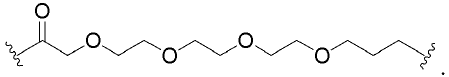 In some embodiments, L is 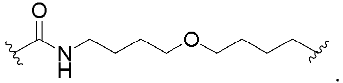 In some embodiments, L is 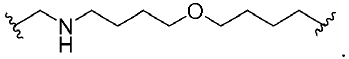 In some embodiments, L is 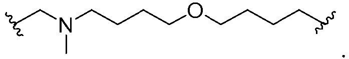 In some embodiments, L is 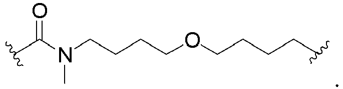 In some embodiments, L is 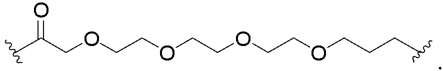 In some embodiments, L is 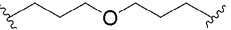 . In some embodiments, L is 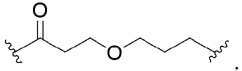 In some embodiments, L is 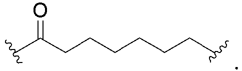 In some embodiments, L is 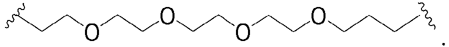 In some embodiments, L is 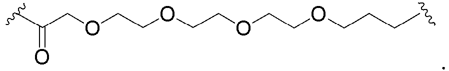 In some embodiments, L is 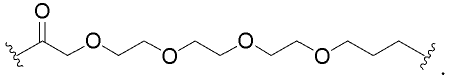 In some embodiments, L is 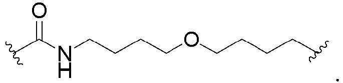 In some embodiments, L is 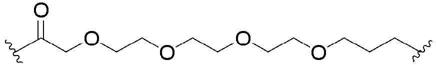 . In some embodiments, L is 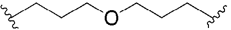 . In some embodiments, L is 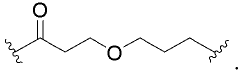 In some embodiments, L is 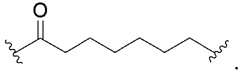 In some embodiments, L is 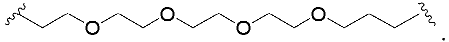 In some embodiments, L is 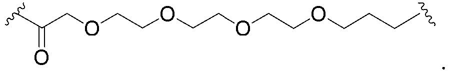 In some embodiments, L is 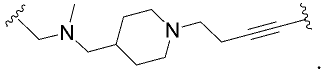 In some embodiments, L is 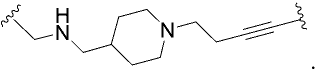 In some embodiments, L is 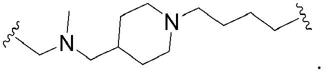 In some embodiments, L is 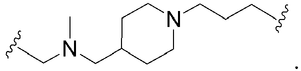 In some embodiments, L is 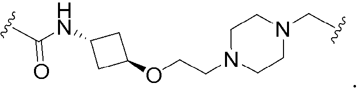 In some embodiments, L is 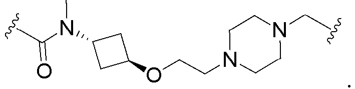 In some embodiments, L is 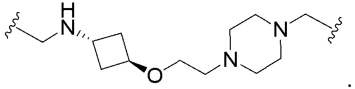 In some embodiments, L is 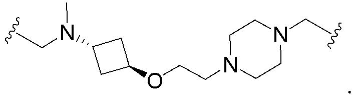 In some embodiments, L is 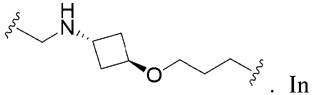 some embodiments, L is 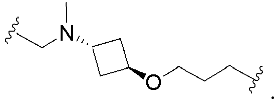 In some embodiments, L is 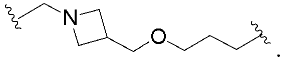 In some embodiments, L is 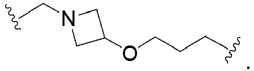 In some embodiments, L is 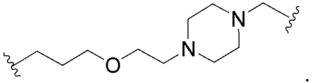 In some embodiments, L is 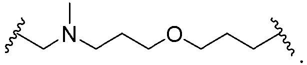 In some embodiments, L is 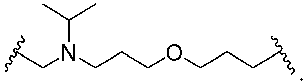 In some embodiments, L is 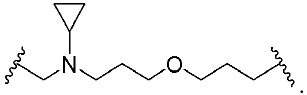 In some embodiments, L is 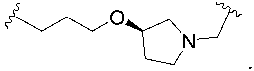 In some embodiments, L is 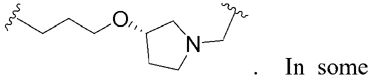 embodiments, L is 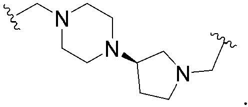 In some embodiments, L is 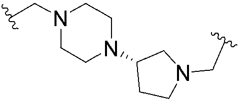 . In some embodiments, L is 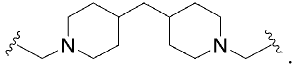 In some embodiments, L is 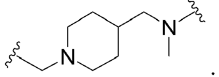 In some embodiments, L is 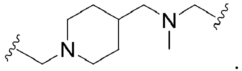 In some embodiments, L is 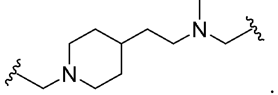 In some embodiments, L is 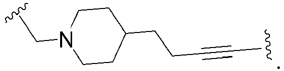 In some embodiments, L is 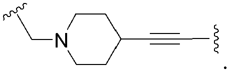 In some embodiments, L is 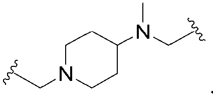 . In some embodiments, L is 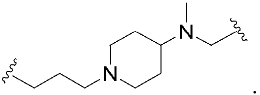 In some embodiments, L is 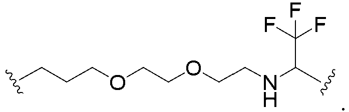 In some embodiments, L is 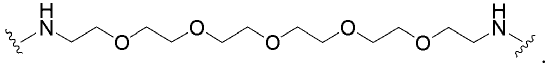 In some embodiments, L is 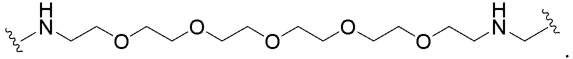 In some embodiments, L is 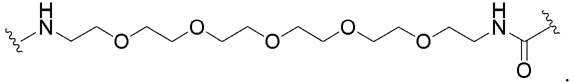 In some embodiments, L is 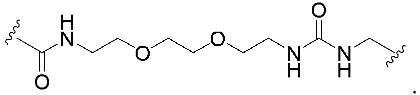 In some embodiments, L is 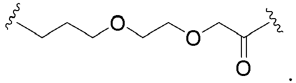 **In** some embodiments, L is 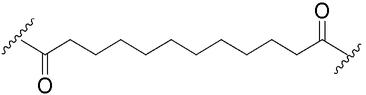 . In some embodiments, L is 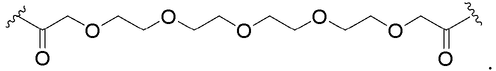 In some embodiments, L is 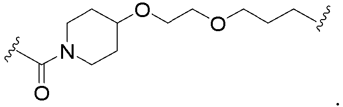 In some embodiments, L is 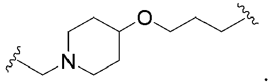 In some embodiments, L is 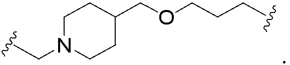 In some embodiments, L is 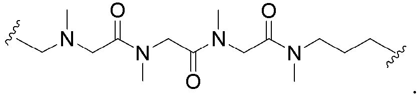 In some embodiments, L is 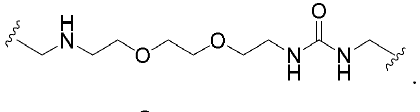 In some embodiment, L is 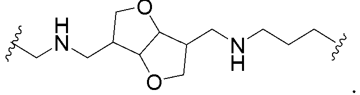 In some embodiments, L is 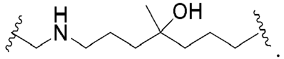 In some embodiments, L is 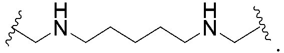 In some embodiments, L is 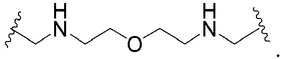 In some embodiments, L is 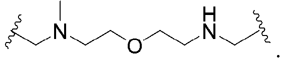 In some embodiments, L is 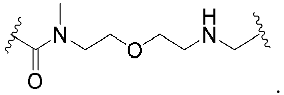 In some embodiments, L is 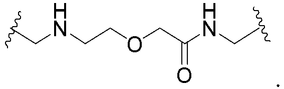 In some embodiments, L is 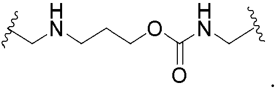 In some embodiments, L is 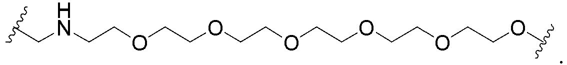 In some embodiments, L is 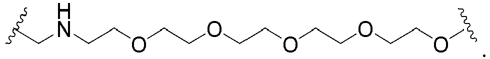 In some embodiments, L 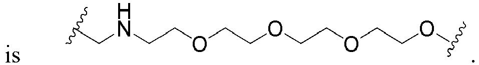 In some embodiments, L is 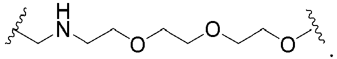 In some embodiments, L is 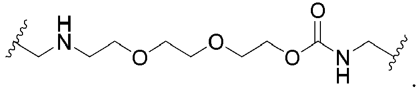 In some embodiments, L is 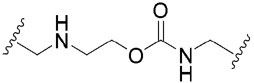 . In some embodiments, L is 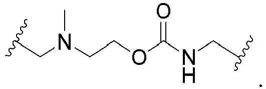 In some embodiments, L is 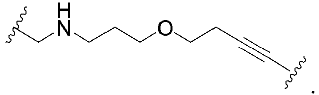 In some embodiments, L is 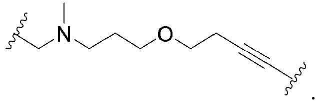 In some embodiments, L is 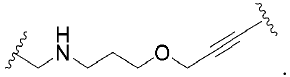 In some embodiments, L is 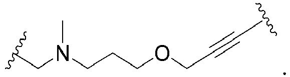 In some embodiments, L is 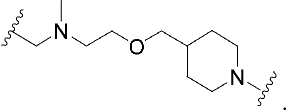 In some embodiments, L is 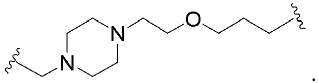 In some embodiments, L is 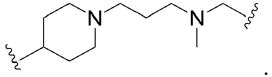 In some embodiments, L is 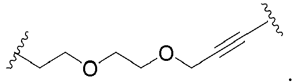 In some embodiments, L is 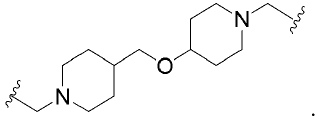 In some embodiments, L is 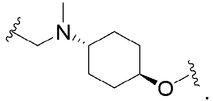 In some embodiments, L is 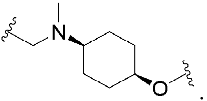 In some embodiments, 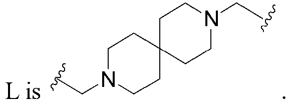 In some embodiments, L is 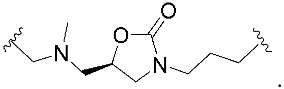 In some embodiments, L is 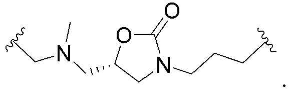 In some embodiments, L is 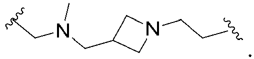 In some embodiments, L is 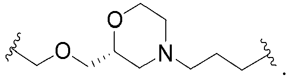 In some embodiments, L is 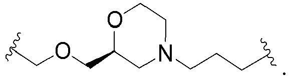 In some embodiments, L is 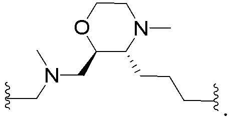 In some embodiments, L is 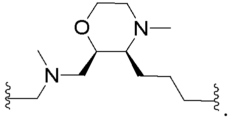 In some embodiments, L is 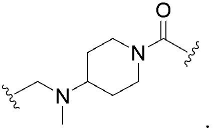 In some embodiments, L is 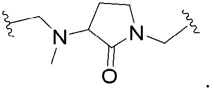 In some embodiments, L is 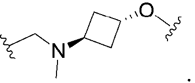 In some embodiments, L is 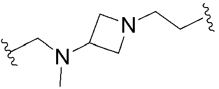 . In some embodiments, L is 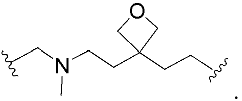 In some embodiments, L is 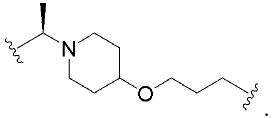 In some embodiments, L is 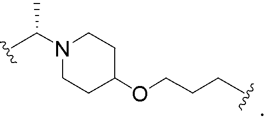 In some embodiments, L is 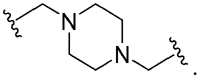 In some embodiments, L is 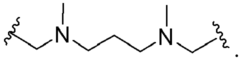 In some embodiments, L is 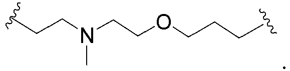 In some embodiments, L is 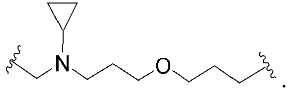 In some embodiments, L is 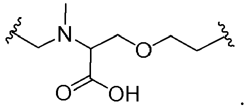 In some embodiments, L is 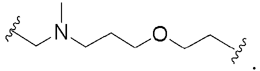 In some embodiments, L is 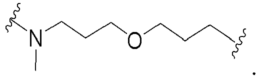 In some embodiments, L is 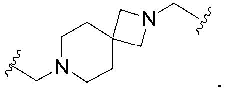 In some embodiments, L is 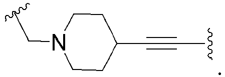 In some embodiments, L is 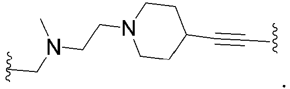 In some embodiments, L is 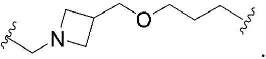 In some embodiments, L is a covalent bond. In some embodiments, L is 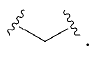 . In some embodiments, L is 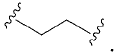 In some embodiments, L is 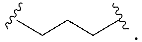 In some embodiments, L is 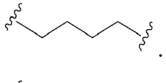 In some embodiments, L is 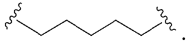 In some embodiments, L is 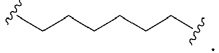 In some embodiments, L is 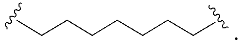 In some embodiments, L is 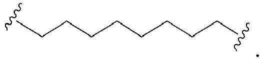 In some embodiments, L is 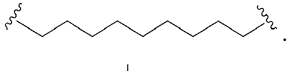 In some embodiments, L is a covalent bond. In some embodiments, L is 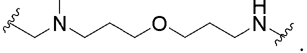 In some embodiments, L is 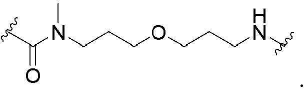 In some embodiments, L is 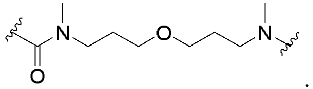 In some embodiments, L is 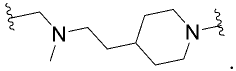 In some embodiments, L is 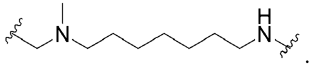 In some embodiments, L is 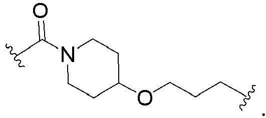 In some embodiments, L is 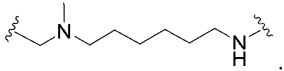 In some embodiments, L is 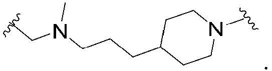 In some embodiments, L is 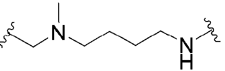 . In some embodiments, L is 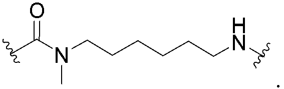 In some embodiments, L is 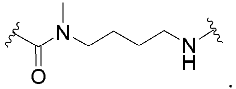 In some embodiments, L is 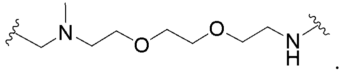 **In** some embodiments, L is 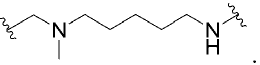 In some embodiments, L is 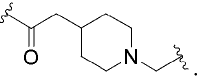 In some embodiments, L is 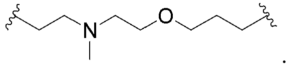 In some embodiments, L is 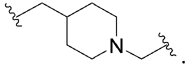 In some embodiments, L is 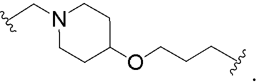 In some embodiments, L is 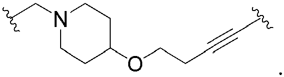 In some embodiments, L is 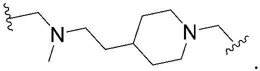 In some embodiments, L is 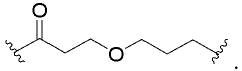 In some embodiments, L is 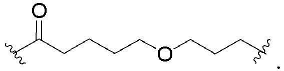 In some embodiments, L is 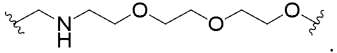 In some embodiments, L is 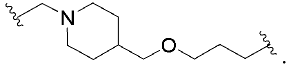 In some embodiments, L is 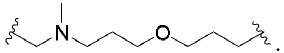 In some embodiments, L is 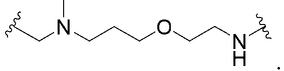 In some embodiments, L is 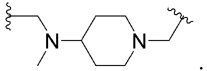 In some embodiments, L is 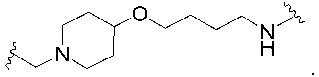 In some embodiments, L is 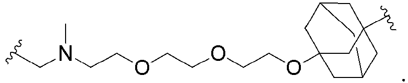 In some embodiments, L is 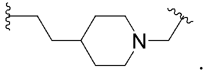 In some embodiments, L is 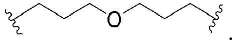 In some embodiments, L is 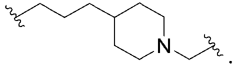 In some embodiments, L is 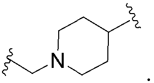 In some embodiments, L is 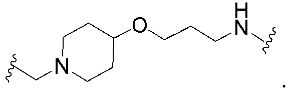 In some embodiments, L is 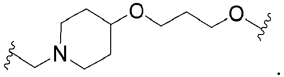 In some embodiments, L is 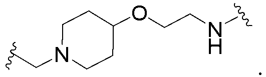 In some embodiments, L is 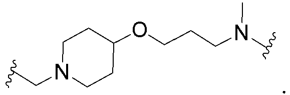 In some embodiments, L is 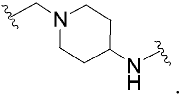 In some embodiments, L is 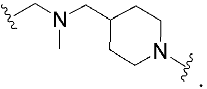 In some embodiments, L is 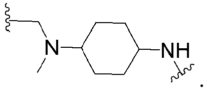 In some embodiments, L is 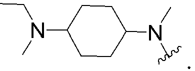 In some embodiments, L is 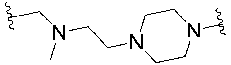 . In some embodiments, L is 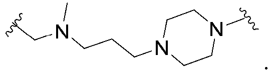 In some embodiments, L is 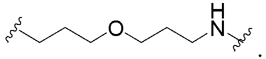 In some embodiments, L is 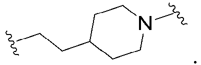 In some embodiments, L is 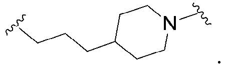 In some embodiments, L is 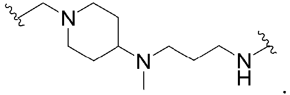 In some embodiments, L is 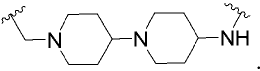 In some embodiments, L is 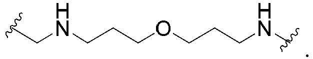 In some embodiments, L is 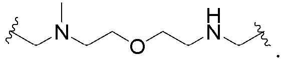 In some embodiments, L is 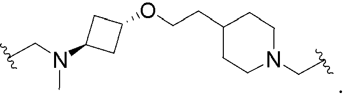 In some embodiments, L is 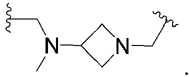 In some embodiments, L is 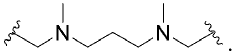 In some embodiments, L is 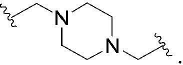 In some embodiments, L is 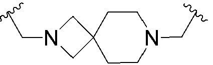 . In some embodiments, L is 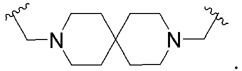 In some embodiments, L is 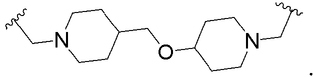 In some embodiments, L is 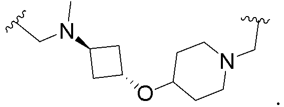 In some embodiments, L is 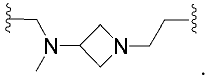 In some embodiments, L is 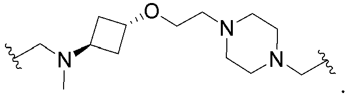 In some embodiments, L is 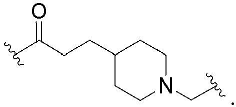 In some embodiments, L is 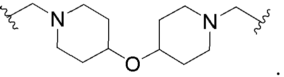 **In** some embodiments, L is 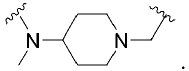 In some embodiments, L is 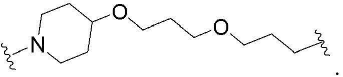 In some embodiments, L is 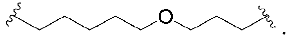 In some embodiments, L is 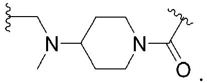 In some embodiments, L is 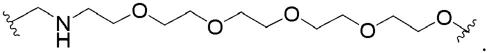 In some embodiments, L is 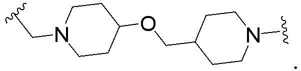 In some embodiments, L is 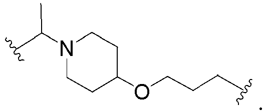 In some embodiments, L is 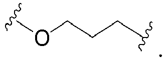 In some embodiments, L is 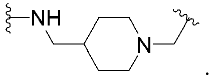 In some embodiments, L is 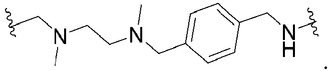 In some embodiments, L is 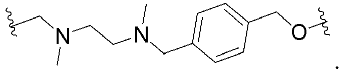 In some embodiments, L is 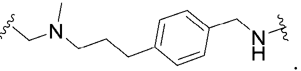 In some embodiments, L is 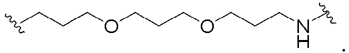 In some embodiments, L is 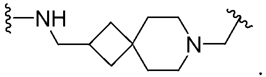 In some embodiments, L is 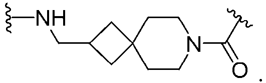 In some embodiments, L is 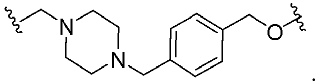 In some embodiments, L is 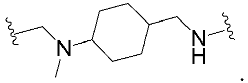 In some embodiments, L is 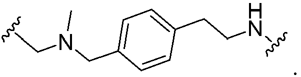 In some embodiments, L is 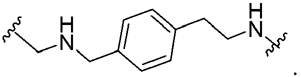 In some embodiments, L is 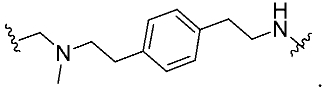 In some embodiments, L is 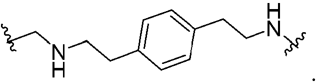 In some embodiments, L is 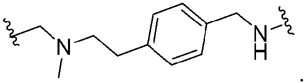 In some embodiments, L is 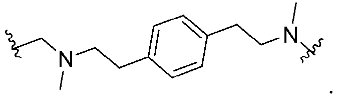 In some embodiments, L is 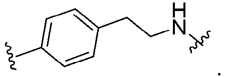 In In some embodiments, L is 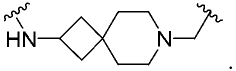 In some In In some embodiments, L is 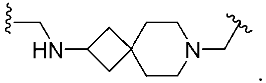 embodiments, L is 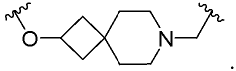 In some embodiments, L is 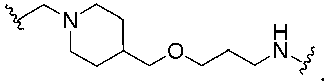 In some embodiments, L is 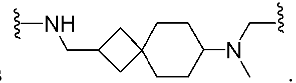 In some embodiments, L is 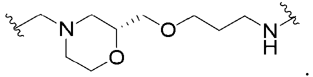 In some embodiments, L is 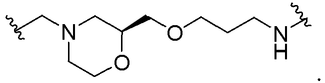 In some embodiments, L is 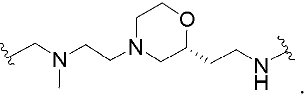 In some embodiments, L is 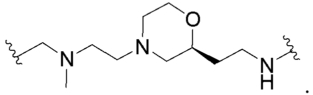 In some embodiments, L is 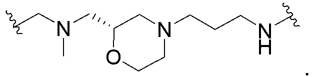 In some embodiments, L is 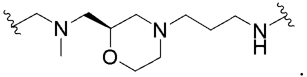 In some embodiments, L is 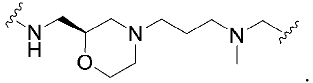 In some embodiments, L is 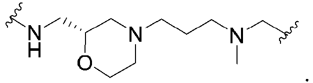 In some embodiments, L is 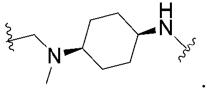 In some embodiments, L is 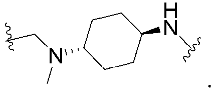 In some embodiments, L is 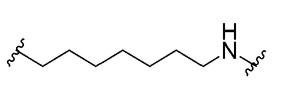 . In some embodiments, L is 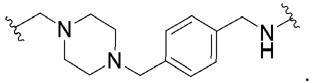 some embodiments, L is 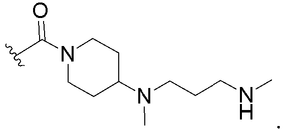 In some embodiments, L is 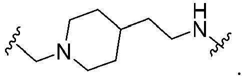 In some embodiments, L is 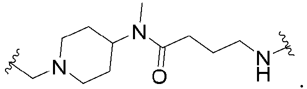 In some embodiments, L is 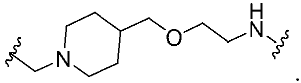 In some embodiments, L is 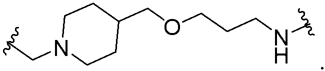 In some embodiments, L is 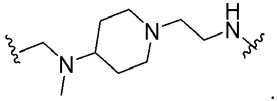 In some embodiments, L is 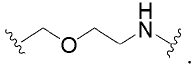 In some embodiments, L is 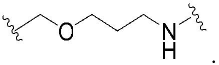 **In** some embodiments, L is 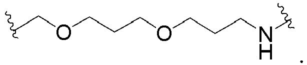 In some embodiments, L is 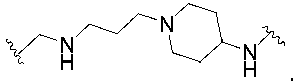 In some embodiments, L is 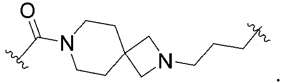 **In** some embodiments, L is 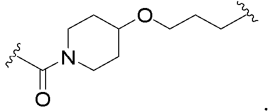 In some embodiments, L is 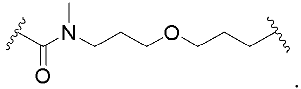 In some embodiments, L is 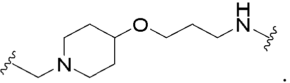 In some embodiments, L is 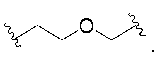 . In some embodiments, L is 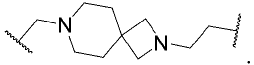 In some embodiments, L is 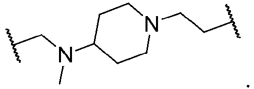 In some embodiments, L is 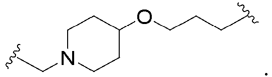 In some embodiments, L is 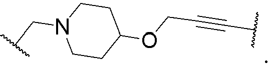 In some embodiments, L is 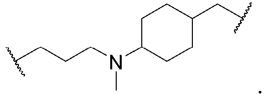 In some embodiments, L is 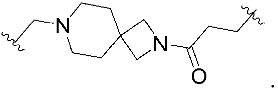 In some embodiments, L is 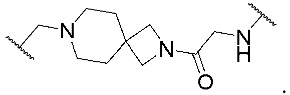 In some embodiments, L is 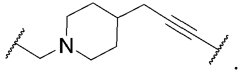 In some embodiments, L is 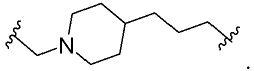 In some embodiments, L is 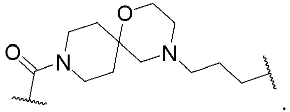 In some embodiments, L is 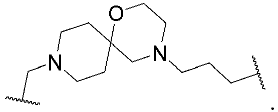 In some embodiments, L is 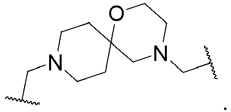 In some embodiments, L is 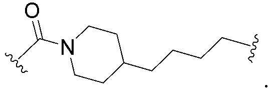 In some embodiments, L is 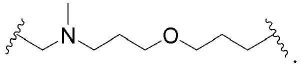 In some embodiments, L is 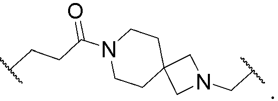 In some embodiments, L is 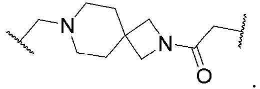 In some embodiments, L is 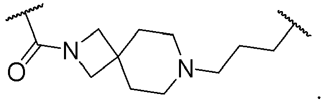 In some embodiments, L is 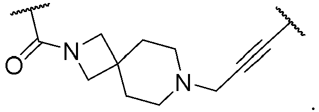 In some embodiments, L is 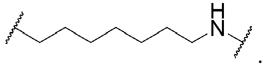 In some embodiments, L is 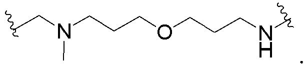 In some embodiments, L is 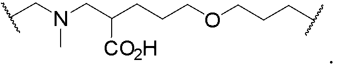 In some embodiments, L is 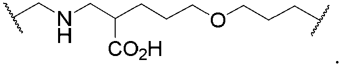 In some embodiments, L is 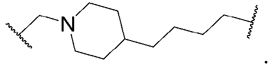 In some embodiments, L is 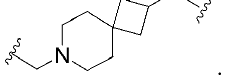 In some embodiments, L is 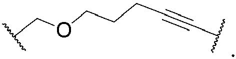 In some embodiments, L is 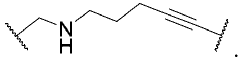 In some embodiments, L is 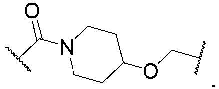 In some embodiments, L is 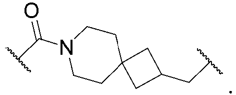 In some embodiments, L is 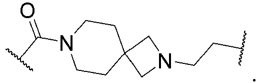 In some embodiments, L is 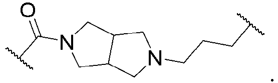 In some embodiments, L is 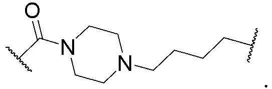 In some embodiments, L is 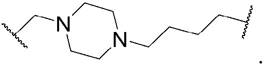 In some embodiments, L is 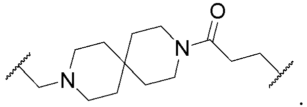 In some embodiments, L is 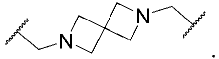 In some embodiments, L is 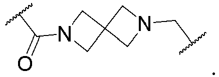 In some embodiments, 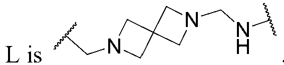 . In some embodiments, L is 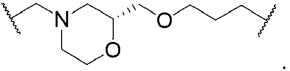 In some embodiments, L is 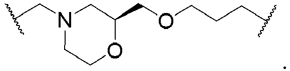 In some embodiments, L is 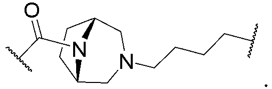 In some embodiments, L is 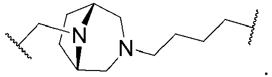 In some embodiments, L is 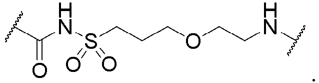 In some embodiments, L is 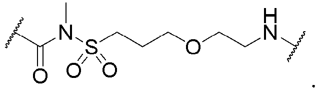 In some embodiments, L is 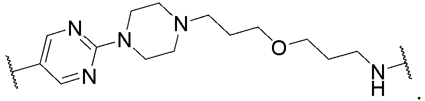 In some embodiments, L is 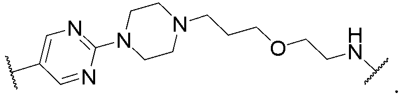 In some embodiments, L is 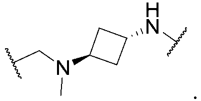 In some embodiments, L is 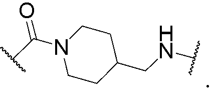 In some embodiments, L is 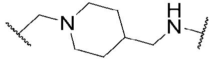 . In some embodiments, L is 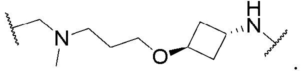 In some embodiments, L is 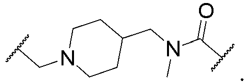 In some embodiments, L is 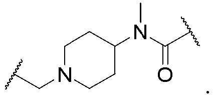 In some embodiments, L is 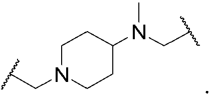 In some embodiments, L is 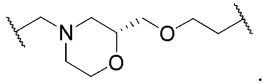 In some embodiments, L is 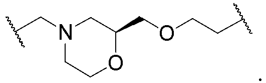 In some embodiments, L is 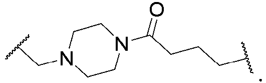 In some embodiments, L is 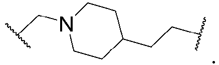 In some embodiments, L is 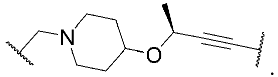 In some embodiments, L is 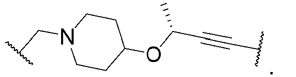 In some embodiments, L is 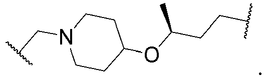 In some embodiments, L is 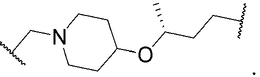 In some embodiments, L is 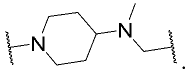 In some embodiments, L is 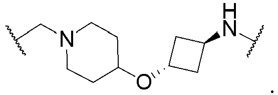 In some embodiments, L is 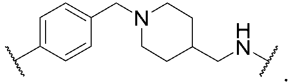 In some embodiments, L is 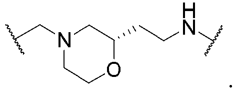 In some embodiments, L is 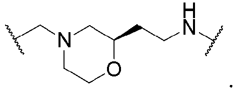 In some embodiments, L is 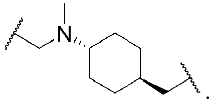 In some embodiments, L is 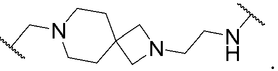 In some embodiments, L is 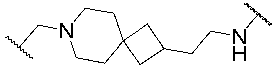 . In some embodiments, L is 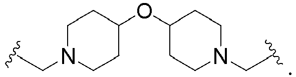 **In** some embodiments, L is 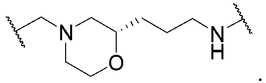 In some embodiments, L is 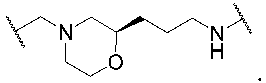 In some embodiments, L is 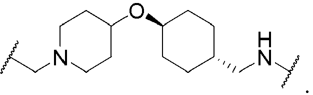 In some embodiments, L is 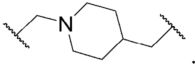 In some embodiments, L is 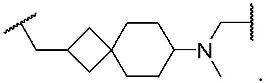 In some embodiments, L is 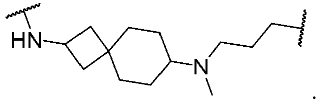 In some embodiments, L is 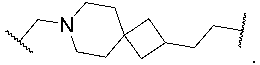 In some embodiments, L is 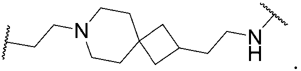 In some embodiments, L is 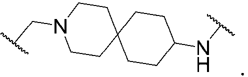 In some embodiments, L is 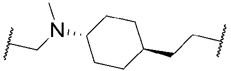 . In some embodiments, L is 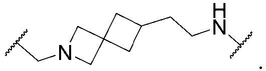 In some embodiments, L is 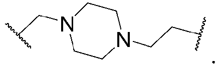 In some embodiments, L is 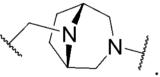 In some embodiments, L is 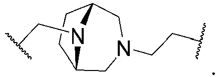 In some embodiments, L is 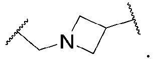 In some embodiments, L is 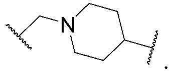 In some embodiments, L is 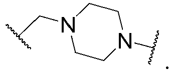 In some embodiments, L is 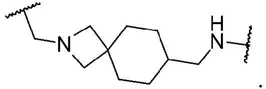 In some embodiments, L is 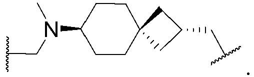 In some embodiments, L is 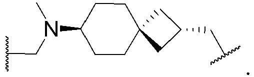 In some embodiments, L is 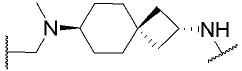 . In some embodiments, L is 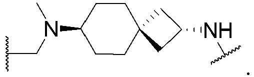 In some embodiments, L is 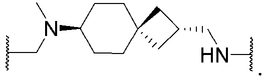 In some embodiments, L is 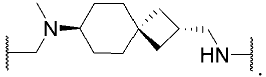 In some embodiments, L is 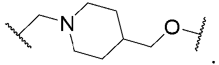 In some embodiments, L is 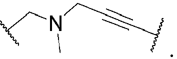 In some embodiments, L is 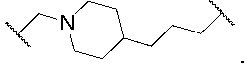 In some embodiments, L is 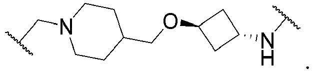 In some embodiments, L is 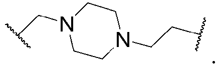 In some embodiments, L is 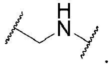 In some embodiments, L is 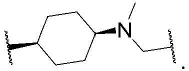 In some embodiments, L is 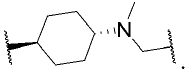 **In** some embodiments, L is 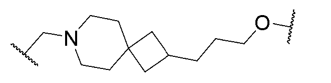 .

. In some embodiments, L is 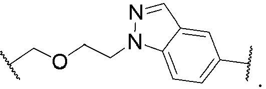 In some embodiments, L is 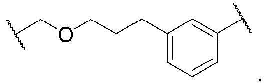 In some embodiments, L is 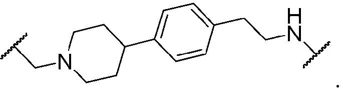 In some embodiments, L is 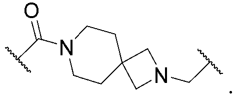 In some embodiments, L is 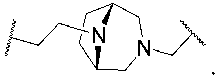 In some embodiments, L is 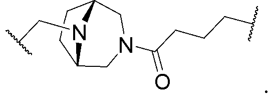 In some embodiments, L is 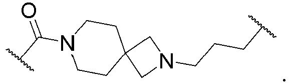 In some embodiments, L is 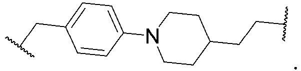 In some embodiments, L is 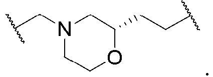 In some embodiments, L is 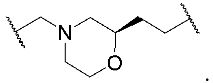 In some embodiments, L is 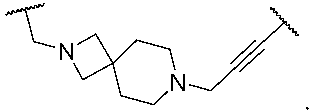 In some embodiments, L is 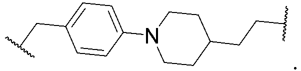 In some embodiments, L is 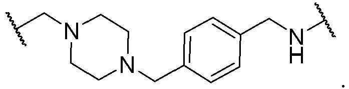 In some embodiments, L is 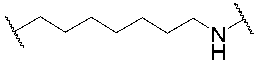 . In some embodiments, L is 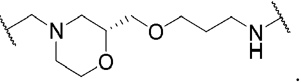 In some embodiments, L is 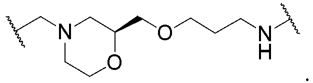 In some embodiments, L is 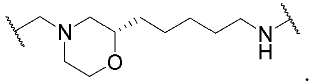 In some embodiments, L is 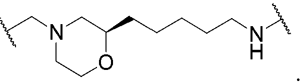 In some embodiments, L is 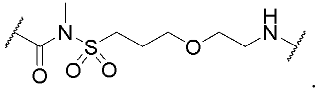 In some embodiments, L is 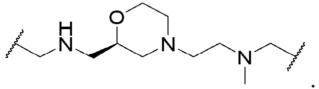 **In** some embodiments, L is 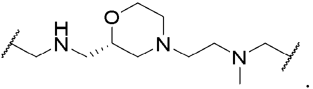 In some embodiments, L is 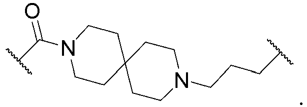 In some embodiments, L is 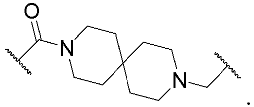 In some embodiments, L is 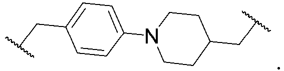 In some embodiments, L is 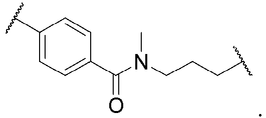 In some embodiments, L is 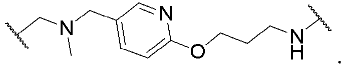 In some embodiments, L is 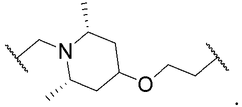 **In** some embodiments, L is 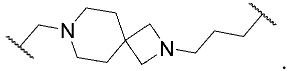 In some embodiments, L is 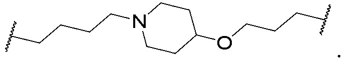 In some embodiments, L is

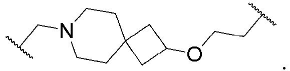

In some embodiments, L is selected from those depicted in Table 1, below.

In some embodiments, a provided compound or pharmaceutically acceptatable salt thereof, is selected from those wherein IRAK is 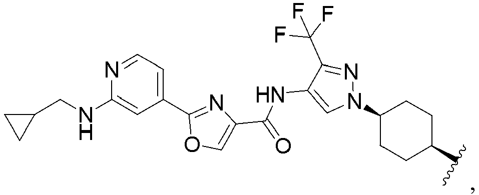 LBM is selected from any of those in **Table A** below, and L is selected from any of those in **Table B** below.

In some embodiments, a provided compound or pharmaceutically acceptatable salt thereof, is selected from those wherein IRAK is 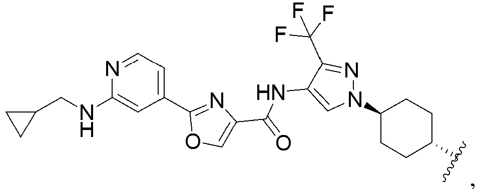 LBM is selected from any of those in **Table A** below, and L is selected from any of those in **Table B** below.

In some embodiments, a provided compound or pharmaceutically acceptatable salt thereof, is selected from those wherein IRAK is 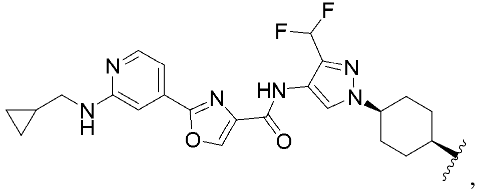 LBM is selected from any of those in **Table A** below, and L is selected from any of those in **Table B** below.

In some embodiments, a provided compound or pharmaceutically acceptatable salt thereof, is selected from those wherein IRAK is 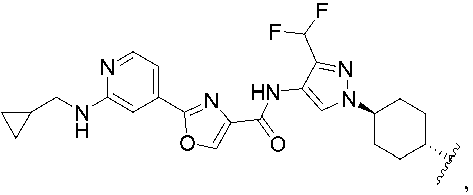 LBM is selected from any of those in **Table A** below, and L is selected from any of those in **Table B** below.

In some embodiments, a provided compound or pharmaceutically acceptatable salt thereof, is selected from those wherein IRAK is 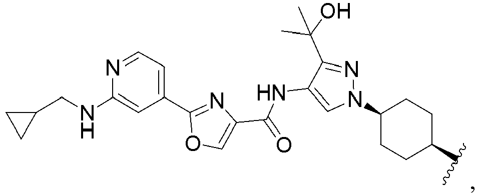 LBM is selected from any of those in **Table A** below, and L is selected from any of those in **Table B** below.

In some embodiments, a provided compound or pharmaceutically acceptatable salt thereof, is selected from those wherein IRAK is 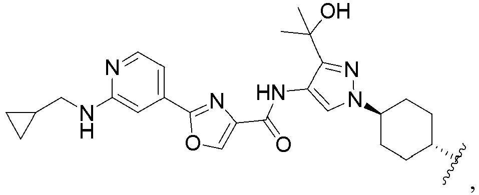 LBM is selected from any of those in **Table A** below, and L is selected from any of those in **Table B** below.

In some embodiments, a provided compound or pharmaceutically acceptatable salt thereof, is selected from those wherein IRAK is 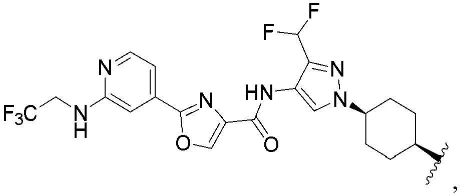 LBM is selected from any of those in **Table A** below, and L is selected from any of those in **Table B** below.

In some embodiments, a provided compound or pharmaceutically acceptatable salt thereof, is selected from those wherein IRAK is 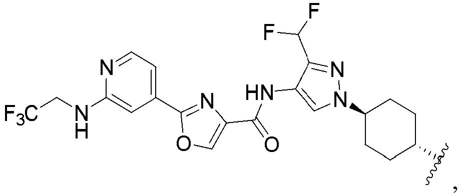 LBM is selected from any of those in **Table A** below, and L is selected from any of those in **Table B** below.

In some embodiments, a provided compound or pharmaceutically acceptatable salt thereof, is selected from those wherein IRAK is 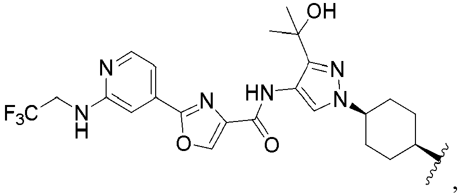 LBM is selected from any of those in **Table A** below, and L is selected from any of those in **Table B** below.

In some embodiments, a provided compound or pharmaceutically acceptatable salt thereof, is selected from those wherein IRAK is 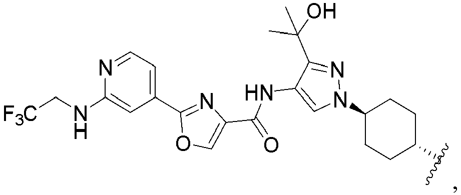 LBM is selected from any of those in **Table A** below, and L is selected from any of those in **Table B** below.

In some embodiments, a provided compound or pharmaceutically acceptatable salt thereof, is selected from those wherein IRAK is 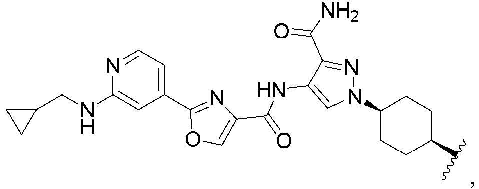 LBM is selected from any of those in **Table A** below, and L is selected from any of those in **Table B** below.

In some embodiments, a provided compound or pharmaceutically acceptatable salt thereof, is selected from those wherein IRAK is 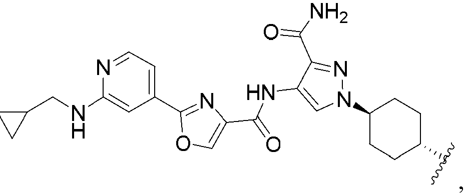 LBM is selected from any of those in **Table A** below, and L is selected from any of those in **Table B** below.

In some embodiments, a provided compound or pharmaceutically acceptatable salt thereof, is selected from those wherein IRAK is 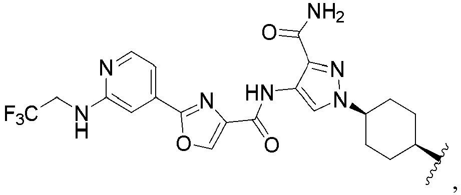 LBM is selected from any of those in **Table A** below, and L is selected from any of those in **Table B** below.

In some embodiments, a provided compound or pharmaceutically acceptatable salt thereof, is selected from those wherein IRAK is 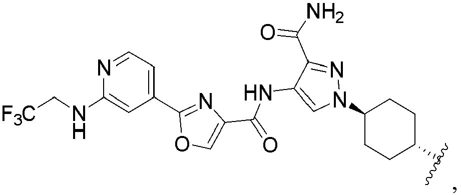 LBM is selected from any of those in **Table A** below, and L is selected from any of those in **Table B** below.

In some embodiments, a provided compound or pharmaceutically acceptatable salt thereof, is selected from those wherein IRAK is 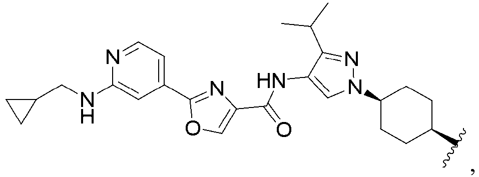 LBM is selected from any of those in **Table A** below, and L is selected from any of those in **Table B** below.

In some embodiments, a provided compound or pharmaceutically acceptatable salt thereof, is selected from those wherein IRAK is 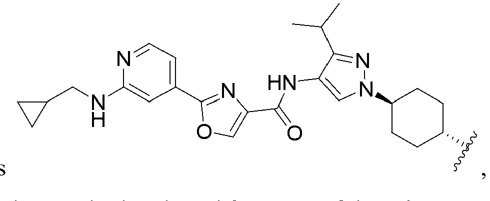 LBM is selected from any of those in **Table A** below, and L is selected from any of those in **Table B** below.

In some embodiments, a provided compound or pharmaceutically acceptatable salt thereof, is selected from those wherein IRAK is 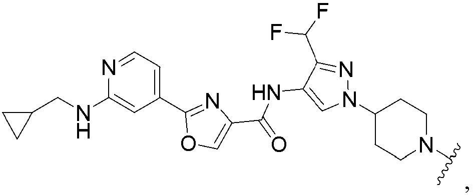 LBM is selected from any of those in **Table A** below, and L is selected from any of those in **Table B** below.

In some embodiments, a provided compound or pharmaceutically acceptatable salt thereof, is selected from those wherein IRAK is 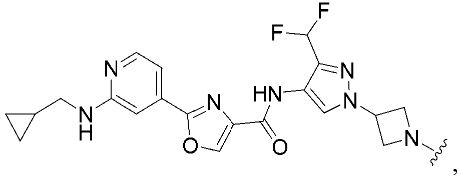 LBM is selected from any of those in **Table A** below, and L is selected from any of those in **Table B** below.

In some embodiments, a provided compound or pharmaceutically acceptatable salt thereof, is selected from those wherein IRAK is 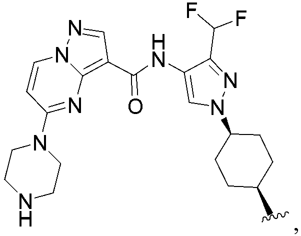 LBM is selected from any of those in **Table A** below, and L is selected from any of those in **Table B** below.

In some embodiments, a provided compound or pharmaceutically acceptatable salt thereof, is selected from those wherein IRAK is 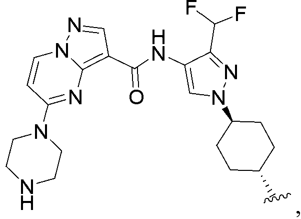 LBM is selected from any of those in **Table A** below, and L is selected from any of those in **Table B** below.

In some embodiments, a provided compound or pharmaceutically acceptatable salt thereof, is selected from those wherein IRAK is 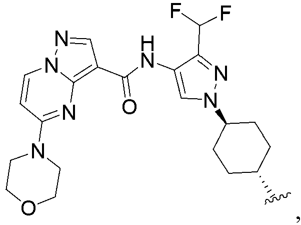 LBM is selected from any of those in **Table A** below, and L is selected from any of those in **Table B** below.

In some embodiments, a provided compound or pharmaceutically acceptatable salt thereof, is selected from those wherein IRAK is 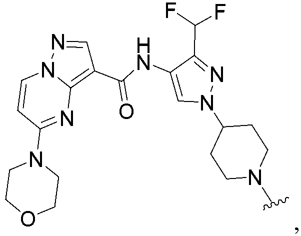 LBM is selected from any of those in **Table A** below, and L is selected from any of those in **Table B** below.

In some embodiments, a provided compound or pharmaceutically acceptatable salt thereof, is selected from those wherein IRAK is 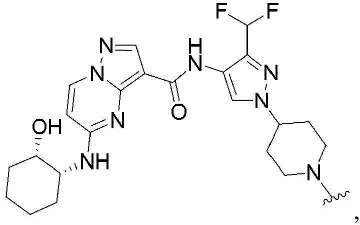 LBM is selected from any of those in **Table A** below, and L is selected from any of those in **Table B** below.

In some embodiments, a provided compound or pharmaceutically acceptatable salt thereof, is selected from those wherein IRAK is 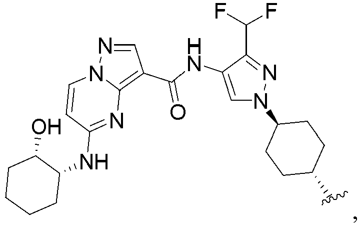 LBM is selected from any of those in **Table A** below, and L is selected from any of those in **Table B** below.

In some embodiments, a provided compound or pharmaceutically acceptatable salt thereof, is selected from those wherein IRAK is 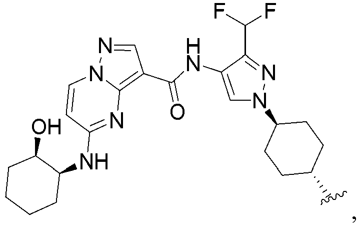 LBM is selected from any of those in **Table A** below, and L is selected from any of those in **Table B** below.

In some embodiments, a provided compound or pharmaceutically acceptatable salt thereof, is selected from those wherein IRAK is 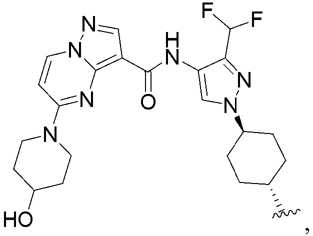 LBM is selected from any of those in **Table A** below, and L is selected from any of those in **Table B** below.

In some embodiments, a provided compound or pharmaceutically acceptatable salt thereof, is selected from those wherein IRAK is 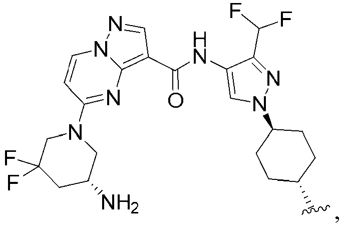 LBM is selected from any of those in **Table A** below, and L is selected from any of those in **Table B** below.

In some embodiments, a provided compound or pharmaceutically acceptatable salt thereof, is selected from those wherein IRAK is 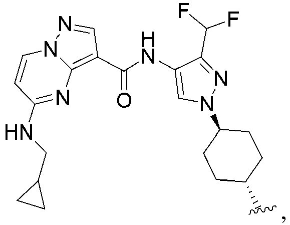 LBM is selected from any of those in **Table A** below, and L is selected from any of those in **Table B** below.

In some embodiments, a provided compound or pharmaceutically acceptatable salt thereof, is selected from those wherein IRAK is 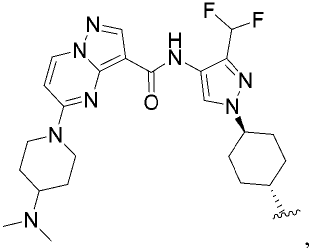 LBM is selected from any of those in **Table A** below, and L is selected from any of those in **Table B** below.

In some embodiments, a provided compound or pharmaceutically acceptatable salt thereof, is selected from those wherein IRAK is 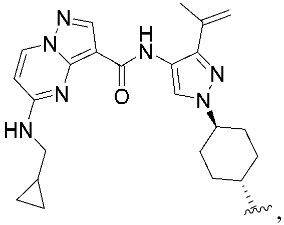 LBM is selected from any of those in **Table A** below, and L is selected from any of those in **Table B** below.

In some embodiments, a provided compound or pharmaceutically acceptatable salt thereof, is selected from those wherein IRAK is 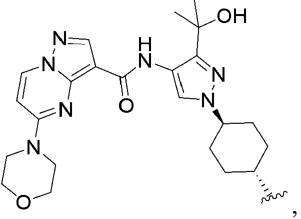 LBM is selected from any of those in **Table A** below, and L is selected from any of those in **Table B** below.

In some embodiments, a provided compound or pharmaceutically acceptatable salt thereof, is selected from those wherein IRAK is 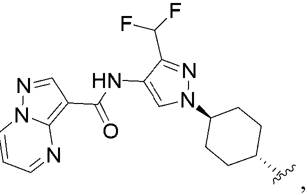 LBM is selected from any of those in **Table A** below, and L is selected from any of those in **Table B** below.

In some embodiments, a provided compound or pharmaceutically acceptatable salt thereof, is selected from those wherein IRAK is 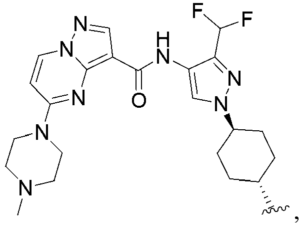 LBM is selected from any of those in **Table A** below, and L is selected from any of those in **Table B** below.

In some embodiments, a provided compound or pharmaceutically acceptatable salt thereof, is selected from those wherein IRAK is 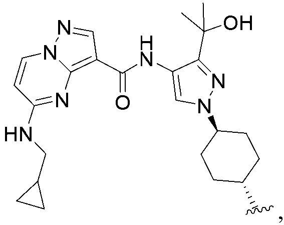 LBM is selected from any of those in **Table A** below, and L is selected from any of those in **Table B** below.

In some embodiments, a provided compound or pharmaceutically acceptatable salt thereof, is selected from those wherein IRAK is 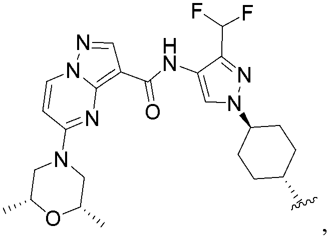 LBM is selected from any of those in **Table A** below, and L is selected from any of those in **Table B** below.

In some embodiments, a provided compound or pharmaceutically acceptatable salt thereof, is selected from those wherein IRAK is 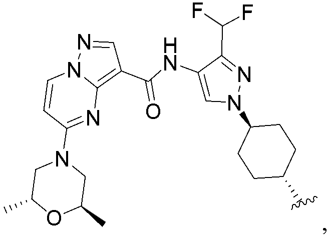 LBM is selected from any of those in **Table A** below, and L is selected from any of those in **Table B** below.

In some embodiments, a provided compound or pharmaceutically acceptatable salt thereof, is selected from those wherein IRAK is 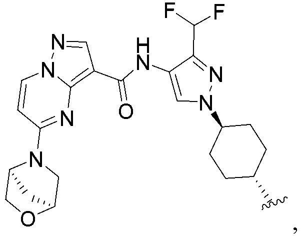 LBM is selected from any of those in **Table A** below, and L is selected from any of those in **Table B** below.

In some embodiments, a provided compound or pharmaceutically acceptatable salt thereof, is selected from those wherein IRAK is 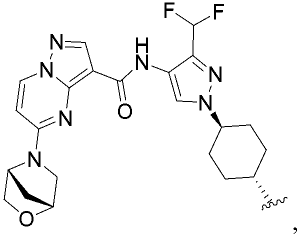 LBM is selected from any of those in **Table A** below, and L is selected from any of those in **Table B** below.

In some embodiments, a provided compound or pharmaceutically acceptatable salt thereof, is selected from those wherein IRAK is 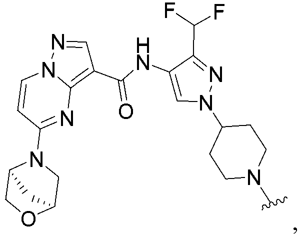 LBM is selected from any of those in **Table A** below, and L is selected from any of those in **Table B** below.

In some embodiments, a provided compound or pharmaceutically acceptatable salt thereof, is selected from those wherein IRAK is 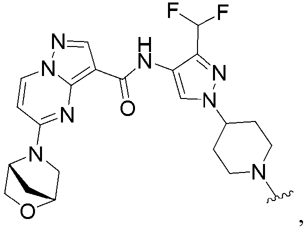 LBM is selected from any of those in **Table** A below, and L is selected from any of those in **Table B** below.

In some embodiments, a provided compound or pharmaceutically acceptatable salt thereof, is selected from those wherein IRAK is 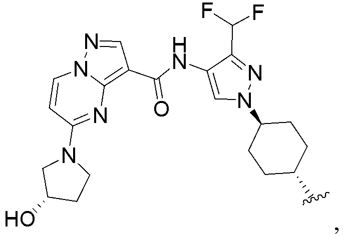 LBM is selected from any of those in **Table A** below, and L is selected from any of those in **Table B** below.

In some embodiments, a provided compound or pharmaceutically acceptatable salt thereof, is selected from those wherein IRAK is 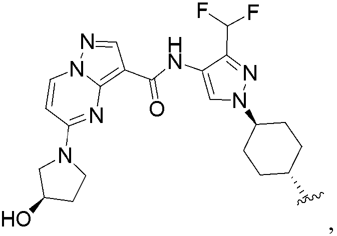 LBM is selected from any of those in **Table A** below, and L is selected from any of those in **Table B** below.

In some embodiments, a provided compound or pharmaceutically acceptatable salt thereof, is selected from those wherein IRAK is 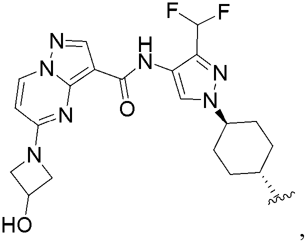 LBM is selected from any of those in Table A below, and L is selected from any of those in Table B below.

In some embodiments, a provided compound or pharmaceutically acceptatable salt thereof, is selected from those wherein IRAK is 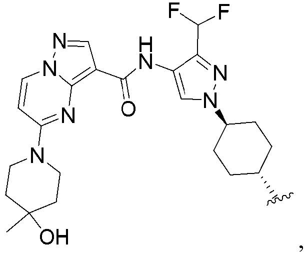 LBM is selected from any of those in Table A below, and L is selected from any of those in Table B below.

In some embodiments, a provided compound or pharmaceutically acceptatable salt thereof, is selected from those wherein IRAK is 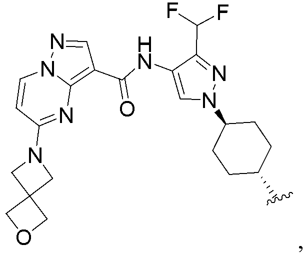 LBM is selected from any of those in **Table A** below, and L is selected from any of those in **Table B** below.

In some embodiments, a provided compound or pharmaceutically acceptatable salt thereof, is selected from those wherein IRAK is 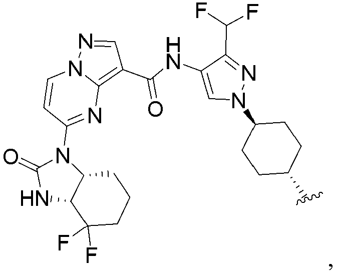 LBM is selected from any of those in **Table A** below, and L is selected from any of those in **Table B** below.

In some embodiments, a provided compound or pharmaceutically acceptatable salt thereof, is selected from those wherein IRAK is 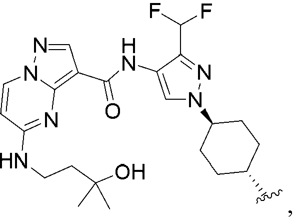 LBM is selected from any of those in **Table A** below, and L is selected from any of those in **Table B** below.

In some embodiments, a provided compound or pharmaceutically acceptatable salt thereof, is selected from those wherein IRAK is 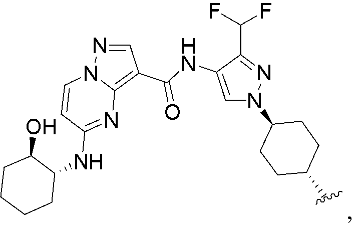 LBM is selected from any of those in **Table A** below, and L is selected from any of those in **Table B** below.

In some embodiments, a provided compound or pharmaceutically acceptatable salt thereof, is selected from those wherein IRAK is 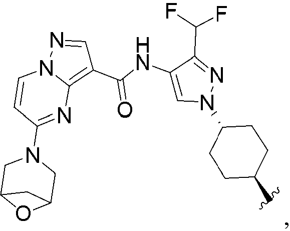 LBM is selected from any of those in **Table A** below, and L is selected from any of those in **Table B** below.

In some embodiments, a provided compound or pharmaceutically acceptatable salt thereof, is selected from those wherein IRAK is 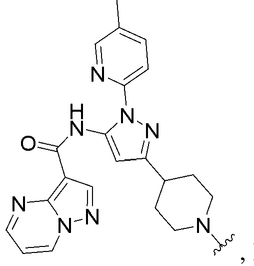 LBM is selected from any of those in **Table A** below, and L is selected from any of those in **Table B** below.

In some embodiments, a provided compound or pharmaceutically acceptatable salt thereof, is selected from those wherein IRAK is 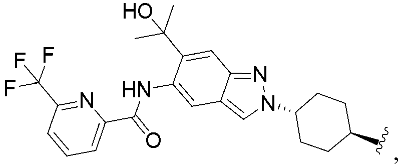 LBM is selected from any of those in **Table A** below, and L is selected from any of those in **Table B** below.

In some embodiments, a provided compound or pharmaceutically acceptatable salt thereof, is selected from those wherein IRAK is 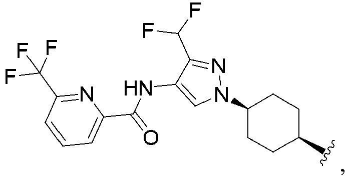 LBM is selected from any of those in **Table A** below, and L is selected from any of those in **Table B** below.

In some embodiments, a provided compound or pharmaceutically acceptatable salt thereof, is selected from those wherein IRAK is 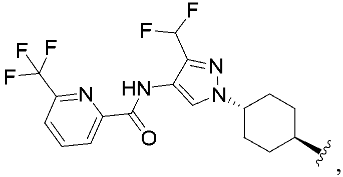 LBM is selected from any of those in **Table A** below, and L is selected from any of those in **Table B** below.

In some embodiments, a provided compound or pharmaceutically acceptatable salt thereof, is selected from those wherein IRAK is 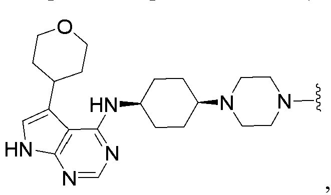 LBM is selected from any of those in **Table A** below, and L is selected from any of those in **Table B** below.

In some embodiments, a provided compound or pharmaceutically acceptatable salt thereof, is selected from those wherein IRAK is 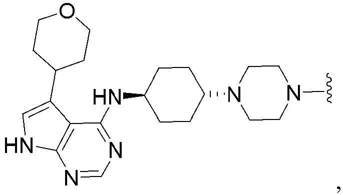 LBM is selected from any of those in **Table A** below, and L is selected from any of those in **Table B** below.

In some embodiments, a provided compound or pharmaceutically acceptatable salt thereof, is selected from those wherein IRAK is 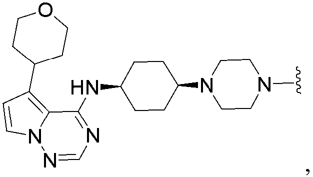 LBM is selected from any of those in **Table A** below, and L is selected from any of those in **Table B** below.

In some embodiments, a provided compound or pharmaceutically acceptatable salt thereof, is selected from those wherein IRAK is 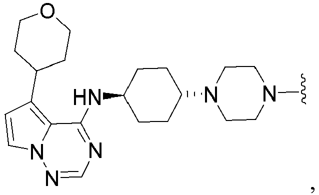 LBM is selected from any of those in **Table A** below, and L is selected from any of those in **Table B** below.

In some embodiments, a provided compound or pharmaceutically acceptatable salt thereof, is selected from those wherein IRAK is 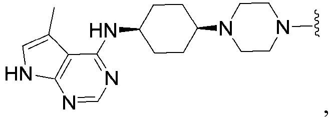 LBM is selected from any of those in **Table A** below, and L is selected from any of those in **Table B** below.

In some embodiments, a provided compound or pharmaceutically acceptatable salt thereof, is selected from those wherein IRAK is 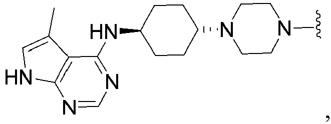 LBM is selected from any of those in **Table A** below, and L is selected from any of those in **Table B** below.

In some embodiments, a provided compound or pharmaceutically acceptatable salt thereof, is selected from those wherein IRAK is 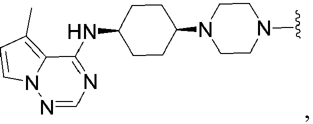 LBM is selected from any of those in **Table A** below, and L is selected from any of those in **Table B** below.

In some embodiments, a provided compound or pharmaceutically acceptatable salt thereof, is selected from those wherein IRAK is 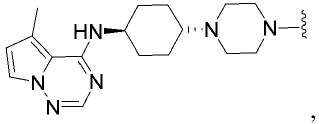 LBM is selected from any of those in **Table A** below, and L is selected from any of those in **Table B** below.

In some embodiments, a provided compound or pharmaceutically acceptatable salt thereof, is selected from those wherein IRAK is 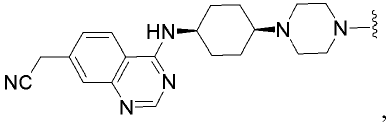 LBM is selected from any of those in **Table A** below, and L is selected from any of those in **Table B** below.

In some embodiments, a provided compound or pharmaceutically acceptatable salt thereof, is selected from those wherein IRAK is 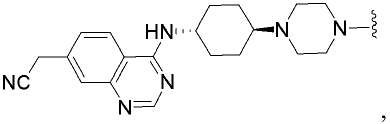 LBM is selected from any of those in **Table A** below, and L is selected from any of those in **Table B** below.

In some embodiments, a provided compound or pharmaceutically acceptatable salt thereof, is selected from those wherein IRAK is 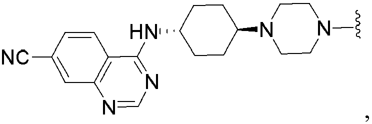 LBM is selected from any of those in **Table A** below, and L is selected from any of those in **Table B** below.

In some embodiments, a provided compound or pharmaceutically acceptatable salt thereof, is selected from those wherein IRAK is 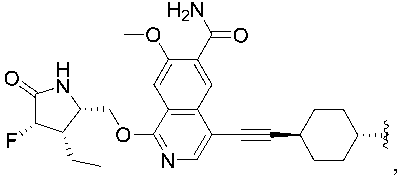 LBM is selected from any of those in **Table A** below, and L is selected from any of those in **Table B** below.

In some embodiments, a provided compound or pharmaceutically acceptatable salt thereof, is selected from those wherein IRAK is 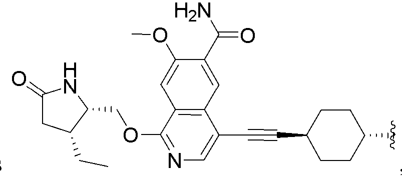 thereof, is is selected from those wherein IRAK is, LBM is selected from any of those in **Table A** below, and L is selected from any of those in **Table B** below.

In some embodiments, a provided compound or pharmaceutically acceptatable salt thereof, is selected from those wherein IRAK is 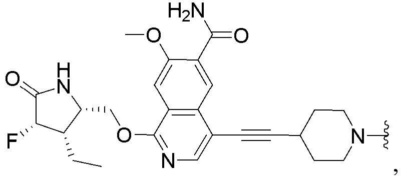 LBM is selected from any of those in **Table A** below, and L is selected from any of those in **Table B** below.

In some embodiments, a provided compound or pharmaceutically acceptatable salt thereof, is selected from those wherein IRAK is 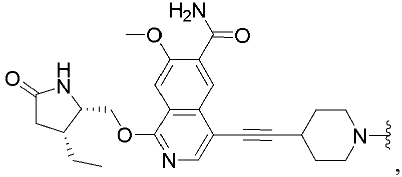 LBM is selected from any of those in **Table A** below, and L is selected from any of those in **Table B** below.

In some embodiments, a provided compound or pharmaceutically acceptatable salt thereof, is selected from those wherein IRAK is 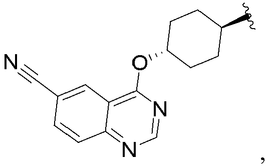 LBM is selected from any of those in **Table A** below, and L is selected from any of those in **Table B** below.

In some embodiments, a provided compound or pharmaceutically acceptatable salt thereof, is selected from those wherein IRAK is 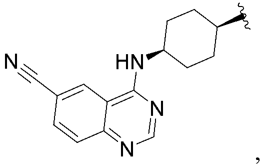 LBM is selected from any of those in **Table A** below, and L is selected from any of those in **Table B** below.

In some embodiments, a provided compound or pharmaceutically acceptatable salt thereof, is selected from those wherein IRAK is 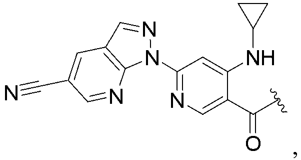 LBM is selected from any of those in **Table A** below, and L is selected from any of those in **Table B** below.

In some embodiments, a provided compound or pharmaceutically acceptatable salt thereof, is selected from those wherein IRAK is 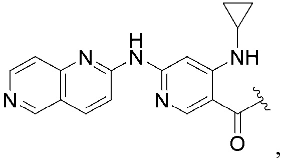 LBM is selected from any of those in **Table A** below, and L is selected from any of those in **Table B** below.

In some embodiments, a provided compound or pharmaceutically acceptatable salt thereof, is selected from those wherein IRAK is 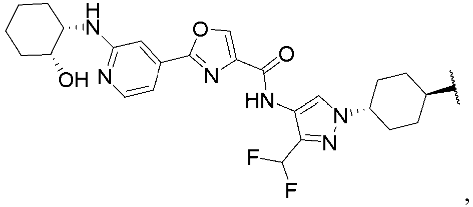 LBM is selected from any of those in **Table A** below, and L is selected from any of those in **Table B** below.

In some embodiments, a provided compound or pharmaceutically acceptatable salt thereof, is selected from those wherein IRAK is 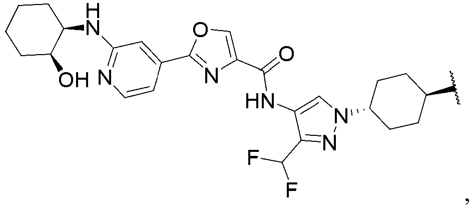 LBM is selected from any of those in **Table A** below, and L is selected from any of those in **Table B** below.

In some embodiments, a provided compound or pharmaceutically acceptatable salt thereof, is selected from those wherein IRAK is 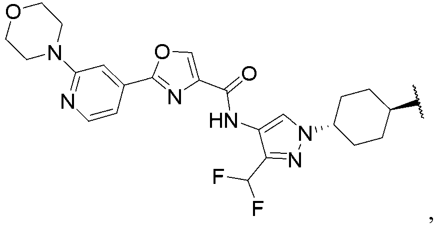 LBM is selected from any of those in **Table A** below, and L is selected from any of those in **Table B** below.

In some embodiments, a provided compound or pharmaceutically acceptatable salt thereof, is selected from those wherein IRAK is 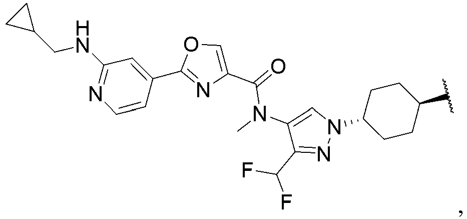 LBM is selected from any of those in **Table A** below, and L is selected from any of those in **Table B** below.

In some embodiments, a provided compound or pharmaceutically acceptatable salt thereof, is selected from those wherein IRAK is 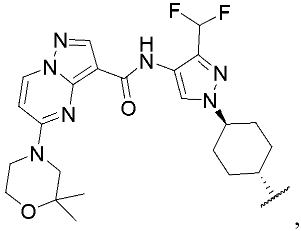 LBM is selected from any of those in **Table A** below, and L is selected from any of those in **Table B** below.

In some embodiments, a provided compound or pharmaceutically acceptatable salt thereof, is selected from those wherein IRAK is 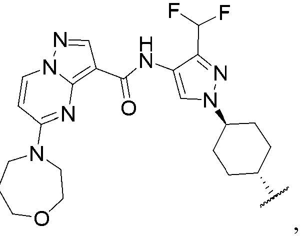 LBM is selected from any of those in **Table A** below, and L is selected from any of those in **Table B** below.

In some embodiments, a provided compound or pharmaceutically acceptatable salt thereof, is selected from those wherein IRAK is 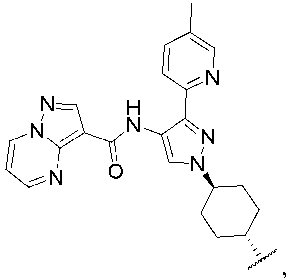 LBM is selected from any of those in **Table A** below, and L is selected from any of those in **Table B** below.

In some embodiments, a provided compound or pharmaceutically acceptatable salt thereof, is selected from those wherein IRAK is 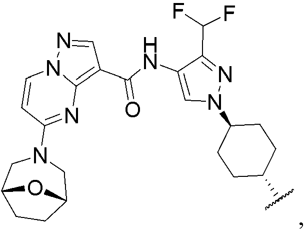 LBM is selected from any of those in **Table A** below, and L is selected from any of those in **Table B** below.

In some embodiments, a provided compound or pharmaceutically acceptatable salt thereof, is selected from those wherein IRAK is 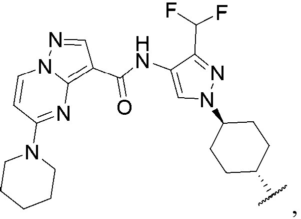 LBM is selected from any of those in **Table A** below, and L is selected from any of those in **Table B** below.

In some embodiments, a provided compound or pharmaceutically acceptatable salt thereof, is selected from those wherein IRAK is 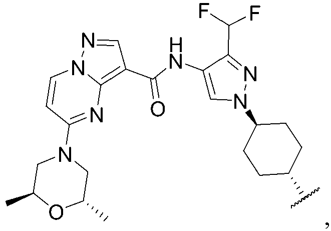 LBM is selected from any of those in **Table A** below, and L is selected from any of those in **Table B** below.

In some embodiments, a provided compound or pharmaceutically acceptatable salt thereof, is selected from those wherein IRAK is 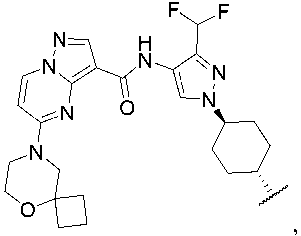 LBM is selected from any of those in **Table A** below, and L is selected from any of those in **Table B** below.

In some embodiments, a provided compound or pharmaceutically acceptatable salt thereof, is selected from those wherein IRAK is 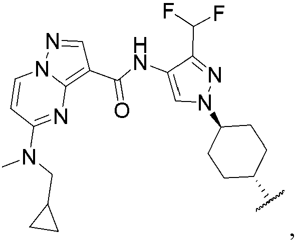 LBM is selected from any of those in **Table A** below, and L is selected from any of those in **Table B** below.

In some embodiments, a provided compound or pharmaceutically acceptatable salt thereof, is selected from those wherein IRAK is 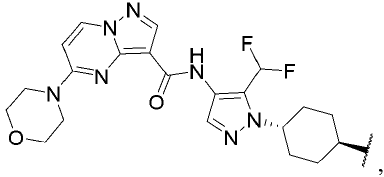 LBM is selected from any of those in **Table A** below, and L is selected from any of those in **Table B** below.

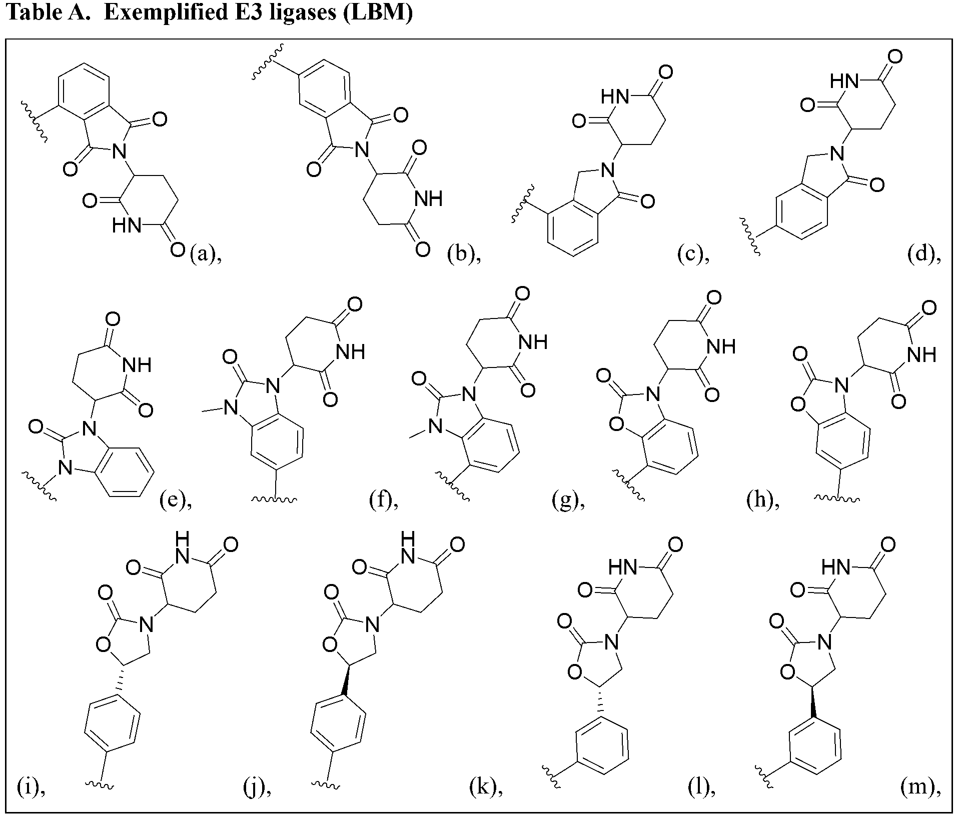

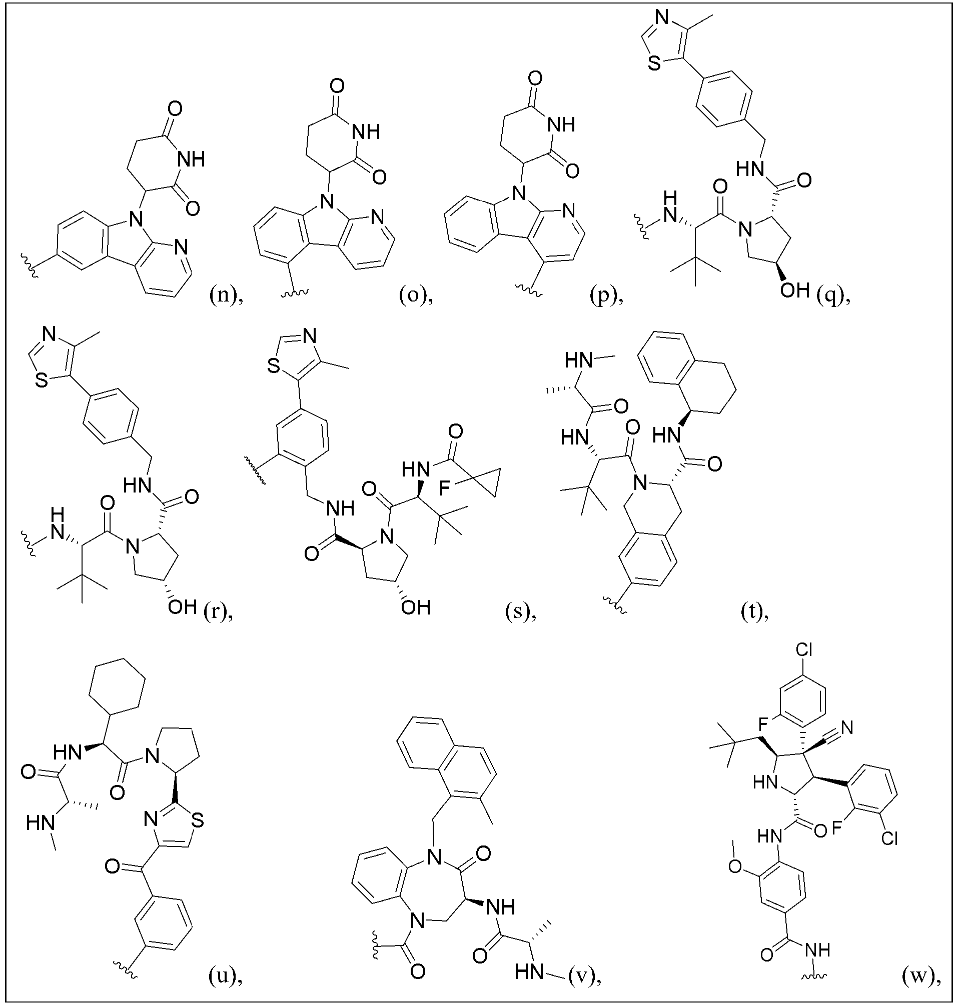

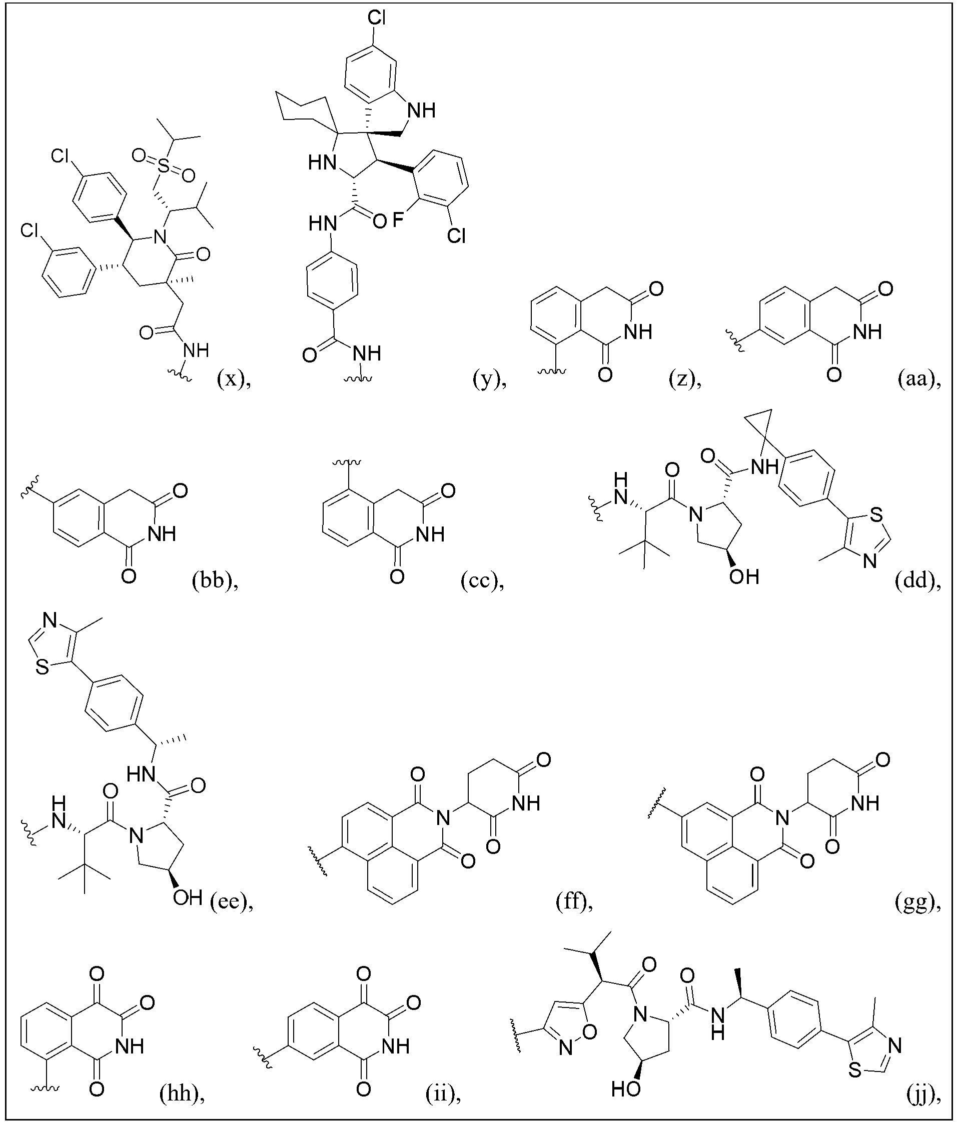

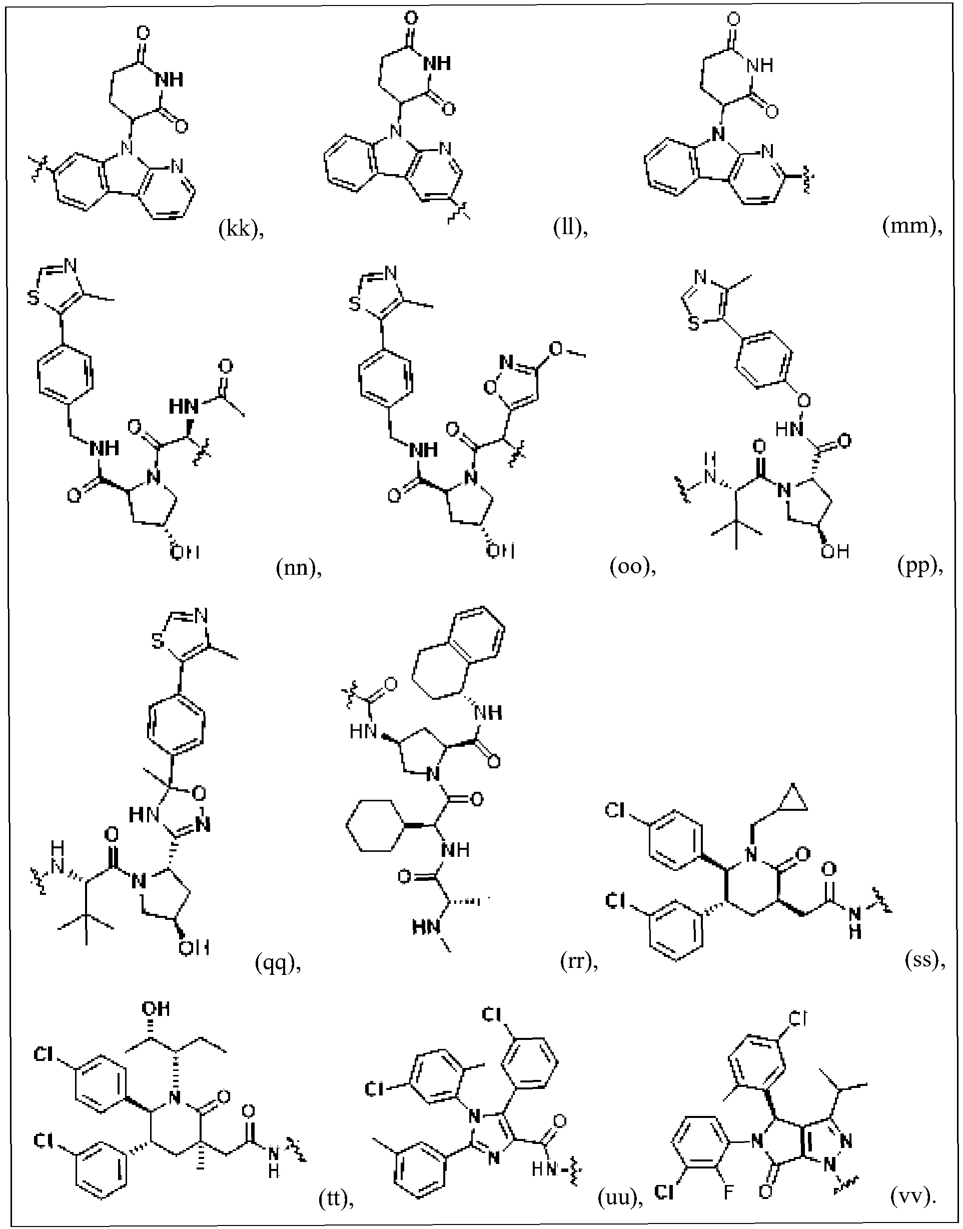

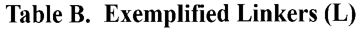

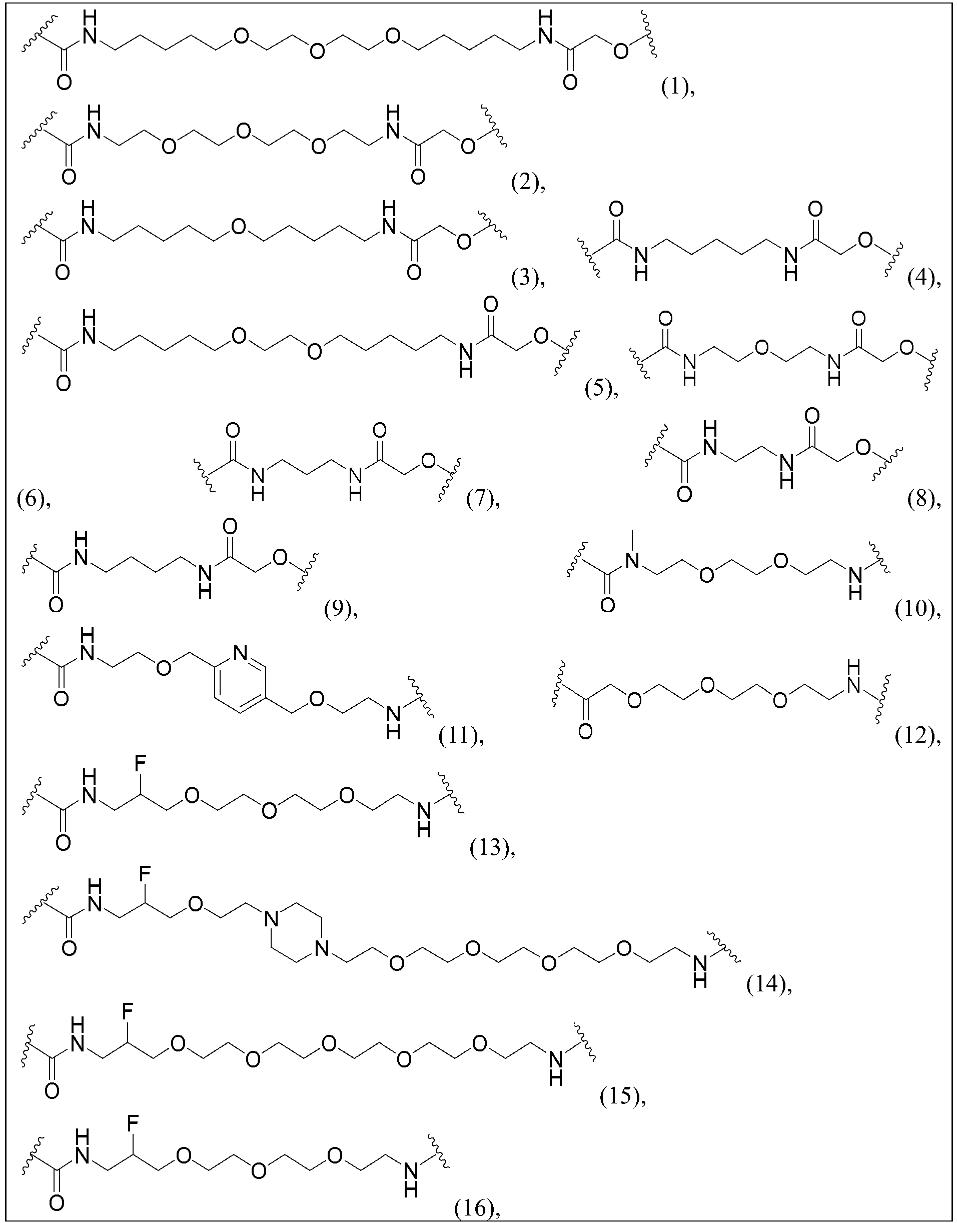

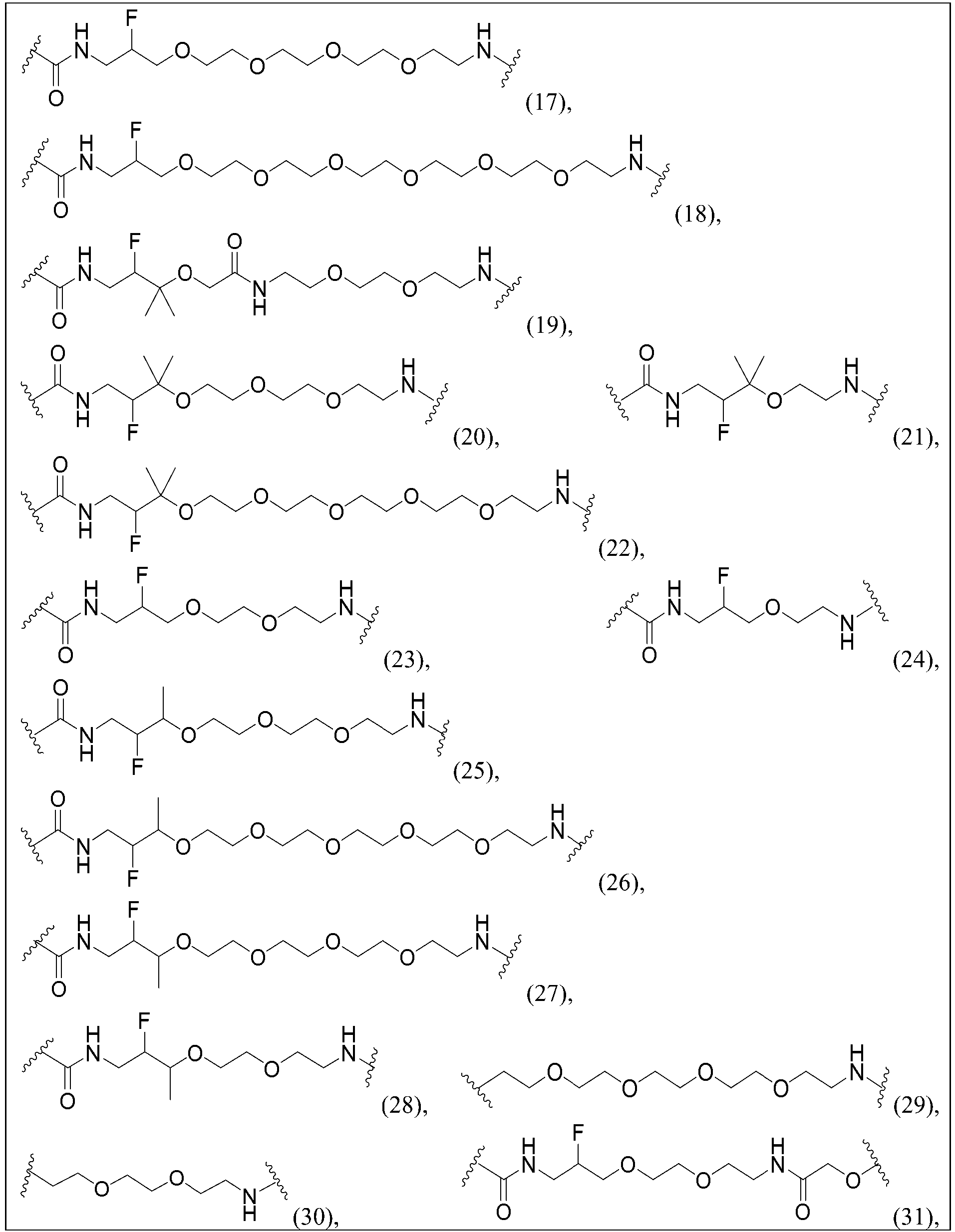

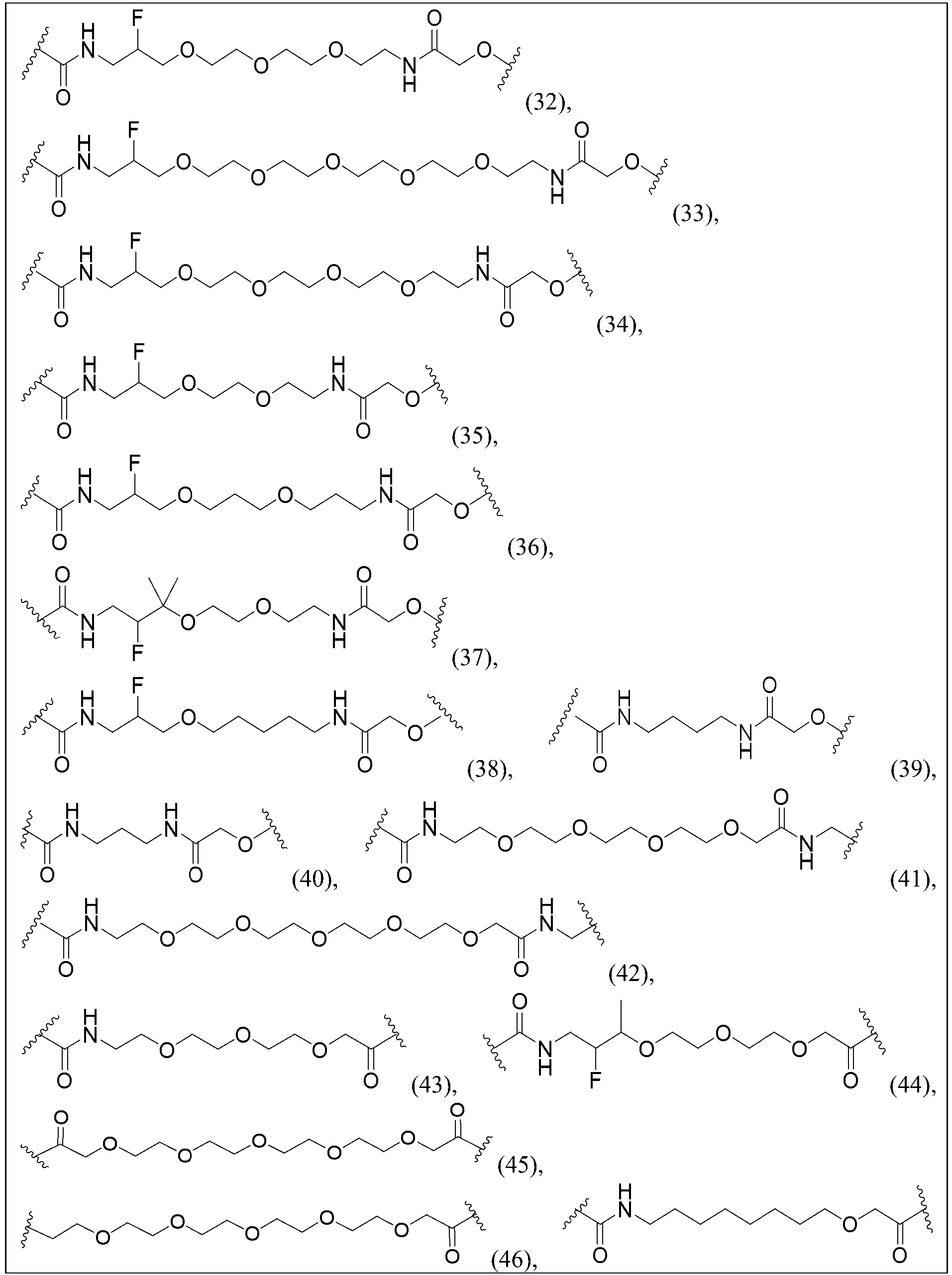

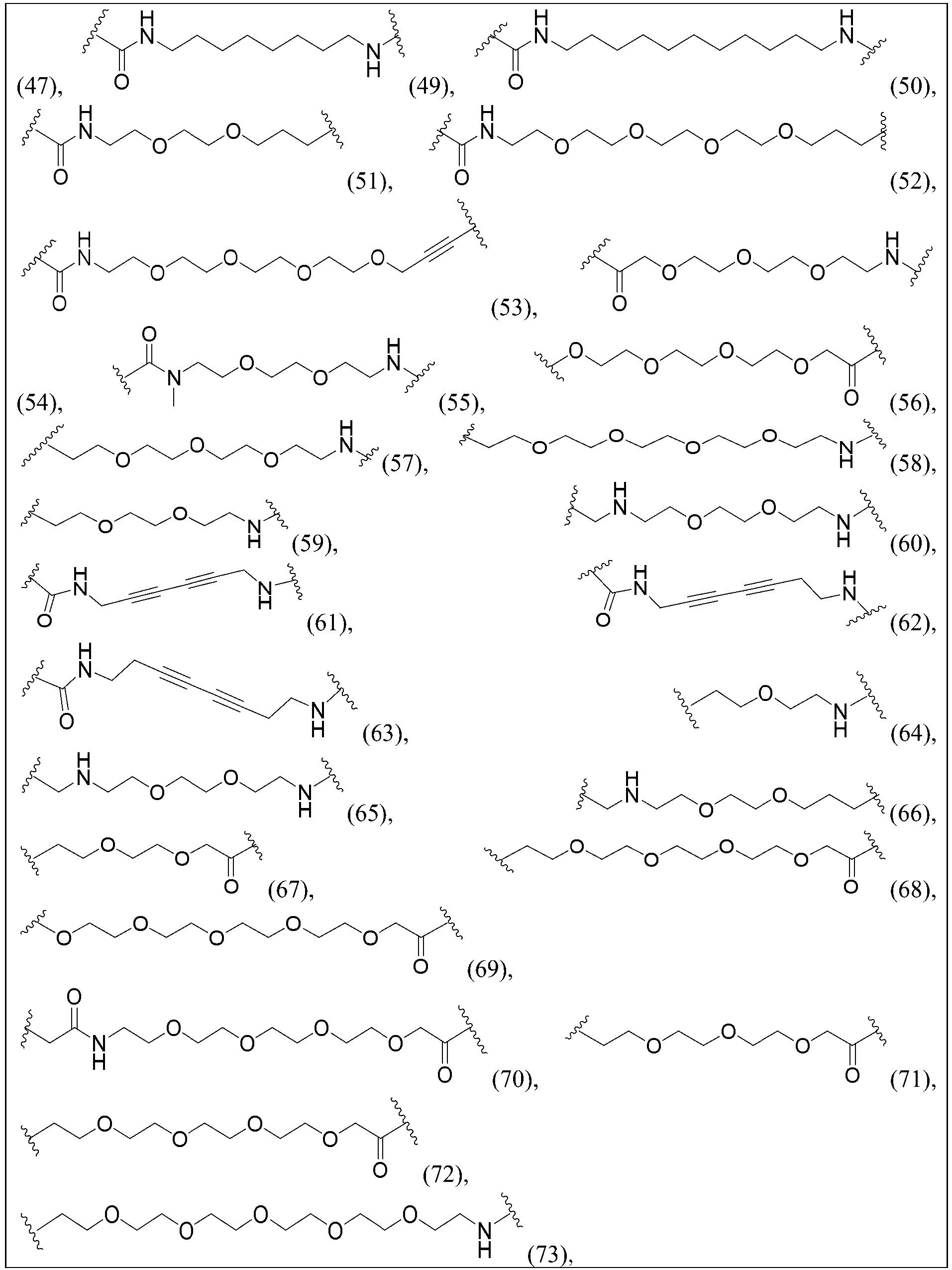

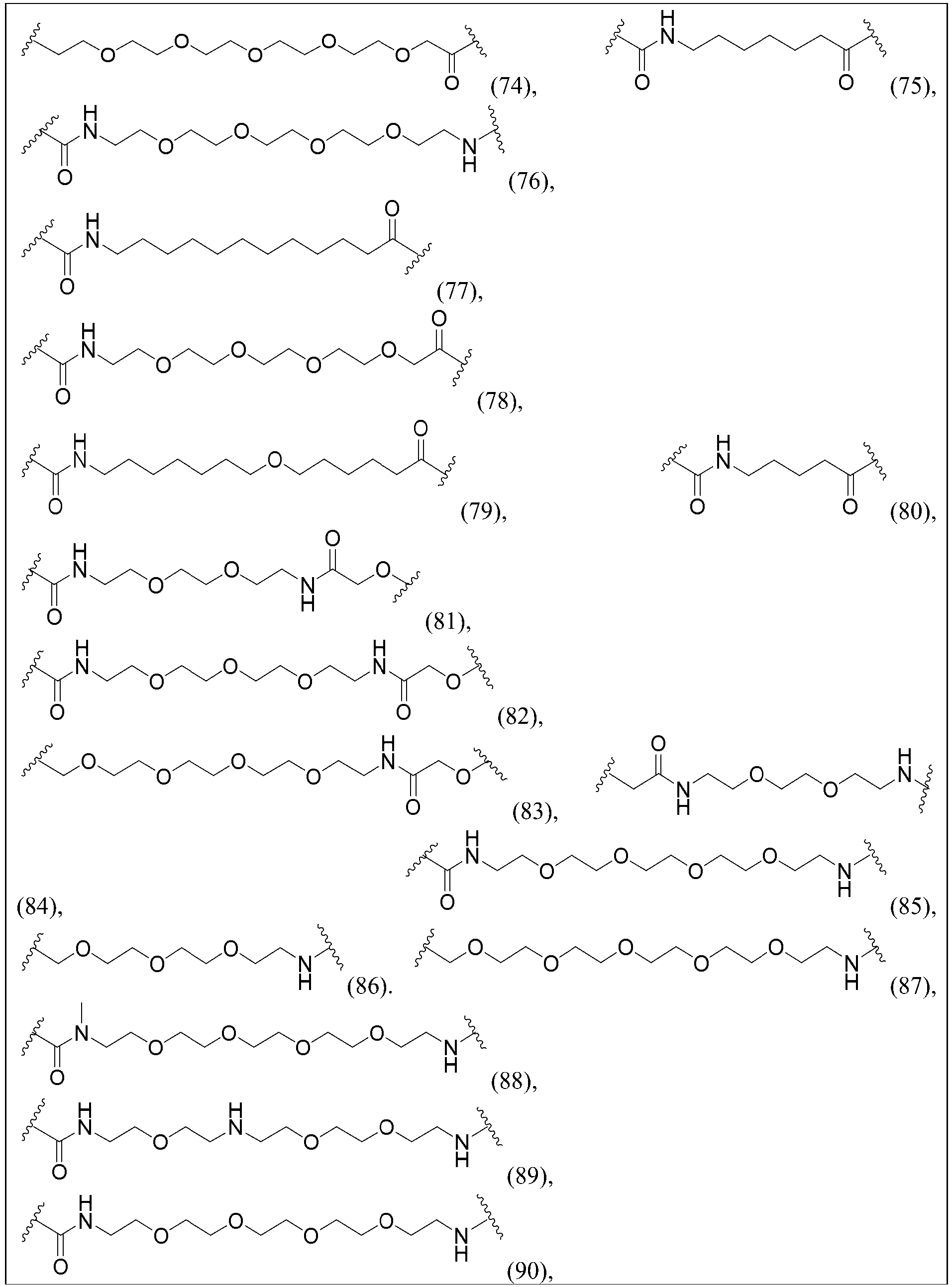

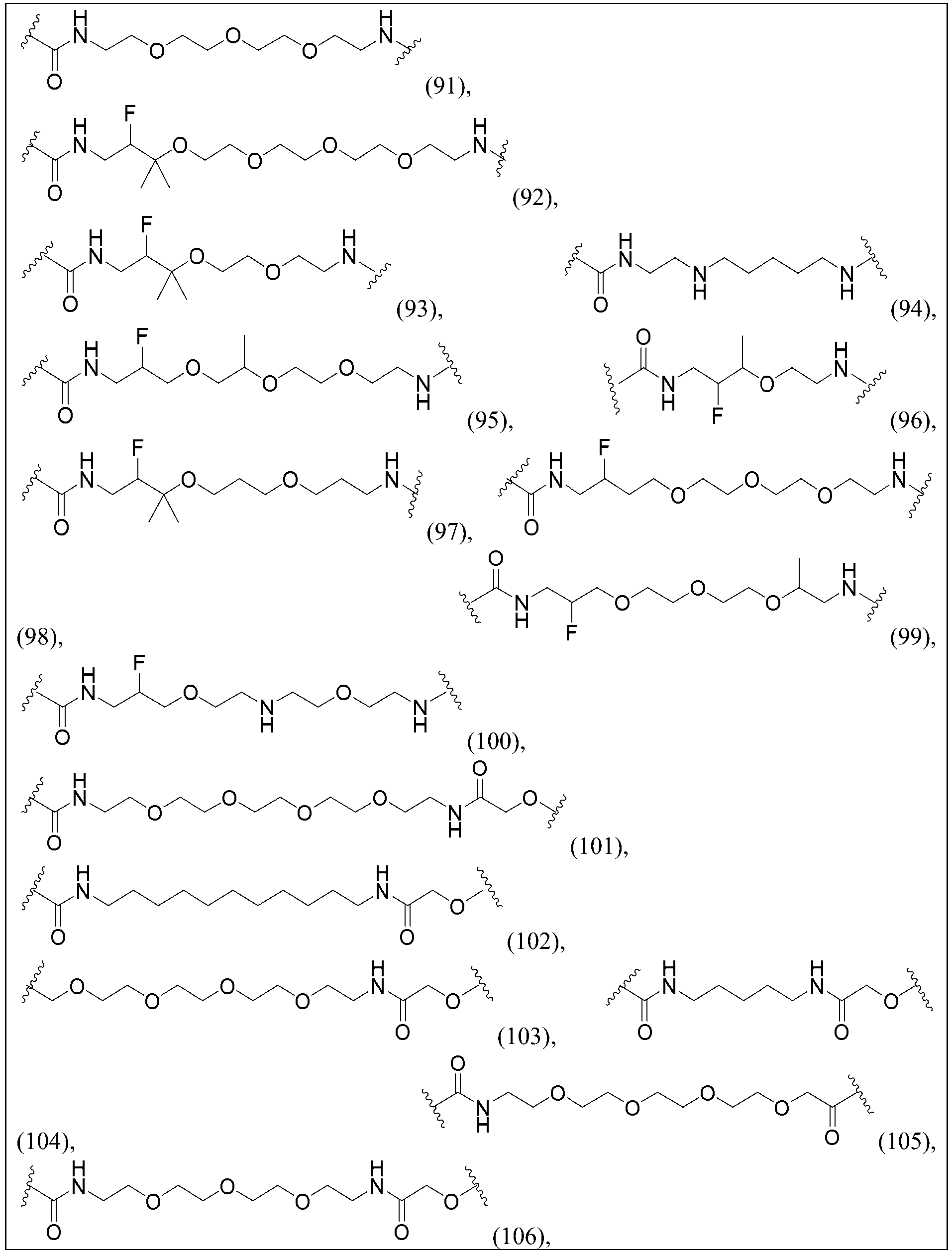

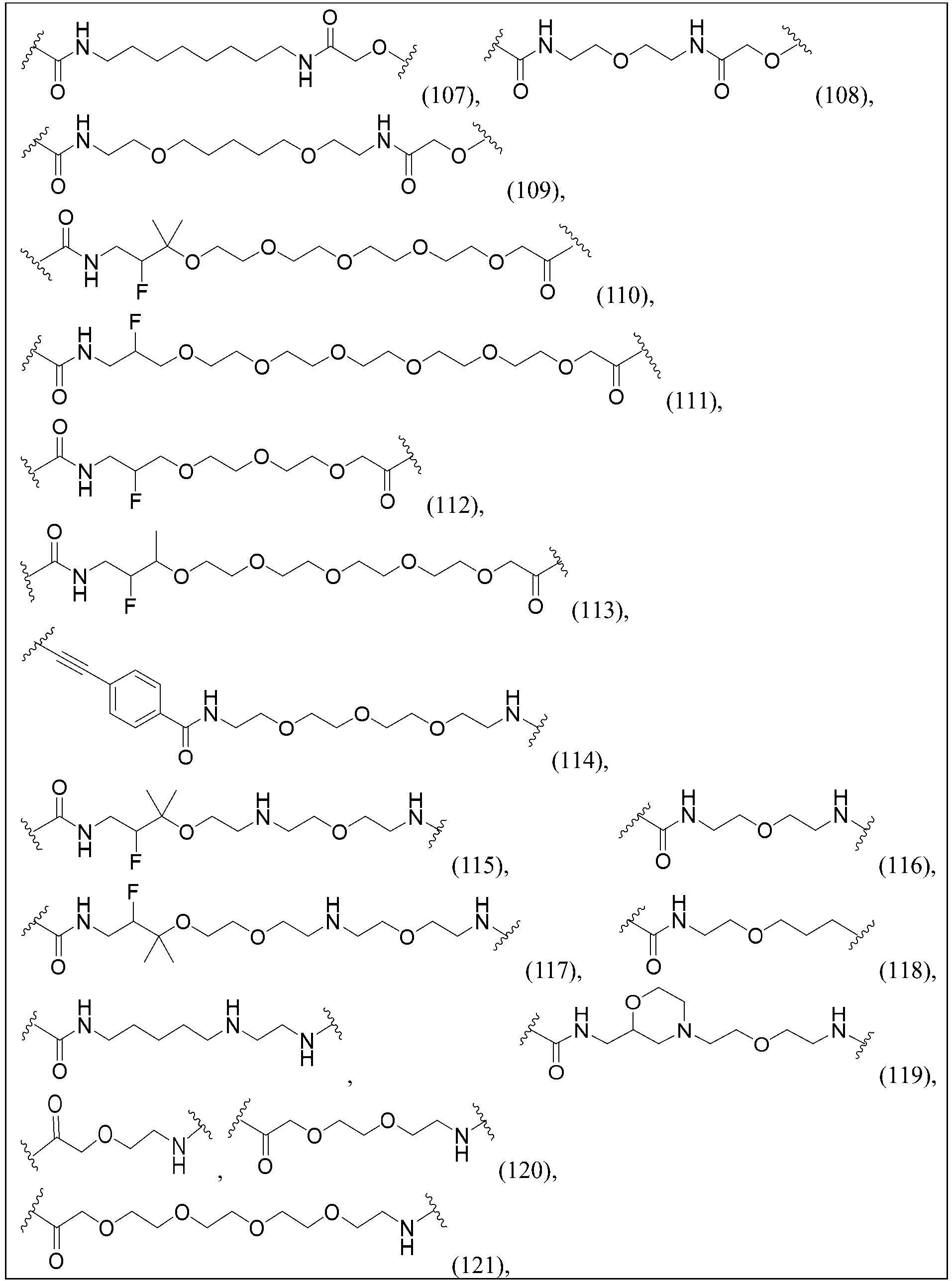

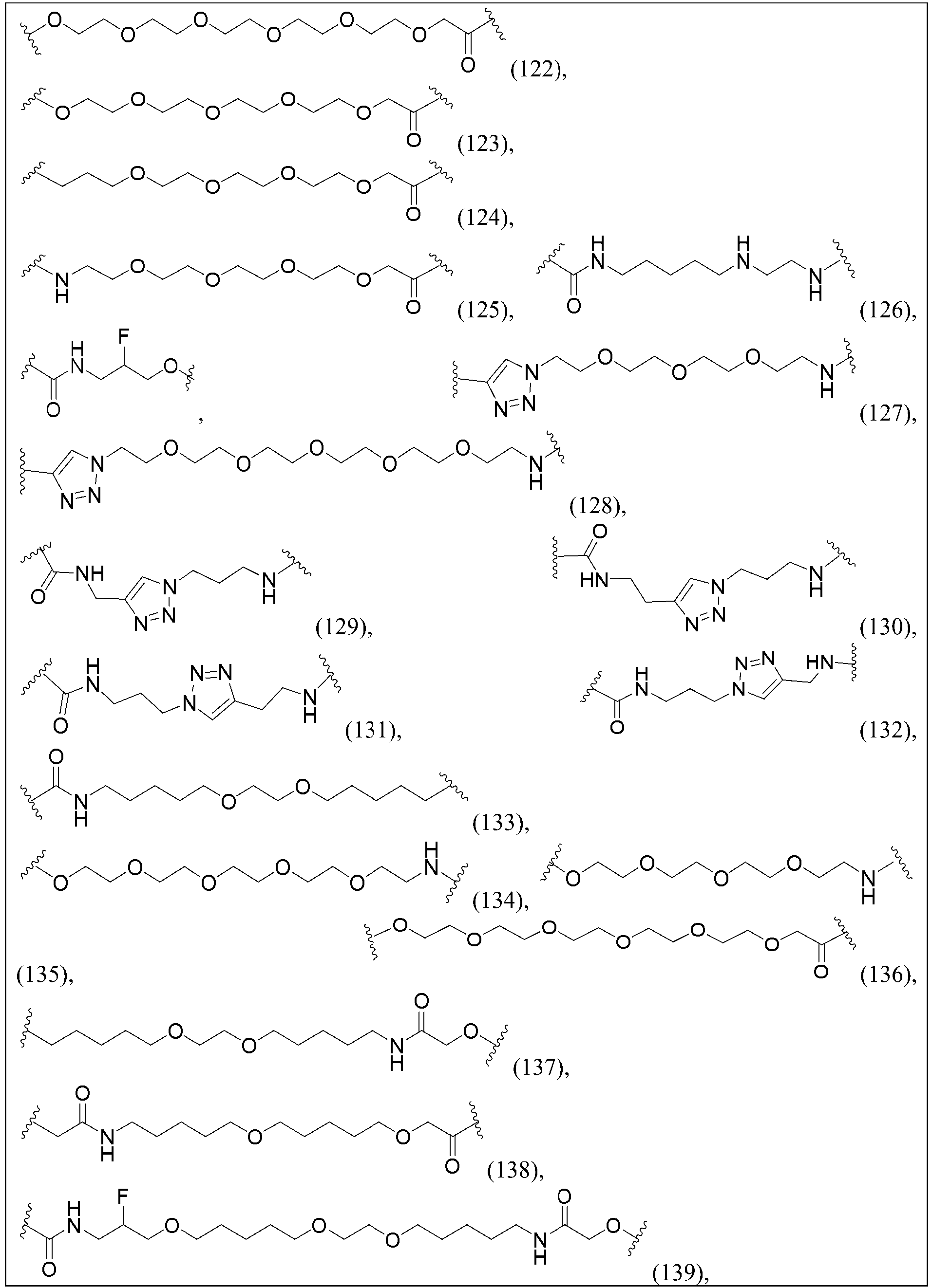

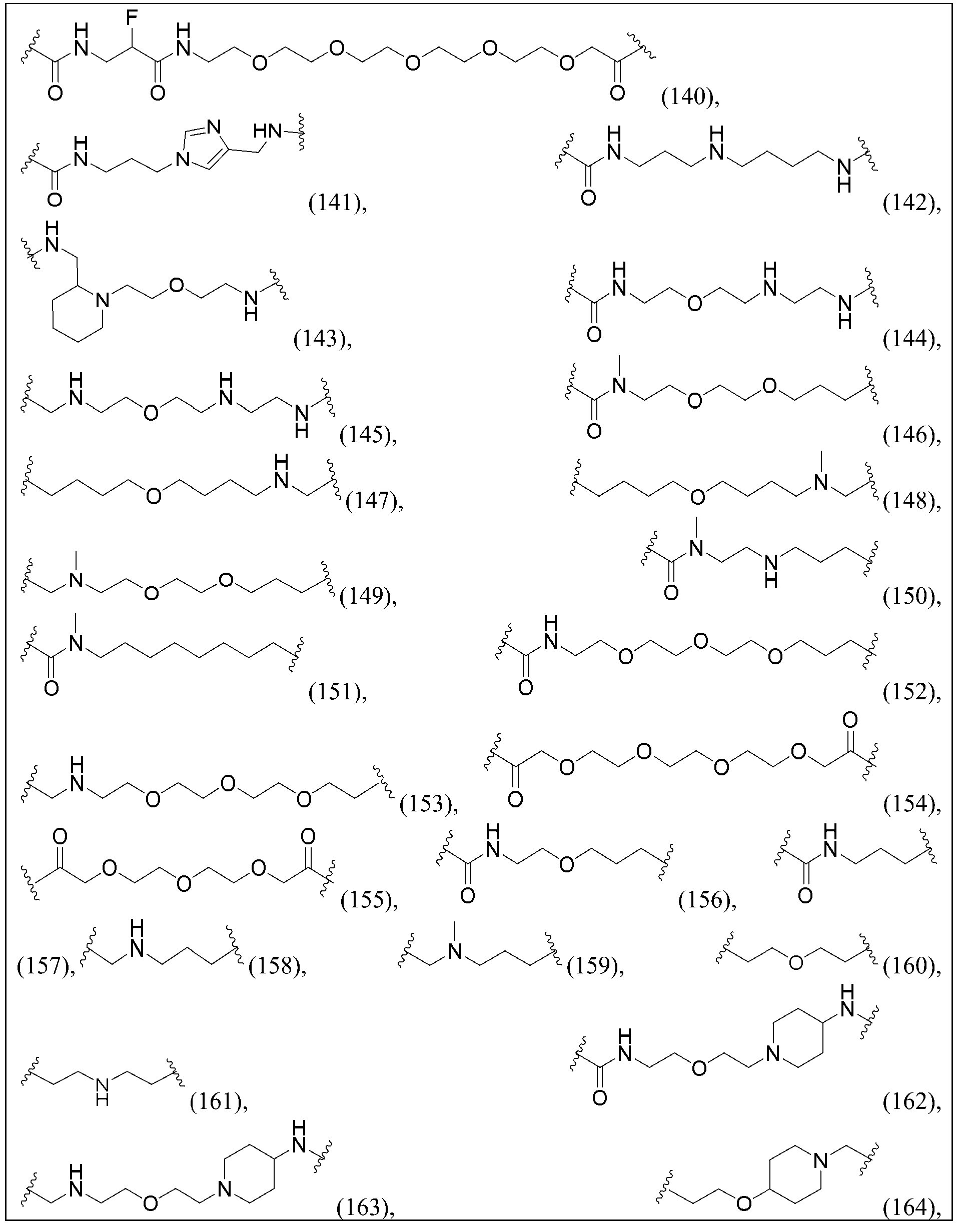

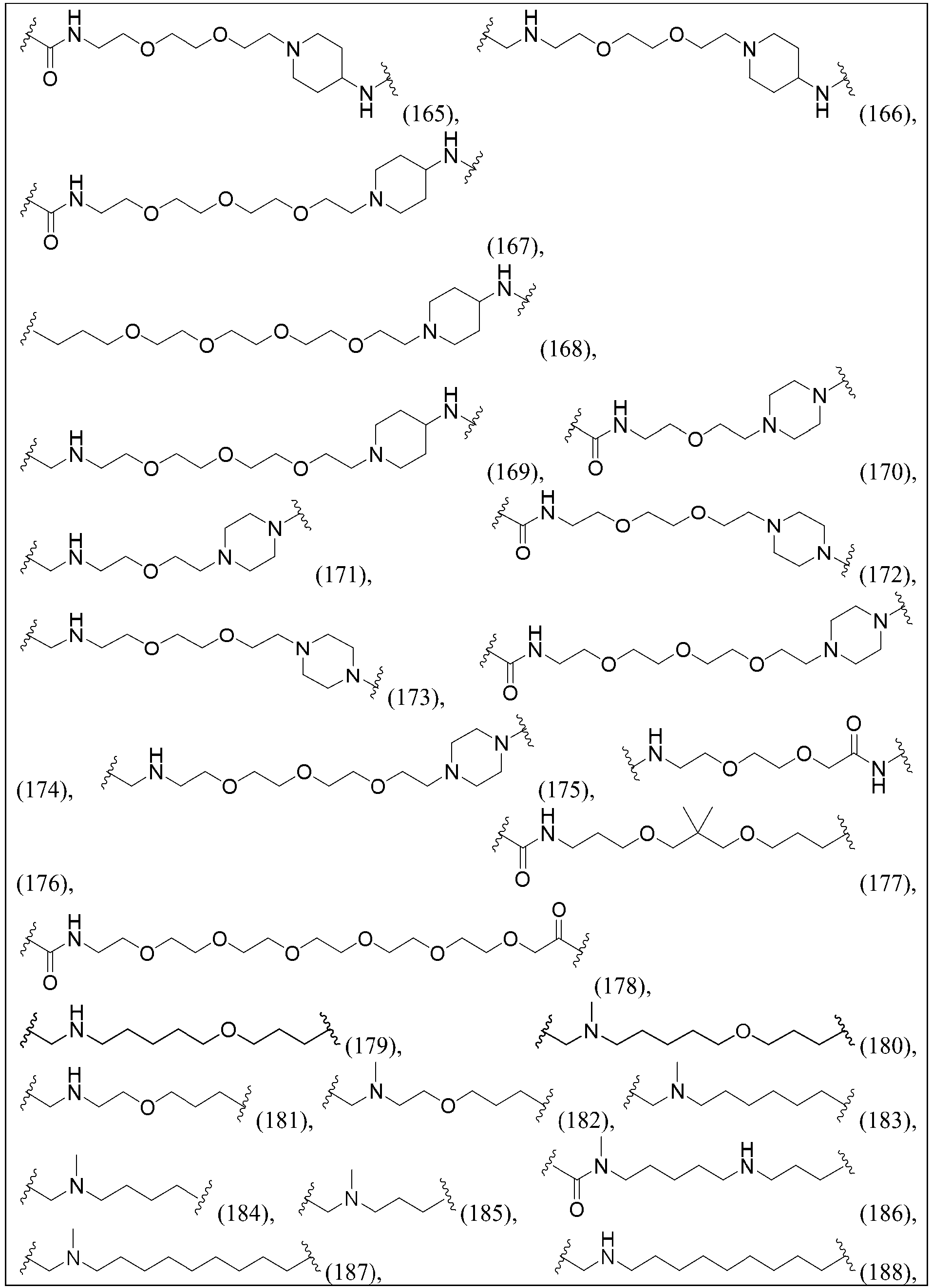

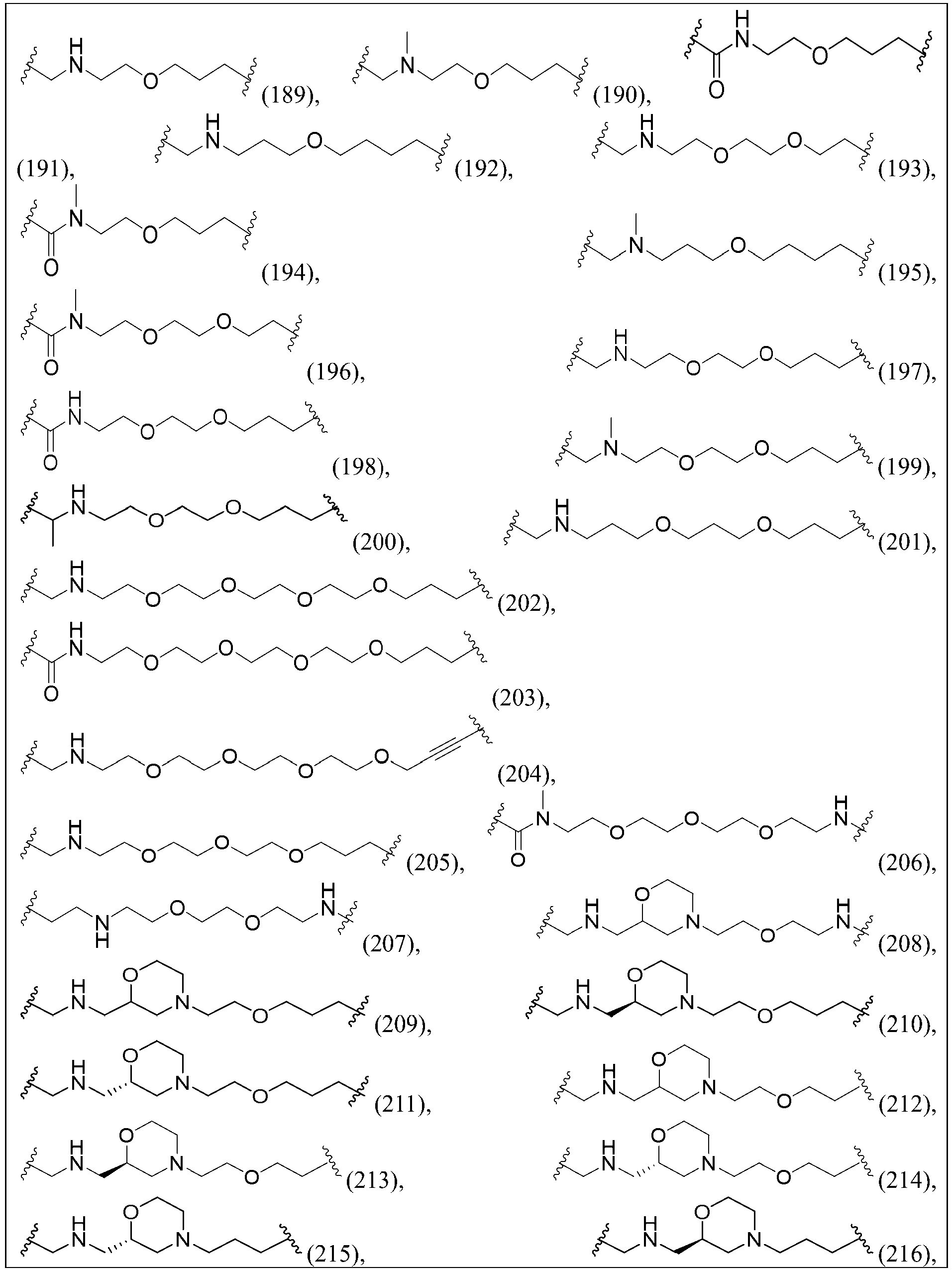

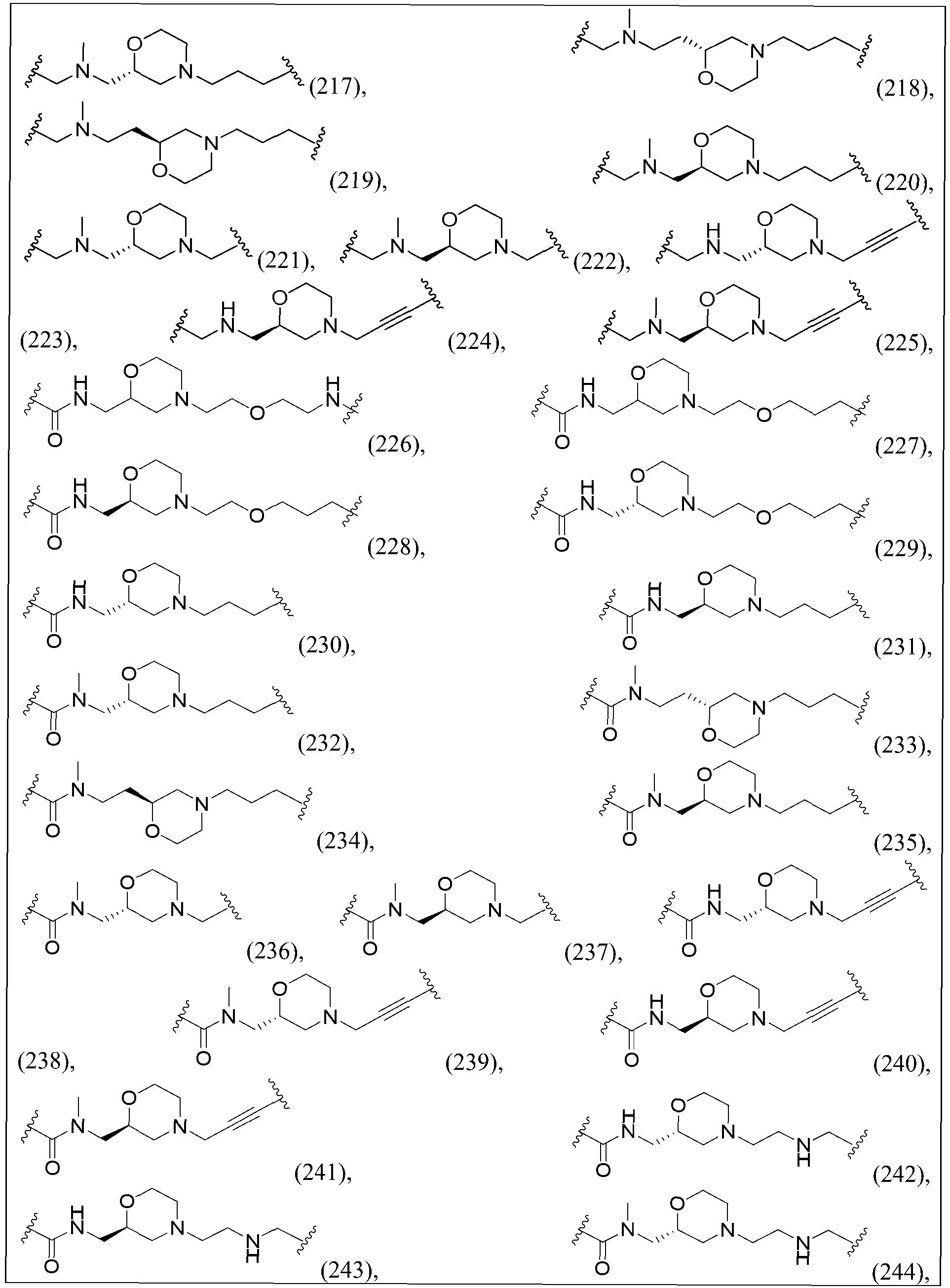

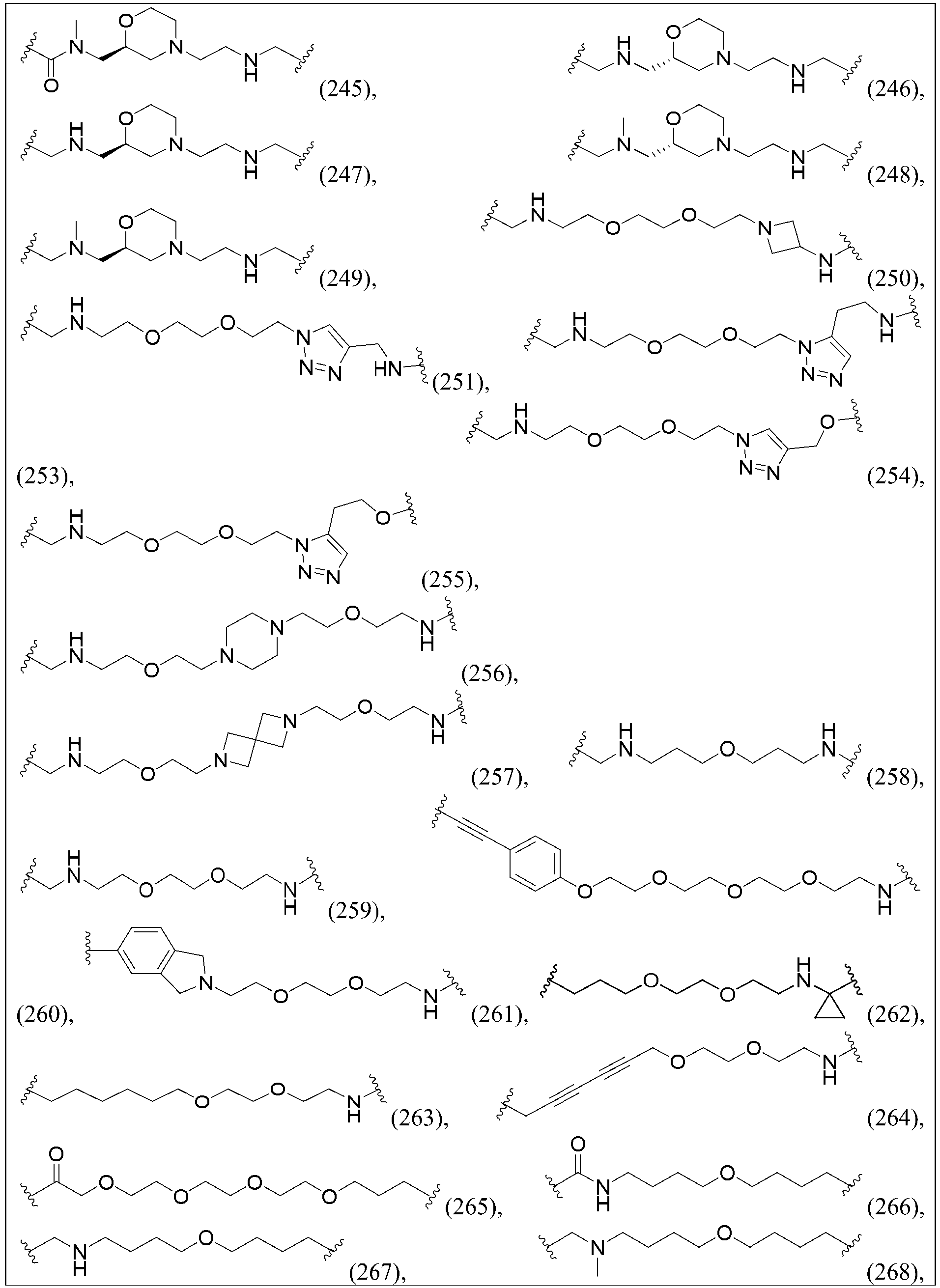

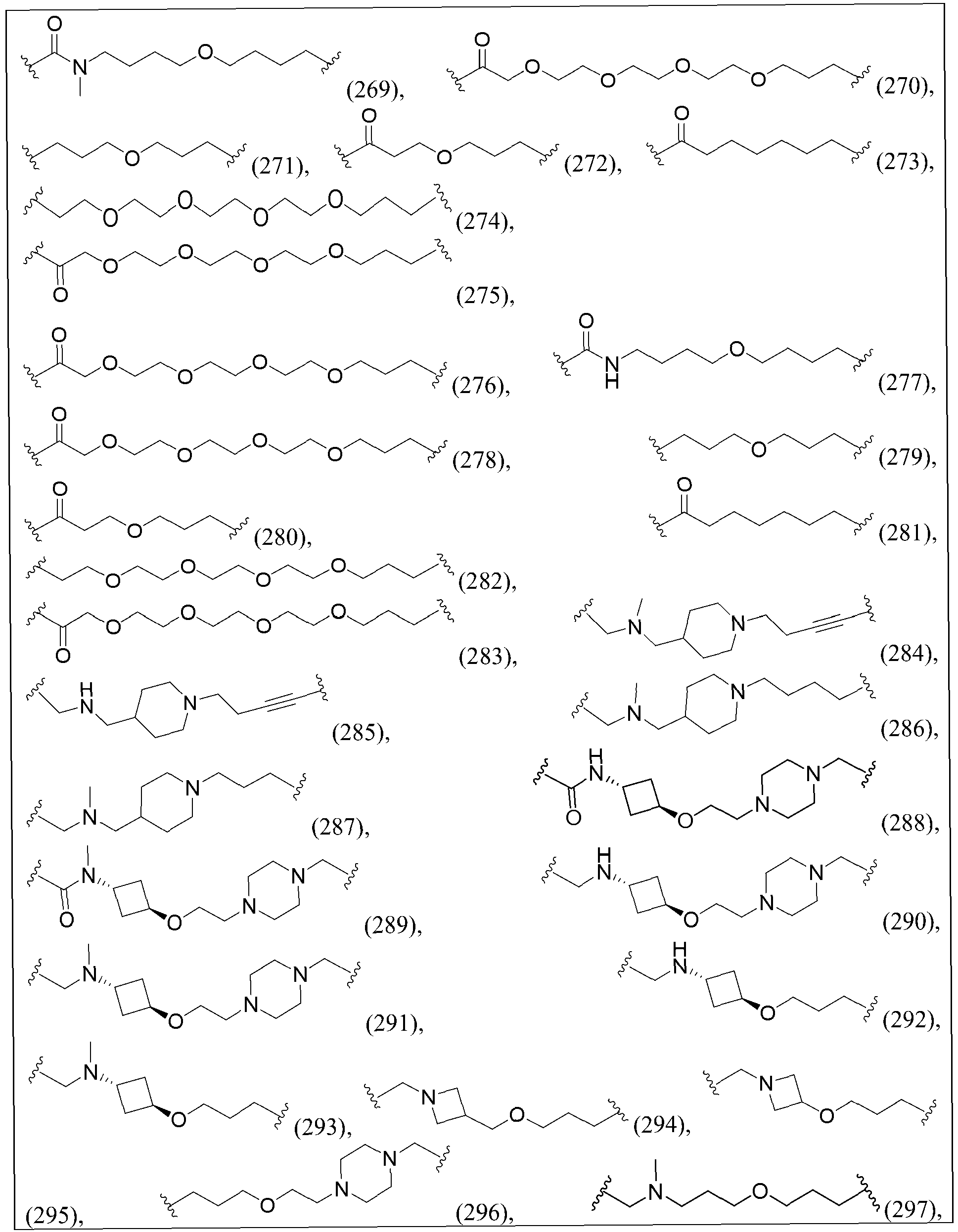

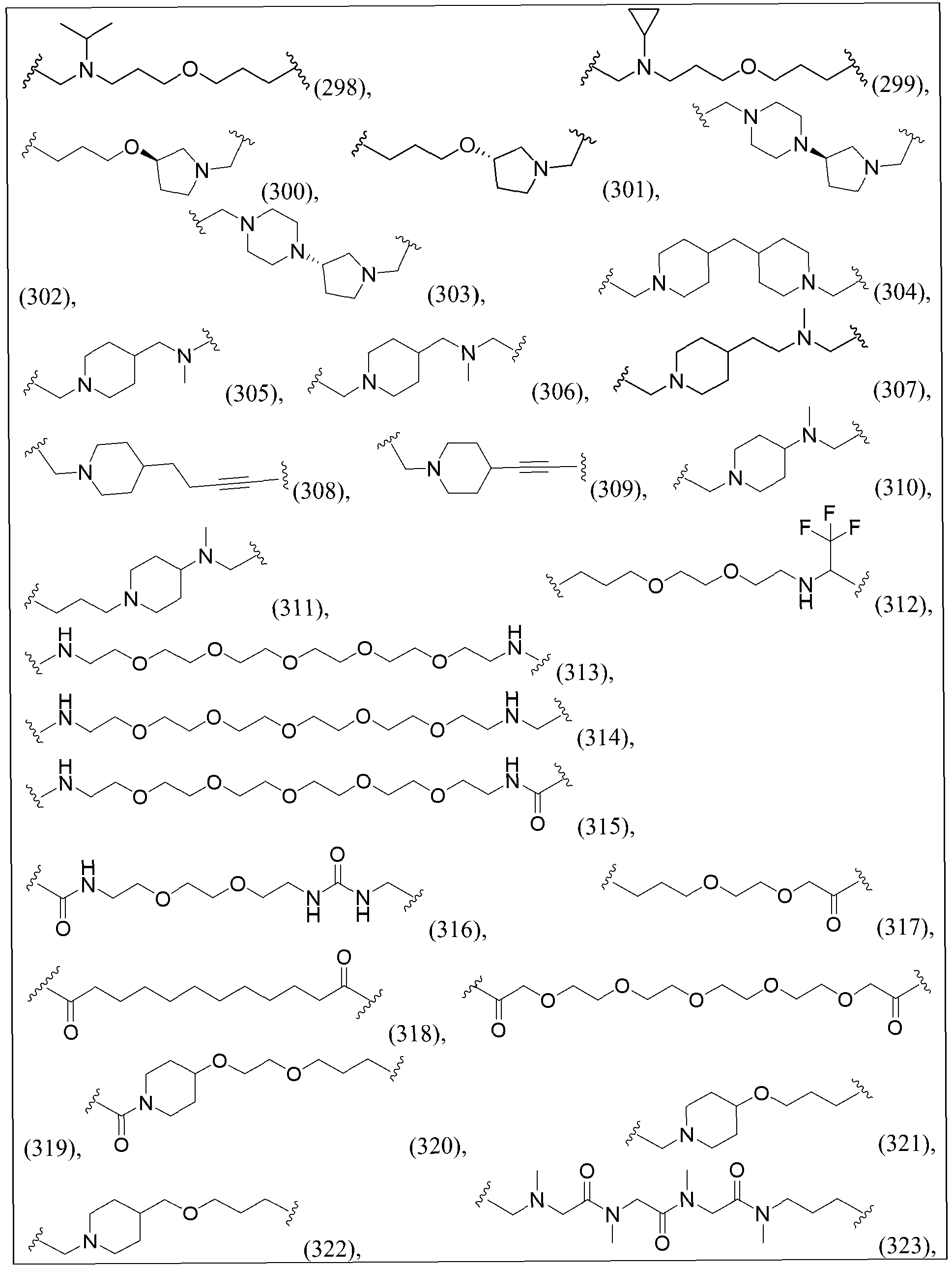

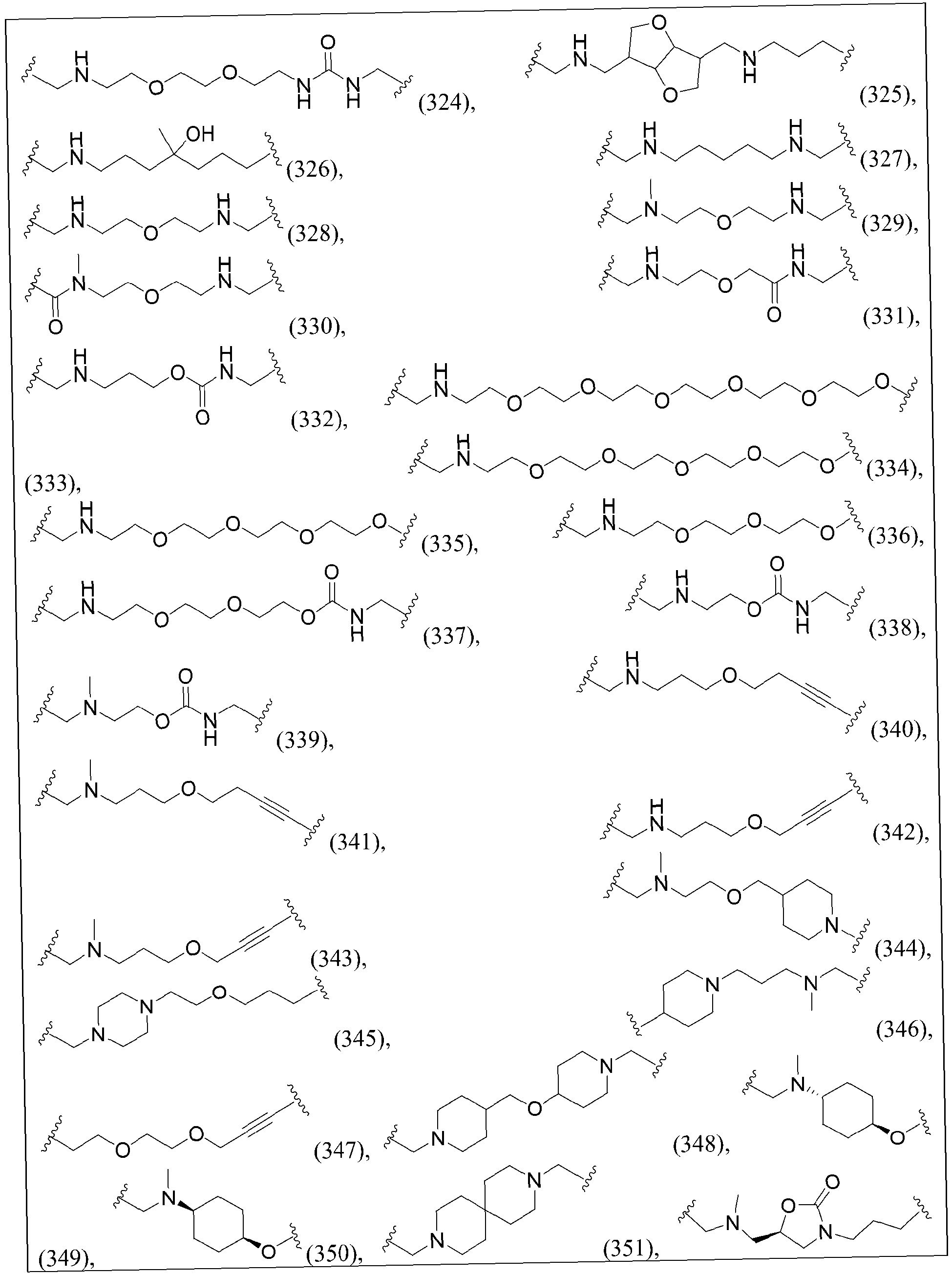

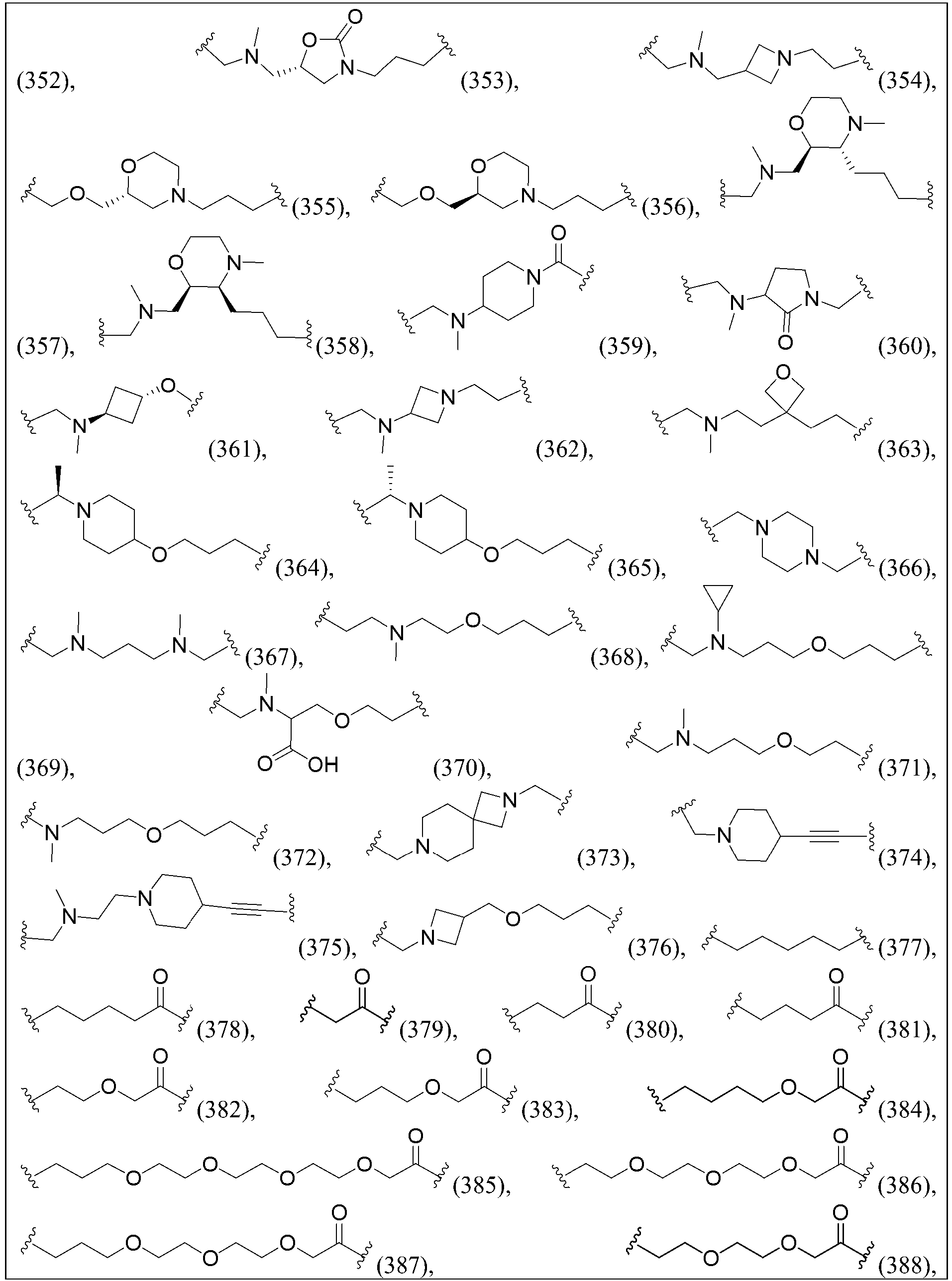

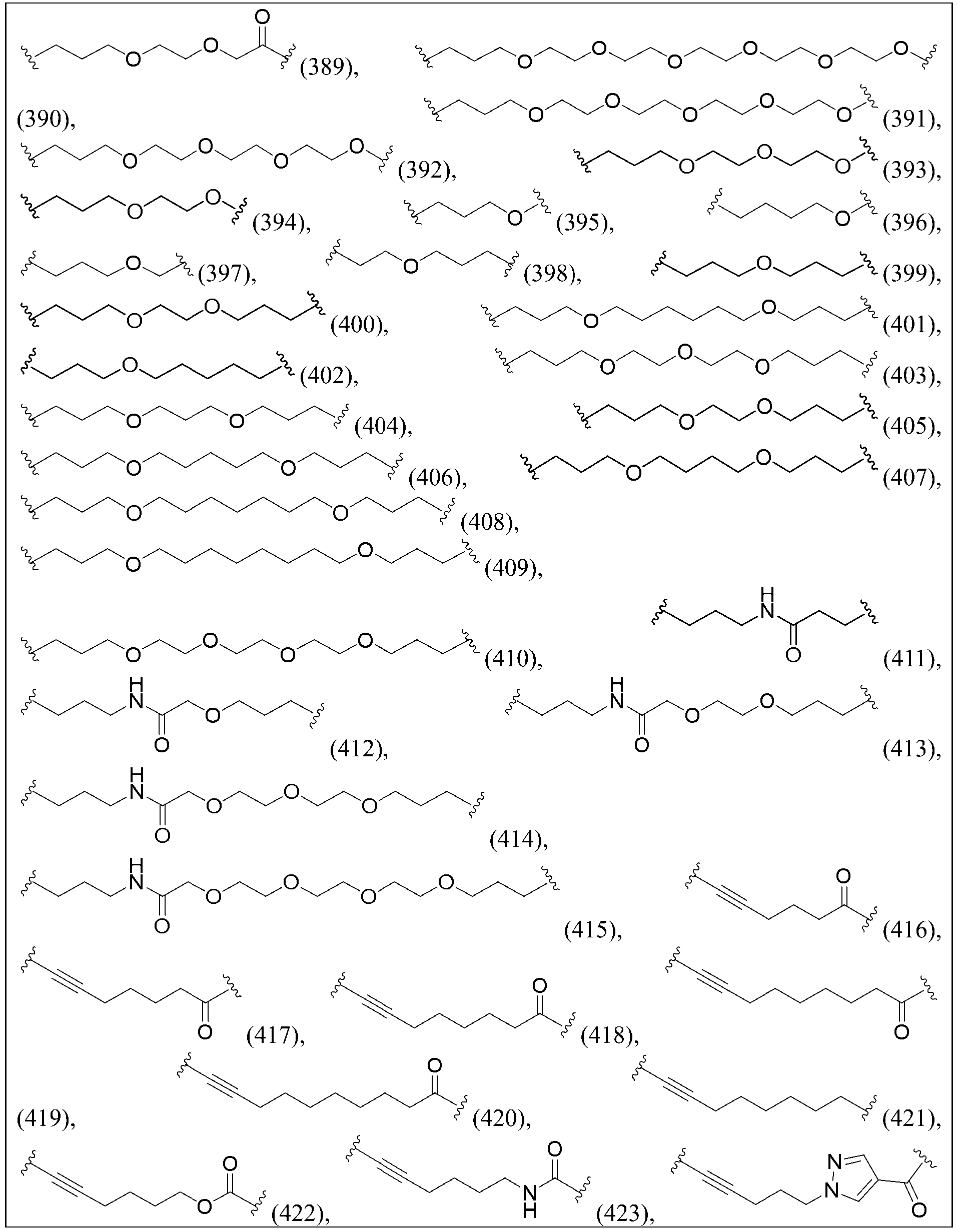

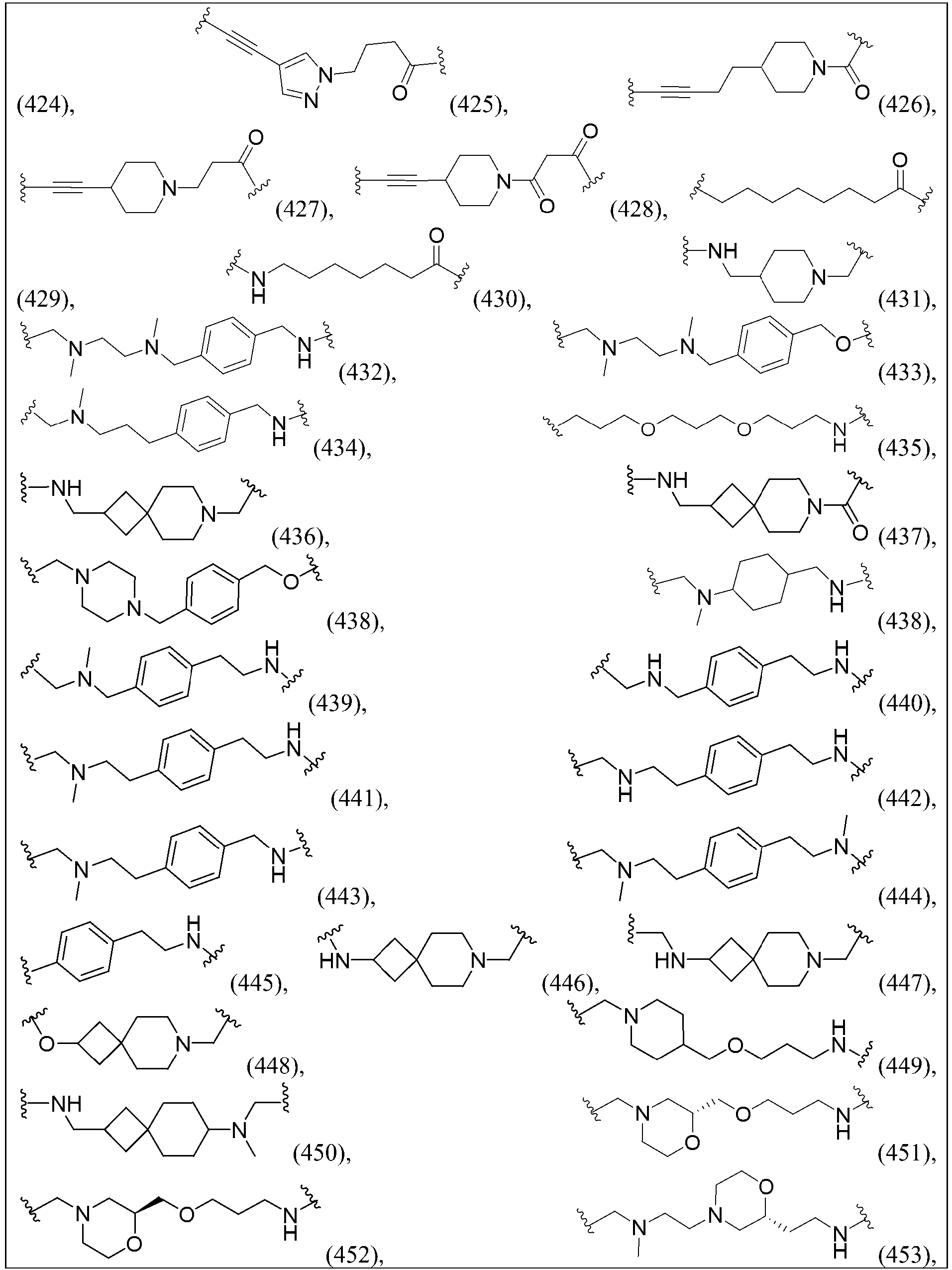

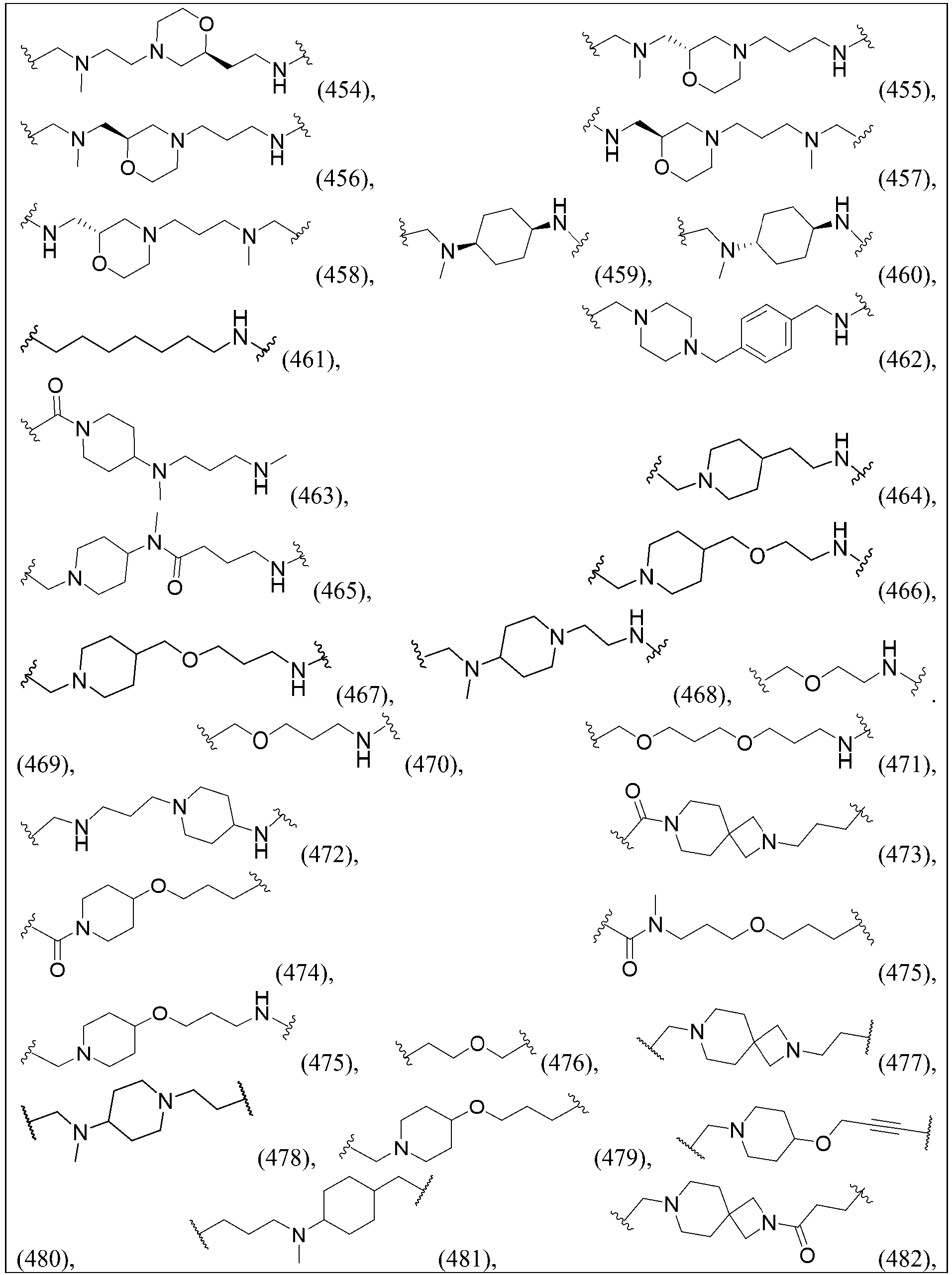

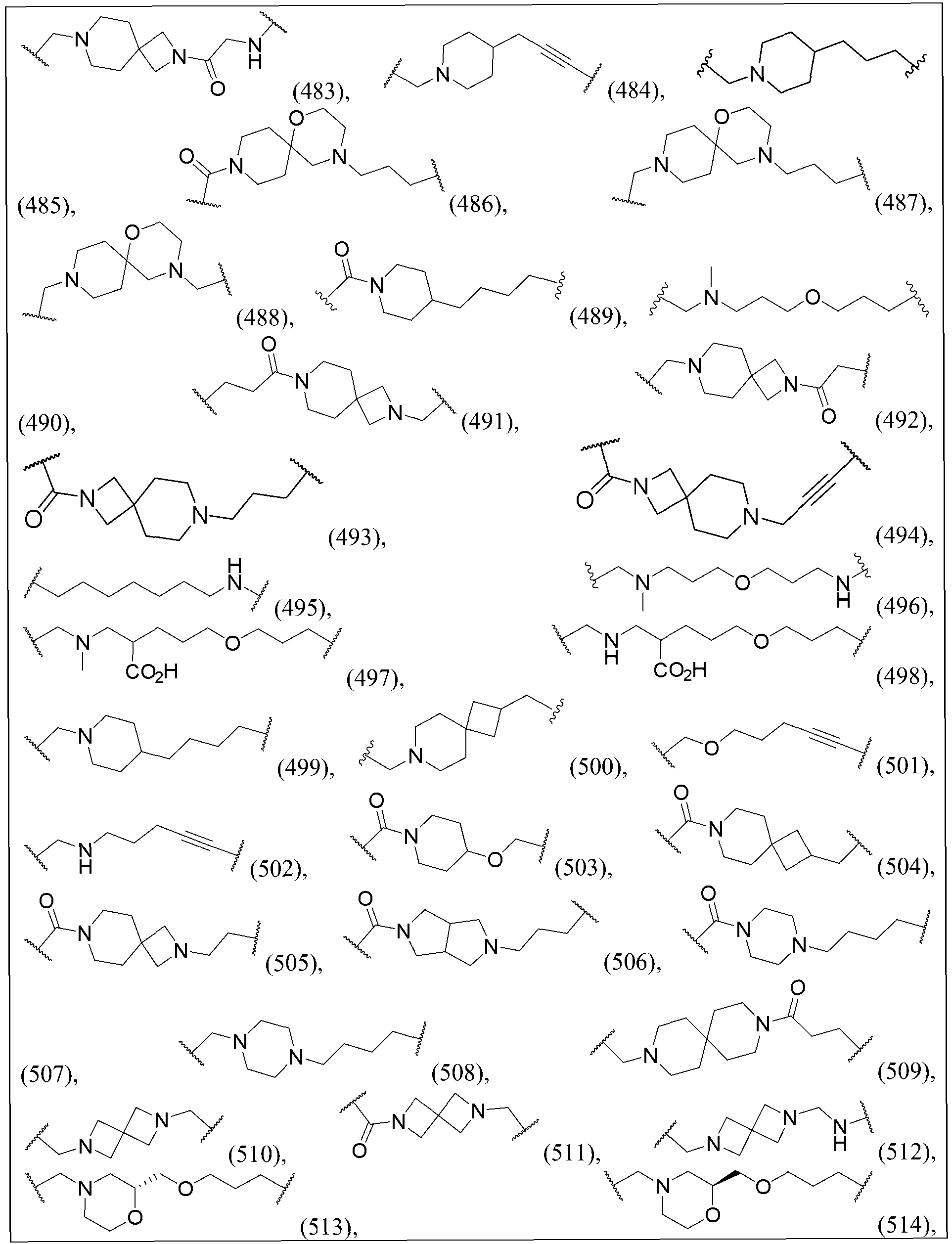

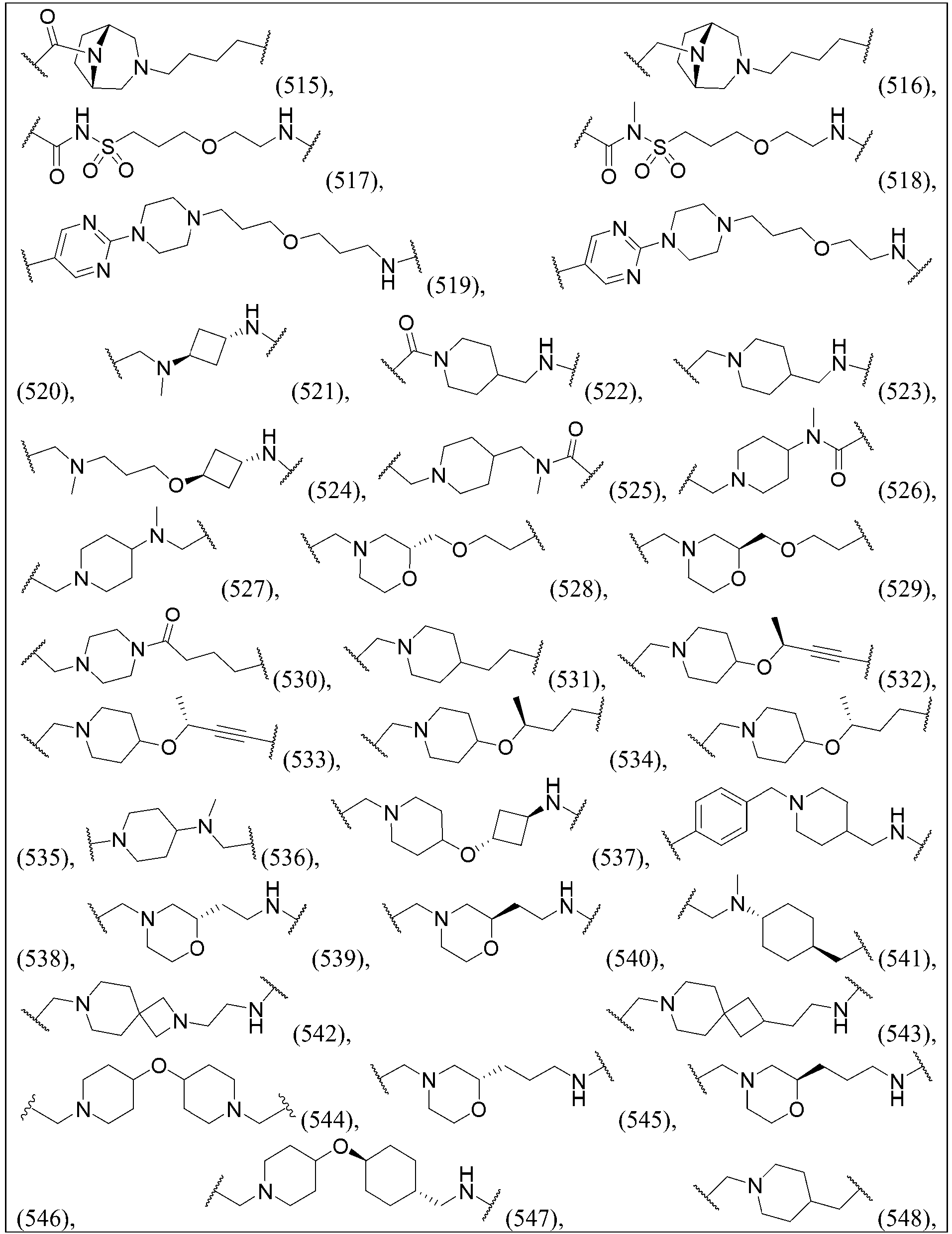

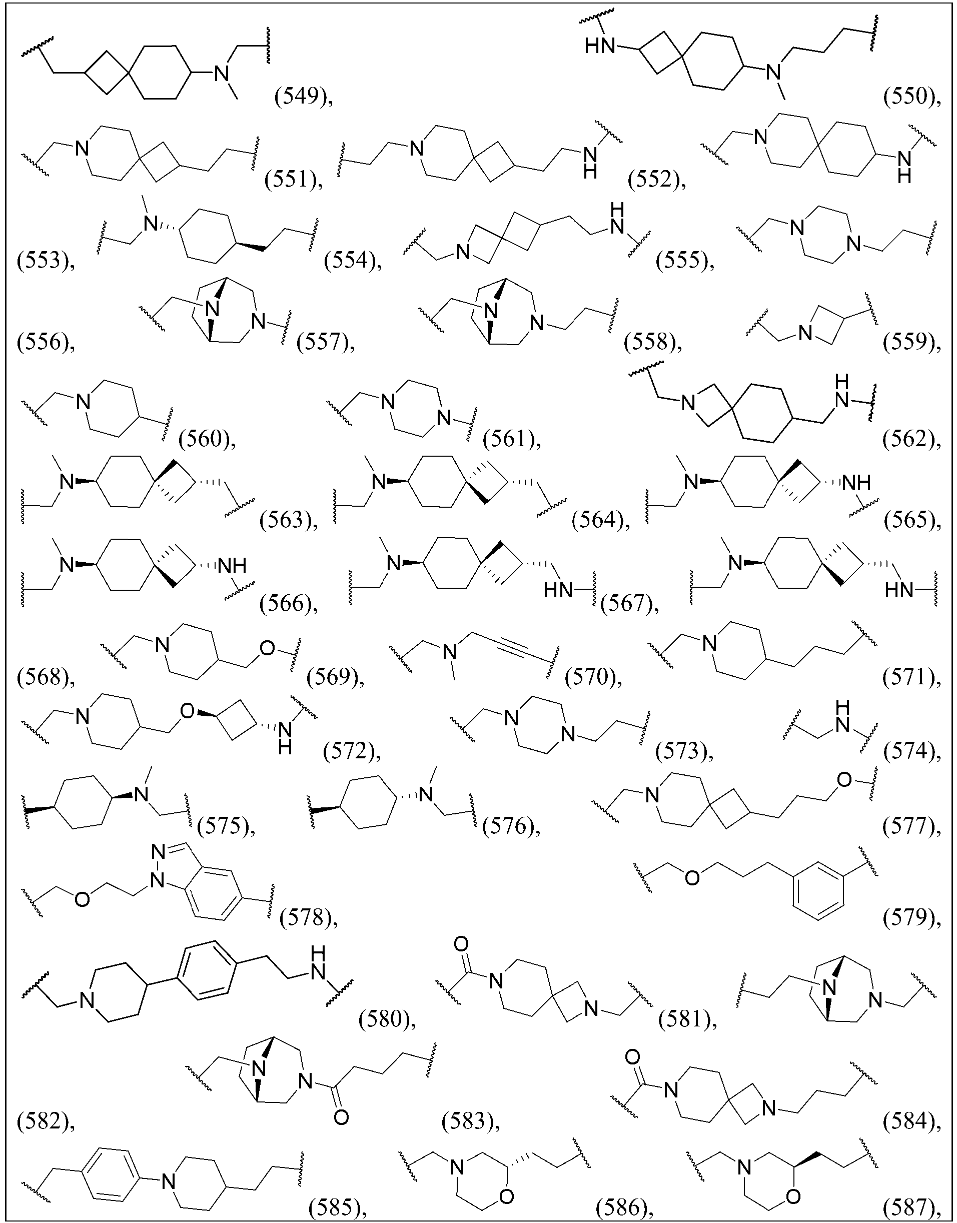

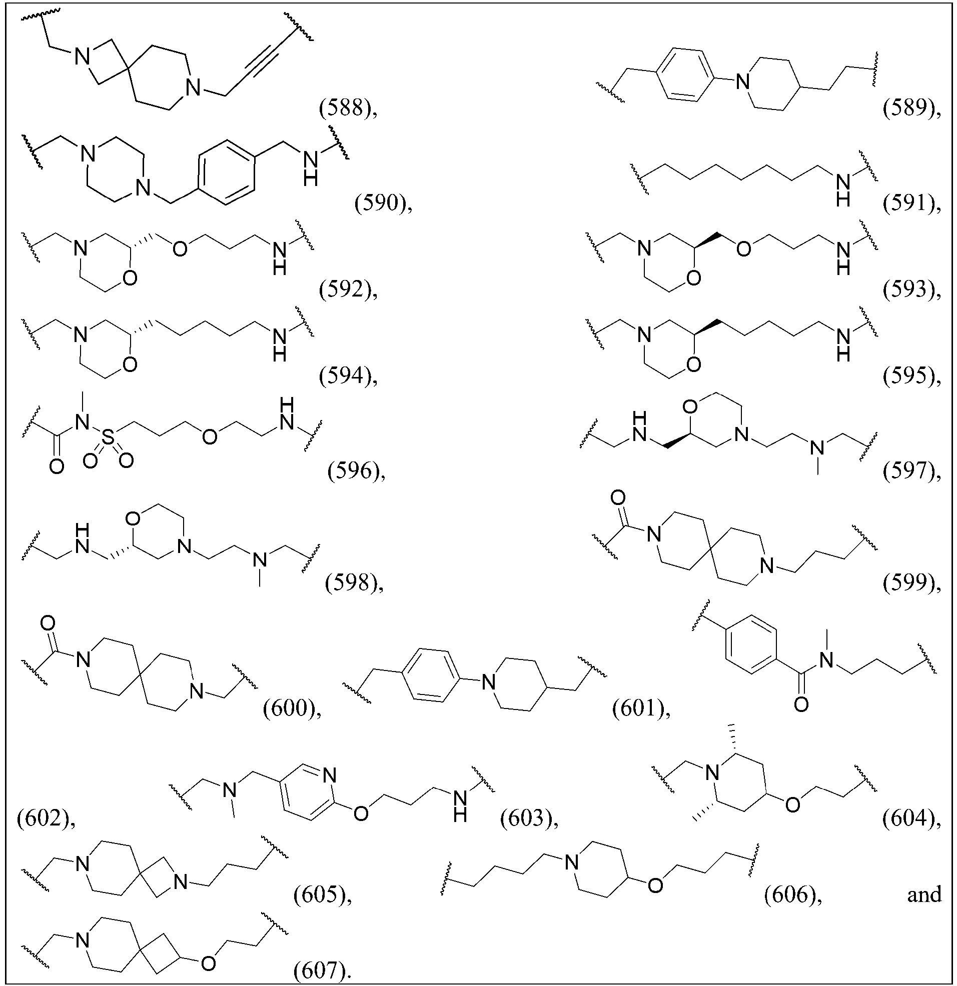

In some embodiments, the present invention provides a compound having an IRAK binding moiety described and disclosed herein, a LBM set forth in **Table A** above, and a linker set forth in **Table B** above, or a pharmaceutically acceptable salt thereof.

Exemplary compounds of the invention are set forth in **Table 1,** below.

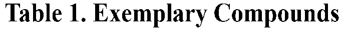

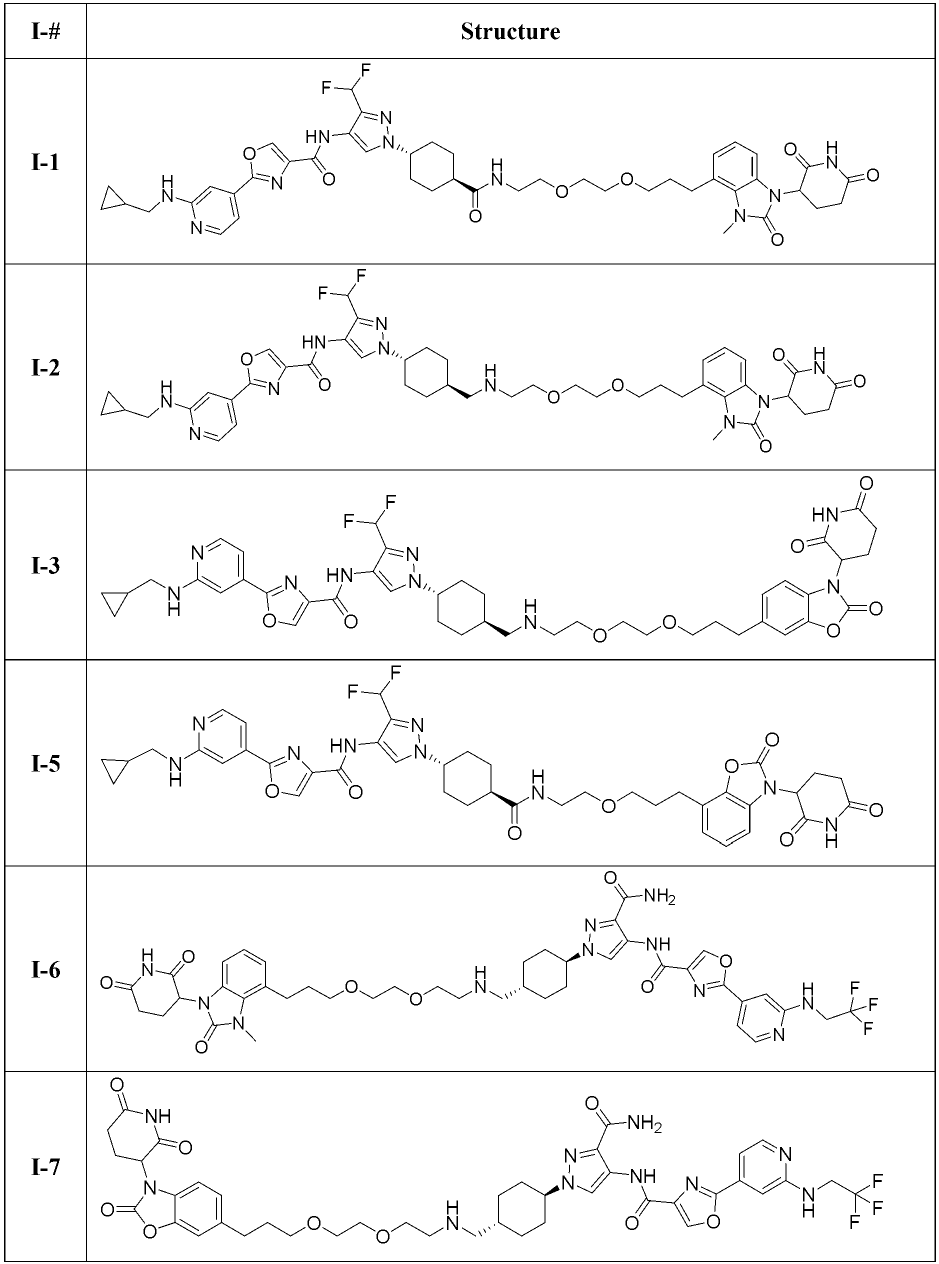

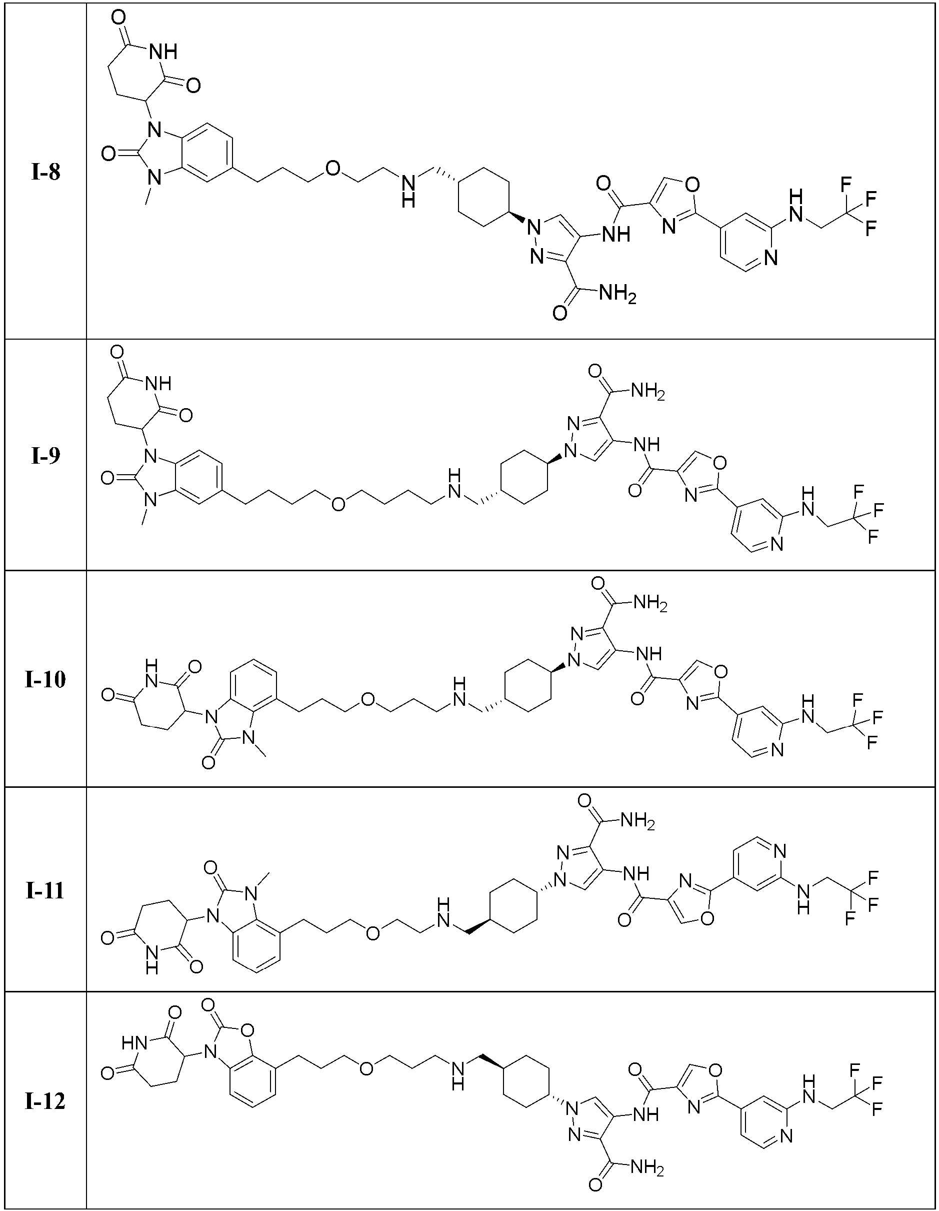

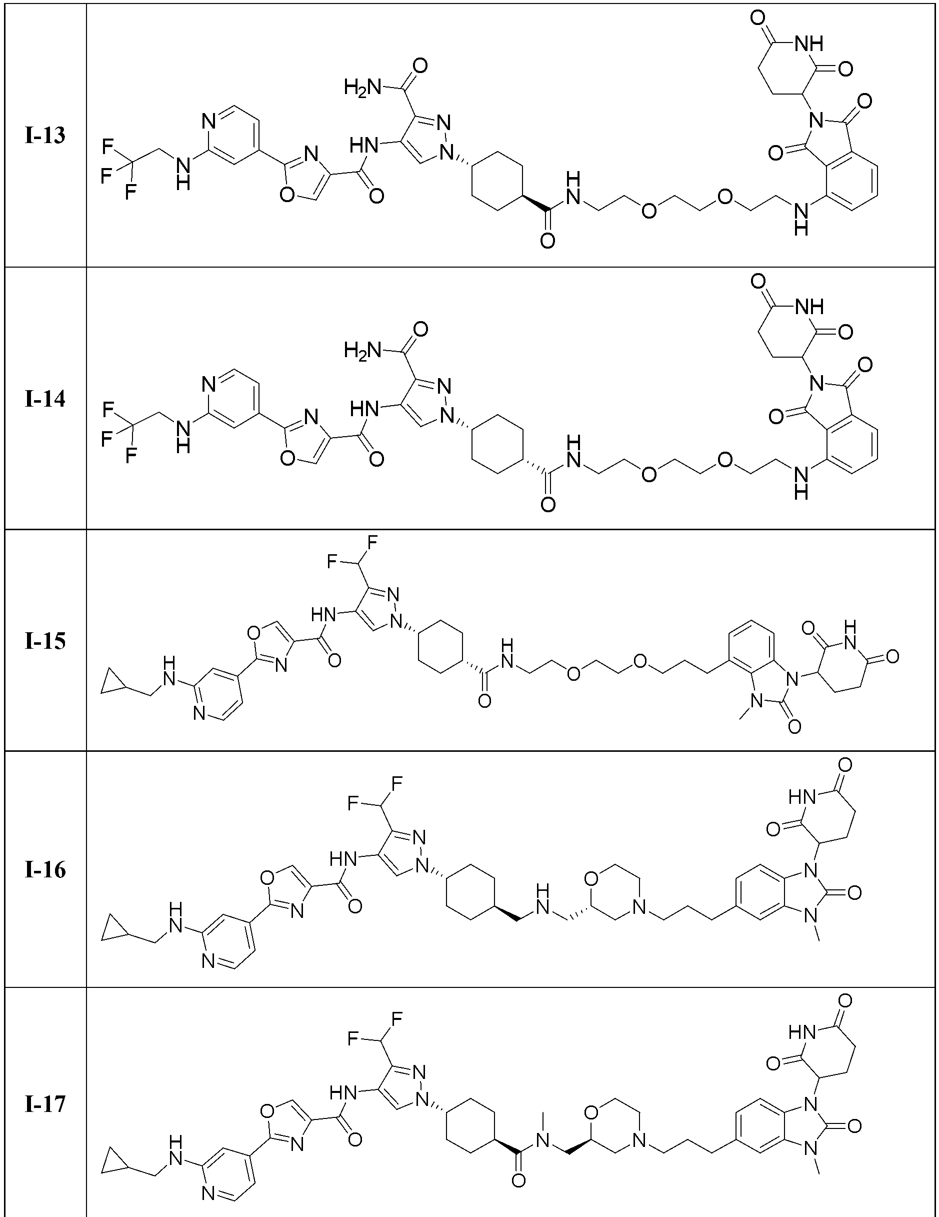

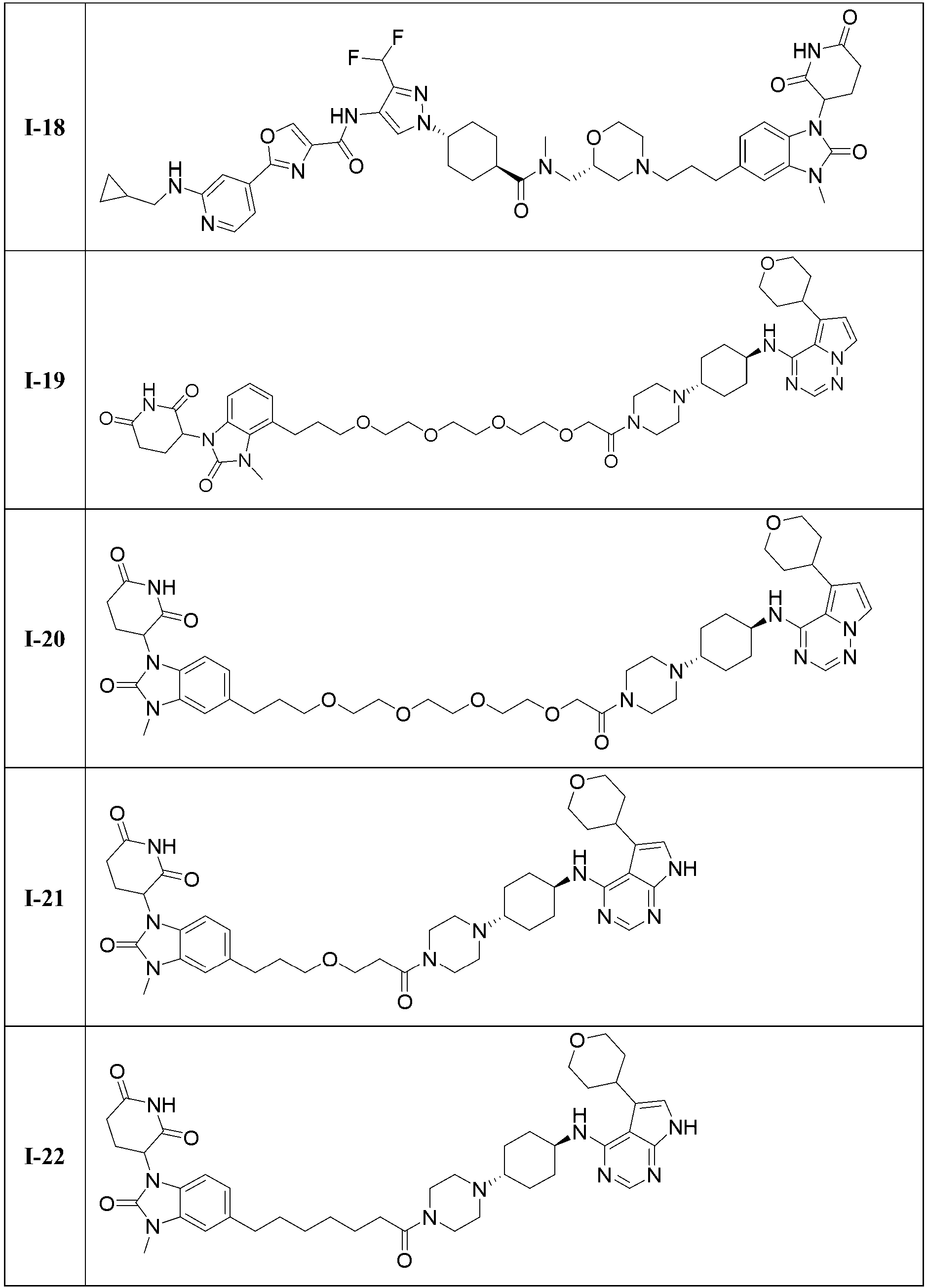

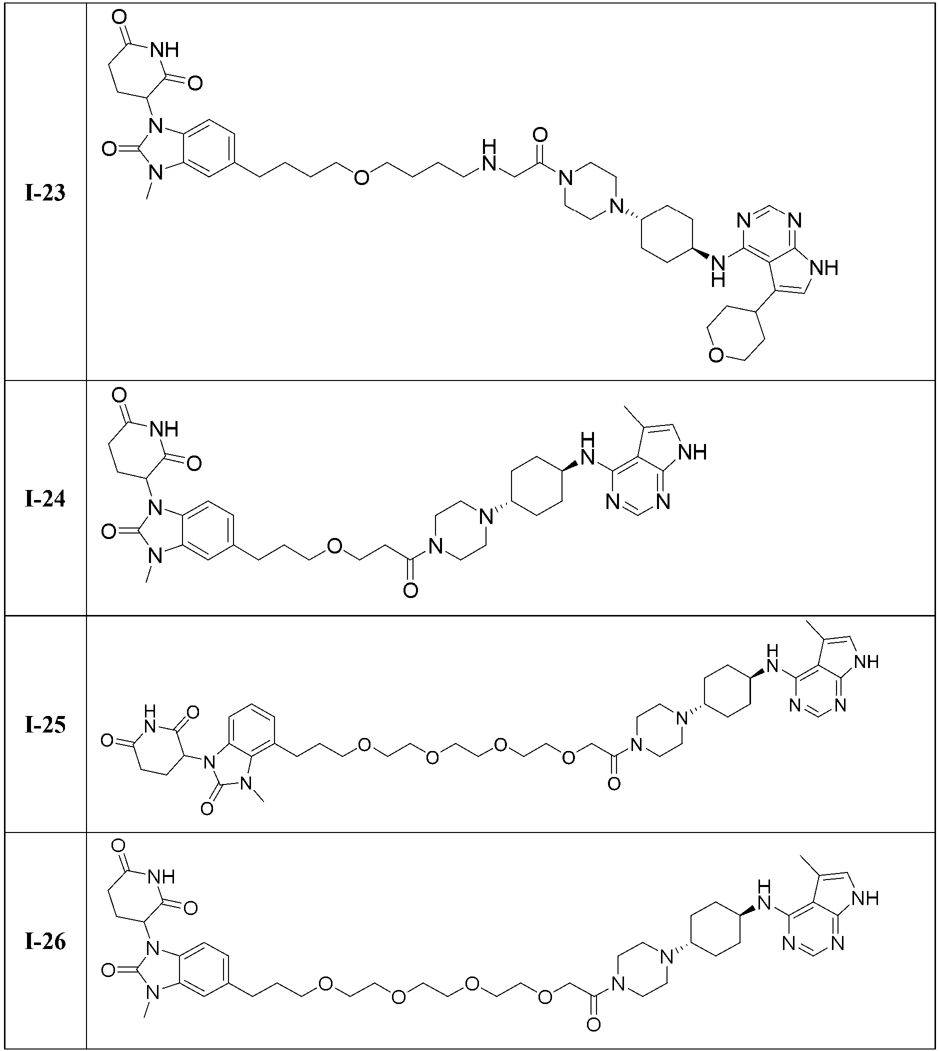

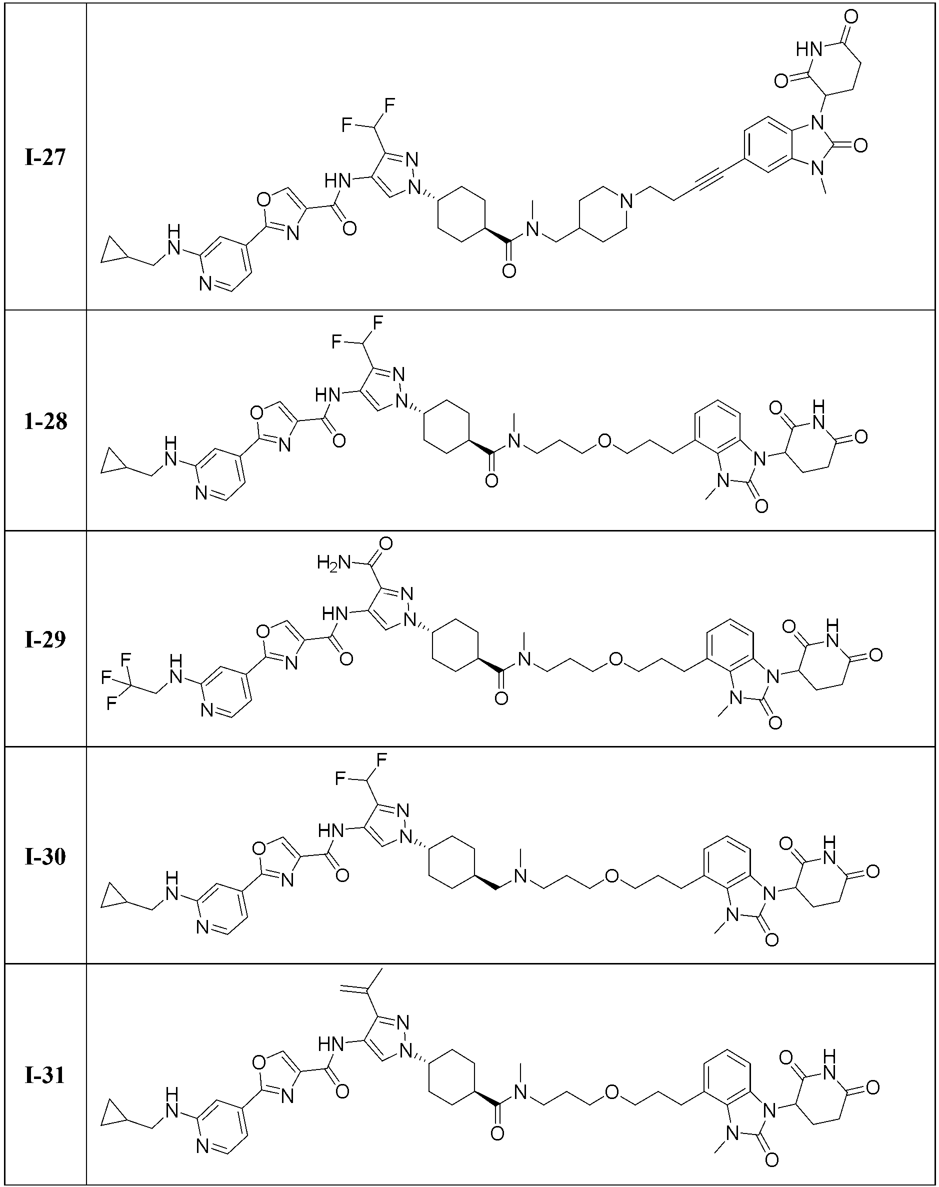

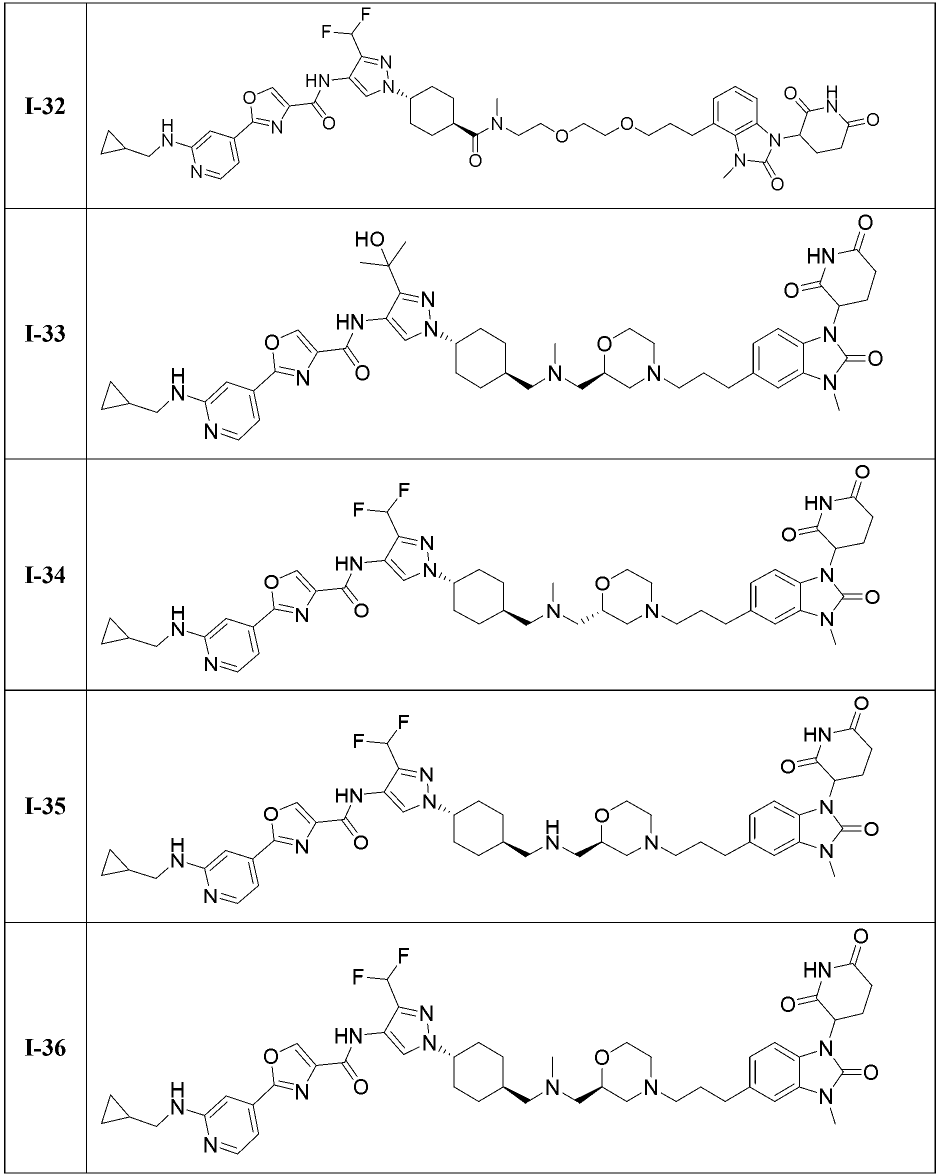

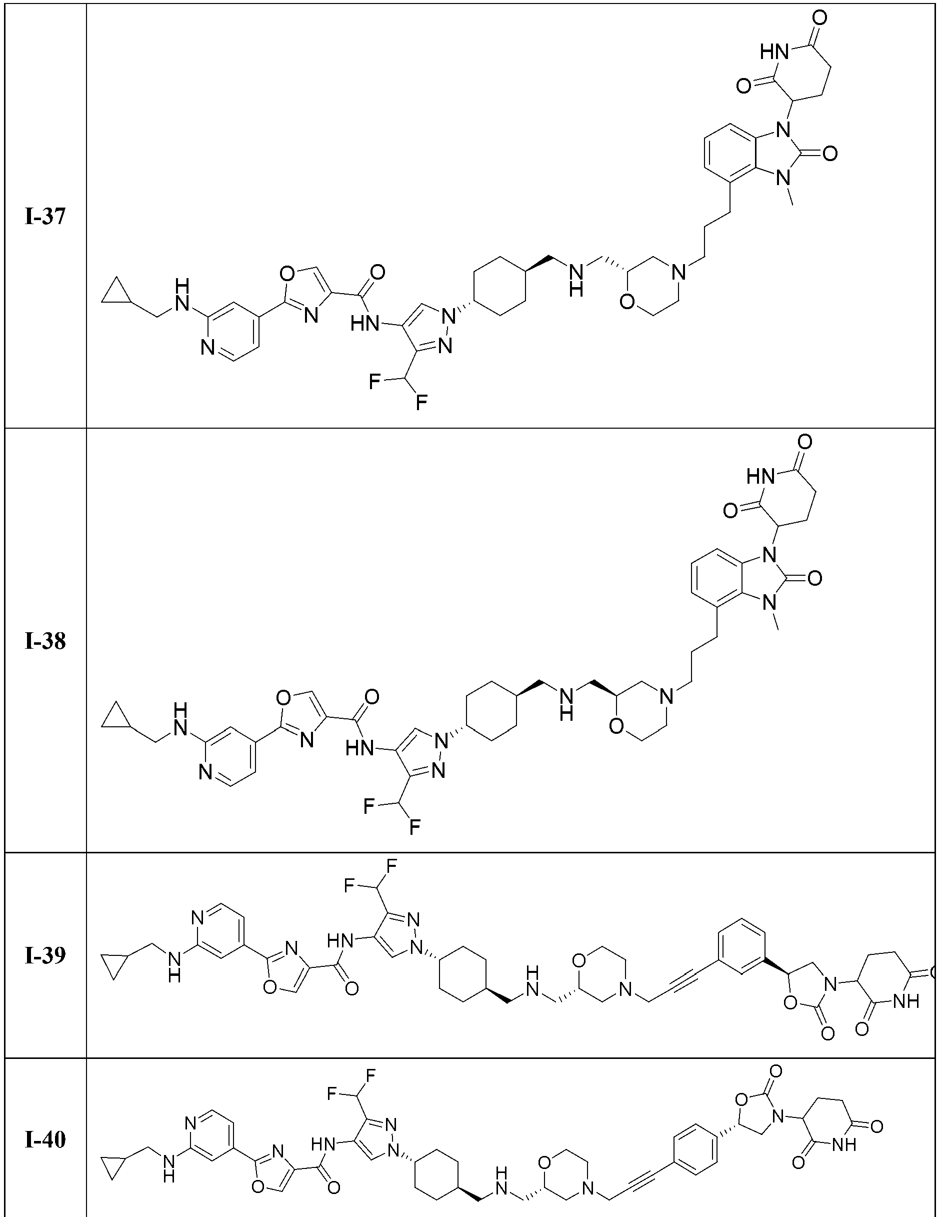

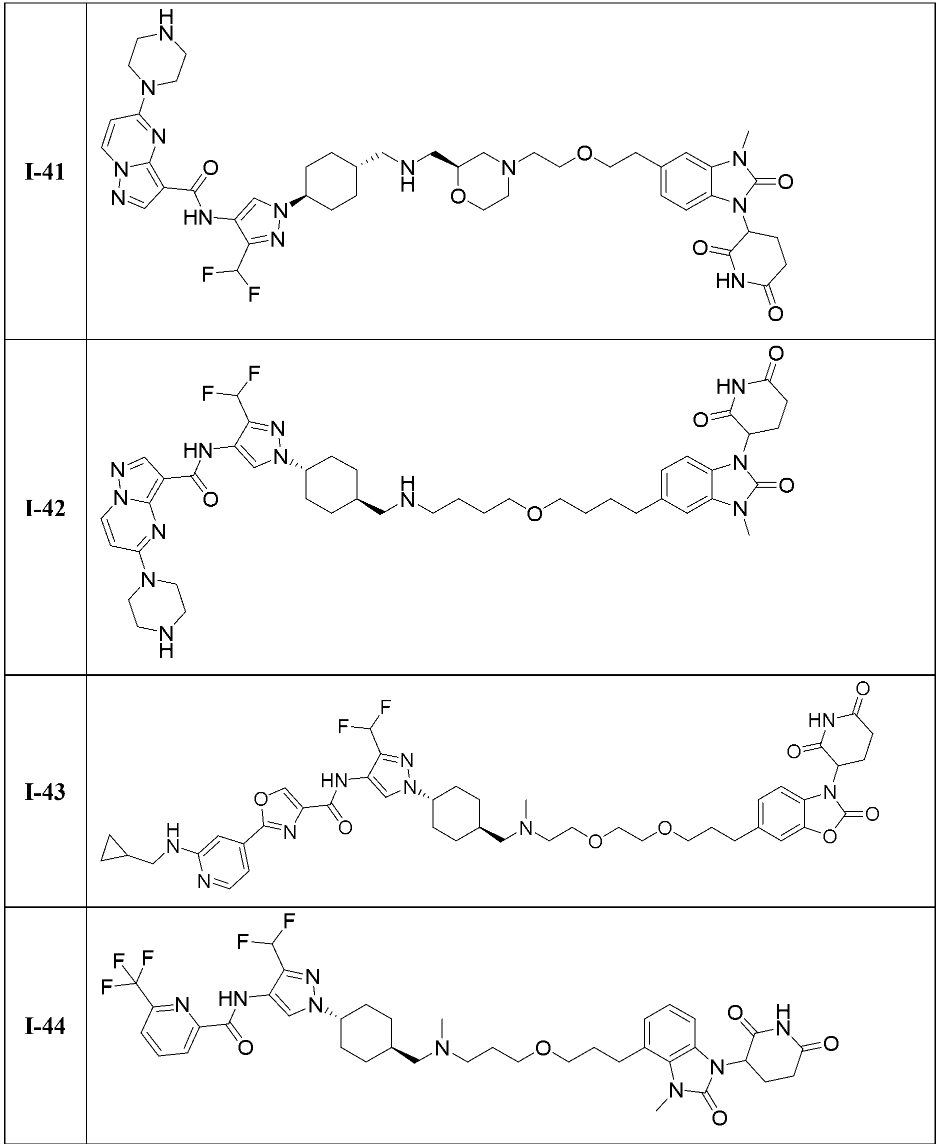

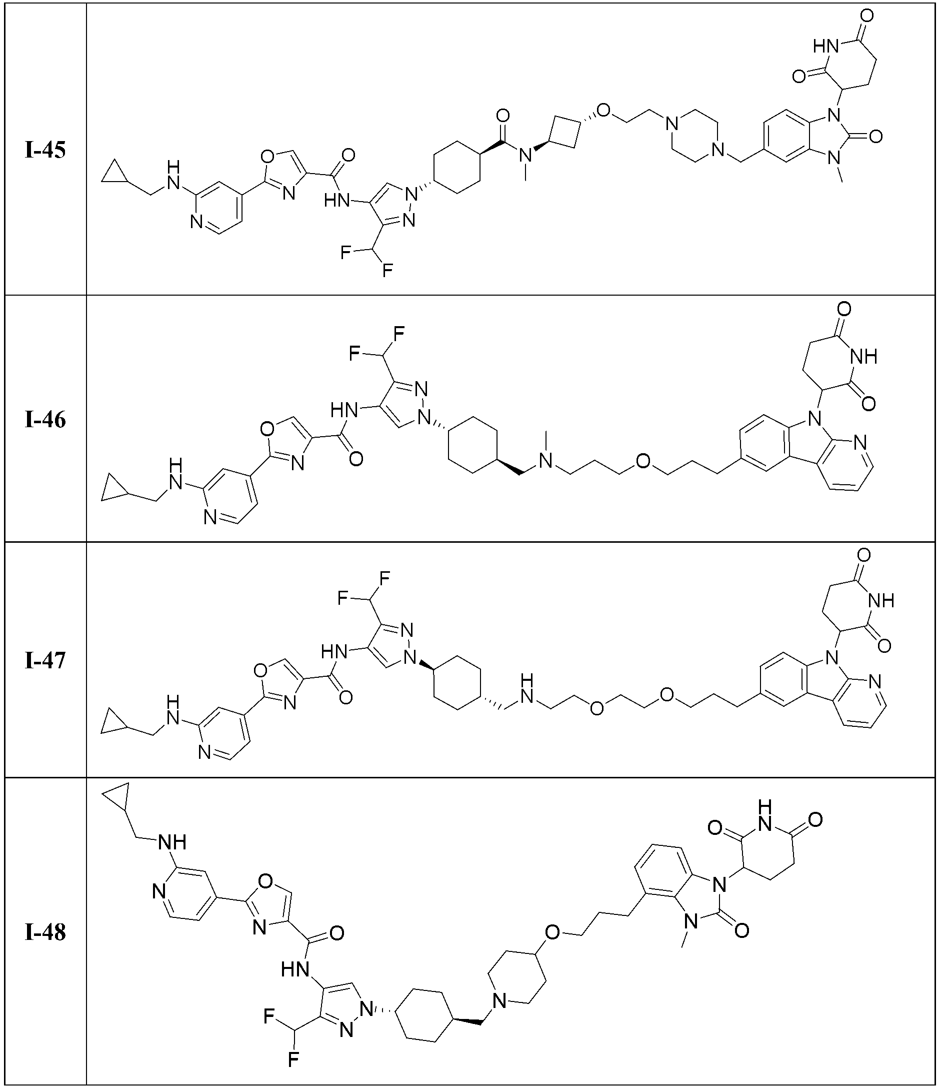

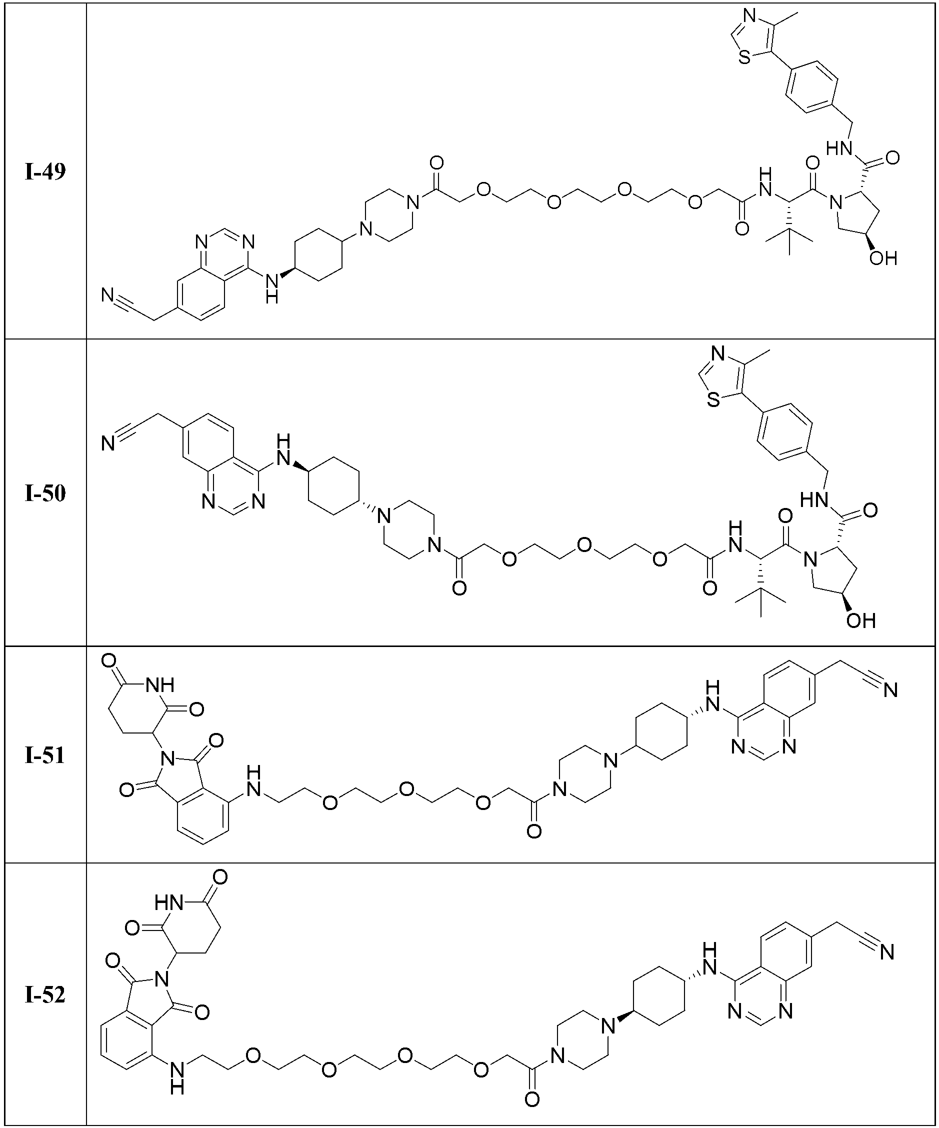

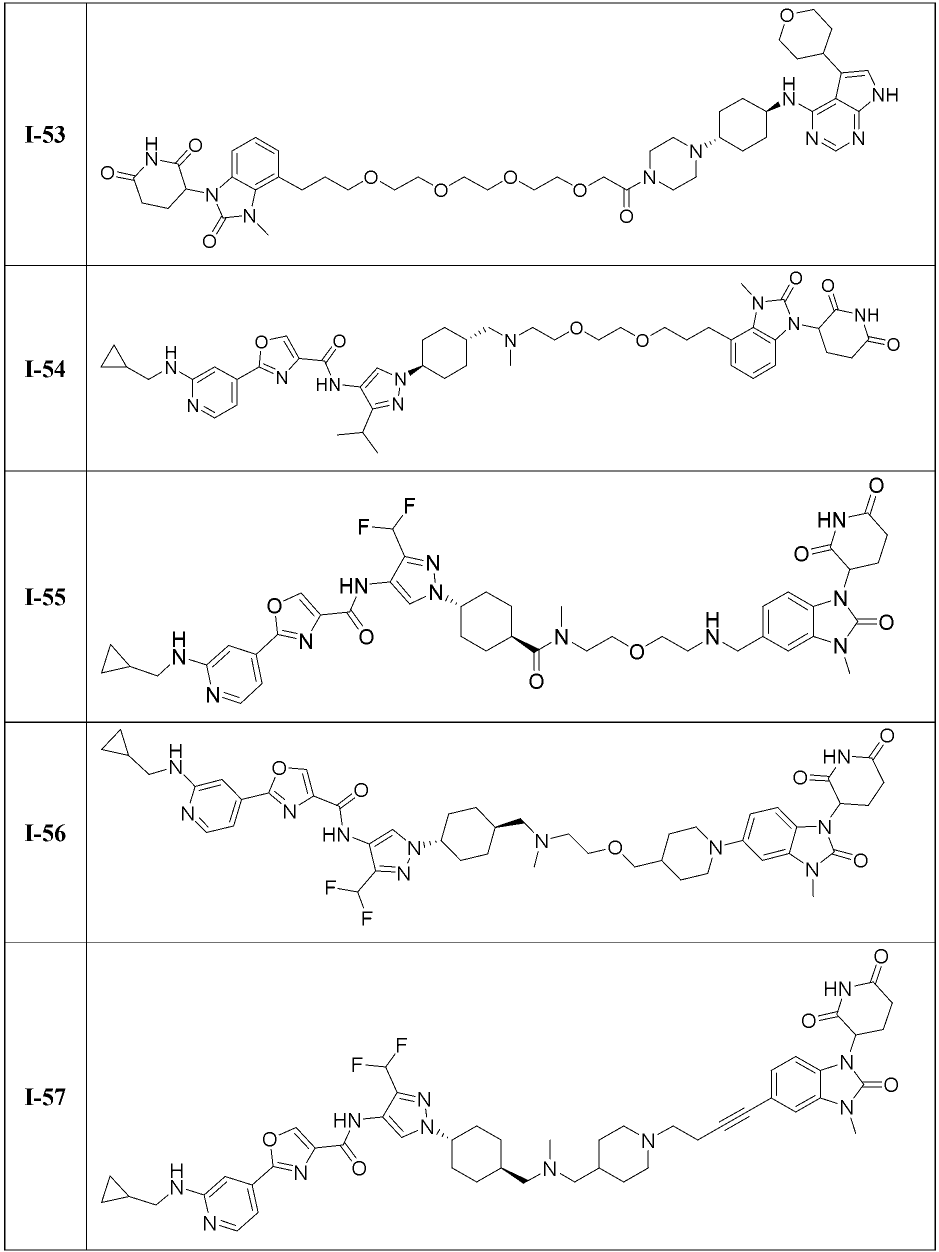

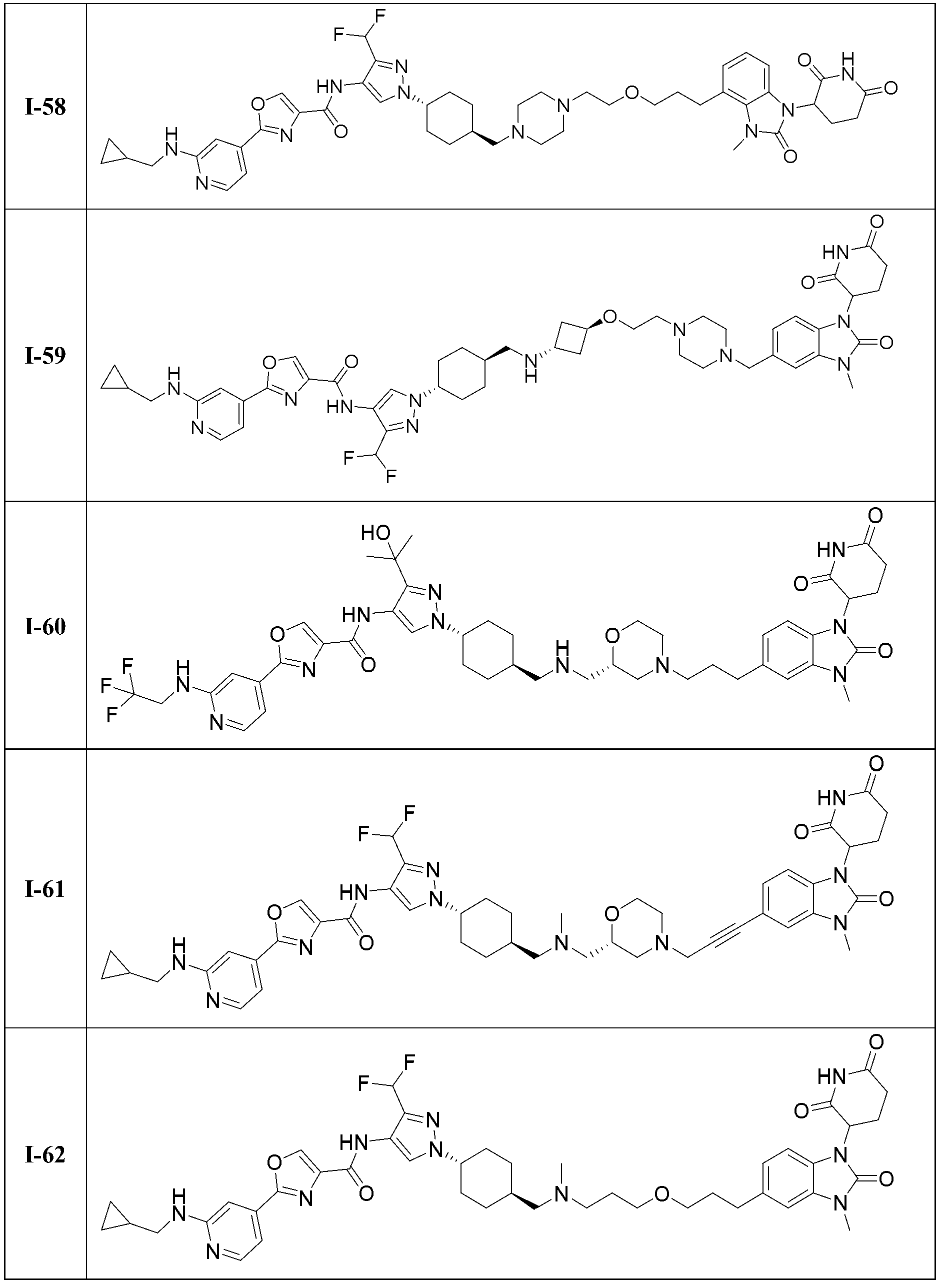

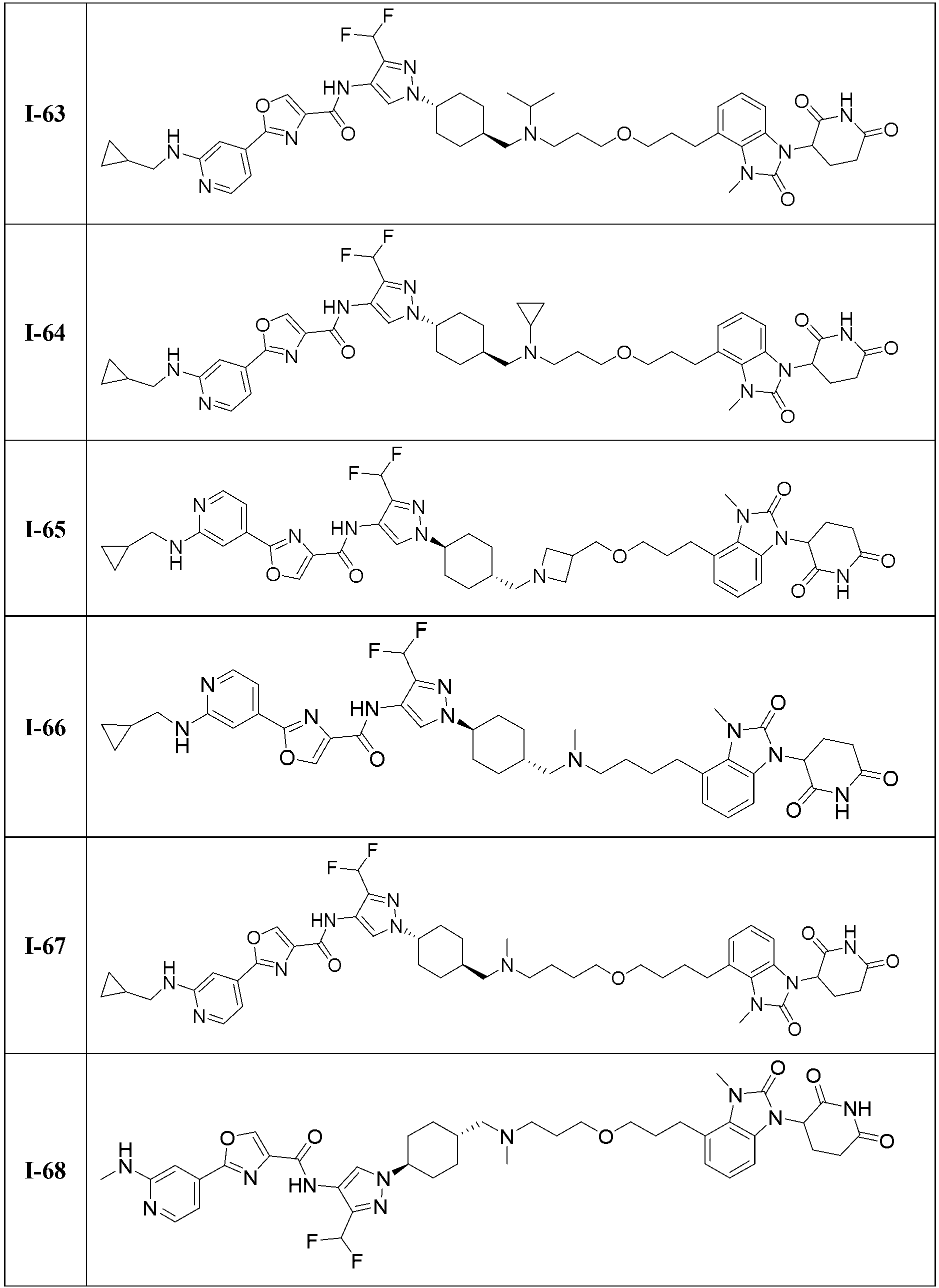

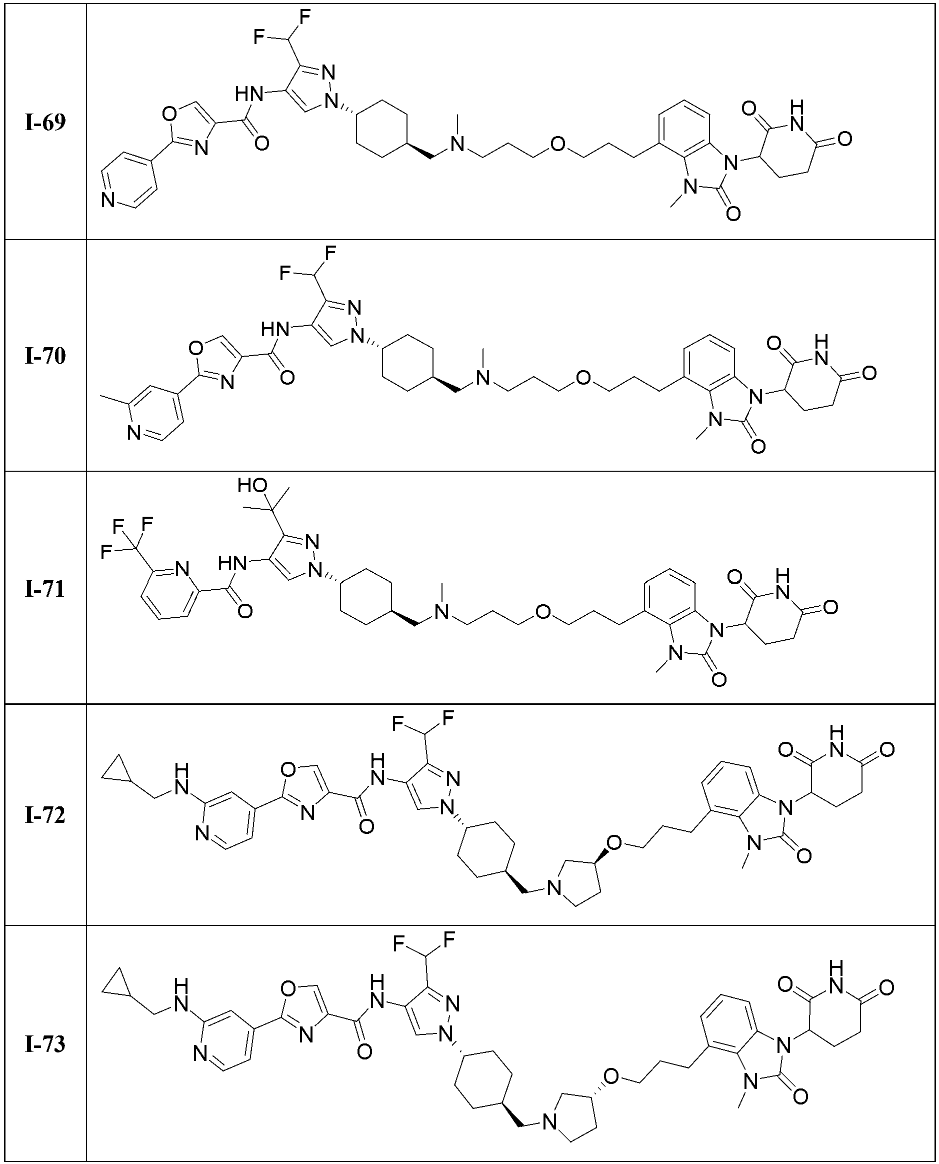

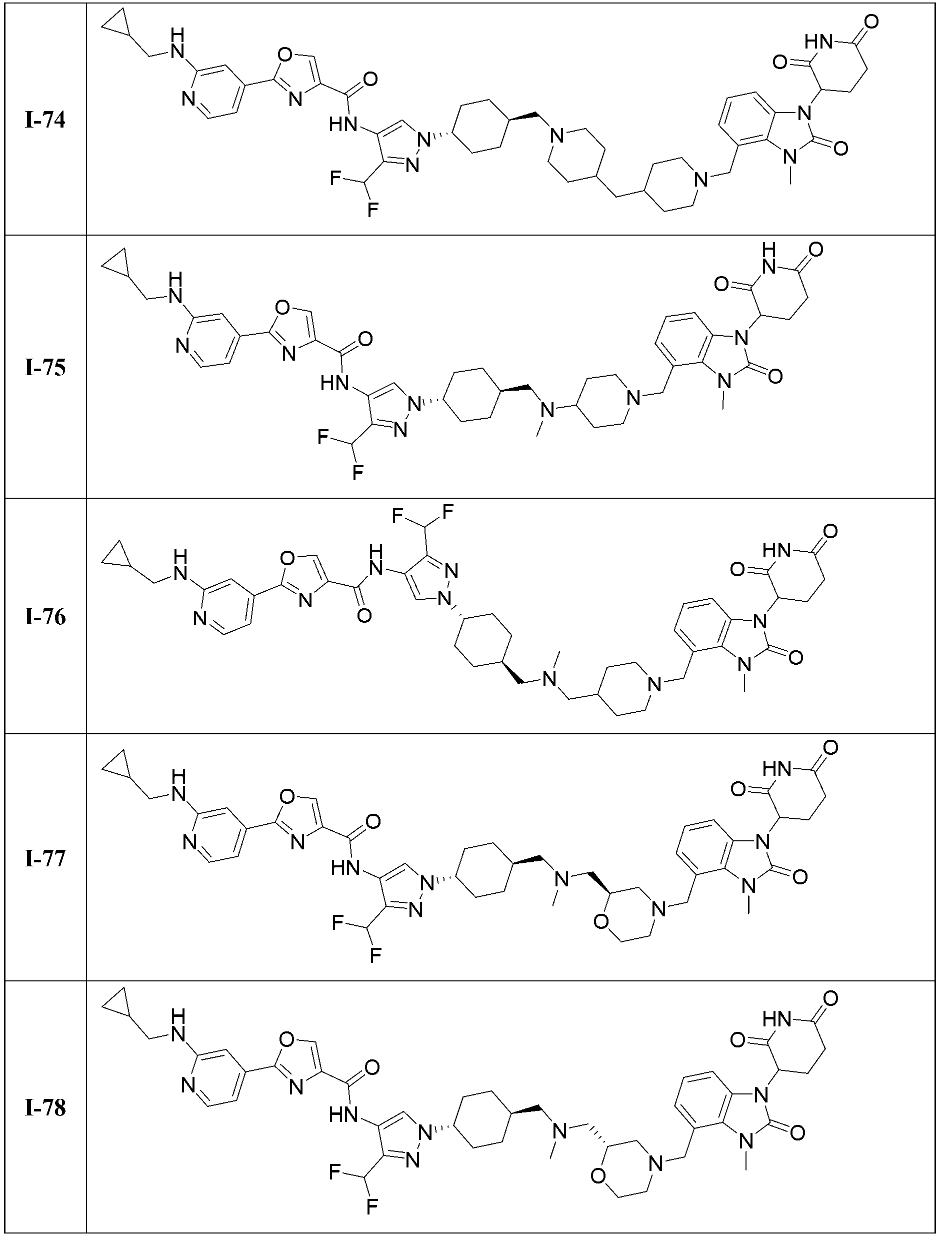

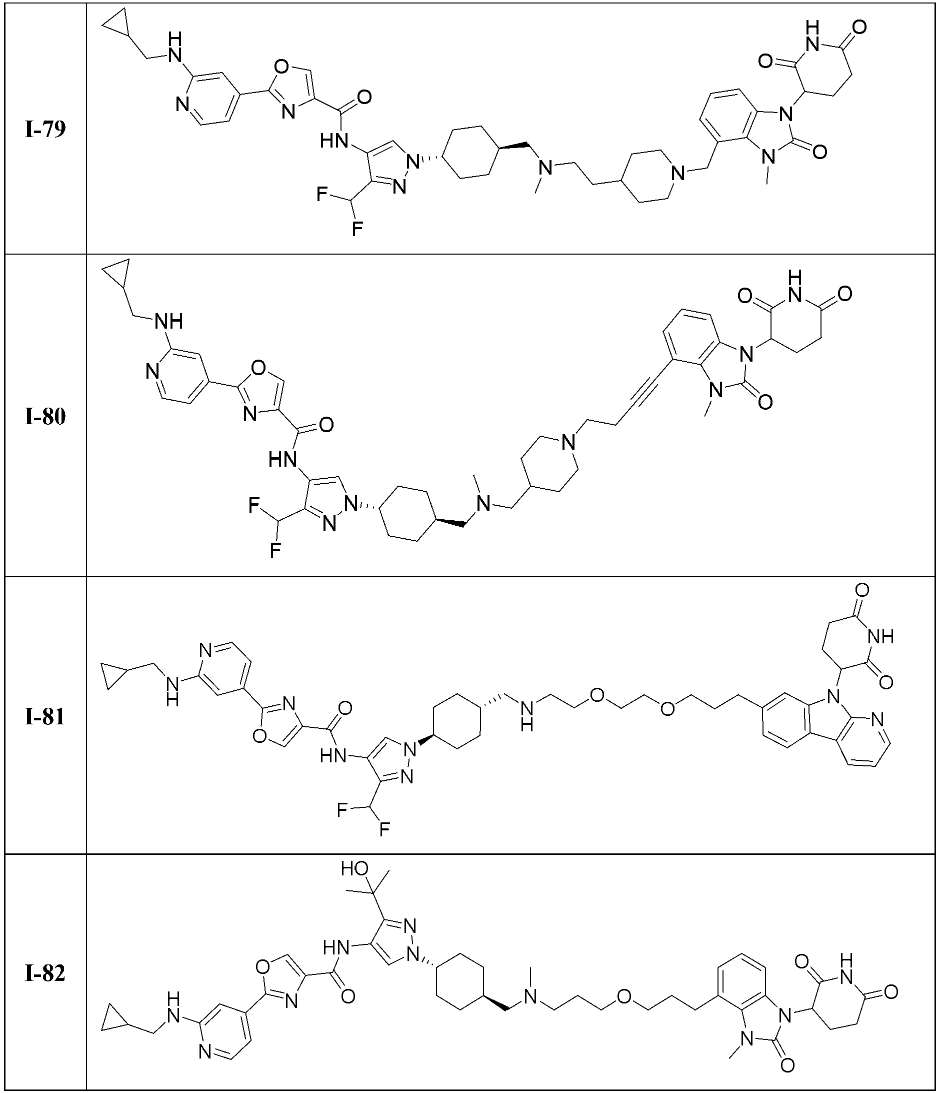

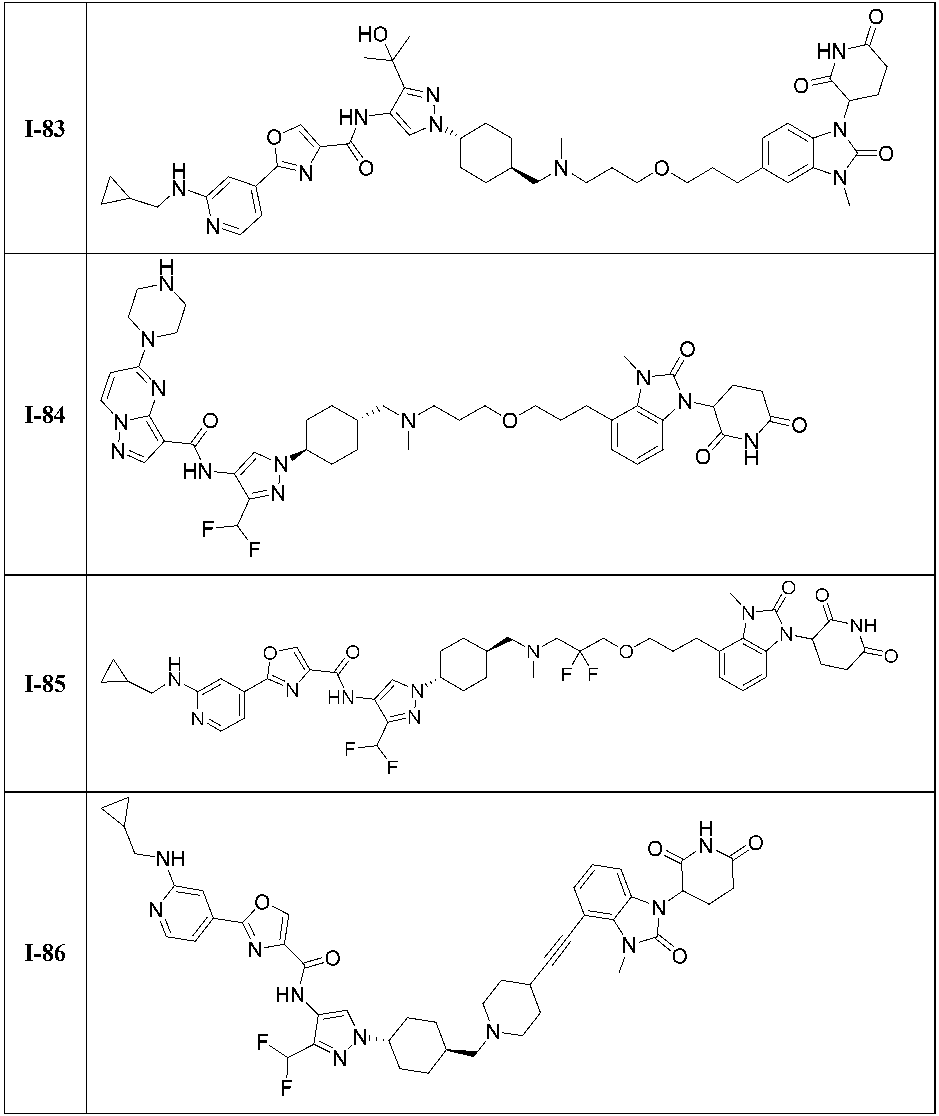

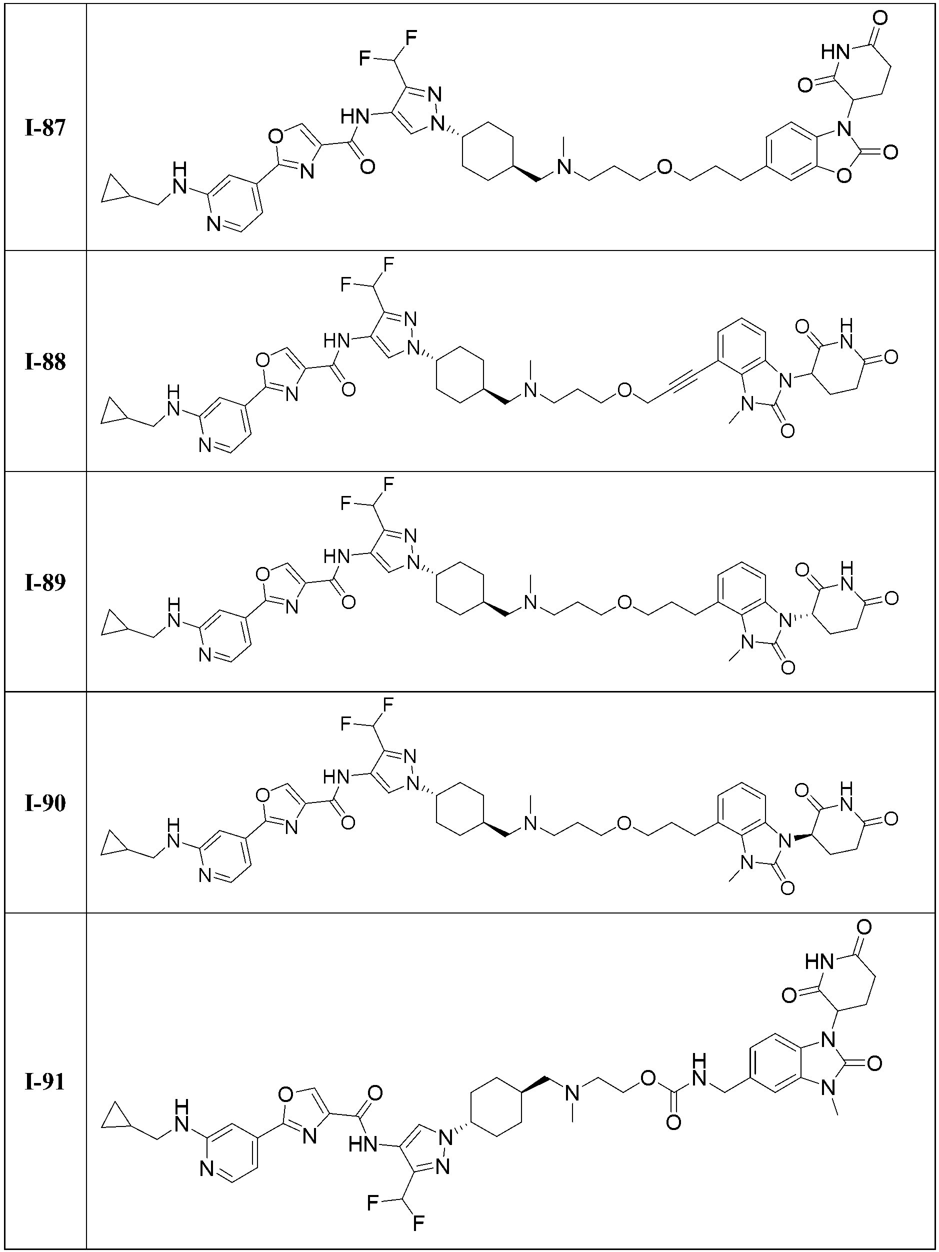

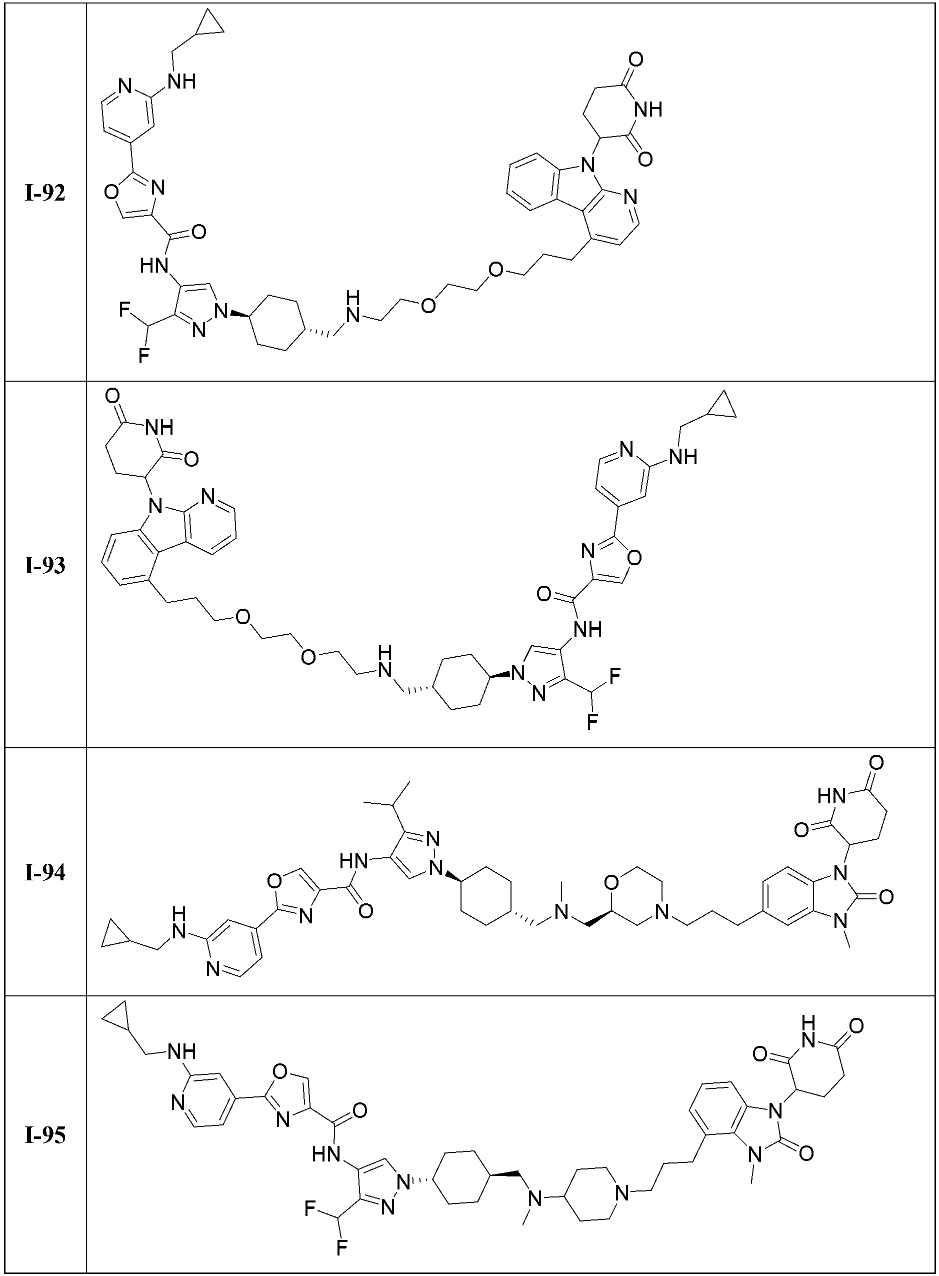

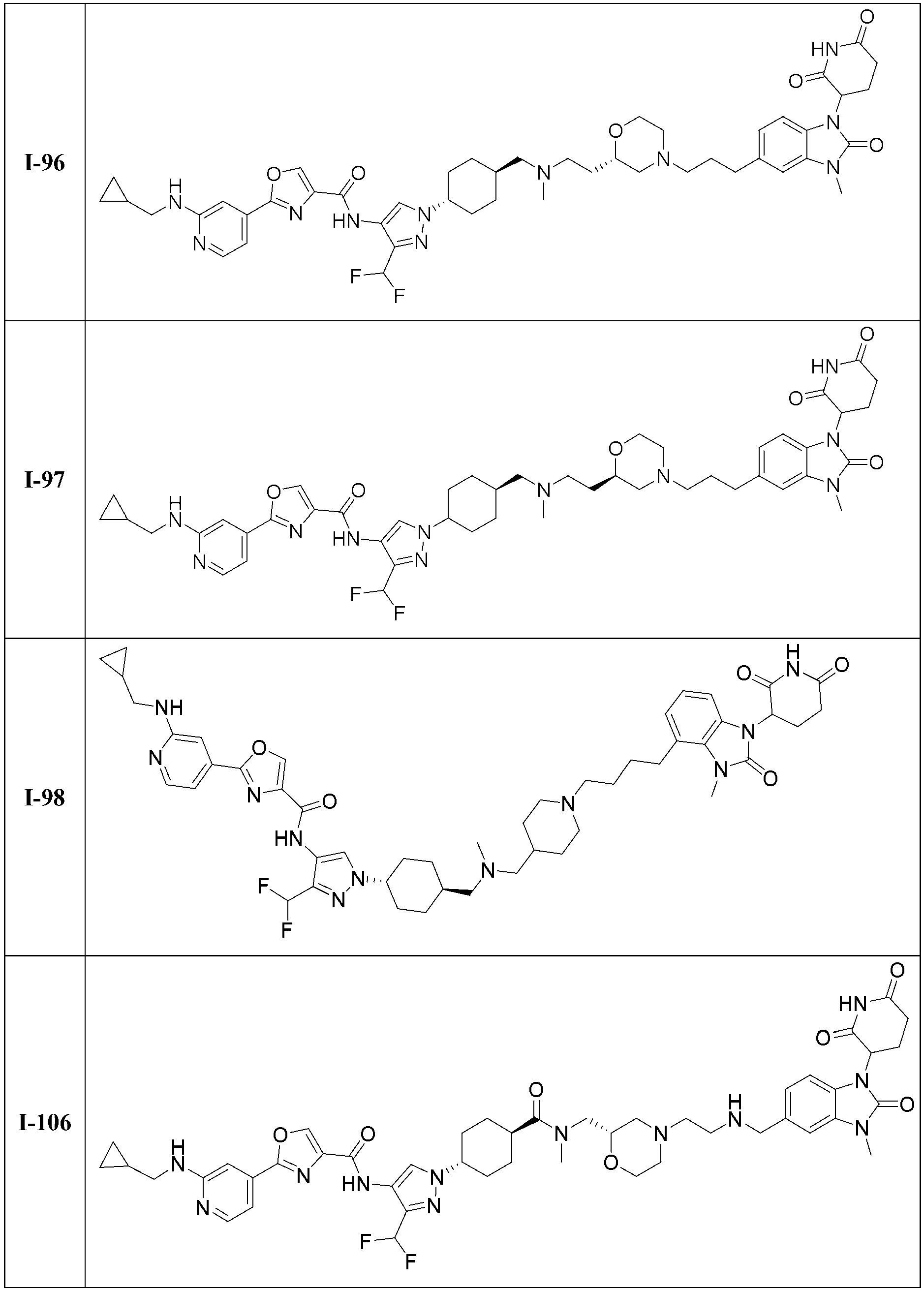

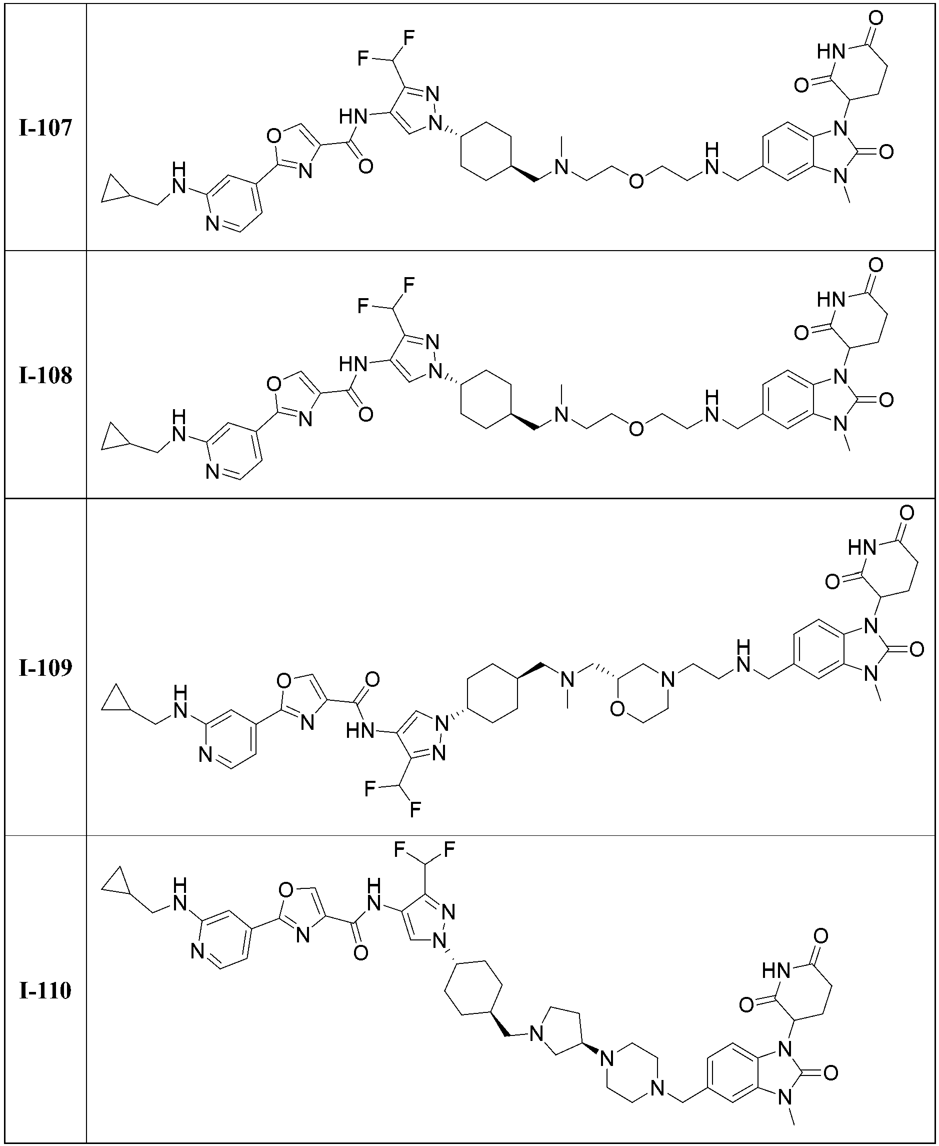

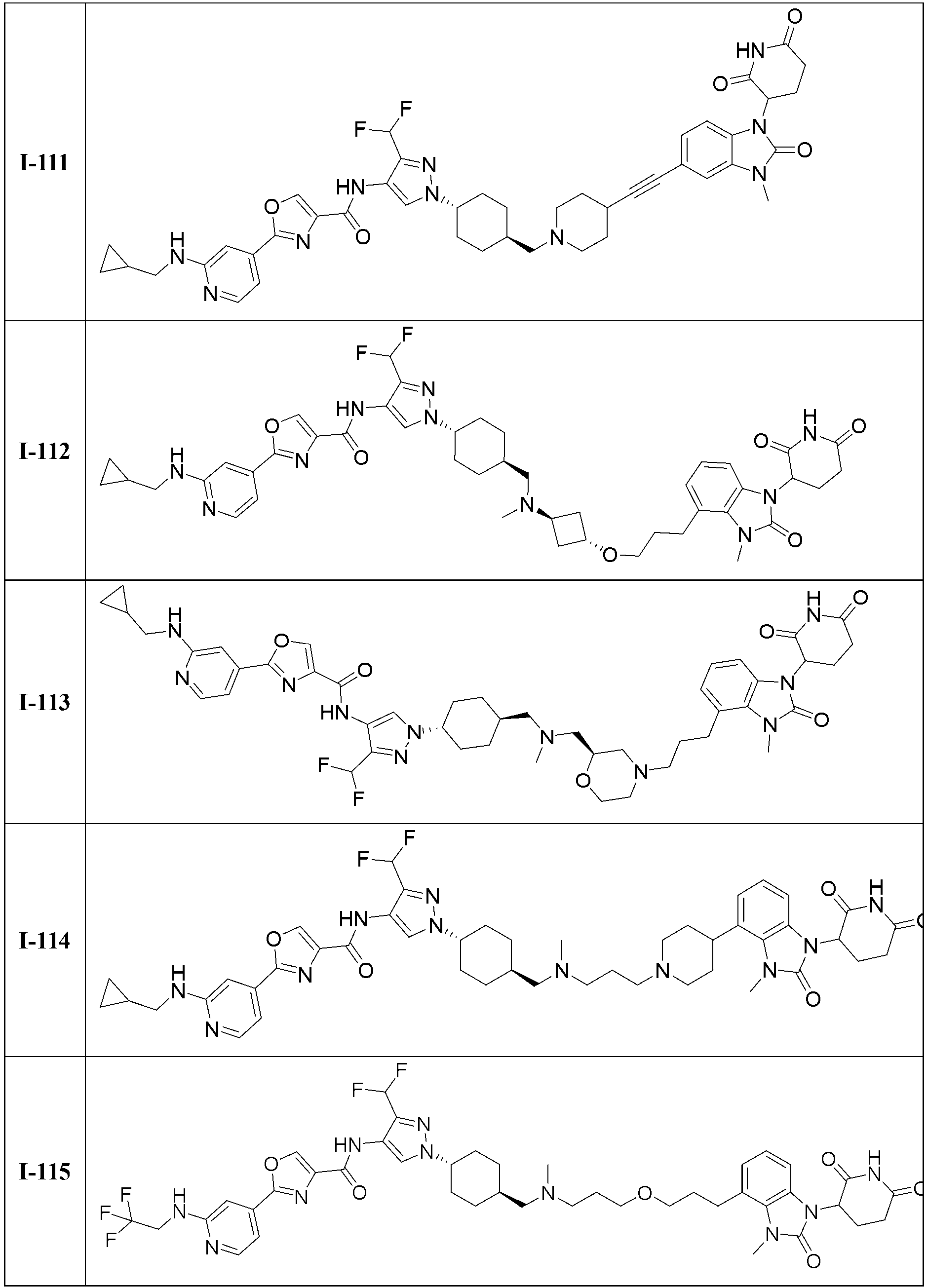

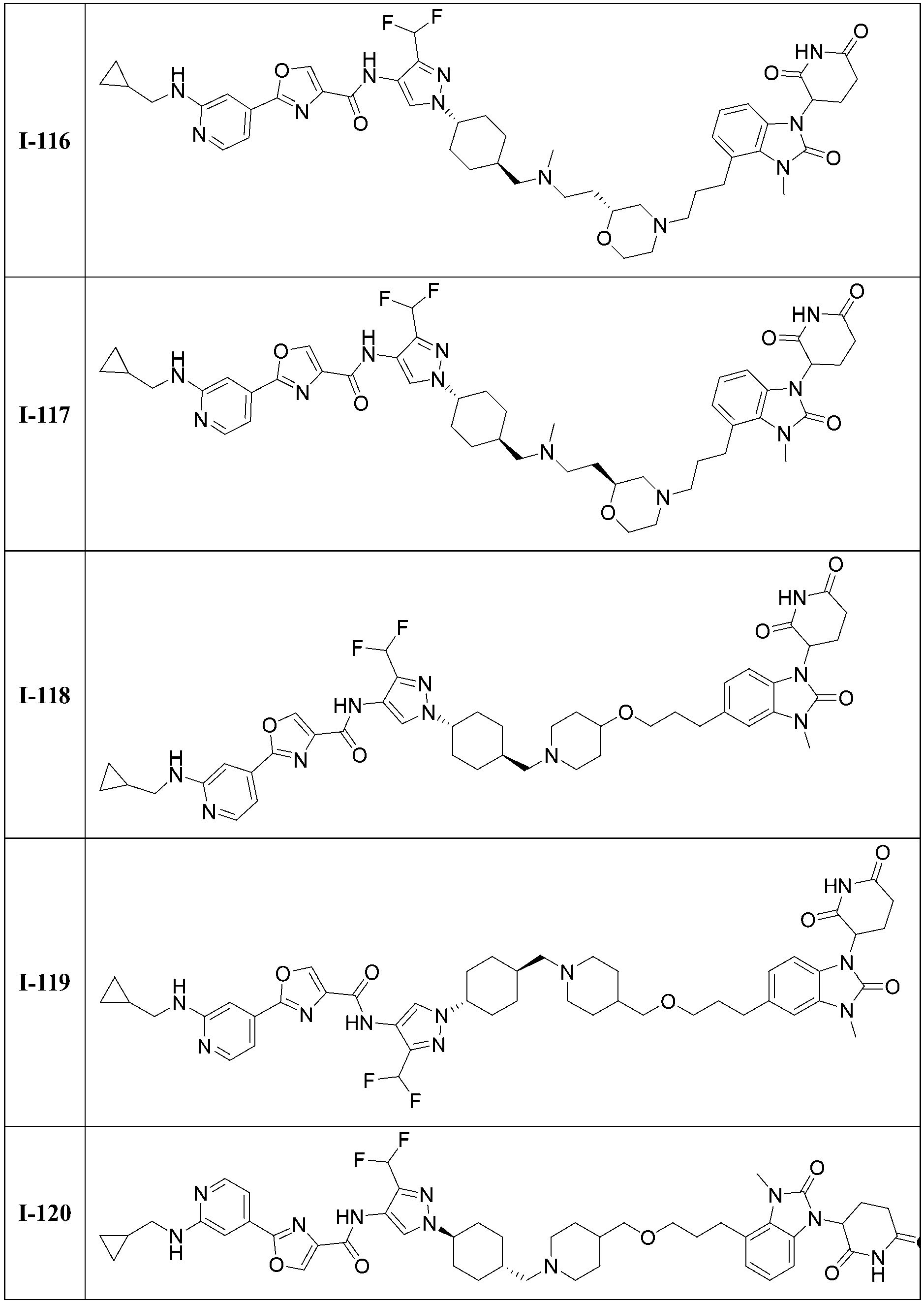

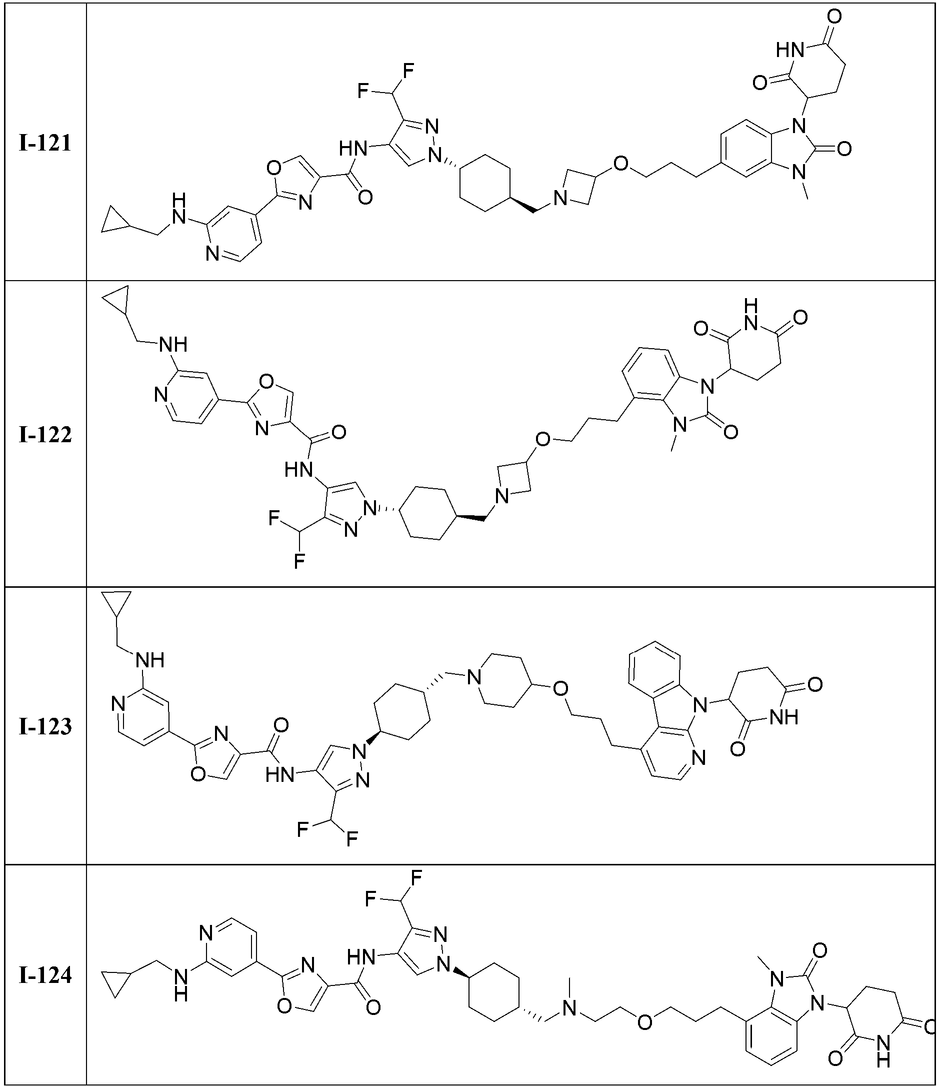

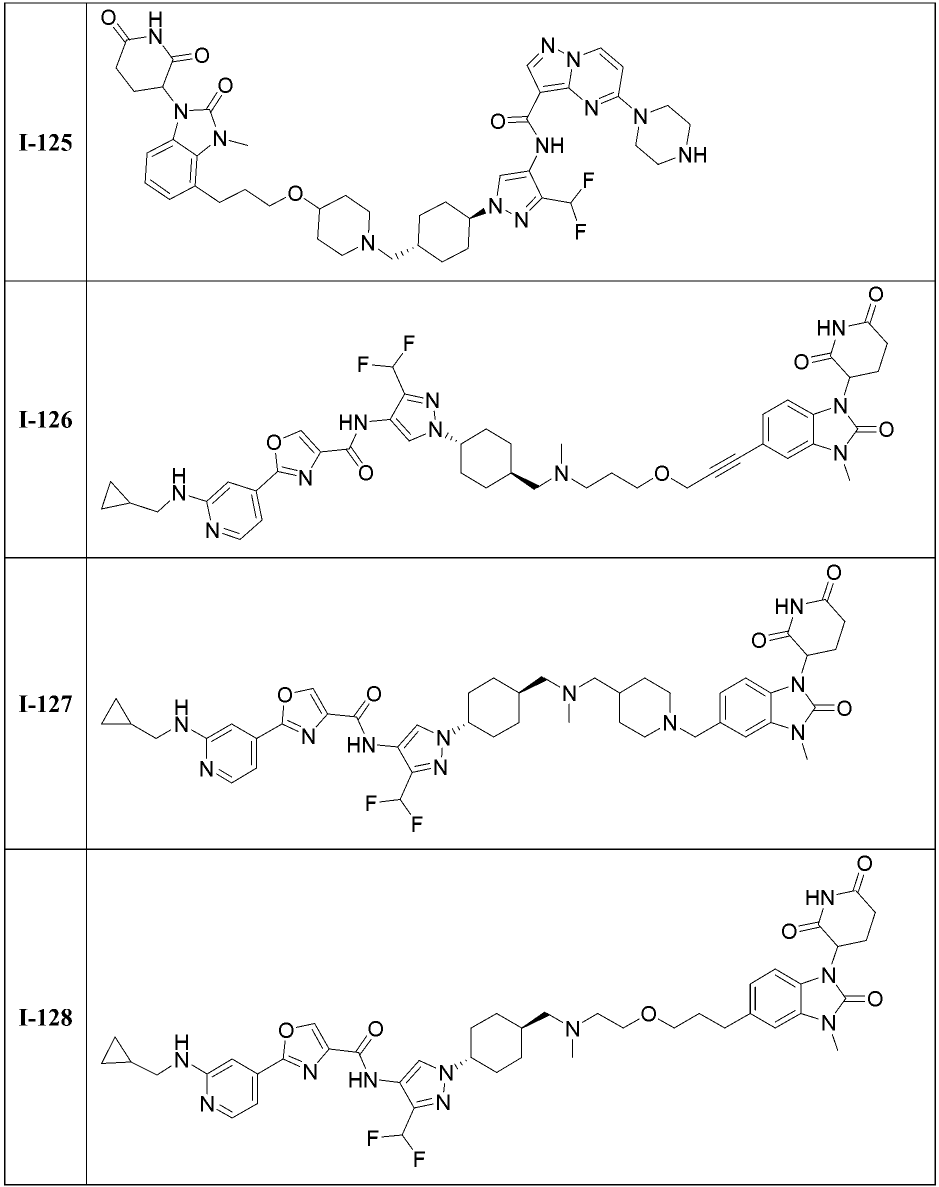

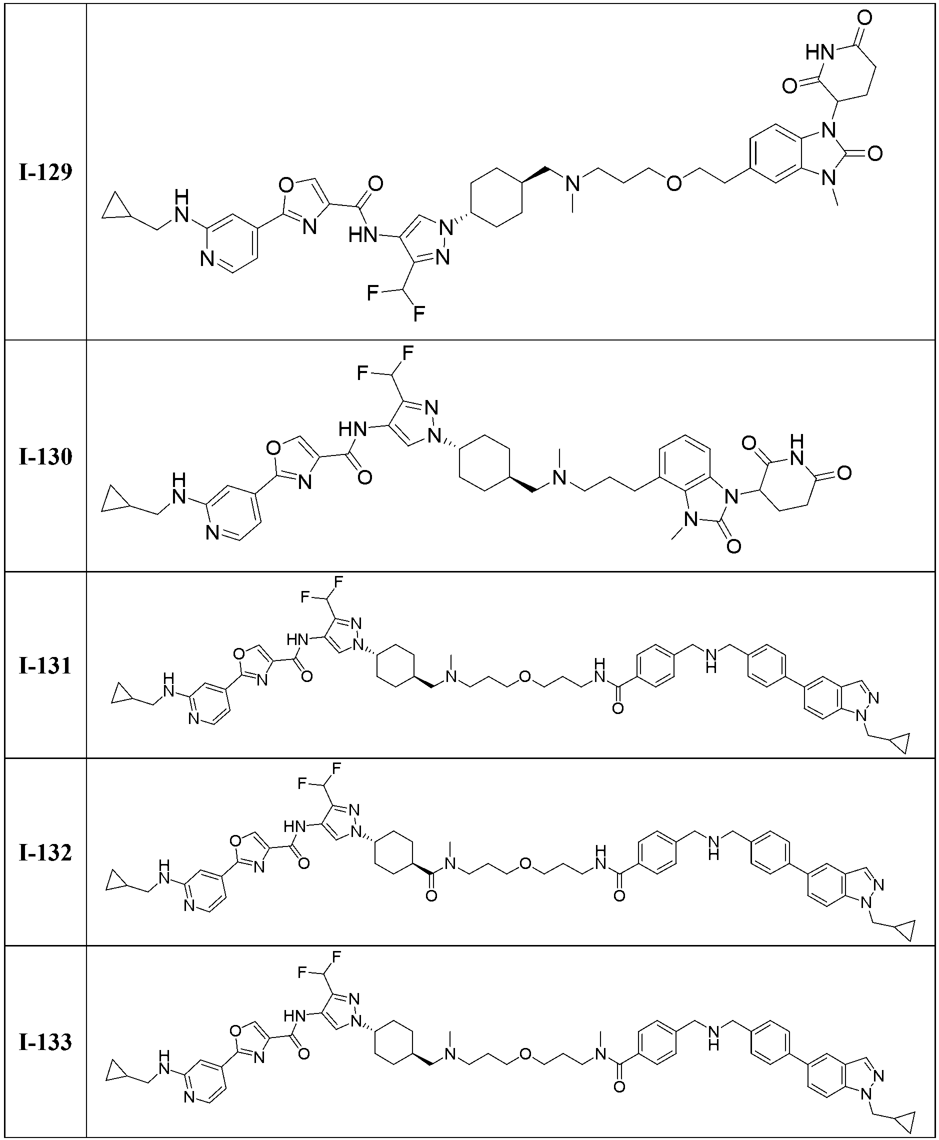

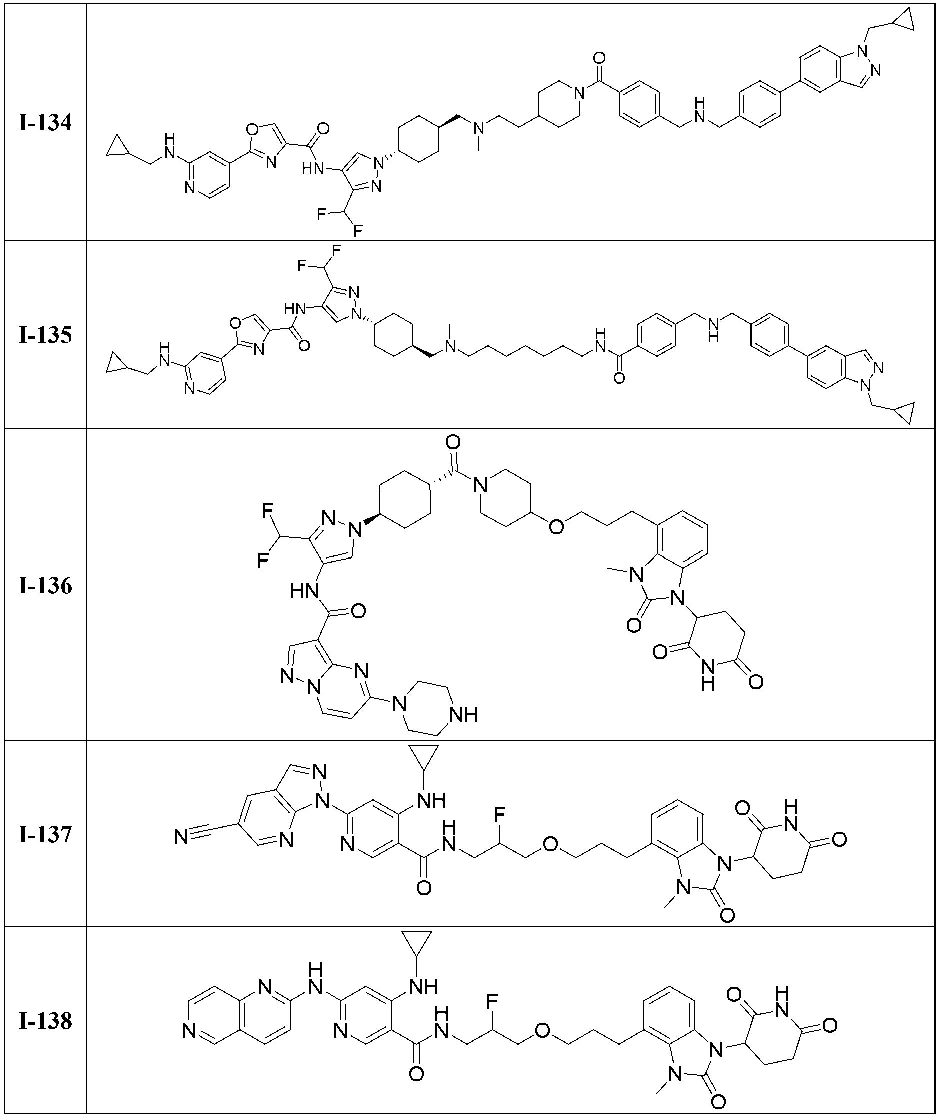

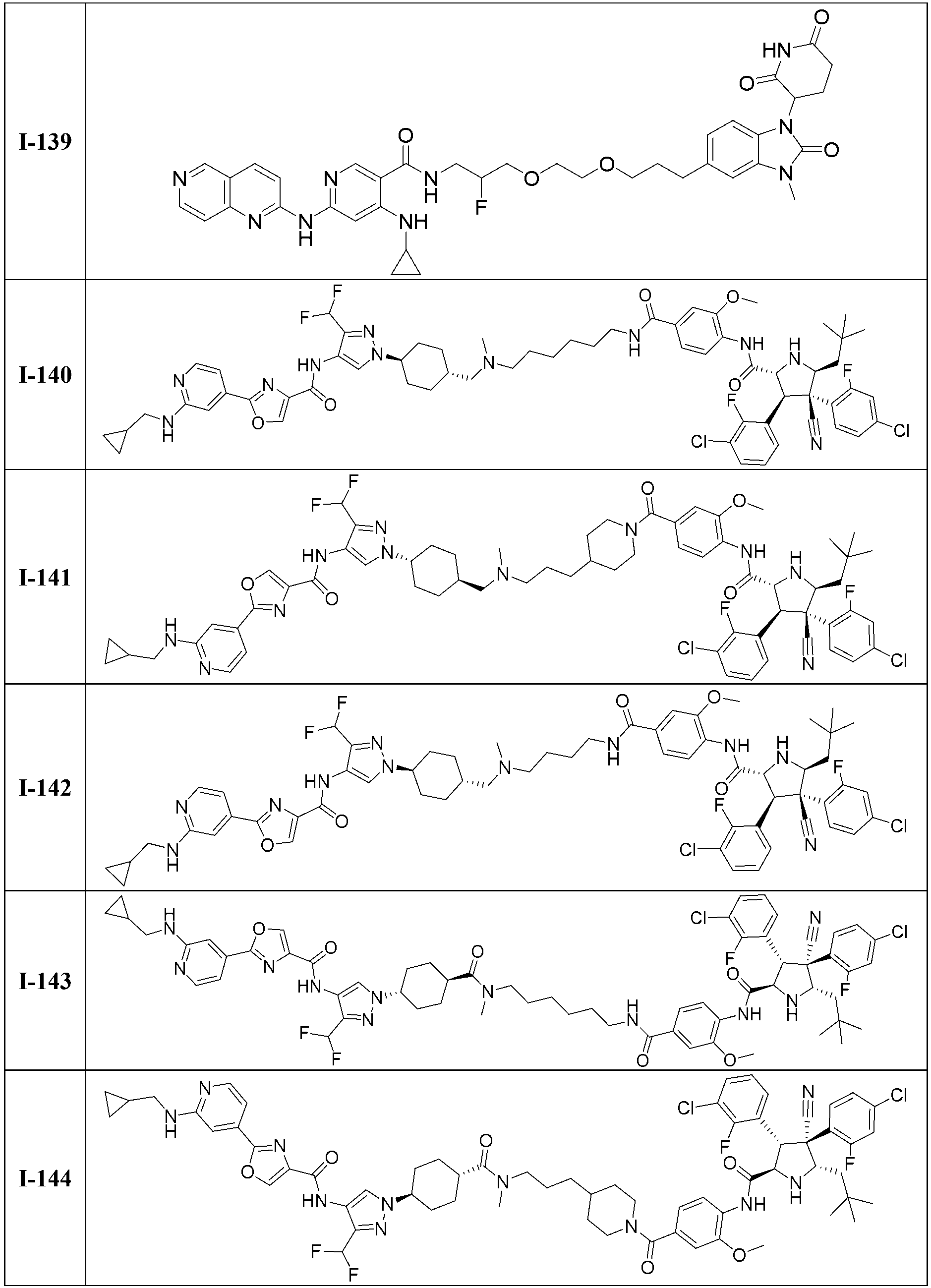

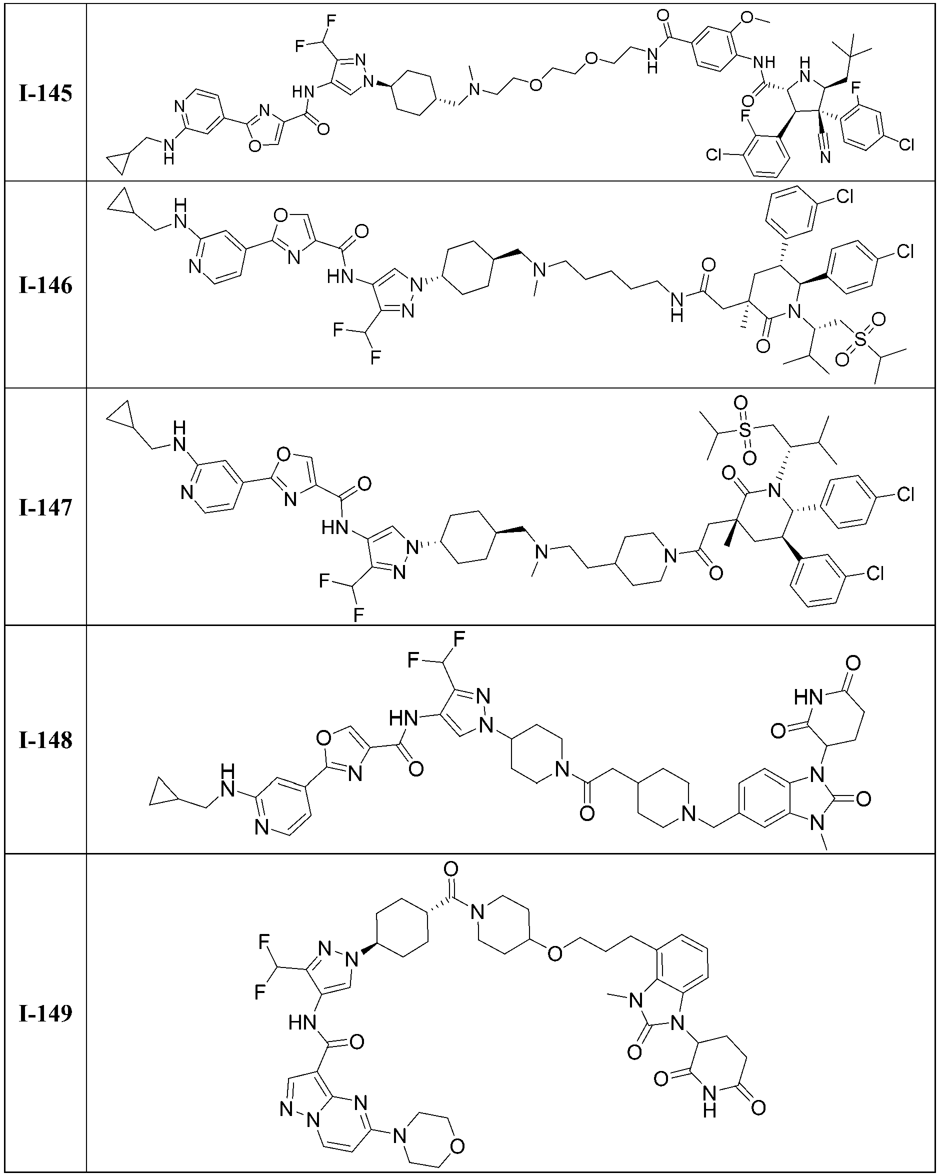

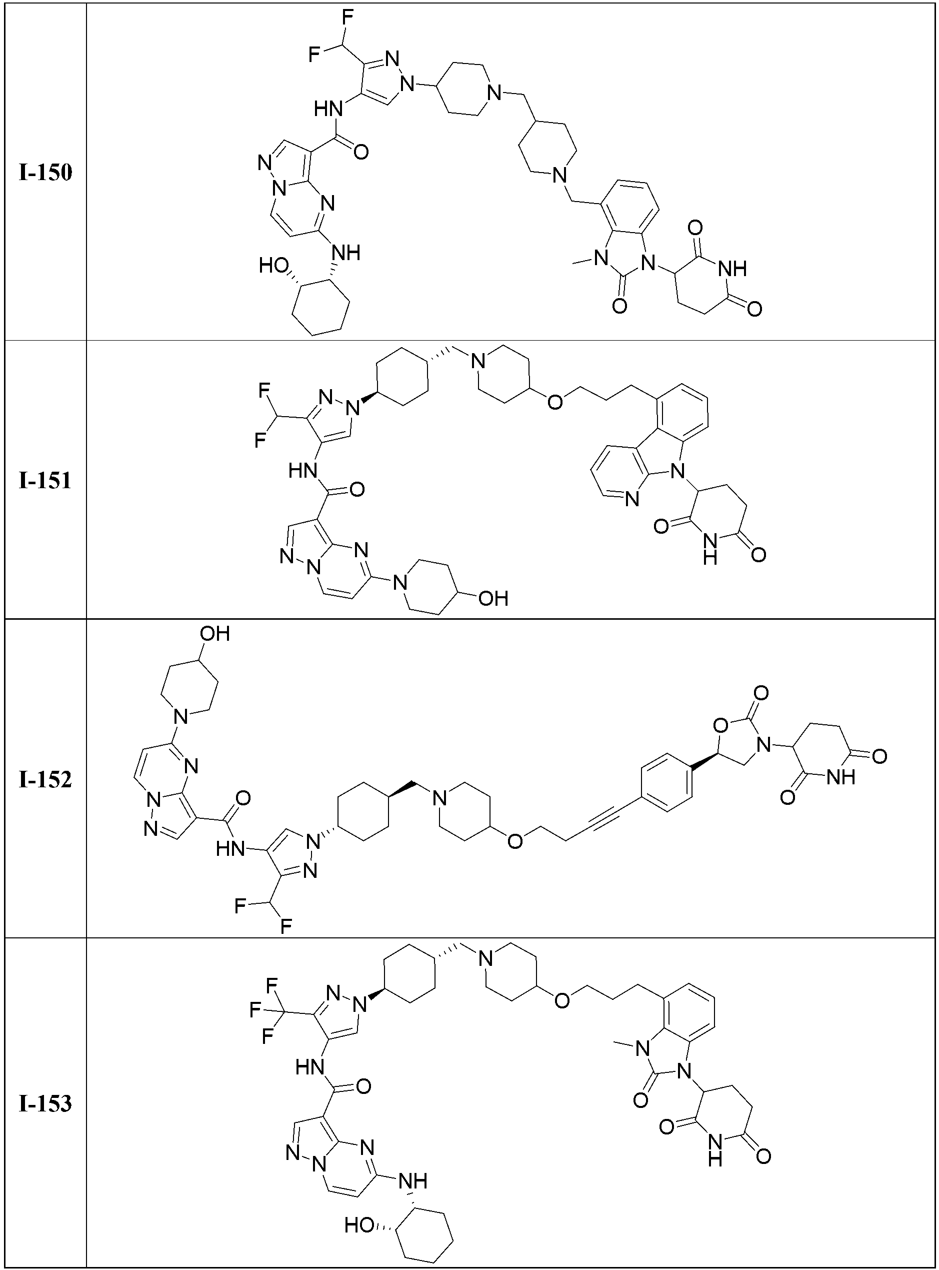

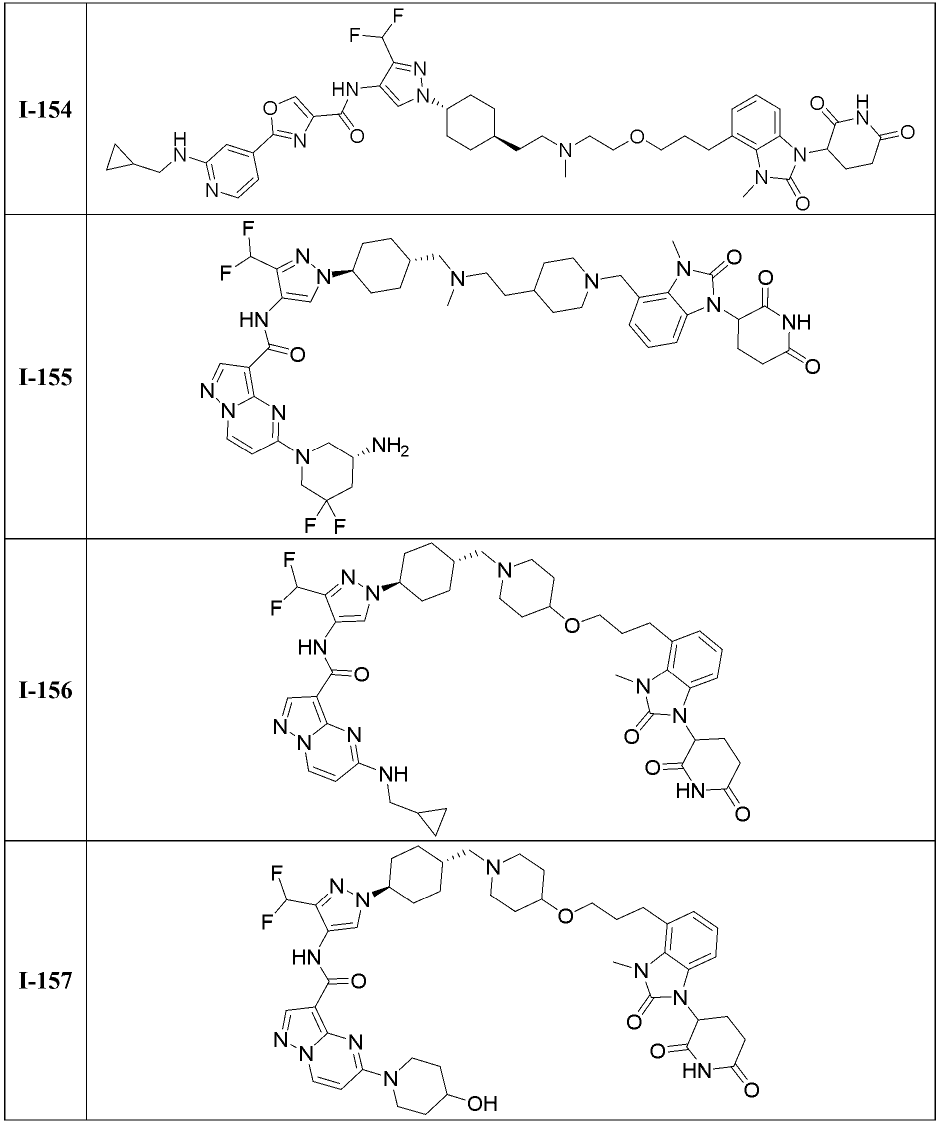

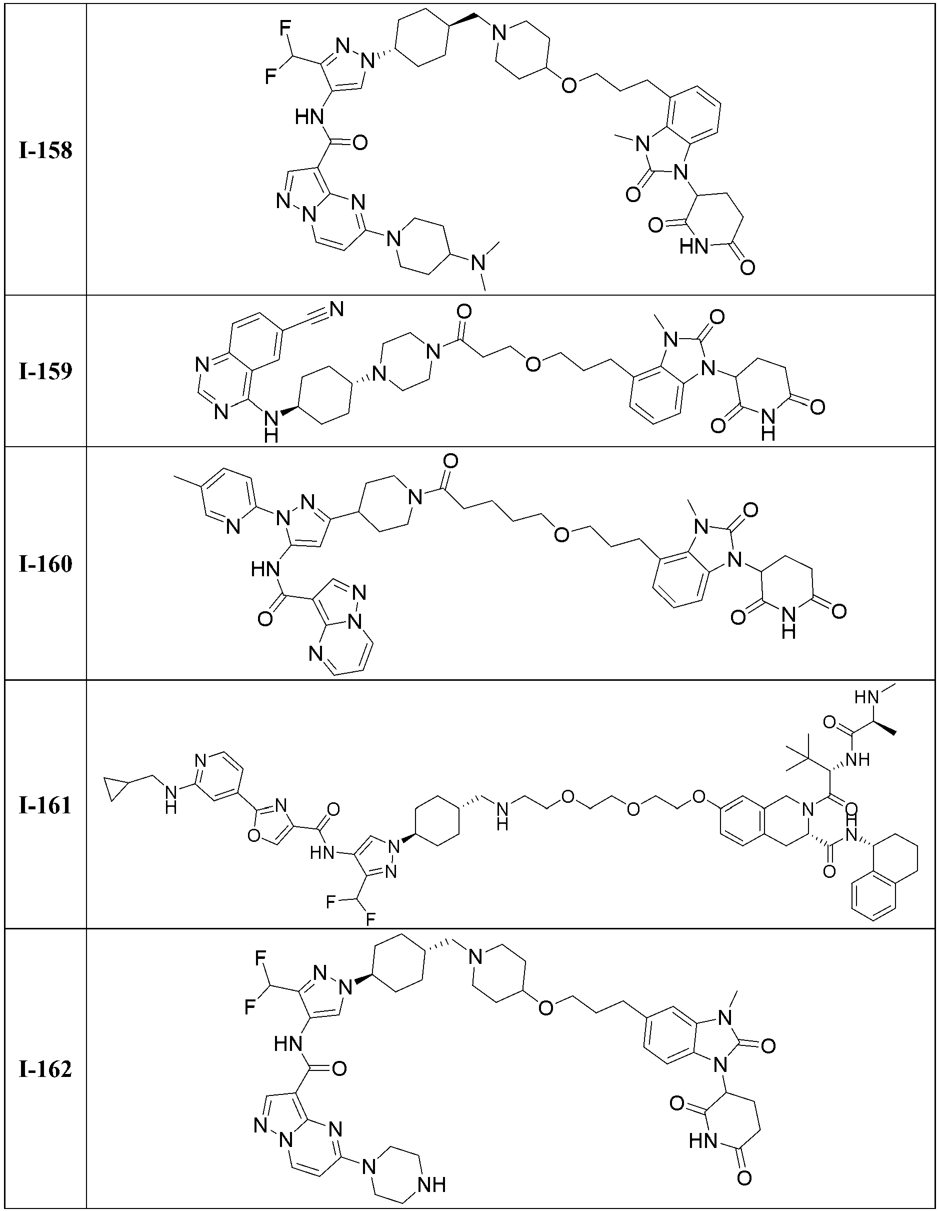

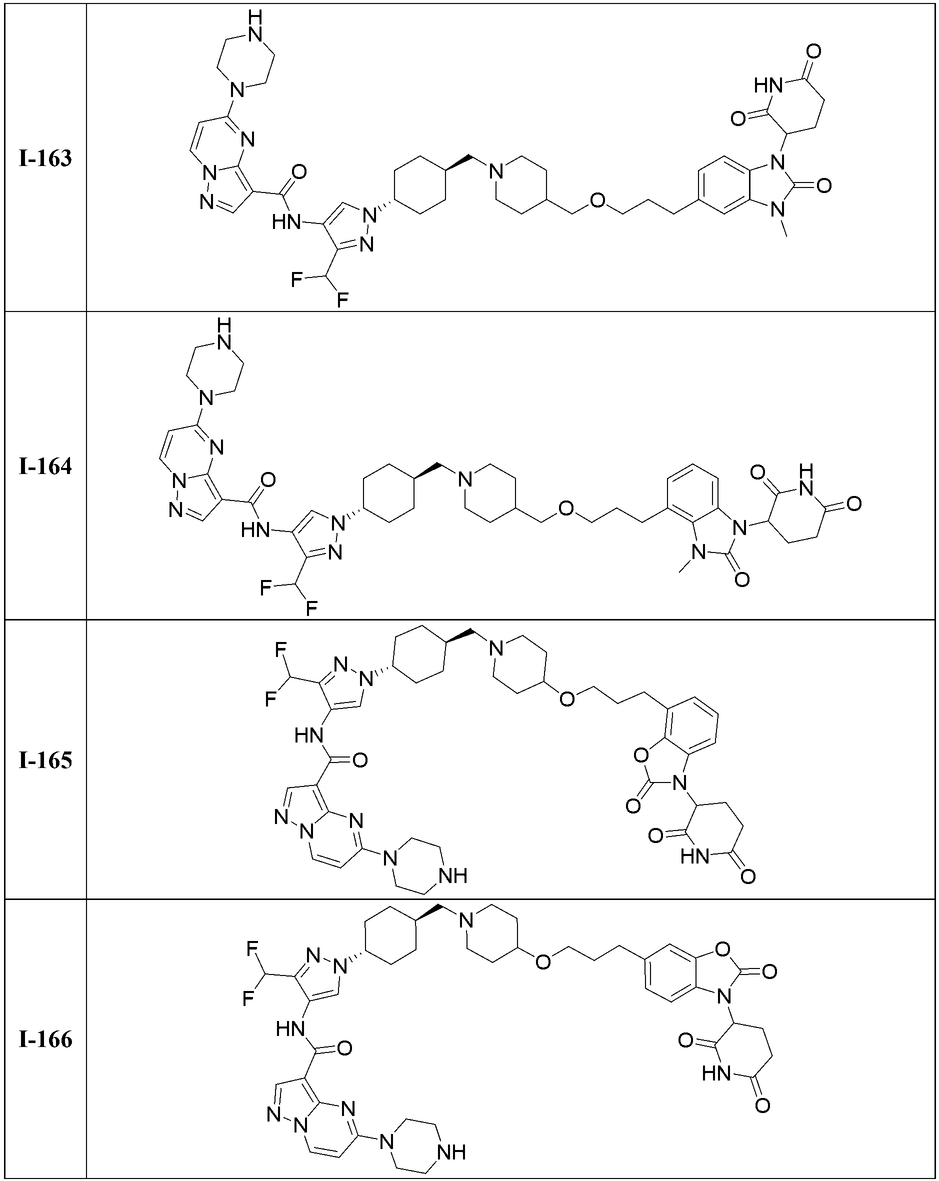

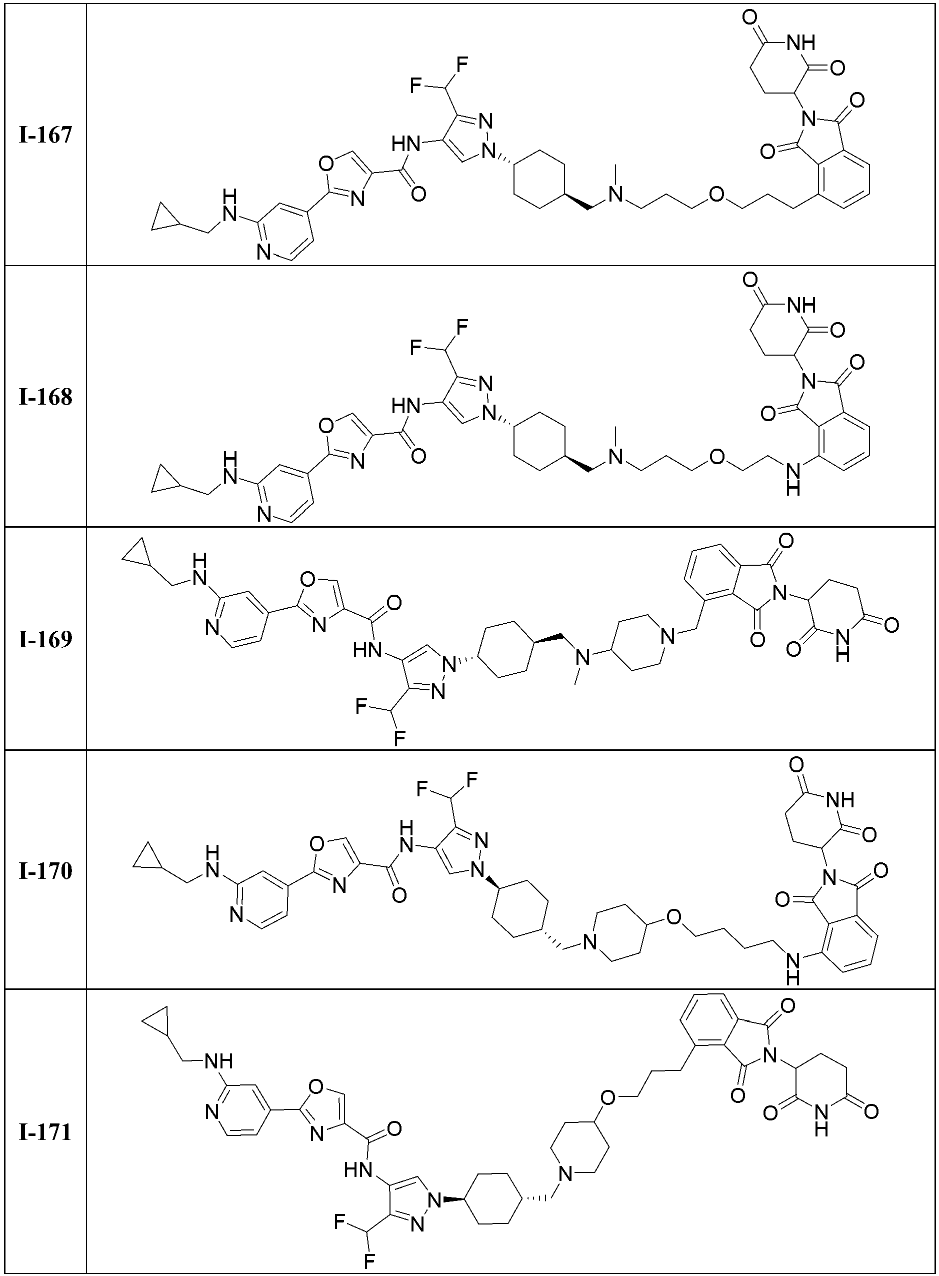

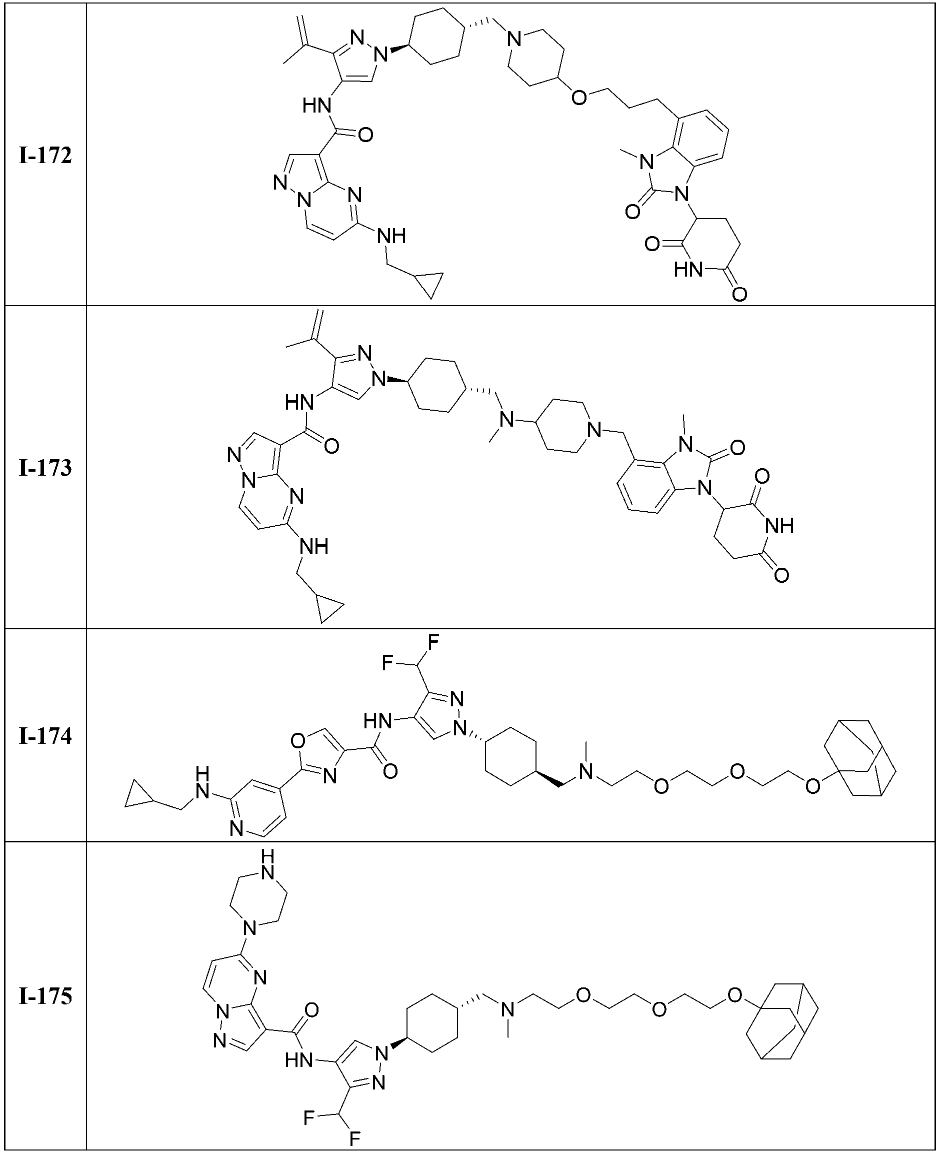

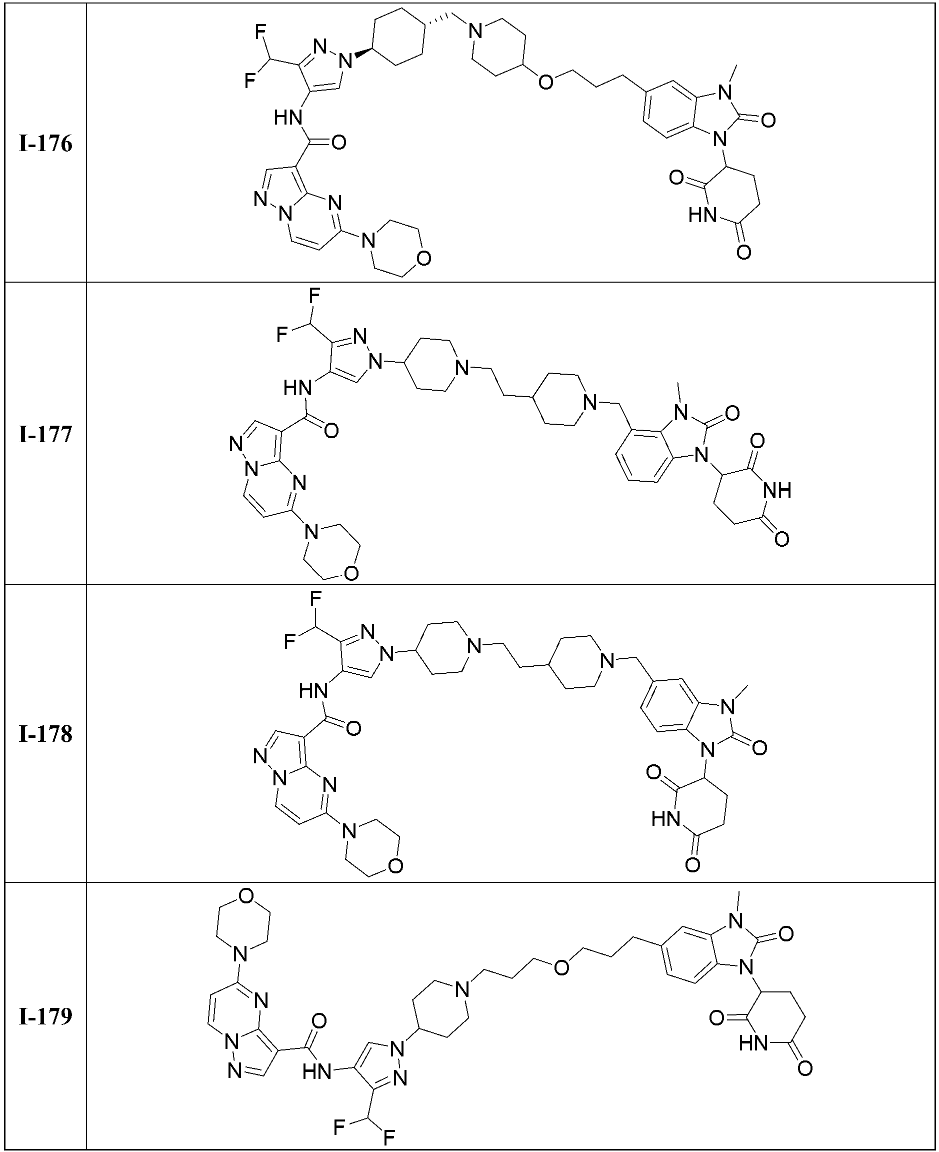

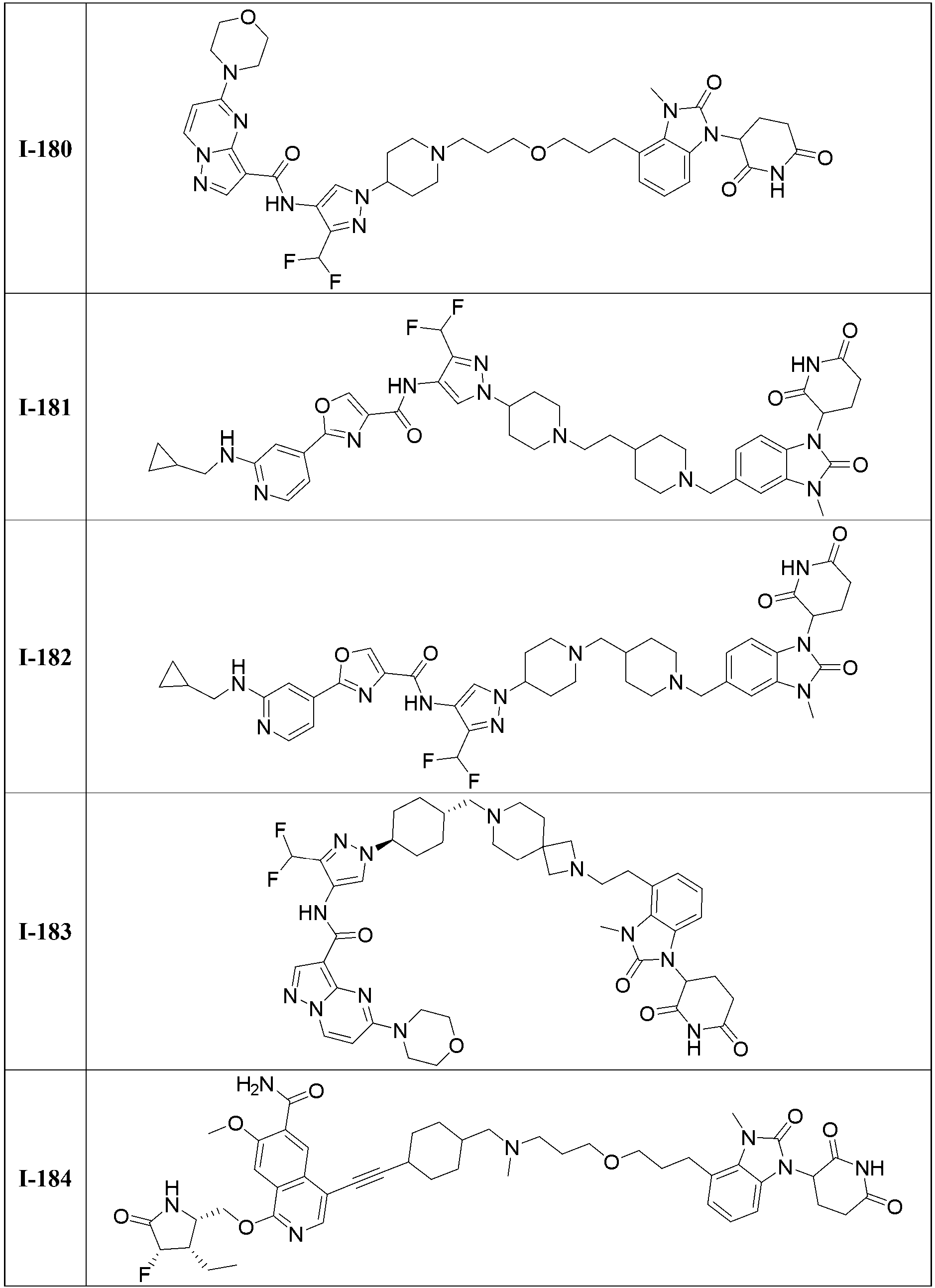

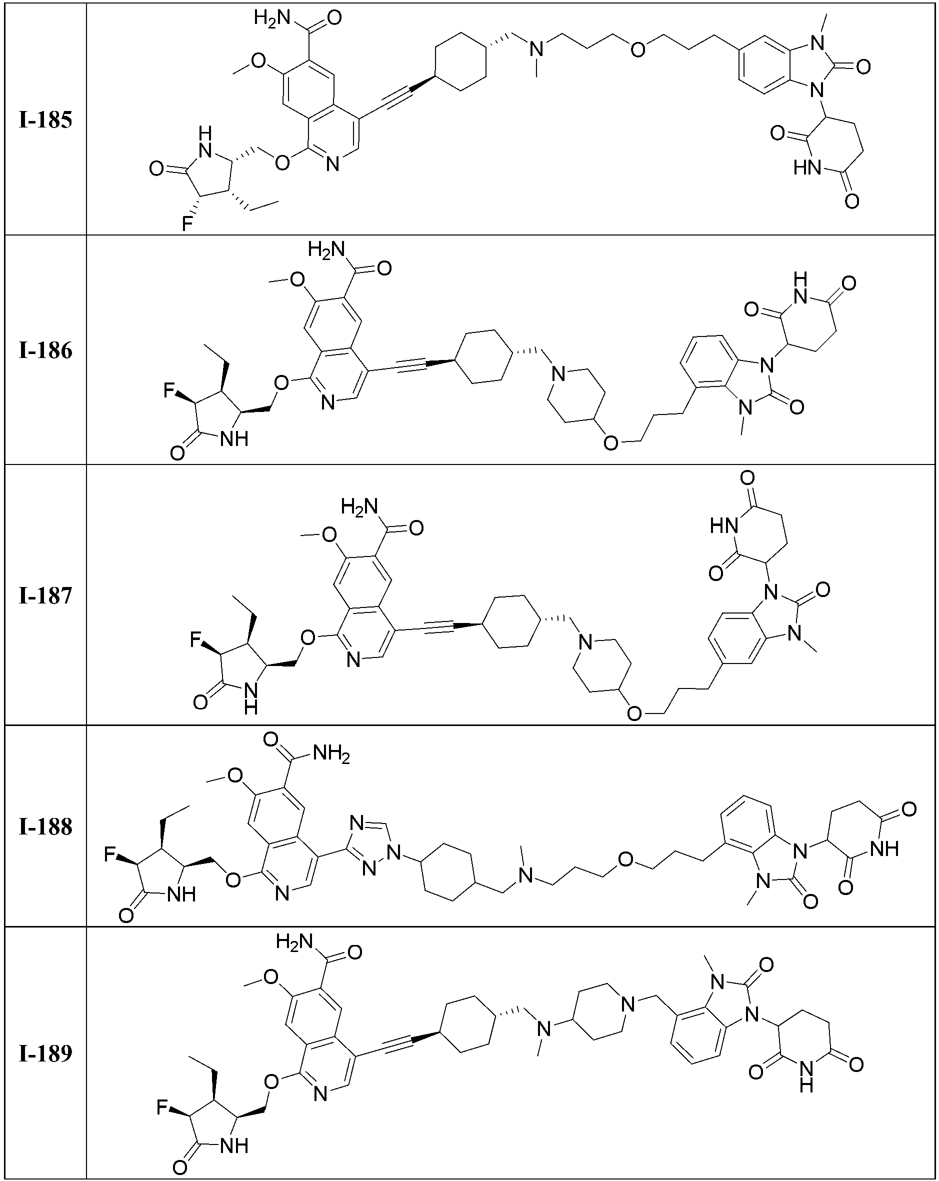

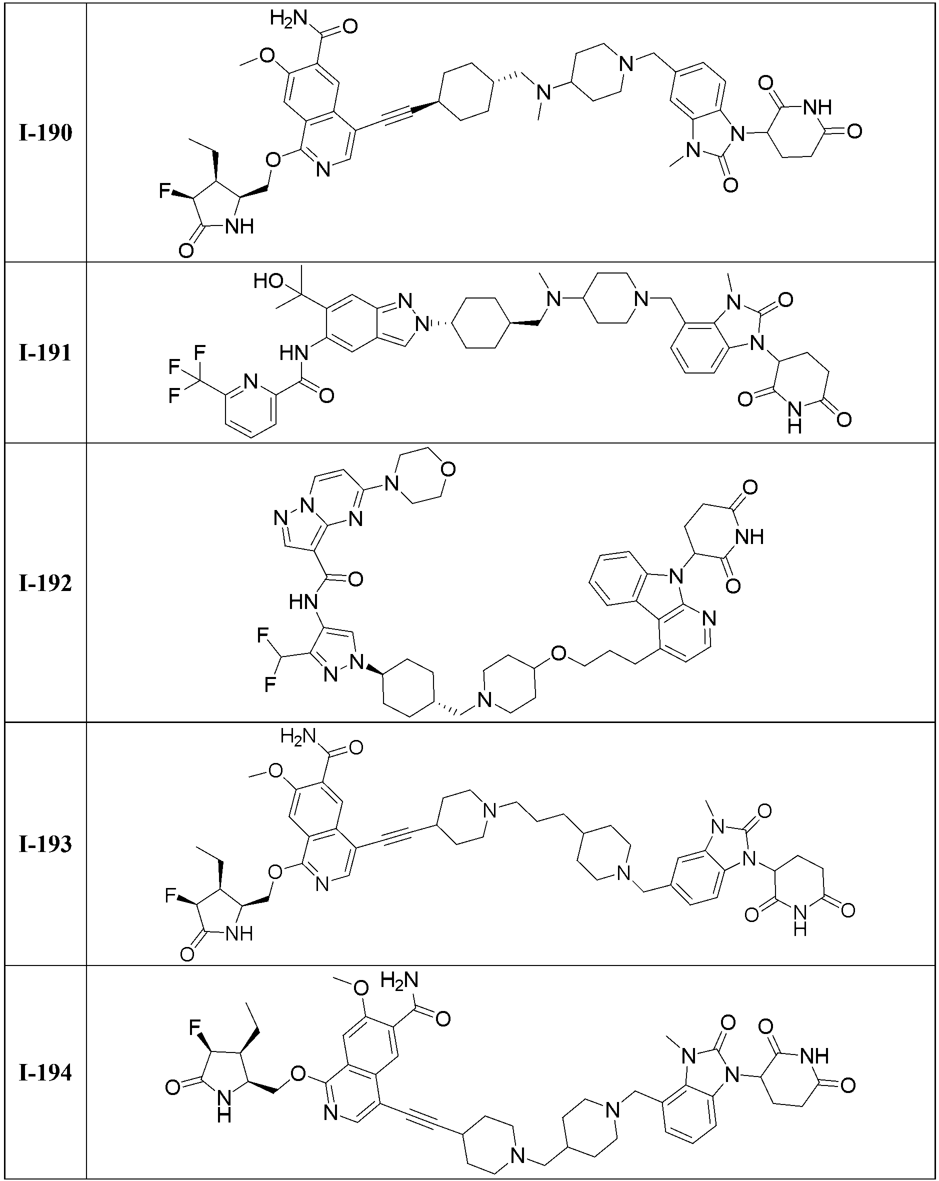

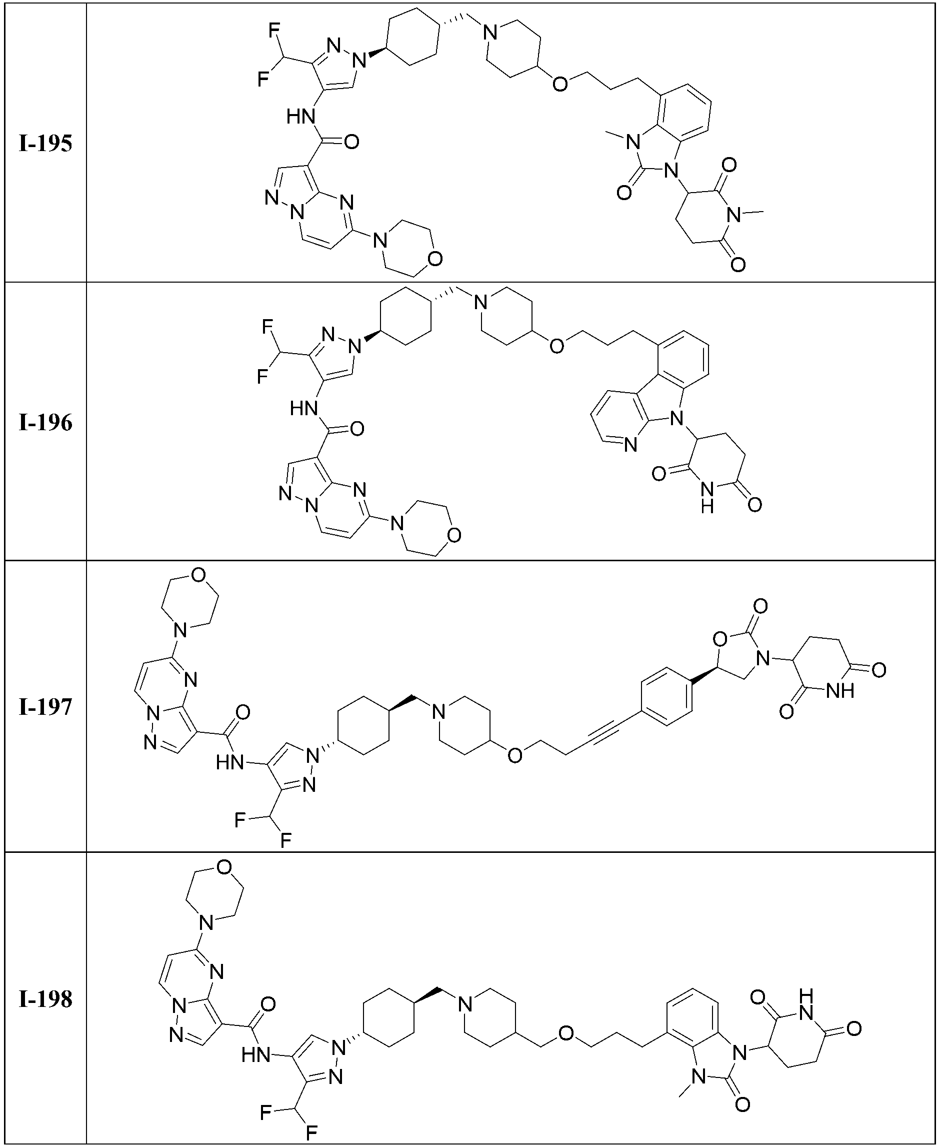

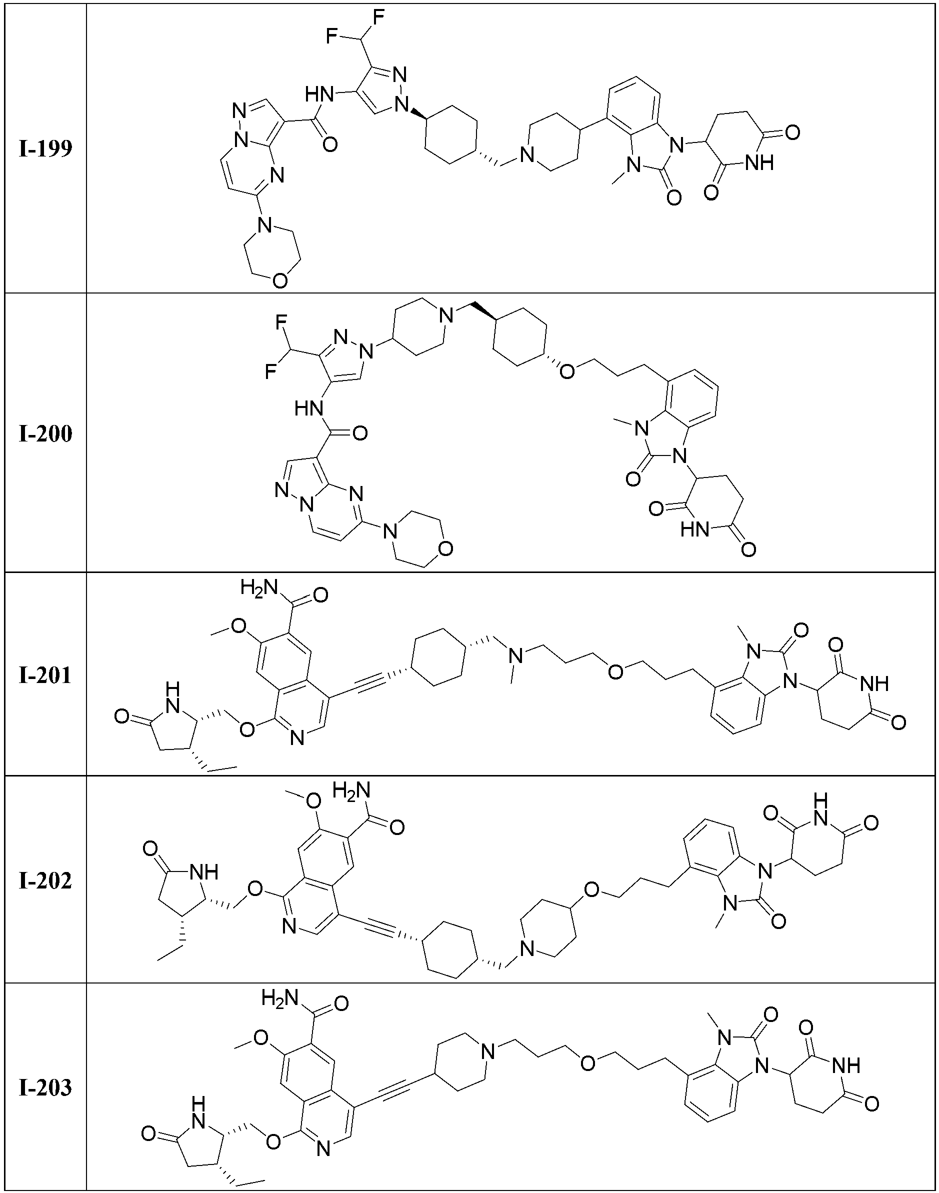

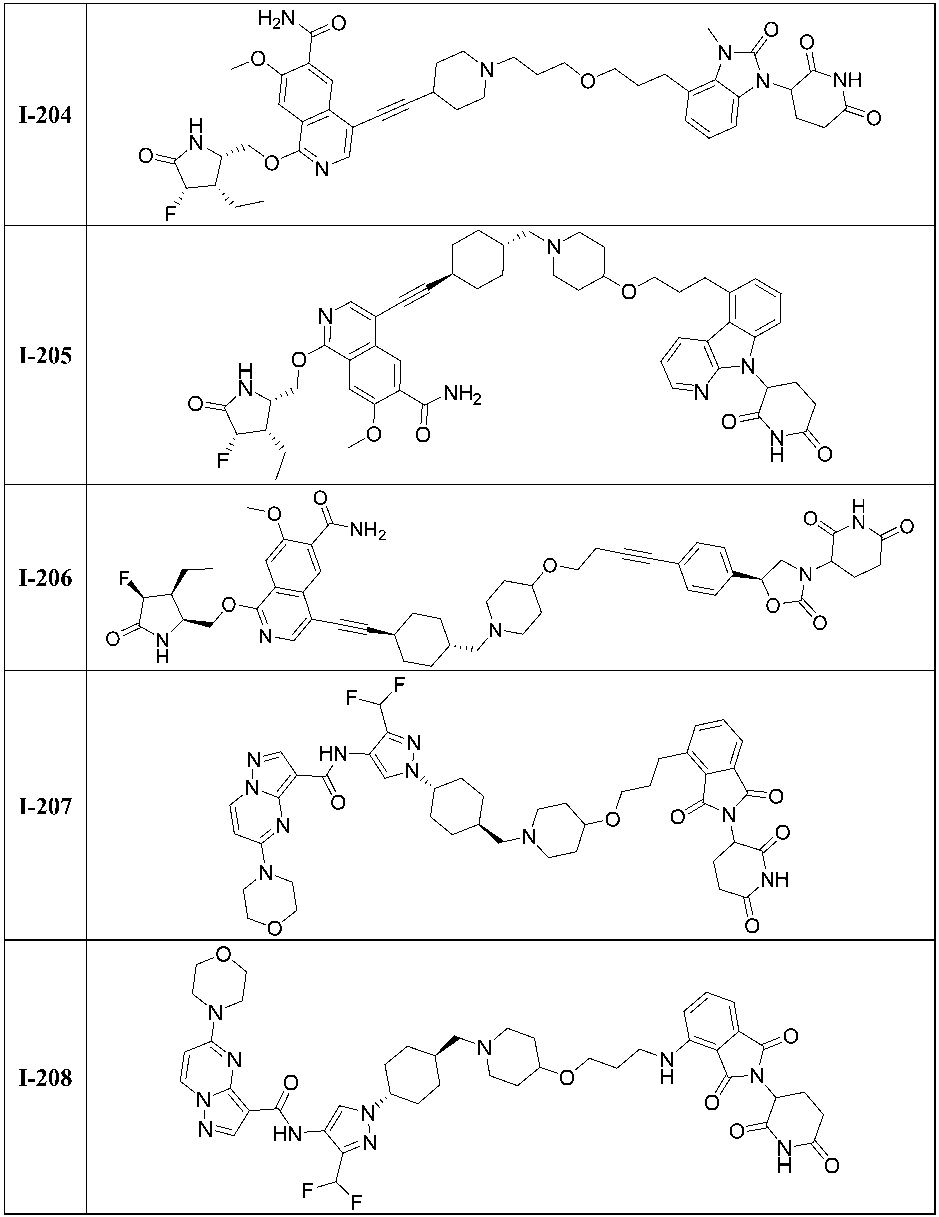

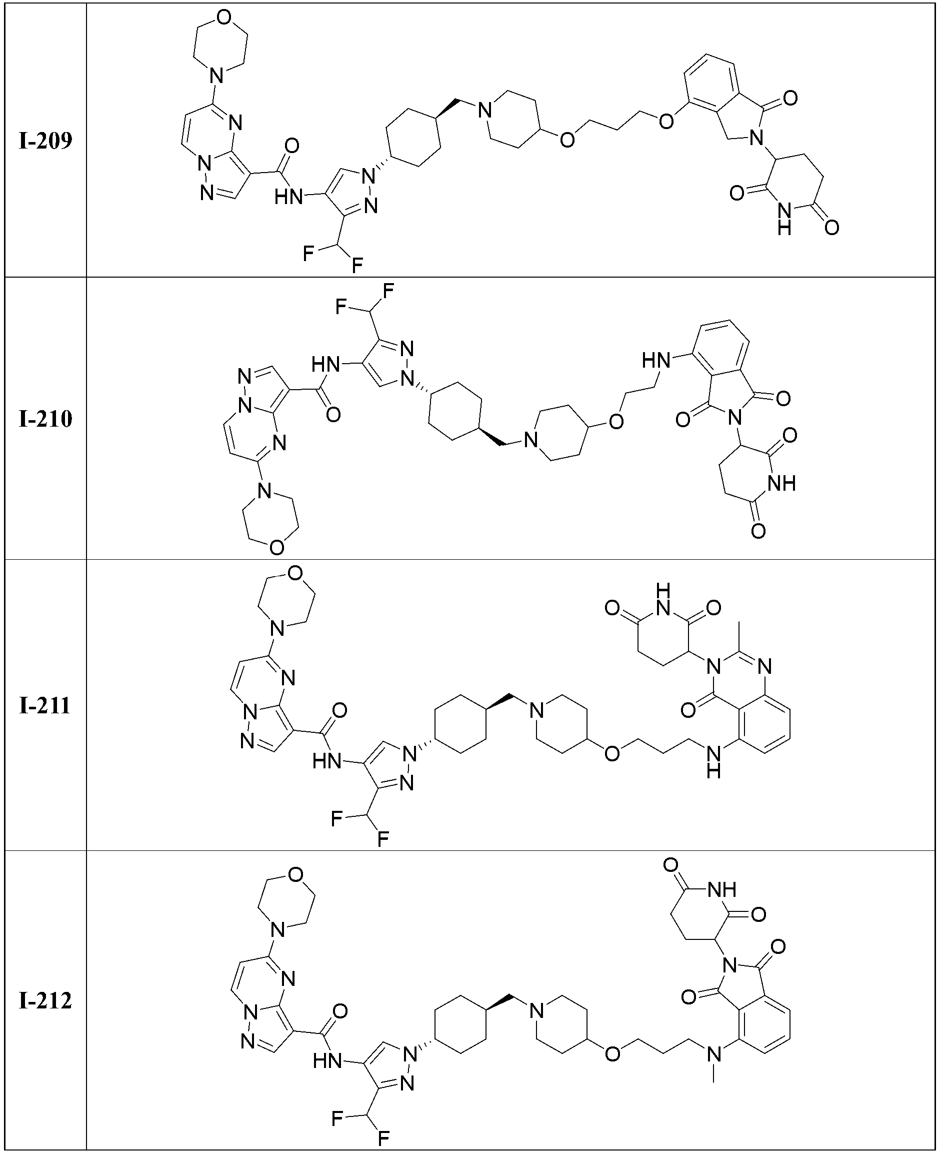

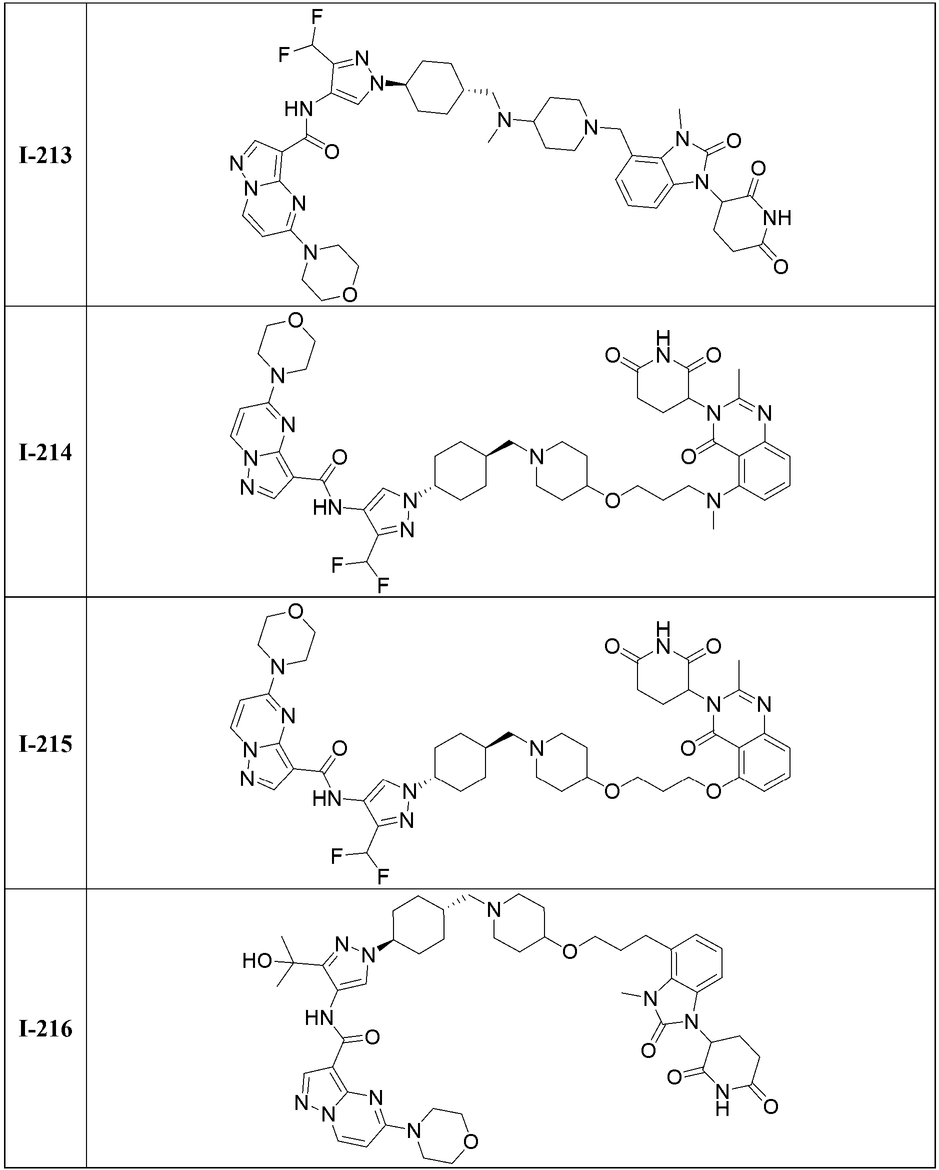

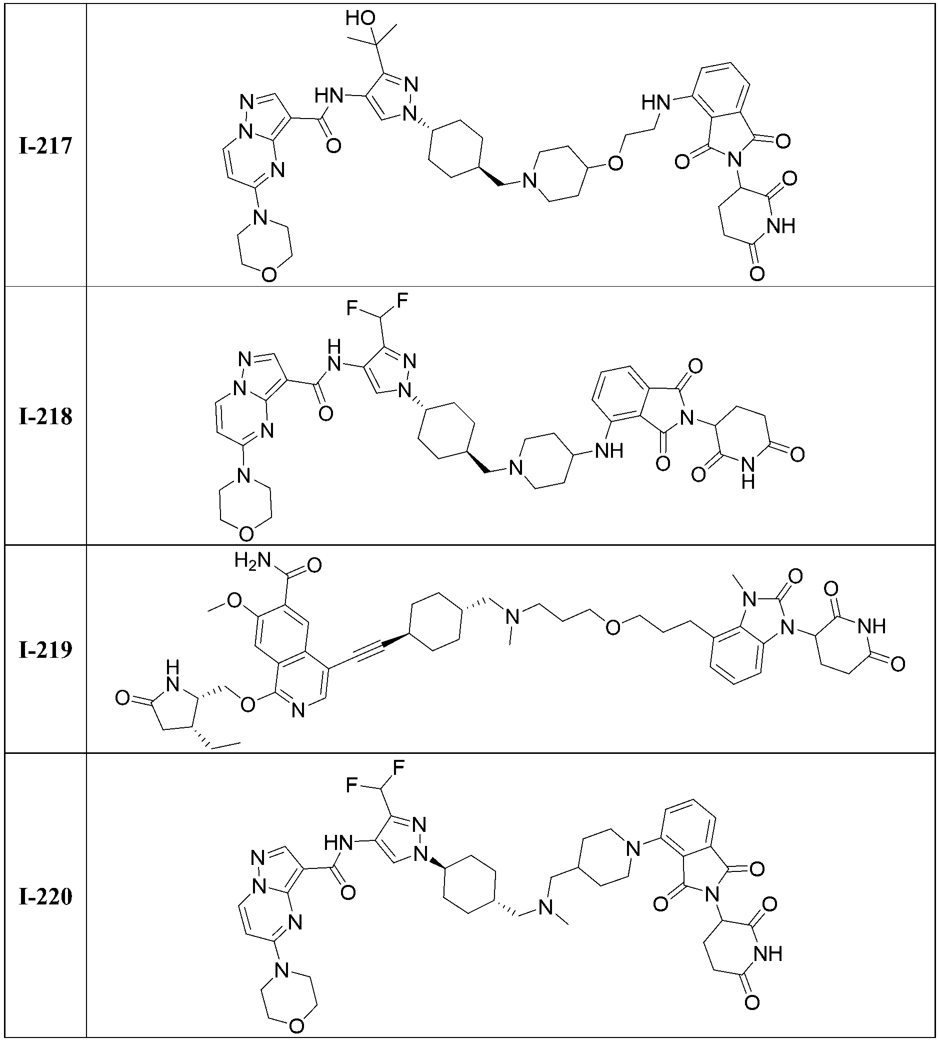

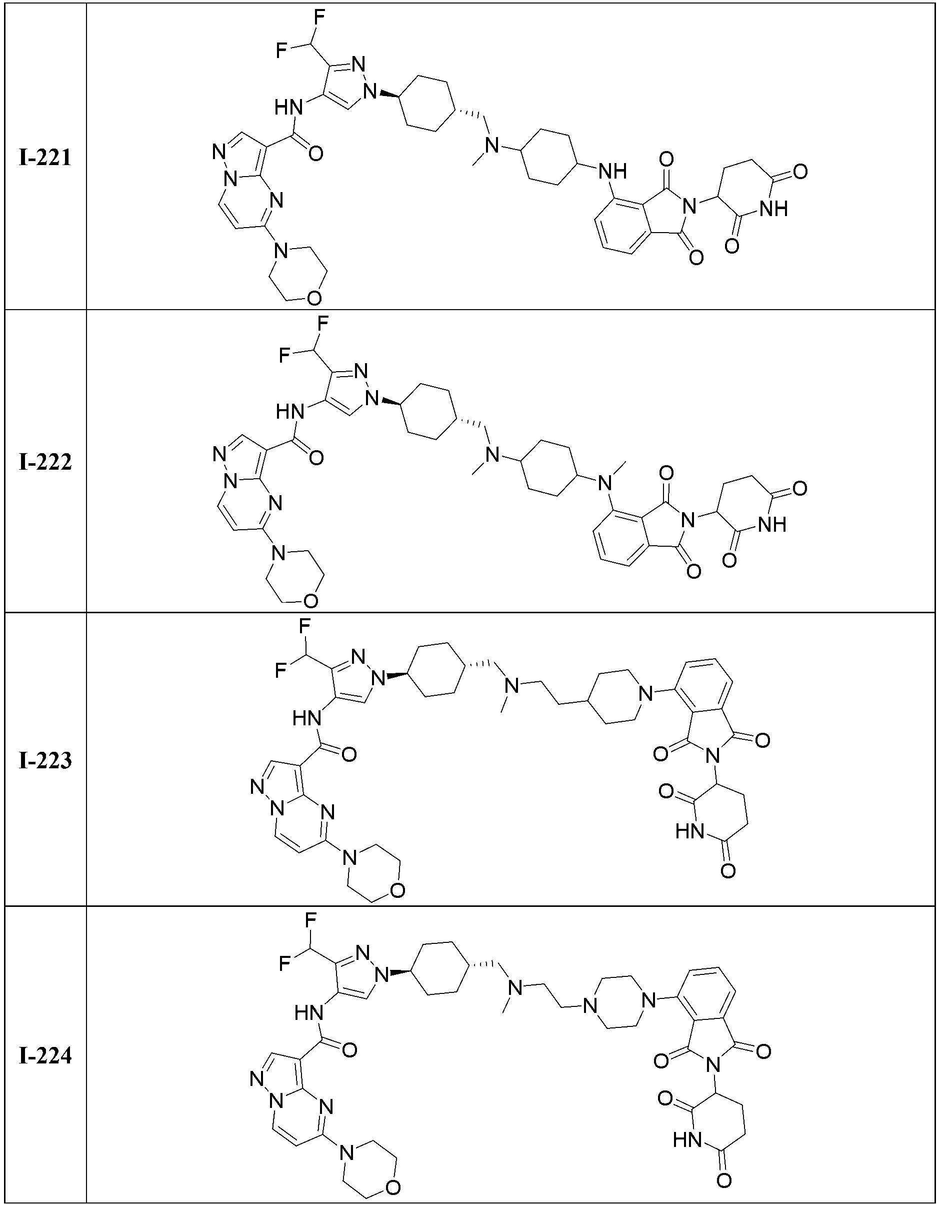

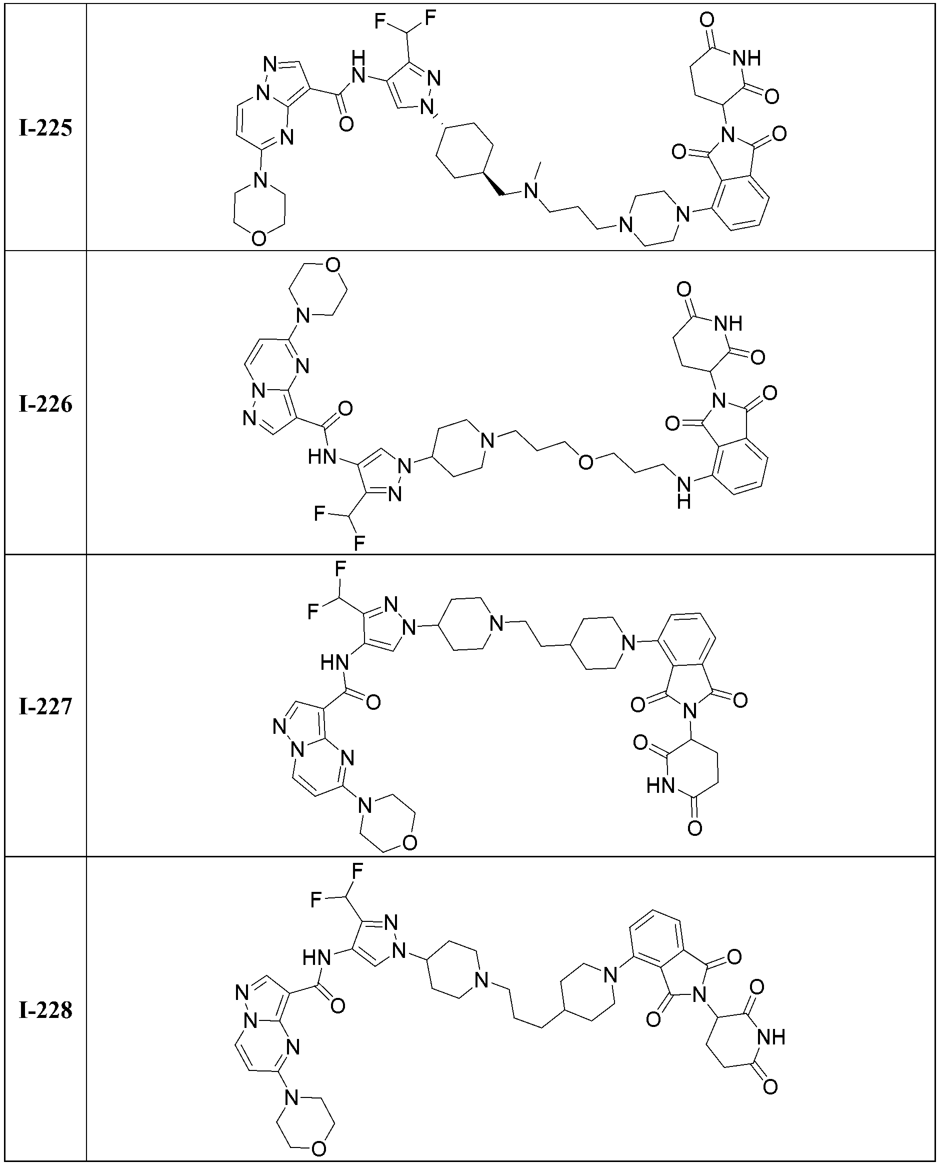

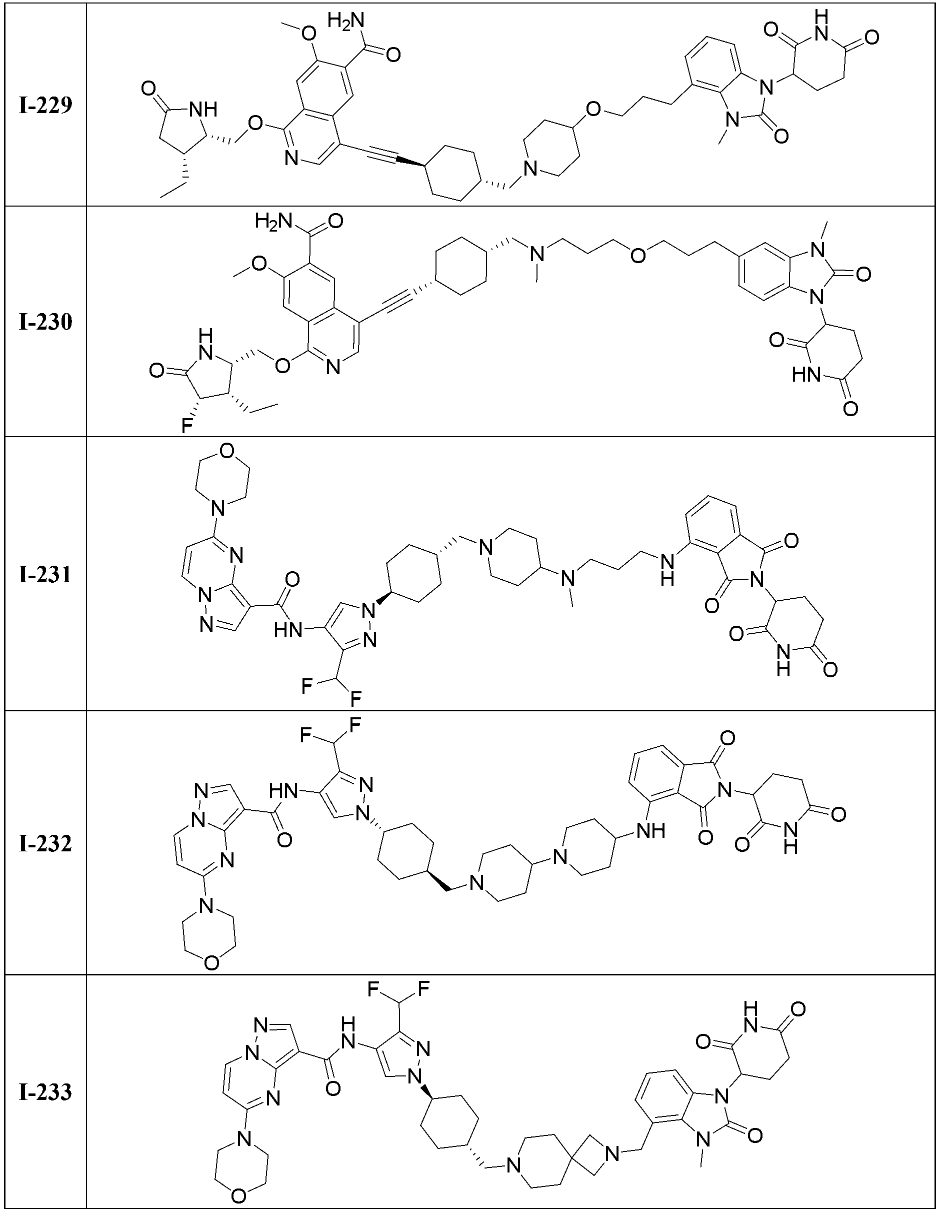

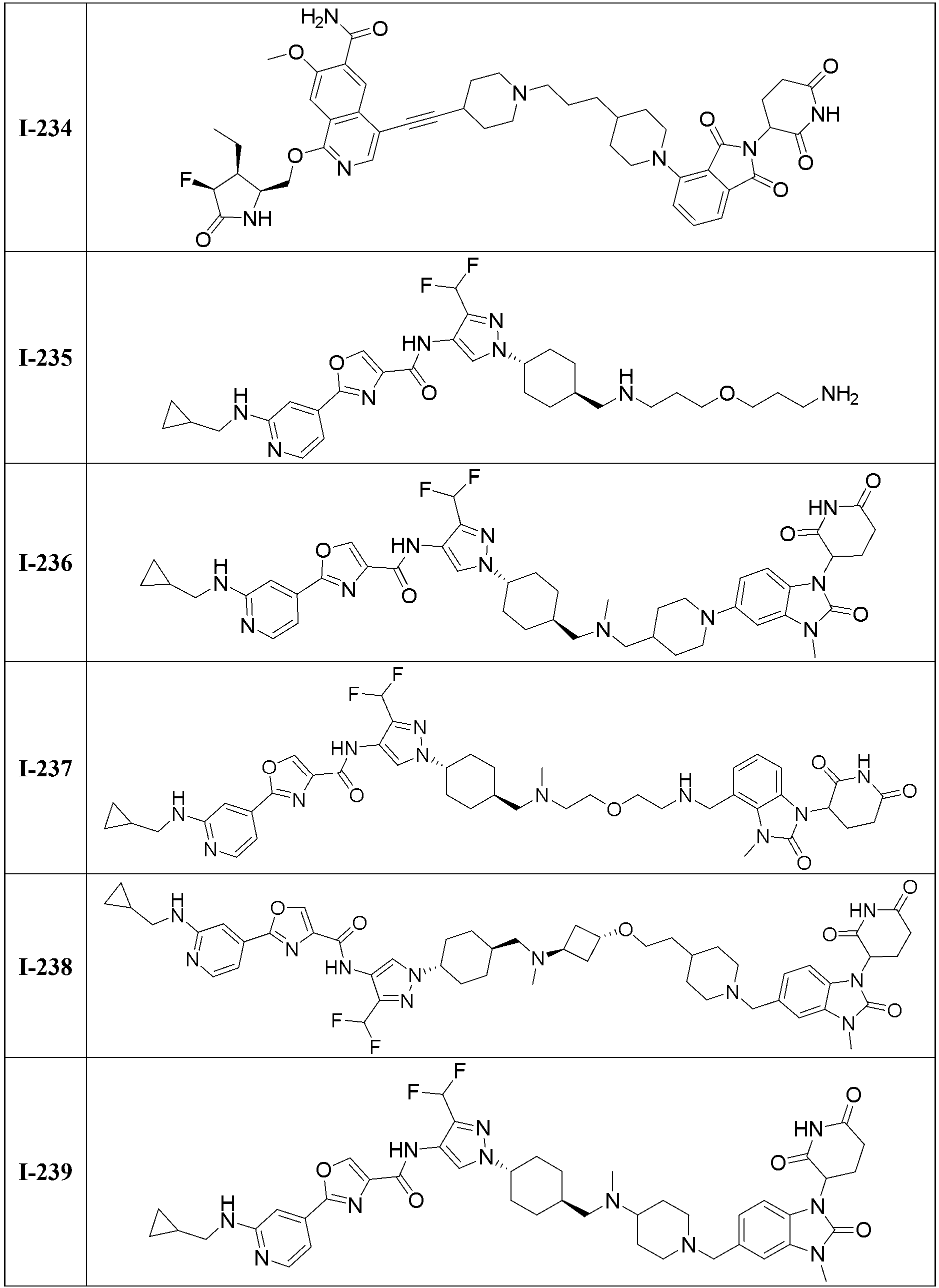

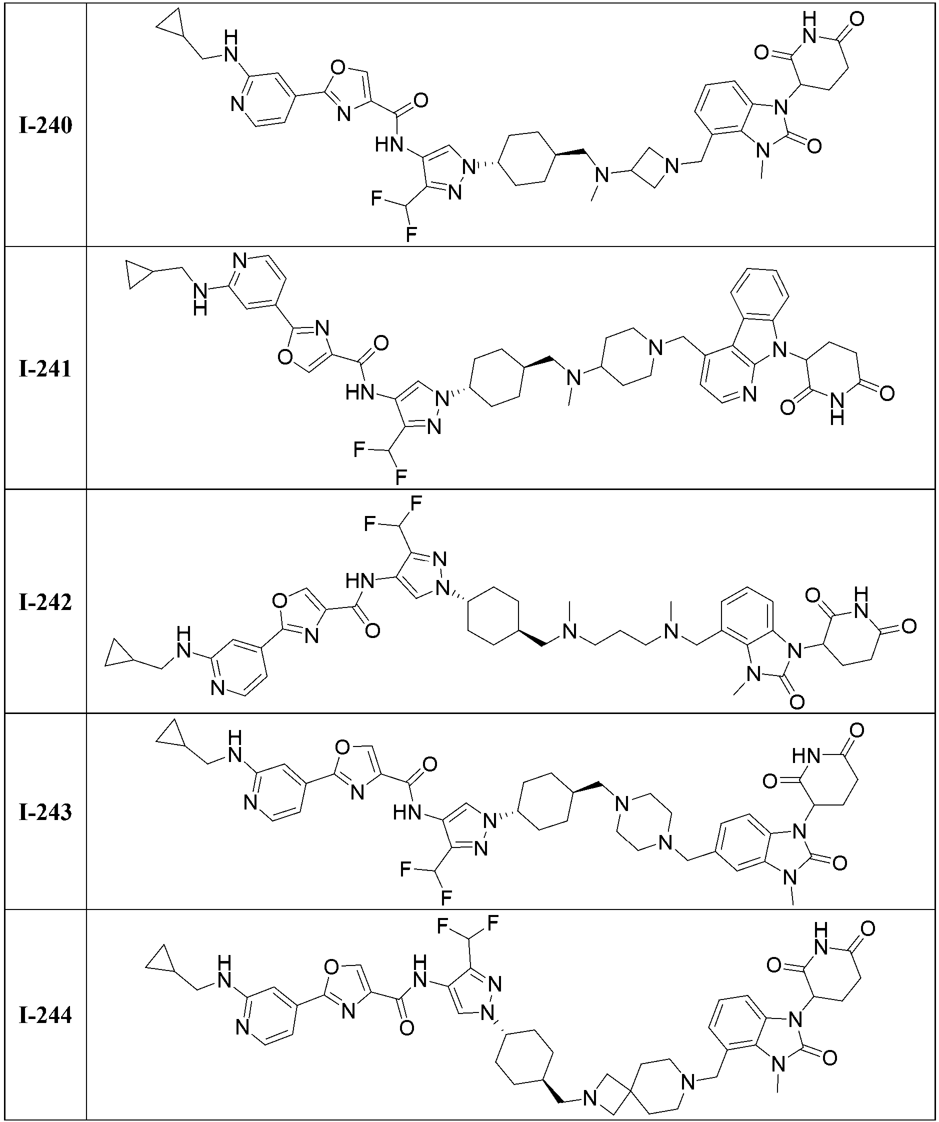

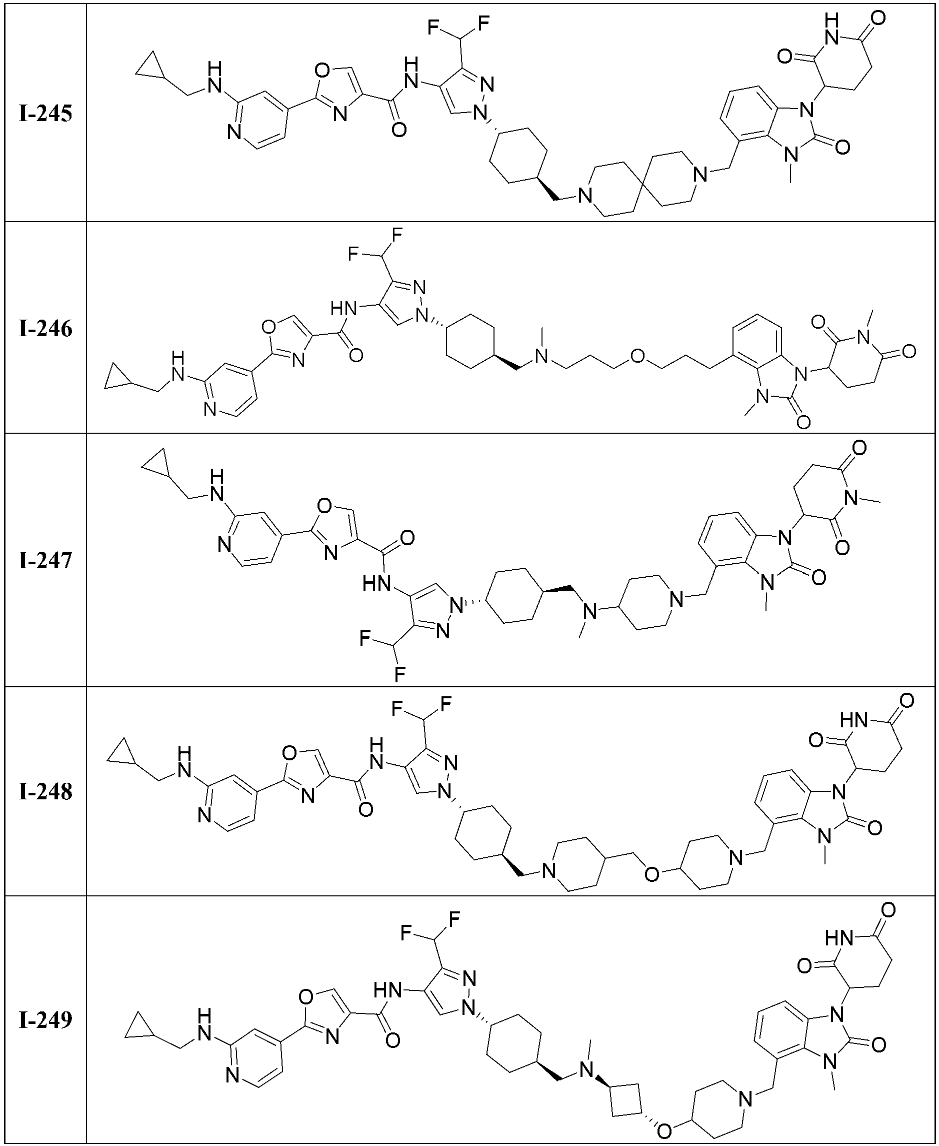

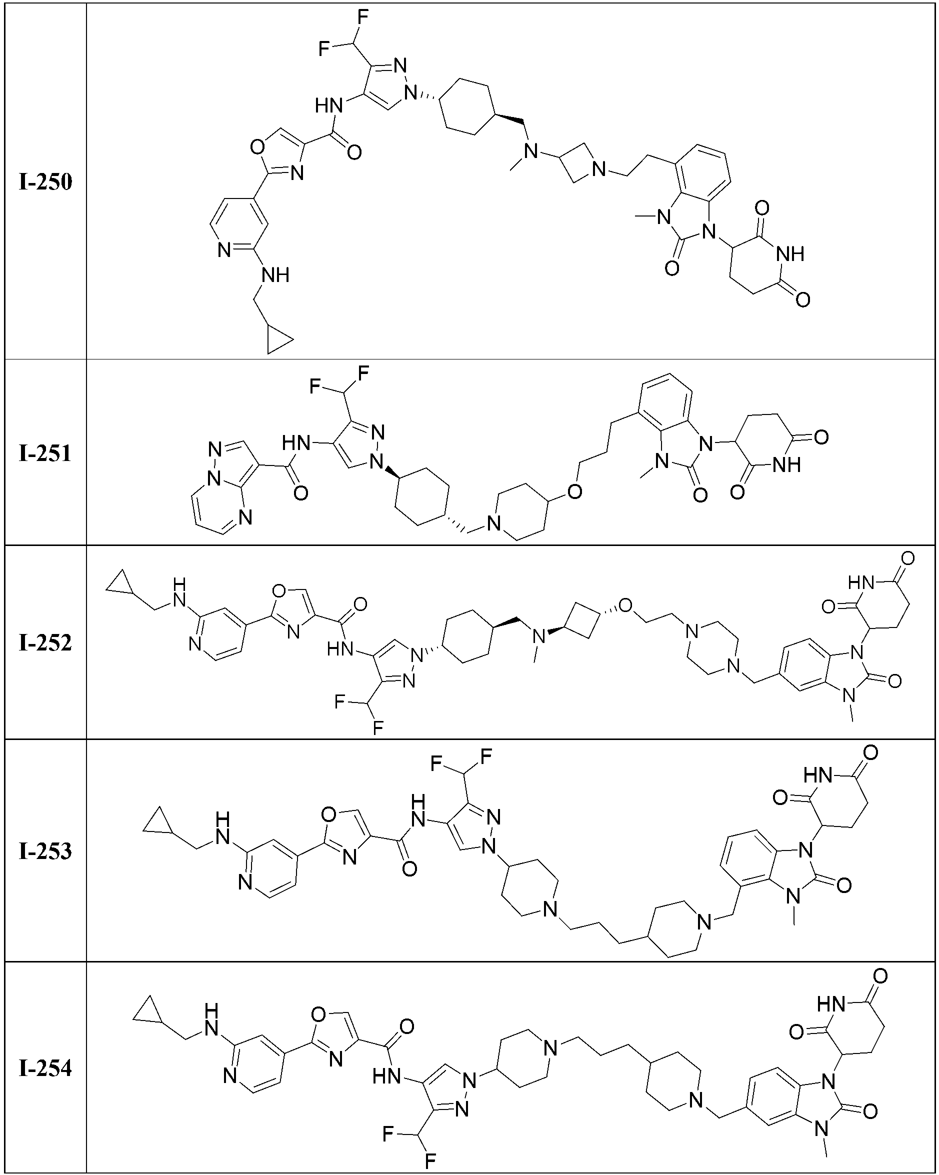

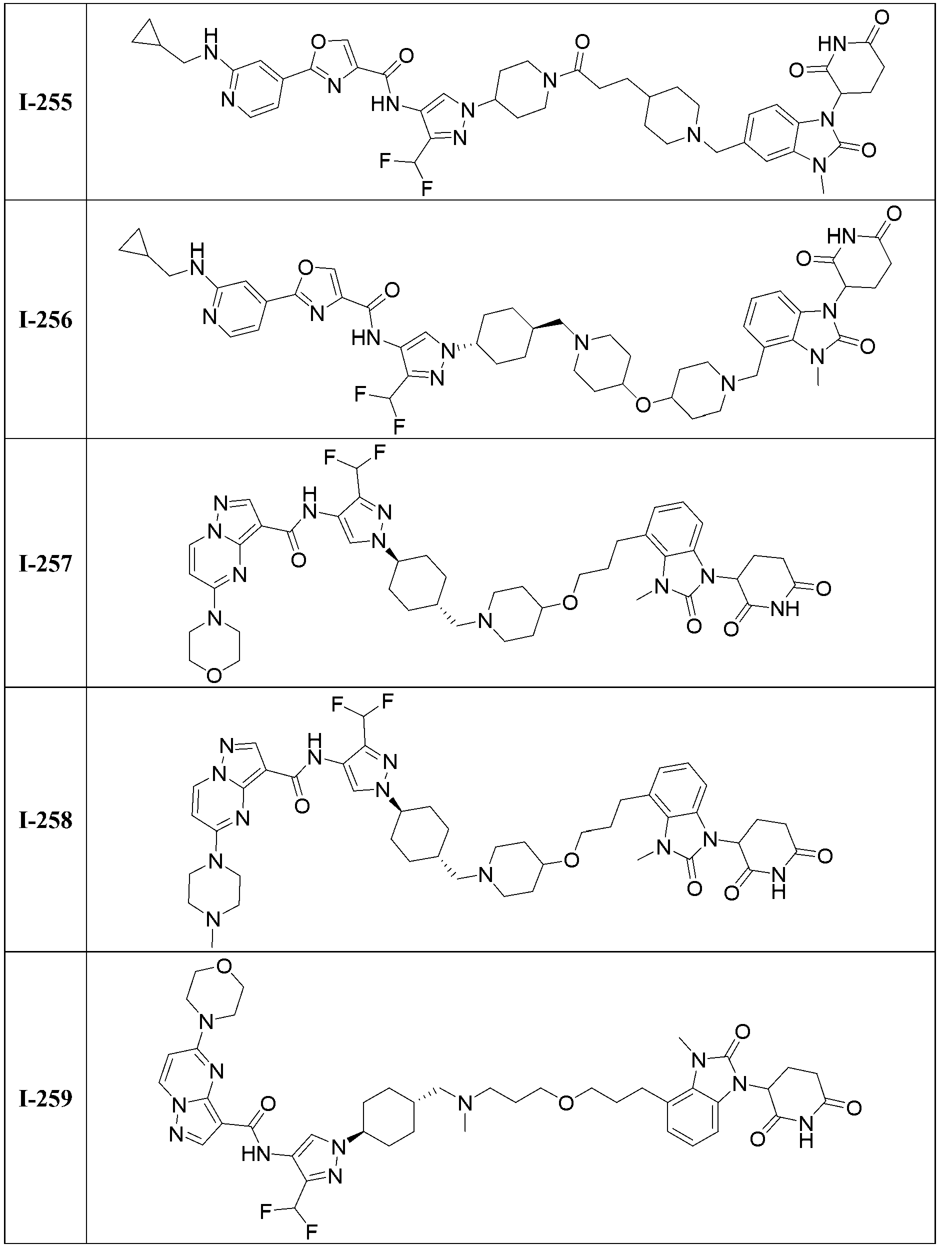

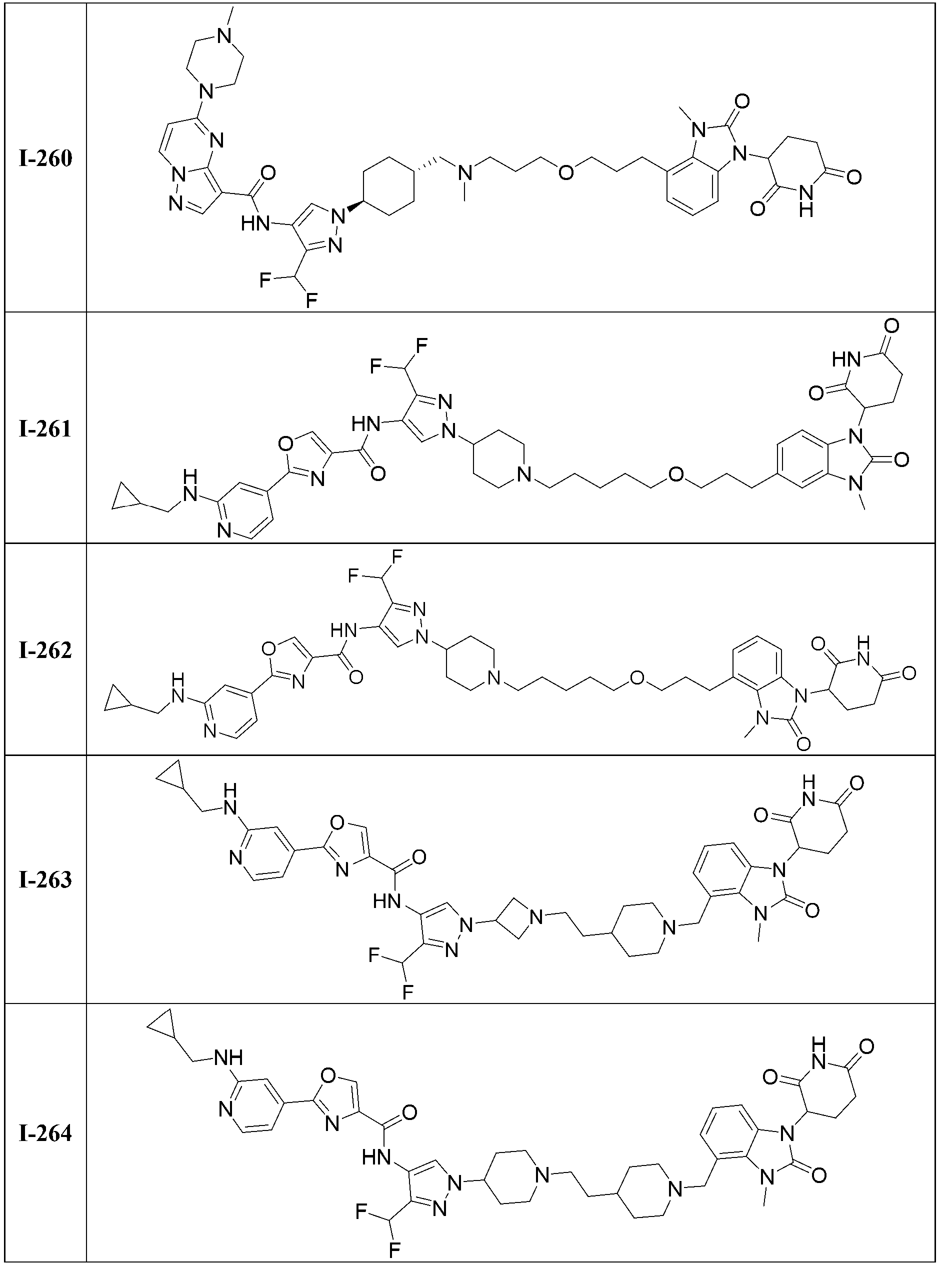

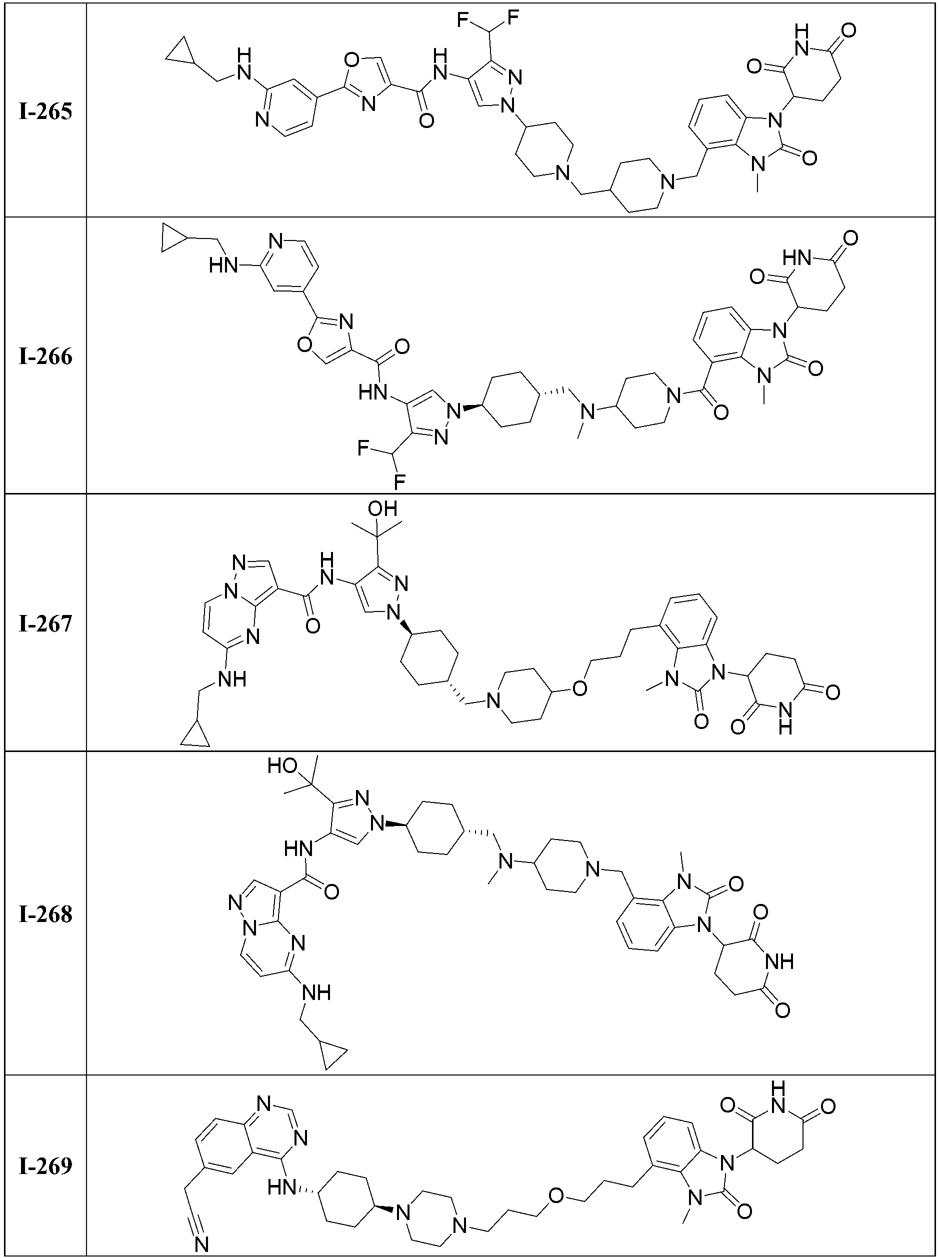

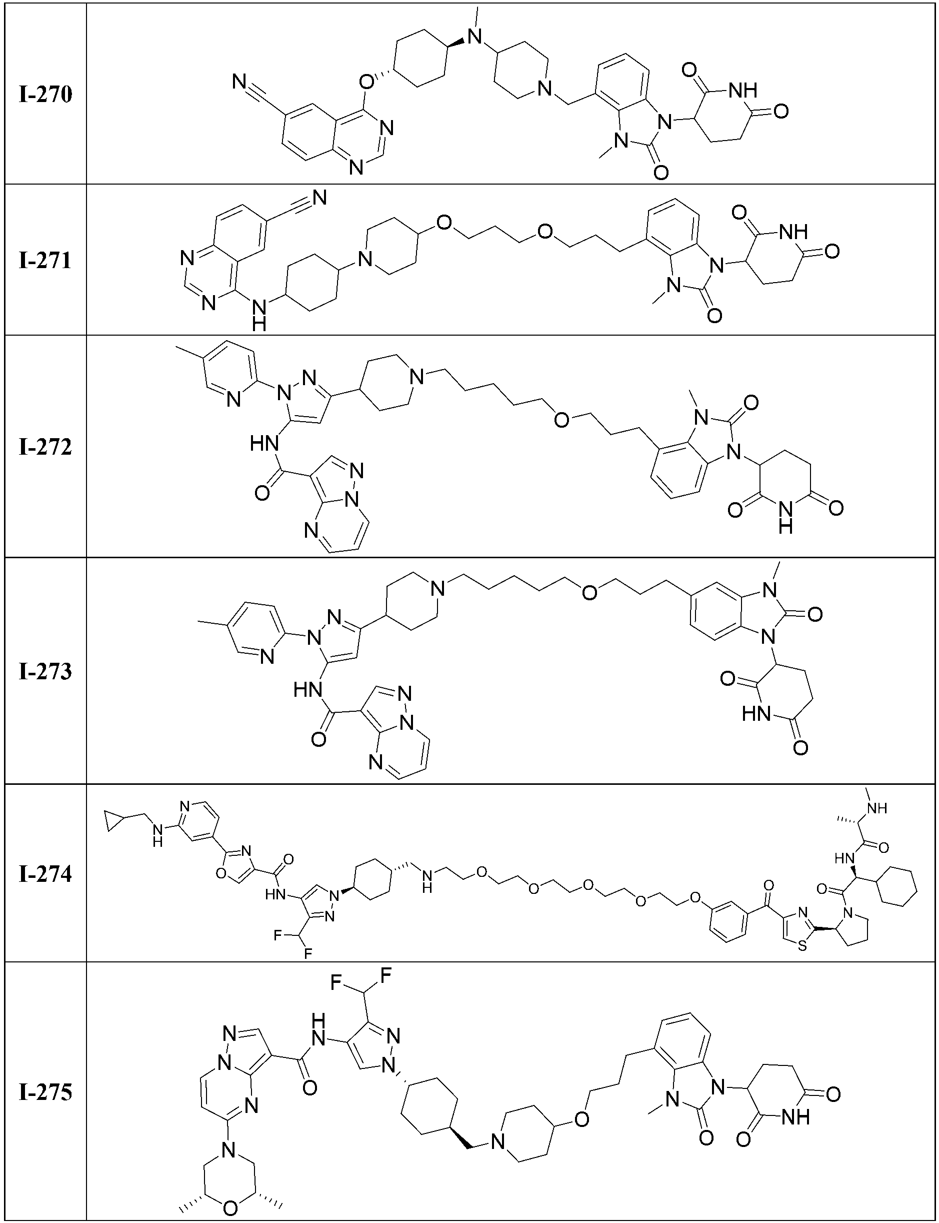

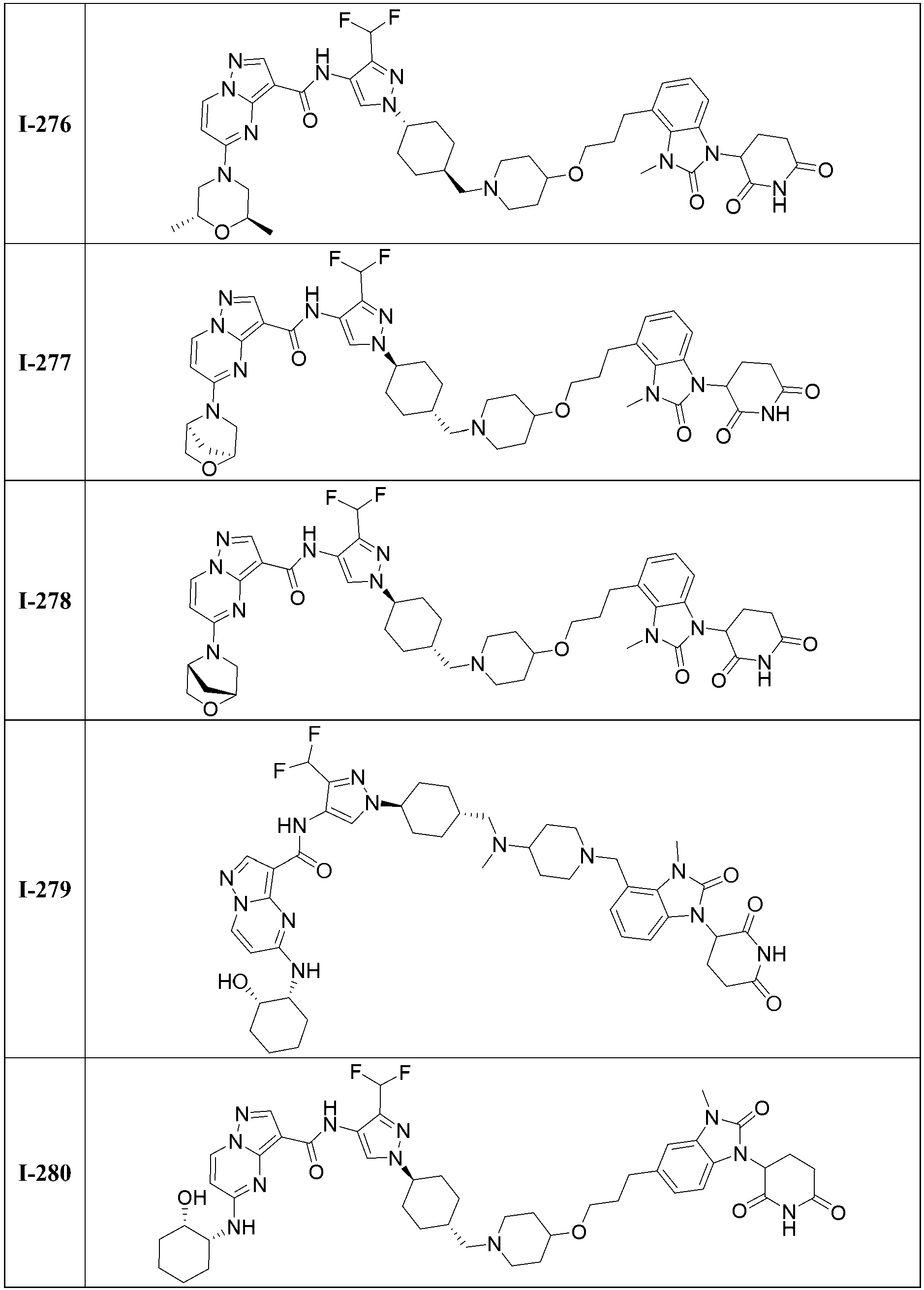

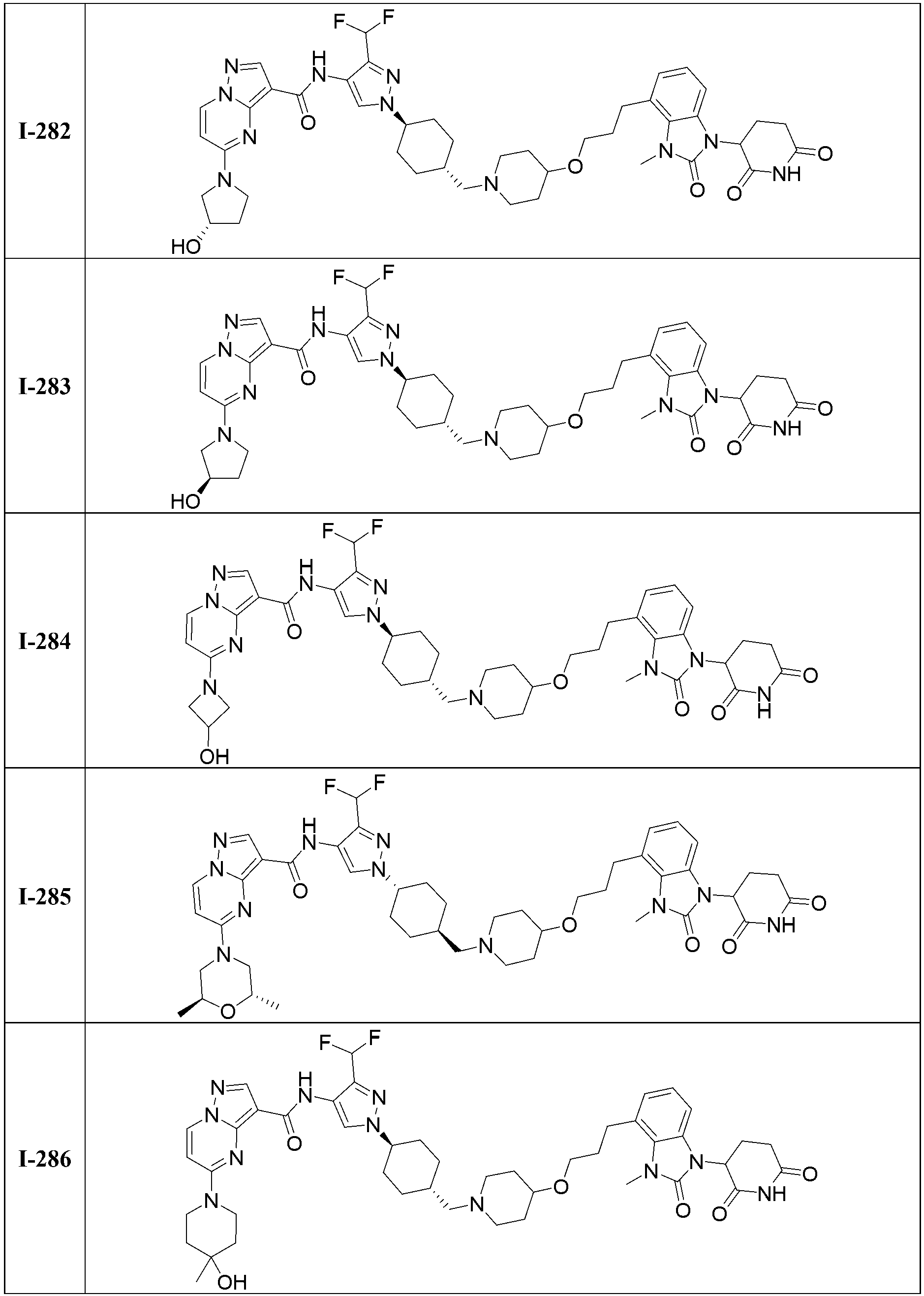

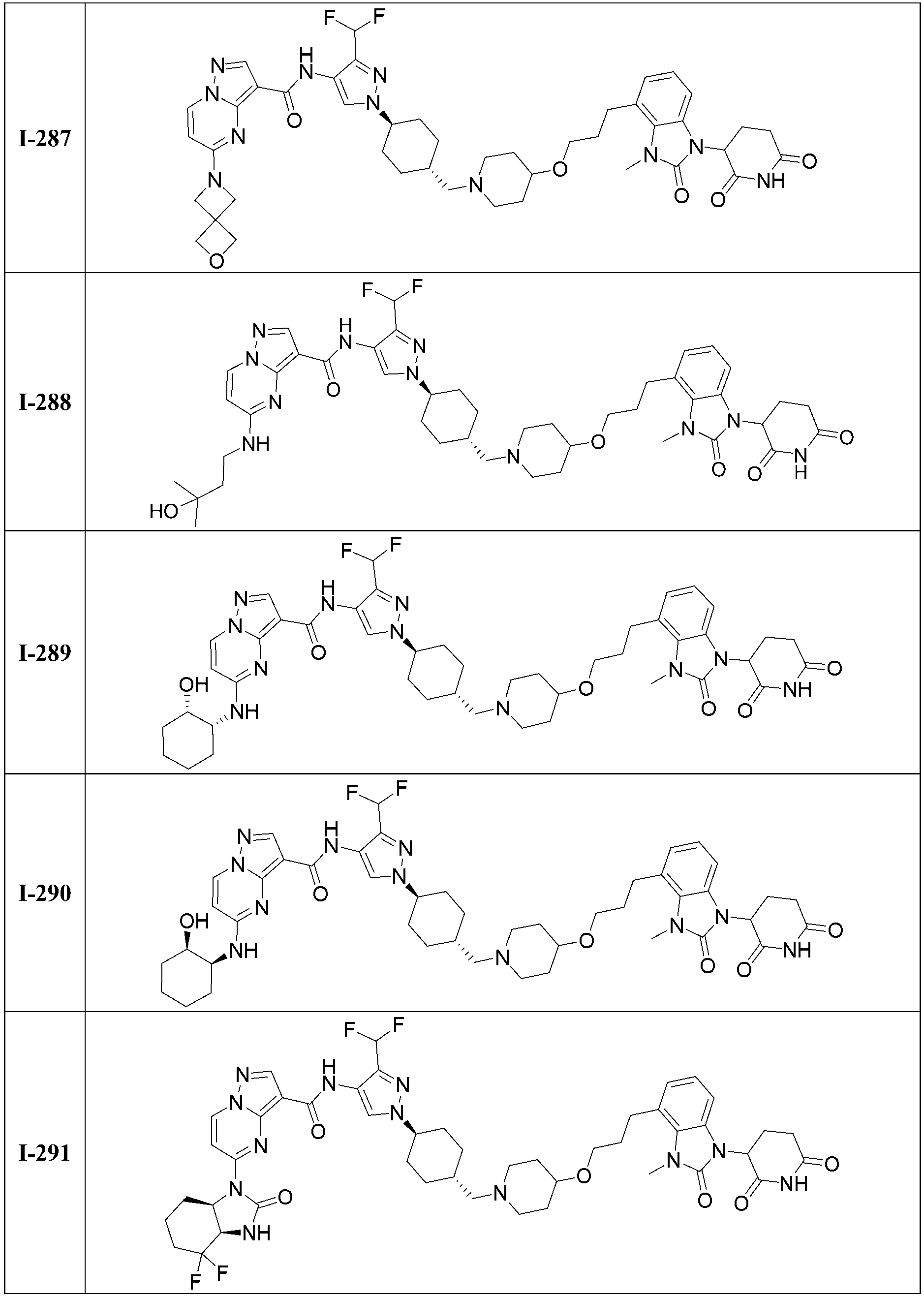

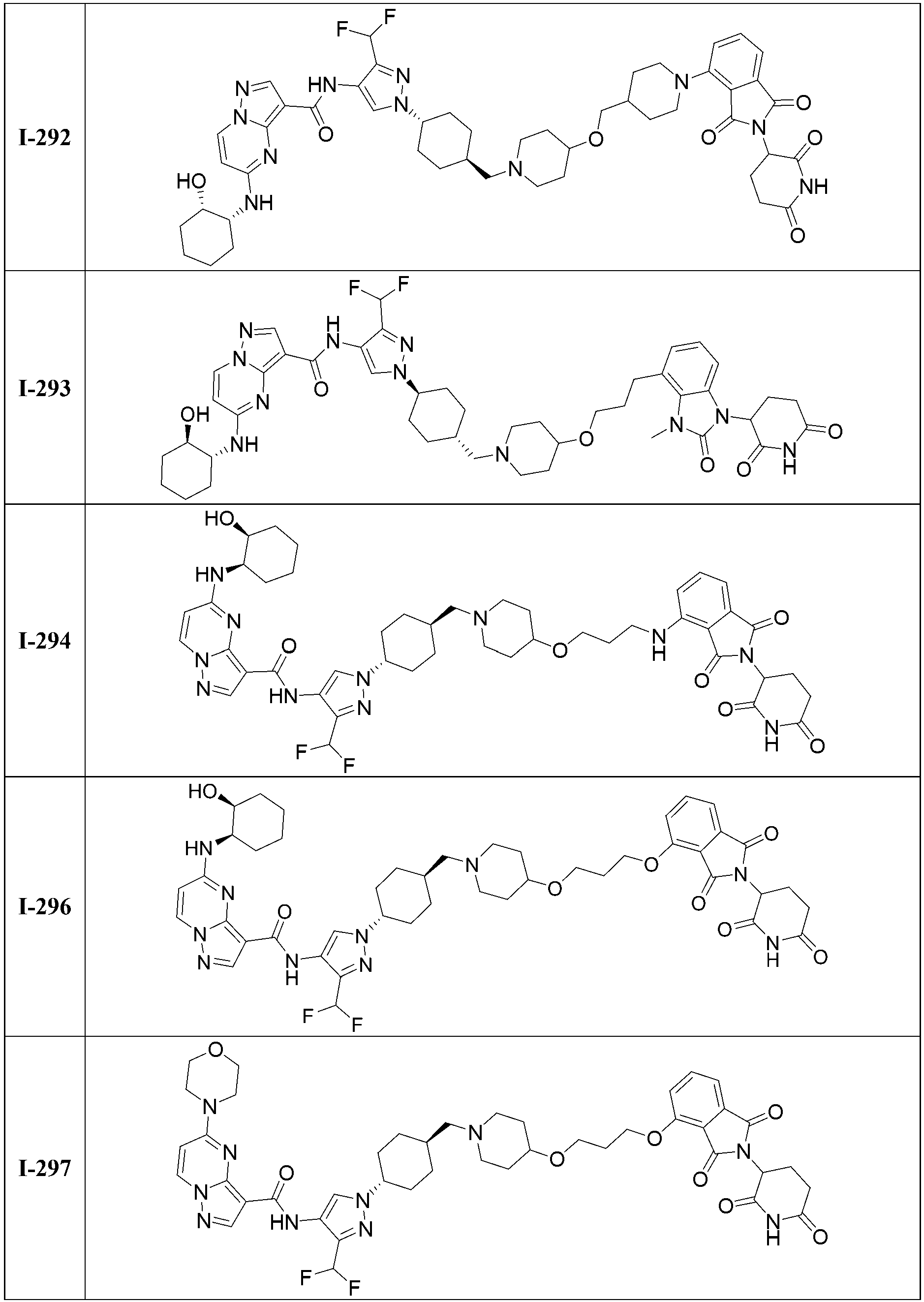

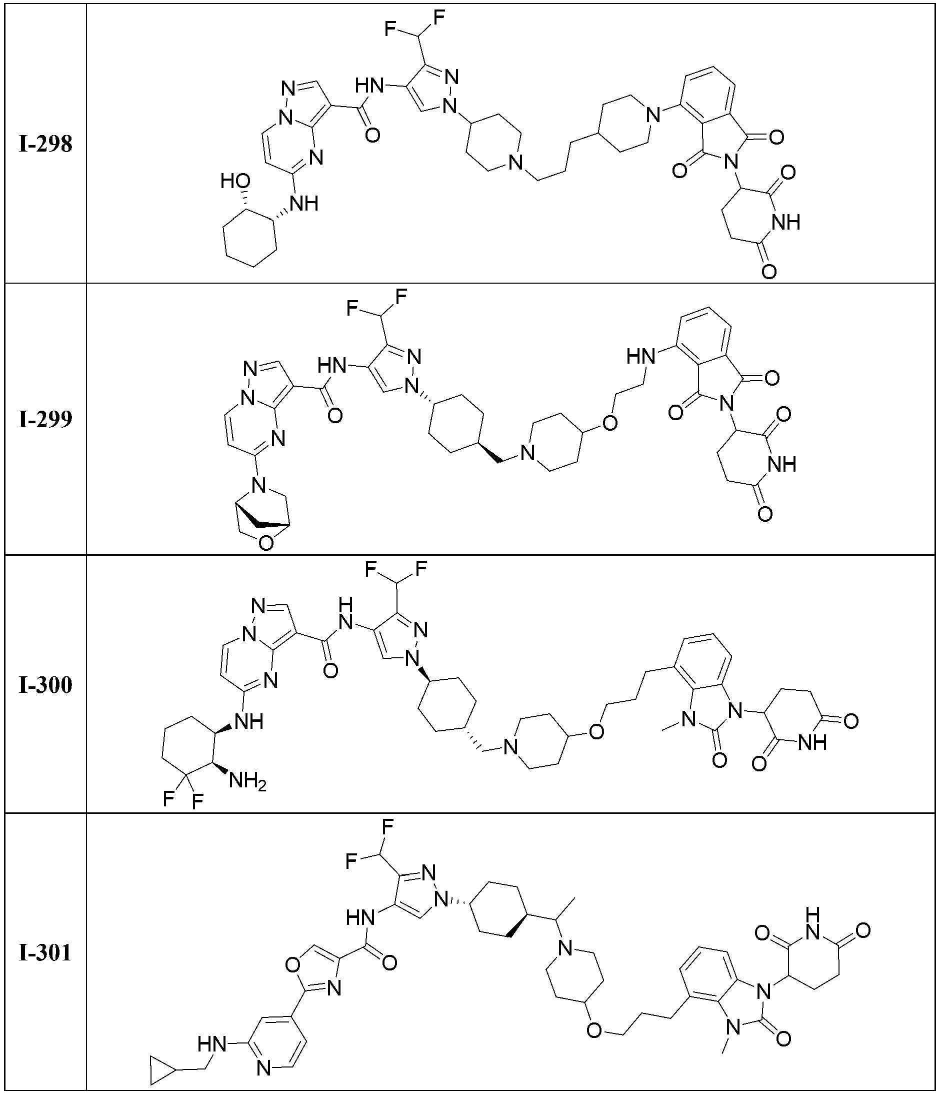

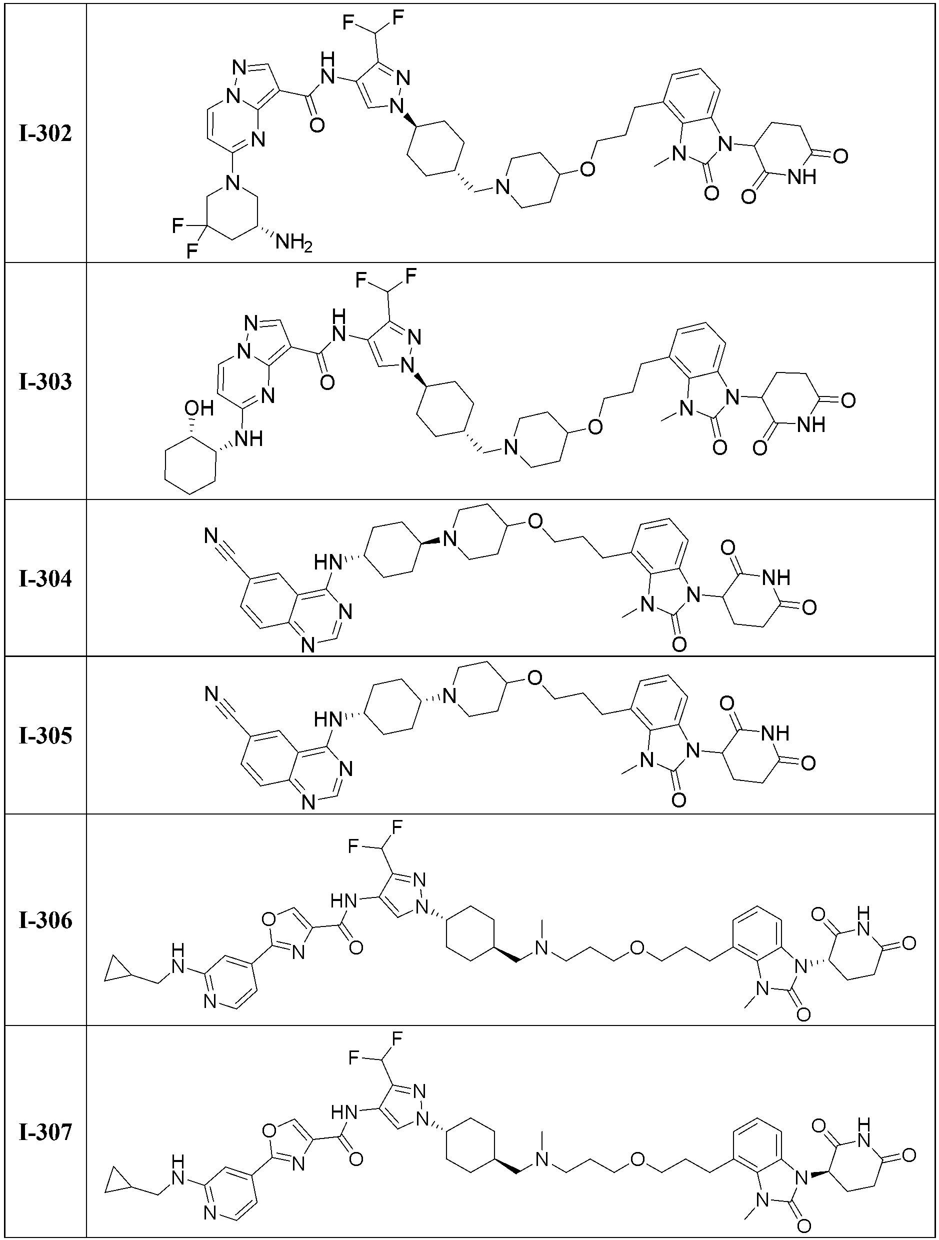

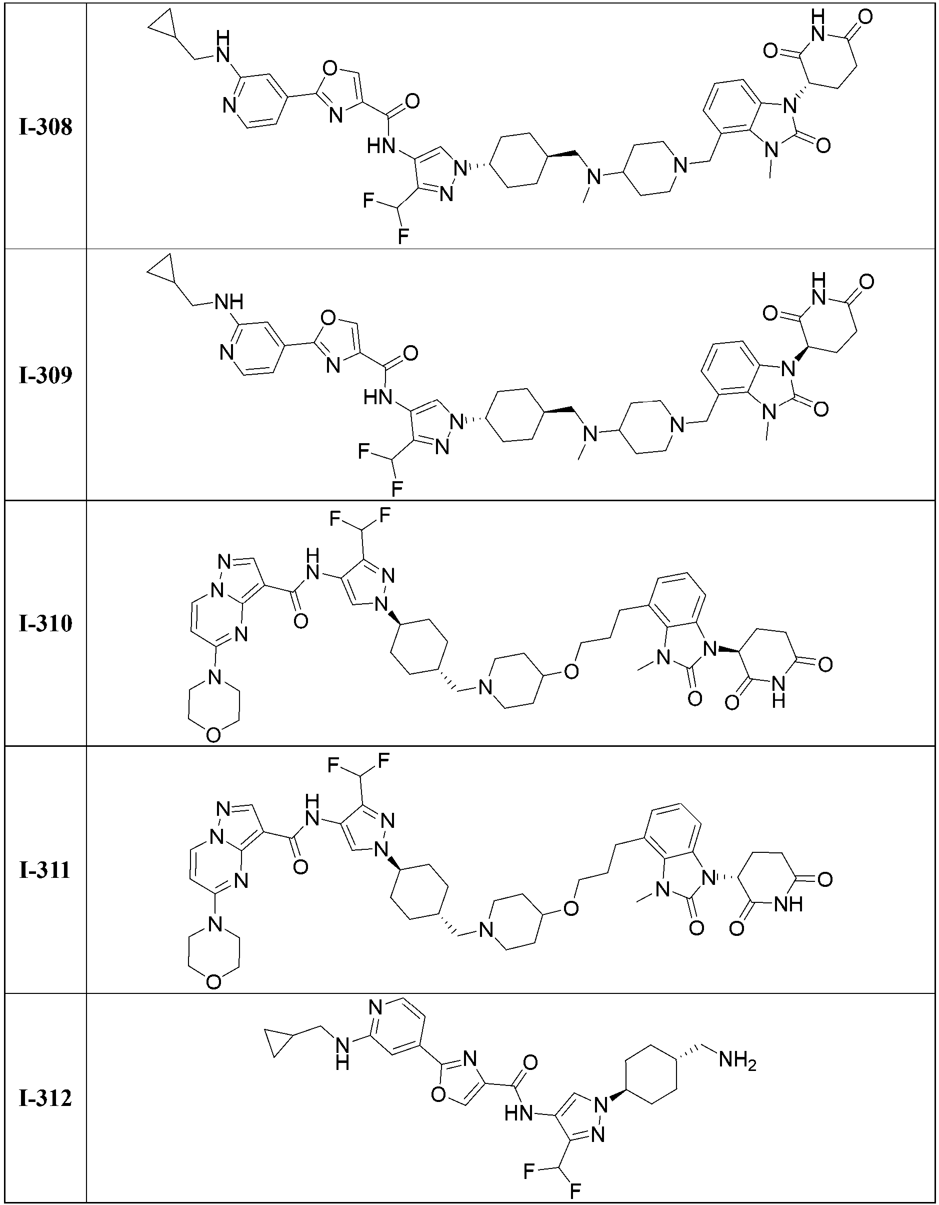

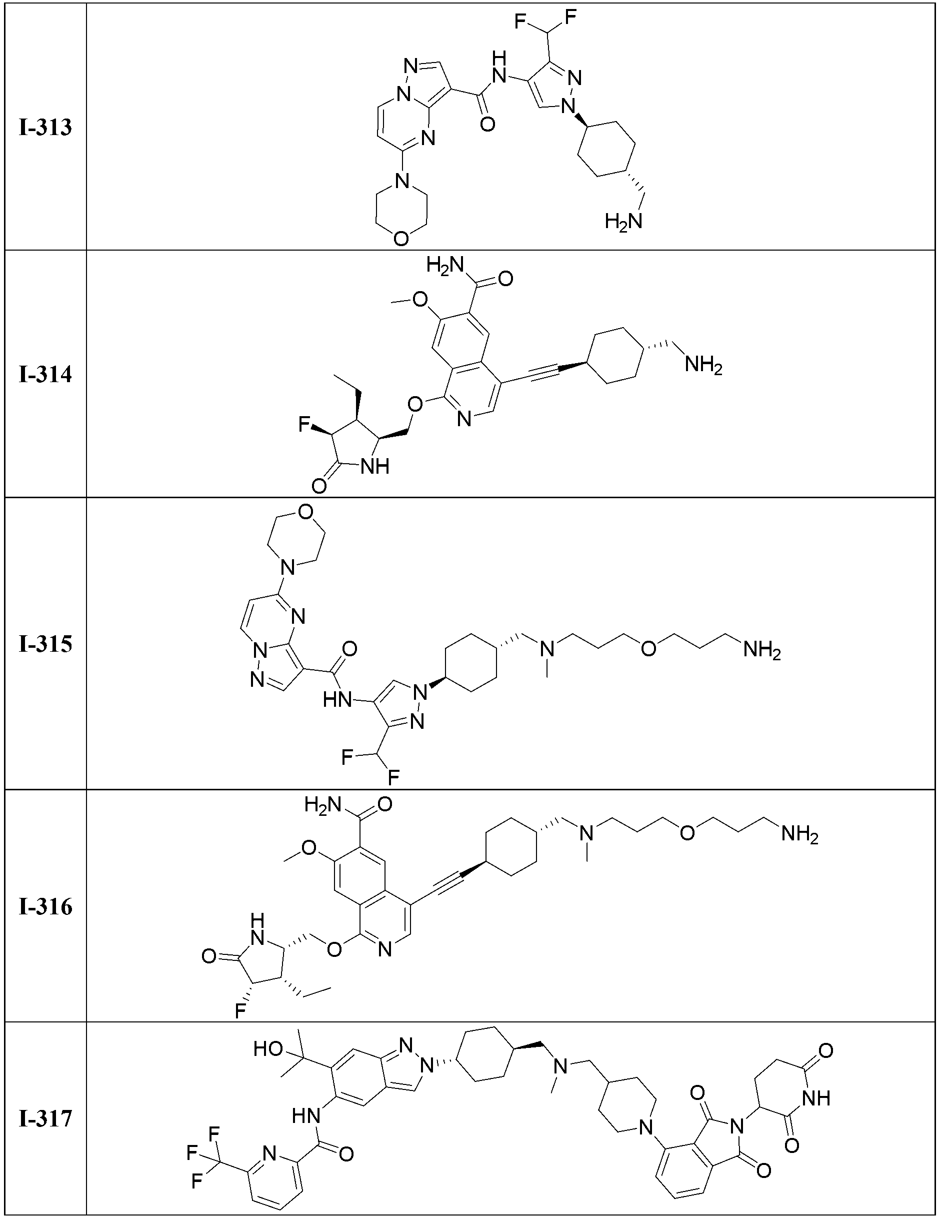

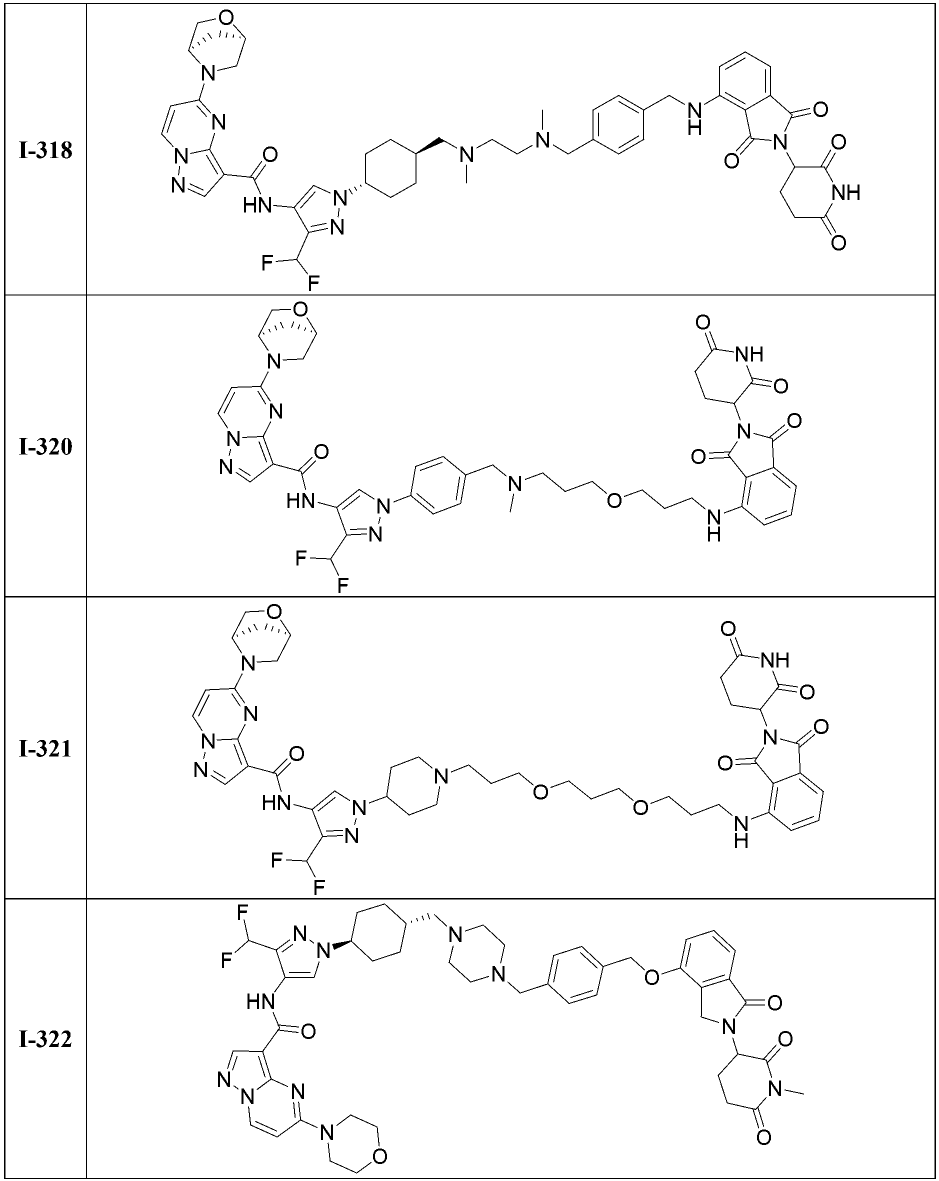

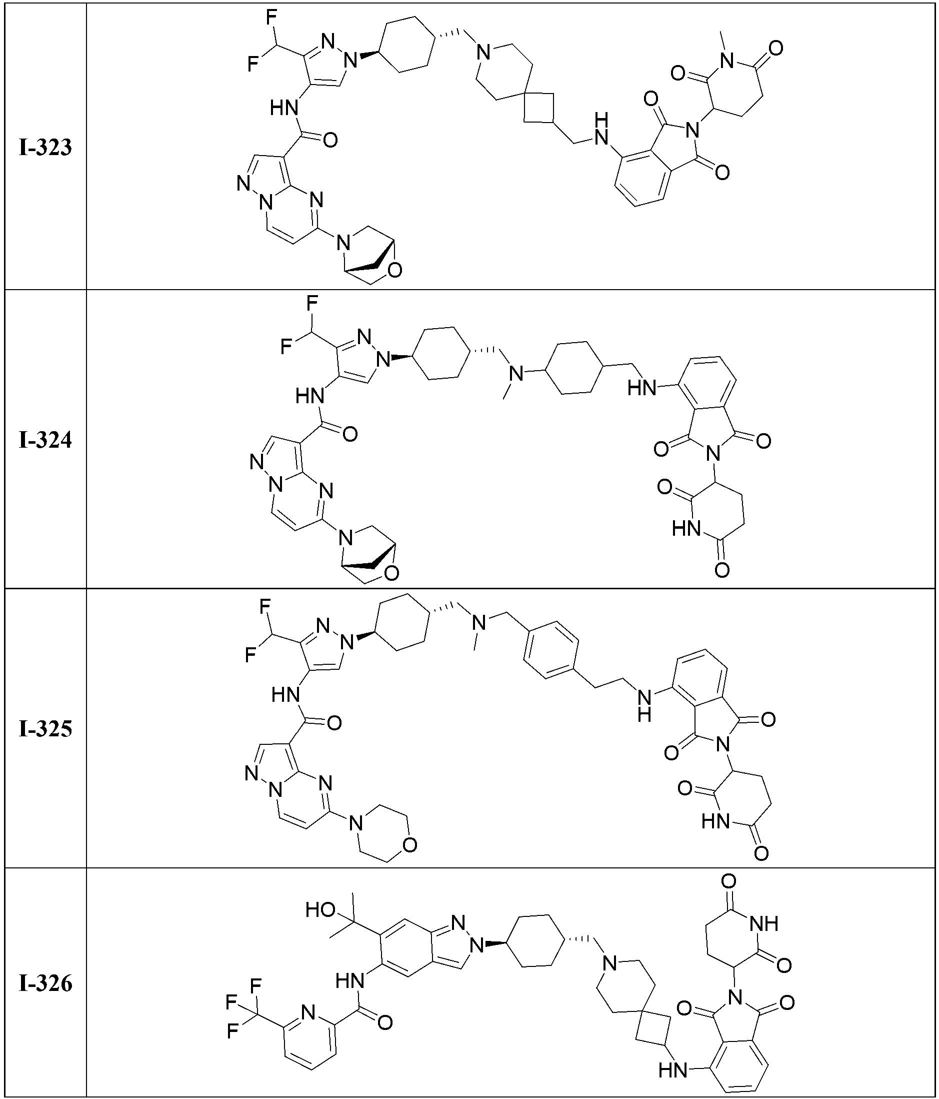

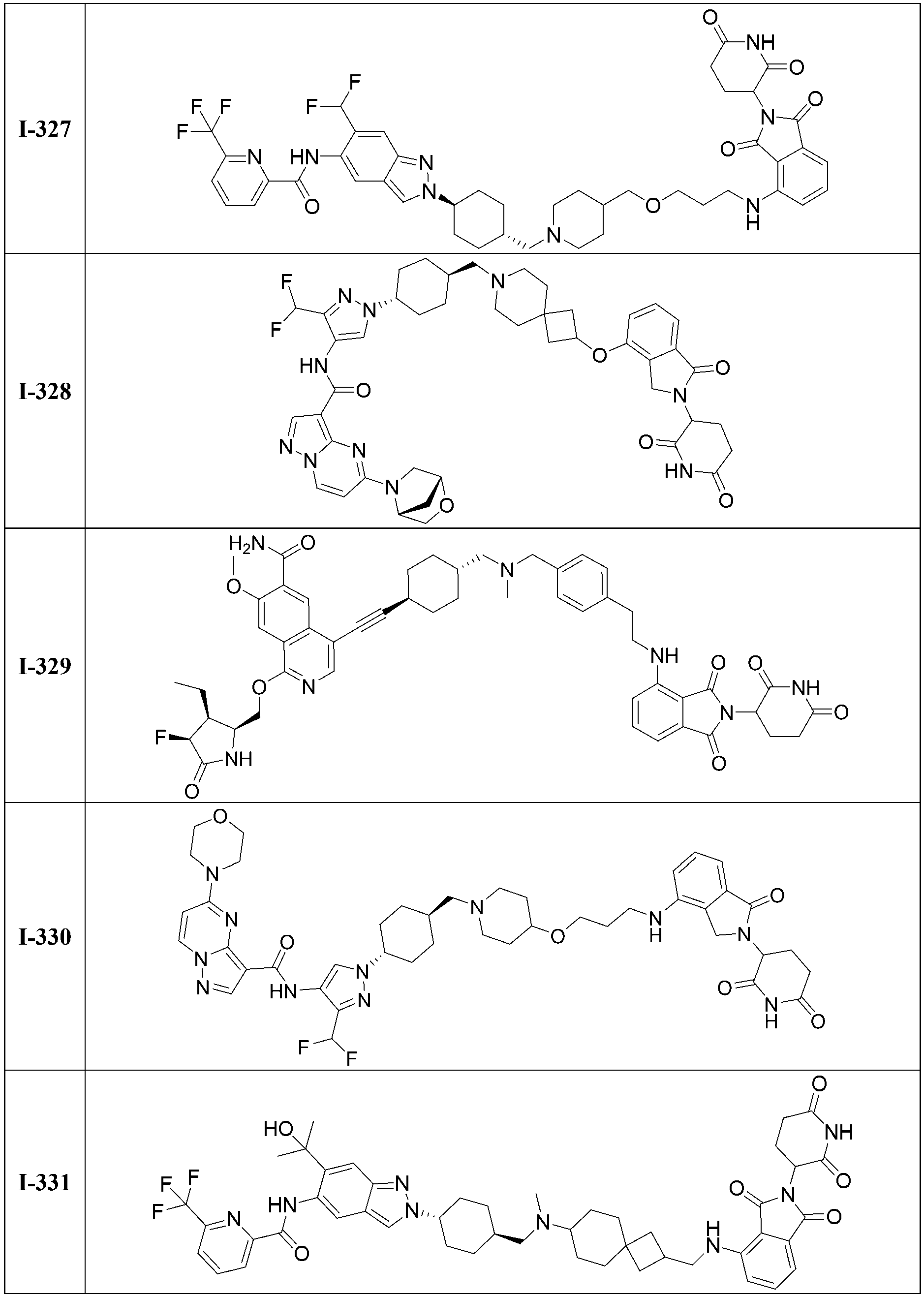

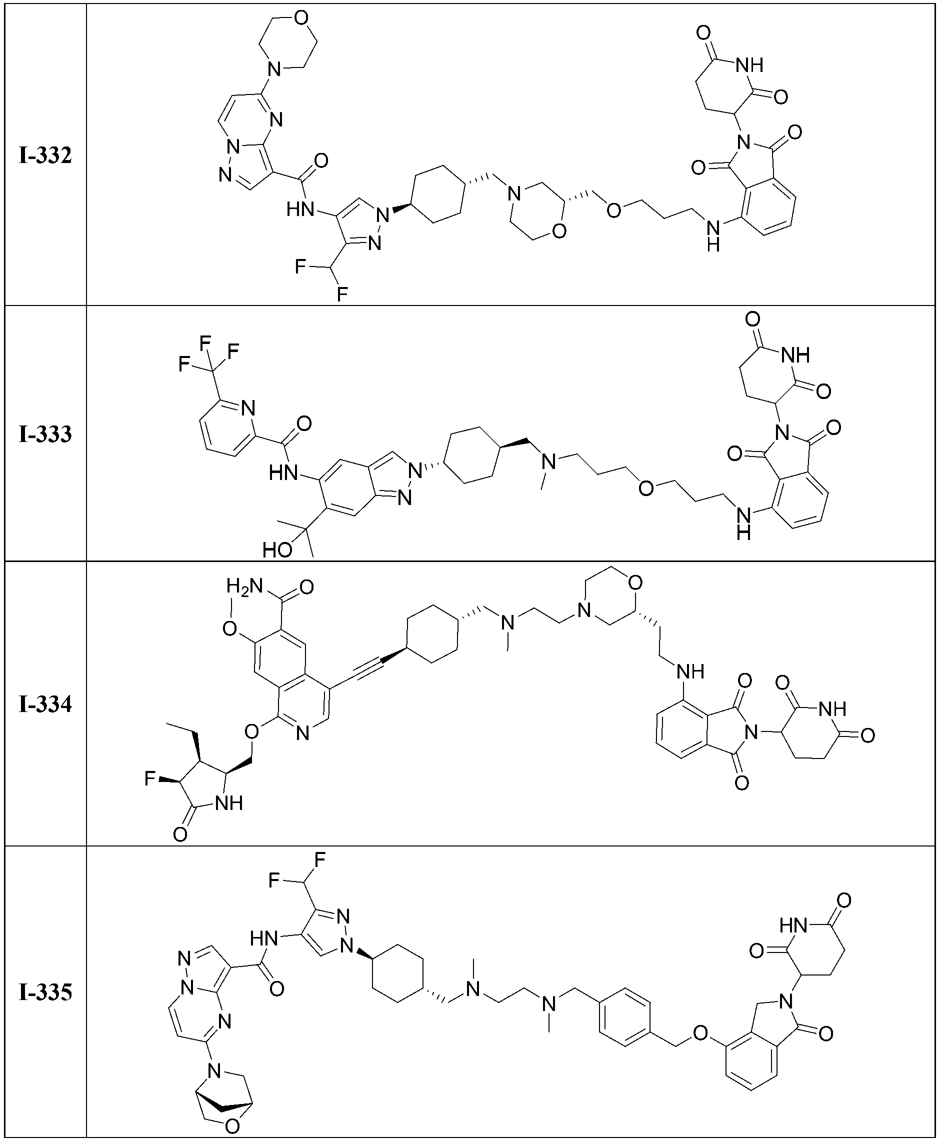

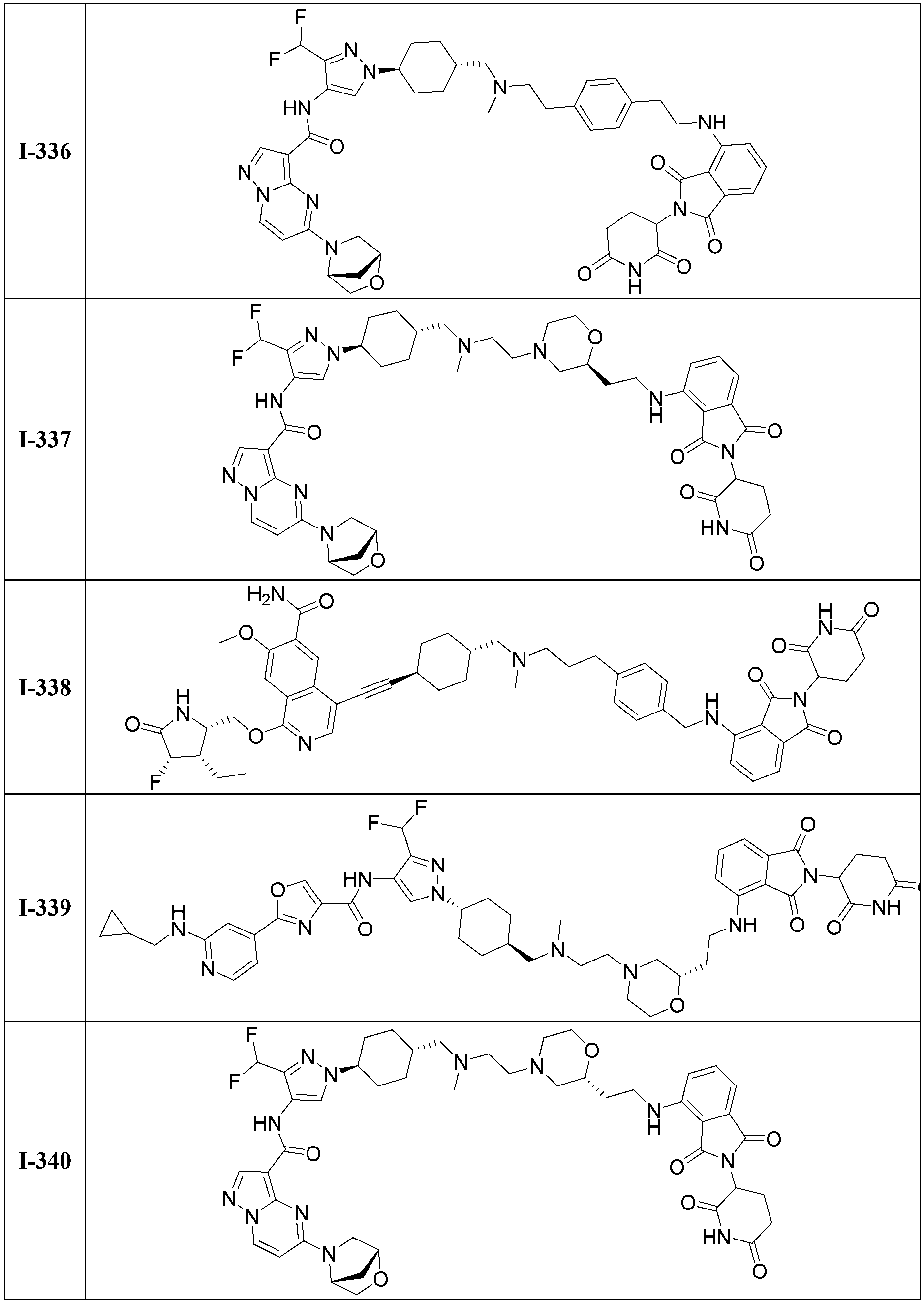

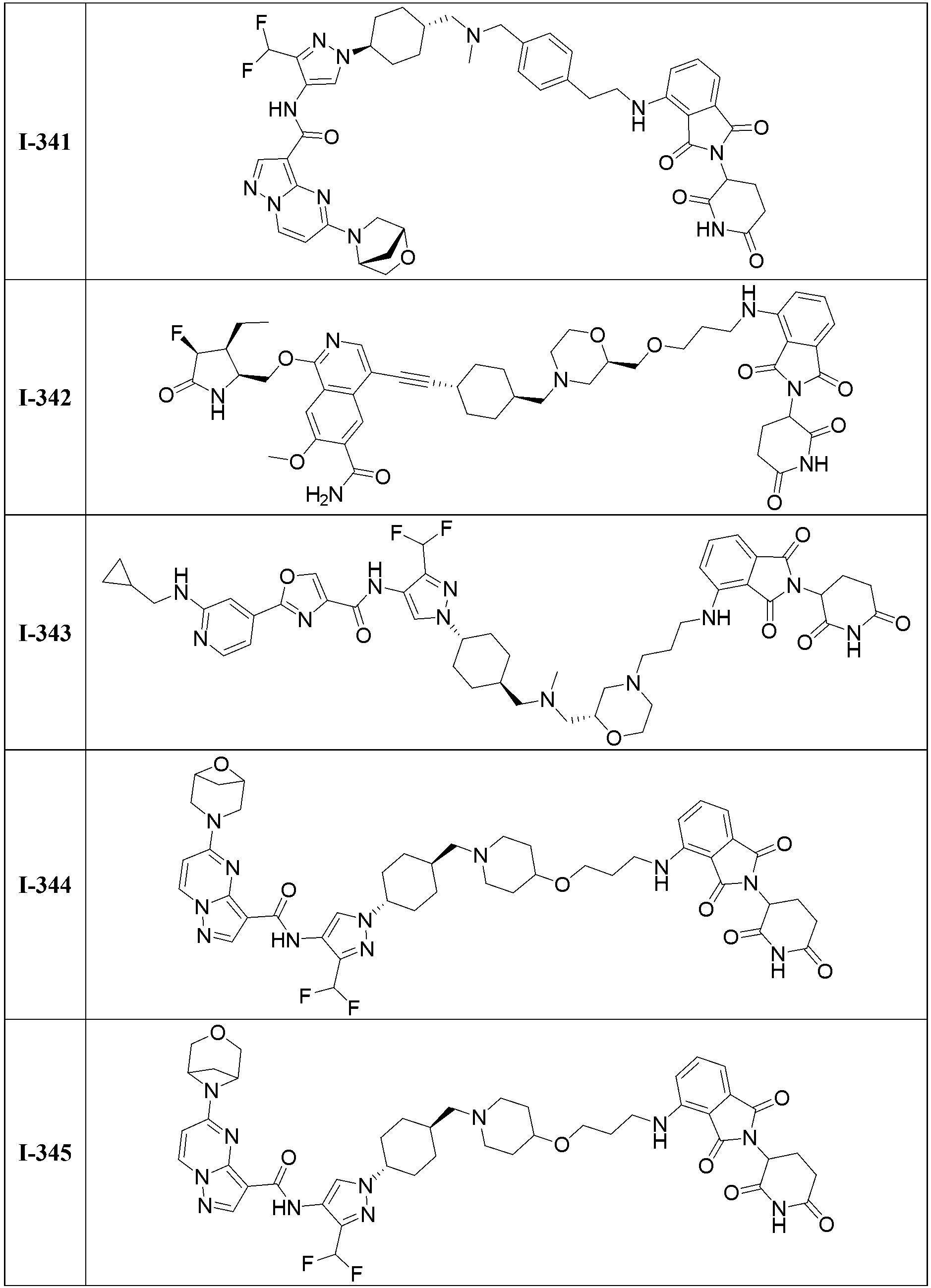

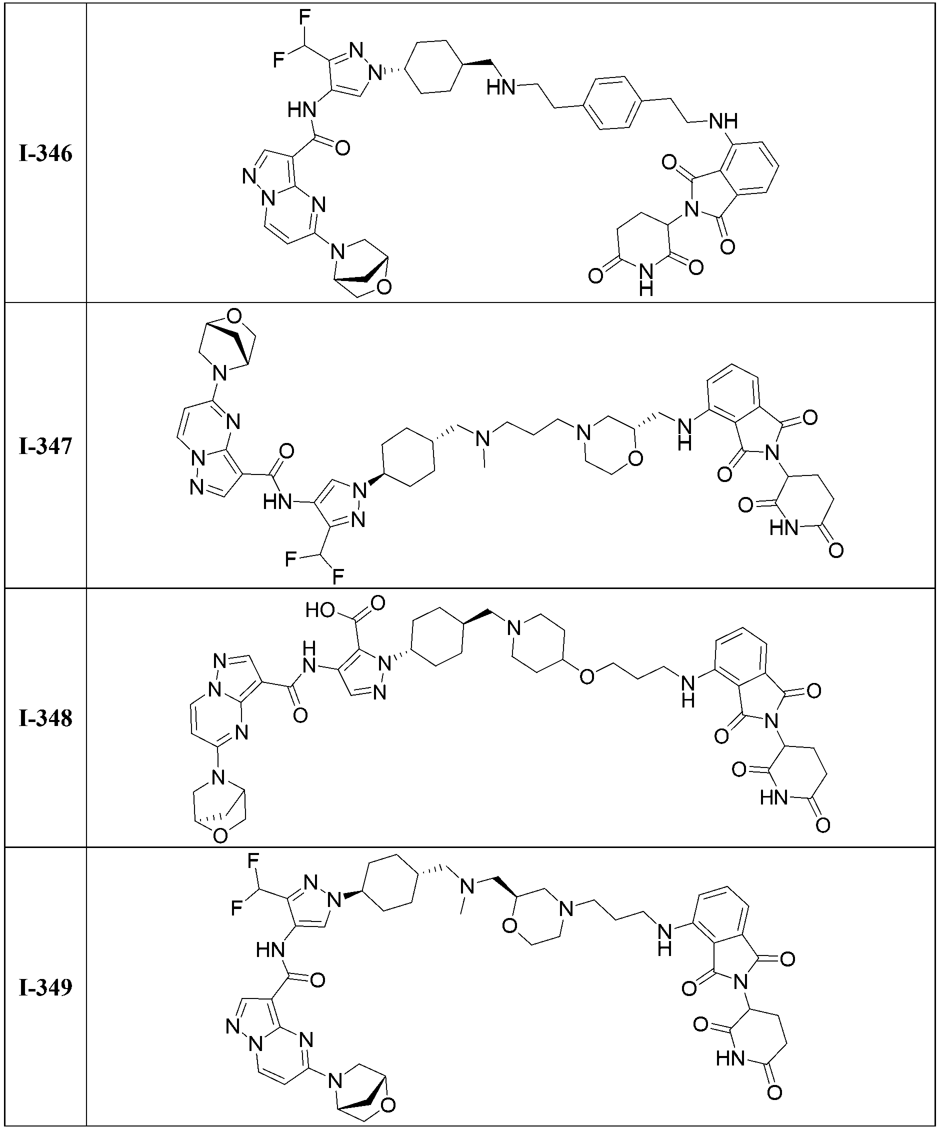

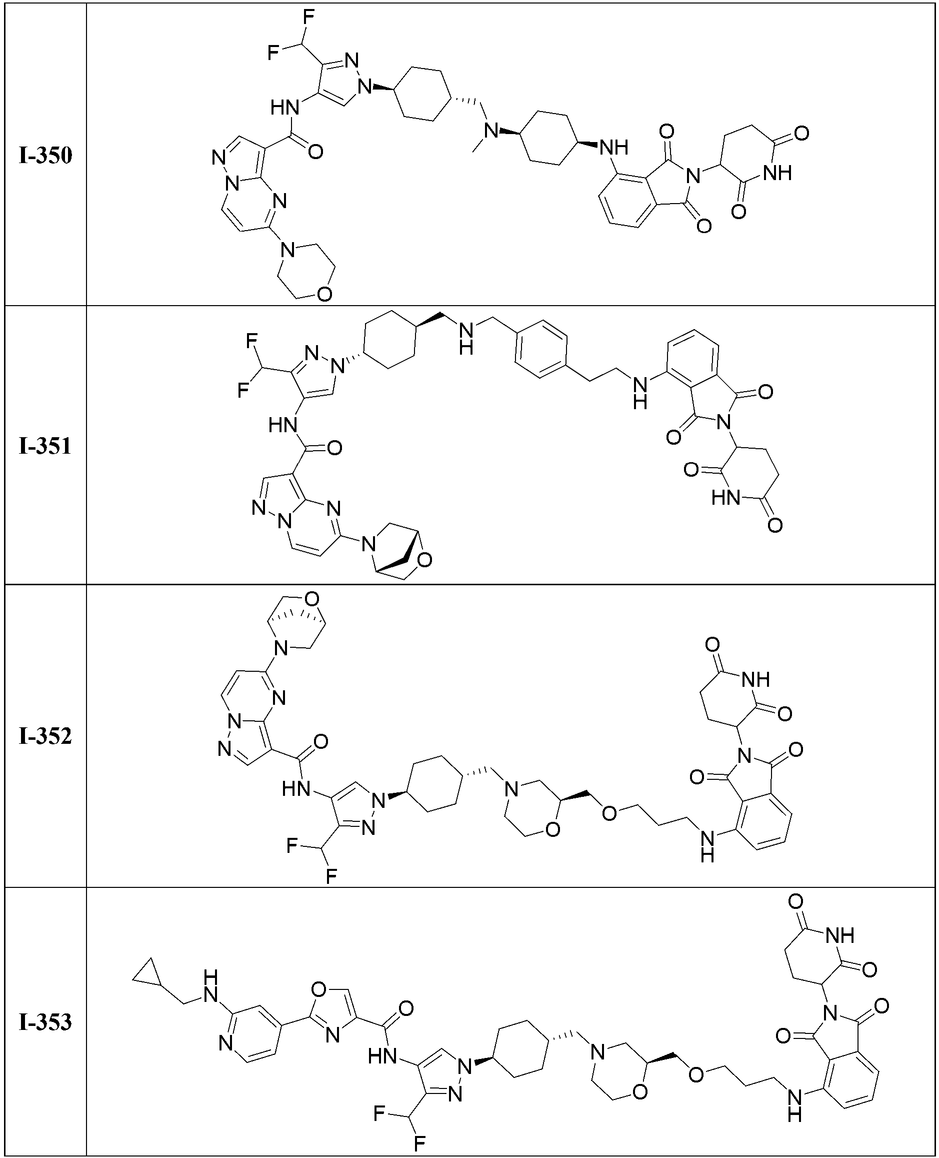

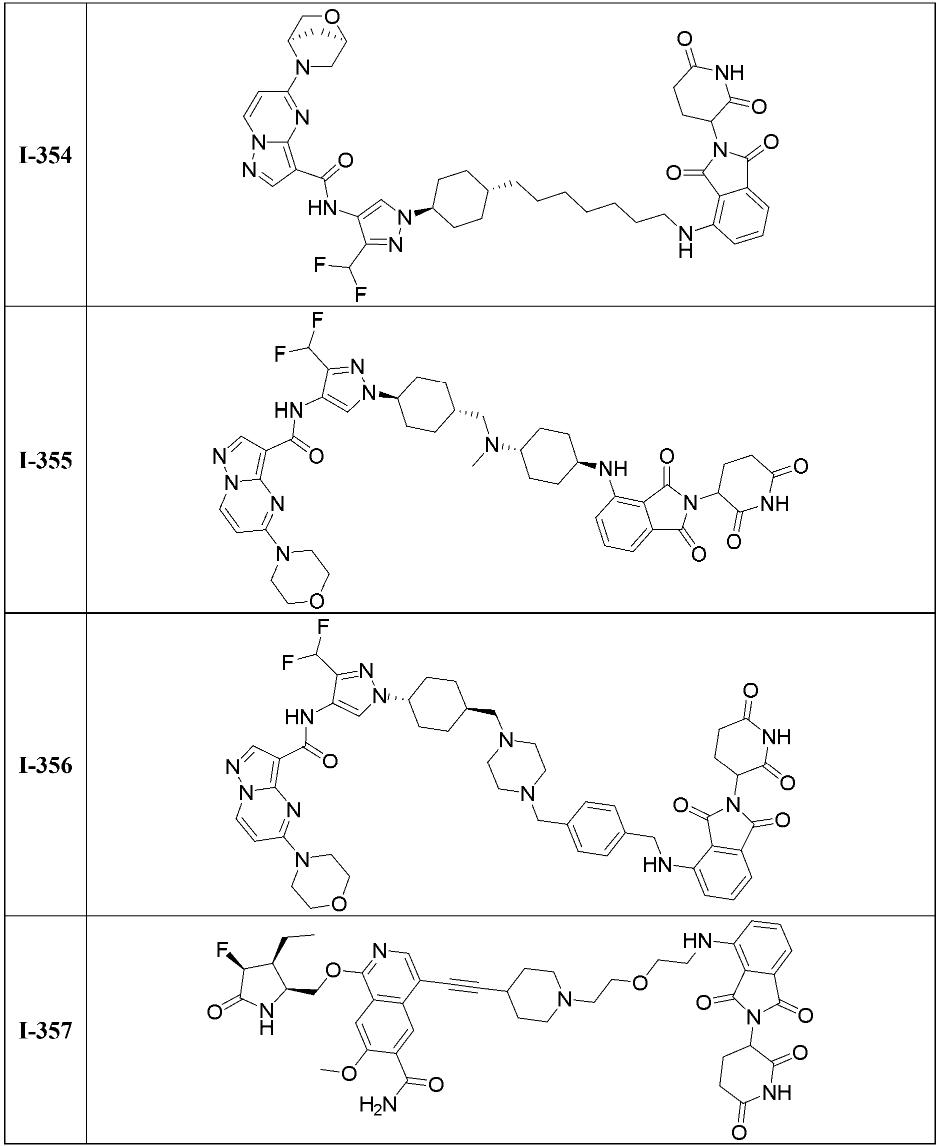

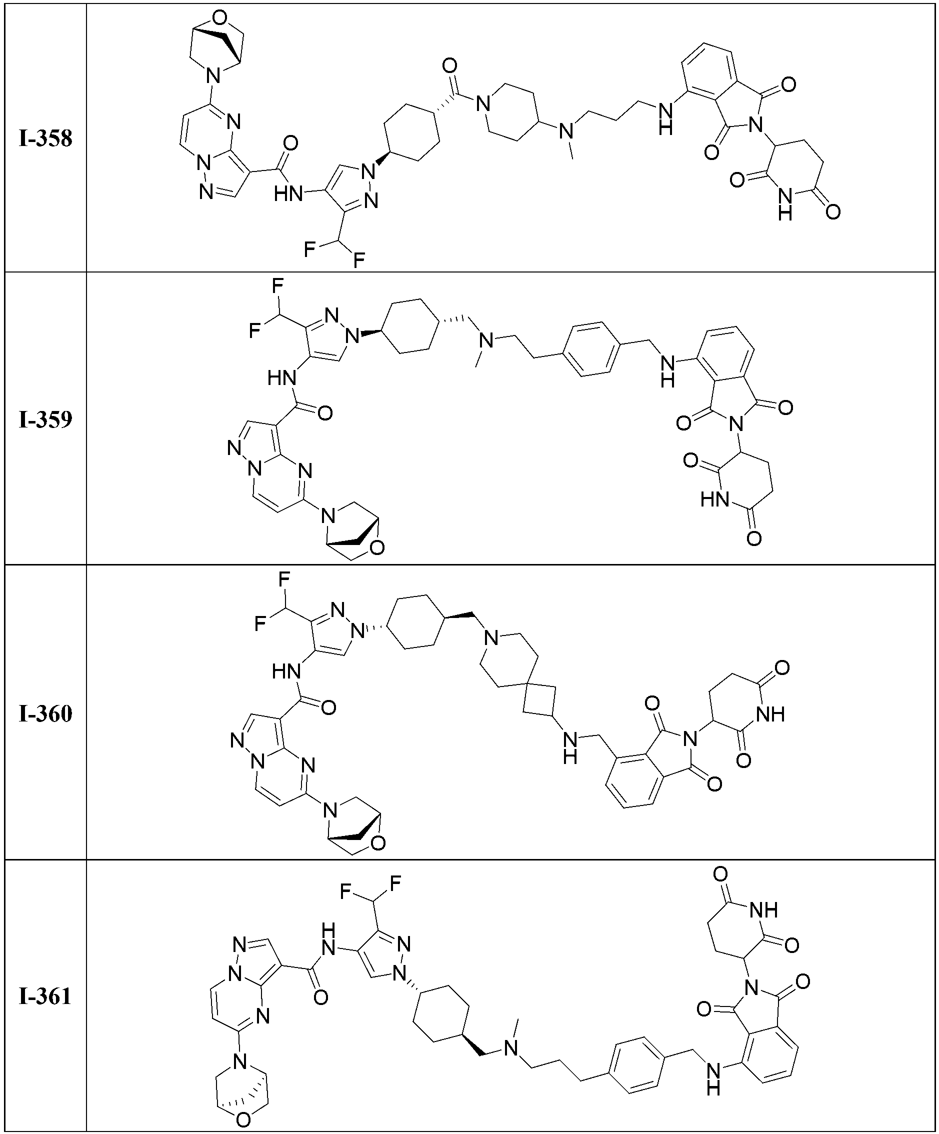

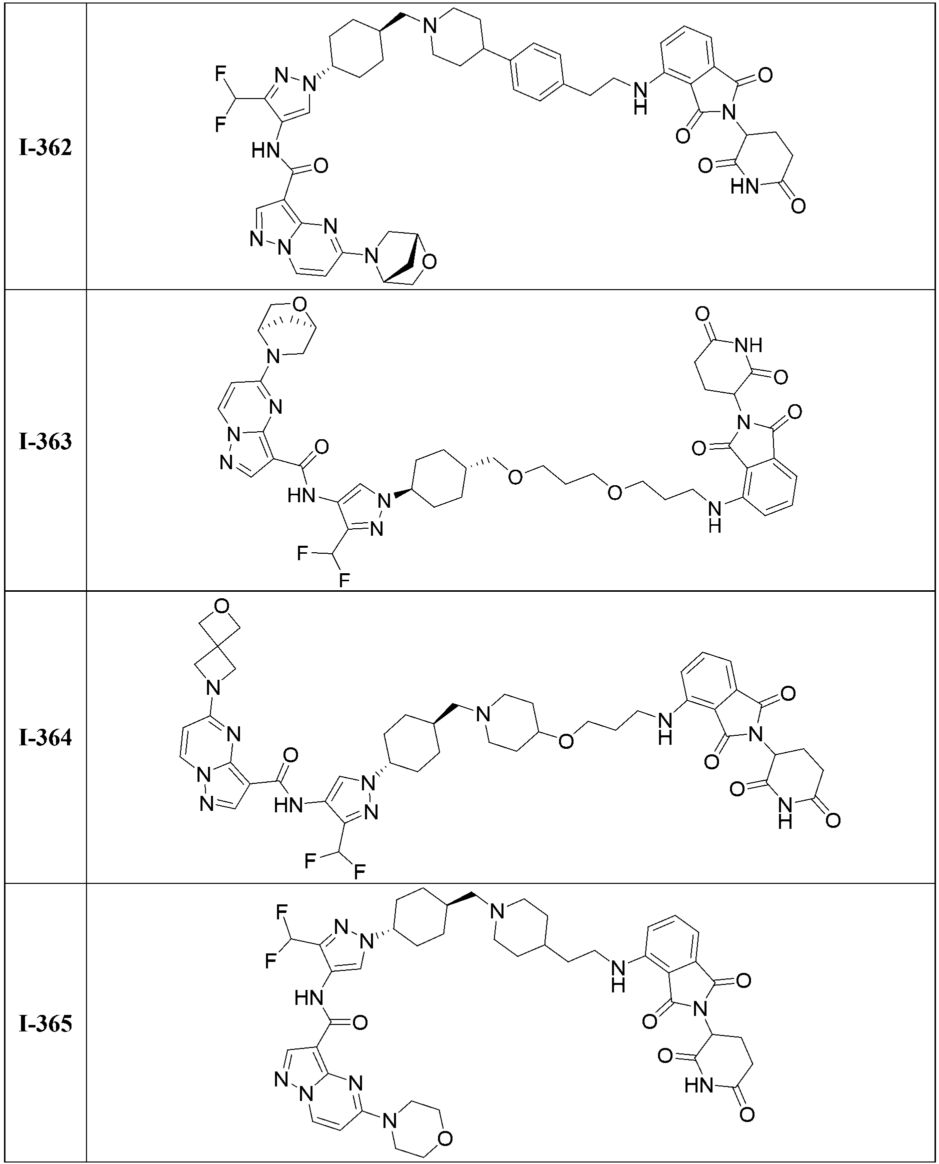

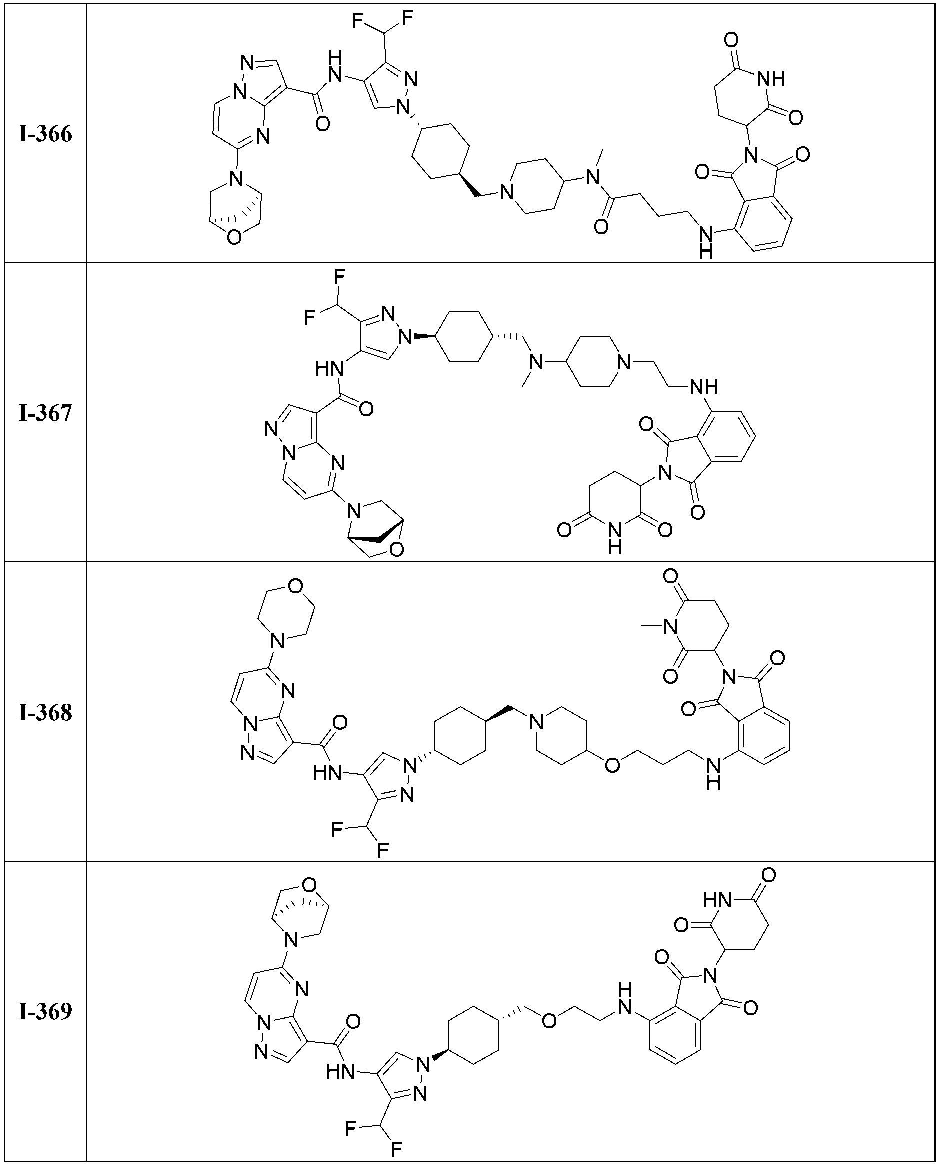

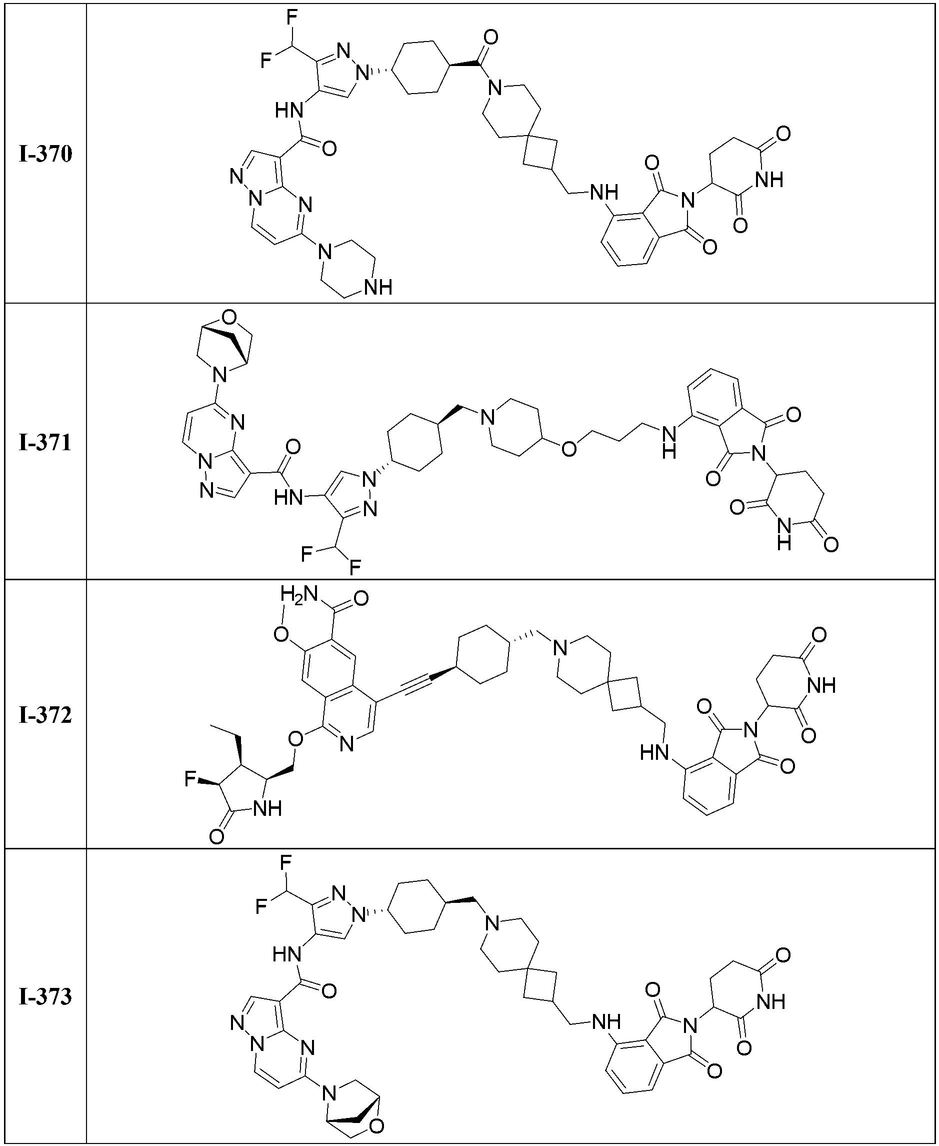

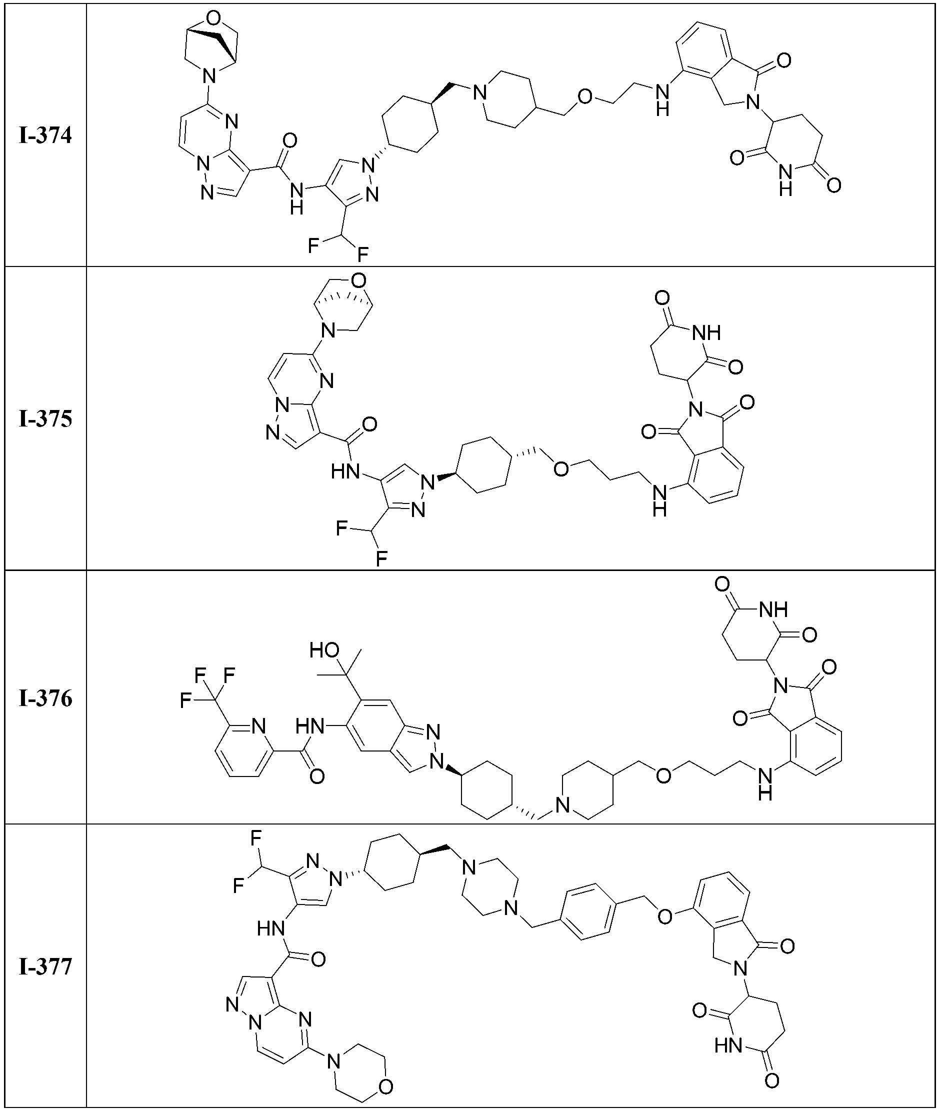

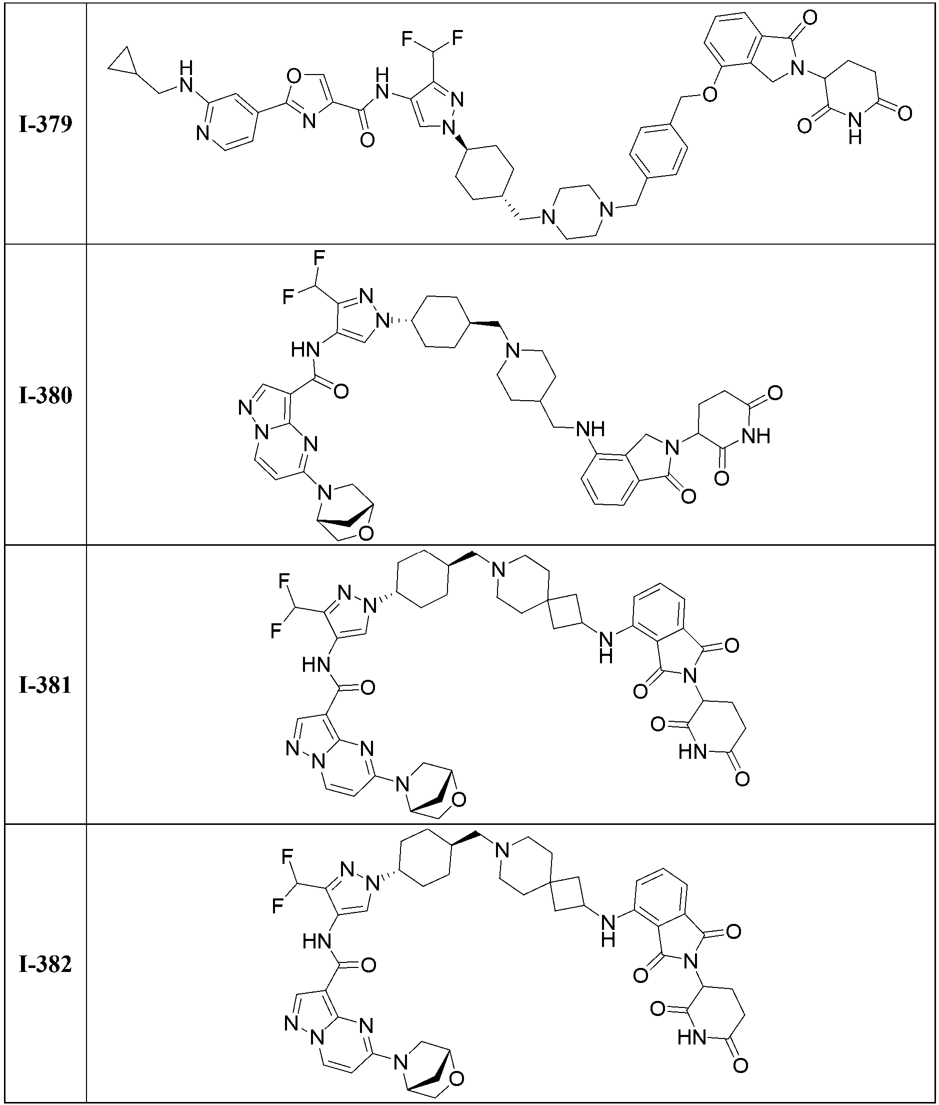

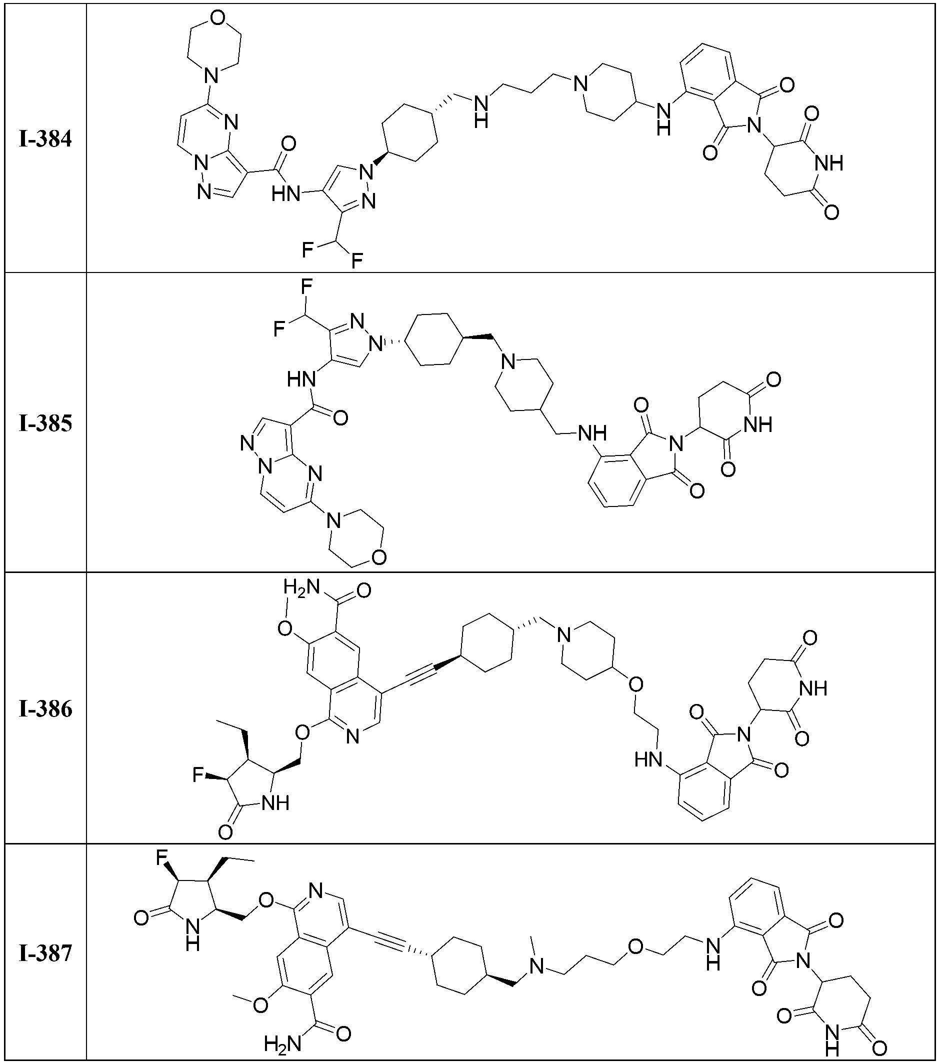

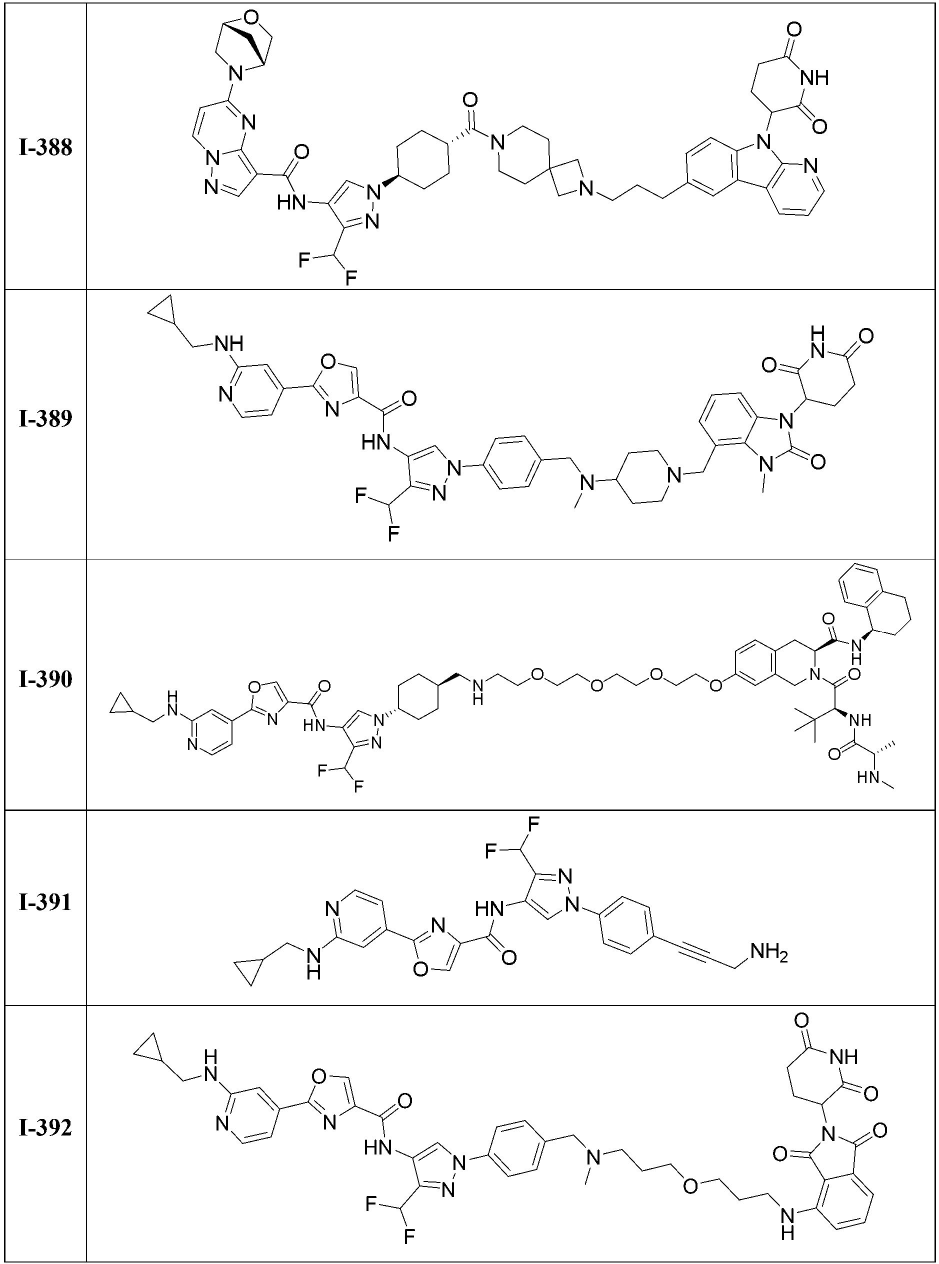

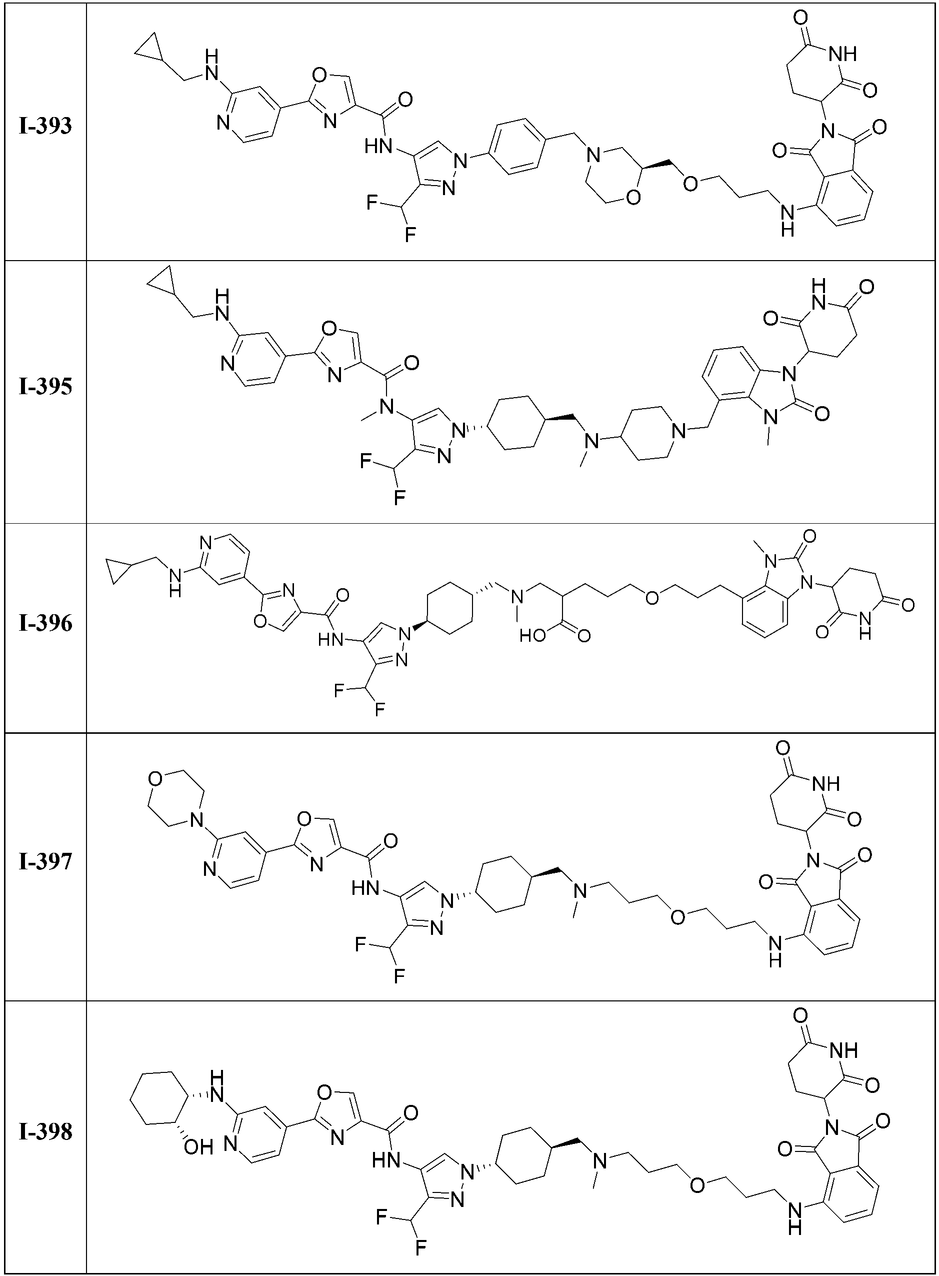

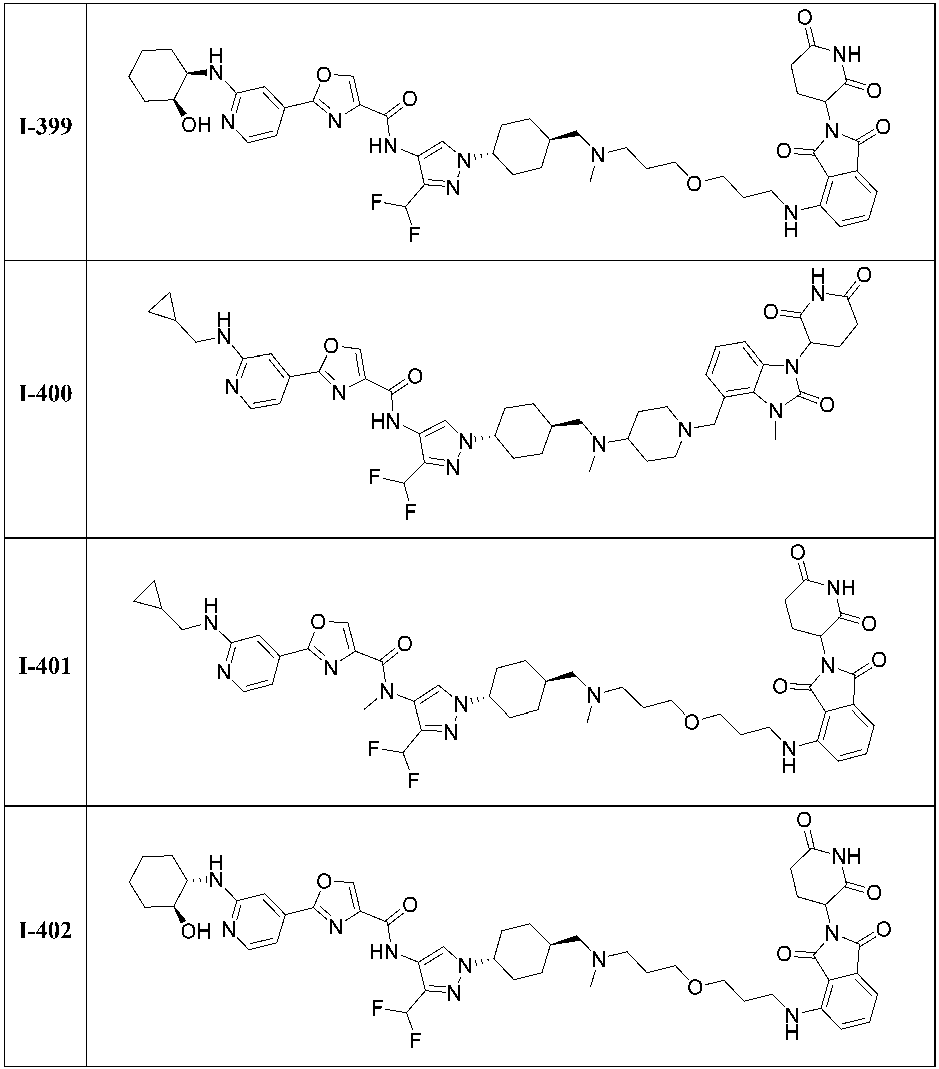

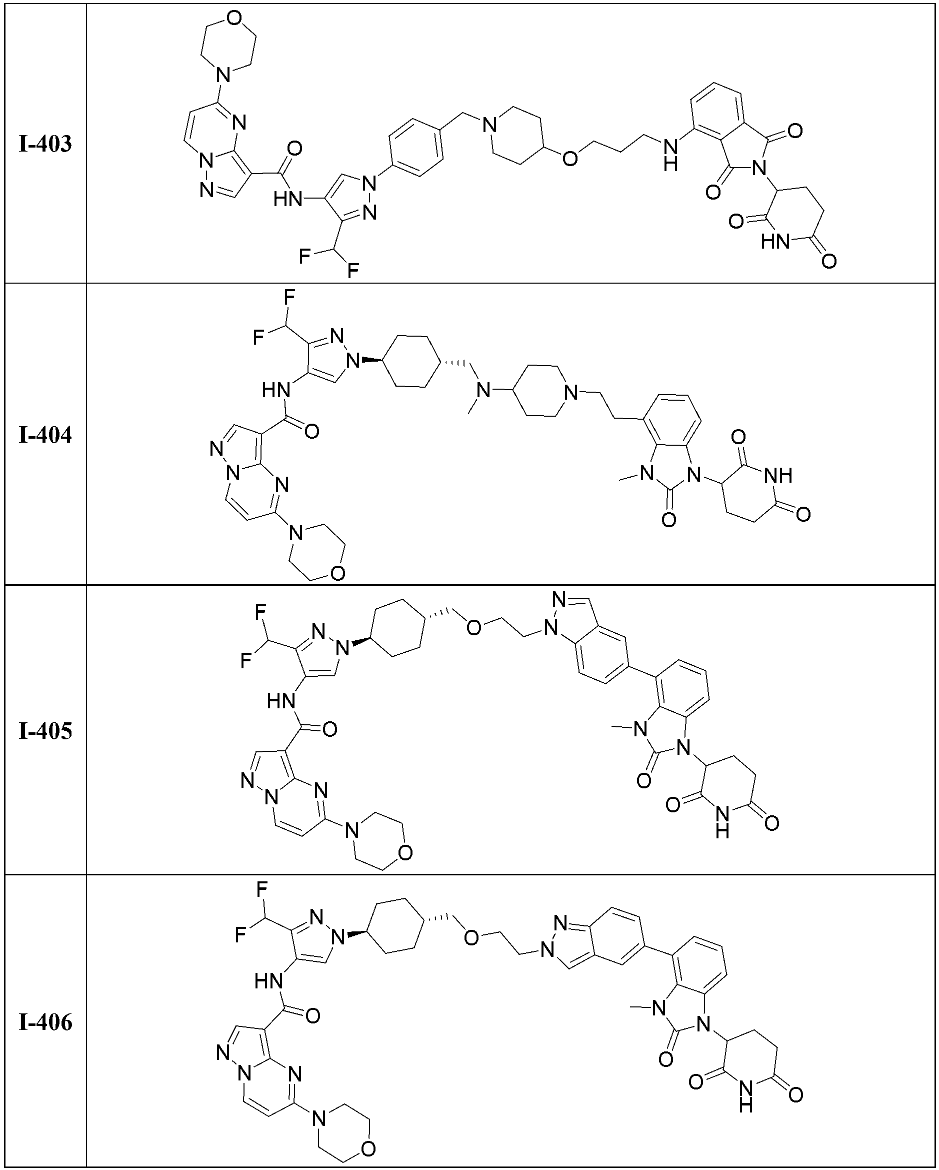

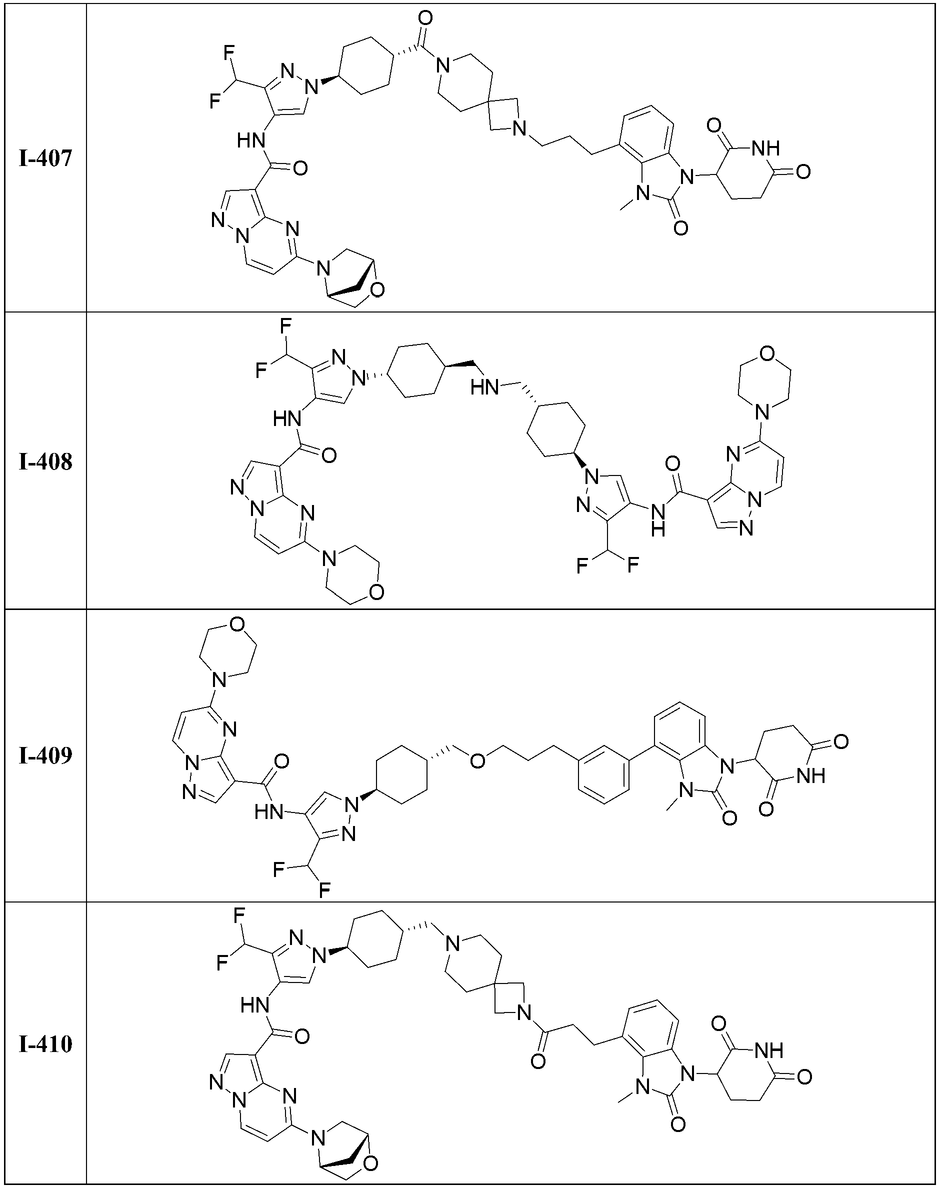

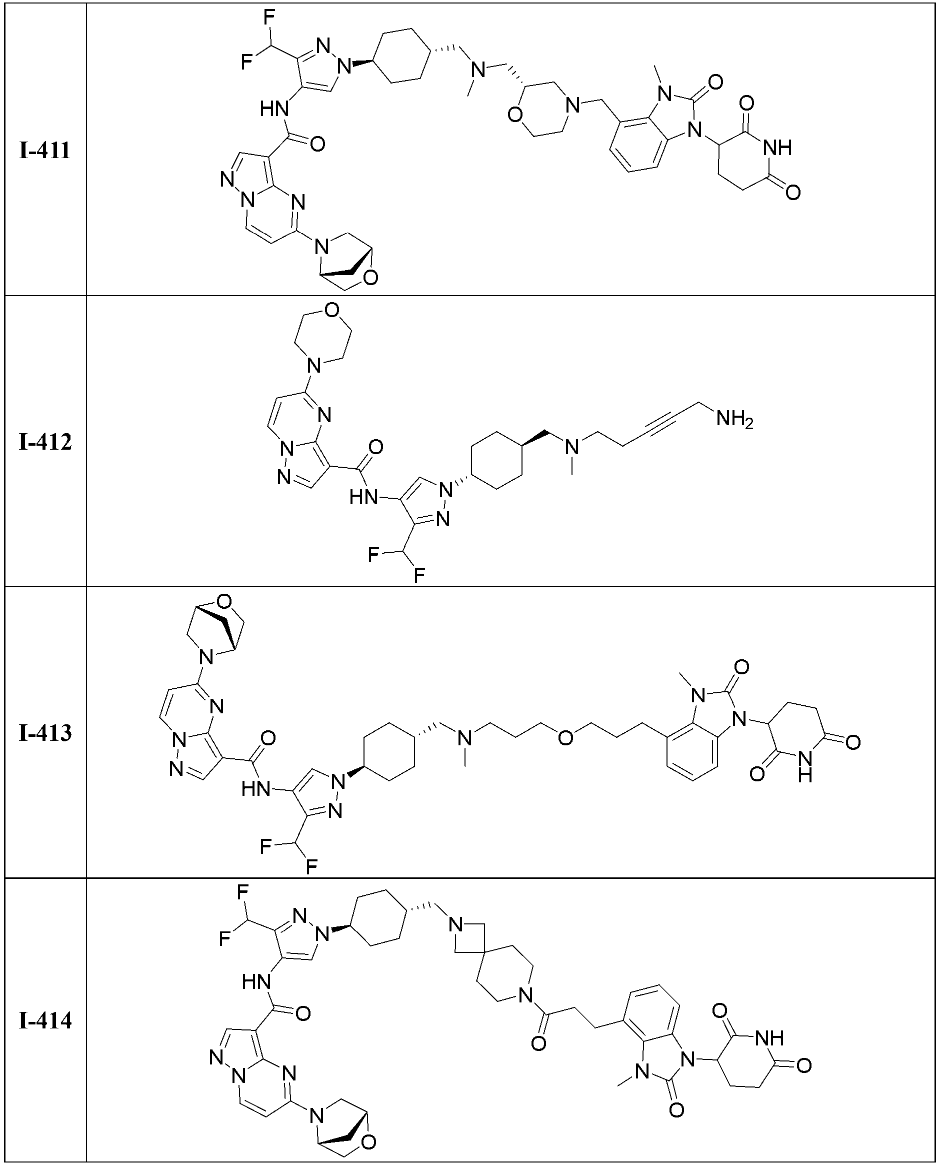

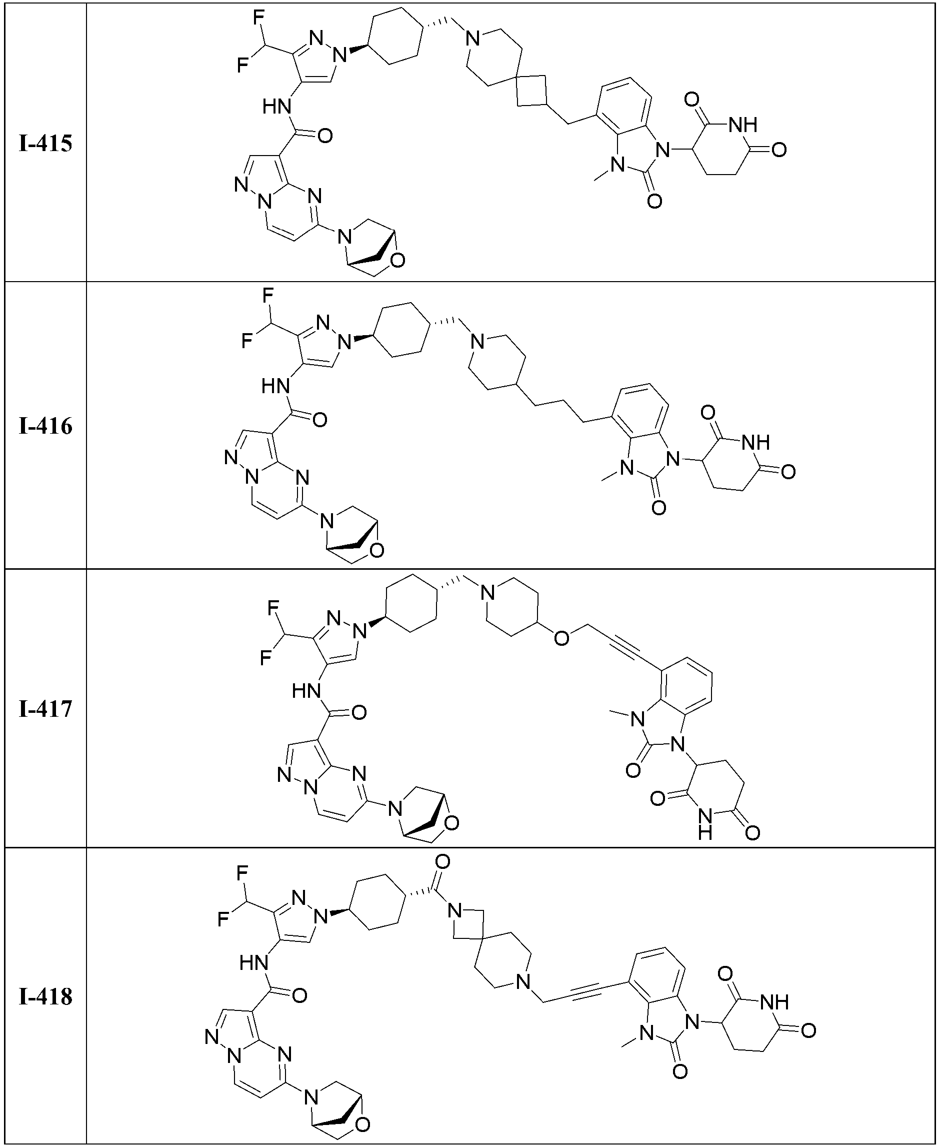

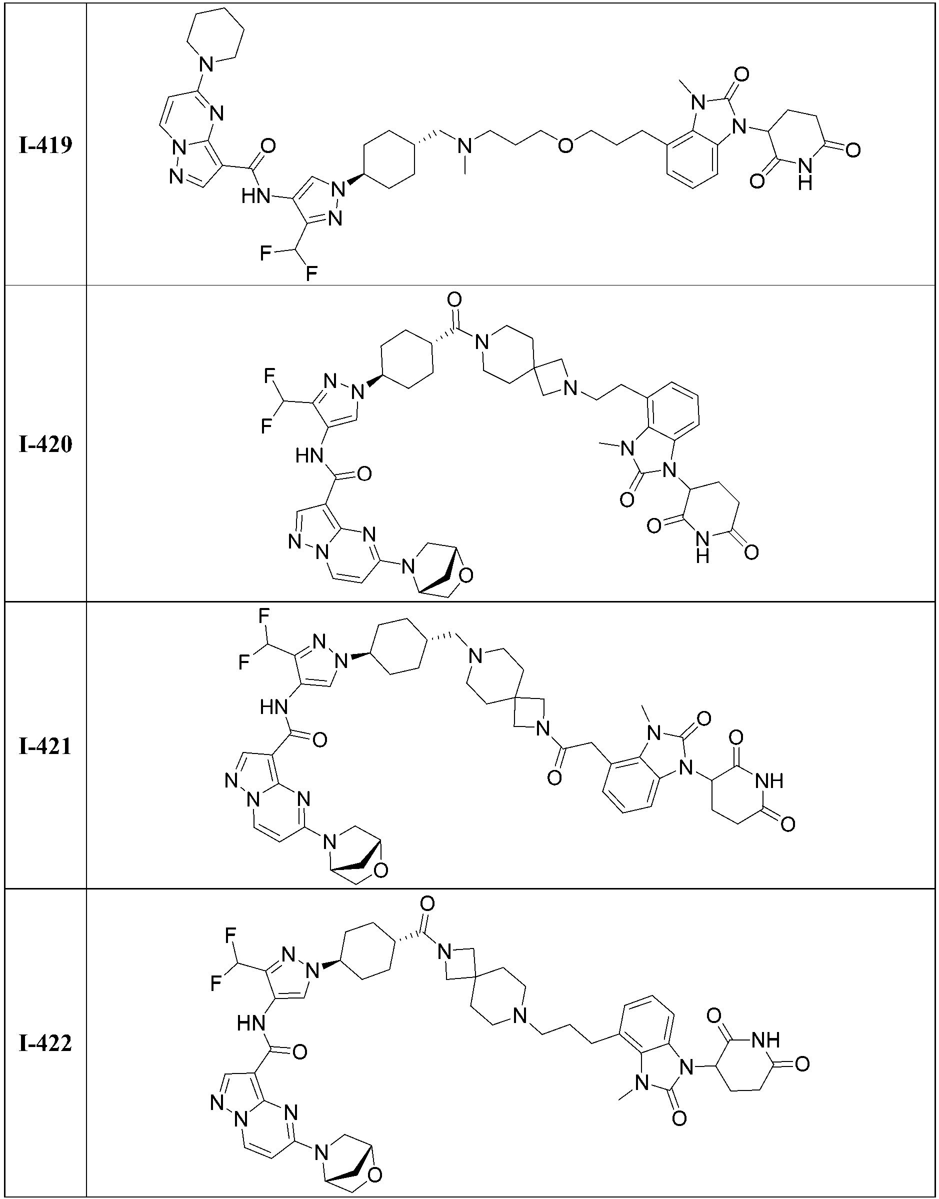

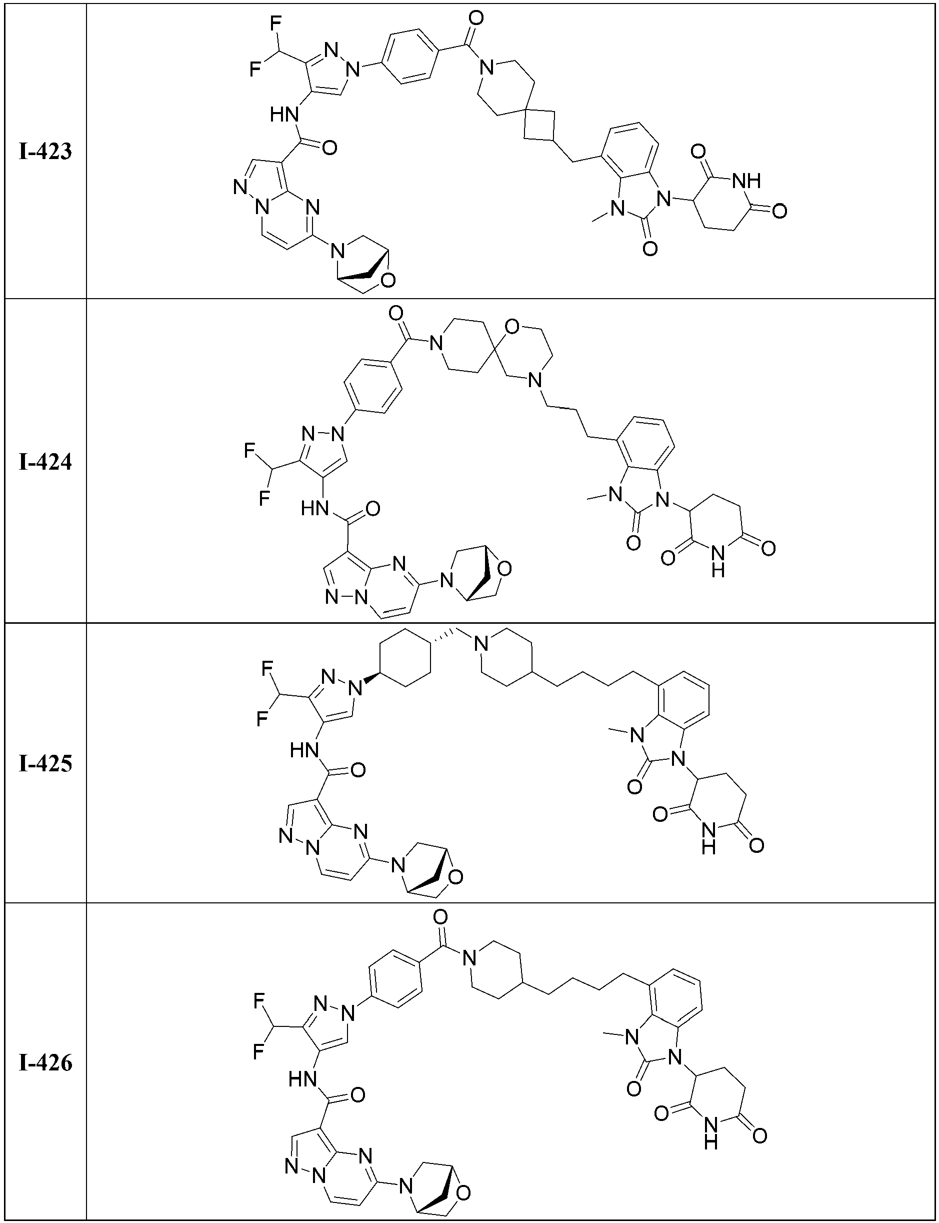

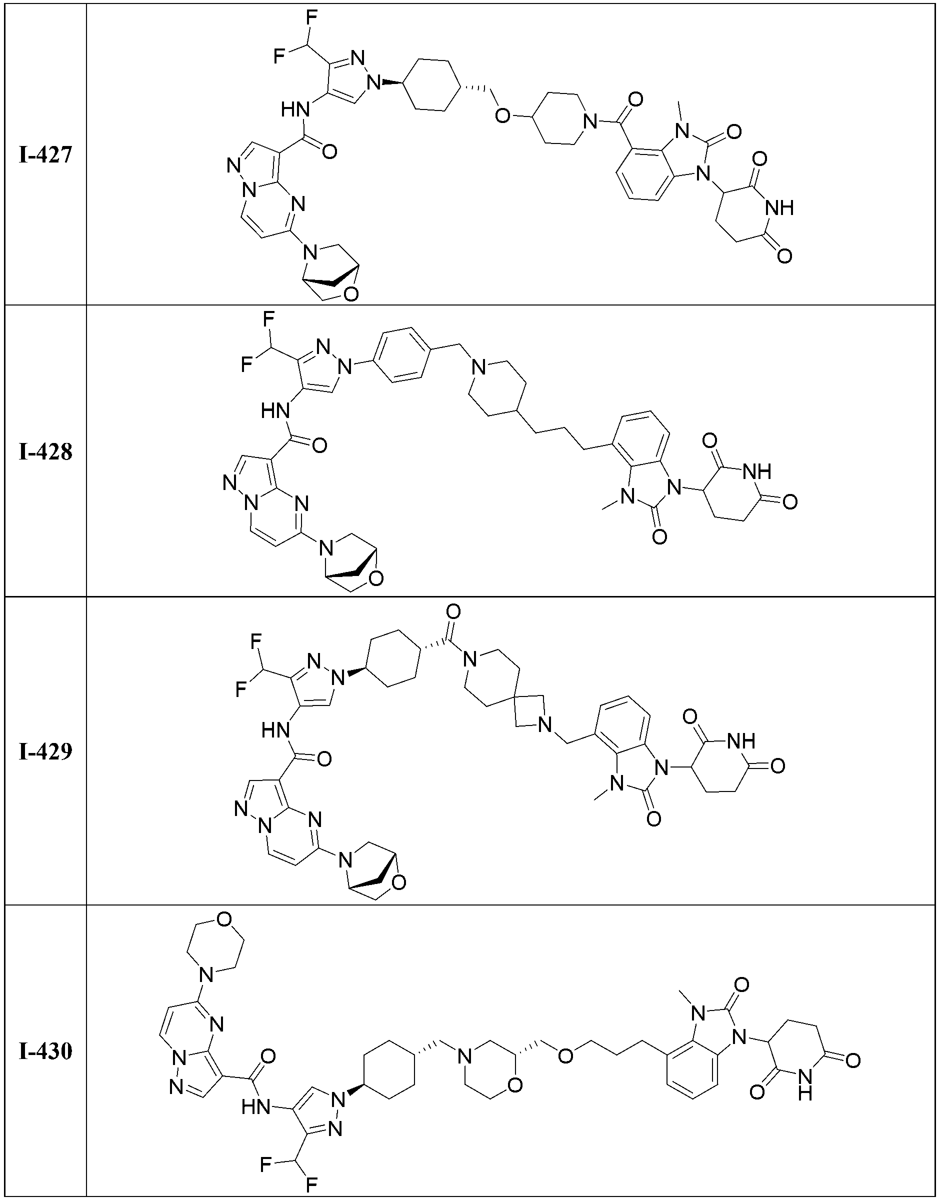

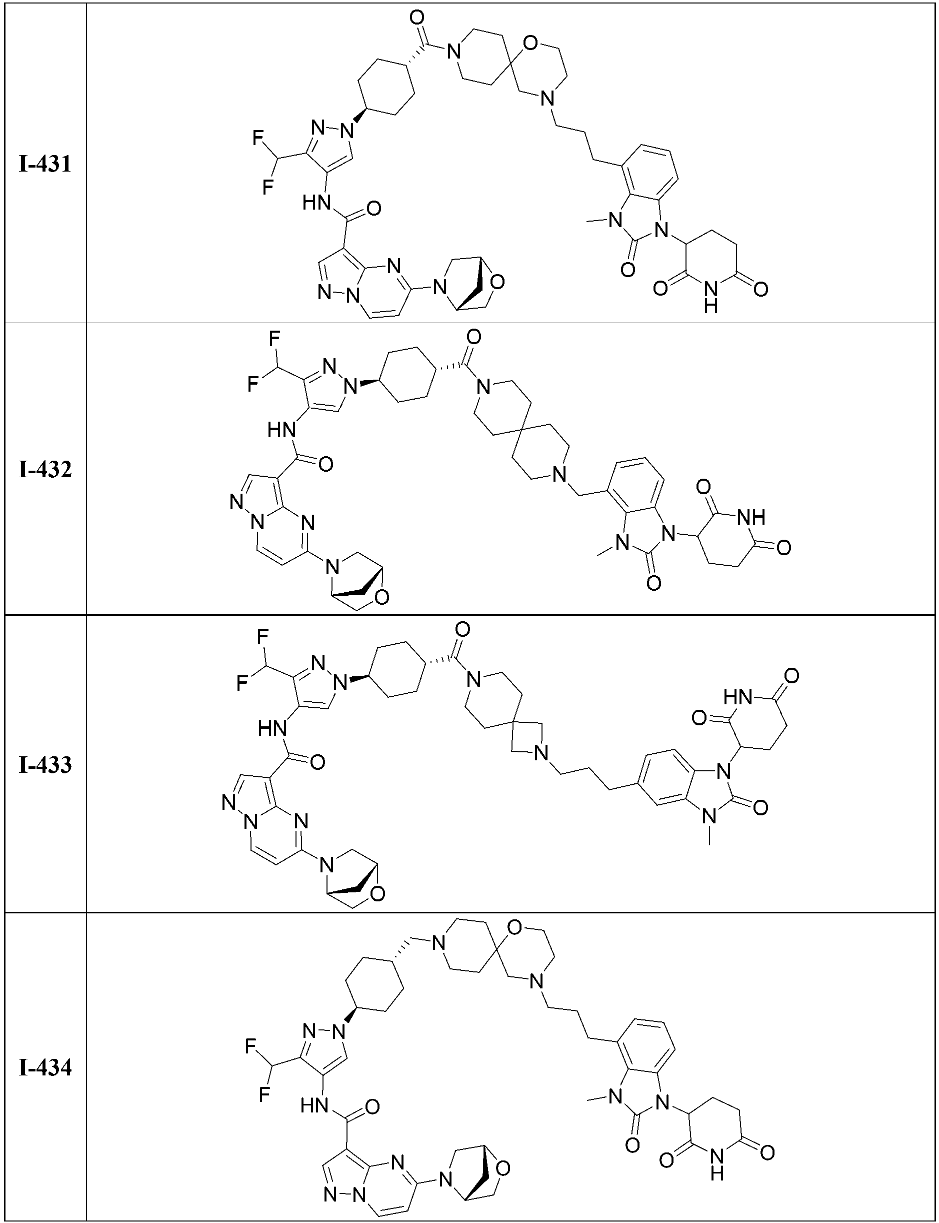

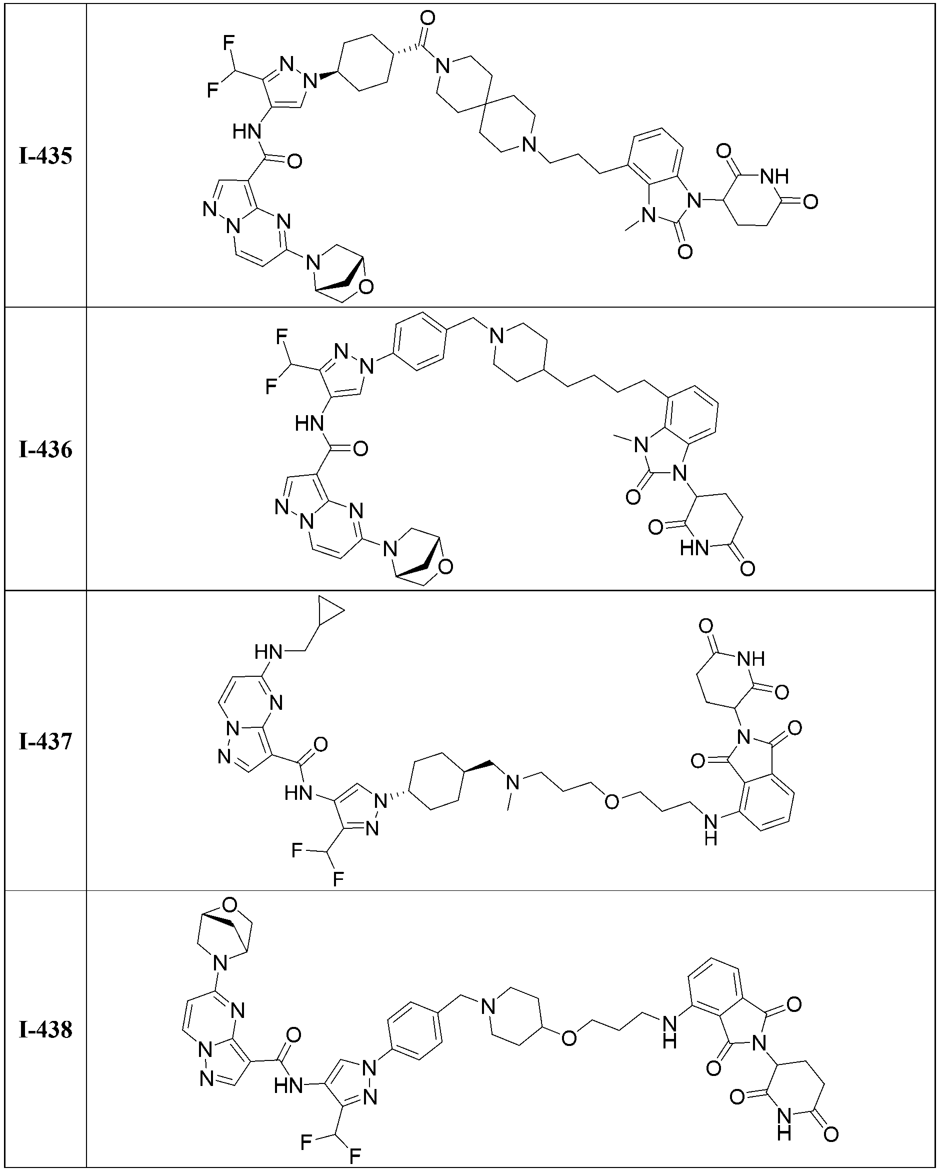

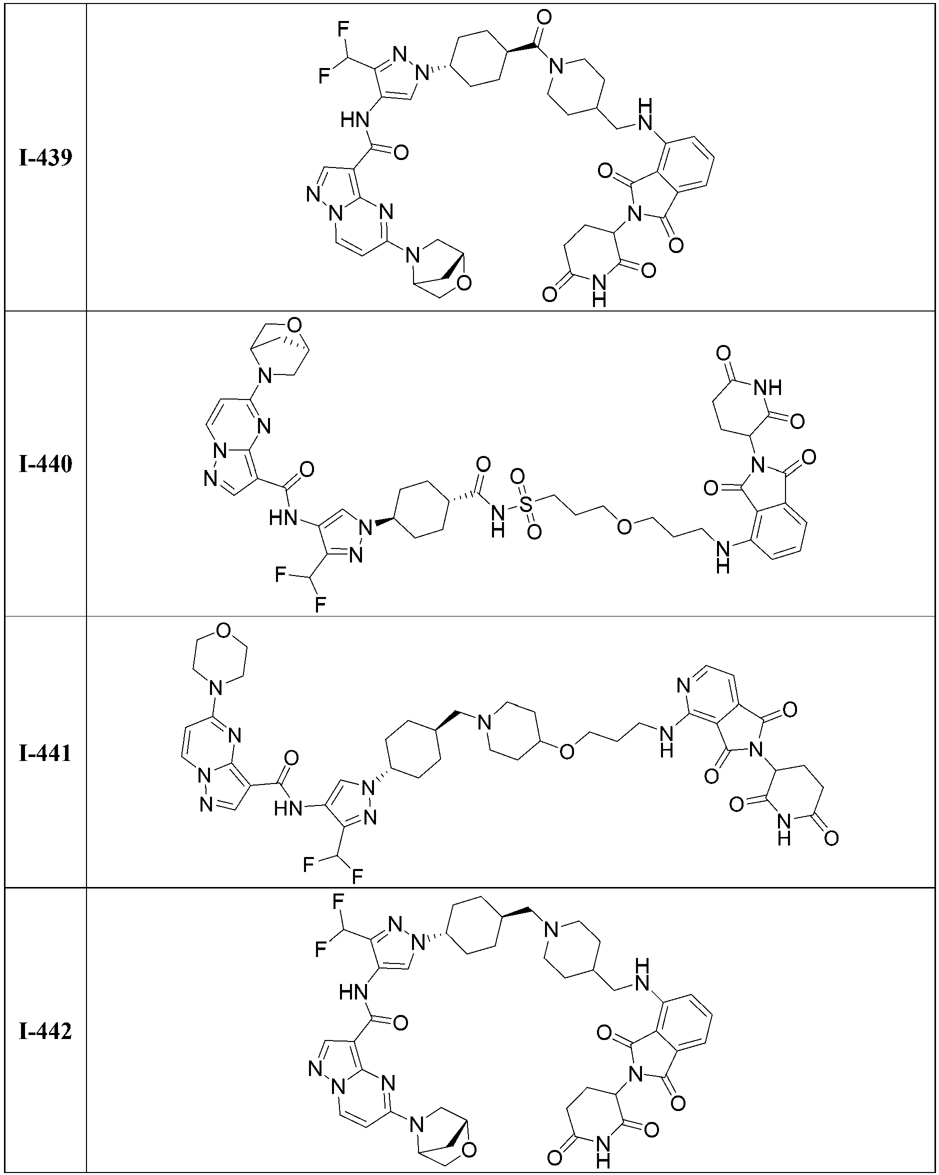

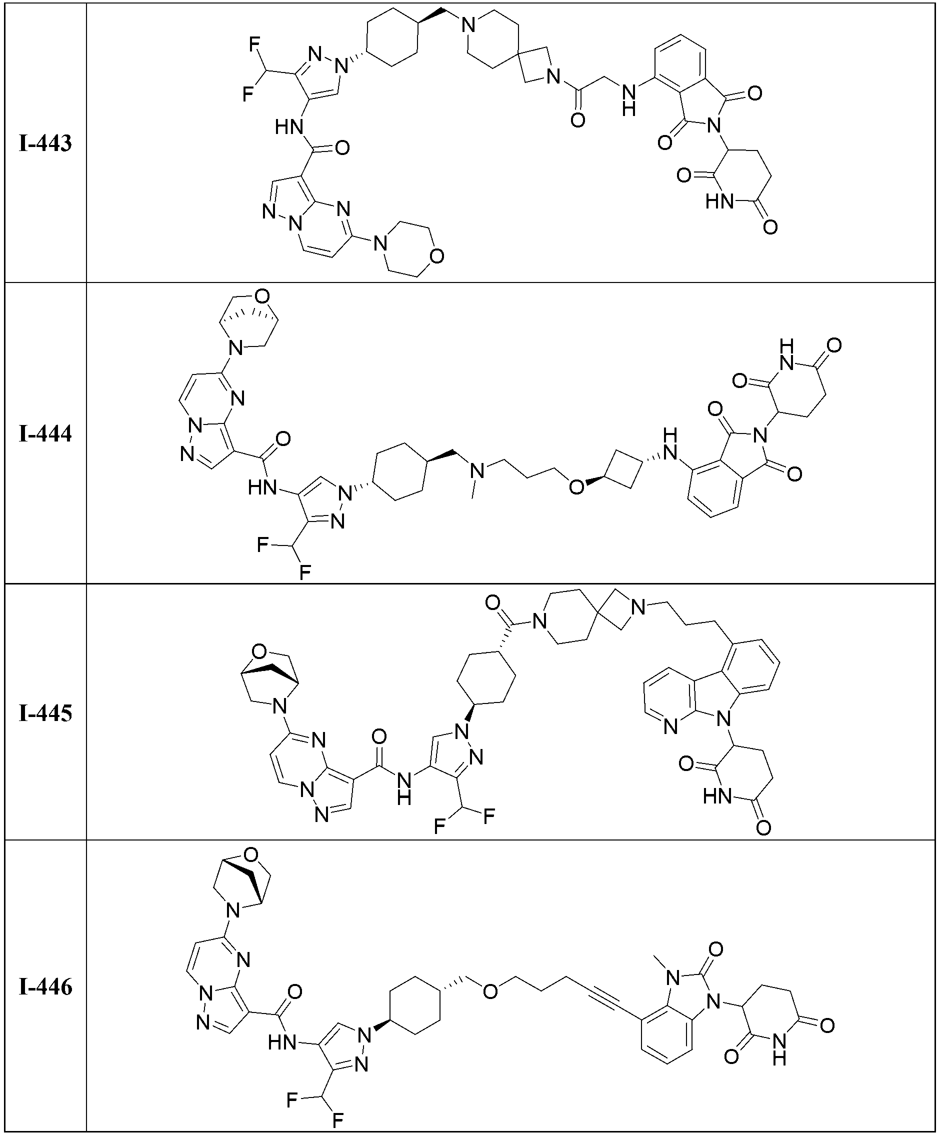

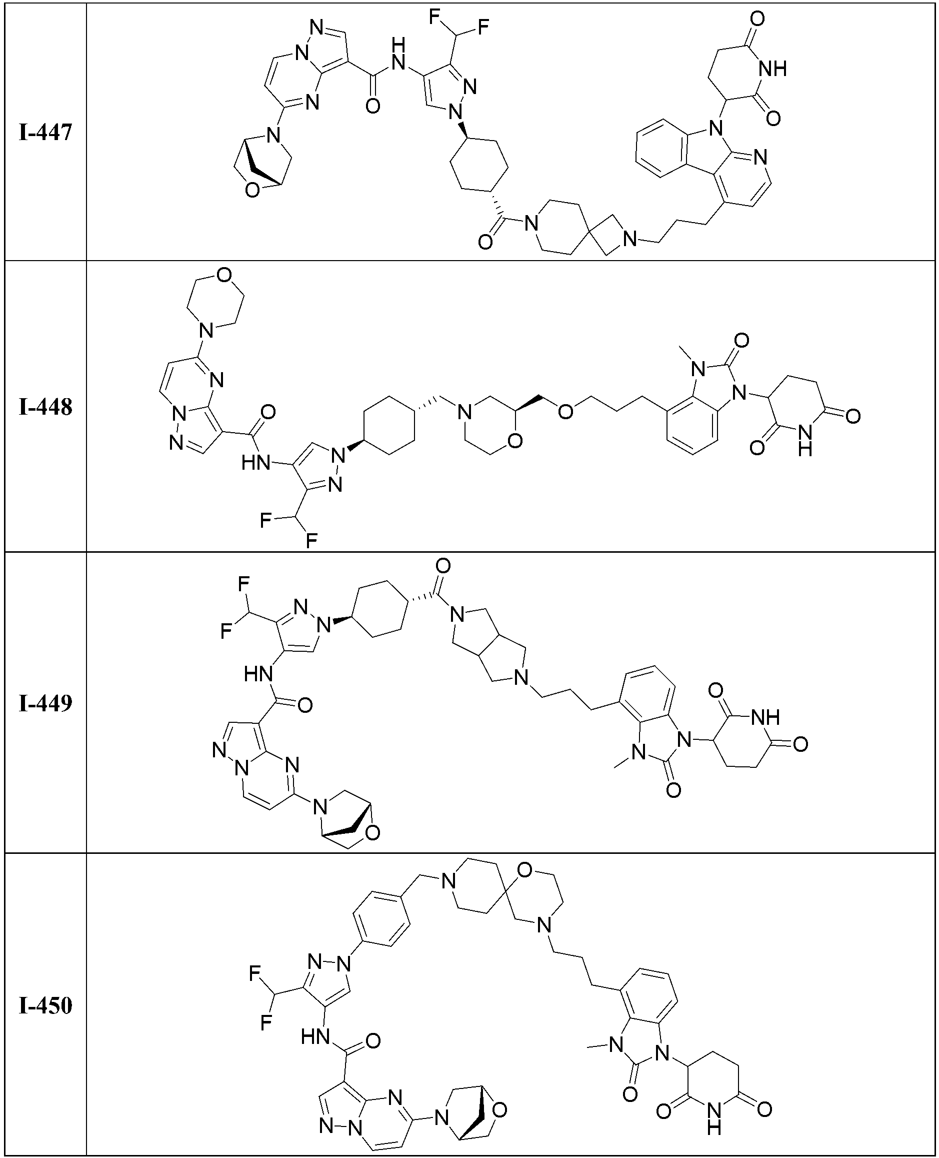

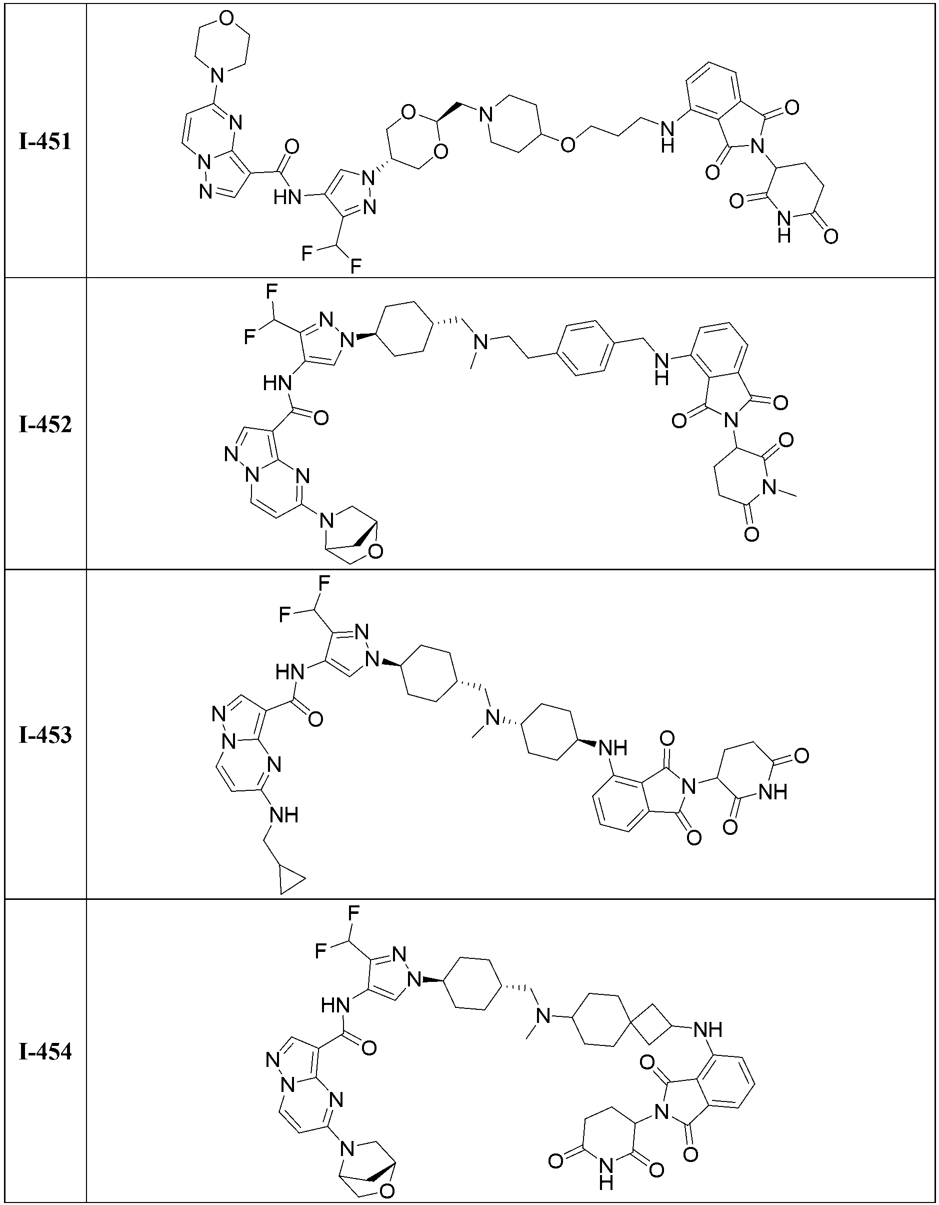

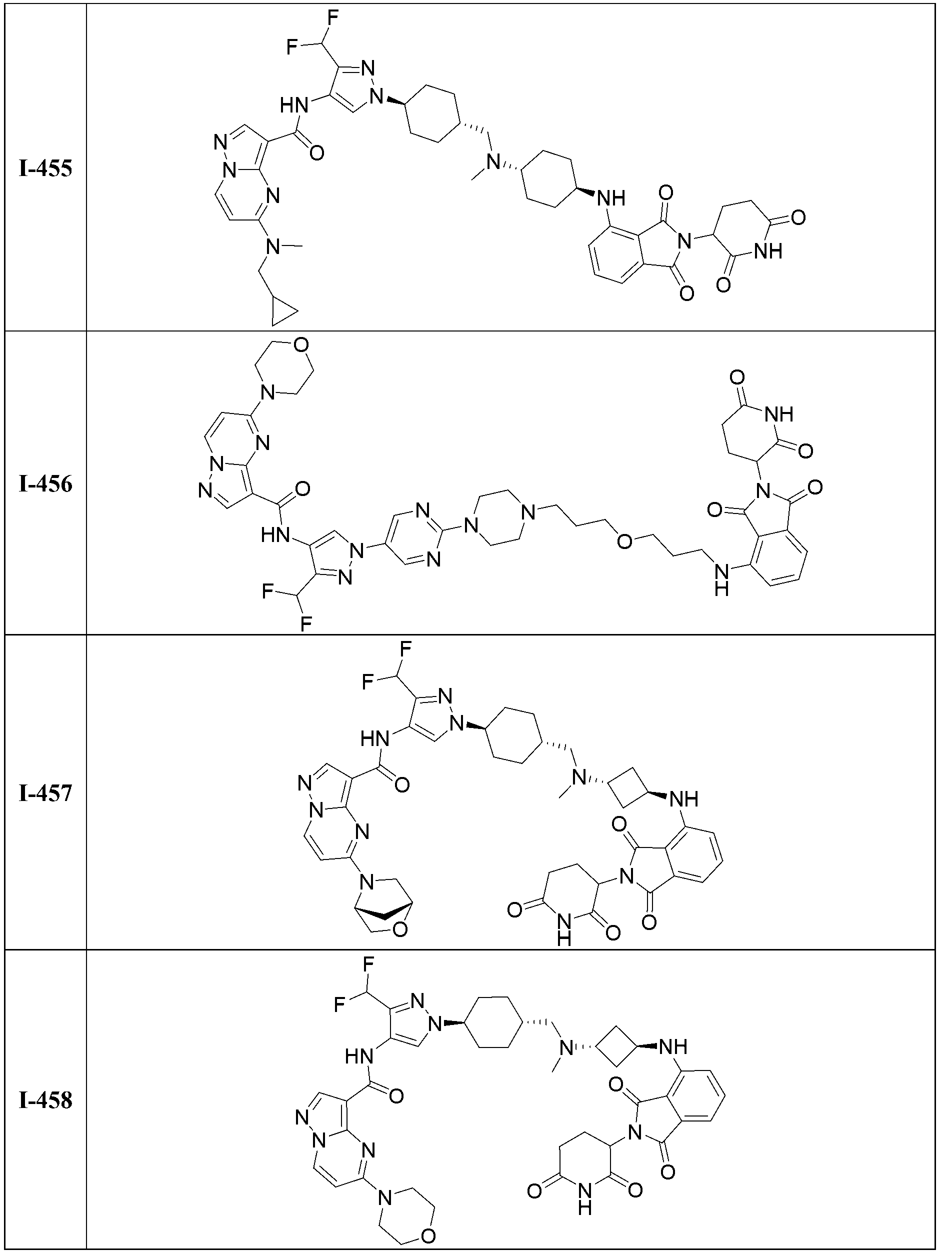

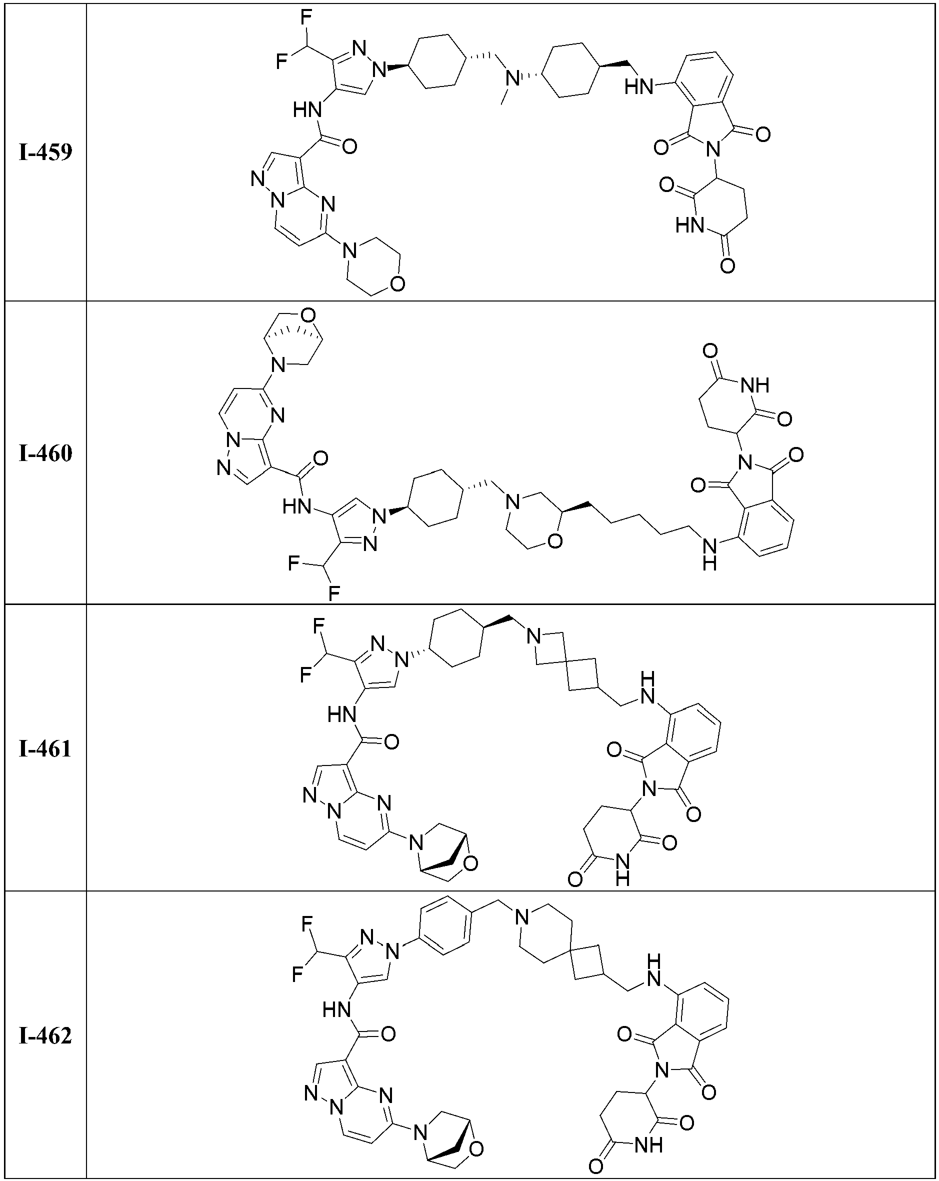

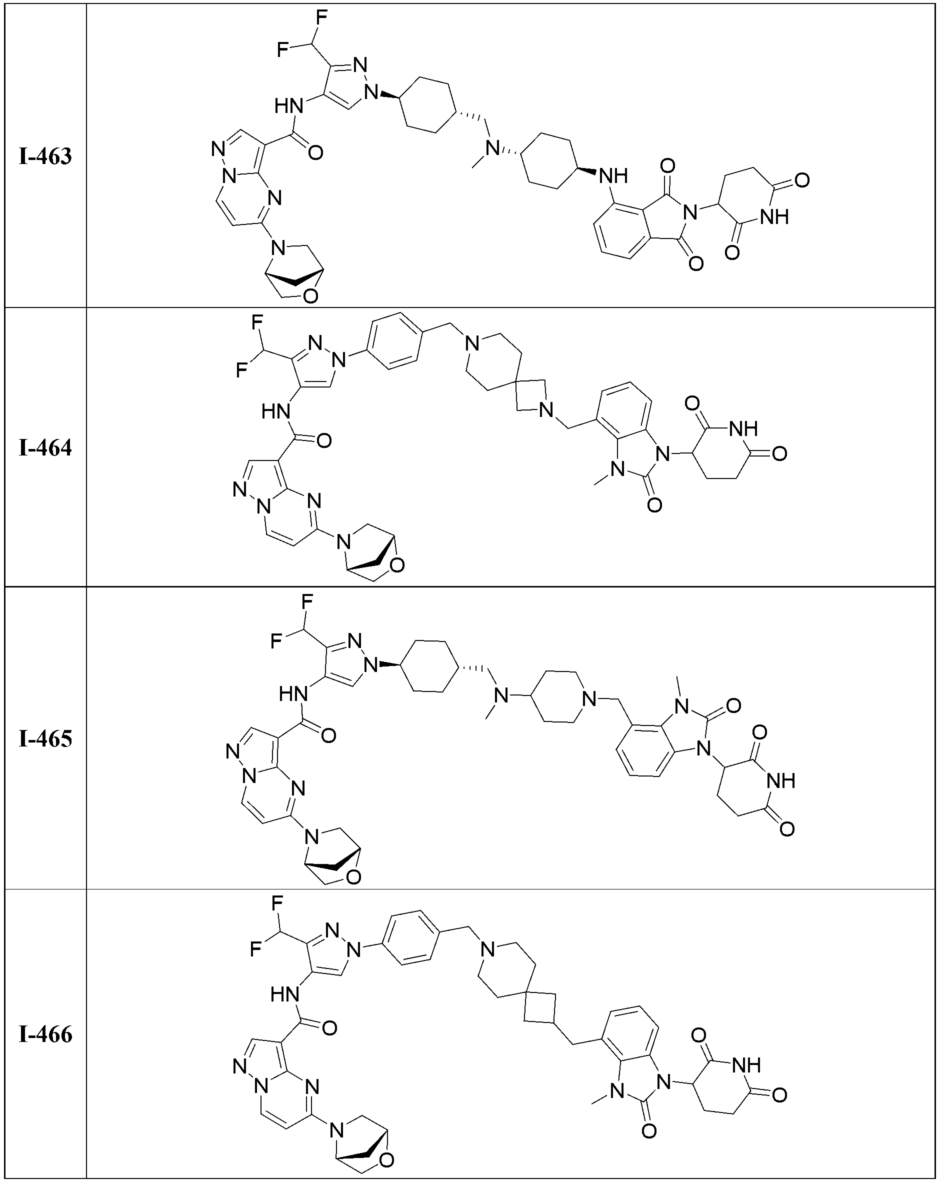

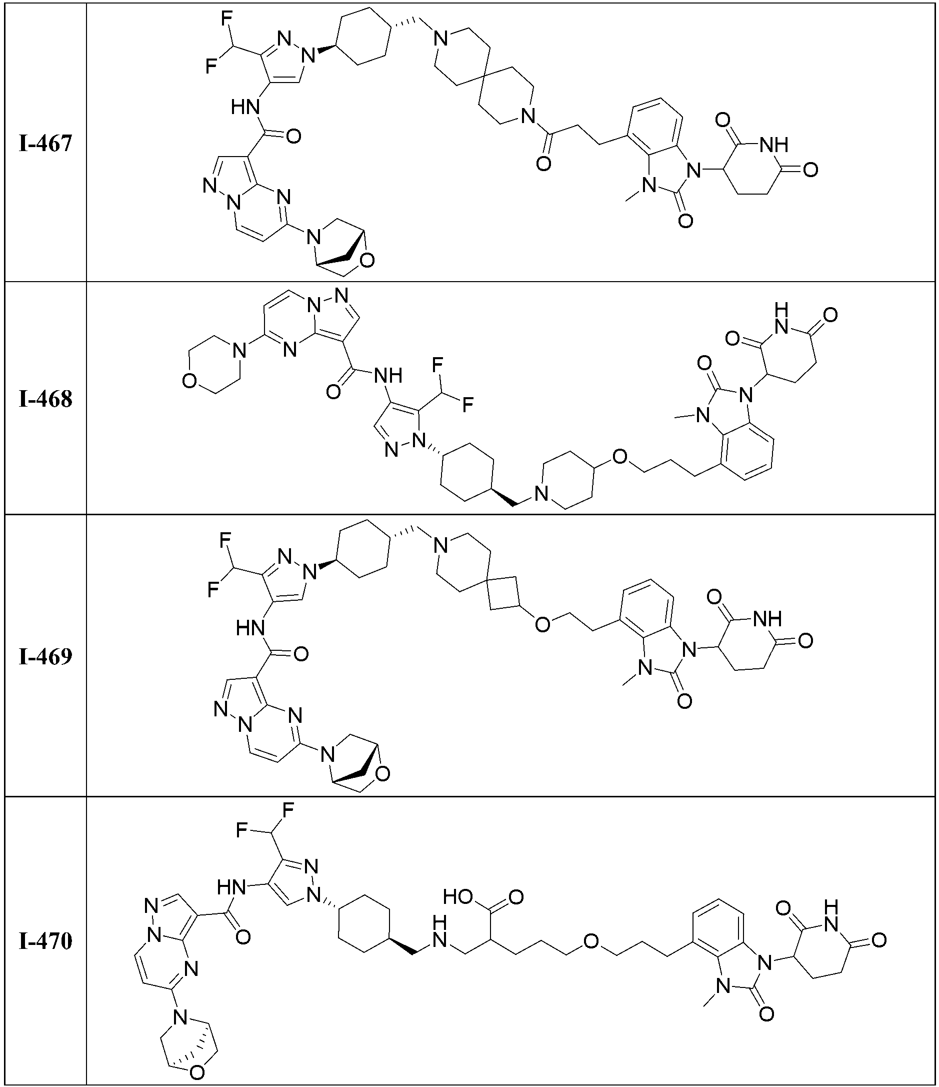

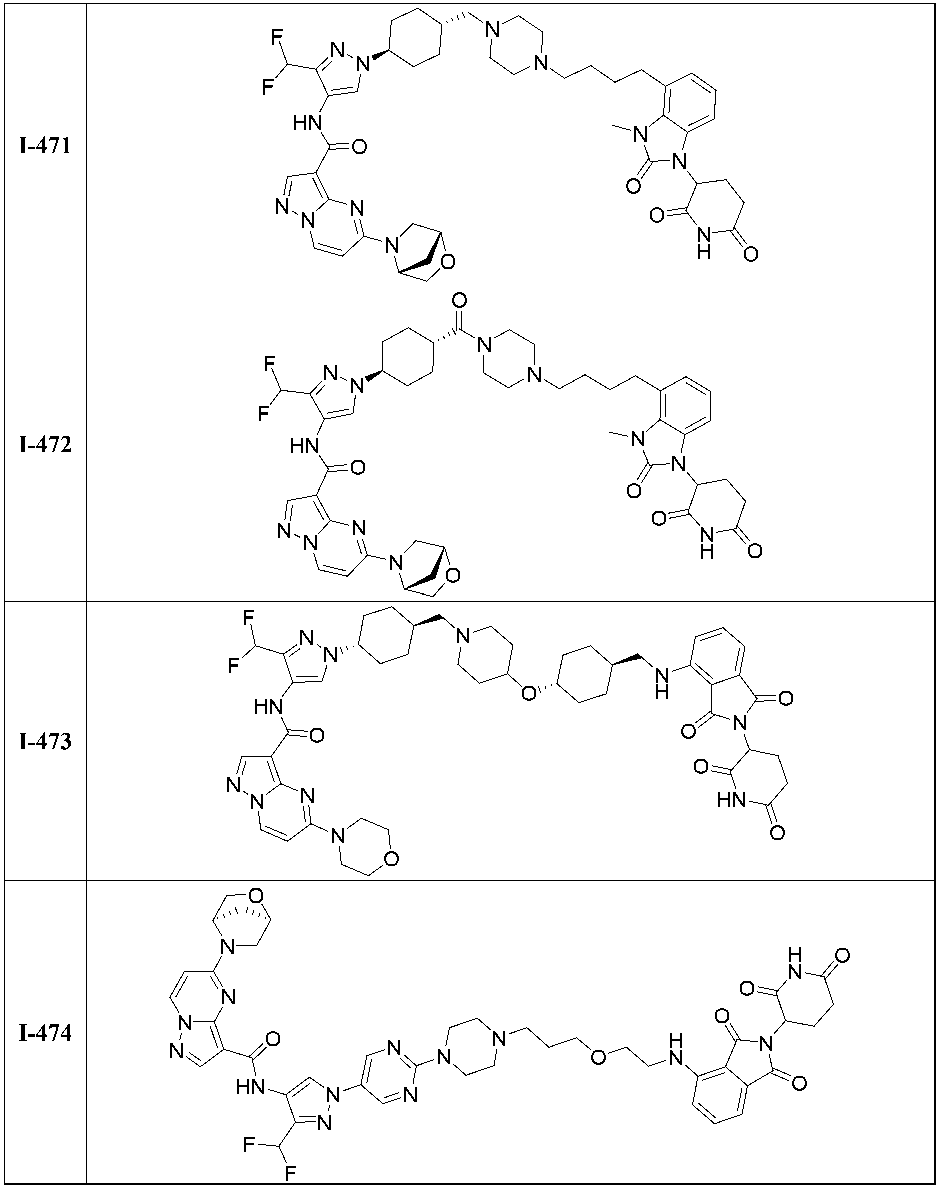

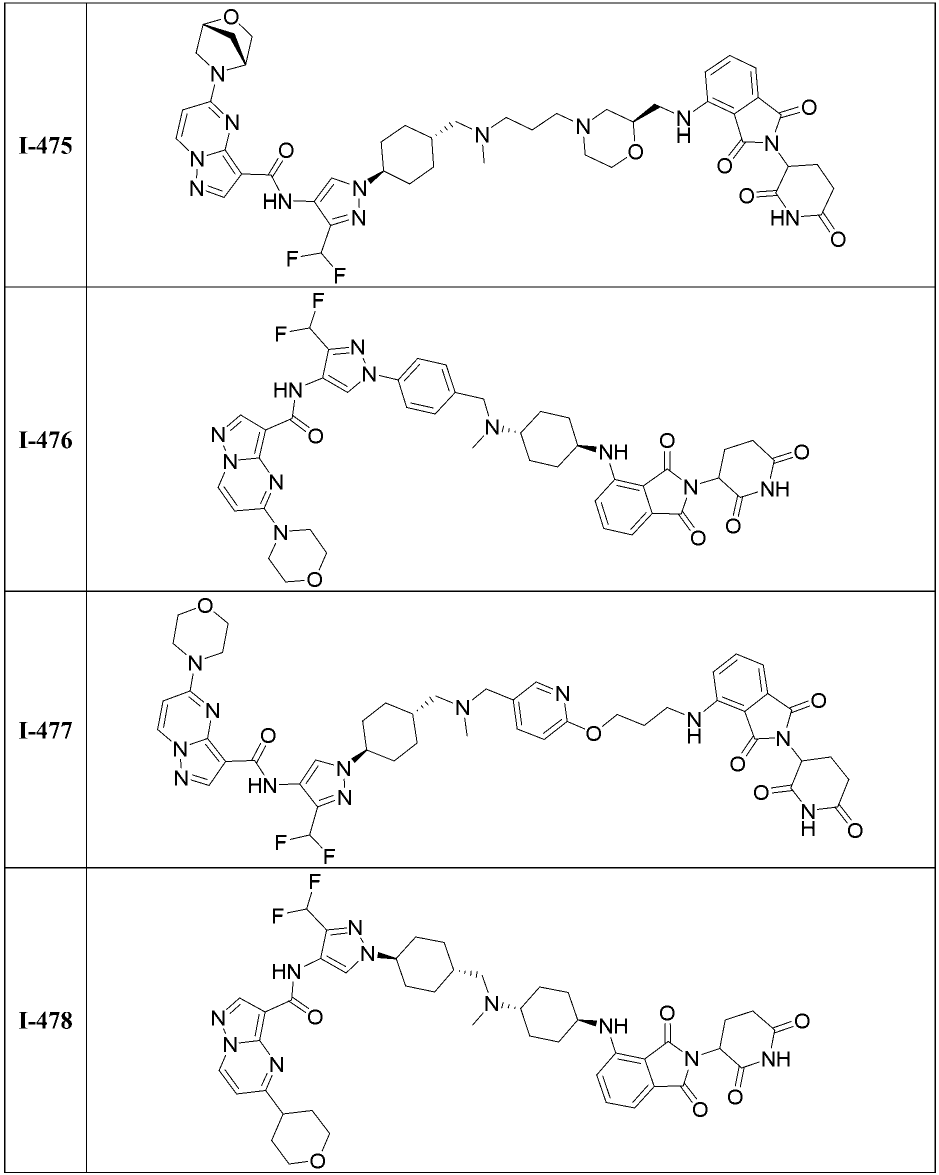

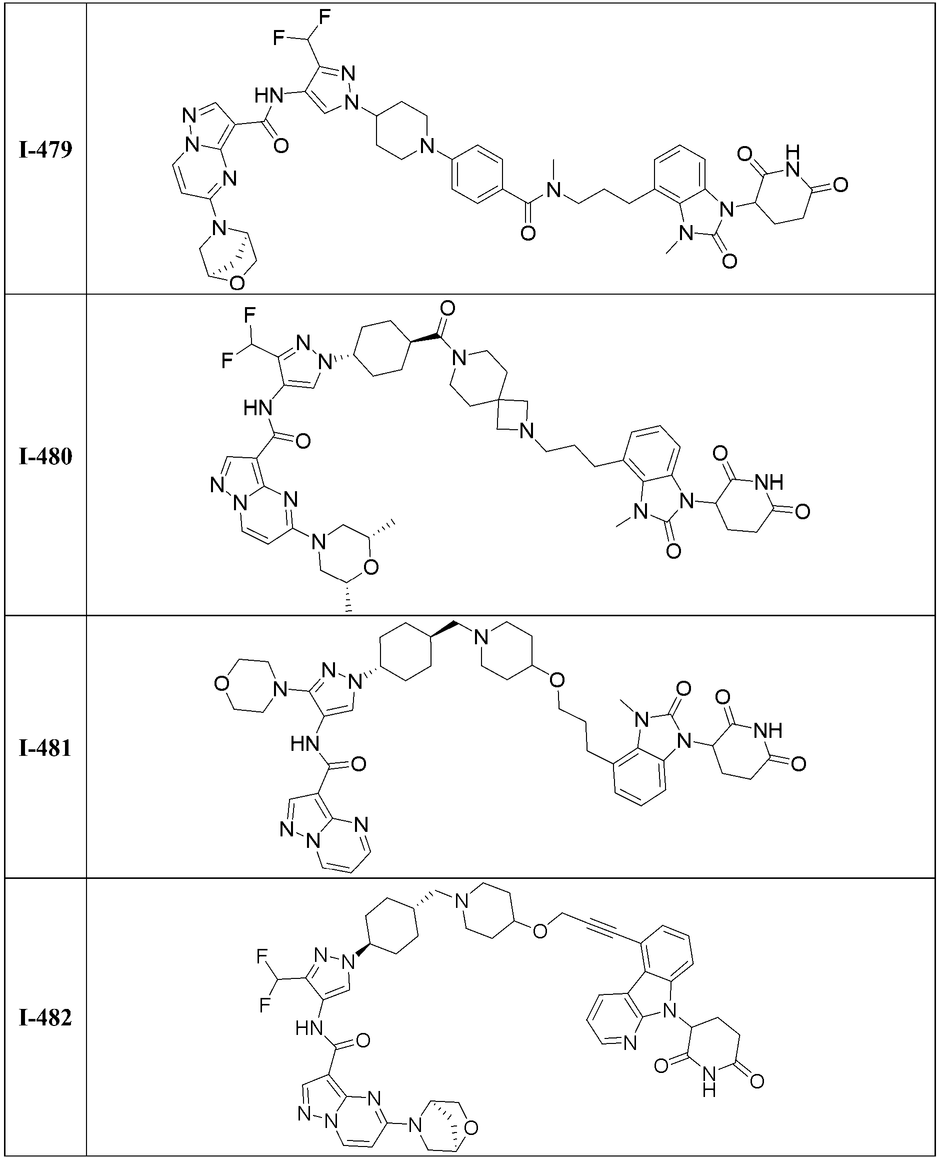

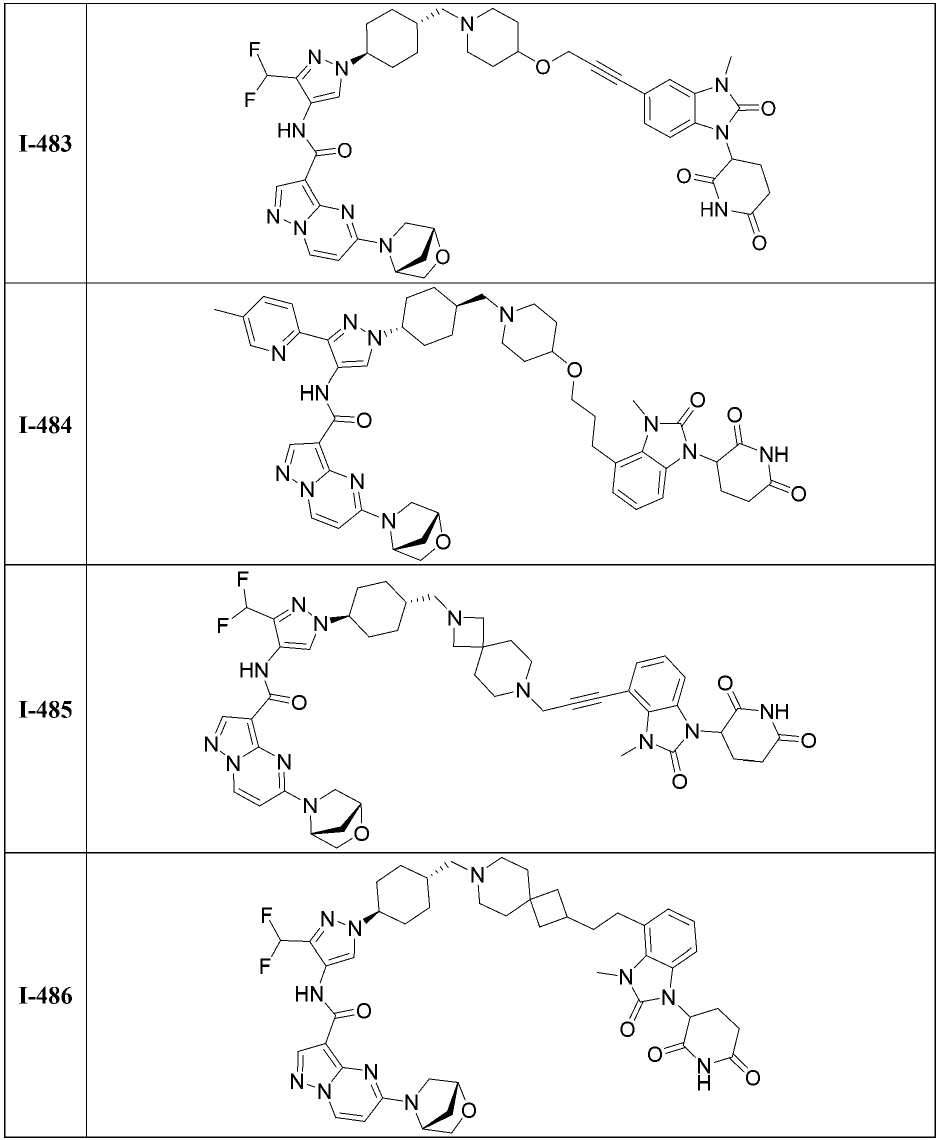

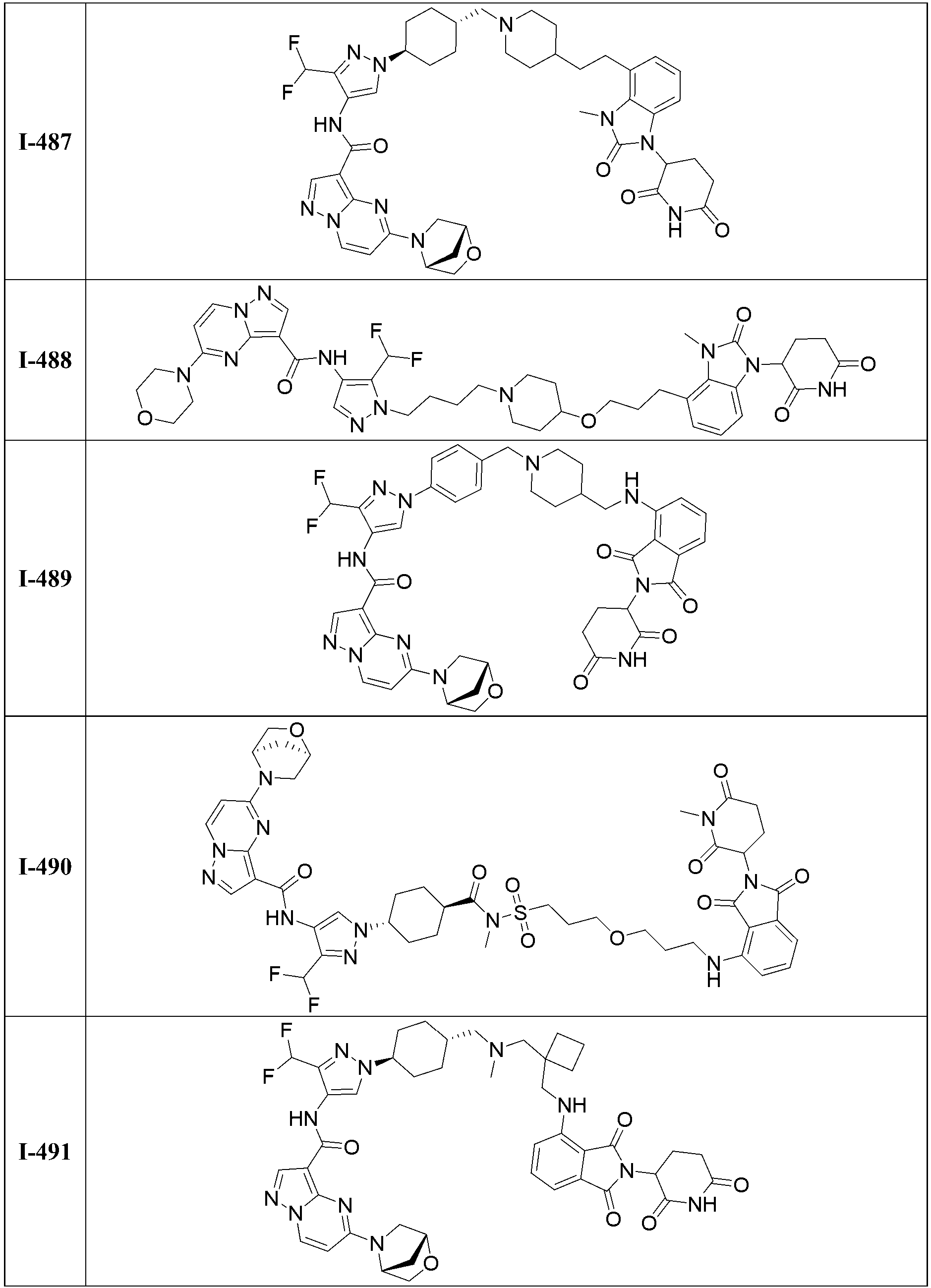

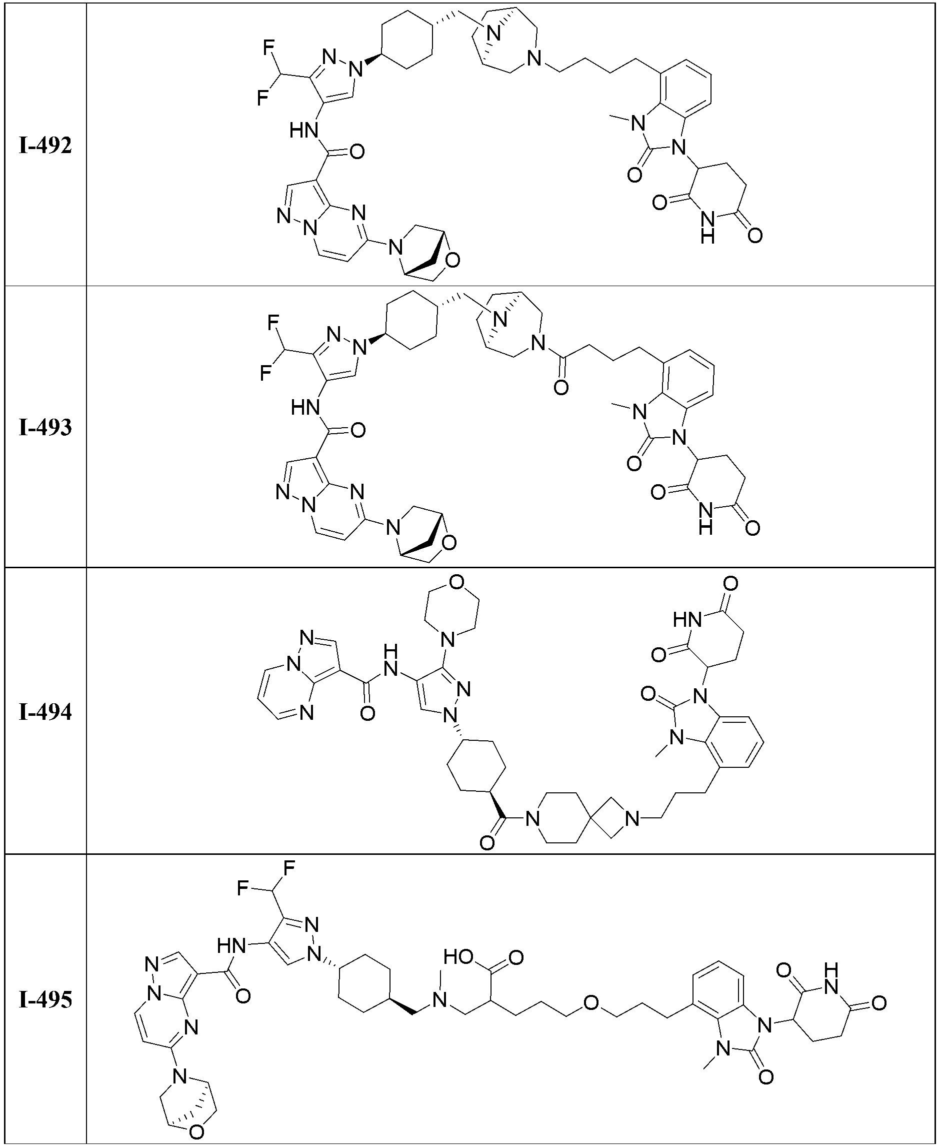

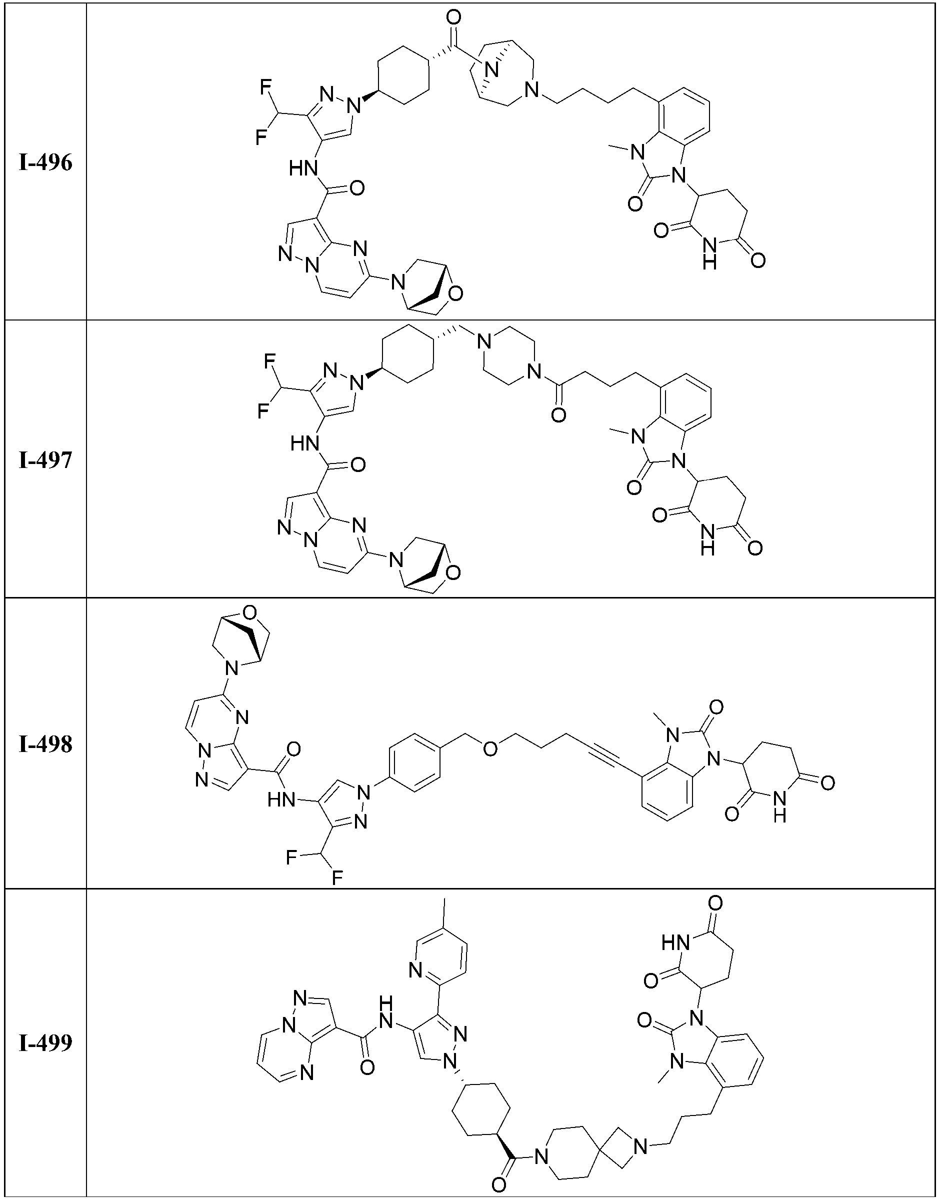

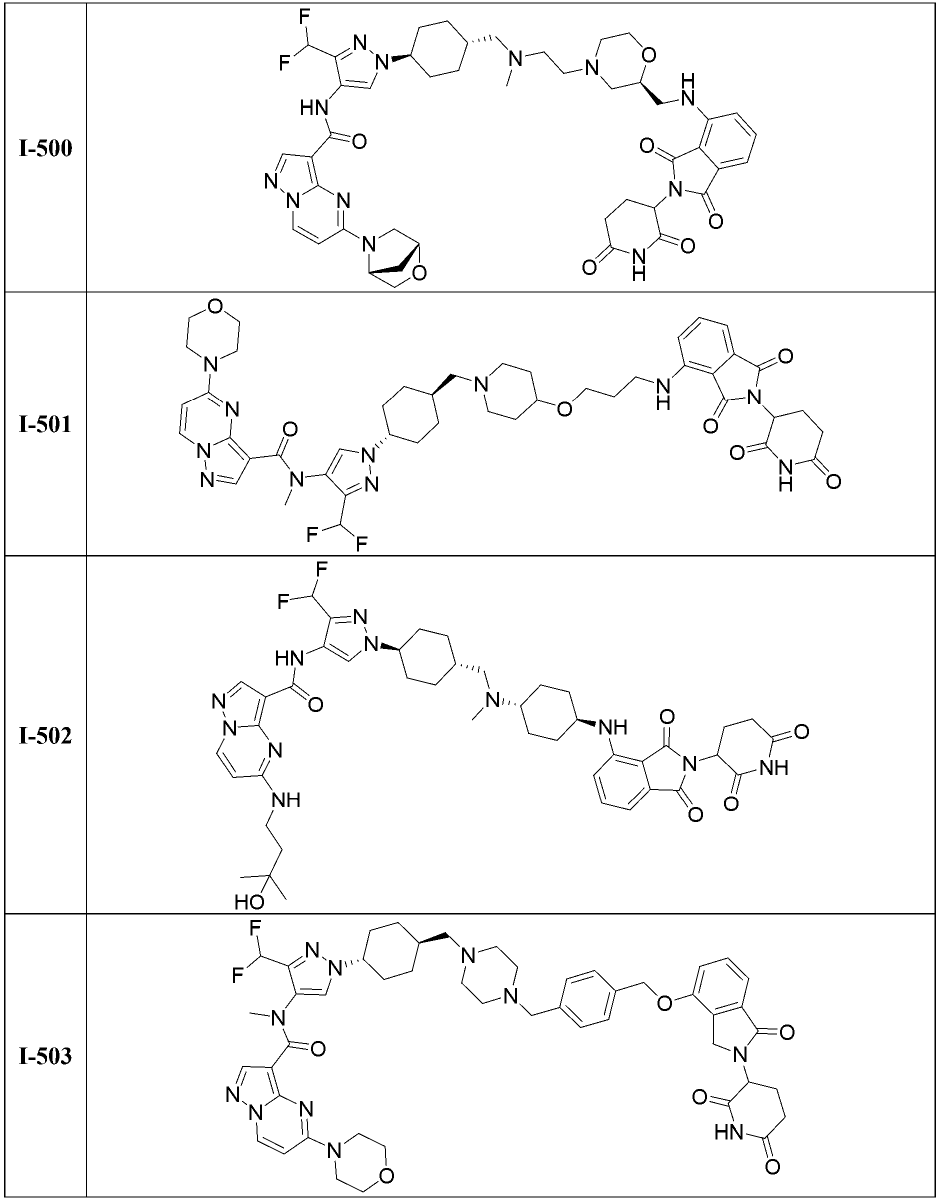

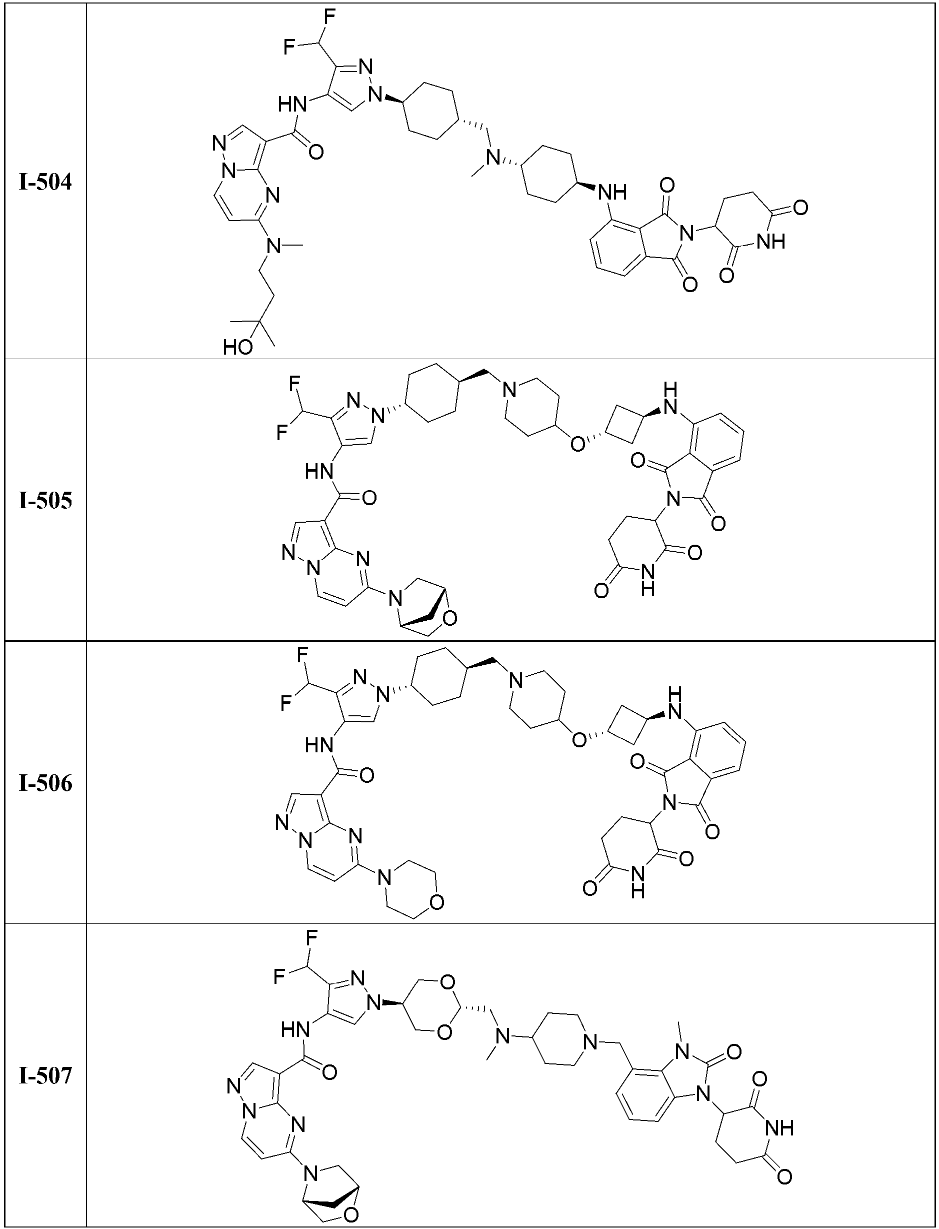

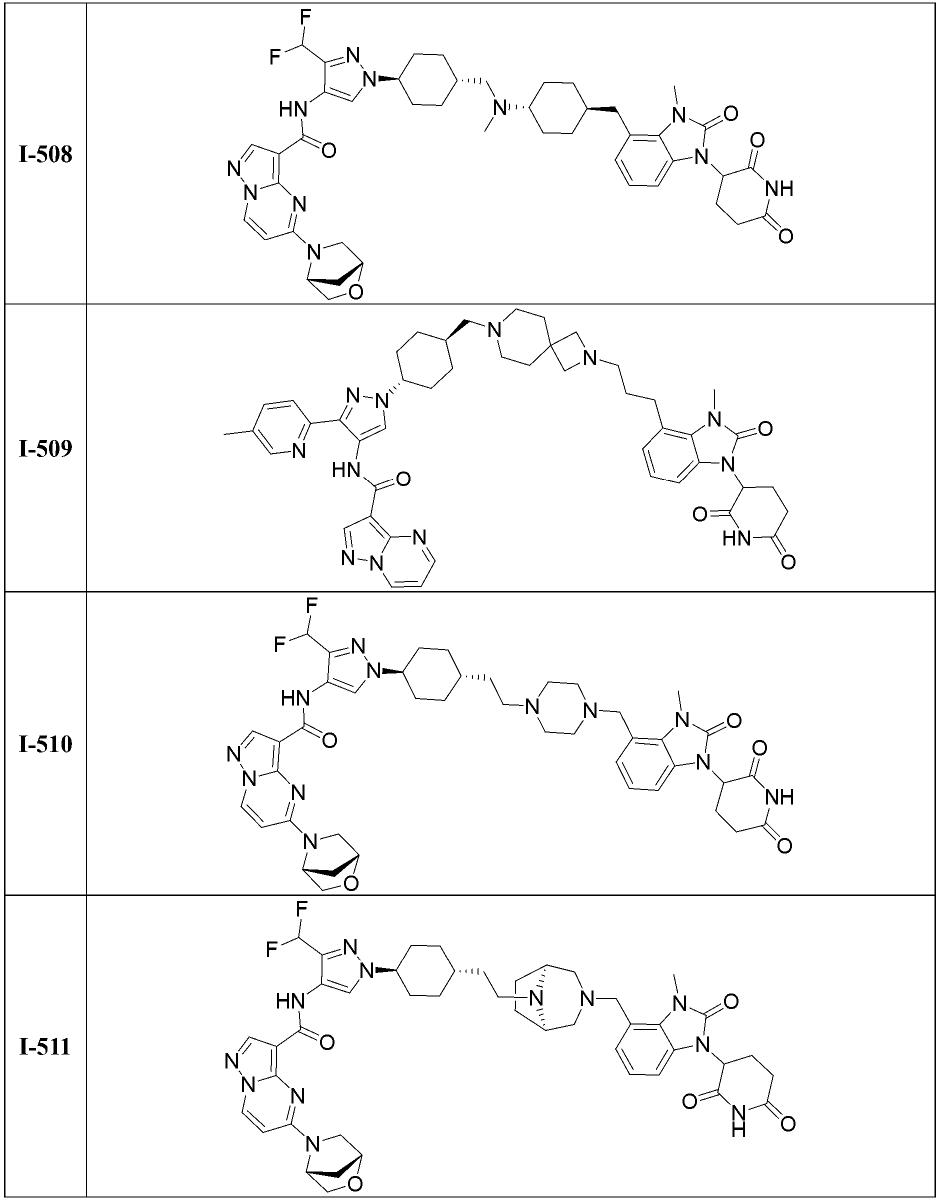

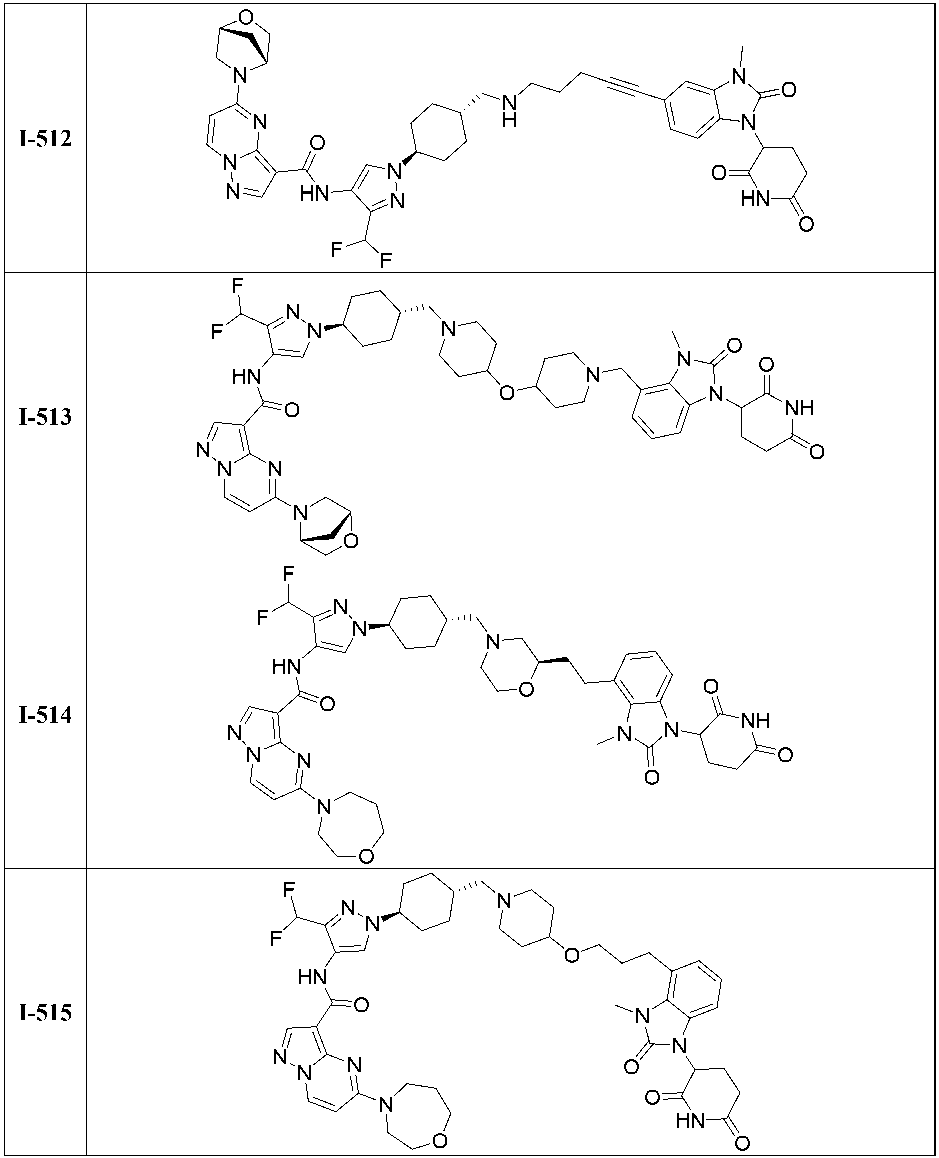

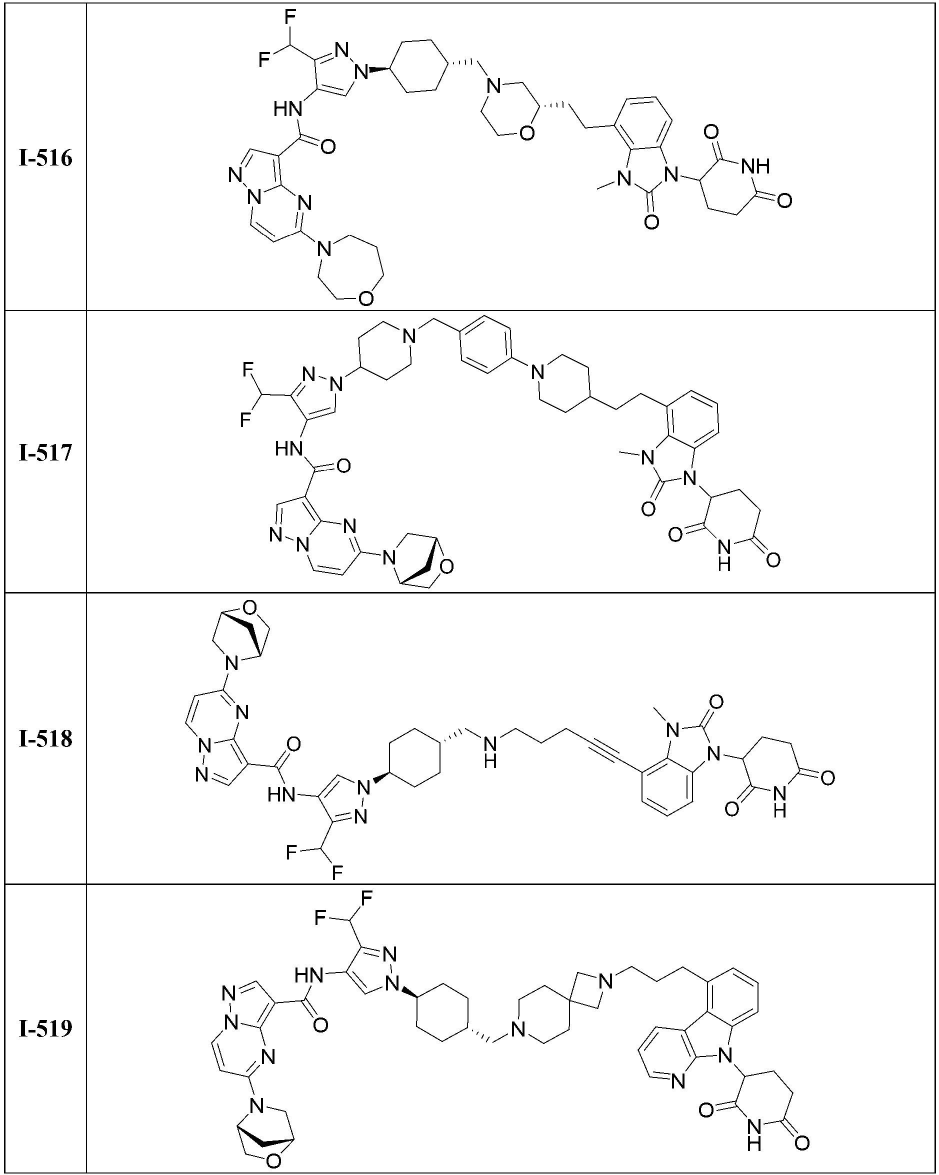

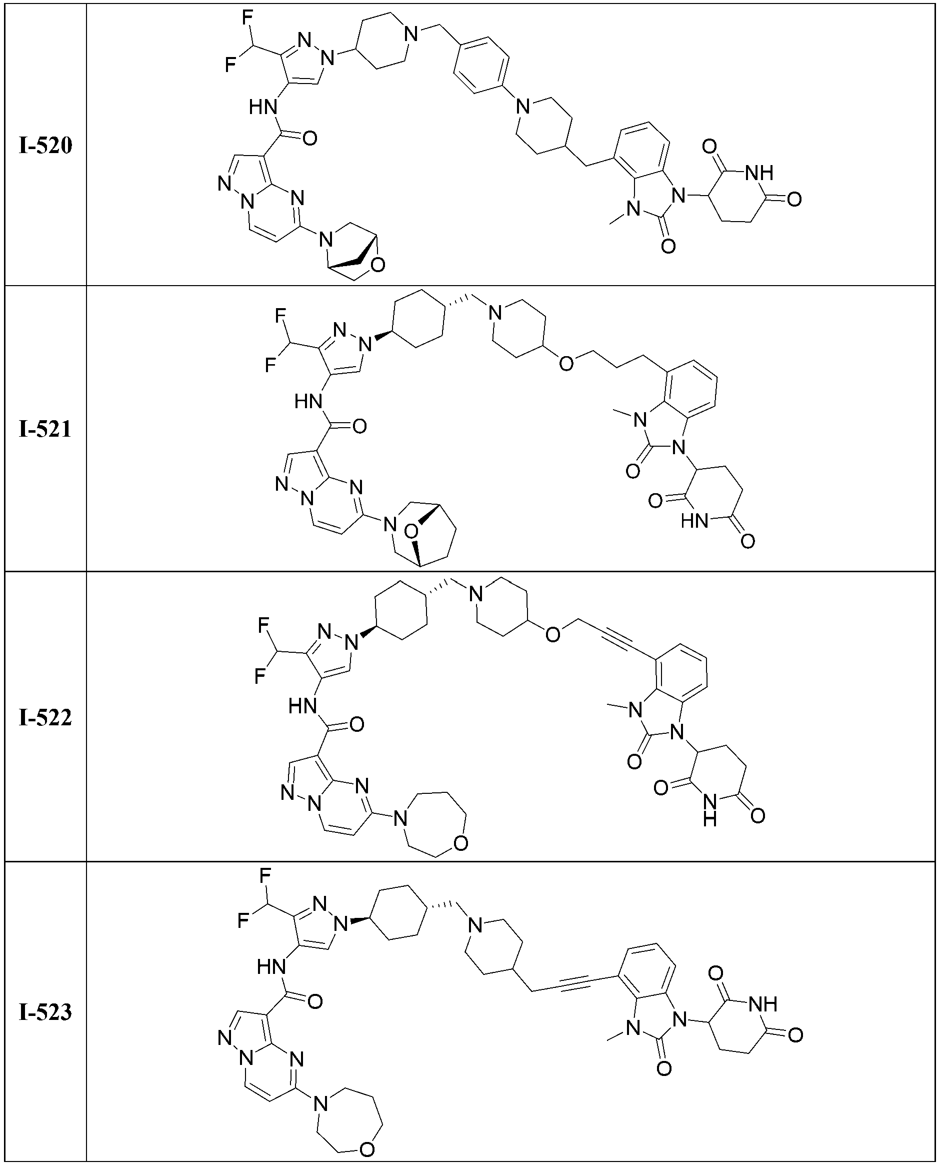

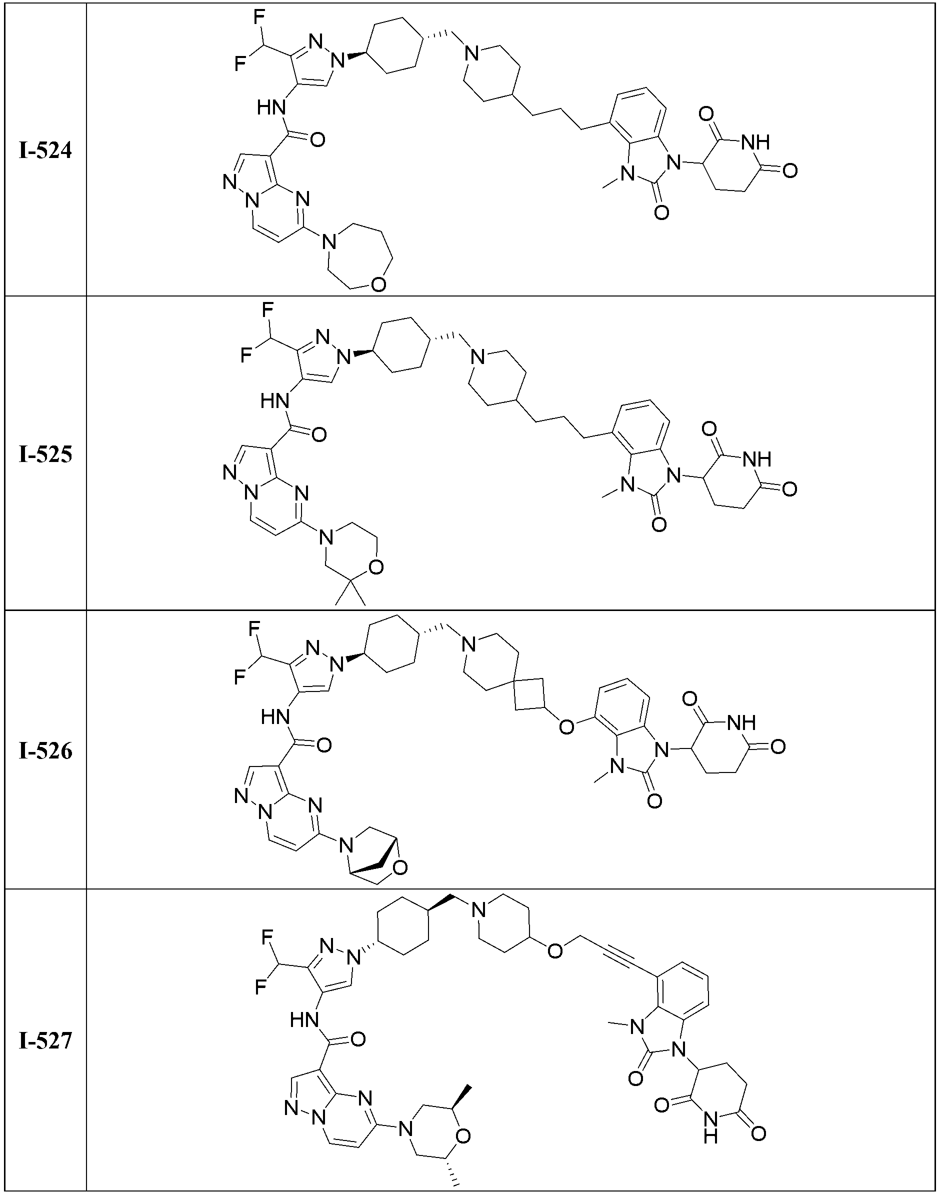

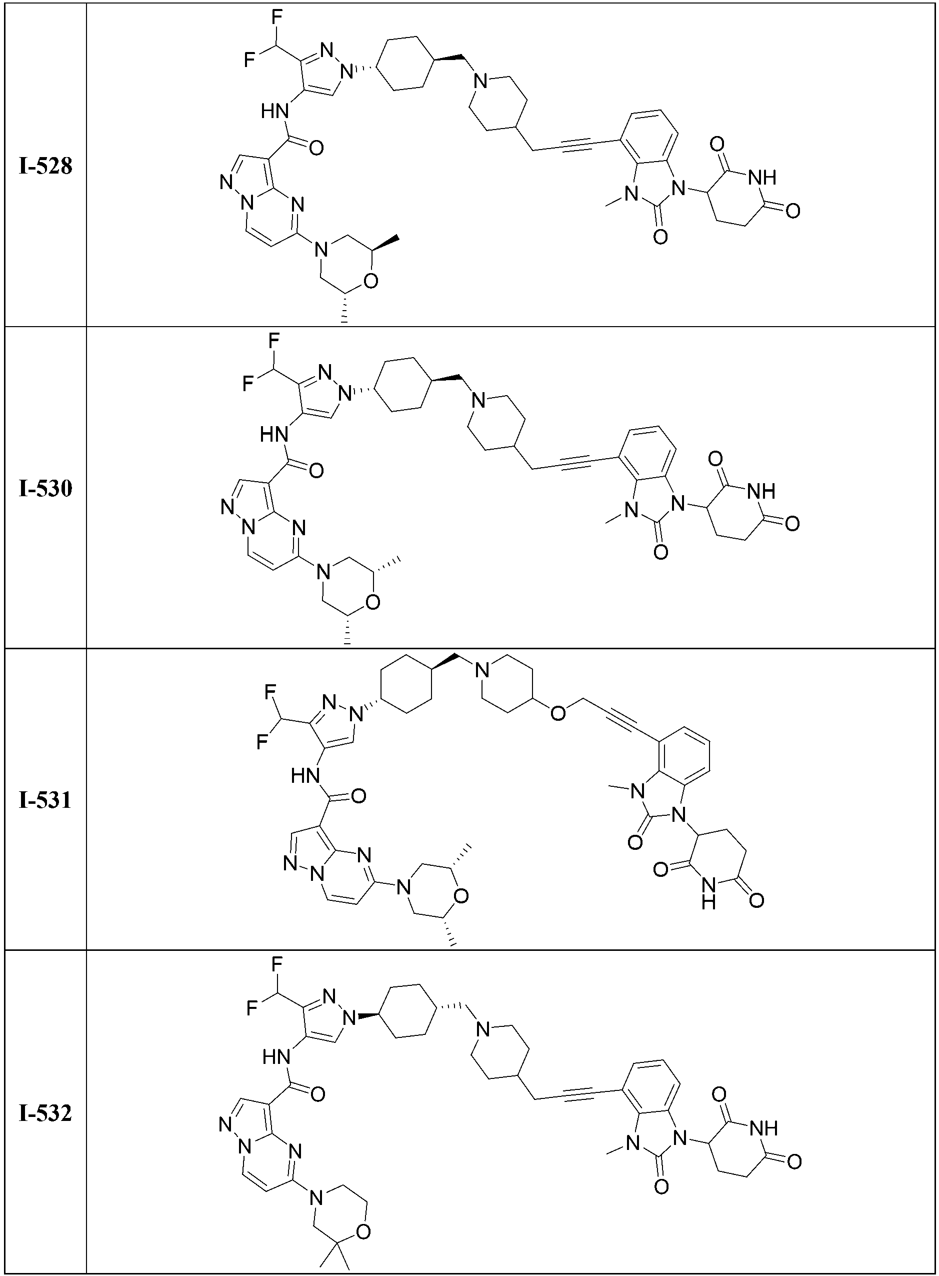

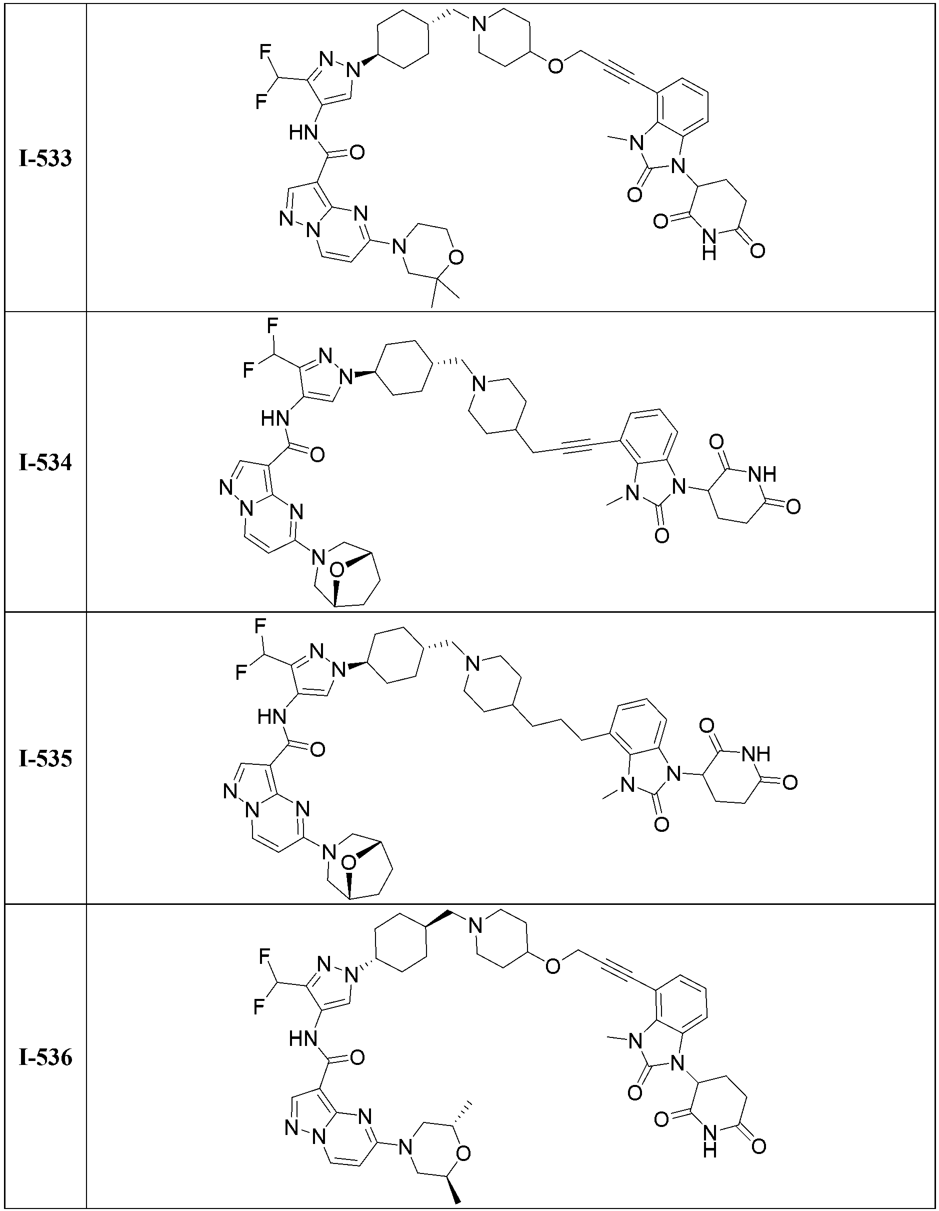

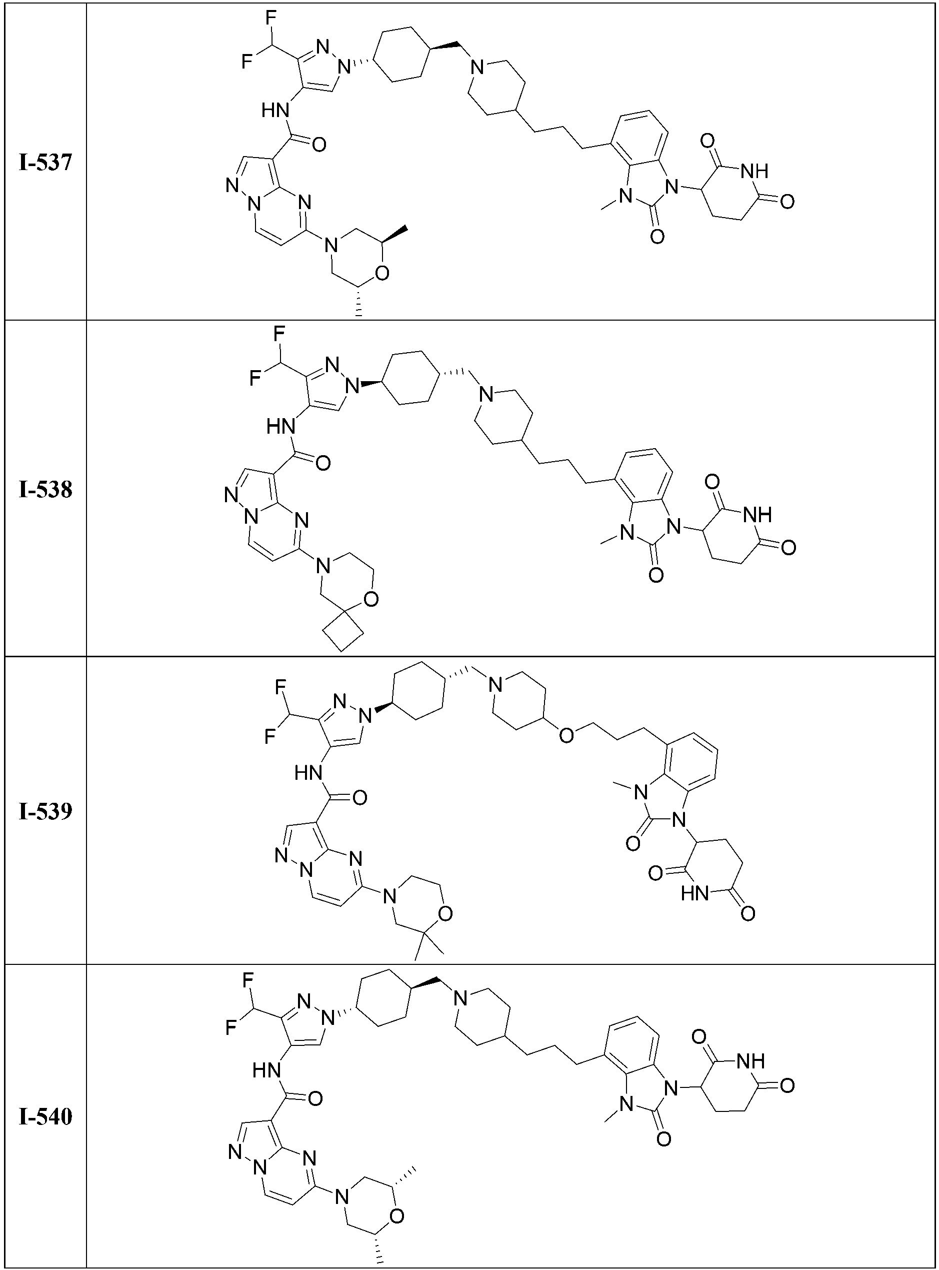

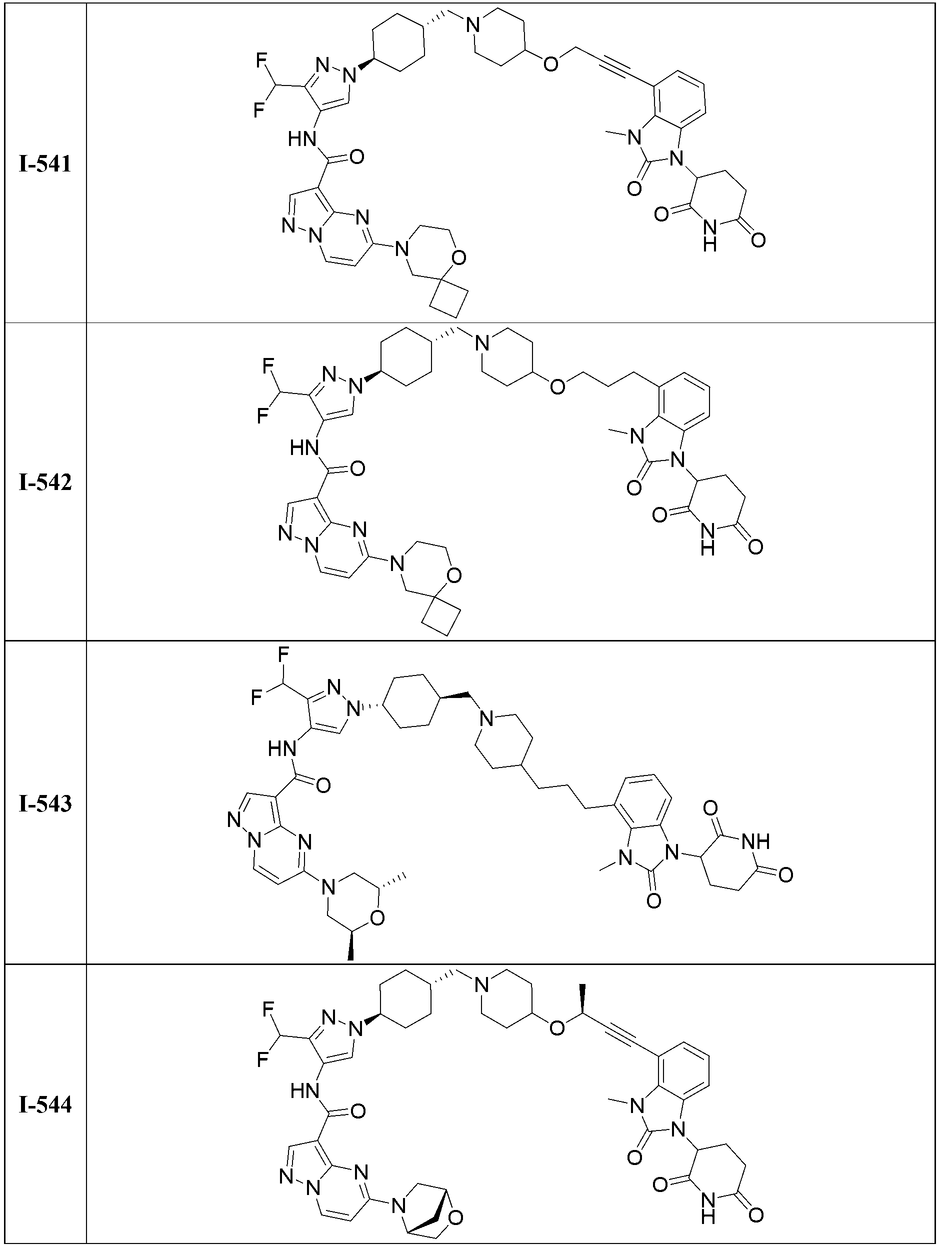

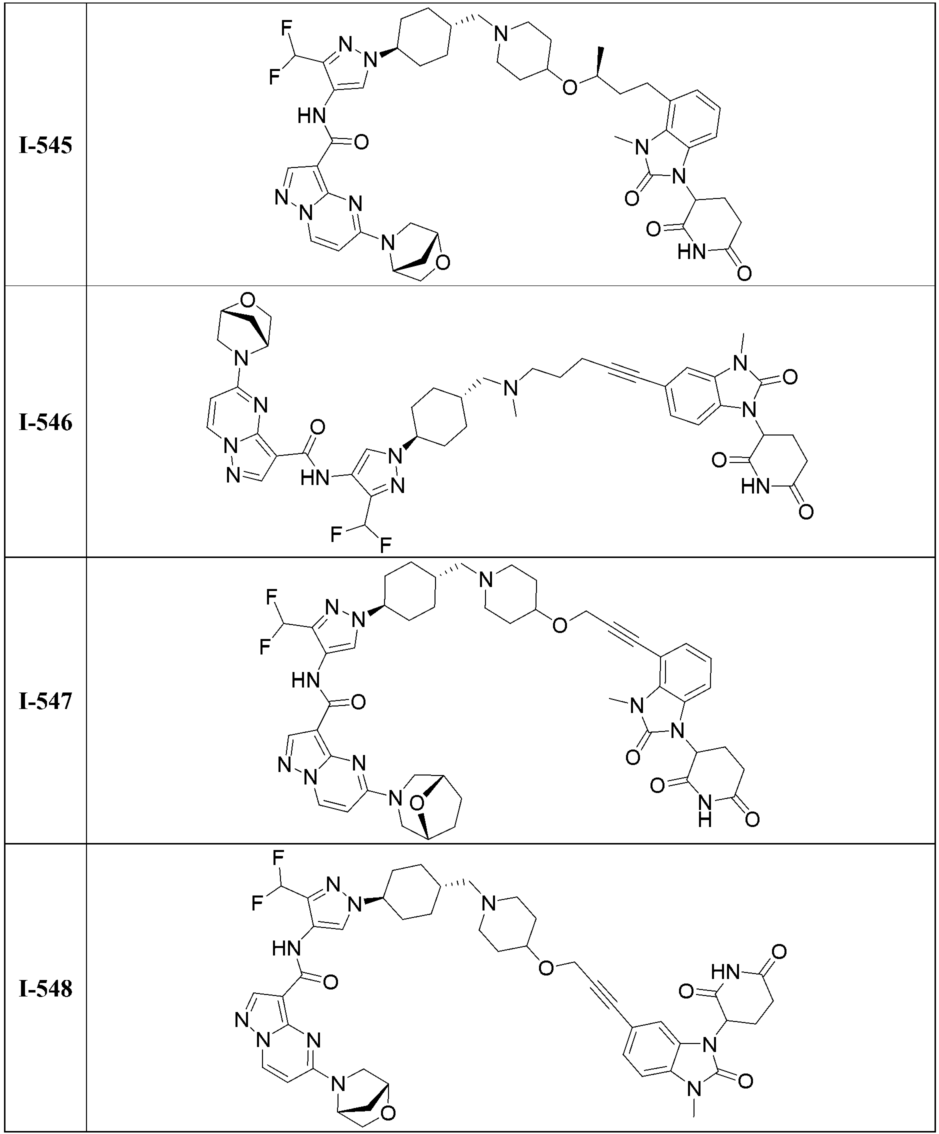

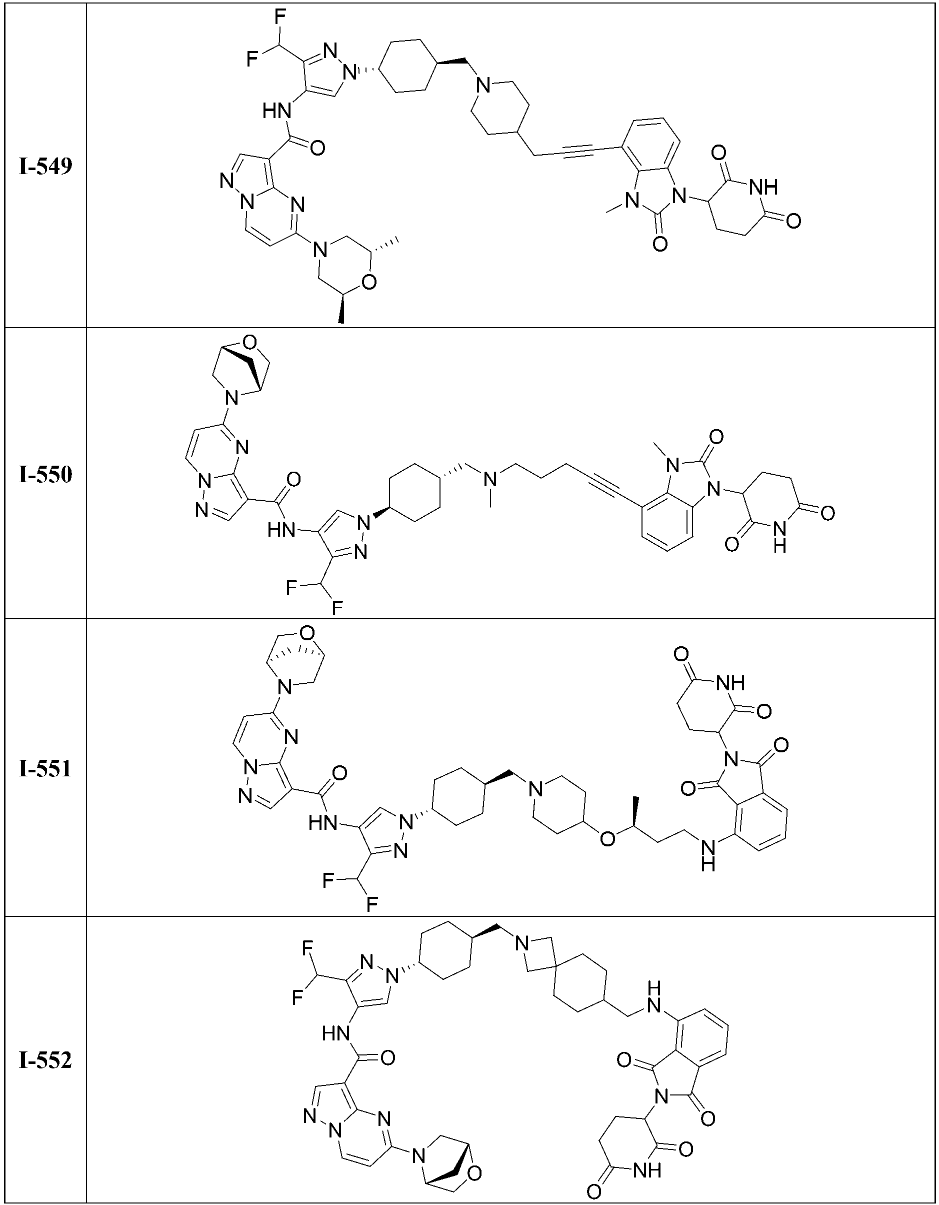

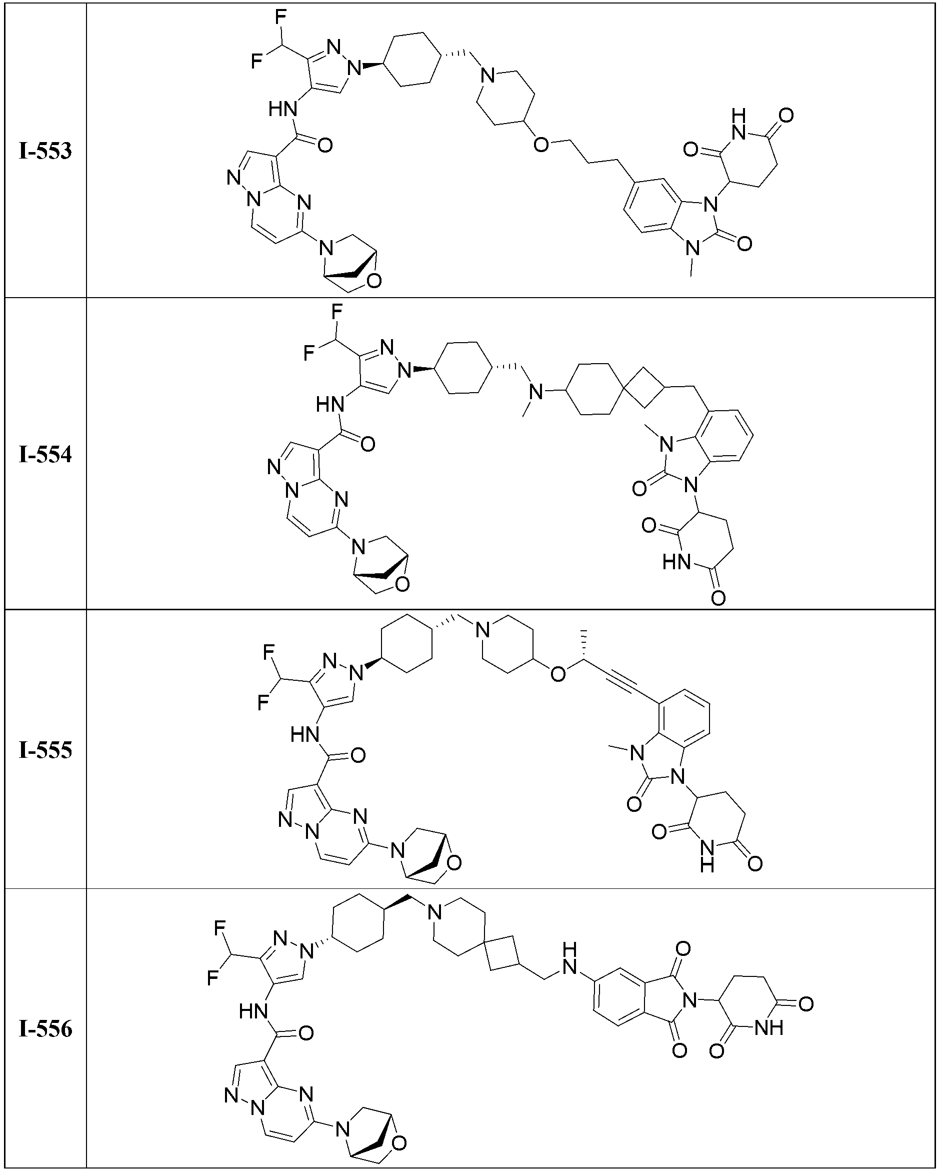

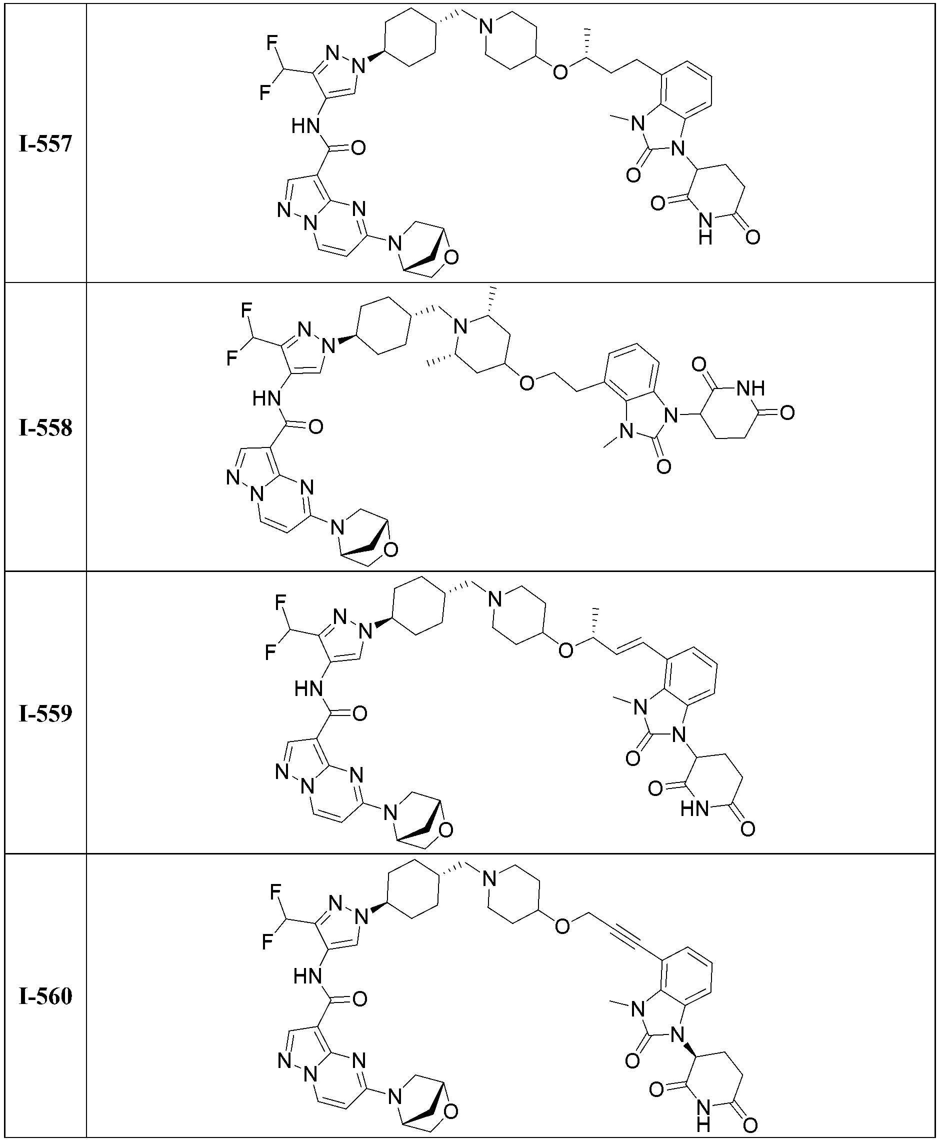

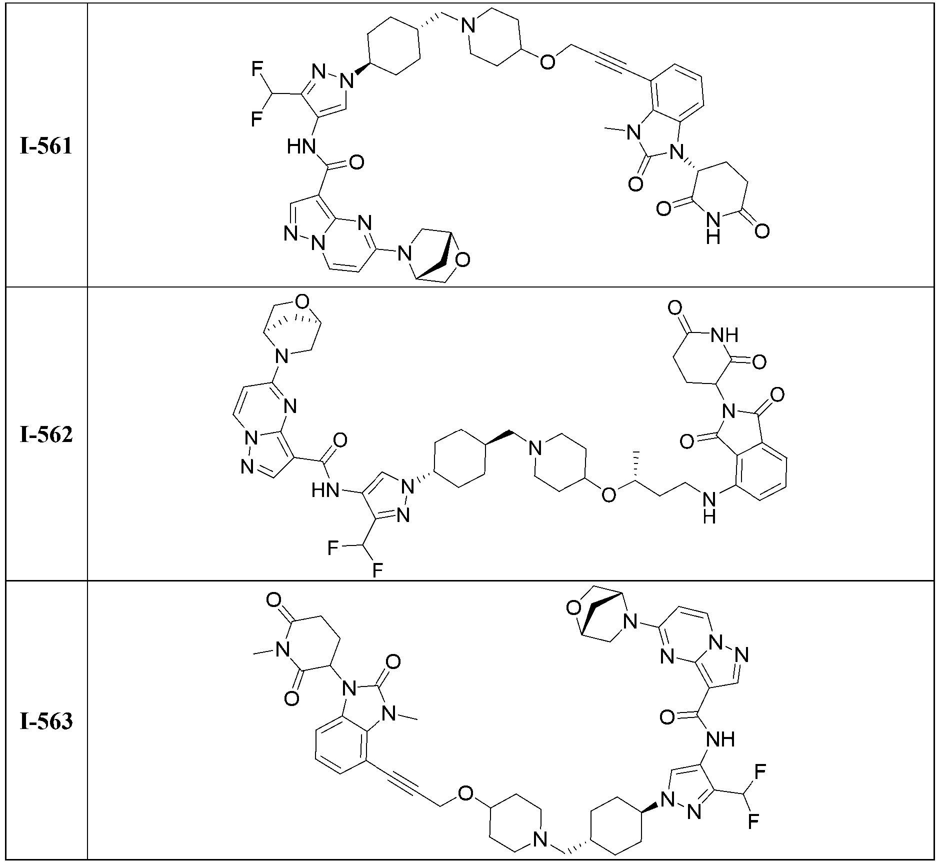

In some embodiments, the present invention provides a compound as depicted in **Table 1,** above, or a pharmaceutically acceptable salt thereof.

In some embodiments, the present invention provides a compound of formula **I,** wherein the compound is not any of the compounds depicted in **Table 1A,** below.

**Table 1A.**

| **I**-# | **Structure** |
|---|---|
| **I-99** | 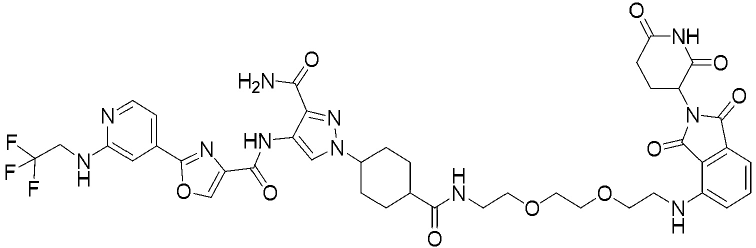 |
| **I-100** | 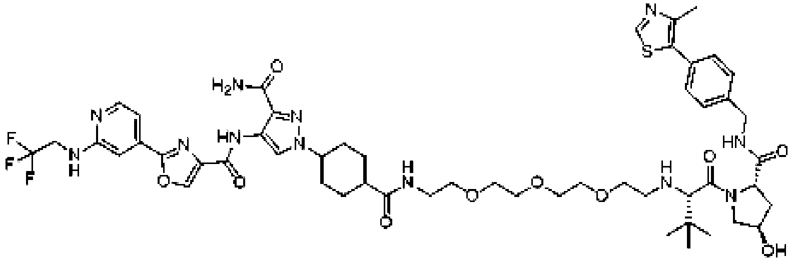 |
| **I-101** | 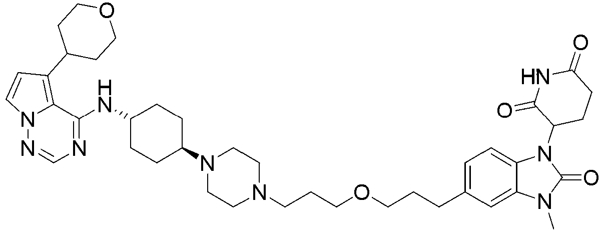 |
| **I-102** | 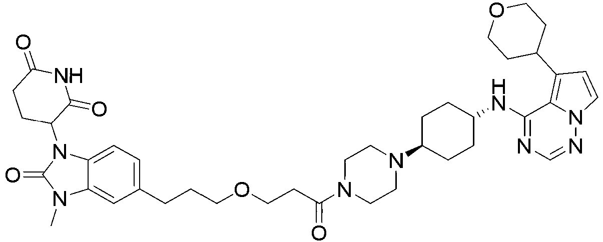 |
| **I-103** | 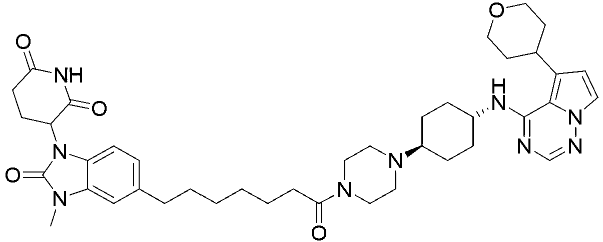 |
| **I-104** | 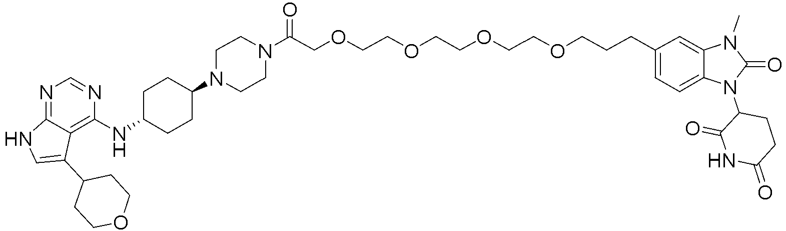 |
| **I-105** | 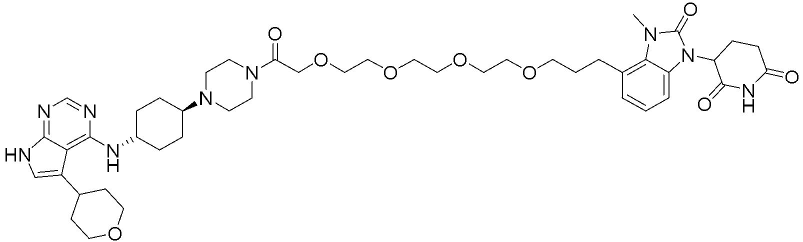 |

In some embodiments, the present invention provides a compound of formula **I,** wherein the compound is not any of the compounds depicted in **Table 1A,** above, or a pharmaceutically acceptable salt thereof.

### 4. General Methods of Providing the Present Compounds

The compounds of this invention may be prepared or isolated in general by synthetic and/or semi-synthetic methods known to those skilled in the art for analogous compounds and by methods described in detail in the Examples, herein.

In the Schemes below, where a particular protecting group, leaving group, or transformation condition is depicted, one of ordinary skill in the art will appreciate that other protecting groups, leaving groups, and transformation conditions are also suitable and are contemplated. Such groups and transformations are described in detail in March's Advanced Organic Chemistry: Reactions, Mechanisms, and Structure, M. B. Smith and J. March, 5th Edition, John Wiley & Sons, 2001, Comprehensive Organic Transformations, R. C. Larock, 2nd Edition, John Wiley & Sons, 1999, and Protecting Groups in Organic Synthesis, T. W. Greene and P. G. M. Wuts, 3rd edition, John Wiley & Sons, 1999, the entirety of each of which is hereby incorporated herein by reference.

As used herein, the phrase "oxygen protecting group" includes, for example, carbonyl protecting groups, hydroxyl protecting groups, etc. Hydroxyl protecting groups are well known in the art and include those described in detail in Protecting Groups in Organic Synthesis, T. W. Greene and P. G. M. Wuts, 3rd edition, John Wiley & Sons, 1999, the entirety of each of which is herein incorporated by reference. Examples of suitable hydroxyl protecting groups include, but are not limited to, esters, allyl ethers, ethers, silyl ethers, alkyl ethers, arylalkyl ethers, and alkoxyalkyl ethers. Examples of such esters include formates, acetates, carbonates, and sulfonates. Specific examples include formate, benzoyl formate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (trimethylacetyl), crotonate, 4-methoxy-crotonate, benzoate, p-benylbenzoate, 2,4,6-trimethylbenzoate, carbonates such as methyl, 9-fluorenylmethyl, ethyl, 2,2,2-trichloroethyl, 2-(trimethylsilyl)ethyl, 2-(phenylsulfonyl)ethyl, vinyl, allyl, and p-nitrobenzyl. Examples of such silyl ethers include trimethylsilyl, triethylsilyl, t-butyldimethylsilyl, t-butyldiphenylsilyl, triisopropylsilyl, and other trialkylsilyl ethers. Alkyl ethers include methyl, benzyl, p-methoxybenzyl, 3,4-dimethoxybenzyl, trityl, t-butyl, allyl, and allyloxycarbonyl ethers or derivatives. Alkoxyalkyl ethers include acetals such as methoxymethyl, methylthiomethyl, (2-methoxyethoxy)methyl, benzyloxymethyl, beta-(trimethylsilyl)ethoxymethyl, and tetrahydropyranyl ethers. Examples of arylalkyl ethers include benzyl, p-methoxybenzyl (MPM), 3,4-dimethoxybenzyl, O-nitrobenzyl, p-nitrobenzyl, p-halobenzyl, 2,6-dichlorobenzyl, p-cyanobenzyl, and 2- and 4-picolyl.

Amino protecting groups are well known in the art and include those described in detail in Protecting Groups in Organic Synthesis, T. W. Greene and P. G. M. Wuts, 3rd edition, John Wiley & Sons, 1999, the entirety of each of which is herein incorporated by reference. Suitable amino protecting groups include, but are not limited to, aralkylamines, carbamates, cyclic imides, allyl amines, amides, and the like. Examples of such groups include t-butyloxycarbonyl (BOC), ethyloxycarbonyl, methyloxycarbonyl, trichloroethyloxycarbonyl, allyloxycarbonyl (Alloc), benzyloxocarbonyl (CBZ), allyl, phthalimide, benzyl (Bn), fluorenylmethylcarbonyl (Fmoc), formyl, acetyl, chloroacetyl, dichloroacetyl, trichloroacetyl, phenylacetyl, trifluoroacetyl, benzoyl, and the like.

In the schemes below, where a provided compound is formed having a reactive DIM moiety (e.g., amine, alcohol, etc.), it is not shown but it is generally appreciated and well known by those having ordinary skill in the art that the reactivity of said reactive DIM moiety may be masked by employing a suitable protecting group that can thereafter be removed in situ or during a separate synthetic step.

In certain embodiments, compounds of the present invention are generally prepared according to Scheme 1 set forth below:

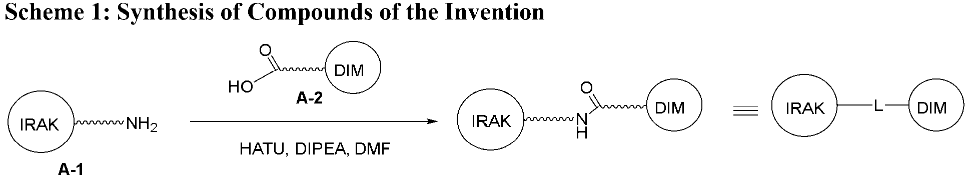

As depicted in Scheme 1, above, amine **A-1** is coupled to acid **A-2** using the coupling agent HATU in the presence of the base DIPEA in DMF to form a compound of the invention with a linker comprising an amide bond. The squiggly bond, 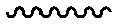 , represents the portion of the linker between IRAK and the terminal amino group of **A-1** or the portion of the linker between DIM and the terminal carboxyl group of **A-2,** respectively. Additionally, an amide bond can be formed using coupling reagents known in the art such as, but not limited to DCC, DIC, EDC, HBTU, HCTU, PyAOP, PyBrOP, BOP, BOP-Cl, DEPBT, T3P, TATU, TBTU, TNTU, TOTU, TPTU, TSTU, or TDBTU.

In certain embodiments, compounds of the present invention are generally prepared according to Scheme 2 set forth below:

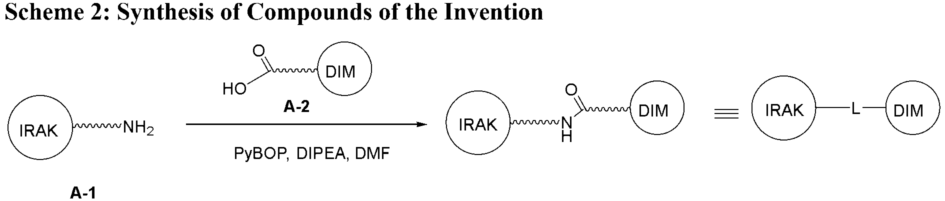

As depicted in Scheme 2, above, amine **A-1** is coupled to acid **A-2** using the coupling agent PyBOP in the presence of the base DIPEA in DMF to form a compound of the invention with a linker comprising an amide bond. The squiggly bond, 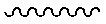 , represents the portion of the linker between IRAK and the terminal amino group of **A-1** or the portion of the linker between DIM and the terminal carboxyl group of **A-2,** respectively. Additionally, an amide bond can be formed using coupling reagents known in the art such as, but not limited to DCC, DIC, EDC, HBTU, HCTU, PyAOP, PyBrOP, BOP, BOP-Cl, DEPBT, T3P, TATU, TBTU, TNTU, TOTU, TPTU, TSTU, or TDBTU.

In certain embodiments, compounds of the present invention are generally prepared according to Scheme 3 set forth below:

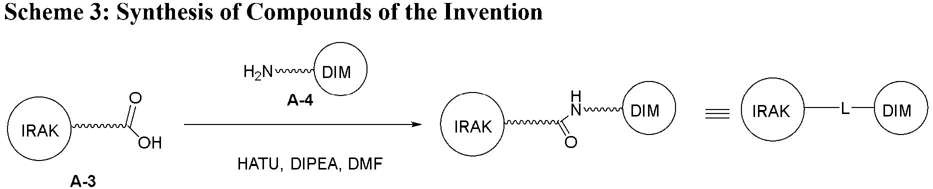

As depicted in Scheme 3, above, acid **A-3** is coupled to amine **A-4** using the coupling agent HATU in the presence of the base DIPEA in DMF to form a compound of the invention with a linker comprising an amide bond. The squiggly bond, 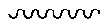 , represents the portion of the linker between IRAK and the terminal carboxyl group of **A-3** or the portion of the linker between DIM and the terminal amino group of **A-4,** respectively. Additionally, an amide bond can be formed using coupling reagents known in the art such as, but not limited to DCC, DIC, EDC, HBTU, HCTU, PyAOP, PyBrOP, BOP, BOP-Cl, DEPBT, T3P, TATU, TBTU, TNTU, TOTU, TPTU, TSTU, or TDBTU.

In certain embodiments, compounds of the present invention are generally prepared according to Scheme 4 set forth below:

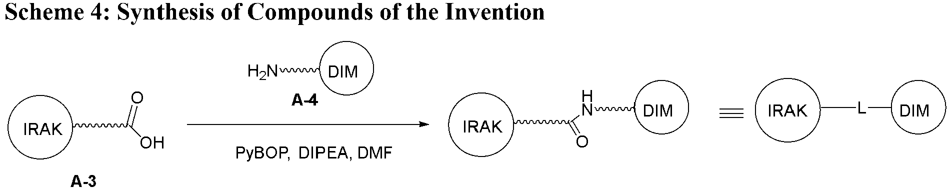

As depicted in Scheme 4, above, acid **A-3** is coupled to amine **A-4** using the coupling agent PyBOP in the presence of the base DIPEA in DMF to form a compound of the invention with a linker comprising an amide bond. The squiggly bond, 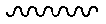 , represents the portion of the linker between IRAK and the terminal carboxyl group of **A-3** or the portion of the linker between DIM and the terminal amino group of **A-4,** respectively. Additionally, an amide bond can be formed using coupling reagents known in the art such as, but not limited to DCC, DIC, EDC, HBTU, HCTU, PyAOP, PyBrOP, BOP, BOP-Cl, DEPBT, T3P, TATU, TBTU, TNTU, TOTU, TPTU, TSTU, or TDBTU.

In certain embodiments, compounds of the present invention are generally prepared according to Scheme 5 set forth below:

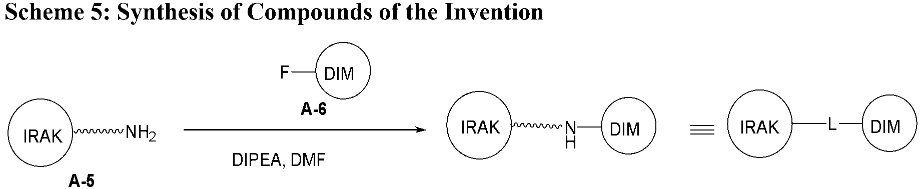

As depicted in Scheme 5, above, an S_{N}Ar displacement of fluoride **A-6** by amine **A-5** is effected in the presence of the base DIPEA in DMF to form a compound of the invention with a linker comprising a secondary amine. The squiggly bond, 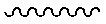 , represents the portion of the linker between IRAK and the terminal amino group of **A-5.**

In certain embodiments, compounds of the present invention are generally prepared according to Scheme 6 set forth below:

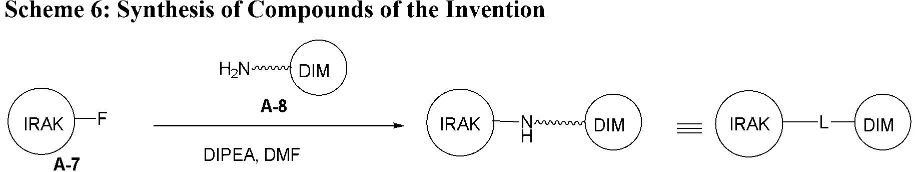

As depicted in Scheme 6, above, an S_{N}Ar displacement of fluoride **A-7** by amine **A-8** is effected in the presence of the base DIPEA in DMF to form a compound of the invention with a linker comprising a secondary amine. The squiggly bond, 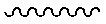 , represents the portion of the linker between DIM and the terminal amino group of **A-8.**

In certain embodiments, compounds of the present invention are generally prepared according to Scheme 7 set forth below:

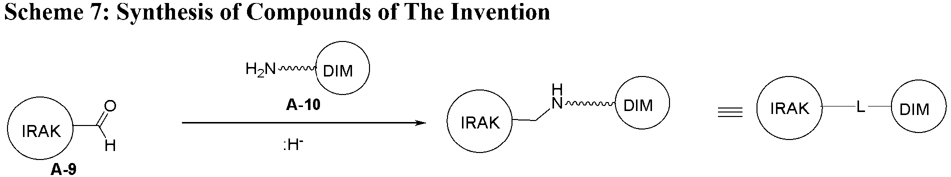

As depicted in Scheme 7, above, reductive alkylation of aldehyde **A-9** by amine **A-10** is effected in the presence of a mild hydride source (*e.g.*, sodium cyanoborohydride or sodium triacetoxyborohydride) to form a provided compound with a linker comprising a secondary amine. The squiggly bond, 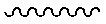 , represents the portion of the linker between DIM and the terminal amino group of **A-10.**

In certain embodiments, compounds of the present invention are generally prepared according to Scheme 8 set forth below:

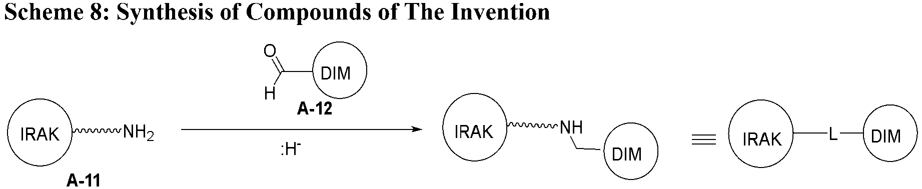

As depicted in Scheme 8, above, reductive alkylation of aldehyde **A-12** by amine **A-11** is effected in the presence of a mild hydride source (*e.g.*, sodium cyanoborohydride or sodium triacetoxyborohydride) to form a provided compound with a linker comprising a secondary amine. The squiggly bond, 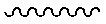 , represents the portion of the linker between IRAK and the terminal amino group of **A-11.**

One of skill in the art will appreciate that various functional groups present in compounds of the invention such as aliphatic groups, alcohols, carboxylic acids, esters, amides, aldehydes, halogens and nitriles can be interconverted by techniques well known in the art including, but not limited to reduction, oxidation, esterification, hydrolysis, partial oxidation, partial reduction, halogenation, dehydration, partial hydration, and hydration. See for example, "March's Advanced Organic Chemistry", 5th Ed., Ed.: Smith, M.B. and March, J., John Wiley & Sons, New York: 2001, the entirety of each of which is herein incorporated by reference. Such interconversions may require one or more of the aforementioned techniques, and certain methods for synthesizing compounds of the invention are described below in the Exemplification.

### 5. Uses, Formulation and Administration

### Pharmaceutically acceptable compositions

According to another embodiment, the invention provides a composition comprising a compound of this invention or a pharmaceutically acceptable derivative thereof and a pharmaceutically acceptable carrier, adjuvant, or vehicle. The amount of compound in compositions of this invention is such that is effective to measurably degrade and/or inhibit an IRAK protein kinase, or a mutant thereof, in a biological sample or in a patient. In certain embodiments, the amount of compound in compositions of this invention is such that is effective to measurably degrade and/or inhibit an IRAK protein kinase, or a mutant thereof, in a biological sample or in a patient. In certain embodiments, a composition of this invention is formulated for administration to a patient in need of such composition. In some embodiments, a composition of this invention is formulated for oral administration to a patient.

The term "patient," as used herein, means an animal, preferably a mammal, and most preferably a human.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

A "pharmaceutically acceptable derivative" means any non-toxic salt, ester, salt of an ester or other derivative of a compound of this invention that, upon administration to a recipient, is capable of providing, either directly or indirectly, a compound of this invention or an inhibitorily or degratorily active metabolite or residue thereof.

As used herein, the term "inhibitorily active metabolite or residue thereof" means that a metabolite or residue thereof is also an inhibitor of an IRAK protein kinase, or a mutant thereof.

As used herein, the term "degratorily active metabolite or residue thereof" means that a metabolite or residue thereof is also a degrader of an IRAK protein kinase, or a mutant thereof.

Compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, provided pharmaceutically acceptable compositions may be formulated in a suitable ointment containing the active component suspended or dissolved in one or more carriers. Carriers for topical administration of compounds of this invention include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, pharmaceutically acceptable compositions of this invention are formulated for oral administration. Such formulations may be administered with or without food. In some embodiments, pharmaceutically acceptable compositions of this invention are administered without food. In other embodiments, pharmaceutically acceptable compositions of this invention are administered with food.

The amount of compounds of the present invention that may be combined with the carrier materials to produce a composition in a single dosage form will vary depending upon the host treated, the particular mode of administration. Preferably, provided compositions should be formulated so that a dosage of between 0.01 - 100 mg/kg body weight/day of the compound can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

### Uses of Compounds and Pharmaceutically Acceptable Compositions

Compounds and compositions described herein are generally useful for the degradation and/or inhibition of kinase activity of one or more enzymes.

Examples of kinases that are degraded and/or inhibited by the compounds and compositions described herein and against which the methods described herein are useful include those of the interleukin-1 receptor-associated kinase (IRAK) family of kinases, the members of which include IRAK-1, IRAK-2, and IRAK-4, or a mutant thereof. Li et al., "IRAK-4: A novel member of the IRAK family with the properties of an IRAK-kinase," PNAS 2002, 99(8), 5567-5572, Flannery et al., " The interleukin-1 receptor-associated kinases: Critical regulators of innate immune signaling" Biochem Pharm 2010, 80(12), 1981-1991 incorporated by reference in its entirety.

The activity of a compound utilized in this invention as a degrader and/or inhibitor of IRAK-1, IRAK-2, and/or IRAK-4, or a mutant thereof, may be assayed in vitro, in vivo or in a cell line. In vitro assays include assays that determine inhibition of either the phosphorylation activity and/or the subsequent functional consequences, or ATPase activity of activated IRAK-1, IRAK-2, and/or IRAK-4, or a mutant thereof. Alternate in vitro assays quantitate the ability of the inhibitor to bind to IRAK-1, IRAK-2 and/or IRAK-4. Inhibitor binding may be measured by radiolabeling the inhibitor prior to binding, isolating the inhibitor/IRAK-1, inhibitor/IRAK-2, or inhibitor/IRAK-4 complex and determining the amount of radiolabel bound. Alternatively, inhibitor binding may be determined by running a competition experiment where new inhibitors are incubated with IRAK-1, IRAK-2, and/or IRAK-4 bound to known radioligands. Representative in vitro and in vivo assays useful in assaying an IRAK-4 inhibitor include those described and disclosed in, *e.g.,* Kim et al., "A critical role for IRAK4 kinase activity in Toll-like receptor-mediated innate immunity," J. Exp. Med. 2007 204(5), 1025-1036; Lebakken et al., "A Fluorescence Lifetime Based Binding Assay to Characterize Kinase Inhibitors," J. Biomol. Screen. 2007, 12(6), 828-841; Maschera et al., "Overexpression of an enzymatically inactive interleukin-1-receptor-associated kinase activates nuclear factor-κB," Biochem. J. 1999, 339, 227-231; Song et al., "The kinase activities of interleukin-e receptor associated kinase (IRAK)-1 and 4 are redundant in the control of inflammatory cytokine expression in human cells," Mol. Immunol. 2009, 46, 1458-1466, each of, the entirety of each of which is herein incorporated by reference. Detailed conditions for assaying a compound utilized in this invention as a degrader and/or inhibitor of IRAK-1, IRAK-2, and/or IRAK-4, or a mutant thereof, are set forth in the Examples below.

The best characterized member of the IRAK family is the serine/threonine kinase IRAK-4. IRAK-4 is implicated in signaling innate immune responses from Toll-like receptors (TLRs) and Toll/IL-1 receptors (TIRs).

Innate immunity detects pathogens through the recognition of pathogen-associated molecular patterns by TLRs, when then links to the adaptive immune response. TLRs recognize conserved structures of both microbes and endogenous molecules. TLRs which recognize bacterial and fungal components are located on the cell surface, whereas TLRs which recognize viral or microbial nucleic acids are localized to intracellular membranes such as endosomes and phagosomes. Cell surface TLRs can be targeted by small molecules and antibodies, whereas intracellular TLRs require targeting with oligonucleotides.

TLRs mediate the innate immune response by upregulating the expression of inflammatory genes in multiple target cells. *See, e.g.,* Sen et al., "Transcriptional signaling by double-stranded RNA: role of TLR3," Cytokine & Growth Factor Rev. 2005, 16, 1-14, incorporated by reference in its entirety. While TLR-mediated inflammatory response is critical for innate immunity and host defense against infections, uncontrolled inflammation is detrimental to the host leading to sepsis and chronic inflammatory diseases, such as chronic arthritis, atherosclerosis, multiple sclerosis, cancers, autoimmune disorders such as rheumatoid arthritis, lupus, asthma, psoriasis, and inflammatory bowel diseases.

Upon binding of a ligand, most TLRs recruit the adaptor molecule MyD88 through the TIR domain, mediating the MyD88-dependent pathway. MyD88 then recruits IRAK-4, which engages with the nuclear factor-κB (NF-κB), mitogen-activated protein (MAP) kinase and interferon-regulatory factor cascades and leads to the induction of pro-inflammatory cytokines. The activation of NF-κB results in the induction of inflammatory cytokines and chemokines, such as TNF-α, IL-1 α, IL-6 and IL-8. The kinase activity of IRAK-4 has been shown to play a critical role in the TLR-mediated immune and inflammatory responses. IRAK4 is a key mediator of the innate immune response orchestrated by interleukin-1 receptor (IL-1R), interleukin-18 receptor (IL-18R), IL-33 receptor (IL-33R), and Toll-like receptors (TLRs). Inactivation of IRAK-1 and/or IRAK-4 activity has been shown to result in diminished production of cytokines and chemokines in response to stimulation of IL-1 and TLR ligands. *See, e.g.,* Picard et al., "Clinical features and outcome of patients with IRAK-4 and MyD88 deficiency," Medicine (Baltimore), 2010, 89(6), 043-25; Li, "IRAK4 in TLR/IL-1R signaling: Possible clinical applications," Eur. J. Immunology 2008, 38:614-618; Cohen et al., "Targeting protein kinases for the development of antiinflammatory drugs," Curr. Opin. Cell Bio. 2009, 21:317-324; Flannery et al., "The interleukin-1 receptor-associated kinases: Critical regulators of innate immune signalling," Biochem. Pharm. 2010, 80(12), 1981-1991; Gottipati et al., "IRAK1: A critical signaling mediator of innate immunity," Cellular Signaling 2008, 20, 269-276; Kim et al., "A critical role for IRAK4 kinase activity in Toll-like receptor-mediated innate immunity," J. Exp. Med. 2007 204(5), 1025-1036; Koziczak-Holbro et al., "IRAK-4 Kinase Activity Is Required for Interleukin-1 (IL-1) Receptor- and Toll-like Receptor 7-mediated Signaling and Gene Expression," J. Biol. Chem. 2007, 282(18), 13552-13560; Kubo-Murai et al., "IRAK-4-dependent Degradation of IRAK-1 is a Negative Feedback Signal for TLR-mediated NF-κB Activation," J. Biochem. 2008, 143, 295-302; Maschera et al., "Overexpression of an enzymatically inactive interleukin-1-receptor-associated kinase activates nuclear factor-κB," Biochem. J. 1999, 339, 227-231; Lin et al., "Helical assembly in the MyD88-IRAK4-IRAK2 complex in TLR /IL-1R signalling," Nature 2010, 465(17), 885-891; Suzuki et al., "IRAK-4 as the central TIR signaling mediator in innate immunity," TRENDS in Immunol. 2002, 23(10), 503-506; Suzuki et al., "Severe impairment of interleukin-1 and Toll-like receptor signalling in mice lacking IRAK-4," Nature 2002, 416, 750-754; Swantek et al., "IL-1 Receptor-Associated Kinase Modulates Host Responsiveness to Endotoxin," J. Immunol. 2000, 164, 4301-4306; Hennessy, E., et al., "Targeting Toll-like receptors: emerging therapeutics?" Nature Reviews, vol. 9, pp: 293-307 (2010); Dinarello, C. "Interleukin-18 and the Pathogenesis of Inflammatory Diseases," Seminars in Nephrology, vol. 27, no. 1, pp: 98-114 (2007), each of, the entirety of each of which is herein incorporated by reference. In fact, knockdown mice that express a catalytically inactive mutant IRAK-4 protein are completely resistant to septic shock and show impaired IL-1 activity. Moreover, these mice are resistant to joint and bone inflammation/destruction in an arthritis model, suggesting that IRAK-4 may be targeted to treat chronic inflammation. Further, while IRAK-4 appears to be vital for childhood immunity against some pyogenic bacteria, it has been shown to play a redundant role in protective immunity to most infections in adults, as demonstrated by one study in which patients older than 14 lacking IRAK-4 activity exhibited no invasive infections. Cohen et al., "Targeting protein kinases for the development of anti-inflammatory drugs," Curr. Opin. Cell Bio. 2009, 21:317-324; Ku et al., "Selective predisposition to bacterial infections in IRAK-4-deficient children: IRAK-4-dependent TLRs are otherwise redundant in protective immunity," J. Exp. Med. 2007, 204(10), 2407-2422; Picard et al., "Inherited human IRAK-4 deficiency: an update," Immunol. Res. 2007, 38, 347-352; Song et al., "The kinase activities of interleukin-e receptor associated kinase (IRAK)-1 and 4 are redundant in the control of inflammatory cytokine expression in human cells," Mol. Immunol. 2009, 46, 1458-1466; Rokosz, L. et al., "Kinase inhibitors as drugs for chronic inflammatory and immunological diseases: progress and challenges," Expert Opinions on Therapeutic Targets, 12(7), pp: 883-903 (2008); Gearing, A. "Targeting toll-like receptors for drug development: a summary of commercial approaches," Immunology and Cell Biology, 85, pp: 490-494 (2007); Dinarello, C. "IL-1: Discoveries, controversies and future directions," European Journal of Immunology, 40, pp: 595-653 (2010), each of, the entirety of each of which is herein incorporated by reference. Because TLR activation triggers IRAK-4 kinase activity, IRAK-4 inhibition presents an attractive target for treating the underlying causes of inflammation in countless diseases.

Representative IRAK-4 inhibitors include those described and disclosed in *e.g.,* Buckley et al., Bioorg. Med. Chem. Lett. 2008, 18, 3211-3214; Buckley et al., Bioorg. Med. Chem. Lett. 2008, 18, 3291-3295; Buckley et al., Bioorg. Med. Chem. Lett. 2008, 18, 3656-3660; Powers et al., "Discovery and initial SAR of inhibitors of interleukin-1 receptor-associated kinase-4," Bioorg. Med. Chem. Lett. 2006, 16, 2842-2845; Wng et al., "IRAK-4 Inhibitors for Inflammation," Curr. Topics in Med. Chem. 2009, 9, 724-737, each of, the entirety of each of which is herein incorporated by reference.

As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Provided compounds are degraders and/or inhibitors of one of more of IRAK-1, IRAK-2, and/or IRAK-4 and are therefore useful for treating one or more disorders associated with activity of one or more of IRAK-1, IRAK-2, and/or IRAK-4. Thus, in certain embodiments, the present invention provides a method for treating a IRAK-1-mediated, a IRAK-2-mediated, and/or a IRAK-4-mediated disorder comprising the step of administering to a patient in need thereof a compound of the present invention, or pharmaceutically acceptable composition thereof.

As used herein, the terms "IRAK-1-mediated", "IRAK-2-mediated", and/or "IRAK-4-mediated" disorders, diseases, and/or conditions as used herein means any disease or other deleterious condition in which one or more of IRAK-1, IRAK-2, and/or IRAK-4, or a mutant thereof, are known to play a role. Accordingly, another embodiment of the present invention relates to treating or lessening the severity of one or more diseases in which one or more of IRAK-1, IRAK-2, and/or IRAK-4, or a mutant thereof, are known to play a role.

In some embodiments, the present invention provides a method for treating one or more disorders, diseases, and/or conditions wherein the disorder, disease, or condition is a cancer, a neurodegenative disorder, a viral disease, an autoimmune disease, an inflammatory disorder, a hereditary disorder, a hormone-related disease, a metabolic disorder, conditions associated with organ transplantation, immunodeficiency disorders, a destructive bone disorder, a proliferative disorder, an infectious disease, a condition associated with cell death, thrombin-induced platelet aggregation, liver disease, pathologic immune conditions involving T cell activation, a cardiovascular disorder, or a CNS disorder.

Diseases and conditions treatable according to the methods of this invention include, but are not limited to, cancer (*see, e.g.,* Ngo, V. et al., "Oncogenically active MYD88 mutations in human lymphoma," Nature, vol. 000, pp: 1-7 (2010); Lust, J. et al., "Induction of a Chronic Disease State in patients With Smoldering of Indolent Multiple Myeloma by Targeting Interleukin 1β-Induced Interleukin 6 Production and the Myeloma Proliferative Component," Mayo Clinic Proceedings, 84(2), pp: 114-122 (2009)), diabetes, cardiovascular disease, viral disease, autoimmune diseases such as lupus *(see, e.g.,* Dinarello, C. " Interleukin-18 and the Pathogenesis of Inflammatory Diseases," Seminars in Nephrology, vol. 27, no. 1, pp: 98-114 (2007); Cohen et al., "Targeting protein kinases for the development of anti-inflammatory drugs," Curr. Opin. Cell Bio. 2009, 21:317-324) and rheumatoid arthritis *(see, e.g.,* Geyer, M. et al., "Actual status of antiinterleukin-1 therapies in rheumatic diseases," Current Opinion in Rheumatology, 22, pp: 246-251 (2010)), autoinflammatory syndromes *(see, e.g.,* Hoffman, H. et al., "Efficacy and Safety of Rilonacept (Interleukin-1 Trap) in Patients with Cryopyrin-Associated Periodic Syndromes," Arthritis & Rheumatism, vol. 58, no. 8, pp: 2443-2452 (2008)), atherosclerosis, psoriasis, allergic disorders, inflammatory bowel disease *(see, e.g.,* Cario, E. "Therapeutic Impact of Toll-like Receptors on Inflammatory Bowel Diseases: A Multiple-edged Sword," Inflamm. Bowel Dis., 14, pp: 411-421 (2008)), inflammation *(see, e.g.,* Dinarello, C. "Interleukin 1 and interleukin 18 as mediators of inflammation and the aging process, " The American Journal of Clinical Nutrition, 83, pp: 447S-455S (2006)), acute and chronic gout and gouty arthritis (*see, e.g.,* Terkeltaub, R. "Update on gout: new therapeutic strategies and options," Nature, vol. 6, pp: 30-38 (2010); Weaver, A. "Epidemiology of gout," Cleveland Clinic Journal of Medicine, vol. 75, suppl. 5, pp: S9-S12 (2008); Dalbeth, N. et al., "Hyperuricaemia and gout: state of the art and future perspectives," Annals of Rheumatic Diseases, 69, pp: 1738-1743 (2010); Martinon, F. et al., "Goutassociated uric acid crystals activate the NALP3 inflammasome," Nature, vol. 440, pp: 237-241

(2006); So, A. et al., "A pilot study of IL-1 inhibition by anakinra in acute gout," Arthritis Research & Therapy, vol. 9, no. 2, pp: 1-6 (2007); Terkeltaub, R. et al., "The interleukin 1 inhibitor rilonacept in treatment of chronic gouty arthritis: results of a placebo-controlled, monosequence crossover, non-randomised, single-blind pilot study," Annals of Rheumatic Diseases, 68, pp: 1613-1617 (2009); Torres, R. et al., "Hyperalgesia, synovitis and multiple biomarkers of inflammation are suppressed by interleukin 1 inhibition in a novel animal model of gouty arthritis," Annals of Rheumatic Diseases, 68, pp: 1602-1608 (2009)), neurological disorders, metabolic syndrome (*see, e.g.,* Troseid, M. "The role of interleukin-18 in the metabolic syndrome," Cardiovascular Diabetology, 9:11, pp:1-8 (2010)), immunodeficiency disorders such as AIDS and HIV (*see, e.g.,* Iannello, A. et al., "Role of Interleukin-18 in the Development and Pathogenesis of AIDS," AIDS Reviews, 11, pp: 115-125 (2009)), destructive bone disorders (*see, e.g.,* Hennessy, E., et al., "Targeting Toll-like receptors: emerging therapeutics?" Nature Reviews, vol. 9, pp: 293-307 (2010)), osteoarthritis, proliferative disorders, Waldenström's Macroglobulinemia (*see, e.g.,* Treon, et al., "Whole genome sequencing reveals a widely expressed mutation (MYD88 L265P) with oncogenic activity in Waldenström's Macroglobulinemia" 53rd ASH Annual Meeting; Xu, et al., "A somatic variant in MYD88 (L256P) revealed by whole genome sequencing differentiates lymphoplasmacytic lymphoma from marginal zone lymphomas" 53rd ASH Annual Meeting; Yang et al., "Disruption of MYD88 pathway signaling leads to loss of constitutive IRAK1, NK-kB and JAK/STAT signaling and induces apoptosis of cells expressing the MYD88 L265P mutation in Waldenström's Macroglobulinemia" 53rd ASH Annual Meeting; Iriyama et al., "Clinical significance of genetic mutations of CD79B, CARD11, MYD88, and EZH2 genes in diffuse large B-cell lymphoma patients" 53rd ASH Annual Meeting; infectious diseases, conditions associated with cell death, pathologic immune conditions involving T cell activation, and CNS disorders in a patient. In one embodiment, a human patient is treated with a compound of the current invention and a pharmaceutically acceptable carrier, adjuvant, or vehicle, wherein said compound is present in an amount to measurably degrade and/or inhibit IRAK-1 only, IRAK-2-only, IRAK-4-only and/or IRAK1 and IRAK4 kinase activity.

Compounds of the current invention are useful in the treatment of a proliferative disease selected from a benign or malignant tumor, solid tumor, carcinoma of the brain, kidney, liver, adrenal gland, bladder, breast, stomach, gastric tumors, ovaries, colon, rectum, prostate, pancreas, lung, vagina, cervix, testis, genitourinary tract, esophagus, larynx, skin, bone or thyroid, sarcoma, glioblastomas, neuroblastomas, multiple myeloma, gastrointestinal cancer, especially colon carcinoma or colorectal adenoma, a tumor of the neck and head, an epidermal hyperproliferation, psoriasis, prostate hyperplasia, a neoplasia, a neoplasia of epithelial character, adenoma, adenocarcinoma, keratoacanthoma, epidermoid carcinoma, large cell carcinoma, non-small-cell lung carcinoma, lymphomas, Hodgkins and Non-Hodgkins, a mammary carcinoma, follicular carcinoma, undifferentiated carcinoma, papillary carcinoma, seminoma, melanoma, an IL-1 driven disorder, an MyD88 driven disorder, Smoldering of indolent multiple myeloma, or hematological malignancies (including leukemia, diffuse large B-cell lymphoma (DLBCL), ABC DLBCL, chronic lymphocytic leukemia (CLL), chronic lymphocytic lymphoma, primary effusion lymphoma, Burkitt lymphoma/leukemia, acute lymphocytic leukemia, B-cell prolymphocytic leukemia, lymphoplasmacytic lymphoma, Waldenström's macroglobulinemia (WM), splenic marginal zone lymphoma, multiple myeloma, plasmacytoma, intravascular large B-cell lymphoma, AML, MDS).

In some embodiments the proliferative disease which can be treated according to the methods of this invention is an MyD88 driven disorder. In some embodiments, the MyD88 driven disorder which can be treated according to the methods of this invention is selected from ABC DLBCL, primary CNS lymphomas, primary extranodal lymphomas, Waldenström's macroglobulinemia, Hodgkin's lymphoma, primary cutaneous T-cell lymphoma and chronic lymphocytic leukemia.

In some embodiments the proliferative disease which can be treated according to the methods of this invention is an IL-1 driven disorder. In some embodiments the IL-1 driven disorder is Smoldering of indolent multiple myeloma.

Compounds according to the invention are useful in the treatment of inflammatory or obstructive airways diseases, resulting, for example, in reduction of tissue damage, airways inflammation, bronchial hyperreactivity, remodeling or disease progression. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics.

Compounds according to the invention are useful in the treatment of heteroimmune diseases. Examples of such heteroimmune diseases include, but are not limited to, graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis.

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, such as therapy for or intended to restrict or abort symptomatic attack when it occurs, for example antiinflammatory or bronchodilatory. Prophylactic benefit in asthma may in particular be apparent in subjects prone to "morning dipping". "Morning dipping" is a recognized asthmatic syndrome, common to a substantial percentage of asthmatics and characterised by asthma attack, e.g. between the hours of about 4 to 6 am, i.e. at a time normally substantially distant form any previously administered symptomatic asthma therapy.

Compounds of the current invention can be used for other inflammatory or obstructive airways diseases and conditions to which the present invention is applicable and include acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, but not limited to, acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

With regard to their anti-inflammatory activity, in particular in relation to inhibition of eosinophil activation, compounds of the invention are also useful in the treatment of eosinophil related disorders, e.g. eosinophilia, in particular eosinophil related disorders of the airways (e.g. involving morbid eosinophilic infiltration of pulmonary tissues) including hypereosinophilia as it effects the airways and/or lungs as well as, for example, eosinophil- related disorders of the airways consequential or concomitant to Loffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma, eosinophilic asthma, eosinophilic COPD, and eosinophil-related disorders affecting the airways occasioned by drug-reaction.

Compounds of the invention are also useful in the treatment of inflammatory or allergic conditions of the skin, for example psoriasis, generalized pustular psoriasis (GPP), psoriasis vulgaris, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, systemic lupus erythematosus, pemphigus vulgaris, pemphigus foliaceus, paraneoplastic pemphigus, epidermolysis bullosa acquisita, acne vulgaris, hidradenitis suppurativa, Sweet Syndrome, pyoderma gangrenosum, and other inflammatory or allergic conditions of the skin.

Compounds of the invention may also be used for the treatment of other diseases or conditions, such as diseases or conditions having an inflammatory component, for example, treatment of diseases and conditions of the eye such as ocular allergy, conjunctivitis, keratoconjunctivitis sicca, and vernal conjunctivitis, diseases affecting the nose including allergic rhinitis, and inflammatory disease in which autoimmune reactions are implicated or having an autoimmune component or etiology, including autoimmune hematological disorders (e.g. hemolytic anemia, aplastic anemia, pure red cell anemia and idiopathic thrombocytopenia), systemic lupus erythematosus, rheumatoid arthritis, polychondritis, scleroderma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), irritable bowel syndrome, celiac disease, periodontitis, hyaline membrane disease, kidney disease, glomerular disease, alcoholic liver disease, multiple sclerosis, endocrine opthalmopathy, Grave's disease, sarcoidosis, alveolitis, chronic hypersensitivity pneumonitis, multiple sclerosis, primary biliary cirrhosis, uveitis (anterior and posterior), Sjogren's syndrome, keratoconjunctivitis sicca and vernal keratoconjunctivitis, interstitial lung fibrosis, psoriatic arthritis, systemic juvenile idiopathic arthritis, cryopyrin-associated periodic syndrome, nephritis, vasculitis, diverticulitis, interstitial cystitis, glomerulonephritis (with and without nephrotic syndrome, e.g. including idiopathic nephrotic syndrome or minal change nephropathy), chronic granulomatous disease, endometriosis, leptospiriosis renal disease, glaucoma, retinal disease, ageing, headache, pain, complex regional pain syndrome, cardiac hypertrophy, musclewasting, catabolic disorders, obesity, fetal growth retardation, hyperchlolesterolemia, heart disease, chronic heart failure, mesothelioma, anhidrotic ecodermal dysplasia, Behcet's disease, incontinentia pigmenti, Paget's disease, pancreatitis, hereditary periodic fever syndrome, asthma (allergic and non-allergic, mild, moderate, severe, bronchitic, and exercise-induced), acute lung injury, acute respiratory distress syndrome, eosinophilia, hypersensitivities, anaphylaxis, nasal sinusitis, ocular allergy, silica induced diseases, COPD (reduction of damage, airways inflammation, bronchial hyperreactivity, remodeling or disease progression), pulmonary disease, cystic fibrosis, acidinduced lung injury, pulmonary hypertension, polyneuropathy, cataracts, muscle inflammation in conjunction with systemic sclerosis, inclusion body myositis, myasthenia gravis, thyroiditis, Addison's disease, lichen planus, Type 1 diabetes, or Type 2 diabetes, appendicitis, atopic dermatitis, asthma, allergy, blepharitis, bronchiolitis, bronchitis, bursitis, cervicitis, cholangitis, cholecystitis, chronic graft rejection, colitis, conjunctivitis, Crohn's disease, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, or vulvitis.

In some embodiments the inflammatory disease which can be treated according to the methods of this invention is an disease of the skin. In some embodiments, the inflammatory disease of the skin is selected from contact dermatitits, atompic dermatitis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, hidradenitis suppurativa, and other inflammatory or allergic conditions of the skin.

In some embodiments the inflammatory disease which can be treated according to the methods of this invention is selected from acute and chronic gout, chronic gouty arthritis, psoriasis, psoriatic arthritis, rheumatoid arthritis, Juvenile rheumatoid arthritis, Systemic jubenile idiopathic arthritis (SJIA), Cryopyrin Associated Periodic Syndrome (CAPS), Adult Onset Still's disease, macrophage activation syndrome (MAS), primary and secondary hemophagocytic lymphohistiocytosis (HLH), Familial Mediterranean Fever, NLRP12 autoinflammatory syndrome, and osteoarthritis.

In some embodiments the inflammatory disease which can be treated according to the methods of this invention is a TH17 mediated disease. In some embodiments the TH17 mediated disease is selected from Systemic lupus erythematosus, Multiple sclerosis, psoriasis vulgaris, hidradenitis suppurativa, and inflammatory bowel disease (including Crohn's disease or ulcerative colitis).

In some embodiments the inflammatory disease which can be treated according to the methods of this invention is selected from Sjogren's syndrome, allergic disorders, osteoarthritis, conditions of the eye such as ocular allergy, conjunctivitis, keratoconjunctivitis sicca and vernal conjunctivitis, and diseases affecting the nose such as allergic rhinitis or chronic rhinosinusitis with nasal polyps (CRSwNP).

Cardiovascular diseases which can be treated according to the methods of this invention include, but are not limited to, restenosis, cardiomegaly, atherosclerosis, myocardial infarction, ischemic stroke, congestive heart failure, angina pectoris, reocclusion after angioplasty, restenosis after angioplasty, reocclusion after aortocoronary bypass, restenosis after aortocoronary bypass, stroke, transitory ischemia, a peripheral arterial occlusive disorder, pulmonary embolism, and deep venous thrombosis.

In some embodiments, the neurodegenerative disease which can be treated according to the methods of this invention include, but are not limited to, Alzheimer's disease, Parkinson's disease, amyotrophic lateral sclerosis, Huntington's disease, cerebral ischemia, and neurodegenerative disease caused by traumatic injury, glutamate neurotoxicity, hypoxia, epilepsy, treatment of diabetes, metabolic syndrome, obesity, organ transplantation and graft versus host disease.

The loss of IRAK4 function results in decreased Aβ levels in an in vivo murine model of Alzheimer's disease and was associated with diminished microgliosis and astrogliosis in aged mice. Analysis of microglia isolated from the adult mouse brain revealed an altered pattern of gene expression associated with changes in microglial phenotype that were associated with expression of IRF transcription factors that govern microglial phenotype. Further, loss of IRAK4 function also promoted amyloid clearance mechanisms, including elevated expression of insulin-degrading enzyme. Finally, blocking IRAK function restored olfactory behavior (Cameron et al. "Loss of Interleukin Receptor-Associated Kinase 4 Signaling Suppresses Amyloid Pathology and Alters Microglial Phenotype in a Mouse Model of Alzheimer's Disease" Journal of Neuroscience (2012) 32(43), 15112-15123).

In some embodiments the invention provides a method of treating, preventing or lessening the severity of Alzheimer's disease comprising administering to a patient in need thereof a provided compound or a pharmaceutically acceptable salt or composition thereof.

In some embodiments the invention provides a method of treating a disease condition commonly occurring in connection with transplantation. In some embodiments, the disease or condition commonly occurring in connection with transplantation is selected from organ transplantation, organ transplant rejection, and graft versus host disease.

In some embodiments the invention provides a method of treating a metabolic disease. In some embodiments the metabolic disease is selected from Type 1 diabetes, Type 2 diabetes, metabolic syndrome, and obesity.

In some embodiments the invention provides a method of treating a viral disease. In some embodiments, the viral infection is HIV infection.

Furthermore, the invention provides the use of a compound according to the definitions herein, or a pharmaceutically acceptable salt, or a hydrate or solvate thereof for the preparation of a medicament for the treatment of a proliferative disease, an inflammatory disease, an obstructive respiratory disease, a cardiovascular disease, a metabolic disease, a neurological disease, a neurodegenerative disease, a viral disease, or a disorder commonly occurring in connection with transplantation.

### Multiple Degradation

In some embodiments, the invention provides compounds that modulate targeted ubiquitination and degradation of one or more IRAK kinase. In some embodiments, a provided compound modulates targeted ubiquitination and degradation of one or more IRAK kinase and one or more additional protein. In some instances, a provided compound modulates targeted ubiquitination and degradation of IRAK4 and one, two, three, four, or five additional proteins.

In certain embodiments, the invention provides compounds that are triple degraders. In certain embodiments, the invention provides compounds that combine IRAK kinase degradation with IKZF1 and IKZF3 degradation. Some of the most commonly employed E3 ligase ligands are thalidomide and its derivatives, lenalidomide and pomalidomide, commonly referred to as IMiDs (immunomodulatory imide drugs). These agents are small-molecule ligands of cereblon (CRBN) (Ito et al. "Identification of a primary target of thalidomide teratogenicity" Science 2010, 327(5971):1345-1350), a substrate adaptor for the ubiquitously expressed cullin ring ligase 4 (CUL4)-RBX1-DDB1-CRBN (CUL4CRBN) E3 ligase. It has been shown that thalidomide interacts with CRBN to form a novel surface, resulting in interactions with neosubstrates such as Ikaros (IKZF1) and Aiolos (IKZF3) and their ubiquitination and subsequent proteasomal degradation (Krönke et al. "Lenalidomide causes selective degradation of IKZF1 and IKZF3 in multiple myeloma cells" Science 2014, 343(6168):301-305; and Lu et al. "The myeloma drug lenalidomide promotes the cereblon-dependent destruction of Ikaros proteins" Science, 2014; 343(6168):305-309). This activity alone has potent antitumor effects in some liquid malignancies, and lenalidomide (Revlimid^{®}) is US Food and Drug Administration approved for the treatment of MCL, multiple myeloma, and myelodysplastic syndromes with deletion of chromosome 5q. Lenalidomide is also undergoing late-stage clinical trials for a number of lymphomas, including MCL and the activated B-cell subtype of diffuse large B-cell lymphoma (ABC DLBCL).

In some instances, degradation of IRAK4 alone is not sufficient to kill the MYD88 L265P mutant DLBCL cell line OCI-LY10 either in vitro or as a flank xenograft in vivo. **Table 2** shows that several IRAK4 binding moieties coupled to non-IMiD CRBN binders mediate effective knockdown of IRAK4 but have little to no effect on the viability of MYD88 mutant ABC-DLBCL cell lines OCI-LY10 and SU-DHL-2 in vitro.

**Table 2. IRAK4 degradation alone is insufficient to kill MYD88 mutant DLBCL cell lines in vitro**

| | **I-75** | **I-257** | **I-229** | **I-191** |
|---|---|---|---|---|
| IRAK4 Degradation in OCI-LY10, DC₅₀ (nM) | 8 | 5 | 26 | 4 |

| **MYD88 Mutant Lines** | | | | |
|---|---|---|---|---|
| OCI-LY10 Viability, CTG IC₅₀ (nM) | 11 | 1,200 | 3,500 | >10,000 |
| SU-DHL2 Viability, CTG IC₅₀ (nM) | 290 | 6,900 | >10,000 | >10,000 |

| **MYD88 WT Lines** | | | | |
|---|---|---|---|---|
| SU-DHL6 Viability, CTG IC₅₀ (nM) | 1,600 | 5,600 | 4,100 | 5,600 |
| U2932 Viability, CTG IC₅₀ (nM) | 374 | >10,000 | >10,000 | >10,000 |
| OCI-LY19 Viability, CTG IC₅₀ (nM) | 480 | 4,700 | >10,000 | >10,000 |

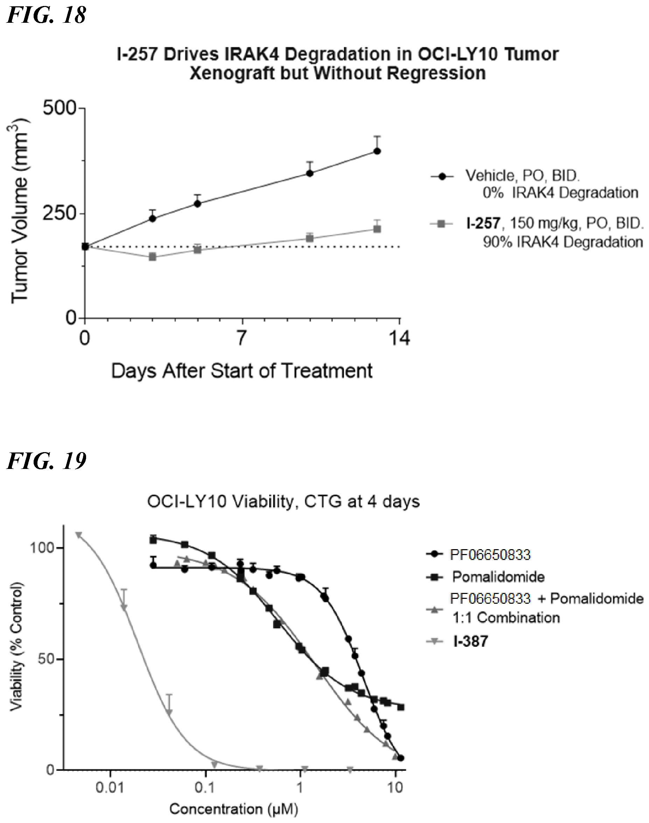

FIG. 18 shows that **I-257** dosing (PO, BID) effects 90% IRAK4 degradation in OCI-LY10 tumor xenograft but without causing regression. This is consistent with literature demonstrating no effect on growth of OCI-LY10 or other MYD88 mutant lines when the gene encoding IRAK4 is removed at the DNA level using CRISPR/Cas9 editing (Phelan et al. "A multiprotein supercomplex controlling oncogenic signaling in lymphoma" Nature, 2018, 7718:387-391).

It has been shown that activating MYD88 mutations increase production of beta-IFN, a pro-apoptotic cytokine, in ABC-DLBCL cells (Yang et al. "Exploiting synthetic lethality for the therapy of ABC diffuse large B cell lymphoma" Cancer Cell 2012, 21(6):723-737). The cells are rendered resistant to this effect by a concomitant MYD88-driven activation of NFkB signaling via IRF4 and SPIB transactivating CARD11 (Yang, Cancer Cell 2012). IMiDs are also known to increase the IFN response in MYD88 mutant ABC-DLBCL to levels sufficient to increase apoptosis (Yang, Cancer Cell 2012; and Hagner et al. "CC-122, a pleiotropic pathway modifier, mimics an interferon response and has antitumor activity in DLBCL" Blood 2015, 126:779-789). This effect has been shown to synergize with inhibition of NFkB signaling to further drive DLBCL cell death (Yang, Cancer Cell 2012).

In some instances, the combination of an IMiD with a small molecule IRAK4 kinase inhibitor shows little to no additive effect on viability of the MYD88 mutant ABC DLBCL cell line OCI-LY10. FIG. 19 shows that the combination of an IRAK4 inhibitor with IMiD is less active than an all-in-one IMiD-based IRAK4 degrader.

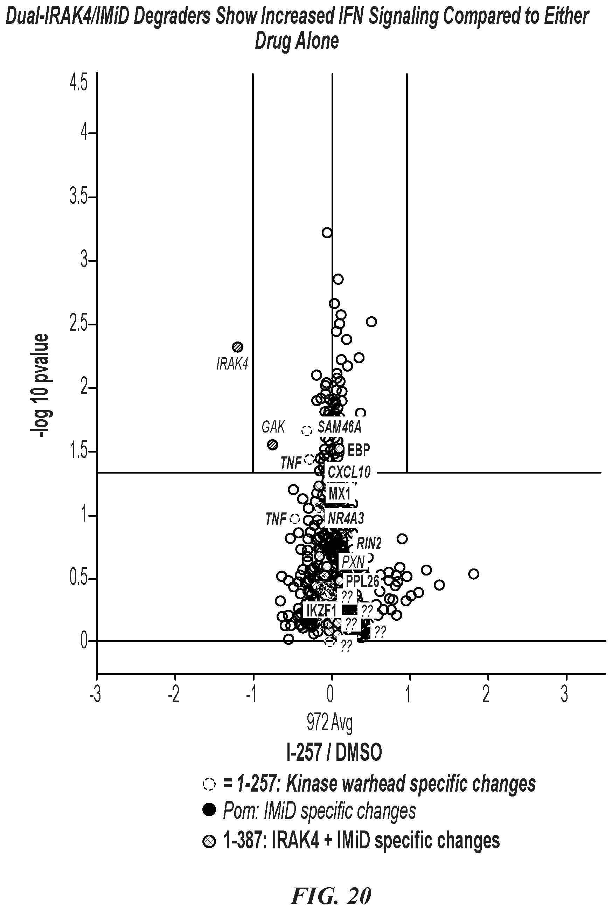

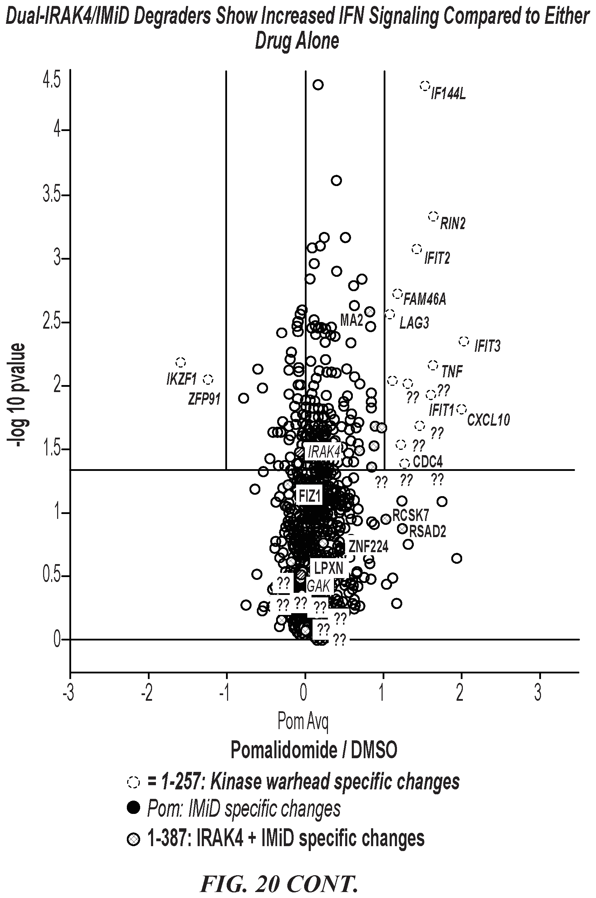

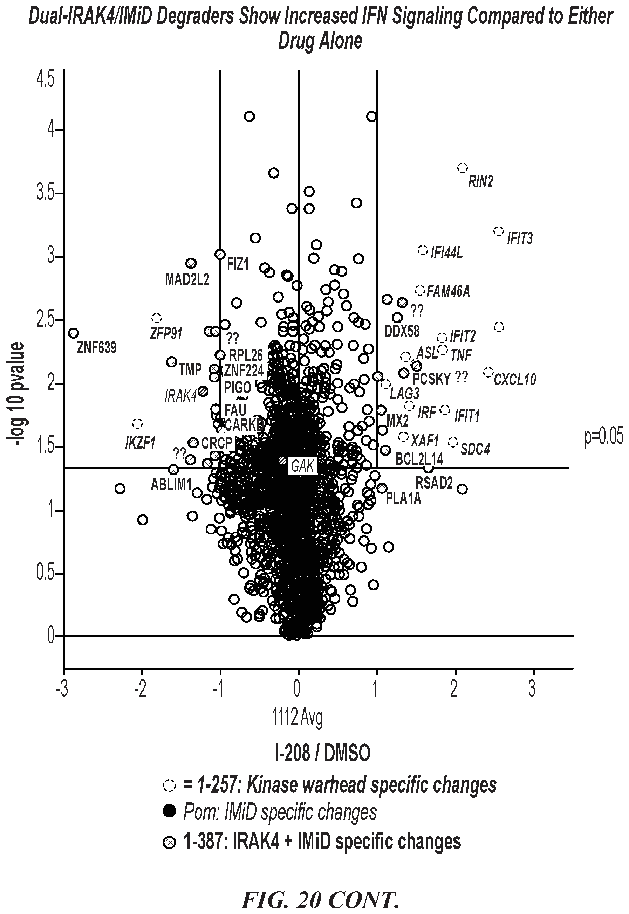

In certain embodiments, the combination of IRAK kinase degradation with IKZF1 and IKZF3 degradation in an all-in-one IMiD-based IRAK4 degrader shows potent, single agent activity versus MYD88 mutant ABC DLBCL cell lines *in vitro* and OCI-LY10 xenograft *in vivo.* FIG. 20 shows that the all-in-one combination of an IMiD-based CRBN-binder and an IRAK4 binding moiety yields IRAK4 degraders that retain degradation of Ikaros (IKZF1) and other known IMiDs neosubstrates, while more strongly inducing an interferon response compared to pomalidomide alone. Surprisingly, these IMiD-based IRAK4 degraders are potent at killing MYD88 mutant ABD-DLBCL cell lines *in vitro* (Table 3), demonstrating increased activity versus that obtained from combining an IRAK4 inhibitor with IMiDs as single agents as shown in FIG. 19.

**Table 3. Substituting pomalidomide as ligase binder enables MYD88-dependent single-agent activity**

| | | **I-75** | **I-168** | **I-257** | **I-208** |
|---|---|---|---|---|---|
| IRAK4 OCI-LY10 DC₅₀ (nM) | | 8 | 20 | 5 | 20 |
| IRAK4 WB DC₅₀ (nM) | | 23 | 78 | 3 | 130 |
| IKAROS OCI-LY10 DC₅₀ (nM) | | >100 | 5 | - | 3 |
| AIOLOS OCI-LY10 DC₅₀ (nM) | | >100 | 5 | - | <3 |
| **MYD88^{MUT}** | OCI-LY10 CTG IC₅₀ (nM) | 11 | 31 | 1,200 | 36 |
| | SU-DHL2 CTG IC₅₀ (nM) | 290 | 64 | 6,900 | 366 |
| | OCI-LY3 CTG IC₅₀ (nM) | 180 | 290 | - | 3000 |
| **MYD88^{WT}** | SU-DHL6 CTG IC₅₀ (nM) | 1,600 | 1,900 | 5,600 | 833 |
| | **U2932 CTG IC₅₀ (nM)** | 374 | 410 | 10,000 | 1,800 |
| | **OCI-LY19 CTG IC₅₀ (nM)** | 480 | 430 | 4,700 | 1,700 |

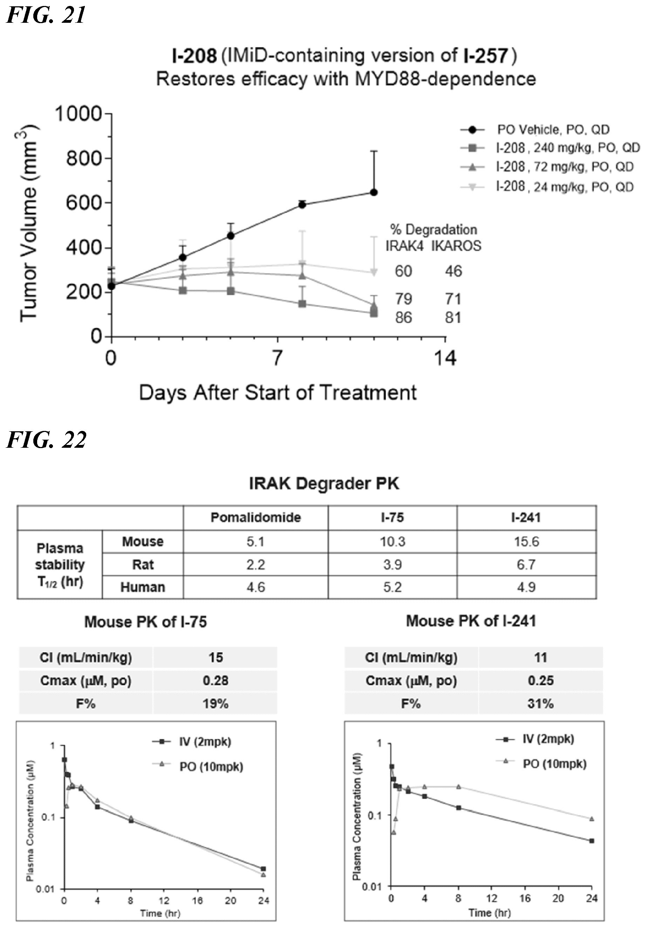

In certain embodiments, a provided compound comprising a IMiD-based E3 ligase (e.g., **I-208**) degrades IRAK4, Ikaros, and Aiolos in OCI-LY10 xenografts in vivo, and strongly induces a signature of interferon-driven proteins exemplified by IFIT1 (interferon-inducible transcript 1) and IFIT3 (interferon-inducible transcript 3) as shown in Table 4. FIG. 21 shows that **I-208** drives regression of these OCI-LY10 tumor xenographs as a single agent.

**Table 4. OCI-LY10 tumor xenograft regression using I-208 (regression results in bold font)**

| Dose (mg/ kg) | Route | Sched | Sample time (h) | **OCI-LY10 Tumor Xenograft** | | | | |
|---|---|---|---|---|---|---|---|---|
| | | | | IRAK4 ↓ (% Control) | Ikaros ↓ (% Control) | Aiolos ↓ (% Control) | Interferon Response | |
| | | | | | | | IFIT1 Increase (Fold) | IFIT3 Increase (Fold) |
| 24 | PO | QD | 24 | 57.8 | 46.3 | 30.9 | >24.4 | >43.1 |
| **72** | **PO** | **QD** | **24** | **78.7** | **71.0** | **62.4** | **>41.5** | **>64.0** |
| **240** | **PO** | **QD** | **24** | **86.5** | **81.2** | **68.6** | **>38.7** | **>59.7** |

In some embodiments, the provided compounds of present invention highlight a synergy obtained by combining IRAK4 degradation with IMiD induction of interferon response to drive single agent anti-tumor activity in MYD88 mutant DLBCL and possibly in other heme malignancies. In certain embodiments, a provided compound comprising an IMiD-based E3 ligase degrade IRAK4, Ikaros, and Aiolos acts synergistically. In some embodiments, a provided compound comprising an IRAK4 binder and an IMiD-based E3 ligase degrades IRAK4, Ikaros, and Aiolos with increased activity in comparison to a provided compound comprising the same IRAK4 binder and a non-IMiD-based E3 ligase and the same IMiD-based E3 ligase as a single agent.

### Combination Therapies

Depending upon the particular condition, or disease, to be treated, additional therapeutic agents, which are normally administered to treat that condition, may be administered in combination with compounds and compositions of this invention. As used herein, additional therapeutic agents that are normally administered to treat a particular disease, or condition, are known as "appropriate for the disease, or condition, being treated."

In certain embodiments, a provided combination, or composition thereof, is administered in combination with another therapeutic agent.

In some embodiments, the present invention provides a method of treating a disclosed disease or condition comprising administering to a patient in need thereof an effective amount of a compound disclosed herein or a pharmaceutically acceptable salt thereof and co-administering simultaneously or sequentially an effective amount of one or more additional therapeutic agents, such as those described herein. In some embodiments, the method includes co-administering one additional therapeutic agent. In some embodiments, the method includes co-administering two additional therapeutic agents. In some embodiments, the combination of the disclosed compound and the additional therapeutic agent or agents acts synergistically.

Examples of agents the combinations of this invention may also be combined with include, without limitation: treatments for Alzheimer's Disease such as Aricept^{®} and Excelon^{®}; treatments for HIV such as ritonavir; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; anti-inflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; agents that prolong or improve pharmacokinetics such as cytochrome P450 inhibitors (i.e., inhibitors of metabolic breakdown) and CYP3A4 inhibitors (e.g., ketokenozole and ritonavir), and agents for treating immunodeficiency disorders such as gamma globulin.

In certain embodiments, combination therapies of the present invention, or a pharmaceutically acceptable composition thereof, are administered in combination with a monoclonal antibody or an siRNA therapeutic.

Those additional agents may be administered separately from a provided combination therapy, as part of a multiple dosage regimen. Alternatively, those agents may be part of a single dosage form, mixed together with a compound of this invention in a single composition. If administered as part of a multiple dosage regime, the two active agents may be submitted simultaneously, sequentially or within a period of time from one another normally within five hours from one another.

As used herein, the term "combination," "combined," and related terms refers to the simultaneous or sequential administration of therapeutic agents in accordance with this invention. For example, a combination of the present invention may be administered with another therapeutic agent simultaneously or sequentially in separate unit dosage forms or together in a single unit dosage form.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

One or more other therapeutic agent may be administered separately from a compound or composition of the invention, as part of a multiple dosage regimen. Alternatively, one or more other therapeutic agents agents may be part of a single dosage form, mixed together with a compound of this invention in a single composition. If administered as a multiple dosage regime, one or more other therapeutic agent and a compound or composition of the invention may be administered simultaneously, sequentially or within a period of time from one another, for example within 1, 2, 3, 4, 5, 6, 7, 8, 9, 10, 11, 12, 13, 14, 15, 16, 17, 18, 18, 20, 21, 22, 23, or 24 hours from one another. In some embodiments, one or more other therapeutic agent and a compound or composition of the invention are administerd as a multiple dosage regimen within greater than 24 hours aparts.

In one embodiment, the present invention provides a composition comprising a provided compound or a pharmaceutically acceptable salt thereof and one or more additional therapeutic agents. The therapeutic agent may be administered together with a provided compound or a pharmaceutically acceptable salt thereof, or may be administered prior to or following administration of a provided compound or a pharmaceutically acceptable salt thereof. Suitable therapeutic agents are described in further detail below. In certain embodiments, a provided compound or a pharmaceutically acceptable salt thereof may be administered up to 5 minutes, 10 minutes, 15 minutes, 30 minutes, 1 hour, 2 hours, 3 hours, 4 hours, 5, hours, 6 hours, 7 hours, 8 hours, 9 hours, 10 hours, 11 hours, 12 hours, 13 hours, 14 hours, 15 hours, 16 hours, 17 hours, or 18 hours before the therapeutic agent. In other embodiments, a provided compound or a pharmaceutically acceptable salt thereof may be administered up to 5 minutes, 10 minutes, 15 minutes, 30 minutes, 1 hour, 2 hours, 3 hours, 4 hours, 5, hours, 6 hours, 7 hours, 8 hours, 9 hours, 10 hours, 11 hours, 12 hours, 13 hours, 14 hours, 15 hours, 16 hours, 17 hours, or 18 hours following the therapeutic agent.

In another embodiment, the present invention provides a method of treating an inflammatory disease, disorder or condition by administering to a patient in need thereof a provided compound or a pharmaceutically acceptable salt thereof and one or more additional therapeutic agents. Such additional therapeutic agents may be small molecules or recombinant biologic agents and include, for example, acetaminophen, non-steroidal anti-inflammatory drugs (NSAIDS) such as aspirin, ibuprofen, naproxen, etodolac (Lodine^{®}) and celecoxib, colchicine (Colcrys^{®}), corticosteroids such as prednisone, prednisolone, methylprednisolone, hydrocortisone, and the like, probenecid, allopurinol, febuxostat (Uloric^{®}), sulfasalazine (Azulfidine^{®}), antimalarials such as hydroxychloroquine (Plaquenil^{®}) and chloroquine (Aralen^{®}), methotrexate (Rheumatrex^{®}), gold salts such as gold thioglucose (Solganal^{®}), gold thiomalate (Myochrysine^{®}) and auranofin (Ridaura^{®}), D-penicillamine (Depen^{®} or Cuprimine^{®}), azathioprine (Imuran^{®}), cyclophosphamide (Cytoxan^{®}), chlorambucil (Leukeran^{®}), cyclosporine (Sandimmune^{®}), leflunomide (Arava^{®}) and "anti-TNF" agents such as etanercept (Enbrel^{®}), infliximab (Remicade^{®}), golimumab (Simponi^{®}), certolizumab pegol (Cimzia^{®}) and adalimumab (Humira^{®}), "anti-IL-1" agents such as anakinra (Kineret^{®}) and rilonacept (Arcalyst^{®}), canakinumab (Ilaris^{®}), anti-Jak inhibitors such as tofacitinib, antibodies such as rituximab (Rituxan^{®}), "anti-T-cell" agents such as abatacept (Orencia^{®}), "anti-IL-6" agents such as tocilizumab (Actemra^{®}), diclofenac, cortisone, hyaluronic acid (Synvisc^{®} or Hyalgan^{®}), monoclonal antibodies such as tanezumab, anticoagulants such as heparin (Calcinparine^{®} or Liquaemin^{®}) and warfarin (Coumadin^{®}), antidiarrheals such as diphenoxylate (Lomotil^{®}) and loperamide (Imodium^{®}), bile acid binding agents such as cholestyramine, alosetron (Lotronex^{®}), lubiprostone (Amitiza^{®}), laxatives such as Milk of Magnesia, polyethylene glycol (MiraLax^{®}), Dulcolax^{®}, Correctol^{®} and Senokot^{®}, anticholinergics or antispasmodics such as dicyclomine (Bentyl^{®}), Singulair^{®}, beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), inhaled corticosteroids such as beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), and flunisolide (Aerobid^{®}), Afviar^{®}, Symbicort^{®}, Dulera^{®}, cromolyn sodium (Intal^{®}), methylxanthines such as theophylline (Theo-Dur^{®}, Theolair^{®}, Slo-bid^{®}, Uniphyl^{®}, Theo-24^{®}) and aminophylline, IgE antibodies such as omalizumab (Xolair^{®}), nucleoside reverse transcriptase inhibitors such as zidovudine (Retrovir^{®}), abacavir (Ziagen^{®}), abacavir/lamivudine (Epzicom^{®}), abacavir/lamivudine/zidovudine (Trizivir^{®}), didanosine (Videx^{®}), emtricitabine (Emtriva^{®}), lamivudine (Epivir^{®}), lamivudine/zidovudine (Combivir^{®}), stavudine (Zerit^{®}), and zalcitabine (Hivid^{®}), non-nucleoside reverse transcriptase inhibitors such as delavirdine (Rescriptor^{®}), efavirenz (Sustiva^{®}), nevairapine (Viramune^{®}) and etravirine (Intelence^{®}), nucleotide reverse transcriptase inhibitors such as tenofovir (Viread^{®}), protease inhibitors such as amprenavir (Agenerase^{®}), atazanavir (Reyataz^{®}), darunavir (Prezista^{®}), fosamprenavir (Lexiva^{®}), indinavir (Crixivan^{®}), lopinavir and ritonavir (Kaletra^{®}), nelfinavir (Viracept^{®}), ritonavir (Norvir^{®}), saquinavir (Fortovase^{®} or Invirase^{®}), and tipranavir (Aptivus^{®}), entry inhibitors such as enfuvirtide (Fuzeon^{®}) and maraviroc (Selzentry^{®}), integrase inhibitors such as raltegravir (Isentress^{®}), doxorubicin (Hydrodaunorubicin^{®}), vincristine (Oncovin^{®}), bortezomib (Velcade^{®}), and dexamethasone (Decadron ^{®}) in combination with lenalidomide (Revlimid ^{®}), or any combination(s) thereof.

In another embodiment, the present invention provides a method of treating gout comprising administering to a patient in need thereof a provided compound or a pharmaceutically acceptable salt thereof and one or more additional therapeutic agents selected from non-steroidal anti-inflammatory drugs (NSAIDS) such as aspirin, ibuprofen, naproxen, etodolac (Lodine^{®}) and celecoxib, colchicine (Colcrys^{®}), corticosteroids such as prednisone, prednisolone, methylprednisolone, hydrocortisone, and the like, probenecid, allopurinol and febuxostat (Uloric^{®}).

In another embodiment, the present invention provides a method of treating rheumatoid arthritis comprising administering to a patient in need thereof a provided compound or a pharmaceutically acceptable salt thereof and one or more additional therapeutic agents selected from non-steroidal anti-inflammatory drugs (NSAIDS) such as aspirin, ibuprofen, naproxen, etodolac (Lodine^{®}) and celecoxib, corticosteroids such as prednisone, prednisolone, methylprednisolone, hydrocortisone, and the like, sulfasalazine (Azulfidine^{®}), antimalarials such as hydroxychloroquine (Plaquenil^{®}) and chloroquine (Aralen^{®}), methotrexate (Rheumatrex^{®}), gold salts such as gold thioglucose (Solganal^{®}), gold thiomalate (Myochrysine^{®}) and auranofin (Ridaura^{®}), D-penicillamine (Depen^{®} or Cuprimine^{®}), azathioprine (Imuran^{®}), cyclophosphamide (Cytoxan^{®}), chlorambucil (Leukeran^{®}), cyclosporine (Sandimmune^{®}), leflunomide (Arava^{®}) and "anti-TNF" agents such as etanercept (Enbrel^{®}), infliximab (Remicade^{®}), golimumab (Simponi^{®}), certolizumab pegol (Cimzia^{®}) and adalimumab (Humira^{®}), "anti-IL-1" agents such as anakinra (Kineret^{®}) and rilonacept (Arcalyst^{®}), antibodies such as rituximab (Rituxan^{®}), "anti-T-cell" agents such as abatacept (Orencia^{®}) and "anti-IL-6" agents such as tocilizumab (Actemra^{®}).

In some embodiments, the present invention provides a method of treating osteoarthritis comprising administering to a patient in need thereof a provided compound or a pharmaceutically acceptable salt thereof and one or more additional therapeutic agents selected from acetaminophen, non-steroidal anti-inflammatory drugs (NSAIDS) such as aspirin, ibuprofen, naproxen, etodolac (Lodine^{®}) and celecoxib, diclofenac, cortisone, hyaluronic acid (Synvisc^{®} or Hyalgan^{®}) and monoclonal antibodies such as tanezumab.

In some embodiments, the present invention provides a method of treating lupus comprising administering to a patient in need thereof a provided compound or a pharmaceutically acceptable salt thereof and one or more additional therapeutic agents selected from acetaminophen, non-steroidal anti-inflammatory drugs (NSAIDS) such as aspirin, ibuprofen, naproxen, etodolac (Lodine^{®}) and celecoxib, corticosteroids such as prednisone, prednisolone, methylprednisolone, hydrocortisone, and the like, antimalarials such as hydroxychloroquine (Plaquenil^{®}) and chloroquine (Aralen^{®}), cyclophosphamide (Cytoxan^{®}), methotrexate (Rheumatrex^{®}), azathioprine (Imuran^{®}) and anticoagulants such as heparin (Calcinparine^{®} or Liquaemin^{®}) and warfarin (Coumadin^{®}).

In some embodiments, the present invention provides a method of treating inflammatory bowel disease comprising administering to a patient in need thereof a provided compound or a pharmaceutically acceptable salt thereof and one or more additional therapeutic agents selected from mesalamine (Asacol^{®}) sulfasalazine (Azulfidine^{®}), antidiarrheals such as diphenoxylate (Lomotil^{®}) and loperamide (Imodium^{®}), bile acid binding agents such as cholestyramine, alosetron (Lotronex^{®}), lubiprostone (Amitiza^{®}), laxatives such as Milk of Magnesia, polyethylene glycol (MiraLax^{®}), Dulcolax^{®}, Correctol^{®} and Senokot^{®} and anticholinergics or antispasmodics such as dicyclomine (Bentyl^{®}), anti-TNF therapies, steroids, and antibiotics such as Flagyl or ciprofloxacin.

In some embodiments, the present invention provides a method of treating asthma comprising administering to a patient in need thereof a provided compound or a pharmaceutically acceptable salt thereof and one or more additional therapeutic agents selected from anti-IL-33 antibodies such as REGN3500 (SAR440340) or CNTO 7160, Singulair^{®}, beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), inhaled corticosteroids such as prednisone, prednisolone, beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), flunisolide (Aerobid^{®}), Afviar^{®}, Symbicort^{®}, and Dulera^{®}, cromolyn sodium (Intal^{®}), methylxanthines such as theophylline (Theo-Dur^{®}, Theolair^{®}, Slo-bid^{®}, Uniphyl^{®}, Theo-24^{®}) and aminophylline, and IgE antibodies such as omalizumab (Xolair^{®}).

In some embodiments, the present invention provides a method of treating COPD comprising administering to a patient in need thereof a provided compound or a pharmaceutically acceptable salt thereof and one or more additional therapeutic agents selected from beta-2 agonists such as albuterol (Ventolin^{®} HFA, Proventil^{®} HFA), levalbuterol (Xopenex^{®}), metaproterenol (Alupent^{®}), pirbuterol acetate (Maxair^{®}), terbutaline sulfate (Brethaire^{®}), salmeterol xinafoate (Serevent^{®}) and formoterol (Foradil^{®}), anticholinergic agents such as ipratropium bromide (Atrovent^{®}) and tiotropium (Spiriva^{®}), methylxanthines such as theophylline (Theo-Dur^{®}, Theolair^{®}, Slo-bid^{®}, Uniphyl^{®}, Theo-24^{®}) and aminophylline, inhaled corticosteroids such as prednisone, prednisolone, beclomethasone dipropionate (Beclovent^{®}, Qvar^{®}, and Vanceril^{®}), triamcinolone acetonide (Azmacort^{®}), mometasone (Asthmanex^{®}), budesonide (Pulmocort^{®}), flunisolide (Aerobid^{®}), Afviar^{®}, Symbicort^{®}, and Dulera^{®}, In some embodiments, the present invention provides a method of treating eosinophilic COPD comprising administering to a patient in need thereof a provided compound or a pharmaceutically acceptable salt thereof and one or more additional therapeutic agents selected from an anti-IL-33 antibody such as REGN3500 (SAR440340) or CNTO 7160. In some embodiments, the present invention provides a method of treating eosinophilic asthma comprising administering to a patient in need thereof a provided compound or a pharmaceutically acceptable salt thereof and one or more additional therapeutic agents selected from an anti-IL-33 antibody such as REGN3500 (SAR440340) or CNTO 7160.

In some embodiments, the present invention provides a method of treating HIV comprising administering to a patient in need thereof a provided compound or a pharmaceutically acceptable salt thereof and one or more additional therapeutic agents selected from nucleoside reverse transcriptase inhibitors such as zidovudine (Retrovir^{®}), abacavir (Ziagen^{®}), abacavir/lamivudine (Epzicom^{®}), abacavir/lamivudine/zidovudine (Trizivir^{®}), didanosine (Videx^{®}), emtricitabine (Emtriva^{®}), lamivudine (Epivir^{®}), lamivudine/zidovudine (Combivir^{®}), stavudine (Zerit^{®}), and zalcitabine (Hivid^{®}), non-nucleoside reverse transcriptase inhibitors such as delavirdine (Rescriptor^{®}), efavirenz (Sustiva^{®}), nevairapine (Viramune^{®}) and etravirine (Intelence^{®}), nucleotide reverse transcriptase inhibitors such as tenofovir (Viread^{®}), protease inhibitors such as amprenavir (Agenerase^{®}), atazanavir (Reyataz^{®}), darunavir (Prezista^{®}), fosamprenavir (Lexiva^{®}), indinavir (Crixivan^{®}), lopinavir and ritonavir (Kaletra^{®}), nelfinavir (Viracept^{®}), ritonavir (Norvir^{®}), saquinavir (Fortovase^{®} or Invirase^{®}), and tipranavir (Aptivus^{®}), entry inhibitors such as enfuvirtide (Fuzeon^{®}) and maraviroc (Selzentry^{®}), integrase inhibitors such as raltegravir (Isentress^{®}), and combinations thereof.

In another embodiment, the present invention provides a method of treating a hematological malignancy comprising administering to a patient in need thereof a provided compound or a pharmaceutically acceptable salt thereof and one or more additional therapeutic agents selected from rituximab (Rituxan^{®}), cyclophosphamide (Cytoxan^{®}), doxorubicin (Hydrodaunorubicin^{®}), vincristine (Oncovin^{®}), prednisone, a hedgehog signaling inhibitor, a BTK inhibitor, a JAK/pan-JAK inhibitor, a TYK2 inhibitor, a PI3K inhibitor, a SYK inhibitor, and combinations thereof.

In another embodiment, the present invention provides a method of treating a solid tumor comprising administering to a patient in need thereof a provided compound or a pharmaceutically acceptable salt thereof and one or more additional therapeutic agents selected from rituximab (Rituxan^{®}), cyclophosphamide (Cytoxan^{®}), doxorubicin (Hydrodaunorubicin^{®}), vincristine (Oncovin^{®}), prednisone, a hedgehog signaling inhibitor, a BTK inhibitor, a JAK/pan-JAK inhibitor, a TYK2 inhibitor, a PI3K inhibitor, a SYK inhibitor, and combinations thereof.

In another embodiment, the present invention provides a method of treating a hematological malignancy comprising administering to a patient in need thereof a provided compound or a pharmaceutically acceptable salt thereof and a Hedgehog (Hh) signaling pathway inhibitor. In some embodiments, the hematological malignancy is DLBCL (Ramirez et al "Defining causative factors contributing in the activation of hedgehog signaling in diffuse large B-cell lymphoma" Leuk. Res. (2012), published online July 17, and incorporated herein by reference in its entirety).

In another embodiment, the present invention provides a method of treating diffuse large B-cell lymphoma (DLBCL) comprising administering to a patient in need thereof a provided compound or a pharmaceutically acceptable salt thereof and one or more additional therapeutic agents selected from rituximab (Rituxan^{®}), cyclophosphamide (Cytoxan^{®}), doxorubicin (Hydrodaunorubicin^{®}), vincristine (Oncovin^{®}), prednisone, a hedgehog signaling inhibitor, and combinations thereof.

In some embodiments, the present invention provides a method of treating DLBCL comprising administering to a patient in need thereof a provided compound or a pharmaceutically acceptable salt thereof and a CHOP (cyclophosphamide, Hydrodaunorubicin^{®}, Oncovin^{®}, and prednisone or prednisolone) or R-CHOP (rituximab, cyclophosphamide, Hydrodaunorubicin^{®}, Oncovin^{®}, and prednisone or prednisolone) chemotherapy regimen.

In some embodiments, the present invention provides a method of treating DLBCL comprising administering to a patient in need thereof a provided compound or a pharmaceutically acceptable salt thereof and a rituximab/bendamustine chemotherapy regimen.

In some embodiments, the present invention provides a method of treating DLBCL comprising administering to a patient in need thereof a provided compound or a pharmaceutically acceptable salt thereof and a BTK inhibitor (e.g., ibrutinib).

In some embodiments, the present invention provides a method of treating DLBCL comprising administering to a patient in need thereof a provided compound or a pharmaceutically acceptable salt thereof and an anti-CD20 antibody (e.g., rituximab).

In some embodiments, the present invention provides a method of treating DLBCL comprising administering to a patient in need thereof a provided compound or a pharmaceutically acceptable salt thereof and an anti-CD79B ADC (e.g., polatuzumab).

In some embodiments, the present invention provides a method of treating DLBCL comprising administering to a patient in need thereof a provided compound or a pharmaceutically acceptable salt thereof and a BCL2 inhibitor (e.g., venetoclax).

In some embodiments, the present invention provides a method of treating DLBCL comprising administering to a patient in need thereof a provided compound or a pharmaceutically acceptable salt thereof and lenalidomide or pomalidomide

In some embodiments, the present invention provides a method of treating DLBCL comprising administering to a patient in need thereof a provided compound or a pharmaceutically acceptable salt thereof and a PI3K inhibitor (e.g., umbralisib).

In some embodiments, the present invention provides a method of treating a T-cell disease or deficiency describing herein comprising administering to a patient in need thereof a provided compound or a pharmaceutically acceptable salt thereof and a PI3K inhibitor (e.g., umbralisib).

In some embodiments, the present invention provides a method of treating DLBCL comprising administering to a patient in need thereof a provided compound or a pharmaceutically acceptable salt thereof and a protesome inhibitor (e.g., bortezomib)

In some embodiments, the present invention provides a method of treating a T-cell disease or deficiency describing herein comprising administering to a patient in need thereof a provided compound or a pharmaceutically acceptable salt thereof and a protesome inhibitor (e.g., bortezomib).

In another embodiment, the present invention provides a method of treating multiple myeloma comprising administering to a patient in need thereof a provided compound or a pharmaceutically acceptable salt thereof and one or more additional therapeutic agents selected from bortezomib (Velcade^{®}), and dexamethasone (Decadron^{®}), a hedgehog signaling inhibitor, a BTK inhibitor, a JAK/pan-JAK inhibitor, a TYK2 inhibitor, a PI3K inhibitor, a SYK inhibitor in combination with lenalidomide (Revlimid^{®}).

In another embodiment, the present invention provides a method of treating Waldenström's macroglobulinemia comprising administering to a patient in need thereof a provided compound or a pharmaceutically acceptable salt thereof and one or more additional therapeutic agents selected from chlorambucil (Leukeran^{®}), cyclophosphamide (Cytoxan^{®}, Neosar^{®}), fludarabine (Fludara^{®}), cladribine (Leustatin^{®}), rituximab (Rituxan^{®}), a hedgehog signaling inhibitor, a BTK inhibitor, a JAK/pan-JAK inhibitor, a TYK2 inhibitor, a PI3K inhibitor, and a SYK inhibitor.

In some embodiments, one or more other therapeutic agent is an antagonist of the hedgehog pathway. Approved hedgehog pathway inhibitors which may be used in the present invention include sonidegib (Odomzo^{®}, Sun Pharmaceuticals); and vismodegib (Erivedge^{®}, Genentech), both for treatment of basal cell carcinoma.

In some embodiments, one or more other therapeutic agent is a Poly ADP ribose polymerase (PARP) inhibitor. In some embodiments, a PARP inhibitor is selected from olaparib (Lynparza^{®}, AstraZeneca); rucaparib (Rubraca^{®}, Clovis Oncology); niraparib (Zejula^{®}, Tesaro); talazoparib (MDV3800/BMN 673/LT00673, Medivation/Pfizer/Biomarin); veliparib (ABT-888, AbbVie); and BGB-290 (BeiGene, Inc.).

In some embodiments, one or more other therapeutic agent is a histone deacetylase (HDAC) inhibitor. In some embodiments, an HDAC inhibitor is selected from vorinostat (Zolinza^{®}, Merck); romidepsin (Istodax^{®}, Celgene); panobinostat (Farydak^{®}, Novartis); belinostat (Beleodaq^{®}, Spectrum Pharmaceuticals); entinostat (SNDX-275, Syndax Pharmaceuticals) (NCT00866333); and chidamide (Epidaza^{®}, HBI-8000, Chipscreen Biosciences, China).

In some embodiments, one or more other therapeutic agent is a CDK inhibitor, such as a CDK4/CDK6 inhibitor. In some embodiments, a CDK 4/6 inhibitor is selected from palbociclib (Ibrance^{®}, Pfizer); ribociclib (Kisqali^{®}, Novartis); abemaciclib (Ly2835219, Eli Lilly); and trilaciclib (G1T28, G1 Therapeutics).

In some embodiments, one or more other therapeutic agent is a folic acid inhibitor. Approved folic acid inhibitors useful in the present invention include pemetrexed (Alimta^{®}, Eli Lilly).

In some embodiments, one or more other therapeutic agent is a CC chemokine receptor 4 (CCR4) inhibitor. CCR4 inhibitors being studied that may be useful in the present invention include mogamulizumab (Poteligeo^{®}, Kyowa Hakko Kirin, Japan).

In some embodiments, one or more other therapeutic agent is an isocitrate dehydrogenase (IDH) inhibitor. IDH inhibitors being studied which may be used in the present invention include AG120 (Celgene; NCT02677922); AG221 (Celgene, NCT02677922; NCT02577406); BAY1436032 (Bayer, NCT02746081); IDH305 (Novartis, NCT02987010).

In some embodiments, one or more other therapeutic agent is an arginase inhibitor. Arginase inhibitors being studied which may be used in the present invention include AEB1102 (pegylated recombinant arginase, Aeglea Biotherapeutics), which is being studied in Phase 1 clinical trials for acute myeloid leukemia and myelodysplastic syndrome (NCT02732184) and solid tumors (NCT02561234); and CB-1158 (Calithera Biosciences).

In some embodiments, one or more other therapeutic agent is a glutaminase inhibitor. Glutaminase inhibitors being studied which may be used in the present invention include CB-839 (Calithera Biosciences).

In some embodiments, one or more other therapeutic agent is an antibody that binds to tumor antigens, that is, proteins expressed on the cell surface of tumor cells. Approved antibodies that bind to tumor antigens which may be used in the present invention include rituximab (Rituxan^{®}, Genentech/BiogenIdec); ofatumumab (anti-CD20, Arzerra^{®}, GlaxoSmithKline); obinutuzumab (anti-CD20, Gazyva^{®}, Genentech), ibritumomab (anti-CD20 and Yttrium-90, Zevalin^{®}, Spectrum Pharmaceuticals); daratumumab (anti-CD38, Darzalex^{®}, Janssen Biotech), dinutuximab (anti-glycolipid GD2, Unituxin^{®}, United Therapeutics); trastuzumab (anti-HER2, Herceptin^{®}, Genentech); ado-trastuzumab emtansine (anti-HER2, fused to emtansine, Kadcyla^{®}, Genentech); and pertuzumab (anti-HER2, Perjeta^{®}, Genentech); and brentuximab vedotin (anti-CD30-drug conjugate, Adcetris^{®}, Seattle Genetics).

In some embodiments, one or more other therapeutic agent is a topoisomerase inhibitor. Approved topoisomerase inhibitors useful in the present invention include irinotecan (Onivyde^{®}, Merrimack Pharmaceuticals); topotecan (Hycamtin^{®}, GlaxoSmithKline). Topoisomerase inhibitors being studied which may be used in the present invention include pixantrone (Pixuvri^{®}, CTI Biopharma).

In some embodiments, one or more other therapeutic agent is an inhibitor of anti-apoptotic proteins, such as BCL-2. Approved anti-apoptotics which may be used in the present invention include venetoclax (Venclexta^{®}, AbbVie/Genentech); and blinatumomab (Blincyto^{®}, Amgen). Other therapeutic agents targeting apoptotic proteins which have undergone clinical testing and may be used in the present invention include navitoclax (ABT-263, Abbott), a BCL-2 inhibitor (NCT02079740).

In some embodiments, one or more other therapeutic agent is an androgen receptor inhibitor. Approved androgen receptor inhibitors useful in the present invention include enzalutamide (Xtandi^{®}, Astellas/Medivation); approved inhibitors of androgen synthesis include abiraterone (Zytiga^{®}, Centocor/Ortho); approved antagonist of gonadotropin-releasing hormone (GnRH) receptor (degaralix, Firmagon^{®}, Ferring Pharmaceuticals).

In some embodiments, one or more other therapeutic agent is a selective estrogen receptor modulator (SERM), which interferes with the synthesis or activity of estrogens. Approved SERMs useful in the present invention include raloxifene (Evista^{®}, Eli Lilly).

In some embodiments, one or more other therapeutic agent is an inhibitor of bone resorption. An approved therapeutic which inhibits bone resorption is Denosumab (Xgeva^{®}, Amgen), an antibody that binds to RANKL, prevents binding to its receptor RANK, found on the surface of osteoclasts, their precursors, and osteoclast-like giant cells, which mediates bone pathology in solid tumors with osseous metastases. Other approved therapeutics that inhibit bone resorption include bisphosphonates, such as zoledronic acid (Zometa^{®}, Novartis).

In some embodiments, one or more other therapeutic agent is an inhibitor of interaction between the two primary p53 suppressor proteins, MDMX and MDM2. Inhibitors of p53 suppression proteins being studied which may be used in the present invention include ALRN-6924 (Aileron), a stapled peptide that equipotently binds to and disrupts the interaction of MDMX and MDM2 with p53. ALRN-6924 is currently being evaluated in clinical trials for the treatment of AML, advanced myelodysplastic syndrome (MDS) and peripheral T-cell lymphoma (PTCL) (NCT02909972; NCT02264613).

In some embodiments, one or more other therapeutic agent is an inhibitor of transforming growth factor-beta (TGF-beta or TGFβ). Inhibitors of TGF-beta proteins being studied which may be used in the present invention include NIS793 (Novartis), an anti-TGF-beta antibody being tested in the clinic for treatment of various cancers, including breast, lung, hepatocellular, colorectal, pancreatic, prostate and renal cancer (NCT 02947165). In some embodiments, the inhibitor of TGF-beta proteins is fresolimumab (GC1008; Sanofi-Genzyme), which is being studied for melanoma (NCT00923169); renal cell carcinoma (NCT00356460); and non-small cell lung cancer (NCT02581787). Additionally, in some embodiments, the additional therapeutic agent is a TGF-beta trap, such as described in Connolly et al. (2012) Int'l J. Biological Sciences 8:964-978. One therapeutic compound currently in clinical trials for treatment of solid tumors is M7824 (Merck KgaA - formerly MSB0011459X), which is a bispecific, anti-PD-L1/TGFβ trap compound (NCT02699515); and (NCT02517398). M7824 is comprised of a fully human IgG1 antibody against PD-L1 fused to the extracellular domain of human TGF-beta receptor II, which functions as a TGFβ "trap."

In some embodiments, one or more other therapeutic agent is selected from glembatumumab vedotin-monomethyl auristatin E (MMAE) (Celldex), an anti-glycoprotein NMB (gpNMB) antibody (CR011) linked to the cytotoxic MMAE. gpNMB is a protein overexpressed by multiple tumor types associated with cancer cells' ability to metastasize.

In some embodiments, one or more other therapeutic agent is an antiproliferative compound. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal^{®}); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, AZd₆244 from AstraZeneca, PD181461 from Pfizer and leucovorin.

In some embodiments, the present invention provides a method of treating Alzheimer's disease comprising administering to a patient in need thereof a provided compound or a pharmaceutically acceptable salt thereof and one or more additional therapeutic agents selected from donepezil (Aricept^{®}), rivastigmine (Excelon^{®}), galantamine (Razadyne^{®}), tacrine (Cognex^{®}), and memantine (Namenda^{®}).

In some embodiments, one or more other therapeutic agent is a taxane compound, which causes disruption of microtubules, which are essential for cell division. In some embodiments, a taxane compound is selected from paclitaxel (Taxol^{®}, Bristol-Myers Squibb), docetaxel (Taxotere^{®}, Sanofi-Aventis; Docefrez^{®}, Sun Pharmaceutical), albumin-bound paclitaxel (Abraxane^{®}; Abraxis/Celgene), cabazitaxel (Jevtana^{®}, Sanofi-Aventis), and SID530 (SK Chemicals, Co.) (NCT00931008).

In some embodiments, one or more other therapeutic agent is a nucleoside inhibitor, or a therapeutic agent that interferes with normal DNA synthesis, protein synthesis, cell replication, or will otherwise inhibit rapidly proliferating cells.

In some embodiments, a nucleoside inhibitor is selected from trabectedin (guanidine alkylating agent, Yondelis^{®}, Janssen Oncology), mechlorethamine (alkylating agent, Valchlor^{®}, Aktelion Pharmaceuticals); vincristine (Oncovin^{®}, Eli Lilly; Vincasar^{®}, Teva Pharmaceuticals; Marqibo^{®}, Talon Therapeutics); temozolomide (prodrug to alkylating agent 5-(3-methyltriazen-1-yl)-imidazole-4-carboxamide (MTIC) Temodar^{®}, Merck); cytarabine injection (ara-C, antimetabolic cytidine analog, Pfizer); lomustine (alkylating agent, CeeNU^{®}, Bristol-Myers Squibb; Gleostine^{®}, NextSource Biotechnology); azacitidine (pyrimidine nucleoside analog of cytidine, Vidaza^{®}, Celgene); omacetaxine mepesuccinate (cephalotaxine ester) (protein synthesis inhibitor, Synribo^{®}; Teva Pharmaceuticals); asparaginase *Erwinia chrysanthemi* (enzyme for depletion of asparagine, Elspar^{®}, Lundbeck; Erwinaze^{®}, EUSA Pharma); eribulin mesylate (microtubule inhibitor, tubulin-based antimitotic, Halaven^{®}, Eisai); cabazitaxel (microtubule inhibitor, tubulin-based antimitotic, Jevtana^{®}, Sanofi-Aventis); capacetrine (thymidylate synthase inhibitor, Xeloda^{®}, Genentech); bendamustine (bifunctional mechlorethamine derivative, believed to form interstrand DNA cross-links, Treanda^{®}, Cephalon/Teva); ixabepilone (semisynthetic analog of epothilone B, microtubule inhibitor, tubulin-based antimitotic, Ixempra^{®}, Bristol-Myers Squibb); nelarabine (prodrug of deoxyguanosine analog, nucleoside metabolic inhibitor, Arranon^{®}, Novartis); clorafabine (prodrug of ribonucleotide reductase inhibitor, competitive inhibitor of deoxycytidine, Clolar^{®}, Sanofi-Aventis); and trifluridine and tipiracil (thymidine-based nucleoside analog and thymidine phosphorylase inhibitor, Lonsurf^{®}, Taiho Oncology).

In some embodiments, one or more other therapeutic agent is a kinase inhibitor or VEGF-R antagonist. Approved VEGF inhibitors and kinase inhibitors useful in the present invention include: bevacizumab (Avastin^{®}, Genentech/Roche) an anti-VEGF monoclonal antibody; ramucirumab (Cyramza^{®}, Eli Lilly), an anti-VEGFR-2 antibody and ziv-aflibercept, also known as VEGF Trap (Zaltrap^{®}; Regeneron/Sanofi). VEGFR inhibitors, such as regorafenib (Stivarga^{®}, Bayer); vandetanib (Caprelsa^{®}, AstraZeneca); axitinib (Inlyta^{®}, Pfizer); and lenvatinib (Lenvima^{®}, Eisai); Raf inhibitors, such as sorafenib (Nexavar^{®}, Bayer AG and Onyx); dabrafenib (Tafinlar^{®}, Novartis); and vemurafenib (Zelboraf^{®}, Genentech/Roche); MEK inhibitors, such as cobimetanib (Cotellic^{®}, Exelexis/Genentech/Roche); trametinib (Mekinist^{®}, Novartis); Bcr-Abl tyrosine kinase inhibitors, such as imatinib (Gleevec^{®}, Novartis); nilotinib (Tasigna^{®}, Novartis); dasatinib (Sprycel^{®}, BristolMyersSquibb); bosutinib (Bosulif^{®}, Pfizer); and ponatinib (Inclusig^{®}, Ariad Pharmaceuticals); Her2 and EGFR inhibitors, such as gefitinib (Iressa^{®}, AstraZeneca); erlotinib (Tarceeva^{®}, Genentech/Roche/Astellas); lapatinib (Tykerb^{®}, Novartis); afatinib (Gilotrif^{®}, Boehringer Ingelheim); osimertinib (targeting activated EGFR, Tagrisso^{®}, AstraZeneca); and brigatinib (Alunbrig^{®}, Ariad Pharmaceuticals); c-Met and VEGFR2 inhibitors, such as cabozanitib (Cometriq^{®}, Exelexis); and multikinase inhibitors, such as sunitinib (Sutent^{®}, Pfizer); pazopanib (Votrient^{®}, Novartis); ALK inhibitors, such as crizotinib (Xalkori^{®}, Pfizer); ceritinib (Zykadia^{®}, Novartis); and alectinib (Alecenza^{®}, Genentech/Roche); Bruton's tyrosine kinase inhibitors, such as ibrutinib (Imbruvica^{®}, Pharmacyclics/Janssen); and Flt3 receptor inhibitors, such as midostaurin (Rydapt^{®}, Novartis).

Other kinase inhibitors and VEGF-R antagonists that are in development and may be used in the present invention include tivozanib (Aveo Pharmaecuticals); vatalanib (Bayer/Novartis); lucitanib (Clovis Oncology); dovitinib (TKI258, Novartis); Chiauanib (Chipscreen Biosciences); CEP-11981 (Cephalon); linifanib (Abbott Laboratories); neratinib (HKI-272, Puma Biotechnology); radotinib (Supect^{®}, IY5511, Il-Yang Pharmaceuticals, S. Korea); ruxolitinib (Jakafi^{®}, Incyte Corporation); PTC299 (PTC Therapeutics); CP-547,632 (Pfizer); foretinib (Exelexis, GlaxoSmithKline); quizartinib (Daiichi Sankyo) and motesanib (Amgen/Takeda).

In another embodiment, the present invention provides a method of treating organ transplant rejection or graft vs. host disease comprising administering to a patient in need thereof a provided compound or a pharmaceutically acceptable salt thereof and one or more additional therapeutic agents selected from a steroid, cyclosporin, FK506, rapamycin, a hedgehog signaling inhibitor, a BTK inhibitor, a JAK/pan-JAK inhibitor, a TYK2 inhibitor, a PI3K inhibitor, and a SYK inhibitor.

In another embodiment, the present invention provides a method of treating or lessening the severity of a disease comprising administering to a patient in need thereof a provided compound or a pharmaceutically acceptable salt thereof and a BTK inhibitor, wherein the disease is selected from inflammatory bowel disease, arthritis, systemic lupus erythematosus (SLE), vasculitis, idiopathic thrombocytopenic purpura (ITP), rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, autoimmune thyroiditis, Sjogren's syndrome, multiple sclerosis, systemic sclerosis, Lyme neuroborreliosis, Guillain-Barre syndrome, acute disseminated encephalomyelitis, Addison's disease, opsoclonus-myoclonus syndrome, ankylosing spondylosis, antiphospholipid antibody syndrome, aplastic anemia, autoimmune hepatitis, autoimmune gastritis, pernicious anemia, celiac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, dysautonomia, membranous glomerulonephropathy, endometriosis, interstitial cystitis, pemphigus vulgaris, bullous pemphigoid, neuromyotonia, scleroderma, vulvodynia, a hyperproliferative disease, rejection of transplanted organs or tissues, Acquired Immunodeficiency Syndrome (AIDS, also known as HIV), type 1 diabetes, graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis, asthma, appendicitis, atopic dermatitis, asthma, allergy, blepharitis, bronchiolitis, bronchitis, bursitis, cervicitis, cholangitis, cholecystitis, chronic graft rejection, colitis, conjunctivitis, Crohn's disease, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, or vulvitis, B-cell proliferative disorder, e.g., diffuse large B cell lymphoma, follicular lymphoma, chronic lymphocytic lymphoma, chronic lymphocytic leukemia, acute lymphocytic leukemia, B-cell prolymphocytic leukemia, lymphoplasmacytic lymphoma/Waldenstrom macroglobulinemia, splenic marginal zone lymphoma, multiple myeloma (also known as plasma cell myeloma), non-Hodgkin's lymphoma, Hodgkin's lymphoma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, Burkitt lymphoma/leukemia, or lymphomatoid granulomatosis, breast cancer, prostate cancer, or cancer of the mast cells (e.g., mastocytoma, mast cell leukemia, mast cell sarcoma, systemic mastocytosis), bone cancer, colorectal cancer, pancreatic cancer, diseases of the bone and joints including, without limitation, rheumatoid arthritis, seronegative spondyloarthropathies (including ankylosing spondylitis, psoriatic arthritis and Reiter's disease), Behcet's disease, Sjogren's syndrome, systemic sclerosis, osteoporosis, bone cancer, bone metastasis, a thromboembolic disorder, (e.g., myocardial infarct, angina pectoris, reocclusion after angioplasty, restenosis after angioplasty, reocclusion after aortocoronary bypass, restenosis after aortocoronary bypass, stroke, transitory ischemia, a peripheral arterial occlusive disorder, pulmonary embolism, deep venous thrombosis), inflammatory pelvic disease, urethritis, skin sunburn, sinusitis, pneumonitis, encephalitis, meningitis, myocarditis, nephritis, osteomyelitis, myositis, hepatitis, gastritis, enteritis, dermatitis, gingivitis, appendicitis, pancreatitis, cholocystitus, agammaglobulinemia, psoriasis, allergy, Crohn's disease, irritable bowel syndrome, ulcerative colitis, Sjogren's disease, tissue graft rejection, hyperacute rejection of transplanted organs, asthma, allergic rhinitis, chronic obstructive pulmonary disease (COPD), autoimmune polyglandular disease (also known as autoimmune polyglandular syndrome), autoimmune alopecia, pernicious anemia, glomerulonephritis, dermatomyositis, multiple sclerosis, scleroderma, vasculitis, autoimmune hemolytic and thrombocytopenic states, Goodpasture's syndrome, atherosclerosis, Addison's disease, Parkinson's disease, Alzheimer's disease, diabetes, septic shock, systemic lupus erythematosus (SLE), rheumatoid arthritis, psoriatic arthritis, juvenile arthritis, osteoarthritis, chronic idiopathic thrombocytopenic purpura, Waldenstrom macroglobulinemia, myasthenia gravis, Hashimoto's thyroiditis, atopic dermatitis, degenerative joint disease, vitiligo, autoimmune hypopituitarism, Guillain-Barre syndrome, Behcet's disease, scleraderma, mycosis fungoides, acute inflammatory responses (such as acute respiratory distress syndrome and ischemia/reperfusion injury), and Graves' disease.

In another embodiment, the present invention provides a method of treating or lessening the severity of a disease comprising administering to a patient in need thereof a provided compound or a pharmaceutically acceptable salt thereof and a PI3K inhibitor, wherein the disease is selected from a cancer, a neurodegenative disorder, an angiogenic disorder, a viral disease, an autoimmune disease, an inflammatory disorder, a hormone-related disease, conditions associated with organ transplantation, immunodeficiency disorders, a destructive bone disorder, a proliferative disorder, an infectious disease, a condition associated with cell death, thrombin-induced platelet aggregation, chronic myelogenous leukemia (CML), chronic lymphocytic leukemia (CLL), liver disease, pathologic immune conditions involving T cell activation, a cardiovascular disorder, and a CNS disorder.

In another embodiment, the present invention provides a method of treating or lessening the severity of a disease comprising administering to a patient in need thereof a provided compound or a pharmaceutically acceptable salt thereof and a PI3K inhibitor, wherein the disease is selected from benign or malignant tumor, carcinoma or solid tumor of the brain, kidney (e.g., renal cell carcinoma (RCC)), liver, adrenal gland, bladder, breast, stomach, gastric tumors, ovaries, colon, rectum, prostate, pancreas, lung, vagina, endometrium, cervix, testis, genitourinary tract, esophagus, larynx, skin, bone or thyroid, sarcoma, glioblastomas, neuroblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, adenoma, adenocarcinoma, keratoacanthoma, epidermoid carcinoma, large cell carcinoma, non-small-cell lung carcinoma, lymphomas, (including, for example, non-Hodgkin's Lymphoma (NHL) and Hodgkin's lymphoma (also termed Hodgkin's or Hodgkin's disease)), a mammary carcinoma, follicular carcinoma, undifferentiated carcinoma, papillary carcinoma, seminoma, melanoma, or a leukemia, diseases include Cowden syndrome, Lhermitte-Dudos disease and Bannayan-Zonana syndrome, or diseases in which the PI3K/PKB pathway is aberrantly activated, asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection, acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy, bronchitis of whatever type or genesis including, but not limited to, acute, arachidic, catarrhal, croupus, chronic or phthinoid bronchitis, pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis, Loffler's syndrome, eosinophilic, pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction, psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, conjunctivitis, keratoconjunctivitis sicca, and vernal conjunctivitis, diseases affecting the nose including allergic rhinitis, and inflammatory disease in which autoimmune reactions are implicated or having an autoimmune component or etiology, including autoimmune hematological disorders (e.g. hemolytic anemia, aplastic anemia, pure red cell anemia and idiopathic thrombocytopenia), systemic lupus erythematosus, rheumatoid arthritis, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary biliary cirrhosis, uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minal change nephropathy, restenosis, cardiomegaly, atherosclerosis, myocardial infarction, ischemic stroke and congestive heart failure, Alzheimer's disease, Parkinson's disease, amyotrophic lateral sclerosis, Huntington's disease, and cerebral ischemia, and neurodegenerative disease caused by traumatic injury, glutamate neurotoxicity and hypoxia.

In some embodiments, one or more other therapeutic agent is a phosphatidylinositol 3 kinase (PI3K) inhibitor. In some embodiments, a PI3K inhibitor is selected from idelalisib (Zydelig^{®}, Gilead), alpelisib (BYL719, Novartis), taselisib (GDC-0032, Genentech/Roche); pictilisib (GDC-0941, Genentech/Roche); copanlisib (BAY806946, Bayer); duvelisib (formerly IPI-145, Infinity Pharmaceuticals); PQR309 (Piqur Therapeutics, Switzerland); and TGR1202 (formerly RP5230, TG Therapeutics).

In some embodiments, the present invention provides a method of treating AML comprising administering to a patient in need thereof a provided compound or a pharmaceutically acceptable salt thereof and one or more additional therapeutic agents selected from: FLT3 inhibitors; targeted agents such as IDH inhibitors, anti-CD33 ADCs (e.g. Mylotarg), BCL2 inhibitors, and Hedgehog inhibitors; and chemotherapy such as AraC, daunarubicin, etoposide, methotrexate, fludarabine, mitozantrone, azacytidine, and corticosteroids.

In some embodiments, the present invention provides a method of treating MDS comprising administering to a patient in need thereof a provided compound or a pharmaceutically acceptable salt thereof and one or more additional therapeutic agents selected from azacytidine, decitabine and revlimid.

In some embodiments, the present invention provides a method of treating inflammatory skin conditions such as hidradenitis suppurativa, comprising administering to a patient in need thereof a provided compound or a pharmaceutically acceptable salt thereof and one or more additional therapeutic agents selected from anti-TNF drugs.

In some embodiments, the present invention provides a method of treating inflammatory skin conditions such as atopic dermatitis, comprising administering to a patient in need thereof a provided compound or a pharmaceutically acceptable salt thereof and one or more additional therapeutic agents selected from IL-4/IL-13-targeted agents such as dupilumab. In some embodiments, the present invention provides a method of treating inflammatory skin conditions such as psoriasis, comprising administering to a patient in need thereof a provided compound or a pharmaceutically acceptable salt thereof and one or more additional therapeutic agents selected from anti-IL-17 and anti-IL-23 antibodies.

The compounds and compositions, according to the method of the present invention, may be administered using any amount and any route of administration effective for treating or lessening the severity of a cancer, an autoimmune disorder, a proliferative disorder, an inflammatory disorder, a neurodegenerative or neurological disorder, schizophrenia, a bone-related disorder, liver disease, or a cardiac disorder. The exact amount required will vary from subject to subject, depending on the species, age, and general condition of the subject, the severity of the infection, the particular agent, its mode of administration, and the like. Compounds of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient", as used herein, means an animal, preferably a mammal, and most preferably a human.

Pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to one embodiment, the invention relates to a method of inhibiting protein kinase activity or degading a protein kinase in a biological sample comprising the step of contacting said biological sample with a compound of this invention, or a composition comprising said compound.

According to another embodiment, the invention relates to a method of inhibiting or degrading IRAK-1, IRAK-2, and/or IRAK-4, or a mutant thereof, activity in a biological sample comprising the step of contacting said biological sample with a compound of this invention, or a composition comprising said compound.

The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition and/or degradation of a protein kinase, or a protein kinase selected from IRAK-1, IRAK-2, and/or IRAK-4, or a mutant thereof, activity in a biological sample is useful for a variety of purposes that are known to one of skill in the art. Examples of such purposes include, but are not limited to, blood transfusion, organ-transplantation, biological specimen storage, and biological assays.

Another embodiment of the present invention relates to a method of degrading a protein kinase and/or inhibiting protein kinase activity in a patient comprising the step of administering to said patient a compound of the present invention, or a composition comprising said compound.

According to another embodiment, the invention relates to a method of degrading and/or inhibiting one or more of IRAK-1, IRAK-2, and/or IRAK-4, or a mutant thereof, activity in a patient comprising the step of administering to said patient a compound of the present invention, or a composition comprising said compound. In other embodiments, the present invention provides a method for treating a disorder mediated by one or more of IRAK-1, IRAK-2, and/or IRAK-4, or a mutant thereof, in a patient in need thereof, comprising the step of administering to said patient a compound according to the present invention or pharmaceutically acceptable composition thereof. Such disorders are described in detail herein.

Depending upon the particular condition, or disease, to be treated, additional therapeutic agents that are normally administered to treat that condition, may also be present in the compositions of this invention. As used herein, additional therapeutic agents that are normally administered to treat a particular disease, or condition, are known as "appropriate for the disease, or condition, being treated."

A compound of the current invention may also be used to advantage in combination with other antiproliferative compounds. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal^{®}); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, AZD6244 from AstraZeneca, PD181461 from Pfizer and leucovorin.

The term "aromatase inhibitor" as used herein relates to a compound which inhibits estrogen production, for instance, the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane is marketed under the trade name Aromasin^{™}. Formestane is marketed under the trade name Lentaron^{™}. Fadrozole is marketed under the trade name Afema^{™}. Anastrozole is marketed under the trade name Arimidex^{™}. Letrozole is marketed under the trade names Femara^{™} or Femar^{™}. Aminoglutethimide is marketed under the trade name Orimeten^{™}. A combination of the invention comprising a chemotherapeutic agent which is an aromatase inhibitor is particularly useful for the treatment of hormone receptor positive tumors, such as breast tumors.

In some embodiments, one or more other therapeutic agent is an mTOR inhibitor, which inhibits cell proliferation, angiogenesis and glucose uptake. In some embodiments, an mTOR inhibitor is everolimus (Afinitor^{®}, Novartis); temsirolimus (Torisel^{®}, Pfizer); and sirolimus (Rapamune^{®}, Pfizer).

In some embodiments, one or more other therapeutic agent is an aromatase inhibitor. In some embodiments, an aromatase inhibitor is selected from exemestane (Aromasin^{®}, Pfizer); anastazole (Arimidex^{®}, AstraZeneca) and letrozole (Femara^{®}, Novartis).

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen is marketed under the trade name Nolvadex^{™}. Raloxifene hydrochloride is marketed under the trade name Evista^{™}. Fulvestrant can be administered under the trade name Faslodex^{™}. A combination of the invention comprising a chemotherapeutic agent which is an antiestrogen is particularly useful for the treatment of estrogen receptor positive tumors, such as breast tumors.

The term "anti-androgen" as used herein relates to any substance which is capable of inhibiting the biological effects of androgenic hormones and includes, but is not limited to, bicalutamide (Casodex^{™}). The term "gonadorelin agonist" as used herein includes, but is not limited to abarelix, goserelin and goserelin acetate. Goserelin can be administered under the trade name Zoladex^{™}.

The term "topoisomerase I inhibitor" as used herein includes, but is not limited to topotecan, gimatecan, irinotecan, camptothecian and its analogues, 9-nitrocamptothecin and the macromolecular camptothecin conjugate PNU-166148. Irinotecan can be administered, e.g. in the form as it is marketed, e.g. under the trademark Camptosar^{™}. Topotecan is marketed under the trade name Hycamptin^{™}.

The term "topoisomerase II inhibitor" as used herein includes, but is not limited to the anthracyclines such as doxorubicin (including liposomal formulation, such as Caelyx^{™}), daunorubicin, epirubicin, idarubicin and nemorubicin, the anthraquinones mitoxantrone and losoxantrone, and the podophillotoxines etoposide and teniposide. Etoposide is marketed under the trade name Etopophos^{™}. Teniposide is marketed under the trade name VM 26-Bristol Doxorubicin is marketed under the trade name Acriblastin ^{™} or Adriamycin^{™}. Epirubicin is marketed under the trade name Farmorubicin^{™}. Idarubicin is marketed. under the trade name Zavedos^{™}. Mitoxantrone is marketed under the trade name Novantron.

The term "microtubule active agent" relates to microtubule stabilizing, microtubule destabilizing compounds and microtublin polymerization inhibitors including, but not limited to taxanes, such as paclitaxel and docetaxel; vinca alkaloids, such as vinblastine or vinblastine sulfate, vincristine or vincristine sulfate, and vinorelbine; discodermolides; cochicine and epothilones and derivatives thereof. Paclitaxel is marketed under the trade name Taxol^{™}. Docetaxel is marketed under the trade name Taxotere^{™}. Vinblastine sulfate is marketed under the trade name Vinblastin R.P^{™}. Vincristine sulfate is marketed under the trade name Farmistin^{™}.

The term "alkylating agent" as used herein includes, but is not limited to, cyclophosphamide, ifosfamide, melphalan or nitrosourea (BCNU or Gliadel). Cyclophosphamide is marketed under the trade name Cyclostin^{™}. Ifosfamide is marketed under the trade name Holoxan^{™}.

The term "histone deacetylase inhibitors" or "HDAC inhibitors" relates to compounds which inhibit the histone deacetylase and which possess antiproliferative activity. This includes, but is not limited to, suberoylanilide hydroxamic acid (SAHA).

The term "antineoplastic antimetabolite" includes, but is not limited to, 5-fluorouracil or 5-FU, capecitabine, gemcitabine, DNA demethylating compounds, such as 5-azacytidine and decitabine, methotrexate and edatrexate, and folic acid antagonists such as pemetrexed. Capecitabine is marketed under the trade name Xeloda^{™}. Gemcitabine is marketed under the trade name Gemzar^{™}.

The term "platin compound" as used herein includes, but is not limited to, carboplatin, cis-platin, cisplatinum and oxaliplatin. Carboplatin can be administered, e.g., in the form as it is marketed, e.g. under the trademark Carboplat^{™}. Oxaliplatin can be administered, e.g., in the form as it is marketed, e.g. under the trademark Eloxatin^{™}.

The term "Bcl-2 inhibitor" as used herein includes, but is not limited to compounds having inhibitory activity against B-cell lymphoma 2 protein (Bcl-2), including but not limited to ABT-199, ABT-731, ABT-737, apogossypol, Ascenta's pan-Bcl-2 inhibitors, curcumin (and analogs thereof), dual Bcl-2/Bcl-xL inhibitors (Infinity Pharmaceuticals/Novartis Pharmaceuticals), Genasense (G3139), HA14-1 (and analogs thereof; see WO 2008/118802, US 2010/0197686), navitoclax (and analogs thereof, see US 7,390,799), NH-1 (Shenayng Pharmaceutical University), obatoclax (and analogs thereof, see WO 2004/106328, US 2005/0014802), S-001 (Gloria Pharmaceuticals), TW series compounds (Univ. of Michigan), and venetoclax. In some embodiments the Bcl-2 inhibitor is a small molecule therapeutic. In some embodiments the Bcl-2 inhibitor is a peptidomimetic.

The term "compounds targeting/decreasing a protein or lipid kinase activity; or a protein or lipid phosphatase activity; or further anti-angiogenic compounds" as used herein includes, but is not limited to, protein tyrosine kinase and/or serine and/or threonine kinase inhibitors or lipid kinase inhibitors, such as a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, such as an N-phenyl-2-pyrimidine-amine derivative, such as imatinib, SU101, SU6668 and GFB-111; b) compounds targeting, decreasing or inhibiting the activity of the fibroblast growth factor-receptors (FGFR); c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, or antibodies that target the extracellular domain of IGF-I receptor or its growth factors; d) compounds targeting, decreasing or inhibiting the activity of the Trk receptor tyrosine kinase family, or ephrin B4 inhibitors; e) compounds targeting, decreasing or inhibiting the activity of the AxI receptor tyrosine kinase family; f) compounds targeting, decreasing or inhibiting the activity of the Ret receptor tyrosine kinase; g) compounds targeting, decreasing or inhibiting the activity of the Kit/SCFR receptor tyrosine kinase, such as imatinib; h) compounds targeting, decreasing or inhibiting the activity of the C-kit receptor tyrosine kinases, which are part of the PDGFR family, such as compounds which target, decrease or inhibit the activity of the c-Kit receptor tyrosine kinase family, especially compounds which inhibit the c-Kit receptor, such as imatinib; i) compounds targeting, decreasing or inhibiting the activity of members of the c-Abl family, their gene-fusion products (e.g. BCR-Abl kinase) and mutants, such as compounds which target decrease or inhibit the activity of c-Abl family members and their gene fusion products, such as an N-phenyl-2-pyrimidine-amine derivative, such as imatinib or nilotinib (AMN107); PD180970; AG957; NSC 680410; PD173955 from ParkeDavis; or dasatinib (BMS-354825); j) compounds targeting, decreasing or inhibiting the activity of members of the protein kinase C (PKC) and Raf family of serine/threonine kinases, members of the MEK, SRC, JAK/pan-JAK, FAK, PDK1, PKB/Akt, Ras/MAPK, PI3K, SYK, TYK2, BTK and TEC family, and/or members of the cyclin-dependent kinase family (CDK) including staurosporine derivatives, such as midostaurin; examples of further compounds include UCN-01, safingol, BAY 43-9006, Bryostatin 1, Perifosine; llmofosine; RO 318220 and RO 320432; GO 6976; lsis 3521; LY333531/LY379196; isochinoline compounds; FTIs; PD184352 or QAN697 (a P13K inhibitor) or AT7519 (CDK inhibitor); k) compounds targeting, decreasing or inhibiting the activity of protein-tyrosine kinase inhibitors, such as compounds which target, decrease or inhibit the activity of protein-tyrosine kinase inhibitors include imatinib mesylate (Gleevec^{™}) or tyrphostin such as Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5- dihydroxyphenyl)methyl]amino}-benzoic acid adamantyl ester; NSC 680410, adaphostin); l) compounds targeting, decreasing or inhibiting the activity of the epidermal growth factor family of receptor tyrosine kinases (EGFR₁ ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, such as EGF receptor, ErbB2, ErbB3 and ErbB4 or bind to EGF or EGF related ligands, CP 358774, ZD 1839, ZM 105180; trastuzumab (Herceptin^{™}), cetuximab (Erbitux^{™}), Iressa, Tarceva, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives; m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF, n) compounds targeting, decreasing or inhibiting the kinase activity of one or more JAK family members (JAK1/JAK2/JAK3/TYK2 and/or pan-JAK), including but not limited to PRT-062070, SB-1578, baricitinib, pacritinib, momelotinib, VX-509, AZD-1480, TG-101348, tofacitinib, and ruxolitinib; o) compounds targeting, decreasing or inhibiting the kinase activity of PI3 kinase (PI3K) including but not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib; and; and q) compounds targeting, decreasing or inhibiting the signaling effects of hedgehog protein (Hh) or smoothened receptor (SMO) pathways, including but not limited to cyclopamine, vismodegib, itraconazole, erismodegib, and IPI-926 (saridegib).

Compounds which target, decrease or inhibit the activity of a protein or lipid phosphatase are e.g. inhibitors of phosphatase 1, phosphatase 2A, or CDC25, such as okadaic acid or a derivative thereof.

In some embodiments, one or more other therapeutic agent is a growth factor antagonist, such as an antagonist of platelet-derived growth factor (PDGF), or epidermal growth factor (EGF) or its receptor (EGFR). Approved PDGF antagonists which may be used in the present invention include olaratumab (Lartruvo^{®}; Eli Lilly). Approved EGFR antagonists which may be used in the present invention include cetuximab (Erbitux^{®}, Eli Lilly); necitumumab (Portrazza^{®}, Eli Lilly), panitumumab (Vectibix^{®}, Amgen); and osimertinib (targeting activated EGFR, Tagrisso^{®}, AstraZeneca).

The term "PI3K inhibitor" as used herein includes, but is not limited to compounds having inhibitory activity against one or more enzymes in the phosphatidylinositol-3-kinase family, including, but not limited to PI3Kα, PI3Kγ, PI3Kδ, PI3Kβ, PI3K-C2α, PI3K-C2β, PI3K-C2γ, Vps34, p110-α, p110-β, p110-γ, p110-δ, p85-α, p85-β, p55-γ, p150, p101, and p87. Examples of PI3K inhibitors useful in this invention include but are not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib.

The term "BTK inhibitor" as used herein includes, but is not limited to compounds having inhibitory activity against Bruton's Tyrosine Kinase (BTK), including, but not limited to AVL-292 and ibrutinib.

The term "SYK inhibitor" as used herein includes, but is not limited to compounds having inhibitory activity against spleen tyrosine kinase (SYK), including but not limited to PRT-062070, R-343, R-333, Excellair, PRT-062607, and fostamatinib

Further examples of BTK inhibitory compounds, and conditions treatable by such compounds in combination with compounds of this invention can be found in WO 2008/039218, US 2008/0108636 and WO 2011/090760, US 2010/0249092, the entirety of each of which is herein incorporated by reference.

Further examples of SYK inhibitory compounds, and conditions treatable by such compounds in combination with compounds of this invention can be found in WO 2003/063794, US 2004/0029902, WO 2005/007623, US 2005/0075306, and WO 2006/078846, US 2006/0211657, the entirety of each of which is herein incorporated by reference.

Further examples of PI3K inhibitory compounds, and conditions treatable by such compounds in combination with compounds of this invention can be found in WO 2004/019973, US 2004/0106569, WO 2004/089925, US 2004/0242631, US 8,138,347, WO 2002/088112, US 2004/0116421, WO 2007/084786, US 2010/0249126, WO 2007/129161, US 2008/0076768, WO 2006/122806, US 2008/0194579, WO 2005/113554, US 2008/0275067, and WO 2007/044729, US 2010/0087440, the entirety of each of which is herein incorporated by reference.

Further examples of JAK inhibitory compounds, and conditions treatable by such compounds in combination with compounds of this invention can be found in WO 2009/114512, US 2009/0233903, WO 2008/109943, US 2010/0197671, WO 2007/053452, US 2007/0191405, WO 2001/0142246, US 2001/0053782, and WO 2007/070514, US 2007/0135461, the entirety of each of which is herein incorporated by reference.

Further anti-angiogenic compounds include compounds having another mechanism for their activity, e.g. unrelated to protein or lipid kinase inhibition e.g. thalidomide (Thalomid^{™}) and TNP-470.

Examples of proteasome inhibitors useful for use in combination with compounds of the invention include, but are not limited to bortezomib, disulfiram, epigallocatechin-3-gallate (EGCG), salinosporamide A, carfilzomib, ONX-0912, CEP-18770, and MLN9708.

Compounds which target, decrease or inhibit the activity of a protein or lipid phosphatase are e.g. inhibitors of phosphatase 1, phosphatase 2A, or CDC25, such as okadaic acid or a derivative thereof.

Compounds which induce cell differentiation processes include, but are not limited to, retinoic acid, α- γ- or δ- tocopherol or α- γ- or δ-tocotrienol.

The term cyclooxygenase inhibitor as used herein includes, but is not limited to, Cox-2 inhibitors, 5-alkyl substituted 2-arylaminophenylacetic acid and derivatives, such as celecoxib (Celebrex^{™}), rofecoxib (Vioxx^{™}), etoricoxib, valdecoxib or a 5-alkyl-2- arylaminophenylacetic acid, such as 5-methyl-2-(2'-chloro-6'-fluoroanilino)phenyl acetic acid, lumiracoxib.

The term "bisphosphonates" as used herein includes, but is not limited to, etridonic, clodronic, tiludronic, pamidronic, alendronic, ibandronic, risedronic and zoledronic acid. Etridonic acid is marketed under the trade name Didronel^{™}. Clodronic acid is marketed under the trade name Bonefos^{™}. Tiludronic acid is marketed under the trade name Skelid^{™}. Pamidronic acid is marketed under the trade name Aredia^{™}. Alendronic acid is marketed under the trade name Fosamax^{™}. Ibandronic acid is marketed under the trade name Bondranat^{™}. Risedronic acid is marketed under the trade name Actonel^{™}. Zoledronic acid is marketed under the trade name Zometa^{™}. The term "mTOR inhibitors" relates to compounds which inhibit the mammalian target of rapamycin (mTOR) and which possess antiproliferative activity such as sirolimus (Rapamune^{®}), everolimus (Certican^{™}), CCI-779 and ABT578.

The term "heparanase inhibitor" as used herein refers to compounds which target, decrease or inhibit heparin sulfate degradation. The term includes, but is not limited to, PI-88. The term "biological response modifier" as used herein refers to a lymphokine or interferons.

The term "inhibitor of Ras oncogenic isoforms", such as H-Ras, K-Ras, or N-Ras, as used herein refers to compounds which target, decrease or inhibit the oncogenic activity of Ras; for example, a "farnesyl transferase inhibitor" such as L-744832, DK8G557 or R115777 (Zarnestra^{™}). The term "telomerase inhibitor" as used herein refers to compounds which target, decrease or inhibit the activity of telomerase. Compounds which target, decrease or inhibit the activity of telomerase are especially compounds which inhibit the telomerase receptor, such as telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase include, but are not limited to, bengamide or a derivative thereof.

The term "proteasome inhibitor" as used herein refers to compounds which target, decrease or inhibit the activity of the proteasome. Compounds which target, decrease or inhibit the activity of the proteasome include, but are not limited to, Bortezomib (Velcade^{™}), ); carfilzomib (Kyprolis^{®}, Amgen); and ixazomib (Ninlaro^{®}, Takeda), and MLN 341.

The term "matrix metalloproteinase inhibitor" or ("MMP" inhibitor) as used herein includes, but is not limited to, collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS-279251 , BAY 12-9566, TAA211 , MMI270B or AAJ996.

The term "compounds used in the treatment of hematologic malignancies" as used herein includes, but is not limited to, FMS-like tyrosine kinase inhibitors, which are compounds targeting, decreasing or inhibiting the activity of FMS-like tyrosine kinase receptors (Flt-3R); interferon, 1-β-D-arabinofuransylcytosine (ara-c) and bisulfan; and ALK inhibitors, which are compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, such as PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, such as 17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to, trastuzumab (Herceptin^{™}), Trastuzumab-DM1, erbitux, bevacizumab (Avastin^{™}), rituximab (Rituxan^{®}), PRO64553 (anti-CD40) and 2C4 Antibody. By antibodies is meant intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), compounds of the current invention can be used in combination with standard leukemia therapies, especially in combination with therapies used for the treatment of AML. In particular, compounds of the current invention can be administered in combination with, for example, farnesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

Other anti-leukemic compounds include, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065 including, but not limited to, N-hydroxy-3-[4-[[[2-(2-methyl-1H-indol-3-yl)-ethyl]- amino]methyl]phenyl]-2E-2-propenamide, or a pharmaceutically acceptable salt thereof and N-hydroxy-3-[4-[(2-hydroxyethyl){2-(1H-indol-3-yl)ethyl]-amino]methyl]phenyl]-2E-2- propenamide, or a pharmaceutically acceptable salt thereof, especially the lactate salt. Somatostatin receptor antagonists as used herein refer to compounds which target, treat or inhibit the somatostatin receptor such as octreotide, and SOM230. Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1 , pp. 248-275 (1993).

Also included are EDG binders and ribonucleotide reductase inhibitors. The term "EDG binders" as used herein refers to a class of immunosuppressants that modulates lymphocyte recirculation, such as FTY720. The term "ribonucleotide reductase inhibitors" refers to pyrimidine or purine nucleoside analogs including, but not limited to, fludarabine and/or cytosine arabinoside (ara-C), 6-thioguanine, 5- fluorouracil, cladribine, 6-mercaptopurine (especially in combination with ara-C against ALL) and/or pentostatin. Ribonucleotide reductase inhibitors are especially hydroxyurea or 2-hydroxy-1H-isoindole-1 ,3-dione derivatives.

Also included are in particular those compounds, proteins or monoclonal antibodies of VEGF such as 1-(4-chloroanilino)-4-(4-pyridylmethyl)phthalazine or a pharmaceutically acceptable salt thereof, 1-(4-chloroanilino)-4-(4-pyridylmethyl)phthalazine succinate; Angiostatin^{™}; Endostatin^{™}; anthranilic acid amides; ZD4190; ZD6474; SU5416; SU6668; bevacizumab; or anti-VEGF antibodies or anti-VEGF receptor antibodies, such as rhuMAb and RHUFab, VEGF aptamer such as Macugon; FLT-4 inhibitors, FLT-3 inhibitors, VEGFR-2 IgGI antibody, Angiozyme (RPI 4610) and Bevacizumab (Avastin^{™}).

Photodynamic therapy as used herein refers to therapy which uses certain chemicals known as photosensitizing compounds to treat or prevent cancers. Examples of photodynamic therapy include treatment with compounds, such as Visudyne^{™} and porfimer sodium.

Angiostatic steroids as used herein refers to compounds which block or inhibit angiogenesis, such as, e.g., anecortave, triamcinolone, hydrocortisone, 11-α-epihydrocotisol, cortexolone, 17α-hydroxyprogesterone, corticosterone, desoxycorticosterone, testosterone, estrone and dexamethasone.

Implants containing corticosteroids refers to compounds, such as fluocinolone and dexamethasone.

Other chemotherapeutic compounds include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The compounds of the invention are also useful as co-therapeutic compounds for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate; non-steroidal glucocorticoid receptor agonists; LTB4 antagonists such LY293111, CGS025019C, CP-195543, SC-53228, BIIL 284, ONO 4057, SB 209247; LTD4 antagonists such as montelukast and zafirlukast; PDE4 inhibitors such cilomilast (Ariflo^{®} GlaxoSmithKline), Roflumilast (Byk Gulden),V-11294A (Napp), BAY19-8004 (Bayer), SCH-351591 (Schering- Plough), Arofylline (Almirall Prodesfarma), PD189659 / PD168787 (ParkeDavis), AWD-12- 281 (Asta Medica), CDC-801 (Celgene), SeICID(TM) CC-10004 (Celgene), VM554/UM565 (Vernalis), T-440 (Tanabe), KW-4490 (Kyowa Hakko Kogyo); A2a agonists; A2b antagonists; and beta-2 adrenoceptor agonists such as albuterol (salbutamol), metaproterenol, terbutaline, salmeterol fenoterol, procaterol, and especially, formoterol and pharmaceutically acceptable salts thereof. Suitable bronchodilatory drugs include anticholinergic or antimuscarinic compounds, in particular ipratropium bromide, oxitropium bromide, tiotropium salts and CHF 4226 (Chiesi), and glycopyrrolate.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine.

Other useful combinations of compounds of the invention with anti-inflammatory drugs are those with antagonists of chemokine receptors, e.g. CCR-1 , CCR-2, CCR-3, CCR-4, CCR-5, CCR-6, CCR-7, CCR-8, CCR-9 and CCR10, CXCR1 , CXCR2, CXCR3, CXCR4, CXCR5, particularly CCR-5 antagonists such as Schering-Plough antagonists SC-351125, SCH-55700 and SCH-D, and Takeda antagonists such as N-[[4-[[[6,7-dihydro-2-(4-methylphenyl)-5H-benzo-cyclohepten-8-yl]carbonyl]amino]phenyl]-methyl]tetrahydro-N,N-dimethyl-2H-pyran-4-aminium chloride (TAK-770).

The structure of the active compounds identified by code numbers, generic or trade names may be taken from the actual edition of the standard compendium "The Merck Index" or from databases, e.g. Patents International (e.g. IMS World Publications).

A compound of the current invention may also be used in combination with known therapeutic processes, for example, the administration of hormones or radiation. In certain embodiments, a provided compound is used as a radiosensitizer, especially for the treatment of tumors which exhibit poor sensitivity to radiotherapy.

A compound of the current invention can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds. A compound of the current invention can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Those additional agents may be administered separately from an inventive compound-containing composition, as part of a multiple dosage regimen. Alternatively, those agents may be part of a single dosage form, mixed together with a compound of this invention in a single composition. If administered as part of a multiple dosage regime, the two active agents may be submitted simultaneously, sequentially or within a period of time from one another normally within five hours from one another.

As used herein, the term "combination," "combined," and related terms refers to the simultaneous or sequential administration of therapeutic agents in accordance with this invention. For example, a compound of the present invention may be administered with another therapeutic agent simultaneously or sequentially in separate unit dosage forms or together in a single unit dosage form. Accordingly, the present invention provides a single unit dosage form comprising a compound of the current invention, an additional therapeutic agent, and a pharmaceutically acceptable carrier, adjuvant, or vehicle.

The amount of both an inventive compound and additional therapeutic agent (in those compositions which comprise an additional therapeutic agent as described above) that may be combined with the carrier materials to produce a single dosage form will vary depending upon the host treated and the particular mode of administration. Preferably, compositions of this invention should be formulated so that a dosage of between 0.01 - 100 mg/kg body weight/day of an inventive compound can be administered.

In those compositions which comprise an additional therapeutic agent, that additional therapeutic agent and the compound of this invention may act synergistically. Therefore, the amount of additional therapeutic agent in such compositions will be less than that required in a monotherapy utilizing only that therapeutic agent. In such compositions a dosage of between 0.01 - 1,000 µg/kg body weight/day of the additional therapeutic agent can be administered.

The amount of one or more other therapeutic agent present in the compositions of this invention may be no more than the amount that would normally be administered in a composition comprising that therapeutic agent as the only active agent. Preferably the amount of one or more other therapeutic agent in the presently disclosed compositions will range from about 50% to 100% of the amount normally present in a composition comprising that agent as the only therapeutically active agent. In some embodiments, one or more other therapeutic agent is administered at a dosage of about 50%, about 55%, about 60%, about 65%, about 70%, about 75%, about 80%, about 85%, about 90%, or about 95% of the amount normally administered for that agent. As used herein, the phrase "normally administered" means the amount an FDA approved therapeutic agent is approvided for dosing per the FDA label insert.

The compounds of this invention, or pharmaceutical compositions thereof, may also be incorporated into compositions for coating an implantable medical device, such as prostheses, artificial valves, vascular grafts, stents and catheters. Vascular stents, for example, have been used to overcome restenosis (re-narrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Implantable devices coated with a compound of this invention are another embodiment of the present invention.

### Exemplary Immuno-Oncology agents

In some embodiments, one or more other therapeutic agent is an immuno-oncology agent. As used herein, the term "an immuno-oncology agent" refers to an agent which is effective to enhance, stimulate, and/or up-regulate immune responses in a subject. In some embodiments, the administration of an immuno-oncology agent with a compound of the invention has a synergic effect in treating a cancer.

An immuno-oncology agent can be, for example, a small molecule drug, an antibody, or a biologic or small molecule. Examples of biologic immuno-oncology agents include, but are not limited to, cancer vaccines, antibodies, and cytokines. In some embodiments, an antibody is a monoclonal antibody. In some embodiments, a monoclonal antibody is humanized or human.

In some embodiments, an immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses.

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α1β2, FAS, FASL, RELT, DR6, TROY, NGFR.

In some embodiments, an immuno-oncology agent is a cytokine that inhibits T cell activation (e.g., IL-6, IL-10, TGF-β, VEGF, and other immunosuppressive cytokines) or a cytokine that stimulates T cell activation, for stimulating an immune response.

In some embodiments, a combination of a compound of the invention and an immuno-oncology agent can stimulate T cell responses. In some embodiments, an immuno-oncology agent is: (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4; or (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

In some embodiments, an immuno-oncology agent is an antagonist of inhibitory receptors on NK cells or an agonists of activating receptors on NK cells. In some embodiments, an immuno-oncology agent is an antagonists of KIR, such as lirilumab.

In some embodiments, an immuno-oncology agent is an agent that inhibits or depletes macrophages or monocytes, including but not limited to CSF-1R antagonists such as CSF-1R antagonist antibodies including RG7155 (WO 2011/070024, US 2011/0165156, WO 2011/0107553, US 2012/0329997, WO 2011/131407, US 2013/0005949, WO 2013/087699, US 2014/0336363, WO 2013/119716, WO 2013/132044, US 2014/0079706) or FPA-008 (WO 2011/140249, US 2011/0274683; WO 2013/169264; WO 2014/036357, US 2014/0079699).

In some embodiments, an immuno-oncology agent is selected from agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell energy or exhaustion) and agents that trigger innate immune activation and/or inflammation at tumor sites.

In some embodiments, an immuno-oncology agent is a CTLA-4 antagonist. In some embodiments, a CTLA-4 antagonist is an antagonistic CTLA-4 antibody. In some embodiments, an antagonistic CTLA-4 antibody is YERVOY (ipilimumab) or tremelimumab.

In some embodiments, an immuno-oncology agent is a PD-1 antagonist. In some embodiments, a PD-1 antagonist is administered by infusion. In some embodiments, an immuno-oncology agent is an antibody or an antigen-binding portion thereof that binds specifically to a Programmed Death-1 (PD-1) receptor and inhibits PD-1 activity. In some embodiments, a PD-1 antagonist is an antagonistic PD-1 antibody. In some embodiments, an antagonistic PD-1 antibody is OPDIVO (nivolumab), KEYTRUDA (pembrolizumab), or MEDI-0680 (AMP-514; WO2012/145493). In some embodiments, an immuno-oncology agent may be pidilizumab (CT-011). In some embodiments, an immuno-oncology agent is a recombinant protein composed of the extracellular domain of PD-L2 (B7-DC) fused to the Fc portion of IgG1, called AMP-224.

In some embodiments, an immuno-oncology agent is a PD-L1 antagonist. In some embodiments, a PD-L1 antagonist is an antagonistic PD-L1 antibody. In some embodiments, a PD-L1 antibody is MPDL3280A (RG7446; WO 2010/077634, US 2010/0203056), durvalumab (MEDI4736), BMS-936559 (WO 2007/005874, US 2009/0055944), and MSB0010718C (WO 2013/079174, US 2014/0341917).

In some embodiments, an immuno-oncology agent is a LAG-3 antagonist. In some embodiments, a LAG-3 antagonist is an antagonistic LAG-3 antibody. In some embodiments, a LAG3 antibody is BMS-986016 (WO 2010/019570, US 2010/0150892, WO 2014/008218, US 2014/0093511), or IMP-731 or IMP-321 (WO 2008/132601, US 2010/0233183, WO 2009/044273, US 2011/0008331).

In some embodiments, an immuno-oncology agent is a CD137 (4-1BB) agonist. In some embodiments, a CD137 (4-1BB) agonist is an agonistic CD137 antibody. In some embodiments, a CD137 antibody is urelumab or PF-05082566 (WO12/32433).

In some embodiments, an immuno-oncology agent is a GITR agonist. In some embodiments, a GITR agonist is an agonistic GITR antibody. In some embodiments, a GITR antibody is BMS-986153, BMS-986156, TRX-518 (WO 2006/105021, US 2007/0098719, WO 2009/009116, US 2009/0136494), or MK-4166 (WO 2011/028683, US 2012/0189639).

In some embodiments, an immuno-oncology agent is an indoleamine (2,3)-dioxygenase (IDO) antagonist. In some embodiments, an IDO antagonist is selected from epacadostat (INCB024360, Incyte); indoximod (NLG-8189, NewLink Genetics Corporation); capmanitib (INC280, Novartis); GDC-0919 (Genentech/Roche); PF-06840003 (Pfizer); BMS:F001287 (Bristol-Myers Squibb); Phy906/KD108 (Phytoceutica); an enzyme that breaks down kynurenine (Kynase, Kyn Therapeutics); and NLG-919 (WO 2009/073620, US 2011/0053941, WO 2009/132238, US 2011/0136796, WO 2011/056652, US 2012/0277217, WO 2012/142237, US 2014/0066625).

In some embodiments, an immuno-oncology agent is an OX40 agonist. In some embodiments, an OX40 agonist is an agonistic OX40 antibody. In some embodiments, an OX40 antibody is MEDI-6383 or MEDI-6469.

In some embodiments, an immuno-oncology agent is an OX40L antagonist. In some embodiments, an OX40L antagonist is an antagonistic OX40 antibody. In some embodiments, an OX40L antagonist is RG-7888 (WO 2006/029879, US 7,501,496).

In some embodiments, an immuno-oncology agent is a CD40 agonist. In some embodiments, a CD40 agonist is an agonistic CD40 antibody. In some embodiments, an immuno-oncology agent is a CD40 antagonist. In some embodiments, a CD40 antagonist is an antagonistic CD40 antibody. In some embodiments, a CD40 antibody is lucatumumab or dacetuzumab.

In some embodiments, an immuno-oncology agent is a CD27 agonist. In some embodiments, a CD27 agonist is an agonistic CD27 antibody. In some embodiments, a CD27 antibody is varlilumab.

In some embodiments, an immuno-oncology agent is MGA271 (to B7H3) (WO 2011/109400, US 2013/0149236).

In some embodiments, an immuno-oncology agent is abagovomab, adecatumumab, afutuzumab, alemtuzumab, anatumomab mafenatox, apolizumab, atezolimab, avelumab, blinatumomab, BMS-936559, catumaxomab, durvalumab, epacadostat, epratuzumab, indoximod, inotuzumab ozogamicin, intelumumab, ipilimumab, isatuximab, lambrolizumab, MED14736, MPDL3280A, nivolumab, obinutuzumab, ocaratuzumab, ofatumumab, olatatumab, pembrolizumab, pidilizumab, rituximab, ticilimumab, samalizumab, or tremelimumab.

In some embodiments, an immuno-oncology agent is an immunostimulatory agent. For example, antibodies blocking the PD-1 and PD-L1 inhibitory axis can unleash activated tumor-reactive T cells and have been shown in clinical trials to induce durable anti-tumor responses in increasing numbers of tumor histologies, including some tumor types that conventionally have not been considered immunotherapy sensitive. See, e.g., Okazaki, T. et al. (2013) Nat. Immunol. 14, 1212-1218; Zou et al. (2016) Sci. Transl. Med. 8. The anti-PD-1 antibody nivolumab (Opdivo^{®}, Bristol-Myers Squibb, also known as ONO-4538, MDX1106 and BMS-936558), has shown potential to improve the overall survival in patients with RCC who had experienced disease progression during or after prior anti-angiogenic therapy.

In some embodiments, the immunomodulatory therapeutic specifically induces apoptosis of tumor cells. Approved immunomodulatory therapeutics which may be used in the present invention include pomalidomide (Pomalyst^{®}, Celgene); lenalidomide (Revlimid^{®}, Celgene); ingenol mebutate (Picato^{®}, LEO Pharma).

In some embodiments, an immuno-oncology agent is a cancer vaccine. In some embodiments, the cancer vaccine is selected from sipuleucel-T (Provenge^{®}, Dendreon/Valeant Pharmaceuticals), which has been approved for treatment of asymptomatic, or minimally symptomatic metastatic castrate-resistant (hormone-refractory) prostate cancer; and talimogene laherparepvec (Imlygic^{®}, BioVex/Amgen, previously known as T-VEC), a genetically modified oncolytic viral therapy approved for treatment of unresectable cutaneous, subcutaneous and nodal lesions in melanoma. In some embodiments, an immuno-oncology agent is selected from an oncolytic viral therapy such as pexastimogene devacirepvec (PexaVec/JX-594, SillaJen/formerly Jennerex Biotherapeutics), a thymidine kinase- (TK-) deficient vaccinia virus engineered to express GM-CSF, for hepatocellular carcinoma (NCT02562755) and melanoma (NCT00429312); pelareorep (Reolysin^{®}, Oncolytics Biotech), a variant of respiratory enteric orphan virus (reovirus) which does not replicate in cells that are not RAS-activated, in numerous cancers, including colorectal cancer (NCT01622543); prostate cancer (NCT01619813); head and neck squamous cell cancer (NCT01166542); pancreatic adenocarcinoma (NCT00998322); and non-small cell lung cancer (NSCLC) (NCT 00861627); enadenotucirev (NG-348, PsiOxus, formerly known as ColoAd1), an adenovirus engineered to express a full length CD80 and an antibody fragment specific for the T-cell receptor CD3 protein, in ovarian cancer (NCT02028117); metastatic or advanced epithelial tumors such as in colorectal cancer, bladder cancer, head and neck squamous cell carcinoma and salivary gland cancer (NCT02636036); ONCOS-102 (Targovax/formerly Oncos), an adenovirus engineered to express GM-CSF, in melanoma (NCT03003676); and peritoneal disease, colorectal cancer or ovarian cancer (NCT02963831); GL-ONC1 (GLU-1h68/GLV-1h153, Genelux GmbH), vaccinia viruses engineered to express beta-galactosidase (beta-gal)/beta-glucoronidase or beta-gal/human sodium iodide symporter (hNIS), respectively, were studied in peritoneal carcinomatosis (NCT01443260); fallopian tube cancer, ovarian cancer (NCT 02759588); or CG0070 (Cold Genesys), an adenovirus engineered to express GM-CSF, in bladder cancer (NCT02365818).

In some embodiments, an immuno-oncology agent is selected from JX-929 (SillaJen/formerly Jennerex Biotherapeutics), a TK- and vaccinia growth factor-deficient vaccinia virus engineered to express cytosine deaminase, which is able to convert the prodrug 5-fluorocytosine to the cytotoxic drug 5-fluorouracil; TG01 and TG02 (Targovax/formerly Oncos), peptide-based immunotherapy agents targeted for difficult-to-treat RAS mutations; and TILT-123 (TILT Biotherapeutics), an engineered adenovirus designated: Ad5/3-E2F-delta24-hTNFα-IRES-hIL20; and VSV-GP (ViraTherapeutics) a vesicular stomatitis virus (VSV) engineered to express the glycoprotein (GP) of lymphocytic choriomeningitis virus (LCMV), which can be further engineered to express antigens designed to raise an antigen-specific CD8⁺ T cell response.

In some embodiments, an immuno-oncology agent is a T-cell engineered to express a chimeric antigen receptor, or CAR. The T-cells engineered to express such chimeric antigen receptor are referred to as a CAR-T cells.

CARs have been constructed that consist of binding domains, which may be derived from natural ligands, single chain variable fragments (scFv) derived from monoclonal antibodies specific for cell-surface antigens, fused to endodomains that are the functional end of the T-cell receptor (TCR), such as the CD3-zeta signaling domain from TCRs, which is capable of generating an activation signal in T lymphocytes. Upon antigen binding, such CARs link to endogenous signaling pathways in the effector cell and generate activating signals similar to those initiated by the TCR complex.

For example, in some embodiments the CAR-T cell is one of those described in U.S. Patent 8,906,682, the entirety of each of which is herein incorporated by reference, which discloses CAR-T cells engineered to comprise an extracellular domain having an antigen binding domain (such as a domain that binds to CD19), fused to an intracellular signaling domain of the T cell antigen receptor complex zeta chain (such as CD3 zeta). When expressed in the T cell, the CAR is able to redirect antigen recognition based on the antigen binding specificity. In the case of CD19, the antigen is expressed on malignant B cells. Over 200 clinical trials are currently in progress employing CAR-T in a wide range of indications. [https://clinicaltrials.gov/ct2/results?term=chimeric+antigen+receptors&pg=1].

In some embodiments, an immunostimulatory agent is an activator of retinoic acid receptor-related orphan receptor y (RORyt). RORγt is a transcription factor with key roles in the differentiation and maintenance of Type 17 effector subsets of CD4+ (Th17) and CD8+ (Tc17) T cells, as well as the differentiation of IL-17 expressing innate immune cell subpopulations such as NK cells. In some embodiments, an activator of RORyt is LYC-55716 (Lycera), which is currently being evaluated in clinical trials for the treatment of solid tumors (NCT02929862).

In some embodiments, an immunostimulatory agent is an agonist or activator of a toll-like receptor (TLR). Suitable activators of TLRs include an agonist or activator of TLR9 such as SD-101 (Dynavax). SD-101 is an immunostimulatory CpG which is being studied for B-cell, follicular and other lymphomas (NCT02254772). Agonists or activators of TLR8 which may be used in the present invention include motolimod (VTX-2337, VentiRx Pharmaceuticals) which is being studied for squamous cell cancer of the head and neck (NCT02124850) and ovarian cancer (NCT02431559).

Other immuno-oncology agents that may be used in the present invention include urelumab (BMS-663513, Bristol-Myers Squibb), an anti-CD137 monoclonal antibody; varlilumab (CDX-1127, Celldex Therapeutics), an anti-CD27 monoclonal antibody; BMS-986178 (Bristol-Myers Squibb), an anti-OX40 monoclonal antibody; lirilumab (IPH2102/BMS-986015, Innate Pharma, Bristol-Myers Squibb), an anti-KIR monoclonal antibody; monalizumab (IPH2201, Innate Pharma, AstraZeneca) an anti-NKG2A monoclonal antibody; andecaliximab (GS-5745, Gilead Sciences), an anti-MMP9 antibody; MK-4166 (Merck & Co.), an anti-GITR monoclonal antibody.

In some embodiments, an immunostimulatory agent is selected from elotuzumab, mifamurtide, an agonist or activator of a toll-like receptor, and an activator of RORyt.

In some embodiments, an immunostimulatory therapeutic is recombinant human interleukin 15 (rhIL-15). rhIL-15 has been tested in the clinic as a therapy for melanoma and renal cell carcinoma (NCT01021059 and NCT01369888) and leukemias (NCT02689453). In some embodiments, an immunostimulatory agent is recombinant human interleukin 12 (rhIL-12). In some embodiments, an IL-15 based immunotherapeutic is heterodimeric IL-15 (hetIL-15, Novartis/Admune), a fusion complex composed of a synthetic form of endogenous IL-15 complexed to the soluble IL-15 binding protein IL-15 receptor alpha chain (IL15:sIL-15RA), which has been tested in Phase 1 clinical trials for melanoma, renal cell carcinoma, non-small cell lung cancer and head and neck squamous cell carcinoma (NCT02452268). In some embodiments, a recombinant human interleukin 12 (rhIL-12) is NM-IL-12 (Neumedicines, Inc.), NCT02544724, or NCT02542124.

In some embodiments, an immuno-oncology agent is selected from those descripted in Jerry L. Adams ET. AL., "Big opportunities for small molecules in immuno-oncology," Cancer Therapy 2015, Vol. 14, pages 603-622, the content of which is incorporated herein by refenrece in its entirety. In some embodimetne, an immuno-oncology agent is selected from the examples described in Table 1 of Jerry L. Adams ET. AL. In some embodiments, an immuno-oncology agent is a small molecule targeting an immuno-oncoloby target selected from those listed in Table 2 of Jerry L. Adams ET. AL. In some embodiments, an immuno-oncology agent is a small molecule agent selectd from those listed in Table 2 of Jerry L. Adams ET. AL.

In some embodiments, an immuno-oncology agent is selected from the small molecule immuno-oncology agents described in Peter L. Toogood, "Small molecule immuno-oncology therapeutic agents," Bioorganic & Medicinal Chemistry Letters 2018, Vol. 28, pages 319-329, the content of which is incorporated herein by refenrece in its entirety. In some embodiments, an immuno-oncology agent is an agent targeting the pathways as described in Peter L. Toogood.

In some embodiments, an immuno-oncology agent is selected from those described in Sandra L. Ross et al., "Bispecific T cell engager (BiTE® ) antibody constructs can mediate bystander tumor cell killing", PLoS ONE 12(8): e0183390, the conten of which is incorporated herein by reference in its entirety. In some embodiments, an immuno-oncology agent is a bispecific T cell engager (BiTE^{®}) antibody construct. In some embodimens, a bispecific T cell engager (BiTE^{®}) antibody construct is a CD19/CD3 bispecific antibody construct. In some embodimens, a bispecific T cell engager (BiTE^{®}) antibody construct is an EGFR/CD3 bispecific antibody construct. In some embodimens, a bispecific T cell engager (BiTE^{®}) antibody construct activates T cells. In some embodimens, a bispecific T cell engager (BiTE^{®}) antibody construct activates T cells, which release cytokines inducing upregulation of intercellular adhesion molecule 1 (ICAM-1) and FAS on bystander cells. In some embodimens, a bispecific T cell engager (BiTE^{®}) antibody construct activates T cells which result in induced bystander cell lysis. In some embodiments, the bystander cells are in solid tumors. In some embodiments, the bystander cells being lysed are in proximity to the BiTE^{®}-acticvated T cells. In some embodiment, the bystander cells comprises tumor-associated antigen (TAA) negatgive cancer cells. In some embodiment, the bystander cells comprise EGFR-negative cancer cells. In some embodiments, an immuno-oncology agent is an antibody which blocks the PD-L1/PD1 axis and/or CTLA4. In some embodiments, an immuno-oncology agent is an ex-vivo expanded tumor-infiltrating T cell. In some embodiments, an immuno-oncology agent is a bispecific antibody construct or chimeric antigen receptors (CARs) that directly connect T cells with tumor-associated surface antigens (TAAs).

### Exemplary Immune Checkpoint Inhibitors

In some embodiments, an immuno-oncology agent is an immune checkpoint inhibitor as described herein.

The term "checkpoint inhibitor" as used herein relates to agents useful in preventing cancer cells from avoiding the immune system of the patient. One of the major mechanisms of anti-tumor immunity subversion is known as "T-cell exhaustion," which results from chronic exposure to antigens that has led to up-regulation of inhibitory receptors. These inhibitory receptors serve as immune checkpoints in order to prevent uncontrolled immune reactions.

PD-1 and co-inhibitory receptors such as cytotoxic T-lymphocyte antigen 4 (CTLA-4, B and T Lymphocyte Attenuator (BTLA; CD272), T cell Immunoglobulin and Mucin domain-3 (Tim-3), Lymphocyte Activation Gene-3 (Lag-3; CD223), and others are often referred to as a checkpoint regulators. They act as molecular "gatekeepers" that allow extracellular information to dictate whether cell cycle progression and other intracellular signaling processes should proceed.

In some embodiments, an immune checkpoint inhibitor is an antibody to PD-1. PD-1 binds to the programmed cell death 1 receptor (PD-1) to prevent the receptor from binding to the inhibitory ligand PDL-1, thus overriding the ability of tumors to suppress the host anti-tumor immune response.

In one aspect, the checkpoint inhibitor is a biologic therapeutic or a small molecule. In another aspect, the checkpoint inhibitor is a monoclonal antibody, a humanized antibody, a fully human antibody, a fusion protein or a combination thereof. In a further aspect, the checkpoint inhibitor inhibits a checkpoint protein selected from CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, B-7 family ligands or a combination thereof. In an additional aspect, the checkpoint inhibitor interacts with a ligand of a checkpoint protein selected from CTLA-4, PDL1, PDL2, PDl, B7-H3, B7-H4, BTLA, HVEM, TIM3, GAL9, LAG3, VISTA, KIR, 2B4, CD160, CGEN-15049, CHK 1, CHK2, A2aR, B-7 family ligands or a combination thereof. In an aspect, the checkpoint inhibitor is an immunostimulatory agent, a T cell growth factor, an interleukin, an antibody, a vaccine or a combination thereof. In a further aspect, the interleukin is IL-7 or IL-15. In a specific aspect, the interleukin is glycosylated IL-7. In an additional aspect, the vaccine is a dendritic cell (DC) vaccine.

Checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptors or antibodies that bind to and block or inhibit immune checkpoint receptor ligands. Illustrative checkpoint molecules that may be targeted for blocking or inhibition include, but are not limited to, CTLA-4, PDL1, PDL2, PD1, B7-H3, B7-H4, BTLA, HVEM, GAL9, LAG3, TIM3, VISTA, KIR, 2B4 (belongs to the CD2 family of molecules and is expressed on all NK, γδ, and memory CD8⁺ (αβ) T cells), CD160 (also referred to as BY55), CGEN-15049, CHK 1 and CHK2 kinases, A2aR, and various B-7 family ligands. B7 family ligands include, but are not limited to, B7- 1, B7-2, B7-DC, B7-H1, B7-H2, B7-H3, B7-H4, B7-H5, B7-H6 and B7-H7. Checkpoint inhibitors include antibodies, or antigen binding fragments thereof, other binding proteins, biologic therapeutics, or small molecules, that bind to and block or inhibit the activity of one or more of CTLA-4, PDL1, PDL2, PD1, BTLA, HVEM, TIM3, GAL9, LAG3, VISTA, KIR, 2B4, CD 160 and CGEN-15049. Illustrative immune checkpoint inhibitors include Tremelimumab (CTLA-4 blocking antibody), anti-OX40, PD-L1 monoclonal Antibody (Anti-B7-Hl; MEDI4736), MK-3475 (PD-1 blocker), Nivolumab (anti-PDl antibody), CT-011 (anti-PDl antibody), BY55 monoclonal antibody, AMP224 (anti-PDL1 antibody), BMS- 936559 (anti-PDL1 antibody), MPLDL3280A (anti-PDL1 antibody), MSB0010718C (anti-PDL1 antibody), and ipilimumab (anti-CTLA-4 checkpoint inhibitor). Checkpoint protein ligands include, but are not limited to PD-L1, PD-L2, B7-H3, B7-H4, CD28, CD86 and TIM-3.

In certain embodiments, the immune checkpoint inhibitor is selected from a PD-1 antagonist, a PD-L1 antagonist, and a CTLA-4 antagonist. In some embodiments, the checkpoint inhibitor is selected from the group consisting of nivolumab (Opdivo^{®}), ipilimumab (Yervoy^{®}), and pembrolizumab (Keytruda^{®}). In some embodiments, the checkpoint inhibitor is selected from nivolumab (anti-PD-1 antibody, Opdivo^{®}, Bristol-Myers Squibb); pembrolizumab (anti-PD-1 antibody, Keytruda^{®}, Merck); ipilimumab (anti-CTLA-4 antibody, Yervoy^{®}, Bristol-Myers Squibb); durvalumab (anti-PD-L1 antibody, Imfinzi^{®}, AstraZeneca); and atezolizumab (anti-PD-L1 antibody, Tecentriq^{®}, Genentech).

In some embodiments, the checkpoint inhibitor is selected from the group consisting of lambrolizumab (MK-3475), nivolumab (BMS-936558), pidilizumab (CT-011), AMP-224, MDX-1105, MEDI4736, MPDL3280A, BMS-936559, ipilimumab, lirlumab, IPH2101, pembrolizumab (Keytruda^{®}), and tremelimumab.

In some embodiments, an immune checkpoint inhibitor is REGN2810 (Regeneron), an anti-PD-1 antibody tested in patients with basal cell carcinoma (NCT03132636); NSCLC (NCT03088540); cutaneous squamous cell carcinoma (NCT02760498); lymphoma (NCT02651662); and melanoma (NCT03002376); pidilizumab (CureTech), also known as CT-011, an antibody that binds to PD-1, in clinical trials for diffuse large B-cell lymphoma and multiple myeloma; avelumab (Bavencio^{®}, Pfizer/Merck KGaA), also known as MSB0010718C), a fully human IgG1 anti-PD-L1 antibody, in clinical trials for non-small cell lung cancer, Merkel cell carcinoma, mesothelioma, solid tumors, renal cancer, ovarian cancer, bladder cancer, head and neck cancer, and gastric cancer; or PDR001 (Novartis), an inhibitory antibody that binds to PD-1, in clinical trials for non-small cell lung cancer, melanoma, triple negative breast cancer and advanced or metastatic solid tumors. Tremelimumab (CP-675,206; Astrazeneca) is a fully human monoclonal antibody against CTLA-4 that has been in studied in clinical trials for a number of indications, including: mesothelioma, colorectal cancer, kidney cancer, breast cancer, lung cancer and non-small cell lung cancer, pancreatic ductal adenocarcinoma, pancreatic cancer, germ cell cancer, squamous cell cancer of the head and neck, hepatocellular carcinoma, prostate cancer, endometrial cancer, metastatic cancer in the liver, liver cancer, large B-cell lymphoma, ovarian cancer, cervical cancer, metastatic anaplastic thyroid cancer, urothelial cancer, fallopian tube cancer, multiple myeloma, bladder cancer, soft tissue sarcoma, and melanoma. AGEN-1884 (Agenus) is an anti-CTLA4 antibody that is being studied in Phase 1 clinical trials for advanced solid tumors (NCT02694822).

In some embodiments, a checkpoint inhibitor is an inhibitor of T-cell immunoglobulin mucin containing protein-3 (TIM-3). TIM-3 inhibitors that may be used in the present invention include TSR-022, LY3321367 and MBG453. TSR-022 (Tesaro) is an anti-TIM-3 antibody which is being studied in solid tumors (NCT02817633). LY3321367 (Eli Lilly) is an anti-TIM-3 antibody which is being studied in solid tumors (NCT03099109). MBG453 (Novartis) is an anti-TIM-3 antibody which is being studied in advanced malignancies (NCT02608268).

In some embodiments, a checkpoint inhibitor is an inhibitor of T cell immunoreceptor with Ig and ITIM domains, or TIGIT, an immune receptor on certain T cells and NK cells. TIGIT inhibitors that may be used in the present invention include BMS-986207 (Bristol-Myers Squibb), an anti-TIGIT monoclonal antibody (NCT02913313); OMP-313M32 (Oncomed); and anti-TIGIT monoclonal antibody (NCT03119428).

In some embodiments, a checkpoint inhibitor is an inhibitor of Lymphocyte Activation Gene-3 (LAG-3). LAG-3 inhibitors that may be used in the present invention include BMS-986016 and REGN3767 and IMP321. BMS-986016 (Bristol-Myers Squibb), an anti-LAG-3 antibody, is being studied in glioblastoma and gliosarcoma (NCT02658981). REGN3767 (Regeneron), is also an anti-LAG-3 antibody, and is being studied in malignancies (NCT03005782). IMP321 (Immutep S.A.) is an LAG-3-Ig fusion protein, being studied in melanoma (NCT02676869); adenocarcinoma (NCT02614833); and metastatic breast cancer (NCT00349934).

Checkpoint inhibitors that may be used in the present invention include OX40 agonists. OX40 agonists that are being studied in clinical trials include PF-04518600/PF-8600 (Pfizer), an agonistic anti-OX40 antibody, in metastatic kidney cancer (NCT03092856) and advanced cancers and neoplasms (NCT02554812; NCT05082566); GSK3174998 (Merck), an agonistic anti-OX40 antibody, in Phase 1 cancer trials (NCT02528357); MEDI0562 (Medimmune/AstraZeneca), an agonistic anti-OX40 antibody, in advanced solid tumors (NCT02318394 and NCT02705482); MEDI6469, an agonistic anti-OX40 antibody (Medimmune/AstraZeneca), in patients with colorectal cancer (NCT02559024), breast cancer (NCT01862900), head and neck cancer (NCT02274155) and metastatic prostate cancer (NCT01303705); and BMS-986178 (Bristol-Myers Squibb) an agonistic anti-OX40 antibody, in advanced cancers (NCT02737475).

Checkpoint inhibitors that may be used in the present invention include CD137 (also called 4-1BB) agonists. CD137 agonists that are being studied in clinical trials include utomilumab (PF-05082566, Pfizer) an agonistic anti-CD137 antibody, in diffuse large B-cell lymphoma (NCT02951156) and in advanced cancers and neoplasms (NCT02554812 and NCT05082566); urelumab (BMS-663513, Bristol-Myers Squibb), an agonistic anti-CD137 antibody, in melanoma and skin cancer (NCT02652455) and glioblastoma and gliosarcoma (NCT02658981).

Checkpoint inhibitors that may be used in the present invention include CD27 agonists. CD27 agonists that are being studied in clinical trials include varlilumab (CDX-1127, Celldex Therapeutics) an agonistic anti-CD27 antibody, in squamous cell head and neck cancer, ovarian carcinoma, colorectal cancer, renal cell cancer, and glioblastoma (NCT02335918); lymphomas (NCT01460134); and glioma and astrocytoma (NCT02924038).

Checkpoint inhibitors that may be used in the present invention include glucocorticoid-induced tumor necrosis factor receptor (GITR) agonists. GITR agonists that are being studied in clinical trials include TRX518 (Leap Therapeutics), an agonistic anti-GITR antibody, in malignant melanoma and other malignant solid tumors (NCT01239134 and NCT02628574); GWN323 (Novartis), an agonistic anti-GITR antibody, in solid tumors and lymphoma (NCT 02740270); INCAGN01876 (Incyte/Agenus), an agonistic anti-GITR antibody, in advanced cancers (NCT02697591 and NCT03126110); MK-4166 (Merck), an agonistic anti-GITR antibody, in solid tumors (NCT02132754) and MEDI1873 (Medimmune/AstraZeneca), an agonistic hexameric GITR-ligand molecule with a human IgG1 Fc domain, in advanced solid tumors (NCT02583165).

Checkpoint inhibitors that may be used in the present invention include inducible T-cell co-stimulator (ICOS, also known as CD278) agonists. ICOS agonists that are being studied in clinical trials include MEDI-570 (Medimmune), an agonistic anti-ICOS antibody, in lymphomas (NCT02520791); GSK3359609 (Merck), an agonistic anti-ICOS antibody, in Phase 1 (NCT02723955); JTX-2011 (Jounce Therapeutics), an agonistic anti-ICOS antibody, in Phase 1 (NCT02904226).

Checkpoint inhibitors that may be used in the present invention include killer IgG-like receptor (KIR) inhibitors. KIR inhibitors that are being studied in clinical trials include lirilumab (IPH2102/BMS-986015, Innate Pharma/Bristol-Myers Squibb), an anti-KIR antibody, in leukemias (NCT01687387, NCT02399917, NCT02481297, NCT02599649), multiple myeloma (NCT02252263), and lymphoma (NCT01592370); IPH2101 (1-7F9, Innate Pharma) in myeloma (NCT01222286 and NCT01217203); and IPH4102 (Innate Pharma), an anti-KIR antibody that binds to three domains of the long cytoplasmic tail (KIR3DL2), in lymphoma (NCT02593045).

Checkpoint inhibitors that may be used in the present invention include CD47 inhibitors of interaction between CD47 and signal regulatory protein alpha (SIRPa). CD47/SIRPa inhibitors that are being studied in clinical trials include ALX-148 (Alexo Therapeutics), an antagonistic variant of (SIRPa) that binds to CD47 and prevents CD47/SIRPa-mediated signaling, in phase 1 (NCT03013218); TTI-621 (SIRPa-Fc, Trillium Therapeutics), a soluble recombinant fusion protein created by linking the N-terminal CD47-binding domain of SIRPa with the Fc domain of human IgG1, acts by binding human CD47, and preventing it from delivering its "do not eat" signal to macrophages, is in clinical trials in Phase 1 (NCT02890368 and NCT02663518); CC-90002 (Celgene), an anti-CD47 antibody, in leukemias (NCT02641002); and Hu5F9-G4 (Forty Seven, Inc.), in colorectal neoplasms and solid tumors (NCT02953782), acute myeloid leukemia (NCT02678338) and lymphoma (NCT02953509).

Checkpoint inhibitors that may be used in the present invention include CD73 inhibitors. CD73 inhibitors that are being studied in clinical trials include MEDI9447 (Medimmune), an anti-CD73 antibody, in solid tumors (NCT02503774); and BMS-986179 (Bristol-Myers Squibb), an anti-CD73 antibody, in solid tumors (NCT02754141).

Checkpoint inhibitors that may be used in the present invention include agonists of stimulator of interferon genes protein (STING, also known as transmembrane protein 173, or TMEM173). Agonists of STING that are being studied in clinical trials include MK-1454 (Merck), an agonistic synthetic cyclic dinucleotide, in lymphoma (NCT03010176); and ADU-S100 (MIW815, Aduro Biotech/Novartis), an agonistic synthetic cyclic dinucleotide, in Phase 1 (NCT02675439 and NCT03172936).

Checkpoint inhibitors that may be used in the present invention include CSF1R inhibitors. CSF1R inhibitors that are being studied in clinical trials include pexidartinib (PLX3397, Plexxikon), a CSF1R small molecule inhibitor, in colorectal cancer, pancreatic cancer, metastatic and advanced cancers (NCT02777710) and melanoma, non-small cell lung cancer, squamous cell head and neck cancer, gastrointestinal stromal tumor (GIST) and ovarian cancer (NCT02452424); and IMC-CS4 (LY3022855, Lilly), an anti-CSF-1R antibody, in pancreatic cancer (NCT03153410), melanoma (NCT03101254), and solid tumors (NCT02718911); and BLZ945 (4-[2((1R,2R)-2-hydroxycyclohexylamino)-benzothiazol-6-yloxyl]-pyridine-2-carboxylic acid methylamide, Novartis), an orally available inhibitor of CSF1R, in advanced solid tumors (NCT02829723).

Checkpoint inhibitors that may be used in the present invention include NKG2A receptor inhibitors. NKG2A receptor inhibitors that are being studied in clinical trials include monalizumab (IPH2201, Innate Pharma), an anti-NKG2A antibody, in head and neck neoplasms (NCT02643550) and chronic lymphocytic leukemia (NCT02557516).

In some embodiments, the immune checkpoint inhibitor is selected from nivolumab, pembrolizumab, ipilimumab, avelumab, durvalumab, atezolizumab, or pidilizumab.

### EXEMPLIFICATION

### Abbreviations

Ac: acetyl
AcOH: acetic acid
ACN: acetonitrile
Ad: adamantly
AIBN: 2,2'-azo bisisobutyronitrile
Anhyd: anhydrous
Aq: aqueous
B₂Pin₂: bis (pinacolato)diboron -4,4,4',4',5,5,5',5'-octamethyl-2,2'-bi(1,3,2-dioxaborolane)
BINAP: 2,2'-bis(diphenylphosphino)-1,1'-binaphthyl
BH₃: Borane
Bn: benzyl
Boc: *tert*-butoxycarbonyl
Boc₂O: di-tert-butyl dicarbonate
BPO: benzoyl peroxide
ⁿBuOH: n-butanol
CDI: carbonyldiimidazole
COD: cyclooctadiene
d: days
DABCO: 1,4-diazobicyclo[2.2.2]octane
DAST: diethylaminosulfur trifluoride
dba: dibenzylideneacetone
DBU: 1,8-diazobicyclo[5.4.0]undec-7-ene
DCE: 1,2-dichloroethane
DCM: dichloromethane
DEA: diethylamine
DHP: dihydropyran
DIBAL-H: diisobutylaluminum hydride
DIPA: diisopropylamine
DIPEA or DIEA: N,N-diisopropylethylamine
DMA: N,N-dimethylacetamide
DME: 1,2-dimethoxyethane
DMAP: 4-dimethylaminopyridine
DMF: N,N-dimethylformamide
DMP: Dess-Martin periodinane
DMSO-dimethyl sulfoxide
DPPA: diphenylphosphoryl azide
dppf: 1,1'-bis(diphenylphosphino)ferrocene
EDC or EDCI: 1-(3-dimethylaminopropyl)-3-ethylcarbodiimide hydrochloride ee: enantiomeric excess
ESI: electrospray ionization
EA: ethyl acetate
EtOAc: ethyl acetate
EtOH: ethanol
FA: formic acid
h or hrs: hours
HATU: N,N,N',N'-tetramethyl-O-(7-azabenzotriazol-1-yl)uronium hexafluorophosphate
HCl: hydrochloric acid
HPLC: high performance liquid chromatography
HOAc: acetic acid
IBX: 2-iodoxybenzoic acid
IPA: isopropyl alcohol
KHMDS: potassium hexamethyldisilazide
K₂CO₃: potassium carbonate
LAH: lithium aluminum hydride
LDA: lithium diisopropylamide
m-CPBA: meta-chloroperbenzoic acid
M: molar
MeCN: acetonitrile
MeOH: methanol
Me₂S: dimethyl sulfide
MeONa: sodium methylate
MeI: iodomethane
min: minutes
mL: milliliters
mM: millimolar
mmol: millimoles
MPa: mega pascal
MOMCl: methyl chloromethyl ether
MsCl: methanesulfonyl chloride
MTBE: methyl *tert*-butyl ether
nBuLi: n-butyllithium
NaNO₂: sodium nitrite
NaOH: sodium hydroxide
Na₂SO₄: sodium sulfate
NBS: N-bromosuccinimide
NCS: N-chlorosuccinimide
NFSI: N-Fluorobenzenesulfonimide
NMO: N-methylmorpholine N-oxide
NMP: N-methylpyrrolidine
NMR: Nuclear Magnetic Resonance
°C: degrees Celsius
Pd/C: Palladium on Carbon
Pd(OAc)₂: Palladium Acetate
PBS: phosphate buffered saline
PE: petroleum ether
POCl₃: phosphorus oxychloride
PPh₃: triphenylphosphine
PyBOP: (Benzotriazol-1-yloxy)tripyrrolidinophosphonium hexafluorophosphate
Rel: relative
R.T. or rt: room temperature
sat: saturated
SEMCl: chloromethyl-2-trimethylsilylethyl ether
SFC: supercritical fluid chromatography
SOCl₂: sulfur dichloride
tBuOK: potassium *tert*-butoxide
TBAB: tetrabutylammonium bromide
TBAI: tetrabutylammonium iodide
TEA: triethylamine
Tf: trifluoromethanesulfonate
TfAA, TFMSA or Tf₂O: trifluoromethanesulfonic anhydride
TFA: trifluoracetic acid
TIPS: triisopropylsilyl
THF: tetrahydrofuran
THP: tetrahydropyran
TLC: thin layer chromatography
TMEDA: tetramethylethylenediamine
pTSA: para-toluenesulfonic acid
wt: weight
Xantphos: 4,5-bis(diphenylphosphino)-9,9-dimethylxanthene

### General Synthetic Methods

The following examples are intended to illustrate the invention and are not to be construed as being limitations thereon. Temperatures are given in degrees centigrade. If not mentioned otherwise, all evaporations are performed under reduced pressure, preferably between about 15 mm Hg and 100 mm Hg (= 20-133 mbar). The structure of final products, intermediates and starting materials is confirmed by standard analytical methods, e.g., microanalysis and spectroscopic characteristics, e.g., MS, IR, NMR. Abbreviations used are those conventional in the art.

All starting materials, building blocks, reagents, acids, bases, dehydrating agents, solvents, and catalysts utilized to synthesis the compounds of the present invention are either commercially available or can be produced by organic synthesis methods known to one of ordinary skill in the art (Houben-Weyl 4th Ed. 1952, Methods of Organic Synthesis, Thieme, Volume 21). Further, the compounds of the present invention can be produced by organic synthesis methods known to one of ordinary skill in the art as shown in the following examples.

All reactions are carried out under nitrogen or argon unless otherwise stated.

Proton NMR (¹H NMR) is conducted in deuterated solvent. In certain compounds disclosed herein, one or more ¹H shifts overlap with residual proteo solvent signals; these signals have not been reported in the experimental provided hereinafter.

**Table 5: Analytical instruments**

| | |
|---|---|
| LCMS | Shimadzu UFLC MS: LCMS-2020 |
| | Agilent Technologies 1200 series MS: Agilent Technologies 6110 |
| | Agilent Technologies 1200 series MS: LC/MSD VL |
| NMR | BRUKER AVANCE III/400; Frequency (MHz) 400.13; Nucleus: 1H; Number of Transients: 8 |
| Prep-HPLC | Gilson GX-281 systems: instruments GX-A, GX-B, GX-C, GX-D, GX-E, GX-F, GX-G and GX-H |
| GCMS | SHIMADZU GCMS-QP2010 Ultra |
| Analytical cSFC | Agilent Technologies 1290 Infinity |
| Prep-cSFC | Waters SFC Prep 80 |

For acidic LCMS data: LCMS was recorded on an Agilent 1200 Series LC/MSD or Shimadzu LCMS2020 equipped with electro-spray ionization and quadruple MS detector [ES+ve to give MH⁺] and equipped with Chromolith Flash RP-18e 25*2.0 mm, eluting with 0.0375 vol% TFA in water (solvent A) and 0.01875 vol% TFA in acetonitrile (solvent B). Other LCMS was recorded on an Agilent 1290 Infinity RRLC attached with Agilent 6120 Mass detector. The column used was BEH C18 50*2.1 mm, 1.7 micron. Column flow was 0.55 ml /min and mobile phase were used (A) 2 mM Ammonium Acetate in 0.1% Formic Acid in Water and (B) 0.1 % Formic Acid in Acetonitrile.

For basic LCMS data: LCMS was recorded on an Agilent 1200 Series LC/MSD or Shimadzu LCMS 2020 equipped with electro-spray ionization and quadruple MS detector [ES+ve to give MH⁺] and equipped with Xbridge C18, 2.1X50 mm columns packed with 5 mm C18-coated silica or Kinetex EVO C18 2.1X30mm columns packed with 5 mm C18-coated silica, eluting with 0.05 vol% NH₃·H₂O in water (solvent A) and acetonitrile (solvent B).

HPLC Analytical Method: HPLC was carried out on X Bridge C18 150*4.6 mm, 5 micron. Column flow was 1.0 ml /min and mobile phase were used (A) 0.1 % Ammonia in water and (B) 0.1 % Ammonia in Acetonitrile.

Prep HPLC Analytical Method: The compound was purified on Shimadzu LC-20AP and UV detector. The column used was X-BRIDGE C18 (250*19)mm, 5µ. Column flow was 16.0 ml/min. Mobile phase were used (A) 0.1% Formic Acid in Water and (B) Acetonitrile Basic method used (A) 5mM ammonium bicarbonate and 0.1% NH3 in Water and (B) Acetonitrile or (A) 0.1% Ammonium Hydroxide in Water and (B) Acetonitrile. The UV spectra were recorded at 202nm & 254nm.

NMR Method: The 1H NMR spectra were recorded on a Bruker Ultra Shield Advance 400 MHz/5 mm Probe (BBFO). The chemical shifts are reported in part-per-million.

As depicted in the Examples below, in certain exemplary embodiments, compounds are prepared according to the following general procedures. It will be appreciated that, although the general methods depict the synthesis of certain compounds of the present invention, the following general methods, and other methods known to one of ordinary skill in the art, can be applied to all compounds and subclasses and species of each of these compounds, as described herein.

The synthesis of intermediates is disclosed in paragraphs [00971] - [005225] of WO2020/113233, which part of WO2020/113233 is incorporated by reference into the present disclosure.

### Example 1 (Method 1). Synthesis of N-[3-carbamoyl-1-[4-[3-[3-[1-(2,6-dioxo-3-piperidyl)-3-methyl-2-oxo-benzimidazol-4-yl] propoxy]propyl-methylcarbamoyl]cyclohexyl]pyrazol-4-yl]-2-[2-(2,2,2-trifluoroethylamino)-4-pyridyl]oxazole-4-carboxamide (I-28)

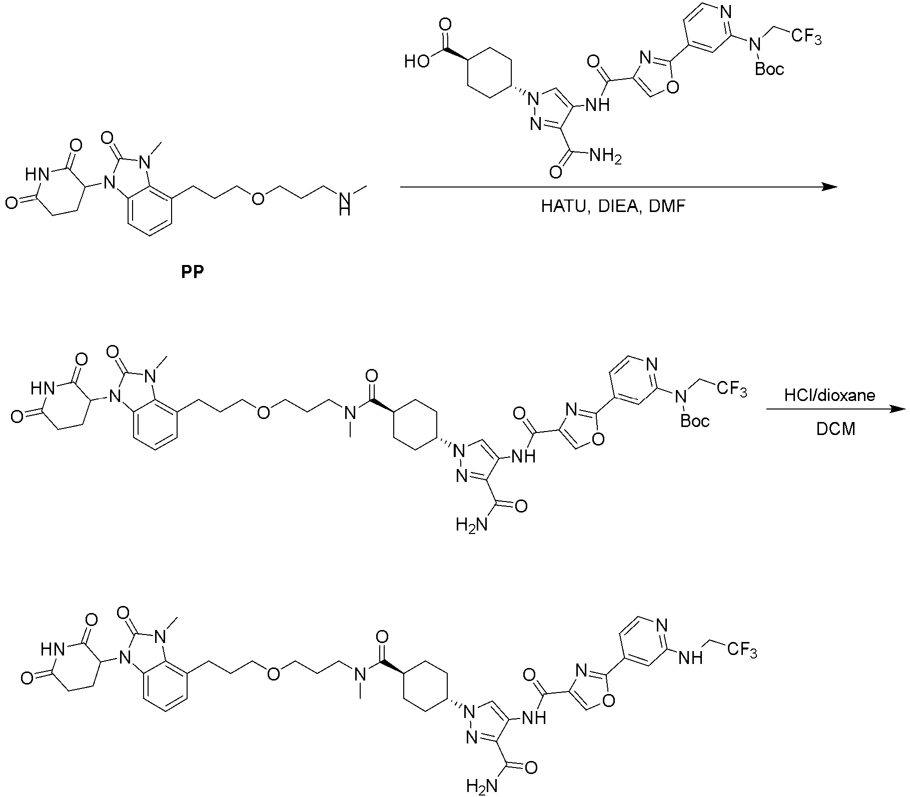

### Step 1 - Tert-butyl N-[4-[4-[[3-carbamoyl-1-[4-[3-[3-[1-(2,6-dioxo-3-piperidyl)-3-methyl-2-oxo -benzimidazol-4-yl]propoxy]propylmethyl-carbamoyl]cyclohexyl]pyrazol-4-yl]carbamoyl]oxazol-2-yl]-2-pyridyl]-N-(2,2,2-trifluoroethyl)carbamate

To a solution of 3-[3-methyl-4-[3-[3-(methylamino)propoxy]propyl]-2-oxo-benzimidazol-1-yl] piperidine-2,6-dione (40.0 mg, 94.1 umol, HCl, Intermediate PP), and 4-[4-[[2-[2-[tert-butoxycarbonyl (2,2,2-trifluoroethyl)amino]-4-pyridyl]oxazole-4-carbonyl]amino]-3-carbamoyl-pyrazol-1-yl]cyclohexanecarboxylic acid (58.5 mg, 94.1 umol, synthesized via Steps 1-2 of Intermediate RF) in DMF (3.00 mL) was added DIPEA (36.5 mg, 282 umol) and HATU (42.9 mg, 112 umol). The mixture was stirred at 25 °C for 0.5 hour. On completion, the mixture was diluted with H₂O (15 mL), filtered and the filter cake was collected and dried *in vacuo* to give the title compound (80.0 mg, 85% yield) as white solid. LC-MS (ESI⁺) *m*/*z* 992.4 (M+H)⁺.

### Step 2 - N-[3-carbamoyl-1-[4-[3-[3-[1-(2,6-dioxo-3-piperidyl)-3-methyl-2-oxo-benzimidazol-4-yl] propoxy]propyl-methylcarbamoyl]cyclohexyl]pyrazol-4-yl]-2-[2-(2,2,2-trifluoroethylamino)-4-pyridyl]oxazole-4-carboxamide

To a solution of tert-butyl N-[4-[4-[[3-carbamoyl-1-[4-[3-[3-[1-(2,6-dioxo-3-piperidyl)-3-methyl-2- oxo-benzimidazol-4-yl]propoxy]propyl-methylcarbamoyl]cyclohexyl]pyrazol-4-yl]carbamoyl]oxazol-2-yl]-2-pyridyl]-N-(2,2,2-trifluoroethyl)carbamate (80.0 mg, 80.6 umol) in DCM (3.00 mL) was added HCl in dioxane (4.00 M, 3.00 mL). The mixture was stirred at 25 °C for 0.5 hour. On completion, the mixture was concentrated *in vacuo*. The residue was purified by prep-HPLC (column: Phenomenex Synergi C18 150*25*10 um; mobile phase: [water (0.225% FA)-ACN]; B%: 31%-61%, 10 min) to give the title compound (19.2 mg, 25% yield) as white solid. ¹H NMR (400MHz, DMSO-*d₆*) δ 11.09 (s, 1H), 10.95 (s, 1H), 8.97 (s, 1H), 8.38 (s, 1H), 8.25 (d, *J* = 5.2 Hz, 1H), 7.73 - 7.58 (m, 2H), 7.50 (s, 1H), 7.26 (s, 1H), 7.17 (d, *J* = 5.2 Hz, 1H), 7.01 - 6.92 (m, 2H), 6.92 - 6.81 (m, 1H), 5.42 - 5.28 (m, 1H), 4.34 - 4.16 (m, 3H), 3.58 (s, 3H), 3.46 - 3.99 (m, 4H), 3.36 (s, 3H), 3.05 (s, 2H), 3.00 - 2.93 (m, 2H), 2.93 - 2.84 (m, 1H), 2.82 - 2.71 (m, 2H), 2.61 - 2.57 (m, 1H), 2.48 - 2.39 (m, 1H), 2.15 - 2.10 (m, 1H), 2.10 - 2.02 (m, 1H), 2.00 - 1.86 (m, 4H), 1.85 - 1.81 (m, 2H), 1.80 - 1.67 (m, 2H), 1.65 - 1.50 (m, 2H), LC-MS (ESI⁺) *m*/*z* 892.4 (M+H)⁺.

**Table 6: Compounds synthesized via Method 1 with the coupling of various amines and acids in Step 1.**

| **I-#** | **Step 1 Intermediate Amine** | **Step 1 Intermediate Acid** | **LCMS (ES+) m/z (M+H)⁺** | **¹HNMR (400MHz, DMSO-d₆) δ** |
|---|---|---|---|---|
| **I-1** | HQ | PU | 887.9 | 11.08 (s, 1H), 9.71 (s, 1H), 8.91 (s, 1H), 8.17 (s, 1H), 8.14 (d, *J* = 5.2 Hz, 1H), 7.87 (s, 1H), 7.30 - 7.01 (m, 4H), 6.96 (d, *J =* 4.0 Hz, 2H), 6.91 - 6.84 (m, 1H), 5.35 (dd, *J* = 4.8, 12.8 Hz, 1H), 4.24 - 4.18 (m, 1H), 3.56 (s, 3H), 3.53 (m, 8H), 3.21 - 3.19 (m, 2H), 3.19 - 3.15 (m, 2H), 2.97 - 2.93 (m, 2H), 2.87 - 2.85 (m, 1H), 2.75 - 2.68 (m, 1H), 2.65 - 2.61 (m, 1H), 2.20 - 2.18 (m, 1H), 2.06 - 1.95 (m, 3H), 1.84 - 1.82 (m, 4H), 1.77 - 1.66 (m, 2H), 1.55 - 1.52 (m, 2H), 1.06 - 1.04 (m, 1H), 0.45 - 0.43 (m, 2H), 0.22 - 0.21 (m, 2H) |
| **I-5^{b}** | QR | QT | 830.5 | 11.20 (s, 1H), 9.70 (s, 1H), 8.92 (s, 1H), 8.19 (s, 1H), 8.15 (d, *J* = 5.2 Hz, 1H), 7.92 - 7.82 (m, 1H), 7.15 (d, *J* = 7.2 Hz, 1H), 7.13 - 7.08 (m, 3H), 7.02 (t, *J =* 5.6 Hz, 2H), 5.39 - 5.32 (m, 1H), 4.29 - 4.17 (m, 1H), 4.11 - 3.97 (m, 1H), 3.46 - 3.39 (m, 6H), 3.25 - 3.21 (m, 4H), 2.83 - 2.72 (m, 2H), 2.09 - 2.04 (m, 2H), 1.99 (s, 2H), 1.89 - 1.82 (m, 4H), 1.78 - 1.71 (m, 2H), 1.59 - 1.52 (m, 2H), 1.18 (t, *J* = 7.2 Hz, 1H), 1.09 - 1.03 (m, 1H), 0.48 - 0.43 (m, 2H), 0.24 - 0.20 (m, 2H) |
| **I-15** | HQ | PU | 887.5 | 11.07 (s, 1H), 9.68 (s, 1H), 8.90 (s, 1H), 8.17 - 8.13 (m, 2H), 7.79 (t, *J* = 5.6 Hz, 1H), 7.31 - 7.02 (m, 3H), 7.02 - 6.99 (m, 1H), 6.95 (d, *J* = 5.6 Hz, 2H), 6.89 - 6.83 (m, 1H), 5.35 (dd, *J =* 5.2, 12.8 Hz, 1H), 4.29 - 4.27 (m, 1H), 3.55 (s, 3H), 3.53 - 3.51 (m, 4H), 3.46 - 3.42 (m, 4H), 3.25 - 3.20 (m, 2H), 3.18 (t, *J =* 6.0 Hz, 2H), 2.97 - 2.91 (m, 2H), 2.91 - 2.81 (m, 1H), 2.75 - 2.67 (m, 1H), 2.64 - 2.57 (m, 1H), 2.42 - 2.38 (m, 1H), 2.18 - 2.14 (m, 2H), 2.03 - 1.94 (m, 1H), 1.89 - 1.77 (m, 6H), 1.64 - 1.53 (m, 2H), 1.12 - 1.00 (m, 1H), 0.50 - 0.41 (m, 2H), 0.27 - 0.16 (m, 2H) |
| **I-17** | SZ | QT | 912.2 | 11.07 (s, 1H), 9.71 (s, 1H), 8.92 (m, 1H), 8.19 (s, 1H), 8.15 (d, *J =* 5.2 Hz, 1H), 7.30 - 7.15 (m, 1H), 7.10 (s, 1H), 7.09 - 7.05 (m, 1H), 7.04 (s, 1H), 7.03 - 6.98 (m, 2H), 6.91 - 6.84 (m, 1H), 5.32 (dd, *J* = 5.2, 12.8 Hz, 1H), 4.31 - 4.24 (m, 1H), 3.83 - 3.75 (m, 2H), 3.32 (s, 3H), 3.18 (t, *J* = 6.0 Hz, 2H), 3.08 (s, 3H), 2.94 - 2.89 (m, 1H), 2.86 - 2.82 (m, 2H), 2.76 - 2.71 (m, 2H), 2.64 - 2.57 (m, 6H), 2.27 (t, *J =* 7.2 Hz, 1H), 2.07 - 1.67 (m, 12H), 1.61 - 1.48 (m, 2H), 1.11 - 1.00 (m, 1H), 0.49 - 0.41 (m, 2H), 0.26 - 0.18 (m, 2H) |
| **I-18^{b}** | RR | QT | 912.5 | 11.07 (s, 1H), 9.71 (s, 1H), 8.92 (s, 1H), 8.20 (s, 1H), 8.15 (d, *J* = 4.4 Hz, 1H), 7.32 - 6.97 (m, 6H), 6.87 (d, *J* = 7.6 Hz, 1H), 5.38 - 5.27 (m, 1H), 4.33 - 4.22 (m, 1H), 3.83 - 3.75 (m, 1H), 3.65 - 3.56 (m, Hz, 2H), 3.54 - 3.49 (m, 2H), 3.47 - 3.42 (m, 2H), 3.32 (s, 3H), 3.18 (t, *J =* 5.2 Hz, 2H), 3.08 (s, 3H), 2.95 - 2.87 (m, 1H), 2.78 - 2.73 (m, 1H), 2.69 - 2.60 (m, 5H), 2.34 - 2.25 (m, 2H), 2.08 - 1.95 (m, 4H), 1.84 - 1.86 (m, 1H), 1.85 - 1.82 (m, 1H), 1.80 - 1.70 (m, 4H), 1.62 - 1.47 (m, 2H), 1.12 - 1.01 (m, 1H), 0.50 - 0.40 (m, 2H), 0.28 - 0.17 (m, 2H) |
| **I-19^{b}** | OP | RX | 874.6 | 11.08 (s, 1H), 7.95 (s, 1H), 7.77 (d, *J =* 2.4 Hz, 1H), 7.38 - 7.24 (m, 1H), 7.02 - 6.92 (m, 2H), 6.88 (d, *J* = 2.4 Hz, 1H), 6.69 (d, *J =* 2.4 Hz, 1H), 5.36 (d, *J* = 5.2 Hz, 1H), 4.43 (d, *J =* 12.0 Hz, 1H), 4.20 (d, *J =* 9.6 Hz, 2H), 4.13 - 3.85 (m, 5H), 3.61 - 3.55 (m, 21H), 3.26 (s, 3H), 3.01 - 2.83 (m, 4H), 2.76 - 2.58 (m, 3H), 2.24 (s, 2H), 2.16 - 2.05 (m, 2H), 2.05 - 1.95 (m, 1H), 1.89 - 1.57 (m, 10H) |
| **I-20^{b}** | OP | HO | 874.4 | 11.08 (s, 1H), 7.77 (s, 1H), 7.53 (d, *J =* 2.4 Hz, 1H), 7.03 (s, 1H), 6.99 (d, *J* = 7.6 Hz, 1H), 6.86 (d, *J =* 8.0 Hz, 1H), 6.53 (d, *J* = 2.0 Hz, 1H), 6.19 (d, *J* = 8.0 Hz, 1H), 5.32 (dd, *J =* 5.2, 12.8 Hz, 1H), 4.11 (s, 2H), 4.08 - 3.99 (m, 1H), 3.94 - 3.83 (m, 2H), 3.57 - 3.49 (m, 16H), 3.41 - 3.37 (m, 6H), 3.36 - 3.35 (m, 1H), 3.31 (s, 3H), 2.97 - 2.82 (m, 1H), 2.70 - 2.62 (m, 3H), 2.60 - 2.52 (m, 1H), 2.46 - 2.42 (m, 2H), 2.38 - 2.27 (m, 1H), 2.04 - 1.93 (m, 3H), 1.92 - 1.69 (m, 6H), 1.69 - 1.57 (m, 2H), 1.50 (q, *J* = 12.0 Hz, 2H), 1.33 (q, *J =* 11.6 Hz, 2H) |
| **I-21^{b}** | SA | ZV | 756.6 | 11.30 (s, 1H), 11.07 (s, 1H), 8.14 (s, 2H), 8.06 (s, 1H), 7.05 - 6.98 (m, 2H), 6.89 - 6.82 (m, 2H), 5.52 (d, *J* = 8.4 Hz, 1H), 5.37 - 5.27 (m, 1H), 4.02 (m, 1H), 3.91 - 3.89 (m, 2H), 3.62 - 3.38 (m, 18H), 2.93 - 2.80 (m, 1H), 2.65 - 2.55 (m, 4H), 2.00 (m, 4H), 1.88 - 1.74 (m, 7H), 1.55 (m, 2H), 1.45 - 1.32 (m, 4H) |
| **I-22^{b}** | SA | ZW | 754.5 | 11.31 (s, 1H), 11.09 (s, 1H), 8.15 (s, 1H), 8.07 (s, 1H), 7.06 - 6.98 (m, 2H), 6.86 (d, *J* = 12.6 Hz, 2H), 5.54 (d, *J* = 7.6 Hz, 1H), 5.37 - 5.29 (m, 1H), 4.03 (s, 1H), 3.91 (d, *J* = 9.2 Hz, 6H), 3.56 (d, *J* = 11.2 Hz, 1H), 3.33 (s, 3H), 2.96 - 2.85 (m, 1H), 2.69 (d, *J* = 11.6 Hz, 4H), 2.62 (d, *J* = 9.2 Hz, 4H), 2.35 (d, *J* = 11.8 Hz, 2H), 2.28 (t, *J* = 6.8 Hz, 2H), 2.02 (s, 4H), 1.84 (d, *J* = 11.2 Hz, 4H), 1.64 - 1.53 (m, 4H), 1.52 - 1.27 (m, 10H) |
| **I-23^{b}** | SA | SB | 827.4 | 11.31 (s, 1H), 11.08 (m, 1H), 8.6 (m, 1H), 7.32 - 7.22 (m, 1H), 7.04 - 6.95 (m, 2H), 6.89 - 6.81 (m, 2H), 5.57 - 5.50 (m, 1H), 5.37 - 5.28 (m, 1H), 4.06 - 3.98 (m, 2H), 3.94 - 3.86 (m, 4H), 3.70 (m, 14H), 3.32 (s, 3 H), 2.88 - 2.81 (m, 2H), 2.69 - 2.68 (m, 3H), 2.65 - 2.59 (m, 4H), 2.07 - 1.96 (m, 3H), 1.89 - 1.78 (m, 4H), 1.66 - 1.40 (m, 13H) |
| **I-24^{b}** | SC | ZV | 686.4 | 11.07 (s, 1H), 8.18 (s, 2H), 8.23 - 8.16 (m, 1H), 8.03 (s, 1H), 7.03 (s, 1H), 7.00 (d, *J* = 8.4 Hz, 1H), 6.87 (d, *J* = 7.6 Hz, 1H), 6.79 (s, 1H), 5.65 (d, *J =* 8.0 Hz, 1H) 5.32 (m, 1 H), 4.02 (s, 2H), 3.59 (t, *J =* 6.4 Hz, 2H), 3.44 (m, 6H), 2.74 - 2.69 (m, 2H), 2.69 - 2.63 (m, 4H), 2.59 - 2.54 (m, 7H), 2.35 (s, 3H), 1.99 (m, 4H), 1.81 - 1.79 (m, 4H), 1.45 - 1.30 (m, 4H) |
| **I-25^{b}** | SC | RX | 804.5 | 11.16 (s, 1H), 11.09 (s, 1H), 8.04 (s, 1H), 6.96 (d, *J* = 4.2 Hz, 2H), 6.88 (d, *J =* 4.0 Hz, 1H), 6.80 (s, 1H), 5.67 (d, *J* = 8.0 Hz, 1H), 5.36 (d, *J =* 5.2, 12.4 Hz, 1H), 4.13 (s, 2H), 4.08 - 3.94 (m, 1H), 3.60 - 3.50 (m, 24H), 3.01 - 2.83 (m, 4H), 2.77 - 2.57 (m, 3H), 2.36 (s, 3H), 2.09 - 1.94 (m, 3H), 1.83 (d, *J =* 8.0 Hz, 4H), 1.50 - 1.29 (m, 4H) |
| **I-26^{b}** | SC | ZX | 804.4 | 11.15 (s, 1H), 11.09 (s, 1H), 8.03 (s, 1H), 7.03 (s, 1H), 7.00 (d, *J =* 8.0 Hz, 1H), 6.87 (d, *J =* 8.0 Hz, 1H), 6.80 (s, 1H), 5.67 (d, *J =* 8.0 Hz, 1H), 5.32 (dd, *J* = 5.2, 12.8 Hz, 1H), 4.12 (s, 2H), 4.06 - 3.98 (m, 1H), 3.56 - 3.53 (m, 12H), 3.44 - 3.35 (m, 12H), 3.32 (s, 3H), 2.93 - 2.84 (m, 1H), 2.67 - 2.63 (m, 2H), 2.35 (s, 3H), 2.05 - 1.97 (m, 3H), 1.86 - 1.77 (m, 4H), 1.47 - 1.29 (m, 4H) |
| **I-27** | SI | QT | 920.2 | 11.11 (s, 1H), 9.71 (s, 1H), 8.92 (s, 1H), 8.19 (d, *J* = 3.2 Hz, 1H), 8.15 (d, *J* = 5.2 Hz, 1H), 7.33 - 7.13 (m, 2H), 7.12 - 7.03 (m, 4H), 7.01 (d, *J* = 5.2 Hz, 1H), 5.37 (dd, *J* = 5.6, 12.8 Hz, 1H), 4.31 - 4.23 (m, 1H), 3.33 (s, 3H), 3.18 (d, *J* = 6.4 Hz, 2H), 3.04 (s, 2H), 3.01 - 2.82 (m, 4H), 2.81 - 2.69 (m, 3H), 2.65 - 2.54 (m, 5H), 2.08 - 1.71 (m, 10H), 1.61 - 1.45 (m, 5H), 1.35 - 1.20 (m, 1H), 1.16 - 1.02 (m, 2H), 0.48 - 0.42 (m, 2H), 0.23 - 0.20 (m, 2H) |
| **I-29** | PP | 4-[4-[[2-[2-[tert-butoxycarbo nyl (2,2,2-trifluoroethy l)amino]-4-pyridyl]oxaz ole-4-carbonyl]am ino]-3-carbamoyl-pyrazol-1-yl]cyclohexa necarboxylic acid (synthesized via Steps 1-2 of Intermediate RF) | 892.4 | 11.09 (s, 1H), 10.95 (s, 1H), 8.97 (s, 1H), 8.38 (s, 1H), 8.25 (d, *J* = 5.2 Hz, 1H), 7.73 - 7.58 (m, 2H), 7.50 (s, 1H), 7.26 (s, 1H), 7.17 (d, *J* = 5.2 Hz, 1H), 7.01 - 6.92 (m, 2H), 6.92 - 6.81 (m, 1H), 5.42 - 5.28 (m, 1H), 4.34 - 4.16 (m, 3H), 3.58 (s, 3H), 3.46 - 3.99 (m, 4H), 3.36 (s, 3H), 3.05 (s, 2H), 3.00 - 2.93 (m, 2H), 2.93 - 2.84 (m, 1H), 2.82 - 2.71 (m, 2H), 2.61 - 2.57 (m, 1H), 2.48 - 2.39 (m, 1H), 2.15 - 2.10 (m, 1H), 2.10 - 2.02 (m, 1H), 2.00 - 1.86 (m, 4H), 1.85 - 1.81 (m, 2H), 1.80 - 1.67 (m, 2H), 1.65 - 1.50 (m, 2H) |
| **I-31** | PP | TS | 861.6 | 11.09 (s, 1H), 9.37 (d, *J =* 9.6 Hz, 1H), 8.88 (d, *J =* 1.2 Hz, 1H), 8.15 (d, *J =* 5.2 Hz, 1H), 8.01 (d, *J =* 11.6 Hz, 1H), 7.13 - 7.04 (m, 2H), 7.01 (d, *J =* 5.6 Hz, 1H), 6.96 (d, *J* = 4.8, 10.0 Hz, 2H), 6.90 - 6.84 (m, 1H), 5.42 - 5.30 (m, 2H), 5.21 (d, *J* = 8.0 Hz, 1H), 4.23 - 4.11 (m, 1H), 3.57 (d, *J* = 6.4 Hz, 3H), 3.48 - 3.39 (m, 8H), 3.18 (d, *J* = 6.0 Hz, 2H), 3.04 (s, 2H), 3.02 - 2.94 (m, 2H), 2.80 (s, 1H), 2.71 - 2.65 (m, 2H), 2.12 - 1.97 (m, 6H), 1.93 - 1.74 (m, 7H), 1.74 - 1.66 (m, 1H), 1.65 - 1.47 (m, 2H), 1.17 - 0.98 (m, 1H), 0.52 - 0.41 (m, 2H), 0.29 - 0.15 (m, 2H) |
| **I-32^{b}** | SP | QT | 923.5 | 11.08 (s, 1H), 9.70 (d, *J* = 6.4 Hz, 1H), 8.94 (d, *J* = 2.0 Hz, 1H), 8.13 (d, *J* = 5.6 Hz, 1H), 7.17 (d, *J* = 5.2 Hz, 1H), 7.15 - 6.99 (m, 2H), 6.98 - 6.92 (m, 2H), 6.90 - 6.82 (m, 1H), 5.41 - 5.30 (m, 1H), 4.26 (d, *J* = 3.6 Hz, 1H), 3.56 (s, 3H), 3.54 (s, 3H), 3.52 - 3.48 (m, 4H), 3.46 - 3.43 (m, 3H), 3.23 - 3.15 (m, 3H), 3.08 (s, 2H), 2.98 - 2.90 (m, 2H), 2.83 (s, 2H), 2.71 - 2.58 (m, 3H), 2.05 - 1.94 (m, 3H), 1.90 - 1.74 (m, 7H), 1.54 (t, *J* = 13.6 Hz, 2H), 1.13 - 1.02 (m, 1H), 0.52 - 0.42 (m, 2H), 0.23 (m, 2H). |
| **I-45^{b}** | SX | QT | 967.6 | 11.09 (s, 1H), 9.71 (s, 1H), 8.92 (d, *J* = 2.4 Hz, 1H), 8.20 - 8.18 (m, 1H), 8.15 (d, *J* = 5.2 Hz, 1H), 7.33 - 6.91 (m, 7H), 5.43 - 5.29 (m, 1H), 5.11 - 4.68 (m, 1H), 4.36 - 4.21 (m, 1H), 4.07 - 3.97 (m, 1H), 3.43 - 3.38 (m, 4H), 3.34 (s, 3H), 3.32 (s, 3H), 3.18 (t, *J* = 6.0 Hz, 2H), 3.04 - 2.95 (m, 2H), 2.94 - 2.86 (m, 1H), 2.84 - 2.74 (m, 2H), 2.70 - 2.58 (m, 2H), 2.46 - 2.37 (m, 6H), 2.35 - 2.32 (m, 1H), 2.32 - 2.21 (m, 3H), 2.11 - 1.98 (m, 4H), 1.95 - 1.84 (m, 2H), 1.82 - 1.70 (m, 2H), 1.62 - 1.48 (m, 2H), 1.13 - 1.01 (m, 1H), 0.50 - 0.40 (m, 2H), 0.26 - 0.19 (m, 2H) |
| **I-49^{b}** | UP | UN | 1033.2 | 8.97 (s, 1H), 8.58 (t, *J =* 6.0 Hz, 1H), 8.45 (s, 1H), 8.35 - 8.27 (m, 1H), 8.32 (d, *J* = 8.4 Hz, 1H), 7.95 (d, *J* = 7.6 Hz, 1H), 7.63 (s, 1H), 7.48 - 7.42 (m, 2H), 7.41 - 7.38 (m, 4H), 5.15 (d, *J* = 3.2 Hz, 1H), 4.56 (d, *J* = 9.6 Hz, 1H), 4.48 - 4.32 (m, 4H), 4.29 - 4.19 (m, 4H), 4.16 - 4.06 (m, 4H), 3.97 (s, 2 H), 3.70 - 3.49 (m, 15H), 2.44 (s, 3H), 2.33 (d, *J* = 1.6 Hz, 1H), 2.10 - 1.97 (m, 4H), 1.94 - 1.81 (m, 3 H), 1.50 - 1.29 (m, 5H), 0.94 (s, 9H) |
| **I-50^{b}** | UP | UR | 967.6 | 8.97 (s, 1H), 8.59 (t, *J* = 6.0 Hz, 1H), 8.32 (d, *J* = 8.4 Hz, 1H), 8.18 (s, 1H), 7.95 (d, *J* = 7.6 Hz, 1H), 7.62 (s, 1H), 7.52 - 7.35 (m, 6H), 4.57 (d, *J =* 9.6 Hz, 1H), 4.48 - 4.33 (m, 3H), 4.30 - 4.24 (m, 1H), 4.21 (s, 2H), 4.15 - 4.07 (m, 3H), 4.02 - 3.91 (m, 2H), 3.68 - 3.55 (m, 15H), 2.49 - 2.45 (m, 4H), 2.44 (s, 3H), 2.37 - 2.29 (m, 1H), 2.09 - 1.80 (m, 6H), 1.50 - 1.29 (m, 4H), 0.94 (s, 9H) |
| **I-51^{b}** | UP | ZY | 796.2 | 11.11 (s, 1H), 8.45 (s, 1H), 8.32 (d, *J* = 8.8 Hz, 1H), 7.97 (br d, *J* = 7.6 Hz, 1H), 7.63 (s, 1H), 7.59 (t, *J* = 7.6 Hz, 1H), 7.45 (d, *J* = 8.8 Hz, 1H), 7.15 (d, *J* = 8.8 Hz, 1H), 7.04 (d, *J =* 7.2 Hz, 1H), 6.62 (s, 1H), 5.06 (dd, *J* = 5.2, 12.8 Hz, 1H), 4.23 (s, 2H), 4.16 - 4.10 (m, 1H), 4.12 (s, 2H), 3.66 - 3.60 (m, 16H), 2.96 - 2.82 (m, 1H), 2.62 - 2.60 (m, 1H), 2.59 - 2.55 (m, 1H), 2.49 - 2.43 (m, 4H), 2.39 - 2.29 (m, 1H), 2.02 - 2.00 (m, 3H), 1.84 - 1.82 (m, 2H), 1.53 - 1.28 (m, 4H) |
| **I-52^{b}** | UP | ZZ | 840.5 | 11.08 (s, 1H), 8.45 (s, 1H), 8.32 (d, *J* = 8.8 Hz, 1H), 8.23 (s, 1H), 7.94 (d, *J* = 7.2 Hz, 1H), 7.62 (s, 1H), 7.57 (t, *J* = 7.6 Hz, 1H), 7.44 (d, *J* = 8.4 Hz, 1H), 7.14 (d, *J* = 8.8 Hz, 1H), 7.03 (d, *J =* 7.2 Hz, 1H), 6.60 (t, *J =* 6.0 Hz, 1H), 5.04 (s, 1H), 4.22 (s, 2H), 4.12 (s, 3H), 3.63 (t, *J* = 5.2 Hz, 4H), 3.57 - 3.49 (m, 17H), 2.94 - 2.80 (m, 1H), 2.63 - 2.54 (s, 2H), 2.49 - 2.42 (m, 4H), 2.08 - 1.95 (m, 3H), 1.88 - 1.78 (m, 2H), 1.51 - 1.28 (m, 4H) |
| **I-53^{b}** | SA | RX | 874.5 | 11.08 (s, 1H), 9.07 (s, 1H), 8.10 (s, 1H), 7.07 - 6.80 (m, 4H), 5.36 (d, *J* = 5.2, 12.4 Hz, 1H), 4.18 (s, 2H), 3.90 ( d, *J* = 10.4 Hz, 2H), 3.66 - 3.50 (m, 22H), 3.48 - 3.40 (m, 6H), 3.17 - 3.06 (m, 4H), 3.00 - 2.83 (m, 5H), 2.75 - 2.56 (m, 3H), 2.25 - 1.94 (m, 4H), 1.84 (d, *J =* 8.0 Hz, 4H), 1.60 - 1.46 (m, 4H) |
| **I-106^{c}** | ZB | QT | 927.3 | 11.10 (s, 1H), 9.73 (s, 1H), 8.92 (d, J = 1.0 Hz, 1H), 8.29 (s, 1H), 8.19 (s, 1H), 8.15 (d, J = 5.2 Hz, 1H), 7.33 - 7.01 (m, 7H), 5.40 - 5.31 (m, 1H), 4.32 - 4.23 (m, 1H), 3.82 (d, J = 3.2 Hz, 2H), 3.77 (d, J = 10.4 Hz, 1H), 3.34 (s, 3H), 3.33 (s, 3H), 3.27 - 3.21 (m, 2H), 3.18 (t, J = 6.0 Hz, 2H), 3.08 (s, 2H), 2.95 - 2.86 (m, 1H), 2.82 (s, 1H), 2.79 - 2.71 (m, 2H), 2.67 - 2.57 (m, 4H), 2.49 - 2.45 (m, 1H), 2.43 (d, J = 6.0 Hz, 1H), 2.10 - 1.97 (m, 4H), 1.92 - 1.72 (m, 5H), 1.65 - 1.47 (m, 2H), 1.39 - 1.37 (m, 1H), 1.11 - 1.02 (m, 1H), 0.50 - 0.41 (m, 2H), 0.26 - 0.18 (m, 2H) |
| **I-131** | AEV | ACO | 1008.4 | (D₂O) δ 8.34 (s, 1H), 7.94 (s, 1H), 7.81 (s, 1H), 7.77 - 7.70 (m, 4H), 7.60 - 7.49 (m, 6H), 7.46 (d, *J* = 8.4 Hz, 2H), 7.30 (d, *J* = 8.4 Hz, 2H), 6.95 - 6.89 (m, 2H), 6.84 - 6.67 (m, 1H), 4.18 (d, *J* = 7.2 Hz, 2H), 4.11 (d, *J* = 2.4 Hz, 2H), 3.94 (m, 2H), 3.87 - 3.80 (m, 1H), 3.65 (m 4H), 3.51 - 3.31 (m, 4H), 3.26 - 3.19 (m, 1H), 3.17 - 3.10 (m, 1H), 2.96 (d, *J* = 7.2 Hz, 2H), 2.85 (s, 3H), 2.12 - 2.03 (m, 2H), 1.92 (m, 2H), 1.81 (m, 2H), 1.62 - 1.52 (m, 2H), 1.27 - 1.15 (m, 3H), 1.13 - 1.01 (m, 3H), 0.59 (d, *J* = 7.4 Hz, 2H), 0.50 (d, *J* = 8.0 Hz, 2H), 0.33 - 0.32 (m, 2H), 0.26- 0.25 (m, 2H) |
| **I-132** | AEW | ACO | 1022.6 | 9.82 (d, *J* = 5.6 Hz, 1H), 9.68 (s, 2H), 9.04 (d, *J* = 0.8 Hz, 1H), 8.55 (d, *J* = 5.2 Hz, 1H), 8.19 - 8.05 (m, 4H), 7.90 (t, *J =* 8.0 Hz, 2H), 7.82 - 7.76 (m, 3H), 7.74 - 7.70 (m, 1H), 7.66 - 7.61 (m, 4H), 7.31 - 7.05 (m, 2H), 4.33 (d, *J* = 7.2 Hz, 2H), 4.26 - 4.19 (m, 4H), 3.46 - 3.44 (m, 3H), 3.26 (d, *J =* 6.0 Hz, 3H), 3.03 (s, 2H), 2.79 (s, 1H), 2.07 - 2.01 (m, 2H), 1.91 - 1.73 (m, 8H), 1.70 - 1.64 (m, 1H), 1.63 - 1.51 (m, 2H), 1.36 - 1.26 (m, 2H), 1.23 (s, 4H), 1.14 - 1.06 (m, 1H), 0.88 - 0.79 (m, 1H), 0.54 - 0.46 (m, 4H), 0.43 - 0.38 (m, 2H), 0.29 (s, 2H) |
| **I-133** | AEY | ACO | 1023.5 | 9.85 - 9.81 (m, 1H), 9.05 (s, 1H), 8.19 - 8.05 (m, 4H), 7.82 - 7.76 (m, 3H), 7.75 - 7.69 (m, 1H), 7.70 - 7.61 (m, 4H), 7.50 - 7.40(m, 3H), 7.33 - 7.03 (m, 2H), 4.33 (d, *J* = 6.8 Hz, 2H), 4.23 (s, 4H), 3.47 (s, 3H), 3.30 - 3.25 (m, 5H), 2.97 - 2.87 (m, 4H), 2.80 - 2.69 (m, 3H), 2.52 (s, 3H), 2.09 - 2.02 (m, 3H), 1.96 - 1.73 (m, 9H), 1.30 - 1.09 (m, 6H), 0.55 - 0.46 (m, 4H), 0.42 - 0.38 (m, 2H), 0.34 - 0.25 (m, 2H) |
| **I-134** | WY | ACO | 1004.6 | 9.68 (s, 1H), 8.91 (s, 1H), 8.18 (s, 1H), 8.16 - 8.13 (m, 2H), 8.09 (s, 1H), 8.00 (s, 1H), 7.81 - 7.75 (m, 1H), 7.72 - 7.62 (m, 3H), 7.52 - 7.40 (m, 4H), 7.34 (d, *J =* 8.0 Hz, 2H), 7.30 - 6.99 (m, 4H), 4.33 (d, *J* = 6.8 Hz, 2H), 4.25 - 4.17 (m, 1H), 3.83 (s, 4H), 3.66 - 3.48 (m, 2H), 3.19 - 3.16 (m, 2H), 2.43 - 2.38 (m, 2H), 2.25 - 2.21 (m, 2H), 2.20 (s, 3H), 2.08 - 2.00 (m, 3H), 1.95 - 1.86 (m, 2H), 1.83 - 1.66 (m, 4H), 1.64 - 1.51 (m, 3H), 1.41 - 1.39 (m, 2H), 1.36 - 1.17 (m, 2H), 1.15 - 1.00 (m, 5H), 0.52 - 0.43 (m, 4H), 0.42 - 0.38 (m, 2H), 0.25 - 0.19 (m, 2H) |
| **I-135** | ACN | ACO | 1006.7 | 9.81 (s, 1H), 9.78 - 9.64 (m, 2H), 9.01 (s, 1H), 8.56 (t, *J* = 6.0 Hz, 1H), 8.19 (s, 1H), 8.11 (s, 2H), 8.06 (s, 1H), 7.90 (d, *J* = 8.4 Hz, 2H), 7.83 - 7.76 (m, 3H), 7.74 - 7.70 (m, 1H), 7.68- 7.62 (m, 4H), 7.47 - 7.00 (m, 3H), 4.34 (d, *J* = 7.2 Hz, 2H), 4.28 - 4.16 (m, 5H), 3.30 - 3.23 (m, 5H), 3.12 - 3.04 (m, 1H), 3.03 - 2.95 (m, 2H), 2.93 - 2.86 (m, 1H), 2.74 (m, 3H), 2.08 - 2.02 (m, 2H), 1.96 - 1.85 (m, 2H), 1.84 - 1.76 (m, 1H), 1.75 - 1.63 (m, 2H), 1.61 - 1.51 (m, 2H), 1.37 - 1.32 (m, 4H), 1.31 - 1.24(m, 2H), 1.23 (s, 3H), 1.20 - 1.16 (m, 1H), 1.16 - 1.07 (m, 2H), 0.88 - 0.82 (m, 1H), 0.54 - 0.46 (m, 4H), 0.44 - 0.37 (m, 2H), 0.28 (m, 2H) |
| **I-136** | TN | ACQ | 871.6 | 11.08 (s, 1H), 9.40 (s, 1H), 8.77 (d, *J* = 8.0 Hz, 1H), 8.39 (s, 1H), 8.26 (s, 1H), 7.27 - 6.92 (m, 3H), 6.91 - 6.83 (m, 2H), 5.36 (dd, *J* = 5.2, 12.8 Hz, 1H), 4.28 - 4.19 (m, 1H), 3.90 - 3.73 (m, 7H), 3.57 (s, 3H), 3.53 - 3.46 (m, 3H), 3.31 - 3.23 (m, 1H), 3.10 (t, *J* = 9.6 Hz, 1H), 3.02 - 2.95 (m, 2H), 2.94 - 2.80 (m, 5H), 2.79 - 2.62 (m, 3H), 2.07 - 1.93 (m, 3H), 1.93 - 1.71 (m, 8H), 1.65 - 1.50 (m, 2H), 1.49 - 1.27 (m, 2H) |
| **I-137^{b}** | ACS | AFB | 695.4 | 11.09 (s, 1H), 9.07 (d, *J* = 2.0 Hz, 1H), 9.03 (d, *J* = 2.0 Hz, 1H), 8.88 (t, *J* = 5.5 Hz, 1H), 8.67 (s, 1H), 8.64 (s, 1H), 8.56 (s, 1H), 7.71 (s, 1H), 7.00 - 6.93 (m, 2H), 6.91 - 6.85 (m, 1H), 5.36 (d, *J =* 5.2 Hz, 1H), 4.92 - 4.73 (m, 1H), 3.71 - 3.61 (m, 2H), 3.57 (s, 3H), 3.57 - 3.50 (m, 4H), 3.02 - 2.94 (m, 2H), 2.70 - 2.63 (m, 5H), 2.04 - 1.95 (m, 1H), 1.91 - 1.82 (m, 2H), 0.90 - 0.83 (m, 2H), 0.61 - 0.53 (m, 2H) |
| **I-138^{b}** | ACS | N | 696.4 | 11.21 (s, 1H), 10.36 (s, 1H), 9.05 (s, 1H), 8.64 - 8.52 (m, 4H), 8.47 (s, 1H), 8.31 - 8.23 (m, 1H), 8.21 - 8.14 (m, 1H), 7.62 - 7.44 (m, 2H), 6.96 (d, *J =* 5.4 Hz, 3H), 5.43 - 5.30 (m, 1H), 4.91 - 4.72 (m, 1H), 3.69 - 3.61 (m, 2H), 3.57 (s, 3H), 3.53 (s, 3H), 3.47 (d, *J* = 5.4 Hz, 1H), 2.98 (s, 2H), 2.93 - 2.82 (m, 2H), 2.63 - 2.59 (m, 2H), 1.97 (s, 1H), 1.92 - 1.81 (m, 2H), 1.01 - 0.92 (m, 2H), 0.63 - 0.57 (m, 2H) |
| **I-139^{b}** | ADX | N | 740.4 | 11.18 - 10.84 (m, 1H), 10.35 (s, 1H), 9.05 (s, 1H), 8.61 - 8.51 (m, 4H), 8.46 (s, 1H), 8.27 (d, *J =* 8.8 Hz, 1H), 7.59 - 7.48 (m, 2H), 7.03 (s, 1H), 7.00 - 6.85 (m, 2H), 5.36 - 5.29 (m, 1H), 4.87 - 4.66 (m, 1H), 3.75 - 3.63 (m, 2H), 3.63 - 3.58 (m, 2H), 3.55 - 3.48 (m, 3H), 3.46 (t, *J* = 5.6 Hz, 1H), 3.41 (t, *J =* 6.4 Hz, 2H), 3.32 - 3.31 (m, 3H), 2.95 - 2.83 (m, 1H), 2.74 - 2.69 (m, 1H), 2.67 - 2.64 (m, 2H), 2.60 - 2.57 (m, 1H), 2.05 - 1.93 (m, 1H), 1.87 - 1.78 (m, 2H), 1.23 (s, 1H), 0.98 - 0.89 (m, 2H), 0.62 - 0.51 (m, 2H) |
| **I-140^{b}** | ADZ | AFE | 1196.7 | 10.41 (s, 1H), 9.71 (s, 1H), 8.93 (s, 1H), 8.41 (t, *J* = 5.6 Hz, 1H), 8.32 (d, *J* = 8.4 Hz, 1H), 8.19 (d, *J* = 4.0 Hz, 2H), 8.15 (d, *J* = 5.2 Hz, 1H), 7.74 (t, *J* = 7.2 Hz, 1H), 7.59 (dd, *J* = 2.0, 12.8 Hz, 1H), 7.56 - 7.52 (m, 2H), 7.51 - 7.46 (m, 1H), 7.42 - 7.33 (m, 3H), 7.31 - 7.08 (m, 3H), 7.03 - 7.00 (m, 1H), 4.62 - 4.55 (m, 2H), 4.43 - 4.34 (m, 1H), 4.25 - 4.14 (m, 1H), 4.00 - 3.94 (m, 1H), 3.92 (s, 3H), 3.26 - 3.24 (m, 2H), 3.20 - 3.17 (m, 2H), 2.21 - 2.15 (m, 5H), 2.07 - 2.02 (m, 2H), 1.93 - 1.86 (m, 2H), 1.82 - 1.73 (m, 2H), 1.71 - 1.63 (m, 1H), 1.58 - 1.50 (m, 3H), 1.48 - 1.39 (m, 2H), 1.38 - 1.23 (m, 6H), 1.11 - 1.01 (m, 3H), 0.98 (s, 9H), 0.49 - 0.43 (m, 2H), 0.25 - 0.20 (m, 2H) |
| **I-141^{b}** | AEB | AFE | 1224.7 | 10.36 (s, 1H), 9.71 (s, 1H), 8.92 (s, 1H), 8.30 (s, 1H), 8.20 - 8.12 (m, 2H), 7.73 (t, *J =* 6.8 Hz, 1H), 7.63 - 7.50 (m, 2H), 7.41 - 6.89 (m, 10H), 4.63 - 4.53 (m, 2H), 4.49 - 4.32 (m, 2H), 4.25 - 4.14 (m, 1H), 3.97 - 3.90 (m, 1H), 3.88 (s, 3H), 3.17 (t, *J* = 6.0 Hz, 2H), 2.25 (t, *J* = 7.2 Hz, 2H), 2.13 (s, 3H), 2.11 - 2.07 (m, 2H), 2.03 (d, *J =* 9.8 Hz, 2H), 1.93 - 1.85 (m, 2H), 1.81 - 1.70 (m, 3H), 1.67 - 1.61 (m, 2H), 1.59 - 1.35 (m, 5H), 1.27-1.22 (m, 3H), 1.15 - 0.98 (m, 6H), 0.96 (s, 9H), 0.50 - 0.41 (m, 2H), 0.26 - 0.16 (m, 2H) |
| **I-142^{b}** | AFC | AFE | 1168.5 | 10.40 (s, 1H), 9.67 (s, 1H), 8.91 (s, 1H), 8.40 (t, *J* = 4.4 Hz, 1H), 8.31 (d, *J* = 8.4 Hz, 1H), 8.17 (s, 1H), 8.16 - 8.13 (m, 2H), 7.73 (t, *J* = 7.2 Hz, 1H), 7.60 - 7.47 (m, 4H), 7.41 - 7.32 (m, 3H), 7.30 - 7.14 (m, 1H), 7.11 - 7.06 (m, 2H), 7.02 - 7.01 (m, 1H), 4.60 - 4.57 (m, 2H), 4.40 - 4.32 (m, 1H), 4.24 - 4.14 (m, 1H), 3.99 - 3.94 (m, 1H), 3.91 (s, 3H), 3.18 (t, *J* = 6.0 Hz, 3H), 2.22 - 2.13 (m, 5H), 2.06 - 1.99 (m, 2H), 1.94 - 1.86 (m, 2H), 1.81 - 1.70 (m, 2H), 1.68 - 1.41 (m, 8H), 1.33 - 1.20 (m, 2H), 1.10 - 1.00 (m, 4H), 0.97 (s, 9H), 0.47 - 0.42 (m, 2H), 0.24 - 0.20 (m, 2H) |
| **I-143^{b}** | AFD | AFE | 1212.4 | 10.40 (s, 1H), 9.74 (s, 1H), 9.02 (s, 1H), 8.42 - 8.34 (m, 1H), 8.31 (dd, *J* = 2.0, 8.4 Hz, 1H), 8.19 (d, *J* = 6.4 Hz, 1H), 8.10 (d, *J* = 6.0 Hz, 1H), 7.73 (t, *J* = 6.8 Hz, 1H), 7.61 - 7.51 (m, 3H), 7.50 - 7.44 (m, 1H), 7.43 - 7.31 (m, 4H), 7.30 - 7.00 (m, 2H), 4.59 (s, 2H), 4.42 - 4.22 (m, 2H), 3.99 - 3.93 (m, 1H), 3.92 (s, 3H), 3.26 - 3.21 (m, 5H), 3.05 - 2.86 (m, 3H), 2.79 (s, 1H), 2.09 - 1.99 (m, 2H), 1.92 - 1.74 (m, 4H), 1.69 - 1.41 (m, 8H), 1.39 - 1.21 (m, 6H), 1.16 - 1.05 (m, 1H), 0.97 (s, 9H), 0.56 - 0.50 (m, 2H), 0.32 - 0.23 (m, 2H) |
| **I-144^{b}** | AEC | AFE | 1238.7 | 10.35 (s, 1H), 9.74 (s, 1H), 8.99 (s, 1H), 8.29 (d, *J* = 8.0 Hz, 1H), 8.19 (d, *J* = 4.4 Hz, 1H), 8.15 - 8.07 (m, 1H), 7.73 (t, *J =* 7.2 Hz, 1H), 7.62 - 7.50 (m, 2H), 7.45 - 6.88 (m, 8H), 4.63 - 4.53 (m, 2H), 4.52 - 4.18 (m, 3H), 3.97 - 3.86 (m, 4H), 3.27 (d, *J =* 7.2 Hz, 2H), 3.23 (d, *J =* 7.2 Hz, 2H), 3.02 (s, 3H), 2.11 - 1.99 (m, 2H), 1.96 - 1.39 (m, 13H), 1.29 - 1.21 (m, 2H), 1.21 - 1.03 (m, 4H), 0.97 (d, *J* = 2.8 Hz, 9H), 0.56 - 0.43 (m, 2H), 0.28 - 0.25 (m, 2H) |
| **I-145^{b}** | ACU | AFE | 1228.7 | 10.40 (s, 1H), 9.67 (s, 1H), 8.91 (s, 1H), 8.46 (t, *J =* 5.6 Hz, 1H), 8.32 (d, *J* = 8.4 Hz, 1H), 8.18 (s, 1H), 8.14 (s, 1H), 7.73 (t, *J =* 7.2 Hz, 1H), 7.60 - 7.46 (m, 4H), 7.42 - 7.28 (m, 3H), 7.17 - 6.99 (m, 4H), 4.63 - 4.55 (m, 2H), 4.42 - 4.31 (m, 1H), 4.25 - 4.12 (m, 1H), 3.99 - 3.94 (m, 1H), 3.91 (s, 3H), 3.58 - 3.47 (m, 8H), 3.46 - 3.39 (m, 2H), 3.18 (t, *J* = 6.0 Hz, 2H), 2.52- 2.51 (m, 2H), 2.30 - 2.26 (m, 1H), 2.23 (s, 3H), 2.21 - 2.16 (m, 1H), 2.07 - 1.99 (m, 2H), 1.92 - 1.84 (m, 2H), 1.81 - 1.71 (m, 2H), 1.71 - 1.59 (m, 1H), 1.58 - 1.48 (m, 1H), 1.31 - 1.20 (m, 1H), 1.10 - 0.99 (m, 3H), 0.97 (s, 9H), 0.48 - 0.41 (m, 2H), 0.25 - 0.18 (m, 2H) |
| **I-146^{b}** | AEN | AHB | 1134.6 | 9.68 (s, 1H), 8.91 (s, 1H), 8.25 (s, 1H), 8.17 (s, 1H), 8.15 (s, 1H), 7.53 - 6.82 (m, 12H), 5.00 (d, *J =* 10.8 Hz, 1H), 4.22 - 4.13 (m, 1H), 3.87 - 3.79 (m, 2H), 3.60 - 3.57 (m, 2H), 3.41 (s, 3H), 3.19 - 3.13 (m, 4H), 3.07 - 3.04 (m, 2H), 2.80 (d, *J =* 12.8 Hz, 1H), 2.37 - 2.32 (m, 2H), 2.20 - 2.11 (m, 3H), 2.07 (s, 3H), 2.04 - 1.96 (m, 4H), 1.87 (d, *J* = 11.6 Hz, 2H), 1.79 - 1.68 (m, 2H), 1.41 - 1.31 (m, 4H), 1.28 (d, *J* = 6.4 Hz, 6H), 1.23 (s, 4H), 1.02 - 0.91 (m, 2H), 0.55 (d, *J* = 6.4 Hz, 3H), 0.47 - 0.43 (m, 2H), 0.40 (d, *J* = 6.0 Hz, 3H), 0.22 (d, *J* = 4.0 Hz, 2H) |
| **I-147^{b}** | ACW | AHB | 1160.6 | 9.69 (s, 1H), 8.91 (s, 1H), 8.18 (s, 1H), 8.14 (s, 1H), 7.46 - 7.19 (m, 4H), 7.19 - 7.13 (m, 2H), 7.10 (s, 1H), 7.09 - 7.05 (m, 1H), 7.03 - 6.99 (m, 1H), 6.98 - 6.89 (m, 2H), 5.02 - 4.89 (m, 1H), 4.59 - 4.45 (m, 1H), 4.26 - 4.15 (m, 1H), 4.10 - 4.01 (m, 1H), 3.85 - 3.75 (m, 1H), 3.62 - 3.52 (m, 1H), 3.21 - 3.14 (m, 4H), 3.14 - 3.00 (m, 3H), 2.84 - 2.72 (m, 2H), 2.52 (s, 3H), 2.30 - 2.20 (m, 4H), 2.17 - 2.01 (m, 6H), 1.95 - 1.85 (m, 2H), 1.83 - 1.68 (m, 4H), 1.65 - 1.52 (m, 2H), 1.46 - 1.34 (m, 2H), 1.29 (d, *J =* 6.8 Hz, 6H), 1.22 (d, *J* = 8.8 Hz, 3H), 1.14 - 0.93 (m, 5H), 0.58 (d, *J =* 5.2 Hz, 3H), 0.48 - 0.43 (m, 2H), 0.42 - 0.38 (m, 3H), 0.25 - 0.18 (m, 2H) |
| **I-148^{b}** | AAY | ACY | 854.3 | 11.10 (s, 1H), 9.73 (s, 1H), 8.92 (s, 1H), 8.14 (s, 1H), 7.31 - 7.11 (m, 3H), 7.10 - 7.07 (m, 2H), 7.01 (dd, *J =* 1.2, 5.2 Hz, 1H), 5.38 (dd, *J* = 4.8, 12.4 Hz, 1H), 4.51 (d, *J* = 11.2 Hz, 2H), 4.01 - 4.00 (m, 1H), 3.90 - 3.74 (m, 2H), 3.35 (s, 3H), 3.22 - 3.13 (m, 4H), 3.08 - 3.00 (m, 2H), 2.95 - 2.85 (m, 2H), 2.78 - 2.68 (m, 4H), 2.12 - 1.97 (m, 4H), 1.91 - 1.68 (m, 6H), 1.41 - 1.26 (m, 2H), 1.16 - 0.98 (m, 2H), 0.49 - 0.41 (m, 2H), 0.25 - 0.18 (m, 2H) |
| **I-149^{b}** | TN | AEO | 872.5 | 11.09 (s, 1H), 9.40 (s, 1H), 8.83 (d, *J* = 8.0 Hz, 1H), 8.39 (s, 1H), 8.29 (s, 1H), 7.25 - 6.93 (m, 3H), 6.93 - 6.84 (m, 2H), 5.37 (dd, *J* = 5.2, 12.8 Hz, 1H), 4.28 - 4.21 (m, 1H), 3.89 - 3.67 (m, 10H), 3.57 (s, 3H), 3.54 - 3.45 (m, 3H), 3.28 - 3.23 (m, 1H), 3.14 - 3.05 (m,, 1H), 3.01 - 2.83 (m, 3H), 2.78 - 2.60 (m, 3H), 2.07 - 1.95 (m, 3H), 1.92 - 1.72 (m, 8H), 1.63 - 1.49 (m, 2H), 1.48 - 1.28 (m, 2H) |
| **I-150^{b}** | AFO | AGJ | 843.6 | 11.09 (s, 1H), 9.36 (s, 1H), 8.53 (d, J = 7.6 Hz, 1H), 8.34 (s, 1H), 8.15 (s, 1H), 7.80 (d, J = 8.8 Hz, 1H), 7.29 - 6.99 (m, 2H), 6.96 (t, J = 8.0 Hz, 1H), 6.91 - 6.83 (m, 1H), 6.62 (d, J = 7.6 Hz, 1H), 5.37 (dd, J = 5.2, 12.8 Hz, 1H), 4.85 - 4.66 (m, 1H), 4.32 - 4.14 (m, 2H), 3.88 - 3.80 (m, 1H), 3.67 (s, 3H), 3.64 - 3.60 (m, 2H), 2.97 - 2.79 (m, 5H), 2.74 - 2.58 (m, 2H), 2.18 (d, J = 6.8 Hz, 2H), 2.13 - 1.83 (m, 9H), 1.77 - 1.63 (m, 4H), 1.63 - 1.45 (m, 5H), 1.43 - 1.28 (m, 2H), 1.14 - 0.98 (m, 2H) |
| **I-151^{b}** | AGN | ABQ | 892.1 | 11.14 (s, 1H), 9.40 (s, 1H), 8.74 (d, *J* = 8.0 Hz, 1H), 8.56 (d, *J =* 7.6 Hz, 1H), 8.44 (d, *J* = 4.8 Hz, 1H), 8.40 (s, 1H), 8.25 (s, 1H), 7.50 - 7.41 (m, 2H), 7.29 (d, *J* = 6.0 Hz, 1H), 7.25 - 6.96 (m, 2H), 6.92 (d, *J* = 8.0 Hz, 1H), 4.29 - 4.08 (m, 3H), 3.82 (t, *J =* 4.0 Hz, 1H), 3.64 - 3.44 (m, 7H), 3.31 - 3.18 (m, 4H), 3.12 - 3.04 (m, 2H), 3.03 - 2.92 (m, 4H), 2.25 - 2.15 (m, 1H), 2.14 - 2.05 (m, 3H), 2.04 - 1.96 (m, 3H), 1.92 (d, *J* = 10.4 Hz, 4H), 1.87 - 1.79 (m, 4H), 1.71 (d, *J* = 2.8 Hz, 2H), 1.45 (dq,*J* = 4.0, 8.4 Hz, 2H), 1.27 - 1.12 (m, 2H) |
| **I-152^{b}** | AFR | ABQ | 897.6 | 10.98 (d, *J =* 5.2 Hz, 1H), 9.38 (s, 1H), 8.73 (d, *J =* 8.0 Hz, 1H), 8.38 (s, 1H), 8.25 (s, 1H), 7.47 - 7.38 (m, 4H), 7.25 - 6.95 (m, 1H), 6.91 (d, *J* = 8.0 Hz, 1H), 5.71 - 5.59 (m, 1H), 4.84 - 4.76 (m, 1H), 4.73 - 4.62 (m, 1H), 4.23 - 4.06 (m, 3H), 3.95 - 3.85 (m, 1H), 3.84 - 3.78 (m, 1H), 3.63 - 3.56 (m, 2H), 3.52 - 3.45 (m, 2H), 3.44 - 3.36 (m, 3H), 2.91 - 2.77 (m, 1H), 2.73 - 2.62 (m, 4H), 2.28 - 2.17 (m, 1H), 2.14 - 2.09 (m, 2H), 2.08 - 1.95 (m, 5H), 1.91 - 1.80 (m, 6H), 1.77 - 1.68 (m, 2H), 1.62 - 1.52 (m, 1H), 1.50 - 1.40 (m, 4H), 1.09 - 0.98 (m, 2H) |
| **I-153^{b}** | AFT | AGJ | 904.3 | 11.10 (s, 1H), 9.29 (s, 1H), 8.54 (d, *J* = 7.6 Hz, 1H), 8.41 (s, 1H), 8.18 (s, 1H), 7.87 (d, *J =* 9.6 Hz, 1H), 6.96 (d, *J =* 4.8 Hz, 2H), 6.90 - 6.86 (m, 1H), 6.63 (d, *J =* 7.6 Hz, 1H), 5.40 - 5.32 (m, 1H), 4.83 - 4.80 (m, 1H), 4.28 - 4.13 (m, 2H), 3.89 - 3.80 (m, 1H), 3.57 (s, 3H), 3.46 (t, *J =* 6.2 Hz, 2H), 3.00 - 2.84 (m, 3H), 2.72 - 2.59 (m, 4H), 2.13 - 1.95 (m, 8H), 1.92 - 1.68 (m, 11H), 1.64 - 1.41 (m, 8H), 1.38 - 1.28 (m, 3H), 1.09 - 0.98 (m, 3H), 0.88 - 0.79 (m, 1H) |
| **I-154** | AAU | OM | 857.2 | 11.07 (s, 1H), 9.68 (s, 1H), 8.91 (s, 1H), 8.25 (s, 1H), 8.18 - 8.13 (m, 2H), 7.31 - 7.00 (m, 4H), 6.96 (d, *J* = 4.8 Hz, 2H), 6.91 - 6.84 (m, 1H), 5.36 (dd, *J* = 5.2, 12.8 Hz, 1H), 4.23 - 4.14 (m, 1H), 3.56 (s, 3H), 3.44 - 3.46 (m, 4H), 3.18 (t, *J* = 6.4 Hz, 2H), 3.00 - 2.93 (m, 2H), 2.92 - 2.83 (m, 1H), 2.75 - 2.67 (m, 1H), 2.65 - 2.58 (m, 1H), 2.55 - 2.53 (m, 2H), 2.41 (t, *J* = 6.4 Hz, 2H), 2.21 (s, 3H), 2.04 - 1.95 (m, 3H), 1.88 - 1.79 (m, 4H), 1.77 - 1.67 (m, 2H), 1.36 (s, 3H), 1.17 - 1.02 (m, 3H), 0.48 - 0.41 (m, 2H), 0.24 - 0.20(m, 2H) |
| **I-155** | ADF | ACE | 920.5 | 11.10 (s, 1H), 9.26 (s, 1H), 8.88 (d, *J* = 8.0 Hz, 1H), 8.38 (s, 1H), 8.32 (s, 1H), 7.29 - 6.99 (m, 3H), 6.95 (t, *J* = 8.0 Hz, 1H), 6.89 - 6.84 (m, 1H), 5.37 (dd, *J* = 5.2, 12.4 Hz, 1H), 4.87 - 4.71 (m, 1H), 4.39 - 4.30 (m, 1H), 4.22 - 4.16 (m, 1H), 3.67 (s, 2H), 3.60 (s, 3H), 3.12 - 3.10 (m, 2H), 3.08 - 3.03 (m, 2H), 2.93 - 2.89 (m, 1H), 2.83 - 2.80 (m, 2H), 2.77 - 2.70 (m, 1H), 2.66 - 2.62 (m, 1H), 2.62 - 2.58 (m, 1H), 2.40 - 2.35 (m, 2H), 2.19 - 2.17 (m, 1H), 2.17 (s, 3H), 2.07 - 1.98 (m, 4H), 1.97 - 1.92 (m, 2H), 1.88 - 1.84 (m, 2H), 1.79 - 1.71 (m, 2H), 1.66 - 1.60 (m, 2H), 1.58 - 1.51 (m, 1H), 1.36 - 1.34 (m, 3H), 1.13 - 1.00 (m, 4H) |
| **I-156^{b,d}** | ABN | ABO | 842.5 | 11.09 (s, 1H), 9.56 (s, 1H), 8.57 (d, *J* = 7.6 Hz, 1H), 8.37 (s, 1H), 8.29 - 8.22 (m, 1H), 8.19 (s, 1H), 7.26 - 6.93 (m, 3H), 6.91 - 6.84 (m, 1H), 6.46 (d, *J =* 7.6 Hz, 1H), 5.42 - 5.30 (m, 1H), 4.25 - 4.09 (m, 1H), 3.57 (s, 3H), 3.46 - 3.44 (m, 2H), 3.30 - 3.30 (m, 1H), 3.01 - 2.94 (m, 2H), 2.93 - 2.85 (m, 1H), 2.76 - 2.70 (m, 1H), 2.70 - 2.65 (m, 2H), 2.65 - 2.57 (m, 1H), 2.19 - 1.95 (m, 8H), 1.93 - 1.78 (m, 7H), 1.77 - 1.66 (m, 2H), 1.63 - 1.54 (m, 1H), 1.51 - 1.41 (m, 2H), 1.12 - 0.97 (m, 3H), 0.57 - 0.50 (m, 2H), 0.29 - 0.23 (m, 2H) |
| **I-157^{b}** | ABR | ABQ | 872.5 | 11.08 (s, 1H), 9.39 (s, 1H), 8.73 (d, *J* = 8.0 Hz, 1H), 8.39 (s, 1H), 8.25 (s, 1H), 7.24 - 6.95 (m, 3H), 6.93 - 6.85 (m, 2H), 5.38 (dd, *J* = 5.2, 12.0 Hz, 1H), 4.29 - 4.07 (m, 3H), 3.86 - 3.78 (m, 1H), 3.71 - 3.64 (m, 1H), 3.59 - 3.56 (m, 2H), 3.50 (s, 3H), 3.38 - 3.31 (m, 3H), 3.09 - 2.82 (m, 8H), 2.78 - 2.62 (m, 2H), 2.15 - 1.75 (m, 17H), 1.50 - 1.39 (m, 2H), 1.24 - 1.13 (m, 2H) |
| **I-158 ^{b,d}** | ABN | ABS | 899.5 | 11.09 (s, 1H), 9.39 (s, 1H), 8.75 (d, *J* = 8.0 Hz, 1H), 8.38 (s, 1H), 8.26 (s, 1H), 7.26 - 6.95 (m, 3H), 6.92 - 6.88 (m, 1H), 6.89 - 6.85 (m, 1H), 5.36 (dd, *J* = 6.0, 12.8 Hz, 1H), 4.61 - 4.51 (m, 1H), 4.23 - 4.11 (m, 1H), 3.57 (s, 3H), 3.45 (t, *J* = 6.0 Hz, 3H), 3.31 - 3.25 (m, 2H), 3.13 - 3.05 (m, 2H), 2.99 - 2.93 (m, 2H), 2.91 - 2.84 (m, 1H), 2.72 - 2.65(m, 1H), 2.65 - 2.58 (m, 2H), 2.22 (s, 6H), 2.11 (d, *J* = 7.2 Hz, 2H), 2.08 - 1.96 (m, 5H), 1.92 - 1.68 (m, 11H), 1.62 - 1.52 (m, 1H), 1.49 - 1.40 (m, 4H), 1.10 - 0.98 (m, 2H) |
| **I-159^{b}** | ACK | ACI | 708.4 | 11.07 (s, 1H), 8.94 (d, *J* = 1.6 Hz, 1H), 8.55 (s, 1H), 8.27 (d, *J* = 7.6 Hz, 1H), 8.05 (dd, *J* = 1.6, 8.8 Hz, 1H), 7.76 (d, *J* = 8.8 Hz, 1H), 6.96 (d, *J* = 4.8 Hz, 2H), 6.89 - 6.82 (m, 1H), 5.35 (dd, *J* = 5.2, 12.4 Hz, 1H), 4.20 - 4.05 (m, 1H), 3.62 (t, *J =* 6.4 Hz, 2H), 3.55 (s, 3H), 3.48 - 3.42 (m, 6H), 2.99 - 2.92 (m, 2H), 2.91 - 2.82 (m, 1H), 2.76 - 2.67 (m, 1H), 2.65 - 2.60 (m, 1H), 2.59 - 2.55 (m, 2H), 2.55- 2.51 (m, 3H), 2.48 - 2.45 (m, 1H), 2.43 - 2.34 (m, 1H), 2.08 - 1.93 (m, 3H), 1.90 - 1.74 (m, 4H), 1.50 - 1.29 (m, 4H) |
| **I-160^{b}** | ABT | ABU | 802.3 | 13.13 (s, 1H), 11.06 (s, 1H), 9.38 (dd, *J* = 1.6, 6.8 Hz, 1H), 9.14 (dd, *J* = 1.6, 4.0 Hz, 1H), 8.72 (s, 1H), 8.51 (d, *J =* 0.8 Hz, 1H), 7.87 - 7.78 (m, 2H), 7.37 (dd, *J* = 4.0, 6.8 Hz, 1H), 6.94 (d, *J* = 4.8 Hz, 2H), 6.90 (s, 1H), 6.88 - 6.83 (m, 1H), 5.34 (dd, *J* = 5.2, 12.8 Hz, 1H), 4.45 (d, *J* = 13.2 Hz, 1H), 3.95 (d, *J* = 12.8 Hz, 1H), 3.55 (s, 3H), 3.47 - 3.38 (m, 4H), 3.17 (t, *J* = 11.6 Hz, 1H), 2.99 - 2.82 (m, 4H), 2.79 - 2.60 (m, 3H), 2.40 (s, 3H), 2.38 - 2.35 (m, 2H), 2.05 - 1.91 (m, 3H), 1.88 - 1.76 (m, 2H), 1.69 - 1.46 (m, 6H) |
| **I-351^{b}** | AHD | ALS | 911.4 | 11.10 (s, 1H), 9.51 (d, *J* = 6.8 Hz, 1H), 9.29 - 9.16 (m, 1H), 8.78 (d, *J* = 7.4 Hz, 1H), 8.40 (d, *J* = 4.4 Hz, 1H), 8.26 (d, *J* = 5.6 Hz, 1H), 7.62 (dd, *J* = 7.2, 8.4 Hz, 1H), 7.26 - 6.96 (m, 3H), 6.88 - 6.44 (m, 2H), 5.33 - 5.00 (m, 2H), 4.84 - 4.70 (m, 1H), 4.62 - 4.49 (m, 1H), 4.33 - 4.10 (m, 2H), 3.85 - 3.71 (m, 2H), 3.59 (s, 1H), 3.57 - 3.48 (m, 1H), 3.47 - 3.42 (m, 1H), 3.26 - 3.23 (m, 1H), 3.14 - 3.02 (m, 2H), 2.95 - 2.83 (m, 1H), 2.79 - 2.69 (m, 4H), 2.64 - 2.58 (m, 1H), 2.58 - 2.54 (m, 1H), 2.09 - 1.74 (m, 15H), 1.71 - 1.33 (m, 5H) |
| **I-370** | AJF | ACQ | 881.5 | 11.09 (s, 1H), 9.40 (s, 1H), 8.76 (d, *J* = 8.0 Hz, 1H), 8.39 (s, 1H), 8.26 (s, 1H), 7.62 - 7.52 (m, 1H), 7.24 - 6.93 (m, 3H), 6.88 (d, J = 8.0 Hz, 1H), 6.50 (s, 1H), 5.08 - 5.01 (m, 1H), 4.29 - 4.18 (m, 1H), 3.74 (s, 3H), 3.39 - 3.37 (m, 5H), 2.94 - 2.86 (m, 1H), 2.82 (s, 4H), 2.63 - 2.53 (m, 3H), 2.52 (m, 2H), 2.07 - 1.70 (m, 10H), 1.63 - 1.36 (m, 9H) |
| **I-388^{b}** | ATN | ALS | 928.9 | 11.14 (s, 1H), 9.50 (d, *J* = 6.8 Hz, 1H), 8.78 (d, *J* = 7.6 Hz, 1H), 8.53 (dd, *J* = 7.6, 1.6 Hz, 1H), 8.44 - 8.34 (m, 2H), 8.26 (d, *J* = 5.6 Hz, 1H), 8.04 (s, 1H), 7.52 (d, *J* = 7.6 Hz, 1H), 7.39 - 7.32 (m, 1H), 7.25 (dd, *J* = 7.6, 4.8 Hz, 1H), 7.10 (d, *J =* 3.6 Hz, 1H), 7.28 - 6.95 (m, 1H), 6.89 - 6.42 (m, 1H), 6.02 (s, 1H), 5.28 (s, 1H), 5.07 (s, 1H), 4.76 (d, *J* = 19.2 Hz, 1H), 4.31 - 4.18 (m, 1H), 3.84 - 3.71 (m, 2H), 3.62 - 3.57 (m, 4H), 3.43 (s, 4H), 3.11 (d, *J* = 4.0 Hz, 4H), 2.81 - 2.70 (m, 3H), 2.58 (t, *J =* 6.8 Hz, 2H), 2.52 (d, *J* = 1.6 Hz, 1H), 2.13 - 1.50 (m, 18H) |
| **I-407^{b}** | AOD | ALS | 909.6 | 11.09 (s, 1H), 9.50 (d, *J* = 6.6 Hz, 1H), 8.78 (d, *J* = 7.6 Hz, 1H), 8.39 (d, *J* = 4.6 Hz, 1H), 8.27 - 8.23 (m, 2H), 7.24 - 6.43 (m, 5H), 5.36 (dd, *J* = 5.2, 12.4 Hz, 1H), 5.30 - 5.03 (m, 1H), 4.77 (d, *J* = 18.4 Hz, 1H), 4.34 - 4.17 (m, 1H), 3.86 - 3.71 (m, 2H), 3.66 - 3.58 (m, 2H), 3.57 (s, 3H), 2.95 - 2.83 (m, 8H), 2.76 - 2.70 (m, 2H), 2.65 - 2.60 (m, 1H), 2.07 - 1.53 (m, 22H) |
| **I-418^{b}** | ALR | ALS | 905.6 | 11.12 (s, 1H), 9.49 (d, *J* = 6.0 Hz, 1H), 8.78 (d, *J* = 7.6 Hz, 1H), 8.38 (d, *J* = 4.4 Hz, 1H), 8.25 (d, *J* = 5.6 Hz, 1H), 7.27 - 6.93 (m, 4H), 6.89 - 6.42 (m, 1H), 5.40 (dd, *J =* 5.2, 12.8 Hz, 1H), 5.30 - 5.04 (m, 1H), 4.76 (d, *J* = 18.8 Hz, 1H), 4.30 - 4.17 (m, 1H), 3.89 - 3.72 (m, 4H), 3.66 (s, 2H), 3.65 - 3.49 (m, 6H), 3.44 (d, *J* = 9.6 Hz, 1H), 3.39 - 3.37 (m, 2H), 2.95 - 2.82 (m, 1H), 2.78 - 2.68 (m, 1H), 2.66 - 2.59 (m, 1H), 2.58 - 2.52 (m, 1H), 2.41 - 2.24 (m, 2H), 2.08 - 1.90 (m, 5H), 1.87 - 1.66 (m, 8H), 1.58 - 1.44 (m, 2H) |
| **I-420^{b}** | ACV | ALS | 895.5 | 11.11 (s, 1H), 9.51 (d, *J* = 6.4 Hz, 1H), 8.79 (d, *J* = 7.6 Hz, 1H), 8.40 (d, J = 5.2 Hz, 1H), 8.26 (d, *J* = 5.6 Hz, 1H), 7.26 - 6.95 (m, 4H), 6.89 - 6.43 (m, 1H), 5.42 - 5.35 (m, 1H), 5.30 - 5.06 (m, 1H), 4.81 - 4.72 (m, 1H), 4.31 - 4.21 (m, 1H), 4.07 - 3.90 (m, 4H), 3.84 - 3.72 (m, 2H), 3.66 - 3.61 (m, 1H), 3.60 (s, 3H), 3.55 - 3.41 (m, 6H), 3.15 - 3.09 (m, 2H), 2.95 - 2.84 (m, 1H), 2.77 - 2.68 (m, 2H), 2.64 - 2.59 (m, 1H), 2.08 - 1.67 (m, 14H), 1.56 (d, *J* = 13.2 Hz, 2H) |
| **I-422^{b}** | ALQ | ALS | 909.6 | 11.10 (s, 1H), 9.51 (d, *J* = 6.4 Hz, 1H), 8.79 (d, *J* = 7.6 Hz, 1H), 8.40 (d, *J =* 4.4 Hz, 1H), 8.26 (d, *J =* 5.6 Hz, 1H), 7.29 - 7.07 (m, 1H), 6.98 (d, *J=* 4.8 Hz, 2H), 6.93 - 6.44 (m, 2H), 5.38 (dd, *J* = 5.2, 12.4 Hz, 1H), 5.31 - 5.05 (m, 1H), 4.78 (d, *J =* 18.8 Hz, 1H), 4.31 - 4.20 (m, 1H), 3.94 - 3.77 (m, 4H), 3.77 - 3.62 (m, 2H), 3.61 - 3.52 (m, 6H), 2.99 - 2.88 (m, 3H), 2.73 - 2.63 (m, 4H), 2.31 - 2.24 (m, 1H), 2.11 - 1.90 (m, 6H), 1.88 - 1.72 (m, 10H), 1.59 - 1.48 (m, 2H) |
| **I-423^{b}** | AMS | AKT | 874.1 | 11.09 (s, 1H), 9.62 (d, *J* = 4.0 Hz, 1H), 9.05 (d, *J* = 3.2 Hz, 1H), 8.81 (dd, *J* = 1.6, 8.0 Hz, 1H), 8.31 (d, *J* = 5.2 Hz, 1H), 7.91 (d, *J* = 8.4 Hz, 2H), 7.52 (d, *J* = 8.4 Hz, 2H), 7.47 - 7.17 (m, 1H), 7.03 - 6.40 (m, 4H), 5.36 (dd, *J* = 5.2, 12.8 Hz, 1H), 5.32 - 5.06 (m, 1H), 4.84 - 4.76 (m, 1H), 3.89 - 3.61 (m, 4H), 3.55 (s, 3H), 3.49 - 3.45 (m 1H), 3.08 - 3.04 (m, 2H), 2.96 - 2.83 (m, 1H), 2.77 - 2.67 (m, 1H), 2.63 - 2.60 (m, 1H), 2.11 - 1.93 (m, 5H), 1.64 - 1.50 (m, 6H), 1.37 - 1.27 (m, 1H), 1.18 - 1.04 (m, 1H), 0.96 - 0.83 (m, 2H) |
| **I-424^{b}** | AKS | AKT | 933.5 | 11.09 (s, 1H), 9.63 (d, *J* = 4.0 Hz, 1H), 9.06 (d, *J* = 3.2 Hz, 1H), 8.85 - 8.77 (m, 1H), 8.31 (d, *J =* 5.2 Hz, 1H), 7.92 (d, *J* = 8.4 Hz, 2H), 7.56 (d, *J* = 8.8 Hz, 2H), 7.47 - 7.14 (m, 1H), 7.00 - 6.45 (m, 4H), 5.42 - 5.33 (m, 1H), 5.32 - 5.08 (m, 1H), 4.84 - 4.75 (m, 1H), 4.24 - 4.00 (m, 1H), 3.88 - 3.80 (m, 2H), 3.79 - 3.71 (m, 1H), 3.70 - 3.60 (m, 4H), 3.57 (s, 3H), 3.51 - 3.39 (m, 2H), 3.27 - 3.11 (m, 2H), 2.98 - 2.87 (m, 3H), 2.79 - 2.68 (m, 1H), 2.65 - 2.57 (m, 1H), 2.40 - 2.19 (m, 6H), 2.12 - 2.02 (m, 1H), 2.00 - 1.92 (m, 2H), 1.81 - 1.71 (m, 2H), 1.58 - 1.45 (m, 2H) |
| **I-426^{b}** | ALL | AKT | 876.5 | 11.08 (s, 1H), 9.62 (d, *J* = 4.0 Hz, 1H), 9.05 (d, *J* = 3.2 Hz, 1H), 8.81 (dd, *J* = 2.0, 7.8 Hz, 1H), 8.31 (d, *J* = 5.4 Hz, 1H), 7.91 (d, *J* = 8.0 Hz, 2H), 7.53 (d, *J* = 8.4 Hz, 2H), 7.48 - 7.17 (m, 1H), 6.95 (d, *J =* 5.0 Hz, 2H), 6.91 - 6.86 (m, 1H), 6.86 - 6.44 (m, 1H), 5.36 (dd, *J* = 5.4, 12.4 Hz, 1H), 5.31 (s, 1H), 4.79 (d, *J =* 12.0 Hz, 1H), 4.59 - 4.29 (m, 1H), 3.88 - 3.61 (m, 4H), 3.55 (s, 3H), 3.47 (d, *J* = 10.0 Hz, 2H), 3.12 - 2.97 (m, 1H), 2.93 - 2.84 (m, 3H), 2.76 - 2.68 (m, 1H), 2.65 - 2.58 (m, 1H), 2.06 - 1.93 (m, 3H), 1.80 - 1.69 (m, 1H), 1.67 - 1.51 (m, 4H), 1.45 - 1.38 (m, 2H), 1.35 - 1.26 (m, 2H), 1.15 - 1.05 (m, 2H) |
| **I-427^{b}** | AMX | AEH | 856.2 | 11.13 (s, 1H), 9.50 (d, *J* = 4.8 Hz, 1H), 8.78 (d, *J* = 7.6 Hz, 1H), 8.45 - 8.35 (m, 1H), 8.25 (d, *J* = 5.6 Hz, 1H), 7.73 - 7.62 (m, 1H), 7.19 (d, *J =* 7.6 Hz, 1H), 7.10 (d, *J* = 7.6 Hz, 1H), 6.99 - 6.93 (m, 1H), 6.89 - 6.42 (m, 1H), 5.42 (dd, *J* = 4.8, 12.4 Hz, 1H), 5.31 - 5.03 (m, 1H), 4.77 (d, *J* = 16.8 Hz, 1H), 4.19 (t, *J =* 12.0 Hz, 1H), 4.07 - 3.91 (m, 1H), 3.83 - 3.79 (m, 1H), 3.77 - 3.61 (m, 1H), 3.60 - 3.53 (m, 2H), 3.48 - 3.42 (m, 2H), 3.39 - 3.37 (m, 3H), 3.25 (d, *J =* 4.8 Hz, 2H), 2.95 - 2.71 (m, 2H), 2.06 - 2.01 (m, 3H), 1.98 - 1.71 (m, 8H), 1.67 - 1.35 (m, 4H), 1.29 - 1.08 (m, 4H) |
| **I-429^{b}** | AGI | ALS | 881.4 | 11.10 (s, 1H), 9.50 (d, *J* = 6.8 Hz, 1H), 8.78 (d, *J* = 7.6 Hz, 1H), 8.38 (d, *J* = 4.8 Hz, 1H), 8.25 (d, *J* = 5.6 Hz, 1H), 7.62 - 7.00 (m, 2H), 6.97 - 6.92 (m, 2H), 6.89 - 6.42 (m, 1H), 5.37 (dd, *J* = 5.2, 12.4 Hz, 1H), 5.30 - 5.03 (m, 1H), 4.76 (d, *J* = 18.4 Hz, 1H), 4.29 - 4.17 (m, 1H), 3.82 (m, 3H), 3.75 - 3.57 (m, 6H), 3.45 (s, 3H), 3.05 - 2.97 (m, 4H), 2.93 - 2.85 (m, 1H), 2.76 - 2.58 (m, 4H), 2.06 - 1.93 (m, 5H), 1.91 - 1.80 (m, 2H), 1.75 (d, *J* = 12.8 Hz, 2H), 1.71 - 1.64 (m, 2H), 1.62 - 1.47 (m, 4H) |
| **I-431^{b}** | AKS | ALS | 939.6 | 11.10 (s, 1H), 9.51 (d, *J* = 6.4 Hz, 1H), 8.79 (d, *J =* 7.6 Hz, 1H), 8.40 (d, *J =* 4.4 Hz, 1H), 8.26 (d, *J =* 5.2 Hz, 1H), 7.30 - 6.90 (m, 4H), 6.89 - 6.44 (m, 1H), 5.43 - 5.33 (m, 1H), 5.31 - 5.05 (m, 1H), 4.82 - 4.71 (m, 1H), 4.36 - 4.18 (m, 1H), 4.16 - 3.85 (m, 2H), 3.85 - 3.78 (m, 2H), 3.77 - 3.60 (m, 4H), 3.59 (s, 3H), 3.52 - 3.40 (m, 2H), 3.06 - 2.83 (m, 5H), 2.79 - 2.60 (m, 4H), 2.31 - 2.16 (m, 2H), 2.11 - 1.86 (m, 9H), 1.83 - 1.69 (m, 4H), 1.66 - 1.29 (m, 5H) |
| **I-432^{b}** | ATQ | ALS | 909.7 | 11.17 - 11.05 (s, 1H), 9.51 (d, *J* = 6.4 Hz, 1H), 8.83 - 8.74 (m, 1H), 8.40 (d, *J* = 5.2 Hz, 1H), 8.26 (d, *J* = 6.4 Hz, 1H), 7.27 - 6.81 (m, 3H), 6.42 (m, 1H), 5.45 - 5.03 (m, 2H), 4.85 - 4.70 (m, 1H), 4.32 - 4.17 (m, 1H), 3.88 - 3.76 (m, 2H), 3.73 - 3.57 (m, 6H), 3.53 - 3.40 (m, 8H), 2.68 (s, 4H), 2.37 - 2.31 (m, 2H), 2.09 - 1.94 (m, 5H), 1.90 - 1.73 (m, 4H), 1.59 - 1.17 (m, 10H) |
| **I-433^{b}** | BAA | ALS | 909.4 | 11.08 (s, 1H), 9.49(d, *J* = 6.4 Hz, 1H), 8.76 (d, *J* = 8.0 Hz, 1H), 8.38 (d, *J* = 4.4 Hz, 1H), 8.25 (d, *J* = 5.6 Hz, 1H), 7.23 - 7.01 (m, 3H), 6.99 - 6.48 (m, 2H), 5.35 - 5.32 (m, 1H), 5.30 - 5.27 (m, 1H), 4.80 - 4.78 (m, 1H), 4.24 - 4.22 (m, 1H), 3.80 (s, 3H), 3.76 - 3.74 (m, 2H), 3.63 - 3.61 (m, 4H), 3.45 - 3.43 (m, 2H), 3.38 - 3.34 (m, 2H), 3.30 - 3.26 (m, 4H), 3.25 - 3.24 (m, 2H), 2.91 - 2.81 (m, 1H), 2.67 - 2.63 (m, 2H), 2.05 - 1.98 (m, 5H), 1.94 - 1.79 (m, 4H), 1.74 - 1.50 (m, 9H) |
| **I-435^{b}** | ATP | ALS | 937.6 | 11.09 (s, 1H), 9.51 (d, *J* = 6.8 Hz, 1H), 8.78 (d, *J* = 7.6 Hz, 1H), 8.40 (d, *J =* 4.8 Hz, 1H), 8.26 (d, *J =* 5.6 Hz, 1H), 7.26 - 6.94 (m, 3H), 6.90 - 6.44 (m, 2H), 5.36 (dd, *J =* 5.2, 12.8 Hz, 1H), 5.30 - 5.05 (m, 1H), 4.77 (d, *J =* 18.4 Hz, 1H), 4.32 - 4.18 (m, 1H), 4.30 - 4.17 (m, 1H), 3.86 - 3.58 (m, 6H), 3.57 (s, 3H), 2.95 - 2.88 (m, 3H), 2.76 - 2.68 (m, 2H), 2.66 - 2.60 (m, 1H), 2.45 - 2.39 (m, 6H), 2.07 - 1.92 (m, 6H), 1.91 - 1.72 (m, 7H), 1.62 - 1.53 (m, 2H), 1.48 (m, 4H), 1.42 (m, 2H), 1.33 (m, 2H) |
| **I-439^{b}** | AJI | ALS | 854.4 | 11.09 (s, 1H), 9.50 (d, *J* = 6.4 Hz, 1H), 8.78 (d, *J* = 7.6 Hz, 1H), 8.39 (d, *J* = 4.8 Hz, 1H), 8.26 (d, *J* = 5.6 Hz, 1H), 7.62 - 7.54 (m, 1H), 7.26 - 6.94 (m, 3H), 6.88 - 6.43 (m, 2H), 5.30 - 5.02 (m, 2H), 4.77 (d, *J =* 17.8 Hz, 1H), 4.42 (d, *J* = 12.8 Hz, 1H), 4.32 - 4.18 (m, 1H), 4.10 - 3.97 (m, 1H), 3.85 - 3.71 (m, 2H), 3.66 - 3.42 (m, 2H), 3.24 (t, *J* = 6.4 Hz, 2H), 3.05 - 2.96 (m, 1H), 2.94 - 2.82 (m, 1H), 2.78 - 2.68 (m, 1H), 2.64 - 2.53 (m, 2H), 2.09 - 1.99 (m, 4H), 1.99 - 1.66 (m, 9H), 1.64 - 1.48 (m, 2H), 1.21 - 0.97 (m, 2H) |
| **I-440^{e}** | AON | ALS | 936.3 | 11.87 (s, 1H), 11.08 (s, 1H), 9.50 (d, J = 6.0 Hz, 1H), 8.78 (d, J = 7.8 Hz, 1H), 8.39 (d, J = 4.2 Hz, 1H), 8.25 (d, J = 5.6 Hz, 1H), 7.65 - 7.52 (m, 1H), 7.26 - 6.96 (m, 3H), 6.89 - 6.42 (m, 2H), 5.30 - 5.00 (m, 2H), 4.81 - 4.72 (m, 1H), 4.28 - 4.17 (m, 1H), 3.85 - 3.71 (m, 2H), 3.65 - 3.58 (m, 1H), 3.51 - 3.35 (m, 9H), 2.92 - 2.82 (m, 1H), 2.62 - 2.53 (m, 2H), 2.39 - 2.29 (m, 1H), 2.10 - 1.87 (m, 9H), 1.85 - 1.66 (m, 4H), 1.59 - 1.46 (m, 2H) |
| **I-445** | AZY | ALS | 929.4 | 11.15 (s, 1H), 9.50 (d, *J =* 4.0 Hz, 1H), 8.78 (, *J =* 8.0 Hz, 1H), 8.60 - 8.50 (m, 1H), 8.47 - 8.36 (m, 2H), 8.26 (d, *J* = 5.6 Hz, 1H), 7.25 - 7.38 (m, 2H), 7.34 - 7.26 (m, 1H), 7.25 - 6.96 (m, 2H), 6.89 - 6.43 (m, 1H), 6.15 - 5.93 (m, 1H), 5.30 - 5.04 (m, 1H), 4.76 (d, *J* = 20.0 Hz, 1H), 4.30 - 4.18 (m, 1H), 3.84 - 3.71 (m, 3H), 3.68 - 3.57 (m, 4H), 3.23 - 2.98 (m, 10H), 2.73 - 2.69 (m, 2H), 2.11 - 1.37 (m, 18H) |
| **I-447^{b}** | ANB | ALS | 929.1 | 11.15 (s, 1H), 9.50 (d, *J* = 6.4 Hz, 1H), 8.77 (dd, *J* = 7.6, 1.6 Hz, 1H), 8.39 (d, *J* = 4.8 Hz, 1H), 8.32 (d, *J* = 5.2 Hz, 1H), 8.27 - 8.18 (m, 3H), 7.72 - 7.57 (m, 1H), 7.53 (t, *J* = 7.6 Hz, 1H), 7.32 (t, *J* = 7.6 Hz, 1H), 7.10 (d, *J* = 5.2 Hz, 2H), 6.88 - 6.41 (m, 1H), 6.19 - 5.92 (m, 1H), 5.32 - 5.02 (m, 1H), 4.76 (d, *J =* 17.2 Hz, 1H), 4.27 - 4.23 (m, 1H), 3.83 - 3.80 (m, 2H), 3.73 (d, *J =* 7.6 Hz, 1H), 3.66 - 3.64 (m, 2H), 3.61 - 3.58 (m, 1H), 3.46 - 3.42 (m, 3H), 3.21 - 3.15 (m, 4H), 3.08 - 3.00 (m, 1H), 2.66 - 2.64 (m, 1H), 2.75 - 2.64 (m, 2H), 2.64 - 2.63 (m, 1H), 2.53 - 2.51 (m, 1H), 2.15 - 1.48 (m, 18H) |
| **I-449^{b}** | AND | ALS | 895.5 | 11.07 (s, 1H), 9.49 (d, *J* = 6.8 Hz, 1H), 8.77 (d, *J* = 7.6 Hz, 1H), 8.37 (d, *J* = 4.4 Hz, 1H), 8.25 (d, *J* = 5.6 Hz, 1H), 7.24 - 6.96 (m, 1H), 6.94 (d, *J* = 4.8 Hz, 2H), 6.88 - 6.86 (m, 1H), 6.85 - 6.43 (m, 1H), 5.35 (dd, *J* = 4.8, 12.8 Hz, 1H), 5.29 - 5.04 (m, 1H), 4.76 (d, *J =* 18.8 Hz, 1H), 4.29 - 4.19 (m, 1H), 3.77 - 3.69 (m, 3H), 3.54 (s, 3H), 2.95 - 2.89 (m, 3H), 2.89 - 2.79 (m, 4H), 2.75 - 2.68 (m, 4H), 2.64 - 2.60 (m, 4H), 2.07 - 1.91 (m, 7H), 1.87 - 1.69 (m, 8H), 1.58 - 1.50 (m, 2H) |
| **I-472^{b}** | ANH | ALS | 883.6 | 11.08 (s, 1H), 9.50 (d, *J* = 6.4 Hz, 1H), 8.78 (d, *J* = 7.6 Hz, 1H), 8.39 (d, *J* = 4.8 Hz, 1H), 8.25 (d, *J* = 5.6 Hz, 1H), 7.25 - 7.07 (m, 1H), 6.97 (d, *J* = 4.8 Hz, 2H), 6.91 - 6.43 (m, 2H), 5.36 (dd, *J* = 5.2, 12.4 Hz, 1H), 5.30 - 5.03 (m, 1H), 4.81 - 4.70 (m, 1H), 4.32 - 4.19 (m, 1H), 3.84 - 3.72 (m, 2H), 3.66 - 3.58 (m, 2H), 3.57 (s, 3H), 3.45 (d, *J* = 9.6 Hz, 2H), 3.40 - 3.35 (m, 6H), 2.98 - 2.82 (m, 4H), 2.77 - 2.68 (m, 2H), 2.66 - 2.62 (m, 1H), 2.59 - 2.55 (d, *J* = 7.6 Hz, 1H), 2.09 - 1.93 (m, 5H), 1.93 - 1.76 (m, 4H), 1.71 - 1.47 (m, 6H) |
| **I-480^{b}** | AOD | ATA | 925.3 | 11.08 (s, 1H), 9.28 (s, 1H), 8.81 (d, *J* = 8.0 Hz, 1H), 8.40 (s, 1H), 8.28 (s, 1H), 7.33 - 7.00 (m, 1H), 6.99 - 6.90 (m, 3H), 6.89 - 6.83 (m, 1H), 5.45 - 5.30 (m, 1H), 4.69 - 4.29 (m, 2H), 4.28 - 4.20 (m, 1H), 3.65 - 3.63 (m, 2H), 3.57 (s, 3H), 3.33 - 3.25 (m, 2H), 3.14 - 3.07 (m, 4H), 2.94 - 2.86 (m, 3H), 2.75 - 2.57 (m, 8H), 2.03 - 2.00 (m, 3H), 2.10 - 1.47 (m, 13H), 1.20 (s, 3H), 1.18 (s, 3H). |
| **I-496^{b}** | AOJ | ALS | 909.4 | 1.47 - 1.69 (m, 7 H), 1.71 - 1.94 (m, 1 H), 1.72 - 1.93 (m, 7 H), 1.94 - 2.10 (m, 7 H), 2.29 - 2.38 (m, 2 H), 2.59 - 2.72 (m, 4 H), 2.88 - 2.98 (m, 3 H), 3.46 (d, *J* = 10.4 Hz, 1 H), 3.57 (s, 3 H), 3.60 - 3.66 (m, 1 H), 3.71 - 3.90 (m, 2 H), 4.18 - 4.66 (m, 3 H), 4.78 (d, *J* = 12.0 Hz, 1 H), 5.05 - 5.31 (m, 1 H), 5.37 (dd, *J* = 12.0, 5.2 Hz, 1 H), 6.43 - 6.92 (m, 2 H), 6.94 - 7.28 (m, 3 H), 8.14 (s, 1 H), 8.27 (d, *J* = 5.6 Hz, 1 H), 8.40 (d, *J =* 4.6 Hz, 1 H), 8.79 (d, *J* = 7.2 Hz, 1 H), 9.51 (d, *J=* 6.8 Hz, 1 H), 11.09 (s, 1 H) |
| **I-499^{b}** | AOD | ARR | 853.5 | 11.08 (s, 1H), 9.35 (dd, *J =* 1.6, 6.8 Hz, 1H), 9.12 (dd, *J =* 1.6, 4.0 Hz, 1H), 8.71 - 8.66 (m, 2H), 8.48 (s, 1H), 7.86 (d, *J* = 8.0 Hz, 1H), 7.72 (dd, *J* = 1.6, 8.4 Hz, 1H), 7.37 - 7.30 (m, 1H), 6.96 (d, *J* = 4.4 Hz, 2H), 6.91 - 6.83 (m, 1H), 5.36 (dd, *J* = 5.6, 12.4 Hz, 1H), 4.27 (tt, *J =* 3.6, 11.6 Hz, 1H), 3.57 (s, 3H), 3.47 - 3.45 (m, 4H), 3.13 - 3.05 (m, 4H), 2.95 - 2.87 (m, 3H), 2.87 - 2.77 (m, 1H), 2.76 - 2.70 (m, 1H), 2.67 - 2.63 (m, 1H), 2.62 - 2.56 (m, 2H), 2.40 (s, 3H), 2.15 - 2.07 (m, 2H), 2.03 - 1.88 (m, 3H), 1.84 - 1.76 (m, 2H), 1.72 - 1.56 (m, 8H) |

| | | | | |
|---|---|---|---|---|
| ^{a} Variations in reaction time for Method 1 were as follows: Step 1 was run anywhere from 0.5-12 h, and Step 2 anywhere from 10 min-17 h. If the product of Step 1 was not a precipitate, a standard work up with water and extraction with ethyl acetate was used to isolate the product. Step 2 deprotection could also be achieved under a variety of standard conditions, including with TFA in DCM at rt. The final products were isolated under standard purification techniques including reverse HPLC, silica gel chromatography, and prep-TLC with appropriate solvent conditions. ^{b} No Step 2 deprotection required. ^{c} Step 2 deprotection was achieved with HBr/HOAc in DCM at rt for 12 h. ^{d} PyBOP and DMAP with DIPEA in DMF at 80 °C for 16 hrs was used for the coupling in Step 1. ^{e} The acid was coupled with CDI in ACN at rt for 3 hr, then the amine was added with DBU and the reaction was stirred for 2 hr at rt. | | | | |

### Example 2 (Method 2). Synthesis of 2-[2-(Cyclopropylmethylamino)-4-pyridyl]-N-[3-(difluoromethyl)-1-[4-[[3-[3-[1-(2,6-dioxo -3-piperidyl)-3-methyl-2-oxo-benzimidazol-4-yl]propoxy]propyl-methyl-amino]methyl]cyclohexyl]pyrazol-4-yl]oxazole-4-carboxamide (I-30)

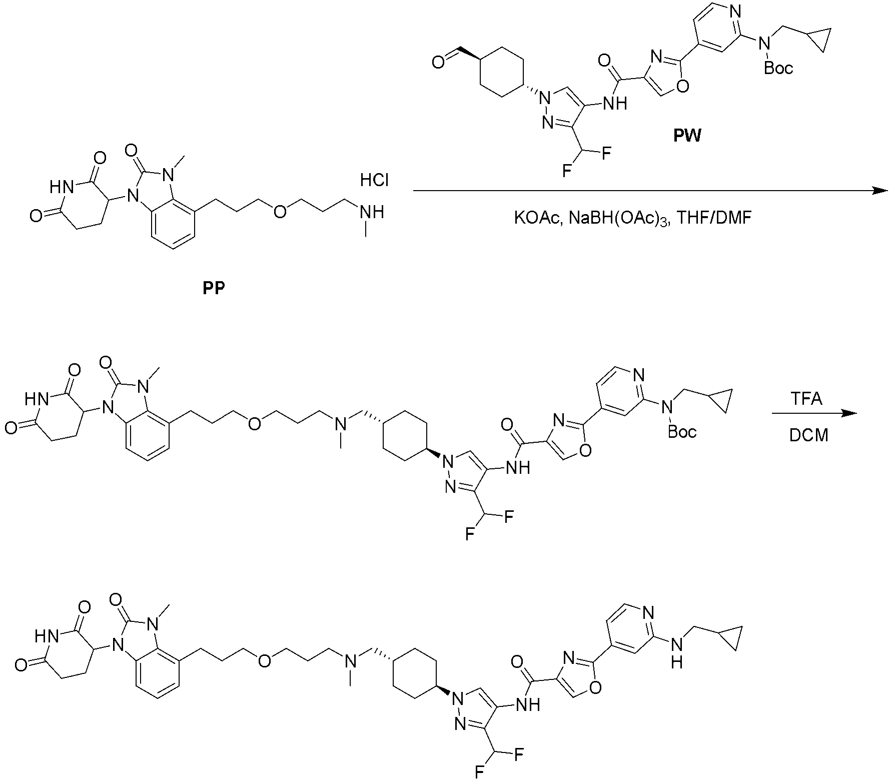

### Step 1 - Tert-butyl N-(cyclopropylmethyl)-N-[4-[4-[[3-(difluoromethyl)-1-[4-[[3-[3-[1-(2,6-dioxo-3- piperidyl)-3-methyl-2-oxo-benzimidazol-4-yl]propoxylpropyl-methyl-amino]methyl]cyclohexyl]pyrazol-4-yl]carbamoyl]oxazol-2-yl]-2-pyridyl]carbamate

To a solution of 3-[3-methyl-4-[3-[3-(methylamino)propoxy]propyl]-2-oxo-benzimidazol-1-yl] piperidine-2,6-dione (45.0 mg, 106 umol, HCl, Intermediate PP) and tert-butyl N-(cyclopropylmethyl) -N-[4-[4-[[3-(difluoromethyl)-1-(4-formylcyclohexyl)pyrazol-4-yl]carbamoyl]oxazol-2-yl]-2-pyridyl]carbamate (61.9 mg, 106 umol, Intermediate PW) in a mixed solvent of DMF (2 mL) and THF (10 mL) was added KOAc (20.8 mg, 212 umol). Thirty minutes later, NaBH(OAc)₃ (44.9 mg, 212 umol) was added into the above mixture and the reaction mixture was stirred at 25 °C for 12 hours. On completion, the mixture was concentrated *in vacuo*. The residue was purified by reverse phase (0.1% FA) to give the title compound (50.0 mg, 49% yield) as a white solid. LC-MS (ESI⁺) *m*/*z* 957.6 (M+H)⁺.

### Step 2 - 2-[2-(Cyclopropylmethylamino)-4-pyridyl]-N-[3-(difluoromethyl)-1-[4-[[3-[3-[1-(2,6-dioxo -3-piperidyl)-3-methyl-2-oxo-benzimidazol-4-yl]propoxylpropyl-methyl-amino]methyl]cyclohexyl]pyrazol-4-yl]oxazole-4-carboxamide

To a solution of tert-butyl N-(cyclopropylmethyl)-N-[4-[4-[[3-(difluoromethyl)-1-[4-[[3-[3-[1-(2,6- dioxo-3-piperidyl)-3-methyl-2-oxo-benzimidazol-4-yl]propoxy]propyl-methyl-amino]methyl]cyclohexyl]pyrazol-4-yl]carbamoyl]oxazol-2-yl]-2-pyridyl]carbamate (48.0 mg, 50.2 umol) in TFA (3 mL) was added DCM (3 mL). The reaction mixture was stirred at 25 °C for 2 hrs. On completion, the mixture was concentrated *in vacuo*. The residue was purified by prep-HPLC (column: Phenomenex Synergi C18 150*25*10 um; mobile phase: [water (0.225%FA)-ACN]; B%: 10%-37%, 9 min) to give the title compound (35.0 mg, 77% yield) as a white solid. ¹H NMR (400 MHz, DMSO-*d₆*) δ 11.09 (s, 1H), 9.71 (s, 1H), 8.91 (s, 1H), 8.17 - 8.13 (m, 2H), 7.29 - 7.05 (m, 3H), 7.03 - 7.00 (m, 1H), 6.96 (d, *J*= 4.8 Hz, 2H), 6.89 - 6.84 (m, 1H), 5.35 (dd, *J* = 5.2, 12.4 Hz, 1H), 4.25 - 4.13 (m, 1H), 3.56 (s, 3H), 3.43 -3.38 (m, 10H), 3.17 (t, *J* = 6.0 Hz, 2H), 2.99 - 2.92 (m, 2H), 2.90 - 2.82 (m, 1H), 2.76 - 2.53 (m, 4H), 2.40 (t, *J* = 6.8 Hz, 2H), 2.22 - 2.19 (m, 1H), 2.18 - 2.16 (m, 3H), 2.17 - 2.14 (m, 1H), 2.07 - 1.94 (m, 3H), 1.59 - 1.46 (m, 1H), 1.12 - 1.05 (m, 1H), 1.05 - 0.95 (m, 2H), 0.48 - 0.41 (m, 2H), 0.25 - 0.18 (m, 2H); LC-MS (ESI⁺) *m*/*z* 857.5 (M+H)⁺.

**Table 7: Compounds synthesized via Method 2 with the reductive amination of various amines and aldehydes in Step 1.**

| **I-#** | **Step 1 Intermediate Amine** | **Step 1 Intermediate Aldehyde** | **LCMS (ES+) m/z (M+H)⁺** | **¹HNMR (400MHz, DMSO-d₆) δ** |
|---|---|---|---|---|
| **I-2** | HQ | PW | 873.4 | 11.50 - 10.71 (m, 1H), 9.72 (s, 1H), 8.90 (s, 1H), 8.30 (s, 1H), 8.19 - 8.09 (m, 2H), 7.32 - 7.12 (m, 1H), 7.12 - 7.05 (m, 2H), 7.04 - 6.99 (m, 1H), 6.98 - 6.915 (m, 2H), 6.88 (t, *J* = 4.4 Hz, 1H), 5.41 - 5.29 (m, 1H), 4.22 - 4.09 (m, 1H), 3.62 - 3.56 (m, 11H), 3.21 - 3.13 (m, 2H), 3.00 - 2.92 (m, 2H), 2.90 - 2.80 (m, 3H), 2.69 - 2.56 (m, 4H), 2.06 - 1.94 (m, 3H), 1.93 - 1.78 (m, 4H), 1.76 - 1.63 (m, 2H), 1.63 - 1.48 (m, 1H), 1.17 - 0.97 (m, 3H), 0.50 - 0.40 (m, 2H), 0.30 - 0.12 (m, 2H) |
| **I-3** | PA | PW | 860.4 | 9.70 (s, 1H), 8.93 (s, 1H), 8.23 - 8.06 (m, 2H), 7.31 - 7.14 (m, 3H), 7.12 - 6.98 (m, 4H), 5.35 (d, *J =* 5.2, 13.2 Hz, 1H), 4.34 - 4.07 (m, 1H), 3.58 - 3.50 (m, 12H), 3.19 (t, *J =* 6.0 Hz, 2H), 3.18 - 3.17 (m, 1H), 2.77 (t, *J =* 5.6 Hz, 2H), 2.71 - 2.60 (m, 4H), 2.19 - 2.10 (m, 1H), 2.04 - 2.02 (m 2H), 1.96 - 1.65 (m, 6H), 1.59 - 1.46 (m, 1H), 1.17 - 1.01 (m, 3H), 0.50 - 0.41 (m, 2H), 0.26 - 0.19 (m, 2H) |
| **I-6** | HQ | RF | 894.4 | 11.0 (s, 1H), 10.9 (s, 1H), 8.96 (s, 1H), 8.34 (s, 1H), 8.24 (d, *J* = 5.2 Hz, 1H), 7.70 - 7.65 (m, 2H), 7.49 (s, 1H), 7.25 (s, 1H), 7.16 (d, *J* = 5.2 Hz, 1H), 6.96 (d, *J* = 4.4 Hz, 2H), 6.88 - 6.86 (m, 1H), 5.40 - 5.31 (m, 1H), 4.29 - 4.21 (m, 2H), 4.20 - 4.13 (m, 1H), 3.57 (s, 3H), 3.55 - 3.48 (m, 9H), 3.00 - 2.94 (m,2H), 2.86 (d, *J* = 5.2, 16.4 Hz, 1H), 2.72 (t, *J* = 5.6 Hz, 2H), 2.61 (d,*J* = 16.4 Hz, 2H), 2.46 - 2.43(m, 2H), 2.07 - 2.00 (m, 2H), 1.99 - 1.94 (m, 1H), 1.92 - 1.79 (m, 4H), 1.74 (d, *J* = 9.6 Hz, 2H), 1.53 - 1.42 (m, 1H), 1.13 - 1.00 (m, 2H) |
| **I-7** | PA | RF | 860.4 | 9.70 (s, 1H), 8.93 (s, 1H), 8.23 - 8.06 (m, 2H), 7.31 - 7.14 (m, 3H), 7.12 - 6.98 (m, 4H), 5.35 (m, 1H), 4.34 - 4.07 (m, 1H), 3.58 - 3.50 (m, 12H), 3.19 (m, 2H), 2.77 (m, 2H), 2.71 - 2.60 (m, 4H), 2.19 - 2.10 (m, 1H), 2.04 (m, 2H), 1.96 - 1.65 (m, 6H), 1.59 - 1.46 (m, 1H), 1.17 - 1.01 (m, 3H), 0.50 - 0.41 (m, 2H), 0.26 - 0.19 (m, 2H) |
| **I-8** | RH | RF | 850.1 | 11.08 (s, 1H), 10.94 (s, 1H), 8.98 (s, 1H), 8.82 (s, 1H), 8.37 (s, 1H), 8.24 (d, *J =* 5.2 Hz, 1H), 8.05 (s, 1H), 7.68 (s, 1H), 7.51 (s, 1H), 7.34 (s, 1H), 7.21 (dd, *J =* 1.2, 5.6 Hz, 1H), 7.08 - 6.96 (m, 2H), 6.89 (dd, *J =* 1.2, 8.0 Hz, 1H), 5.34 (dd, *J* = 5.6, 13.2 Hz, 1H), 4.37 - 4.19 (m, 3H), 3.69 (t, *J* = 5.2 Hz, 2H), 3.45 - 3.43 (m, 2H), 3.32 (s, 3H), 3.16 - 3.08 (m, 2H), 2.93 - 2.79 (m, 3H), 2.73 - 2.60 (m, 4H), 2.09 (d, *J* = 10.4 Hz, 2H), 2.10 -1.95 (m, 3H), 1.91 - 1.76 (m, 5H), 1.27 - 1.11 (m, 2H) |
| **I-9** | OD | RF | 892.5 | 10.93 (s, 1H), 8.96 - 8.93 (m, 1H), 8.36 (s, 1H), 8.24 (d, *J =* 5.2 Hz, 1H), 7.71 - 7.62 (m, 2H), 7.48 (s, 1H), 7.24 (s, 1H), 7.18 - 7.14 (m, 1H), 7.05 - 6.97 (m, 2H), 6.89 - 6.83 (m, 1H), 5.35 - 5.29 (m, 1H), 4.28 - 4.18 (m, 3H), 3.33 - 3.31 (m, 7H), 2.95 - 2.84 (m, 1H), 2.75 - 2.56 (m, 8H), 2.07 (d, *J =* 10.0 Hz, 2H), 2.03 - 1.96 (m, 1H), 1.90 (d, *J =* 11.6 Hz, 2H), 1.84 - 1.72 (m, 2H), 1.65 - 1.59 (m, 2H), 1.57 - 1.47 (m, 7H), 1.18 - 0.99 (m, 2H) |
| **I-10** | LF | RF | 864.5 | 10.95 (s, 1H), 8.97 (s, 1H), 8.37 (s, 1H), 8.30 (s, 1H), 8.25 (d, *J* = 5.6 Hz, 1H), 7.74 - 7.64 (m, 2H), 7.50 (s, 1H), 7.25 (s, 1H), 7.16 (dd, *J* = 1.6, 5.2 Hz, 1H), 6.97 (d, *J* = 4.8 Hz, 2H), 6.90 - 6.81 (m, 1H), 5.37 (dd, *J* = 5.2, 12.8 Hz, 1H), 4.29 - 4.19 (m, 3H), 3.58 (s, 3H), 3.48 - 3.46 (m, 4H), 3.01 - 2.83 (m, 4H), 2.77 - 2.58 (m, 6H), 2.37 - 2.31 (m, 1H), 2.13 - 2.03 (m, 2H), 1.96 - 1.92 (m, 3H), 1.88 - 1.80 (m, 3H), 1.79 - 1.69 (m, 3H), 1.19 - 1.05 (m, 2H) |
| **I-11** | RI | RF | 850.4 | 11.07 (s, 1H), 10.93 (s, 1H), 8.94 (s, 1H), 8.36 (s, 1H), 8.24 (d, *J =* 5.2 Hz, 1H), 7.70 - 7.62 (m, 2H), 7.48 (s, 1H), 7.25 (s, 1H), 7.16 (d, *J* = 5.2 Hz, 1H), 6.97 (d, *J* = 4.4 Hz, 2H), 6.91 - 6.84 (m, 1H), 5.38 - 5.32 (m, 1H), 4.30 - 4.16 (m, 3H), 3.57 (s, 3H), 3.51 - 3.48 (m, 4H), 2.97 (t, *J* = 7.6 Hz, 2H), 2.92 - 2.82 (m, 1H), 2.76 (t, *J* = 5.2 Hz, 2H), 2.72 - 2.63 (m, 2H), 2.61 - 2.54 (m, 2H), 2.07 (d, *J* = 10.0 Hz, 2H), 2.03 - 1.97 (m, 1H), 1.96 - 1.89 (m, 2H), 1.87 - 1.76 (m, 4H), 1.61 - 1.45 (m, 1H), 1.20 - 1.01 (m, 2H) |
| **I-12** | PY | RF | 851.4 | 10.94 (s, 1H), 8.96 (s, 1H), 8.36 (s, 1H), 8.24 (d, *J* = 5.2 Hz, 1H), 7.71 - 7.64 (m, 2H), 7.50 (s, 1H), 7.24 (s, 1H), 7.17 - 7.08 (m, 3H), 7.03 (d, *J* = 7.6 Hz, 1H), 5.39 - 5.32 (m, 1H), 4.29 - 4.17 (m, 3H), 3.61 - 3.52 (m, 4H), 2.90 - 2.83 (m, 1H), 2.79 - 2.64 (m, 8H), 2.17 - 2.11 (m, 1H), 2.10 - 2.03 (m, 2H), 1.95 - 1.89 (m, 2H), 1.88 - 1.83 (m, 2H), 1.80 - 1.77 (m, 1H), 1.75 - 1.71 (m, 2H), 1.65 - 1.48 (m, 2H), 1.17 - 1.03 (m, 2H) |
| **I-16** | RK | PW | 884.3 | 11.09 (s, 1H), 9.72 (s, 1H), 8.91 (s, 1H), 8.25 (s, 2H), 8.18 (s, 1H), 8.14 (d, *J* = 5.2 Hz, 1H), 7.15 - 7.09 (m, 2H), 7.06 - 6.96 (m, 3H), 6.87 (d, *J =* 8.0 Hz, 1H), 5.34 - 5.30 (m, 1H), 4.20 (t, *J* = 12.0 Hz, 1H), 3.80 (d, *J* = 10.8 Hz, 2H), 3.32 (s, 3H), 3.17 (t, *J =* 6.0 Hz, 2H), 2.93 - 2.86 (m, 1H), 2.80 (d, *J* = 11.2 Hz, 1H), 2.75 - 2.61 (m, 8H), 2.53 - 2.52 (m, 2H), 2.29 (t, *J* = 6.8 Hz, 2H), 2.07 (s, 1H), 2.05 - 1.94 (m, 4H), 1.89 (s, 2H), 1.81 - 1.67 (m, 5H), 1.58 (s, 2H), 1.16 - 0.99 (m, 3H), 0.48 - 0.42 (m, 2H), 0.25 - 0.18 (m, 2H) |
| **I-33** | SZ | UW | 906.3 | 11.09 (s, 1H), 10.38 (s, 1H), 8.90 - 8.83 (m, 1H), 8.17 (s, 1H), 8.15 (d, *J* = 5.6 Hz, 1H), 7.12 (t, *J* = 5.2 Hz, 1H), 7.07 (s, 1H), 7.04 (s, 1H), 7.02 - 6.96 (m, 2H), 6.88 (d, *J* = 7.6 Hz, 1H), 6.36 - 5.55 (m, 1H), 5.33 (dd, *J* = 5.2, 12.8 Hz, 1H), 4.11 - 3.98 (m, 2H), 3.77 - 3.74 (m, 2H), 3.31 (s, 3H), 3.18 (t, *J* = 6.0 Hz, 2H), 2.90 - 2.80 (m, 2H), 2.71 - 2.61 (m, 4H), 2.35 - 2.27 (m, 4H), 2.18 (s, 3H), 2.16 - 2.07 (m, 2H), 2.04 - 1.63 (m, 12H), 1.60 - 1.53 (m, 1H), 1.50 (s, 6H), 1.12 - 0.92 (m, 3H), 0.51 - 0.41 (m, 2H), 0.27 - 0.16 (m, 2H) |
| **I-34** | RR | PW | 898.6 | 11.08 (s, 1H), 9.72 (s, 1H), 8.92 (s, 1H), 8.19 (s, 1H), 8.16 - 8.14 (m, 1H), 7.30 - 7.07 (m, 3H), 7.05 (s, 1H), 7.01 (d, *J* = 7.2 Hz, 2H), 6.89 (d, *J* = 8.0 Hz, 1H), 5.37 - 5.31 (m, 1H), 4.28 - 4.18 (m, 1H), 3.90 (d, *J =* 9.6 Hz, 2H), 3.65 (t, *J* = 10.8 Hz, 1H), 3.46 - 3.37 (m, 3H), 3.18 (t, *J =* 6.0 Hz, 2H), 3.07 - 2.84 (m, 6H), 2.75 - 2.54 (m, 12H), 2.06 (d, *J* = 10.0 Hz, 2H), 2.10 - 1.97 (m, 1H), 1.91 - 1.75 (m, 7H), 1.21 - 1.09 (m, 2H), 1.08 - 1.03 (m, 1H), 0.48 - 0.42 (m, 2H), 0.24 - 0.20 (m, 2H) |
| **I-35** | RJ | PW | 884.5 | 11.08 (s, 1H), 9.72 (s, 1H), 8.92 (s, 1H), 8.86 - 8.48 (m, 1H), 8.20 (s, 1H), 8.16 - 8.13 (m, 1H), 7.31 - 7.01 (m, 6H), 6.90 (d, *J =* 8.0 Hz, 1H), 5.38 - 5.31 (m, 1H), 4.28 - 4.18 (m, 1H), 3.97 (d, *J* = 1.2 Hz, 2H), 3.67 (s, 1H), 3.50 (s, 3H), 3.20 - 3.16 (m, 2H), 3.11 (s, 1H), 3.02 - 2.98 (m, 1H), 2.97 - 2.93 (m, 1H), 2.92 - 2.90 (m, 1H), 2.89 - 2.84 (m, 2H), 2.71 (t, *J =* 4.4 Hz, 1H), 2.69 - 2.65 (m, 3H), 2.62 - 2.58 (m, 2H), 2.54 (s, 2H), 2.12 - 2.06 (m, 2H), 2.04 - 1.96 (m, 2H), 1.94 - 1.85 (m, 3H), 1.81 - 1.69 (m, 3H), 1.68 - 1.56 (m, 1H), 1.24 - 1.11 (m, 2H), 1.10 - 1.01 (m, 1H), 0.49 - 0.43 (m, 2H), 0.25 - 0.19 (m, 2H) |
| **I-36** | SZ | PW | 898.5 | 11.07 (s, 1H), 9.70 (s, 1H), 8.92 (s, 1H), 8.23 - 8.10 (m, 3H), 7.15 (t, J = 54 Hz, 1H), 7.10 (s, 1H), 7.05 - 6.97 (m, 3H), 6.92 - 6.87 (m, 1H), 5.38 - 5.26 (m, 1H), 4.30 - 4.13 (m, 1H), 3.76 (d, *J* = 10.8 Hz, 2H), 3.50 - 3.49 (m, 1H), 3.19 (s, 1H), 3.17 (s, 3H), 2.93 - 2.76 (m, 4H), 2.62 (m, 3H), 2.40 - 2.34 (m, 3H), 2.29 (s, 2H), 2.19 (s, 3H), 2.06 - 1.87 (m, 6H), 1.83 - 1.66 (m, 6H), 1.61 - 1.47 (m, 2H), 1.11 - 0.92 (m, 3H), 0.46 - 0.44 (m, 2H), 0.22 (m, 2H) |
| **I-37** | SQ | PW | 884.2 | 11.09 (s, 1H), 9.70 (s, 1H), 8.92 (s, 1H), 8.24 (s, 2H), 8.15 (d, *J* = 5.2 Hz, 1H), 7.30 - 7.06 (m, 3H), 7.03 - 7.00 (m, 1H), 6.99 - 6.94 (m, 2H), 6.91 - 6.87(m, 1H), 5.36 (dd, *J* = 5.2, 12.4 Hz, 1H), 4.27 - 4.15 (m, 1H), 3.80 (d, *J* = 10.8 Hz, 1H), 3.57 (s, 3H), 3.50 (m, 1H), 3.18 (t, *J* = 6.0 Hz, 2H), 2.95 - 2.89 (m, 2H), 2.86 - 2.81(m, 1H), 2.72 - 2.64 (m, 4H), 2.60 - 2.52 (m, 4H), 2.10 - 1.87 (m, 7H), 1.83 - 1.68 (m, 6H), 1.65 - 1.49 (m, 2H), 1.16 - 1.03 (m, 3H), 0.48 - 0.42 (m, 2H), 0.22 (q, *J* = 4.8 Hz, 2H) |
| **I-38** | SR | PW | 884.6 | 11.11 (s, 1H), 9.73 (s, 1H), 8.92 (s, 1H), 8.19 (s, 1H), 8.14 (d, *J =* 5.2 Hz, 1H), 7.32 - 7.02 (m, 3H), 7.01 (dd, *J* = 1.2, 5.2 Hz, 1H), 6.96 (d, *J* = 4.8 Hz, 2H), 6.92 - 6.85 (m, 1H), 5.37 (dd, *J* = 5.2, 12.4 Hz, 1H), 4.25 - 4.19 (m, 1H), 3.83 - 3.82 (m, 1H), 3.57 (s, 3H), 3.53 - 3.45 (m, 2H), 3.17 (t, *J* = 6.0 Hz, 2H), 2.96 - 2.81 (m, 4H), 2.76 - 2.54 (m, 7H), 2.43 - 2.27 (m, 2H), 2.09 - 1.96 (m, 4H), 1.94 - 1.85 (m, 2H), 1.82 - 1.70 (m, 5H), 1.67 - 1.50 (m, 2H), 1.20 - 0.97 (m, 3H), 0.49 - 0.42 (m, 2H), 0.25 - 0.19 (m, 2H) |
| **I-39** | SM | PW | 895.5 | 9.69 (s, 1H), 8.92 (s, 1H), 8.23 (s, 1H), 8.17 (s, 1H), 8.15 (d, *J =* 5.2 Hz, 1H), 7.54 (s, 1H), 7.51 - 7.43 (m, 3H), 7.32 - 7.05 (m, 3H), 7.04 - 6.99 (m, 1H), 5.71 - 5.60 (m, 1H), 4.74 - 4.62 (m, 1H), 4.24 - 4.14 (m, 1H), 3.94 (t, *J* = 8.8 Hz, 1H), 3.91 - 3.82 (m, 2H), 3.55 (s, 2H), 3.27 - 3.24 (m, 2H), 3.19 - 3.16 (m, 2H), 2.84 (d, *J* = 12.0 Hz, 2H), 2.71 (s, 1H), 2.66 - 2.58 (m, 6H), 2.30 - 2.14 (m, 2H), 2.10 - 1.97 (m, 4H), 1.92 - 1.88 (m, 1H), 1.79 - 1.67 (m, 2H), 1.63 - 1.44 (m, 2H), 1.17 - 1.00 (m, 3H), 0.48 - 0.41 (m, 2H), 0.25 - 0.20 (m, 2H) |
| **I-40** | SO | PW | 895.4 | 10.98 (s, 1H), 9.69 (s, 1H), 8.92 (s, 1H), 8.23 - 8.17 (m, 2H), 8.15 (d, *J =* 5.2 Hz, 1H), 7.57 - 7.14 (m, 5H), 7.11 - 7.00 (m, 3H), 5.73 - 5.60 (m, 1H), 4.74 - 4.61 (m, 1H), 4.25 - 4.15 (m, 1H), 3.93 (t, *J =* 8.8 Hz, 1H), 3.90 - 3.80 (m, 2H), 3.56 (s, 2H), 3.43 - 3.38 (m, 2H), 3.24 (t, *J* = 8.0 Hz, 2H), 3.18 (t, *J* = 6.0 Hz, 2H), 2.86 - 2.82 (m, 1H), 2.76 - 2.71 (m, 2H), 2.56 - 2.54 (m, 2H), 2.25 - 2.18 (m, 1H), 2.10 - 1.81 (m, 7H), 1.80 - 1.68 (m, 2H), 1.67 - 1.44 (m, 2H), 1.17 - 0.99 (m, 3H), 0.49 - 0.42 (m, 2H), 0.27 - 0.18 (m, 2H) |
| **I-41** | TC | TE | 902.5 | 9.39 (s, 1H), 8.77 (d, *J* = 8.4 Hz, 1H), 8.40 - 8.36 (m, 1H), 8.28 - 8.24 (m, 1H), 8.23 - 8.19 (m, 3H), 7.14 - 7.04 (m, 2H), 7.00 (d, *J* = 7.6 Hz, 1H), 6.93 - 6.86 (m, 2H), 5.38 - 5.29 (m, 1H), 4.24 - 4.12 (m, 2H), 3.75 (m, 4H), 3.63 - 3.61 (m, 4H), 3.32 (s, 3H), 2.94 - 2.88 (m, 2H), 2.84 (d, *J =* 6.4 Hz, 6H), 2.79 (d, *J =* 12.8 Hz, 2H), 2.71 (d, *J* = 4.4 Hz, 1H), 2.65 - 2.59 (m, 4H), 2.46 - 2.43 (m, 2H), 2.09 - 1.97 (m, 4H), 1.88 (m, 3.7 Hz, 2H), 1.83 - 1.65 (m, 4H), 1.62 - 1.48 (m, 2H), 1.16 - 1.01 (m, 2H) |
| **I-42** | OD | TE | 859.5 | 11.79 - 10.37 (m, 1H), 9.40 (s, 1H), 8.77 (d, *J* = 8.0 Hz, 1H), 8.28 (d, *J =* 18.0 Hz, 2H), 7.25 - 6.95 (m, 3H), 6.92 - 6.82 (m, 2H), 5.33 (d, *J* = 5.2, 12.8 Hz, 1H), 4.26 - 4.13 (m, 1H), 3.75 (s, 5H), 3.40 - 3.34 (m, 5H), 3.32 (s, 3H), 2.91 - 2.75 (m, 7H), 2.72 - 2.57 (m, 6H), 2.11 - 1.95 (m, 3H), 1.90 (d, *J* = 12.0 Hz, 2H), 1.79 - 1.45 (m, 11H), 1.20 - 1.05 (m, 2H) |
| **I-43** | TF | PW | 874.5 | 11.20 (s, 1H), 9.96 (s, 1H), 9.55 - 9.17 (m, 1H), 9.12 (s, 1H), 8.16 (s, 1H), 8.07 (d, *J =* 6.4 Hz, 1H), 7.65 (s, 1H), 7.35 - 6.94 (m, 5H), 5.38 (dd, *J* = 5.2, 12.8 Hz, 1H), 4.27 - 4.16 (m, 1H), 3.92 - 3.75 (m, 3H), 3.58 (s, 3H), 3.32 - 3.28 (m, 4H), 3.15 - 3.01 (m, 2H), 2.99 - 2.85 (m, 2H), 2.80 (s, 3H), 2.73 - 2.57 (m, 4H), 2.18 - 2.00 (m, 4H), 1.97 - 1.86 (m, 2H), 1.85 - 1.72 (m, 4H), 1.71 - 1.55 (m, 1H), 1.24 - 1.06 (m, 3H), 0.59 - 0.51 (m, 2H), 0.27 - 0.36 (m, 2H) |
| **I-44^{b}** | PP | TG | 789.2 | 11.09 (s, 1H), 10.10 (s, 1H), 8.42 - 8.27 (m, 3H), 8.22 - 8.18 (m, 1H), 7.16 (t, *J* = 12.4 Hz 1H), 6.99 - 6.92 (m, 2H), 6.90 - 6.82 (m, 1H), 5.36 (dd, *J* = 5.2, 12.4 Hz, 1H), 4.25 - 4.18 (m, 1H), 3.57 (s, 3H), 3.47 - 3.36 (m, 4H), 3.01 - 2.92 (m, 2H), 2.92 - 2.83 (m, 1H), 2.75 - 2.57 (m, 2H), 2.39 (t, *J* = 7.2 Hz, 2H), 2.21 - 2.11 (m, 5H), 2.08 - 1.95 (m, 3H), 1.95 - 1.86 (m, 2H), 1.86 - 1.73 (m, 4H), 1.72 - 1.63 (m, 2H), 1.61 - 1.51 (m, 1H), 1.12 - 0.95 (m, 2H) |
| **I-46** | TH | PW | 877.5 | 11.15 (s, 1H), 9.72 (s, 1H), 8.92 (s, 1H), 8.52 (dd, *J*= 1.6, 7.6 Hz, 1H), 8.40 (dd, *J* = 1.6, 4.8 Hz, 1H), 8.20 - 8.13 (m, 2H), 8.05 (s, 1H), 7.51 (s, 1H), 7.36 (d, *J =* 7.6 Hz, 1H), 7.31 - 6.97 (m, 5H), 6.02 (s, 1H), 4.23 - 4.17 (m, 1H), 3.43 (q, *J =* 6.8 Hz, 4H), 3.18 (t, *J* = 6.0 Hz, 2H), 3.09 - 2.95 (m, 2H), 2.84 - 2.65 (m, 4H), 2.60 - 2.53 (m, 3H), 2.46 (s, 3H), 2.14 - 2.00 (m, 3H), 1.98 - 1.85 (m, 4H), 1.84 - 1.62 (m, 5H), 1.21 - 0.98 (m, 3H), 0.51 - 0.39 (m, 2H), 0.31 - 0.16 (m, 2H) |
| **I-47** | TL | PW | 893.5 | 11.14 (s, 1H), 9.72 (s, 1H), 8.92 (s, 1H), 8.52 (dd, *J*= 1.2, 7.6 Hz, 1H), 8.40 (dd, *J* = 1.2, 4.8 Hz, 1H), 8.20 - 8.12 (m, 2H), 8.06 (s, 1H), 7.51 (s, 1H), 7.37 (d, *J =* 8.0 Hz, 1H), 7.29 - 7.01 (m, 5H), 6.01 (s, 1H), 4.21 - 4.15 (m, 1H), 3.63 - 3.52 (m, 7H), 3.45 (t, *J* = 6.4 Hz, 2H), 3.18 (t, *J* = 6.0 Hz, 2H), 3.07 - 2.95 (m, 2H), 2.91 (t, *J* = 5.2 Hz, 2H), 2.80 (t, *J* = 7.2 Hz, 2H), 2.69 - 2.61 (m, 3H), 2.13 - 2.00 (m, 3H), 1.96 - 1.84 (m, 4H), 1.79 - 1.66 (m, 2H), 1.62 - 1.52 (m, 1H), 1.18 - 1.02 (m, 3H), 0.50 - 0.41 (m, 2H), 0.23 - 0.20 (m, 2H) |
| **I-48** | TN | PW | 869.5 | 11.11 (s, 1H), 9.79 (s, 1H), 9.02 (s, 1H), 8.59 (d,*J* = 5.2 Hz, 1H), 8.30 (s, 1H), 8.18 (d, *J =* 1.6 Hz, 1H), 7.69 (dd, *J* = 1.6, 5.2 Hz, 1H), 7.32 - 7.01 (m, 1H), 6.97 (d, *J =* 4.9 Hz, 2H), 6.91 - 6.85 (m, 1H), 5.37 (dd, *J* = 5.2, 12.8 Hz, 1H), 4.27 - 4.14 (m, 1H), 3.87 (d, *J =* 6.8 Hz, 2H), 3.58 (s, 3H), 3.48 - 3.45 (m, 2H), 3.33 - 3.26 (m, 2H), 3.00 - 2.94 (m, 2H), 2.94 - 2.84 (m, 1H), 2.79 - 2.71 (m, 1H), 2.70 - 2.66 (m, 2H), 2.65 - 2.60 (m, 1H), 2.15 - 2.11 (m, 2H), 2.10 - 1.96 (m, 5H), 1.91 - 1.72 (m, 8H), 1.52 (s, 1H), 1.46 (br d, *J =* 9.8 Hz, 2H), 1.24 - 1.13 (m, 1H), 1.10 - 0.97 (m, 2H), 0.45 - 0.37 (m, 2H), 0.27 - 0.21 (m, 2H) |
| **I-55** | PH | WL | 872.5 | 11.13 (s, 1H), 9.72 (s, 1H), 8.92 (s, 1H), 8.22 - 8.10 (m, 2H), 7.19 - 6.98 (m, 7H), 5.40 - 5.28 (m, 1H), 4.30 - 4.19 (m, 1H), 3.76 (d, *J* = 9.6 Hz, 2H), 3.54 (s, 3H), 3.35 - 3.30 (m, 4H), 3.19 - 3.16 (m, 2H), 3.09 - 0.03 (m, 2H), 2.93 - 2.86 (m, 1H), 2.82 (s, 2H), 2.72 - 2.63 (m, 5H), 2.62 - 2.56 (m, 1H), 2.05 - 1.95 (m, 3H), 1.89 - 1.74 (m, 4H), 1.61 - 1.44 (m, 2H), 1.13 - 0.99 (m, 1H), 0.49 - 0.42 (m, 2H), 0.26 - 0.18 (m, 2H) |
| **I-56** | XK | PW | 898.5 | 11.04 (s, 1H), 9.68 (s, 1H), 8.91 (s, 1H), 8.19 (d, *J* = 10.2 Hz, 2H), 8.15 (d, *J* = 5.3 Hz, 1H), 7.37 - 6.98 (m, 4H), 6.92 (d, *J* = 8.6 Hz, 1H), 6.81 (d, *J* = 2.1 Hz, 1H), 6.65 - 6.60 (m, 1H), 5.33 - 5.20 (m, 1H), 4.23 - 4.16 (m, 1H), 3.57 (s, 3H), 3.50 - 3.46 (m, 2H), 3.29 (s, 5H), 3.18 (s, 2H), 2.94 - 2.82 (m, 1H), 2.73 - 2.56 (m, 4H), 2.21 (s, 3H), 2.20 - 2.18 (m, 1H), 2.08 - 1.87 (m, 5H), 1.76 (d, *J* = 12.3 Hz, 7H), 1.40 - 1.26 (m, 2H), 1.12 - 0.95 (m, 3H), 0.48 - 0.41 (m, 2H), 0.26 - 0.18 (m, 2H) |
| **I-57^{c}** | SI | PW | 906.3 | 11.10 (s, 1H), 9.69 (s, 1H), 8.92 (s, 1H), 8.18 (s, 1H), 8.15 - 8.13 (m, 1H), 7.29 - 7.01 (m, 8H), 5.40 - 5.34 (m, 1H), 4.26 - 4.14 (m, 1H), 3.30 (s, 3H), 3.18 (t, *J =* 6.1 Hz, 2H), 2.93 - 2.83 (m, 3H), 2.64 (s, 1H), 2.56 (s, 4H), 2.12 (s, 3H), 2.11 - 1.87 (m, 12H), 1.78 - 1.65 (m, 4H), 1.18 - 0.91 (m, 6H), 0.48 - 0.42 (m, 2H), 0.24 - 0.19 (m, 2H) |
| **I-58** | XM | PW | 898.6 | 11.08 (s, 1H), 9.69 (s, 1H), 8.91 (s, 1H), 8.16 (s, 1H), 8.14 (d, *J* = 5.2 Hz, 1H), 7.30 - 7.04 (m, 3H), 7.03 - 6.94 (m, 3H), 6.90 - 6.84 (m, 1H), 5.35 (dd, *J* = 5.2, 12.4 Hz, 1H), 4.24 - 4.14 (m, 1H), 3.56 (s, 3H), 3.52 - 3.50 (m, 4H), 3.17 (t, *J* = 6.0 Hz, 2H), 2.97 - 2.92 (m, 2H), 2.89 - 2.82 (m, 1H), 2.76 - 2.66 (m, 2H), 2.65 - 2.57 (m, 2H), 2.41 - 2.37 (m, 2H), 2.11 (d, *J =* 7.2 Hz, 2H), 2.06 (s, 6H), 2.03 - 1.95 (m, 2H), 1.93 - 1.66 (m, 7H), 1.57 (s, 1H), 1.09 - 0.96 (m, 3H), 0.48 - 0.41 (m, 2H), 0.24 - 0.18 (m, 2H) |
| **I-59** | SL | PW | 1039.4 | 11.1 (s, 1H), 9.78 (s, 1H), 9.00 (s, 1H), 8.59 (d, *J* = 5.2 Hz, 1H), 8.29 (s, 1H), 8.20 (d, *J* = 1.8 Hz, 2H), 7.72 - 7.64 (m, 1H), 7.30 - 6.93 (m, 4H), 5.39 - 5.31 (m, 1H), 4.21 (t, *J* = 11.8 Hz, 1H), 4.06 (d, *J =* 4.8 Hz, 2H), 3.86 (d, *J* = 6.8 Hz, 2H), 3.47 (s, 5H), 3.37 (s, 3H), 3.33 (s, 3H), 2.95 - 2.84 (m, 2H), 2.76 - 2.58 (m, 5H), 2.44 (d, *J* = 6.1 Hz, 3H), 2.33 (d, *J =* 1.7 Hz, 1H), 2.18 - 1.87 (m, 10H), 1.80 - 1.68 (m, 2H), 1.51 (s, 9H), 1.22 - 1.05 (m, 3H), 0.44 - 0.36 (m, 2H), 0.27 - 0.19 (m, 2H) |
| **I-60^{c}** | RK | WO | 920.6 | 11.07 (s, 1H), 10.38 (s, 1H), 8.88 (s, 1H), 8.27 (s, 1H), 8.23 (d, *J* = 5.6 Hz, 1H), 8.16 (s, 1H), 7.65 (t, *J* = 6.4 Hz, 1H), 7.21 (s, 1H), 7.15 (d, *J* = 5.2 Hz, 1H), 7.04 (s, 1H), 7.02 - 6.95 (m, 1H), 6.90 - 6.83 (m, 1H), 5.88 (s, 1H), 5.35 - 5.30 (m, 1H), 4.30 - 4.15 (m, 2H), 4.03 (t, *J* = 12 Hz, 1H), 3.81 - 3.74 (m, 1H), 3.53 (s, 2H), 3.32 (s, 3H), 2.96 - 2.84 (m, 2H), 2.80 (d, *J =* 10.8 Hz, 1H), 2.66 - 2.57 (m, 6H), 2.45 (s, 3H), 2.33 (s, 1H), 2.05 - 1.95 (m, 4H), 1.91 - 1.82 (m, 2H), 1.80 - 1.59 (m, 6H), 1.50 (s, 6H), 1.13 - 0.98 (m, 2H) |
| **I-61** | WQ | PW | 894.5 | 11.14 (s, 1H), 9.76 (s, 1H), 9.06 (s, 1H), 8.94 (s, 1H), 8.20 (s, 1H), 8.15 (d, *J* = 5.6 Hz, 1H), 7.30 (s, 1H), 7.22 - 7.09 (m, 4H), 7.04 (d, *J* = 5.2 Hz, 1H), 5.44 - 5.35 (m, 1H), 4.30 - 4.18 (m, 1H), 4.04 - 3.88 (m, 2H), 3.75 - 3.67 (m, 1H), 3.65 - 3.59 (m, 2H), 3.35 (s, 3H), 3.23 - 3.15 (m, 2H), 2.95 - 2.77 (m, 6H), 2.77 - 2.69 (m, 1H), 2.67 - 2.63 (m, 1H), 2.63 - 2.55 (m, 1H), 2.53 (s, 3H), 2.45 - 2.40 (m, 1H), 2.24 - 2.13 (m, 1H), 2.12 - 1.97 (m, 4H), 1.96 - 1.58 (m, 4H), 1.28 - 1.13 (m, 2H), 1.12 - 1.00 (m, 1H), 0.51 - 0.42 (m, 2H), 0.26 - 0.19 (m, 2H) |
| **I-62** | QI | PW | 857.6 | 11.08 (s, 1H), 9.71 (s, 1H), 8.92 - 8.89 (m, 1H), 8.17 (s, 1H), 8.14 (d, *J =* 5.2 Hz, 1H), 7.31 - 7.06 (m, 3H), 7.03 - 6.97 (m, 3H), 6.89 - 6.84 (m, 1H), 5.36 - 5.29 (m, 1H), 4.20 - 4.15 (m, 1H), 3.41 - 3.38 (m, 4H), 3.31 (s, 3H), 3.19 - 3.15 (m, 2H), 2.93 - 2.83 (m, 1H), 2.75 - 2.62 (m, 4H), 2.60 - 2.55 (m, 2H), 2.42 - 2.29 (m, 5H), 2.07 - 1.95 (m, 3H), 1.89 (d, *J =* 11.6 Hz, 2H), 1.85 - 1.75 (m, 3H), 1.76 - 1.67 (m, 3H), 1.63 (s, 1H), 1.23 - 1.00 (m, 3H), 0.47 - 0.42 (m, 2H), 0.24 - 0.19 (m, 2H) |
| **I-63** | XO | PW | 885.5 | 11.08 (s, 1H), 9.68 (s, 1H), 8.92 (s, 1H), 8.20 - 8.13 (m, 2H), 7.30 - 7.11 (m, 1H), 7.10 (s, 1H), 7.04 - 7.00 (m, 1H), 6.96 (d, *J* = 5.2 Hz, 2H), 6.89 - 6.85 (m, 1H), 5.38 - 5.34 (m, 1H), 4.33 - 4.12 (m, 1H), 3.57 (s, 3H), 3.41 ( s, 5H), 3.18 (t, *J* = 6.0 Hz, 2H), 3.00 - 2.94 (m, 2H), 2.90 - 2.83 (m, 2H), 2.71 (d, *J* = 4.4 Hz, 1H), 2.63 (d, *J* = 4.8 Hz, 1H), 2.61 - 2.57 (m, 1H), 2.55 - 2.54 (m, 1H), 2.41 (t, *J* = 6.8 Hz, 2H), 2.17 (d, *J* = 6.8 Hz, 2H), 2.05 ( d, *J =* 10.8 Hz, 2H), 1.98 - 1.89 (m, 2H), 1.87 - 1.80 (m, 2H), 1.78 - 1.69 (m, 2H), 1.59 (t, *J* = 6.4 Hz, 2H), 1.11 - 0.98 (m, 3H), 0.92 (d, *J* = 6.4 Hz, 6H), 0.50 - 0.42 (m, 2H), 0.23 - 0.22 (m, 2H) |
| **I-64** | XQ | PW | 883.6 | 11.08 (s, 1H), 9.67 (s, 1H), 8.92 (s, 1H), 8.17 (s, 1H), 8.15 (d, *J* = 5.6 Hz, 1H), 7.28 - 7.00 (m, 4H), 6.96 (d, *J* = 4.8 Hz, 2H), 6.89 - 6.85 (m, 1H), 5.39 - 5.33 (m, 1H), 4.29 - 4.08 (m, 1H), 3.57 (s, 3H), 3.43 - 3.38 (m, 4H), 3.18 (t, *J =* 6.0 Hz, 2H), 2.99 - 2.94 (m, 2H), 2.93 - 2.82 (m, 1H), 2.76 - 2.69 (m, 1H), 2.65 - 2.62 (m, 1H), 2.59 (t, *J =* 6.8 Hz, 2H), 2.53 - 2.52 (m, 1H), 2.36 (d, *J =* 7.2 Hz, 2H), 2.08 - 2.01 (m, 2H), 2.00 - 1.94 (m, 1H), 1.90 - 1.80 (m, 4H), 1.74 - 1.68 (m, 4H), 1.64 - 1.54 (m, 1H), 1.10 - 1.03 (m, 1H), 1.02 - 0.92 (m, 2H), 0.48 - 0.42 (m, 4H), 0.31 - 0.26 (m, 2H), 0.24 - 0.20 (m, 2H) |
| **I-65** | YB | PW | 855.6 | 11.10 - 11.05 (m, 1H), 9.69 (s, 1H), 8.92 (s, 1H), 8.18 - 8.18 (m, 1H), 8.18 - 8.12 (m, 1H), 7.29 - 7.06 (m, 3H), 7.02 - 6.95 (m, 3H), 6.90 - 6.84 (m, 1H), 5.41 - 5.32 (m, 1H), 4.22 - 4.14 (m, 1H), 3.56 (s, 3H), 3.49 - 3.51 (m, 4H), 3.19 - 3.16 (m, 4H), 2.98 - 2.96 (m, 2H), 2.92 - 2.88 (m, 4H), 2.85 -.2.83 (m, 1H), 2.62 - 2.60 (m, 2H), 2.07 - 1.94 (m, 4H), 1.88 - 1.80 (m, 4H), 1.76 - 1.66 (m, 2H), 1.60 - 1.51 (m, 1H), 1.40 - 1.29 (m, 1H), 1.12 - 0.97 (m, 3H), 0.48 - 0.42 (m, 2H), 0.22 - 0.20 (m, 2H) |
| **I-66** | YD | PW | 813.4 | 11.08 (s, 1H), 9.69 (s, 1H), 8.92 (s, 1H), 8.20 - 8.18 (s, 1H), 8.15 (d, *J =* 4.8 Hz, 1H), 7.29 - 6.96 (m, 5H), 6.90 - 6.83 (m, 1H), 5.42 - 5.31 (m, 1H), 4.25 - 4.13 (m, 1H), 3.60 - 3.52 (m, 3H), 3.30 - 3.29 (m, 3H), 3.21 - 3.15 (m, 2H), 2.94 - 2.86 (m, 3H), 2.63 - 2.56 (m, 2H), 2.55 - 2.52 (m, 12H), 2.17 - 2.09 (m, 5H), 2.06 - 1.97 (m, 3H), 1.88 (d, *J =* 11.2 Hz, 2H), 1.83 - 1.68 (m, 2H), 1.64 - 1.51 (m, 4H), 1.15 - 0.94 (m, 3H), 0.48 - 0.42 (m, 2H), 0.25 - 0.20 (m, 2H) |
| **I-67** | WR | PW | 885.5 | 11.09 (s, 1H), 9.72 (s, 1H), 8.92 (s, 1H), 8.14 (s, 1H), 7.16 (t, *J =* 54.4 Hz, 1H), 7.11 - 7.06 (m, 2H), 7.01 (dd, *J =* 1.2, 5.2 Hz, 1H), 6.98 - 6.93 (m, 2H), 6.88 - 6.83 (m, 1H), 5.36 (dd, *J =* 5.2, 12.4 Hz, 1H), 4.26 - 4.14 (m, 1H), 3.57 - 3.54 (s, 3H), 3.44 - 3.40 (m, 3H), 3.37 (s, 2H), 3.18 (t, *J =* 6.0 Hz, 3H), 2.94 - 2.83 (m, 3H), 2.76 - 2.65 (m, 2H), 2.65 - 2.58 (m, 2H), 2.52 (s, 1H), 2.41 (s, 3H), 2.08 - 1.95 (m, 3H), 1.88 (d, *J* = 11.2 Hz, 2H), 1.83 - 1.72 (m, 2H), 1.68 - 1.58 (m, 5H), 1.53 (s, 4H), 1.13 - 0.99 (m, 3H), 0.49 - 0.41 (m, 2H), 0.25 - 0.18 (m, 2H) |
| **I-68** | PP | YF | 816.9 | 11.10 (s, 1H), 9.84 (s, 1H), 9.13 - 9.02 (m, 2H), 8.18 - 8.14 (m, 2H), 7.35 - 7.29 (m, 1H), 7.29 - 7.02 (m, 2H), 6.98 - 6.97 (m, 2H), 6.88 - 6.86 (m, 1H), 5.39 - 5.34 (m, 1H), 4.28 - 4.21 (m, 1H), 3.58 (s, 3H), 3.47 (d, *J* = 5.2 Hz, 4H), 3.22 - 3.17 (m, 2H), 3.10 - 3.06 (m, 2H), 3.02 - 2.98 (m, 2H), 2.94 (s, 3H), 2.89 - 2.84 (m, 1H), 2.81 - 2.80 (m, 3H), 2.72 - 2.64 (m, 2H), 2.07 - 1.81 (m, 13H), 1.23 - 1.16 (m, 2H) |
| **I-69^{b}** | WR | XR | 788.5 | 11.07 (s, 1H), 9.79 (s, 1H), 9.00 (s, 1H), 8.86 - 8.79 (m, 2H), 8.16 (s, 1H), 7.98 - 7.94 (m, 2H), 7.15 (t, *J =* 54.4 Hz, 1H), 6.98 - 6.93 (m, 2H), 6.90 - 6.84 (m, 1H), 5.40 - 5.32 (m, 1H), 4.24 - 4.15 (m, 1H), 3.57 (s, 3H), 3.42 (t, *J =* 6.0 Hz, 4H), 3.01 - 2.93 (m, 2H), 2.92 - 2.83 (m, 1H), 2.75 - 2.57 (m, 2H), 2.37 (t, *J =* 7.0 Hz, 2H), 2.15 (s, 3H), 2.13 (s, 2H), 2.07 - 1.99 (m, 3H), 1.99 - 1.95 (m, 1H), 1.90 (d, *J* = 11.6 Hz, 2H), 1.86 - 1.79 (m, 2H), 1.78 - 1.72 (m, 2H), 1.69 - 1.62 (m, 2H), 1.58 - 1.49 (m, 1H), 1.09 - 0.97 (m, 2H) |
| **I-70^{b}** | PP | WS | 802.5 | 11.09 (s, 1H), 9.81 (s, 1H), 8.99 (s, 1H), 8.68 (d, *J =* 5.2 Hz, 1H), 8.15 (s, 1H), 7.84 (s, 1H), 7.76 (d, *J =* 5.2 Hz, 1H), 7.15 (t, *J* = 54.4 Hz, 1H), 6.98 - 6.93 (m, 2H), 6.89 - 6.85 (m, 1H), 5.36 (dd, *J* = 5.6, 12.5 Hz, 1H), 4.26 - 4.14 (m, 1H), 3.57 (s, 3H), 3.42 (t, *J =* 6.0 Hz, 4H), 2.99 - 2.93 (m, 2H), 2.92 - 2.81 (m, 1H), 2.76 - 2.62 (m, 2H), 2.59 (s, 3H), 2.40 - 2.37 (m, 1H), 2.38 (t, *J* = 7.2 Hz, 2H), 2.19 - 2.12 (m, 5H), 2.05 - 1.95 (m, 3H), 1.94 - 1.86 (m, 2H), 1.86 - 1.79 (m, 2H), 1.76 (d, *J =* 12.0 Hz, 2H), 1.69 - 1.62 (m, 2H), 1.55 - 1.54 (m, 1H), 1.59 - 1.49 (m, 1H), 1.10 - 0.97 (m, 2H) |
| **I-71^{b}** | PP | WT | 869.5 | 11.19 - 11.03 (m, 1H), 7.66 - 7.52 (m, 1H), 7.32 (d, *J* = 0.8 Hz, 1H), 7.16 - 7.13 (m, 1H), 5.39 (dd, *J =* 5.2, 12.8 Hz, 1H), 4.36 (s, 2H), 4.10 (d, *J* = 2.4 Hz, 1H), 3.41 - 3.36 (m, 1H), 3.36 (s, 1H), 3.32 - 3.27 (m, 6H), 2.97 - 2.82 (m, 1H), 2.77 - 2.62 (m, 5H), 2.11 - 1.96 (m, 1H), 1.64 (s, 2H), 1.42 - 1.35 (m, 1H), 1.07 - 1.01 (m, 2H) |
| **I-72** | YH | PW | 855.4 | 11.08 (s, 1H), 9.69 (s, 1H), 9.68 (s, 1H), 8.92 (s, 1H), 8.25 - 8.19 (m, 1H), 8.15 (d, *J* = 5.2 Hz, 1H), 7.31 - 7.05 (m, 3H), 7.02 - 7.00 (m, 1H), 6.98 - 6.93 (m, 2H), 6.90 - 6.85 (m, 1H), 5.39 - 5.32 (m, 1H), 4.25 - 4.15 (m, 1H), 4.02 - 3.93 (m, 1H), 3.57 (s, 3H), 3.21 - 3.14 (m, 2H), 2.98 - 2.86 (m, 3H), 2.66 - 2.57 (m, 2H), 2.44 - 2.39 (m, 2H), 2.37 - 2.29 (m, 2H), 2.24 (d, *J =* 7.2 Hz, 2H), 2.08 - 1.89 (m, 6H), 1.85 - 1.68 (m, 5H), 1.68 - 1.47 (m, 3H), 1.14 - 0.98 (m, 3H), 0.48 - 0.42 (m, 2H), 0.25 - 0.20 (m, 2H) |
| **I-73** | WV | PW | 855.5 | 11.09 (s, 1H), 9.71 (s, 1H), 8.92 (s, 1H), 8.20 - 8.13 (m, 2H), 7.31 - 6.93 (m, 6H), 6.91 - 6.84 (m, 1H), 5.39 - 5.34 (m, 1H), 4.36 - 4.15 (m, 1H), 4.13 - 3.99 (m, 1H), 3.57 (s, 3H), 3.50 -3. 49 (m, 1H), 3.18 (t, *J* = 6.0 Hz, 3H), 3.00 - 2.93 (m, 3H), 2.92 - 2.83 (m, 2H), 2.77-2.70 (m, 2H), 2.69 - 2.58 (m, 2H), 2.53 - 2.52 (m, 1H), 2.11 - 1.89 (m, 6H), 1.88 - 1.71 (m, 5H), 1.70-1.54 (m, 2H), 1.17 - 0.97 (m, 3H), 0.48 - 0.41 (m, 2H), 0.24 - 0.19 (m, 2H) |
| **I-74** | XT | PW | 922.6 | 11.09 (s, 1H), 9.68 (s, 1H), 8.91 (s, 1H), 8.22 - 8.11 (m, 3H), 7.15 (t, *J* = 54.8 Hz, 1H), 7.10 (s, 1H), 7.03 - 6.98 (m, 1H), 6.95 (t, *J* = 7.6 Hz, 1H), 6.86 (d, *J* = 7.6 Hz, 1H), 5.39 - 5.34 (m, 1H), 4.26 - 4.12 (m, 1H), 3.66 (s, 3H), 3.60 (s, 2H), 3.18 (t, *J* = 6.0 Hz, 3H), 2.95 - 2.84 (m, 2H), 2.80 (d, *J* = 9.2 Hz, 4H), 2.76 - 2.69 (m, 1H), 2.65 - 2.58 (m, 2H), 2.10 (d, *J* = 6.0 Hz, 2H), 2.07 - 1.71 (m, 12H), 1.58 (d, *J =* 11.2 Hz, 5H), 1.11 - 1.00 (m, 8H), 0.49 - 0.42 (m, 2H), 0.26 - 0.18 (m, 2H) |
| **I-75** | WX | PW | 854.5 | 11.11 (s, 1H), 9.70 (s, 1H), 8.92 (s, 1H), 8.17 (s, 1H), 8.16 - 8.13 (m, 2H), 7.29 - 7.06 (m, 3H), 7.01 (dd,*J* = 1.2, 5.2 Hz, 1H), 6.96 (t, *J* = 7.6 Hz, 1H), 6.87 (d, *J* = 7.2 Hz, 1H), 5.40 - 5.36 (m, 1H), 4.24 - 4.12 (m, 1H), 3.67 (s, 3H), 3.62 (s, 2H), 3.21 - 3.15 (m, 3H), 2.94 - 2.83 (m, 3H), 2.75 - 2.60 (m, 2H), 2.53 - 2.51 (m, 1H), 2.30 - 2.25 (m, 2H), 2.22 (s, 3H), 2.08 - 1.93 (m, 5H), 1.92 - 1.83 (m, 2H), 1.81 - 1.70 (m, 2H), 1.69 - 1.61 (m, 2H), 1.57 - 1.33 (m, 3H), 1.13 - 0.93 (m, 3H), 0.48 - 0.42 (m, 2H), 0.25 - 0.19 (m, 2H) |
| **I-76** | YI | PW | 868.2 | 11.11 (s, 1H), 9.70 (s, 1H), 8.92 (s, 1H), 8.18 - 8.13 (m, 4H), 7.31 - 6.83 (m, 7H), 5.43 - 5.30 (m, 1H), 4.25 - 4.13 (m, 1H), 3.67 (s, 3H), 3.64 - 3.57 (m, 2H), 3.17 (t, *J* = 6.2 Hz, 2H), 2.95 - 2.57 (m, 4H), 2.16 (s, 6H), 2.06 - 1.42 (m, 14H), 1.12 - 0.93 (m, 5H), 0.50 - 0.38 (m, 2H), 0.26 - 0.15 (m, 2H) |
| **I-77** | YJ | PW | 870.5 | 11.09 - 11.08 (m, 1H), 9.72 (s, 1H), 8.93 (s, 1H), 8.19 (d, *J* = 5.2 Hz, 1H), 8.15 (d, *J* = 5.2 Hz, 1H), 7.31 - 7.17 (m, 1H), 7.10 - 7.08 (m, 3H), 6.97 (t, *J =* 7.6 Hz, 1H), 6.89 (d, *J* = 7.6 Hz, 1H), 5.38 (dd, *J =* 5.2 Hz, *J* = 12.0 Hz, 1H), 4.19 - 4.15 (m, 1H), 3.80 - 3.74 (m, 2H), 3.69 (s, 3H), 3.62 - 3.57 (m, 1H), 3.54 - 3.47 (m, 2H), 3.18 (t, *J =* 6.0 Hz, 2H), 2.92 - 2.82 (m, 2H), 2.75 - 2.71 (m, 1H), 2.63 (s, 1H), 2.59 - 2.56 (m, 1H), 2.54 - 2.52 (m, 3H), 2.26 - 2.25 (m, 2H), 2.17 - 2.12 (m, 2H), 2.00 - 1.99 (m, 4H), 1.82 - 1.73 (m, 6H), 1.58 - 1.44 (m, 1H), 1.10 - 0.98 (m, 2H), 0.94 - 0.84 (m, 1H), 0.48 - 0.43 (m, 2H), 0.24 - 0.21 (m, 2H) |
| **I-78** | YK | PW | 870.5 | 11.06 (s, 1H), 9.69 (s, 1H), 8.92 (s, 1H), 8.20 - 8.14 (m, 2H), 7.20 - 6.99 (m, 5H), 6.98 - 6.86 (m, 2H), 5.40 - 5.32 (m, 1H), 4.21 - 4.06 (m, 1H), 3.79 - 3.59 (m, 6H), 3.51 - 3.42 (m, 2H), 3.18 (t, *J =* 5.6 Hz, 2H), 2.93 - 2.82 (m, 2H), 2.75 - 2.59 (m, 3H), 2.34 - 2.25 (m, 2H), 2.19 - 1.94 (m, 9H), 1.86 - 1.65 (m, 5H), 1.55 - 1.36 (m, 1H), 1.13 - 1.03 (m, 1H), 0.99 - 0.75 (m, 2H), 0.49 - 0.41 (m, 2H), 0.25 - 0.19 (d, 2H) |
| **I-79^{b}** | WY | WW | 882.4 | 11.14 (s, 1H), 9.77 (s, 1H), 9.26 - 9.01 (m, 1H), 8.97 (d, *J* = 4.4 Hz, 1H), 8.20 (s, 1H), 8.13 (d, *J* = 5.6 Hz, 1H), 7.35 - 7.21 (m, 2H), 7.21 - 6.96 (m, 4H), 5.47 - 5.43 (m, 1H), 4.73 - 4.54 (m, 2H), 4.31 - 4.19 (m, 1H), 3.61 (s, 3H), 3.21 (d, *J* = 6.0 Hz, 3H), 3.15 - 2.90 (m, 6H), 2.79 (d, *J* = 4.4 Hz, 2H), 2.76 - 2.69 (m, 1H), 2.62 (s, 1H), 2.52 (s, 3H), 2.10 - 1.96 (m, 3H), 1.94 - 1.74 (m, 7H), 1.59 (s, 3H), 1.46 - 1.30 (m, 2H), 1.26 - 1.14 (m, 2H), 1.13 - 1.04 (m, 1H), 0.49 (d, *J* = 7.6 Hz, 2H), 0.25 (d, *J* = 4.4 Hz, 2H) |
| **I-80^{b}** | WZ | ZC | 906.3 | 11.10 (s, 1H), 9.68 (s, 1H), 8.92 (s, 1H), 8.19 (s, 1H), 8.16 - 8.13 (m, 1H), 7.30 - 7.09 (m, 3H), 7.08 - 7.05 (m, 1H), 7.05 - 6.96 (m, 3H), 5.42 - 5.34 (m, 1H), 4.25 - 4.15 (m, 1H), 3.68 (s, 3H), 3.20 - 3.17 (m, 2H), 2.96 - 2.87 (m, 3H), 2.77 - 2.70 (m, 1H), 2.67 - 2.64 (m, 2H), 2.63 - 2.57 (m, 3H), 2.17 - 2.15 (m, 1H), 2.14 (s, 3H), 2.12 - 2.10 (m, 2H), 2.06 - 1.96 (m, 5H), 1.95 - 1.88 (m, 2H), 1.82 - 1.69 (m, 4H), 1.63 - 1.35 (m, 3H), 1.15 - 0.97 (m, 5H), 0.49 - 0.42 (m, 2H), 0.25 - 0.20 (m, 2H) |
| **I-81** | XV | PW | 893.6 | 9.72 (s, 1H), 8.93 (s, 1H), 8.48 (dd, *J* = 1.2, 7.6 Hz, 1H), 8.37 (d, *J* = 3.6 Hz, 1H), 8.31 (s, 1H), 8.15 - 8.09 (m, 2H), 7.47 (s, 1H), 7.33 - 6.98 (m, 6H), 6.01 (s, 1H), 4.23 - 4.11 (m, 1H), 3.54 - 3.52 (m, 9H), 3.44 (t, *J =* 6.4 Hz, 2H), 3.17 (t, *J* = 6.0 Hz, 2H), 3.12 - 2.95 (m, 2H), 2.83 - 2.66 (m, 5H), 2.15 - 1.81 (m, 7H), 1.78 - 1.63 (m, 2H), 1.55 - 1.45 (m, 1H), 1.13 - 0.99 (m, 3H), 0.50 - 0.41 (m, 2H), 0.23 - 0.20 (m, 2H) |
| **I-82^{b}** | PP | XX | 865.5 | 11.07 (s, 1H), 10.37 (s, 1H), 8.85 (s, 1H), 8.23 - 8.11 (m, 2H), 7.15 - 7.04 (m, 2H), 7.01 - 6.92 (m, 3H), 6.91 - 6.83 (m, 1H), 6.01 - 5.64 (m, 1H), 5.36 (d, *J* = 5.6, 12.4 Hz, 1H), 4.10 - 3.95 (m, 1H), 3.59 - 3.55 (m, 3H), 3.42 (d, *J* = 6.0 Hz, 5H), 3.18 (d, *J* = 6.0 Hz, 2H), 3.01 - 2.91 (m, 2H), 2.90 - 2.83 (m, 1H), 2.76 - 2.56 (m, 2H), 2.34 (d, *J* = 6.8 Hz, 2H), 2.13 (s, 3H), 2.10 (d, *J* = 7.2 Hz, 2H), 2.06 - 1.96 (m, 3H), 1.93 - 1.79 (m, 4H), 1.75 - 1.59 (m, 4H), 1.49 (s, 6H), 1.12 - 0.94 (m, 3H), 0.49 - 0.41 (m, 2H), 0.28 - 0.18 (m, 2H) |
| **I-83^{b}** | QI | XX | 865.6 | 11.15 - 10.99 (m, 1H), 10.36 (s, 1H), 8.85 (s, 1H), 8.17 - 8.12 (m, 2H), 7.12 -7.05 (m, 2H), 7.04 - 6.97 (m, 3H), 6.87 (d, *J* = 7.2 Hz, 1H), 5.95 - 5.73 (m, 1H), 5.32 (dd, *J* = 5.2, 12.6 Hz, 1H), 4.10 - 3.97 (m, 1H), 3.50 - 3.44 (m, 4H), 3.31 (s, 3H), 3.19 - 3.16 (m, 2H), 2.92 - 2.84 (m, 1H), 2.70 - 2.63 (m, 4H), 2.34 - 2.31 (m, 2H), 2.13 (s, 3H), 2.10 (d, *J* = 7.2 Hz, 2H), 2.05 - 1.96 (m, 3H), 1.91 - 1.78 (m, 4H), 1.74 - 1.53 (m, 5H), 1.49 (s, 6H), 1.11 - 0.93 (m, 3H), 0.50 - 0.40 (m, 2H), 0.26 - 0.18 (m, 2H) |
| **I-84** | PP | TE | 845.6 | 11.10 (s, 1H), 9.37 (s, 1H), 8.85 (d, *J* = 8.0 Hz, 1H), 8.39 (s, 1H), 8.30 (s, 1H), 7.29 - 6.83 (m, 5H), 5.37 (dd, *J* = 5.2, 12.4 Hz, 1H), 4.25 - 4.10 (m, 1H), 4.00 - 3.80 (m, 4H), 3.57 (s, 3H), 3.46 - 3.38 (m, 4H), 3.12 - 3.00 (m, 4H), 2.99 - 2.82 (m, 3H), 2.77 - 2.51 (m, 2H), 2.32 - 2.22 (m, 5H), 2.08 - 1.51 (m, 14H), 1.12 - 0.97 (m, 2H) |
| **I-85** | XB | PW | 893.2 | 11.08 (s, 1H), 9.68 (s, 1H), 8.92 (s, 1H), 8.21 - 8.12 (m, 2H), 7.15 (t, *J =* 54.4 Hz, 1H), 7.10 (s, 1H), 7.08 (t, *J =* 5.6 Hz, 1H), 7.02 (m, 1H), 6.98 - 6.96 (m, 2H), 6.91 - 6.87 (m, 1H), 5.42 - 5.30 (m, 1H), 4.25 - 4.14 (m, 1H), 3.79 - 3.68 (m, 2H), 3.63 - 3.58 (m, 2H), 3.57 (s, 3H), 3.21 - 3.15 (m, 2H), 3.02 - 2.95 (m, 2H), 2.94 - 2.73 (m, 3H), 2.73 - 2.58 (m, 2H), 2.35 - 2.29 (m, 2H), 2.29 (s, 3H), 2.11 - 1.82 (m, 7H), 1.82 - 1.69 (m, 2H), 1.62 - 1.45 (m, 1H), 1.11 - 0.94 (m, 3H), 0.49 - 0.42 (m, 2H), 0.26 - 0.19 (m, 2H) |
| **I-86^{b}** | YL | PW | 835.5 | 11.10 (s, 1H), 9.68 (s, 1H), 8.92 (s, 1H), 8.39 (s, 1H), 8.18 (s, 1H), 8.15 (d, *J =* 5.2 Hz, 1H), 7.29 - 6.97 (m, 8H), 5.40 - 5.36 (m, 1H), 4.25 - 4.17 (m, 1H), 3.65 (s, 3H), 3.21 - 3.16 (m, 5H), 2.69 - 2.66 (m, 7H), 2.34 - 2.32 (m, 4H), 2.13 - 2.12 (m, 3H), 2.07 (s, 1H), 2.04 - 2.02 (m, 2H), 1.92 - 1.89 (m, 5H), 1.77 - 1.58 (m, 6H), 1.09 - 1.00 (m, 4H), 0.47 - 0.43 (m, 3H), 0.24 - 0.20 (m, 2H) |
| **I-87** | XC | PW | 843.8 | 11.19 (s, 1H), 9.71 (s, 1H), 8.91 (s, 1H), 8.18 (s, 1H), 8.16 - 8.11 (m, 1H), 7.31 - 6.97 (m, 7H), 5.34 (d, *J* = 5.2, 12.8 Hz, 1H), 4.28 - 4.16 (m, 1H), 3.42 (d, *J =* 6.0 Hz, 2H), 3.39 - 3.34 (m, 3H), 3.17 (d, *J =* 6.0 Hz, 2H), 2.97 - 2.81 (m, 3H), 2.71 - 2.58 (m, 7H), 2.34 - 2.30 (m, 1H), 2.19 - 2.01 (m, 3H), 1.98 - 1.70 (m, 9H), 1.21 - 1.00 (m, 3H), 0.53 - 0.37 (m, 2H), 0.28 - 0.14 (m, 2H) |
| **I-88** | XD | PW | 853.5 | 11.09 (s, 1H), 9.67 (s, 1H), 8.92 (s, 1H), 8.33 (s, 1H), 8.14 (s, 1H), 7.29 - 7.04 (m, 5H), 7.01 (d, *J* = 7.2 Hz, 2H), 5.42 - 5.35 (m, 1H), 4.43 (s, 2H), 4.16 - 4.01 (m, 1H), 3.65 (s, 3H), 3.59 (t, *J* = 6.4 Hz, 2H), 3.20 - 3.16 (m, 2H), 2.92 - 2.81 (m, 1H), 2.64 - 2.57 (m, 2H), 2.38 - 2.34 (m, 2H), 2.12 (s, 3H), 2.10 (d, *J* = 6.8 Hz, 2H), 2.01 - 1.93 (m, 3H), 1.91 - 1.85 (m, 2H), 1.73 - 1.69 (m, 4H), 1.55 - 1.47 (m, 1H), 1.10 - 0.93 (m, 3H), 0.48 - 0.42 (m, 2H), 0.25 - 0.19 (m, 2H) |
| **I-91** | XE | PW | 858.2 | 11.06 (s, 1H), 9.66 (s, 1H), 8.91 (s, 1H), 8.17 - 8.14 (m, 2H), 7.63 (m, 1H), 7.29 - 7.02 (m, 4H), 7.01 (d, *J =* 1.6, 5.2 Hz, 1H), 6.94 (d, *J* = 8.0 Hz, 1H), 5.38 - 5.27 (m, 1H), 4.24 - 4.12 (m, 3H), 4.04 (d, *J* = 6.0 Hz, 2H), 3.22 - 3.13 (m, 5H), 2.93 - 2.82 (m, 2H), 2.70 (d, *J* = 4.0 Hz, 1H), 2.62 (d, *J* = 4.8 Hz, 3H), 2.19 (s, 3H), 2.17 (d, *J* = 7.6 Hz, 2H), 2.06 - 1.94 (m, 3H), 1.92 - 1.82 (m, 2H), 1.80 - 1.67 (m, 2H), 1.58 - 1.42 (m, 1H), 1.12 - 0.93 (m, 3H), 0.50 - 0.40 (m, 2H), 0.27 - 0.17 (m, 2H) |
| **I-92** | XZ | PW | 893.5 | 11.13 (s, 1H), 9.70 (s, 1H), 8.92 (s, 1H), 8.32 (d, *J =* 4.8 Hz, 1H), 8.23 (d, *J* = 7.6 Hz, 1H), 8.18 - 8.10 (m, 2H), 7.63 (s, 1H), 7.53 - 7.49 (m, 1H), 7.32 (t, *J* = 7.6 Hz, 1H), 7.29 - 6.98 (m, 5H), 6.05 (s, 1H), 4.14 - 4.08 (m, 1H), 3.66 - 3.54 (m, 11H), 3.24 (t, *J* = 8.0 Hz, 2H), 3.17 (t, *J =* 6.0 Hz, 2H), 3.04 - 3.00 (m, 2H), 2.77 (t, *J =* 5.2 Hz, 2H), 2.71 (s, 1H), 2.11 - 1.91 (m, 5H), 1.83 (d, *J =* 11.6 Hz, 2H), 1.72 - 1.58 (m, 2H), 1.50 - 1.46 (m, 1H), 1.13 - 0.92 (m, 3H), 0.50 - 0.41 (m, 2H), 0.27 - 0.18 (m, 2H) |
| **I-93** | XG | PW | 893.5 | 11.15 (s, 1H), 9.71 (s, 1H), 8.96 - 8.88 (m, 1H), 8.61 - 8.51 (m, 1H), 8.46 - 8.39 (m, 1H), 8.28 - 8.19 (m, 1H), 8.19 - 8.11 (m, 2H), 7.57 - 7.38 (m, 2H), 7.34 - 7.06 (m, 5H), 7.03 - 7.00 (m, 1H), 6.34 - 5.84 (m, 1H), 4.17 - 4.05 (m, 1H), 3.66 - 3.54 (m, 9H), 3.25 - 3.21 (m, 2H), 3.18 (t, *J* = 6.0, 2H), 3.08 - 2.95 (m, 2H), 2.91 - 2.69 (m, 4H), 2.15 - 2.04 (m, 1H), 2.03 - 1.91 (m, 4H), 1.86 - 1.74 (m, 2H), 1.69 - 1.44 (m, 3H), 1.15 - 0.92 (m, 3H), 0.49 - 0.42 (m, 2H), 0.26 - 0.18 (m, 2H) |
| **I-95** | XI | PW | 882.6 | 11.08 (s, 1H), 9.68 (s, 1H), 8.92 (s, 1H), 8.18 (s, 1H), 8.15 (d, *J* = 5.2 Hz, 1H), 7.31 - 6.93 (m, 6H), 6.92 - 6.85 (m, 1H), 5.36 (dd, *J* = 5.2, 12.4 Hz, 1H), 4.25 - 4.16 (m, 1H), 3.57 (s, 3H), 3.18 (t, *J =* 6.0 Hz, 2H), 3.00 - 2.83 (m, 5H), 2.76 - 2.68 (m, 1H), 2.65 - 2.60 (m, 1H), 2.40 (t, *J =* 6.4 Hz, 2H), 2.32 - 2.27 (m, 1H), 2.23 (d, *J* = 6.8 Hz, 2H), 2.20 (s, 3H), 2.08 - 1.86 (m, 7H), 1.81 - 1.63 (m, 6H), 1.55 - 1.38 (m, 3H), 1.12 - 0.93 (m, 3H), 0.50 - 0.40 (m, 2H), 0.27 - 0.17 (m, 2H) |
| **I-96** | TY | PW | 912.3 | 11.07 (s, 1H), 9.69 (s, 1H), 8.92 (s, 1H), 8.18 (s, 1H), 8.15 (d, *J =* 5.2 Hz, 1H), 7.38 - 6.97 (m, 6H), 6.87 (d, *J* = 8.0 Hz, 1H), 5.33 (dd, *J* = 5.2, 12.8 Hz, 1H), 4.24 - 4.14 (m, 1H), 3.76 (d, *J =* 11.2 Hz, 1H), 3.52 - 3.42 (m, 2H), 3.31 (s, 3H), 3.18 (t, *J* = 6.0 Hz, 2H), 2.95 - 2.83 (m, 1H), 2.80 - 2.72 (m, 1H), 2.72 - 2.57 (m, 5H), 2.47 - 2.41 (m, 2H), 2.29 (t, *J* = 7.2 Hz, 2H), 2.25 - 2.17 (m, 5H), 2.06 - 1.94 (m, 4H), 1.93 - 1.84 (m, 2H), 1.81 - 1.66 (m, 5H), 1.61 - 1.50 (m, 3H), 1.10 - 0.95 (m, 3H), 0.49 - 0.40 (m, 2H), 0.26 - 0.18 (m, 2H) |
| **I-97** | TW | PW | 912.2 | 11.08 (s, 1H), 9.70 (s, 1H), 8.92 (s, 1H), 8.17 (s, 1H), 8.14 (d, *J =* 5.2 Hz, 1H), 7.31 - 6.95 (m, 6H), 6.87 (d, *J* = 8.4 Hz, 1H), 5.35 - 5.30 (m, 1H), 4.24 - 4.14 (m, 1H), 3.77 - 3.74 (m, 1H), 3.45 - 3.43(m, 2H), 3.31 (s, 3H), 3.17 (t, *J =* 6.0 Hz, 3H), 2.94 - 2.83 (m, 1H), 2.79 - 2.72 (m, 1H), 2.68 - 2.66 (m, 1H), 2.65 - 2.58 (m, 3H), 2.52 - 2.51 (m, 1H), 2.41 - 2.34 (m, 2H), 2.30 - 2.23 (m, 2H), 2.14 (s, 3H), 2.12 - 2.10 (m, 1H), 2.06 - 1.94 (m, 4H), 1.93 - 1.83 (m, 2H), 1.81 - 1.65 (m, 5H), 1.57 - 1.45 (m, 3H), 1.13 - 0.93 (m, 3H), 0.50 - 0.39 (m, 2H), 0.26 - 0.17 (m, 2H) |
| **I-107** | AAB | PW | 858.6 | 11.1 (s, 1H), 9.71 (s, 1H), 8.91 (s, 1H), 8.24 (s, 2H), 8.16 - 8.14 (m, 1H), 7.31 - 7.13 (m, 2H), 7.11 - 7.04 (m, 4H), 7.03 - 7.00 (m, 1H), 5.40 - 5.30 (m, 1H), 4.22 - 4.09 (m, 1H), 3.95 - 3.85 (m, 2H), 3.46 - 3.44 (m, 6H), 3.33 (s, 3H), 3.20 - 3.14 (m, 2H), 2.94 - 2.85 (m, 1H), 2.83 - 2.77 (m, 2H), 2.71 - 2.65 (m, 1H), 2.65 - 2.59 (m, 1H), 2.21 - 2.14 (m, 5H), 2.04 - 1.96 (m, 3H), 1.91 - 1.81 (m, 2H), 1.79 - 1.65 (m, 2H), 1.55 - 1.43 (m, 1H), 1.11 - 0.94 (m, 3H), 0.49 - 0.41 (m, 2H), 0.25 - 0.18 (m, 2H) |
| **I-108** | YN | WL | 872.5 | 11.06 (s, 1H), 9.69 (s, 1H), 8.92 (s, 1H), 8.18 (d, J = 3.2 Hz, 1H), 8.15 (d, J = 5.4 Hz, 1H), 7.31 - 6.99 (m, 6H), 6.98 - 6.91 (m, 2H), 5.38 - 5.32 (m, 1H), 4.30 - 4.19 (m, 1H), 3.93 (d, J = 4.0 Hz, 2H), 3.66 (d, J = 6.0 Hz, 2H), 3.54 (s, 2H), 3.49 - 3.41 (m, 3H), 3.34 - 3.33 (m, 3H), 3.18 (t, J = 6.0 Hz, 2H), 3.05 (s, 2H), 2.81 (s, 1H), 2.77 - 2.72 (m, 2H), 2.65 - 2.58 (m, 1H), 2.52 (s, 3H), 1.97 (m, 1H), 1.84 - 1.74 (m, 4H), 1.62 - 1.45 (m, 4H), 1.08 - 1.04 (m, 1H), 0.49 - 0.41 (m, 2H), 0.25 - 0.19 (m, 2H) |
| **I-109^{d}** | ZB | PW | 913.6 | 11.09 (s, 1H), 9.70 (s, 1H), 8.92 (s, 1H), 8.26 (s, 1H), 8.18 (s, 1H), 8.15 (d, J = 5.2 Hz, 1H), 7.32 - 7.04 (m, 6H), 7.03 - 7.01 (m, 1H), 5.40 - 5.33 (m, 1H), 4.20 - 4.15 (m, 1H), 3.88 (s, 2H), 3.80 - 3.67 (m, 2H), 3.68 - 3.51 (m, 1H), 3.51 - 3.45 (m, 1H), 3.34 (s, 3H), 3.21 - 3.14 (m, 2H), 2.96 - 2.84 (m, 1H), 2.83 - 2.76 (m, 1H), 2.76 - 2.71 (m, 2H), 2.71 - 2.65 (m, 1H), 2.64 - 2.53 (m, 1H), 2.47 - 2.44 (m, 2H), 2.33 - 2.28 (m, 2H), 2.17 (s, 3H), 2.16 - 2.08 (m, 2H), 2.06 - 1.96 (m, 4H), 1.93 - 1.83 (m, 2H), 1.79 - 1.68 (m, 3H), 1.58 - 1.47 (m, 1H), 1.12 - 0.94 (m, 3H), 0.50 - 0.40 (m, 2H), 0.26 - 0.19 (m, 2H) |
| **I-110** | ZF | PW | 895.2 | 11.08 (s, 1H), 9.68 (s, 1H), 8.91 (s, 1H), 8.15 (d, J = 5.2 Hz, 1H), 7.36 - 6.82 (m, 7H), 5.35 (dd, J = 5.4, 12.8 Hz, 1H), 4.20 (t, J = 11.6 Hz, 1H), 3.48 (s, 2H), 3.33 (s, 3H), 3.18 (t, J = 6.0 Hz, 2H), 2.96 - 2.59 (m, 4H), 2.46 - 2.21 (m, 13H), 2.06 - 1.97 (m, 3H), 1.95 - 1.69 (m, 6H), 1.67 - 1.48 (m, 3H), 1.12 - 0.90 (m, 3H), 0.51 - 0.38 (m, 2H), 0.28 - 0.14 (m, 2H) |
| **I-111^{b}** | ZD | ZC | 835.6 | 11.11 (s, 1H), 10.14 - 9.80 (m, 2H), 9.08 (s, 1H), 8.26 - 8.15 (m, 1H), 8.08 (dd, J = 6.4 Hz, 1H), 7.58 (s, 1H), 7.33 - 7.01 (m, 4H), 5.45 - 5.32 (m, 1H), 4.30 - 4.20 (m, 1H), 3.31 (d, J = 7.6 Hz, 5H), 3.20 - 3.11 (m, 2H), 3.10 - 3.07 (m, 1H), 3.01 - 2.80 (m, 4H), 2.77 - 2.63 (m, 3H), 2.29 - 2.15 (m, 1H), 2.14 - 1.70 (m, 11H), 1.30 - 1.08 (m, 3H), 0.55 (d, J = 7.6 Hz, 2H), 0.32 (d, J = 4.4 Hz, 2H) |
| **I-112** | YP | PW | 869.5 | 11.08 (s, 1H), 9.69 (s, 1H), 8.92 (s, 1H), 8.18 (s, 1H), 8.15 (d, J = 5.6 Hz, 1H), 7.15 (t, J = 54.4 Hz, 1H), 7.10 (s, 1H), 7.03 - 6.99 (m, 1H), 6.96 (d, J = 4.4 Hz, 2H), 6.90 - 6.84 (m, 1H), 5.36 (dd, J = 4.8, 11.6 Hz, 1H), 4.28 - 4.12 (m, 1H), 4.00 - 3.90 (m, 1H), 3.57 (s, 3H), 3.18 (t, J = 6.0 Hz, 3H), 2.96 (t, J = 8.0 Hz, 2H), 2.91 - 2.80 (m, 2H), 2.76 - 2.68 (m, 1H), 2.65 - 2.58 (m, 1H), 2.06 (s, 1H), 2.03 (s, 3H), 2.03 - 1.65 (m, 16H), 1.57 - 1.44 (m, 1H), 1.13 - 0.94 (m, 3H), 0.50 - 0.39 (m, 2H), 0.25 - 0.16 (m, 2H) |
| **I-113** | YQ | PW | 898.3 | 11.06 (s, 1H), 9.68 (s, 1H), 8.91 (s, 1H), 8.20 - 8.12 (m, 3H), 7.15 (t, J = 54.4 Hz, 1H), 7.10 (s, 1H), 7.03 - 7.00 (m, 1H), 6.97 - 6.93 (m, 2H), 6.89 - 6.85 (m, 1H), 5.35 (dd, J = 5.2, 12.4 Hz, 1H), 4.23 - 4.14 (m, 1H), 3.75 (d, J = 10.8 Hz, 2H), 3.70 - 3.66 (m, 1H), 3.57 (s, 3H), 3.20 - 3.15 (m, 4H), 2.98 - 2.90 (m, 3H), 2.89 - 2.83 (m, 2H), 2.72 - 2.70 (m, 1H), 2.63 - 2.61(m, 3H), 2.33 (d, J = 5.2 Hz, 2H), 2.18 (s, 3H), 2.05 - 1.95 (m, 4H), 1.93 - 1.83 (m, 2H), 1.81 - 1.67 (m, 5H), 1.57 - 1.45 (m, 1H), 1.11 - 0.93 (m, 3H), 0.49 - 0.41 (m, 2H), 0.27 - 0.18 (m, 2H) |
| **I-114** | ZI | PW | 882.3 | 11.09 (s, 1H), 9.69 (s, 1H), 8.92 (s, 1H), 8.25 - 8.12 (m, 2H), 7.30 - 7.05 (m, 3H), 7.04 - 6.93 (m, 4H), 5.37 (dd, J = 12.8 Hz, J = 5.2 Hz, 1H), 4.27 - 4.15 (m, 1H), 3.58 (s, 3H), 3.18 (m, 3H), 3.06 (m, 3H), 2.95 - 2.80 (m, 2H), 2.75 - 2.57 (m, 1H), 2.43 - 2.30 (m, 3H), 2.23 - 2.13 (m, 7H), 2.06 (d, J = 10.4 Hz, 2H), 2.12 - 1.96 (m, 1H), 1.92 (d, J = 11.4 Hz, 2H), 1.86 - 1.70 (m, 6H), 1.69 - 1.50 (m, 3H), 1.15 - 0.97 (m, 3H), 0.49 - 0.40 (m, 2H), 0.25 - 0.18 (m, 2H) |
| **I-115^{b}** | PP | ZK | 884.7 | 11.08 (s, 1H), 9.72 - 9.65 (m, 1H), 8.95 - 8.92 (m, 1H), 8.25 - 8.16 (m, 3H), 7.61 (d, J = 6.4 Hz, 1H), 7.30 - 7.00 (m, 3H), 6.96 (d, J = 4.8 Hz, 2H), 6.90 - 6.82 (m, 1H), 5.36 (d, J = 4.8, 12.8 Hz, 1H), 4.32 - 4.15 (m, 3H), 3.57 (s, 3H), 3.55 (s, 1H), 3.44 - 3.41 (m, 3H), 2.99 - 2.93 (m, 2H), 2.90 - 2.83 (m, 1H), 2.75 - 2.56 (m, 4H), 2.27 (d, J = 7.6 Hz, 1H), 2.18 - 2.14 (m, 3H), 2.13 (s, 1H), 2.07 - 1.95 (m, 3H), 1.86 - 1.74 (m, 4H), 1.69 - 1.62 (m, 2H), 1.59 - 1.46 (m, 2H), 1.11 - 0.95 (m, 2H) |
| **I-116** | ZM | PW | 912.3 | 11.08 (s, 1 H), 9.68 (s, 1H), 8.91 (s, 1H), 8.30 - 8.10 (m, 2H), 7.30 - 6.80 (m, 7H), 5.50 - 5.30 (m, 1H), 4.30 - 4.10 (m, 1H), 3.75 (d, J = 11.0 Hz, 1H), 3.56 (s, 3H), 3.45 (s, 1H), 3.18 (t, J = 6.1 Hz, 2H), 2.97 - 2.84 (m, 3H), 2.79 ( d, J = 10.8 Hz, 1H), 2.73 - 2.63 (m, 3H), 2.59 (m, 1H), 2.45 - 2.29 (m, 5H), 2.17 (m, 5H), 2.06 - 1.94 (m, 4H), 1.94 - 1.83 (m, 2H), 1.81 - 1.68 (m, 5H), 1.57 - 1.45 (m, 3H), 1.11 - 0.95 (m, 3H), 0.45 (d, J = 7.1 Hz, 2H), 0.22 (d, J = 4.4 Hz, 2H) |
| **I-117** | YR | PW | 912.3 | 11.08 (s, 1H), 9.69 (s, 1H), 8.91 (s, 1H), 8.19 - 8.12 (m, 3H), 7.15 (t, J = 54.8 Hz, 1H), 7.10 - 7.07 (m, 1H), 7.10 - 7.06 (m, 1H), 6.97 - 6.93 (m, 1H), 6.90 - 6.85 (m, 1H), 5.41 - 5.30 (m, 1H), 4.26 - 4.14 (m, 1H), 3.75 (d, J = 10.4 Hz, 2H), 3.56 (s, 3H), 3.47 (s, 1H), 3.20 - 3.15 (m, 4H), 2.95 - 2.87 (m, 3H), 2.79 (d, J = 11.2 Hz, 1H), 2.74 - 2.68 (m, 1H), 2.65 - 2.57 (m, 1H), 2.52 (s, 3H), 2.46 - 2.38 (m, 3H), 2.36 - 2.34 (m, 1H), 2.19 (s, 3H), 2.06 - 1.95 (m, 4H), 1.93 - 1.83 (m, 2H), 1.80 - 1.69 (m, 4H), 1.59 - 1.47 (m, 3H), 1.12 - 0.93 (m, 3H), 0.50 - 0.40 (m, 2H), 0.26 - 0.17 (m, 2H) |
| **I-118** | ZM | PW | 869.2 | 11.09 (s, 1H), 9.71 (s, 1H), 8.92 (s, 1H), 8.25 - 8.19 (m, 1H), 8.17 (s, 1H), 8.16 - 8.13 (m, 1H), 7.32 - 6.97 (m, 6H), 6.89 - 6.83 (m, 1H), 5.37 - 5.29 (m, 1H), 4.23 - 4.14 (m, 1H), 3.42 - 3.36 (m, 4H), 3.32 (s, 3H), 3.17 (s, 2H), 2.96 - 2.83 (m, 1H), 2.67 (s, 6H), 2.17 - 1.95 (m, 7H), 1.93 - 1.67 (m, 8H), 1.44 (d, J = 9.2 Hz, 3H), 1.12 - 0.96 (m, 3H), 0.48 - 0.42 (m, 2H), 0.24 - 0.19 (m, 2H) |
| **I-119** | YT | PW | 883.7 | 11.08 (s, 1H), 9.69 (s, 1H), 8.92 (s, 1H), 8.17 (s, 1H), 8.15 (d, J = 5.4 Hz, 1H), 7.31 - 7.02 (m, 4H), 7.02 - 6.98 (m, 2H), 6.86 (d, J = 8.2 Hz, 1H), 5.33 (dd, J = 12.8 Hz, 1H), 4.24 - 4.14 (m, 1H), 3.35 (br s, 2H), 3.32 (s, 3H), 3.22 - 3.19 (m, 3H), 3.18 - 3.15 (m, 2H), 2.94 - 2.84 (m, 3H), 2.67 - 2.63 (m, 2H), 2.14 (d, J = 6.4 Hz, 2H), 2.04 (d, J = 12.4 Hz, 2H), 1.99 (d, J = 5.2 Hz, 1H), 1.93 - 1.85 (m, 4H), 1.84 - 1.78 (m, 2H), 1.78 - 1.71 (m, 2H), 1.69 - 1.54 (m, 4H), 1.54 - 1.48 (m, 1H), 1.26 - 1.14 (m, 2H), 1.11 - 0.97 (m, 3H), 0.48 - 0.42 (m, 2H), 0.22 (q, J = 4.8 Hz, 2H) |
| **I-120** | YU | PW | 883.7 | 11.10 (s, 1H), 9.71 (s, 1H), 8.93 (s, 1H), 8.16 - 8.12 (m, 2H), 7.30 - 7.01 (m, 4H), 6.96 (d, J = 4.4 Hz, 2H), 6.88 - 6.84 (m, 1H), 5.40 - 5.33 (m, 1H), 4.26 - 4.15 (m, 1H), 3.56 (s, 3H), 3.42 (t, J = 6.0 Hz, 2H), 3.25 (d, J = 6.0 Hz, 2H), 3.17 (t, J = 6.0 Hz, 2H), 2.99 - 2.93 (m, 4H), 2.91 - 2.82 (m, 1H), 2.74 - 2.67 (m, 1H), 2.59 (s, 1H), 2.30 - 2.24 (m, 2H), 2.08 - 2.00 (m, 4H), 2.00 - 1.95 (m, 1H), 1.92- 1.86 (m, 2H), 1.85 - 1.80 (m, 2H), 1.78 - 1.64 (m, 5H), 1.60 - 1.53 (m, 1H), 1.33 - 1.20 (m, 2H), 1.13 - 1.01 (m, 3H), 0.48 - 0.40 (m, 2H), 0.25 - 0.16 (m, 2H) |
| **I-121** | YW | PW | 841.5 | 11.07 (s, 1H), 9.69 (s, 1H), 8.91 (s, 1H), 8.18 (s, 1H), 8.16 - 8.12 (m, 1H), 7.30 - 7.03 (m, 4H), 7.03 - 6.99 (m, 2H), 6.87 (d, J = 7.6 Hz, 1H), 5.34 (dd, J = 12.8 Hz, 1H), 4.20 - 4.14 (m, 1H), 3.94 - 3.83 (m, 1H), 3.37 - 3.33 (m, 7H), 3.18 (t, J = 6.0 Hz, 2H), 2.94 - 2.85 (m, 1H), 2.75 - 2.54 (m, 8H), 2.09 - 2.01 (m, 2H), 2.01 - 1.95 (m, 1H), 1.88 - 1.81 (m, 3H), 1.80 - 1.68 (m, 2H), 1.63 - 1.54 (m, 1H), 1.53 - 1.42 (m, 1H), 1.17 - 1.00 (m, 3H), 0.51 - 0.40 (m, 2H), 0.26 - 0.17 (m, 2H) |
| **I-122** | ZN | PW | 841.5 | 11.08 (s, 1H), 9.67 (s, 1H), 8.91 (s, 1H), 8.18 (d, J = 5.6 Hz, 1H), 8.15 (d, J = 5.2 Hz, 1H), 7.29 - 7.00 (m, 4H), 6.97 (d, J = 4.4 Hz, 2H), 6.90 - 6.85 (m, 1H), 5.40 - 5.31 (m, 1H), 4.23 - 4.12 (m, 1H), 4.10 - 3.99 (m, 1H), 3.57 (s, 3H), 3.56 - 3.53 (m, 2H), 3.36 (t, J = 6.0 Hz, 2H), 3.18 (t, J = 6.0 Hz, 2H), 2.97 - 2.92 (m, 2H), 2.91 - 2.84 (m, 1H), 2.84 - 2.78 (m, 2H), 2.74 - 2.59 (m, 2H), 2.31 (d, J = 6.8 Hz, 2H), 2.06 - 2.00 (m, 2H), 1.99 - 1.90 (m, 1H), 1.87 - 1.80 (m, 3H), 1.78 - 1.65 (m, 2H), 1.59 - 1.51 (m, 1H), 1.42 - 1.30 (m, 1H), 1.14 - 0.99 (m, 3H), 0.48 - 0.42 (m, 2H), 0.25 - 0.19 (m, 2H) |
| **I-123** | ZO | PW | 889.6 | 11.14 (s, 1H), 9.69 (s, 1H), 8.92 (s, 1H), 8.35 - 8.23 (m, 2H), 8.18 (s, 2H), 8.15 (d, J = 5.2 Hz, 1H), 7.68 - 7.60 (m, 1H), 7.56 - 7.49 (m, 1H), 7.33 - 7.27 (m, 1H), 7.18 - 6.99 (m, 5H), 6.06 (s, 1H), 4.25 - 4.14 (m, 1H), 3.56 (t, J = 5.6 Hz, 2H), 3.24 - 3.22(m, 2H), 3.18 (t, J = 6.0 Hz, 3H), 3.11 - 2.96 (m, 2H), 2.15 - 1.96 (m, 10H), 1.94 - 1.82 (m, 5H), 1.82 - 1.82 (m, 1H), 1.80 - 1.70 (m, 2H), 1.61 - 1.48 (m, 3H), 1.11 - 0.96 (m, 3H), 0.48 - 0.42 (m, 2H), 0.25 - 0.20 (m, 2H) |
| **I-124** | ZP | PW | 843.5 | 11.08 (s, 1H), 9.69 (s, 1H), 8.92 (s, 1H), 8.20 - 8.10 (m, 2H), 7.29 - 6.95 (m, 6H), 6.90 - 6.85 (m, 1H), 5.36 (dd, J = 5.2, 12.4 Hz, 1H), 4.24 - 4.14 (m, 1H), 3.56 (s, 3H), 3.48 - 3.44 (m, 4H), 3.18 (t, J = 6.0 Hz, 2H), 3.00 - 2.93 (m, 2H), 2.92 - 2.82 (m, 1H), 2.77 - 2.59 (m, 3H), 2.25 - 2.15 (m, 5H), 2.09 - 1.67 (m, 10H), 1.60 - 1.44 (m, 1H), 1.13 - 0.95 (m, 3H), 0.51 - 0.38 (m, 2H), 0.27 - 0.17 (m, 2H) |
| **I-125** | TN | TE | 857.6 | 11.01 (s, 1H), 9.38 (s, 1H), 8.80 (d, J = 8.0 Hz, 1H), 8.38 (s, 1H), 8.28 (s, 1H), 8.23 (d, J = 1.2 Hz, 2H), 7.25 - 6.94 (m, 3H), 6.92 - 6.85 (m, 2H), 5.36 (d, J = 5.2, 12.6 Hz, 1H), 4.21 - 4.12 (m, 3H), 3.57 (s, 3H), 3.45 (d, J = 6.0 Hz, 2H), 3.28 (d, J = 3.6 Hz, 1H), 3.01 - 2.82 (m, 7H), 2.77 - 2.57 (m, 4H), 2.18 - 2.07 (m, 4H), 2.06 - 1.96 (m, 3H), 1.93 - 1.77 (m, 7H), 1.76 - 1.67 (m, 2H), 1.57 (s, 1H), 1.52 - 1.40 (m, 2H), 1.11 - 0.96 (m, 2H) |
| **I-126** | ZQ | ZC | 853.4 | 11.10 (s, 1H), 9.67 (s, 1H), 8.90 (s, 1H), 8.15 - 8.14 (m, 2H), 7.31 (s, 1H), 7.17 - 7.01 (m, 6H), 5.39 - 5.34 (m, 1H), 4.35 (s, 2H), 4.17 - 4.11 (m, 1H), 3.57 (t, J = 6.0 Hz, 2H), 3.33 (s, 1H), 3.17 (t, J = 6.0 Hz, 2H), 2.90 - 2.83 (m, 1H), 2.63 - 2.59 (m, 2H), 2.37 - 2.35 (m, 2H), 2.15 - 2.10 (m, 5H), 2.02 - 1.99 (m, 3H), 1.90 - 1.87 (m, 2H), 1.78 - 1.65 (m, 4H), 1.53 - 1.50 (m, 1H), 1.09 - 0.96 (m, 3H), 0.47 - 0.43 (m, 2H), 0.24 - 0.20 (m, 2H) |
| **I-127** | ZR | PW | 868.3 | 11.08 (s, 1H), 9.68 (s, 1H), 8.91 (s, 1H), 8.17 (s, 1H), 8.15 (d, J = 5.2 Hz, 1H), 7.31 - 7.08 (m, 3H), 7.08 - 6.95 (m, 4H), 5.35 (dd, J = 5.2, 12.8 Hz, 1H), 4.24 - 4.13 (m, 1H), 3.55 (s, 2H), 3.33 (s, 3H), 3.18 (t, J = 6.0 Hz, 2H), 2.95 - 2.84 (m, 2H), 2.59 - 2.76 (m, 1H), 2.47 - 2.38 (m, 2H), 2.17 - 2.11 (m, 4H), 2.11 - 2.08 (m, 2H), 2.06 - 1.97 (m, 5H), 1.94 - 1.86 (m, 2H), 1.81 - 1.66 (m, 4H), 1.62 - 1.40 (m, 3H), 1.19 - 0.89 (m, 5H), 0.48 - 0.39 (m, 2H), 0.25 - 0.16 (m, 2H) |
| **I-128** | ZS | PW | 843.6 | 11.07 (s, 1H), 9.69 (s, 1H), 8.92 (s, 1H), 8.17 (s, 1H), 8.14 (d, J = 5.6 Hz, 1H), 7.29 - 7.05 (m, 3H), 7.03 - 6.99 (m, 3H), 6.87 (d, J = 8.4 Hz, 1H), 5.33 (dd, J = 5.2, 12.8 Hz, 1H), 4.25 - 4.13 (m, 1H), 3.48 - 3.45 (m, 4H), 3.31 (s, 3H), 3.17 (t, J = 6.4 Hz, 2H), 2.94 - 2.83 (m, 1H), 2.75 - 2.61 (m, 4H), 2.53 - 2.51 (m, 2H), 2.22 - 2.16 (m, 5H), 2.07 - 1.96 (m, 3H), 1.91 (d, J = 11.6 Hz, 2H), 1.86 - 1.70 (m, 4H), 1.60 - 1.47 (m, 1H), 1.11 - 0.95 (m, 3H), 0.48 - 0.42 (m, 2H), 0.24 - 0.19 (m, 2H) |
| **I-129** | ZU | PW | 843.5 | 11.06 (s, 1H), 9.69 - 9.65 (m, 1H), 8.91 (s, 1H), 8.18 - 8.12 (m, 2H), 7.30 - 6.99 (m, 5H), 6.91 (d, J = 8.0 Hz, 1H), 5.35 - 5.31 (m, 1H), 4.21 - 4.15 (m, 1H), 3.57 (t, J = 7.2 Hz, 2H), 3.43 (t, J = 7.2 Hz, 2H), 3.31 (s, 3H), 3.18 (t, J = 7.0 Hz, 2H), 2.93 - 2.80 (m, 3H), 2.76 - 2.68 (m, 2H), 2.63 - 2.58 (m, 2H), 2.16 - 2.08 (m, 5H), 2.05 - 1.96 (m, 3H), 1.92 - 1.81 (m, 2H), 1.80 - 1.70 (m, 2H), 1.67 - 1.60 (m, 2H), 1.55 - 1.48 (m, 1H), 1.08 - 0.97 (m, 3H), 0.48 - 0.42 (m, 2H), 0.25 - 0.20 (m, 2H) |
| **I-130** | VD | PW | 799.5 | 11.07 (s, 1H), 9.68 (s, 1H), 8.91 (s, 1H), 8.18 (s, 1H), 8.16 - 8.14 (m, 1H), 7.29 - 7.00 (m, 4H), 6.97 (d, J = 4.4 Hz, 2H), 6.92 - 6.87 (m, 1H), 5.40 - 5.33 (m, 1H), 4.26 - 4.14 (m, 1H), 3.57 (s, 3H), 3.18 (t, J = 6.0 Hz, 2H), 2.93 (t, J = 7.6 Hz, 2H), 2.90 - 2.83 (m, 1H), 2.76 - 2.68 (m, 1H), 2.64 - 2.59 (m, 1H), 2.45 (t, J = 6.4 Hz, 2H), 2.22 (s, 3H), 2.22 - 2.18 (m, 2H), 2.08 - 2.02 (m, 2H), 2.01 - 1.95 (m, 1H), 1.94 - 1.87 (m, 2H), 1.82 - 1.68 (m, 4H), 1.61 - 1.51 (m, 1H), 1.11 - 0.98 (m, 3H), 0.48 - 0.42 (m, 2H), 0.25 - 0.19 (m, 2H) |
| **I-161** | ACL | PW | 1120.8 | 9.68 (s, 1H), 8.91 (s, 1H), 8.19 (s, 1H), 8.17 (s, 1H), 8.14 (d, *J* = 5.2 Hz, 1H), 8.02 - 7.88 (m, 1H), 7.31 - 6.75 (m, 11H), 4.99 - 4.80 (m, 2H), 4.79 - 4.61 (m, 2H), 4.23 - 4.13 (m, 1H), 4.11 - 4.03 (m, 2H), 3.79 - 3.73 (m, 2H), 3.64 - 3.60 (m, 3H), 3.59 - 3.56 (m, 4H), 3.18 (s, 2H), 3.04 (d, *J* = 7.2 Hz, 2H), 3.01 - 2.95 (m, 2H), 2.89 - 2.82 (m, 2H), 2.70 ( d, *J* = 8.0 Hz, 2H), 2.59 (d, *J* = 6.4 Hz, 2H), 2.52 (d, *J* = 2.0 Hz, 3H), 2.20 - 2.14 (m, 3H), 2.08 - 2.01 (m, 2H), 1.89 (d, *J* = 13.2 Hz, 2H), 1.77 (d, *J* = 2.4 Hz, 1H), 1.75 - 1.67 (m, 2H), 1.64 - 1.51 (m, 3H), 1.15 - 1.05 (m, 5H), 1.05 - 0.92 (m, 9H), 0.49 - 0.41 (m, 2H), 0.25 - 0.18 (m, 2H) |
| **I-162** | ZM | TE | 857.5 | 11.07 (s, 1H), 9.36 (s, 1H), 8.85 (d, *J* = 8.0 Hz, 1H), 8.39 (s, 1H), 8.30 (s, 1H), 8.17 (s, 1H), 7.28 - 6.96 (m, 3H), 6.93 (d, *J* = 8.0 Hz, 1H), 6.87 (dd, *J =* 0.8, 8.0 Hz, 1H), 5.34 (dd, *J* = 5.2, 12.8 Hz, 1H), 4.23 - 4.13 (m, 1H), 3.95 - 3.85 (m, 4H), 3.40 (t, *J =* 6.4 Hz, 2H), 3.32 (s, 3H), 3.31 - 3.25 (m, 1H), 3.07 (t, *J* = 4.4 Hz, 4H), 2.95 - 2.85 (m, 1H), 2.82 - 2.73 (m, 2H), 2.72 - 2.58 (m, 4H), 2.31 - 2.11 (m, 4H), 2.08 - 1.96 (m, 3H), 1.94 - 1.68 (m, 8H), 1.67 - 1.43 (m, 3H), 1.13 - 0.94 (m, 2H) |
| **I-163** | YT | TE | 871.6 | 11.09 (s, 1H), 9.38 (s, 1H), 8.82 (d, *J* = 8.0 Hz, 1H), 8.38 (s, 1H), 8.28 (s, 1H), 8.19 (s, 1H), 7.25 - 6.97 (m, 3H), 6.91 (d, *J =* 8.0 Hz, 1H), 6.86 (d, *J =* 7.6 Hz, 1H), 5.39 - 5.29 (m, 1H), 4.24 - 4.13 (m, 1H), 3.83 (s, 4H), 3.38 - 3.33 (m, 2H), 3.32 (s, 3H), 3.21 (d, *J* = 6.0 Hz, 2H), 2.98 - 2.93 (m, 4H), 2.89 - 2.80 (m, 1H), 2.76 - 2.55 (m, 4H), 2.21 (d, *J* = 6.8 Hz, 2H), 2.08 - 1.93 (m, 6H), 1.88 (d, *J =* 12.0 Hz, 2H), 1.84 - 1.78 (m, 2H), 1.77 - 1.71 (m, 2H), 1.66 (d, *J =* 13.6 Hz, 2H), 1.62 - 1.45 (m, 3H), 1.29 - 1.16 (m, 2H), 1.13 - 0.96 (m, 2H) |
| **I-164** | YU | TE | 871.6 | 11.09 (s, 1H), 9.39 (s, 1H), 8.78 (d, *J* = 8.0 Hz, 1H), 8.38 (s, 1H), 8.26 (s, 1H), 8.21 (s, 1H), 7.24 - 6.93 (m, 3H), 6.89 (d, *J* = 8.0 Hz, 1H), 6.87 - 6.84 (m, 1H), 5.41 - 5.32 (m, 1H), 4.21 - 4.14 (m, 1H), 3.76 (s, 4H), 3.56 (s, 3H), 3.42 (t, *J* = 6.0 Hz, 2H), 3.23 (d, *J* = 6.0 Hz, 2H), 3.02 - 2.91 (m, 2H), 2.91 - 2.88 (m, 1H), 2.88 - 2.82 (m, 6H), 2.73 - 2.59 (m, 2H), 2.13 (d, *J* = 6.8 Hz, 2H), 2.08 - 1.94 (m, 3H), 1.92 - 1.77 (m, 6H), 1.76 - 1.68 (m, 2H), 1.67 - 1.46 (m, 4H), 1.28 - 1.13 (m, 2H), 1.11 - 0.96 (m, 2H) |
| **I-165** | ABV | TE | 844.4 | 11.20 (s, 1H), 9.40 (s, 1H), 8.79 (d, *J* = 8.0 Hz, 1H), 8.38 (m, 1H), 8.29 - 8.21 (m, 2H), 7.26 - 6.94 (m, 4H), 6.90 (d, *J* = 8.0 Hz, 1H), 5.36 (dd, *J* = 5.2, 12.8 Hz, 1H), 4.21 - 4.14 (m, 1H), 3.89 - 3.75 (m, 4H), 3.43 (t, *J =* 6.0 Hz, 2H), 3.30 - 3.20 (m, 1H), 3.01 - 2.82 (m, 5H), 2.76 (t, *J* = 7.6 Hz, 2H), 2.72 - 2.61 (m, 4H), 2.30 - 2.01 (m, 7H), 1.94 - 1.67 (m, 8H), 1.61 - 1.53 (m, 1H), 1.44 (q, *J* = 9.2 Hz, 2H), 1.22 - 0.75 (m, 2H) |
| **I-166** | ADP | TE | 844.4 | 11.21 (s, 1H), 10.11 (s, 1H), 9.61 (s, 2H), 9.35 (s, 1H), 8.91 (d, *J* = 7.6 Hz, 1H), 8.40 (s, 1H), 8.33 (s, 1H), 7.30 - 6.96 (m, 5H), 5.38 (dd, *J* = 3.2, 12.2 Hz, 1H), 4.23 (t, *J* = 12.0 Hz, 1H), 4.08 (s, 4H), 3.55 - 3.37 (m, 4H), 3.22 (s, 4H), 3.01 - 2.84 (m, 5H), 2.75 - 2.62 (m, 4H), 2.12 - 1.98 (m, 6H), 1.94 - 1.76 (m, 7H), 1.25 - 1.12 (m, 2H) |
| **I-167** | ABW | PW | 856.5 | 11.11 (s, 1H), 9.68 (s, 1H), 8.92 (s, 1H), 8.18 - 8.12 (m, 2H), 7.80 - 7.65 (m, 3H), 7.33 - 6.92 (m, 4H), 5.12 (dd, *J =* 5.2, 12.8 Hz, 1H), 4.24 - 4.12 (m, 1H), 3.42 - 3.38 (m, 4H), 3.18 (t, *J =* 6.0 Hz, 2H), 3.11 - 3.05 (m, 2H), 2.95 - 2.83 (m, 1H), 2.64 - 2.52 (m, 2H), 2.35 - 2.29 (m, 2H), 2.16 - 2.08 (m, 5H), 2.08 - 1.99 (m, 3H), 1.93 - 1.80 (m, 4H), 1.80 - 1.68 (m, 2H), 1.66 - 1.58 (m, 2H), 1.57 - 1.46 (m, 1H), 1.15 - 0.87 (m, 3H), 0.49 - 0.41 (m, 2H), 0.26 - 0.18 (m, 2H) |
| **I-168** | ADR | PW | 857.5 | 11.09 (s, 1H), 9.68 (s, 1H), 8.92 (s, 1H), 8.18 (s, 1H), 8.17 - 8.12 (m, 2H), 7.59 (dd, *J* = 7.2, 8.4 Hz, 1H), 7.29 - 7.00 (m, 6H), 6.62 (t, *J* = 5.6 Hz, 1H), 5.06 (dd, *J* = 5.2, 12.8 Hz, 1H), 4.22 - 4.04 (m, 1H), 3.61 - 3.55 (m, 2H), 3.50 - 3.45 (m, 4H), 3.18 (t, *J* = 6.0 Hz, 2H), 2.94 - 2.82 (m, 1H), 2.64 - 2.53 (m, 2H), 2.34 - 2.30 (m, 2H), 2.10 (s, 3H), 2.09 - 1.98 (m, 5H), 1.88 - 1.82 (m, 2H), 1.77 - 1.60 (m, 4H), 1.57 - 1.46 (m, 1H), 1.13 - 0.90 (m, 3H), 0.49 - 0.40 (m, 2H), 0.25 - 0.18 (m, 2H) |
| **I-169** | ADT | PW | 853.6 | 11.17 - 11.04 (m, 1H), 9.68 (s, 1H), 8.91 (s, 1H), 8.20 - 8.12 (m, 2H), 7.94 - 7.89 (m, 1H), 7.87 - 7.77 (m, 2H), 7.30 - 7.04 (m, 3H), 7.03 - 6.99 (m, 1H), 5.13 (dd, *J* = 5.6, 12.8 Hz, 1H), 4.23 - 4.15 (m, 1H), 3.93 (s, 2H), 3.18 - 3.16 (m, 2H), 2.94 - 2.85 (m, 4H), 2.25 - 2.16 (m, 6H), 2.10 - 1.99 (m, 6H), 1.94 - 1.85 (m, 2H), 1.75 (d, *J* = 12.0 Hz, 2H), 1.68 - 1.60 (m, 2H), 1.53 - 1.42 (m, 3H), 1.10 - 0.94 (m, 3H), 0.48 - 0.42 (m, 2H), 0.22 - 0.19 (m, *J =* 4.6 Hz, 2H) |
| **I-170** | ADV | PW | 897.5 | 11.03 (s, 1H), 9.80 (s, 1H), 9.65 (s, 1H), 9.04 (s, 1H), 8.13 (s, 1H), 8.02 (d, *J =* 6.4 Hz, 1H), 7.58 - 7.50 (m, 2H), 7.27 - 7.09 (m, 2H), 7.08 - 7.02 (m, 1H), 7.00 - 6.95 (m, 1H), 6.59 - 6.44 (m, 1H), 5.02 - 4.96 (m, 1H), 4.22 - 4.15 (m, 1H), 3.79 - 3.72 (m, 2H), 3.68 - 3.62 (m, 2H), 3.28 - 3.26 (m, 2H), 2.92 - 2.86 (m, 3H), 2.84 - 2.79 (m, 1H), 2.58 - 2.47 (m, 1H), 2.05 - 1.99 (m, 3H), 1.87 - 1.73 (m, 4H), 1.64 - 1.48 (m, 5H), 1.26 - 1.04 (m, 11H), 0.80 - 0.75 (m, 1H), 0.55 - 0.47 (m, 2H), 0.30 - 0.24 (m, 2H) |
| **I-171** | ABX | PW | 868.2 | 11.12 (s, 1H), 9.70 (s, 1H), 8.92 (s, 1H), 8.17 (s, 1H), 8.14 (d, *J* = 5.2 Hz, 1H), 7.81 - 7.66 (m, 3H), 7.33 - 6.97 (m, 4H), 5.13 (dd, *J* = 5.2, 12.8 Hz, 1H), 4.24 - 4.13 (m, 1H), 3.41 (t, *J* = 6.0 Hz, 2H), 3.25 - 3.21 (m, 1H), 3.17 (t, *J* = 6.0 Hz, 2H), 3.09 (t, *J* = 7.2 Hz, 2H), 2.94 - 2.85 (m, 1H), 2.66 - 2.60 (m, 2H), 2.58 - 2.52 (m, 2H), 2.13 - 1.94 (m, 7H), 1.91 - 1.67 (m, 8H), 1.60 - 1.50 (m, 1H), 1.46 - 1.33 (m, 2H), 1.13 - 0.94 (m, 3H), 0.50 - 0.40 (m, 2H), 0.26 - 0.17 (m, 2H) |
| **I-172^{b}** | TN | ABP | 832.6 | 11.10 (s, 1H), 9.47 (s, 1H), 9.21 - 9.09 (m, 1H), 8.57 (d, *J* = 7.6 Hz, 1H), 8.25 (s, 1H), 8.16 (d, *J* = 2.8 Hz, 2H), 7.00 - 6.95 (m, 3H), 6.92 - 6.86 (m, 1H), 6.45 (d, *J* = 7.6 Hz, 1H), 5.42 (s, 1H), 5.37 (d, *J* = 4.8 Hz, 1H), 5.23 (s, 1H), 4.19 - 4.08 (m, 1H), 3.68 (s, 1H), 3.59 (d, *J* = 9.2 Hz, 4H), 3.55 - 3.46 (m, 6H), 3.03 - 2.93 (m, 9H), 2.11 (s, 4H), 1.95 (s, 9H), 1.29 - 1.13 (m, 4H), 0.56 - 0.50 (m, 2H), 0.29 - 0.22 (m, 2H) |
| **I-173^{b}** | WX | ABP | 817.3 | 11.11 (s, 1H), 9.44 (s, 1H), 8.56 (d, *J* = 7.6 Hz, 1H), 8.25 - 8.21 (m, 1H), 8.16 (s, 1H), 8.12 (s, 1H), 7.10 - 7.03 (m, 1H), 6.95 (t, *J* = 8.0 Hz, 1H), 6.90 - 6.83 (m, 1H), 6.43 (d, *J* = 7.6 Hz, 1H), 5.43 - 5.34 (m, 2H), 5.20 (s, 1H), 4.09 - 4.01 (m, 1H), 3.67 (s, 3H), 3.61 (s, 2H), 3.29 - 3.25 (m, 2H), 2.91 - 2.83 (m, 3H), 2.77 - 2.69 (m, 1H), 2.65 - 2.59 (m, 1H), 2.37 - 2.33 (m, 1H), 2.20 (d, *J* = 6.8 Hz, 2H), 2.17 (s, 3H), 2.10 (s, 3H), 2.04 - 1.93 (m, 5H), 1.87 (d, *J* = 12.0 Hz, 2H), 1.76 - 1.67 (m, 2H), 1.63 (d, *J* = 12.0 Hz, 2H), 1.52 - 1.44 (m, 1H), 1.43 - 1.33 (m, 2H), 1.10 - 0.95 (m, 3H), 0.55 - 0.49 (m, 2H), 0.27 - 0.22 (m, 2H) |
| **I-174** | AED | PW | 766.5 | 9.68 (s, 1H), 8.92 (s, 1H), 8.18 (s, 1H), 8.15 (d, *J* = 5.2 Hz, 1H), 7.31 - 6.97 (m, 4H), 4.23 - 4.15 (m, 1H), 3.56 - 3.38 (m, 11H), 3.18 (t, *J* = 6.0 Hz, 2H), 2.24 - 2.16 (m, 5H), 2.10 - 2.01 (m, 5H), 1.95 - 1.85 (m, 2H), 1.81 - 1.49 (m, 16H), 1.11 - 0.96 (m, 3H), 0.48 - 0.42 (m, 2H), 0.25 - 0.20 (m, 2H) |
| **I-175** | AED | TE | 754.6 | 9.82 (s, 2H), 9.34 (s, 1H), 8.90 (d, *J* = 7.6 Hz, 1H), 8.39 (s, 1H), 8.32 (s, 1H), 7.33 - 6.85 (m, 2H), 4.27 - 4.18 (m, 1H), 4.15 - 4.03 (m, 4H), 3.87 - 3.76 (m, 2H), 3.61 - 3.53 (m, 4H), 3.47 (s, 3H), 3.37 - 3.16 (m, 6H), 3.15 - 3.04 (m, 1H), 2.99 - 2.89 (m, 1H), 2.79 (d, *J =* 4.4 Hz, 3H), 2.11 - 1.76 (m, 10H), 1.67 - 1.49 (m, 12H), 1.25 - 1.10 (m, Hz, 2H) |
| **I-176** | ZM | ABC | 858.6 | 11.07 (s, 1H), 9.39 (s, 1H), 8.82 (d, *J* = 7.8 Hz, 1H), 8.37 (s, 1H), 8.28 (s, 1H), 7.26 - 6.94 (m, 3H), 6.90 - 6.85 (m, 2H), 5.33 (dd, *J* = 5.2, 12.8 Hz, 1H), 4.17 (t, *J* = 11.6 Hz, 1H), 3.82 -3.77 (m, 4H), 3.74 -3.72 (m,, 4H), 3.39 (t, *J* = 6.0 Hz, 2H), 3.32 (s, 3H), 3.24 (s, 1H), 2.94 - 2.84 (m, 1H), 2.73 - 2.56 (m, 6H), 2.13 (br d, *J* = 6.8 Hz, 2H), 2.10 - 1.95 (m, 5H), 1.91 - 1.68 (m, 8H), 1.56 (s, 1H), 1.50 - 1.39 (m, 2H), 1.07 - 0.98 (m, 2H) |
| **I-177^{b}** | AEK | WW | 829.5 | 11.09 (s, 1H), 9.39 (s, 1H), 8.82 (d, *J* = 7.6 Hz, 1H), 8.39 (s, 1H), 8.28 (s, 1H), 7.24 - 6.85 (m, 5H), 5.37 (dd, *J* = 5.2, 12.4 Hz, 1H), 4.26 - 4.13 (m, 1H), 3.82 - 3.75 (m, 4H), 3.74 - 3.70 (m, 4H), 3.67 (s, 3H), 3.60 (s, 2H), 2.99 - 2.62 (m, 6H), 2.38 - 2.33 (m, 2H), 2.10 - 1.85 (m, 10H), 1.66 - 1.59 (m, 2H), 1.39 - 1.33 (m, 2H), 1.32 - 1.25 (m, 1H), 1.17 - 1.04 (m, 2H) |
| **I-178^{b}** | AEK | SK | 829.4 | 11.12 (s, 1H), 9.42 (s, 1H), 8.82 (d, *J* = 8.0 Hz, 1H), 8.53 - 8.41 (m, 1H), 8.29 (s, 1H), 7.53 - 7.44 (m, 1H), 7.33 - 7.11 (m, 3H), 6.91 (d, *J* = 8.0 Hz, 1H), 5.42 (dd, *J* = 5.2, 12.8 Hz, 1H), 4.61 - 4.52 (m, 1H), 4.29 (s, 2H), 3.79 (s, 3H), 3.74 - 3.70 (m, 4H), 3.64 - 3.58 (m, 4H), 3.16 - 2.96 (m, 6H), 2.95 - 2.79 (m, 4H), 2.77 - 2.70 (m, 2H), 2.34 - 2.22 (m, 5H), 2.07 - 1.99 (m, 1H), 1.90 - 1.82 (m, 2H), 1.71 - 1.48 (m, 5H) |
| **I-179^{b}** | AEJ | AEM | 804.5 | 11.06 (s, 1H), 9.40 (s, 1H), 8.81 (d, *J* = 8.0 Hz, 1H), 8.38 (s, 1H), 8.29 (s, 1H), 7.24 - 6.95 (m, 3H), 6.92 - 6.85 (m, 2H), 5.32 (dd, *J* = 5.2, 12.4 Hz, 1H), 4.27 - 4.14 (m, 1H), 3.78 - 3.78 (m, 1H), 3.84 - 3.67 (m, 9H), 3.46 - 3.41 (m, 2H), 3.41-3.40 (m, 4H), 3.32 (s, 3H), 2.98 - 2.83 (m, 4H), 2.71-2.70 (m, 1H), 2.09 - 1.95 (m, 6H), 1.90 (d, *J* = 8.4 Hz, 2H), 1.85 - 1.78 (m, 2H), 1.71 - 1.63 (m, 2H) |
| **I-180^{b}** | AEJ | ADI | 804.5 | 11.09 (s, 1H), 9.43 (s, 1H), 8.84 (d, *J* = 7.6 Hz, 1H), 8.45 (s, 1H), 8.30 (s, 1H), 7.27 - 6.95 (m, 3H), 6.92 (d, *J* = 8.0 Hz, 1H), 6.90 - 6.85 (m, 1H), 5.37 (dd, *J* = 5.2, 12.4 Hz, 1H), 4.62 - 4.49 (m, 1H), 3.80 (s, 4H), 3.73 (d, *J* = 4.4 Hz, 4H), 3.67 (d, *J* = 11.6 Hz, 2H), 3.61 - 3.56 (m, 3H), 3.48 - 3.46 (m, 5H), 3.21 - 3.07 (m, 4H), 3.04 - 2.94 (m, 2H), 2.93 - 2.83 (m, 1H), 2.77 - 2.68 (m, 1H), 2.66 - 2.58 (m, 1H), 2.29 (s, 1H), 2.23 - 2.13 (m, 2H), 2.05 - 1.82 (m, 5H) |
| **I-181^{b}** | AAY | ABY | 840.6 | 11.09 (s, 1H), 9.71 (s, 1H), 8.92 (s, 1H), 8.20 - 8.18 (m, 2H), 8.15 (d, *J* = 4.8 Hz, 1H), 7.30 - 7.05 (m, 4H), 7.03 - 7.00 (m, 1H), 5.35 (dd, *J* = 4.8, 12.4 Hz, 1H), 4.29 - 4.16 (m, 1H), 3.49 (s, 3H), 3.20 - 3.15 (m, 4H), 3.00 - 2.92 (m, 3H), 2.92 - 2.85 (m, 1H), 2.84 - 2.78 (m, 2H), 2.65 - 2.57 (m, 1H), 2.52 - 2.52 (m, 2H), 2.09 - 1.84 (m, 10H), 1.64 (d, *J =* 12.4 Hz, 2H), 1.42 - 1.33 (m, 2H), 1.32 - 1.24 (m, 1H), 1.23 - 1.11 (m, 2H), 1.10 - 1.01 (m, 1H), 0.48 - 0.42 (m, 2H), 0.26 - 0.18 (m, 2H) |
| **I-182^{b}** | ACX | SK | 826.6 | 11.11 (s, 1H), 10.94 (d, *J* = 2.8 Hz, 1H), 10.64 (d, *J* = 4.0 Hz, 1H), 10.00 (s, 1H), 9.13 (s, 1H), 8.37 - 8.01 (m, 2H), 7.64 (s, 1H), 7.54 (s, 1H), 7.39 - 7.06 (m, 4H), 5.43 (dd, *J* = 5.6, 12.8 Hz, 1H), 4.68 - 4.50 (m, 1H), 4.43 - 4.21 (m, 2H), 3.72 - 3.63 (m, 2H), 3.38 - 3.36 (m, 2H), 3.36 (s, 3H), 3.35 - 3.32 (m, 3H), 3.10 (d, *J* = 11.6 Hz, 2H), 3.06 - 2.98 (m, 2H), 2.97 - 2.90 (m, 1H), 2.90 - 2.78 (m, 2H), 2.76 - 2.59 (m, 2H), 2.57 - 2.52 (m, 1H), 2.40 - 2.31 (m, 1H), 2.30 - 2.19 (m, 2H), 2.17 - 1.94 (m, 4H), 1.77 - 1.57 (m, 2H), 1.24 - 1.09 (m, 1H), 0.59 - 0.52 (m, 2H), 0.36 - 0.30 (m, 2H) |
| **I-183** | ACV | ABC | 869.3 | 11.09 (s, 1H), 9.41 (s, 1H), 8.83 (d, *J* = 8.0 Hz, 1H), 8.39 (s, 1H), 8.29 (s, 1H), 7.24 - 6.88 (m, 5H), 5.43 - 5.34 (m, 1H), 4.28 - 4.19 (m, 1H), 4.10 - 4.02 (m, 1H), 4.00 - 3.87 (m, 3H), 3.80 (s, 4H), 3.73 (d, *J* = 4.6 Hz, 4H), 3.61 (s, 3H), 3.49 (s, 1H), 3.22 - 3.16 (m, 2H), 3.11 - 2.99 (m, 2H), 2.92 (d, *J* = 6.8 Hz, 5H), 2.77 - 2.71 (m, 1H), 2.22 - 1.76 (m, 12H), 1.26 - 1.12 (m, 3H) |
| **I-184^{b}** | PP | AEP | 868.6 | 11.07 (s, 1H), 8.85 (s, 1H), 8.38 - 8.27 (m, 1H), 8.04 - 8.00 (m, 1H), 7.88 (s, 1H), 7.79 - 7.74 (m, 1H), 7.74 - 7.66 (m, 1H), 6.99 - 6.91 (m, 2H), 6.90 - 6.78 (m, 1H), 5.35 (dd, *J =* 5.2, 12.4 Hz, 1H), 5.02 - 4.80 (m, 1H), 4.54 (dd, *J =* 3.2, 11.2 Hz, 1H), 4.26 (dd, *J =* 6.0, 11.2 Hz, 1H), 4.15 - 4.05 (m, 1H), 3.98 (s, 3H), 3.56 (s, 3H), 3.43 - 3.40 (m, 4H), 2.98 - 2.84 (m, 3H), 2.76 - 2.55 (m, 4H), 2.36 - 2.30 (m, 2H), 2.17 - 1.97 (m, 8H), 1.88 - 1.78 (m, 4H), 1.68 - 1.58 (m, 4H), 1.54 - 1.41 (m, 3H), 1.01 (t, *J* = 7.4 Hz, 3H), 0.97 - 0.87 (m, 2H) |
| **I-185^{b}** | QI | AFM | 868.6 | 11.07 (s, 1H), 8.86 (s, 1H), 8.29 (s, 1H), 8.01 (s, 1H), 7.89 (s, 1H), 7.76 (s, 1H), 7.73 (s, 1H), 7.05 - 6.96 (m, 2H), 6.87 (d, *J* = 8.0 Hz, 1H), 5.32 (dd, *J* = 5.2, 12.8 Hz, 1H), 5.01 - 4.76 (m, 1H), 4.54 (dd, *J* = 3.2*,* 11.2 Hz, 1H), 4.27 (dd, *J* = 6.4, 11.2 Hz, 1H), 4.15 - 4.05 (m, 1H), 3.98 (s, 3H), 3.43 - 3.38 (m, 4H), 3.31 (s, 3H), 2.96 - 2.82 (m, 1H), 2.76 - 2.54 (m, 6H), 2.34 (t, *J* = 6.8 Hz, 2H), 2.13 (s, 3H), 2.11 - 1.94 (m, 5H), 1.88 - 1.75 (m, 4H), 1.69 - 1.41 (m, 7H), 1.08 - 0.79 (m, 5H) |
| **I-186^{b}** | TN | AFM | 880.6 | 11.10 (s, 1H), 8.88 (s, 1H), 8.28 (s, 1H), 8.02 (s, 1H), 7.92 - 7.84 (m, 1H), 7.80 - 7.68 (m, 2H), 6.96 (d, *J* = 4.8 Hz, 2H), 6.90 - 6.83 (m, 1H), 5.36 (dd, *J* = 5.2, 12.4 Hz, 1H), 4.99 - 4.81 (m, 1H), 4.54 (dd, *J* = 3.6, 11.2 Hz, 1H), 4.26 (dd, *J* = 6.4, 11.2 Hz, 1H), 4.12 - 4.05 (m, 1H), 3.97 (s, 3H), 3.57 (s, 3H), 3.44 (d, *J* = 6.4 Hz, 4H), 2.99 - 2.93 (m, 2H), 2.70 - 2.54 (m, 6H), 2.10 - 1.95 (m, 7H), 1.86 - 1.76 (m, 6H), 1.62 - 1.39 (m, 7H), 1.04 - 0.87 (m, 5H) |
| **I-187^{b}** | ZM | AFM | 880.6 | 11.08 (s, 1H), 8.86 (s, 1H), 8.28 (s, 1H), 8.01 (s, 1H), 7.89 (s, 1H), 7.80 - 7.67 (m, 2H), 7.07 - 6.96 (m, 2H), 6.87 (d, *J* = 8.0 Hz, 1H), 5.33 (dd, *J* = 5.2, 12.8 Hz, 1H), 5.00 - 4.81 (m, 1H), 4.54 (dd, *J* = 3.6, 11.2 Hz, 1H), 4.29 - 4.20 (m, 1H), 4.13 - 4.05 (m, 1H), 3.98 (s, 3H), 3.47 - 3.43 (m, 2H), 3.32 (s, 3H), 3.26 - 3.20 (m, 2H), 2.93 - 2.84 (m, 1H), 2.71 - 2.61 (m, 6H), 2.12 - 1.94 (m, 7H), 1.88 - 1.73 (m, 6H), 1.65 - 1.34 (m, 7H), 1.10 - 0.86 (m, 6H) |
| **I-188^{b}** | PP | AIK | 911.6 | 11.08 (s, 1H), 9.30 - 9.25 (m,1H), 8.86 (s, 1H), 8.77 - 8.68 (m, 1H), 8.53 - 8.47 (m, 1H), 7.88 - 7.79 (m, 2H), 7.69 - 7.60 (m, 1H), 6.98 - 6.78 (m, 4H), 5.39 - 5.31 (m, 1H), 5.00 - 4.83 (m, 1H), 4.62 - 4.55 (m, 1H), 4.40 - 4.28 (m, 2H), 4.15 - 4.07 (m, 1H), 3.98 (s, 3H), 3.82 - 3.74 (m, 2H), 3.56 (s, 3H), 2.97 (d, *J* = 7.2 Hz, 2H), 2.64 - 2.57 (m, 2H), 2.40 - 2.35 (m, 2H), 2.25 - 2.17 (m, 3H), 2.14 (s, 3H), 2.03 - 1.89 (m, 4H), 1.86 - 1.75 (m, 4H), 1.68 - 1.61 (m, 4H), 1.58 - 1.47 (m, 2H), 1.14 - 0.99 (m, 5H) |
| **I-189** | AFM | WX | 865.6 | 11.10 (s, 1H), 8.86 (s, 1H), 8.28 (s, 1H), 8.01 (s, 1H), 7.89 (s, 1H), 7.76 (s, 1H), 7.73 (s, 1H), 7.07 (d, *J* = 7.6 Hz, 1H), 6.95 (t, *J* = 7.6 Hz, 1H), 6.86 (d, *J* = 7.6 Hz, 1H), 5.38 (dd, *J* = 5.2, 12.8 Hz, 1H), 4.99 - 4.81 (m, 1H), 4.54 (dd, *J* = 3.2, 11.2 Hz, 1H), 4.26 (dd, *J* = 6.0, 10.8 Hz, 1H), 4.09 - 4.06 (m, 1H), 3.97 (s, 3H), 3.67 (s, 3H), 3.61 (s, 2H), 2.91 - 2.83 (m, 3H), 2.73 - 2.64 (m, 2H), 2.62 - 2.53 (m, 2H), 2.36 - 2.29 (m, 1H), 2.18 (d, *J* = 7.2 Hz, 2H), 2.16 (s, 3H), 2.09 - 1.92 (m, 5H), 1.80 (d, *J* = 12.0 Hz, 2H), 1.66 - 1.54 (m, 4H), 1.51 - 1.32 (m, 5H), 1.03 - 0.97 (m, 3H), 0.96 - 0.84 (m, 2H) |
| **I-190^{b}** | AAD | AFM | 865.5 | 11.11 (s, 1H), 8.87 (s, 1H), 8.29 (s, 1H), 8.01 (s, 1H), 7.89 (s, 1H), 7.76 (s, 1H), 7.73 (s, 1H), 7.12 - 6.93 (m, 3H), 5.35 (dd, *J* = 5.2, 12.8 Hz, 1H), 5.00 - 4.81 (m, 1H), 4.54 (dd, *J* = 3.6, 11.2 Hz, 1H), 4.26 (dd, *J* = 6.4, 11.2 Hz, 1H), 4.12 - 4.05 (m, 1H), 3.97 (s, 3H), 3.46 (s, 2H), 3.34 (s, 3H), 2.88 - 2.84 (m, 2H), 2.74 - 2.53 (m, 4H), 2.31 - 2.13 (m, 6H), 2.10 - 1.76 (m, 8H), 1.66 - 1.54 (m, 4H), 1.50 - 1.37 (m, 5H), 1.01 (t, *J* = 7.2 Hz, 3H), 0.97 - 0.86 (m, 2H) |
| **I-191** | WX | AGL | 844.3 | 12.37 (s, 1H), 11.14 (s, 1H), 8.72 (s, 1H), 8.49 - 8.42 (m, 1H), 8.40 - 8.33 (m, 2H), 8.17 (d, *J* = 8.0 Hz, 1H), 7.55 (s, 1H), 7.35 - 6.84 (m, 3H), 6.02 - 5.88 (m, 1H), 5.50 - 5.36 (m, 1H), 4.71 - 4.56 (m, 1H), 4.52 - 4.41 (m, 1H), 3.64 (s, 3H), 3.36 - 3.15 (m, 3H), 3.10 - 2.85 (m, 4H), 2.83 - 2.69 (m, 4H), 2.64 - 2.53 (m, 3H), 2.23 - 2.12 (m, 3H), 2.10 - 1.80 (m, 9H), 1.62 (s, 6H), 1.38 - 1.13 (m, 2H) |
| **I-192^{b}** | ZO | ABC | 878.3 | 11.15 (s, 1H), 9.40 (s, 1H), 8.82 (d, *J* = 8.0 Hz, 1H), 8.38 (s, 1H), 8.32 (d, *J* = 5.2 Hz, 1H), 8.29 (s, 1H), 8.26 (d, *J* = 8.0 Hz, 1H), 7.78 - 7.57 (m, 1H), 7.52 (t, *J* = 8.0 Hz, 1H), 7.30 (t, *J* = 8.0 Hz, 1H), 7.25 - 6.95 (m, 2H), 6.90 (d, *J* = 7.6 Hz, 1H), 6.31 - 5.78 (m, 1H), 4.21 - 4.13 (m, 1H), 3.86 - 3.76 (m, 4H), 3.75 - 3.69 (m, 4H), 3.55 (t, *J* = 6.0 Hz, 2H), 3.28 - 3.23 (m, 2H), 3.15 - 2.95 (m, 3H), 2.74 - 2.65 (m, 3H), 2.19 - 1.94 (m, 9H), 1.93 - 1.81 (m, 4H), 1.80 - 1.68 (m, 2H), 1.64 - 1.44 (m, 3H), 1.10 - 0.96 (m, 2H) |
| **I-193^{b}** | AHH | AHI | 865.6 | 11.05 (s, 1H), 8.85 (s, 1H), 8.32 (s, 1H), 8.03 (s, 1H), 7.88 (s, 1H), 7.76 (s, 1H), 7.72 (s, 1H), 7.08 (s, 1H), 7.05 - 7.00 (m, 1H), 6.98 - 6.93 (m, 1H), 5.40 - 5.27 (m, 1H), 5.02 - 4.79 (m, 1H), 4.60 - 4.49 (m, 1H), 4.33 - 4.21 (m, 1H), 4.13 - 4.05 (m, 1H), 3.98 (s, 3H), 3.43 (m, 3H), 2.95 - 2.87 (m, 1H), 2.82 - 2.75 (m, 3H), 2.75 - 2.68 (m, 3H), 2.65 - 2.58 (m, 2H), 2.29 - 2.22 (m, 2H), 2.20 - 2.11 (m, 2H), 2.05 - 1.98 (m, 1H), 1.94 - 1.84 (m, 4H), 1.73 - 1.66 (m, 2H), 1.65 - 1.52 (m, 5H), 1.46 - 1.37 (m, 2H), 1.25 - 1.15 (m, 4H), 1.15 - 1.05 (m, 2H), 1.04 - 0.98 (m, 3H) |
| **I-194^{b}** | AHJ | WW | 837.6 | 11.11 (s, 1H), 8.88 (s, 1H), 8.32 (s, 1H), 8.04 (s, 1H), 7.90 (s, 1H), 7.77 (s, 1H), 7.74 (s, 1H), 7.07 (d, *J* = 8.0 Hz, 1H), 6.96 (t, *J* = 7.6 Hz, 1H), 6.88 (d, *J* = 7.2 Hz, 1H), 5.45 - 5.34 (m, 1H), 5.02 - 4.80 (m, 1H), 4.60 - 4.50 (m, 1H), 4.33 - 4.20 (m, 1H), 4.15 - 4.05 (m, 1H), 3.98 (s, 3H), 3.68 (s, 3H), 3.62 (s, 2H), 3.32 - 3.30 (m, 2H), 2.97 - 2.73 (m, 5H), 2.66 - 2.54 (m, 2H), 2.21 - 2.16 (m, 1H), 2.15 - 2.10 (m, 2H), 2.07 - 1.98 (m, 2H), 1.98 - 1.91 (m, 3H), 1.74 - 1.47 (m, 8H), 1.10 - 0.99 (m, 5H) |
| **I-195^{b}** | ACZ | ABC | 872.5 | 9.46 - 9.29 (m, 1H), 8.87 - 8.73 (m, 1H), 8.42 - 8.34 (m, 1H), 8.30 - 8.25 (m, 1H), 7.24 - 6.83 (m, 5H), 5.49 - 5.34 (m, 1H), 4.17 (s, 1H), 3.79 (s, 4H), 3.72 (d, J = 4.6 Hz, 4H), 3.57 (s, 3H), 3.51 - 3.42 (m, 3H), 3.06 - 3.01 (m, 3H), 3.00 (s, 3H), 2.82 - 2.61 (m, 4H), 2.15 - 1.95 (m, 7H), 1.81 (s, 8H), 1.62 - 1.36 (m, 3H), 1.11 - 0.94 (m, 2H) |
| **I-196^{b}** | AFH | ABC | 878.3 | 11.14 (s, 1H), 9.39 (s, 1H), 8.82 (d, *J* = 8.0 Hz, 1H), 8.64 - 8.56 (m, 1H), 8.43 (d, *J* = 4.0 Hz, 1H), 8.37 (s, 1H), 8.29 (s, 1H), 7.53 - 7.38 (m, 2H), 7.27 (d, *J* = 5.6 Hz, 1H), 7.24 - 6.95 (m, 2H), 6.90 (d, *J* = 8.0 Hz, 1H), 6.19 - 5.84 (m, 1H), 4.22 - 4.13 (m, 1H), 3.84 - 3.68 (m, 8H), 3.56 (d, *J* = 6.0 Hz, 3H), 3.25 - 3.17 (m, 4H), 2.73 - 2.64 (m, 3H), 2.17 - 2.00 (m, 7H), 1.99 - 1.83 (m, 6H), 1.80 - 1.67 (m, 2H), 1.64 - 1.47 (m, 3H), 1.11 - 0.96 (m, 2H) |
| **I-197^{b}** | AFQ | ABC | 883.5 | 11.00 (s, 1H), 9.40 (s, 1H), 8.83 (d, *J* = 8.0 Hz, 1H), 8.38 (s, 1H), 8.29 (s, 1H), 7.50 - 7.39 (m, 4H), 7.25 - 6.96 (m, 1H), 6.91 (d, *J* = 8.0 Hz, 1H), 5.72 - 5.60 (m, 1H), 4.74 - 4.62 (m, 1H), 4.24 - 4.11 (m, 1H), 3.96 - 3.85 (m, 1H), 3.83 - 3.77 (m, 4H), 3.75 - 3.70 (m, 4H), 3.60 (t, *J* = 6.4 Hz, 2H), 2.91 - 2.81 (m, 1H), 2.71 - 2.60 (m, 5H), 2.37 - 2.30 (m, 2H), 2.27 - 2.17 (m, 1H), 2.11-2.07 (m, 2H), 2.06 - 1.96 (m, 5H), 1.91 - 1.81 (m, 4H), 1.79 - 1.68 (m, 2H), 1.61 - 1.52 (m, 1H), 1.51 - 1.40 (m, 2H), 1.09 - 0.97 (m, 2H) |
| **I-198^{b}** | TN | ABC | 872.5 | 11.08 (s, 1H), 9.39 (s, 1H), 8.82 (d, *J* = 8.0 Hz, 1H), 8.37 (s, 1H), 8.28 (s, 1H), 7.23 - 6.96 (m, 2H), 6.96 (s, 1H), 6.91 (d, *J* = 8.0 Hz, 1H), 6.88 - 6.85 (m, 1H), 5.42 - 5.32 (m, 1H), 4.22 - 4.13 (m, 1H), 3.79 (s, 4H), 3.74 - 3.70 (m, 4H), 3.56 (s, 3H), 3.42 (t, *J* = 6.0 Hz, 2H), 3.24 (d, *J* = 6.4 Hz, 2H), 2.99 - 2.92 (m, 2H), 2.85 - 2.80 (m, 2H), 2.76 - 2.68 (m, 1H), 2.11 - 2.09 (m, 2H), 2.04 - 2.02 (m, 3H), 1.90 - 1.79 (m, 7H), 1.74 (d, *J* = 10.8 Hz, 2H), 1.66 - 1.61 (m, 2H), 1.54 - 1.45 (m, 2H), 1.26 - 1.12 (m, 3H), 1.08 - 0.99 (m, 2H) |
| **I-199^{b}** | ZH | ABC | 800.3 | 11.08 (s, 1H), 9.40 (s, 1H), 8.82 (d, *J* = 8.0 Hz, 1H), 8.39 (s, 1H), 8.29 (s, 1H), 7.27 - 6.94 (m, 4H), 6.91 (d, *J* = 8.0 Hz, 1H), 5.37 (dd, *J* = 5.2, 12.4 Hz, 1H), 4.26 - 4.14 (m, 1H), 3.83 - 3.76 (m, 4H), 3.75 -3.69 (m, 4H), 3.58 (s, 3H), 3.26 - 3.17 (m, 2H), 3.04 - 2.82 (m, 3H), 2.76 - 2.59 (m, 2H), 2.22 - 2.14 (m, 2H), 2.10 - 2.01 (m, 4H), 2.01-1.96 (m, 1H), 1.96 - 1.89 (m, 2H), 1.83 - 1.75 (m, 4H), 1.75 - 1.70 (m, 1H), 1.67 - 1.55 (m, 1H), 1.15 - 1.00 (m, 2H) |
| **I-200^{b}** | AEJ | AGP | 858.5 | 11.10 (s, 1H), 9.40 (s, 1H), 8.83 (d, J = 8.0 Hz, 1H), 8.39 (s, 1H), 8.29 (s, 1H), 7.25 - 6.83 (m, 5H), 5.39 - 5.34 (m, 1H), 4.27 - 4.15 (m, 1H), 3.79 (m, 4H), 3.72 (m, 4H), 3.56 (s, 3H), 3.46 (t, *J* = 6.0 Hz, 2H), 3.17 - 3.15 (m, 1H), 2.99 - 2.84 (m, 5H), 2.76 - 2.59 (m, 2H), 2.14 - 2.06 (m, 2H), 2.06 - 1.88 (m, 9H), 1.85 - 1.73 (m, 4H), 1.50 - 1.39 (m, 1H), 1.20 - 1.06 (m, 2H), 0.94 - 0.80 (m, 2H) |
| **I-201^{b}** | PP | AHY | 850.3 | 11.09 (s, 1H), 8.38 (s, 1H), 8.23 (s, 1H), 8.08 - 8.03 (m, 2H), 7.88 (s, 1H), 7.71 (s, 1H), 7.59 (s, 1H), 6.99 - 6.90 (m, 2H), 6.82 (dd, *J* = 2.4, 6.8 Hz, 1H), 5.36 (dd, *J* = 5.2, 12.8 Hz, 1H), 4.45 (br d, *J* = 3.9 Hz, 2H), 3.99 (s, 3H), 3.98 - 3.94 (m, 1H), 3.54 (s, 3H), 3.45 - 3.44 (m, 2H), 3.12 (d, *J* = 3.6 Hz, 2H), 2.95 - 2.88 (m, 3H), 2.78 - 2.64 (m, 2H), 2.36 (t, *J* = 7.2 Hz, 2H), 2.32 - 2.25 (m, 1H), 2.21 (d, *J* = 6.8 Hz, 2H), 2.18 - 2.12 (m, 4H), 2.04 - 1.97 (m, 1H), 1.87 - 1.76 (m, 4H), 1.75 - 1.47 (m, 9H), 1.46 - 1.32 (m, 3H), 0.93 (t, *J* = 7.2 Hz, 3H) |
| **I-202^{b}** | TN | AHY | 862.3 | 11.08 (s, 1H), 8.37 (s, 1H), 8.07 - 8.02 (m, 2H), 7.88 (s, 1H), 7.72 (s, 1H), 7.60 (s, 1H), 6.96 (d, *J* = 4.8 Hz, 2H), 6.91 - 6.84 (m, 1H), 5.36 (dd, *J* = 5.2, 12.8 Hz, 1H), 4.46 (d, *J* = 4.4 Hz, 2H), 3.99 (s, 3H), 3.97 - 3.94 (m, 1H), 3.57 (s, 3H), 3.47 - 3.45 (m, 2H), 3.28 - 3.26 (m, 2H), 3.14 - 3.10 (m, 1H), 2.99 - 2.93 (m, 2H), 2.91 - 2.83 (m, 1H), 2.74 - 2.65 (m, 3H), 2.32 - 2.24 (m, 1H), 2.24 - 2.20 (m, 2H), 2.17 - 2.11 (m, 1H), 2.10 - 2.03 (m, 2H), 2.02 - 1.96 (m, 1H), 1.87 - 1.76 (m, 6H), 1.68 - 1.66 (m, 4H), 1.61 - 1.55 (m, 2H), 1.54 - 1.29 (m, 6H), 0.92 (t, *J* = 7.2 Hz, 3H) |
| **I-203^{b}** | AIL | ADI | 808.5 | 11.09 (s, 1H), 8.33 (s, 1H), 8.07 - 8.01 (m, 2H), 7.90 (s, 1H), 7.73 (s, 1H), 7.59 (s, 1H), 6.97 - 6.94 (m, 2H), 6.90 - 6.83 (m, 1H), 5.35 (dd, *J* = 5.2, 12.8 Hz, 1H), 4.45 (d, *J* = 4.4 Hz, 2H), 4.02 - 3.92 (m, 4H), 3.56 (s, 3H), 3.45 - 3.44 (m, 6H), 3.00 - 2.93 (m, 2H), 2.92 - 2.80 (m, 2H), 2.78 - 2.69 (m, 3H), 2.69 - 2.57 (m, 2H), 2.37 (t, *J* = 6.8 Hz, 2H), 2.31 - 2.24 (m, 1H), 2.16 - 2.05 (m, 1H), 2.03 - 1.88 (m, 3H), 1.87 - 1.77 (m, 2H), 1.74 - 1.63 (m, 4H), 1.61 - 1.51 (m, 1H), 1.42 - 1.31 (m, 1H), 0.92 (t, *J* = 7.2 Hz, 3H) |
| **I-204^{b}** | AHH | ADI | 826.5 | 11.09 (s, 1H), 8.88 (s, 1H), 8.32 (s, 1H), 8.04 (s, 1H), 7.90 (s, 1H), 7.80 - 7.70 (m, 2H), 6.97-6.96 (m, 2H), 6.90 - 6.84 (m, 1H), 5.36 (dd, *J* = 5.2, 12.4 Hz, 1H), 5.04 - 4.78 (m, 1H), 4.54 (dd, *J* = 3.2, 11.2 Hz, 1H), 4.26 (dd, *J* = 6.4, 11.2 Hz, 1H), 4.15 - 4.03 (m, 1H), 3.98 (s, 3H), 3.56 (s, 3H), 3.43-3.40 (m, 4H), 2.99 - 2.93 (m, 2H), 2.91 - 2.70 (m, 5H), 2.67 - 2.58 (m, 2H), 2.37 (t, *J* = 7.2 Hz, 2H), 2.24 - 2.11 (m, 2H), 2.03 - 1.90 (m, 3H), 1.87 - 1.77 (m, 2H), 1.75 - 1.56 (m, 6H), 1.01 (t, *J* = 7.2 Hz, 3H) |
| **I-205^{b}** | AFH | AFM | 900.7 | 11.15 (s, 1H), 8.87 (s, 1H), 8.60 (d, *J* = 4.0 Hz, 1H), 8.43 (d, *J* = 4.8 Hz, 1H), 8.29 (s, 1H), 8.02 (s, 1H), 7.90 (s, 1H), 7.80 - 7.69 (m, 2H), 7.45 (d, *J* = 7.2 Hz, 1H), 7.28 (d, *J* = 6.4 Hz, 1H), 7.12 (d, *J =* 8.0 Hz, 1H), 6.30 - 5.90 (m, 1H), 5.00 - 4.81 (m, 1H), 4.54 (d, *J* = 7.2 Hz, 1H), 4.27 (d, *J* = 8.0 Hz, 1H), 4.15 - 4.05 (m, 1H), 3.98 (s, 3H), 3.56 (d, *J* = 6.0 Hz, 2H), 3.26 - 3.17 (m, 5H), 3.10 - 2.94 (m, 2H), 2.76 - 2.62 (m, 4H), 2.15 - 2.03 (m, 7H), 1.95 (d, *J* = 7.6 Hz, 2H), 1.91 - 1.78 (m, 4H), 1.63 - 1.43 (m, 7H), 1.02 (d, *J* = 7.2 Hz, 3H), 0.99 - 0.89 (m, 2H) |
| **I-206^{b}** | AFQ | AFM | 905.6 | 11.00 (s, 1H), 8.88 (s, 1H), 8.29 (s, 1H), 8.03 (s, 1H), 7.90 (s, 1H), 7.80 - 7.70 (m, 2H), 7.50 - 7.39 (m, 4H), 5.73 - 5.60 (m, 1H), 5.00 - 4.82 (m, 1H), 4.76 - 4.63 (m, 1H), 4.59 - 4.51 (m, 1H), 4.31 - 4.24 (m, 1H), 4.14 - 4.05 (m, 1H), 3.99 (s, 3H), 3.97 - 3.86 (m, 1H), 3.60 (t, *J* = 6.4 Hz, 2H), 3.29 - 3.18 (m, 2H), 2.94 - 2.77 (m, 1H), 2.67 - 2.55 (m, 5H), 2.26 - 2.19 (m, 1H), 2.12 - 1.91 (m, 8H), 1.87 - 1.79 (m, 4H), 1.66 - 1.37 (m, 8H), 1.02 (t, *J* = 7.2 Hz, 3H), 0.98 - 0.86 (m, 2H) |
| **I-207^{b}** | ABX | ABC | 857.3 | 11.11 (s, 1H), 9.39 (s, 1H), 8.82 (d, *J* = 7.6 Hz, 1H), 8.37 (s, 1H), 8.31 - 8.25 (m, 1H), 7.80 - 7.68 (m, 3H), 7.23 - 6.95 (t, *J* = 5.2 Hz 1H), 6.90 (d, *J* = 7.6 Hz, 1H), 5.13 (m, 1H), 4.22 - 4.12 (m, 1H), 3.79 (s, 4H), 3.75 - 3.68 (m, 4H), 3.43 - 3.39 (m, 5H), 3.09 (m, 2H), 2.89 (m, 1H), 2.64 - 2.56 (m, 2H), 2.13 - 1.96 (m, 7H), 1.92 - 1.67 (m, 8H), 1.61 - 1.49 (m, 1H), 1.46 - 1.33 (m, 2H), 1.11 - 0.94 (m, 2H) |
| **I-208^{b}** | AFJ | ABC | 872.3 | 11.08 (s, 1H), 9.39 (s, 1H), 8.82 (d, *J* = 8.0 Hz, 1H), 8.37 (s, 1H), 8.29 (s, 1H), 7.62 - 7.55 (m, 1H), 7.24 - 6.94 (m, 3H), 6.90 (d, *J* = 8.0 Hz, 1H), 6.63 (t, *J* = 5.6 Hz, 1H), 5.05 (dd, *J* = 4.2, 12.8 Hz, 1H), 4.22 - 4.11 (m, 1H), 3.79 (s, 4H), 3.74 - 3.71 (m, 4H), 3.49 (t, *J* = 6.0 Hz, 4H), 3.37 (d, *J* = 6.0 Hz, 3H), 2.90 - 2.80 (m, 1H), 2.62 - 2.53 (m, 3H), 2.09 - 1.99 (m, 6H), 1.90 - 1.66 (m, 8H), 1.59 - 1.38 (m, 3H), 1.09 - 0.93 (m, 2H) |
| **I-209^{b}** | AHL | ABC | 859.6 | 10.99 (s, 1H), 9.40 (s, 1H), 8.83 (d, *J* = 8.0 Hz, 1H), 8.37 (s, 1H), 8.29 (s, 1H), 7.53 - 7.42 (m, 1H), 7.35 - 6.85 (m, 4H), 5.12 (dd, *J* = 5.2, 13.2 Hz, 1H), 4.44 - 4.33 (m, 1H), 4.27 - 4.09 (m, 4H), 3.87 - 3.76 (m, 4H), 3.7 5 - 3.66 (m, 4H), 3.58 - 3.53 (m, 2H), 3.29 - 3.27 (m, 1H), 2.98 - 2.85 (m, 1H), 2.63 - 2.54 (m, 3H), 2.48 - 2.40 (m, 1H), 2.11 - 1.90 (m, 9H), 1.88 - 1.66 (m, 6H), 1.59 - 1.35 (m, 3H), 1.06 - 0.91 (m, 2H) |
| **I-210^{b}** | AGU | ABC | 858.2 | 11.09 (s, 1H), 9.40 (s, 1H), 8.83 (d, *J* = 8.0 Hz, 1H), 8.37 (s, 1H), 8.29 (s, 1H), 7.59 (dd, *J* = 7.2, 8.4 Hz, 1H), 7.24 - 6.95 (m, 3H), 6.91 (d, *J* = 8.0 Hz, 1H), 6.62 (t, *J* = 5.6 Hz, 1H), 5.07 (dd, *J* = 6.0, 13.2 Hz, 1H), 4.24 - 4.11 (m, 1H), 3.79 (s, 3H), 3.75 - 3.68 (m, 4H), 3.62 (t, *J* = 5.2 Hz, 2H), 3.48 - 3.40 (m, 2H), 2.94 - 2.82 (m, 1H), 2.62 - 2.54 (m, 3H), 2.52 (s, 3H), 2.11 - 1.96 (m, 7H), 1.90 - 1.67 (m, 6H), 1.59 - 1.39 (m, 3H), 1.10 - 0.93 (m, 2H) |
| **I-211^{b}** | AFV | ABC | 885.5 | 11.06 (s, 1H), 9.39 (s, 1H), 8.82 (d, *J* = 8.0 Hz, 1H), 8.44 (t, *J* = 5.0 Hz, 1H), 8.37 (s, 1H), 8.28 (s, 1H), 7.48 (t, *J* = 8.2 Hz, 1H), 7.24 - 6.94 (m, 1H), 6.90 (d, *J* = 8.0 Hz, 1H), 6.63 (d, *J* = 7.6 Hz, 1H), 6.50 (d, *J* = 8.4 Hz, 1H), 5.16 (d, *J* = 11.2 Hz, 1H), 4.21 - 4.11 (m, 1H), 3.79 (s, 4H), 3.72 (d, *J* = 4.4 Hz, 4H), 3.53 - 3.46 (m, 2H), 3.22 (d, *J* = 5.6 Hz, 4H), 2.88 - 2.79 (m, 1H), 2.69 - 2.63 (m, 2H), 2.59 (s, 1H), 2.55 (s, 3H), 2.19 - 2.11 (m, 1H), 2.11 - 1.94 (m, 6H), 1.93 - 1.64 (m, 9H), 1.59 - 1.41 (m, 3H), 1.08 - 0.95 (m, 2H) |
| **I-212^{b}** | AFX | ABC | 886.7 | 11.09 (s, 1H), 9.40 (s, 1H), 8.83 (d, *J* = 8.0 Hz, 1H), 8.38 (s, 1H), 8.29 (s, 1H), 7.61 (dd, *J* = 7.2, 8.4 Hz, 1H), 7.29 (d, *J* = 8.8 Hz, 1H), 7.24 - 6.86 (m, 3H), 5.09 (dd, *J* = 5.2, 12.8 Hz, 1H), 4.22 - 4.11 (m, 1H), 3.83 - 3.68 (m, 8H), 3.55 (t, *J* = 6.0 Hz, 2H), 3.40 - 3.36 (m, 1H), 3.33 - 3.30 (m, 2H), 3.07 (s, 1H), 3.00 (s, 3H), 2.93 - 2.82 (m, 1H), 2.62 - 2.53 (m, 3H), 2.19 - 1.93 (m, 7H), 1.89 - 1.66 (m, 8H), 1.58 - 1.47 (m, 1H), 1.35 (d, *J* = 9.2 Hz, 2H), 1.09 - 0.94 (m, 2H) |
| **I-213^{b}** | WX | ABC | 843.3 | 11.09 (s, 1H), 9.39 (s, 1H), 8.82 (d, *J =* 8.0 Hz, 1H), 8.37 (s, 1H), 8.23 (s, 1H), 7.24 - 6.82 (m, 5H), 5.38 (dd, *J =* 5.6, 12.8 Hz, 1H), 4.20 - 4.11 (m, 1H), 3.81 - 3.76 (m, 4H), 3.74 - 3.69 (m, 4H), 3.67 (s, 3H), 3.61 (s, 2H), 2.95 - 2.88 (m, 1H), 2.88 - 2.82 (m, 2H), 2.74 - 2.59 (m, 1H), 2.53 - 2.51 (m, 1H), 2.35 - 2.28 (m, 1H), 2.23 - 2.18 (m, 2H), 2.17 (s, 3H), 2.06 - 1.98 (m, 3H), 1.98 - 1.91 (m, 2H), 1.91 - 1.83 (m, 2H), 1.79 - 1.68 (m, 2H), 1.67 - 1.58 (m, 2H), 1.52 - 1.43 (m, 1H), 1.42 - 1.29 (m, 2H), 1.12 - 0.86 (m, 2H) |
| **I-214^{b}** | AHM | ABC | 899.4 | 10.98 (s, 1H), 9.40 (s, 1H), 8.84 (d, *J* = 7.6 Hz, 1H), 8.38 (s, 1H), 8.30 (s, 1H), 7.53 (t, *J =* 8.0 Hz, 1H), 7.24 - 6.96 (m, 1H), 6.92 (m, 3H), 5.17 - 5.10 (m, 1H), 4.22 - 4.12 (m, 1H), 3.80 (d, *J* = 2.8 Hz, 4H), 3.73 (d, *J* = 4.4 Hz, 4H), 3.27 - 3.25 (m, 1H), 3.19 (t, *J* = 6.4 Hz, 3H), 3.10 - 3.01 (m, 2H), 2.89 - 2.80 (m, 1H), 2.76 (s, 3H), 2.66 - 2.59 (m, 1H), 2.56 (s, 3H), 2.18 - 2.09 (m, 1H), 2.07 - 1.99 (m, 4H), 1.98 - 1.82 (m, 5H), 1.79 - 1.61 (m, 7H), 1.54 - 1.44 (m, 1H), 1.37 - 1.25 (m, 2H), 1.07 - 0.94 (m, 2H) |
| **I-215^{b}** | AHO | ABC | 886.6 | 11.20 (s, 1H), 9.34 (s, 1H), 8.73 (d, *J* = 7.6 Hz, 1H), 8.36 (s, 1H), 8.26 (s, 1H), 7.70 (t, *J* = 8.0 Hz, 1H), 7.19 - 7.01 (m, 2H), 6.92 - 6.82 (m, 2H), 5.49 - 5.32 (m, 1H), 4.86 - 7.74 (m, 1H), 4.25 - 4.14 (m, 1H), 3.84 - 3.78 (m, 8H), 3.58 - 3.44 (m, 4H), 3.38 - 3.23 (m, 1H), 3.18 - 3.06 (m, 1H), 3.04 - 2.90 (m, 4H), 2.82 - 2.60 (m, 5H), 2.30 - 2.22 (m, 1H), 2.15 - 2.10 (m, 2H), 2.00 - 1.90 (m, 4H), 1.85 - 1.75 (m, 4H), 1.21 - 1.18 (m, 6H) |
| **I-216^{b}** | TN | AGV | 866.4 | 11.08 (s, 1H), 9.78 (s, 1H), 8.94 - 8.82 (m, 1H), 8.77 (d, *J* = 8.0 Hz, 1H), 8.24 (d, *J* = 6.4 Hz, 2H), 7.01 - 6.95 (m, 2H), 6.91 - 6.86 (m, 1H), 6.84 (d, *J* = 8.4 Hz, 1H), 5.47 - 5.40 (m, 1H), 5.40 - 5.33 (m, 1H), 4.10 - 4.02 (m, 1H), 3.88 (s, 4H), 3.72 - 3.69 (m, 4H), 3.60 (s, 3H), 3.53 - 3.47 (m, 4H), 3.40 - 3.34 (m, 1H), 3.07 - 2.92 (m, 6H), 2.91 - 2.83 (m, 1H), 2.74 - 2.57 (m, 2H), 2.19 - 1.99 (m, 4H), 1.98 - 1.81 (m, 7H), 1.78 - 1.59 (m, 3H), 1.46 (s, 6H), 1.26 - 1.11 (m, 2H) |
| **I-217^{b}** | AGU | AGV | 866.4 | 11.08 (s, 1H), 9.76 (s, 1H), 8.76 (d, *J =* 8.0 Hz, 1H), 8.23 (s, 1H), 7.59 (dd, *J* = 7.2, 8.4 Hz, 1H), 7.15 (d, *J* = 8.8 Hz, 1H), 7.04 (d, *J =* 6.8 Hz, 1H), 6.84 (d, *J =* 8.0 Hz, 1H), 6.62 (t, *J* = 6.0 Hz, 1H), 5.42 (s, 1H), 5.07 (dd, *J =* 5.2, 12.8 Hz, 1H), 4.04 - 3.95 (m, 1H), 3.94 - 3.81 (m, 4H), 3.70 (t, *J* = 4.8 Hz, 4H), 3.62 (t, *J* = 4.8 Hz, 2H), 3.48 - 3.41 (m, 4H), 3.36 - 3.32 (m, 4H), 2.95 - 2.82 (m, 1H), 2.65 - 2.57 (m, 2H), 2.57 - 2.53 (m, 1H), 2.10 - 1.97 (m, 7H), 1.90 - 1.78 (m, 4H), 1.72 - 1.59 (m, 2H), 1.46 (s, 6H), 1.08 - 0.95 (m, 2H) |
| **I-218^{b}** | AGB | ABC | 814.2 | 11.09 (s, 1H), 9.39 (s, 1H), 8.82 (d, *J* = 8.0 Hz, 1H), 8.38 (s, 1H), 8.28 (s, 1H), 7.63 - 7.54 (m, 1H), 7.24 - 6.94 (m, 3H), 6.90 (d, *J* = 8.0 Hz, 1H), 6.25 (d, *J* = 8.0 Hz, 1H), 5.05 (dd, *J* = 5.2, 12.8 Hz, 1H), 4.23 - 4.13 (m, 1H), 3.84 - 3.76 (m, 4H), 3.75 - 3.67 (m, 4H), 3.63 - 3.56 (m, 1H), 2.93 - 2.83 (m, 1H), 2.80 - 2.68 (m, 2H), 2.64 - 2.51 (m, 2H), 2.22 - 2.09 (m, 4H), 2.08 - 2.00 (m, 3H), 1.98 - 1.85 (m, 4H), 1.81 - 1.68 (m, 2H), 1.65 - 1.45 (m, 3H), 1.12 - 0.97 (m, 2H) |
| **I-219^{b}** | PP | AIM | 850.6 | 11.07 (s, 1H), 8.30 (s, 1H), 8.10 - 7.97 (m, 2H), 7.88 (s, 1H), 7.73 (s, 1H), 7.59 (s, 1H), 7.01 - 6.91 (m, 2H), 6.90 - 6.84 (m, 1H), 5.35 (dd, *J =* 5.2, 12.4 Hz, 1H), 4.50 - 4.39 (m, 2H), 4.01 - 3.92 (m, 4H), 3.56 (s, 3H), 3.43 - 3.42 (m, 4H), 2.99 - 2.93 (m, 2H), 2.92 - 2.83 (m, 1H), 2.75 - 2.66 (m, 1H), 2.65 - 2.60 (m, 1H), 2.58 (s, 1H), 2.37 - 2.22 (m, 3H), 2.18 - 2.13 (m, 1H), 2.13 (s, 3H), 2.11 - 2.03 (m, 4H), 2.02 - 1.95 (m, 1H), 1.89 - 1.77 (m, 4H), 1.71 - 1.23 (m, 8H), 1.02 - 0.88 (m, 5H) |
| **I-220^{b}** | AGW | ABC | 842.3 | 11.08 (s, 1H), 9.39 (s, 1H), 8.82 (d, *J* = 8.0 Hz, 1H), 8.38 (s, 1H), 8.28 (s, 1H), 7.67 (dd, *J* = 7.2, 8.4 Hz, 1H), 7.36 - 7.28 (m, 2H), 7.25 - 6.94 (m, 1H), 6.91 (d, *J =* 8.0 Hz, 1H), 5.09 (dd, *J =* 5.2, 12.4 Hz, 1H), 4.25 - 4.12 (m, 1H), 3.83 - 3.75 (m, 4H), 3.74 - 3.70 (m, 4H), 3.39 - 3.36 (m, 2H), 2.93 - 2.81 (m, 3H), 2.64 - 2.53 (m, 2H), 2.28 - 2.09 (m, 7H), 2.08 - 1.65 (m, 10H), 1.61 - 1.50 (m, 1H), 1.39 - 1.21 (m, 2H), 1.14 - 0.93 (m, 2H) |
| **I-221^{b}** | AIO | ABC | 842.5 | 11.09 (s, 1H), 9.40 (s, 1H), 8.83 (d, *J =* 8.0 Hz, 1H), 8.40 - 8.37 (m, 1H), 8.29 (s, 1H), 7.63 - 7.54 (m, 1H), 7.26 - 7.12 (m, 1H), 7.11 - 6.94 (m, 2H), 6.91 (d, *J* = 8.0 Hz, 1H), 6.53 - 6.12 (m, 1H), 5.12 - 5.00 (m, 1H), 4.25 - 4.12 (m, 1H), 3.82 - 3.77 (m, 4H), 3.75 - 3.70 (m, 4H), 3.54 - 3.47 (m, 2H), 2.93 - 2.81 (m, 1H), 2.63 - 2.53 (m, 2H), 2.27 - 2.22 (m, 2H), 2.22 - 2.18 (m, 3H), 2.10 - 1.97 (m, 5H), 1.95 - 1.87 (m, 2H), 1.84 - 1.71 (m, 4H), 1.67 - 1.57 (m, 1H), 1.53 - 1.43 (m, 2H), 1.39 - 1.22 (m, 2H), 1.10 - 0.92 (m, 2H) |
| **I-222^{b}** | AGY | ABC | 856.5 | 11.09 - 11.07 (m, 1H), 9.40 (s, 1H), 8.82 (d, *J* = 8.0 Hz, 1H), 8.41 - 8.37 (m, 1H), 8.29 (s, 1H), 7.65 - 7.55 (m, 1H), 7.33 - 6.95 (m, 3H), 6.91 (d, *J* = 8.0 Hz, 1H), 5.15 - 5.00 (m, 1H), 4.23 - 4.13 (m, 1H), 3.79 (m, 4H), 3.72 (m, 4H), 3.59 - 3.49 (m, 6H), 2.89 - 2.81 (m, 2H), 2.62 - 2.56 (m, 1H), 2.25 - 2.13 (m, 4H), 2.09 - 1.68 (m, 12H), 1.53 - 1.25 (m, 4H), 1.18 - 0.92 (m, 2H) |
| **I-223^{b}** | AIB | ABC | 856.3 | 11.08 (s, 1H), 9.39 (s, 1H), 8.82 (d, *J* = 7.6 Hz, 1H), 8.38 (s, 1H), 8.28 (s, 1H), 7.67 (t, *J* = 7.6 Hz, 1H), 7.37 - 7.29 (m, 2H), 7.25 - 6.95 (m, 1H), 6.91 (d, *J* = 7.6 Hz, 1H), 5.13 - 5.04 (m, 1H), 4.25 - 4.14 (m, 1H), 3.79 (br s, 4H), 3.74 - 3.65 (m, 7H), 2.89 - 2.81 (m, 3H), 2.61 (br d, *J* = 2.4 Hz, 1H), 2.39 - 2.33 (m, 2H), 2.18 - 2.12 (m, 5H), 2.08 - 1.99 (m, 3H), 1.95 - 1.87 (m, 2H), 1.82 - 1.71 (m, 4H), 1.61 - 1.48 (m, 2H), 1.46 - 1.32 (m, 4H), 1.11 - 0.98 (m, 2H) |
| **I-224^{b}** | AIE | ABC | 857.3 | 11.07 (s, 1H), 9.38 (s, 1H), 8.82 (d, *J* = 8.0 Hz, 1H), 8.37 (s, 1H), 8.17 (s, 1H), 7.69 (dd, *J* = 7.2, 1H), 7.33 - 7.21 (m, 2H), 7.03 (t, *J* = 5.2 Hz, 1H), 6.90 (d, *J =* 8.1 Hz, 1H), 5.12 - 5.04 (m, 1H), 4.24 - 4.13 (m, 1H), 3.78 - 3.75 (m, 4H), 3.74 - 3.69 (m, 4H), 3.29 - 3.20 (m, 8H), 2.60 (br s, 4H), 2.46 (s, 3H), 2.21 - 2.14 (m, 5H), 2.05 - 1.97 (m, 3H), 1.94 - 1.85 (m, 2H), 1.82 - 1.69 (m, 2H), 1.59 - 1.50 (m, 1H), 1.11 - 0.96 (m, 2H) |
| **I-225** | AHA | ABC | 871.6 | 11.09 (s, 1H), 9.39 (s, 1H), 8.83 (d, *J* = 8.0 Hz, 1H), 8.38 (s, 1H), 8.28 (s, 1H), 7.70 (dd, *J* = *7.2,* 8.4 Hz, 1H), 7.35 (dd, *J* = 6.0, 7.6 Hz, 2H), 7.23 - 6.94 (m, 1H), 6.91 (d, *J =* 8.0 Hz, 1H), 5.09 (dd, *J* = 5.2, 12.8 Hz, 1H), 4.26 - 4.14 (m, 1H), 3.85 - 3.76 (m, 4H), 3.75 - 3.68 (m, 4H), 3.30 - 3.29 (m, 4H), 2.94 - 2.78 (m, 1H), 2.63 - 2.58 (m, 1H), 2.58 - 2.52 (m, 5H), 2.38 (t, *J =* 6.8 Hz, 4H), 2.23 - 2.20 (m, 1H), 2.19 (s, 3H), 2.18 - 2.16 (m, 1H), 2.08 - 1.98 (m, 3H), 1.95 -1.86 (m, 2H), 1.82 - 1.69 (m, 2H), 1.67 - 1.49 (m, 3H), 1.13 - 0.95 (m, 2H) |
| **I-226^{b}** | AEJ | AGD | 818.5 | 11.09 (s, 1H), 9.39 (s, 1H), 8.82 (d, *J* = 8.0 Hz, 1H), 8.39 (s, 1H), 8.29 (s, 1H), 7.62 - 7.55 (m, 1H), 7.24 - 6.95 (m, 3H), 6.90 (d, *J* = 8.0 Hz, 1H), 6.67 (t, *J* = 5.8 Hz, 1H), 5.09 - 5.01 (m, 1H), 4.24 - 4.14 (m, 1H), 3.79 (s, 4H), 3.75 - 3.68 (m, 4H), 3.47 (t, *J* = 5.8 Hz, 2H), 3.44 - 3.33 (m, 5H), 2.96 - 2.82 (m, 3H), 2.62 - 2.52 (m, 2H), 2.37 (t, *J* = 7.2 Hz, 2H), 2.09 - 1.77 (m, 9H), 1.75 - 1.64 (m, 2H) |
| **I-227^{b}** | AEJ | AGE | 814.2 | 11.08 (s, 1H), 9.40 (s, 1H), 8.82 (d, *J* = 8.0 Hz, 1H), 8.40 (s, 1H), 8.29 (s, 1H), 7.67 (dd, *J* = 7.2, 8.4 Hz, 1H), 7.32 (dd, *J* = 4.8, 7.6 Hz, 2H), 7.26 - 6.95 (m, 1H), 6.90 (d, *J* = 8.0 Hz, 1H), 5.09 (dd, *J* = 5.2, 12.8 Hz, 1H), 4.28 - 4.17 (m, 1H), 3.81 - 3.76 (m, 4H), 3.73 - 3.66 (m, 6H), 2.98 (d, *J =* 11.2 Hz, 2H), 2.91 - 2.78 (m, 3H), 2.64 - 2.51 (m, 2H), 2.39 (t, *J* = 7.2 Hz, 2H), 2.11 - 1.88 (m, 7H), 1.84 - 1.73 (m, 2H), 1.56 - 1.30 (m, 5H) |
| **I-228^{b}** | AEJ | AGF | 828.3 | 11.08 (s, 1H), 9.40 (s, 1H), 8.82 (d, *J* = 8.0 Hz, 1H), 8.40 (s, 1H), 8.29 (s, 1H), 7.71 - 7.60 (m, 1H), 7.32 (t, *J* = 7.2 Hz, 2H), 7.25 - 6.95 (m, 1H), 6.90 (d, *J* = 8.0 Hz, 1H), 5.09 (dd, *J* = 5.2, 12.8 Hz, 1H), 4.27 - 4.15 (m, 1H), 3.83 - 3.76 (m, 4H), 3.74 - 3.67 (m, 6H), 3.00 - 2.93 (m, 2H), 2.90 - 2.79 (m, 3H), 2.64 - 2.51 (m, 2H), 2.33 (t, *J* = 7.2 Hz, 2H), 2.10 - 1.89 (m, 7H), 1.82 - 1.73 (m, 2H), 1.56 - 1.24 (m, 7H) |
| **I-229^{b}** | TN | AIM | 862.4 | 11.08 (s, 1H), 8.29 (s, 1H), 8.07 - 7.99 (m, 2H), 7.89 (s, 1H), 7.73 (s, 1H), 7.59 (s, 1H), 6.96 (d, *J =* 4.8 Hz, 2H), 6.90 - 6.83 (m, 1H), 5.36 (dd, *J =* 5.2, 12.4 Hz, 1H), 4.45 (d, *J =* 4.0 Hz, 2H), 4.03 - 3.94 (m, 4H), 3.57 (s, 3H), 3.48 - 3.44 (m, 3H), 3.01 - 2.83 (m, 4H), 2.75 - 2.58 (m, 5H), 2.33 - 2.24 (m, 1H), 2.18 - 2.07 (m, 3H), 2.06 - 1.95 (m, 4H), 1.86 - 1.76 (m, 6H), 1.67 - 1.24 (m, 8H), 0.99 - 0.88 (m, 5H) |
| **I-230^{b}** | QI | AHR | 868.6 | 11.06 (s, 1H), 8.85 (s, 1H), 8.35 (s, 1H), 8.03 (s, 1H), 7.88 - 7.84 (s, 1H), 7.75 (s, 1H), 7.70 - 7.68 (m, 1H), 6.99 - 6.95 (m, 2H), 6.84 - 6.81 (m, 1H), 5.32 (dd, *J* = 5.2, 12.8 Hz, 1H), 4.98 - 4.81 (m, 1H), 4.53 (dd, *J* = 3.2, 11.2 Hz, 1H), 4.26 (dd, *J* = 5.6, 11.2 Hz, 1H), 4.12 - 4.05 (m, 1H), 3.97 (s, 3H), 3.38 (s, 2H), 3.30 (s, 3H), 3.12 - 3.06 (m, 2H), 2.71 - 2.56 (m, 4H), 2.34 - 2.31 (m, 2H), 2.18 (d, *J* = 7.2 Hz, 2H), 2.13 (s, 3H), 2.03 - 1.97 (m, 1H), 1.87 - 1.73 (m, 5H), 1.72 - 1.52 (m, 10H), 1.46 - 1.33 (m, 3H), 1.00 (t, *J* = 7.2 Hz, 3H) |
| **I-231^{b}** | AHD | ABC | 885.6 | 11.08 (s, 1H), 9.39 (s, 1H), 8.83 (d, *J* = 8.0 Hz, 1H), 8.37 (s, 1H), 8.29 (s, 1H), 7.57 (dd, *J* = 7.2, 8.4 Hz, 1H), 7.25 - 6.87 (m, 5H), 5.05 (dd, *J* = 5.2, 12.8 Hz, 1H), 4.21 - 4.12 (m, 1H), 3.82 - 3.76 (m, 4H), 3.74 - 3.70 (m, 4H), 3.35 - 3.30 (m, 3H), 2.91 - 2.81 (m, 3H), 2.62 - 2.52 (m, 2H), 2.33 - 2.27 (m, 1H), 2.20 (s, 3H), 2.09 - 1.99 (m, 5H), 1.93 - 1.59 (m, 11H), 1.57 - 1.50 (m, 1H), 1.48 - 1.38 (m, 2H), 1.08 - 0.96 (m, 2H) |
| **I-232^{b}** | AGH | ABC | 897.5 | 11.09 (s, 1H), 9.39 (s, 1H), 8.82 (d, *J* = 7.6 Hz, 1H), 8.37 (s, 1H), 8.29 (s, 1H), 7.58 (t, *J* = 7.6 Hz, 1H), 7.24 - 6.95 (m, 3H), 6.91 (d, *J* = 8.0 Hz, 1H), 6.24 (d, *J* = 7.6 Hz, 1H), 5.08 - 5.02 (m, 1H), 4.23 - 4.12 (m, 1H), 3.79 (s, 4H), 3.72 (s, 4H), 2.93 - 2.86 (m, 3H), 2.85 - 2.80 (m, 2H), 2.63 - 2.53 (m, 2H), 2.35 (d, *J* = 12.0 Hz, 2H), 2.28 - 2.21 (m, 1H), 2.10 (d, *J* = 6.4 Hz, 2H), 2.06 - 2.00 (m, 3H), 2.00 - 1.79 (m, 7H), 1.76 - 1.66 (m, 4H), 1.61 - 1.53 (m, 1H), 1.50 - 1.39 (m, 4H), 1.10 - 0.94 (m, 2H) |
| **I-233^{b}** | AGI | ABC | 855.7 | 11.09 (s, 1H), 9.39 (s, 1H), 8.82 (d, *J* = 8.0 Hz, 1H), 8.36 (s, 1H), 8.28 (s, 1H), 7.22 - 6.96 (m, 2H), 6.96 - 6.89 (m, 3H), 5.41 - 5.32 (m, 1H), 4.22 - 4.11 (m, 1H), 3.79 (s, 6H), 3.72 (d, *J* = 4.4 Hz, 4H), 3.65 (s, 3H), 2.92 (s, 4H), 2.89 - 2.83 (m, 1H), 2.72 - 2.60 (m, 2H), 2.23 (s, 3H), 2.12 - 1.94 (m, 6H), 1.86 (d, *J* = 11.6 Hz, 2H), 1.77 - 1.69 (m, 2H), 1.69 - 1.62 (m, 4H), 1.58 - 1.49 (m, 1H), 1.09 - 0.95 (m, 2H) |
| **I-234^{b}** | AHH | AGF | 850.6 | 11.10 (s, 1H), 8.89 (s, 1H), 8.33 (s, 1H), 8.05 (s, 1H), 7.91 (s, 1H), 7.77 (s, 1H), 7.76 (s, 1H), 7.71 - 7.63 (m, 1H), 7.33 (t, *J* = 8.0 Hz, 2H), 5.16 - 5.04 (m, 1H), 5.02 - 4.81 (m, 1H), 4.62 - 4.50 (m, 1H), 4.35 - 4.20 (m, 1H), 4.16 - 4.04 (m, 1H), 3.98 (s, 3H), 3.73 - 3.65 (m, 2H), 2.97 - 2.68 (m, 7H), 2.64 - 2.54 (m, 2H), 2.33 - 2.28 (m, 2H), 2.26 - 2.15 (m, 2H), 2.07 - 1.99 (m, 1H), 1.98 - 1.89 (m, 2H), 1.83 - 1.75 (m, 2H), 1.75 - 1.65 (m, 2H), 1.64 - 1.55 (m, 2H), 1.53 - 1.41 (m, 3H), 1.39 - 1.24 (m, 4H), 1.05 - 0.98 (m, 3H) |
| **I-235** | AIH | PW | 601.4 | 9.73 (s, 1H), 8.92 (s, 1H), 8.34 (s, 2H), 8.19 (s, 1H), 8.15 (d, *J* = 5.6 Hz, 1H), 7.30 - 7.06 (m, 3H), 7.01 (d, *J* = 5.6 Hz, 1H), 4.27 - 4.16 (m, 1H), 3.43 - 3.42 (m, 3H), 3.17 (t, *J* = 6.0 Hz, 3H), 2.81 (t, *J* = 7.6 Hz, 2H), 2.72 - 2.67 (m, 2H), 2.55 (s, 2H), 2.09 - 2.02 (m, 2H), 1.96 - 1.88 (m, 2H), 1.80 - 1.69 (m, 6H), 1.63 - 1.54 (m, 1H), 1.18 - 1.01 (m, 3H), 0.48 - 0.42 (m, 2H), 0.24 - 0.19 (m, 2H) |
| **I-236** | AAA | PW | 854.2 | 11.07 (s, 1H), 9.71 (s, 1H), 8.93 (s, 1H), 8.19 (s, 1H), 8.15 (d, *J* = 5.2 Hz, 1H), 7.32 - 7.05 (m, 3H), 7.04 - 6.98 (m, 1H), 6.92 (d, *J* = 8.6 Hz, 1H), 6.83 (d, *J* = 1.8 Hz, 1H), 6.63 (dd, *J* = 2.0, 8.4 Hz, 1H), 5.33 - 5.24 (m, 1H), 4.26 - 4.15 (m, 1H), 3.59 (d, *J* = 12.0 Hz, 2H), 3.30 (s, 3H), 3.20 - 3.15 (m, 2H), 2.97 - 2.68 (m, 1H), 2.65 - 2.59 (m, 2H), 2.22 - 2.10 (m, 7H), 2.09 - 1.87 (m, 6H), 1.85 - 1.73 (m, 4H), 1.68 - 1.47 (m, 3H), 1.31 - 1.17 (m, 2H), 1.12 - 0.97 (m, 3H), 0.49 - 0.41 (m, 2H), 0.26 - 0.18 (m, 2H) |
| **I-237** | AAB | WW | 858.5 | 11.07 (s, 1H), 9.69 (s, 1H), 8.91 (s, 1H), 8.22 (s, 2H), 8.15 (d, *J* = 2.4 Hz, 1H), 7.30 - 6.95 (m, 7H), 5.36 (dd, *J*₁ = 5.4 Hz, *J*₂ = 12.4 Hz, 1H), 4.17 - 4.09 (m, 1H), 3.96 (s, 2H), 3.73 - 3.63 (m, 5H), 3.52 - 3.51 (m, 2H), 3.20 - 3.16 (m, 2H), 2.93 - 2.82 (m, 1H), 2.76 (t, *J* = 5.4 Hz, 2H), 2.72 - 2.67 (m, 1H), 2.61 (d, *J* = 17.2 Hz, 1H), 2.53 - 2.52 ( m, 2H), 2.21 - 2.13 (m, 5H), 2.04 - 1.93 (m, 3H), 1.86 (d, *J* = 10.4 Hz, 2H), 1.78 - 1.63 (m, 2H), 1.56 - 1.44 (m, 1H), 1.12 - 0.90 (m, 3H), 0.49 - 0.40 (m, 2H), 0.25 - 0.18 (m, 2H) |
| **I-238** | AER | PW | 952.7 | 11.11 (s, 1H), 9.71 (s, 1H), 8.92 (s, 1H), 8.18 (s, 1H), 8.14 (d, *J* = 5.2 Hz, 1H), 7.30 - 7.04 (m, 5H), 7.03 - 6.98 (m, 2H), 5.36 (dd, *J* = 4.8, 12.4 Hz, 1H), 4.21 - 4.20 (m, 1H), 3.90 - 3.86 (m, 1H), 3.61 (s, 2H), 3.44 (q, *J* = 6.8 Hz, 2H), 3.33 (s, 3H), 3.28 (t, *J* = 5.6 Hz, 2H), 3.17 (t, *J* = 6.0 Hz, 2H), 2.99 - 2.82 (m, 4H), 2.79 - 2.60 (m, 3H), 2.05 (s, 3H), 2.04 - 1.98 (m, 7H), 1.89 (d, *J* = 12.0 Hz, 2H), 1.81 - 1.70 (m, 2H), 1.64 (d, *J* = 12.0 Hz, 2H), 1.55 - 1.49 (m, 1H), 1.42-1.41 (m, 2H), 1.25 - 1.14 (m, 2H), 1.05 (t, *J* = 7.2 Hz, 4H), 1.02 - 0.96 (m, 1H), 0.54 - 0.36 (m, 2H), 0.28 - 0.16 (m, 2H) |
| **I-239** | AAD | PW | 854.5 | 11.10 (s, 1H), 9.71 (s, 1H), 8.91 (s, 1H), 8.21 (s, 1H), 8.17 (s, 1H), 8.14 (d, *J* = 5.6 Hz, 1H), 7.30 - 6.95 (m, 7H), 5.35 (dd, *J* = 5.2, 12.8 Hz, 1H), 4.25 - 4.12 (m, 1H), 3.61 - 3.59 (m, 2H), 3.33 (s, 3H), 3.17 (t, *J* = 6.0 Hz, 2H), 2.96 - 2.84 (m, 3H), 2.78 - 2.68 (m, 1H), 2.68 - 2.63 (m, 1H), 2.63 - 2.60 (s, 1H), 2.41 - 2.34 (m, 1H), 2.26 (d, *J* = 5.6 Hz, 2H), 2.21 (s, 3H), 2.06 - 2.01 (m, 2H), 2.00 - 1.93 (m, 2H), 1.89 (d, *J* = 11.6 Hz, 2H), 1.81 - 1.69 (m, 2H), 1.65 (d, *J* = 11.2 Hz, 2H), 1.57 - 1.38 (m, 3H), 1.11 - 0.93 (m, 3H), 0.48 - 0.41 (m, 2H), 0.23 - 0.20 (m, 2H) |
| **I-240** | AAE | PW | 826.3 | 11.08 (s, 1H), 9.68 (s, 1H), 8.91 (s, 1H), 8.18 (s, 1H), 8.15 (s, 1H), 7.31 - 7.11 (m, 1H), 7.11 - 7.08 (m, 1H), 7.07 - 7.04 (m, 1H), 7.03 - 6.99 (m, 1H), 6.98 - 6.93 (m, 2H), 5.36 (dd, *J* = 5.2, 12.8 Hz, 1H), 4.25 - 4.15 (m, 1H), 3.83 (s, 2H), 3.63 (s, 3H), 3.20 - 3.16 (m, 4H), 2.97 - 2.84 (m, 5H), 2.77 - 2.70 (m, 1H), 2.65 - 2.58 (m, 2H), 2.07 (s, 2H), 2.03 (s, 3H), 2.02 - 1.97 (m, 2H), 1.91 (d, *J* = 10.8 Hz, 2H), 1.81 - 1.70 (m, 2H), 1.56 - 1.45 (m, 1H), 1.11 - 0.94 (m, 3H), 0.49 - 0.42 (m, 2H), 0.25 - 0.19 (m, 2H) |
| **I-241** | AAG | PW | 874.3 | 11.14 (s, 1H), 9.68 (s, 1H), 8.91 (s, 1H), 8.37 (d, *J* = 4.8 Hz, 1H), 8.25 - 8.20 (s, 1H), 8.17 (s, 1H), 8.14 (d, *J* = 5.2 Hz, 1H), 7.70 - 7.56 (m, 1H), 7.51 (t, *J* = 7.6 Hz, 1H), 7.32 (t, *J* = 7.6 Hz, 1H), 7.29 - 6.99 (m, 5H), 6.27 - 5.83 (m, 1H), 4.21 - 4.15 (m, 1H), 4.04 - 3.96 (m, 2H), 3.18 (t, *J* = 6.0 Hz, 2H), 3.10 - 3.05 (m, 1H), 3.04 - 2.95 (m, 3H), 2.75 - 2.68 (m, 1H), 2.39 - 2.34 (m, 1H), 2.21 (d, *J* = 6.8 Hz, 2H), 2.18 (s, 3H), 2.16 - 2.07 (m, 3H), 2.06 - 1.99 (m, 2H), 1.89 (d, *J* = 12.0 Hz, 2H), 1.80 - 1.71 (m, 2H), 1.67 (d, *J* = 11.2 Hz, 2H), 1.56 - 1.37 (m, 3H), 1.13 - 0.91 (m, 3H), 0.49 - 0.41 (m, 2H), 0.25 - 0.19 (m, 2H) |
| **I-242** | AAI | PW | 842.6 | 11.10 (s, 1H), 9.72 (s, 1H), 8.93 (s, 1H), 8.14 (d, *J* = 5.2 Hz, 1H), 7.30 - 6.99 (m, 5H), 6.96 t, *J* = 7.6 Hz, 1H), 6.92 - 6.88 (m, 1H), 5.42 - 5.34 (m, 1H), 4.21 - 4.13 (m, 1H), 3.68 (s, 3H), 3.65 (s, 2H), 3.17 (t, *J* = 6.0 Hz, 2H), 2.94 - 2.84 (m, 1H), 2.76 - 2.58 (m, 2H), 2.39 (t, *J* = 6.8 Hz, 2H), 2.26 (t, *J* = 5.6 Hz, 2H), 2.12 (s, 3H), 2.10 (s, 3H), 2.10 - 2.06 (m, 2H), 2.04 - 1.96 (m, 3H), 1.81 (d, *J* = 11.6 Hz, 2H), 1.76 - 1.66 (m, 2H), 1.62 - 1.53 (m, 2H), 1.52 - 1.42 (m, 1H), 1.10 - 1.03 (m, 1H), 1.02 - 0.89 (m, 2H), 0.48 - 0.42 (m, 2H), 0.25 - 0.19 (m, 2H) |
| **I-243** | AAJ | PW | 826.6 | 11.10 (s, 1H), 9.70 (s, 1H), 8.92 (s, 1H), 8.17 (s, 1H), 8.14 (s, 1H), 7.30 - 7.03 (m, 5H), 7.01 (d, *J* = 5.6 Hz, 1H), 6.99 - 6.95 (m, 1H), 5.39 - 5.32 (m, 1H), 4.24 - 4.15 (m, 1H), 3.50 (s, 2H), 3.33 (s, 3H), 3.17 (t, *J* = 6.0 Hz, 2H), 2.96 - 2.84 (m, 1H), 2.76 - 2.68 (m, 1H), 2.65 - 2.58 (m, 1H), 2.48 - 2.31 (m, 8H), 2.14 (d, *J* = 6.4 Hz, 2H), 2.07 - 1.97 (m, 3H), 1.88 (d, *J* = 12.4 Hz, 2H), 1.81 - 1.68 (m, 2H), 1.63 - 1.50 (m, 1H), 1.10 - 0.98 (m, 3H), 0.48 - 0.42 (m, 2H), 0.24 - 0.19 (m, 2H) |
| **I-244** | AAK | PW | 866.4 | 11.10 (s, 1H), 9.69 (s, 1H), 8.92 (s, 1H), 8.18 - 8.11 (m, 2H), 7.30 - 7.09 (m, 2H), 7.07 - 7.04 (m, 1H), 7.01 (d, *J* = 4.4 Hz, 1H), 6.95 (t, *J* = 7.6 Hz, 1H), 6.86 (d, *J* = 7.6 Hz, 1H), 5.37 (dd, *J* = 4.8, 12.0 Hz, 1H), 4.24 - 4.12 (m, 1H), 3.66 (s, 3H), 3.58 (s, 2H), 3.21 - 3.15 (m, 4H), 2.98 (s, 4H), 2.91 - 2.83 (m, 2H), 2.76 - 2.69 (m, 3H), 2.65 - 2.62 (m, 2H), 2.06 - 1.97 (m, 3H), 1.89 - 1.81 (m, 2H), 1.78 - 1.68 (m, 2H), 1.63 (s, 4H), 1.58 - 1.52 (m, 1H), 1.41 - 1.31 (m, 1H), 1.14 - 0.96 (m, 3H), 0.50 - 0.39 (m, 2H), 0.27 - 0.18 (m, 2H) |
| **I-245** | AAL | PW | 894.3 | 11.09 (s, 1H), 9.68 (s, 1H), 8.92 (s, 1H), 8.17 (s, 1H), 8.15 (d, *J* = 5.2 Hz, 1H), 7.31 - 6.98 (m, 5H), 6.95 (t, *J* = 8.0 Hz, 1H), 6.90 - 6.84 (m, 1H), 5.42 - 5.31 (m, 1H), 4.23 - 4.15 (m, 1H), 3.66 (s, 3H), 3.63 (s, 2H), 3.18 (t, *J* = 6.0 Hz, 2H), 2.94 - 2.84 (m, 1H), 2.78 - 2.68 (m, 1H), 2.65 - 2.58 (1H), 2.43 - 2.33 (m, 8H), 2.18 (d, *J* = 6.4 Hz, 2H), 2.07 - 1.98 (m, 3H), 1.88 (d, *J* = 12.0 Hz, 2H), 1.80 - 1.69 (m, 2H), 1.63 - 1.54 (m, 1H), 1.51 - 1.28 (m, 8H), 1.12 - 0.97 (m, 3H), 0.50 - 0.40 (m, 2H), 0.26 - 0.18 (m, 2H) |
| **I-246** | ADC | PW | 871.6 | 9.67 (s, 1H), 8.91 (s, 1H), 8.18 - 8.12 (m, 2H), 7.29 - 7.04 (m, 3H), 7.01 (dd, *J* = 1.6, 5.2 Hz, 1H), 6.98 - 6.92 (m, 2H), 6.89 - 6.85 (m, 1H), 5.42 (dd, *J* = 5.2, 12.0 Hz, 1H), 4.24 - 4.14 (m, 1H), 3.57 (s, 3H), 3.42 (t, *J* = 6.4 Hz, 4H), 3.18 (t, *J* = 6.4 Hz, 2H), 3.02 (s, 3H), 2.98 - 2.94 (m, 2H), 2.81 - 2.68 (m, 2H), 2.37 (t, *J* = 7.2 Hz, 2H), 2.18 - 2.11 (m, 5H), 2.08 - 1.61 (m, 12H), 1.59 - 1.48 (m, 1H), 1.11 - 0.96 (m, 3H), 0.48 - 0.42 (m, 2H), 0.25 - 0.19 (m, 2H) |
| **I-247** | AAM | PW | 868.3 | 9.68 (s, 1H), 8.91 (s, 1H), 8.17 (s, 1H), 8.14 (d, *J* = 5.2 Hz, 1H), 7.29 - 7.00 (m, 5H), 6.94 (t, *J* = 7.6 Hz, 1H), 6.90 - 6.84 (m, 1H), 5.44 (dd, *J* = 5.2, 12.8 Hz, 1H), 4.24 - 4.12 (m, 1H), 3.68 (s, 3H), 3.62 (s, 2H), 3.18 (t, *J* = 6.0 Hz, 2H), 3.04 (s, 3H), 2.99 - 2.92 (m, 1H), 2.87 (d, *J* = 10.0 Hz, 2H), 2.82 - 2.69 (m, 2H), 2.40 - 2.35 (m, 1H), 2.24 (d, *J* = 6.8 Hz, 2H), 2.19 (s, 3H), 2.06 - 1.93 (m, 5H), 1.88 (d, *J* = 11.2 Hz, 2H), 1.80 - 1.70 (m, 2H), 1.65 (d, *J* = 10.8 Hz, 2H), 1.54 - 1.45 (m, 1H), 1.44 - 1.34 (m, 2H), 1.11 - 0.96 (m, 3H), 0.49 - 0.41 (m, 2H), 0.26 - 0.18 (m, 2H) |
| **I-248^{b}** | ACD | ACA | 938.4 | 11.09 (s, 1H), 9.69 (s, 1H), 8.92 (s, 1H), 8.19 - 8.11 (m, 2H), 7.31 - 6.99 (m, 5H), 6.95 (t, *J* = 7.6 Hz, 1H), 6.87 (d, *J* = 7.6 Hz, 1H), 5.37 (dd, *J* = 5.2, 12.4 Hz, 1H), 4.26 - 4.12 (m, 1H), 3.67 (s, 3H), 3.61 (s, 2H), 3.25 - 3.12 (m, 4H), 2.88 (d, *J* = 11.2 Hz, 2H), 2.77 - 2.58 (m, 3H), 2.26 - 2.09 (m, 4H), 2.09 - 1.83 (m, 8H), 1.82 - 1.70 (m, 4H), 1.64 (d, *J* = 11.6 Hz, 4H), 1.54 - 1.29 (m, 4H), 1.23 - 1.15 (m, 2H), 1.10 - 0.96 (m, 3H), 0.51 - 0.39 (m, 2H), 0.26 - 0.18 (m, 2H) |
| **I-249^{b}** | AAO | PW | 924.6 | 11.10 (s, 1H), 9.72 (s, 1H), 8.92 (s, 1H), 8.20 (s, 1H), 8.15 (d, *J* = 5.2 Hz, 1H), 7.32 - 6.85 (m, 7H), 5.44 - 5.33 (m, 1H), 4.28 - 4.16 (m, 2H), 3.95 - 3.84 (m, 1H), 3.73 - 3.57 (m, 5H), 3.35 - 3.33 (m, 3H), 3.18 (t, *J* = 6.0 Hz, 2H), 2.97 - 2.79 (m, 3H), 2.72 - 2.69 (m, 4H), 2.61 (m, 1H), 2.52 (s, 3H), 2.29 - 2.19 (m, 2H), 2.16 - 1.96 (m, 6H), 1.91 - 1.73 (m, 6H), 1.46 - 1.37 (m, 1H), 1.26 - 1.02 (m, 3H), 0.49 - 0.41 (m, 2H), 0.25 - 0.19 (m, 2H) |
| **I-250** | AAP | PW | 840.5 | 11.09 (s, 1H), 9.70 (s, 1H), 8.92 (s, 1H), 8.34 - 8.22 (m, 1H), 8.18 (s, 1H), 8.16 - 8.12 (m, 1H), 7.31 - 6.86 (m, 7H), 5.39 - 5.32 (m, 1H), 4.24 - 4.15 (m, 1H), 3.56 (s, 3H), 3.43 (s, 2H), 3.18 (s, 2H), 2.89 (d, *J* = 7.6 Hz, 4H), 2.76 - 2.58 (m, 7H), 2.08 - 2.01 (m, 2H), 1.99 (s, 3H), 1.97 - 1.86 (m, 4H), 1.81 - 1.68 (m, 2H), 1.05 (s, 4H), 0.49 - 0.41 (m, 2H), 0.25 - 0.18 (m, 2H) |
| **I-251^{b}** | TN | ACF | 773.5 | 11.10 (s, 1H), 10.04 (s, 1H), 9.37 (dd, *J* = 1.2, 6.8 Hz, 1H), 8.86 (dd, *J* = 1.2, 4.0 Hz, 1H), 8.70 (s, 1H), 8.37 (s, 1H), 7.35 - 7.26 (m, 1H), 7.14 (s, 1H), 7.03 - 6.93 (m, 2H), 6.89 - 6.82 (m, 1H), 5.41 - 5.32 (m, 1H), 4.36 - 4.07 (m, 1H), 3.57 (s, 3H), 3.44 (t, *J* = 6.0 Hz, 2H), 3.30 - 3.25 (m, 1H), 2.99 - 2.84 (m, 3H), 2.77 - 2.70 (m, 1H), 2.69 - 2.60 (m, 3H), 2.14 - 1.98 (m, 7H), 1.90 - 1.72 (m, 8H), 1.62 - 1.55 (m, 1H), 1.50 - 1.42 (m, 2H), 1.09 - 0.97 (m, 2H) |
| **I-252** | SX | PW | 953.6 | 11.11 (s, 1H), 9.73 (s, 1H), 8.92 (s, 1H), 8.17 - 8.11 (m, 2H), 7.32 - 6.98 (m, 7H), 5.37 (dd, *J* = 5.2, 12.8 Hz, 1H), 4.25 - 4.18 (m, 1H), 4.03 - 3.96 (m, 2H), 3.66 (s, 3H), 3.47 - 3.44 (m, 5H), 3.34 (s, 3H), 3.20 - 3.15 (m, 3H), 2.96 - 2.88 (m, 2H), 2.77 - 2.63 (m, 7H), 2.32 (s, 3H), 2.26 - 1.97 (m, 8H), 1.90 (d, *J* = 12.4 Hz, 2H), 1.84 - 1.72 (m, 2H), 1.66 (s, 1H), 1.17 - 0.99 (m, 3H), 0.48 - 0.41 (m, 2H), 0.25 - 0.18 (m, 2H) |
| **I-253^{b}** | AAX | WW | 854.5 | 11.10 (s, 1H), 9.71 (s, 1H), 8.92 (s, 1H), 8.27 (s, 1H), 8.14 (d, *J* = 5.2 Hz, 1H), 7.31 - 7.11 (m, 1H), 7.10 - 7.06 (m, 2H), 7.03 - 6.99 (m, 1H), 6.95 (t, *J* = 7.6 Hz, 1H), 6.87 (d, *J* = 7.9 Hz, 1H), 5.37 (dd, *J* = 5.6, 13.2 Hz, 1H), 4.25 - 4.17 (m, 1H), 3.67 (s, 3H), 3.60 (s, 2H), 3.20 - 3.15 (m, 3H), 2.98 - 2.78 (m, 6H), 2.77 - 2.69 (m, 1H), 2.66 - 2.55 (m, 3H), 2.27 (t, *J* = 7.6 Hz, 2H), 2.07 - 1.86 (m, 9H), 1.62 (d, *J* = 12.0 Hz, 2H), 1.48 - 1.37 (m, 2H), 1.27 - 1.14 (m, 3H), 0.50 - 0.40 (m, 2H), 0.28 - 0.17 (m, 2H) |
| **I-254^{b}** | AAX | SK | 854.3 | 11.10 (s, 1H), 9.73 (s, 1H), 8.92 (s, 1H), 8.19 (s, 1H), 8.14 (d, *J* = 5.2 Hz, 1H), 7.30 - 7.12 (m, 1H), 7.11 - 7.07 (m, 2H), 7.05 - 6.99 (m, 2H), 6.99 - 6.95 (m, 1H), 5.36 (dd, *J* = 5.2, 12.8 Hz, 1H), 4.26 - 4.20 (m, 1H), 3.53 (s, 2H), 3.33 (s, 3H), 3.17 (t, *J* = 6.0 Hz, 2H), 3.02 - 2.81 (m, 5H), 2.68 (s, 1H), 2.65 - 2.56 (m, 1H), 2.53 - 2.52 (m, 1H), 2.30 (s, 1H), 2.13 - 1.87 (m, 10H), 1.63 (d, *J* = 11.2 Hz, 2H), 1.45 (s, 2H), 1.26 - 1.10 (m, 5H), 1.10 - 1.01 (m, 1H), 0.49 - 0.39 (m, 2H), 0.30 - 0.22 (m, 2H) |
| **I-255** | AAZ | SK | 868.6 | 11.18 - 11.03 (m, 1H), 9.73 (s, 1H), 8.91 (s, 1H), 8.18 - 8.12 (m, 2H), 7.30 - 7.03 (m, 5H), 7.03 - 6.96 (m, 2H), 5.35 (dd, *J* = 5.2, 12.8 Hz, 1H), 4.56 - 4.45 (m, 2H), 3.98 (d, *J* = 12 Hz, 2H), 3.33 (s, 3H), 3.19 - 3.15 (m, 3H), 2.93 - 2.81 (m, 4H), 2.37 - 2.32 (m, 2H), 2.09 - 1.62 (m, 11H), 1.49 - 1.40 (m, 2H), 1.32 - 1.01 (m, 5H), 0.48 - 0.40 (m, 2H), 0.24 - 0.16 (m, 2H) |
| **I-256** | ABA | PW | 924.6 | 11.12 (s, 1H), 9.71 (s, 1H), 8.93 (s, 1H), 8.17 (s, 1H), 8.14 (d, *J* = 5.2 Hz, 1H), 7.32 - 6.82 (m, 7H), 5.40 - 5.36 (m, 1H), 4.23 - 4.14 (m, 1H), 3.67 (s, 3H), 3.60 (s, 2H), 3.42 - 3.40 (m, 3H), 3.17 (t, *J* = 6.2 Hz, 2H), 2.96 - 2.82 (m, 1H), 2.77 - 2.57 (m, 6H), 2.15 (d, *J* = 6.8 Hz, 2H), 2.12 - 2.07 (m, 2H), 2.03 (d, *J* = 9.2 Hz, 4H), 1.88 (d, *J* = 11.2 Hz, 2H), 1.82 - 1.66 (m, 6H), 1.65 - 1.28 (m, 5H), 1.14 - 0.93 (m, 3H), 0.50 - 0.40 (m, 2H), 0.27 - 0.17 (m, 2H) |
| **I-257^{b}** | TN | ABC | 858.5 | 11.11 (s, 1H), 9.40 (s, 1H), 8.83 (d, *J* = 8.0 Hz, 1H), 8.38 (s, 1H), 8.30 (s, 1H), 7.24 - 6.95 (m, 3H), 6.93 - 6.86 (m, 2H), 5.44 - 5.32 (m, 1H), 4.22 - 4.13 (m, 1H), 3.82 - 3.77 (m, 4H), 3.75 - 3.70 (m, 4H), 3.58 (s, 3H), 3.47 - 3.44 (m, 2H), 3.29 - 3.27 (m, 1H), 3.00 - 2.94 (m, 2H), 2.92 - 2.84 (m, 1H), 2.77 - 2.70 (m, 1H), 2.67 - 2.59 (m, 3H), 2.16 - 2.10 (m, 2H), 2.08 - 1.97 (m, 5H), 1.91 - 1.79 (m, 6H), 1.78 - 1.67 (m, 2H), 1.61 - 1.52 (m, 1H), 1.51 - 1.40 (m, 2H), 1.11 - 0.93 (m, 2H) |
| **I-258^{b}** | TN | ACH | 871.6 | 11.78 (s, 1H), 11.09 (s, 1H), 10.43 (s, 1H), 9.33 (s, 1H), 8.93 (d, *J* = 7.6 Hz, 1H), 8.40 (s, 1H), 8.34 (s, 1H), 7.31 - 6.93 (m, 4H), 6.91 - 6.84 (m, 1H), 5.48 - 5.34 (m, 1H), 4.88 - 4.54 (m, 2H), 4.31 - 4.16 (m, 1H), 3.73 (s, 3H), 3.70 - 3.62 (m, 2H), 3.60 - 3.57 (m, 2H), 3.52 - 3.47 (m, 4H), 3.36 - 3.28 (m, 1H), 3.22 - 3.10 (m, 2H), 3.05 - 2.85 (m, 7H), 2.80 (d, *J* = 3.2 Hz, 3H), 2.75 - 2.67 (m, 1H), 2.66 - 2.58 (m, 1H), 2.22 - 2.12 (m, 1H), 2.10 - 1.97 (m, 6H), 1.94 - 1.72 (m, 7H), 1.26 - 1.12 (m, 2H) |
| **I-259^{b}** | PP | ABC | 846.5 | 11.07 (s, 1H), 9.39 (s, 1H), 8.81 (d, *J* = 8.0 Hz, 1H), 8.37 (s, 1H), 8.28 (s, 1H), 7.24 - 6.82 (m, 5H), 5.36 (dd, *J* = 5.2, 12.4 Hz, 1H), 4.21 - 4.12 (m, 1H), 3.82 - 3.76 (m, 4H), 3.74 - 3.70 (m, 4H), 3.56 (s, 3H), 3.44 - 3.38 (m, 4H), 2.99 - 2.82 (m, 3H), 2.75 - 2.58 (m, 2H), 2.38 - 2.31 (m, 2H), 2.16 - 2.08 (m, 5H), 2.07 - 1.94 (m, 3H), 1.91 - 1.60 (m, 8H), 1.57 - 1.45 (m, 1H), 1.12 - 0.90 (m, 2H) |
| **I-260^{b}** | PP | ACH | 859.6 | 11.07 (s, 1H), 9.40 (s, 1H), 8.78 (d, *J* = 8.0 Hz, 1H), 8.38 (s, 1H), 8.27 (s, 1H), 7.24 - 6.93 (m, 3H), 6.93 - 6.84 (m, 2H), 5.36 (d, *J* = 12.8 Hz, 1H), 4.24 - 4.12 (m, 3H), 3.80 (s, 5H), 3.57 (s, 3H), 3.42 (d, *J* = 6.0 Hz, 4H), 3.00 - 2.92 (m, 2H), 2.87 (d, *J* = 12.8 Hz, 1H), 2.76 - 2.57 (m, 3H), 2.43 (d, *J* = 5.2 Hz, 5H), 2.25 (s, 3H), 2.19 (s, 4H), 2.09 - 1.95 (m, 3H), 1.94 - 1.79 (m, 4H), 1.77 - 1.64 (m, 3H), 1.13 - 0.96 (m, 2H) |
| **I-261^{b}** | AAY | ABF | 843.5 | 11.10 (s, 1H), 9.74 (s, 1H), 8.92 (s, 1H), 8.20 - 8.18 (m, 1H), 8.14 (d, *J* = 5.2 Hz, 1H), 7.31 - 7.06 (m, 3H), 7.05 - 6.97 (m, 3H), 6.86 (d, *J* = 8.0 Hz, 1H), 5.33 (dd, *J* = 5.2, 12.8 Hz, 1H), 4.29 - 4.20 (m, 1H), 3.40 - 3.28 (m, 7H), 3.17 (t, *J* = 6.0 Hz, 2H), 3.03 - 2.84 (m, 3H), 2.75 - 2.59 (m, 4H), 2.36 (t, *J* = 7.2 Hz, 2H), 2.16 - 2.06 (m, 2H), 2.04 - 1.88 (m, 5H), 1.86 - 1.74 (m, 2H), 1.58 - 1.40 (m, 4H), 1.37 - 1.24 (m, 2H), 1.12 - 0.99 (m, 1H), 0.50 - 0.39 (m, 2H), 0.27 - 0.16 (m, 2H) |
| **I-262^{b}** | AAY | ABG | 843.4 | 11.86 (s, 1H), 9.72 (s, 1H), 8.93 (s, 1H), 8.20 (s, 1H), 8.15 (d, *J* = 5.6 Hz, 1H), 7.33 - 7.00 (m, 4H), 6.97 (d, *J* = 4.8 Hz, 2H), 6.91 - 6.85 (m, 1H), 5.37 (dd, *J* = 5.2, 12.4 Hz, 1H), 4.26 - 4.19 (m, 1H), 3.57 (s, 3H), 3.45 - 3.41 (m, 2H), 3.39 (t, *J* = 6.4 Hz, 2H), 3.21 - 3.17 (m, 2H), 3.01 - 2.92 (m, 4H), 2.91 - 2.83 (m, 1H), 2.76 - 2.68 (m, 1H), 2.65 - 2.59 (m, 1H), 2.34 - 2.29 (m, 2H), 2.08 - 1.90 (m, 7H), 1.87 - 1.79 (m, 2H), 1.59 - 1.51 (m, 2H), 1.50 - 1.41 (m, 2H), 1.39 - 1.31 (m, 2H), 1.12 - 1.02 (m, 1H), 0.51 - 0.38 (m, 2H), 0.30 - 0.16 (m, 2H) |
| **I-263^{b}** | ABJ | WW | 812.5 | 11.10 (s, 1H), 9.74 (s, 1H), 8.93 (s, 1H), 8.18 - 8.13 (m, 2H), 7.34 - 7.04 (m, 4H), 7.01 (d, *J* = 5.2 Hz, 1H), 6.98 - 6.92 (m, 1H), 6.88 - 6.84 (m, 1H), 5.41 - 5.32 (m, 1H), 5.08 - 5.00 (m, 1H), 3.70 - 3.67 (m, 1H), 3.67 (s, 3H), 3.65 - 3.63 (m, 1H), 3.60 (s, 2H), 3.33 - 3.29 (m, 2H), 3.19 - 3.16 (m, 2H), 2.94 - 2.87 (m, 1H), 2.83 - 2.77 (m, 2H), 2.73 - 2.60 (m, 4H), 2.06 - 1.99 (m, 1H), 1.99 - 1.90 (m, 2H), 1.66 - 1.57 (m, 2H), 1.38 - 1.27 (m, 1H), 1.27 - 1.18 (m, 2H), 1.14 - 1.02 (m, 3H), 0.48 - 0.41 (m, 2H), 0.24 - 0.19 (m, 2H) |
| **I-264^{b}** | AEL | WW | 840.6 | 11.09 (s, 1H), 9.70 (s, 1H), 8.91 (s, 1H), 8.19 (s, 1H), 8.15 (d, *J* = 5.6 Hz, 1H), 7.31 - 7.04 (m, 4H), 7.03 - 7.00 (m, 1H), 6.95 (t, *J* = 7.8 Hz, 1H), 6.87 (d, *J* = 7.2 Hz, 1H), 5.37 (dd, *J* = 5.2, 12.8 Hz, 1H), 4.28 - 4.18 (m, 1H), 3.67 (s, 3H), 3.61 (s, 2H), 3.20 - 3.16 (m, 2H), 3.01 - 2.94 (m, 2H), 2.93 - 2.85 (m, 1H), 2.85 - 2.77 (m, 2H), 2.77 - 2.69 (m, 1H), 2.65 - 2.60 (m, 1H), 2.52 - 2.51 (m, 1H), 2.39 - 2.33 (m, 2H), 2.11 - 2.07 (m, 1H), 2.07 - 2.03 (m, 1H), 2.03 -2.00 (m, 1H), 2.00 - 1.97 (m, 2H), 1.97 - 1.93 (m, 2H), 1.93 - 1.87 (m, 1H), 1.69- 1.57 (m, 2H), 1.42 - 1.24 (m, 3H), 1.18 - 1.01 (m, 3H), 0.51 - 0.40 (m, 2H), 0.26 - 0.17 (m, 2H) |
| **I-265^{b}** | ACX | WW | 826.5 | 11.10 (s, 1H), 9.71 (s, 1H), 8.92 (s, 1H), 8.19 (s, 1H), 8.15 (d, *J* = 5.4 Hz, 1H), 7.30 - 7.04 (m, 4H), 7.01 (dd, *J* = 5.4 Hz, 1H), 6.95 (t, *J* = 7.6 Hz, 1H), 6.87 (d, *J* = 7.6 Hz, 1H), 5.38 (dd, *J* = 5.4, 1H), 4.27 - 4.19 (m, 1H), 3.67 (s, 3H), 3.62 (s, 2H), 3.19 - 3.16 (m, 2H), 2.91 (d, *J* = 11.6 Hz, 3H), 2.86 - 2.79 (m, 2H), 2.76 - 2.68 (m, 1H), 2.65 - 2.58 (m, 1H), 2.15 (d, *J* = 6.4 Hz, 2H), 2.05 - 1.89 (m, 9H), 1.74 - 1.62 (m, 2H), 1.58 - 1.46 (m, 1H), 1.14 - 0.98 (m, 3H), 0.48 - 0.42 (m, 2H), 0.25 - 0.19 (m, 2H) |
| **I-266** | ABL | PW | 868.2 | 11.12 (s, 1H), 9.68 (s, 1H), 8.91 (s, 1H), 8.19 (s, 1H), 8.14 (d, *J* = 5.2 Hz, 1H), 7.31 - 6.88 (m, 7H), 5.44 - 5.40 (m, 1H), 4.61 ( t, *J* = 11.2 Hz, 1H), 4.23-4.16 (m, 11.6 Hz, 1H), 3.60 (d, *J* = 2.8 Hz, 2H), 3.31 (s, 3H), 3.21 (s, 2H), 3.18 (t, *J* = 6.0 Hz, 2H), 3.05 - 2.99 (m, 1H), 2.95 - 2.87 (m, 1H), 2.85 - 2.82 (m, 1H), 2.79 - 2.69 (m, 1H), 2.63 - 2.58 (m, 1H), 2.25 (d, *J* = 7.2 Hz, 1H), 2.22 - 2.17 (m, 3H), 2.06 - 2.02 (m, 2H), 1.95 - 1.86 (m, 2H), 1.85 - 1.68 (m, 3H), 1.62 (d, *J* = 11.2 Hz, 1H), 1.53 - 1.22 (m, 3H), 1.13 - 0.92 (m, 3H), 0.49 - 0.40 (m, 2H), 0.26 - 0.17 (m, 2H) |
| **I-267^{b}** | TN | ABD | 850.2 | 11.10 (s, 1H), 9.87 (s, 1H), 8.52 (d, *J* = 7.6 Hz, 1H), 8.23 (s, 1H), 8.13 (s, 1H), 6.97 (d, *J* = 4.8 Hz, 2H), 6.91 - 6.85 (m, 1H), 6.41 (d, *J* = 7.6 Hz, 1H), 5.37 (d, *J* = 9.2 Hz, 1H), 5.32 (s, 1H), 4.05 - 3.93 (m, 1H), 3.57 (s, 3H), 3.53 - 3.43 (m, 6H), 3.01 - 2.93 (m, 2H), 2.92 - 2.84 (m, 1H), 2.77 - 2.58 (m, 6H), 2.10 (d, *J* = 6.8 Hz, 2H), 2.05 - 1.97 (m, 5H), 1.91 - 1.77 (m, 7H), 1.49 (s, 6H), 1.46 - 1.39 (m, 2H), 1.10 - 0.96 (m, 3H), 0.54 - 0.47 (m, 2H), 0.32 - 0.26 (m, 2H) |
| **I-268^{b}** | WX | ABD | 835.2 | 11.11 (s, 1H), 9.86 (s, 1H), 8.51 (d, *J* = 7.6 Hz, 1H), 8.22 (s, 1H), 8.18 (s, 1H), 7.07 (d, *J* = 7.6 Hz, 1H), 6.96 (d, *J* = 7.6 Hz, 1H), 6.86 (d, *J* = 7.6 Hz, 1H), 6.41 (d, *J* = 7.6 Hz, 1H), 5.38 (d, *J =* 9.2 Hz, 1H), 5.31 (s, 1H), 4.02 - 3.93 (m, 1H), 3.67 (s, 4H), 3.61 (s, 2H), 3.54 - 3.47 (m, 3H), 2.87 (d, *J* = 10.0 Hz, 3H), 2.78 - 2.58 (m, 2H), 2.22 (d, *J* = 6.8 Hz, 2H), 2.18 (s, 3H), 2.05 - 1.92 (m, 5H), 1.86 (d, *J* = 11.2 Hz, 2H), 1.72 - 1.58 (m, 4H), 1.48 (s, 6H), 1.46 - 1.31 (m, 3H), 1.09 - 0.92 (m, 3H), 0.54 - 0.47 (m, 2H), 0.31 - 0.25 (m, 2H) |
| **I-269^{b}** | ADG | ADI | 708.5 | 11.09 (s, 1H), 8.44 (s, 1H), 8.18 (s, 1H), 8.01 (d, *J =* 7.6 Hz, 1H), 7.74 - 7.66 (m, 2H), 6.99 - 6.92 (m, 2H), 6.90 - 6.83 (m, 1H), 5.40 - 5.33 (m, 1H), 4.16 (s, 2H), 4.13 (s, 1H), 3.56 (s, 3H), 3.45 - 3.37 (m, 4H), 3.02 - 2.93 (m, 2H), 2.92 - 2.81 (m, 1H), 2.77 - 2.61 (m, 2H), 2.58 (s, 4H), 2.42 - 2.29 (m, 7H), 2.06 - 1.98 (m, 3H), 1.89 (d, *J* = 10.4 Hz, 2H), 1.85 - 1.78 (m, 2H), 1.71 - 1.63 (m, 2H), 1.49 - 1.33 (m, 4H) |
| **I-270^{b}** | AHG | WW | 637.4 | - |
| **I-271^{b,e}** | ADL | ADJ | 709.5 | 11.08 (s, 1H), 9.16 - 8.84 (m, 1H), 8.55 (s, 1H), 8.32 - 8.10 (m, 1H), 8.09 - 7.71 (m, 2H), 7.10 - 6.82 (m, 3H), 5.35 (dd, *J* = 4.8, 12.4 Hz, 1H), 4.36 - 4.04 (m, 1H), 3.56 (s, 3H), 3.46 - 3.41 (m, 6H), 2.95 (t, *J =* 7.2 Hz, 2H), 2.90 - 2.80 (m, 2H), 2.78 - 2.68 (m, 2H), 2.65 - 2.57 (m, 2H), 2.31 - 2.17 (m, 2H), 2.15 - 2.08 (m, 1H), 2.04 - 1.96 (m, 2H), 1.91 - 1.78 (m, 7H), 1.76 - 1.69 (m, 2H), 1.67 - 1.59 (m, 1H), 1.56 - 1.48 (m, 1H), 1.46 - 1.34 (m, 4H) |
| **I-272^{b}** | ABT | ADN | 788.2 | 13.14 (s, 1H), 11.08 (s, 1H), 9.38 (dd, *J* = 1.6, 7.2 Hz, 1H), 9.13 (dd, *J* = 1.6, 4.4 Hz, 1H), 8.73 (s, 1H), 8.51 (s, 1H), 7.88 - 7.76 (m, 2H), 7.37 (dd, *J* = 4.4, 7.2 Hz, 1H), 6.96 (d, *J* = 4.4 Hz, 2H), 6.91 - 6.84 (m, 2H), 5.36 (dd, *J* = 5.2, 12.4 Hz, 1H), 3.56 (s, 3H), 3.44 - 3.36 (m, 4H), 3.06 - 2.81 (m, 5H), 3.06 - 2.81 (m, 1H), 2.64 - 2.57 (m, 1H), 2.54 - 2.51 (m, 1H), 2.40 (s, 3H), 2.39 - 2.36 (m, 2H), 2.13 (t, *J =* 10.8 Hz, 2H), 2.02 - 1.92 (m, 3H), 1.87 - 1.79 (m, 2H), 1.76 - 1.66 (m, 2H), 1.58 - 1.46 (m, 4H), 1.39 - 1.29 (m, 2H) |
| **I-273^{b}** | AND | ABF | 788.5 | 13.14 (s, 1H), 11.08 (s, 1H), 9.37 (dd, *J* = 1.6, 7.2 Hz, 1H), 9.13 (dd, *J =* 1.6, 4.4 Hz, 1H), 8.73 (s, 1H), 8.50 (s, 1H), 7.88 - 7.74 (m, 2H), 7.36 (dd, *J* = 4.4, 7.2 Hz, 1H), 7.09 - 6.96 (m, 2H), 6.92 - 6.83 (m, 2H), 5.33 (dd, *J* = 5.2, 12.8 Hz, 1H), 3.36 (t, *J* = 6.4 Hz, 4H), 3.32 (s, 3H), 3.03 - 2.95 (m, 2H), 2.94 - 2.83 (m, 1H), 2.74 - 2.62 (m, 3H), 2.41 - 2.32 (m, 6H), 2.16 - 2.06 (m, 2H), 2.03 - 1.90 (m, 3H), 1.87 - 1.63 (m, 5H), 1.56 - 1.15 (m, 4H), 1.37 - 1.29 (m, 2H) |
| **I-274^{f}** | QG | PW | 1186.6 | 9.70 (s, 1H), 8.92 (s, 1H), 8.48 (s, 1H), 8.26 (s, 2H), 8.18 (s, 1H), 8.14 (d, *J* = 5.6 Hz, 1H), 7.98 (d, *J* = 8.4 Hz, 1H), 7.68 (d, *J* = 7.6 Hz, 1H), 7.64 (d, *J* = 2.4 Hz, 1H), 7.46 (t, *J* = 8.0 Hz, 1H), 7.31 - 7.08 (m, 4H), 7.08 - 6.98 (m, 2H), 5.39 (dd, *J* = 3.2, 8.0 Hz, 1H), 4.51 - 4.49 (m, 1H), 4.18 - 4.15 (m, 2H), 3.82 - 3.76 (m, 6H), 3.62 -3.59 (m, 10H), 3.19 - 3.17 (m, 2H), 3.06 - 3.04 (m, 1H), 2.79 - 2.77 (m, 2H), 2.20 (s, 3H), 2.07 (s, 4H), 2.05 - 2.03 (m, 4H), 1.93 - 1.86 (m, 2H), 1.80 - 1.49 (m, 11H), 1.13 (s, 3H), 0.99 (m, 7H), 0.94 (m, 1H), 0.47 - 0.42 (m, 2H), 0.24 - 0.20 (m, 2H) |
| **I-317^{b}** | AGW | AGL | 843.5 | 12.36 (s, 1H), 11.08 (s, 1H), 8.71 (s, 1H), 8.48 - 8.42 (m, 1H), 8.40 - 8.31 (m, 2H), 8.16 (d, *J* = 7.6 Hz, 1H), 7.71 - 7.63 (m, 1H), 7.57 (s, 1H), 7.38 - 7.27 (m, 2H), 5.94 (s, 1H), 5.09 (dd, *J* = 5.6, 12.8 Hz, 1H), 4.50 - 4.35 (m, 1H), 3.78 - 3.59 (m, 2H), 2.97 - 2.79 (m, 3H), 2.63 - 2.53 (m, 2H), 2.24 - 1.80 (m, 16H), 1.76 - 1.63 (m, 2H), 1.62 (s, 6H), 1.39 - 1.25 (m, 2H), 1.18 - 1.05 (m, 2H) |
| **I-318^{b}** | ANV | AJB | 933.5 | 11.16 - 11.02 (m, 1H), 9.49 (d, *J* = 6.4 Hz, 1H), 8.78 (d, *J* = 7.6 Hz, 1H), 8.38 (d, *J* = 4.4 Hz, 1H), 8.25 (d, *J* = 5.6 Hz, 1H), 7.48 (dd, *J* = 7.2, 8.4 Hz, 1H), 7.34 - 7.29 (m, 2H), 7.28 - 7.09 (m, 4H), 7.02 - 6.94 (m, 2H), 6.88 - 6.43 (m, 1H), 5.30 - 5.02 (m, 2H), 4.76 (d, *J* = 17.6 Hz, 1H), 4.53 (d, *J* = 6.0 Hz, 2H), 4.15 (t, *J* = 2.4, 11.6 Hz, 1H), 3.80 (s, 1H), 3.74 (d, *J* = 7.6 Hz, 1H), 3.65 - 3.57 (m, 2H), 3.45 (s, 3H), 2.95 - 2.88 (m, 1H), 2.62 - 2.55 (m, 1H), 2.41 (s, 4H), 2.12 (s, 6H), 2.10 - 2.07 (m, 2H), 2.05 - 1.97 (m, 5H), 1.85 (br d, *J* = 11.2 Hz, 2H), 1.76 - 1.66 (m, 2H), 1.54 - 1.43 (m, 1H), 1.04 - 0.91 (m, 2H) |
| **I-320^{b}** | AMC | AMT | 866.4 | 11.12 - 10.99 (m, 1H), 9.63 - 9.55 (m, 1H), 8.94 (d, *J* = 3.2 Hz, 1H), 8.80 (d, *J* = 7.6 Hz, 1H), 8.30 (d, *J* = 5.6 Hz, 1H), 7.75 (d, *J* = 8.4 Hz, 2H), 7.56 - 7.50 (m, 1H), 7.42 (d, *J =* 8.4 Hz, 2H), 7.29 - 7.11 (m, 1H), 7.02 (d, *J* = 8.4 Hz, 1H), 6.98 (d, *J* = 7.2 Hz, 1H), 6.91 - 6.45 (m, 2H), 5.32 - 5.07 (m, 1H), 5.06 - 4.97 (m, 1H), 4.83 - 4.74 (m, 1H), 3.87 - 3.73 (m, 2H), 3.68 - 3.60 (m, 2H), 3.47 (s, 2H), 3.45 (s, 1H), 3.44 (s, 1H), 3.42 (s, 1H), 3.41 (s, 1H), 3.40 (s, 1H), 2.92 - 2.80 (m, 2H), 2.57 (d, *J* = 2.0 Hz, 1H), 2.39 (t, *J =* 7.0 Hz, 2H), 2.12 (s, 3H), 2.09 - 1.93 (m, 4H), 1.80 - 1.75 (m, 2H), 1.75 - 1.69 (m, 2H) |
| **I-321^{b}** | AQN | AQM | 888.5 | 11.31 - 10.74 (m, 1H), 9.50 (d, *J* = 5.6 Hz, 1H), 8.78 (d, *J* = 7.6 Hz, 1H), 8.41 - 8.32 (m, 1H), 8.25 (d, *J* = 5.6 Hz, 1H), 7.62 - 7.52 (m, 1H), 7.26 - 6.94 (m, 3H), 6.89 - 6.39 (m, 2H), 5.29 - 5.00 (m, 2H), 4.77 (d, *J* = 16.0 Hz, 1H), 4.25 - 4.06 (m, 1H), 4.02 - 3.84 (m, 1H), 3.84 - 3.71 (m, 2H), 3.70 - 3.42 (m, 4H), 3.19 - 3.10 (m, 2H), 2.96 - 2.79 (m, 4H), 2.62 - 2.55 (m, 2H), 2.13 - 1.80 (m, 10H), 1.25 - 0.87 (m, 11H) |
| **I-322^{b}** | AOC | ABC | 920.2 | 9.39 (s, 1H), 8.82 (d, J *=* 7.9 Hz, 1H), 8.37 (s, 1H), 8.28 (s, 1H), 7.52 - 7.46 (m, 1H), 7.43 (d, J = 8.0 Hz, 2H), 7.36 - 7.28 (m, 4H), 7.24 - 6.94 (m, 1H), 6.90 (d, J = 7.6 Hz, 1H), 5.25 - 5.20 (m, 2H), 5.20 - 5.15 (m, 1H), 4.44 - 4.37 (m, 1H), 4.28 - 4.22 (m, 1H), 4.21 - 4.11 (m, 1H), 3.79 (s, 4H), 3.74 - 3.68 (m, 4H), 3.45 (s, 2H), 3.03 - 2.93 (m, 4H), 2.78 - 2.69 (m, 1H), 2.53 - 2.51 (m, 1H), 2.48 - 2.34 (m, 8H), 2.10 (d, J = 6.8 Hz, 2H), 2.06 - 1.94 (m, 3H), 1.87 (d, J = 12.8 Hz, 2H), 1.79 - 1.65 (m, 2H), 1.60 - 1.49 (m, 1H), 1.09 - 0.95 (m, 2H) |
| **I-323^{b}** | ATW | AJB | 894.6 | 9.49 (d, *J* = 6.0 Hz, 1H), 8.78 (d, *J* = 7.6 Hz, 1H), 8.38 (d, *J* = 4.4 Hz, 1H), 8.25 (d, *J* = 5.6 Hz, 1H), 7.64 - 7.51 (m, 1H), 7.26 - 6.94 (m, 3H), 6.90 - 6.41 (m, 2H), 5.33 - 5.02 (m, 2H), 4.76 (d, *J =* 17.2 Hz, 1H), 4.24 - 4.09 (m, 1H), 3.85 - 3.55 (m, 4H), 3.37 - 3.34 (m, 4H), 3.02 (s, 3H), 2.99 - 2.88 (m, 1H), 2.81 - 2.71 (m, 1H), 2.59 - 2.52 (m, 2H), 2.29 - 2.18 (m, 2H), 2.13 - 2.10 (m, 2H), 2.06 - 1.91 (m, 5H), 1.90 - 1.80 (m, 4H), 1.79 - 1.66 (m, 2H), 1.61 - 1.52 (s, 3H), 1.53 - 1.44 (m, 4H), 1.10 - 0.92 (m, 2H) |
| **I-324^{b}** | AOL | AJB | 868.5 | 11.10 (s, 1H), 9.49 (d, *J* = 6.0 Hz, 1H), 8.78 (d, *J* = 7.6 Hz, 1H), 8.38 (d, *J* = 4.4 Hz, 1H), 8.25 (d, *J* = 5.6 Hz, 1H), 7.57 (dd, *J* = 7.2, 8.4 Hz, 1H), 7.27 - 6.91 (m, 3H), 6.90 - 6.40 (m, 2H), 5.33 - 4.98 (m, 2H), 4.76 (d, *J* = 16.8 Hz, 1H), 4.23 - 4.10 (m, 1H), 3.83 - 3.74 (m, 2H), 3.65 - 3.59 (m, 2H), 3.46 - 3.44 (m, 1H), 3.16 (t, *J* = 6.0 Hz, 2H), 2.93 - 2.83 (m, 1H), 2.64 - 2.56 (m, 1H), 2.40 - 2.34 (m, 1H), 2.24 (d, *J* = 6.8 Hz, 2H), 2.19 (s, 3H), 2.08 - 1.98 (m, 4H), 1.97 - 1.79 (m, 5H), 1.78 - 1.66 (m, 4H), 1.55 - 1.42 (m, 2H), 1.29 - 1.12 (m, 2H), 1.09 - 0.88 (m, 4H) |
| **I-325^{b}** | ANM | ABC | 878.3 | 11.08 (s, 1H), 9.39 (s, 1H), 8.82 (d, *J* = 8.0 Hz, 1H), 8.37 (s, 1H), 8.28 (s, 1H), 7.57 (dd, *J* = 7.2, 8.4 Hz, 1H), 7.28 - 6.88 (m, 8H), 6.57 (t, *J* = 6.0 Hz, 1H), 5.04 (dd, *J* = 5.6, 12.8 Hz, 1H), 4.22 - 4.09 (m, 1H), 3.83 - 3.75 (m, 4H), 3.75 - 3.68 (m, 4H), 3.59 - 3.51 (m, 2H), 3.41 (s, 2H), 2.94 - 2.81 (m, 3H), 2.62 - 2.53 (m, 2H), 2.15 - 2.11 (m, 2H), 2.10 (s, 3H), 2.06 - 1.97 (m, 3H), 1.97 - 1.89 (m, 2H), 1.81 - 1.68 (m, 2H), 1.66 - 1.54 (m, 1H), 1.07 - 0.87 (m, 2H) |
| **I-326^{b}** | AML | AGL | 855.5 | 12.36 (s, 1H), 11.10 (s, 1H), 8.71 (s, 1H), 8.47 - 8.42 (m, 1H), 8.39 - 8.33 (m, 2H), 8.17 - 8.13 (m, 1H), 7.64 - 7.53 (m, 2H), 7.06 (d, *J* = 7.2 Hz, 1H), 7.00 (d, *J* = 8.4 Hz, 1H), 6.45 (d, *J* = 6.4 Hz, 1H), 5.94 (s, 1H), 5.06 (dd, *J* = 5.2, 12.8 Hz, 1H), 4.47 - 4.35 (m, 1H), 4.17 - 4.06 (m, 1H), 2.94 - 2.83 (m, 1H), 2.63 - 2.52 (m, 2H), 2.48 - 2.44 (m, 1H), 2.39 - 2.20 (m, 6H), 2.17 - 2.09 (m, 4H), 2.06 - 1.99 (m, 1H), 1.97 - 1.85 (m, 4H), 1.72 - 1.64 (m, 4H), 1.62 (s, 6H), 1.58 - 1.54 (m, 2H), 1.18 - 1.03 (m, 2H) |
| **I-327^{b}** | AJH | ALU | 879.2 | 11.08 (s, 1H), 10.48 (s, 1H), 8.55 (s, 1H), 8.48 (s, 1H), 8.47 - 8.38 (m, 2H), 8.22 (dd, *J* = 7.6 Hz, 1H), 7.98 (s, 1H), 7.58 (dd, *J* = 8.4 Hz, 1H), 7.40 - 7.07 (m, 2H), 7.03 (d, *J* = 6.8 Hz, 1H), 6.67 (t, *J* = 3.2 Hz, 1H), 5.08-5.03 (m, 1H), 4.58 - 4.43 (m, 1H), 3.48-3.45 (m, 2H), 3.41 - 3.37 (m, 3H), 3.22 (br d, *J* = 6.8 Hz, 2H), 2.94 - 2.80 (m, 4H), 2.21 - 2.12 (m, 4H), 2.07 - 1.82 (m, 9H), 1.70 - 1.54 (m, 4H), 1.23 - 1.06 (m, 4H) |
| **I-328^{b}** | ALF | AJB | 853.0 | 10.96 (s, 1H), 9.49 (d, *J* = 6.0 Hz, 1H), 8.78 (d, *J* = 8.0 Hz, 1H), 8.38 (d, *J* = 4.4 Hz, 1H), 8.25 (d, *J* = 5.6 Hz, 1H), 7.49 - 7.41 (m, 1H), 7.30 (d, *J* = 7.6 Hz, 1H), 7.24 - 6.96 (m, 2H), 6.90 - 6.41 (m, 1H), 5.30 - 5.05 (m, 2H), 4.91 - 4.84 (m, 1H), 4.76 (d, *J* = 18.4 Hz, 1H), 4.42 - 4.33 (m, 1H), 4.26 - 4.14 (m, 2H), 3.80 (s, 2H), 3.63 (d, *J* = 11.2 Hz, 1H), 3.59 (s, 1H), 3.47 - 3.42 (m, 2H), 2.97 - 2.84 (m, 2H), 2.30 - 2.23 (m, 2H), 2.10 - 1.70 (m, 15H), 1.65 - 1.50 (m, 6H), 1.10 - 0.97 (m, 2H) |
| **I-329^{b}** | ANM | AFM | 900.4 | 11.09 (s, 1H), 8.86 (s, 1H), 8.30 (s, 1H), 8.01 (s, 1H), 7.89 (s, 1H), 7.76 (s, 1H), 7.73 (s, 1H), 7.61 - 7.54 (m, 1H), 7.28 - 7.20 (m, 4H), 7.15 (d, *J* = 8.4 Hz, 1H), 7.02 (d, *J* = 6.8 Hz, 1H), 6.57 (t, *J* = 6.0 Hz, 1H), 5.06 - 5.02 (m, 1H), 4.99 - 4.81 (m, 1H), 4.55 - 4.53 (m, 1H), 4.28 - 4.26 (m, 1H), 4.09 (s, 1H), 3.97 (s, 3H), 3.58 - 3.51 (m, 3H), 3.40 (s, 2H), 2.88 (m, 3H), 2.62 - 2.54 (m, 3H), 2.12 - 2.10 (m, 2H), 2.09 (s, 3H), 2.08 - 1.98 (m, 3H), 1.91 - 1.81 (m, 2H), 1.64 - 1.43 (m, 5H), 1.01 (t, *J* = 7.2 Hz, 3H), 0.96 - 0.83 (m, 2H) |
| **I-330^{b}** | AVA | ABC | 858.4 | 11.01 (s, 1H), 9.40 (s, 1H), 8.83 (d, *J* = 8.0 Hz, 1H), 8.38 (s, 1H), 8.29 (s, 1H), 7.29 (t, *J* = 7.6 Hz, 1H), 7.09(t, *J =* 53.6 Hz, 1H), 6.95 - 6.89 (m, 2H), 6.77 (d, *J* = 8.0 Hz, 1H), 5.58 (t, *J* = 5.6 Hz, 1H), 5.16 - 5.09 (m, 1H), 4.26 - 4.09 (m, 3H), 3.83 - 3.76 (m, 4H), 3.75 - 3.70 (m, 4H), 3.52 (t, *J =* 6.0 Hz, 2H), 3.46 - 3.37 (m, 2H), 3.23 - 3.17 (m, 2H), 3.00 - 2.79 (m, 3H), 2.65 - 2.56 (m, 2H), 2.46 - 2.37 (m, 2H), 2.31 - 2.24 (m, 1H), 2.08 - 2.01 (m, 3H), 1.88 (d, *J* = 10.0 Hz, 4H), 1.81 (t, *J =* 6.4 Hz, 2H), 1.78 - 1.70 (m, 2H), 1.70 - 1.51 (m, 3H), 1.15 - 1.03 (m, 2H) |
| **I-331^{b}** | ANK | AGL | 897.4 | 11.10 (s, 1H), 8.71 (s, 1H), 8.50 - 8.33 (m, 3H), 8.18 - 8.15 (m, 1H), 7.64 - 7.53 (m, 2H), 7.15 - 7.01 (m, 2H), 6.47 (t, *J =* 5.2 Hz, 1H), 5.95 (s, 1H), 5.06 (dd, *J =* 5.2, 12.8 Hz, 1H), 4.46 - 4.36 (m, 1H), 2.94 - 2.83 (m, 2H), 2.64 - 2.57 (m, 2H), 2.25 - 2.23 (m, 2H), 2.20 (s, 3H), 2.16 - 2.10 (m, 2H), 2.07 - 2.01 (m, 1H), 1.98 - 1.75 (m, 8H), 1.68 - 1.67 (m, 1H), 1.65 - 1.62 (m, 6H), 1.60 - 1.42 (m, 6H), 1.33 - 1.00 (m, 7H) |
| **I-332^{b}** | ALJ | ABC | 888.3 | 11.09 (s, 1H), 9.40 (s, 1H), 8.83 (d, *J* = 8.0 Hz, 1H), 8.37 (d, *J* = 2.0 Hz, 1H), 8.29 (d, *J* = 0.4 Hz, 1H), 7.62 - 7.53 (m, 1H), 7.28 - 6.94 (m, 3H), 6.91 (d, *J* = 8.0 Hz, 1H), 6.66 (q, *J =* 5.6 Hz, 1H), 5.05 (dd, *J =* 5.2, 12.8 Hz, 1H), 4.23 - 4.11 (m, 1H), 3.83 - 3.69 (m, 9H), 3.64 - 3.56 (m, 1H), 3.51 - 3.48 (m, 3H), 3.44 - 3.34 (m, 4H), 2.95 - 2.83 (m, 1H), 2.76 - 2.57 (m, 3H), 2.18 - 1.95 (m, 6H), 1.94 - 1.65 (m, 8H), 1.61 - 1.50 (m, 1H), 1.10 - 0.96 (m, 2H) |
| **I-333^{b}** | AMC | AGL | 861.3 | 12.37 (s, 1H), 11.10 (s, 1H), 8.72 (s, 1H), 8.48 - 8.43 (m, 1H), 8.41 - 8.31 (m, 2H), 8.21 - 8.16 (m, 1H), 7.61 - 7.56 (m, 2H), 7.11 (d, *J* = 8.8 Hz, 1H), 7.03 (d, *J* = 6.8 Hz, 1H), 6.68 (t, *J* = 5.6 Hz, 1H), 5.97 (s, 1H), 5.05 (dd, *J =* 12.8, 5.4 Hz, 1H), 4.45 - 4.43 (m, 1H), 3.50 - 3.40 (m, 5H), 2.93 - 2.83 (m, 1H) 2.62 - 2.52 (m, 2H), 2.38 - 2.33 (m, 2H), 2.16 - 2.10 (m, 6H), 2.05 - 1.97 (m, 1H), 1.95 - 1.80 (m, 6H), 1.70 - 1.65 (m, 2H), 1.64 - 1.52 (m, 7H), 1.14 - 1.03 (m, 2H) |
| **I-334^{b}** | ALE | AFM | 923.6 | 11.08 (s, 1H), 8.85 (s, 1H), 8.28 (s, 1H), 8.01 (s, 1H), 7.89 (s, 1H), 7.82 - 7.67 (m, 2H), 7.57 (t, *J =* 7.8 Hz, 1H), 7.13 - 6.94 (m, 2H), 6.73 (s, 1H), 5.08 - 5.00 (m, 1H), 4.99 - 4.78 (m, 1H), 4.53 (d, *J* = 9.8 Hz, 1H), 4.32 - 4.22 (m, 1H), 4.09 (s, 1H), 3.97 (s, 3H), 3.83 - 3.76 (m, 1H), 2.92 - 2.77 (m, 3H), 2.73 - 2.64 (m, 3H), 2.38 - 2.30 (s, 5H), 2.13 (s, 3H), 2.11 - 1.93 (m, 8H), 1.80 (s, 3H), 1.72 - 1.52 (m, 5H), 1.51 - 1.39 (m, 3H), 1.22 - 1.12 (m, 1H), 1.01 (t, *J =* 6.8 Hz, 3H), 0.96 - 0.86 (m, 2H) |
| **I-335^{b}** | AMH | AJB | 920.5 | 10.96 (s, 1H), 9.49 (d, *J* = 6.0 Hz, 1H), 8.78 (d, *J* = 8.0 Hz, 1H), 8.38 (d, *J* = 4.4 Hz, 1H), 8.25 - 8.24 (m, 1H), 7.49 - 6.94 (m, 8H), 6.89 - 6.38 (m, 1H), 5.32 - 5.03 (m, 4H), 4.85 - 4.72 (m, 1H), 4.47 - 4.36 (m, 1H), 4.32 - 4.21 (m, 1H), 4.21 - 4.10 (m, 1H), 3.86 -3.76 (m, 2H), 3.66 - 3.59 (m, 4H), 2.95 - 2.84 (m, 1H), 2.62 - 2.53 (m, 2H), 2.47 - 2.37 (m, 5H), 2.16 - 2.10 (m, 7H), 2.07 - 1.92 (m, 5H), 1.92 - 1.82 (m, 2H), 1.78 - 1.65 (m, 2H), 1.56 - 1.44 (m, 1H), 1.08 - 0.90 (m, 2H) |
| **I-337^{b}** | AMD | AJB | 913.2 | 11.08 (s, 1H), 9.95 (s, 1H), 8.96 (s, 1H), 8.77 (s, 1H), 8.16 (d, *J* = 5.2 Hz, 1H), 7.78 (d, *J* = 8.4 Hz, 2H), 7.58 - 7.50 (m, 1H), 7.43 (d, *J* = 8.4 Hz, 2H), 7.31 - 7.01 (m, 5H), 6.99 (d, *J =* 7.2 Hz, 1H), 6.63 (t, *J* = 6.0 Hz, 1H), 5.03 (dd, *J =* 5.2, 12.4 Hz, 1H), 3.49 (s, 2H), 3.45 - 3.41 (m, 4H), 3.33 - 3.31 (m, 2H), 3.18 (t, *J =* 6.0 Hz, 2H), 2.92 - 2.80 (m, 1H), 2.61 - 2.52 (m, 2H), 2.40 (t, *J =* 6.8 Hz, 2H), 2.13 (s, 3H), 2.06 - 1.96 (m, 1H), 1.84 - 1.66 (m, 4H), 1.15 - 0.98 (m, 1H), 0.51 - 0.36 (m, 2H), 0.27 - 0.14 (m, 2H) |
| **I-339^{b}** | AMD | PW | 912.4 | 11.07 (s, 1H), 9.69 (s, 1H), 8.91 (s, 1H), 8.19 - 8.11 (m, 2H), 7.57 (dd, *J =* 7.2, 8.4 Hz, 1H), 7.30 - 6.97 (m, 6H), 6.77 - 6.70 (m, 1H), 5.04 (dd, *J* = 5.2, 12.8 Hz, 1H), 4.24 - 4.12 (m, 1H), 3.82 -3.75 (m, 1H), 3.53 -3.45 (m, 2H), 3.44 - 3.38 (m, 2H), 3.18 (t, *J* = 6.4 Hz, 2H), 2.93 - 2.76 (m, 2H), 2.73 -2.68 (m, 1H), 2.62 - 2.54 (m, 2H), 2.42 - 2.35 (m, 4H), 2.14 (s, 3H), 2.12 (br d, *J =* 7.2 Hz, 2H), 2.09 - 1.98 (m, 4H), 1.92 - 1.85 (m, 2H), 1.84 - 1.75 (m, 2H), 1.74 - 1.63 (m, 3H), 1.57 - 1.45 (m, 1H), 1.11 - 0.93 (m, 3H), 0.49 - 0.39 (m, 2H), 0.27 - 0.16 (m, 2H) |
| **I-340^{b}** | ALE | AJB | 913.1 | 11.08 (s, 1H), 9.49 (d, *J =* 6.0 Hz, 1H), 8.78 (d, *J =* 7.6 Hz, 1H), 8.38 (d, *J =* 4.4 Hz, 1H), 8.25 (d, *J =* 5.6 Hz, 1H), 7.57 (dd, *J* = 7.2, 8.4 Hz, 1H), 7.26 - 6.91 (m, 3H), 6.90 - 6.42 (m, 2H), 5.32 - 4.99 (m, 2H), 4.77 (d, *J* = 16.8 Hz, 1H), 4.21 - 4.10 (m, 1H), 3.87 - 3.67 (m, 4H), 3.66 - 3.51 (m, 5H), 2.94 - 2.77 (m, 2H), 2.73 - 2.52 (m, 3H), 2.43 - 2.37 (m, 4H), 2.16 (s, 3H), 2.13 (s, 2H), 2.09 - 1.93 (m, 6H), 1.93 - 1.80 (m, 3H), 1.79 - 1.65 (m, 4H), 1.58 - 1.44 (m, 1H), 1.09 - 0.91 (m, 2H) |
| **I-342^{b}** | ALJ | AFM | 910.4 | 11.08 (s, 1H), 8.86 (s, 1H), 8.28 (s, 1H), 8.01 (s, 1H), 7.89 (s, 1H), 7.76 (s, 1H), 7.73 (s, 1H), 7.57 (dd, *J* = 7.2, 8.4 Hz, 1H), 7.11 (d, *J =* 8.4 Hz, 1H), 7.02 (d, *J* = 7.2 Hz, 1H), 6.69 - 6.61 (m, 1H), 5.05 (dd, *J* = 5.2, 12.8 Hz, 1H), 4.99 - 4.81 (m, 1H), 4.54 (dd, *J* = 3.2, 11.2 Hz, 1H), 4.26 (dd, *J* = 6.0, 11.2 Hz, 1H), 4.10 (d, *J* = 6.4 Hz, 1H), 3.98 (s, 3H), 3.74 (d, *J =* 11.2 Hz, 1H), 3.64 - 3.55 (m, 1H), 3.49 (t, *J =* 5.6 Hz, 3H), 3.43 - 3.37 (m, 2H), 3.37 - 3.35 (m, 2H), 2.94 - 2.82 (m, 1H), 2.73 - 2.68 (m, 1H), 2.65 - 2.53 (m, 4H), 2.10 - 1.99 (m, 5H), 1.97 - 1.90 (m, 1H), 1.85 - 1.72 (m, 5H), 1.69 - 1.36 (m, 6H), 1.01 (t, *J* = 7.2 Hz, 3H), 0.98 - 0.84 (m, 2H) |
| **I-343^{b}** | ANQ | PW | 912.5 | 11.09 (s, 1H), 9.68 (s, 1H), 8.92 (s, 1H), 8.16 - 8.13 (m, 2H), 7.57 (t, *J* = 8.0 Hz, 1H), 7.30 - 6.98 (m, 6H), 6.91 - 6.79 (m, 1H), 5.04 (dd, *J =* 5.2, 12.8 Hz, 1H), 4.15 - 4.11 (m, 1H), 3.76 (d, *J* = 10.8 Hz, 1H), 3.69 - 3.61 (m, 2H), 3.59 (d, *J* = 10.8 Hz, 1H), 3.40 - 3.33 (m, 2H), 3.18 ( t, *J* = 6.0 Hz, 2H), 2.86 (d, *J =* 11.6 Hz, 2H), 2.74 - 2.59 (m, 2H), 2.43 - 2.33 (m, 4H), 2.24 - 2.13 (m, 5H), 2.06 - 1.94 (m, 4H), 1.91 - 1.81 (m, 2H), 1.79 - 1.66 (m, 5H), 1.55 - 1.54 (m, 1H), 1.10 - 0.91 (m, 3H), 0.50 - 0.41 (m, 2H), 0.27 - 0.17 (m, 2H) |
| **I-344^{b}** | AFJ | ALZ | 884.5 | 11.09 (s, 1H), 9.53 (s, 1H), 8.87 (d, *J* = 8.0 Hz, 1H), 8.38 (s, 1H), 8.30 (s, 1H), 7.62 - 7.55 (m, 1H), 7.23 - 6.94 (m, 3H), 6.74 (d, *J =* 8.0 Hz, 1H), 6.63 ( t, *J* = 5.6 Hz, 1H), 5.09 - 5.01 (m, 1H), 4.80 - 4.68 (m, 2H), 4.22 - 4.11 (m, 1H), 4.03 - 3.77 (m, 4H), 3.49 (t, *J* = 5.6 Hz, 2H), 3.40 - 3.35 (m, 2H), 3.30 - 3.22 (m, 2H), 3.22 - 3.16 (m, 1H), 2.97 - 2.82 (m, 1H), 2.68 - 2.53 (m, 4H), 2.10 - 2.00 (m, 6H), 1.93 - 1.67 (m, 9H), 1.61 - 1.52 (m, 1H), 1.50 - 1.38 (m, 2H), 1.11 - 0.94 (m, 2H) |
| **I-345^{b}** | AFJ | AMB | 884.2 | 11.09 (s, 1H), 9.54 (s, 1H), 8.85 (d, *J =* 7.6 Hz, 1H), 8.38 (s, 1H), 8.30 (s, 1H), 7.58 (dd, *J* = 7.2, 8.4 Hz, 1H), 7.23 - 6.57 (m, 5H), 5.05 (dd, *J =* 5.2, 12.8 Hz, 1H), 4.77 - 4.58 (m, 2H), 4.34 (d, *J =* 10.8 Hz, 1H), 4.22 - 4.11 (m, 1H), 4.05 (d, *J* = 10.4 Hz, 1H), 3.86 (d, *J =* 11.2Hz, 1H), 3.76 (d, *J =* 10.8 Hz, 1H), 3.49 (t, *J* = 6.0 Hz, 2H), 3.28 - 3.22 (m, 3H), 2.94 - 2.79 (m, 2H), 2.66 - 2.53 (m, 4H), 2.09 - 1.91 (m, 8H), 1.89 - 1.66 (m, 8H), 1.56 - 1.39 (m, 3H), 1.10 - 0.94 (m, 2H) |
| **I-346^{b}** | ANX | AJB | 890.2 | 9.49 (d, *J =* 5.6 Hz, 1H), 8.78 (d, *J =* 7.6 Hz, 1H), 8.40 - 8.25 (m, 2H), 7.59-7.57 (m, 1H), 7.26 - 6.77 (m, 8H), 6.56 - 6.44 (m, 1H), 5.32 - 4.95 (m, 2H), 4.77 (br d, *J =* 1.2 Hz, 1H), 4.21 - 4.07 (m, 1H), 3.87 - 3.61 (m, 3H), 3.59 - 3.42 (m, 6H), 2.90 - 2.82 (m, 3H), 2.70 - 2.63 (m, 4H), 2.61 - 2.55 (m, 1H), 2.24 - 2.14 (m, 6H), 2.04 - 2.00 (m, 2H), 1.88 - 1.79 (m, 2H), 1.70 (br d, *J =* 2.4 Hz, 1H), 1.05 - 0.95 (m, 2H) |
| **I-347^{b}** | ALC | AJB | 913.5 | 11.09 (s, 1H), 9.49 (d, *J =* 6.0 Hz, 1H), 8.78 (d, *J =* 7.8 Hz, 1H), 8.38 (d, *J =* 4.0 Hz, 1H), 8.25 (d, *J =* 5.6 Hz, 1H), 8.20 - 8.15 (m, 1H), 7.57 (t, *J =* 7.8 Hz, 1H), 7.26 - 6.94 (m, 3H), 6.92 - 6.40 (m, 2H), 5.31 - 5.07 (m, 1H), 5.07 - 4.99 (m, 1H), 4.76 (d, *J =* 18.4 Hz, 1H), 4.23 - 4.12 (m, 1H), 3.86 - 3.78 (m, 3H), 3.67 - 3.59 (m, 3H), 3.55 - 3.47 (m, 3H), 3.46 (s, 1H), 3.29 (d, *J =* 7.2 Hz, 1H), 2.92 - 2.79 (m, 2H), 2.70 - 2.64 (m, 1H), 2.63 - 2.56 (m, 1H), 2.32 - 2.28 (m, 2H), 2.17 (s, 5H), 2.08 - 1.95 (m, 6H), 1.95 - 1.80 (m, 4H), 1.77 - 1.66 (m, 2H), 1.62 - 1.50 (m, 3H), 1.02 (q, *J =* 11.4 Hz, 2H) |
| **I-348^{b}** | AFJ | AOA | 878.3 | 11.11 (s, 1H), 10.90 - 10.58 (m, 1H), 8.69 (d, *J =* 7.6 Hz, 1H), 8.20 - 8.15 (m, 1H), 8.13 - 8.04 (m, 1H), 7.61 - 7.50 (m, 1H), 7.08 (d, *J =* 8.0 Hz, 1H), 7.01 (d, *J=* 6.0 Hz, 1H), 6.81 - 6.02 (m, 3H), 5.56 - 5.41 (m, 1H), 5.09 - 4.63 (m, 3H), 3.93 - 3.64 (m, 4H), 3.50 (d, *J =* 5.2 Hz, 4H), 2.95 - 2.62 (m, 6H), 2.08 - 1.70 (m, 20H), 1.20 - 1.06 (m, 2H) |
| **I-349^{b}** | AME | AJB | 913.6 | 11.08 (d, *J =* 3.2 Hz, 1H), 9.50 (d, *J =* 6.4 Hz, 1H), 8.78 (d, *J* = 7.6 Hz, 1H), 8.36 (t, *J =* 4.2 Hz, 1H), 8.26 (d, *J* = 5.6 Hz, 1H), 7.61 - 7.53 (m, 1H), 7.25 - 6.95 (m, 3H), 6.91 - 6.43 (m, 2H), 5.31 - 5.00 (m, 2H), 4.77 (d, *J =* 17.6 Hz, 1H), 4.17 - 4.02 (m, 1H), 3.84 - 3.72 (m, 3H), 3.67 - 3.56 (m, 3H), 3.47 - 3.37 (m, 3H), 2.94 - 2.82 (m, 2H), 2.74 - 2.68 (m, 1H), 2.62 - 2.52 (m, 2H), 2.42 - 2.33 (m, 4H), 2.23 - 2.08 (m, 5H), 2.05 - 1.91 (m, 6H), 1.90 - 1.79 (m, 2H), 1.78 - 1.64 (m, 5H), 1.59 - 1.38 (m, 1H), 1.06 - 0.86 (m, 2H) |
| **I-350^{b}** | ALG | ABC | 842.5 | 11.10 (s, 1H), 9.40 (s, 1H), 8.83 (d, *J* = 8.0 Hz, 1H), 8.38 (s, 1H), 8.29 (s, 1H), 7.63 - 7.55 (m, 1H), 7.24 - 6.95 (m, 3H), 6.91 (d, *J =* 8.0 Hz, 1H), 6.49 (d, *J =* 8.0 Hz, 1H), 5.12 - 5.05 (m, 1H), 4.23 - 4.13 (m, 1H), 3.92 - 3.85 (m, 1H), 3.83 - 3.76 (m, 4H), 3.75 - 3.70 (m, 4H), 2.93 - 2.83 (m, 1H), 2.63 - 2.56 (m, 1H), 2.43 - 2.43 (m, 1H), 2.44 - 2.35 (m, 1H), 2.26 - 2.22 (m, 2H), 2.20 (s, 3H), 2.07 - 2.00 (m, 3H), 2.08 - 1.99 (m, 2H), 1.85 - 1.72 (m, 4H), 1.69 - 1.57 (m, 4H), 1.55 - 1.42 (m, 3H), 1.10 - 0.94 (m, 2H) |
| **I-351^{b}** | AOP | AJB | 876.5 | 11.34 - 10.82 (m, 1H), 9.49 (d*, J =* 5.6 Hz, 1H), 8.77 (d, *J= 7.6* Hz, 1H), 8.38 (d, *J =* 4.0 Hz, 1H), 8.25 (t, *J =* 2.8 Hz, 1H), 7.59 - 7.57 (m, 1H), 7.35 - 7.22 (m, 4H), 7.18 - 6.95 (m, 3H), 6.88 - 6.43 (m, 2H), 5.29 - 5.00 (m, 2H), 4.76 (d, *J =* 16.8 Hz, 1H), 4.17 (t, *J =* 11.2 Hz, 1H), 3.81 - 3.75 (m, 4H), 3.62 (m, 6H), 2.95 - 2.81 (m, 3H), 2.62 - 2.53 (m, 1H), 2.45 - 2.43 (m, 2H), 2.09 - 1.87 (m, 7H), 1.80 - 1.66 (m, 2H), 1.54 (m, 1H), 1.17 - 1.01 (m, 2H) |
| **I-352^{b}** | ALA | AJB | 900.3 | 11.09 (s, 1H), 9.50 (d, *J =* 6.4 Hz, 1H), 8.78 (d, *J =* 7.6 Hz, 1H), 8.40 - 8.34 (m, 1H), 8.26 (d, *J =* 5.2 Hz, 1H), 7.62 - 7.52 (m, 1H), 7.25 - 6.94 (m, 3H), 6.89 - 6.41 (m, 2H), 5.30 - 5.02 (m, 2H), 4.76 (d, *J =* 18.0 Hz, 1H), 4.22 - 4.10 (m, 1H), 3.84 - 3.71 (m, 3H), 3.66 - 3.57 (m, 2H), 3.49 (t, *J =* 6.0 Hz, 3H), 3.46 - 3.37 (m, 4H), 2.95 - 2.83 (m, 1H), 2.71 (d, *J =* 10.8 Hz, 1H), 2.65 - 2.53 (m, 3H), 2.19 - 1.64 (m, 16H), 1.60 - 1.49 (m, 1H), 1.11 - 0.93 (m, 2H) |
| **I-353^{b}** | ALA | PW | 899.4 | 11.09 (s, 1H), 9.69 (s, 1H), 8.92 (s, 1H), 8.21 - 8.11 (m, 2H), 7.62 - 7.53 (m, 1H), 7.33 - 6.95 (m, 6H), 6.66 (d, *J* = 4.4 Hz, 1H), 5.05 (dd, *J =* 5.6, 12.8 Hz, 1H), 4.24 - 4.13 (m, 1H), 3.79 -3.71 (m, 1H), 3.64 - 3.55 (m, 1H), 3.53 - 3.46 (m, 3H), 3.43 -3.40 (m, 1H), 3.39 -3.36 (m, 3H), 3.18 (t, *J =* 6.0 Hz, 2H), 2.95 - 2.83 (m, 1H), 2.76 -2.68 (m, 1H), 2.65 - 2.53 (m, 3H), 2.14 - 1.99 (m, 5H), 1.98 - 1.84 (m, 3H), 1.83 - 1.66 (m, 5H), 1.63 - 1.49 (m, 1H), 1.12 - 0.95 (m, 3H), 0.48 - 0.41 (m, 2H), 0.25 - 0.19 (m, 2H) |
| **I-354** | ALX | AEH | 827.5 | (CDCl₃) 9.62 (s, 1H), 8.45 (s, 2H), 8.34 (d, *J =* 7.5 Hz, 1H), 8.31 (br s, 1H), 7.56 - 7.48 (m, 1H), 7.11 (d, *J =* 7.0 Hz, 1H), 6.91 (d, *J =* 8.5 Hz, 1H), 6.81 (t, *J =* 54.4 Hz, 1H), 6.33 - 6.23 (m, 1H), 6.14 (d, *J =* 7.8 Hz, 1H), 5.48 (s, 1H), 4.97 - 4.87 (m, 1H), 4.82 - 4.60 (m, 1H), 4.12 - 4.03 (m, 1H), 4.00 (s, 2H), 3.77 - 3.57 (m, 1H), 3.54 - 3.48 (m, 1H), 3.30 (q, *J =* 6.4 Hz, 2H), 2.95 - 2.74 (m, 3H), 2.22 - 2.09 (m, 4H), 2.05 - 1.98 (m, 1H), 1.97 - 1.65 (m, 8H), 1.50 - 1.37 (m, 4H), 1.36 - 1.30 (m, 3H), 1.27- 1.20 (m, 2H), 1.16 - 1.04 (m, 2H) |
| **I-355^{b}** | ALH | ABC | 842.5 | 11.08 (s, 1H), 9.39 (s, 1H), 8.81 (d, *J =* 8.0 Hz, 1H), 8.37 (s, 1H), 8.28 (s, 1H), 7.57 (dd, *J* =7.2, 8.4 Hz, 1H), 7.23 - 6.86 (m, 4H), 6.14 (d, *J =* 8.4 Hz, 1H), 5.10 - 4.95 (m, 1H), 4.24 - 4.11 (m, 1H), 3.81 - 3.75 (m, 4H), 3.74 - 3.68 (m, 4H), 2.91 - 2.82 (m, 1H), 2.62 - 2.55 (m, 2H), 2.46 - 2.35 (m, 2H), 2.27 - 2.23 (m, 2H), 2.20 (s, 3H), 2.10 - 1.99 (m, 5H), 1.95 - 1.84 (m, 2H), 1.80 - 1.68 (m, 4H), 1.55 - 1.38 (m, 3H), 1.35 - 1.19 (m, 2H), 1.07 - 0.92 (m, 2H) |
| **I-356^{b}** | ANO | ABC | 919.5 | 11.10 (s, 1H), 9.39 (s, 1H), 8.82 (d, *J* = 8.0 Hz, 1H), 8.37 (s, 1H), 8.28 (s, 1H), 7.51 (dd, *J* = 7.2*,* 8.4 Hz, 1H), 7.34 - 7.29 (m, 2H), 7.27 - 7.23 (m, 2H), 7.19 (t, *J =* 6.4 Hz, 1H), 7.11 - 6.94 (m, 3H), 6.90 (d, *J =* 8.0 Hz, 1H), 5.07 (dd, *J =* 5.2, 12.8 Hz, 1H), 4.54 (d, *J =* 6.0 Hz, 2H), 4.22 - 4.11 (m, 1H), 3.81 - 3.75 (m, 4H), 3.74 - 3.70 (m, 4H), 3.41 (s, 2H), 2.94 - 2.83 (m, 1H), 2.64 - 2.54 (m, 2H), 2.43 - 2.28 (m, 7H), 2.14 - 1.94 (m, 6H), 1.86 (d, *J =* 11.4 Hz, 2H), 1.77 - 1.64 (m, 2H), 1.61 - 1.47 (m, 1H), 1.09 - 0.94 (m, 2H) |
| **I-359^{b}** | AKY | AJB | 890.5 | 11.10 (s, 1H), 9.50 (d, *J =* 6.8 Hz, 1H), 8.76 (d, *J =* 7.6 Hz, 1H), 8.36 (d, *J =* 4.4 Hz, 1H), 8.25 (d, *J =* 5.4 Hz, 1H), 7.54 - 7.43 (m, 1H), 7.30 - 7.24 (m, 2H), 7.24 - 6.94 (m, 6H), 6.91 - 6.39 (m, 1H), 5.32 - 4.97 (m, 2H), 4.84 - 4.69 (m, 1H), 4.50 (d, *J =* 6.0 Hz, 2H), 4.21 - 4.07 (m, 1H), 3.85 - 3.54 (m, 5H), 2.91 - 2.81 (m, 1H), 2.73 - 2.52 (m, 5H), 2.20 (s, 3H), 2.16 (d, *J =* 6.8 Hz, 2H), 2.05 - 1.92 (m, 5H), 1.81 (d*, J =* 11.8 Hz, 2H), 1.75 - 1.63 (m, 2H), 1.57 - 1.43 (m, 1H), 1.04 - 0.91 (m, 2H) |
| **I-360^{b}** | ALK | AJB | 880.6 | 11.22 - 11.14 (m, 1H), 9.87 - 9.70 (m, 1H), 9.55 - 9.46 (m, 1H), 8.82 - 8.74 (m, 1H), 8.44 - 8.35 (m, 1H), 8.26 (d, *J* = 3.6 Hz, 1H), 8.12 (d, *J =* 6.0 Hz, 1H), 8.03 - 7.90 (m, 2H), 7.28 - 6.96 (m, 1H), 6.90 - 6.41 (m, 1H), 5.32 - 5.05 (m, 2H), 4.81 - 4.72 (m, 1H), 4.46 (s, 2H), 4.28 - 4.16 (m, 1H), 3.84 - 3.76 (m, 3H), 3.42 - 3.29 (m, 3H), 2.99 - 2.86 (m, 4H), 2.82 - 2.58 (m, 3H), 2.16 - 1.77 (m, 19H), 1.24 - 1.12 (m, 2H) |
| **I-361^{b}** | AKG | AJB | 904.6 | 11.10 (s, 1H), 9.49 (d, *J =* 5.6 Hz, 1H), 8.78 (d, *J =* 7.6 Hz, 1H), 8.38 (d, *J =* 4.4 Hz, 1H), 8.25 (d, *J =* 5.6 Hz, 1H), 7.49 (dd, *J =* 7.2, 8.4 Hz, 1H), 7.28 (d, *J =* 8.0 Hz, 2H), 7.25 - 7.07 (m, 4H), 7.01 (d, *J =* 7.2 Hz, 1H), 6.99 - 6.95 (m, 1H), 6.89 - 6.41 (m, 1H), 5.30 - 5.02 (m, 2H), 4.76 (d, *J =* 17.2 Hz, 1H), 4.52 (d, *J* = 6.0 Hz, 2H), 4.23 - 4.10 (m, 1H), 3.84 - 3.72 (m, 2H), 3.65 - 3.40 (m, 1H), 3.59 (s, 1H), 2.93 (s, 1H), 2.62 - 2.54 (m, 3H), 2.28 (t, *J =* 6.8 Hz, 2H), 2.13 (s, 3H), 2.10 (d, *J =* 7.2 Hz, 2H), 2.07 - 1.99 (m, 4H), 1.99 - 1.82 (m, 4H), 1.79 - 1.62 (m, 4H), 1.57 - 1.42 (m, 1H), 1.10 - 0.93 (m, 2H) |
| **I-362^{b}** | AKC | AJB | 930.2 | 11.09 (s, 1H), 9.50 (d, *J =* 6.0 Hz, 1H), 8.78 (d, *J =* 8.0 Hz, 1H), 8.39 (d, *J =* 4.4 Hz, 1H), 8.26 (d, *J =* 6.0 Hz, 1H), 7.58 (dd, *J =* 7.2, 8.4 Hz, 1H), 7.26 - 6.96 (m, 7H), 6.89 - 6.43 (m, 2H), 5.31 - 5.00 (m, 2H), 4.77 (d, *J =* 16.8 Hz, 1H), 4.26 - 4.12 (m, 1H), 3.84 - 3.72 (m, 2H), 3.66 - 3.56 (m, 2H), 3.55 - 3.49 (m, 2H), 3.46 (s, 1H), 3.00 - 2.92 (m, 2H), 2.87 - 2.82 (m, 2H), 2.64 - 2.55 (m, 1H), 2.16 (s, 2H), 2.11 - 1.86 (m, 10H), 1.83 - 1.54 (m, 8H), 1.15 - 0.98 (m, 2H) |
| **I-364^{b}** | AFJ | AJT | 884.5 | 11.10 (s, 1H), 9.69 (s, 1H), 8.75 (d, *J =* 7.6 Hz, 1H), 8.37 (s, 1H), 8.25 (s, 1H), 7.58 (dd, *J* =7.2, 8.4 Hz, 1H), 7.32 - 6.98 (m, 3H), 6.64 (t, *J =* 6.0 Hz, 1H), 6.37 (d, *J* = 7.6 Hz, 1H), 5.05 (dd, *J =* 5.6, 12.8 Hz, 1H), 4.74 (s, 4H), 4.38 (s, 4H), 4.24 - 4.10 (m, 1H), 3.50 - 3.48 (m, 2H), 3.40 - 3.39 (m, 2H), 3.27 - 3.24 (m, 2H), 2.94 - 2.84 (m, 1H), 2.66 - 2.59 (m, 2H), 2.59 - 2.53 (m, 1H), 2.12 - 1.94 (m, 7H), 1.92 - 1.66 (m, 8H), 1.62 - 1.38 (m, 3H), 1.10 - 0.94 (m, 2H) |
| **I-365^{b}** | AVB | ABC | 842.2 | 11.10 (s, 1H), 9.40 (s, 1H), 8.82 (d, *J* = 8.0 Hz, 1H), 8.39 (s, 1H), 8.28 (s, 1H), 7.59 (d, *J =* 7.6 Hz, 1H), 7.18 - 6.81 (m, 4H), 5.05 (d, *J =* 8.4 Hz, 1H), 4.28 - 4.17 (m, 1H), 3.79 (s, 4H), 3.72 (d, *J =* 4.8 Hz, 4H), 3.49 (d, *J =* 11.2 Hz, 3H), 3.35 (d, *J =* 6.4 Hz, 2H), 2.95 - 2.82 (m, 5H), 2.69 - 2.53 (m, 2H), 2.08 - 1.95 (m, 5H), 1.92 - 1.73 (m, 5H), 1.68 - 1.58 (m, 3H), 1.55 (s, 2H), 1.26 - 1.11 (m, 2H) |
| **I-366^{b}** | AJS | AJB | 925.5 | 11.09 (s, 1H), 9.50 (d, *J =* 6.4 Hz, 1H), 8.78 (d, *J =* 7.6 Hz, 1H), 8.38 (d, *J =* 4.4 Hz, 1H), 8.25 (d, *J =* 5.6 Hz, 1H), 7.58 (t, *J =* 7.6 Hz, 1H), 7.26 - 6.95 (m, 3H), 6.89 - 6.42 (m, 2H), 5.31 - 5.00 (m, 2H), 4.76 (d, *J =* 18.0 Hz, 1H), 4.43 - 4.03 (m, 2H), 3.87 - 3.77 (m, 2H), 3.73 (d, *J=* 7.6 Hz, 1H), 3.63 (d, *J =* 10.4 Hz, 1H), 3.59 (s, 1H), 2.95 - 2.82 (m, 3H), 2.80 - 2.68 (m, 3H), 2.67 - 2.59 (m, 1H), 2.59 - 2.53 (m, 1H), 2.45 - 2.34 (m, 2H), 2.19 - 2.12 (m, 1H), 2.11 - 1.46 (m, 19H), 1.46 - 1.37 (m, 1H), 1.11 - 0.94 (m, 2H) |
| **I-367^{b}** | AJA | AJB | 883.5 | 11.10 (s, 1H), 9.50 (d, *J =* 6.4 Hz, 1H), 8.78 (d, *J =* 8.0 Hz, 1H), 8.40 (d, *J =* 4.4 Hz, 1H), 8.25 (d, *J=* 5.6 Hz, 1H), 7.60 (t, *J =* 8.0 Hz, 1H), 7.28 - 6.36 (m, 6H), 5.30 - 5.02 (m, 2H), 4.76 (d, *J =* 18.4 Hz, 1H), 4.24 - 4.13 (m, 1H), 3.84 - 3.78 (m, 2H), 3.73 (d, *J =* 7.6 Hz, 1H), 3.66 - 3.57 (m, 3H), 3.03 (d, *J* = 9.2 Hz, 3H), 2.95 - 2.82 (m, 2H), 2.62 (d, *J =* 5.6 Hz, 3H), 2.57 (s, 1H), 2.55 (s, 1H), 2.17 - 1.69 (m, 16H), 1.66 - 1.48 (m, 3H), 1.13 - 1.01 (m, 2H) |
| **I-368^{b}** | AJM | ABC | 886.3 | 9.39 (s, 1H), 8.82 (d, *J =* 8.0 Hz, 1H), 8.37 (s, 1H), 8.28 (s, 1H), 7.59 (t, *J =* 8.0 Hz, 1H), 7.25 - 6.94 (m, 3H), 6.90 (d, *J =* 8.0 Hz, 1H), 6.65 (t, *J =* 5.6 Hz, 1H), 5.12 (dd, *J* = 5.2, 13.2 Hz, 1H), 4.21 - 4.12 (m, 1H), 3.79 (s, 4H), 3.72 (s, 4H), 3.50 - 3.46(m, 2H), 3.38 - 3.36 (m, 2H), 3.02 (s, 3H), 2.98 - 2.87 (m, 2H), 2.74 - 2.61 (m, 4H), 2.13 - 1.99 (m, 7H), 1.88 - 1.67 (m, 8H), 1.60 - 1.41 (m, 3H), 1.10 - 0.93 (m, 2H) |
| **I-371^{b}** | AFJ | AJB | 884.5 | 11.10 (s, 1H), 9.50 (d, *J =* 6.4 Hz, 1H), 8.79 (d, *J =* 7.6 Hz, 1H), 8.39 (d, *J =* 4.0 Hz, 1H), 8.26 (d, *J =* 5.6 Hz, 1H), 8.15 (s, 1H), 7.59 (dd, *J =* 7.2, 8.4 Hz, 1H), 7.27 - 7.08 (m, 2H), 7.05 - 6.97 (m, 1H), 6.90 - 6.42 (m, 2H), 5.31 - 5.02 (m, 2H), 4.77 (d, *J =* 16.8 Hz, 1H), 4.25 - 4.09 (m, 1H), 3.86 - 3.74 (m, 2H), 3.74 - 3.62 (m, 1H), 3.61 - 3.58 (m, 1H), 3.50 (t, *J =* 5.6 Hz, 2H), 3.45 (d, *J* = 9.6 Hz, 1H), 3.41 - 3.38 (m, 2H), 2.95 - 2.84 (m, 1H), 2.66 - 2.60 (m, 1H), 2.59 - 2.56 (m, 1H), 2.20 - 1.92 (m, 10H), 1.91 - 1.65 (m, 9H), 1.60 - 1.42 (m, 3H), 1.10 - 0.97 (m, 2H) |
| **I-372^{b}** | AJF | AFM | 890.6 | 11.09 (s, 1H), 8.87 (s, 1H), 8.28 (s, 1H), 8.01 (s, 1H), 7.89 (s, 1H), 7.76 (s, 1H), 7.73 (s, 1H), 7.60 - 7.54 (m, 1H), 7.10 (d, *J =* 8.8 Hz, 1H), 7.02 (d, *J =* 7.2 Hz, 1H), 6.46 (t, *J =* 5.6 Hz, 1H), 5.08 - 5.02 (m, 1H), 4.98 - 4.82 (m, 1H), 4.56 - 4.51 (m, 1H), 4.29 - 4.23 (m, 1H), 4.09 (s, 1H), 3.97 (s, 3H), 3.35 - 3.30 (m, 4H), 2.95 - 2.83 (m, 1H), 2.65 - 2.56 (m, 2H), 2.56 - 2.52 (m, 2H), 2.31 - 2.13 (m, 4H), 2.09 - 2.00 (m, 5H), 1.91 - 1.76 (m, 4H), 1.62 - 1.54 (m, 4H), 1.52 - 1.43 (m, 6H), 1.01 (t, *J =* 7.2 Hz, 3H), 0.98 - 0.85 (m, 2H) |
| **I-373^{b}** | AJF | AJB | 880.5 | 11.09 (s, 1H), 9.49 (d, *J =* 6.4 Hz, 1H), 8.77 (d, *J =* 7.6 Hz, 1H), 8.37 (d, *J =* 4.4 Hz, 1H), 8.24 (d, *J =* 5.6 Hz, 1H), 7.56 (dd, *J =* 7.2, 8.4 Hz, 1H), 7.25 - 6.94 (m, 3H), 6.89 - 6.38 (m, 2H), 5.31 - 4.95 (m, 2H), 4.82 - 4.70 (m, 1H), 4.21 - 4.09 (m, 1H), 3.85 - 3.74 (m, 2H), 3.61 - 3.56 (m, 2H), 3.35 - 3.30 (m, 4H), 2.90 - 2.81 (m, 1H), 2.63 - 2.52 (m, 2H), 2.30 - 2.15 (m, 3H), 2.11 - 1.82 (m, 11H), 1.78 - 1.64 (m, 2H), 1.61 - 1.40 (m, 7H), 1.08 - 0.93 (m, 2H) |
| **I-374^{b}** | AJE | AJB | 870.6 | 11.00 (s, 1H), 9.50 (d, *J =* 5.2 Hz, 1H), 8.78 (d, *J =* 6.8 Hz, 1H), 8.38 (s, 1H), 8.29 - 8.21 (m, 1H), 7.33 - 6.92 (m, 3H), 6.88 - 6.40 (m, 2H), 5.65 - 5.50 (m, 1H), 5.35 - 5.02 (m, 2H), 4.85 - 4.68 (m, 1H), 4.28 - 4.16 (m, 2H), 4.16 - 4.06 (m, 2H), 3.87 - 3.78 (m, 2H), 3.70 - 3.57 (m, 4H), 2.98 - 2.92 (m, 1H), 2.88 - 2.80 (m, 2H), 2.69 - 2.56 (m, 2H), 2.36 - 2.29 (m, 1H), 2.20 - 2.10 (m, 2H), 2.09 - 1.95 (m, 6H), 1.92 - 1.83 (m, 4H), 1.77 - 1.69 (m, 2H), 1.67 - 1.42 (m, 5H), 1.23 - 1.12 (m, 2H), 1.07 - 0.96 (m, 2H) |
| **I-376^{b}** | AJH | AGL | 887.5 | 12.36 (s, 1H), 11.10 (s, 1H), 8.71 (s, 1H), 8.48 - 8.43 (m, 1H), 8.40 - 8.33 (m, 2H), 8.16 (d, *J =* 8.4 Hz, 1H), 7.62 - 7.55 (m, 2H), 7.10 (d, *J =* 8.6 Hz, 1H), 7.03 (d, *J* = 7.2 Hz, 1H), 6.67 (t, *J =* 6.0 Hz, 1H), 5.94 (s, 1H), 5.08 - 5.03 (m, 1H), 4.49 - 4.38 (m, 1H), 3.48 (t, *J =* 5.2 Hz, 2H), 3.41 - 3.37 (m, 3H), 3.25 - 3.24 (m, 2H), 2.95 - 2.80 (m, 2H), 2.62 - 2.55 (m, 5H), 2.18 - 2.09 (m, 2H), 2.08 - 1.88 (m, 6H), 1.85 - 1.65 (m, 6H), 1.62 (s, 6H), 1.38 - 1.09 (m, 4H) |
| **I-377^{b}** | AIR | ABC | 906.3 | 10.97 (s, 1H), 9.39 (s, 1H), 8.83 (d, *J =* 8.0 Hz, 1H), 8.37 (s, 1H), 8.28 (s, 1H), 7.51 - 7.46 (m, 1H), 7.44 (d, *J =* 7.6 Hz, 2H), 7.35 - 7.29 (m, 4H), 7.23 - 6.95 (m, 1H), 6.91 (d, *J =* 8.4 Hz, 1H), 5.22 (s, 2H), 5.11 (dd, *J* = 4.8, 12.8 Hz, 1H), 4.45 - 4.38 (m, 1H), 4.30 - 4.22 (m, 1H), 4.21 - 4.12 (m, 1H), 3.84 - 3.68 (m, 10H), 3.46 (s, 4H), 2.13 - 1.94 (m, 8H), 1.93 - 1.82 (m, 3H), 1.79 - 1.66 (m, 3H), 1.62 - 1.46 (m, 2H), 1.11 - 0.92 (m, 3H) |
| **I-379^{b}** | AIR | ACA | 917.3 | 10.13 (s, 1H), 9.70 (s, 1H), 8.92 (s, 1H), 8.19 - 8.12 (m, 2H), 7.37 - 7.14 (m, 7H), 7.09 (s, 2H), 7.04 - 6.99 (m, 2H), 5.34 - 5.21 (m, 1H), 4.88 - 4.76 (m, 2H), 4.40 - 4.31 (m, 1H), 4.23 - 4.11 (m, 2H), 3.57 - 3.47 (m, 1H), 3.39 - 3.33 (m, 8H), 3.20 - 3.15 (m, 2H), 3.13 - 3.06 (m, 1H), 2.87 - 2.72 (m, 1H), 2.28 - 2.16 (m, 2H), 2.11 - 1.97 (m, 4H), 1.93 - 1.84 (m, 2H), 1.81 - 1.68 (m, 2H), 1.65 - 1.49 (m, 2H), 1.14 - 0.91 (m, 3H), 0.50 - 0.40 (m, 2H), 0.26 - 0.18 (m, 2H) |
| I-380^{b} | AJC | AJB | 826.5 | 11.01 (s, 1H), 9.50 (d, *J =* 6.4 Hz, 1H), 8.78 (d, J= 7.6 Hz, 1H), 8.38 (d, *J =* 4.4 Hz, 1H), 8.25 (d, *J =* 5.6 Hz, 1H), 7.27 (t, *J =* 8.0 Hz, 1H), 7.25 - 6.95 (m, 1H), 6.91 (d, *J* = 7.2 Hz, 1H), 6.88 - 6.43 (m, 2H), 5.63 (t, *J =* 5.6 Hz, 1H), 5.31 - 5.05 (m, 2H), 4.84 - 4.70 (m, 1H), 4.28 - 4.08 (m, 3H), 3.83 - 3.72 (m, 2H), 3.02 (t, *J =* 6.0 Hz, 2H), 2.97 - 2.92 (m, 1H), 2.90 - 2.84 (m, 2H), 2.65 - 2.58 (m, 2H), 2.31 - 2.24 (m, 1H), 2.13 (d, *J* = 7.2 Hz, 2H), 2.07 - 1.68 (m, 14H), 1.63 - 1.53 (m, 2H), 1.27 - 1.14 (m, 2H), 1.09 - 0.97 (m, 2H) |
| **I-381^{b}** | AML | AJB | 866.4 | 11.10 (s, 1H), 9.50 (d, *J =* 6.4 Hz, 1H), 8.78 (d, *J =* 8.0 Hz, 1H), 8.38 (d, *J =* 4.4 Hz, 1H), 8.25 (d, *J =* 5.6 Hz, 1H), 7.58 (d, *J =* 7.8 Hz, 1H), 7.26 - 6.40 (m, 5H), 5.30 - 5.01 (m, 2H), 4.77 (d, *J =* 16.8 Hz, 1H), 4.23 - 4.07 (m, 2H), 3.85 - 3.71 (m, 2H), 3.66 - 3.56 (m, 2H), 2.94 - 2.82 (m, 1H), 2.69 - 2.52 (m, 2H), 2.39 - 2.27 (m, 4H), 2.21 (s, 2H), 2.11 - 1.93 (m, 7H), 1.93 - 1.83 (m, 2H), 1.79 - 1.60 (m, 6H), 1.55 (d, *J* = 5.2 Hz, 3H), 1.10 - 0.95 (m, 2H) |
| **I-384^{b}** | AJV | ABC | 871.6 | 9.40 (s, 1H), 8.83 (d, *J =* 8.0 Hz, 1H), 8.39 (s, 1H), 8.34 - 8.27 (m, 2H), 7.62 - 7.55 (m, 1H), 7.25 - 6.95 (m, 3H), 6.91 (d, *J =* 8.0 Hz, 1H), 6.25 (d, *J =* 8.0 Hz, 1H), 5.08 - 5.01 (m, 1H), 4.24 - 4.16 (m, 1H), 3.82 - 3.78 (m, 4H), 3.74 - 3.71 (m, 4H), 2.92 - 2.84 (m, 1H), 2.83 - 2.75 (m, 4H), 2.69 - 2.63 (m, 2H), 2.61 - 2.56 (m, 3H), 2.38 (t, *J* = 6.4 Hz, 2H), 2.21 - 2.12 (m, 2H), 2.10 - 2.00 (m, 3H), 1.94 (t, *J* = 10.4 Hz, 4H), 1.80 - 1.68 (m, 4H), 1.66 - 1.59 (m, 1H), 1.54 - 1.45 (m, 2H), 1.22 - 1.07 (m, 2H) |
| **I-385^{b}** | AJI | ABC | 828.5 | 11.09 (s, 1H), 9.39 (s, 1H), 8.82 (d, *J* = 8.0 Hz, 1H), 8.37 (s, 1H), 8.29 (s, 1H), 7.57 (t, *J =* 8.0 Hz, 1H), 7.24 - 6.94 (m, 3H), 6.90 (d, *J =* 8.0 Hz, 1H), 6.63 - 6.56 (m, 1H), 5.11 - 5.00 (m, 1H), 4.21 - 4.12 (m, 1H), 3.82 - 3.77 (m, 4H), 3.75 - 3.70 (m, 4H), 3.25 - 3.20 (m, 2H), 2.94 - 2.83 (m, 3H), 2.64 - 2.55 (m, 2H), 2.16 - 2.09 (m, 2H), 2.08 - 2.00 (m, 3H), 1.93 - 1.82 (m, 4H), 1.79 - 1.65 (m, 4H), 1.63 - 1.52 (m, 2H), 1.33 - 1.19 (m, 2H), 1.08 - 0.94 (m, 2H) |
| **I-386^{b}** | AGU | AFM | 880.1 | 11.09 (s, 1H), 8.86 (s, 1H), 8.28 (s, 1H), 8.16 (s, 1H), 8.02 (s, 1H), 7.89 (s, 1H), 7.76 (s, 1H), 7.73 (s, 1H), 7.59 (t, *J =* 8.0 Hz, 1H), 7.15 (d, *J =* 8.0 Hz, 1H), 7.04 (d, *J* = 7.2 Hz, 1H), 6.62 (t, *J =* 5.2 Hz, 1H), 5.07 (dd, *J =* 5.2, 12.8 Hz, 1H), 4.99 - 4.81 (m, 1H), 4.54 (dd, *J* = 3.2, 11.2 Hz, 1H), 4.28 - 4.24 (m, 1H), 4.13 - 4.06 (m, 1H), 3.98 (s, 3H), 3.65 - 3.59 (m, 2H), 3.46 - 3.44 (m, 2H), 2.92 - 2.84 (m, 1H), 2.65 - 2.63 (m, 1H), 2.61 - 2.57 (m, 4H), 2.10 - 2.04 (m, 8H), 1.81 - 1.78 (m, 4H), 1.62 - 1.41 (m, 8H), 1.01 (t, *J =* 7.2 Hz, 3H), 0.98 - 0.88 (m, 2H) |
| **I-387^{b}** | ADR | AFM | 868.2 | 11.10 (s, 1H), 8.87 (s, 1H), 8.29 (s, 1H), 8.20 (s, 1H), 8.01 (s, 1H), 7.90 (s, 1H), 7.77 (s, 1H), 7.75 (s, 1H), 7.62 - 7.55 (m, 1H), 7.16 (d, *J =* 8.4 Hz, 1H), 7.04 (d, *J* = 7.2 Hz, 1H), 6.62 (t, *J =* 5.6 Hz, 1H), 5.06 (dd, *J =* 5.2, 12.8 Hz, 1H), 5.00 - 4.81 (m, 1H), 4.55 (dd, *J =* 3.2, 11.2 Hz, 1H), 4.27 (dd, *J =* 6.4, 11.2 Hz, 1H), 4.12 - 4.08 (m, 1H), 3.98 (s, 3H), 3.60 - 3.57 (m, 2H), 3.49 - 3.46 (m, 6H), 2.92 - 2.85 (m, 1H), 2.62 - 2.59 (m, 1H), 2.58 - 2.56 (m, 1H), 2.34 - 2.30 (m, 2H), 2.10 (s, 3H), 2.07 - 1.98 (m, 5H), 1.83 - 1.75 (m, 2H), 1.68 - 1.56 (m, 4H), 1.50 - 1.37 (m, 3H), 1.02 (t, *J =* 7.2 Hz, 3H), 0.93 - 0.82 (m, 2H) |
| **I-389** | WX | GF | 848.2 | 11.10 (s, 1H), 9.98 (s, 1H), 8.96 (s, 1H), 8.78 (s, 1H), 8.15 (s, 1H), 7.79 (d, *J =* 8.8 Hz, 2H), 7.45 (d, *J =* 8.8 Hz, 2H), 7.43 - 7.14 (m, 1H), 7.12 - 7.05 (m, 3H), 7.05 - 7.02 (m, 1H), 6.96 (t, *J =* 7.6 Hz, 1H), 6.87 (d, *J* = 7.2 Hz, 1H), 5.38 (dd, *J =* 5.6, 12.4 Hz, 1H), 3.68 (s, 3H), 3.62 (s, 2H), 3.59 (s, 2H), 3.21 - 3.15 (m, 4H), 2.95 - 2.84 (m, 3H), 2.77 - 2.68 (m, 1H), 2.11 (s, 3H), 2.06 - 1.92 (m, 3H), 1.74 (d, *J =* 12.0 Hz, 2H), 1.56 - 1.42 (m, 2H), 1.13 - 1.01 (m, 1H), 0.54 - 0.39 (m, 2H), 0.28 - 0.14 (m, 2H) |
| **I-390^{g}** | PF | PW | 1164.5 | 9.75 (s, 1H), 8.95 (s, 1H), 8.25 - 8.18 (m, 2H), 8.15 (d, *J =* 5.2 Hz, 1H), 7.33 - 6.99 (m, 11H), 6.92 (d, *J* = 2.4 Hz, 1H), 6.84 - 6.77 (m, 2H), 5.01 (d, *J =* 9.6 Hz, 1H), 4.91 - 4.83 (m, 2H), 4.73 - 4.65 (m, 2H), 4.29 - 4.19 (m, 2H), 4.07 - 4.05 (m, 3H), 3.80 - 3.73 (m, 4H), 3.70-3.68 (m, 4H), 3.60 (s, 9H), 3.20 (d, *J =* 5.2 Hz, 2H), 3.16 - 3.12 (m, 2H), 2.86 (d, *J =* 4.4 Hz, 3H), 1.99 - 1.89 (m, 3H), 1.80 - 1.72 (m, 4H), 1.29 (d, *J =* 6.8 Hz, 1H), 1.25 (d, *J =* 6.8 Hz, 3H), 1.20 - 1.15 (m, 3H), 1.09 (s, 9H), 0.98 (s, 3H), 0.51 - 0.45 (m, 2H), 0.25 - 0.23 (m, 2H) |
| **I-392** | AMC | GF | 865.3 | 11.08 (s, 1H), 9.95 (s, 1H), 8.96 (s, 1H), 8.77 (s, 1H), 8.16 (d, *J =* 5.2 Hz, 1H), 7.78 (d*, J =* 8.4 Hz, 2H), 7.58 - 7.50 (m, 1H), 7.43 (d, *J =* 8.4 Hz, 2H), 7.31 - 7.01 (m, 5H), 6.99 (d, *J =* 7.2 Hz, 1H), 6.63 (t, *J =* 6.0 Hz, 1H), 5.03 (dd, *J =* 5.2, 12.8 Hz, 1H), 3.49 (s, 2H), 3.45 - 3.41 (m, 4H), 3.36 - 3.32 (m, 2H), 3.18 (t, *J =* 6.0 Hz, 2H), 2.93 - 2.80 (m, 1H), 2.61 - 2.52 (m, 2H), 2.40 (t, *J =* 6.8 Hz, 2H), 2.13 (s, 3H), 2.06 - 1.96 (m, 1H), 1.84 - 1.66 (m, 4H), 1.15 - 0.98 (m, 1H), 0.51 - 0.36 (m, 2H), 0.28 - 0.14 (m, 2H) |
| **I-393** | ALA | GF | 893.4 | 11.09 (s, 1H), 9.98 (s, 1H), 8.97 (s, 1H), 8.78 (s, 1H), 8.16 (d, *J =* 5.2 Hz, 1H), 7.80 (d*, J =* 8.8 Hz, 2H), 7.59 - 7.50 (m, 1H), 7.45 (d, *J =* 8.4 Hz, 2H), 7.43 - 7.14 (m, 1H), 7.13 - 6.98 (m, 5H), 6.67 - 6.55 (m, 1H), 5.04 (dd, *J =* 5.2, 12.8 Hz, 1H), 3.81 - 3.73 (m, 1H), 3.67 - 3.59 (m, 1H), 3.51 (s, 3H), 3.48 - 3.45 (m, 2H), 3.42 - 3.39 (m, 1H), 3.37 - 3.34 (m, 3H), 3.18 (t, *J =* 6.0 Hz, 2H), 2.93 - 2.81 (m, 1H), 2.76 -2.70 (m, 1H), 2.65 - 2.53 (m, 3H), 2.13 - 1.97 (m, 2H), 1.88 (t, *J =* 10.8 Hz, 1H), 1.83 - 1.71 (m, 2H), 1.12 - 1.00 (m, 1H), 0.50 - 0.41 (m, 2H), 0.27 - 0.17 (m, 2H) |
| **I-395** | WX | AMK | 868.4 | 11.13 (s, 1H), 11.10 - 10.96 (m, 1H), 10.74 (br s, 1H), 9.73 - 8.90 (m, 1H), 8.20 (br s, 1H), 8.11 (s, 1H), 8.01 (d, *J* = 5.3 Hz, 1H), 7.54 (br s, 1H), 7.38 (d, *J =* 7.8 Hz, 1H), 7.31 - 7.21 (m, 1H), 7.18 - 7.09 (m, 1H), 7.08 - 6.72 (m, 2H), 5.46 (dd, *J =* 5.4, 12.5 Hz, 1H), 4.87 - 4.49 (m, 2H), 4.25 - 4.15 (m, 1H), 3.67 (s, 3H), 3.62 - 3.45 (m, 3H), 3.34 (d, *J =* 6.7 Hz, 2H), 3.25 (br s, 4H), 3.11 - 2.99 (m, 1H), 2.98 - 2.80 (m, 2H), 2.78 - 2.59 (m, 5H), 2.37 - 2.23 (m, 4H), 2.21 - 2.09 (m, 1H), 2.08 - 1.91 (m, 4H), 1.90 - 1.63 (m, 3H), 1.29 - 1.04 (m, 3H), 0.61 - 0.47 (m, 2H), 0.32 (q, *J =* 4.6 Hz, 2H) |
| **I-397^{b}** | AMC | APD | 887.5 | 9.85 - 9.79 (m, 1H), 8.78 - 8.75 (m, 1H), 8.28 (d, *J* = 5.2 Hz, 1H), 8.02 (s, 1H), 7.58 - 7.50 (m, 1H), 7.31 - 7.27 (m, 1H), 7.24 - 7.20 (m, 1H), 7.19 - 6.90 (m, 3H), 6.60 (t, *J =* 5.6 Hz, 1H), 5.00 - 4.93 (m, 1H), 4.12 (s, 1H), 3.70 - 3.68 (m, 4H), 3.51 - 3.47 (m, 4H), 3.44 (t, *J* = 5.8 Hz, 2H), 3.38 (t, *J =* 6.0 Hz, 2H), 3.35 - 3.29 (m, 2H), 2.86 - 2.76 (m, 1H), 2.63 - 2.51 (m, 2H), 2.48 - 2.42 (m, 2H), 2.22 (d, *J =* 7.2 Hz, 2H), 2.20 (s, 3H), 2.04 - 1.92 (m, 3H), 1.85 - 1.75 (m, 4H), 1.74 - 1.58 (m, 4H), 1.57 - 1.43 (m, 1H), 1.06 - 0.90 (m, 2H) |
| **I-400** | WX | PW | 854.3 | 11.10 (s, 1H), 9.69 (s, 1H), 8.92 (s, 1H), 8.18 (s, 1H), 7.31 - 6.82 (m, 7H), 5.40 - 5.36 (m, 1H), 4.27 - 4.11 (m, 1H), 3.67 (s, 3H), 3.62 (s, 2H), 3.23 - 3.11 (m, 3H), 2.97 - 2.81 (m, 3H), 2.78 - 2.69 (m, 1H), 2.68 - 2.59 (m, 2H), 2.36 - 2.30 (m, 2H), 2.27 (s, 3H), 2.08 - 2.00 (m, 3H), 2.00 - 1.94 (m, 2H), 1.93 - 1.84 (m, 2H), 1.82 - 1.63 (m, 4H), 1.60 - 1.50 (m, 1H), 1.48 - 1.34 (m, 2H), 1.11 - 0.95 (m, 3H), 0.50 - 0.40 (m, 2H), 0.25 - 0.16 (m, 2H) |
| **I-401** | AMC | AMK | 885.5 | 11.08 (s, 1H), 8.15 - 7.91 (m, 3H), 7.61 - 7.52 (m, 1H), 7.09 - 6.73 (m, 6H), 6.66 (t, *J =* 5.6 Hz, 1H), 5.04 (dd, *J =* 5.2, 12.8 Hz, 1H), 4.13 - 4.04 (m, 1H), 3.48 - 3.41 (m, 6H), 3.23 (s, 3H), 3.13 (t, *J =* 6.0 Hz, 2H), 2.92 - 2.83 (m, 1H), 2.62 - 2.52 (m, 2H), 2.30 (t, *J =* 7.2 2H), 2.10 (s, 3H), 2.07 (d, *J =* 6.8 Hz, 2H), 2.03 - 1.95 (m, 3H), 1.86 - 1.77 (m, 4H), 1.71 - 1.60 (m, 4H), 1.50 - 1.41 (m, 1H), 1.07 - 0.91 (m, 3H), 0.46 - 0.39 (m, 2H), 0.22 - 0.17 (m, 2H) |
| **I-404^{b}** | AKA | ABC | 857.6 | 11.09 (s, 1H), 9.39 (s, 1H), 8.82 (d, *J* = 8.0 Hz, 1H), 8.38 (s, 1H), 8.29 (s, 1H), 8.19 (s, 1H), 7.24 - 6.93 (m, 3H), 6.92 - 6.88 (m, 1H), 5.42 - 5.32 (m, 1H), 4.23 - 4.12 (m, 1H), 3.83 - 3.76 (m, 4H), 3.75 - 3.70 (m, 4H), 3.57 (s, 3H), 3.11 - 2.97 (m, 4H), 2.93 - 2.84 (m, 1H), 2.76 - 2.68 (m, 1H), 2.66 - 2.61 (m, 1H), 2.60 - 2.54 (m, 3H), 2.31 - 2.26 (m, 1H), 2.26 - 2.15 (m, 5H), 2.06 - 1.95 (m, 4H), 1.90 (d, *J =* 11.2 Hz, 2H), 1.80 - 1.61 (m, 4H), 1.55 - 1.37 (m, 3H), 1.08 - 0.93 (m, 2H) |
| **I-410^{b}** | AKN | AJB | 909.1 | 11.09 (s, 1H), 9.49 (d, *J =* 6.0 Hz, 1H), 8.78 (d, *J =* 7.6 Hz, 1H), 8.37 (d, *J =* 4.0 Hz, 1H), 8.25 (d, *J =* 5.6 Hz, 1H), 7.24 - 6.94 (m, 3H), 6.91 - 6.43 (m, 2H), 5.37 (dd, *J =* 5.2, 12.8 Hz, 1H), 5.30 - 5.04 (m, 1H), 4.76 (d, *J =* 17.6 Hz, 1H), 4.22 - 4.10 (m, 1H), 3.89 - 3.69 (m, 3H), 3.65 - 3.63 (m, 1H), 3.58 (s, 2H), 3.57 (s, 3H), 3.45 (s, 2H), 3.12 (t, *J =* 7.6 Hz, 2H), 2.93 - 2.85 (m, 1H), 2.76-2.65 (m, 2H), 2.41 (t, *J =* 7.6 Hz, 2H), 2.27 - 2.12 (m, 3H), 2.09 - 1.91 (m, 8H), 1.86 (d, *J =* 12.0 Hz, 2H), 1.77 - 1.68 (m, 2H), 1.61 - 1.50 (m, 5H), 1.07 - 0.94 (m, 2H) |
| **I-411^{b}** | YK | AJB | 871.6 | 11.07 (s, 1H), 9.60 - 9.41 (m, 1H), 8.78 (d, *J =* 7.6 Hz, 1H), 8.40 (dd,*J* = 3.6, 14.8 Hz, 1H), 8.27 (t, *J =* 4.8 Hz, 1H), 7.29 - 7.10 (m, 1H), 7.08 (d, *J =* 7.8 Hz, 1H), 7.02 - 6.87 (m, 2H), 6.87 - 6.41 (m, 1H), 5.42 - 5.33 (m, 1H), 5.32 - 5.03 (m, 1H), 4.77 (d, *J =* 15.8 Hz, 1H), 4.19 - 4.02 (m, 1H), 3.84 - 3.78 (m, 2H), 3.78 - 3.73 (m, 2H), 3.69 (s, 3H), 3.67 - 3.55 (m, 3H), 3.54 - 3.50 (m, 1H), 3.45 - 3.40 (m, 2H), 2.94 - 2.82 (m, 2H), 2.75 - 2.65 (m, 2H), 2.65-2.57 (m, 1H), 2.36 - 2.27 (m, 2H), 2.13 (s, 3H), 2.08 - 1.91 (m, 7H), 1.82 - 1.62 (m, 5H), 1.48 - 1.32 (m, 1H), 1.02 - 0.88 (m, 1H), 0.86 - 0.69 (m, 1H) |
| **I-413^{b}** | ADC | AJB | 858.3 | 11.09 (s, 1H), 9.51 (d, *J =* 6.0 Hz, 1H), 8.80 (d, *J =* 7.6 Hz, 1H), 8.39 (d, *J =* 4.0 Hz, 1H), 8.27 (d, *J =* 5.2 Hz, 1H), 8.16 (s, 1H), 7.26 - 6.96 (m, 2H), 6.91 - 6.44 (m, 2H), 5.41 - 5.06 (m, 1H), 4.78 (br d, *J =* 18.4 Hz, 1H), 4.24 - 4.13 (m, 1H), 3.85 - 3.62 (m, 5H), 3.58 (s, 3H), 3.46 (s, 2H), 3.02 - 2.91 (m, 4H), 2.63 - 2.56 (m, 2H), 2.25 (m, 4H), 2.09 - 1.97 (m, 6H), 1.95 - 1.83 (m, 6H), 1.72 (dd, *J =* 5.6, 12.8 Hz, 4H), 1.63 - 1.54 (m, 2H), 1.10 - 1.01 (m, 2H) |
| **I-414^{b}** | ALM | AJB | 909.3 | 11.09 (s, 1H), 9.49 (d, *J =* 6.0 Hz, 1H), 8.77 (d, *J =* 7.6 Hz, 1H), 8.37 (d, *J =* 4.0 Hz, 1H), 8.25 (d, *J =* 5.6 Hz, 1H), 7.25 - 6.89 (m, 4H), 6.88 - 6.43 (m, 1H), 5.36 (dd, *J =* 5.2, 12.4 Hz, 1H), 5.29 - 5.05 (m, 1H), 4.76 (d, *J =* 17.6 Hz, 1H), 4.19 - 4.12 (m, 1H), 3.81 - 3.74 (m, 2H), 3.59 (s, 2H), 3.57 (s, 3H), 3.40 - 3.37 (m, 2H), 3.33 - 3.28 (m, 2H), 3.13 (t, *J =* 7.6 Hz, 2H), 3.05 - 2.96 (m, 4H), 2.93 - 2.84 (m, 1H), 2.72 - 2.59 (m, 4H), 2.34 (d, *J =* 6.4 Hz, 2H), 2.06 - 1.91 (m, 5H), 1.84 (d, *J =* 11.2 Hz, 2H), 1.75 - 1.63 (m, 2H), 1.59 - 1.50 (m, 4H), 1.42 - 1.28 (m, 1H), 1.13 - 0.94 (m, 2H) |
| **I-415^{b}** | AMS | AJB | 886.2 | 11.09 (s, 1H), 9.50 (d, *J =* 6.0 Hz, 1H), 8.78 (d, *J =* 7.6 Hz, 1H), 8.38 (d, *J =* 4.8 Hz, 1H), 8.26 (d, *J =* 5.6 Hz, 1H), 7.27 - 6.97 (m, 1H), 6.97 - 6.90 (m, 2H), 6.89 - 6.42 (m, 2H), 5.36 (dd, *J =* 5.2, 12.8 Hz, 1H), 5.30 - 5.05 (m, 1H), 4.83 - 4.72 (m, 1H), 4.17 - 4.15 (m, 1H), 3.84 - 3.58 (m, 4H), 3.54 (s, 3H), 3.46 - 3.42 (m, 1H), 3.05 - 3.01 (m, 2H), 2.95 - 2.84 (m, 1H), 2.76 - 2.67 (m, 1H), 2.64 - 2.54 (m, 1H), 2.32 - 2.18 (m, 3H), 2.16 - 2.10 (m, 2H), 2.09 - 1.80 (m, 10H), 1.78 - 1.68 (m, 2H), 1.60 - 1.44 (m, 7H), 1.10 - 0.94 (m, 2H) |
| **I-416^{b}** | AKP | AJB | 854.5 | 11.09 (s, 1H), 9.49 (d, *J =* 6.0 Hz, 1H), 8.77 (d, *J =* 7.8 Hz, 1H), 8.38 (d, *J =* 4.0 Hz, 1H), 8.25 (d, *J =* 5.6 Hz, 1H), 7.27 - 6.94 (m, 3H), 6.90 - 6.42 (m, 2H), 5.44 - 5.32 (m, 1H), 5.30 - 5.02 (m, 1H), 4.85 - 4.70 (m, 1H), 4.21 - 4.11 (m, 1H), 3.85 - 3.78 (m, 2H), 3.65 - 3.60 (m, 2H), 3.54 (s, 3H), 2.91 - 2.81 (m, 5H), 2.76 - 2.69 (m, 1H), 2.65 - 2.57 (m, 1H), 2.18 - 2.09 (m, 2H), 2.08 - 1.95 (m, 5H), 1.93 - 1.85 (m, 4H), 1.79 - 1.68 (m, 2H), 1.67 - 1.55 (m, 5H), 1.37 - 1.29 (m, 2H), 1.28 - 1.20 (m, 1H), 1.19 - 1.10 (m, 2H), 1.09 - 0.96 (m, 2H) |
| **I-417^{b}** | APT | AJB | 866.5 | 11.12 (s, 1H), 9.50 (d, *J =* 6.4 Hz, 1H), 8.78 (d, *J =* 7.6 Hz, 1H), 8.39 (d, *J =* 4.4 Hz, 1H), 8.25 (d, *J =* 5.6 Hz, 1H), 7.26 - 6.96 (m, 4H), 6.88 - 6.43 (m, 1H), 5.40 (dd, *J =* 5.2, 12.8 Hz, 1H), 5.31 - 5.04 (m, 1H), 4.79 - 4.77 (m, 1H), 4.52 (s, 2H), 4.27 - 4.14 (m, 1H), 3.84 - 3.71 (m, 3H), 3.65 (s, 3H), 3.62 - 3.58 (m, 1H), 3.46 - 3.43 (m, 1H), 3.13 - 2.97 (m, 2H), 2.97 - 2.79 (m, 2H), 2.78 - 2.68 (m, 2H), 2.68 - 2.53 (m, 3H), 2.09 - 1.86 (m, 9H), 1.84 - 1.60 (m, 5H), 1.16 - 1.10 (m, 2H) |
| **I-419^{b}** | PP | AKV | 844.6 | 11.08 (s, 1H), 9.39 (s, 1H), 8.72 (d, *J* = 8.0 Hz, 1H), 8.38 (s, 1H), 8.24 (s, 1H), 7.22 - 6.97 (m, 1H), 6.97 - 6.85 (m, 4H), 5.36 (dd, *J =* 5.6, 12.4 Hz, 1H), 4.22 - 4.12 (m, 1H), 3.79 (s, 4H), 3.57 (s, 3H), 3.44 - 3.40 (m, 4H), 3.00 - 2.93 (m, 2H), 2.90 - 2.83 (m, 1H), 2.76 - 2.68 (m, 1H), 2.65 - 2.57 (m, 1H), 2.41 (t, *J =* 7.2 Hz, 2H), 2.19 (s, 5H), 2.06 - 1.95 (m, 3H), 1.93 - 1.86 (m, 2H), 1.86 - 1.80 (m, 2H), 1.80 - 1.71 (m, 2H), 1.70 - 1.64 (m, 4H), 1.63 - 1.51 (m, 5H), 1.11 - 0.96 (m, 2H) |
| **I-421^{b}** | ALO | AJB | 895.5 | 11.11 (s, 1H), 9.52 (d, *J =* 6.8 Hz, 1H), 8.80 (d, *J* = 7.6 Hz, 1H), 8.41 (d, *J =* 3.6 Hz, 1H), 8.27 (d, *J =* 5.6 Hz, 1H), 7.29 - 6.94 (m, 3H), 6.91 - 6.43 (m, 2H), 5.40 - 5.38 (m, 1H), 5.31 - 5.06 (m, 1H), 4.83 - 4.72 (m, 1H), 4.32 - 4.19 (m, 1H), 4.09 (s, 1H), 4.04 (s, 1H), 3.84 - 3.78 (m, 3H), 3.77 - 3.70 (m, 2H), 3.69 - 3.58 (m, 3H), 3.53 (s, 3H), 3.06 - 2.84 (m, 5H), 2.79 - 2.67 (m, 2H), 2.15 - 1.75 (m, 14H), 1.26 - 1.16 (m, 2H) |
| **I-425^{b}** | ALL | AJB | 868.5 | 11.09 (s, 1H), 9.50 (d, *J =* 6.4 Hz, 1H), 8.76 (d, *J =* 7.8 Hz, 1H), 8.37 (d, *J =* 4.4 Hz, 1H), 8.25 (d, *J =* 5.2 Hz, 1H), 7.26 - 6.85 (m, 4H), 6.85 - 6.37 (m, 1H), 5.35 (dd, *J =* 5.4, 12.4 Hz, 1H), 5.30 - 5.00 (m, 1H), 4.76 (d, *J =* 16.8 Hz, 1H), 4.23 - 4.10 (m, 1H), 3.83 - 3.72 (m, 2H), 3.65 - 3.59 (m, 2H), 3.54 (s, 3H), 2.93 - 2.83 (m, 5H), 2.69 - 2.58 (m, 2H), 2.17 (d, *J* = 6.4 Hz, 2H), 2.09 - 1.83 (m, 10H), 1.80 - 1.68 (m, 2H), 1.66 - 1.53 (m, 5H), 1.45 - 1.32 (m, 2H), 1.30 - 1.20 (m, 2H), 1.19 - 1.12 (m, 2H), 1.09 - 0.96 (m, 2H) |
| **I-428^{b}** | AKP | AMT | 848.5 | 11.07 (s, 1H), 9.60 (d, *J =* 4.0 Hz, 1H), 8.95 (d, *J* = 3.2 Hz, 1H), 8.79 (d, *J =* 6.8 Hz, 1H), 8.29 (d, *J =* 5.2 Hz, 1H), 7.77 (d, *J =* 8.0 Hz, 2H), 7.42 (d, *J =* 8.4 Hz, 2H), 7.35 - 6.42 (m, 5H), 5.39 - 5.31 (m, 1H), 5.30 - 5.04 (m, 1H), 4.85 - 4.70 (m, 1H), 3.85 - 3.71 (m, 2H), 3.68 - 3.58 (m, 2H), 3.53 (s, 3H), 3.49 - 3.46 (m, 2H), 2.88 - 2.84 (m, 2H), 2.82 - 2.77 (m, 2H), 2.75 - 2.68 (m, 1H), 2.64 - 2.54 (m, 2H), 2.08 - 2.00 (m, 1H), 1.99 - 1.86 (m, 4H), 1.68 - 1.56 (m, 4H), 1.37 - 1.29 (m, 2H), 1.27 - 1.20 (m, 1H), 1.18 - 1.10 (m, 2H) |
| **I-430^{b}** | AOH | ABC | 874.5 | 11.08 (s, 1H), 9.39 (s, 1H), 8.82 (d, *J* = 8.0 Hz, 1H), 8.37 (s, 1H), 8.28 (s, 1H), 7.25 - 6.82 (m, 5H), 5.42 - 5.25 (m, 1H), 4.24 - 4.12 (m, 1H), 3.83 - 3.77 (m, 4H), 3.77 - 3.69 (m, 5H), 3.61 - 3.47 (m, 5H), 3.49 - 3.44 (m, 2H), 3.43 - 3.37 (m, 2H), 2.99 - 2.92 (m, 2H), 2.92 - 2.83 (m, 1H), 2.78 - 2.68 (m, 2H), 2.66 - 2.56 (m, 2H), 2.16 - 2.08 (m, 2H), 2.07 - 1.94 (m, 4H), 1.94 - 1.86 (m, 2H), 1.85 - 1.80 (m, 2H), 1.79 - 1.68 (m, 3H), 1.67 - 1.51 (m, 1H), 1.15 - 0.97 (m, 2H) |
| **I-434^{b}** | AKS | AJB | 925.5 | 11.09 (s, 1H), 9.50 (d, *J =* 6.8 Hz, 1H), 8.78 (d, *J =* 7.6 Hz, 1H), 8.38 (d, *J =* 4.4 Hz, 1H), 8.26 (d, *J =* 5.6 Hz, 1H), 7.27 - 6.40 (m, 5H), 5.40 - 5.33 (m, 1H), 5.31 - 5.05 (m, 1H), 4.77 (d, *J =* 18.0 Hz, 1H), 4.24 - 4.12 (m, 1H), 3.84 - 3.72 (m, 3H), 3.66 - 3.59 (m, 4H), 3.57 (s, 3H), 2.93 (d, *J =* 7.6 Hz, 3H), 2.75 - 2.61 (m, 4H), 2.33 - 2.28 (m, 5H), 2.19 (s, 4H), 2.09 - 1.94 (m, 6H), 1.89 (d, *J =* 12.0 Hz, 2H), 1.84 - 1.72 (m, 6H), 1.57 - 1.50 (m, 2H), 1.10 - 0.99 (m, 2H) |
| **I-436^{b}** | ALL | AMT | 862.5 | 11.08 (s, 1H), 9.61 (d, *J =* 4.8 Hz, 1H), 8.96 (d, *J =* 3.6 Hz, 1H), 8.79 (d, *J =* 7.6 Hz, 1H), 8.30 (d, *J =* 5.2 Hz, 1H), 7.93 - 7.75 (m, 2H), 7.46 - 7.14 (m, 3H), 6.95 (d, *J =* 4.8 Hz, 2H), 6.91 - 6.43 (m, 2H), 5.39 - 5.05 (m, 2H), 4.78 (d, *J =* 13.2 Hz, 1H), 3.84 - 3.73 (m, 3H), 3.67 - 3.60 (m, 3H), 3.54 (s, 3H), 2.90 - 2.86 (m, 2H), 2.79 (d, *J =* 10.8 Hz, 2H), 2.66 - 2.56 (m, 2H), 2.02 - 1.87 (m, 5H), 1.63 - 1.53 (m, 4H), 1.43 - 1.33 (m, 2H), 1.30 - 1.13 (m, 6H) |
| **I-437^{b}** | AMC | AST | 844.4 | 11.09 (s, 1H), 9.56 (s, 1H), 8.57 (d, *J* = 7.6 Hz, 1H), 8.36 (s, 1H), 8.28 - 8.16 (m, 2H), 7.58 (dd, *J =* 8.4, 7.2 Hz, 2H), 7.24 - 6.94 (m, 3H), 6.68 (t, *J =* 5.6 Hz, 1H), 6.47 (d, *J* = 7.6 Hz, 1H), 5.05 (dd, *J* = 12.8, 5.4 Hz, 1H), 4.20 - 4.10 (m, 1H), 3.50 - 3.40 (m, 4H), 2.95 - 2.83 (m, 1H), 2.62 - 2.52 (m, 7H), 2.32 - 2.30 (m, 1H), 2.12 (s, 3H), 2.08 (d, *J =* 6.8 Hz, 2H), 2.05 - 1.97 (m, 3H), 1.91 - 1.78 (m, 4H), 1.77 - 1.62 (m, 4H), 1.55 - 1.43 (m, 1H), 1.14 - 0.93 (m, 3H), 0.57 - 0.50 (m, 2H), 0.29 - 0.23 (m, 2H) |
| **I-438^{h}** | AFJ | AMT | 878.4 | 11.09 (s, 1H), 9.62 (d, *J =* 4.8 Hz, 1H), 8.98 (d, *J =* 3.2 Hz, 1H), 8.81 (d, *J =* 7.6 Hz, 1H), 8.31 (d, *J =* 5.2 Hz, 1H), 7.79 (d, *J =* 8.4 Hz, 2H), 7.59 (d, *J =* 15.6 Hz, 1H), 7.44 (d, *J =* 8.4 Hz, 2H), 7.32 - 6.47 (m, 5H), 5.34 - 5.09 (m, 1H), 5.05 (d, *J =* 13.2 Hz, 1H), 4.80 (d, *J* = 14.4 Hz, 1H), 3.87 - 3.75 (m, 2H), 3.64 (s, 2H), 3.54 - 3.47 (m, 7H), 2.89 (d, *J =* 22.8 Hz, 1H), 2.71 - 2.56 (m, 4H), 2.34 (d, *J =* 5.2 Hz, 1H), 2.15 - 2.07 (m, 2H), 2.06 - 1.96 (m, 3H), 1.86 - 1.77 (m, 4H), 1.56 - 1.44 (m, 2H) |
| **I-441^{b}** | ASR | ABC | 873.5 | 11.13 (s, 1H), 9.39 (s, 1H), 8.82 (d, *J =* 7.6 Hz, 1H), 8.59 (d*, J =* 4.8 Hz, 1H), 8.37 (s, 1H), 8.29 (s, 1H), 8.17 (s, 1H), 7.10 -7.05 (m, 1H), 7.00 -6.95 (m, 1H), 7.24 - 6.94 (m, 1H), 6.90 (d, *J =* 8.0 Hz, 1H), 5.09 (dd, *J =* 12.8, 5.2 Hz, 1H), 4.23 -4.12 (m, 1H), 3.83 -3.69 (m, 9H), 3.63 -3.53 (m, 4H), 2.93-2.83 (m, 1H), 2.71-2.55 (m, 3H), 2.14 -2.00 (m, 7H), 1.89 -1.68 (m, 8H), 1.59 - 1.39 (m, 3H), 1.09 -0.94 (m, 2H) |
| **I-442^{b}** | AJI | AJB | 840.4 | 11.09 (s, 1H), 9.49 (d, *J =* 6.4 Hz, 1H), 8.78 (d, *J =* 7.6 Hz, 1H), 8.38 (d, *J =* 4.4 Hz, 1H), 8.25 (d, *J =* 5.6 Hz, 1H), 7.58 (t, *J =* 7.6 Hz, 1H), 7.26 - 6.95 (m, 3H), 6.89 - 6.43 (m, 2H), 5.29 - 5.01 (m, 2H), 4.76 (d, *J =* 20.0 Hz, 1H), 4.26 - 4.11 (m, 1H), 3.84 - 3.72 (m, 2H), 3.65 - 3.54 (m, 2H), 3.24 - 3.21 (m, 2H), 3.00 - 2.90 (m, 2H), 2.89 - 2.82 (m, 1H), 2.63 - 2.52 (m, 2H), 2.30 - 2.20 (m, 2H), 2.08 - 1.85 (m, 9H), 1.79 - 1.58 (m, 6H), 1.36 - 1.23 (m, 2H), 1.12 - 0.99 (m, 2H) |
| **I-443^{b}** | AOS | ABC | 897.2 | 11.21 - 10.99 (m, 1H), 9.40 (s, 1H), 8.82 (d, *J =* 7.6 Hz, 1H), 8.37 (s, 1H), 8.29 (s, 1H), 7.60 (t, *J =* 7.6 Hz, 1H), 7.23 - 6.95 (m, 3H), 6.90 (d, *J =* 8.0 Hz, 1H), 6.85 (t, *J* = 4.8 Hz, 1H), 5.06 (dd, *J =* 5.2, 12.8 Hz, 1H), 4.23 - 4.14 (m, 1H), 3.98 (d, *J =* 4.8 Hz, 2H), 3.88 (s, 2H), 3.79 (s, 4H), 3.75 - 3.70 (m, 4H), 3.61 (s, 2H), 2.95 - 2.82 (m, 1H), 2.63 - 2.54 (m, 2H), 2.31 - 2.17 (m, 3H), 2.12 - 1.99 (m, 6H), 1.88 (d, *J =* 12.0 Hz, 2H), 1.80 - 1.67 (m, 6H), 1.63 - 1.53 (m, 1H), 1.10 - 0.96 (m, 2H) |
| **I-444^{b}** | AOQ | AJB | 884.2 | 11.09 (s, 1H), 9.49 (d, *J =* 6.0 Hz, 1H), 8.78 (d, *J =* 7.6 Hz, 1H), 8.38 (d, *J =* 4.0 Hz, 1H), 8.25 (d, *J =* 5.6 Hz, 1H), 7.58 (t, *J =* 8.0 Hz, 1H), 7.25 - 6.94 (m, 2H), 6.93 - 6.41 (m, 3H), 5.32 - 4.99 (m, 2H), 4.77 (d, *J =* 18.4 Hz, 1H), 4.23 - 4.09 (m, 3H), 3.83 - 3.61 (m, 3H), 3.59 (s, 1H), 3.44 (d, *J =* 11.6 Hz, 2H), 2.94 - 2.83 (m, 1H), 2.58 (d, *J =* 18.0 Hz, 1H), 2.42 - 2.31 (m, 5H), 2.27 - 2.20 (m, 2H), 2.13 (s, 3H), 2.11 (d, *J* = 7.6 Hz, 2H), 2.05 - 1.87 (m, 7H), 1.81 - 1.69 (m, 2H), 1.68 - 1.59 (m, 2H), 1.57 - 1.46 (m, 1H), 1.11 - 0.95 (m, 2H) |
| I-44₈^{b} | APR | ABC | 874.5 | 11.09 (s, 1H), 9.39 (s, 1H), 8.82 (d, *J* = 8.0 Hz, 1H), 8.38 (s, 1H), 8.28 (s, 1H), 7.23 - 6.85 (m, 5H), 5.36 (dd, *J =* 5.2, 12.4 Hz, 1H), 4.26 - 4.11 (m, 1H), 3.86 - 3.74 (m, 5H), 3.74 - 3.69 (m, 4H), 3.57 (s, 5H), 3.51 - 3.43 (m, 3H), 3.43 - 3.36 (m, 2H), 3.00 - 2.92 (m, 2H), 2.92 - 2.84 (m, 1H), 2.81 - 2.67 (m, 2H), 2.67 - 2.53 (m, 2H), 2.23 - 2.10 (m, 1H), 2.09 - 2.05 (m, 1H), 2.04 - 2.00 (m, 2H), 1.99 - 1.95 (m, 1H), 1.91 (d*, J =* 12.4 Hz, 2H), 1.87 - 1.78 (m, 3H), 1.78 - 1.68 (m, 2H), 1.68 - 1.56 (m, 1H), 1.15 - 0.96 (m, 2H) |
| **I-449ⁱ** | AKS | AMT | 919.4 | 11.09 (s, 1H), 9.61 (d, *J =* 4.0 Hz, 1H), 8.97 (d, *J =* 3.2 Hz, 1H), 8.80 (d, *J =* 9.2 Hz, 1H), 8.30 (d, *J =* 5.2 Hz, 1H), 7.78 (d, *J =* 8.4 Hz, 2H), 7.45 (d, *J =* 8.4 Hz, 2H), 7.31 - 6.45 (m, 5H), 5.36 (d, *J =* 17.6 Hz, 1H), 5.32 - 5.07 (m, 1H), 4.83 - 4.76 (m, 1H), 3.84 (s, 2H), 3.75 (d, *J* = 7.6 Hz, 1H), 3.63 (s, 2H), 3.58 (s, 3H), 3.56 (s, 3H), 3.52 (s, 4H), 2.97 - 2.87 (m, 3H), 2.73 - 2.62 (m, 2H), 2.29 (d, *J =* 6.4 Hz, 4H), 2.20 (s, 2H), 2.09 - 2.02 (m, 1H), 2.01 - 1.95 (m, 2H), 1.86 - 1.70 (m, 4H), 1.59 - 1.48 (m, 2H) |
| **I-452** | AYI | AJB | 904.4 | 9.50 (d, *J =* 6.0 Hz, 1H), 8.79 (d, *J =* 7.6 Hz, 1H), 8.38 (d, *J* = 4.4 Hz, 1H), 8.26 (d, *J =* 5.6 Hz, 1H), 7.53-7.44 (m, 1H), 7.32-7.10 (m, 6H), 7.03-6.95 (m, 2H), 6.90-6.43 (m, 1H), 5.30-5.06 (m, 2H), 4.82-4.73 (m, 1H), 4.52 (d, *J =* 6.0 Hz, 2H), 4.22-4.10 (m, 1H), 3.85-3.73 (m, 2H), 3.68-3.63 (m, 1H), 3.48-3.43 (m, 1H), 3.05-2.89 (m, 4H), 2.80-2.72 (m, 1H), 2.69-2.64 (m, 2H), 2.60-2.53 (m, 2H), 2.21 (s, 3H), 2.18-2.14 (m, 2H), 2.10-1.90 (m, 6H), 1.86-1.78 (m, 2H), 1.77-1.67 (m, 2H), 1.55-1.45 (m, 1H), 1.03-0.93 (m, 2H) |
| **I-453^{b}** | ALH | AST | 825.3 | 11.10 (s, 1H), 9.57 (m, 1H), 8.57 (d, *J =* 7.6 Hz, 1H), 8.38 (s, 1H), 8.29 - 8.23 (m, 1H), 8.19 (s, 1H), 7.59 (dd, *J =* 8.4, 7.2 Hz, 1H), 7.24 - 6.95 (m, 3H), 6.47 (d, *J =* 7.6 Hz, 1H), 6.16 (d, *J =* 8.2 Hz, 1H), 5.05 (dd, *J =* 12.8, 5.6 Hz, 1H), 4.24 - 4.13 (m, 1H), 3.56 - 3.45 (m, 2H), 2.93 - 2.83 (m, 1H), 2.63 - 2.55 (m, 1H), 2.30 - 2.20 (m, 5H), 2.13 - 1.85 (m, 8H), 1.83 - 1.72 (m, 4H), 1.63 - 1.38 (m, 4H), 1.35 - 1.25 (m, 2H), 1.20 - 0.95 (m, 4H), 0.57 - 0.48 (m, 2H), 0.29 - 0.23 (m, 2H) |
| **I-454** | AWI | AJB | 894.3 | 11.09 (s, 1H), 9.50 (d, *J =* 6.0 Hz, 1H), 8.78 (d, *J =* 7.6 Hz, 1H), 8.39 (d, *J =* 4.4 Hz, 1H), 8.26 (d, *J =* 5.6 Hz, 1H), 7.62-7.55 (m, 1H), 7.25-6.95 (m, 3H), 6.90-6.40 (m, 2H), 5.31-5.03 (m, 2H), 4.82-4.72 (m, 1H), 4.23-4.14 (m, 1H), 4.12-4.05 (m, 1H), 3.85-3.72 (m, 2H), 3.66-3.56 (m, 2H), 2.93-2.85 (m, 1H), 2.65-2.56 (m, 1H), 2.45-2.40 (m, 1H), 2.30-2.16 (m, 7H), 2.08-1.85 (m, 9H), 1.77-1.65 (m, 4H), 1.63-1.48 (m, 4H), 1.40-1.33 (m, 3H), 1.24-1.16 (m, 1H), 1.08-0.95 (m, 2H) |
| **I-455^{b}** | AOV | AQP | 840.1 | 11.09 (s, 1H), 9.40 (s, 1H), 8.72 (d, *J* = 8.0 Hz, 1H), 8.37 (s, 1H), 8.25 (s, 1H), 7.58 (dd, *J* =7.2, 8.4 Hz, 1H), 7.25 - 6.95 (m, 3H), 6.88 - 6.75 (m, 1H), 6.15 (d, *J =* 8.4 Hz, 1H), 5.04 (dd, *J =* 5.2, 12.8 Hz, 1H),4.23 - 4.12 (m, 1H), 3.56 (s, 2H), 3.25 (s, 3H), 2.93 - 2.83 (m, 1H), 2.62 - 2.52 (m, 2H), 2.48 - 2.45 (m, 1H), 2.43 - 2.35 (m, 1H), 2.22 (d, *J =* 6.8 Hz, 2H), 2.20 (s, 3H), 2.08 - 1.99 (m, 5H), 1.90 (d, *J =* 11.6 Hz, 2H), 1.80 - 1.69 (m, 4H), 1.52 - 1.36 (m, 3H), 1.34 - 1.23 (m, 2H), 1.12 - 0.96 (m, 3H), 0.54 - 0.45 (m, 2H), 0.38 - 0.28 (m, 2H) |
| **I-456^{b}** | AOU | AGD | 897.3 | 11.09 (s, 1H), 9.48 (s, 1H), 8.87 (s, 1H), 8.84 (d, *J =* 8.0 Hz, 1H), 8.78 (s, 2H), 8.32 (s, 1H), 7.62 - 7.56 (m, 1H), 7.26 (t, *J =* 53.2 Hz, 1H), 7.10 (d, *J =* 8.4 Hz, 1H), 7.02 (d, *J* = 7.2 Hz, 1H), 6.92 (d, *J =* 8.0 Hz, 1H), 6.67 (t, *J* = 6.0 Hz, 1H), 5.08 - 5.01 (m, 1H), 3.87 - 3.68 (m, 12H), 3.49 - 3.38 (m, 8H), 2.95 - 2.82 (m, 1H), 2.63 - 2.52 (m, 2H), 2.42 (t, *J =* 4.4 Hz, 2H), 2.38 (t, *J =* 7.6 Hz, 2H), 2.07 - 1.98 (m, 1H), 1.86 - 1.78 (m, 2H), 1.77 - 1.69 (m, 2H) |
| **I-457^{b}** | AOW | AJB | 826.2 | 11.10 (s, 1H), 9.50 (d, *J =* 6.4 Hz, 1H), 8.78 (d, *J =* 7.6 Hz, 1H), 8.39 (d, *J =* 4.4 Hz, 1H), 8.26 (d, *J =* 5.6 Hz, 1H), 7.66 - 7.53 (m, 1H), 7.27 - 6.96 (m, 2H), 6.93 - 6.40 (m, 3H), 5.31 - 4.72 (m, 3H), 4.25 - 4.00 (m, 2H), 3.85 - 3.42 (m, 4H), 3.33 - 3.33 (m, 3H), 3.30 - 3.30 (m, 1H), 2.95 - 2.84 (m, 1H), 2.64 - 2.52 (m, 2H), 2.15 - 1.89 (m, 14H), 1.82 - 1.71 (m, 2H), 1.13 - 0.99 (m, 2H) |
| **I-458^{b}** | AOW | ABC | 814.1 | 11.11 (s, 1H), 9.40 (s, 1H), 8.83 (d, *J =* 8.0 Hz, 1H), 8.39 (s, 1H), 8.29 (s, 1H), 7.61 (t, *J =* 7.2 Hz, 1H), 7.28 - 6.86 (m, 4H), 6.52 - 6.49 (m, 1H), 5.06 (dd, *J =* 5.6, 12.8 Hz, 1H), 4.26 - 4.14 (m, 1H), 4.10 - 3.99 (m, 1H), 3.86 - 3.68 (m, 8H), 3.37 - 3.34 (m, 3H), 2.96 - 2.81 (m, 1H), 2.64 - 2.52 (m, 2H), 2.21 - 1.85 (m, 12H), 1.82 - 1.71 (m, 2H), 1.63 - 1.46 (m, 1H), 1.13 - 0.98 (m, 2H) |
| **I-459** | AYG | ABC | 856.4 | 11.10 (s, 1H), 9.40 (s, 1H), 8.83 (d, *J* = 8.0 Hz, 1H), 8.38 (s, 1H), 8.31 - 8.24 (m, 2H), 7.58 (dd, *J=* 7.2, 8.4 Hz, 1H), 7.24 - 6.96 (m, 3H), 6.93 - 6.89 (m, 1H), 6.58 (t, *J=* 6.4 Hz, 1H), 5.10 - 5.02 (m, 1H), 4.23 - 4.09 (m, 1H), 3.84 - 3.69 (m, 9H), 2.89 (s, 2H), 2.24 - 2.15 (m, 5H), 2.10 - 1.63 (m, 13H), 1.52 (s, 2H), 1.28 - 0.93 (m, 7H) |
| **I-460^{b}** | ASP | AJB | 898.4 | 11.08 (s, 1H), 9.49 (d, *J =* 6.4 Hz, 1H), 8.77 (d, *J =* 8.0 Hz, 1H), 8.37 (d, *J =* 4.4 Hz, 1H), 8.25 (d, *J =* 5.6 Hz, 1H), 7.61 - 7.54 (m, 1H), 7.27 - 6.94 (m, 3H), 6.89 - 6.41 (m, 2H), 5.30 - 5.00 (m, 2H), 4.76 (d, *J=* 17.2 Hz, 1H), 4.17 (t, *J =* 11.2 Hz, 1H), 3.84 - 3.56 (m, 5H), 3.50 - 3.41 (m, 2H), 3.28 - 3.25 (m, 2H), 2.93 - 2.82 (m, 1H), 2.69 (d, *J =* 10.4 Hz, 1H), 2.65 - 2.54 (m, 2H), 2.52 (d, *J* = 2.0 Hz, 1H), 2.12 - 1.82 (m, 10H), 1.80 - 1.52 (m, 6H), 1.45 - 1.28 (m, 6H), 1.10 - 0.96 (m, 2H) |
| **I-461^{b}** | APB | AJB | 852.5 | 11.10 (s, 1H), 9.50 (d, *J =* 5.9 Hz, 1H), 8.79 (d, *J* = 7.8 Hz, 1H), 8.44 - 8.35 (m, 1H), 8.26 (d, *J =* 5.6 Hz, 1H), 7.58 (t, *J =* 7.8 Hz, 1H), 7.27 - 6.96 (m, 3H), 6.89 - 6.44 (m, 2H), 5.30 - 5.02 (m, 2H), 4.77 (br d, *J =* 17.6 Hz, 1H), 4.22 - 4.12 (m, 1H), 3.86 - 3.77 (m, 2H), 3.74 (br d, *J =* 7.8 Hz, 1H), 3.64 (br d, *J =* 10.0 Hz, 1H), 3.60 (s, 1H), 3.21 (br s, 3H), 2.95 - 2.87 (m, 1H), 2.63 - 2.56 (m, 1H), 2.53 (d, *J =* 2.0 Hz, 2H), 2.43 - 2.38 (m, 1H), 2.31 - 2.27 (m, 1H), 2.19 (br t, *J =* 10.4 Hz, 2H), 2.08 - 1.78 (m, 10H), 1.77 - 1.64 (m, 2H), 1.57- 1.46 (m, 1H), 1.43 - 1.25 (m, 1H), 1.13 - 0.98 (m, 2H) |
| **I-462^{b}** | AJF | AMT | 874.2 | 11.07 (s, 1H), 9.59 (d, *J =* 5.2 Hz, 1H), 9.03 - 8.90 (m, 1H), 8.79 (d, *J =* 6.4 Hz, 1H), 8.28 (d, *J =* 5.2 Hz, 1H), 7.85 - 7.70 (m, 2H), 7.59 - 7.52 (m, 1H), 7.48 - 7.39 (m, 2H), 7.30 - 6.89 (m, 3H), 6.46 - 6.43 (m, 1H), 5.31 - 5.02 (m, 2H), 4.82 - 4.73 (m, 1H), 3.86 - 3.74 (m, 2H), 3.66 - 3.58 (m, 2H), 3.48 - 3.40 (m, 2H), 3.29 - 3.27 (m, 3H), 2.93 - 2.77 (m, 2H), 2.61 - 2.51 (m, 6H), 2.09 - 1.78 (m, 5H), 1.66 - 1.45 (m, 6H) |
| **I-463^{b}** | AOV | AJB | 854.0 | 11.09 (s, 1H), 9.50 (d, *J =* 6.0 Hz, 1H), 8.78 (d, *J =* 7.6 Hz, 1H), 8.39 (d, *J =* 4.4 Hz, 1H), 8.25 (d, *J =* 5.6 Hz, 1H), 7.58 (t, *J =* 8.0 Hz, 1H), 7.27 - 6.94 (m, 3H), 6.90 - 6.41 (m, 1H), 6.15 (d, *J =* 8.0 Hz, 1H), 5.31 - 4.99 (m, 2H), 4.77 (d, *J =* 16.8 Hz, 1H), 4.23 - 4.12 (m, 1H), 3.83 - 3.72 (m, 2H), 3.66 - 3.57 (m, 2H), 3.52 - 3.49 (m, 1H), 2.92 - 2.83 (m, 1H), 2.64 - 2.52 (m, 2H), 2.42 - 2.36 (m, 1H), 2.22 (d, *J =* 6.8 Hz, 2H), 2.20 (s, 3H), 2.07 - 1.98 (m, 6H), 1.96 - 1.85 (m, 3H), 1.79 - 1.68 (m, 4H), 1.52 - 1.36 (m, 3H), 1.35 - 1.22 (m, 2H), 1.08 - 0.94 (m, 2H) |
| **I-465^{b}** | WX | AJB | 855.1 | 11.10 (s, 1H), 9.60 (d, *J =* 6.0 Hz, 1H), 8.78 (d, *J =* 7.6 Hz, 1H), 8.37 (d, *J =* 4.4 Hz, 1H), 8.25 (d, *J =* 5.6 Hz, 1H), 7.25 - 6.42 (m, 5H), 5.38 (dd, *J =* 5.2, 12.8 Hz, 1H), 5.30 - 5.03 (m, 1H), 4.77 (d, *J =* 17.6 Hz, 1H), 4.24 - 4.09 (m, 1H), 3.85 - 3.72 (m, 2H), 3.67 (s, 3H), 3.63 - 3.58 (m, 3H), 3.46 - 3.42 (m, 2H), 2.92 - 2.81 (m, 4H), 2.20 (d, *J =* 6.8 Hz, 2H), 2.17 (s, 3H), 2.06 - 1.83 (m, 10H), 1.75 - 1.60 (m, 4H), 1.55 - 1.43 (m, 1H), 1.41 - 1.30 (m, 2H), 1.08 - 0.93 (m, 2H) |
| **I-466ⁱ** | AMS | AMT | 860.5 | 11.09 (s, 1H), 9.61 (d, *J =* 4.4 Hz, 1H), 8.96 (d, *J =* 3.2 Hz, 1H), 8.80 (d, *J =* 7.6 Hz, 1H), 8.30 (d, *J =* 5.2 Hz, 1H), 7.77 (d, *J =* 8.4 Hz, 2H), 7.42 (d, *J =* 8.4 Hz, 2H), 7.32 - 7.12 (m, 1H), 6.98 - 6.46 (m, 4H), 5.35 (dd, *J =* 5.2, 12.8 Hz, 1H), 5.31 - 5.05 (m, 1H), 4.78 (d, *J =* 13.6 Hz, 1H), 4.45 - 4.30 (m, 1H), 3.83 (s, 1H), 3.78 - 3.70 (m, 1H), 3.63 (s, 1H), 3.53 (s, 3H), 3.45 - 3.42 (m, 2H), 3.02 (d, *J* = 7.2 Hz, 2H), 2.93 - 2.83 (m, 1H), 2.75 - 2.68 (m, 1H), 2.64 - 2.59 (m, 1H), 2.30 - 2.21 (m, 2H), 2.06 - 1.93 (m, 3H), 1.87 (t, *J =* 9.6 Hz, 2H), 1.59 - 1.47 (m, 5H), 1.05 (t, *J =* 6.8 Hz, 4H) |
| **I-467^{b}** | ANI | AJB | 937.6 | 11.09 (s, 1H), 9.50 (d, *J =* 6.4 Hz, 1H), 8.78 (d, *J =* 7.6 Hz, 1H), 8.38 (d, *J =* 4.4 Hz, 1H), 8.26 (d, *J =* 5.6 Hz, 1H), 7.27 - 6.89 (m, 4H), 6.89 - 6.42 (m, 1H), 5.43 - 5.31 (m, 1H), 5.30 - 5.05 (m, 1H), 4.82 - 4.70 (m, 1H), 4.24 - 4.11 (m, 1H), 3.84 - 3.74 (m, 2H), 3.67 - 3.59 (m, 2H), 3.58 (s, 3H), 3.48 - 3.46 (m, 2H), 3.16 - 3.12 (m, 2H), 2.93 - 2.84 (m, 1H), 2.76 - 2.63 (m, 4H), 2.35 - 2.28 (m, 4H), 2.19 - 2.10 (m, 2H), 2.08 - 1.82 (m, 8H), 1.79 - 1.67 (m, 2H), 1.63 - 1.52 (m, 1H), 1.48 - 1.38 (m, 4H), 1.34 - 1.23 (m, 4H), 1.17 - 0.93 (m, 3H) |
| **I-468** | TN | AUY | 858.5 | 11.09 (s, 1H), 9.42 (s, 1H), 8.83 (d, *J =* 8.0 Hz, 1H), 8.28 (s, 1H), 8.05 (s, 1H), 7.65 -7.36 (m, 1H), 6.98 - 6.85 (m, 4H), 5.36 (dd, *J =* 5.2 Hz, 12.8 Hz, 1H), 4.23 (m, 1H), 3.77 (d, *J =* 4.4 Hz, 4H), 3.72 (br d, *J =* 4.8 Hz, 4H), 3.57 (s, 3H), 3.47 -3.44 (m, 3H), 2.98 - 2.93 (m, 2H), 2.92 - 2.84 (m, 1H), 2.77 - 2.69 (m, 1H), 2.65 - 2.56 (m, 2H), 2.12 - 1.77 (m, 16H), 1.58 - 1.40 (m, 3H), 1.09 - 0.97 (m, 2H) |
| **I-469** | AUW | AJB | 896.5 | 11.09 (s, 1H), 9.49 (d, *J =* 6.4 Hz, 1H), 8.78 (d, *J =* 7.6 Hz, 1H), 8.37 (d, *J =* 4.4 Hz, 1H), 8.25 (d, *J =* 5.6 Hz, 1H), 7.25 - 6.89 (m, 4H), 6.88 - 6.42 (m, 1H), 5.37 (dd, *J =* 5.6, 12.4 Hz, 1H), 5.28 - 5.07 (m, 1H), 4.81 - 4.73 (m, 1H), 4.22 - 4.11 (m, 1H), 3.94 (t, *J =* 6.8 Hz, 1H), 3.83 - 3.74 (m, 2H), 3.66 - 3.48 (m, 9H), 3.14 - 3.10 (m, 2H), 2.85 - 2.71 (m, 2H), 2.25 - 2.15 (m, 3H), 2.09 - 1.98 (m, 8H), 1.86 (d, *J =* 11.6 Hz, 2H), 1.77 - 1.67 (m, 2H), 1.59 - 1.38 (m, 8H), 1.06 - 0.96 (m, 2H) |
| **I-470** | AMP | AJB | 916.4 | 11.08 (s, 1H), 9.50 (d, *J =* 5.6 Hz, 1H), 8.78 (d, *J =* 7.6 Hz, 1H), 8.39 (d, *J =* 4.0 Hz, 1H), 8.26 (d, *J =* 5.6 Hz, 1H), 7.25 - 6.43 (m, 5H), 5.41 - 5.05 (m, 2H), 4.77 (d, *J* = 18.4 Hz, 1H), 4.23 - 4.15 (m, 1H), 3.85 - 3.78 (m, 2H), 3.74 (d, *J =* 7.6 Hz, 1H), 3.65 - 3.58 (m, 3H), 3.57 (s, 3H), 3.00 - 2.93 (m, 3H), 2.89 - 2.81 (m, 2H), 2.74 - 2.70 (m, 1H), 2.64 - 2.57 (m, 2H), 2.25 - 2.23 (m, 2H), 2.08 - 1.66 (m, 15H), 1.59 - 1.49 (m, 3H), 1.46 - 1.37 (m, 1H), 1.20 - 1.10 (m, 2H) |
| **I-471^{b}** | ANH | AJB | 869.6 | 11.08 (s, 1H), 9.49 (d, *J =* 6.4 Hz, 1H), 8.77 (d, *J=* 7.6 Hz, 1H), 8.37 (d, *J =* 4.4 Hz, 1H), 8.25 (d, *J =* 5.6 Hz, 1H), 7.10 (d, *J =* 3.6 Hz, 1H), 6.96 (d, *J =* 4.8 Hz, 2H), 6.89 - 6.43 (m, 2H), 5.36 (dd, *J =* 5.6, 12.4 Hz, 1H), 5.29 - 5.05 (m, 1H), 4.81 - 4.72 (m, 1H), 4.22 - 4.11 (m, 1H), 3.84 - 3.72 (m, 3H), 3.65 - 3.59 (m, 2H), 3.57 (s, 3H), 2.93 - 2.86 (m, 3H), 2.73 - 2.58 (m, 3H), 2.52 (d, *J* = 2.0 Hz, 1H), 2.40 - 2.32 (m, 6H), 2.11 (d, *J =* 7.6 Hz, 2H), 2.07 - 1.83 (m, 8H), 1.79 - 1.68 (m, 2H), 1.63 - 1.50 (m, 5H), 1.08 - 0.097 (m, 2H) |
| **I-473** | AYE | ABC | 926.6 | 11.09 (s, 1H), 9.39 (s, 1H), 8.82 (d, *J* = 8.0 Hz, 1H), 8.37 (s, 1H), 8.29 (s, 1H), 7.57 (dd, *J* = 8.4, 7.2 Hz, 1H), 7.25 - 6.95 (m, 3H), 6.90 (d, *J =* 8.0 Hz, 1H), 6.57 (t, *J* = 6.0 Hz, 1H), 5.05 (dd, *J =* 12.8, 5.2 Hz, 1H), 4.23 - 4.13 (m, 1H), 3.83 - 3.76 (m, 4H), 3.75 - 3.69 (m, 4H), 3.43 - 3.39 (m, 1H), 3.30 - 3.37 (m, 1H), 3.16 (t, *J =* 6.4 Hz, 2H), 2.95 - 2.83 (m, 1H), 2.72 - 2.65 (m, 2H), 2.63 - 2.53 (m, 2H), 2.13 - 2.00 (m, 6H), 1.95 - 1.83 (m, 4H), 1.81 - 1.68 (m, 6H), 1.61 - 1.47 (m, 2H), 1.45 - 1.34 (m, 2H), 1.15 - 0.95 (m, 6H) |
| **I-474^{b}** | APH | APJ | 895.3 | 11.09 (s, 1H), 10.89 - 10.78 (m, 1H), 9.60 (d, *J =* 3.2 Hz, 1H), 8.95 (d, *J =* 3.6 Hz, 1H), 8.90 (s, 2H), 8.80 (d, *J =* 7.2 Hz, 1H), 8.29 (d, *J =* 5.2 Hz, 1H), 7.64 - 7.57 (m, 1H), 7.44 - 7.14 (m, 2H), 7.05 (d, *J =* 7.2 Hz, 1H), 6.91 - 6.46 (m, 2H), 5.31 - 5.03 (m, 2H), 4.78 d, *J =* 16.0 Hz, 1H), 4.71 (d, *J =* 13.6 Hz, 2H), 3.87 - 3.72 (m, 3H), 3.66 - 3.61 (m, 5H), 3.52 - 3.43 (m, 6H), 3.21 - 3.12 (m, 2H), 3.11 - 3.00 (m, 2H), 2.94 - 2.83 (m, 1H), 2.63 - 2.53 (m, 2H), 2.10 - 1.92 (m, 5H) |
| I-475 | AUE | AJB | 913.5 | 11.1 (s, 1H), 9.49 (d, *J =* 6.4 Hz, 1H), 8.78 (d, *J =* 8.0 Hz, 1H), 8.37 (d, *J =* 4.4 Hz, 1H), 8.25 (d, *J =* 5.6 Hz, 1H), 7.60 - 7.55 (m, 1H), 7.23 - 6.96 (m, 3H), 6.87 - 6.44 (m, 2H), 5.28 - 5.02 (m, 2H), 4.76 (d, *J =* 18.4 Hz, 1H), 4.20 - 4.14 (m, 1H), 3.83 - 3.59 (m, 6H), 3.54 - 3.44 (m, 3H), 2.92 - 2.80 (m, 2H), 2.65 - 2.56 (m, 2H), 2.30 - 2.26 (m, 4H), 2.11 (s, 3H), 2.09 (d, *J =* 7.2 Hz, 2H), 2.04 - 1.80 (m, 10H), 1.78 - 1.67 (m, 2H), 1.57 - 1.49 (m, 3H), 1.06 - 0.97 (m, 2H) |
| **I-476^{j}** | AOV | ATY | 836.4 | 11.08 (s, 1H), 9.50 (s, 1H), 8.96 (s, 1H), 8.84 (d, *J =* 8.0 Hz, 1H), 8.32 (s, 1H), 7.77 (d, *J =* 8.4 Hz, 2H), 7.58 (d, *J =* 15.6 Hz, 1H), 7.45 (d, *J =* 8.4 Hz, 2H), 7.41 - 7.13 (m, 2H), 7.02 (d, *J =* 6.8 Hz, 1H), 6.92 (d, *J =* 8.0 Hz, 1H), 6.16 (d, *J =* 8.0 Hz, 1H), 5.04 (d, *J =* 16.0 Hz, 1H), 3.81 (s, 4H), 3.74 (d, *J =* 4.4 Hz, 4H), 3.60 (s, 2H), 2.93 - 2.78 (m, 1H), 2.69 - 2.51 (m, 3H), 2.14 (s, 3H), 2.10 - 1.94 (m, 4H), 1.87 (d, *J =* 12.0 Hz, 2H), 1.53 (d, *J =* 11.2 Hz, 2H), 1.37 - 1.21 (m, 2H) |
| I-478 | AOV | AUU | 841.2 | 11.09 (s, 1H), 9.90 (s, 1H), 9.29 (d, *J =* 7.1 Hz, 1H), 8.64 (s, 1H), 8.41 (s, 1H), 7.59 (dd, *J* = 7.2*,* 8.4 Hz, 1H), 7.33 (d, *J =* 7.3 Hz, 1H), 7.30 - 7.01 (m, 3H), 6.16 (d, *J* = 8.1 Hz, 1H), 5.05 (dd, *J =* 5.4, 12.7 Hz, 1H), 4.28 - 4.17 (m, 1H), 4.04 - 3.98 (m, 2H), 3.51 - 3.47 (m, 2H), 3.19 - 3.14 (m, 1H), 2.94 - 2.84 (m, 1H), 2.63 - 2.56 (m, 1H), 2.43 (d, *J =* 1.1 Hz, 1H), 2.26 (d, *J =* 6.8 Hz, 2H), 2.23 (s, 3H), 2.11 - 1.99 (m, 6H), 1.96 - 1.88 (m, 6H), 1.78 (d, *J =* 11.6 Hz, 4H), 1.52 - 1.39 (m, 3H), 1.38 - 1.22 (m, 3H), 1.10 - 0.96 (m, 2H) |
| **I-481^{b}** | TN | APN | 808.2 | 11.09 (s, 1H), 9.63 (s, 1H), 9.38 (dd, *J =* 1.6, 7.2 Hz, 1H), 8.90 (dd, *J =* 1.6, 4.4 Hz, 1H), 8.66 (s, 1H), 8.03 (s, 1H), 7.33 (dd, *J =* 4.4, 7.2 Hz, 1H), 7.01 - 6.94 (m, 2H), 6.92 - 6.84 (m, 1H), 5.40 - 5.31 (m, 1H), 4.08 - 3.97 (m, 1H), 3.87 - 3.76 (m, 4H), 3.71 - 3.64 (m, 1H), 3.58 (d, *J =* 9.6 Hz, 3H), 3.53 - 3.48 (m, 2H), 3.40 - 3.34 (m, 1H), 3.05 - 2.83 (m, 11H), 2.75 - 2.61 (m, 2H), 2.20 - 1.55 (m, 15H), 1.31 - 1.05 (m, 2H) |
| **I-482^{b}** | AYC | AJB | 886.5 | 11.18 (s, 1H), 9.50 (s, 1H), 8.91 - 8.74 (m, 2H), 8.55 - 8.23 (m, 3H), 7.71 (s, 1H), 7.60-7.50 (m, 1H), 7.45 - 7.30 (m, 2H), 7.26 - 6.94 (m, 1H), 6.88 - 6.40 (m, 1H), 6.10 (s, 1H), 5.30 - 5.03 (m, 1H), 4.82 - 4.72 (m, 1H), 4.71 - 4.61 (m, 2H), 4.22 - 4.12 (m, 1H), 3.81 (s, 1H), 3.75 - 3.67 (m, 2H), 3.61 - 3.58 (m, 1H), 3.09 - 2.96 (m, 2H), 2.78 - 2.68 (m, 3H), 2.21 - 2.14 (m, 4H), 2.09 - 1.53 (m, 15H), 1.10 - 0.96 (m, 2H) |
| I-483 | AUS | AJB | 866.0 | 11.13 ( s, 1H), 9.51 (d, *J =* 6.0 Hz, 1H), 8.80 (d, *J* = 7.6 Hz, 1H), 8.39 (d, *J =* 4.0 Hz, 1H), 8.27 (d, *J =* 5.6 Hz, 1H), 7.34 (s, 1H), 7.30 - 6.95 (m, 3H), 6.94 - 6.40 (m, 1H), 5.40 (dd, *J* = 5.2, 12.8 Hz, 1H), 5.32 - 5.05 (m, 1H), 4.78 (d, *J =* 17.2 Hz, 1H), 4.40 (s, 2H), 4.18 (d, *J* = 2.8 Hz, 1H), 3.85 - 3.73 (m, 2H), 3.66 - 3.36 (m, 3H), 3.32 (s, 3H), 2.97 - 2.85 (m, 1H), 2.78 - 2.61 (m, 4H), 2.15 - 1.99 (m, 8H), 1.98 - 1.83 (m, 5H), 1.80 - 1.68 (m, 2H), 1.68 - 1.49 (m, 3H), 1.10 - 0.98 (m, 2H) |
| **I-484** | TN | ATR | 911.5 | 11.19 - 10.80 (m, 2H), 8.78 (d, *J =* 7.8 Hz, 1H), 8.49 - 8.11 (m, 3H), 7.94 - 7.60 (m, 2H), 6.97 (d, *J =* 4.6 Hz, 2H), 6.92 - 6.43 (m, 2H), 5.37 (dd, *J =* 5.6, 12.4 Hz, 1H), 5.24 - 5.05 (m, 1H), 4.81 - 4.10 (m, 2H), 3.87 - 3.60 (m, 2H), 3.58 (s, 3H), 3.52 - 3.42 (m, 3H), 3.28 - 3.22 (m, 1H), 3.01 - 2.83 (m, 3H), 2.74 - 2.60 (m, 4H), 2.36 - 2.30 (m, 3H), 2.14 - 2.06 (m, 4H), 2.06 - 1.95 (m, 4H), 1.93 - 1.91 (m, 2H), 1.88 - 1.70 (m, 7H), 1.67 - 1.35 (m, 4H), 1.19 - 0.98 (m, 2H) |
| **I-485^{b}** | ASB | AJB | 891.6 | 11.13 (s, 1H), 9.50 (d, *J =* 6.4 Hz, 1H), 8.79 (d, *J =* 7.6 Hz, 1H), 8.39 (d, *J =* 4.0 Hz, 1H), 8.25 (d, *J =* 5.6 Hz, 1H), 7.26 - 6.96 (m, 4H), 6.90 - 6.43 (m, 1H), 5.40 (dd, *J =* 5.6, 12.4 Hz, 1H), 5.30 - 5.05 (m, 1H), 4.83 - 4.70 (m, 1H), 4.31 - 4.13 (m, 1H), 3.85 - 3.77 (m, 2H), 3.76 - 3.71 (m, 1H), 3.65 (s, 3H), 3.63 - 3.51 (m, 6H), 3.47 - 3.42 (m, 1H), 2.95 - 2.74 (m, 3H), 2.73 - 2.67 (m, 1H), 2.65 - 2.58 (m, 1H), 2.45 - 2.39 (m, 2H), 2.09 - 1.89 (m, 6H), 1.88 - 1.48 (m, 10H), 1.20 - 1.04 (m, 2H) |
| **I-486^{b}** | APZ | AJB | 880.4 | 11.09 (s, 1H), 9.50 (d, *J =* 6.0 Hz, 1H), 8.78 (d, *J =* 7.6 Hz, 1H), 8.38 (d, *J =* 4.8 Hz, 1H), 8.26 (d, *J =* 5.6 Hz, 1H), 7.28 - 6.93 (m, 3H), 6.88 - 6.43 (m, 2H), 5.36 (*J =* 5.2, 12.8 Hz, 1H), 5.30 - 5.06 (m, 1H), 4.80 - 4.76 (m, 1H), 4.22 - 4.14 (m, 1H), 3.83 - 3.67 (m, 3H), 3.56 (s, 3H), 2.93 - 2.60 (m, 6H), 2.31 - 2.19 (m, 4H), 2.10 - 1.86 (m, 11H), 1.79 - 1.67 (m, 4H), 1.60 - 1.35 (m, 7H), 1.10 - 0.97 (m, 2H) |
| **I-487^{b}** | APY | AJB | 840.5 | 11.08 (s, 1H) 9.49 (d, *J =* 5.6 Hz, 1H) 8.78 (d, *J =* 7.6 Hz, 1H) 8.38 (d, *J =* 4.0 Hz, 1H) 8.25 (d, *J =* 5.6 Hz, 1H) 7.10 (d, *J =* 3.2 Hz, 1H) 6.91 - 7.28 (m, 1H) 6.43 - 6.89 (m, 2H) 5.36 (dd, *J =* 12.4, 5.6 Hz, 1H) 5.04 - 5.30 (m, 1H) 4.71 - 4.82 (m, 1H) 4.12 - 4.22 (m, 1H) 3.60 - 3.80 (m, 3H) 3.56 (s, 3H) 2.70 - 2.95 (m, 6H) 1.94 - 2.13 (m, 8H) 1.68 - 1.91 (m, 9H) 1.48 - 1.58 (m, 3H) 1.14 - 1.32 (m, 3H) 0.98 - 1.09 (m, 2H) |
| **I-488** | TN | AUR | 818.5 | 11.08 (s, 1H), 9.42 (s, 1H), 8.82 (d, *J =* 8.0 Hz, 1H), 8.28 (s, 1H), 8.06 (s, 1H), 7.63 - 7.29 (m, 1H), 6.95 (d, *J* = 4.8 Hz, 2H), 6.90 (d, *J =* 8.0 Hz, 1H), 6.87 - 6.83 (m, 1H), 5.38 - 5.31 (m, 1H), 4.23 - 4.17 (m, 2H), 3.80 - 3.68 (m, 9H), 3.55 (s, 3H), 3.49 - 3.39 (m, 3H), 3.29 (s, 3H), 2.94 (t, *J =* 7.2 Hz, 2H), 2.63 - 2.57 (m, 1H), 2.06 - 1.88 (m, 3H), 1.87 - 1.70 (m, 7H), 1.68 - 1.23 (m, 5H) |
| **I-491^{b}** | AYB | AJB | 854.5 | 11.08 (s, 1H), 9.49 (d, *J =* 5.8 Hz, 1H), 8.79 (d, *J =* 7.8 Hz, 1H), 8.34 (d, *J =* 4.4 Hz, 1H), 8.26 (d, *J =* 5.6 Hz, 1H), 7.64 - 7.56 (m, 1H), 7.29 (d, *J =* 8.8 Hz, 1H), 7.24 - 6.93 (m, 2H), 6.90 - 6.43 (m, 2H), 5.32 - 5.03 (m, 2H), 4.78 (d, *J =* 16.8 Hz, 1H), 4.17 - 4.05 (m, 1H), 3.85 - 3.72 (m, 2H), 3.67 - 3.57 (m, 2H), 3.53 (d, *J =* 5.4 Hz, 2H), 3.45 (d, *J =* 10.0 Hz, 2H), 2.93 - 2.83 (m, 1H), 2.59 (d, *J =* 2.6 Hz, 1H), 2.56 - 2.53 (m, 2H), 2.42 (s, 2H), 2.15 (s, 3H), 2.12 d, *J =* 7.0 Hz, 2H), 1.97 - 1.77 (m, 10H), 1.72 - 1.58 (m, 2H), 1.56 - 1.45 (m, 1H), 0.98 (q, *J* = 11.8 Hz, 2H) |
| **I-492^{b}** | AOJ | AJB | 895.4 | 1.02 - 1.17 (m, 2 H), 1.43 - 1.55 (m, 3 H), 1.57 - 1.68 (m, 4 H), 1.71 - 1.83 (m, 4 H), 1.89 - 1.91 (m, 1 H), 1.90 - 2.10 (m, 7 H), 2.15 - 2.25 (m, 4 H), 2.29 - 2.34 (m, 2 H), 2.58 - 2.75 (m, 3 H), 2.80 - 3.00 (m, 3 H), 3.16 (s, 2 H), 3.52 - 3.68 (m, 5 H), 3.70 - 3.90 (m, 2 H), 4.18 (t, *J =* 10.0 Hz, 1 H), 4.77 (d, *J =* 14.0 Hz, 1 H), 5.05 - 5.31 (m, 1 H), 5.37 (dd, *J =* 12.0, 6.0 Hz, 1 H), 6.41 - 6.91 (m, 2 H), 6.91 - 7.30 (m, 3 H), 8.10 - 8.48 (m, 2 H), 8.79 (d, *J =* 8.0 Hz, 1 H), 9.50 (d, *J =* 6.0 Hz, 1 H), 11.09 (s, 1 H) |
| **I-493^{b}** | ARP | AJB | 909.5 | 11.08 (s, 1H), 9.50 (d, *J =* 6.4 Hz, 1H), 8.77 (d, *J =* 7.8 Hz, 1H), 8.39 (d, *J =* 4.0 Hz, 1H), 8.26 (d, *J =* 5.4 Hz, 1H), 7.26 - 7.00 (m, 1H), 6.99 - 6.94 (m, 2H), 6.89 - 6.42 (m, 2H), 5.36 (dd, *J =* 5.2, 12.4 Hz, 1H), 5.30 - 5.05 (m, 1H), 4.77 (d, *J =* 16.4 Hz, 1H), 4.17 (t, *J =* 10.4 Hz, 1H), 3.98 (d, *J =* 11.6 Hz, 1H), 3.82 - 3.73 (m, 2H), 3.66 - 3.54 (m, 6H), 3.48 - 3.42 (m, 3H), 3.22 - 3.15 (m, 2H), 2.96 - 2.83 (m, 3H), 2.75 - 2.65 (m, 2H), 2.44 - 2.25 (m, 2H), 2.18 - 1.93 (m, 9H), 1.85 - 1.68 (m, 6H), 1.52 - 1.38 (m, 2H), 1.08 - 1.04 (m, 2H) |
| **I-497^{b}** | APX | AJB | 883.6 | 11.08 (s, 1H), 9.50 (d, *J =* 6.0 Hz, 1H), 8.78 (d, *J =* 7.8 Hz, 1H), 8.39 (d, *J =* 4.4 Hz, 1H), 8.26 (d, *J =* 5.6 Hz, 1H), 7.28 - 6.94 (m, 3H), 6.91 - 6.41 (m, 2H), 5.37 (dd, *J =* 5.4, 12.3 Hz, 1H), 5.31 - 5.05 (m, 1H), 4.77 (d, *J =* 16.8 Hz, 1H), 4.26 - 4.12 (m, 1H), 3.86 - 3.72 (m, 2H), 3.68 - 3.51 (m, 5H), 3.48 - 3.42 (m, 4H), 2.97 - 2.84 (m, 3H), 2.70 - 2.59 (m, 2H), 2.43 (t, *J =* 7.2 Hz, 2H), 2.36 - 2.27 (m, 4H), 2.14 (d, *J =* 7.2 Hz, 2H), 2.07 - 1.70 (m, 11H), 1.67 - 1.54 (m, 1H), 1.13 - 0.99 (m, 2H) |
| **I-500^{b}** | ASU | AJB | 899.3 | 11.08 (s, 1H), 9.49 (d, *J =* 5.6 Hz, 1H), 8.78 (d, *J* = 7.6 Hz, 1H), 8.37 (d, *J =* 4.4 Hz, 1H), 8.25 (d, *J* = 5.4 Hz, 1H), 7.62 - 7.54 (m, 1H), 7.25 - 6.95 (m, 3H), 6.89 - 6.43 (m, 2H), 5.31 - 5.00 (m, 2H), 4.81 - 4.72 (m, 1H), 4.21 - 4.11 (m, 1H), 3.84 - 3.71 (m, 3H), 3.66 - 3.57 (m, 3H), 3.53 - 3.41 (m, 6H), 2.91 - 2.83 (m, 2H), 2.73 - 2.68 (m, 1H), 2.61 - 2.56 (m, 1H), 2.41 (d, *J =* 4.0 Hz, 4H), 2.15 (s, 3H), 2.12 - 1.86 (m, 10H), 1.81 - 1.68 (m, 2H), 1.08 - 0.96 (m, 2H) |
| **I-501^{b}** | AFJ | AQU | 886.6 | 11.09 (s, 1H), 8.63 (d, *J* = 7.6 Hz, 1H), 7.87 (s, 1H), 7.78 - 7.35 (m, 2H), 7.10 (d, *J =* 8.8 Hz, 1H), 7.02 (d, *J* = 6.8 Hz, 1H), 6.99 - 6.69 (m, 2H), 6.63 (t, *J =* 5.2 Hz, 1H), 5.05 (dd, *J =* 5.2, 13.2 Hz, 1H), 4.07 - 3.97 (m, 1H), 3.71 - 3.67 (m, 4H), 3.66 - 3.63 (m, 4H), 3.52 - 3.46 (m, 2H), 3.40 - 3.36 (m, 4H), 3.25 (s, 3H), 2.94 - 2.84 (m, 1H), 2.65 - 2.55 (m, 3H), 2.07 - 1.95 (m, 5H), 1.90 - 1.74 (m, 8H), 1.65 - 1.52 (m, 2H), 1.51 - 1.38 (m, 3H), 1.04 - 0.89 (m, 2H) |
| **I-503^{b}** | AIR | AQU | 920.3 | 10.98 (s, 1H), 8.63 (d, *J* = 8.0 Hz, 1H), 7.87 (s, 1H), 7.73 - 7.52 (m, 1H), 7.52 - 7.40 (m, 3H), 7.35 - 7.28 (m, 4H), 7.01 - 6.69 (m, 2H), 5.22 (s, 2H), 5.11 (dd, *J =* 4.8, 13.2 Hz, 1H), 4.44 - 4.22 (m, 2H), 4.08 - 3.97 (m, 1H), 3.70 - 3.62 (m, 8H), 3.45 (s, 2H), 3.25 (s, 3H), 3.00 - 2.82 (m, 2H), 2.62 - 2.53 (m, 2H), 2.45 - 2.30 (m, 7H), 2.08 (d, *J* = 6.8 Hz, 2H), 2.03 - 1.93 (m, 1H), 1.92 - 1.78 (m, 4H), 1.65 - 1.43 (m, 3H), 1.03 - 0.91 (m, 2H) |
| **I-505** | AQS | AJB | 896.3 | 11.10 (s, 1H), 9.49 (d, *J =* 6.0 Hz, 1H), 8.78 (d, *J* = 7.6 Hz, 1H), 8.38 (d, *J =* 4.4 Hz, 1H), 8.27 - 8.22 (m, 1H), 7.59 (d, *J =* 15.6 Hz, 1H), 7.26 - 6.42 (m, 5H), 5.30 - 5.02 (m, 2H), 4.81 - 4.73 (m, 1H), 4.30 (d, *J =* 6.0 Hz, 1H), 4.23 - 4.07 (m, 2H), 3.83 - 3.72 (m, 2H), 3.66 - 3.57 (m, 2H), 2.89 (d, *J =* 35.6 Hz, 1H), 2.73 - 2.66 (m, 2H), 2.66 - 2.55 (m, 2H), 2.41 - 2.31 (m, 3H), 2.30 - 2.20 (m, 2H), 2.12 - 1.93 (m, 9H), 1.87 (d, *J =* 10.4 Hz, 2H), 1.83 - 1.67 (m, 4H), 1.60 - 1.49 (m, 1H), 1.47 - 1.36 (m, 2H), 1.09 - 0.96 (m, 2H) |
| **I-506^{b}** | AQS | ABC | 884.5 | 11.10 (s, 1H), 9.39 (s, 1H), 8.82 (d, *J* = 8.0 Hz, 1H), 8.37 (s, 1H), 8.28 (s, 1H), 7.63 - 7.54 (m, 1H), 7.24 - 6.94 (m, 2H), 6.90 (dd, *J =* 4.0, 8.0 Hz, 2H), 6.49 (d, *J* = 5.6 Hz, 1H), 5.06 (dd, *J =* 5.2, 12.8 Hz, 1H), 4.34 - 4.25 (m, 1H), 4.21 - 4.06 (m, 2H), 3.82 - 3.76 (m, 4H), 3.74 - 3.70 (m, 4H), 2.93 - 2.85 (m, 1H), 2.71 - 2.65 (m, 2H), 2.64 - 2.53 (m, 2H), 2.40 - 2.34 (m, 2H), 2.28 - 2.21 (m, 2H), 2.08 (d, *J =* 7.2 Hz, 2H), 2.06 - 1.66 (m, 12H), 1.59 - 1.50 (m, 1H), 1.47 - 1.36 (m, 2H), 1.11 - 0.93 (m, 2H) |
| **I-508** | AVU | AJB | 854.6 | 11.09 (s, 1H), 9.49 (d, *J =* 6.0 Hz, 1H), 8.77 (d, *J* = 7.8 Hz, 1H), 8.37 (d, *J =* 4.4 Hz, 1H), 8.25 (d, *J =* 5.6 Hz, 1H), 7.25 - 6.98 (m, 1H), 6.97 - 6.90 (m, 2H), 6.87 - 6.43 (m, 2H), 5.36 (dd, *J =* 5.6, 12.6 Hz, 1H), 5.29 - 5.02 (m, 1H), 4.76 (d, *J =* 17.6 Hz, 1H), 4.16 (t, *J =* 10.0 Hz, 1H), 3.82 - 3.60 (m, 4H), 3.53 (s, 3H), 2.99 - 2.79 (m, 2H), 2.79 - 2.56 (m, 5H), 2.42 - 2.30 (m, 2H), 2.23 (d, *J =* 6.4 Hz, 2H), 2.18 (s, 3H), 2.04 - 1.86 (m, 6H), 1.72 (t, *J =* 13.2 Hz, 6H), 1.50 - 1.39 (m, 2H), 1.24 - 0.94 (m, 6H) |
| **I-509** | AOD | AUN | 839.1 | 12.19 (s, 1H), 11.11 (s, 1H), 9.37 (dd, *J* = 1.6, 7.2 Hz, 1H), 9.14 (dd, *J* = 1.6, 4.4 Hz, 1H), 8.72 - 8.65 (m, 2H), 8.48 (s, 1H), 7.86 (d, *J =* 8.4 Hz, 1H), 7.76 - 7.68 (m, 1H), 7.35 (dd, *J =* 4.4, 6.4 Hz, 1H), 6.9 - 6.83 (m, 3H), 5.37 (dd, *J =* 6.4, 12.4 Hz, 1H), 4.25 - 4.18 (m, 1H), 3.58 (s, 3H), 3.09 - 3.01 (m, 4H), 2.96 - 2.84 (m, 4H), 2.65 (d, *J =* 4.4 Hz, 3H), 2.41 (s, 3H), 2.36 - 2.32 (m, 2H), 2.16 - 2.07 (m, 4H), 2.04 - 1.97 (m, 1H), 1.96 - 1.87 (m, 2H), 1.85 - 1.75 (m, 2H), 1.71 - 1.58 (m, 7H), 1.14 - 1.00 (m, 2H) |
| **I-510^{b}** | ARK | ARM | 841.6 | 11.10 (s, 1H), 9.49 (d, *J =* 6.4 Hz, 1H), 8.76 (d, *J =* 7.6 Hz, 1H), 8.36 (d, *J =* 4.8 Hz, 1H), 8.30 - 8.20 (m, 1H), 7.25 - 6.93 (m, 3H), 6.88 (d, *J =* 7.6 Hz, 1H), 6.86 - 6.36 (m, 1H), 5.38 - 5.26 (m, 1H), 5.30 - 5.01 (m, 1H), 4.76 (d, *J =* 15.6 Hz, 1H), 4.24 - 4.09 (m, 1H), 3.86 - 3.72 (m, 2H), 3.66 (s, 3H), 3.64 - 3.56 (m, 4H), 3.44 - 3.44 (m, 2H), 2.93 - 2.83 (m, 1H), 2.78 - 2.58 (m, 3H), 2.45 - 2.35 (m, 4H), 2.31 - 2.24 (m, 3H), 2.04 - 1.90 (m, 5H), 1.82 (d, *J =* 12.4 Hz, 2H), 1.76 - 1.63 (m, 2H), 1.36 - 1.25 (m, 3H), 1.14 - 1.01 (m, 2H) |
| **I-511^{b}** | ARN | ARM | 867.6 | 11.10 (s, 1H), 9.50 (d, *J =* 6.0 Hz, 1H), 8.76 (d, *J =* 7.6 Hz, 1H), 8.37 (d, *J =* 4.4 Hz, 1H), 8.25 - 8.21 (m, 1H), 7.26 - 6.97 (m, 2H), 6.96 (s, 1H), 6.88 (d, *J =* 7.6 Hz, 1H), 6.86 - 6.40 (m, 1H), 5.36 (dd, *J =* 5.2, 12.4 Hz, 1H), 5.30 - 5.00 (m, 1H), 4.76 (d*, J =* 16.4 Hz, 1H), 4.17 (t, *J =* 10.4 Hz, 1H), 3.83 - 3.72 (m, 2H), 3.68 (s, 3H), 3.64 - 3.56 (m, 4H), 3.48 - 3.46 (m, 2H), 3.19 (s, 2H), 2.87 (dd, *J =* 4.8, 16.4 Hz, 1H), 2.74 - 2.59 (m, 2H), 2.38 - 2.31 (m, 2H), 2.27 (d, *J =* 9.6 Hz, 2H), 2.05 - 1.90 (m, 5H), 1.84 (d, *J =* 12.8 Hz, 2H), 1.79 - 1.64 (m, 4H), 1.55 (d, *J=* 7.6 Hz, 2H), 1.36 (s, 3H), 1.18 - 1.04 (m, 2H) |
| **I-512** | ATV | AJB | 810.1 | 11.61 - 10.64 (m, 1H), 9.50 (d, *J =* 6.0 Hz, 1H), 8. 78 (d, *J =* 8.0 Hz, 1H), 8. 40 (d, *J =* 4.4 Hz, 1H), 8. 25 (d, *J* = 5.6 Hz, 1H), 7.24 (s, 1H), 7.14 - 6.95 (m, 3H), 6.45 (d, *J =* 8.0 Hz, 1H), 6.87 (d, *J =* 8.0 Hz, 1H), 5.37 (dd, *J =* 5.6, 12.8 Hz, 1H), 5.27 (s, 1H), 5.08 (s, 1H), 4.81 - 4.73 (m, 1H), 4.24 - 4.15 (m, 1H), 3.84 - 3.73 (m, 2H), 3.59 (s, 3H), 3.33 (s, 3H), 2.83 (d, *J =* 6.4 Hz, 3H), 2.75 - 2.66 (m, 1H), 2.74 - 2.57 (m, 1H), 2.07 - 1.90 (m, 7H), 1.86 - 1.53 (m, 6H), 1.15 - 1.11 (m, 2H) |
| **I-513^{b}** | AZX | AJB | 925.0 | 11.10 (s, 1H), 9.49 (d, *J =* 6.4 Hz, 1H), 8.78 (d, *J =* 7.6 Hz, 1H), 8.38 (d, *J =* 4.4 Hz, 1H), 8.25 (d, *J =* 5.6 Hz, 1H), 8.18 (s, 2H), 7.24 - 6.85 (m, 5H), 5.38 - 5.35 (m, 1H), 5.30 - 5.27 (m, 1H), 4.79 - 4.74 (m, 1H), 4.17 - 4.13 (m, 2H), 3.80 - 3.75 (m, 2H), 3.74 - 3.71 (m, 1H), 3.45 - 3.44 (m, 3H), 3.43 - 3.40 (m, 2H), 2.73 - 2.70 (m, 1H), 2.71 - 2.65 (m, 6H), 2.14 - 1.39 (m, 26H), 1.06 - 1.03 (m, 2H) |
| **I-514^{b}** | ASA | ARY | 844.1 | 11.09 (s, 1H), 9.30 (s, 1H), 8.78 (d, *J* = 8.0 Hz, 1H), 8.37 (s, 1H), 8.27 (s, 1H), 8.14 (s, 1H), 7.24 - 6.94 (m, 3H), 6.90 - 6.79 (m, 2H), 5.39 - 5.34 (m, 1H), 4.24 - 4.11 (m, 1H), 4.10 - 3.73 (m, 7H), 3.68 - 3.65 (m, 2H), 3.56 (s, 3H), 3.50 - 3.41 (m, 1H), 3.10 - 3.05 (m, 1H), 2.97 - 2.84 (m, 2H), 2.80 - 2.68 (m, 2H), 2.67 - 2.57 (m, 2H), 2.11 (m, 2H), 2.02 - 1.99 (m, 4H), 1.94 - 1.84 (m, 4H), 1.81 - 1.66 (m, 5H), 1.61 - 1.59 (m, 1H), 1.12 - 0.96 (m, 2H) |
| **I-515^{b}** | TN | AQF | 872.1 | 11.09 (s, 1H), 9.30 (m, 1H), 8.77 (d, *J =* 8.0 Hz, 1H), 8.37 (s, 1H), 8.27 (s, 1H), 8.18 (s, 1H), 7.25 - 6.97 (m, 1H), 6.96 (d, *J =* 4.8 Hz, 2H), 6.89 - 6.79 (m, 2H), 5.36 (dd, *J* = 5.6 Hz, 12.4, 1H), 4.24 - 4.12 (m, 1H), 4.09 - 3.70 (m, 8H), 3.70 - 3.61 (m, 3H), 3.57 (s, 3H), 3.01 - 2.84 (m, 3H), 2.70 - 2.66 (m, 2H), 2.16 - 1.95 (m, 8H), 1.94 - 1.74 (m, 10H), 1.67 - 1.34 (m, 4H), 1.11 - 0.94 (m, 2H) |
| **I-516** | AUM | AQF | 844.4 | 11.10 (s, 1H), 9.31 (s, 1H), 8.79 (d, *J* = 8.0 Hz, 1H), 8.38 (s, 1H), 8.28 (s, 1H), 7.27 - 6.92 (m, 3H), 6.90 - 6.77 (m, 2H), 5.43 - 5.29 (m, 1H), 4.26 - 4.13 (m, 1H), 4.05 - 3.76 (m, 7H), 3.67 (t, *J =* 5.6 Hz, 2H), 3.57 (s, 3H), 3.55 - 3.48 (m, 2H), 3.09 - 3.04 (m, 1H), 2.99 - 2.86 (m, 2H), 2.81 - 2.69 (m, 2H), 2.68 - 2.58 (m, 2H), 2.21 - 2.09 (m, 2H), 2.07 - 1.94 (m, 4H), 1.93 - 1.82 (m, 4H), 1.80 - 1.66 (m, 5H), 1.65 - 1.51 (m, 1H), 1.12 - 0.94 (m, 2H) |
| **I-517^{b}** | AQN | ARS | 917.5 | 11.09 (s, 1H), 9.50 (d, *J =* 6.0 Hz, 1H), 8.78 (d, *J =* 7.6 Hz, 1H), 8.39 (d, *J =* 4.0 Hz, 1H), 8.25 (d, *J =* 5.6 Hz, 1H), 7.27 - 7.07 (m, 3H), 6.99 - 6.94 (m, 2H), 6.91 - 6.43 (m, 4H), 5.40 - 5.32 (m, 1H), 5.30 - 5.03 (m, 1H), 4.76 (d, *J =* 17.2 Hz, 1H), 4.25 - 4.15 (m, 1H), 3.84 - 3.72 (m, 2H), 3.70 - 3.64 (m, 2H), 3.57 (s, 3H), 3.02 - 2.79 (m, 6H), 2.75 - 2.58 (m, 6H), 2.09 - 1.84 (m, 12H), 1.60 - 1.54 (m, 2H), 1.38 - 1.27 (m, 2H) |
| **I-518^{b}** | AQJ | AJB | 810.1 | 11.39 - 10.86 (m, 1H), 9.49 (d*, J =* 6.0 Hz, 1H), 8.78 (d, *J =* 7.6 Hz, 1H), 8.45 - 8.16 (m, 2H), 7.38 - 6.33 (m, 5H), 5.38 (dd, *J =* 5.2, 12.8 Hz, 1H), 5.30 - 5.02 (m, 1H), 4.77 (d, *J =* 17.6 Hz, 1H), 4.16 (s, 1H), 3.86 - 3.70 (m, 2H), 3.66 - 3.41 (m, 5H), 2.94 - 2.82 (m, 1H), 2.76 - 2.53 (m, 7H), 2.40 (d, *J =* 6.4 Hz, 2H), 2.08 - 1.86 (m, 7H), 1.71 (t, *J =* 6.8 Hz, 4H), 1.51 - 1.38 (m, 1H), 1.17 - 1.00 (m, 2H) |
| **I-519^{b}** | AXZ | AXY | 915.5 | 11.15 (s, 1H), 9.50 (d, *J =* 6.4 Hz, 1H), 8.78 (d, *J =* 7.6 Hz, 1H), 8.55 (d, *J =* 8.0 Hz, 1H), 8.44 (d, *J =* 4.0 Hz, 1H), 8.38 (d, *J =* 4.0 Hz, 1H), 8.25 (d, *J =* 5.6 Hz, 1H), 7.53 - 7.42 (m, 2H), 7.32 - 7.27 (m, 1H), 7.25-6.95 (m, 2H), 6.90 - 6.43 (m, 1H), 6.19 - 5.91 (m, 1H), 5.31 - 5.03 (m, 1H), 4.76 (d, *J =* 18.8 Hz, 1H), 4.22 - 4.12 (m, 1H), 3.92 - 3.72 (m, 3H), 3.67 - 3.56 (m, 3H), 3.46 - 3.43 (m, 2H), 3.20-3.17 (m, 2H), 3.06 - 3.02 (m, 1H), 2.90 - 2.33 (m, 2H), 2.71 (d, *J =* 14.0 Hz, 1H), 2.53 - 2.52 (m, 1H), 2.31 - 2.23 (m, 3H), 2.13 - 1.69 (m, 19H), 1.61 - 1.53 (m, 1H), 1.08 - 0.96 (m, 2H) |
| **I-520** | AQN | ATS | 903.5 | 11.10 (s, 1H), 9.50 (d, *J =* 5.6 Hz, 1H), 8.79 (d, *J =* 7.6 Hz, 1H), 8.40 (d, *J =* 4.0 Hz, 1H), 8.31 - 8.22 (m, 1H), 7.31 -7.01 (m, 3H), 7.01 - 6.94 (m, 2H), 6.91 - 6.85 (m, 3H), 6.85 (s, 1H), 5.38 (dd, *J =* 5.6, 12.4 Hz, 1H), 5.30 - 5.02 (m, 1H), 4.77 (d, *J =* 18.4 Hz, 1H), 4.35 - 4.14 (m, 1H), 3.84 - 3.72 (m, 2H), 3.70 - 3.60 (m, 3H), 3.58 (s, 3H), 3.49 - 3.43 (m, 2H), 3.04 - 2.84 (m, 5H), 2.80 - 2.69 (m, 1H), 2.68 - 2.56 (m, 4H), 2.26 - 2.09 (m, 2H), 2.08 - 1.90 (m, 7H), 1.78 - 1.64 (m, 3H), 1.46 - 1.30 (m, 2H) |
| **I-521^{b}** | TN | ASE | 884.4 | 11.10 (s, 1H), 9.42 (s, 1H), 8.82 (d, *J =* 8.0 Hz, 1H), 8.38 (s, 1H), 8.29 (s, 1H), 7.30 - 6.94 (m, 3H), 6.91 - 6.79 (m, 2H), 5.37 (dd, *J* = 5.6, 12.4 Hz, 1H), 4.46 (d, *J =* 0.8 Hz, 2H), 4.29 - 4.07 (m, 2H), 3.58 (s, 3H), 3.46 (t, *J =* 6.4 Hz, 2H), 3.30 - 3.16 (m, 4H), 3.00 - 2.85 (m, 3H), 2.74 - 2.69 (m, 1H), 2.67 - 2.60 (m, 2H), 2.22 - 1.95 (m, 7H), 1.95 - 1.65 (m, 13H), 1.63 - 1.41 (m, 3H), 1.14 - 0.93 (m, 2H). LC-MS (ESI⁺) m/z 884.4 (M+H)⁺. |
| **I-522** | APT | AQF | 868.6 | 11.13 (s, 1H), 9.30 (s, 1H), 8.78 (d, *J* = 8.0 Hz, 1H), 8.38 (s, 1H), 8.27 (s, 1H), 7.24 - 6.96 (m, 4H), 6.83 (d, *J =* 8.0 Hz, 1H), 5.40 (dd, *J =* 5.6, 12.4 Hz, 1H), 4.48 (s, 2H), 4.24 - 4.12 (m, 1H), 4.06 - 3.82 (m, 4H), 3.80 - 3.75 (m, 2H), 3.68 - 3.65 (m, 2H), 3.64 (s, 3H), 3.61 - 3.53 (m, 1H), 2.93 - 2.84 (m, 1H), 2.78 - 2.68 (m, 3H), 2.65 - 2.59 (m, 1H), 2.19 - 2.09 (m, 3H), 2.08 - 1.98 (m, 4H), 1.93 - 1.85 (m, 6H), 1.78 - 1.67 (m, 2H), 1.62 - 1.44 (m, 3H), 1.12 - 0.97 (m, 2H) |
| **I-523^{b}** | AQI | AQF | 852.5 | 11.11 (s, 1H), 9.30 (s, 1H), 8.78 (d, *J =* 8.0 Hz, 1H), 8.38 (s, 1H), 8.27 (s, 1H), 7.25 - 6.95 (m, 4H), 6.83 (d, *J* = 8.0 Hz, 1H), 5.38 (dd, *J =* 5.6, 12.8 Hz, 1H), 4.24 - 4.10 (m, 1H), 4.08 - 3.81 (m, 4H), 3.80 - 3.75 (m, 2H), 3.69 - 3.65 (m, 2H), 3.63 (s, 3H), 2.92 - 2.83 (m, 3H), 2.76 - 2.68 (m, 1H), 2.65 - 2.59 (m, 1H), 2.45 (d, *J =* 6.4 Hz, 2H), 2.13 (d, *J =* 7.2 Hz, 2H), 2.07 - 1.99 (m, 3H), 1.94 - 1.85 (m, 6H), 1.80 - 1.68 (m, 4H), 1.62 - 1.50 (m, 2H), 1.40 - 1.28 (m, 2H), 1.10 - 0.97 (m, 2H) |
| **I-524^{b}** | AKP | AQF | 856.5 | 11.10 (s, 1H), 9.31 (s, 1H), 8.78 (d, *J* = 8.0 Hz, 1H), 8.38 (s, 1H), 8.28 (s, 1H), 7.26 - 6.92 (m, 3H), 6.90 - 6.80 (m, 2H), 5.41 - 5.33 (m, 1H), 4.27 - 4.12 (m, 1H), 4.08 - 3.84 (m, 4H), 3.81 - 3.76 (m, 2H), 3.69 - 3.65 (m, 2H), 3.55 (s, 3H), 2.91 - 2.78 (m, 5H), 2.77 - 2.68 (m, 1H), 2.66 - 2.57 (m, 1H), 2.15 - 2.08 (m, 2H), 2.07 - 1.98 (m, 3H), 1.95 - 1.82 (m, 6H), 1.79 - 1.69 (m, 2H), 1.67 - 1.54 (m, 5H), 1.39 - 1.29 (m, 2H), 1.28 - 1.20 (m, 1H), 1.19 - 1.10 (m, 2H), 1.07 - 0.95 (m, 2H) |
| **I-525^{b}** | AKP | AQD | 870.6 | 11.11 (s, 1H), 9.30 (s, 1H), 8.81 (d, *J =* 8.0 Hz, 1H), 8.39 (s, 1H), 8.31 - 8.25 (m, 1H), 7.30 - 7.00 (m, 1H), 6.99 - 6.91 (m, 3H), 6.90 - 6.83 (m, 1H), 5.37 (dd, *J =* 5.6, 12.4 Hz, 1H), 4.23 - 4.13 (m, 1H), 3.83 - 3.75 (m, 5H), 3.67 (s, 3H), 3.55 (s, 3H), 2.93 - 2.83 (m, 5H), 2.76 - 2.62 (m, 2H), 2.19 (d, *J =* 6.8 Hz, 2H), 2.07 - 1.85 (m, 7H), 1.77 - 1.56 (m, 7H), 1.36 - 1.29 (m, 2H), 1.20 (s, 6H), 1.19 - 1.16 (m, 1H), 1.10 - 1.04 (m, 2H) |
| **I-526^{b}** | ARJ | AJB | 868.4 | 11.08 (s, 1H), 9.50 (d, *J =* 6.4 Hz, 1H), 8.78 (d, *J =* 7.6 Hz, 1H), 8.38 (d, *J =* 4.4 Hz, 1H), 8.26 (d, *J =* 5.6 Hz, 1H), 7.27 - 6.96 (m, 1H), 6.95 - 6.90 (m, 1H), 6.87 - 6.43 (m, 3H), 5.33 (dd, *J =* 5.6, 12.8 Hz, 1H), 5.28 (s, 1H), 4.86 - 4.73 (m, 2H), 4.22 - 4.11 (m, 1H), 3.84 - 3.73 (m, 2H), 3.66 - 3.57 (m, 2H), 3.54 (s, 3H), 2.92 - 2.84 (m, 1H), 2.71 - 2.59 (m, 2H), 2.41 - 2.36 (m, 2H), 2.29 - 2.20 (m, 2H), 2.11 (d, *J =* 6.4 Hz, 2H), 2.06 - 1.96 (m, 5H), 1.94 - 1.80 (m, 5H), 1.78 - 1.53 (m, 8H), 1.08 - 0.97 (m, 2H) |
| **I-527^{b}** | APT | ASH | 882.6 | 11.12 (s, 1H), 9.30 (s, 1H), 8.79 (d, *J* = 8.0 Hz, 1H), 8.38 (s, 1H), 8.27 (s, 1H), 7.29 - 6.97 (m, 4H), 6.90 (d, *J* = 8.0 Hz, 1H), 5.46 - 5.34 (m, 1H), 4.47 (s, 2H), 4.17 (t, *J* = 11.6 Hz, 1H), 4.11 - 4.02 (m, 2H), 3.98 - 3.83 (m, 2H), 3.64 (s, 3H), 3.61 - 3.54 (m, 2H), 3.53 - 3.46 (m, 2H), 2.91 - 2.84 (m, 1H), 2.77 - 2.70 (m, 2H), 2.65 - 2.58 (m, 1H), 2.23 - 2.08 (m, 4H), 2.07 - 1.99 (m, 3H), 1.96 - 1.83 (m, 4H), 1.81 - 1.67 (m, 2H), 1.64 - 1.45 (m, 3H), 1.17 (d, *J =* 6.4 Hz, 6H), 1.10 - 0.97 (m, 2H) |
| **I-528^{b}** | AQI | ASH | 891.6 | 11.11 (s, 1H), 9.30 (s, 1H), 8.79 (d, *J =* 7.6 Hz, 1H), 8.38 (s, 1H), 8.27 (s, 1H), 7.29 - 6.95 (m, 4H), 6.90 (d, *J =* 8.0 Hz, 1H), 5.38 (dd, *J* = 5.2*,* 12.4 Hz, 1H), 4.24 - 4.12 (m, 1H), 4.10 - 4.01 (m, 2H), 3.99 - 3.82 (m, 2H), 3.63 (s, 3H), 3.53 - 3.46 (m, 2H), 2.97 - 2.90 (m, 2H), 2.90 - 2.82 (m, 1H), 2.75 - 2.68 (m, 1H), 2.65 - 2.58 (m, 1H), 2.47 - 2.44 (m, 2H), 2.28 - 2.16 (m, 2H), 2.08 - 1.97 (m, 5H), 1.88 (d, *J =* 11.6 Hz, 2H), 1.83 - 1.67 (m, 4H), 1.66 - 1.51 (m, 2H), 1.43 - 1.30 (m, 2H), 1.17 (d, *J =* 6.4 Hz, 6H), 1.11 - 0.98 (m, 2H) |
| **I-529** | AQI | ASH | 866.5 | 11.11 (s, 1H), 9.31 (s, 1H), 8.80 (d, *J =* 8.0 Hz, 1H), 8.40 (s, 1H), 8.27 (s, 1H), 8.14 (s, 1H), 7.29 - 6.97 (m, 4H), 6.91 (d, *J =* 8.0 Hz, 1H), 5.38 (dd, *J =* 5.2, 12.8 Hz, 1H), 4.26 - 4.14 (m, 1H), 4.12 - 4.02 (m, 2H), 4.00 - 3.79 (m, 2H), 3.64 (s, 3H), 3.50 (dd, *J =* 6.8, 13.2 Hz, 2H), 3.20 - 3.12 (m, 2H), 2.95 - 2.82 (m, 1H), 2.77 - 2.67 (m, 1H), 2.65 - 2.59 (m, 1H), 2.54 - 2.51 (m, 2H), 2.47 - 2.21 (m, 3H), 2.17 - 1.57 (m, 12H), 1.56 - 1.41 (m, 2H), 1.17 (d*, J =* 6.4 Hz, 6H), 1.14 - 1.01 (m, 2H) |
| **I-530^{b}** | AQI | ASF | 866.6 | 11.11 (s, 1H), 9.27 (s, 1H), 8.82 (d, *J =* 8.0 Hz, 1H), 8.39 (s, 1H), 8.28 (s, 1H), 7.41 - 6.71 (m, 5H), 5.47-5.28 (m, 1H), 4.58-4.29 (m, 2H), 4.22-4.17 (m, 1H), 3.69-3.65 (m, 1H), 3.64 (s, 3H), 3.62 - 3.59 (m, 1H), 2.98 - 2.85 (m, 3H), 2.77 - 2.62 (m, 4H), 2.47-2.45 (m, 2H), 2.30-2.13 (m, 2H), 2.12 - 1.94 (m, 5H), 1.94-1.85 (m, 2H), 1.83-1.67 (m, 4H), 1.66-1.49 (m, 2H), 1.45-1.29 (m, 2H), 1.20 (s, 3H), 1.18 (s, 3H), 1.11-0.96 (m, 2H) |
| **I-531^{b}** | APT | ASF | 882.6 | 11.14 (s, 1H), 9.29 (s, 1H), 8.97 - 8.74 (m, 1H), 8.50 - 8.39 (m, 1H), 8.29 (s, 1H), 7.36 - 7.16 (m, 2H), 7.15 - 7.10 (m, 1H), 7.09 - 7.01 (m, 1H), 7.00 - 6.91 (m, 1H), 5.51 - 5.34 (m, 1H), 4.53 (s, 2H), 4.51 - 4.09 (m, 3H), 3.84 - 3.67 (m, 2H), 3.66 (s, 3H), 3.10 - 3.00 (m, 2H), 2.96 - 2.86 (m, 2H), 2.79 - 2.68 (m, 4H), 2.15 - 1.46 (m, 16H), 1.21 (s, 3H), 1.20 (s, 3H), 1.17 - 1.07 (m, 2H) |
| **I-532^{b}** | AQI | AQD | 866.5 | 11.11 (s, 1H), 9.29 (s, 1H), 8.80 (d, *J =* 7.6 Hz, 1H), 8.41 - 8.24 (m, 2H), 7.29 - 7.09 (m, 2H), 7.08 - 7.04 (m, 1H), 7.02 - 6.97 (m, 1H), 6.93 (d, *J =* 8.0 Hz, 1H), 5.38 (dd, *J* = 5.2, 12.8 Hz, 1H), 4.23 - 4.13 (m, 1H), 3.82 - 3.75 (m, 4H), 3.67 (s, 2H), 3.63 (s, 3H), 2.93 - 2.82 (m, 3H), 2.69 - 2.58 (m, 2H), 2.45 (d, *J =* 6.4 Hz, 2H), 2.14 (d, *J =* 6.4 Hz, 2H), 2.06 - 1.98 (m, 3H), 1.96 - 1.84 (m, 4H), 1.80 - 1.70 (m, 4H), 1.62 - 1.51 (m, 2H), 1.40 - 1.28 (m, 2H), 1.19 (s, 6H), 1.10 - 0.96 (m, 2H) |
| **I-533^{b}** | APT | AQD | 882.6 | 11.13 (s, 1H), 9.29 (s, 1H), 8.80 (d, *J =* 8.0 Hz, 1H), 8.39 (s, 1H), 8.28 (s, 1H), 7.29 - 7.08 (m, 3H), 7.07 - 6.99 (m, 1H), 6.93 (d, *J =* 8.0 Hz, 1H), 5.40 (dd, *J =* 5.2, 12.8 Hz, 1H), 4.50 (s, 2H), 4.26 - 4.13 (m, 1H), 3.75 - 3.70 (s, 4H), 3.67 (s, 2H), 3.64 (s, 3H), 3.48 (s, 1H), 3.00 - 2.79 (m, 3H), 2.77 - 2.70 (m, 1H), 2.67 - 2.55 (m, 2H), 2.61 (s, 1H), 2.15 - 1.85 (m, 8H), 1.84 - 1.44 (m, 6H), 1.19 (s, 6H), 1.15 - 1.00 (m, 2H) |
| **I-534^{b}** | AQI | ASE | 864.5 | 11.11 (s, 1H), 9.41 (s, 1H), 8.81 (d, *J =* 8.0 Hz, 1H), 8.38 (s, 1H), 8.28 (s, 1H), 7.25 - 6.95 (m, 4H), 6.82 (d, *J* = 8.0 Hz, 1H), 5.38 (dd, *J =* 5.2, 12.8 Hz, 1H), 4.45 (s, 2H), 4.28 - 4.11 (m, 2H), 3.64 (s, 3H), 3.28 - 3.16 (m, 4H), 3.04 (d, *J =* 6.8 Hz, 2H), 2.93 - 2.58 (m, 4H), 2.40 - 2.34 (m, 1H), 2.28 - 2.09 (m, 2H), 2.07 - 1.98 (m, 3H), 1.96 - 1.55 (m, 13H), 1.49 - 1.36 (m, 2H), 1.14 - 1.00 (m, 2H) |
| **I-535^{b}** | AKP | ASE | 868.6 | 11.09 (s, 1H), 9.41 (s, 1H), 8.80 (d, *J* = 8.0 Hz, 1H), 8.37 (s, 1H), 8.28 (s, 1H), 7.24 - 6.93 (m, 3H), 6.88 - 6.78 (m, 2H), 5.36 (dd, *J =* 5.6, 12.4 Hz, 1H), 4.44 (s, 2H), 4.18 - 4.14 (m, 2H), 3.54 (s, 3H), 3.27 - 3.18 (m, 2H), 2.92 - 2.78 (m, 5H), 2.74 - 2.60 (m, 2H), 2.13 - 1.97 (m, 5H), 1.91 - 1.54 (m, 16H), 1.35 - 1.20 (m, 3H), 1.17 - 0.96 (m, 4H) |
| **I-536** | APT | ASL | 882.1 | 11.13 (s, 1H), 9.31 (s, 1H), 8.81 (d, *J =* 8.0 Hz, 1H), 8.40 (s, 1H), 8.28 (s, 1H), 7.29 - 7.00 (m, 4H), 6.91 (d, *J =* 8.0 Hz, 1H), 5.40 (dd, *J =* 12.8, 5.2 Hz, 1H), 4.51 (s, 2H), 4.22 - 4.18 (m, 1H), 4.09 - 4.06 (m, 2H), 3.93 - 3.89 (m, 2H), 3.80 - 3.68 (m, 1H), 3.65 (s, 3H), 3.50 (dd, *J =* 13.2, 6.8 Hz, 2H), 2.96 - 2.83 (m, 2H), 2.78 - 2.63 (m, 3H), 2.52 (d, *J =* 2.0 Hz, 3H), 2.12 - 1.83 (m, 8H), 1.82 - 1.46 (m, 5H), 1.17 (d, *J =* 6.4 Hz, 6H), 1.16 - 1.12 (m, 2H) |
| **I-537^{b}** | AKP | ASH | 870.6 | 11.09 (s, 1H), 9.30 (s, 1H), 8.80 (d, *J =* 8.0 Hz, 1H), 8.38 (s, 1H), 8.27 (s, 1H), 7.31 - 6.98 (m, 1H), 6.98 - 6.84 (m, 4H), 5.36 (dd, *J =* 5.2, 12.8 Hz, 1H), 4.22 - 4.12 (m, 1H), 4.09 - 4.05 (m, 2H), 3.98 - 3.84 (m, 2H), 3.54 (s, 3H), 3.50 (d, *J =* 6.4 Hz, 2H), 2.90 - 2.77 (m, 5H), 2.76 - 2.68 (m, 1H), 2.65 - 2.57 (m, 1H), 2.11 (d, *J* = 7.2 Hz, 2H), 2.07 - 1.97 (m, 3H), 1.92 - 1.80 (m, 4H), 1.78 - 1.68 (m, 2H), 1.67 - 1.55 (m, 5H), 1.37 - 1.28 (m, 2H), 1.27 - 1.20 (m, 1H), 1.17 (d, *J =* 6.4 Hz, 6H), 1.15 - 1.08 (m, 2H), 1.07 - 0.96 (m, 2H) |
| **I-538^{b}** | AKP | ASJ | 882.5 | 11.18 - 10.99 (m, 1H), 9.34 (s, 1H), 8.83 (d, *J =* 7.6 Hz, 1H), 8.39 (s, 1H), 8.3 (s, 1H), 7.29 - 6.84 (m, 5H), 5.39-5.35 (m, 1H), 4.23 - 4.11 (m, 1H), 3.77 (s, 4H), 3.70 - 3.61 (m, 2H), 3.55 (s, 3H), 2.92 - 2.58 (m, 8H), 2.11 - 1.96 (m, 8H), 1.92 - 1.56 (m, 13H), 1.39 - 0.96 (m, 7H) |
| **I-539^{b}** | TN | AQD | 886.5 | 11.17 - 11.00 (m, 1H), 9.30 (s, 1H), 8.81 (d, *J =* 8.0 Hz, 1H), 8.40 (s, 1H), 8.29 (s, 1H), 7.32 - 7.00 (m, 1H), 6.99 - 6.86 (m, 4H), 5.39-5.35 (m, 1H), 4.24 - 4.12 (m, 1H), 3.83 - 3.72 (m, 4H), 3.68 (s, 2H), 3.58 (s, 3H), 3.46 (t, *J =* 5.6 Hz, 2H), 3.03 - 2.82 (m, 4H), 2.78 - 2.58 (m, 5H), 2.14 - 2.03 (m, 4H), 1.95 - 1.41 (m, 13H), 1.20 (s, 6H), 1.06 - 1.02 (m, 2H) |
| **I-540** | AKP | ASF | 870.6 | 9.38 (s, 1H), 8.44 (s, 1H), 8.40 (s, 1H), 8.34 (d, *J =* 8.0 Hz, 1H), 7.02 - 6.95 (m, 1H), 6.93 - 6.63 (m, 3H), 6.44 (d, *J* = 8.0 Hz, 1H), 5.28 - 5.13 (m, 1H), 4.48 - 4.12 (m, 2H), 4.11 - 4.01 (m, 1H), 3.82 - 3.68 (m, 2H), 3.66 (s, 3H), 3.08 - 3.01 (m, 2H), 2.98 - 2.72 (m, 8H), 2.37 - 2.33 (m, 2H), 2.25 - 2.17 (m, 4H), 2.05 - 1.99 (m, 4H), 1.82 - 1.78 (m, 2H), 1.72 - 1.67 (m, 3H), 1.46 - 1.34 (m, 5H), 1.33 (s, 3H), 1.32 (s, 3H), 1.18 - 1.09 (m, 2H) |
| **I-541** | APT | ASJ | 894.6 | 11.14 (s, 1H), 9.35 (s, 1H), 8.84 (d, *J =* 8.0 Hz, 1H), 8.39 (s, 1H), 8.30 (s, 1H), 7.31 - 6.99 (m, 5H), 5.51 - 5.30 (m, 1H), 4.49 (s, 2H), 4.27 - 4.09 (m, 1H), 3.77 (s, 4H), 3.69 - 3.55 (m, 6H), 2.96 - 2.72 (m, 3H), 2.69 - 2.59 (m, 2H), 2.29 - 2.12 (m, 3H), 2.10 - 1.85 (m, 12H), 1.81 - 1.69 (m, 4H), 1.65 - 1.48 (m, 3H), 1.14 - 0.98 (m, 2H) |
| **I-542^{b}** | TN | ASJ | 898.1 | 11.21 - 10.93 (m, 1H), 9.34 (s, 1H), 8.81 (d, *J =* 8.0 Hz, 1H), 8.38 (s, 1H), 8.29 (s, 1H), 7.27 - 7.03 (m, 1H), 7.01 (d, *J =* 1.2 Hz, 1H), 6.96 (d, *J =* 4.8 Hz, 2H), 6.87 (m, 1H), 5.36 (dd, *J* = 5.2, 12.4 Hz, 1H), 4.20 - 4.13 (m, 1H), 3.79 - 3.72 (m, 5H), 3.67 - 3.61 (m, 3H), 3.57 (s, 4H), 3.51 (s, 1H), 3.45 (s, 2H), 2.99 - 2.92 (m, 3H), 2.92 - 2.83 (m, 2H), 2.70 - 2.66 (m, 2H), 2.09 (d, *J =* 6.8 Hz, 2H), 1.96 (m, 3H), 1.92 - 1.69 (m, 13H), 1.59 - 1.51 (m, 1H), 1.48 - 1.40 (m, 2H), 1.06 - 0.97 (m, 2H) |
| **I-543^{b}** | AKP | ASL | 870.5 | 11.09 (s, 1H), 9.30 (s, 1H), 8.80 (d, *J* = 8.0 Hz, 1H), 8.38 (s, 1H), 8.27 (s, 1H), 7.29 - 6.99 (m, 1H), 6.95 (d, *J* =5.2 Hz, 2H), 6.91 (d, *J =* 8.0 Hz, 1H), 6.89 - 6.83 (m, 1H), 5.36 (dd, *J =* 5.6, 12.4 Hz, 1H), 4.23 - 4.12 (m, 1H), 4.07 (m, 2H), 3.97 - 3.85 (m, 2H), 3.54 (s, 3H), 3.50 (d, *J =* 6.4 Hz, 2H), 2.92 - 2.77 (m, 6H), 2.67 - 2.58 (m, 2H), 2.08 (d, *J =* 6.8 Hz, 2H), 1.97 - 1.77 (m, 6H), 1.75 - 1.68 (m, 2H), 1.68 - 1.56 (m, 6H), 1.37 - 1.26 (m, 3H), 1.17 (d, *J =* 6.4 Hz, 6H), 1.08 - 0.95 (m, 3H) |
| **I-544^{b}** | AQB | AJB | 880.6 | 11.12 (s, 1H), 9.50 (d, *J =* 6.4 Hz, 1H), 8.78 (d, *J =* 8.0 Hz, 1H), 8.38 (d, *J =* 4.0 Hz, 1H), 8.25 (d, *J =* 5.6 Hz, 1H), 7.29 - 6.95 (m, 4H), 6.92 - 6.40 (m, 1H), 5.44 - 5.34 (m, 1H), 5.30 - 5.03 (m, 1H), 4.82 - 4.70 (m, 1H), 4.70 - 4.60 (m, 1H), 4.27 - 4.12 (m, 1H), 3.84 - 3.69 (m, 3H), 3.64 (s, 3H), 3.61 - 3.42 (m, 3H), 2.97 - 2.78 (m, 3H), 2.76 - 2.68 (m, 1H), 2.65 - 2.58 (m, 1H), 2.11 - 1.82 (m, 11H), 1.81 - 1.50 (m, 6H), 1.44 (d, *J =* 6.4 Hz, 3H), 1.14 - 1.01 (m, 2H) |
| **I-545^{b}** | AQC | AJB | 884.6 | 11.09 (s, 1H), 9.50 (d, *J =* 6.0 Hz, 1H), 8.78 (d, *J =* 7.6 Hz, 1H), 8.38 (d, *J =* 4.4 Hz, 1H), 8.25 (d, *J =* 5.6 Hz, 1H), 7.27 - 6.43 (m, 5H), 5.41 - 5.32 (m, 1H), 5.30 - 5.05 (m, 1H), 4.83 - 4.71 (m, 1H), 4.28 - 4.14 (m, 1H), 3.83 - 3.73 (m, 2H), 3.68 - 3.60 (m, 2H), 3.58 (s, 3H), 3.48 - 3.45 (m, 2H), 3.12 - 3.03 (m, 2H), 2.96 - 2.78 (m, 3H), 2.76 - 2.69 (m, 1H), 2.65 - 2.59 (m, 1H), 2.16 - 2.06 (m, 4H), 2.05 - 1.97 (m, 4H), 1.95 - 1.85 (m, 3H), 1.84 - 1.76 (m, 2H), 1.76 - 1.64 (m, 4H), 1.61 - 1.53 (m, 1H), 1.51 - 1.41 (m, 2H), 1.12 (d*, J =* 6.0 Hz, 3H), 1.09 - 0.98 (m, 2H) |
| **I-547** | APT | ASE | 880.4 | 11.14 (s, 1H), 9.42 (s, 1H), 8.82 (d, *J =* 8.0 Hz, 1H), 8.4 (s, 1H), 8.29 (s, 1H), 8.17 (s, 1H), 7.25 (s, 1H), 7.18 (d, *J* = 7.6 Hz, 1H), 7.06 - 7.02 (m, 1H), 6.98 (s, 1H), 6.83 (d, *J* = 8.0 Hz, 1H), 5.41 (dd, *J =* 5.6, 12.8 Hz, 1H), 4.48 (s, 2H), 4.46 (s, 2H), 4.24 - 4.12 (m, 2H), 3.65 (s, 3H), 2.95 - 2.83 (m, 2H), 2.74 - 2.66 (m, 4H), 2.17 - 2.00 (m, 8H), 1.88 (d, *J =* 4.4 Hz, 7H), 1.81 - 1.71 (m, 4H), 1.70 - 1.44 (m, 4H), 1.09 - 0.99 (m, 2H) |
| **I-548^{b}** | ASD | AJB | 866.5 | 11.11 (s, 1H), 9.51 (d, *J =* 6.0 Hz, 1H), 8.79 (d, *J =* 7.6 Hz, 1H), 8.39 (d, *J =* 4.0 Hz, 1H), 8.26 (d, *J =* 5.6 Hz, 1H), 7.30 (s, 1H), 7.25 - 7.19 (m, 2H), 7.14 - 6.96 (m, 1H), 6.89 - 6.43 (m, 1H), 5.40 (dd, *J =* 5.6, 12.8 Hz, 1H), 5.31 - 5.06 (m, 1H), 4.77 (d, *J =* 17.6 Hz, 1H), 4.40 (s, 2H), 4.21 - 4.14 (m, 1H), 3.85 - 3.74 (m, 3H), 3.64 ( d, *J =* 10.0 Hz, 2H), 3.36 (s, 3H), 2.94 - 2.59 (m, 6H), 2.21 (d, *J =* 6.4 Hz, 4H), 2.07 - 1.99 (m, 4H), 1.91 - 1.87 (m, 4H), 1.80 - 1.70 (m, 2H), 1.63 - 1.48 (m, 3H), 1.05 (q, *J =* 12.0 Hz, 2H) |
| **I-549^{b}** | AQI | ASL | 866.2 | 11.12 (s, 1H), 9.31 (s, 1H), 8.81 (d, *J =* 8.0 Hz, 1H), 8.39 (s, 1H), 8.28 (s, 1H), 7.31 - 6.85 (m, 5H), 5.47 - 5.30 (m, 1H), 4.23 - 4.13 (m, 1H), 4.11 - 4.04 (m, 2H), 4.00 - 3.84 (m, 2H), 3.64 (s, 3H), 3.51-3.47 (m, 2H), 2.91 - 2.81 (m, 3H), 2.74 - 2.63 (m, 2H), 2.46 (d, *J =* 6.4 Hz, 2H), 2.13 (d, *J =* 7.2 Hz, 2H), 2.07 - 1.97 (m, 3H), 1.95 - 1.85 (m, 4H), 1.82 - 1.70 (m, 4H), 1.63 - 1.51 (m, 2H), 1.42 - 1.23 (m, 2H), 1.18 (s, 3H), 1.17 (s, 3H), 1.10 - 0.98 (m, 2H) |
| **I-551** | AVS | AJB | 898.3 | 11.09 (s, 1H), 9.50 (d, *J =* 6.0 Hz, 1H), 8.78 (d, *J =* 8.0 Hz, 1H), 8.43 - 8.33 (m, 1H), 8.26 (d, *J =* 5.6 Hz, 1H), 8.20 - 8.13 (m, 1H), 7.65 - 7.53 (m, 1H), 7.27 - 6.92 (m, 3H), 6.90 - 6.36 (m, 2H), 5.32 - 4.99 (m, 2H), 4.76 (d, *J =* 18.4 Hz, 1H), 4.22 - 4.11 (m, 1H), 3.84 - 3.72 (m, 3H), 3.68 - 3.57 (m, 5H), 2.94 - 2.84 (m, 1H), 2.65 - 2.52 (m, 3H), 2.11 - 1.94 (m, 9H), 1.89 - 1.81 (m, 2H), 1.78 - 1.61 (m, 6H), 1.58 - 1.39 (m, 3H), 1.11 (d, *J* = 6.0 Hz, 3H), 1.06 - 0.94 (m, 2H) |
| **I-552** | AUK | AJB | 880.3 | 11.10 (s, 1H), 9.50 (d, *J =* 5.6 Hz, 1H), 8.79 (d, *J =* 7.6 Hz, 1H), 8.43 - 8.36 (m, 1H), 8.27 - 8.21 (m, 2H), 7.61 - 7.54 (m, 1H), 7.27 - 6.96 (m, 3H), 6.90 - 6.44 (m, 2H), 5.32 - 5.00 (m, 2H), 4.77 (br d, *J =* 17.2 Hz, 1H), 4.23 - 4.10 (m, 1H), 3.84 - 3.73 (m, 3H), 3.66 - 3.58 (m, 3H), 3.18 - 3.13 (m, 3H), 3.02 - 2.85 (m, 6H), 2.07 - 1.92 (m, 6H), 1.90 - 1.81 (m, 4H), 1.77 - 1.61 (m, 4H), 1.41 - 1.29 (m, 3H), 1.12 - 0.93 (m, 4H) |
| **I-553^{b}** | ASC | AJB | 870.6 | 11.08 (s, 1H), 9.50 ( d, *J =* 5.6 Hz, 1H), 8.77 (d, *J* = 7.6 Hz, 1H), 8.38 (d, *J =* 4.0 Hz, 1H), 8.26 (d, *J =* 5.2 Hz, 1H), 7.25 - 7.00 (m, 2H), 7.00 - 6.90 (m, 2H), 6.86 - 6.43 (m, 1H), 5.35 ( dd, *J =* 5.2, 12.8 Hz, 1H), 5.29 - 5.05 (m, 1H), 4.76 ( d, *J =* 16.4 Hz, 1H), 4.16 ( t, *J =* 10.0 Hz, 1H), 3.65 - 3.57 (m, 3H), 3.37 ( t, *J =* 6.0 Hz, 2H), 3.31 (s, 3H), 3.26 - 3.22 (m, 1H), 2.95 - 2.83 (m, 1H), 2.74 - 2.58 (m, 6H), 2.16 - 1.93 (m, 10H), 1.89 - 1.71 (m, 8H), 1.55 (s, 1H), 1.48 - 1.44 (m, 2H), 1.07 - 0.97 (m, 2H) |
| **I-554** | ARW | AJB | 894.6 | 11.11 (s, 1H), 9.50 (d, *J =* 6.0 Hz, 1H), 8.80 (d, *J =* 7.6 Hz, 1H), 8.39 (d, *J =* 4.4 Hz, 1H), 8.26 (d, *J =* 5.6 Hz, 1H), 7.28 - 6.41 (m, 5H), 5.41 - 5.32 (m, 1H), 5.29 (s, 1H), 4.82 - 4.73 (m, 1H), 4.21 - 4.12 (m, 1H), 3.81 (s, 1H), 3.77 - 3.72 (m, 1H), 3.66 - 3.62 (m, 1H), 3.60 (s, 1H), 3.55 (s, 3H), 3.05 - 2.99 (m, 2H), 2.93 - 2.84 (m, 1H), 2.67 - 2.59 (m, 1H), 2.28 - 2.20 (m, 3H), 2.18 (s, 3H), 2.07 - 1.62 (m, 15H), 1.55 - 1.48 (m, 3H), 1.47 - 1.38 (m, 2H), 1.31 - 1.15 (m, 4H), 1.05 - 0.94 (m, 2H) |
| **I-555^{b}** | AVF | AJB | 880.4 | 11.13 (s, 1H), 9.51 (d, *J =* 6.8 Hz, 1H), 8.77 (d, *J =* 7.6 Hz, 1H), 8.38 (d, *J =* 4.4 Hz, 1H), 8.26 (d, *J =* 5.6 Hz, 1H), 7.31 - 6.94 (m, 4H), 6.90 - 6.40 (m, 1H), 5.40 (d, *J =* 12.8 Hz, 1H), 5.29 - 5.05 (m, 1H), 4.77 (d, *J =* 16.4 Hz, 1H), 4.65 (q, *J =* 6.8 Hz, 1H), 4.22 - 4.12 (m, 1H), 3.81 (s, 1H), 3.65 - 3.58 (m, 5H), 2.92 - 2.82 (m, 1H), 2.77 - 2.59 (m, 5H), 2.39 - 2.31 (m, 1H), 2.11 - 1.85 (m, 13H), 1.74 (d, *J =* 12.4 Hz, 2H), 1.58 - 1.50 (m, 2H), 1.44 (d, *J =* 6.4 Hz, 4H), 1.08 - 0.98 (m, 2H) |
| **I-556^{b}** | AQZ | AJB | 800.2 | 11.06 (s, 1H), 9.49 (d, *J =* 6.0 Hz, 1H), 8.77 (d, *J =* 7.6 Hz, 1H), 8.38 (d, *J =* 4.8 Hz, 1H), 8.25 (d, *J =* 5.6 Hz, 1H), 7.55 (d, *J =* 8.4 Hz, 1H), 7.26 - 6.91 (m, 3H), 6.89 - 6.42 (m, 2H), 5.30 - 5.06 (m, 1H), 5.03 (dd, *J =* 5.2, 12.8 Hz, 1H), 4.76 (d, *J =* 16.4 Hz, 1H), 4.19 - 4.12 (m, 1H), 3.83 - 3.79 (m, 2H), 3.64 - 3.58 (m, 2H), 3.19 (t, *J* = 6.0 Hz, 2H), 2.93 - 2.82 (m, 1H), 2.63 - 2.52 (m, 2H), 2.36 - 2.17 (m, 4H), 2.10 (d, *J* = 6.8 Hz, 2H), 2.07 - 1.80 (m, 10H), 1.77 - 1.67 (m, 2H), 1.59 - 1.43 (m, 7H), 1.07 - 0.95 (m, 2H) |
| **I-557** | ATO | AJB | 884.4 | 11.09 (s, 1H), 9.50 (d, *J =* 6.0 Hz, 1H), 8.78 (d, *J =* 7.6 Hz, 1H), 8.38 (d, *J =* 4.4 Hz, 1H), 8.25 (d, *J =* 5.6 Hz, 1H), 7.26 - 6.98 (m, 1H), 6.96 (d, *J =* 4.8 Hz, 2H), 6.90 - 6.41 (m, 2H), 5.36 (dd, *J =* 5.6, 12.8 Hz, 1H), 5.31 - 5.03 (m, 1H), 4.77 (m, 1H), 4.22 - 4.12 (m, 1H), 3.85 - 3.71 (m, 2H), 3.64 - 36.0 (m, 2H), 3.58 (s, 3H), 3.43 (m, 2H), 3.15 - 3.01 (m, 1H), 2.93 - 2.80 (m, 2H), 2.72 - 2.64 (m, 3H), 2.15 - 1.43 (m, 21H), 1.12 (d, *J =* 6.0 Hz, 3H), 1.09 - 0.97 (m, 2H) |
| **I-558** | AUI | AJB | 884.6 | 11.09 (s, 1H), 9.50 (d, *J =* 6.4 Hz, 1H), 8.78 (d, *J =* 7.6 Hz, 1H), 8.37 (d, *J =* 4.4 Hz, 1H), 8.26 (d, *J =* 5.6 Hz, 1H), 7.25 - 6.96 (m, 3H), 6.95 - 6.91 (m, 1H), 6.88 - 6.43 (m, 1H), 5.37 (dd, *J =* 5.2, 12.8 Hz, 1H), 5.29 - 5.06 (m, 1H), 4.77 (d, *J =* 17.6 Hz, 1H), 4.21 - 4.11 (m, 1H), 3.86 - 3.77 (m, 2H), 3.76 - 3.64 (m, 3H), 3.58 (s, 4H), 3.48 - 3.43 (m, 4H), 3.12 (t, *J =* 6.8 Hz, 2H), 3.04 (d, *J* = 4.0 Hz, 1H), 2.95 - 2.83 (m, 1H), 2.77 - 2.67 (m, 2H), 2.28 (dd, *J =* 6.8, 12.8 Hz, 1H), 2.15 (dd, *J =* 6.4, 13.2 Hz, 1H), 2.04 - 1.94 (m, 4H), 1.93 - 1.86 (m, 2H), 1.80 - 1.63 (m, 4H), 1.46 - 1.32 (m, 2H), 1.02 - 0.90 (m, 8H) |
| **I-559^{b}** | AXW | AJB | 882.2 | 11.11 (s, 1H), 9.50 (d, *J* = 6.0 Hz, 1H), 8.79 (d, *J* = 8.0 Hz, 1H), 8.38 (d, *J =* 4.4 Hz, 1H), 8.26 (d, *J =* 5.6 Hz, 1H), 7.25 - 7.20 (m, 1H), 7.15 - 6.95 (m, 4H), 6.92 - 6.43 (m, 1H), 6.03 (d, *J =* 15.6 Hz, 1H), 5.39 (d, *J* = 12.8 Hz, 1H), 5.30 - 5.05 (m, 1H), 4.78 (d, *J =* 16.4 Hz, 1H), 4.28 (q, *J=* 6.4 Hz, 1H), 4.17 (t, *J=* 11.2 Hz, 1H), 3.81 (s, 1H), 3.76 - 3.62 (m, 1H), 3.60 (s, 1H), 3.56 (s, 3H), 3.50 - 3.36 (m, 2H), 2.96 - 2.84 (m, 1H), 2.74 - 2.59 (m, 4H), 2.11 - 1.95 (m, 9H), 1.89 (d, *J=* 10.8 Hz, 3H), 1.80 - 1.69 (m, 3H), 1.57 - 1.39 (m, 3H), 1.26 (d, *J* = 6.4 Hz, 3H), 1.10 - 0.96 (m, 2H) |
| **I-562^{b}** | BBC | AJB | 898.0 | 11.09 (s, 1H), 9.50 (d, *J =* 6.0 Hz, 1H), 8.78 (d, *J=* 7.6 Hz, 1H), 8.38 (s, *J* = 2.8 Hz, 1H), 8.26 (m, 1H), 8.16 (s, 1H), 7.59 (m, 1H), 7.09 - 7.02 (m, 3H), 6.85 - 6.44 (m, 2H), 5.27 - 5.02 (m, 2H), 4.78 (m, 1H), 4.12 (m, 1H), 3.80 - 3.59 (m, 6H), 2.60 (m, 2H), 2.50 (m, 4H), 2.00 (m, 10H), 1.98 - 1.67 (m, 11H), 1.11 (m, 3H), 1.02 (m, 2H) |

| | | | | |
|---|---|---|---|---|
| ^{a} For Method 2, when the amine is the HCl salt, TEA was added to free base the salt, followed by HOAc to adjust the pH to 3-4. KOAc could also be used in place of the TEA/HOAc combination. Step 2 deprotection could also be achieved under a variety of standard conditions, including with HCl in Dioxane with DCM as the solvent at rt. Steps 1 and 2 were run anywhere from 0.5-48 hrs The final products were isolated under standard purification techniques including reverse HPLC, silica gel chromatography, and prep-TLC with appropriate solvent conditions. ^{b} No deprotection Step 2 required. ^{c} ZnBr₂ in DCM was used in Step 2 for the deprotection. ^{d}Step 2 deprotection was achieved using HBr/HOAc in DCM at rt for 16 h. ^{e} A ketone was used in place of an aldehyde for the coupling in Step 1. ^{f} Step 2 deprotection was achieved using HBr/HOAc in DCM at rt for 100 h. ^{g} Step 2 deprotection was achieved using HBr/HOAc in DCM in DCM at rt for 2 h. ^{h} TEA in THF was added to the amine, then the aldehyde and tetraethoxytitanium were added and the reaction was stirred at 80 C for 2 hrs. Then the mixture was cooled to rt and NaBH4 was added and the mixture was stirred for 2 h at rt. No deprotection Step 2 required. ⁱ Tetraethoxytitanium and optionally TEA was used for the coupling, which was run at 80 C for 12-16 hrs. Then NaBH₄ was added and the reaction was stirred at 25 C for 1-2 h. No deprotection Step 2 required. ^{j} The coupling of the amine and aldehyde was achieved by first free basing the amine with TEA at rt for 30 min. Next the aldehyde and tetraethoxytitanium was added into the mixture and the reaction was heated at 80 °C for 12 hours. After cooled to 25 °C, NaBH3CN (75.6 mg, 1.20 mmol) was added and the reaction mixture was stirred at 25 °C for 0.5 hour. | | | | |

### Examples 3. Synthesis of N-(3-carbamoyl-1-((1r,4r)-4-((2-(2-(2-((2-(2,6-dioxo piperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide (I-13) and N-(3-carbamoyl-1-((1s,4s)-4-((2-(2-(2-((2- (2,6-dioxopiperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide (I-14)

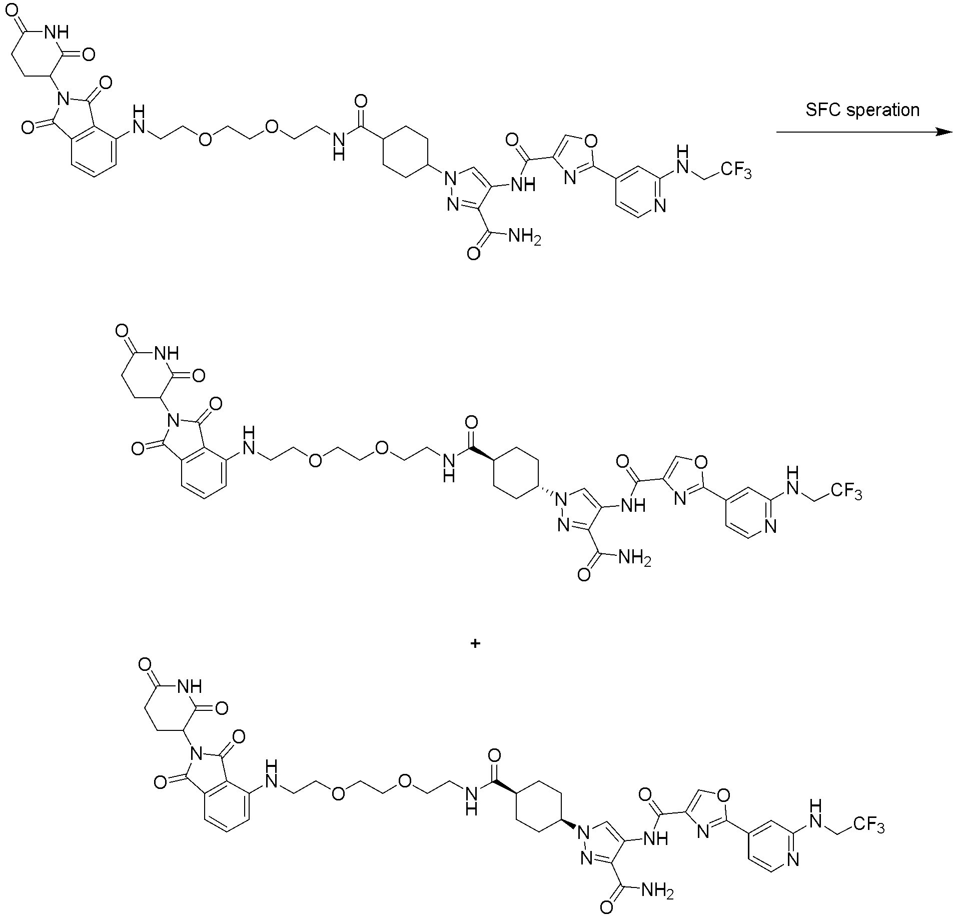

N-(3-carbamoyl-1-(4-((2-(2-(2-((2-(2,6-dioxopiperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide (13.0 mg 14.3 umol) was seperated by SFC (column: DAICEL CHIRALCEL OD(250mm*50mm,10um);mobile phase: [0.1%NH₃.H₂O MEOH]; B%: 60%-60%, 7.0 min; 130 mi) to give the title compound N-(3-carbamoyl-1-((1r,4r)-4-((2-(2-(2-((2-(2,6-dioxo piperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide (5.00 mg, 38% yield, 100% purity) as yellow solid and N-(3-carbamoyl-1-((1s,4s)-4-((2-(2-(2-((2- (2,6-dioxopiperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide (5.00 mg, 33% yield, 87% purity) as yellow solid. Absolute stereochemistry of the diastereomers was arbitrarily assigned. Characterization of N-(3-carbamoyl-1-((1r,4r)-4-((2-(2-(2-((2-(2,6-dioxo piperidin-3-yl)-1,3-dioxoisoindolin-4-yl)amino)ethoxy)ethoxy)ethyl)carbamoyl)cyclohexyl)-1H-pyrazol-4-yl)-2-(2-((2,2,2-trifluoroethyl)amino)pyridin-4-yl)oxazole-4-carboxamide: ¹H NMR (400MHz, MeOD-*d₄*) δ 8.46 (s, 1H), 8.25 (s, 1H), 8.10 - 8.08 (m, 1H), 7.42 - 7.38 (m, 1H), 7.24 - 7.21 (m, 2H), 6.90 - 6.94 (m, 2H), 4.96 - 4.94 (m,, 1H), 4.10 - 4.05 (m, 2H), 3.61 - 3.59 (m, 2H), 3.48 - 3.44 (m, 4H), 3.37 - 3.35 (m, 2H), 3.29 - 3.27 (m, 2H), 2.72 - 2.62 (m, 3H), 2.36 - 2.34 (m, 1H), 2.20 - 2.18 (m, 2H), 1.95 - 1.91 (m, 4H), 1.86 - 1.83 (m, 2H), 1.64 - 1.60 (m, 2H); LC-MS (ESI⁺) *m*/*z* 908.5 (M+H)⁺. Characterization of N-(3-carbamoyl-1-((1s,4s)-4-((2-(2-(2-((2- (2,6-dioxopiperidin-3-yl)-1,3-dioxoisoindolin-4-yl)amino)ethoxy)ethoxy)ethyl)carbamoyl)-cyclohexyl)-1H-pyrazol-4-yl)-2-(2-((2,2,2-trifluoroethyl)amino)pyridin-4-yl)oxazole-4-carboxamide: ¹H NMR (400MHz, MeOD-*d₄*) δ 8.74 (s, 1H), 8.30 (s, 1H), 8.17 - 8.15 (m, 1H), 7.72 (s, 1H), 7.64 - 7.63 (m, 1H), 7.58 - 7.54 (m, 1H), 7.09 - 7.06 (m, 2H), 5.09 - 5.05 (m,, 1H), 4.96 - 4.90 (m,, 1H),4.37 - 4.32 (m, 2H), 4.28 - 4.24 (m, 1H), 3.76 - 3.74 (m, 2H), 3.69 - 3.64 (m, 4H), 3.57 - 3.51 (m, 4H), 2.76 - 2.69 (m, 3H), 2.25 - 2.22 (m, 1H), 2.13 - 2.10 (m, 3H), 1.98 - 1.94 (m, 2H), 1.80 - 1.77 (m, 2H), 1.69 - 1.63 (m, 2H); LC-MS (ESI⁺) *m*/*z* 908.5 (M+H)⁺.

### Example 4. Synthesis of 2-[2-(Cyclopropylmethylamino)-4-pyridyl]-N-[1-[4-[[2-[2-[3-[1-(2,6-dioxo-3-piperidyl)-3- methyl-2-oxo-benzimidazol-4-yl]propoxy]ethoxy]ethyl-methyl-amino]methyl]cyclohexyl]-3-isopropyl-pyrazol-4-yl]oxazole-4-carboxamide (I-54)

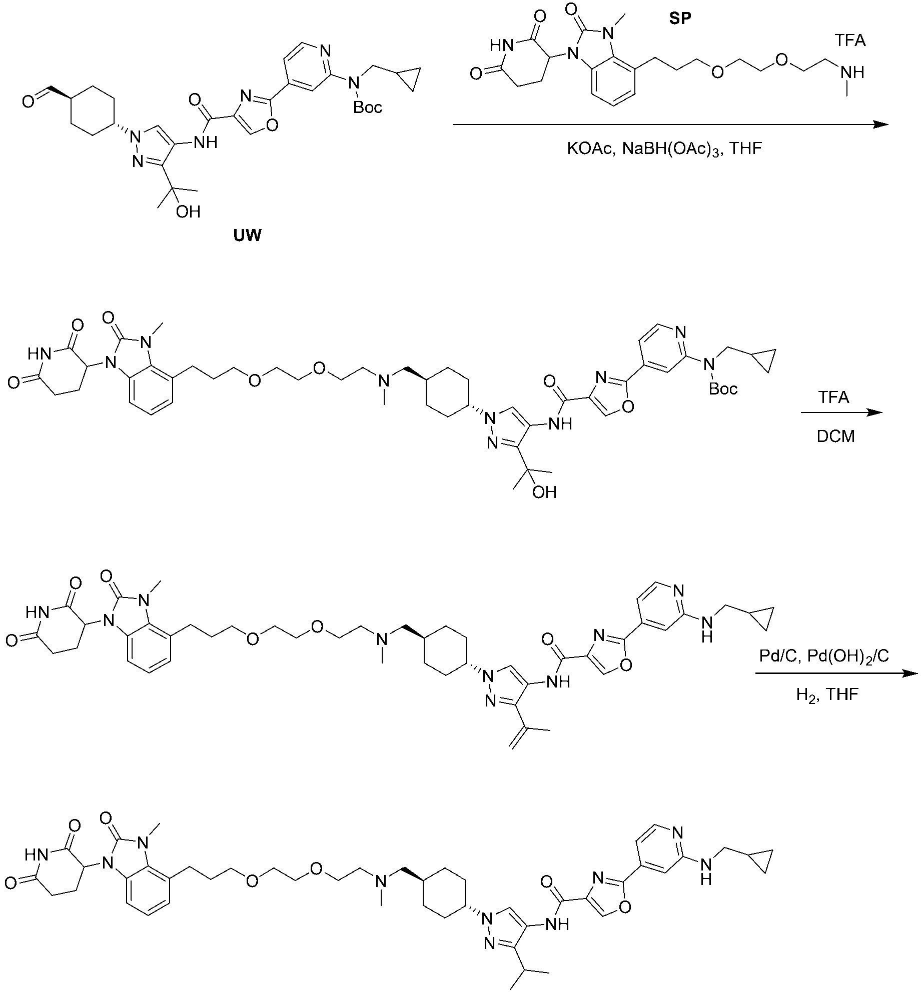

### Step 1 - Tert-butyl N-(cyclopropylmethyl)-N-[4-[4-[[1-[4-[[2-[2-[3-[1-(2,6-dioxo-3-piperidyl)-3- methyl-2-oxo-benzimidazol-4-yl]propoxy]ethoxy]ethyl-methyl-amino]methyl]cyclohexyl]-3-(1-hydroxy-1-methyl-ethyl)pyrazol-4-yl]carbamoyl]oxazol-2-yl]-2-pyridyl]carbamate

To a mixture of 3-[3-methyl-4-[3-[2-[2-(methylamino)ethoxy]ethoxy]propyl]-2-oxo-benzimidazol-1- yl]piperidine-2,6-dione (49.4 mg, 92.8 umol, TFA salt, Intermediate SP) in THF (10 mL) was added KOAc (57.9 mg, 590 umol). The mixture was stirred at 25 °C for 20 minutes. Then tert-butyl N-(cyclopropylmethyl)-N-[4-[4-[[1-(4-formylcyclohexyl)-3-(1-hydroxy-1-methyl-ethyl)pyrazol-4-yl]carbamoyl]oxazol-2-yl]-2-pyridyl]carbamate (70.0 mg, 118 umol, Intermediate UW) and NaBH(OAc)₃ (50.0 mg, 236 umol) was added into the mixture. The reaction mixture was stirred at 25 °C for 12 hours. On completion, the reaction was quenched by water (2 mL) and concentrated *in vacuo* to give a residue. The residue was purified by reversed-phase HPLC (0.1% FA condition) to give the title compound (75.0 mg, 61% yield) as a white solid. LC-MS (ESI⁺) *m*/*z* 877.0 (M-100-17)⁺.

### Step 2 - 2-[2-(Cyclopropylmethylamino)-4-pyridyl]-N-[1-[4-[[2-[2-[3-[1-(2,6-dioxo-3-piperidyl)-3- methyl-2-oxo-benzimidazol-4-yl]propoxy]ethoxy]ethy]-methyl-amino]methyl]cyclohexyl]-3-isopropenyl-pyrazol-4-yl]oxazole-4-carboxamide

To a mixture of tert-butyl N-(cyclopropylmethyl)-N-[4-[4-[[1-[4-[[2-[2-[3-[1-(2,6-dioxo-3-piperidyl)-3 -methyl-2-oxo-benzimidazol-4-yl]propoxy]ethoxy]ethyl-methyl-amino]methyl]cyclohexyl]-3-(1-hydroxy-1-methyl-ethyl)pyrazol-4-yl]carbamoyl]oxazol-2-yl]-2-pyridyl]carbamate (75.0 mg, 75.4 umol) in DCM (5 mL) was added TFA (17.2 mg, 150 umol). The reaction mixture was stirred at 25 °C for 1 hour. On completion, the reaction mixture was concentrated *in vacuo* to give a residue. The residue was purified by prep-HPLC (column: Phenomenex Synergi C18 150*25*10 um; mobile phase: [water (0.225% FA)-ACN] B%: 7%-37%) to give a title compound (39.8 mg, 57% yield, FA salt). ¹H NMR (400 MHz, DMSO-*d₆*) δ 11.10 (s, 1H), 9.38 (s, 1H), 8.95 - 8.77 (m, 1H), 8.18 (s, 1H), 8.14 (d, *J* = 5.6 Hz, 1H), 8.06 - 7.97 (m, 1H), 7.10 - 7.07 (m, 1H), 7.01 (d, *J* = 1.2, 5.2 Hz, 1H), 6.98 - 6.92 (m, 2H), 6.88 (d, *J=* 4.2 Hz, 1H), 5.41 - 5.30 (m, 2H), 5.20 (s, 1H), 4.12 - 4.01 (m, 1H), 3.57 - 3.49 (m, 18H), 3.20 - 3.15 (m, 3H), 3.00 - 2.82 (m, 4H), 2.75 - 2.59 (m, 3H), 2.25 - 2.15 (m, 5H), 2.08 (s, 3H), 2.05 - 1.94 (m, 3H), 1.93 - 1.78 (m, 4H), 1.75 - 1.65 (m, 2H), 1.63 - 1.46 (m, 2H), 1.10 - 0.97 (m, 2H), 0.50 - 0.39 (m, 2H), 0.26 - 0.18 (m, 2H).

### Step 3 - 2-[2-(Cyclopropylmethylamino)-4-pyridyl]-N-[1-[4-[[2-[2-[3-[1-(2,6-dioxo-3-piperidyl)-3- methyl-2-oxo-benzimidazol-4-yl]propoxy]ethoxy]ethyl-methyl-amino]methyl]cyclohexyl]-3-isopropyl-pyrazol-4-yl]oxazole-4-carboxamide

To a mixture of 2-[2-(cyclopropylmethylamino)-4-pyridyl]-N-[1-[4-[[2-[2-[3-[1-(2,6-dioxo-3- piperidyl)-3-methyl-2-oxo-benzimidazol-4-yl]propoxy]ethoxy]ethyl-methyl-amino]methyl]cyclohexyl]-3-isopropenyl-pyrazol-4-yl]oxazole-4-carboxamide (39.8 mg, 43.1 umol, FA salt) in THF (5 mL) was added Pd/C (20.0 mg, 10 wt%) and Pd(OH)₂/C (20.0 mg, 10 wt%) under N₂. The suspension was degassed under vacuum and purged with H₂ several times. The mixture was stirred at 25 °C for 2 hours under H₂ (15 psi). On completion, the reaction mixture was filtered and the filtrate was concentrated *in vacuo* to give a residue. The residue was purified by prep-HPLC (column: Phenomenex Synergi C18 150*25*10 um; mobile phase: [water (0.225%FA)-ACN]; B%: 10%-40%) to give the title compound (2.80 mg, 6% yield, FA salt) as a white solid. ¹H NMR (400 MHz, DMSO-*d₆*) δ 11.08 (s, 1H), 9.38 (s, 1H), 8.89 - 8.80 (m, 1H), 8.36 (s, 1H), 8.14 (d, *J=* 5.2 Hz, 1H), 7.90 - 7.77 (m, 1H), 7.10 (s, 1H), 7.08 - 7.00 (m, 2H), 6.96 (d, *J* = 4.4 Hz, 2H), 6.92 - 6.82 (m, 1H), 5.36 (d, *J* = 4.8, 12.4 Hz, 1H), 4.04 - 3.92 (m, 1H), 3.56 (s, 2H), 3.54 - 3.46 (m, 11H), 3.18 (s, 2H), 3.02 (d, *J* = 6.4 Hz, 2H), 2.96 (s, 2H), 2.90 - 2.81 (m, 2H), 2.18 (s, 3H), 1.99 (d, *J* = 2.8, 7.8 Hz, 3H), 1.91 - 1.78 (m, 4H), 1.72 - 1.59 (m, 2H), 1.57 - 1.43 (m, 2H), 1.21 - 1.16 (m, 6H), 1.10 - 0.95 (m, 3H), 0.49 - 0.40 (m, 2H), 0.22 (d, *J =* 4.2 Hz, 2H). LC-MS (ESI⁺) *m*/*z* 879.6 (M+H)⁺.

### Example 5. Synthesis of 2-[2-(Cyclopropylmethylamino)-4-pyridyl]-N-[3-(difluoromethyl)-1-[4-[[3-[3-[1-[(3S)-2,6- dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (I-89) and 2-[2-(cyclopropylmethylamino)-4-pyridyl]- N-[3-(difluoromethyl)-1-[4-[[3-[3-[1-[(3R)-2,6-dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (I-90)

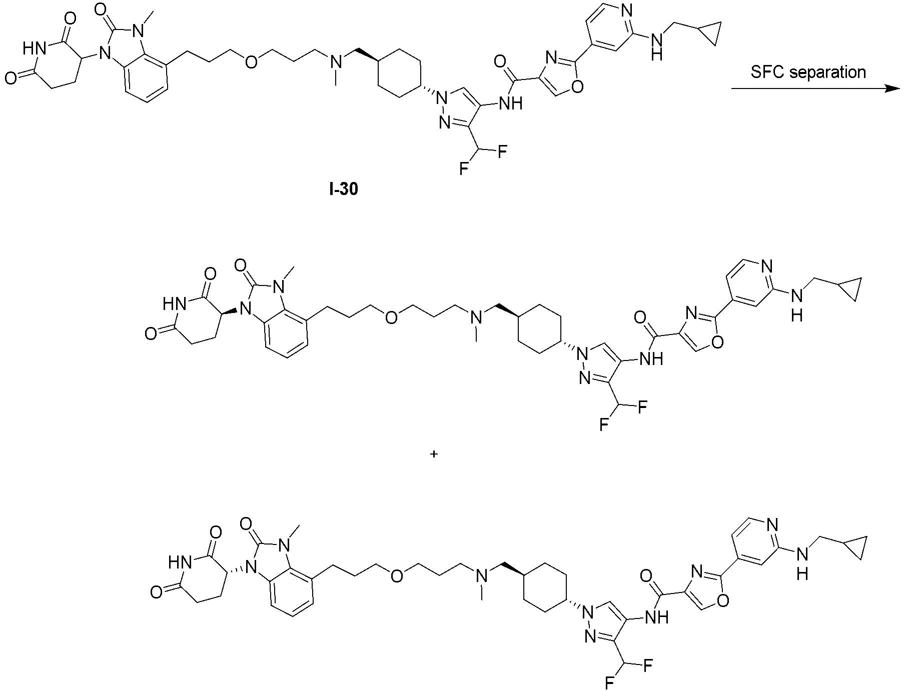

2-[2-(Cyclopropylmethylamino)-4-pyridyl]-N-[3-(difluoromethyl)-1-[4-[[3-[3-[1-(2,6-dioxo-3-piperidyl)-3-methyl-2-oxo-benzimidazol-4-yl]propoxy]propyl-methyl-amino]methyl]cyclohexyl]pyrazol-4-yl]oxazole-4-carboxamide (190 mg, 221 umol, **I-30**) was carried out on a super-fluid chromatography unit using sample preparation (add CH₃CN 25 mL into sample, Instrument: Thar 80 SFC Mobile Phase:70% IPA +ACN(0.1% DEA) in Supercritical CO₂ Flow Rate:70 g/min Cycle Time:5.0 min, total time:170 min Single injetion volume: 0.8ml Back Pressure:100 bar to keep the CO₂ in Supercritical flow). This afforded two enantiomers with retention times of 1.1 minutes: 2-[2-(Cyclopropylmethylamino)-4-pyridyl]-N-[3-(difluoromethyl)-1-[4-[[3-[3-[1-[(3S)-2,6- dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (36.4 mg, 33% yield, > 87 % ee value) as white solid; and 1.6 minutes: 2-[2-(cyclopropylmethylamino)-4-pyridyl]- N-[3-(difluoromethyl)-1-[4-[[3-[3-[1-[(3R)-2,6-dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (28.21 mg, 25% yield, > 80 % ee value) as white solid. The absolute configurations of the two enantiomers was arbitrarily assigned.

2-[2-(Cyclopropylmethylamino)-4-pyridyl]-N-[3-(difluoromethyl)-1-[4-[[3-[3-[1-[(3S)-2,6- dioxo-3-piperidyl]-3-methyl-2-oxo-benzimidazol-4-yl]propoxy]propyl-methyl-amino]methyl]cyclohexyl]pyrazol-4-yl]oxazole-4-carboxamide: ¹H NMR (400 MHz, DMSO-*d₆*) δ 11.07 (s, 1H), 9.67 (s, 1H), 8.91 (s, 1H), 8.17 (s, 1H), 8.15 (d, *J=* 5.2 Hz, 1H), 7.29 - 7.00 (m, 5H), 6.96 (d, *J* = 5.2 Hz, 1H), 6.88 - 6.86 (m, 1H), 5.38 - 5.33 (m, 1H), 4.25 - 4.13 (m, 1H), 3.57 (s, 3H), 3.42 (t, *J* = 6.0 Hz, 4H), 3.18 (t, *J* = 6.0 Hz, 2H), 2.99 - 2.93 (m, 2H), 2.93 - 2.83 (m, 1H), 2.75 - 2.67 (m, 1H), 2.65 - 2.58 (m, 1H), 2.56 - 2.52 (m, 2H), 2.14 - 2.10 (m, 4H), 2.06 - 1.97 (m, 4H), 1.77 - 1.73 (m, 1H), 1.67 - 1.63 (m, 2H), 1.36 (s, 3H), 1.23 (s, 3H), 0.87 - 0.80 (m, 3H), 0.48 - 0.42 (m, 2H), 0.25 - 0.20 (m, 2H); LC-MS (ESI⁺) *m*/*z* 857.5 (M+H)⁺.

2-[2-(cyclopropylmethylamino)-4-pyridyl]- N-[3-(difluoromethyl)-1-[4-[[3-[3-[1-[(3R)-2,6-dioxo-3-piperidyl]-3-methyl-2-oxo-benzimidazol-4-yl]propoxy]propyl-methyl-amino]methyl]cyclohexyl]pyrazol-4-yl]oxazole-4-carboxamide: ¹H NMR (400 MHz, DMSO-*d₆*) δ 11.07 (s, 1H), 9.67 (s, 1H), 8.91 (s, 1H), 8.17 (s, 1H), 8.15 (d, *J=* 5.6 Hz, 1H), 7.30 - 6.93 (m, 6H), 6.89 - 6.85 (m, 1H), 5.42 - 5.30 (m, 1H), 4.25 - 4.15 (m, 1H), 3.57 (s, 3H), 3.42 (t, *J=* 6.0 Hz, 4H), 3.18 (t, *J =* 6.0 Hz, 2H), 3.00 - 2.92 (m, 2H), 2.90 - 2.83 (m, 1H), 2.66 - 2.59 (m, 4H), 2.15 - 2.10 (m, 4H), 2.07 - 1.95 (m, 4H), 1.77 - 1.73 (m, 1H), 1.69 - 1.61 (m, 2H), 1.36 (s, 3H), 1.24 (s, 3H), 0.88 - 0.81 (m, 3H), 0.49 - 0.41 (m, 2H), 0.26 - 0.19 (m, 2H); LC-MS (ESI⁺) *m*/*z* 857.6 (M+H)⁺.

### Example 6. Synthesis of 2-[2-(Cyclopropylmethylamino)-4-pyridyl]-N-[1-[4-[[[(2R)-4-[3-[1-(2,6-dioxo-3-piperidyl) -3-methyl-2-oxo-benzimidazol-5-yl]propyl]morpholin-2-yl]methyl-methyl-amino]methyl]cyclohexyl]-3-isopropyl-pyrazol-4-yl]oxazole-4-carboxamide (I-94)

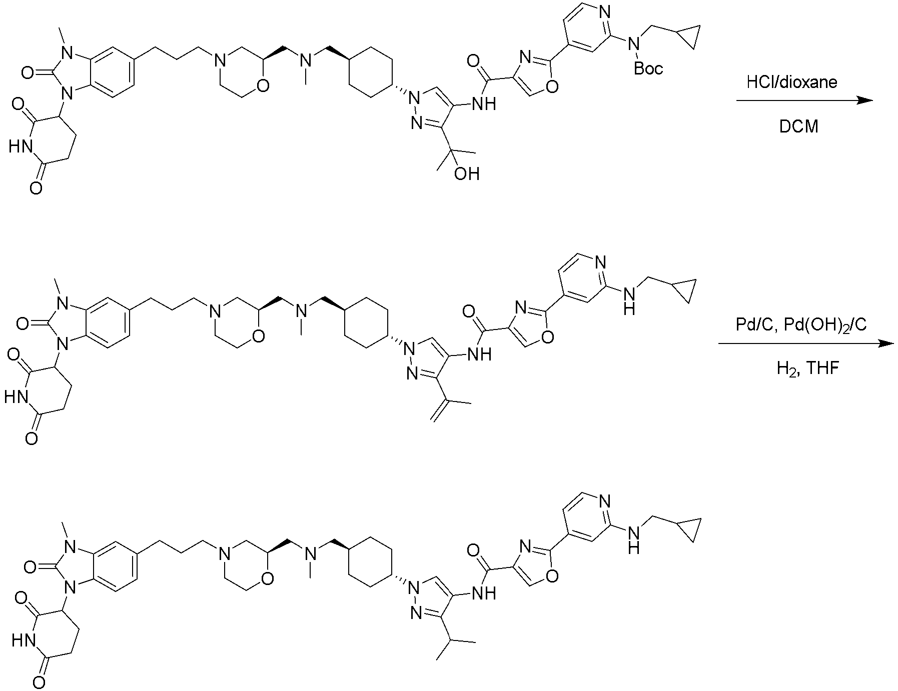

### Step 1 - 2-[2-(Cyclopropylmethylamino)-4-pyridyl]-N-[1-[4-[[[(2R)-4-[3-[1-(2,6-dioxo-3-piperidyl)-3 -methyl-2-oxo-benzimidazol-5-yl]propyl]morpholin-2-yl]methyl-methyl-amino]methyl]cyclohexyl]-3-isopropenyl-pyrazol-4-yl]oxazole-4-carboxamide

To a solution of tert-butyl N-(cyclopropylmethyl)-N-[4-[4-[[1-[4-[[[(2R)-4-[3-[1-(2,6-dioxo-3- piperidyl)-3-methyl-2-oxo-benzimidazol-5-yl]propyl]morpholin-2-yl]methyl-methyl-amino]methyl]cyclohexyl]-3-(1-hydroxy-1-methyl-ethyl)pyrazol-4-yl]carbamoyl]oxazol-2-yl]-2-pyridyl]carbamate (70.0 mg, 69.57 umol, synthesized via Step 1 of **I-33**) in DCM (4 mL) was added HCl/dioxane (4 M, 1.75 mL). The reaction mixture was stirred at 25 °C for 2 hrs. On completion, the mixture was concentrated *in vacuo* to give a residue. The residue was purified by prep-HPLC (column: Phenomenex Synergi C18 150*25*10 um; mobile phase: [water (0.225%FA)-ACN]; B%: 5%-35%, 10 min) to give the title compound (25.0 mg, 38% yield) as a white solid. LC-MS (ESI⁺) m/z 888.3 (M+H)⁺.

### Step 2 - 2-[2-(Cyclopropylmethylamino)-4-pyridyl]-N-[1-[4-[[[(2R)-4-[3-[1-(2,6-dioxo-3-piperidyl) -3-methyl-2-oxo-benzimidazol-5-yl]propyl]morpholin-2-yl]methyl-methyl-amino]methyl]cyclohexyl]-3-isopropyl-pyrazol-4-yl]oxazole-4-carboxamide

To a solution of 2-[2-(cyclopropylmethylamino)-4-pyridyl]-N-[1-[4-[[[(2R)-4-[3-[1-(2,6-dioxo-3- piperidyl)-3-methyl-2-oxo-benzimidazol-5-yl]propyl]morpholin-2-yl]methyl-methyl-amino]methyl]cyclohexyl]-3-isopropenyl-pyrazol-4-yl]oxazole-4-carboxamide (20.0 mg, 22.5 umol) in THF (1 mL) was added Pd/C (5 mg, 10 wt%) and Pd(OH)₂/C (5 mg, 10 wt%). The reaction mixture was stirred at 25 °C for 12 hrs under H₂ (15 psi). On completion, the reaction mixture was concentrated *in vacuo* to give a residue. The residue was purified by prep-HPLC (column: Phenomenex Synergi C18 150*25*10 um; mobile phase: [water (0.225%FA)-ACN]; B%: 7%-27%, 10 min) to give the title compound (3.50 mg, 16% yield) as a yellow solid. ¹H NMR (400MHz, DMSO-*d₆*) δ 11.07 (s, 1H), 9.39 (s, 1H), 8.83 (s, 1H), 8.14 (d, *J=* 5.2 Hz, 1H), 7.81 (s, 1H), 7.10 (s, 1H), 7.07 - 6.94 (m, 4H), 6.87 (d, *J=* 8.0 Hz, 1H), 5.31 (dd, *J=* 5.2, 12.8 Hz, 1H), 4.08 - 3.95 (m, 1H), 3.86 - 3.66 (m, 2H), 3.30 (s, 3H), 3.19 - 3.16 (m, 2H), 3.06 - 2.98 (m, 2H), 2.93 - 2.80 (m, 3H), 2.64 - 2.61 (m, 2H), 2.32 - 2.24 (m, 4H), 2.16 (s, 3H), 2.15 - 2.06 (m, 2H), 2.05 - 1.93 (m, 4H), 1.92 - 1.82 (m, 2H), 1.81 - 1.59 (m, 6H), 1.58 - 1.43 (m, 2H), 1.18 (d, *J* = 6.8 Hz, 6H), 1.10 - 0.95 (m, 3H), 0.49 - 0.41 (m, 2H), 0.25 - 0.18 (m, 2H); LC-MS (ESI⁺) m/z 890.6 (M+H)⁺.

### Example 7. Synthesis of 2-[2-(Cyclopropylmethylamino)-4-pyridyl]-N-[3-(difluoromethyl)-1-[4-[[[1-[4-[1- (2,6-dioxo- 3-piperidyl)-3-methyl-2-oxo-benzimidazol-4-yl]butyl]-4-piperidyl]methyl-methyl-amino]methyl]cyclohexyl]pyrazol-4-yl]oxazole-4-carboxamide (I-98)

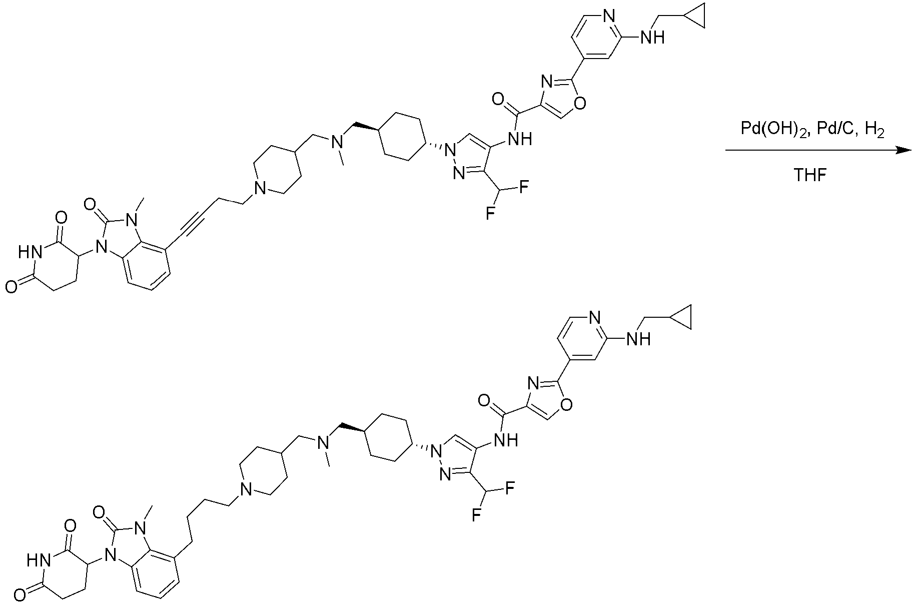

To a solution of 2-[2-(cyclopropylmethylamino)-4-pyridyl]-N-[3-(difluoromethyl)-1-[4-[[[1-[4-[1- (2,6-dioxo-3-piperidyl)-3-methyl-2-oxo-benzimidazol-4-yl]but-3-ynyl]-4-piperidyl]methyl-methyl-amino]methyl]cyclohexyl]pyrazol-4-yl]oxazole-4-carboxamide (60.0 mg, 63 umol, FA, **I-80**) in THF (20 mL) was added Pd/C (20.0 mg, 63.0 umol, 10 wt) and Pd(OH)₂/C (20.0 mg, 10 wt%). The reaction mixture was stirred at 25 °C for 24 hrs under H₂ (15 psi). On completion, the mixture was filtered and the filtrate was concentrated in *vacuo* to give a residue. The residue was purified by prep-HPLC (column: Phenomenex Synergi C18 150*30mm*4 um; mobile phase: [water (0.225%FA)-ACN]; B%: 5%-35%, 10 min) to give the title compound (24.8 mg, 40% yield) as a white solid. ¹H NMR (400 MHz, DMSO-*d₆*) δ 11.09 (s, 1H), 9.69 (s, 1H), 8.92 (s, 1H), 8.18 (s, 1H), 8.15 (d, *J* = 5.2 Hz, 1H), 7.29 - 7.00 (m, 4H), 6.98 - 6.92 (m, 2H), 6.89 - 6.84 (m, 1H), 5.36 (dd, *J* = 5.2, 12.8 Hz, 1H), 4.24 - 4.15 (m, 1H), 3.56 (s, 3H), 3.19 - 3.16 (m, 2H), 2.93 - 2.85 (m, 5H), 2.77 - 2.70 (m, 1H), 2.65 - 2.56 (m, 3H), 2.45 - 2.36 (m, 3H), 2.11 (s, 3H), 2.09 - 2.06 (m, 2H), 2.06 - 1.95 (m, 5H), 1.93 - 1.88 (m, 2H), 1.79 - 1.67 (m, 4H), 1.62 - 1.52 (m, 5H), 1.13 - 0.96 (m, 5H), 0.48 - 0.42 (m, 2H), 0.25 - 0.18 (m, 2H); LC-MS (ESI⁺) m/z 910.6 (M+1)⁺.

### Example 8. IRAK OCI-LY10 Degradation in whole blood

### Methods

Compound treatment: Compounds were reconstituted in DMSO to make the stock at concentration of 60 mM. OCI-LY10 cells were maintained in RPMI-1640 medium containing 10% FBS, 0.5 µM 2-ME or 20% FBS, 55 µM 2-ME, and 1% L-Glutamine respectively.

Cells were seeded into 6-well plates with 5e6 cells per well. 200 µL of diluted compounds were added to cells to the final concentration of 0.003 - 10 µM. After 4 or 24 hourincubation at 37 °C, cells were collected into 2 mL Eppendorf tubes and centrifuged at 1,000 rpm for 5 min. The cell pellets were washed with 1×DPBS once and resuspended in 60 µL lysis buffer. The cells were lysed on ice for 10 min, then centrifuged at 14,000 rpm for 10 min at 4 °C and the supernatants were collected for western blots. RIPA buffer (Thermo Fisher, 89900) with Halt Protease and Phophatase Inhibitor Cocktail (Thermo Fisher, 78446) was applied.

Protein concentration determination: The protein concentration of cell lysates was quantified with Pierce^{™} BCA Protein Assay Kit (Pierce, 23227). Albumin standards at different concentrations were prepared, involving 2,000 ug/mL, 1,500 ug/mL, 1,000 ug/mL, 750 ug/mL, 500 ug/mL, 250 ug/mL, 125 ug/mL, and 25 ug/mL. BCA working reagents were prepared by mixing BCA reagent A with reagent B in 50 : 1 ratio. 200 µL of the BCA working reagents were added to 25 µL of BCA standard or cell lysates in microplate, and mixed thoroughly on a plate shaker for 30 seconds. After incubation at 37 °C for 30 min, the absorbance of samples at 562 nm were measured with EnVision Plate Reader.

Western blot assay: Protein lysates were prepared in NuPAGE^{™} LDS sample buffer and NuPAGE^{™} sample reducing agent, and incubated at 95 °C for 5 min. For western blots, 20 - 25 µg of total proteins were resolved in 4 - 12% Bis-Tris gels (Introgen, WG1403A) or 10% Bis-Tris Midi gels (Invitrogen, WG1202BOX) running with 1×MOPS SDS running buffer (Invitrogen, NP0001) or 1×MES SDS running buffer (Invitrogen, NP0002). The proteins were transferred to low fluorescence PVDF membranes using the Trans-Blot Turbo Transfer System. Membranes were then blocked in Odyssey blocking buffer at RT for 1 h followed by primary incubation at 4 °C overnight. The primary antibodies were IRAK1 rabbit monoclonal antibody (CST, #4504S, 1:500), IRAK3 rabbit polyclonal antibody (CST, #4369, 1:500), IRAK4 rabbit polyclonal antibody (CST, #4363S, 1:1,000), MyD88 rabbit monoclonal antibody (Abcam, Ab133739, 1:2,000), β-actin mouse monoclonal antibody (Sigma, A5441, 10,000), and Gapdh mouse monoclonal antibody (Millipore, MAB374, 1:5,000). Membranes were washed three times with 1×TBST, and then incubated with IR Dye 800 CW Goat anti-rabbit (Licor, #926-32211) and IR Dye 700 CW Goat anti-mouse (Licor, #926-68070) secondary antibodies in 1:10,000 dilution at RT for 1 h. The western blot images were obtained using Odyssey Imaging System.

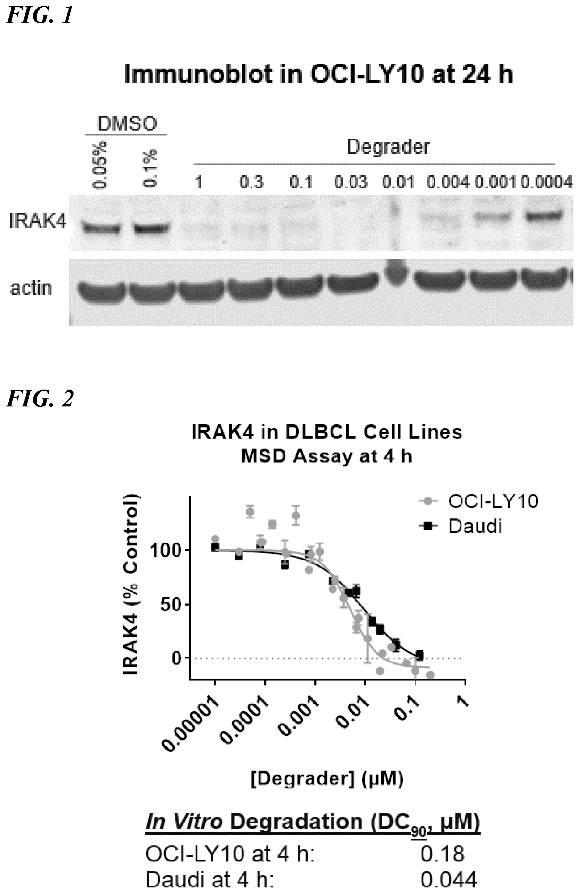

FIG. 1 shows an immunblot in OCI-LY10 at 24 h using **I-30**.

### Example 9. IRAK4 degradation in OCI-LY10 (MSD) and hPBMC (flow assay)

Degradation of IRAK4 in OCI-LY10 was quantitatively measured using Meso Scale Discovery technology. OCI-LY10 cells were seeded in 96-well plates (Corning 3799) with a density of 300,000 cells per well in 100 µL fresh media. Compounds were then added to the assay plates with a final top concentration of 1 to 10 µM in a 1:3 dilution series with total of 8 doses. The assay plates were then incubated for 4 to 24 hours at 37 °C under 5% CO2. The assay plates were then centrifuged for 5 minutes and the cell pellets were treated with 100 µL/well RIPA lysis buffer (Boston BioProducts BP-115D) with proteinase inhibitors. To prepare MSD assay plates (Meso Scale Discovery Catalog number L15XA-3), the plates were coated with 2µg/mL capture antibody (mouse Anti-IRAK4 antibody [2H9], ab119942) in PBS, at 40 µL/well. The plates were then incubated overnight at 4 °C, washed 3 times with 150 µL/well TBST buffer (Cell Signaling Technology, Catalog number 9997S) and blocked with 150 µL/well blocking buffer (Meso Scale Discovery Catalog number R93BA-4). Cell lysates were then added to MSD assay plates and the plates were incubated at room temperature for 1 hour. The plates were then washed 3 times with 150 µL/well TBST buffer and 25µL/well primary detection antibody (rabbit Anti-IRAK4 antibody [Y279], from Abcam. Catalog number ab32511, 1 µg/mL). The assay plates were then incubated at room temperature for 1 hour, washed 3 times with 150 µL/well TBST buffer and 25µL/well secondary detection antibody, SULFO-TAG anti-rabbit antibody were added (anti rabbit antibody from Meso Scale Discovery, Catalog number R32AB-1, 1 µg/mL ). The assay plates were then incubated at room temperature for 1 hour, washed 3 times with 150 µL/well TBST buffer, and 150 µL/well MSD reading buffer (Meso Scale Discovery catalog number R92TC-2) was added. The plates were then analyzed by a MSD reader (Meso Scale Discovery, Model Quick Plex SQ 120). The data was then analyzed by software Prism 7.0 from GraphPad and the dose-depended IRAK4 degradation were fit using a three-parameter logistic equation to calculate DC₅₀.

hPBMC IRAK4 degradation flow assay. Frozen PBMCs were thawed into RPMI with 10% FBS and allowed to recover. On the same day as thawed, PBMCs were plated in 96 well plate, 90uL per well. Compound plates were prepared and a 10 point, 5-fold dilution was performed with a final DMSO concentration of 0.1 %. Compound 10 µL per well was addd, sealed and incubated at 37 °C*,* 5% CO₂ for 20 hours (for 4 hour treatment, compounds were prepared and added the following day). Following the treatment incubation period (day 1), 1.6% PFA was added to PBMC plate and placed on plate shaker for 30 seconds and incubated for 10 mins at room temperature. Cells were spun down and washed two times with PBS/0.5%BSA, aspirated to pellet and placed into -80 °C freezer until further processing for flow. On the flow run day, PBMC plates were thawed and samples were transferred to PCR plates. The pre-perm staining cocktail (CD3 Ax488/CD8 BUV805/CD14 BUV395/CD16/56 BV711/CD19 BV785) was added to samples and incubated for 30 minutes at room temperature. Samples were washed two times and permeabilized with methanol for 10 minutes at 4 °C. Samples were washed two times and the post-perm staining cocktail (CD4 PE/IRAK4 Ax647 BD#560315) was added and incubated for 30 minutes at room temperature. Samples were washed two times with PBS/BSA and run on a BD LSRFortessa. Mononuclear cells are gated by SSCH/FSCH and single cells. Monocytes are then gated through CD14 positive gate and lymphocytes are gated through CD14 negative gate. To determine absolute DC50s and max degradation values, MFI values were normalized to DMSO max and 20 hour 10 µM min control. Twenty hour dose curves were calculated using a 4 parameter logistic regression curve fit, no constraints (Top doses were removed if hook effects were observed and the bottom was constrained to 0).

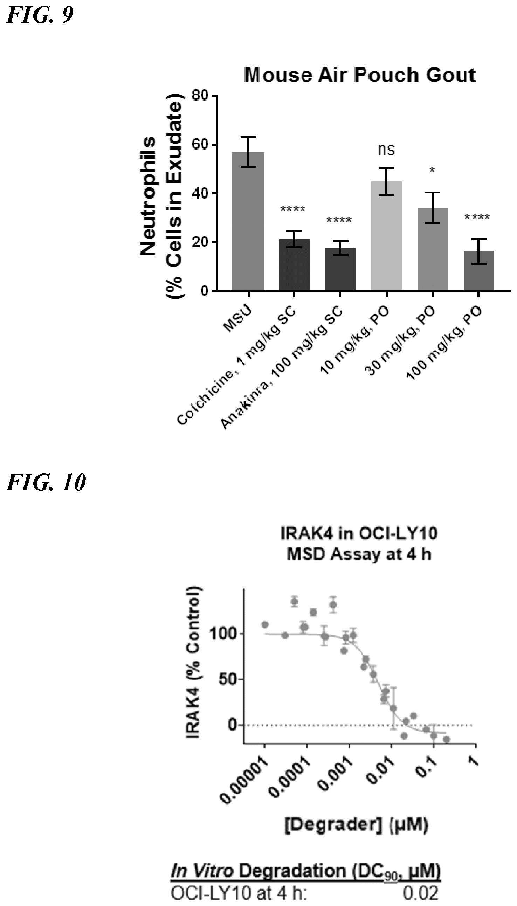

FIG. 2 and 10 show a dose response curves for **I-30** in the MSD assay for DLBCL cell lines OCI-LY10 and TMD8 at 4h with in vitro degradation results (DC₉₀, µM) depicted.

IRAK4 MSD degradation in OCI-LY10 results at 4 and 24 hrs and hPBMC at 20 hrs for compounds of the invention are presented in **Table 8.** The letter codes for IRAK4 DC₅₀ include: A (<0.01 µM), B (0.01 - 0.1 µM), C (0.1 - 1.0 µM), and D (>1.0 µM).

**Table 8. IRAK4 MSD degradation in OCI-LY10 and hPMBC Results.**

| **Compound #** | **IRAK4 MSD degradation in OCI-LY10 at 4 hrs: Average external-Abs DC₅₀ (µM)** | **IRAK4 MSD degradation in OCI-LY10 at 24 hrs: Average external-Abs DC₅₀ (µM)** | **IRAK4 degradation in hPBMC monocyte at 20hr: Average external Abs DC₅₀ (µM)** |
|---|---|---|---|
| **I-1** | A | B | - |
| **I-2** | C | - | - |
| **I-3** | A | - | - |
| **I-5** | A | - | - |
| **I-6** | A | - | - |
| **I-7** | A | - | - |
| **I-8** | B | - | - |
| **I-9** | A | - | - |
| **I-10** | A | - | - |
| **I-11** | A | - | - |
| **I-12** | B | - | - |
| **I-13** | D | - | - |
| **I-14** | B | - | - |
| **I-15** | B | B | - |
| **I-16** | A | - | - |
| **I-17** | A | - | - |
| **I-18** | A | - | - |
| **I-19** | D | D | - |
| **I-20** | D | D | - |
| **I-21** | D | D | - |
| **I-22** | D | D | - |
| **I-23** | B | A | - |
| **I-24** | D | D | - |
| **I-25** | D | D | - |
| **I-26** | D | D | - |
| **I-27** | A | - | - |
| **I-28** | A | - | - |
| **I-29** | A | - | - |
| **I-30** | A | - | - |
| **I-31** | C | - | - |
| **I-32** | A | - | - |
| **I-33** | A | - | - |
| **I-34** | A | - | - |
| **I-35** | B | - | - |
| **I-36** | B | - | - |
| **I-37** | B | - | - |
| **I-38** | A | - | - |
| **I-39** | B | - | - |
| **I-40** | B | - | - |
| **I-41** | A | - | - |
| **I-42** | B | - | - |
| **I-43** | B | - | - |
| **I-44** | D | D | - |
| **I-45** | A | - | - |
| **I-46** | B | B | - |
| **I-47** | B | B | - |
| **I-48** | A | - | - |
| **I-49** | D | - | - |
| **I-50** | D | - | - |
| **I-51** | D | - | - |
| **I-52** | D | - | - |
| **I-53** | C | B | - |
| **I-54** | C | - | - |
| **I-55** | A | - | - |
| **I-56** | C | - | - |
| **I-57** | A | - | - |
| **I-58** | A | - | - |
| **I-59** | A | - | - |
| **I-60** | B | - | - |
| **I-61** | B | - | - |
| **I-62** | A | - | - |
| **I-63** | B | - | - |
| **I-64** | C | - | - |
| **I-65** | A | - | - |
| **I-66** | A | - | - |
| **I-67** | A | - | - |
| **I-68** | B | - | - |
| **I-69** | C | - | - |
| **I-71** | C | - | - |
| **I-72** | B | - | - |
| **I-73** | A | - | - |
| **I-74** | A | - | - |
| **I-75** | A | - | - |
| **I-76** | A | - | - |
| **I-77** | B | - | - |
| **I-78** | B | - | - |
| **I-79** | B | - | - |
| **I-81** | C | - | - |
| **I-83** | C | - | - |
| **I-84** | B | - | - |
| **I-87** | A | - | - |
| **I-88** | C | - | - |
| **I-91** | C | - | - |
| **I-92** | B | - | - |
| **I-93** | B | - | - |
| **I-96** | B | - | - |
| **I-97** | A | - | - |
| **I-107** | A | - | - |
| **I-108** | A | - | - |
| **I-118** | A | - | - |
| **I-120** | A | - | - |
| **I-127** | A | A | - |
| **I-129** | A | - | - |
| **I-130** | A | - | - |
| **I-131** | D | D | - |
| **I-132** | D | D | - |
| **I-133** | D | D | - |
| **I-134** | D | D | - |
| **I-135** | C | D | - |
| **I-136** | A | - | - |
| **I-138** | D | D | - |
| **I-139** | D | D | - |
| **I-140** | D | D | - |
| **I-141** | D | D | - |
| **I-142** | D | D | - |
| **I-143** | D | D | - |
| **I-144** | D | D | - |
| **I-145** | D | D | - |
| **I-146** | D | D | - |
| **I-147** | D | D | - |
| **I-148** | C | - | - |
| **I-149** | A | - | - |
| **I-150** | B | - | - |
| **I-151** | B | - | - |
| **I-152** | B | - | - |
| **I-153** | B | - | - |
| **I-154** | B | B | - |
| **I-155** | B | - | - |
| **I-156** | B | - | - |
| **I-157** | A | - | - |
| **I-159** | D | D | - |
| **I-160** | D | D | - |
| **I-161** | D | D | - |
| **I-162** | A | - | - |
| **I-163** | A | - | - |
| **I-164** | A | - | - |
| **I-165** | B | - | - |
| **I-166** | B | - | - |
| **I-167** | C | - | - |
| **I-168** | B | A | - |
| **I-169** | A | - | - |
| **I-170** | B | - | - |
| **I-171** | B | - | - |
| **I-172** | C | - | - |
| **I-173** | C | - | - |
| **I-174** | D | D | - |
| **I-175** | D | D | - |
| **I-176** | B | - | - |
| **I-177** | A | - | - |
| **I-179** | C | - | - |
| **I-180** | B | - | - |
| **I-181** | C | - | - |
| **I-182** | C | - | - |
| **I-183** | A | A | - |
| **I-184** | B | B | - |
| **I-185** | B | B | A |
| **I-186** | B | A | - |
| **I-187** | D | A | - |
| **I-188** | C | C | - |
| **I-189** | B | A | - |
| **I-190** | C | B | |
| **I-191** | A | - | A |
| **I-192** | B | - | - |
| **I-193** | C | B | - |
| **I-194** | B | - | - |
| **I-195** | C | - | - |
| **I-196** | B | - | - |
| **I-197** | B | - | - |
| **I-198** | A | - | - |
| **I-199** | C | - | - |
| **I-200** | A | - | - |
| **I-201** | D | C | - |
| **I-202** | C | B | - |
| **I-203** | D | C | - |
| **I-204** | C | B | - |
| **I-205** | C | B | - |
| **I-206** | B | B | - |
| **I-207** | B | - | - |
| **I-208** | B | - | - |
| **I-209** | B | A | - |
| **I-210** | B | B | - |
| **I-211** | D | D | - |
| **I-212** | B | - | - |
| **I-213** | A | - | - |
| **I-214** | D | D | - |
| **I-215** | D | D | - |
| **I-216** | C | - | - |
| **I-217** | C | D | - |
| **I-218** | C | C | - |
| **I-219** | B | B | - |
| **I-220** | D | C | - |
| **I-221** | A | - | - |
| **I-222** | B | - | - |
| **I-223** | B | - | - |
| **I-224** | C | - | - |
| **I-225** | B | - | - |
| **I-226** | C | B | - |
| **I-227** | C | - | - |
| **I-228** | D | - | - |
| **I-229** | B | B | - |
| **I-230** | C | C | - |
| **I-231** | B | - | - |
| **I-232** | A | - | - |
| **I-233** | A | - | - |
| **I-234** | C | B | - |
| **I-235** | D | D | - |
| **I-236** | A | - | - |
| **I-237** | A | - | - |
| **I-238** | B | - | - |
| **I-239** | B | - | - |
| **I-240** | A | - | - |
| **I-241** | B | - | - |
| **I-242** | A | - | - |
| **I-243** | A | - | - |
| **I-244** | A | - | - |
| **I-245** | B | - | - |
| **I-246** | C | - | - |
| **I-247** | C | - | - |
| **I-248** | A | - | - |
| **I-249** | B | - | - |
| **I-250** | A | - | - |
| **I-251** | C | - | - |
| **I-252** | A | - | - |
| **I-253** | B | - | - |
| **I-254** | B | - | - |
| **I-255** | B | A | - |
| **I-256** | A | - | - |
| **I-257** | A | A | A |
| **I-258** | B | B | - |
| **I-259** | A | A | - |
| **I-260** | C | - | - |
| **I-261** | A | A | - |
| **I-262** | A | A | - |
| **I-263** | B | - | - |
| **I-264** | A | A | - |
| **I-265** | B | - | - |
| **I-266** | A | A | - |
| **I-267** | C | - | - |
| **I-268** | C | - | - |
| **I-269** | D | D | - |
| **I-270** | D | D | - |
| **I-271** | D | D | - |
| **I-272** | D | D | - |
| **I-273** | D | D | - |
| **I-274** | C | C | - |
| **I-275** | B | - | - |
| **I-276** | A | - | - |
| **I-277** | A | - | - |
| **I-278** | A | A | - |
| **I-279** | A | - | - |
| **I-280** | B | - | - |
| **I-282** | A | - | - |
| **I-283** | A | - | - |
| **I-284** | A | - | - |
| **I-285** | B | - | - |
| **I-286** | B | - | - |
| **I-287** | A | - | - |
| **I-288** | A | - | - |
| **I-291** | B | B | - |
| **I-292** | B | - | - |
| **I-293** | B | - | - |
| **I-294** | B | - | - |
| **I-296** | B | - | - |
| **I-297** | B | - | - |
| **I-298** | C | - | - |
| **I-299** | B | - | - |
| **I-300** | C | - | - |
| **I-301** | B | - | - |
| **I-302** | B | - | - |
| **I-303** | B | - | - |
| **I-304** | D | D | - |
| **I-305** | D | D | - |
| **I-306** | A | - | - |
| **I-307** | B | - | - |
| **I-308** | A | - | - |
| **I-309** | B | - | - |
| **I-310** | A | - | - |
| **I-311** | B | - | - |
| **I-312** | D | D | - |
| **I-313** | D | D | - |
| **I-314** | D | D | - |
| **I-315** | D | D | - |
| **I-316** | D | D | - |
| **I-317** | B | B | B |
| **I-320** | D | - | B |
| **I-321** | C | - | - |
| **I-322** | C | D | - |
| **I-323** | C | D | - |
| **I-324** | C | - | - |
| **I-325** | C | B | - |
| **I-326** | A | A | A |
| **I-327** | B | B | B |
| **I-328** | B | A | - |
| **I-329** | C | - | - |
| **I-330** | 0 | A | - |
| **I-331** | B | A | B |
| **I-332** | C | B | - |
| **I-333** | B | B | A |
| **I-334** | C | - | - |
| **I-335** | C | - | - |
| **I-336** | C | B | - |
| **I-337** | C | B | - |
| **I-338** | C | - | - |
| **I-339** | B | B | - |
| **I-340** | C | B | - |
| **I-341** | C | B | - |
| **I-342** | C | - | - |
| **I-343** | B | - | - |
| **I-344** | C | - | - |
| **I-345** | C | B | - |
| **I-346** | D | - | - |
| **I-347** | C | B | - |
| **I-348** | D | D | - |
| **I-349** | C | B | - |
| **I-350** | C | B | - |
| **I-351** | D | - | - |
| **I-352** | D | B | - |
| **I-353** | B | B | - |
| **I-354** | D | - | - |
| **I-355** | B | A | - |
| **I-356** | D | B | - |
| **I-357** | D | - | - |
| **I-358** | D | B | - |
| **I-359** | D | D | - |
| **I-360** | B | - | - |
| **I-361** | D | B | - |
| **I-362** | C | B | - |
| **I-363** | D | - | - |
| **I-364** | - | B | - |
| **I-365** | B | - | - |
| **I-366** | A | - | - |
| **I-367** | B | - | - |
| **I-368** | D | - | - |
| **I-369** | D | D | - |
| **I-370** | B | - | - |
| **I-371** | | B | - |
| **I-372** | D | - | - |
| **I-373** | D | B | - |
| **I-374** | D | B | - |
| **I-375** | D | - | - |
| **I-376** | B | B | A |
| **I-377** | D | A | - |
| **I-379** | D | D | - |
| **I-380** | C | B | - |
| **I-381** | B | A | - |
| **I-384** | B | - | - |
| **I-385** | D | B | - |
| **I-386** | C | B | - |
| **I-387** | C | - | - |
| **I-388** | B | - | - |
| **I-389** | B | B | - |
| **I-390** | D | - | - |
| **I-391** | D | D | - |
| **I-392** | C | B | - |
| **I-393** | C | B | - |
| **I-395** | C | - | - |
| **I-396** | B | - | B |
| **I-397** | C | C | - |
| **I-398** | B | B | - |
| **I-399** | B | A | - |
| **I-400** | A | - | - |
| **I-401** | C | D | - |
| **I-403** | D | B | - |
| **I-404** | A | - | - |
| **I-407** | A | - | A |
| **I-408** | D | D | - |
| **I-409** | D | - | - |
| **I-410** | B | - | A |
| **I-411** | D | - | B |
| **I-412** | D | D | - |
| **I-413** | C | - | A |
| **I-414** | A | - | A |
| **I-415** | A | - | A |
| **I-416** | A | - | A |
| **I-417** | A | - | A |
| **I-418** | B | - | B |
| **I-419** | B | - | A |
| **I-420** | A | - | A |
| **I-421** | A | - | A |
| **I-422** | B | - | A |
| **I-423** | C | - | A |
| **I-424** | A | - | A |
| **I-425** | B | - | A |
| **I-426** | C | - | A |
| **I-427** | - | - | C |
| **I-428** | - | - | A |
| **I-429** | - | - | A |
| **I-430** | - | - | A |
| **I-431** | - | - | A |
| **I-432** | - | - | A |
| **I-433** | - | - | A |
| **I-434** | - | - | A |
| **I-435** | - | - | A |
| **I-436** | - | - | C |
| **I-427** | - | - | C |
| **I-437** | B | B | - |
| **I-438** | C | B | - |
| **I-439** | C | D | - |
| **I-440** | C | D | - |
| **I-441** | B | B | - |
| **I-442** | C | - | C |
| **I-443** | A | A | - |
| **I-444** | C | A | A |
| **I-445** | B | - | A |
| **I-447** | B | - | A |
| **I-448** | B | - | A |
| **I-449** | A | - | A |
| **I-450** | B | - | A |
| **I-451** | B | B | - |
| **I-452** | C | D | - |
| **I-453** | B | A | - |
| **I-454** | C | B | - |
| **I-455** | B | B | - |
| **I-456** | C | C | - |
| **I-457** | C | D | - |
| **I-458** | C | D | - |
| **I-459** | C | B | - |
| **I-460** | C | B | - |
| **I-461** | C | - | - |
| **I-462** | C | D | - |
| **I-463** | B | A | - |
| **I-464** | B | - | B |
| **I-465** | A | - | A |
| **I-466** | C | - | A |
| **I-467** | A | - | A |
| **I-468** | C | - | C |
| **I-469** | A | - | A |
| **I-470** | C | - | C |
| **I-471** | A | - | A |
| **I-472** | A | - | A |
| **I-473** | B | A | - |
| **I-474** | C | B | - |
| **I-475** | B | A | - |
| **I-476** | B | B | - |
| **I-477** | B | B | - |
| **I-478** | C | B | - |
| **I-479** | C | - | C |
| **I-480** | B | - | A |
| **I-481** | C | - | C |
| **I-482** | B | - | C |
| **I-483** | C | - | A |
| **I-484** | C | - | C |
| **I-485** | B | - | A |
| **I-486** | A | - | A |
| **I-487** | B | - | A |
| **I-488** | C | - | C |
| **I-489** | C | D | - |
| **I-490** | C | D | - |
| **I-491** | C | D | - |
| **I-492** | A | - | A |
| **I-493** | A | - | A |
| **I-494** | C | - | C |
| **I-495** | C | - | B |
| **I-496** | B | - | A |
| **I-497** | B | - | A |
| **I-498** | C | - | C |
| **I-499** | A | - | A |
| **I-500** | C | B | - |
| **I-501** | D | D | - |
| **I-502** | A | A | - |
| **I-503** | D | D | - |
| **I-504** | D | B | - |
| **I-505** | B | A | - |
| **I-506** | B | A | - |
| **I-507** | - | - | A |
| **I-508** | - | - | A |
| **I-509** | B | - | B |
| **I-510** | - | - | A |
| **I-511** | B | - | A |
| **I-512** | D | - | C |
| **I-513** | A | - | A |
| **I-514** | A | - | A |
| **I-515** | A | - | A |
| **I-516** | B | - | A |
| **I-517** | B | - | A |
| **I-518** | B | - | A |
| **I-519** | B | - | C |
| **I-520** | B | - | A |
| **I-521** | B | - | A |
| **I-522** | A | - | A |
| **I-523** | A | - | A |
| **I-524** | A | - | A |
| **I-525** | B | - | A |
| **I-526** | A | - | A |
| **I-527** | B | - | A |
| **I-528** | B | - | A |
| **I-530** | B | - | A |
| **I-531** | B | - | A |
| **I-532** | B | - | B |
| **I-533** | B | - | A |
| **I-534** | B | - | A |
| **I-535** | A | - | A |
| **I-536** | B | - | A |
| **I-537** | A | - | A |
| **I-538** | B | - | A |
| **I-539** | B | - | A |
| **I-540** | B | - | A |
| **I-541** | B | - | A |
| **I-542** | B | - | A |
| **I-543** | B | - | A |
| **I-544** | B | - | C |
| **I-545** | A | - | A |
| **I-546** | C | - | B |
| **I-547** | A | - | A |
| **I-548** | C | - | C |
| **I-549** | B | - | A |
| **I-550** | B | - | A |
| **I-551** | C | - | - |
| **I-552** | C | - | - |
| **I-553** | C | - | B |
| **I-554** | B | - | A |
| **I-555** | A | - | A |
| **I-556** | B | A | - |
| **I-557** | A | - | A |
| **I-558** | - | - | A |
| **I-559** | - | - | A |
| **I-560** | A | - | - |
| **I-561** | B | - | - |

### Example 10. Proteomics Protocol

Total protein was isolated from OCI-LY10 cells treated with 10nM **I-30**, 30nM **I-30** and 30nM 2-{2-[(cyclopropylmethyl)amino]pyridin-4-yl}-N-[3-(difluoromethyl)-1-[4-({[2-(2-{3-[3-methyl-1-(1-methyl-2,6-dioxopiperidin-3-yl)-2-oxo-2,3-dihydro-1H-1,3-benzodiazol-4-yl]propoxy}ethoxy)ethyl]amino}methyl)phenyl]-1H-pyrazol-4-yl]-1,3-oxazole-4-carboxamide for 8 hours. Vehicle (DMSO)-treated cells were used as controls. Protein lysates, 2 biological replicates per condition, were prepared at 4C in 8M urea, 75mM NaCl, 1mM EDTA in 50mM Tris HCl (pH 8), 10 mM NaF, phosphatase inhibitor cocktail 2 (1:100; Sigma, P5726) and cocktail 3 (1:100; Sigma, P0044), 2 µg/mL aprotinin (Sigma, A6103), 10 µg/mL Leupeptin (Roche, 11017101001), and 1 mM PMSF (Sigma, 78830). Lysates were spun at 20,000 rcf for 10 min and supernatant (containing extracted proteins) was transferred to a clean microcentrifuge tube. Protein concentrations were determined using the Pierce BCA assay. Protein lysates were reduced with 5 mM dithiothreitol (Thermo Scientific, 20291) for 45 min at room temperature and alkylated with 10 mM iodoacetamide (Sigma, A3221) for an additional 45 min. Protein digests were diluted 1:4 with 50 mM Tris HCl (pH 8) before digestion with LysC (Wako, 100369-826) for 2 h and with trypsin (Promega, V511X) overnight. Both lysis steps were performed at a 1:50 enzyme-to-protein ratio and at room temperature. Digested samples were acidified with formic acid (FA; Fluka, 56302) to a final concentration of 1% (final pH of < 3), and then centrifuged at 2,000 rcf for 5 min to clear precipitated urea. Peptide lysates were desalted on C18 SepPak columns (Waters, 100mg/1cc) and dried down using a SpeedVac Concentrator (Savant SC210A). Desalted peptides were then labeled with tandem mass tag (TMT, Thermo Fisher Scientific) reagents according to the manufacturer's instructions. TMT labeling was quenched and TMT11-plex was combined, desalted on a C18 SepPak column (Waters, 500mg/6cc) and fractionated by high-pH reversed phase off-line chromatography into 24 fractions. Briefly, desalted TMT labelled peptides were loaded on a 4.6 mm x 250 mm column RP Zorbax 300 A Extend-C18 column (Agilent, 3.5 µm bead size), and separated on an Agilent 1100 Series HPLC instrument using basic reversed-phase chromatography. Ninety-six fractions were collected and subsequently concatenated as described earlier into 24 fractions. Each fraction was dried down and resuspended in 3 % MeCN/0.1 % FA to a peptide concentration of 1 µg/µL for LC-MS/MS analyses of the proteome. Online fractionation was performed using a nanoflow Proxeon EASY-nLC 1200 UHPLC system (Thermo Fisher Scientific) and separated peptides were analyzed on a benchtop Orbitrap Q Exactive plus mass spectrometer (Thermo Fisher Scientific). All data were analyzed using Spectrum Mill software package (Agilent Technologies). Identities interpreted for individual spectra were automatically designated as confidently assigned using the Spectrum Mill autovalidation module to use target-decoy based false discovery rate (FDR) estimates to apply score threshold at the spectral and protein levels. In total, 10,992 proteins were quantified. Downstream bioinformatic analysis was performed in Perseus software (developed by Max Planck Institute of Biochemistry, Munich, Germany)."

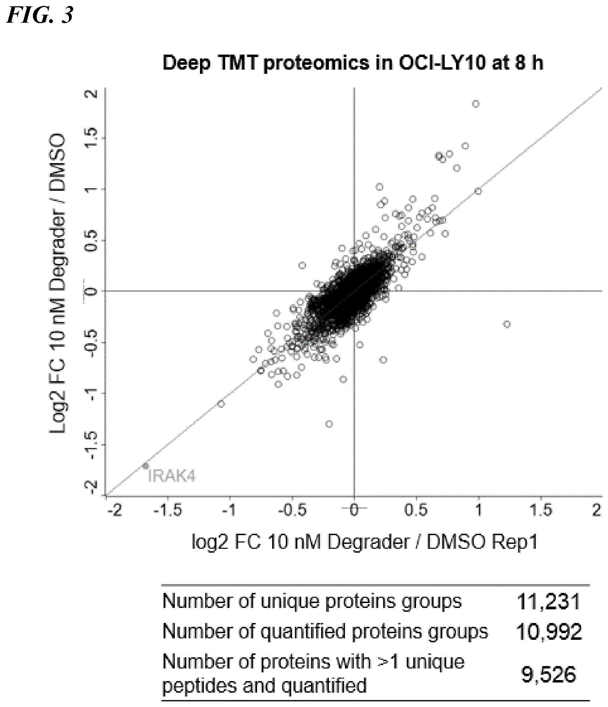

FIG. 3 shows the results of the deep tamdem mass tag (TMT) proteomics protocol.

FIGs. 1-3 and 10 show that **I-30** causes potent depletion of IRAK4 in MYD88 mutant lymphoma cell lines and is highly selective for IRAK4 vs. >10,000 other proteins in OCI-LY10.

### Example 11. OCI-LY10 flow staining experiments

OCl-LY10 cell line was cultured in IMDM medium supplemented with 20% FBS, B-ME and pen/strep. On Day 1, cells were plated at 200K per well in 96 well plates. Compounds were diluted to 1000X in DMSO, then further diluted to 100X in media, and added to cells at 4 concentrations over 4 different timepoints (24h, 48h, 72h and 96h). Timepoints were staggered so all cells were harvested and stained at the same time. Paclitaxel was added as a positive control and a DMSO control for each timepoint was also included. On day 5, plates were harvested and cells were fixed, permeabilized, and stored at -4C until further processing. On day 8, the Ki67 plates were stained using KI-67-APC antibody (Biolegend, Cat# 350514). The Caspase 3 plates were stained using the PE Active Caspase-3 Apoptosis Kit (BD Biosciences, Cat# 550914) and the IRAK 4 plates were stained with Hu IRAK4 Ab Alexa 647 Clone: L29-525 antibody (BD, Cat#560315). Cells were resuspended in staining buffer and run on a Beckman Coulter CytoFlex 13 color Cytometer (Model # A00-1-1102, Serial # AS10086).

FIG. 4 shows the results of the staining experiments using **I-30**.

### Example 12. Cell viability assay with OCI-LY10 and TMD8

Compound-mediated viability effect on OCI-LY10 or TMD8 was quantitatively determined using the CellTiter-Glo^{®} Luminescent Cell Viability Assay kit from Promega (Catalog number G7570) following manufacturer's recommended procedures. Briefly, OCI-LY10 or TMD8 cells were seeded into 384 well plates (Grenier Bio-One, Catalog number 781080) with a density of 10,000 cells per well. Compounds were then added to the assay plate with final top concentration of 10µM and 1:3 dilution series with total of 9 doses. The final DMSO concentration was normalized to 0.2%. The assay plates were incubated at 37 °C for 4 days under 5% CO₂. Then the assay plate was equilibrated at room temperature for 10 minutes. To determine cell viability, 30 µL CellTiter Glo reagent was added to each well and the assay plate was centrifuged at 1000 rpm for 30 second, incubated at room temperature for 10 min, and analyzed by detecting the luminescence using a multimode plate reader (EnVision 2105, PerkinElmer). The data was then analyzed by software Prism 7.0 from GraphPad and the dose response curves were fit using a three-parameter logistic equation to calculate IC₅₀.

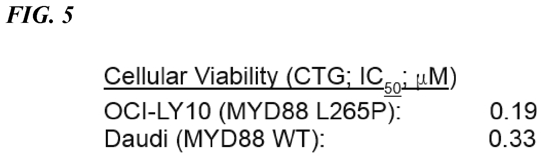

FIG. 5 depicts the cellular viability results using **I-30.** FIGs. 4 and 5 show that **I-30** treatment is cytotoxic and induces apoptosis in OCI-LY10 lymphoma line.

CTG Cell Viability Assay - OCI-LY10 results for compounds of the invention are presented in **Table 9.** The letter codes for IRAK4 IC₅₀ include: A (<0.05 µM), B (0.05 - 0.1 µM), C (0.1 - 0.5 µM), D (0.5 - 1.0 µM), and E (>1.0 µM).

**Table 9. CTG Cell Viability Assay Results.**

| **Compound #** | **CTG Cell Viability Assay - OCI-LY10: Average external-IC₅₀ (µM)** |
|---|---|
| **I-1** | B |
| **I-2** | C |
| **I-3** | E |
| **I-5** | A |
| **I-6** | E |
| **I-7** | E |
| **I-8** | E |
| **I-9** | E |
| **I**-**10** | E |
| **I-11** | E |
| **I-12** | D |
| **I-13** | E |
| I-**14** | C |
| **I-15** | E |
| **I-16** | C |
| **I-17** | C |
| **I-18** | B |
| **I-20** | E |
| **I-21** | D |
| **I-22** | C |
| **I-23** | B |
| **I-24** | E |
| **I-25** | E |
| **I-26** | E |
| **I-27** | A |
| **I-28** | A |
| **I-29** | E |
| **I-30** | C |
| **I-31** | C |
| **I-32** | C |
| **I-33** | E |
| **I-34** | E |
| **I-35** | A |
| **I-36** | D |
| **I-37** | A |
| **I-38** | A |
| **I-39** | B |
| **I-40** | A |
| **I-41** | E |
| **I-42** | E |
| **I-43** | E |
| **I-44** | E |
| **I-45** | A |
| **I-46** | D |
| **I-47** | C |
| **I-48** | A |
| **I-49** | E |
| **I-50** | E |
| **I-51** | E |
| **I-52** | C |
| **I-53** | E |
| **I-58** | B |
| **I-59** | A |
| **I-61** | C |
| **I-62** | B |
| **I-65** | A |
| **I-67** | A |
| **I-75** | A |
| **I-76** | A |
| **I-81** | E |
| **I-92** | C |
| **I-106** | E |
| **I-107** | E |
| **I-108** | E |
| **I-109** | E |
| **I-110** | E |
| **I-111** | E |
| **I-112** | E |
| **I-113** | E |
| **I-114** | C |
| **I-115** | E |
| **I-116** | E |
| **I-117** | E |
| **I-118** | E |
| **I-119** | E |
| **I-120** | E |
| **I-121** | E |
| **I-122** | E |
| **I-123** | D |
| **I-124** | E |
| **I-125** | E |
| **I-126** | E |
| **I-127** | E |
| **I-128** | E |
| **I-129** | E |
| **I-130** | E |
| **I-131** | E |
| **I-132** | E |
| **I-133** | E |
| **I-134** | E |
| **I-135** | E |
| **I-136** | E |
| **I-137** | E |
| **I-138** | E |
| **I-139** | E |
| **I-140** | E |
| **I-141** | E |
| **I-142** | E |
| **I-143** | E |
| **I-144** | E |
| **I-145** | E |
| **I-146** | E |
| **I-147** | E |
| **I-148** | C |
| **I-149** | E |
| **I-150** | A |
| **I-151** | E |
| **I-152** | E |
| **I-153** | E |
| **I-155** | E |
| **I-156** | E |
| **I-157** | E |
| **I-159** | E |
| **I-160** | E |
| **I-161** | E |
| **I-162** | E |
| **I-163** | E |
| **I-164** | E |
| **I-165** | D |
| **I-166** | E |
| **I-167** | C |
| **I-168** | A |
| **I-169** | E |
| **I-170** | A |
| **I-171** | C |
| **I-172** | E |
| **I-173** | E |
| **I-174** | E |
| **I-175** | E |
| **I-176** | E |
| **I-177** | C |
| **I-179** | E |
| **I-180** | E |
| **I-181** | C |
| **I-182** | E |
| **I-183** | A |
| **I-184** | E |
| **I-185** | E |
| **I-186** | E |
| **I-187** | E |
| **I-188** | E |
| **I-189** | E |
| **I-190** | E |
| **I-191** | E |
| **I-192** | E |
| **I-193** | E |
| **I-194** | E |
| **I-195** | E |
| **I-196** | E |
| **I-197** | E |
| **I-198** | E |
| **I-199** | E |
| **I-200** | B |
| **I-201** | E |
| **I**-**202** | E |
| **I-203** | E |
| **I-204** | E |
| **I-205** | E |
| **I-206** | E |
| **1-207** | E |
| **I-208** | A |
| **I-209** | E |
| **I-210** | E |
| **I-211** | B |
| **I-212** | E |
| **I-213** | E |
| **I-214** | E |
| **I-215** | E |
| **I-216** | E |
| **I-217** | B |
| **I-218** | E |
| **I-219** | E |
| **I-220** | E |
| **I-221** | B |
| **I-222** | C |
| **I-223** | E |
| **I-224** | E |
| **I-225** | E |
| **I-226** | A |
| **I-227** | A |
| **I-228** | E |
| **I-229** | E |
| **I-230** | E |
| **I-231** | A |
| **I-232** | A |
| **I-233** | C |
| **I-234** | E |
| **I-235** | E |
| **I-236** | E |
| **I-237** | E |
| **I-239** | E |
| **I-240** | E |
| **I-241** | D |
| **I-242** | D |
| **I-243** | E |
| **I-244** | C |
| **I-245** | A |
| **I-246** | E |
| **I-247** | E |
| **I-249** | A |
| **I-250** | A |
| **I-252** | C |
| **I-254** | C |
| **I-257** | E |
| **I-259** | E |
| **I-260** | E |
| **I-263** | A |
| **I-265** | A |
| **I-267** | E |
| **I-268** | E |
| **I-269** | E |
| **I-270** | E |
| **I-271** | E |
| **I-272** | E |
| **I-273** | E |
| **I-274** | D |
| **I-275** | E |
| **I-276** | E |
| **I-277** | E |
| **I-278** | E |
| **I-279** | E |
| **I-280** | E |
| **I-282** | E |
| **I-283** | E |
| **I-284** | E |
| **I-285** | E |
| **I-286** | E |
| **I-287** | E |
| **I-288** | E |
| **I-289** | E |
| **I-302** | E |
| **I-290** | E |
| **I-291** | A |
| **I-292** | E |
| **I-293** | E |
| **I-294** | A |
| **I-296** | E |
| **I-297** | E |
| **I-298** | C |
| **I-299** | B |
| **I-300** | D |
| **I-301** | A |
| **I-303** | E |
| **I-304** | E |
| **I-305** | E |
| **I-306** | E |
| **I-307** | E |
| **I-308** | A |
| **I-309** | A |
| **I-310** | E |
| **I-311** | E |
| **I-312** | E |
| **I-313** | E |
| **I-314** | E |
| **I-315** | E |
| **I-316** | E |
| **I-317** | E |
| **I-320** | E |
| **I-321** | E |
| **I-322** | E |
| **I-323** | E |
| **I-324** | A |
| **I-325** | E |
| **I-326** | A |
| **I-327** | D |
| **I-328** | A |
| **I-329** | E |
| **I-330** | E |
| **I-331** | A |
| **I-332** | C |
| **I-333** | C |
| **I-334** | C |
| **I-335** | D |
| **I-336** | B |
| **I-337** | B |
| **I-338** | A |
| **I-339** | B |
| **I-340** | C |
| **I-341** | E |
| **I-342** | B |
| **I-343** | A |
| **I-344** | A |
| **I-345** | A |
| **I-346** | A |
| **I-347** | A |
| **I-348** | E |
| **I-349** | A |
| **I-350** | E |
| **I-351** | E |
| **I-352** | D |
| **I-353** | C |
| **I-354** | E |
| **I-355** | C |
| **I-356** | A |
| **I-357** | E |
| **I-358** | B |
| **I-359** | A |
| **I-360** | E |
| **I-361** | D |
| **I-362** | D |
| **I-363** | E |
| **I-364** | B |
| **I-365** | A |
| **I-366** | A |
| **I-367** | C |
| **I-368** | E |
| **I-369** | C |
| **I-370** | C |
| **I-371** | B |
| **I-372** | A |
| **I-373** | A |
| **I-374** | E |
| **I-375** | D |
| **I-376** | C |
| **I-377** | A |
| **I-379** | C |
| **I-380** | E |
| **I-381** | B |
| **I-384** | A |
| **I-385** | A |
| **I-386** | A |
| **I-387** | A |
| **I-389** | A |
| **I-390** | E |
| **I-391** | E |
| **I-392** | C |
| **I-393** | B |
| **I-395** | E |
| **I-396** | B |
| **I-397** | B |
| **I-398** | B |
| **I-399** | A |
| **I-400** | A |
| **I-401** | D |
| **I-403** | B |
| **I-404** | A |
| **I-405** | E |
| **I-406** | E |
| **I-407** | E |
| **I-408** | E |
| **I-409** | E |
| **I-410** | E |
| **I-411** | E |
| **I-412** | E |
| **I-413** | E |
| **I-414** | E |
| **I-415** | E |
| **I-416** | E |
| **I-417** | E |
| **I-418** | E |
| **I-419** | E |
| **I-420** | A |
| **I-421** | E |
| **I-422** | E |
| **I-423** | E |
| **I-424** | E |
| **I-425** | E |
| **I-426** | E |
| **I-437** | C |
| **I-438** | B |
| **I-439** | A |
| **I-440** | E |
| **I-441** | E |
| **I-442** | A |
| **I-443** | A |
| **I-444** | A |
| **I-445** | E |
| **I-447** | E |
| **I-448** | E |
| **I-449** | E |
| **I-450** | E |
| **I-451** | C |
| **I-452** | E |
| **I-453** | B |
| **I-454** | B |
| **I-455** | B |
| **I-456** | A |
| **I-457** | E |
| **I-458** | E |
| **I-459** | A |
| **I-460** | B |
| **I-461** | A |
| **I-462** | A |
| **I-463** | C |
| **I-473** | A |
| **I-474** | A |
| **I-475** | A |
| **I-476** | E |
| **I-477** | D |
| **I-478** | E |
| **I-479** | E |
| **I-480** | E |
| **I-481** | E |
| **I-482** | E |
| **I-483** | E |
| **I-484** | E |
| **I-485** | E |
| **I-486** | E |
| **I-487** | E |
| **I-488** | E |
| **I-489** | A |
| **I-490** | E |
| **I-491** | E |
| **I-492** | E |
| **I-493** | E |
| **I-494** | E |
| **I-495** | E |
| **I-496** | E |
| **I-497** | E |
| **I-498** | E |
| **I-499** | E |
| **I-500** | B |
| **I-501** | B |
| **I-502** | A |
| **I-503** | A |
| **I-504** | C |
| **I-505** | E |
| **I-506** | D |
| **I-508** | E |
| **I-509** | E |
| **I-510** | E |
| **I-511** | E |
| **I-512** | E |
| **I-513** | E |
| **I-514** | E |
| **I-515** | E |
| **I-516** | E |
| **I-517** | E |
| **I-518** | E |
| **I-519** | C |
| **I-520** | E |
| **I-521** | E |
| **I-522** | C |
| **I-523** | E |
| **I-524** | E |
| **I-525** | E |
| **I-526** | E |
| **I-532** | E |
| **I-544** | E |
| **I-551** | A |
| **I-552** | A |
| **I-553** | E |
| **I-554** | E |
| **I-555** | E |
| **I-557** | E |
| **I-558** | E |
| **I-559** | E |
| **I-563** | E |

### Example 13. Evaluation of efficacy in OCI-LY10 or TMD8 large B lymphoma xenograft models in female C.B. 17 SCID mice

Cell Culture: The OCI-LY10 or TMD8 tumor cells were maintained as suspension in RPMI1640 medium supplemented with 10% fetal bovine serum, 100 U/mL penicillin and 100 µg/mL streptomycin at 37 °C in an atmosphere of 5% CO₂ in air. The tumor cells were routinely subcultured twice weekly by trypsin-EDTA treatment. The cells growing in an exponential growth phase were harvested and counted for tumor inoculation.

Animals: C.B. 17 SCID, female, 6-8 weeks, weighing approximately 16-18g were used. Animals were housed and maintained according to IACUC protocols.

Tumor Inoculation: Each mouse was inoculated subcutaneously at the right flank with OCI-LY10 or TMD8 tumor cells (10 x 10⁶) in 0.2 mL of PBS with matrigel for tumor development. The treatments were started when the tumor sizes reached approximately 150 - 450 mm³ for the studies.

Assignment to Groups: Before commencement of treatment, all animals were weighed and the tumor volumes were measured. Since the tumor volume can affect the compound PK/PD, mice are assigned into groups using an Excel-based randomization procedure performing stratified randomization based upon their tumor volumes.

Observation: After tumor inoculation, the animals were checked daily for morbidity and mortality. During routine monitoring, the animals were checked for any effects of tumor growth and treatments on behavior such as mobility, food and water consumption, body weight gain/loss, eye/hair matting and any other abnormalities. Mortality and observed clinical signs were recorded for individual animals in detail.

Data Collection: Tumor volumes were measured in two dimensions using a caliper, and the volumes were expressed in mm³ using the formula: "V = (L × W × W)/2, where V is tumor volume, L is tumor length (the longest tumor dimension) and W is tumor width (the longest tumor dimension perpendicular to L).

At termination: At pre-determined time points based on study design, animals were humanely sacrificed by CO₂. Blood was obtained by cardiac puncture for isolation of plasma, any residual tumor was removed and divided in 2 portions, 1 (minimal) for terminal compound exposure and 1 to determine IRAK4 and actin. Compound was determined in tumor and plasma using LC/MS with calibrated standards.

Interleukin-1 receptor-associated kinase 4 (IRAK4) was quantified in human OCI-LY10 and TMD8 xenograft tumors, together with mouse splenocytes and peripheral blood mononuclear cells (PBMCs), by ultra-performance liquid chromatography-tandem mass spectrometry (UPLC-MS/MS). The concentrations of IRAK4 were normalized by the concentrations of actin in the respective samples. The tumors, splenocytes and PBMCs were lysed in tissue protein extraction reagent (T-PER, ThermoFisher). The samples were centrifuged at 10,000 rpm for 10 minutes. The supernatant (cell lysate) was transferred to another tube. The cell lysates were denatured, reduced, and alkylated with iodoacetamide. The alkylated samples were treated with trypsin to generate the IRAK4 peptide LAVAIK and the actin peptide GYSFTTTAER. These peptides are unique and specific to IRAK4 and actin, respectively, in human, rat and mouse cells and tissues due to sequence conservation between these species.

Signature peptide concentrations were quantitated using a sensitive and specific targeted LC-MS/MS method. Corresponding mass-shifted, stable isotope-labeled peptides (LAV(d8)AIK and GYSF(d8)TTTAE(d6)R) were used as internal standards (ISs). Calibration standards and were prepared fresh on the day of analysis by diluting synthetic LAVAIK and GYSF(d8)TTTAER peptides into 0.1% formic acid in 90/10 water acetonitrile (v/v). The standards and study samples) were aliquoted into a 96-well plate and mixed with IS spiking solution. The sample plate was covered with heating foil.

Signature peptide levels (LAVAIK, GYSFTTTAER) were quantified by UPLC-MS/MS. Injections were made using a Shimadzu ultra performance liquid chromatograph (UPLC) platform. Mobile phase A was 0.1% formic acid in water. Mobile phase B was 0.1% formic acid in 90:10 acetonitrile/water (v/v). A SCIEX TripleTOF 6600 LC-MS/MS system was used for the detection and quantitation of analytes. The intensities of the analytes and ISs were determined by integration of extracted ion peak areas using Analyst and MultiQuant 3.0 software. Calibration curves were prepared by plotting the analyte to IS peak area ratio vs. concentration. The model for the calibration curves was linear with 1/x² weighting. The working range of the assay was 0.02-50 ng/mL for LAVAIK and 1-2500 ng/mL for GYSFTTTAER in digested cell lysate. Measured peptide levels were corrected for sample work up and converted to actual protein concentrations in ng/mg total protein of cell lysate. The concentrations of IRAK4 were normalized across samples by actin concentration.

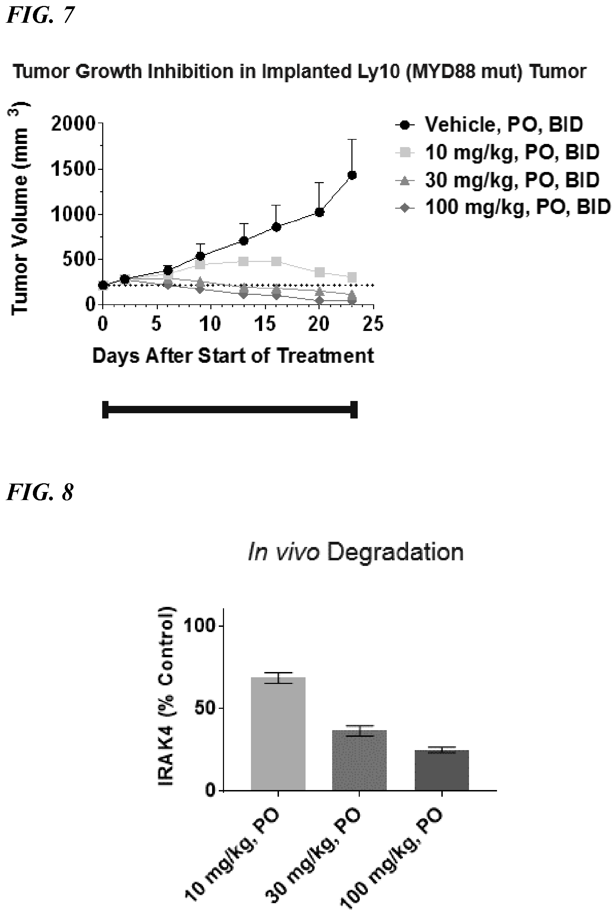

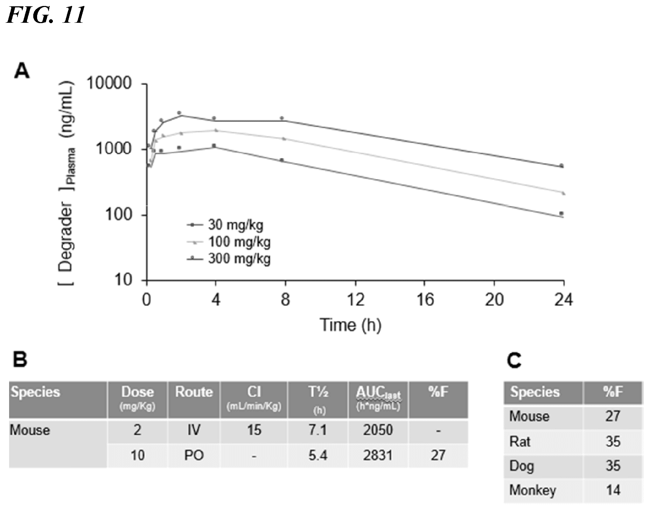

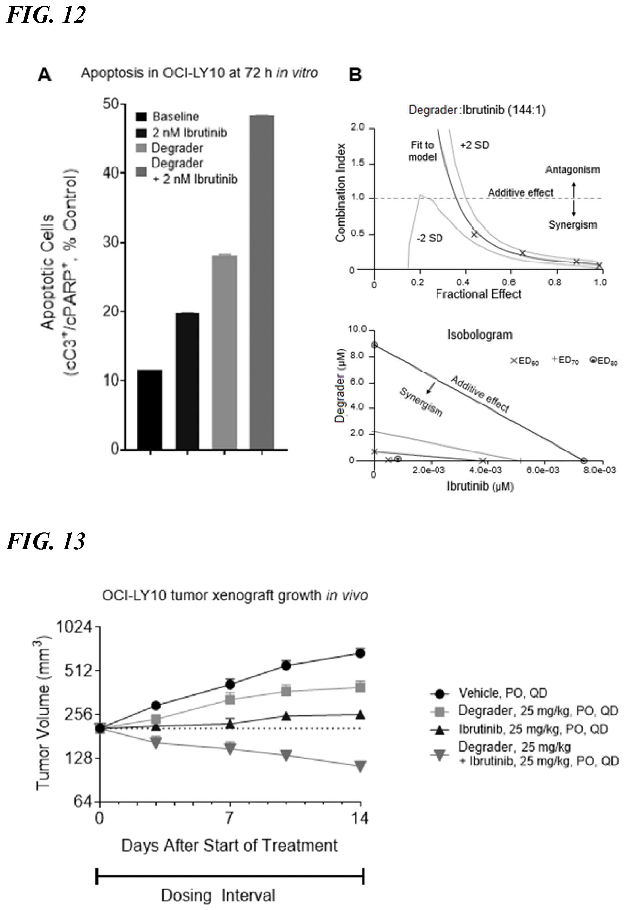

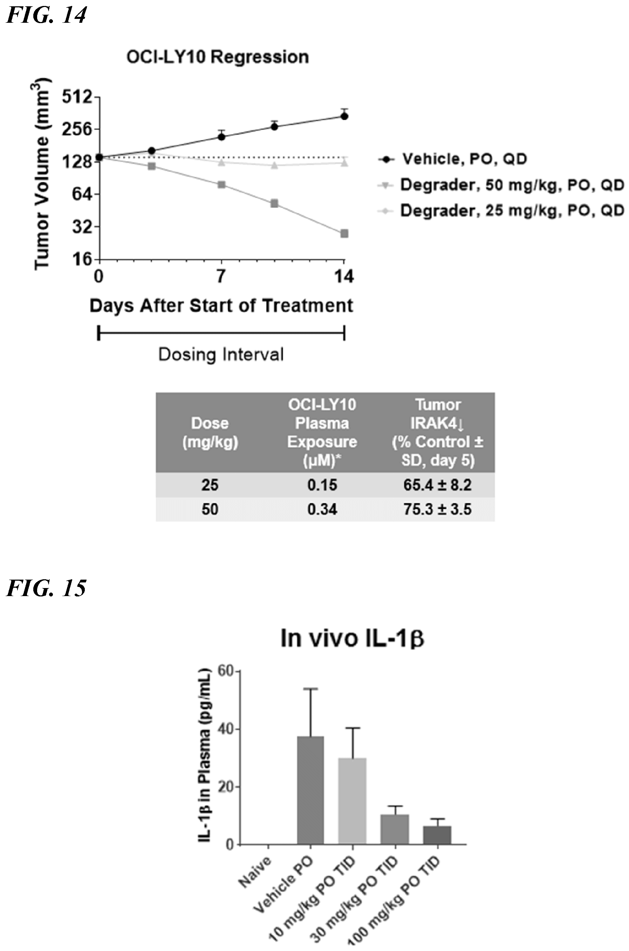

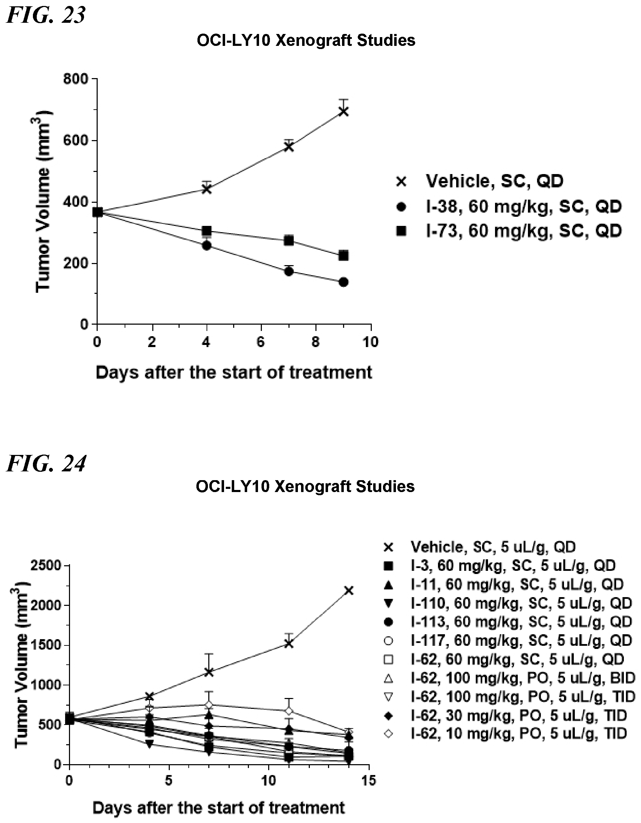

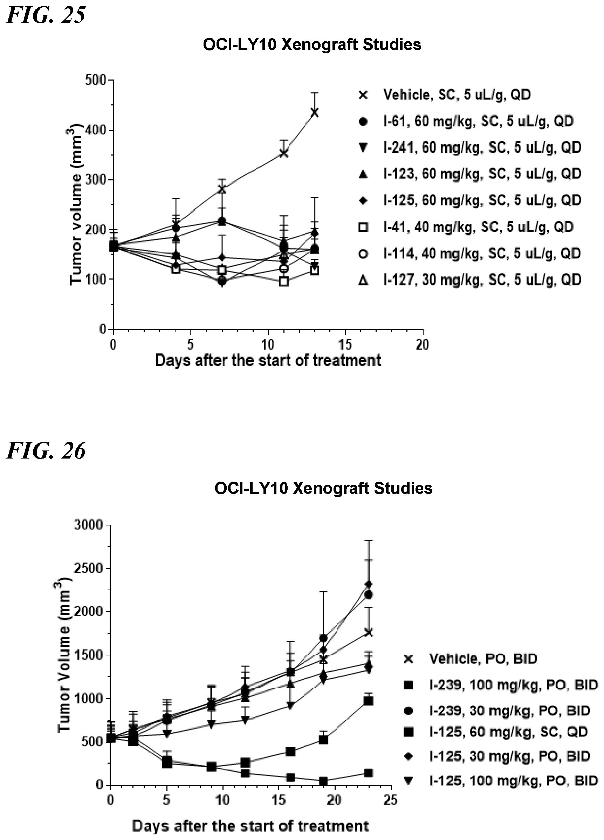

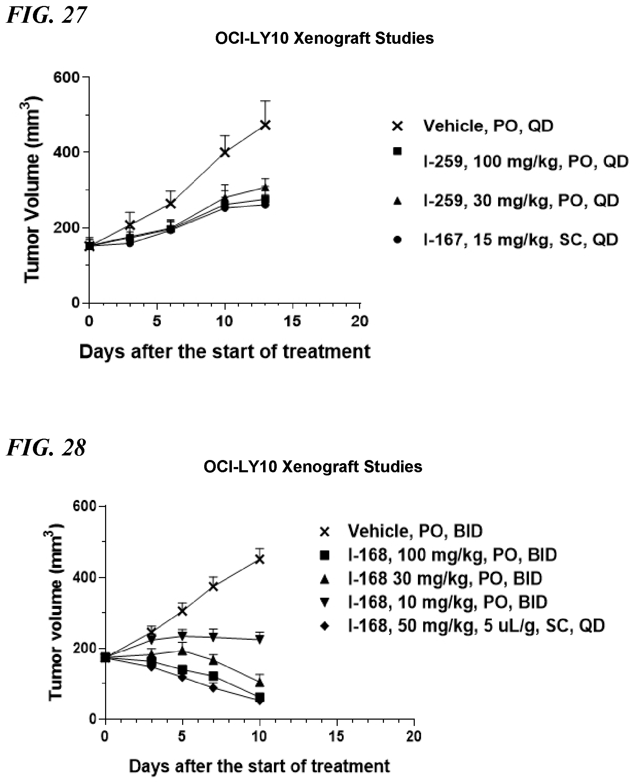

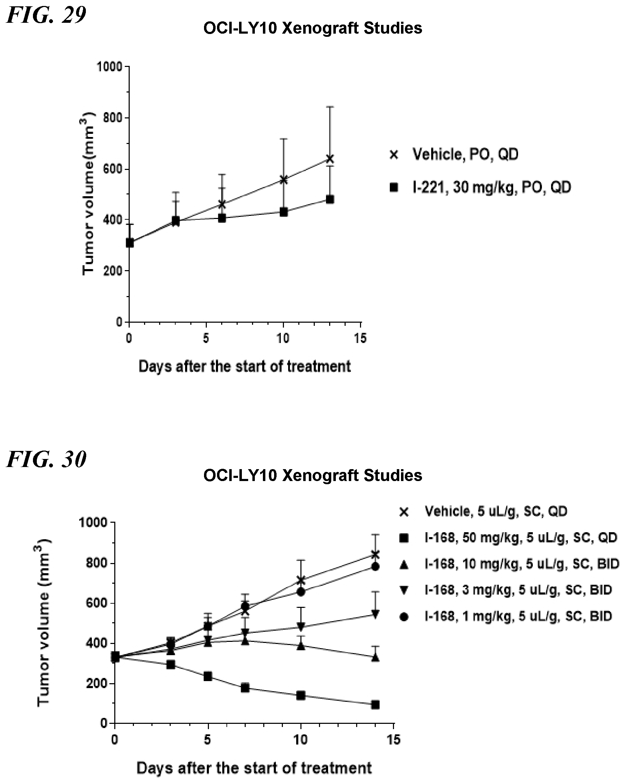

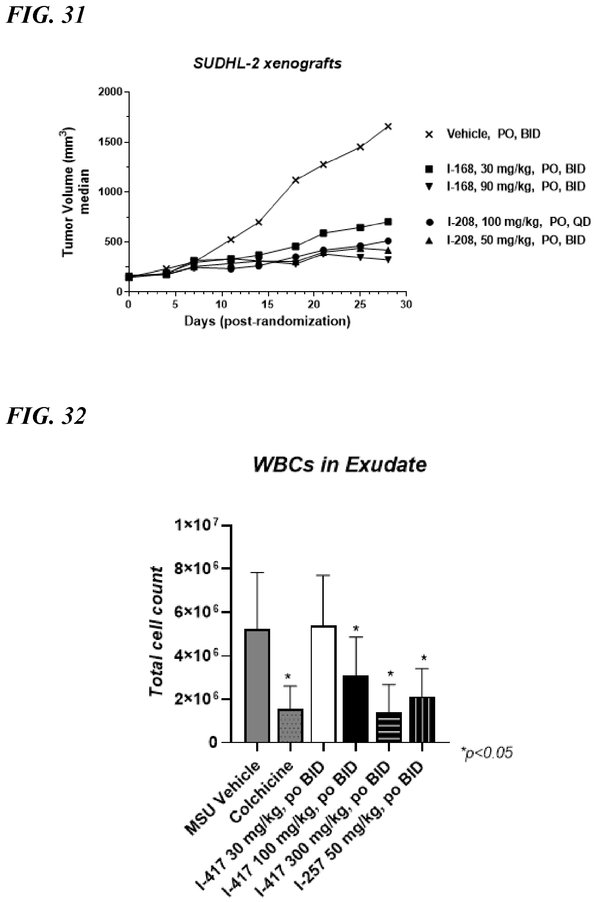

FIG. 6 A-C show that **I-30** causes tumor regression in OCI-LY10 and is active in TMD8 xenograft models. FIG. 6 D-E shows **I-30** tumor PK/PD and relationship to antitumor effect. FIG. 7 shows results from a xenograph model showing that PO BID administration of**I-30** inhibits in vivo tumor growth of implanted OCI-LY10 (MYD88 mutant) cells. FIG. 11 shows that **I-75** shows dose proportional oral exposure in plasma up to 300 mg/kg and is oral bioavailable in rat, dog, and monkey. FIG. 13 shows the results from a xenograph model showing the synergistic in vivo tumor growth inhibition of implanted OCI-LY10 (MYD88 L265P, CD79 mutant) cells using a combination of **I-30** and ibrutinib at concentrations that are sub-optimal for single agent administration alone. FIG. 14 shows oral dosing of **I-75** showed dose-dependent antitumor activity against OCI-LY10, with >75% degradation of IRAK4 correlating with tumor regression in xenograft-bearing mice. FIGs. 23-30 show additional results for compounds of the invention in OCI-LY10 xenographs. FIG. 31 shows results for compounds of the invention in SUDHL-2 xenographs. SUDHL-2 xenographs are prepared substantially as described above using OCI-LY10.

### Example 14. In vitro Degrader Potency Data

Human whole blood IRAK4 degradation flow assay. Whole blood was collected in heparinized tubes and plated on the same day as draw (day 0). Whole blood was aliquoted into deep well plates. Compound plates were prepared and a 10 point, 5-fold dilution was performed with a final DMSO concentration of 0.1 %. Compound was added to whole blood deep well plate, sealed and incubated at 37 °C, 5% CO₂ for 20 hours (for 4hour treatment, compounds were prepared and added the following day). Following the treatment incubation period (day 1), BD lyse fix (BD#558049) was added to the whole blood plate, placed on plate shaker for 30 seconds and incubated for 10mins at room temperature. Whole blood was then spun down and washed two times with PBS/0.5%BSA, aspirated to pellet and placed into -80 °C freezer until further processing for flow. On the flow run day, whole blood plates were thawed and samples were transferred to PCR plates. The pre-perm staining cocktail (CD3 Ax488/CD8 BUV805/CD14 BUV395/CD16/56 BV711/CD19 BV785) was added to samples and incubated for 30 minutes at room temperature. Samples were washed two times and permeabilized with Methanol for 10 minutes at 4°C. Samples were washed two times and the post-perm staining cocktail (CD4 PE/IRAK4 Ax647 BD#560315) was added and incubated for 30 minutes at room temperature. Samples were washed two times with PBS/BSA and run on a BD LSRFortessa. Mononuclear cells are gated by SSCH/FSCH and single cells. Monocytes are then gated through CD14 positive gate and lymphocytes are gated through CD14 negative gate. To determine absolute DC₅₀ and max degradation values, MFI values were normalized to DMSO max and 20 hour 10 µM min control. Twenty hour dose curves were calculated using a 4 parameter logistic regression curve fit, no constraints (Top doses were removed if hook effects were observed and the bottom was constrained to 0).

Human PBMC LPS TNFa assay. Frozen PBMCs were thawed into RPMI with 10% FBS/ 1% penicillin/streptomycin and same day plated into 96 well plates at 200,000 PBMC/2298.8 uL media per well. Compound plates were prepared in DMSO with D300 cassette as 8-point dose curves in duplicate, starting dose 2 µM, 5-fold. Compounds are added 200nL into cells for final 200 µL per well. Compound and cells were incubated at 37 °C, 5% CO₂ for 20 hours. After twenty hours of pretreatment with the compounds, LPS (01 1 1 :B4) (Sigma#L2637) was added at 100ng/ml final concentration and PBMCs incubated an additional five hours at 37 °C, 5% CO₂. Following assay completion, PBMC plates were spun down at 2000rpm for 10 minutes. 100uL of supernatant was carefully removed and placed into a new 96 well Costar (#3879) plate and stored at -80 °C until further analysis. Meso scale Discovery (MSD) human V-plex assays were used to measure cytokine levels. On day of cytokine analysis, supernatant samples were thawed, diluted with V-plex diluent#2, and directly to MSD plates. Assay was further completed per standard manufacturer's protocol. Cytokine data was normalized to LPS stimulated and unstimulated controls. Prism Graphpad was used to generate IC50 using 4 parameter logistic regression curve, free-fit.

Human Whole blood LPS TNFa assay. Whole blood was collected fresh and plated same day into 96 well plates at 180 µL per well. Compound plates were prepared in DMSO as 8-point dose curves in duplicate, starting dose 2 µM, 5-fold. Compounds are added 20 µL into blood for final 200 µL per well. Compound and cells were incubated at 37 °C, 5% CO₂ for 20 hours. After twenty hours of pretreatment with the compounds, LPS (01 1 1 :B4) (Sigma#L2637) was added at 100 ng/ml final concentration and blood incubated an additional five hours at 37 °C, 5% CO₂. Following assay completion, whole blood plates were spun down at 2000 rpm for 15 minutes at 4 °C. 50 µL of plasma was carefully removed and placed into a new 96 well Costar (#3879) plate and stored at -80 °C until further analysis. Meso scale Discovery (MSD) human V-plex assays were used to measure cytokine levels. On day of cytokine analysis, plasma samples were thawed, diluted with V-plex diluent#2, and directly to MSD plates. Assay was further completed per standard manufacturer's protocol. Cytokine data was normalized to LPS stimulated and unstimulated controls. Prism Graphpad was used to generate IC₅₀ using 4 parameter logistic regression curve, free-fit.

IRAK4 degradation in whole blood monocyte-Flow cytometry results for compounds of the invention are presented in **Table 10.** The letter codes for IRAK4 IC₅₀ include: A (<0.05 µM), B (0.05 - 0.1 µM), C (0.1 - 0.5 µM), D (0.5 - 1.0 µM), and E (>1.0 µM).

**Table 10. IRAK4 degradation in whole blood monocyte-Flow cytometry Results.**

| **Compound #** | **IRAK4 degradation in whole blood monocyte-Flow cytometry: Average external Abs IC₅₀ (uM)** |
|---|---|
| **I-1** | **C** |
| **I-2** | **A** |
| **I-3** | **A** |
| **I-5** | **C** |
| **I-6** | **A** |
| **I-7** | **A** |
| **I-8** | **A** |
| **I-9** | **A** |
| **I-10** | **A** |
| **I-11** | **A** |
| **I-12** | **D** |
| **I-16** | **A** |
| **I-17** | **A** |
| **I-18** | **A** |
| **I-27** | **A** |
| **I-28** | **B** |
| **I-29** | **D** |
| **I-30** | **A** |
| **I-31** | **B** |
| **I-32** | **A** |
| **I-33** | **D** |
| **I-34** | **A** |
| **I-35** | **A** |
| **I-36** | **A** |
| **I-37** | **A** |
| **I-38** | **A** |
| **I-39** | **B** |
| **I-40** | **A** |
| **I-41** | **A** |
| **I-42** | **A** |
| **I-43** | **B** |
| **I-45** | **A** |
| **I-46** | **D** |
| **I-47** | **D** |
| **I-48** | **A** |
| **I-58** | **A** |
| **I-59** | **A** |
| **I-61** | **B** |
| **I-62** | **A** |
| **I-65** | **A** |
| **I-67** | **B** |
| **I-75** | **B** |
| **I-76** | **B** |
| **I-81** | **E** |
| **I-92** | **C** |
| **I-94** | **C** |

IRAK4 human whole blood LPS TNFa inhibition results for compounds of the invention are reported in **Table 11.** The letter codes for IRAK4 IC₅₀ include: A (<0.05 µM), B (0.05 - 0.1 µM), C (0.1 - 0.5 µM), D (0.5 - 1.0 µM), and E (>1.0 µM).

**Table 11. IRAK4 human whole blood LPS TNFa inhibition Results.**

| **Compound #** | **IRAK4 human whole blood LPS TNFa inhibition: Average external-IC₅₀ (uM)** |
|---|---|
| **I-2** | **E** |
| **I-5** | **E** |
| **I-6** | **E** |
| **I-7** | **E** |
| **I-8** | **E** |
| **I-9** | **E** |
| **I-10** | **E** |
| **I-11** | **E** |
| **I-12** | **D** |
| **I-16** | **E** |
| **I-17** | **D** |
| **I-18** | **C** |
| **I-27** | **C** |
| **I-28** | **E** |
| **I-29** | **E** |
| **I-30** | **C** |
| **I-31** | **E** |
| **I-32** | **E** |
| **I-34** | **C** |
| **I-35** | **D** |
| **I-36** | **C** |
| **I-37** | **C** |
| **I-38** | **C** |
| **I-39** | **C** |
| **I-40** | **C** |
| **I-41** | **D** |
| **I-42** | **E** |
| **I-43** | **C** |
| **I-44** | **E** |
| **I-45** | **D** |
| **I-46** | **E** |
| **I-47** | **E** |
| **I-48** | **C** |
| **I-58** | **E** |
| **I-59** | **E** |
| **I-61** | **E** |
| **I-62** | **E** |
| **I-65** | **E** |
| **I-75** | **E** |

IRAK4 hPBMC LPS TNF-α inhibition results for compounds of the invention are reported in **Table 12.** The letter codes for IRAK4 IC₅₀ include: A (<0.05 µM), B (0.05 - 0.1 µM), C (0.1 - 0.5 µM), D (0.5 - 1.0 µM), and E (>1.0 µM).

**Table 12. IRAK4 hPBMC LPS TNFa inhibition Results.**

| **Compound #** | **IRAK4 hPBMC LPS TNF-α inhibition: Average external-IC₅₀ (µM)** |
|---|---|
| **I-2** | **C** |
| **I-11** | **A** |
| **I-16** | **C** |
| **I-17** | **A** |
| **I-30** | **B** |
| **I-31** | **C** |
| **I-32** | **B** |
| **I-33** | **D** |
| **I-34** | **C** |
| **I-35** | **C** |
| **I-36** | **C** |
| **I-37** | **A** |
| **I-38** | **A** |
| **I-39** | **B** |
| **I-40** | **A** |
| **I-41** | **C** |
| **I-42** | **C** |
| **I-43** | **C** |
| **I-44** | **E** |
| **I-45** | **A** |
| **I-46** | **C** |
| **I-47** | **C** |
| **I-48** | **C** |
| **I-58** | **C** |
| **I-59** | **A** |
| **I-61** | **C** |
| **I-62** | **C** |
| **I-65** | **A** |
| **I-67** | **B** |
| **I-75** | **A** |
| **I-76** | **A** |
| **I-81** | **D** |

IRAK4 degradation in whole blood lymphocyte-Flow cytometry results for compounds of the invention are reported in **Table 13.** The letter codes for IRAK4 IC₅₀ include: A (<0.05 µM), B (0.05 - 0.1 µM), C (0.1 - 0.5 µM), D (0.5 - 1.0 µM), and E (>1.0 µM).

**Table 13. IRAK4 degradation in whole blood lymphocyte-Flow cytometry Results.**

| **Compound #** | **IRAK4 degradation in whole blood lymphocyte-Flow cytometry: Average external Abs IC₅₀ (µM)** |
|---|---|
| **I-1** | C |
| **I-2** | A |
| **I-3** | A |
| **I-5** | C |
| **I-6** | A |
| **I-7** | C |
| **I-8** | A |
| **I-9** | B |
| **I-10** | C |
| **I-11** | A |
| **I-12** | C |
| **I-16** | B |
| **I-17** | B |
| **I-18** | C |
| **I-27** | A |
| **I-28** | C |
| **I-29** | D |
| **I-30** | A |
| **I-31** | C |
| **I-32** | B |
| **I-33** | A |
| **I-34** | A |
| **I-35** | B |
| **I-36** | A |
| **I-37** | A |
| **I-38** | B |
| **I-39** | C |
| **I-40** | C |
| **I-41** | A |
| **I-42** | A |
| **I-43** | C |
| **I-45** | B |
| **I-46** | C |
| **I-47** | C |
| **I-48** | A |
| **I-58** | B |
| **I-59** | B |
| **I-61** | C |
| **I-62** | A |
| **I-65** | B |
| **I-67** | B |
| **I-75** | B |
| **I-76** | B |
| **I-81** | E |
| **I-92** | D |
| **I-94** | C |

### Example 15. Evaluation of efficacy of I-30 in murine model of monosodium urate (MSU) crystal-induced inflammatory cell infiltration into the six-day air pouch; a model of human gout in male BALB/c mice.

Animals: BALB/c mice, male, 5-6 weeks, weighing approximately 19-23g were used. Animals were housed and maintained according to IACUC protocols.

Air Pouch: Each mouse was anesthetized and injected with 6 mL of sterile air subcutaneously (SC) at the nape of the neck and the injection site was sealed with flexible colloidon to create an air pouch. Three days later, the mice were injected with 3 mL of sterile air SC at the same site and the injection site was sealed with flexible colloidon.

Preparation of MSU Crystals: 10 g uric acid (Sigma, Cat. U2625, lot BCBM5312V) was heated to 60°C in 2 L purified water, containing 5.8 mL 10 N NaOH. The pH of the heated mixture was adjusted to 8.9, yielding a clear solution which was stored overnight at 4-8°C to allow MSU crystals to form and precipitate. The MSU crystals were washed three times with 1L purified water and stored at 37 °C to dry.

Formulation of Degrader: 0.5% CMC/0.25% Tween-80 was prepared by dissolving 0.5 g carboxymethylcellulose (Sigma, Cat. C4888, lot 101K0185) in 100 mL de-ionized water followed by the addition of 0.25 mL Tween-80 (Sigma, Cat. P1754, lot 073K0064). **I-30** was sonicated in 0.5% CMC/0.25% Tween-80 to prepare a 10 mg/ml suspension and this was diluted in 0.5%CMC/0.25%Tween-80 to prepare a 3 mg/mL suspension and a 1 mg/mL suspension for dosing.

Formulation of Reference Compounds: Colchicine was dissolved in sterile saline to prepare a 0.1 mg/mL solution. Anakinra was diluted in sterile saline to prepare 10 mg/mL solution.

Preparation of MSU Crystal Suspension: MSU crystals were suspended in sterile 0.9% saline for injection, USP (Baxter, lot 256131, exp. JUN19). The mixture was placed on a stir plate to maintain a constant 10 mg/mL homogenous suspension.

Dosing of Degrader: Mice were dosed orally at 10 mL/kg with suspensions above, giving 10 mg/kg, 30 mg/kg and 100 mg/kg final dose levels, three times at 8-hour intervals.

Dosing of Reference Compounds: Mice were dosed subcutaneously with colchicine at 1 mg/kg final and with Anakinra at 100 mg/kg final.

MSU Challenge: One hour after dosing with reference compounds or four hours after dosing the third time with degrader, mice were injected into the air pouch with 3 mL MSU (30 mg/mouse) and the injection sites were sealed with flexible collodion. The mice were returned to their cages, no adverse effects were observed.

Termination, Cell Collection and Data: Four hours after MSU/saline injection, the mice were anesthetized and humanely exsanguinated. The spleens were removed, halved, flash frozen in labeled Eppendorf tubes in liquid nitrogen, and stored at -80°C for determination of IRAK4 using targeted MS/MS with labeled peptide internal standard. Immediately after the bleed, 3 ml 10 U/mL heparinized saline was injected into the air pouch. The air pouch was gently massaged, the contents immediately removed, and the exudate volume recorded. After allowing the MSU crystals to settle out for fifteen minutes in an ice bath, an aliquot of the exudate was transferred to labeled Eppendorf tubes for differential white blood cell counts, including neutrophils.

FIG. 8 shows in vivo degradation of IRAK4 in BALB/c mouse spleen following three PO doses of **I-30**. FIG. 9 shows the results of neotrophils count in exudate collected from air pouch in mouse following MSU crystal challenge using PO TID dosing of **I-30.** FIG. 15 shows that orally dosed **I-30** mediates a dose-dependent decrease in IL-1β in plasma.

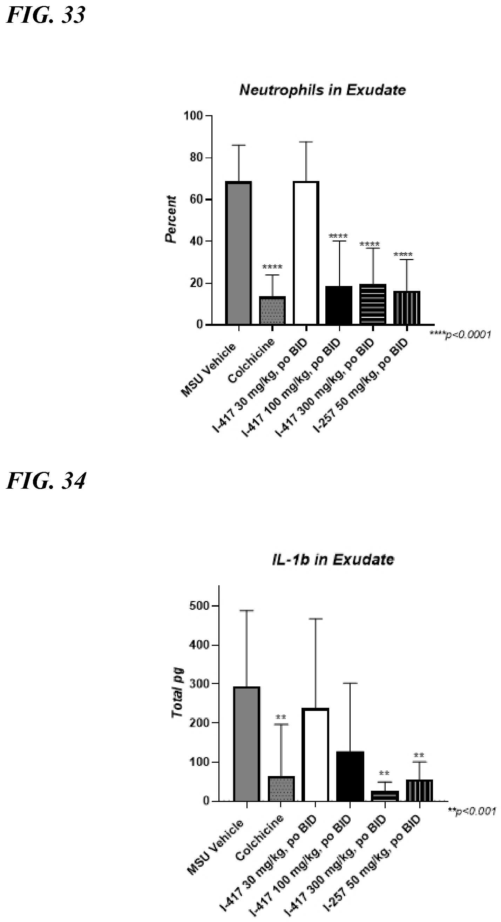

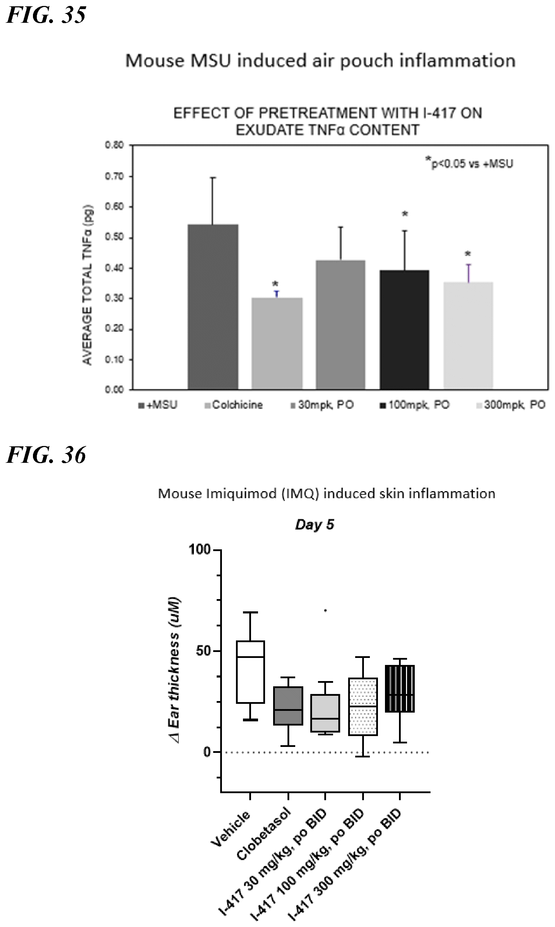

FIGs. 32-34 show results of additional MSU crystal challenges using **I-257** and **I-417** in place of **I-30** above. Compounds of the invention significantly reduce cellular infiltration and IL-1 production in the exudate in air pouch following MSU crystal injection. FIG. 35 shows the effect of pretreatment with **I-417** exudate TNFα content in the MSU model. Compounds of the invention significantly reduce TNFα production in the exudate and systemic IL-6 in the plasma following MSU crystal injection into an air pouch.

### Example 16. Human whole blood cytokine release assays

Fresh human whole blood was collected in Na Heparin tubes and received from ALLCELLS same day as collection. 200uL of whole blood was aliquoted per well into a 96 well plate. Whole blood was pre-incubated with compounds overnight at 37 °C, 5% CO₂. Following compound incubation, samples were stimulated with I-75 0.25µg/mL (invivogen #tlrl-r848), LPS 10ng/mL (Sigma#L2637) or IL-1β 25ng/mL (R&D systems 201-LB) and incubated for an additional 5 hours. At the end of the assay, blood was spun down for 15 min at 4 °C and plasma was collected and immediately stored at -80 °C. Cytokine and chemokine levels were measured by custom ordered U-plex MSD panels (panel 1: IFNy, IL-10, IL-1β, IL-6, IL-8, TNFα) and (panel 2: IP-10, MIP1-α, MIP-1β, IL-12/IL-23p40, GROα).

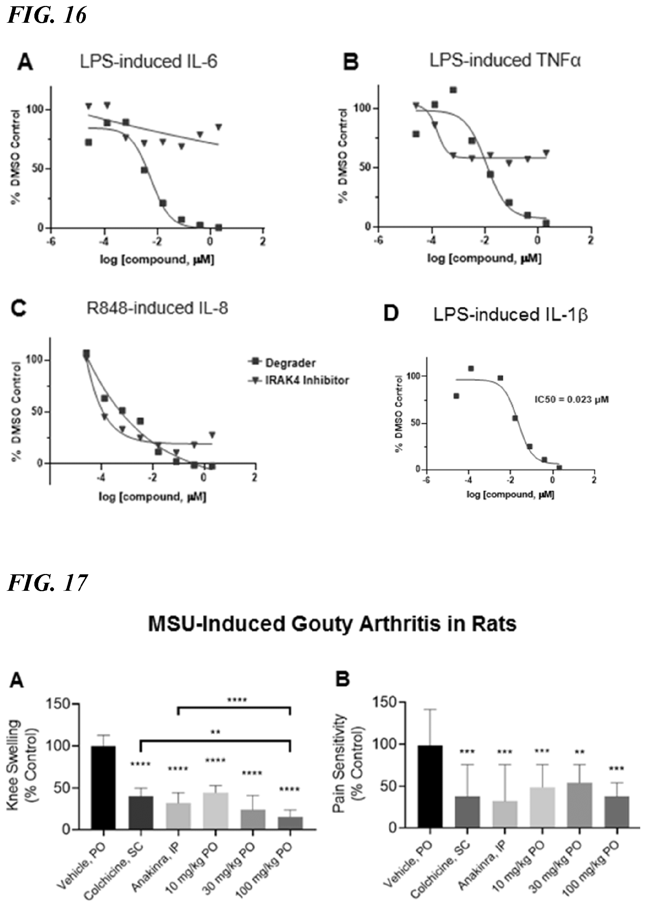

FIG. 16 depicts the in vitro results of the cytokine release assays showing that IRAK4 degradation using **I-75** inhibits a broader range of cytokines induced by different TLR stimuli than IRAK4 inhibition with PF-06550833.

Results of IL-6 secretion in hPBMC using R848 stimulation for compounds of the invention are reported in **Table 13b.** The letter codes for IC₅₀ include: A (<0.001 µM), B (0.001 - 0.01 µM), C (0.01 - 0.1 µM), D E (>0.1 µM).

**Table 13b. IL-6 secretion in hPBMC results.**

| **I-#** | **IL-6 Secretion in hPBMC, R848 as stim, t=20h: IC₅₀ (µM)** |
|---|---|
| **I-185** | A |
| **I-191** | B |
| **I-257** | A |
| **I-317** | B |
| **I-396** | B |
| **I-411** | A |
| **I-413** | A |
| **I-414** | A |
| **I-415** | A |
| **I-416** | A |
| **I-417** | A |
| **I-418** | B |
| **I-419** | B |
| **I-420** | A |
| **I-421** | A |
| **I-422** | A |
| **I-423** | B |
| **I-424** | B |
| **I-425** | B |
| **I-426** | B |
| **I-428** | B |
| **I-430** | B |
| **I-431** | A |
| **I-432** | B |
| **I-433** | A |
| **I-434** | A |
| **I-446** | C |
| **I-447** | B |
| **I-448** | B |
| **I-450** | B |
| **I-464** | B |
| **I-465** | A |
| **I-466** | B |
| **I-467** | A |
| **I-468** | D |
| **I-469** | A |
| **I-470** | D |
| **I-471** | A |
| **I-472** | A |
| **I-479** | C |
| **I-480** | A |
| **I-481** | C |
| **I-482** | B |
| **I-483** | B |
| **I-484** | D |
| **I-485** | B |
| **I-486** | A |
| **I-487** | A |
| **I-488** | D |
| **I-492** | A |
| **I-493** | A |
| **I-494** | D |
| **I-495** | C |
| **I-496** | A |
| **I-497** | A |
| **I-498** | C |
| **I-499** | B |
| **I-508** | B |
| **I-509** | B |
| **I-510** | B |
| **I-511** | B |
| **I-512** | D |
| **I-513** | A |
| **I-514** | A |
| **I-515** | A |
| **I-516** | B |
| **I-517** | B |
| **I-518** | B |
| **I-519** | D |
| **I-525** | A |
| **I-526** | A |
| **I-528** | B |
| **I-530** | A |
| **I-532** | B |
| **I-537** | B |
| **I-540** | B |
| **I-543** | A |
| **I-547** | B |
| **I-549** | B |
| **I-553** | B |
| **I-554** | A |
| **I-555** | B |

### Example 17. In vitro Combination Studies

The ABC-DLBCL cell line OCI-LY10 was MV4-11 cells were maintained in IMDM (Invitrogen, supplemented with 20% heat inactivated fetal bovine serum (Life Technologies, Grand Island, N.Y.) plus +BME. Cultures were maintained in a humidified atmosphere including 5% CO₂. Studies were performed using OCI-LY10 cells in vitro to evaluate the anti-proliferative effect of a combination of two agents together on cell growth. The cell counts were measured by ATP quantitation using the Promega Cell Titer Glo kit and luminescence values corresponded to the amount of ATP in a given well. Compounds evaluated for synergy were tested in a range which was bracketed around the IC₅₀'s. The compounds were plated to a 384 well plate in a matrix format which includes increasing concentrations of each drug in the combination in a constant ratio, in addition to the effect of each compound alone in the study. Cells were seeded and grown in the log-linear phase for 4 days. DMSO concentration was less than 0.2% v/v.

The drug combination analysis was performed utilizing the Chou-Talalay method [Chou TC Pharmacological Reviews 2006]. Maximum and minimum inhibition (DMSO alone) controls were used in each plate to calculate fraction affected (Fa) of a test well. The combination index (CI) is the term used to describe the level of synergy or antagonism in a given test system. A combination index less than one indicates synergy, and a CI greater than one indicates antagonism. Synergy was determined using the software package Calcusyn by Biosoft. Graphs representing values of combination index (CI) versus Fractional effect (Fa) known as Fa-CI plots were generated and synergy was evaluated.

FIG. 12 depicts the results of a cell viability assay showing that the combined activity of **I-30** and a BTK inhibitor leads to a greater than additive increase in apoptosis within 72 h in n OCI-LY10 bearing both MYD88 L265P and CD79 mutations. Further, **I-30** is synergistic with BTK inhibition *in vitro* vs cell viability, assessed with four day Cell Titer Glo read out.

### Example 18. MSU Induced Gouty Arthritis in Rats

The anti-inflammatory efficacy and pain reduction for prophylactic treatment in an MSU-induced knee inflammation rat model of human gout was evaluated. *See* Kou, Y.-Y et al., Evid-Based Compl. Alt. (doi: http://dx.doi.org/10.1155/2015/527019).

Preparation of MSU crystals: 4 g uric acid is dissolved in 800 ml 0.03N NaOH and heated to 60°C. NaOH is added as required to maintain pH 8.9. The solution is stored overnight at 4-8^{O}C. Crystals are filtered, washed and dried. Crystals are suspended in sterile saline just prior to use.

Efficacy Study: Receive and quarantine 70 Wistar Strain rats (male, 250-300 g). Following quarantine, rats are accepted for the Study if no signs of clinical distress are noted during the 3-day quarantine period. The rats are maintained on certified laboratory diet and water *ad libitum.* The rats are ear-notched for individual identification. The rat weights are recorded. The rats are distributed to 7 groups of 10 rats per group such that each group has a similar mean weight. Baseline readings are taken on DAY -3. Baseline knee diameters are measured by digital caliper. Baseline responses to mechanical allodynia, as measured by von Frey fibers, are recorded.

Day -2 and day -1: The rats are weighed and dosed by oral gavage with **I-75** in groups 2, 3, 4 and with vehicle in group 1.

DAY 0: The rats are weighed and dosed daily by oral gavage or intraperitoneal or subcutaneous as in **Table 14.** One hour after dosing, rats in Groups 1-7 are anesthetized and injected IA into the left knee with 2 mg MSU in 50 µl PBS.

**Table 14.**

| **Group** | **Treatment** | **Dose** | **ROA** | **Frequency*** | **MSU** | **No. Rats** |
|---|---|---|---|---|---|---|
| 1 | Vehicle | 5 ml/kg | PO | QD | YES | 10 |
| 2 | **I-75** | 10 mg/kg | PO | QD X 5 days | YES | 10 |
| 3 | **I-75** | 30 mg/kg | PO | QD X 5 days | YES | 10 |
| 4 | **I-75** | 100 mg/kg | PO | QD x 5 days | YES | 10 |
| 5 | PF06650883 | 30 mg/kg | PO | QD x 1 day | YES | 10 |
| 6 | Anakinra | 50 mg/kg | IP | QD x 3 days | YES | 10 |
| 7 | Colchicine | 0.5 mg/kg | SC | Day 0, Day 2 | YES | 10 |

| | | | | | | |
|---|---|---|---|---|---|---|
| *Dose regime: Group 1 (vehicle), Groups 2-4: Dose on day -2, -1, day 0, day 1 and day 2; Group 5 (PF06650883): Dose on day 0; Group 6 (Anakinra): Dose on day 0, day 1 and day 2; Group 7 (Colchicine): Dose on day 0 and day 2 | | | | | | |

DAY 1: Rats are weighed and dosed as in **Table 14.** One hour after dosing, mechanical allodynia and knee diameters are measured and recorded.

DAY 2: Rats are weighed and dosed as in **Table 14** One hour after dosing, mechanical allodynia and knee diameters are measured and recorded.

Groups 1-4: 4 hours after final dosing, rats in groups 1-4 are sedated with ketamine and are exsanguinated into pre-chilled EDTA-treated tubes: The whole blood is processed to plasma which is stored in labeled 15 ml conical tubes at -80 °C. The cell pellet is processed to PBMCs within two hours and stored at -80 °C. Spleens are removed, divided into two portions and each half is snap frozen in tubes. Tubes are stored at -80 °C until quantification of IRAK4 by targeted MS/MS.

Groups 5-7 (Group 5: only one dose on day 0, there is no dose on day 2): 4 hours after final dosing, rats are sedated with ketamine and are exsanguinated into pre-chilled EDTA-treated tubes: The whole blood is processed to plasma which is stored in labeled 15 ml conical tubes at -80 °C. The cell pellet is processed to PBMCs within two hours and stored at -80 °C. Spleens are removed, divided into two portions and each half is snap frozen in tubes. Tubes are stored at -80 °C until quantification of IRAK4 by targeted MS/MS.

Results are tabulated, graphed and statistically analyzed for anti-inflammatory efficacy and pain reduction. FIG. 17. shows that the prophylactic treatment of **I-75** results in a dramatic anti-inflammatory and analgesic effect of decreasing knee joint swelling (A) and pain sensitivity (B) in a MSU-induced knee inflammation rat model of human gout.

### Example 19 (Method 3). Synthesis of N-[3-(difluoromethyl)-1-[4-[[4-[3-[1-(2,6-dioxo-3-piperidyl)-3-methyl-2-oxo-benzimidazol-4 -yl]propoxy]-1-piperidyl]methyl]cyclohexyl]pyrazol-4-yl]-5-[(2R,6S)-2,6-dimethylmorpholin-4-yl]pyrazolo[1,5-a]pyrimidine-3-carboxamide (I-275)

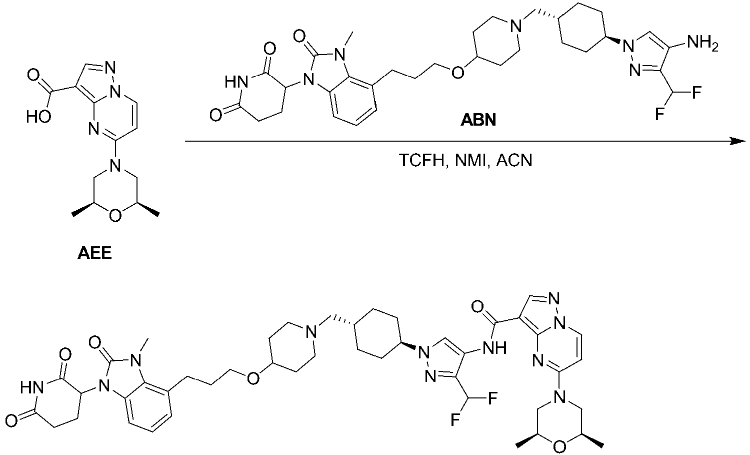

To a solution of 5-[(2R,6S)-2,6-dimethylmorpholin-4-yl]pyrazolo[1,5-a]pyrimidine-3-carboxylic acid (16.6 mg, 60.2 umol, Intermediate AEE) in ACN (1 mL) was added [chloro(dimethylamino)methylene]- dimethyl-ammonium;hexafluorophosphate (20.2 mg, 72.2 umol), 1-methylimidazole (17.3 mg, 210 umol) and the mixture was stirred at 15°C for 1 hr. Next, 3-[4-[3-[[1-[[4-[4-amino-3-(difluoromethyl) pyrazol-1-yl]cyclohexyl]methyl]-4-piperidyl]oxy]propyl]-3-methyl-2-oxobenzimidazol-1-yl]piperidine-2,6-dione (40.0 mg, 60.2 umol, HCl, Intermediate ABN) was added into the above mixture. The mixture was stirred at 15 °C for 1 hr. On completion, the mixture was quenched with H₂O (0.5 mL) and concentrated *in vacuo.* The residue was purified by prep-HPLC (column: Phenomenex Synergi C18 150*25*10um; mobile phase: [water (0.225% FA) - ACN]; B%: 18%-48%, 10 min) to give the title compound (23.0 mg, 40% yield) as white solid. ¹H NMR (400MHz, DMSO-*d₆*) δ 11.09 (s, 1H), 9.28 (s, 1H), 8.82 (d, *J* = 8.0 Hz, 1H), 8.39 (s, 1H), 8.29 (s, 1H), 7.31 - 7.02 (m, 1H), 7.00 - 6.92 (m, 3H), 6.92 - 6.85 (m, 1H), 5.41 - 5.31 (m, 1H), 4.60 - 4.32 (m, 2H), 4.23 - 4.12 (m, 1H), 3.68 - 3.63 (m, 2H), 3.58 (s, 3H), 3.48 - 3.46 (m, 2H), 3.01 - 2.94 (m, 2H), 2.93 - 2.83 (m, 1H), 2.78 - 2.70 (m, 1H), 2.67 - 2.63 (m, 2H), 2.63 - 2.55 (m, 1H), 2.14 - 1.94 (m, 9H), 1.92 - 1.70 (m, 9H), 1.62 - 1.53 (m, 1H), 1.51 - 1.41 (m, 2H), 1.21 (s, 3H), 1.19 (s, 3H), 1.09 - 0.99 (m, 2H); LC-MS (ESI⁺) *m*/*z* 886.6 (M+H)⁺.

**Table 15. Compounds synthesized via Method 3 with the coupling of various amines and acids.**

| **I-#^{a}** | **Intermediate Amine** | **Intermediate Acid** | **LCMS (ES+) m/z (M+H)⁺** | **¹HNMR (400MHz, DMSO-d₆) δ** |
|---|---|---|---|---|
| **I-276** | ABN | AEF | 886.6 | 11.08 (s, 1H), 9.31 (s, 1H), 8.80 (d, *J* = 8.0 Hz, 1H), 8.40 (s, 1H), 8.27 (s, 1H), 7.29 - 6.85 (m, 5H), 5.36 (dd, *J* = 5.2, 12.8 Hz, 1H), 4.28 - 4.15 (m, 1H), 4.11 - 4.02 (m, 2H), 3.98 - 3.82 (m, 2H), 3.58 (s, 3H), 3.52 (d, *J* = 6.8 Hz, 2H), 3.48 (t, *J* = 6.4 Hz, 4H), 3.06 - 2.81 (m, 6H), 2.78 - 2.67 (m, 2H), 2.67 - 2.60 (m, 2H), 2.12 - 2.04 (m, 2H), 2.01 - 1.96 (m, 1H), 1.96 - 1.86 (m, 4H), 1.86 - 1.72 (m, 5H), 1.70-1.56 (m, 2H), 1.17 (d, *J* = 6.4 Hz, 6H), 1.14 - 1.02 (m, 2H). |
| **I-277** | ABN | AEG | 870.6 | 11.09 (s, 1H), 9.51 (d, *J* = 6.0 Hz, 1H), 8.78 (d, *J* = 8.0 Hz, 1H), 8.39 (d, *J* = 4.0 Hz, 1H), 8.26 (d, *J* = 5.6 Hz, 1H), 7.28 - 6.96 (m, 3H), 6.92 - 6.88 (m, 1H), 6.87 - 6.44 (m, 1H), 5.42 - 5.33 (m, 1H), 5.30 - 5.05 (m, 1H), 4.83 - 4.72 (m, 1H), 4.28 - 4.13 (m, 1H), 3.85 - 3.79 (m, 2H), 3.76 - 3.61 (m, 2H), 3.58 (s, 3H), 3.50 - 3.47 (m, 2H), 3.00 - 2.96 (m, 2H), 2.94 - 2.84 (m, 3H), 2.77 - 2.69 (m, 1H), 2.66 - 2.60 (m, 1H), 2.45 - 2.38 (m, 2H), 2.07 - 1.95 (m, 5H), 1.95 - 1.86 (m, 5H), 1.86 - 1.80 (m, 3H), 1.79 - 1.73 (m, 2H), 1.71 - 1.51 (m, 4H), 1.15 - 1.04 (m, 2H) |
| **I-278** | ABN | AEH | 870.5 | 11.08 (s, 1H), 9.51 (d, *J* = 6.8 Hz, 1H), 8.78 (d, *J* = 7.6 Hz, 1H), 8.40 (d, *J* = 4.4 Hz, 1H), 8.25 (d, *J* = 5.4 Hz, 1H), 7.26 - 6.43 (m, 5H), 5.36 (dd, *J* = 5.2, 12.0 Hz, 1H), 5.31 - 5.02 (m, 1H), 4.83 - 4.70 (m, 1H), 4.32 - 4.16 (m, 1H), 3.87 - 3.57 (m, 7H), 3.57-3.46 (m, 4H), 3.45 (d, *J* = 9.6 Hz, 2H), 3.08 - 2.82 (m, 7H), 2.79 - 2.68 (m, 1H), 2.66 - 2.59 (m, 1H), 2.17 - 2.08 (m, 2H), 2.04 - 1.77 (m, 12H), 1.71 - 1.57 (m, 1H), 1.28 - 1.11 (m, 2H) |
| **I-279** | AFF | AAQ | 871.1 | 11.09 (s, 1H), 9.35 (s, 1H), 8.52 (d, *J* = 7.6 Hz, 1H), 8.31 (s, 1H), 8.20 (s, 1H), 8.16 (s, 1H), 7.79 (d, *J* = 8.8 Hz, 1H), 7.29 - 6.92 (m, 3H), 6.86 (d, *J* = 7.2 Hz, 1H), 6.61 (d, *J* = 7.6 Hz, 1H), 5.37 (d, *J* = 7.6 Hz, 1H), 4.26 (d, *J* = 8.8 Hz, 1H), 4.20 - 4.09 (m, 1H), 3.82 (s, 2H), 3.67 (s, 4H), 3.61 (s, 3H), 2.93 - 2.82 (m, 3H), 2.77 - 2.58 (m, 2H), 2.21 (d, *J* = 6.4 Hz, 2H), 2.17 (s, 3H), 2.04 - 1.91 (m, 5H), 1.87 (d, *J* = 12.8 Hz, 2H), 1.74 - 1.54 (m, 10H), 1.42 - 1.31 (m, 4H), 1.08 - 0.93 (m, 2H) |
| **I-280** | AFG | AAQ | 886.6 | 11.08 (s, 1H), 9.65 (s, 1H), 9.37 (s, 1H), 8.53 (d, *J* = 7.6 Hz, 1H), 8.35 (s, 1H), 8.17 (s, 1H), 7.84 (d, *J* = 8.4 Hz, 1H), 7.31 - 6.99 (m, 3H), 6.93 - 6.84 (m, 1H), 6.63 (d, *J* = 7.6 Hz, 1H), 5.34 (dd, *J* = 4.0, 12.0 Hz, 1H), 4.30 - 4.18 (m, 2H), 3.64 (s, 1H), 3.55 - 3.37 (m, 4H), 3.33 (s, 3H), 3.09 - 2.87 (m, 5H), 2.76 - 2.62 (m, 4H), 2.16 - 1.91 (m, 9H), 1.89 - 1.65 (m, 9H), 1.63 - 1.52 (m, 4H), 1.42 - 1.30 (m, 2H), 1.24 - 1.13 (m, 2H) |
| **I-282**^{b} | ABN | ADA | 858.3 | 11.08 (s, 1H), 9.57 (d, *J* = 10.4 Hz, 1H), 8.74 (t, *J* = 7.2 Hz, 1H), 8.38 (d, *J* = 2.8 Hz, 1H), 8.24 (s, 1H), 7.25 - 6.97 (m, 1H), 6.96 (s, 1H), 6.89 - 6.85 (m, 1H), 6.57 (dd, *J* = 8.0, 12.0 Hz, 1H), 5.36 (dd, *J* = 5.2, 12.4 Hz, 1H), 5.19 - 5.01 (m, 1H), 4.50 - 4.40 (m, 1H), 4.25 - 4.12 (m, 1H), 3.85 - 3.73 (m, 1H), 3.64 (s, 2H), 3.57 (s, 3H), 3.49 - 3.44 (m, 4H), 3.00 - 2.94 (m, 2H), 2.91 - 2.70 (m, 4H), 2.66 - 2.57 (m, 2H), 2.53 - 2.51 (m, 2H), 2.11 - 1.95 (m, 5H), 1.94 - 1.70 (m, 9H), 1.68 - 1.49 (m, 3H), 1.16 - 1.01 (m, 2H) |
| **I-283** | ABN | AFI | 858.3 | 11.08 (s, 1H), 9.57 (d, *J* = 10.8 Hz, 1H), 8.74 (t, *J* = 7.2 Hz, 1H), 8.38 (d, *J* = 2.8 Hz, 1H), 8.24 (s, 1H), 7.25 - 7.00 (t, *J* = 5.2 Hz 1H), 6.96 (d, *J* = 4.8 Hz, 2H), 6.90 - 6.85 (m, 1H), 6.57 (dd, *J* = 8.0, 12.0 Hz, 1H), 5.40 - 5.32 (m, 1H), 5.18 - 5.06 (m, 1H), 4.50 - 4.39 (m, 1H), 4.23 - 4.13 (m, 1H), 3.83 - 3.75 (m, 1H), 3.64 ( d, *J* = 2.8 Hz, 2H), 3.57 (s, 3H), 3.49 - 3.44 (m, 4H), 3.00 - 2.94 (m, 2H), 2.91 - 2.84 (m, 1H), 2.79 - 2.70 (m, 2H), 2.64 (br s, 1H), 2.28 - 2.16 (m, 3H), 2.09 - 1.97 (m, 4H), 1.93 - 1.69 (m, 9H), 1.64 - 1.46 (m, 3H), 1.12 - 1.00 (m, 2H) |
| **I-284^{c}** | ABN | ADB | 844.3 | 11.08 (s, 1H), 9.70 (s, 1H), 8.74 (d, *J* = 7.6 Hz, 1H), 8.38 (s, 1H), 8.24 (s, 1H), 7.25 - 6.98 (m, 1H), 6.96 (d, *J* = 4.4 Hz, 2H), 6.89 - 6.86 (m, 1H), 6.38 (d, *J* = 7.6 Hz, 1H), 5.89 - 5.87 (m, 1H), 5.37 - 5.34 (m, 1H), 4.71 - 4.59 (m, 1H), 4.47 - 4.35 (m, 2H), 4.24 - 4.12 (m, 1H), 4.01 - 3.89 (m, 2H), 3.57 (s, 3H), 3.46 (t, *J* = 6.0 Hz, 2H), 2.97 (t, *J* = 7.6 Hz, 2H), 2.89 - 2.85 (m, 1H), 2.79 - 2.69 (m, 2H), 2.66 - 2.58 (m, 1H), 2.27 - 2.14 (m, 4H), 2.09 - 1.96 (m, 4H), 1.92 - 1.72 (m, 9H), 1.63 - 1.46 (m, 3H), 1.12 - 0.98 (m, 2H) |
| **I-285** | ABN | ABZ | 886.7 | 11.09 (s, 1H), 9.31 (s, 1H), 8.81 (d, *J* = 8.0 Hz, 1H), 8.41 (s, 1H), 8.28 (s, 1H), 7.29 - 6.97 (m, 3H), 6.94 - 6.86 (m, 2H), 5.43 - 5.34 (m, 1H), 4.29 - 4.17 (m, 1H), 4.11 - 4.04 (m, 2H), 3.98 - 3.85 (m, 2H), 3.60 (s, 3H), 3.55 - 3.50 (m, 3H), 3.50 - 3.48 (m, 2H), 3.37 - 3.30 (m, 1H), 3.07 - 2.83 (m, 8H), 2.77 - 2.68 (m, 1H), 2.66 - 2.59 (m, 1H), 2.16 - 2.04 (m, 4H), 2.03 - 1.95 (m, 4H), 1.93 - 1.86 (m, 2H), 1.86 - 1.77 (m, 4H), 1.21 - 1.15 (m, 8H) |
| **I-286^{b}** | ABN | AGQ | 886.4 | 11.09 (s, 1H), 9.40 (s, 1H), 8.73 (d, *J* = 8.0 Hz, 1H), 8.41 (s, 1H), 8.24 (s, 1H), 7.25 - 6.94 (m, 3H), 6.91 (d, *J* = 8.0 Hz, 1H), 6.89 - 6.85 (m, 1H), 5.36 (dd, *J* = 5.2, 12.4 Hz, 1H), 4.27 - 4.19 (m, 1H), 3.71 - 3.64 (m, 2H), 3.60 (s, 1H), 3.57 (s, 3H), 3.06 - 2.92 (m, 7H), 2.91 - 2.85 (m, 1H), 2.76 - 2.69 (m, 2H), 2.65 - 2.57 (m, 2H), 2.54 (s, 1H), 2.18 - 2.07 (m, 4H), 2.01 - 1.75 (m, 12H), 1.68 - 1.49 (m, 5H), 1.25 - 1.13 (m, 5H) |
| **I-287^{c}** | ABN | AGR | 870.3 | 11.10 (s, 1H), 9.71 (s, 1H), 8.76 (d, *J* = 7.6 Hz, 1H), 8.39 (s, 1H), 8.25 (s, 1H), 7.31 - 7.01 (m, 1H), 6.98 (d, *J* = 3.2 Hz, 2H), 6.92 - 6.84 (m, 1H), 6.38 (d, *J* = 7.6 Hz, 1H), 5.43 - 5.30 (m, 1H), 4.74 (s, 4H), 4.39 (s, 4H), 4.29 - 4.18 (m, 1H), 3.59 (d, *J* = 8.8 Hz, 3H), 3.53 - 3.47 (m, 3H), 3.35 - 3.35 (m, 3H), 3.04 - 2.92 (m, 6H), 2.71 - 2.63 (m, 1H), 2.19 - 2.05 (m, 3H), 2.02 - 1.78 (m, 11H), 1.69 - 1.56 (m, 1H), 1.25 - 1.11 (m, 2H) |
| **I-288^{d}** | ABN | AHZ | 874.3 | 10.98 (s, 1H), 9.40 (s, 1H), 8.84 (d, *J* = 7.6 Hz, 1H), 8.38 (s, 1H), 8.30 (s, 1H), 7.53 (t, *J* = 8.2 Hz, 1H), 7.24 - 6.96 (m, 1H), 6.92 (m, 3H), 5.17 - 5.10 (m, 1H), 4.22 - 4.12 (m, 1H), 3.80 (m, 4H), 3.73 (m, 4H), 3.27 - 3.25 (m, 1H), 3.19 (m, 3H), 3.10 - 3.01 (m, 2H), 2.89 - 2.80 (m, 1H), 2.76 (s, 3H), 2.66 - 2.59 (m, 1H), 2.56 (s, 3H), 2.18 - 2.09 (m, 1H), 2.07 - 1.99 (m, 4H), 1.98 - 1.82 (m, 5H), 1.79 - 1.61 (m, 7H), 1.54 - 1.44 (m, 1H), 1.37 - 1.25 (m, 2H), 1.07 - 0.94 (m, 2H) |
| **I-289** | ABN | AGJ | 886.6 | 11.09 (s, 1H), 9.37 (s, 1H), 8.53 (d, *J* = 7.6 Hz, 1H), 8.35 (s, 1H), 8.17 (s, 1H), 7.86 (d, *J* = 8.8 Hz, 1H), 7.30 - 7.00 (m, 1H), 7.00 - 6.93 (m, 2H), 6.92 - 6.82 (m, 1H), 6.63 (d, *J* = 8.0 Hz, 1H), 5.45 - 5.33 (m, 1H), 4.24 - 4.20 (m, 2H), 3.86 - 3.80 (m, 1H), 3.58 (d, *J* = 9.6 Hz, 3H), 3.53 - 3.45 (m, 3H), 3.38 - 3.29 (m, 1H), 3.06 - 2.89 (m, 7H), 2.77 - 2.58 (m, 2H), 2.14 - 2.03 (m, 4H), 2.02 - 1.87 (m, 6H), 1.85 - 1.65 (m, 7H), 1.63 - 1.42 (m, 5H), 1.41 - 1.29 (m, 2H), 1.24 - 1.11 (m, 2H) |
| **I-290** | ABN | ABT | 886.6 | 11.11 (s, 1H), 9.38 (s, 1H), 8.54 (d, *J* = 7.6 Hz, 1H), 8.36 (s, 1H), 8.17 (s, 1H), 7.83 (d, *J* = 8.8 Hz, 1H), 7.15 (t, *J* = 54 Hz, 1H), 7.00 - 6.95 (m, 2H), 6.92 - 6.85 (m, 1H), 6.62 (d, *J* = 7.6 Hz, 1H), 5.43 - 5.32 (m, 1H), 4.93 - 4.64 (m, 1H), 4.34 - 4.18 (m, 2H), 3.82 (s, 1H), 3.52 - 3.46 (m, 5H), 3.40 - 3.33 (m, 2H), 3.09 - 2.92 (m, 7H), 2.91 - 2.84 (m, 1H), 2.73 - 2.67 (m, 1H), 2.65 - 2.60 (m, 1H), 2.18 - 2.12 (m, 1H), 2.08 (d, *J* = 11.6 Hz, 2H), 2.03 - 1.76 (m, 11H), 1.76 - 1.64 (m, 3H), 1.61 - 1.54 (m, 3H), 1.41 - 1.30 (m, 2H), 1.24 - 1.13 (m, 2H) |
| **I-291^{e}** | ABN | AHP | 946.4 | 11.08 (s, 1H), 9.20 - 9.11 (m, 1H), 9.09 (d, *J* = 7.6 Hz, 1H), 9.04 (s, 1H), 8.50 (s, 1H), 8.46 (s, 1H), 8.40 (s, 1H), 8.18 (d, *J* = 8.0 Hz, 1H), 7.31 - 6.83 (m, 4H), 5.37 (dd, *J* = 5.6, 12.4 Hz, 1H), 5.04 (d, *J* = 7.2 Hz, 1H), 4.30 - 4.12 (m, 2H), 3.59 (d, *J* = 8.0 Hz, 3H), 3.54 - 3.46 (m 3H), 3.39 - 3.33 (m, 1H), 3.05 - 2.85 (m, 7H), 2.76 - 2.67 (m, 1H), 2.65 - 2.59 (m, 1H), 2.37 - 2.32 (m, 1H), 2.18 - 2.05 (m, 4H), 2.04 - 1.91 (m, 7H), 1.89 - 1.76 (m, 5H), 1.75 - 1.64 (m, 2H), 1.61 - 1.52 (m, 1H), 1.51 - 1.40 (m, 1H), 1.27 - 1.12 (m, 2H) |
| **I-292** | AGA | AGJ | 940.3 | 11.08 (s, 1H), 9.37 (s, 1H), 8.53 (d, *J* = 7.6 Hz, 1H), 8.34 (s, 1H), 8.17 (s, 1H), 7.80 (d, *J* = 8.8Hz, 1H), 7.68 (dd, *J* = 7.2, 8.4 Hz, 1H), 7.33 (dd, *J* = 4.4, 7.6 Hz, 2H), 7.29 - 6.98 (m, 1H), 6.62 (d, *J* = 7.8 Hz, 1H), 5.09 (dd, *J* = 5.2, 12.8 Hz, 1H), 4.77 (s, 1H), 4.31 - 4.13 (m, 2H), 3.83 (s, 1H), 3.70 (d, *J* = 11.2 Hz, 2H), 3.32 (d, *J* = 6.0 Hz, 4H), 2.94 - 2.81 (m, 5H), 2.65 - 2.52 (m, 2H), 2.42 (s, 3H), 2.09 - 1.97 (m, 3H), 1.89 (d, *J* = 11.2 Hz, 4H), 1.83 - 1.65 (m, 8H), 1.62 - 1.48 (m, 6H), 1.45 - 1.29 (m, 4H), 1.15 - 1.02 (m, 2H) |
| **I-293^{f}** | ABR | AHQ | 886.6 | 11.08 (s, 1H), 9.40 (s, 1H), 8.51 (d, *J* = 7.6 Hz, 1H), 8.32 (s, 1H), 7.90 (d, *J* = 8.0 Hz, 1H), 7.33 - 7.00 (m, 1H), 6.96 (d, *J* = 4.8 Hz, 2H), 6.90 - 6.84 (m, 1H), 6.39 (d, *J* = 7.6 Hz, 1H), 5.36 (dd, *J* = 5.6, 12.4 Hz, 1H), 4.68 (s, 1H), 4.24 - 4.11 (m, 1H), 4.08 - 3.95 (m, 1H), 3.57 (s, 3H), 3.50 - 3.43 (m, 3H), 3.35 - 3.32 (m, 2H), 3.03 - 2.93 (m, 2H), 2.92 - 2.81 (m, 1H), 2.77 - 2.68 (m, 2H), 2.68 - 2.57 (m, 2H), 2.22 - 2.11 (m, 3H), 2.11 - 2.00 (m, 3H), 2.00 - 1.95 (m, 1H), 1.94 - 1.87 (m, 3H), 1.86 - 1.77 (m, 4H), 1.77 - 1.63 (m, 3H), 1.63 - 1.53 (m, 2H), 1.53 - 1.42 (m, 2H), 1.41 - 1.29 (m, 2H), 1.29 - 1.12 (m, 2H), 1.12 - 0.97 (m, 2H) |
| **I-294^{g}** | AGS | AGJ | 900.4 | 11.10 (s, 1H), 9.37 (s, 1H), 8.96 - 8.71 (m, 1H), 8.53 (d, *J* = 7.6 Hz, 1H), 8.35 (s, 1H), 8.17 (s, 1H), 7.80 (d, *J* = 8.8 Hz, 1H), 7.65 - 7.56 (m, 1H), 7.30 - 6.95 (m, 3H), 6.62 (d, *J* = 7.6 Hz, 1H), 5.10 - 4.99 (m, 1H), 4.86 - 4.65 (m, 1H), 4.34 - 4.14 (m, 2H), 3.83 (s, 1H), 3.57 - 3.50 (m, 3H), 3.45 - 3.41 (m, 5H), 3.09 - 2.83 (m, 5H), 2.65 - 2.51 (m, 2H), 2.19 - 1.95 (m, 5H), 1.94 - 1.65 (m, 11H), 1.63 - 1.47 (m, 4H), 1.43 - 1.28 (m, 2H), 1.25 - 1.10 (m, 2H) |
| **I-296** | AIG | AGJ | 901.6 | 11.12 (s, 1H), 9.37 (s, 1H), 8.89 - 8.78 (m, 1H), 8.54 (d, *J* = 8.0 Hz, 1H), 8.35 (d, *J* = 3.2 Hz, 1H), 8.17 (d, *J* = 3.6 Hz, 1H), 7.88 - 7.78 (m, 2H), 7.58 - 7.51 (m, 1H), 7.49 - 7.46 (m, 1H), 7.30 - 6.99 (m, 1H), 6.62 (d, *J* = 8.0 Hz, 1H), 5.14 - 5.05 (m, 1H), 4.85 - 4.71 (m, 1H), 4.36 - 4.34 (m, 1H), 4.27 - 4.26 (m, 2H), 3.82 (m, 1H), 3.71 - 3.61 (m, 2H), 3.53 - 3.48 (m, 1H), 3.06 - 2.83 (m, 5H), 2.65 - 2.56 (m, 1H), 2.17 - 1.96 (m, 7H), 1.96 - 1.65 (m, 11H), 1.64 - 1.47 (m, 6H), 1.43 - 1.28 (m, 2H), 1.25 - 1.11 (m, 2H) |
| **I-297** | AIG | ABB | 873.6 | 11.12 (s, 1H), 9.43 - 9.38 (m, 1H), 8.83 (d, *J* = 8.0 Hz, 2H), 8.40 (d, *J* = 2.4 Hz, 1H), 8.31 - 8.26 (m, 1H), 7.85 - 7.83 (m, 1H), 7.60 - 7.52 (m, 1H), 7.49 - 7.47 (m, 1H), 7.24 - 6.95 (m, 1H), 6.91 (d, *J* = 8.0 Hz, 1H), 5.15 - 5.05 (m, 1H), 4.37 - 4.30 (m, 1H), 4.30 - 4.16 (m, 2H), 3.79 (m, 4H), 3.73 (m, 4H), 3.69 - 3.59 (m, 2H), 3.54 - 3.47 (m, 1H), 3.03 - 2.84 (m, 4H), 2.64 - 2.56 (m, 1H), 2.52 (m, 2H), 2.16 - 1.96 (m, 7H), 1.95 - 1.70 (m, 8H), 1.58 (m, 1H), 1.24 - 1.09 (m, 2H) |
| **I-298** | AGG | AGJ | 856.6 | 11.09 (s, 1H), 9.41 (s, 1H), 8.58 - 8.50 (m, 1H), 8.41 (s, 1H), 8.18 (s, 1H), 7.87 (d, *J* = 8.4 Hz, 1H), 7.72 - 7.05 (m, 4H), 6.64 (d, *J* = 7.8 Hz, 1H), 5.10 (dd, *J* = 5.2, 12.8 Hz, 1H), 4.68 - 4.52 (m, 1H), 4.32 - 4.23 (m, 1H), 4.12 - 4.01 (m, 3H), 3.74 - 3.63 (m, 4H), 3.24 - 3.02 (m, 4H), 2.95 - 2.80 (m, 3H), 2.64 - 2.55 (m, 2H), 2.33 - 2.20 (m, 3H), 2.08 - 2.00 (m, 1H), 1.85 - 1.67 (m, 6H), 1.62 - 1.28 (m, 11H) |
| **I-299** | AHE | AEH | 870.5 | 11.11 (d, *J* = 6.4 Hz, 1H), 9.56 - 9.42 (m, 1H), 8.78 (d, *J* = 7.6 Hz, 1H), 8.44 - 8.38 (m, 1H), 8.26 (d, *J* = 4.4 Hz, 1H), 7.67 - 7.56 (m, 1H), 7.29 - 6.96 (m, 3H), 6.91 - 6.42 (m, 2H), 5.34 - 5.00 (m, 2H), 4.82 - 4.71 (m, 1H), 4.29 - 4.16 (m, 1H), 3.88 - 3.56 (m, 7H), 3.52 (s, 3H), 3.02 - 2.81 (m, 5H), 2.70 - 2.54 (m, 2H), 2.19 - 1.72 (m, 14H), 1.71 - 1.58 (m, 1H), 1.27 - 1.08 (m, 2H) |
| **I-30**0^{h} | ABN | AET | 921.3 | 11.10 (s, 1H), 9.74 (br s, 1H), 9.33 (s, 1H), 8.77 - 8.60 (m, 4H), 8.33 - 8.21 (m, 2H), 7.84 (d, *J* = 7.6 Hz, 1H), 7.34 - 6.83 (m, 4H), 6.72 (d, *J* = 7.6 Hz, 1H), 5.37 (dd, *J* = 5.2, 12.0 Hz, 1H), 5.11 - 4.97 (m, 1H), 4.31 - 4.14 (m, 1H), 4.05- 3.89 (m, 1H), 3.58 (d, *J* = 9.2 Hz, 3H), 3.54 - 3.46 (m, 4H), 3.32 - 3.30 (m, 1H), 3.05 - 2.99 (m, 2H), 2.98 - 2.95 (m, 2H), 2.95 - 2.91(m, 2H), 2.91 - 2.84 (m, 1H), 2.78 - 2.68 (m, 1H), 2.65 - 2.59 (m, 1H), 2.52 - 2.51 (m, 2H), 2.30 - 2.18 (m, 1H), 2.14 - 2.07 (m, 2H), 2.07 - 2.03 (m, 2H), 2.02 - 1.98 (m, 2H), 1.97 -1.93(m, 2H), 1.92 - 1.86 (m, 2H), 1.86 - 1.83 (m, 2H), 1.83 -1.78 (m, 3H), 1.77 - 1.75 (m, 1H), 1.73 - 1.65 (m, 1H), 1.29 - 1.07 (m, 2H) |
| **I-369** | AKE | AEH | 787.4 | 11.09 (s, 1H), 9.50 (d, *J* = 6.0 Hz, 1H), 8.78 (d, *J* = 7.6 Hz, 1H), 8.38 (d, *J* = 4.4 Hz, 1H), 8.26 (d, *J* = 5.6 Hz, 1H), 7.60 (dd, *J* = 7.2, 8.4 Hz, 1H), 7.25 - 6.94 (m, 3H), 6.90 - 6.38 (m, 2H), 5.31 - 5.00 (m, 2H), 4.81 - 4.71 (m, 1H), 4.24 - 4.09 (m, 1H), 3.86 - 3.71 (m, 2H), 3.66 - 3.56 (m, 3H), 3.52 - 3.39 (m, 3H), 3.30 (d, *J* = 6.4 Hz, 2H), 2.94 - 2.81 (m, 1H), 2.63 - 2.54 (m, 2H), 2.08 - 1.91 (m, 5H), 1.90 - 1.80 (m, 2H), 1.79 - 1.66 (m, 2H), 1.64 - 1.55 (m, 1H), 1.21 - 1.06 (m, 2H) |
| **I-375** | AJK | AEH | 801.4 | 11.09 (s, 1H), 9.50 (d, *J* = 6.4 Hz, 1H), 8.78 (d, *J* = 8.0 Hz, 1H), 8.39 (d, *J* = 4.0 Hz, 1H), 8.26 (d, *J=* 5.6 Hz, 1H), 7.59 (dd, *J* = 7.6, 8.4 Hz, 1H), 7.26 - 6.41 (m, 5H), 5.31 - 5.00 (m, 2H), 4.77 (d, *J* = 16.4 Hz, 1H), 4.24 - 4.12 (m, 1H), 3.85 - 3.72 (m, 2H), 3.65 - 3.58 (m, 1H), 3.52 - 3.45 (m, 2H), 3.44 - 3.37 (m, 2H), 3.24 (d, *J* = 6.4 Hz, 2H), 2.94 - 2.81 (m, 1H), 2.63 - 2.53 (m, 2H), 2.08 - 1.99 (m, 4H), 1.98 - 1.88 (m, 2H), 1.88 - 1.77 (m, 4H), 1.76 - 1.59 (m, 3H), 1.25 - 1.06 (m, 2H) |
| **I-398** | AQW | AQV | 915.6 | 11.11 (s, 1H), 9.76 (s, 1H), 9.04 (s, 1H), 8.88 - 8.72 (m, 1H), 8.19 (s, 1H), 8.08 (d, *J* = 6.0 Hz, 1H), 7.76 - 7.44 (m, 2H), 7.32 - 7.00 (m, 4H), 6.73 - 6.60 (m, 1H), 5.05 (dd, *J* = 5.2, 12.8 Hz, 1H), 4.30 - 4.18 (m, 1H), 3.92 - 3.81 (m, 2H), 3.52 - 3.46 (m, 4H), 3.25 - 3.18 (m, 2H), 3.12 - 3.02 (m, 2H), 2.98 - 2.84 (m, 2H), 2.80 (d, *J* = 4.4 Hz, 3H), 2.64 - 2.53 (m, 2H), 2.14 - 1.89 (m, 7H), 1.88 - 1.47 (m, 13H), 1.41 - 1.31 (m, 2H), 1.24 - 1.13 (m, 2H) |
| **I-399** | AQW | ATK | 915.6 | 11.09 (s, 1H), 9.67 (s, 1H), 8.93 (s, 1H), 8.27 - 8.13 (m, 2H), 7.63 - 7.54 (m, 1H), 7.31 - 7.02 (m, 4H), 7.00 (dd, *J* = 1.3, 5.3 Hz, 1H), 6.72 - 6.61 (m, 2H), 5.05 (dd, *J* = 5.3, 12.8 Hz, 1H), 4.22 -4.13 (m, 1H), 3.92 - 3.84 (m, 2H), 3.49 - 3.46 (m, 3H), 2.92 -2.80 (m, 2H), 2.61 (d, *J* = 2.9 Hz, 1H), 2.58 - 2.54 (m, 2H), 2.34 - 2.29 (m, 2H), 2.12 (s, 3H), 2.09 (d, *J* = 7.3 Hz, 2H), 2.06 - 1.99 (m, 3H), 1.91 - 1.80 (m, 4H), 1.78 - 1.60 (m, 8H), 1.59 - 1.45 (m, 4H), 1.37 - 1.28 (m, 2H), 1.05 - 0.94 (m, 2H) |
| **I-402** | AQW | AZW | 915.6 | 11.09 (s, 1H), 9.68 (s, 1H), 8.93 (s, 1H), 8.23 - 8.18 (m, 1H), 8.13 (d, *J* = 5.2 Hz, 1H), 7.62 - 7.54 (m, 1H), 7.32 - 7.02 (m, 4H), 7.00 (dd, *J* = 1.2, 5.2 Hz, 1H), 6.80 (d, *J* = 7.2 Hz, 1H), 6.71 - 6.64 (m, 1H), 5.05 (dd, *J* = 5.2, 12.8 Hz, 1H), 4.22 - 4.13 (m, 1H), 3.67 - 3.58 (m, 3H), 2.96-2.82 (m, 2H), 2.63- 2.59 (m, 1H), 2.59- 2.55 (m, 2H), 2.35 - 2.31 (m, 2H), 2.15 - 2.07 (m, 5H), 2.06- 1.97 (m, 4H), 1.94 - 1.73 (m, 7H), 1.72 - 1.46 (m, 7H), 1.36 - 1.10 (m, 5H), 1.07 - 0.93 (m, 2H) |
| **I-403ⁱ** | AIT | ABB | 866.5 | 11.10 (s, 1H), 9.52 (s, 1H), 9.10 - 9.03 (m, 1H), 8.86 (d, *J* = 8.0 Hz, 1H), 8.34 (s, 1H), 8.02 - 7.92 (m, 2H), 7.66 (d, *J* = 8.4 Hz, 2H), 7.58 (t, *J* = 7.6 Hz, 1H), 7.44 - 7.13 (m, 1H), 7.08 (d, *J* = 8.8 Hz, 1H), 7.03 (d, *J* = 7.2 Hz, 1H), 6.94 (d, *J* = 8.0 Hz, 1H), 6.68 - 6.59 (m, 1H), 5.10 - 4.97 (m, 1H), 4.45 - 4.26 (m, 2H), 3.82 (s, 3H), 3.77 - 3.68 (m, 4H), 3.66 (s, 1H), 3.55 - 3.48 (m, 2H), 3.27 - 3.18 (m, 2H), 3.17 - 3.05 (m, 2H), 3.04 - 2.92 (m, 1H), 2.92 - 2.79 (m, 1H), 2.62 - 2.54 (m, 1H), 2.48 - 2.41 (m, 2H), 2.23 - 2.12 (m, 1H), 2.06 - 1.96 (m, 2H), 1.91 - 1.69 (m, 4H), 1.63 - 1.49 (m, 1H) |
| **I-405^{j}** | AJY | ABB | 877.5 | 11.12 (s, 1H), 9.38 (s, 1H), 8.82 (d, *J* = 8.0 Hz, 1H), 8.35 (s, 1H), 8.28 (s, 1H), 8.13 (s, 1H), 7.82 - 7.70 (m, 2H), 7.47 (dd, *J* = 1.6, 8.4 Hz, 1H), 7.27 - 6.85 (m, 5H), 5.44 (dd, *J* = 5.6, 12.8 Hz, 1H), 4.62 (t, *J* = 5.2 Hz, 2H), 4.15 - 4.02 (m, 1H), 3.84 (t, *J* = 5.2 Hz, 2H), 3.81 -3.75 (m, 4H), 3.75 - 3.68 (m, 4H), 3.20 (d, *J* = 6.4 Hz, 2H), 2.99 - 2.87 (m, 1H), 2.84 (s, 3H), 2.80 - 2.68 (m, 1H), 2.66 - 2.60 (m, 1H), 2.10 - 1.99 (m, 1H), 1.98 - 1.88 (m, 2H), 1.72 - 1.57 (m, 4H), 1.51 - 1.38 (m, 1H), 1.04 - 0.87 (m, 2H) |
| **I-406** | AJQ | ABB | 877.5 | 11.12 (s, 1H), 9.38 (s, 1H), 8.82 (d, *J* = 8.0 Hz, 1H), 8.42 (s, 1H), 8.35 (s, 1H), 8.27 (s, 1H), 7.75 (s, 1H), 7.67 (d, *J* = 8.8 Hz, 1H), 7.30 (dd, *J* = 1.6, 8.8 Hz, 1H), 7.24 - 6.84 (m, 5H), 5.43 (dd, *J* = 5.6, 12.8 Hz, 1H), 4.62 (t, *J* = 4.8 Hz, 2H), 4.20 - 4.03 (m, 1H), 3.91 (t, *J* = 5.2 Hz, 2H), 3.83 - 3.63 (m, 8H), 3.26 (d, *J* = 6.4 Hz, 2H), 2.98 - 2.88 (m, 1H), 2.87 (s, 3H), 2.74 (dd, *J* = 4.4, 12.8 Hz, 1H), 2.66 - 2.59 (m, 1H), 2.09 - 2.01 (m, 1H), 2.00 - 1.92 (m, 2H), 1.78 - 1.70 (m, 2H), 1.70 - 1.60 (m, 2H), 1.59 - 1.46 (m, 1H), 1.12 - 0.97 (m, 2H) |
| **I-446** | AMV | AEH | 811.4 | 11.11 (s, 1H), 9.50 (d, *J* = 5.6 Hz, 1H), 8.78 (d, *J* = 7.6 Hz, 1H), 8.37 (d, *J* = 4.0 Hz, 1H), 8.26 (d, *J* = 5.6 Hz, 1H), 7.28 - 6.93 (m, 4H), 6.45 (d, *J* = 7.6 Hz, 1H), 5.43 - 5.34 (m, 1H), 5.28 (s, 1H), 4.77 (d, *J* = 18.4 Hz, 1H), 4.23 - 4.11 (m, 1H), 3.85 - 3.71 (m, 2H), 3.65 (s, 1H), 3.63 - 3.57 (m, 1H), 3.51 (t, *J* = 6.0 Hz, 1H), 3.48 - 3.40 (m, 10H), 3.26 (d, *J* = 6.4 Hz, 1H), 2.95 - 2.83 (m, 1H), 2.76 - 2.69 (m, 1H), 2.55 (s, 1H), 2.07 - 1.93 (m, 3H), 1.92 - 1.78 (m, 3H), 1.77 - 1.65 (m, 2H), 1.64 - 1.54 (m, 1H), 1.25 - 1.11 (m, 2H) |
| **I-479** | AVD | ATU | 891.5 | 11.12 - 11.02 (m, 1H), 9.51 (d, J = 5.2 Hz, 1H), 8.78 (d, J = 7.6 Hz, 1H), 8.45 (d, J = 4.0 Hz, 1H), 8.26 (d, J = 5.6 Hz, 1H), 7.27 (d, J = 9.2 Hz, 2H), 7.16 - 6.42 (m, 7H), 5.41 - 5.03 (m, 2H), 4.83 - 4.72 (m, 1H), 4.56 - 4.47 (m, 1H), 4.00 - 3.89 (m, 2H), 3.84 - 3.72 (m, 2H), 3.65 - 3.58 (m, 2H), 3.54 - 3.45 (m, 4H), 3.29 (s, 3H), 2.98 (s, 3H), 2.96 - 2.82 (m, 4H), 2.60 (m, 1H), 2.12 - 1.89 (m, 9H) |
| **I-494** | AOD | AOI | 847.5 | 11.08 (s, 1H), 9.63 (s, 1H), 9.37 (dd, *J =* 7.2, 1.6 Hz, 1H), 8.90 (dd, *J =* 4.0, 1.6 Hz, 1H), 8.66 (s, 1H), 8.02 (s, 1H), 7.33 (dd, *J* = 7.2, 4.0 Hz, 1H), 6.97 (d, *J* = 4.4 Hz, 2H), 6.91 -6.83 (m, 1H), 5.36 (dd, *J =* 12.8, 5.2 Hz, 1H), 4.06 - 3.99 (m, 1H), 3.83 -3.79 (m, 4H), 3.57 (s, 3H), 3.20 -3.16 (m, 4H), 3.07 -2.99 (m, 5H), 2.96 -2.83 (m, 4H), 2.76 - 2.60 (m, 6H), 2.04 -1.96 (m, 3H), 1.86 -1.51 (m, 13H) |
| **I-502** | AQR | AHZ | 858.3 | 11.20 - 10.98 (m, 1H), 9.62 - 9.36 (m, 1H), 8.55 (d, *J =* 7.6 Hz, 1H), 8.32 (s, 1H), 8.18 (s, 1H), 8.00 - 7.87 (m, 1H), 7.64 - 7.51 (m, 1H), 7.23 - 6.92 (m, 3H), 6.43 (d, *J* = 7.6 Hz, 1H), 6.15 (d, *J =* 8.4 Hz, 1H), 5.09 - 4.98 (m, 1H), 4.43 - 4.28 (m, 1H), 4.24 - 4.08 (m, 1H), 3.52 - 3.44 (m, 3H), 2.97 - 2.79 (m, 1H), 2.62 - 2.55 (m, 1H), 2.43 - 2.35 (m, 2H), 2.23 - 2.17 (m, 5H), 2.07 - 1.99 (m, 5H), 1.95 - 1.87 (m, 2H), 1.79 - 1.70 (m, 6H), 1.50 - 1.37 (m, 3H), 1.34 - 1.23 (m, 2H), 1.14 (s, 6H), 1.07 - 0.96 (m, 2H) |
| **I-504** | AQR | AVV | 872.6 | 11.11 (s, 1H), 9.55 - 9.33 (m, 1H), 8.76 (d, *J =* 7.6 Hz, 1H), 8.25 (s, 1H), 7.59 (dd, *J =* 7.2, 8.4 Hz, 1H), 7.30 - 6.94 (m, 3H), 6.69 (d, *J =* 8.0 Hz, 1H), 6.16 (d, *J* = 7.2 Hz, 1H), 5.05 (dd, *J* = 5.2, 12.8 Hz, 1H), 4.50-4.48 (m, 1H), 4.25 - 4.13 (m, 1H), 3.76 - 3.59 (m, 3H), 3.56 - 3.47 (m, 2H), 3.22 (s, 3H), 2.27 - 2.18 (m, 5H), 2.10 - 1.98 (m, 6H), 1.96 - 1.87 (m, 2H), 1.80 - 1.65 (m, 7H), 1.52 - 1.22 (m, 7H), 1.16 (s, 6H), 1.06 - 0.97 (m, 2H) |

| | | | | |
|---|---|---|---|---|
| ^{a}The reaction for Method 3 was run anywhere from 2-48 hrs at room temperature. A mixed solvent of ACN:DMF could be used for the reaction as well. The final products were isolated under standard purification techniques including reverse HPLC, silica gel chromatography, and prep-TLC with appropriate solvent conditions. ^{b}A mixed solvent of ACN:DMF (4:1) was utilized for the reaction. ^{c}A mixed solvent of ACN:DMF (3:1) was utilized for the reaction. ^{d}A mixed solvent of ACN:DMF (1:1) was utilized for the reaction. ^{e}A mixed solvent of ACN:DMF (10:1) was utilized for the reaction. ^{f}A mixed solvent of ACN:DMF (5:1) was utilized for the reaction. ^{g}A mixed solvent of ACN:DMF (2:1) was utilized for the reaction. ^{h}The product of Step 1 was then deprotected with HCl/dioxane in DCM at rt for 16 h. ⁱ 12 hr at rt, ACN/DMF 6:1. ^{j} 3 hr at rt, ACN/DMF 10:1. | | | | |

### Example 20. Synthesis of 2-[2-(Cyclopropylmethylamino)-4-pyridyl]-N-[3-(difluoromethyl)-1-[4-[1-[4-[3-[1-(2,6-dioxo -3-piperidyl)-3-methyl-2-oxo-benzimidazol-4-yl]propoxy]-1-piperidyl]ethyl]cyclohexyl]pyrazol-4-yl]oxazole-4-carboxamide (I-301)

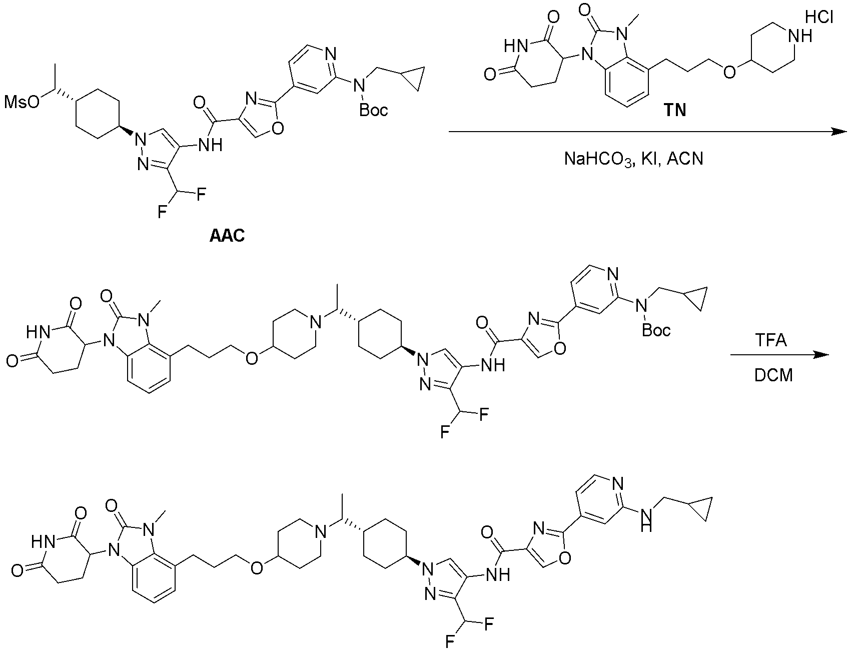

### Step 1 - Tert-butyl N-(cyclopropylmethyl)-N-[4-[4-[[3-(difluoromethyl)-1-[4-[1-[4-[3-[1-(2,6-dioxo-3- piperidyl)-3-methyl-2-oxo-benzimidazol-4-yl]propoxy]-1-piperidyl]ethyl]cyclohexyl]pyrazol-4-yl]carbamoyl]oxazol-2-yl]-2-pyridyl]carbamate

To a solution of 3-[3-methyl-2-oxo-4-[3-(4-piperidyloxy)propyl]benzimidazol-1-yl]piperidine-2,6- dione (218 mg, 500 umol, HCl, Intermediate TN) in ACN (6 mL) was added NaHCO₃ (105 mg, 1.25 mmol), KI (4.16 mg, 25.0 umol) and 1-[4-[4-[[2-[2-[tert-butoxycarbonyl(cyclopropylmethyl)amino]- 4-pyridyl]oxazole-4-carbonyl]amino]-3-(difluoromethyl)pyrazol-1-yl]cyclohexyl]ethylmethanesulfonate (170 mg, 250 umol, Intermediate AAC), and the mixture was stirred at 80 °C for 48 hrs. On completion, the reaction mixture was filtered and concentrated under reduced pressure to give a residue. The residue was purified by prep-HPLC (FA condition) to give the title compound (58 mg, 23% yield) as white solid. ¹H NMR (400MHz, DMSO-*d*₆) δ 11.08 (s, 1H), 9.75 (s, 1H), 9.00 (s, 1H), 8.59 (d, *J* = 5.2 Hz, 1H), 8.29 (s, 1H), 8.17 (s, 1H), 7.68 (d, *J* = 5.2 Hz, 1H), 7.66 - 7.49 (m, 2H), 7.17 - 6.99 (m, 1H), 6.96 (br d, *J* = 4.4 Hz, 2H), 6.89 - 6.84 (m, 1H), 5.36 (dd, *J* = 4.4, 11.6 Hz, 1H), 4.26 - 4.12 (m, 1H), 3.86 (d, *J* = 7.2 Hz, 2H), 3.57 (s, 3H), 2.98 - 2.94 (m, 2H), 2.69 - 2.62 (m, 3H), 2.23 - 2.18 (m, 2H), 2.13 - 1.96 (m, 6H), 1.85 - 1.72 (m, 7H), 1.52 (s, 9H), 1.41 - 1.33 (m, 2H), 1.25 - 0.98 (m, 5H), 0.89 (d, *J =* 6.4 Hz, 3H), 0.41 - 0.39 (m, 2H), 0.27 - 0.21 (m, 2H).

### Step 2 - 2-[2-(Cyclopropylmethylamino)-4-pyridyl]-N-[3-(difluoromethyl)-1-[4-[1-[4-[3-[1-(2,6-dioxo -3-piperidyl)-3-methyl-2-oxo-benzimidazol-4-yl]propoxy]-1-piperidyl]ethyl]cyclohexyl]pyrazol-4-yl]oxazole-4-carboxamide

To a solution of tert-butyl N-(cyclopropylmethyl)-N-[4-[4-[[3-(difluoromethyl)-1-[4-[1-[4-[3-[1-(2,6- dioxo-3-piperidyl)-3-methyl-2-oxo-benzimidazol-4-yl]propoxy]-1-piperidyl]ethyl]cyclohexyl]pyrazol-4-yl]carbamoyl]oxazol-2-yl]-2-pyridyl]carbamate (55 mg, 55.9 umol) in DCM (1.2 mL) was added TFA (924 mg, 8.10 mmol), and the mixture was stirred at 10 °C for 3 hrs. On completion, the mixture was concentrated *in vacuo* to give a residue. The residue was purified by prep-HPLC (column: Phenomenex Synergi C18 150*25*10 um; mobile phase: [water (0.225%FA)-ACN]) to give the title compound (6.33 mg, 11% yield, FA) as white solid. ¹H NMR (400MHz, DMSO-*d*₆) δ 11.09 (s, 1H), 9.70 (s, 1H), 8.92 (s, 1H), 8.20 - 8.12 (m, 2H), 7.33 - 6.99 (m, 4H), 6.96 (d, *J =* 4.8 Hz, 2H), 6.90 - 6.84 (m, 1H), 5.36 (dd, *J =* 5.2*,* 12.8 Hz, 1H), 4.21 - 4.12 (m, 1H), 3.57 (s, 3H), 3.44 (t, *J* = 5.2 Hz, 2H), 3.25 - 3.23 (m, 1H), 3.18 (t, *J* = 6.0 Hz, 2H), 2.99 - 2.94 (m, 2H), 2.91 - 2.82 (m, 1H), 2.67 - 2.58 (m, 3H), 2.44 - 2.36 (m, 1H), 2.26 - 2.14 (m, 2H), 2.11 - 1.96 (m, 4H), 1.89 - 1.68 (m, 7H), 1.51 - 1.31 (m, 3H), 1.13 - 0.96 (m, 3H), 0.89 (d, *J* = 6.4 Hz, 3H), 0.49 - 0.41 (m, 2H), 0.26 - 0.19 (m, 2H); LC-MS (ESI⁺) *m*/*z* 883.3 (M+H)⁺.

### Example 21. Synthesis of N-[3-(difluoromethyl)-1-[4-[[4-[3-[1-(2,6-dioxo-3-piperidyl)-3-methyl-2-oxo-benzimidazol -4-yl]propoxy]-1-piperidyl]methyl]cyclohexyl]pyrazol-4-yl]-5-[[(1R,2S)-2-hydroxycyclohexyl]amino]pyrazolo[1,5-a]pyrimidine-3-carboxamide (I-302)

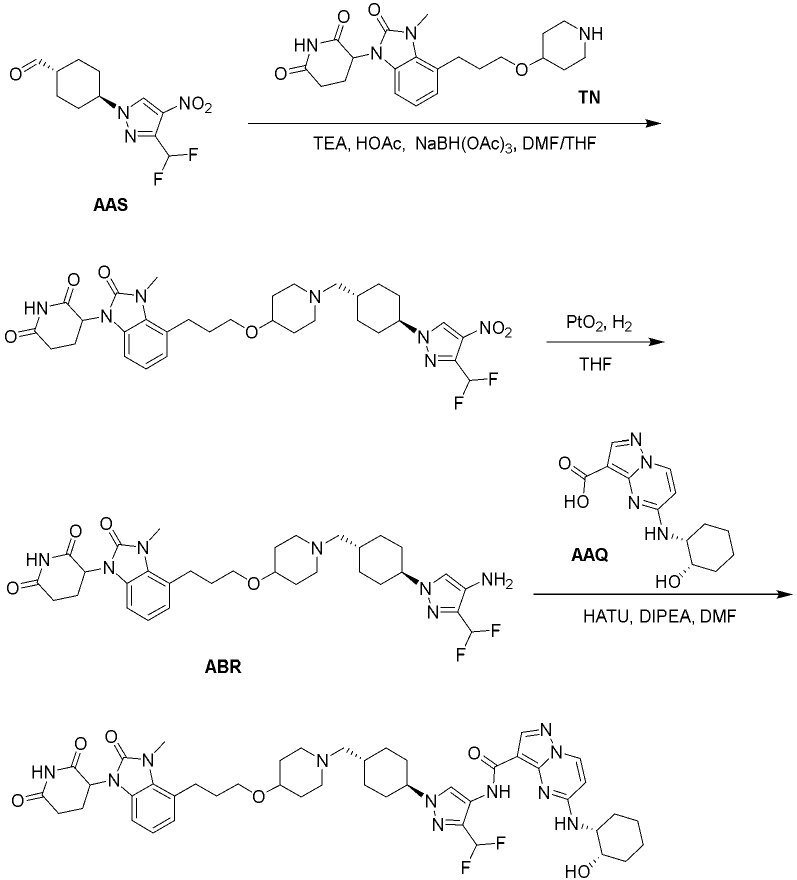

### Step 1 - 3-[4-[3-[[1-[[4-[3-(Difluoromethyl)-4-nitro-pyrazol-1-yl]cyclohexyl]methyl]-4-piperidyl] oxy]propyl]-3-methyl-2-oxo-benzimidazol-1-yl]piperidine-2,6-dione

To a solution of 3-[3-methyl-2-oxo-4-[3-(4-piperidyloxy)propyl]benzimidazol-1-yl]piperidine-2,6- dione (271 mg, 622 umol, HCl, Intermediate TN) in DMF (1.00 mL) and THF (2.00 mL) was added TEA (62.9 mg, 622 umol), the mixture was stirred at 20 °C for 15 mins. Then 4-[3-(difluoromethyl)-4-nitro-pyrazol-1-yl] cyclohexanecarbaldehyde (170 mg, 622 umol, Intermediate AAS) and HOAc (74.7 mg, 1.24 mmol) were added to the mixture, and the reaction mixture was stirred at 20 °C for 30 minutes. Then NaBH(OAc)₃ (197 mg, 933 umol) was added to the mixture, the reaction mixture was stirred at 25°C for 12 hrs. On completion, the mixture was quenched by addition H₂O (0.5 mL), then the mixture was concentrated *in vacuo* to give a residue. The residue was purified by prep-HPLC (column: Phenomenex Synergi Max-RP 250*50mm*10 um; mobile phase: [water (0.2% FA)-ACN]; B%: 12%-42%, 11 min) to give the title compound (290 mg, 70% yield) as white solid. ¹H NMR (400MHz, DMSO-*d₆*) δ 11.09 (s, 1H), 9.05 (s, 1H), 7.31 (t, *J* = 53.2 Hz, 1H), 6.97 (d, *J* = 4.4 Hz, 2H), 6.92 - 6.84 (m, 1H), 5.37 (dd, *J* = 5.2, 12.4Hz, 1H), 4.34 - 4.25 (m, 1H), 3.58 (s, 3H), 3.47 (t, *J =* 5.2 Hz, 2H), 3.36 - 3.31 (m, 1H), 2.98 (t, *J =* 7.6 Hz, 2H), 2.94 - 2.85 (m, 1H), 2.78 - 2.59 (m, 4H), 2.27 - 2.14 (m, 4H), 2.13 - 2.05 (m, 2H), 2.04 - 1.97 (m, 1H), 1.94 - 1.73 (m, 8H), 1.67 - 1.57 (m, 1H), 1.55 - 1.46 (m, 2H), 1.11 - 0.99 (m, 2H).

### Step 2 - 3-[4-[3-[[1-[[4-[4-Amino-3-(difluoromethyl)pyrazol-1-yl]cyclohexyl]methyl]-4-piperidyl] oxy]propyl]-3-methyl-2-oxobenzimidazol-1-yl]piperidine-2,6-dione (Intermediate ABR)

To a solution of 3-[4-[3-[[1-[[4-[3-(difluoromethyl)-4-nitro-pyrazol-1-yl]cyclohexyl]methyl]-4- piperidyl]oxy]propyl]-3-methyl-2-oxo-benzimidazol-1-yl]piperidine-2,6-dione (290 mg, 440 umol) in THF (10.0 mL) was added PtO₂ (10.0 mg, 44.0 umol). The mixture was stirred at 15 °C for 2 hrs under H₂ (15 psi). On completion, the mixture was filtered and concentrated *in vacuo* to give the title compound (260 mg, 93% yield) as white solid. ¹H NMR (400MHz, DMSO-*d₆*) δ 11.09 (s, 1H), 7.13 (s, 1H), 7.03 - 6.91 (m, 2H), 6.90 - 6.65 (m, 2H), 5.40 - 5.33 (m, 1H), 4.03 (s, 2H), 4.00 - 3.90 (m, 1H), 3.62 - 3.58 (m, 1H), 3.57 (s, 3H), 3.48 - 3.42 (m, 2H), 3.30 - 3.21 (m, 1H), 3.00 - 2.92 (m, 2H), 2.91 - 2.83 (m, 1H), 2.77 - 2.68 (m, 1H), 2.66 - 2.56 (m, 3H), 2.12 - 2.04 (m, 2H), 2.03 - 1.91 (m, 5H), 1.88 - 1.78 (m, 6H), 1.77 - 1.72 (m, 1H), 1.70 - 1.56 (m, 2H), 1.55 - 1.48 (m, 1H), 1.04 - 0.90 (m, 2H).

### Step 3 - N-[3-(difluoromethyl)-1-[4-[[4-[3-[1-(2,6-dioxo-3-piperidyl)-3-methyl-2-oxo-benzimidazol -4-yl]propoxy]-1-piperidyl]methyl]cyclohexyl]pyrazol-4-yl]-5-[[(1R,2S)-2-hydroxycyclohexyl]amino]pyrazolo[1,5-a]pyrimidine-3-carboxamide

To a solution of 3-[4-[3-[[1-[[4-[4-amino-3-(difluoromethyl)pyrazol-1-yl]cyclohexyl]methyl]-4- piperidyl]oxy]propyl]-3-methyl-2-oxo-benzimidazol-1-yl]piperidine-2,6-dione (50.0 mg, 79.6 umol), 5-[[(1R,2S)-2-hydroxycyclohexyl]amino]pyrazolo[1,5-a]pyrimidine-3-carboxylic acid (20.8 mg, 75.2 umol, Intermediate AAQ) in DMF (1.00 mL) was added PYBOP (62.1 mg, 119 umol), DIEA (51.4 mg, 398 umol) and DMAP (973 ug, 7.97 umol). The mixture was stirred at 50 °C for 16 hrs. On completion, the mixture was quenched with H₂O (1 mL) and concentrated *in vacuo.* The mixture was purified by prep-HPLC (column: Phenomenex Synergi C18 150*25*10 um; mobile phase: [water (0.225% FA)-ACN]; B%: 13%-43%, 10 min) to give the title compound as cis-racemic (30.7 mg, 41% yield) as white solid. ¹H NMR (400MHz, DMSO-*d₆*) δ 11.09 (s, 1H), 9.37 (s, 1H), 8.54 (d, *J* = 7.6 Hz, 1H), 8.33 (s, 1H), 8.17 (s, 1H), 7.83 (d, *J =* 8.8 Hz, 1H), 7.29 - 7.00 (m, 1H), 6.97 (d, *J* = 4.8 Hz, 2H), 6.91 - 6.84 (m, 1H), 6.63 (d, *J =* 7.6 Hz, 1H), 5.41 - 5.32 (m, 1H), 4.32 - 4.24 (m, 1H), 4.22 - 4.15 (m, 1H), 3.86 - 3.82 (m, 1H), 3.58 (s, 3H), 3.48 - 3.45 (m, 2H), 3.32 - 3.24 (m, 2H), 3.00 - 2.94 (m, 2H), 2.92 - 2.83 (m, 1H), 2.77 - 2.70 (m, 1H), 2.67 - 2.59 (m, 3H), 2.15 - 2.08 (m, 2H), 2.08 - 1.98 (m, 5H), 1.95 - 1.86 (m, 2H), 1.85 - 1.79 (m, 4H), 1.78 - 1.65 (m, 4H), 1.64 - 1.52 (m, 5H), 1.50 - 1.32 (m, 4H), 1.12 - 0.97 (m, 2H); LC-MS (ESI⁺) *m*/*z* 886.5 (M+H)⁺.

### Example 22. Synthesis of 5-[(SR)-5-amino-3,3-difluoro-1-piperidyl]-N-[3-(difluoromethyl)-1-[4-[[4-[3-[1-(2,6-dioxo- 3-piperidyl)-3-methyl-2-oxo-benzimidazol-4-yl]propoxy]-1-piperidyl]methyl]cyclohexyl]pyrazol-4-yl]pyrazolo[1,5-a]pyrimidine-3-carboxamide (I-303)

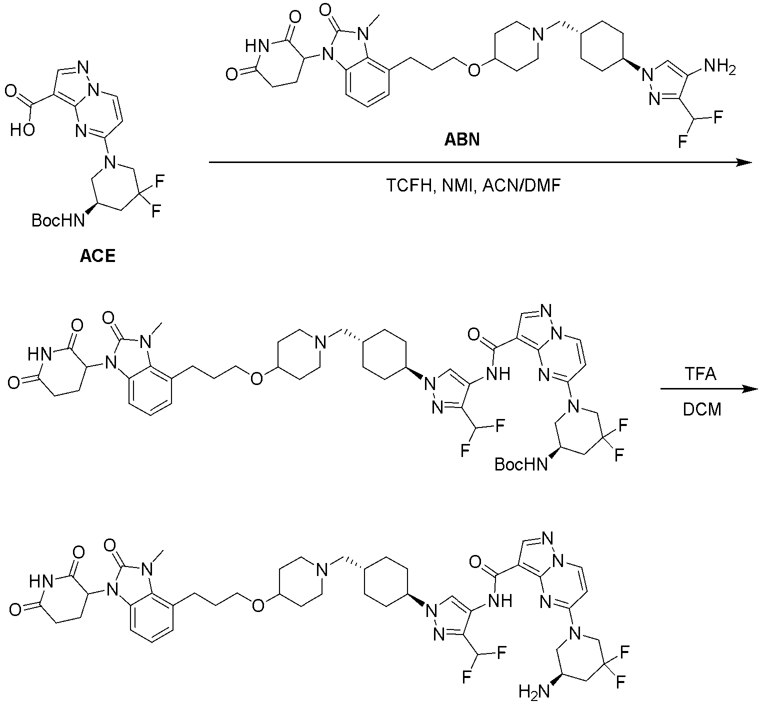

### Step 1 - Tert-butyl N-[(3R)-1-[3-[[3-(difluoromethyl)-1-[4-[[4-[3-[1-(2,6-dioxo-3-piperidyl)-3-methyl -2-oxo-benzimidazol-4-yl]propoxy]-1-piperidyl]methyl]cyclohexyl]pyrazol-4-yl]carbamoyl]pyrazolo[1,5-a]pyrimidin-5-yl]-5,5-difluoro-3-piperidyl]carbamate

To a solution of and 5-[(5R)-5-(tert-butoxycarbonylamino)-3,3-difluoro-1-piperidyl]pyrazolo[1,5-a] pyrimidine-3-carboxylic acid (50.0 mg, 125 umol, Intermediate ACE) and [chloro(dimethylamino)methylene]-dimethyl- ammonium;hexafluorophosphate (42.3 mg, 150 umol) in ACN (2 mL) was added 1-methylimidazole (36.1 mg, 440 umol), and the mixture was stirred at 15 °C for 0.1 hr. After that, 3-[4-[3-[[1-[[4-[4-amino-3- (difluoromethyl)pyrazol-1-yl]cyclohexyl]methyl]-4-piperidyl]oxy]propyl]-3-methyl-2-oxo-benzimidazol-1-yl]piperidine-2,6-dione (78.9 mg, 125 umol, Intermediate ABN) was added, and the mixture was stirred at 15 °C for 40 hrs. On completion, the reaction mixture was quenched with water (0.2 mL). Then the residue was purified by prep-HPLC (FA condition) to give the title compound (70.0 mg, 55% yield) as white solid. LC-MS (ESI⁺) m/z 1007.3 (M+H)⁺.

### Step 2 - 5-[(5R)-5-amino-3,3-difluoro-1-piperidyl]-N-[3-(difluoromethyl)-1-[4-[[4-[3-[1-(2,6-dioxo- 3-piperidyl)-3-methyl-2-oxo-benzimidazol-4-yl]propoxy]-1-piperidyl]methyl]cyclohexyl]pyrazol-4-yl]pyrazolo[1,5-a]pyrimidine-3-carboxamide

To a solution of tert-butyl N-[(3R)-1-[3-[[3-(difluoromethyl)-1-[4-[[4-[3-[1-(2,6-dioxo-3-piperidyl)-3 -methyl-2-oxo-benzimidazol-4-yl]propoxy]-1-piperidyl]methyl]cyclohexyl]pyrazol-4-yl]carbamoyl]pyrazolo[1,5-a]pyrimidin-5-yl]-5,5-difluoro-3-piperidyl]carbamate (55.0 mg, 54.6 umol) in DCM (2 mL) was added TFA (3.08 g, 27.0 mmol). The mixture was stirred at 10 °C for 0.5 hr. On completion, the mixture was concentrated *in vacuo* to give a residue. The residue was purified by prep-HPLC (column: Phenomenex Synergi C18 150*25*10um; mobile phase: [water (0.225%FA)-ACN]) to give the title compound (21.1 mg, 42% yield) as white solid. ¹H NMR (400 MHz, DMSO-*d₆*) δ 11.09 (s, 1H), 9.27 (s, 1H), 8.88 (d, *J* = 8.0 Hz, 1H), 8.38 (s, 1H), 8.32 (s, 1H), 7.31 - 6.99 (m, 2H), 6.98 - 6.93 (m, 2H), 6.90 - 6.84 (m, 1H), 5.36 (dd, *J* = 5.2, 12.8 Hz, 1H), 4.86 - 4.71 (m, 1H), 4.42 - 4.30 (m, 1H), 4.20 - 4.14 (m, 1H), 3.65 (d, *J* = 14.5 Hz, 1H), 3.57 (s, 3H), 3.45 (t, *J =* 6.0 Hz, 2H), 3.30 - 3.27 (m, 1H), 3.14 - 2.83 (m, 6H), 2.73 - 2.62 (m, 4H), 2.37 (d, *J =* 8.8 Hz, 1H), 2.17 - 1.94 (m, 8H), 1.92 - 1.68 (m, 9H), 1.62 - 1.53 (m, 1H), 1.51 - 1.40 (m, 2H), 1.10 - 0.96 (m, 2H); LC-MS (ESI⁺) m/z 907.6 (M+H)⁺.

### Examples 23. Syntheses of 4-[[4-[4-[3-[1-(2,6-dioxo-3-piperidyl)-3-methyl-2-oxo-benzimidazol-4-yl]propoxy]-1- piperidyl]cyclohexyl]amino]quinazoline-6-carbonitrile (I-304) and 4-[[4-[4-[3-[1-(2,6-dioxo-3 -piperidyl)-3-methyl-2-oxo-benzimidazol-4-yl]propoxy]-1-piperidyl]cyclohexyl]amino]quinazoline-6-carbonitrile (I-305)

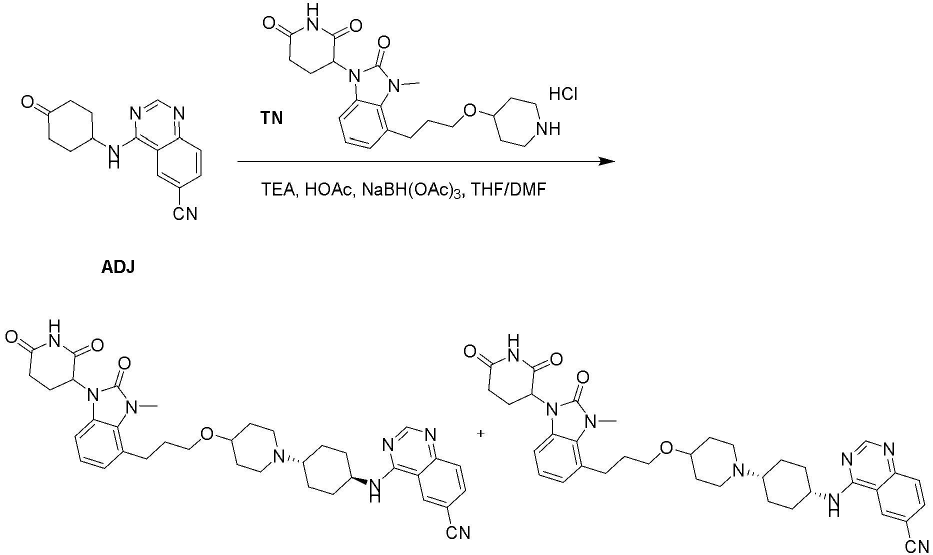

To a solution of 4-[(4-oxocyclohexyl)amino]quinazoline-6-carbonitrile (60.0 mg, 225 umol, Intermediate ADJ) and 3-[3-methyl-2-oxo-4-[3-(4-piperidyloxy)propyl]benzimidazol-1-yl]piperidine-2,6-dione (98.5 mg, 225 umol, HCl, Intermediate TN) in a mixed solvent of DMF (1.00 mL) and THF (2.00 mL) was added TEA (22.8 mg, 225 umol), and the mixture was stirred at 20 °C for 10 minutes. Then HOAc (27.1 mg, 450 umol) was added, and the mixture was stirred at 20 °C for 0.5 hr. Next, NaBH(OAc)₃ (57.3 mg, 270 umol) was added, then the mixture was stirred at 20 °C for 16 hrs. On completion, the reaction mixture was quenched by addition water (0.5 mL), and then concentrated *in vacuo* to give a residue. The residue was purified by prep HPLC (column: Phenomenex Luna C18 150*25mm*10um;mobile phase: [water(0.1%TFA)-ACN];B%: 15%-35%,10min) to give 4-[[4-[4-[3-[1-(2,6-dioxo-3-piperidyl)-3-methyl-2-oxo-benzimidazol-4-yl]propoxy]-1- piperidyl]cyclohexyl]amino]quinazoline-6-carbonitrile (4.24 mg, 3% yield) as a white solid and 4-[[4-[4-[3-[1-(2,6-dioxo-3 -piperidyl)-3-methyl-2-oxo-benzimidazol-4-yl]propoxy]-1-piperidyl]cyclohexyl]amino]quinazoline-6-carbonitrile (4.21 mg, 6.47 umol, 3% yield) as a yellow solid. Characterization of 4-[[4-[4-[3-[1-(2,6-dioxo-3-piperidyl)-3-methyl-2-oxo-benzimidazol-4-yl]propoxy]-1- piperidyl]cyclohexyl]amino]quinazoline-6-carbonitrile:

¹H NMR (400 MHz, DMSO-*d*₆) δ 11.08 (s, 1H), 8.99 (s, 1H), 8.70 (s, 1H), 8.16 (d, *J* = 8.8 Hz, 1H), 7.82 (d, *J =* 8.8 Hz, 1H), 7.03 - 6.94 (m, 2H), 6.93 - 6.85 (m, 1H), 5.36 (dd, *J =* 5.2, 12.4 Hz, 1H), 4.35 - 4.15 (m, 1H), 3.70 (s, 1H), 3.59 (d, *J=* 10.8 Hz, 3H), 3.51 (d, *J* = 3.2 Hz, 2H), 3.15 - 2.83 (m, 8H), 2.76 - 2.68 (m, 1H), 2.65 - 2.60 (m, 1H), 2.22 - 2.10 (m, 5H), 2.03 - 1.95 (m, 2H), 1.95 - 1.78 (m, 4H), 1.74 - 1.59 (m, 3H), 1.59 - 1.47 (m, 2H). LC-MS (ESI⁺) *m*/*z* 651.4 (M+H)⁺. Characterization of 4-[[4-[4-[3-[1-(2,6-dioxo-3 -piperidyl)-3-methyl-2-oxo-benzimidazol-4-yl]propoxy]-1-piperidyl]cyclohexyl]amino]quinazoline-6-carbonitrile: ¹H NMR (400 MHz, DMSO-*d*₆) δ 11.08 (s, 1H), 9.16 (s, 1H), 8.71 (s, 1H), 8.17 (d, *J* = 8.8 Hz, 1H), 7.84 (d, *J* = 8.8 Hz, 1H), 7.01 - 6.93 (m, 2H), 6.91 - 6.83 (m, 1H), 5.35 (dd, *J* = 5.2, 12.4 Hz, 1H), 4.39 (s, 1H), 3.76 - 3.65 (m, 1H), 3.58 (d, *J* = 6.4 Hz, 3H), 3.50 (d, *J =* 6.0 Hz, 2H), 3.24 - 2.83 (m, 8H), 2.76 - 2.68 (m, 1H), 2.65 - 2.59 (m, 1H), 2.28 - 2.15 (m, 3H), 2.06 - 1.54 (m, 13H). LC-MS (ESI⁺) *m*/*z* 651.4 (M+H)⁺.

### Examples 24. Syntheses of 2-[2-(cyclopropylmethylamino)-4-pyridyl]-N-[3-(difluoromethyl)-1-[4-[[3-[3-[1-[(3S)-2,6 -dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (I-306) and 2-[2-(cyclopropylmethylamino)-4- pyridyl]-N-[3-(difluoromethyl)-1-[4-[[3-[3-[1-[(3R)-2,6-dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (I-307)

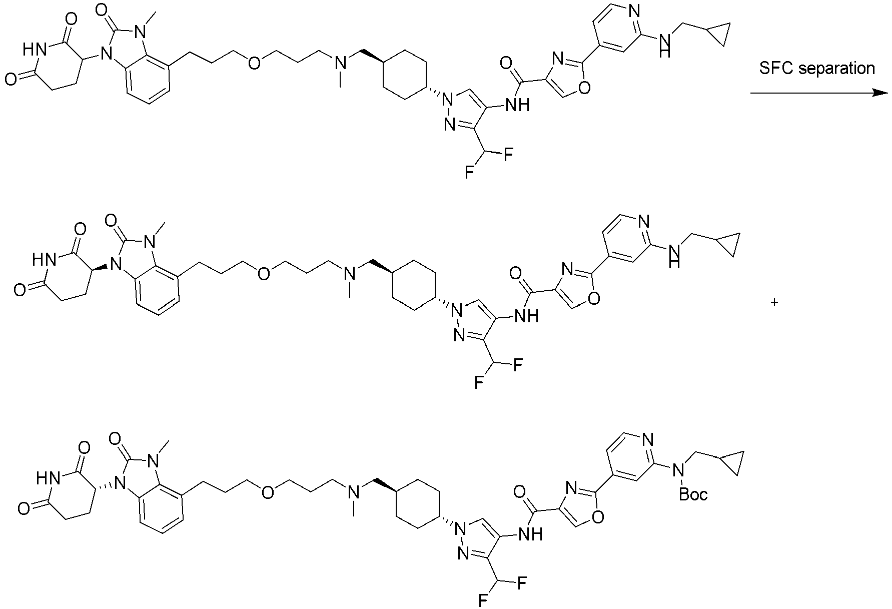

2-[2-(Cyclopropylmethylamino)-4-pyridyl]-N-[3-(difluoromethyl)-1-[4-[[3-[3-[1-(2,6-dioxo-3-piperidyl)-3-methyl-2-oxo-benzimidazol-4-yl]propoxy]propyl-methyl-amino]methyl]cyclohexyl]pyrazol-4-yl]oxazole-4-carboxamide (500 mg, 583 umol, Example 2, I-30) was separated by SFC. The residue was purified by SFC (Sample preparation: Add CH₃CN 200 ml into sample Instrument: Waters 80Q Mobile Phase: 70% IPA+ACN (0.1% DEA) in Supercritical CO₂ Flow Rate:70 g/min Cycle Time:4 min, total time: 350min Single injetion volume:3.0ml Back Pressure:100 bar to keep the CO₂ in Supercritical flow) to give peak 1 (72% ee value) and peak 2 (81% ee value). The fraction was received in a solution of 2.5% hydrochloride in isopropanol and concentrated *in vacuo.* Peak 1 was re-purified by SFC (Sample preparation: Add CH₃CN and CH₂CL₂ 40ml into sample Instrument: Waters 80Q Mobile Phase:70% IPA (0.1% DEA) in Supercritical CO₂ Flow Rate: 70 g/min Cycle Time:6 min, total time:110min Single injetion volume:2.5ml Back Pressure: 100 bar to keep the CO₂ in Supercritical flow). Peak 2 was also re-purified by SFC (Sample preparation: Add CH₃CN and CH₂CL₂ 80ml into sample Instrument: Waters 80Q Mobile Phase:70% IPA + CAN (0.1% DEA) in Supercritical CO₂ Flow Rate:70 g/min Cycle Time: 4 min, total time: 80 min Single injetion volume: 4.0ml Back Pressure: 100 bar to keep the CO₂ in Supercritical flow). The fraction was received in a solution of 2.5% hydrochloride in isopropanol and concentrated *in vacuo.* After that, Peak 1 was purified by prep-HPLC (column: Phenomenex Synergi C18 150*25*10 um; mobile phase: [water (0.225%FA)-ACN]; B%: 9%-39%, 10 min) to give 2-[2-(cyclopropylmethylamino)-4-pyridyl]-N-[3-(difluoromethyl)-1-[4-[[3-[3-[1-[(3S)-2,6 -dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (122 mg, 23% yield, FA) as white solid; ¹H NMR (400 MHz, DMSO-*d₆*) δ 11.08 (s, 1H), 9.69 (s, 1H), 8.92 (s, 1H), 8.17 (s, 1H), 8.15 (d, *J* = 5.2 Hz, 1H), 7.29 - 7.00 (m, 4H), 6.96 (d, *J* = 5.2 Hz, 2H), 6.89 - 6.84 (m, 1H), 5.39 - 5.33 (m, 1H), 4.25 - 4.15 (m, 1H), 3.57 (s, 3H), 3.42 (t, *J* = 6.0 Hz, 4H), 3.18 (t, *J* = 6.0 Hz, 2H), 2.99 - 2.93 (m, 2H), 2.92 - 2.82 (m, 1H), 2.75 - 2.57 (m, 2H), 2.56 - 2.51 (m, 2H), 2.39 (t, *J =* 7.2 Hz, 2H), 2.19 - 2.14 (m, 5H), 2.07 - 1.97 (m, 3H), 1.90 (d, *J =* 12.4 Hz, 2H), 1.86 - 1.79 (m, 2H), 1.78 - 1.73 (m, 1H), 1.68 - 1.65 (m, 1H), 1.60 - 1.49 (m, 1H), 1.10 - 0.97 (m, 3H), 0.48 - 0.42 (m, 2H), 0.25 - 0.20 (m, 2H); LC-MS (ESI⁺) *m*/*z* 857.6 (M+H)⁺. Peak 2 was purified by prep-HPLC (column: Phenomenex Synergi C18 150*25*10 um; mobile phase: [water (0.225%FA)-ACN]; B%: 9%-39%, 10 min) to give 2-[2-(cyclopropylmethylamino)-4- pyridyl]-N-[3-(difluoromethyl)-1-[4-[[3-[3-[1-[(3R)-2,6-dioxo-3-piperidyl]-3-methyl-2-oxo-benzimidazol-4-yl]propoxy]propyl-methyl-amino]methyl]cyclohexyl]pyrazol-4-yl]oxazole-4-carboxamide (99.2 mg, 18% yield, FA) as white solid; ¹H NMR (400 MHz, DMSO-*d₆*) δ 11.08 (s, 1H), 9.69 (s, 1H), 8.92 (s, 1H), 8.17 (s, 1H), 8.15 (d, *J* = 5.2 Hz, 1H), 7.29 - 7.00 (m, 4H), 6.96 (d, *J* = 5.2 Hz, 2H), 6.89 - 6.85 (m, 1H), 5.39 - 5.33 (m, 1H), 4.24 - 4.14 (m, 1H), 3.57 (s, 3H), 3.42 (t, *J =* 6.0 Hz, 4H), 3.18 (t, *J =* 6.0 Hz, 2H), 3.00 - 2.93 (m, 2H), 2.92 - 2.84 (m, 1H), 2.75 - 2.68 (m, 1H), 2.65 - 2.58 (m, 1H), 2.52 (s, 2H), 2.37 (t, *J* = 6.8 Hz, 2H), 2.16 (s, 3H), 2.13 (s, 2H), 2.03 (d, *J =* 12.8 Hz, 2H), 2.00 - 1.95 (m, 1H), 1.90 (d, *J =* 11.2 Hz, 2H), 1.85 - 1.80 (m, 2H), 1.75 (d, *J =* 12 Hz, 1H), 1.66 (t, *J =* 6.8 Hz, 1H), 1.59 - 1.48 (m, 1H), 1.11 - 0.97 (m, 3H), 0.48 - 0.42 (m, 2H), 0.25 - 0.19 (m, 2H); LC-MS (ESI⁺) *m*/*z* 857.6 (M+H)⁺.

### Examples 25. Syntheses of 2-(2-((cyclopropylmethyl)amino)pyridin-4-yl)-N-(3-(difluoromethyl)-1-((1S,4r)-4-(((1-((1-((S)-2,6-dioxopiperidin-3-yl)-3-methyl-2-oxo-2,3-dihydro-1H-benzo[d]imidazol-4-yl)methyl)piperidin-4-yl)(methyl)amino)methyl)cyclohexyl)-1H-pyrazol-4-yl)oxazole-4-carboxamide (I-308) and 2-(2-((cyclopropylmethyl)amino)pyridin-4-yl)-N-(3-(difluoromethyl)-1-((1R,4r)-4-(((1-((1-((R)-2,6-dioxopiperidin-3-yl)-3-methyl-2-oxo-2,3-dihydro-1H-benzo[d]imidazol-4-yl)methyl)piperidin-4-yl)(methyl)amino)methyl)cyclohexyl)-1H-pyrazol-4-yl)oxazole-4-carboxamide (I-309)

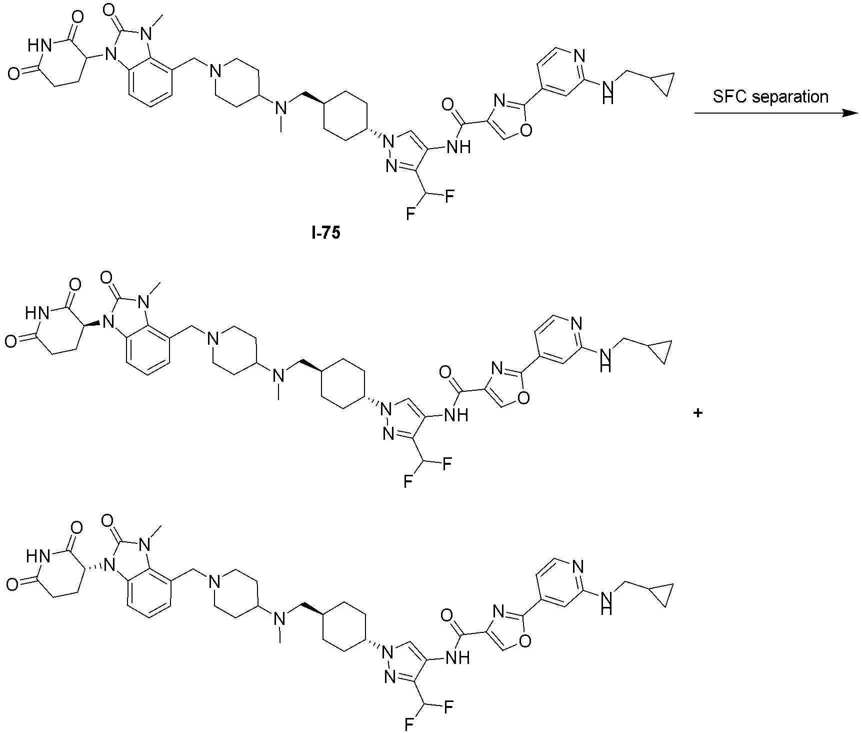

The trans diastereomer 2-(2-((cyclopropylmethyl)amino)pyridin-4-yl)-N-(3-(difluoromethyl)-1-((1r,4r)-4-(((1-((1-(2,6-dioxopiperidin-3-yl)-3-methyl-2-oxo-2,3-dihydro-1H-benzo[d]imidazol-4-yl)methyl)piperidin-4-yl)(methyl)amino)methyl)cyclohexyl)-1H-pyrazol-4-yl)oxazole-4-carboxamide (1.00 g, Example I-75) was separated by SFC (column: DAICEL CHIRALPAKAD (250mm*30mm, 10um); mobile phase: [0.1%NH_{3•}H₂OIPA]) twice and purified by Prep-HPLC (column: Phenomenex Synergi C18 150*25*10um; mobile phase: [water (0.225%FA)-ACN]) to give the two title compounds.

The first peak, 2-(2-((cyclopropylmethyl)amino)pyridin-4-yl)-N-(3-(difluoromethyl)-1-((1S,4r)-4-(((1-((1-((S)-2,6-dioxopiperidin-3-yl)-3-methyl-2-oxo-2,3-dihydro-1H-benzo[d]imidazol-4-yl)methyl)piperidin-4-yl)(methyl)amino)methyl)cyclohexyl)-1H-pyrazol-4-yl)oxazole-4-carboxamide (128 mg, 24% yield, 100% ee, FA salt) was obtained as white solid. ¹H NMR (400 MHz, DMSO-*d₆*) δ 11.09 (s, 1H), 9.68 (s, 1H), 8.91 (s, 1H), 8.17 (s, 1H), 7.33 - 6.92 (m, 6H), 6.87 (d, *J* = 7.2 Hz, 1H), 5.38 (dd, *J* = 5.2, 12.8 Hz, 1H), 4.25 - 4.11 (m, 1H), 3.67 (s, 3H), 3.62 (s, 2H), 3.18 (t, *J* = 6.0 Hz, 3H), 2.95 - 2.82 (m, 3H), 2.71 (dd, *J* = 4.4, 12.8 Hz, 1H), 2.66 - 2.59 (m, 1H), 2.52 (d, *J* = 2.0 Hz, 1H), 2.40 (t, *J* = 11.2 Hz, 1H), 2.27 (d, *J* = 6.0 Hz, 1H), 2.22 (s, 3H), 2.08 - 1.98 (m, 4H), 1.97 - 1.84 (m, 3H), 1.74 (d, *J =* 9.6 Hz, 2H), 1.66 (d, *J* = 11.2 Hz, 2H), 1.50 (s, 1H), 1.47 - 1.35 (m, 2H), 1.11 - 0.95 (m, 3H), 0.51 - 0.41 (m, 2H), 0.26 - 0.19 (m, 2H); LC-MS (ESI⁺) *m*/*z* 854.5 (M+H)⁺.

The second peak, 2-(2-((cyclopropylmethyl)amino)pyridin-4-yl)-N-(3-(difluoromethyl)-1-((1R,4r)-4-(((1-((1-((R)-2,6-dioxopiperidin-3-yl)-3-methyl-2-oxo-2,3-dihydro-1H-benzo[d]imidazol-4-yl)methyl)piperidin-4-yl)(methyl)amino)methyl)cyclohexyl)-1H-pyrazol-4-yl)oxazole-4-carboxamide (185 mg, 36% yield, 94.3% ee, FA salt) was obtained as white solid. ¹H NMR (400 MHz, DMSO-*d₆*) δ 11.09 (s, 1H), 9.67 (s, 1H), 8.91 (s, 1H), 8.17 (s, 1H), 7.30 - 6.99 (m, 5H), 6.96 (t, *J =* 7.6 Hz, 1H), 6.87 (d, *J =* 7.2 Hz, 1H), 5.38 (dd, *J =* 5.6*,* 12.8 Hz, 1H), 4.24 - 4.12 (m, 1H), 3.67 (s, 3H), 3.62 (s, 2H), 3.18 (t, *J* = 6.0 Hz, 3H), 2.88 (d, *J* = 11.2 Hz, 3H), 2.71 (dd, *J =* 4.4, 13.2 Hz, 1H), 2.66 - 2.59 (m, 1H), 2.52 (d, *J =* 2.0 Hz, 1H), 2.37 (s, 1H), 2.25 (d, *J =* 6.8 Hz, 1H), 2.20 (s, 3H), 2.07 - 2.00 (m, 3H), 2.00 - 1.93 (m, 2H), 1.92 - 1.84 (m, 2H), 1.74 (d, *J =* 10.0 Hz, 2H), 1.65 (d, *J* = 11.6 Hz, 2H), 1.57 - 1.33 (m, 3H), 1.13 - 0.95 (m, 3H), 0.50 - 0.41 (m, 2H), 0.25 - 0.19 (m, 2H); LC-MS (ESI⁺) *m*/*z* 854.5 (M+H)⁺.

### Examples 26. Syntheses of N-(3-(difluoromethyl)-1-((1r,4r)-4-((4-(3-(1-((S)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (I-310) and N-(3-(difluoromethyl)-1-((1r,4r)-4-((4-(3-(1-((R)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (I-311)

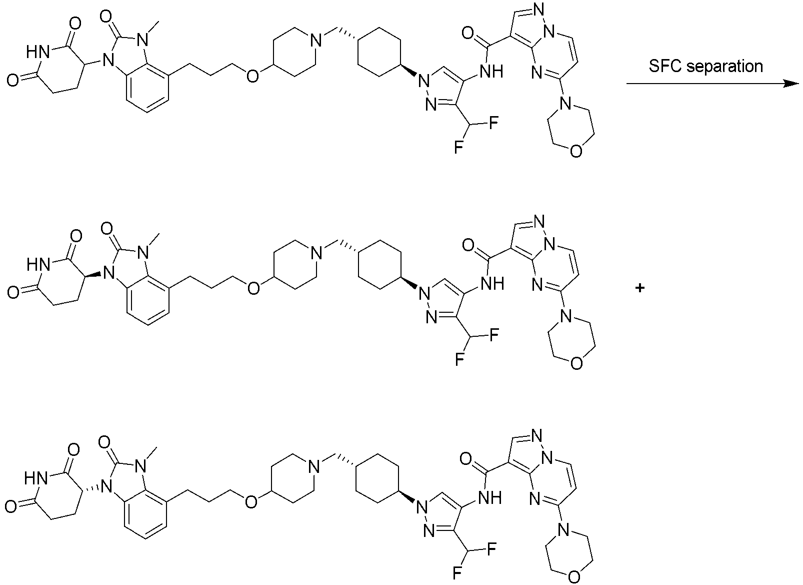

N-[3-(difluoromethyl)-1-[4-[[4-[3-[1-[(3S)-2,6-dioxo-3-piperidyl]-3-methyl-2-oxo-benzimidazol-4-yl] propoxy]-1-piperidyl]methyl]cyclohexyl]pyrazol-4-yl]-5-morpholino-pyrazolo[1,5-a]pyrimidine-3-carboxamide (200 mg, 223 umol, Compound **I-257,** HCl salt) was separated by SFC (Add CH₃CN and CH₂Cl₂, IPA+01%DEA 150 mL into sample; Instrument: Waters 80Q SFC; Mobile Phase: 65% ( IPA+20%ACN (0.1%DEA) ) in Supercritical CO₂; Flow Rate: 70 g/min; Cycle Time: 6.1 min, total time: 240 min; Single injection volume:4.0ml; Back Pressure:100 bar to keep the CO₂ in Supercritical flow) to give peak 1, N-(3-(difluoromethyl)-1-((1r,4r)-4-((4-(3-(1-((S)-2,6-dioxopiperidin-3-yl)-3-methyl-2-oxo-2,3-dihydro-1H-benzo[d]imidazol-4-yl)propoxy)piperidin-1-yl)methyl)cyclohexyl)- 1H-pyrazol-4-yl)-5-morpholinopyrazolo[1,5-a]pyrimidine-3-carboxamide (99% ee value) and peak 2, N-(3-(difluoromethyl)-1-((1r,4r)-4-((4-(3-(1-((R)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (93% ee value). The fractions were added to a solution of 2.5% HCl in MeCN solution and then concentrated *in vacuo* respectively. Peak 2 was further purified by SFC (Sample preparation: Add CH3CN and IPA+0.1%DEA 60ml into sample; Instrument: Waters 80Q SFC; Mobile Phase: 65% (IPA+20%ACN (0.1%DEA)) in Supercritical CO₂; Flow Rate: 75 g/min; Cycle Time:5.5 min, total time:100 min; Single injection volume:3.0 mL; Back Pressure:100 bar to keep the CO₂ in Supercritical flow.") to afford N-(3-(difluoromethyl)-1-((1r,4r)-4-((4-(3-(1-((R)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (99% ee value).

Peak 1 was then purified by prep-HPLC (column: Phenomenex Synergi C18 150*25*10 um; mobile phase: [water (0.05%HCl)-ACN]; B%: 25%-45%, 12 min) to afford N-(3-(difluoromethyl)-1-((1r,4r)-4-((4-(3-(1-((S)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (42.8 mg, 14% yield, 99% ee value, HCl salt) as an off-white solid; ¹H NMR (400 MHz, DMSO-*d*₆) δ 11.12 (s, 1H), 9.42 (s, 1H), 8.88 - 8.78 (m, 1H), 8.40 (s, 1H), 8.30 (s, 1H), 7.26 - 6.84 (m, 5H), 5.44 - 5.32 (m, 1H), 4.24 (t, *J* = 11.6 Hz, 1H), 3.80 (s, 4H), 3.73 (d, *J* = 3.6 Hz, 4H), 3.61 - 3.60 (m, 3H), 3.51 (s, 3H), 3.41 (s, 2H), 3.34 (d, *J* = 10.6 Hz, 1H), 3.06 - 2.84 (m, 7H), 2.77 - 2.59 (m, 2H), 2.16 - 1.74 (m, 15H), 1.25 - 1.12 (m, 2H); LC-MS (ESI⁺) *m*/*z* 858.6 (M+H)⁺.

Peak 2 was purified by prep-HPLC (column: Phenomenex Synergi C18 150*25*10 um; mobile phase: [water (0.05%HCl)-ACN]; B%: 25%-45%, 12 min) to afford N-(3-(difluoromethyl)-1-((1r,4r)-4-((4-(3-(1-((R)-2,6-dioxopiperidin-3-yl)-3-methyl-2-oxo-2,3-dihydro-1H-benzo[d]imidazol-4-yl)propoxy)piperidin-1-yl)methyl)cyclohexyl)-1H-pyrazol-4-yl)-5-morpholinopyrazolo[1,5-a]pyrimidine-3-carboxamide (23.6 mg, 8% yield, 99% ee value, HCl salt) as an off-white solid; ¹H NMR (400 MHz, DMSO-*d*₆) δ 11.12 (s, 1H), 9.42 (s, 1H), 8.87 - 8.81 (m, 1H), 8.41 (s, 1H), 8.30 (s, 1H), 7.26 - 6.86 (m, 5H), 5.46 - 5.28 (m, 1H), 4.30 - 4.19 (m, 1H), 3.80 (s, 4H), 3.74 - 3.71 (m, 6H), 3.59 (d, J = 9.2 Hz, 3H), 3.54 - 3.48 (m, 3H), 3.35 (d, *J =* 10.0 Hz, 1H), 3.06 - 2.85 (m, 7H), 2.78 - 2.59 (m, 2H), 2.16 - 1.73 (m, 14H), 1.27 - 1.12 (m, 2H); LC-MS (ESI⁺) *m*/*z* 858.6 (M+H)⁺.

### Example 27. Synthesis of N-[1-[4-(aminomethyl)cyclohexyl]-3-(difluoromethyl)pyrazol-4-yl]-2-[2- (cyclopropylmethyl amino)-4-pyridyl]oxazole-4-carboxamide (I-312)

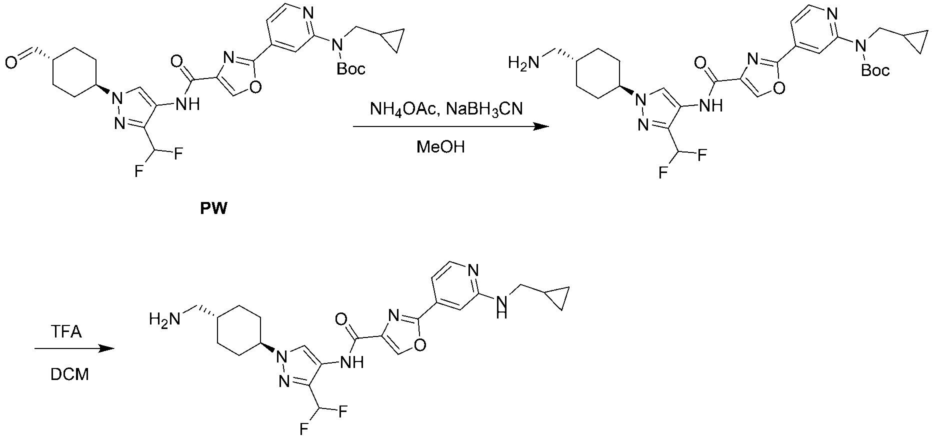

### Step 1 - Tert-butyl N-[4-[4-[[1-[4-(aminomethyl)cyclohexyl]-3-(difluoromethyl)pyrazol-4-yl] carbamoyl]oxazol-2-yl]-2-pyridyl]-N-(cyclopropylmethyl)carbamate

To a mixture of tert-butyl N-(cyclopropylmethyl)-N-[4-[4-[[3-(difluoromethyl)-1-(4-formylcyclohexyl) pyrazol-4-yl]carbamoyl]oxazol-2-yl]-2-pyridyl]carbamate (500 mg, 855 umol, Intermediate PW) and NH₄OAc (1.32 g, 17.1 mmol) in MeOH (10 mL) was added NaBH₃CN (80.6 mg, 1.28 mmol). The reaction mixture was stirred at 25 °C for 12 hours. On completion, the reaction mixture was filtered and concentrated *in vacuo.* The residue was purified by reverse phase (0.1 % FA condition) to give the title compound (120 mg, 23% yield) as white solid. LC-MS (ESI⁺) *m*/*z* 586.4 (M+H)⁺.

### Step 2 - N-[1-[4-(aminomethyl)cyclohexyl]-3-(difluoromethyl)pyrazol-4-yl]-2-[2-(cyclopropylmethyl amino)-4-pyridyl]oxazole-4-carboxamide

To a mixture of tert-butyl N-[4-[4-[[1-[4-(aminomethyl)cyclohexyl]-3-(difluoromethyl)pyrazol-4-yl] carbamoyl]oxazol-2-yl]-2-pyridyl]-N-(cyclopropylmethyl)carbamate (110 mg, 187 umol) in DCM (2 mL) was added TFA (3.08 g, 27.0 mmol, 2 mL). The reaction mixture was stirred at 25 °C for 0.5 hour. On completion, the reaction mixture was concentrated *in vacuo.* The residue was purified by prep-HPLC (column: Phenomenex Synergi C18 150*25*10 um; mobile phase: [water(0.225% FA)- ACN]; B%: 2%-32%, 10 min) to give the title compound (41.1 mg, 40% yield, 98% purity) as white solid. ¹H NMR (400 MHz, DMSO-*d₆*) δ 9.73 (s, 1H), 8.92 (s, 1H), 8.33 (s, 1H), 8.15 (d, *J* = 5.2 Hz, 1H), 7.32 - 7.04 (m, 3H), 7.03 - 6.99 (m, 1H), 4.25 - 4.19 (m, 1H), 3.18 (t, *J* = 6.0 Hz, 2H), 2.75 - 2.65 (m, 2H), 2.08 (d, *J* = 10.4 Hz, 2H), 1.91 (d, *J* = 12.4 Hz, 2H), 1.81 - 1.69 (m, 2H), 1.67 - 1.57 (m, 1H), 1.21 - 1.09 (m, 2H), 1.09 - 1.01 (m, 1H), 0.48 - 0.42 (m, 2H), 0.24 - 0.19 (m, 2H); LC-MS (ESI⁺) *m*/*z* 486.3 (M+H)⁺.

### Example 28. Synthesis of N-[1-[4-(aminomethyl)cyclohexyl]-3-(difluoromethyl)pyrazol-4-yl]-5-morpholino-pyrazolo [1,5-a]pyrimidine-3-carboxamide (I-313)

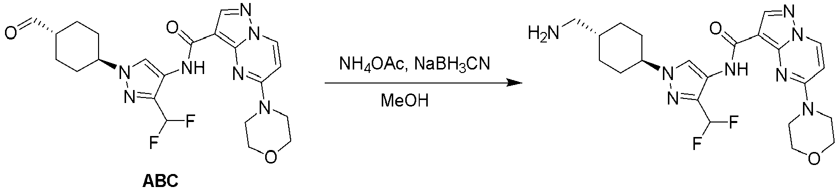

To a mixture of N-[3-(difluoromethyl)-1-(4-formylcyclohexyl)pyrazol-4-yl]-5-morpholino-pyrazolo [1,5-a]pyrimidine-3-carboxamide (0.20 g, 422 umol, Intermediate ABC) in MeOH (100 mL) was added NH₄OAc (6.51 g, 84.5 mmol) and the mixture was stirred for 1 hour. Then NaBH₃CN (39.8 mg, 634 umol) was added into the mixture and the mixture was stirred at 20 °C for 13 hours. On completion, the reaction mixture was quenched with water (5 mL) and filtered to give the filtrate. The filtrate was concentrated *in vacuo* to give the residue. The residue was purified by prep-HPLC column: Phenomenex Synergi C18 150*30 mm*4 um; mobile phase: [water (0.225%FA)-ACN]; B%: 8%-38%) to give the title compound (18.6 mg, 9% yield) as a off-white solid. ¹H NMR (400 MHz, DMSO-*d₆*) δ 9.39 (s, 1H), 8.83 (d, *J =* 8.0 Hz, 1H), 8.46 - 8.37 (m, 2H), 7.27 - 6.95 (m, 1H), 6.91 (d, *J =* 8.0 Hz, 1H), 4.25 - 4.14 (m, 1H), 3.79 (s, 4H), 3.75 - 3.69 (m, 4H), 2.69 - 2.51 (m, 4H), 2.06 (d, *J* = 10.4 Hz, 2H), 1.91 (d, *J* = 12.0 Hz, 2H), 1.80 - 1.65 (m, 2H), 1.55 (d, *J =* 3.6 Hz, 1H), 1.20 - 1.06 (m, 2H); LC-MS (ESI⁺) *m*/*z* 475.2 (M+H)⁺.

### Example 29. Synthesis of 4-[2-[4-(Aminomethyl)cyclohexyl]ethynyl]-1-[[(2S,3S,4S)-3-ethyl-4-fluoro-5-oxo-pyrrolidin- 2- yl]methoxy]-7-methoxy-isoquinoline-6-carboxamide (I-314)

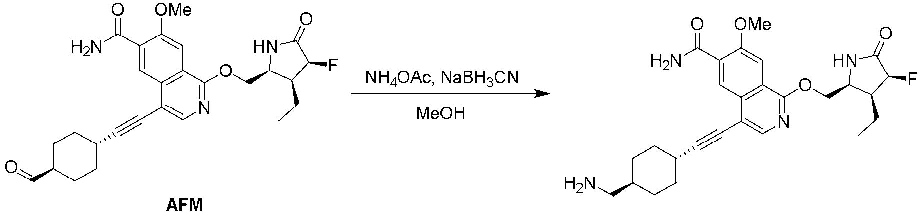

To a solution of 1-[[(2S,3S,4S)-3-ethyl-4-fluoro-5-oxo-pyrrolidin-2-yl]methoxy]-4-[2-(4-formyl cyclohexyl)ethynyl]-7-methoxy-isoquinoline-6-carboxamide (50.0 mg, 100 umol, Intermediate AFM) in methanol (2 mL) was added NH₄OAc (1.56 g, 20.1 mmol). After the reaction mixture was stirred at 20 °C for 3 hr, NaBH₃CN (9.51 mg, 151 umol) was added. The reaction mixture was stirred at 20 °C for 16 hrs. On completion, the reaction mixture was quenched by water (0.2 mL) and concentrated *in vacuo.* The residue was purified by prep-HPLC (column: Phenomenex Synergi C18 150*25*10 um; mobile phase: [water (0.225%FA)-ACN]; B%: 15%-35%, 10 min) to give the title compound (10.5 mg, 18% yield, FA salt) as off-white solid. ¹H NMR (400 MHz, DMSO-*d₆*) δ 8.87 (s, 1H), 8.40 (s, 1H), 8.02 (s, 1H), 7.95 - 7.68 (m, 3H), 7.33 - 6.42 (m, 2H), 4.99 - 4.81 (m, 1H), 4.53 (dd, *J =* 3.2*,* 11.2 Hz, 1H), 4.26 (dd, *J =* 6.4*,* 11.2 Hz, 1H), 4.12 - 4.05 (m, 1H), 3.98 (s, 3H), 2.66 -2.62 (m, 1H), 2.62-2.59 (m, 2H), 2.58 - 2.52 (m, 2H), 2.17 - 2.02 (m, 2H), 1.90 - 1.76 (m, 2H), 1.68 - 1.55 (m, 2H), 1.55 - 1.40 (m, 3H), 1.10 - 0.97 (m, 4H); LC-MS (ESI⁺) *m*/*z* 497.2 (M+H)⁺.

### Example 30. Synthesis of N-[1-[4-[[3-(3-aminopropoxy)propyl-methyl-amino]methyl]cyclohexyl]-3-(difluoromethyl) pyrazol-4-yl]-5-morpholino-pyrazolo[1,5-a]pyrimidine-3-carboxamide (I-315)

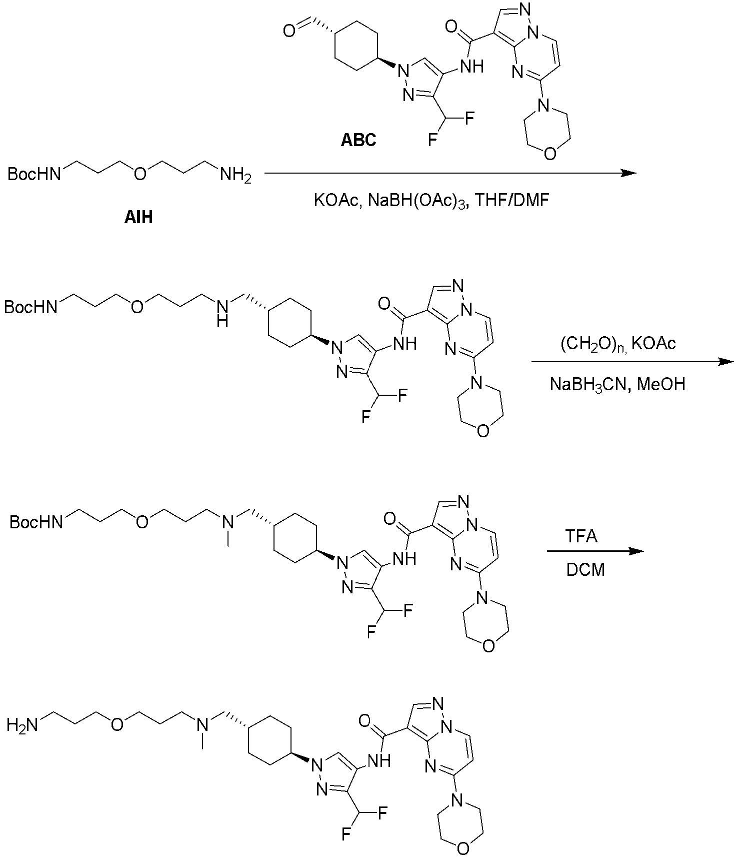

### Step 1 - Tert-butyl N-[3-[3-[[4-[3-(difluoromethyl)-4-[(5-morpholinopyrazolo[1,5-a]pyrimidine-3- carbonyl)amino]pyrazol-1-yl]cyclohexyl]methylamino]propoxy]propyl]carbamate

To a mixture of tert-butyl N-[3-(3-aminopropoxy)propyl]carbamate (294 mg, 1.27 mmol, Intermediate AIH) in THF (10 mL) was added KOAc (186 mg, 1.90 mmol). The mixture was stirred at 25 °C for 0.5 hour. N-[3-(difluoromethyl)-1-(4-formylcyclohexyl) pyrazol-4-yl]-5-morpholino-pyrazolo [1,5-a]pyrimidine-3-carboxamide (300 mg, 633 umol, Intermediate ABC) and NaBH(OAc)₃ (161 mg, 760 umol) were added into the mixture. The reaction mixture was stirred at 25 °C for 11.5 hours. On completion, the reaction was quenched by water (5 mL) and CH₃CN (10 mL), the mixture was concentrated *in vacuo* to give the residue. The residue was purified by reverse phase HPLC (0.1% FA condition) to give the title compound (340 mg, 77% yield) as a white solid. ¹H NMR (400 MHz, CDCl₃) δ 9.51 (s, 1H), 8.48 - 8.31 (m, 4H), 6.91 - 6.59 (m, 1H), 6.45 - 6.37 (m, 1H), 4.06 (d, *J =* 7.6 Hz, 1H), 3.88 - 3.83 (m, 3H), 3.82 (s, 3H), 3.57 (d, *J =* 5.6 Hz, 2H), 3.46 (d, *J =* 5.6 Hz, 2H), 3.27 - 3.14 (m, 4H), 2.93 (d, *J =* 6.8 Hz, 1H), 2.27 - 2.17 (m, 2H), 2.11 - 2.04 (m, 2H), 2.02 (s, 2H), 1.93 - 1.78 (m, 2H), 1.77 - 1.68 (m, 2H), 1.45 (s, 9H), 1.31 - 1.17 (m, 2H); LC-MS (ESI⁺) *m*/*z* 690.2 (M+H)⁺.

### Step 2 - Tert-butyl N-[3-[3-[[4-[3-(difluoromethyl)-4-[(5-morpholinopyrazolo[1,5-a]pyrimidine-3- carbonyl)amino]pyrazol-1-yl]cyclohexyl]methyl-methyl-amino]propoxy]propyl]carbamate

To a mixture of tert-butyl N-[3-[3-[[4-[3-(difluoromethyl)-4-[(5-morpholinopyrazolo[1,5-a] pyrimidine-3-carbonyl)amino]pyrazol-1-yl]cyclohexyl]methylamino]propoxy]propyl]carbamate (73.0 mg, 105.8 umol) in MeOH (2 mL) was added KOAc (31.2 mg, 317 umol). The mixture was stirred at 25 °C for 0.5 hour. HCHO (31.7 mg, 1.06 mmol) and NaBH₃CN (7.98 mg, 127 umol) was added into the mixture. The reaction mixture was stirred at 25 °C for 0.5 hour. On completion, the reaction was quenched by water (5 mL) and CH₃CN (10 mL), the mixture was concentrated *in vacuo* to give the residue. The residue was purified by reverse phase (0.1 % FA condition) to give the title compound (70.0 mg, 93% yield) as a white solid. LC-MS (ESI⁺) *m*/*z* 704.2 (M+H)⁺.

### Step 3 - N-[1-[4-[[3-(3-aminopropoxy)propyl-methyl-amino]methyl]cyclohexyl]-3-(difluoromethyl) pyrazol-4-yl]-5-morpholino-pyrazolo[1,5-a]pyrimidine-3-carboxamide

To a mixture of tert-butyl N-[3-[3-[[4-[3-(difluoromethyl)-4-[(5-morpholinopyrazolo[1,5-a] pyrimidine-3-carbonyl)amino]pyrazol-1-yl]cyclohexyl]methyl-methyl-amino]propoxy]propyl]carbamate (700 mg, 99.4 umol) in DCM (10 mL) was added TFA (34.0 mg, 298 umol). The reaction mixture was stirred at 20 °C for 0.5 hour. On completion, the reaction mixture was concentrated *in vacuo* to give the residue. The residue was purified by prep-HPLC (column: Phenomenex Synergi C18 150*30 mm*4 um; mobile phase: [water (0.225%FA)-ACN]; B%: 4%-34%) to give the title compound (12.8 mg, 21% yield) as a yellow gum. ¹H NMR (400 MHz, DMSO-*d₆*) δ 9.40 (s, 1H), 8.83 (d, *J =* 8.0 Hz, 1H), 8.38 (s, 1H), 8.29 (s, 1H), 7.24 - 6.95 (m, 1H), 6.91 (d, *J* = 8.0 Hz, 1H), 4.18 (d, *J* = 10.4 Hz, 1H), 3.79 (s, 4H), 3.74 - 3.70 (m, 4H), 3.69 (s, 1H), 3.41 (s, 2H), 2.81 (d, *J* = 7.6 Hz, 2H), 2.34 - 2.28 (m, 3H), 2.12 (s, 3H), 2.11 - 2.01 (m, 4H), 1.89 (d, *J=* 7.2 Hz, 2H), 1.80 - 1.70 (m, 4H), 1.67 - 1.58 (m, 2H), 1.56 - 1.47 (m, 1H), 1.28 (s, 2H), 1.09 - 0.95 (m, 2H). ¹H NMR (400 MHz, MeOH-*d₄*) δ 8.60 - 8.51 (m, 2H), 8.35 (d, *J* = 15.6 Hz, 2H), 7.03 - 6.72 (m, 2H), 4.27 - 4.16 (m, 1H), 3.93 - 3.79 (m, 8H), 3.58 (d, *J* = 9.2 Hz, 4H), 3.07 (d, *J* = 7.2 Hz, 2H), 2.85 (d, *J* = 7.2 Hz, 2H), 2.66 (d, *J =* 7.2 Hz, 2H), 2.58 (s, 3H), 2.22 (d, *J =* 7.6 Hz, 2H), 2.09 - 2.00 (m, 2H), 1.99 - 1.86 (m, 6H), 1.84 - 1.75 (m, 1H), 1.39 (s, 2H), 1.33 - 1.19 (m, 2H); LC-MS (ESI⁺) *m*/*z* 604.2 (M+H)⁺.

### Example 31. Synthesis of 4-[2-[4-[[3-(3-Aminopropoxy)propyl-methyl-amino]methyl]cyclohexyl]ethynyl]-1-[[(2S, 3S, 4S)-3-ethyl-4-fluoro-5-oxo-pyrrolidin-2-yl]methoxy]-7-methoxy-isoquinoline-6-carboxamide (I-316)

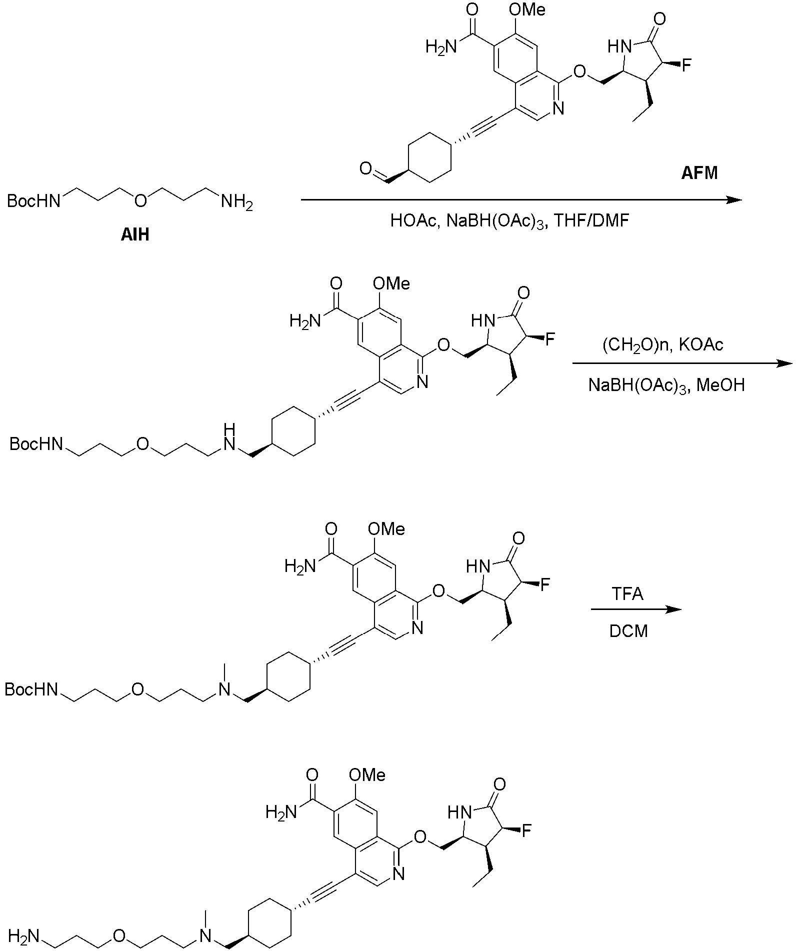

### Step 1 - Tert-butyl N-[3-[3-[[4-[2-[6-carbamoyl-1-[[(2S,3S,4S)-3-ethyl-4-fluoro-5-oxo-pyrrolidin-2-yl] methoxy]-7-methoxy-4-isoquinolyl]ethynyl]cyclohexyl]methylamino]propoxy]propyl]carbamate

To a mixture of tert-butyl N-[3-(3-aminopropoxy)propyl]carbamate (37.5 mg, 161 umol, Intermediate AIH) and 1-[[(2S,3S,4S)-3-ethyl-4-fluoro-5-oxo-pyrrolidin-2-yl]methoxy]-4-[2-(4-formyl cyclo hexyl)ethynyl]-7-methoxy-isoquinoline-6-carboxamide (40 mg, 80.7 umol, Intermediate AFM) in a mixed solvent of THF (2.0 mL) and DMF (0.5 mL) was added HOAc (4.85 mg, 80.7 umol) at 0°C. The mixture was stirred at 0 °C for 0.5 hour. Then, NaBH(OAc)₃ (34.2 mg, 161 umol) was added, and the mixture was stirred at 0 °C for 1.5 hours. On completion, the mixture was quenched with H₂O (5 mL) and then extracted with DCM (4 X 10 mL). The combined organic phase was dried over Na₂SO₄, filtered and the filtrate was concentrated *in vacuo* to give the title compound (67.0 mg, 96% yield) as a yellow solid. LC-MS (ESI⁺) m/z 712.2 (M+H)⁺.

### Step 2 - Tert-butyl N-[3-[3-[[4-[2-[6-carbamoyl-1-[[(2S,3S,4S)-3-ethyl-4-fluoro-5-oxo-pyrrolidin-2-yl] methoxy]-7-methoxy-4-isoquinolyl]ethynyl]cyclohexyl]methyl-methyl-amino]propoxy]propyl]carbmate

To a mixture of tert-butyl N-[3-[3-[[4-[2-[6-carbamoyl-1-[[(2S,3S,4S)-3-ethyl-4-fluoro-5-oxo-pyrro lidin-2-yl]methoxy]-7-methoxy-4-isoquinolyl]ethynyl]cyclohexyl]methylamino]propoxy]propyl]carbamate (67.0 mg, 94.1 umol) and HCHO (28.3 mg, 941 umol) in MeOH (5 mL) was added KOAc (27.7 mg, 282 umol) at 25 °C. The mixture was stirred at 25 °C for 0.5 hour. Then, NaBH₃CN (11.8 mg, 188 umol) was added, and the mixture was stirred at 25 °C for 16 hours. On completion, the reaction mixture was quenched with water (0.2 mL) at 25 °C, and then extracted with DCM (2 X 20 mL). The combined organic layers were dried over Na₂SO₄, filtered and concentrated *in vacuo* to give the title compound (40.0 mg, 59% yield) as yellow solid. LC-MS (ESI⁺) m/z 726.5 (M+H)⁺.

### Step 3 - 4-[2-[4-[[3-(3-Aminopropoxy)propyl-methyl-amino]methyl]cyclohexyl]ethynyl]-1-[[(2S, 3S, 4S)-3-ethyl-4-fluoro-5-oxo-pyrrolidin-2-yl]methoxy]-7-methoxy-isoquinoline-6-carboxamide

To a mixture of tert-butyl N-[3-[3-[[4-[2-[6-carbamoyl-1-[[(2S,3S,4S)-3-ethyl-4-fluoro-5-oxo-pyrro lidin-2-yl]methoxy]-7-methoxy-4-isoquinolyl]ethynyl]cyclohexyl]methyl-methyl-amino]propoxy]propyl]carbamate (40.0 mg, 55.1 umol) in DCM (2 mL) was added TFA (3.08 g, 2 mL) at 25 °C. The mixture was stirred at 25 °C for 2 hours. On completion, the mixture was concentrated *in vacuo* to give a residue. The residue was purified by prep-HPLC (column: Phenomenex luna C18 150*25 mm* 10 um; mobile phase: [water (0.1%TFA)-ACN]; B%: 11%-41%, 10 min) to afford the title compound (13.6 mg, 33% yield, TFA salt) as white solid. ¹H NMR (400 MHz, DMSO-*d*₆) δ 8.89 (s, 1H), 8.30 (s, 1H), 8.03 (s, 1H), 7.92 (br s, 1H), 7.78 (s, 1H), 7.76 (br s, 1H), 7.67 - 7.61 (m, 2H), 4.99 - 4.83 (m, 1H), 4.56 - 4.51 (m, 1H), 4.29 - 4.23 (m, 1H), 4.13 - 4.07 (m, 1H), 3.99 (s, 3H), 3.45 (t, *J* = 6.2 Hz, 4H), 2.88 - 2.82 (m, 2H), 2.80 (d, *J* = 4.8 Hz, 3H), 2.16 - 2.10 (m, 3H), 1.93 - 1.84 (m, 5H), 1.82 - 1.75 (m, 4H), 1.61 - 1.52 (m, 4H), 1.25 - 1.23 (m, 1H), 1.19 - 1.18(m, 1H), 1.17 - 1.06 (m, 4H), 1.02 (t, *J* = 7.4 Hz, 3H). LC-MS (ESI⁺) m/z 626.3 (M+H)⁺.

### Example 32. Quantification of Ikaros and Aiolos degradation.

Degradation of Ikaros (protein product of gene IKZF1) and Aiolos (protein product of gene IKZF3) were determined by quantitative immunoblotting as follows. OCI-LY10 cells, 2x10⁶ cells/well, were treated with listed concentrations of IRAK4 degraders or control compounds in 6 well plates for 6 h. Cells were collected, washed with cold PBS, lysed in RIPA buffer (Boston BioProducts BP-115D) with protease / phosphatase inhibitor cocktail (Roche 05892791001 / Roche 04906837001) and centrifuged at 13000 RPM for 20 min to precipitate insoluble material. The supernatant fraction was diluted in SDS-PAGE loading buffer (Beyotime Bio P0015) and 20 µL of each sample was resolved on 4-12% Bis-Tris SDS-PAGE gels (Novex, WG1402BOX). Resolved samples were transferred to nitrocellulose membranes by wet electro-transfer method at 250 mV for 1.5 h. The membrane was blocked with LICOR blocking buffer (LI-COR, 927-50000) for 1 hour, washed three times with TBST (CST#9997S) for 5 minutes each and incubated with primary antibody prepared in block buffer with 0.1% Tween-20 (Solarbio, P8220) at 4 °C overnight. Ikaros antibody was rabbit monoclonal D6N9Y (CST#14859), at 1:1000 dilution. Aiolos antibody was rabbit monoclonal D1C1E (CST#15103), at 1:1000 dilution. Signal was normalized to mouse anti-beta-Actin monoclonal 8H10D10 (CST#3700) used at 1:10,000 dilution. After incubation in primary antibodies, membranes were washed three times with TBST, 5 minutes each, incubated with fluorescently labeled secondary antibodies anti-rabbit IgG (Licor,926-32211) at 1:5000 dilution; anti-mouse IgG (LI-COR, 926-68070) at 1:5000 dilution, for 1 hour at RT. After incubation in secondary, membranes were washed three times with TBST, 5 minutes each and read on LICOR Odyssey imager. Data are reported as signal for Ikaros or Aiolos relative to signal for actin, and normalized to DMSO-treated control.

### Example 33. Synthesis of N-[1-[4-(3-aminoprop-1-ynyl)phenyl]-3-(difluoromethyl)pyrazol-4-yl]-2-[2-(cyclopropyl methylamino)-4-pyridyl]oxazole-4-carboxamide (I-391)

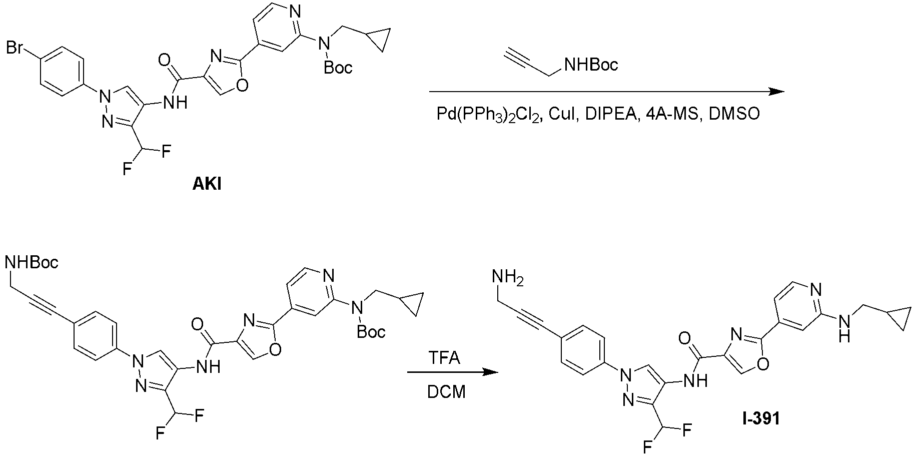

### Step 1 - Tert-butyl N-[4-[4-[[1-[4-[3-(tert-butoxycarbonylamino)prop-1-ynyl]phenyl]-3- (difluoromethyl)pyrazol-4-yl]carbamoyl]oxazol-2-yl]-2-pyridyl]-N-(cyclopropylmethyl)carbamate

A mixture of tert-butyl N-[4-[4-[[1-(4-bromophenyl)-3-(difluoromethyl)pyrazol-4-yl]carbamoyl] oxazol-2-yl]-2-pyridyl]-N-(cyclopropylmethyl)carbamate (100 mg, 158 umol, Intermediate AKI), tert-butyl N-prop-2- ynylcarbamate (49.3 mg, 318 umol, CAS# 92136-39-5), Pd(PPh₃)₂Cl₂ (11.2 mg, 15.9 umol), CuI (3.03 mg, 15.9 umol), DIPEA (103 mg, 794 umol, 138 uL) and 4Å molecular sieves (100 mg) in DMSO (4.00 mL) was degassed and purged with N₂ for 3 times, and then the mixture was stirred at 80 °C for 2 hours under N₂ atmosphere. On completion, the reaction mixture was filtered and concentrated *in vacuo* to give a residue. The residue was purified by reversed-phase (0.1% FA condition) to give the title compound (90.0 mg, 80% yield) as a yellow solid. ¹H NMR (400 MHz, Chloroform-d) δ 9.16 (s, 1H), 8.85 (s, 1H), 8.54 (d, *J* = 4.8 Hz, 1H), 8.42 (s, 1H), 8.36 (s, 1H), 7.73 - 7.65 (m, 3H), 7.58 - 7.52 (m, 2H), 7.12 - 6.76 (m, 1H), 4.18 (d, *J* = 4.8 Hz, 2H), 3.97 (d, *J* = 7.0 Hz, 2H), 1.58 (s, 9H), 1.49 (s, 9H), 1.33 - 1.18 (m, 2H), 0.50 - 0.38 (m, 2H), 0.35 - 0.21 (m, 2H).

### Step 2 - N-[1-[4-(3-aminoprop-1-ynyl)phenyl]-3-(difluoromethyl)pyrazol-4-yl]-2-[2-(cyclopropyl methylamino)-4-pyridyl]oxazole-4-carboxamide

To a solution of tert-butyl N-[4-[4-[[1-[4-[3-(tert-butoxycarbonylamino)prop-1-ynyl]phenyl] -3-(difluoromethyl)pyrazol-4-yl]carbamoyl]oxazol-2-yl]-2-pyridyl]-N-(cyclopropylmethyl)carbamate (80.0 mg, 113 umol) in DCM (2 mL) was added TFA (1.54 g, 13.5 mmol, 1.00 mL). The mixture was stirred at 20 °C for 1 hour. On completion, the reaction mixture was concentrated *in vacuo* to give a residue. The residue was purified by prep-HPLC (column: Waters Xbridge 150*25mm* 5um; mobile phase: [water (0.05% ammoniahydroxide v/v)-ACN]; B%: 40%-70%, 0 min]; B%: 40%-70%,10 min) to give the title compound (26.8 mg, 46% yield) as a white solid. ¹H NMR (400 MHz, DMSO-*d₆*) δ 10.46 - 9.62 (m, 1H), 8.97 (s, 1H), 8.84 (s, 1H), 8.16 (d, *J=* 5.2 Hz, 1H), 7.87 (d, *J =* 8.8 Hz, 2H), 7.55 (d, *J =* 8.8 Hz, 2H), 7.46 - 7.14 (m, 1H), 7.13 - 7.06 (m, 2H), 7.04 (dd, *J* = 1.3, 5.3 Hz, 1H), 3.53 (s, 2H), 3.18 (t, *J =* 6.4 Hz, 2H), 2.15 - 1.62 (m, 2H), 1.14 - 0.99 (m, 1H), 0.51 - 0.41 (m, 2H), 0.26 - 0.17 (m, 2H). LC-MS (ESI⁺) *m*/*z* 504.3 (M+H)⁺.

### Example 34. Synthesis of 2-[[[4-[4-[[2-[2-(Cyclopropylmethylamino)-4-pyridyl]oxazole-4-carbonyl]amino]-3 -(difluoromethyl)pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoic acid (I-396)

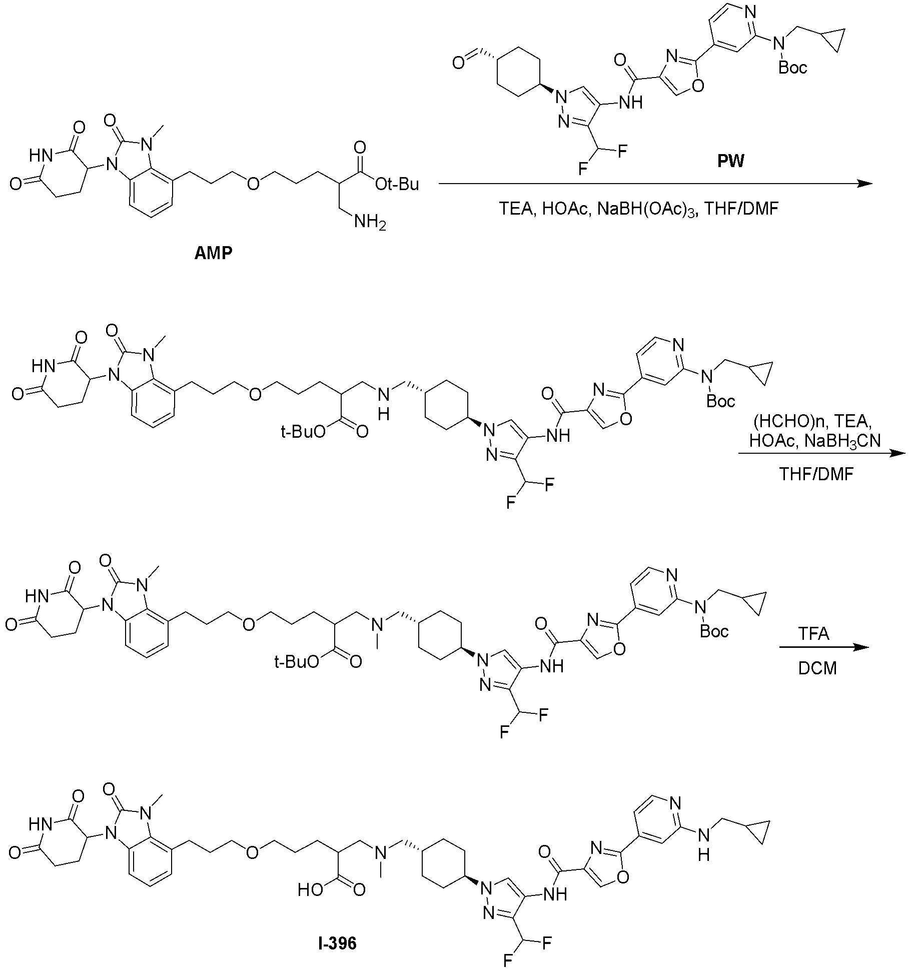

### Step 1 - Tert-butyl 2-[[[4-[4-[[2-[2-[tert-butoxycarbonyl(cyclopropylmethyl)amino]-4-pyridyl]oxazole -4-carbonyl]amino]-3-(difluoromethyl)pyrazol-1-yl]cyclohexyl]methylamino]methyl]-5-[3-[1-(2,6-dioxo-3-piperidyl)-3-methyl-2-oxo-benzimidazol-4-yl]propoxy]pentanoate

A mixture of tert-butyl 2-(aminomethyl)-5-[3-[1-(2,6-dioxo-3-piperidyl)-3-methyl-2-oxo -benzimidazol-4-yl]propoxy]pentanoate (100 mg, 198 umol, Intermediate AMP), tert-butylN- (cyclopropylmethyl)-N-[4-[4-[[3-(difluoromethyl)-1-(4-formylcyclohexyl)pyrazol-4-yl]carbamoyl]oxazol-2-yl]-2-pyridyl]carbamate (65.0 mg, 111 umol, Intermediate PW) and HOAc (35.8 mg, 596 umol) in THF (3 mL) and DMF (1 mL) was stirred at 0 °C for 0.5 hour. Then NaBH(OAc)₃ (51.0 mg, 240 umol) was added at 0 °C. The mixture was stirred at 0 - 25 °C for 16 hours. On completion, the reaction was quenched with water (0.2 mL). The mixture was concentrated *in vacuo.* The residue was purified by reversed phase flash (FA condition) to give the title compound (90.0 mg, 42% yield) as light yellow solid. ¹H NMR (400 MHz, DMSO-*d₆*) δ 11.09 (s, 1H), 9.76 (s, 1H), 9.00 (s, 1H), 8.59 (d, *J* = 5.2 Hz, 1H), 8.29 (s, 1H), 8.17 (s, 1H), 7.69 - 7.68 (m, 1H), 7.29 - 7.00 (m, 1H), 6.96 (d, *J =* 5.2 Hz, 2H), 6.89 - 6.81 (m, 1H), 5.43 - 5.31 (m, 1H), 4.23 - 4.12 (m, 1H), 3.86 (d*, J =* 7.2 Hz, 2H), 3.56 (s, 3H), 3.41 (m, 4H), 2.98 - 2.83 (m, 3H), 2.77 - 2.69 (m, 1H), 2.65 - 2.52 (m, 4H), 2.42 - 2.34 (m, 4H), 2.05 - 1.94 (m, 3H), 1.92 - 1.79 (m, 5H), 1.77 - 1.66 (m, 2H), 1.51 (s, 9H), 1.50 - 1.48 (m, 2H), 1.40 (s, 9H), 1.27 - 1.12 (m, 2H), 1.11 - 0.98 (m, 2H), 0.42 - 0.41 (m, 2H), 0.24 - 0.23 (m, 2H).

### Step 2 - Tert-butyl 2-[[[4-[4-[[2-[2-[tert-butoxycarbonyl(cyclopropylmethyl)amino]-4-pyridyl]oxazole -4-carbonyl]amino]-3-(difluoromethyl)pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoate

A mixture of tert-butyl 2-[[[4-[4-[[2-[2-[tert-butoxycarbonyl(cyclopropylmethyl)amino]-4-pyridyl] oxazole-4-carbonyl]amino]-3-(difluoromethyl)pyrazol-1-yl]cyclohexyl]methylamino]methyl]-5-[3-[1-(2,6-dioxo-3-piperidyl)-3-methyl-2-oxo-benzimidazol-4-yl]propoxy]pentanoate (90.0 mg, 84.0 umol), paraformaldehyde (8.00 mg, 88.0 umol, CAS# 30525-89-4) and KOAc (2.05 mg, 254 umol) in THF (1.5 mL) and DMF (0.5 mL) was stirred at 25 °C for 0.5 hour. Then NaBH₃CN (8.00 mg, 127 umol) was added at 25 °C. The mixture was stirred at 25 °C for 2 hours. On completion, the reaction was quenched with water (0.2 mL). The mixture was concentrated *in vacuo.* The residue was purified by reversed phase flash (FA condition) to give the title compound (65.0 mg, 71% yield) as light yellow solid. LC-MS (ESI⁺) m/z 1085.2 (M+H)⁺.

### Step 3 - 2-[[[4-[4-[[2-[2-(Cyclopropylmethylamino)-4-pyridyl]oxazole-4-carbonyl]amino]-3 -(difluoromethyl)pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoic acid

To a solution of tert-butyl 2-[[[4-[4-[[2-[2-[tert-butoxycarbonyl(cyclopropylmethyl)amino]-4-pyridyl] oxazole-4-carbonyl]amino]-3-(difluoromethyl)pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoate (60.0 mg, 55.2 umol) in DCM (2 mL) was added TFA (1 mL) at 25 °C. The mixture was stirred at 25 °C for 6 hours. On completion, the mixture was concentrated *in vacuo.* The residue was purified by prep-HPLC (column: Waters Xbridge 150*25mm* 5um; mobile phase: [water (10mM NH4HCO3)-ACN]; B%: 22%-52%, 10min) to give the title compound (28.6 mg, 54% yield) as white solid. ¹H NMR (400 MHz, DMSO-*d₆*) δ 11.07 (m, 1H), 9.67 (s, 1H), 8.91 (s, 1H), 8.17 (s, 1H), 8.15 (d, *J* = 5.2 Hz, 1H), 7.30 - 6.83 (m, 8H), 5.38 - 5.33 (m, 1H), 4.22 - 4.13 (m, 1H), 3.56 (s, 3H), 3.44 (m, 2H), 3.20 - 3.16 (m, 4H), 2.99 - 2.83 (m, 4H), 2.65 - 2.57 (m, 2H), 2.25 (m, 1H), 2.22 (s, 3H), 2.20 - 2.15 (m, 1H), 2.07 - 1.88 (m, 5H), 1.87 - 1.72 (m, 5H), 1.65 - 1.43 (m, 6H), 1.12 - 0.98 (m, 3H), 0.49 - 0.41 (m, 2H), 0.26 - 0.17 (m, 2H); LC-MS (ESI⁺) m/z 929.6 (M+H)⁺.

### Example 35. Synthesis of5-((1R,4R)-2-oxa-5-azabicyclo[2.2.1]heptan-5-yl)-N-(3-(difluoromethyl)-1-((1r,4R)-4-(((4-(2-((2-(2,6-dioxopiperidin-3-yl)-1,3-dioxoisoindolin-4-yl)amino)ethyl)phenethyl)(methyl)amino)methyl)cyclohexyl)-1H-pyrazol-4-yl)pyrazolo|[1,5-a]pyrimidine-3-carboxamide (I-336)

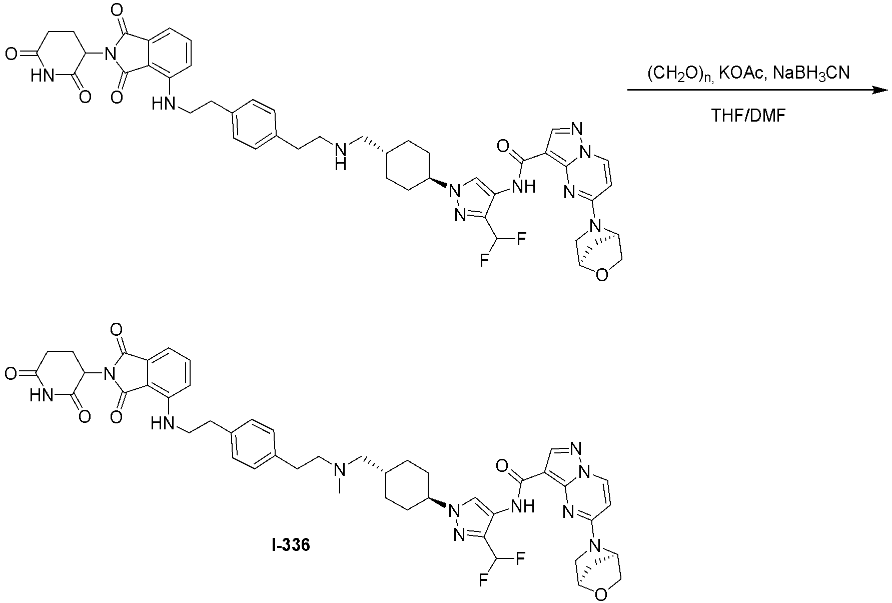

To a solution of N-[3-(difluoromethyl)-1-[4-[[2-[4-[2-[[2-(2,6-dioxo-3-piperidyl)-1, 3-dioxo-isoindolin-4-yl]amino]ethyl]phenyl]ethylamino]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (30.0 mg, 33.7 umol, **I-346)** in DMF (1 mL) and THF (1 mL) was added (CH₂O)ₙ (3.24 mg, 101 umol) and KOAc (16.5 mg, 168 umol). The mixture was stirred at 15 °C for 0.5 hr, then NaBH₃CN (2.12 mg, 33.7 umol) was added to the mixture. The mixture was stirred at 15 °C for 2.5 hrs. On completion, the mixture was added 0.5 mL of water and concentrated to remove most of solvent *in vacuo.* The residue was purified by pre-HPLC (column: Phenomenex Synergi C18 150*25*10um;mobile phase: [water(0.225%FA)-ACN];B%: 22%-52%,10min) to give the title compound (20.5 mg, 64% yield) as yellow solid. ¹H NMR (400 MHz, DMSO-*d₆*) δ 11.08 (s, 1H), 9.49 (d, *J* = 6.4 Hz, 1H), 8.78 (d, *J =* 7.6 Hz, 1H), 8.40 - 8.22 (m, 2H), 7.57 (dd, *J =* 7.6, 8.4 Hz, 1H), 7.26 - 6.77 (m, 8H), 6.56 - 6.44 (m, 1H), 5.32 - 4.95 (m, 2H), 4.21 - 4.07 (m, 1H), 3.87 - 3.61 (m, 4H), 3.59 - 3.42 (m, 4H), 2.90 - 2.82 (m, 3H), 2.70 - 2.63 (m, 2H), 2.61 - 2.55 (m, 2H), 2.24 - 2.14 (m, 5H), 2.04 - 2.00 (m, 6H), 1.88 - 1.79 (m, 2H), 1.72 - 1.60 (m, 2H), 1.70 (br d, *J* = 2.4 Hz, 1H), 1.05 - 0.95 (m, 2H); LC-MS (ESI+) m/z 904.2 (M+1)⁺.

### Example 36. Synthesis of N-[3-(difluoromethyl)-1-[4-[[[4-[2-[[2-(2,6-dioxo-3-piperidyl)-1,3-dioxo-isoindolin-4-yl] amino]ethyl]phenyl]methyl-methyl-amino]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (I-341)

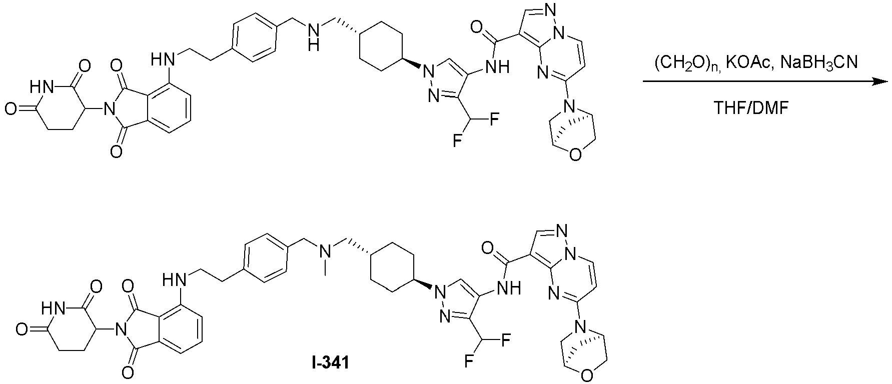

A mixture of N-[3-(difluoromethyl)-1-[4-[[[4-[2-[[2-(2,6-dioxo-3-piperidyl)-1,3-dioxo-isoindolin-4-yl] amino]ethyl]phenyl]methylamino]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (60.0 mg, 68.5 umol, **I-351),** and paraformaldehyde (6.20 mg, 205 umol, CAS# 30525-89-4), KOAc (20.0 mg, 203 umol) in THF (1.2 mL) and DMF (0.4 mL) was stirred at 25 °C for 1 hour. Then NaBH₃CN (6.46 mg, 102 umol) was added to the reaction mixture at 25 °C. The mixture was stirred at 25 °C for 2 hours. On completion, the reaction was quenched with water (0.2 mL). The mixture was concentrated *in vacuo.* The residue was purified by prep-HPLC (column: Waters Xbridge 150*25 mm* 5 um; mobile phase: [water (10 mM NH₄HCO₃)-ACN]; B%: 43%-73%, 10 min) to give the title compound (38.2 mg, 60% yield) as yellow solid. ¹H NMR (400 MHz, DMSO-d₆) δ 11.08 (s, 1H), 9.49 (d, *J =* 6.0 Hz, 1H), 8.78 (d, *J =* 7.6 Hz, 1H), 8.37 (d, *J =* 4.0 Hz, 1H), 8.25 (d, *J* = 5.6 Hz, 1H), 7.57 (t, *J =* 8.0 Hz, 1H), 7.28 - 7.21 (m, 4H), 7.14 (d, *J =* 8.8 Hz, 1H), 7.12 - 6.95 (m, 2H), 6.86 (d, *J =* 8.0 Hz, 2H), 5.31 - 4.98 (m, 2H), 4.77 (d, *J* = 16.4 Hz, 1H), 4.23 - 4.10 (m, 1H), 3.84 - 3.72 (m, 2H), 3.65 - 3.52 (m, 3H), 3.47 - 3.38 (m, 3H), 2.88 (t, *J =* 6.8 Hz, 3H), 2.62 - 2.53 (m, 2H), 2.14 - 2.12 (m, 2H), 2.10 (s, 3H), 2.06 - 1.91 (m, 7H), 1.79 - 1.57 (m, 3H), 1.05 - 0.91 (m, 2H); LC-MS (ESI⁺) m/z 890.4 (M+H)⁺.

### Example 37. Synthesis of 4-[2-[1-[2-[2-[[2-(2,6-dioxo-3-piperidyl)-1,3-dioxo-isoindolin-4-yl]amino]ethoxy]ethyl]- 4-piperidyl]ethynyl]-1-[[(2S,3S,4S)-3-ethyl-4-fluoro-5-oxopyrrolidin-2-yl]methoxy]-7-methoxy-isoquinoline-6-carboxamide (I-357)

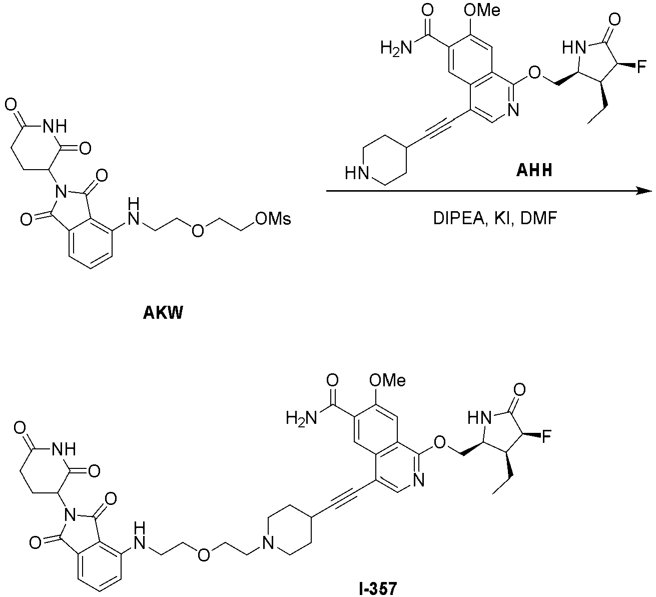

To a solution of 2-[2-[[2-(2,6-dioxo-3-piperidyl)-1,3-dioxo-isoindolin-4-yl]amino]ethoxy]ethyl methanesulfonate (75.4 mg, 171 umol, Intermediate AKW), 1-[[(2S,3S,4S)-3-ethyl-4-fluoro-5-oxo- pyrrolidin-2-yl]methoxy]-7-methoxy-4-[2-(4-piperidyl)ethynyl]isoquinoline-6-carboxamide (50.0 mg, 85.8 umol, TFA salt, Intermediate AHH) in DMF (2.00 mL) was added DIPEA (55.4 mg, 429 umol) and KI (1.42 mg, 8.58 umol). The reaction mixture was stirred at 80 °C for 3 hrs. On completion, the reaction mixture was concentrated *in vacuo.* The residue was purified by Prep-HPLC (column: Phenomenex Synergi C18 150*25*10 um; mobile phase: [water (0.225% FA)-ACN]; B%: 12%-42%, 10 min) to give the title compound (16.4 mg, 22% yield) as yellow solid. ¹H NMR (400MHz, DMSO-*d₆*) δ 11.09 (s, 1H), 8.88 (s, 1H), 8.33 (s, 1H), 8.25 (s, 1H), 8.04 (s, 1H), 7.90 (s, 1H), 7.77 (s, 1H), 7.75 (s, 1H), 7.62 - 7.52 (m, 1H), 7.15 (d, *J =* 8.4 Hz, 1H), 7.03 (d, *J =* 7.2 Hz, 1H), 6.65 - 6.58 (m, 1H), 5.10 - 5.02(m, 1H), 5.00 - 4.82 (m, 1H), 4.60 - 4.50 (m, 1H), 4.32 - 4.22 (m, 1H), 4.16 - 4.07 (m, 1H), 3.99 (s, 3H), 3.64 - 3.60 (m, 2H), 3.60 - 3.57 (m, 2H), 3.51 - 3.48 (m, 2H), 3.46 - 3.45 (m, 2H), 2.91 - 2.83 (m, 1H), 2.81 - 2.70 (m, 3H), 2.66 - 2.54 (m, 2H), 2.32 - 2.23 (m, 2H), 2.06 - 1.99 (m, 1H), 1.95 - 1.85 (m, 2H), 1.71 - 1.56 (m, 4H), 1.02 (t, *J =* 7.2 Hz, 3H), LC-MS (ESI⁺) *m*/*z* 812.5 (M+H)⁺.

### Example 38. Synthesis of N-[3-(difluoromethyl)-1-[4-[3-[3-[1-(2,6-dioxo-3-piperidyl)-3-methyl-2-oxo-benzimidazol- 4-yl]phenyl]propoxymethyl]cyclohexyl]pyrazol-4-yl]-5-morpholino-pyrazolo[1,5-a]pyrimidine-3-carboxamide

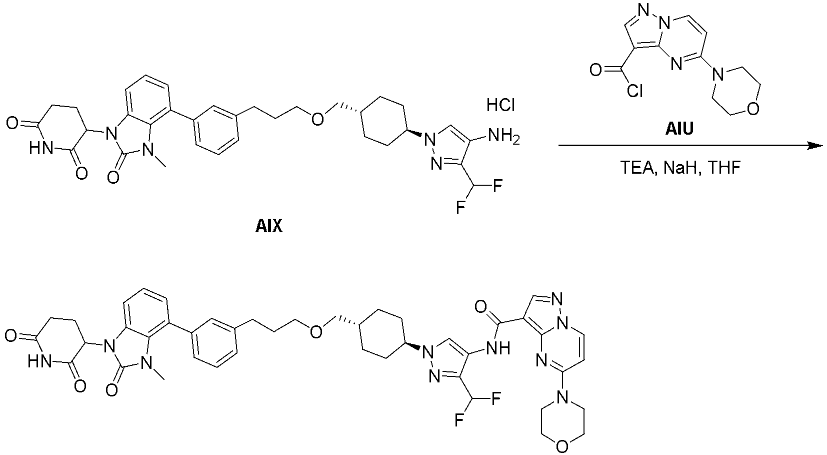

To a solution of 3-[4-[3-[3-[[4-[4-amino-3-(difluoromethyl)pyrazol-1-yl]cyclohexyl]methoxy] propyl]phenyl]-3-methyl-2-oxo-benzimidazol-1-yl]piperidine-2,6-dione (40.0 mg, 60.8 umol, HCl salt, Intermediate AIX) and TEA (18.4 mg, 182 umol) in THF (1 mL) was added NaH (9.74 mg, 243 umol, 60% dispersion in mineral oil) at 0 °C. The mixture was stirred at 0 °C for 0.5 hr, then 5-morpholinopyrazolo[1,5-a]pyrimidine-3-carbonyl chloride (24.3 mg, 91.3 umol, Intermediate AIU) was added. The mixture was stirred at 20 °C for 2 hrs. On completion, the reaction was quenched with water (0.1 mL) and concentrated *in vacuo* to give a residue. The residue was purified by prep-HPLC (column: Phenomenex Synergi C18 150*25*10um; mobile phase: [water (0.05%HCl)-ACN]) to give the title compound (1.78 mg, 3% yield, HCl salt) as yellow solid. ¹H NMR (400MHz, DMSO-*d*₆) δ11.13 (s, 1H), 8.76 (d, *J =* 8.0 Hz, 1H), 8.26 (s, 1H), 7.85 (s, 1H), 7.43 - 7.36 (m, 1H), 7.32 - 7.24 (m, 3H), 7.23 - 6.95 (m, 3H), 6.90 - 6.85 (m, 2H), 5.44 (dd, *J =* 5.2*,* 12.8 Hz, 1H), 4.19 - 4.10 (m, 1H), 4.03 (d, *J =* 6.0 Hz, 2H), 3.78 - 3.68 (m, 8H), 3.08 (s, 2H), 2.89 (t, *J =* 8.8 Hz, 2H), 2.84 (s, 3H), 2.77 - 2.70 (m, 3H), 2.68 - 2.64 (m, 1H), 2.08 - 1.90 (m, 7H), 1.81 - 1.65 (m, 3H), 1.34 - 1.19 (m, 2H); LC-MS (ESI⁺) *m*/*z* 851.4 (M+H)⁺.

### Example 39. Synthesis of 2-[[[4-[3-(Difluoromethyl)-4-[[5-[(1R,4R)-2-oxa-5-azabicyclo[2.2.1]heptan-5-yl]pyrazolo [1,5-a]pyrimidine-3-carbonyl]amino]pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoic acid (I-495)

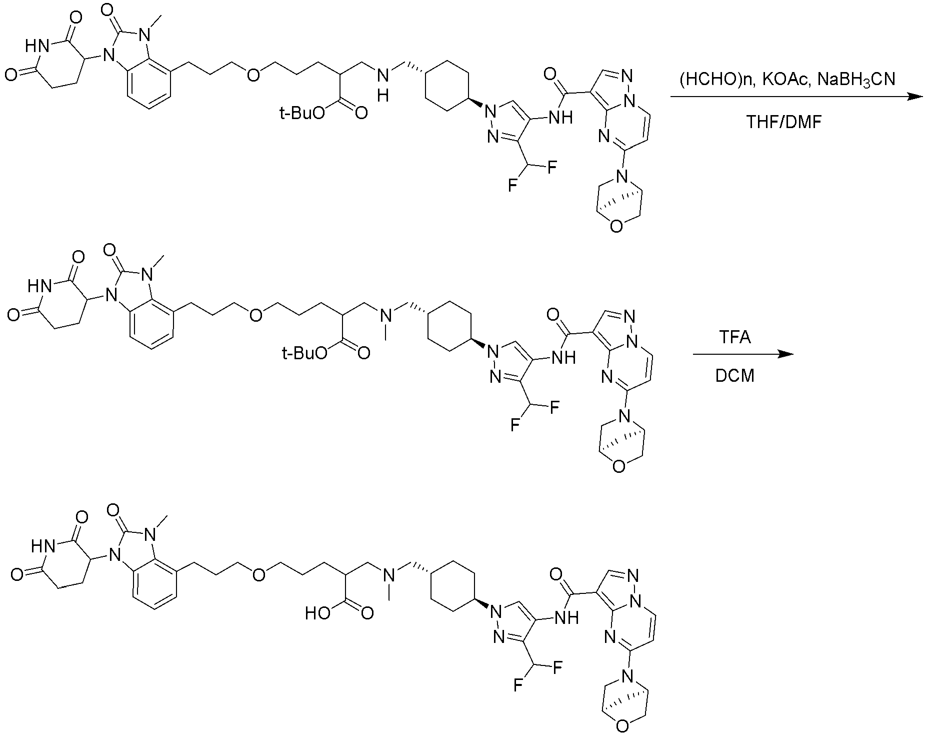

### Step 1 - Tert-butyl 2-[[[4-[3-(difluoromethyl)-4-[[5-[(1R,4R)-2-oxa-5-azabicyclo[2.2.1]heptan-5 -yl]pyrazolo[1,5-a]pyrimidine-3-carbonyl]amino]pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoate

A mixture of tert-butyl 2-[[[4-[3-(difluoromethyl)-4-[[5-[(1R,4R)-2-oxa-5-azabicyclo[2.2.1]heptan-5 -yl]pyrazolo[1,5-a]pyrimidine-3-carbonyl]amino]pyrazol-1-yl]cyclohexyl]methylamino]methyl]-5-[3-[1-(2,6-dioxo-3-piperidyl)-3-methyl-2-oxo-benzimidazol-4-yl]propoxy]pentanoate (50.0 mg, 51.4 umol, synthesized via Step 1 of Method 2 using **I-470),** KOAc (15.0 mg, 152 umol) and paraformaldehyde (5.00 mg, 154 umol) in THF (1.2 mL) and DMF (0.4 mL) was stirred at 25 °C for 0.5 hour. Then NaBH₃CN (5.00 mg, 79.5 umol) was added at 25 °C and stirred for 2 hours. On completion, the reaction was quenched with water (0.2 mL). The mixture was concentrated *in vacuo.* The residue was purified by reversed phase flash (FA condition) to give the title compound (35.0 mg, 69% yield) as light yellow solid. LC-MS (ESI⁺) m/z 986.5 (M+H)⁺.

### Step 2 - 2-[[[4-[3-(Difluoromethyl)-4-[[5-[(1R,4R)-2-oxa-5-azabicyclo[2.2.1]heptan-5-yl]pyrazolo [1,5-a]pyrimidine-3-carbonyl]amino]pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoic acid

To a solution of tert-butyl 2-[[[4-[3-(difluoromethyl)-4-[[5-[(1R,4R)-2-oxa-5-azabicyclo[2.2.1] heptan-5-yl]pyrazolo[1,5-a]pyrimidine-3-carbonyl]amino]pyrazol-1-yl]cyclohexyl]methyl-methyl-amino]methyl]-5-[3-[1-(2,6-dioxo-3-piperidyl)-3-methyl-2-oxo-benzimidazol-4-yl]propoxy]pentanoate (35.0 mg, 35.4 umol) in DCM (1.5 mL) was added TFA (0.5 mL) at 25 °C. The mixture was stirred at 25 °C for 3 hours. On completion, the mixture was concentrated *in vacuo.* The residue was purified by prep-HPLC (Phenomenex Synergi C18 150*25*10um; mobile phase: [water (0.225%FA)-ACN]; B%: 21%-51%, 10min) to give the title compound (10.8 mg, 33% yield) as white solid. ¹H NMR (400 MHz, DMSO-*d₆*) δ 11.07 (s, 1H), 9.49 (d, *J =* 6.4 Hz, 1H), 8.78 (d, *J =* 8.0 Hz, 1H), 8.37 (d, *J =* 4.0 Hz, 1H), 8.25 (d, *J =* 5.6 Hz, 1H), 7.26 - 6.41 (m, 5H), 5.42 - 5.02 (m, 2H), 4.77 (d, *J* = 16.4 Hz, 1H), 4.24 - 4.09 (m, 1H), 3.85 - 3.71 (m, 2H), 3.66 - 3.58 (m, 2H), 3.56 (s, 3H), 3.43 (m, 2H), 2.99 - 2.83 (m, 4H), 2.64 - 2.62 (m, 1H), 2.31 - 2.24 (m, 2H), 2.21 (s, 3H), 2.17 - 2.15 (m, 2H), 2.04 - 1.92 (m, 6H), 1.89 - 1.68 (m, 7H), 1.62 - 1.46 (m, 6H), 1.02 - 1.00 (m, 2H); LC-MS (ESI⁺) m/z 930.6 (M+H)⁺.

### Example 40. Synthesis of N-[3-(difluoromethyl)-1-[2-[[[1-[[1-(2,6-dioxo-3-piperidyl)-3-methyl-2-oxo-benzimidazol-4- yl]methyl]-4-piperidyl]-methyl-amino]methyl]-1,3-dioxan-5-yl]pyrazol-4-yl]-5-[(1R,4R)-2-oxa-5-azabicyclo[2.2.1]heptan-5-yl]pyrazolo[1,5-a]pyrimidine-3-carboxamide (I-507)

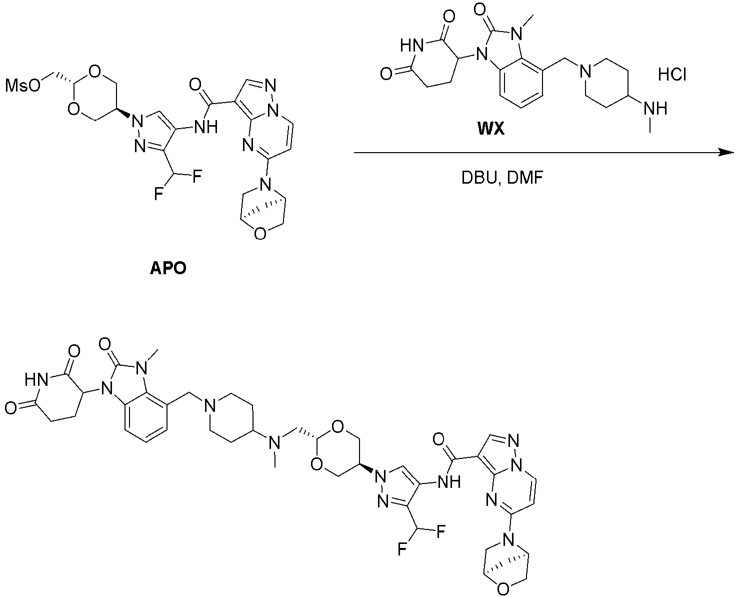

To a solution of 3-[3-methyl-4-[[4-(methylamino)-1-piperidyl]methyl]-2-oxo-benzimidazol-1-yl] piperidine-2,6-dione (18.5 mg, 43.9 umol, HCl, Intermediate WX) and [5-[3-(difluoromethyl)-4 -[[5-[(1R,4R)-2-oxa-5-azabicyclo[2.2.1]heptan-5-yl]pyrazolo[1,5-a]pyrimidine-3-carbonyl]amino]pyrazol-1-yl]-1,3-dioxan-2-yl]methyl methanesulfonate (25.0 mg, 43.9 umol, Intermediate APO) in DMF (2 mL) was added DBU (13.4 mg, 87.7 umol, 13.2 uL). The reaction mixture was stirred at 100 °C for 12 hrs . On completion, the reaction mixture was quenched with water (0.5 mL) and concentrated *in vacuo* to give the residue. The residue was purified by Prep-HPLC (column: Waters Xbridge 150*25mm* 5um;mobile phase: [water(10mM NH4HCO3)-ACN]) (3 times) to give the title compound (1.02 mg, 2% yield) as a off-white solid. LC-MS (ESI⁺) m/z 859.4 (M+1)⁺.

### Example 41. Synthesis of N-[3-(difluoromethyl)-1-[4-[5-[1-(2,6-dioxo-3-piperidyl)-3-methyl-2-oxo-benzimidazol-4-yl] pent-4-ynoxymethyl]phenyl]pyrazol-4-yl]-5-[(1R,4R)-2-oxa-5-azabicyclo[2.2.1]heptan-5-yl]pyrazolo[1,5-a]pyrimidine-3-carboxamide (I-498)

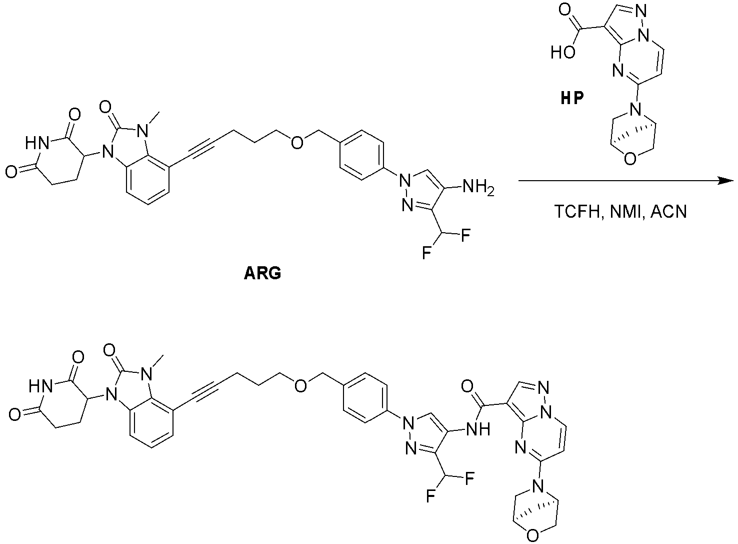

To a mixture of 5-[(1R)-2-oxa-5-azabicyclo[2.2.1]heptan-5-yl]pyrazolo[1,5-a]pyrimidine-3-carboxylic acid (23.1 mg, 88.8 umol, Intermediate AEH) in ACN (10 mL) was added [chloro (dimethylamino) methylene]-dimethyl-ammonium;hexafluorophosphate (32.4 mg, 115 umol) and 1-methylimidazole (25.5 mg, 311 umol). Then 3-[4-[5-[[4-[4-amino-3-(difluoromethyl)pyrazol-1-yl]phenyl]methoxy] pent-1-ynyl]-3-methyl-2-oxo-benzimidazol-1-yl]piperidine-2,6-dione (50.0 mg, 88.8 umol, Intermediate ARG) was added. The reaction mixture was stirred at 25 °C for 0.5 hour. On completion, the reaction mixture was concentrated *in vacuo* to give the residue. The residue was as purified by Prep-HPLC (column: Phenomenex Synergi C18 150*25*10um; mobile phase: [water (0.225%FA)-ACN]; B%: 44%-74%) to give the title compound (24.3 mg, 34% yield) as a white solid. LC-MS (ESI⁺) *m*/*z* 805.4 (M+H) ⁺; ¹H NMR (400 MHz, DMSO-*d₆*) δ 11.09 (s, 1H), 9.61 (d, *J* = 4.0 Hz, 1H), 8.96 (d, *J* = 2.0 Hz, 1H), 8.80 (d, *J* = 7.6 Hz, 1H), 8.31 (d, *J* = 4.8 Hz, 1H), 7.79 (d, *J =* 8.4 Hz, 2H), 7.49 (d, *J =* 8.4 Hz, 2H), 7.45 - 7.14 (m, 1H), 7.10 (d, *J =* 8.0 Hz, 1H), 7.06 - 7.01 (m, 1H), 7.00 - 6.94 (m, 1H), 6.91 - 6.45 (m, 1H), 5.38 (d, *J =* 18.0 Hz, 1H), 5.33 - 5.06 (m, 1H), 4.79 (d, *J =* 12.8 Hz, 1H), 4.55 (s, 2H), 3.84 (s, 2H), 3.67 - 3.63 (m, 2H), 3.62 (s, 3H), 3.61 - 3.44 (m, 2H), 2.93 - 2.82 (m, 1H), 2.67 (s, 1H), 2.64 - 2.56 (m, 3H), 2.09 - 1.95 (m, 3H), 1.88 (d, *J =* 6.4 Hz, 2H).

### Example 42. Synthesis of N-[3-(difluoromethyl)-1-[4-[[2-[[1-(2,6-dioxo-3-piperidyl)-3-methyl-2-oxo-benzimidazol-4- yl]methyl]-2,7-diazaspiro[3.5]nonan-7-yl]methyl]phenyl]pyrazol-4-yl]-5-[(1R)-2-oxa-5-azabicyclo[2.2.1]heptan-5-yl]pyrazolo[1,5-a]pyrimidine-3-carboxamide (I-464)

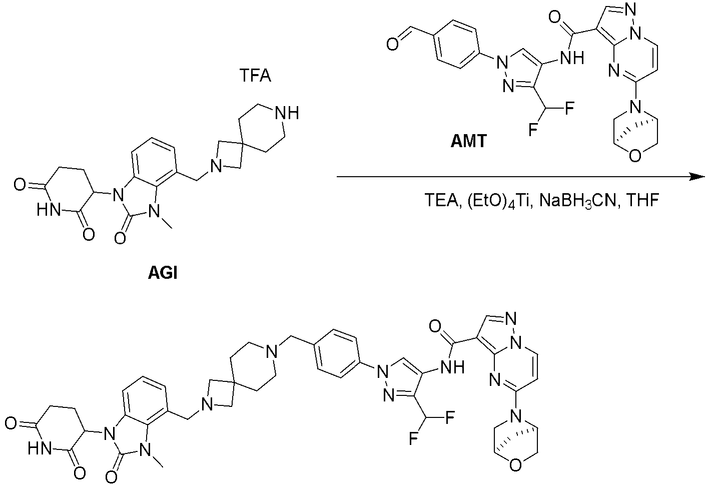

To a mixture of 3-[4-(2,7-diazaspiro[3.5]nonan-2-ylmethyl)-3-methyl-2-oxo-benzimidazol-1-yl] piperidine-2,6-dione (45.8 mg, 89.7 umol, TFA, Intermediate AGI) in THF (10 mL) was added TEA (27.2 mg, 269 umol). The mixture was stirred at 25 °C for 0.5 hour. N-[3-(difluoromethyl)-1-(4-formylphenyl)pyrazol -4-yl] -5-[(1R)-2-oxa-5-azabicyclo[2.2.1]heptan-5-yl]pyrazolo[1,5-a]pyrimidine-3-carboxamide (43.0 mg, 89.7 umol, Intermediate AMT) and tetraethoxytitanium (61.4 mg, 269 umol) was added. The reaction mixture was stirred at 80 °C for 11.5 hours. Then the reaction mixture was cooled to 25 °C, and NaBH₃CN (16.9 mg, 269 umol) was addded and stirred at 25 °C for 2 hours. On completion, the reaction was quenched with water (2 mL) and filtered. The filtrate was concentrated *in vacuo* to give the residue. The residue was purified by Prep-HPLC (column: Phenomenex Synergi C18 150*25*10um; mobile phase: [water (0.225%FA)-ACN]; B%: 4%-34%) to give the title compound (8.28 mg, 9% yield) as a white slid. LC-MS (ESI⁺) *m*/*z* 861.5 (M+H) ⁺. ¹H NMR (400 MHz, DMSO-*d₆*) δ 11.09 (s, 1H), 9.60 (d, *J =* 4.4 Hz, 1H), 8.96 (d, *J* = 3.6 Hz, 1H), 8.80 (d, *J* = 7.6 Hz, 1H), 8.30 (d, *J* = 5.2 Hz, 1H), 7.77 (d, *J* = 8.4 Hz, 2H), 7.42 (d, *J =* 8.4 Hz, 2H), 7.30 - 6.45 (m, 5H), 5.36 (d, *J* = 18.0 Hz, 1H), 5.32 - 5.07 (m, 1H), 4.78 (d, *J =* 13.6 Hz, 1H), 3.83 (s, 2H), 3.80 - 3.73 (m, 3H), 3.67 - 3.62 (m, 5H), 2.93 (s, 4H), 2.90 - 2.83 (m, 1H), 2.77 - 2.58 (m, 3H), 2.28 (s, 3H), 2.08 - 1.92 (m, 4H), 1.67 (s, 4H).

### Example 43. Synthesis of N-[3-(difluoromethyl)-1-[4-[methyl-[3-[3-[[2-(1-methyl-2,6-dioxo-3-piperidyl)-1,3-dioxo -isoindolin-4-yl]amino]propoxy]propylsulfonyl]carbamoyl]cyclohexyl]pyrazol-4-yl]-5-(2-oxa-5-azabicyclo[2.2.1]heptan-5-yl)pyrazolo[1,5-a]pyrimidine-3-carboxamide (I-490)

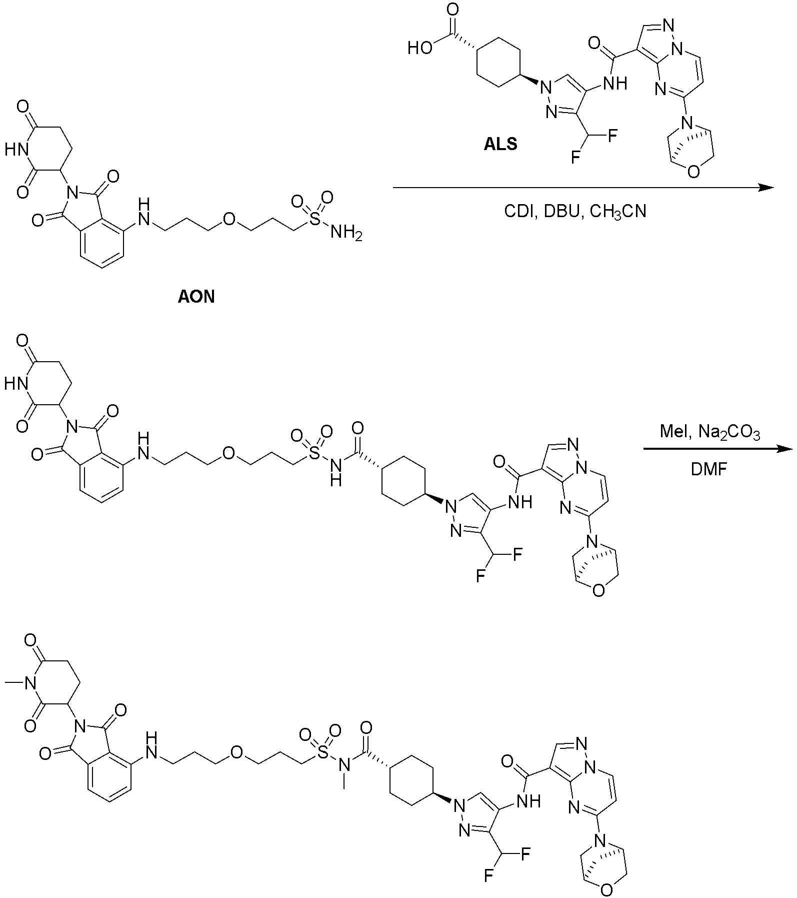

### Step 1 - N-[3-(difluoromethyl)-1-[4-[3-[3-[[2-(2,6-dioxo-3-piperidyl)-1,3-dioxo-isoindolin-4-yl] amino]propoxy]propylsulfonylcarbamoyl]cyclohexyl]pyrazol-4-yl]-5-(2-oxa-5-azabicyclo[2.2.1]heptan-5-yl)pyrazolo[1 ,5-a]pyrimidine-3-carboxamide

To a mixture of 4-[3-(difluoromethyl)-4-[[5-(2-oxa-5-azabicyclo[2.2.1]heptan-5-yl)pyrazolo [1,5-a] pyrimidine-3-carbonyl]amino]pyrazol-1-yl]cyclohexanecarboxylic acid (44.3 mg, 88.4 umol, Intermediate ALS) in CH₃CN (10 mL) was added CDI (43.0 mg, 265 umol) at 25 °C. The mixture was stirred at 25 °C for 3 hours. Then, a solution of 3-[3-[[2-(2,6-dioxo-3-piperidyl)-1,3-dioxo- isoindolin-4-yl]amino]propoxy]propane-1-sulfonamide (40 mg, 88.4 umol, Intermediate AON) in CH₃CN (2 mL) and DBU (26.9 mg, 176 umol) were added to the mixture. The reaction mixture was stirred at 25 °C for 2 hours. On completion, the reaction mixture was quenched with water (0.5 mL) at 25 °C, and then concentrated *in vacuo* to give a residue. The residue was purified by reverse phase (FA conditions) to afford the title compound (15 mg, 18% yield) as a yellow solid. LC-MS (ESI⁺) m/z 936.2 (M+H)⁺.

### Step 2 - N-[3-(difluoromethyl)-1-[4-[methyl-[3-[3-[[2-(1-methyl-2,6-dioxo-3-piperidyl)-1,3-dioxo -isoindolin-4-yl]amino]propoxy]propylsulfonyl]carbamoyl]cyclohexyl]pyrazol-4-yl]-5-(2-oxa-5-azabicyclo[2.2.1]heptan-5-yl)pyrazolo[1,5-a]pyrimidine-3-carboxamide

To a mixture of N-[3-(difluoromethyl)-1-[4-[3-[3-[[2-(2,6-dioxo-3-piperidyl)-1,3-dioxo-isoindolin-4- yl]amino]propoxy]propylsulfonylcarbamoyl]cyclohexyl]pyrazol-4-yl]-5-(2-oxa-5-azabicyclo[2.2.1]heptan-5-yl)pyrazolo[1,5-a]pyrimidine-3-carboxamide (15.0 mg, 16.0 umol) in DMF (1.0 mL) was added Na₂CO₃ (3.40 mg, 32.0 umol) at 25 °C. Then MeI (22.7 mg, 160 umol) was added to the reaction mixture. The mixture was stirred at 25 °C for 2 hours. On completion, the reaction mixture was quenched with water (0.2 mL) at 25 °C, and then concentrated *in vacuo* to give a residue. The residue was purified by prep-HPLC (column: Phenomenex Synergi C18 150*25*10 um; mobile phase: [water (0.225%FA)-ACN]; B%: 46%-76%, 10 min) to afford the title compound (3.0 mg, 19% yield) as a yellow solid. ¹H NMR (400 MHz, DMSO-*d*₆) δ 9.50 (d, *J =* 6.6 Hz, 1H), 8.77 (d, *J =* 7.8 Hz, 1H), 8.39 (d, *J =* 5.0 Hz, 1H), 8.25 (d, *J* = 5.6 Hz, 1H), 7.62 - 7.54 (m, 1H), 7.25 - 6.93 (m, 3H), 6.88 - 6.42 (m, 2H), 5.29 - 5.04 (m, 2H), 4.76 (d, *J =* 18.0 Hz, 1H), 4.33 - 4.20 (m, 1H), 3.85 - 3.71 (m, 2H), 3.63 - 3.58 (m, 3H), 3.51 - 3.45 (m, 5H), 3.24 (s, 3H), 3.01 (s, 3H), 2.98 - 2.95 (m, 1H), 2.94 - 2.91 (m, 1H), 2.89 (d, *J* = 4.2 Hz, 1H), 2.79 - 2.74 (m, 1H), 2.74 - 2.69 (m, 1H), 2.61 - 2.55 (m, 1H), 2.09 - 1.99 (m, 4H), 1.99 - 1.91 (m, 5H), 1.88 - 1.78 (m, 4H), 1.60 - 1.49 (m, 2H). LC-MS (ESI⁺) m/z 964.5 (M+H)⁺.

### Example 44. Synthesis of 5-((1R,4R)-2-oxa-5-azabicyclo[2.2.1]heptan-5-yl)-N-(3-(difluoromethyl)-1-((1r,4R)-4-(((5- (1-(2,6-dioxopiperidin-3-yl)-3-methyl-2-oxo-2,3-dihydro-1H-benzo[d]imidazol-4-yl)pent-4-yn-1-yl)(methyl)amino)methyl)cyclohexyl)-1H-pyrazol-4-yl)pyrazolo[1,5-a]pyrimidine-3-carboxamide (I-550)

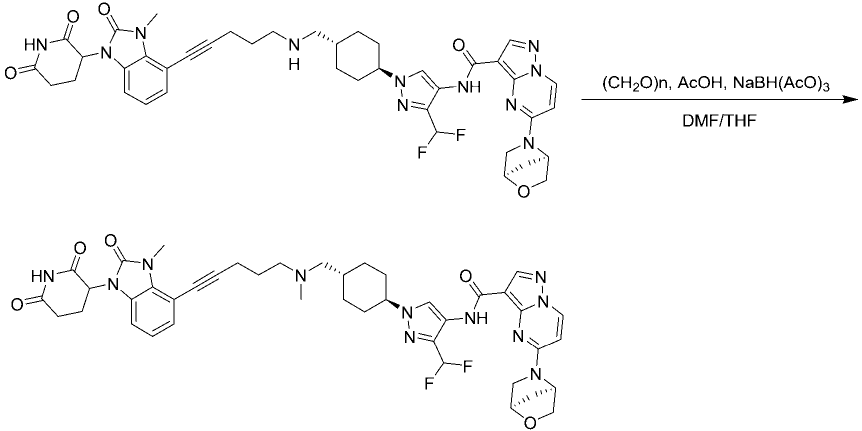

To a solution of N-[3-(difluoromethyl)-1-[4-[[5-[1-(2,6-dioxo-3-piperidyl)-3-methyl-2-oxo-benz imidazol-4-yl]pent-4-ynylamino]methyl]cyclohexyl]pyrazol-4-yl]-5-[(1R)-2-oxa-5-azabicyclo[2.2.1]heptan-5-yl]pyrazolo[1,5-a]pyrimidine-3-carboxamide (0.18 g, 216 umol, **I-518)** in DMF (2 mL) and THF (8 mL) was added HOAc (13.0 mg, 216 umol) and HCHO (130 mg, 4.31 mmol, 119 uL) under N₂. The mixture was stirred at 0 °C for 0.5 hr, then NaBH(OAc)₃ (1.14 g, 5.39 mmol) was added to the reaction mixture. The mixture was stirred at 0 °C for 1.5 hrs. On completion, the reaction mixture was quenched by addition H₂O (0.2 mL) and concentrated *in vacuo* to give a residue. The residue was purified by prep-HPLC (column: Waters Xbridge 150*25 mm*5 um; mobile phase: [water (10mM NH₄HCO₃)-ACN]; B%: 36%-66%, 10 min) to give the title compound (81.9mg, 46% yield) as a white solid. ¹H NMR (400 MHz, DMSO-d₆) δ 11.10 (s, 1H), 9.49 (d, J = 6.0 Hz, 1H), 8.78 (d, J = 7.6 Hz, 1H), 8.43 - 8.19 (m, 2H), 7.27 - 6.40 (m, 5H), 5.38 (dd, J = 5.6, 12.8 Hz, 1H), 5.30 - 4.94 (m, 1H), 4.79 (s, 1H), 4.16 (s, 1H), 3.86 - 3.38 (m, 8H), 2.92 - 2.80 (m, 1H), 2.70 - 2.65 (m, 1H), 2.41 (t, J = 6.4 Hz, 2H), 2.29 - 1.46 (m, 19H), 1.17 - 0.91 (m, 2H); LC-MS (ESI⁺) m/z 824.5 (M+H)⁺.

### Example 45. Synthesis of 5-((1R,4R)-2-oxa-5-azabicyclo[2.2.1]heptan-5-yl)-N-(3-(difluoromethyl)-1-((1r,4R)-4-(((5- (1-(2,6-dioxopiperidin-3-yl)-3-methyl-2-oxo-2,3-dihydro-1H-benzo[d]imidazol-5-yl)pent-4-yn-1-yl)(methyl)amino)methyl)cyclohexyl)-1H-pyrazol-4-yl)pyrazolo[1,5-a]pyrimidine-3-carboxamide (I-546)

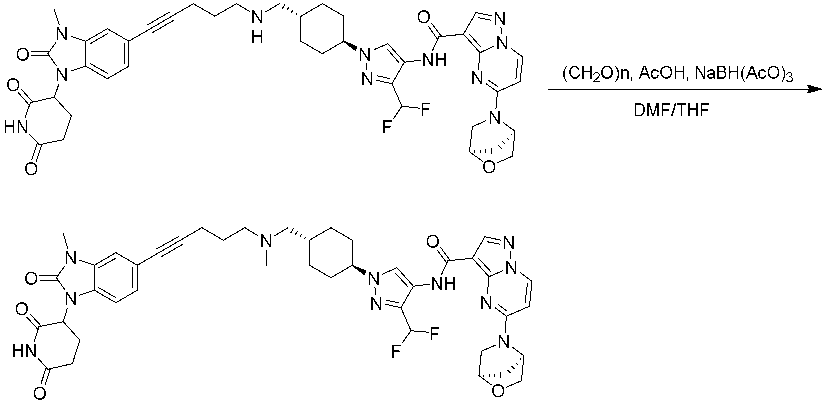

A solution of N-[3-(difluoromethyl)-1-[4-[[5-[1-(2,6-dioxo-3-piperidyl)-3-methyl-2-oxo-benzimidazol-5-yl]pent-4-ynylamino]methyl]cyclohexyl]pyrazol-4-yl]-5-(2-oxa-5-azabicyclo[2.2.1]heptan-5-yl)pyrazolo[1,5-a]pyrimidine-3-carboxamide (130 mg, 160 umol, **I-512),** Formaldehyde (24.1 mg, 802 umol) in a mixture solvent of THF (9.0 mL) and DMF (3 mL) was added TEA (16. 2 mg, 160 umol). The mixture was stirred at 20 °C for 0.5 hr. Then HOAc (19. 2 mg, 321 umol) was added into the above mixture. The mixture was stirred at 40 °C for 0.5 hr. Next, NaBH(OAc)₃ (170 mg, 802 umol) was added and the resulting reaction was stirred at 25 °C for 12 hrs. On completion, the reaction mixture was quenched with water (0.1 mL) and concentrated *in vacuo.* The residue was purified by prep-HPLC (column: Waters Xbridge 150*25 mm* 5 um; mobile phase: [water (10 mM NH₄HCO₃)-ACN]; B%:34%-64%, 10 min) to give the title compound (22.0 mg, 16% yield) as off-white solid. ¹H NMR (400 MHz, DMSO-*d₆*) δ 11.11 (s, 1H), 9.50 (d, *J* = 6.0 Hz, 1H), 8.79 (d, *J =* 7.6 Hz, 1H), 8.37 (d, *J* = 4.0 Hz, 1H), 8.27 (d, *J* = 5.6 Hz, 1H), 7.10 (s, 2H), 7.27 - 6.93 (m, 2H), 6.91 - 6.40 (m, 1H), 5.37 (dd, *J* = 5.2, 12.8 Hz, 1H), 5.30 - 5.07 (m, 1H), 4.78 (d, *J* = 18.0 Hz, 1H), 4.24 - 4.13 (m, 1H), 3.87 - 3.72 (m, 2H), 3.66 - 3.44 (m, 2H), 3.33 (s, 3H), 2.94 - 2.82 (m, 1H), 2.68 (s, 2H), 2.46 - 2.38 (m, 4H), 2.21 - 2.11 (m, 5H), 2.08 - 1.91 (m, 7H), 1.79 - 1.65 (m, 4H), 1.62 - 1.51 (m, 1H), 1.11 - 0.99 (m, 2H); LC-MS (ESI⁺) m/z 824.4 (M+H)⁺.

### Example 46. Synthesis of N-[3-(difluoromethyl)-1-[2-[[4-[3-[[2-(2,6-dioxo-3-piperidyl)-1,3-dioxo-isoindolin-4-yl] amino]propoxy]-1-piperidyl]methyl]-1,3-dioxan-5-yl]pyrazol-4-yl]-5-morpholino-pyrazolo[1,5-a]pyrimidine-3-carboxamide (I-451)

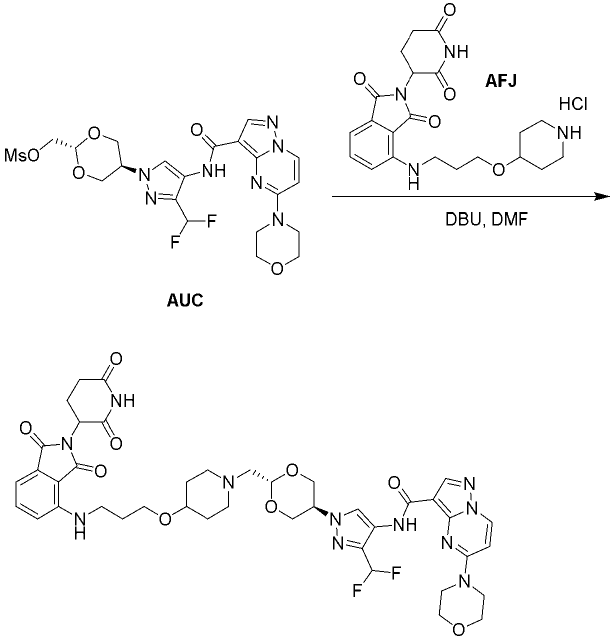

To a mixture of [5-[3-(difluoromethyl)-4-[(5-morpholinopyrazolo[1,5-a]pyrimidine-3-carbonyl)amino] pyrazol-1-yl]-1,3-dioxan-2-yl]methylmethanesulfonate (35.0 mg, 62.7 umol, Intermediate AUC) and 2-(2,6-dioxo-3-piperidyl) -4-[3-(4-piperidyloxy)propylamino]isoindoline-1,3-dione (31.2 mg, 75.3 umol, Intermediate AFJ) in DMF (5 mL) was added DBU (19.1 mg, 125 umol, 18.9 uL) at 25 °C. The mixture was stirred at 100 °C for 20 hours. On completion, the reaction mixture was quenched by water (0.5 mL) at 25 °C, and then concentrated *in vacuo* to give a residue. The residue was purified by prep-HPLC (column: Waters Xbridge 150*25mm* 5um; mobile phase: [water (10mM NH₄HCO₃)-ACN]; B%: 27%-57%, 10 min) to afford the title compound (0.75 mg, 1.4% yield) as a yellow solid. ¹H NMR (400 MHz, ACN-*d*₃) δ 9.46 (s, 1H), 8.46 - 8.41 (m, 2H), 8.29 (s, 1H), 7.60 - 7.51 (m, 1H), 7.06 - 7.01 (m, 2H), 6.99 - 6.71 (m, 1H), 6.63 (d, *J =* 8.0 Hz, 1H), 6.55 (t, J = 4.8 Hz, 1H), 4.96 - 4.90 (m, 1H), 4.87 (s, 1H), 4.58 - 4.49 (m, 1H), 4.34 - 4.28 (m, 2H), 4.08 - 3.99 (m, 2H), 3.77 (s, 8H), 3.56 (t, *J =* 5.8 Hz, 2H), 3.45 - 3.35 (m, 3H), 3.03 - 2.88 (m, 2H), 2.79 - 2.49 (m, 6H), 1.90 - 1.86 (m, 4H), 1.79 - 1.75 (m, 1H), 1.72 - 1.62 (m, 3H), 1.24 (t, *J =* 7.4 Hz, 1H); LC-MS (ESI⁺) *m*/*z* 876.3 (M+H)⁺.

### Example 47. Synthesis of N-(3-(difluoromethyl)-1-((1r,4r)-4-((((6-(3-((2-(2,6-dioxopiperidin-3-yl)-1,3 -dioxoisoindolin-4-yl)amino)propoxy)pyridin-3-yl)methyl)(methyl)amino)methyl)cyclohexyl)-1H-pyrazol-4-yl)-5-morpholinopyrazolo[1,5-a]pyrimidine-3-carboxamide (I-477)

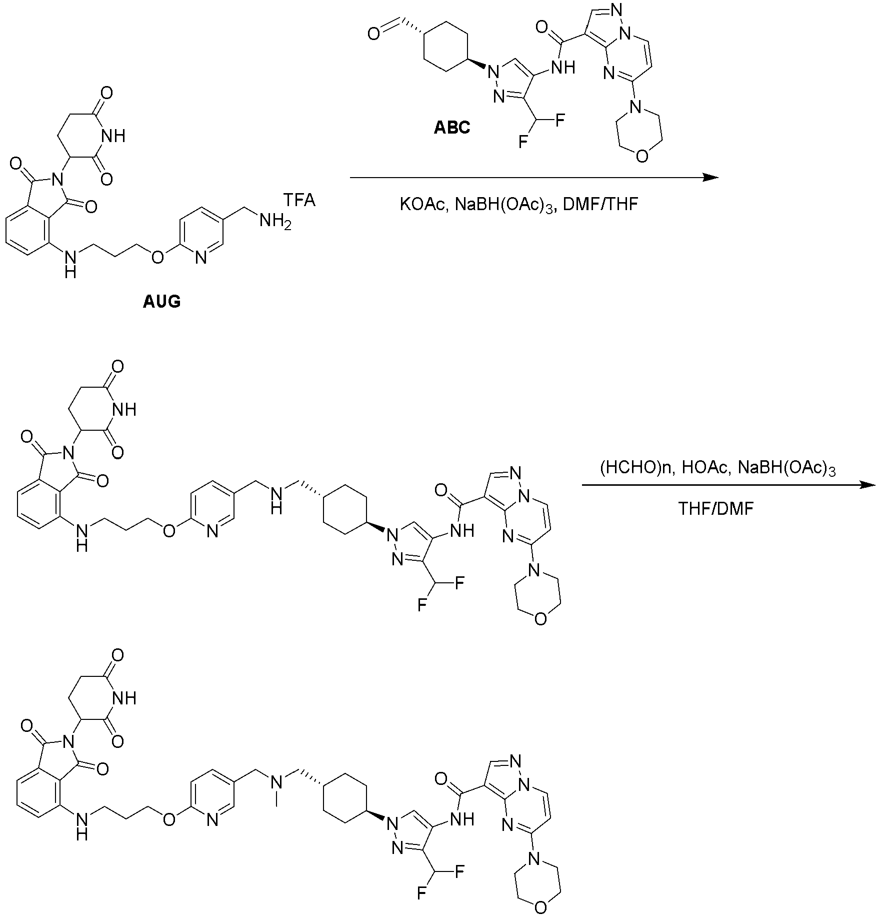

### Step 1 - N-(3-(difluoromethyl)-1-((1r,4r)-4-((((6-(3-((2-(2,6-dioxopiperidin-3-yl)-1,3-dioxoisoindolin -4-yl)amino)propoxy)pyridin-3-yl)methyl)amino)methyl)cyclohexyl)-1H-pyrazol-4-yl)-5-morpholinopyrazolo[1,5-a]pyrimidine-3-carboxamide

To a solution of 4-[3-[[5-(aminomethyl)-2-pyridyl]oxy]propylamino]-2-(2,6-dioxo-3-piperidyl) isoindoline-1,3-dione (50.0 mg, 77.9 umol, Intermediate AUG) in DMF (1 mL) and THF (4 mL) was added KOAc (11.4 mg, 116 umol). N-[3-(difluoromethyl)-1-(4-formylcyclohexyl)pyrazol-4-yl]-5-morpholino-pyrazolo[1,5-a]pyrimidine-3-carboxamide (36.9 mg, 77.9 umol, Intermediate ABC) was added at -10 °C. The mixture was stirred at -10 °C for 0.5 hour. Next, NaBH(OAc)₃ (49.5 mg, 233 umol) was added and the mixture was stirred at 0 °C for 2 hours. The reaction was quenched with water (0.1 mL) and the mixture was concentrated *in vacuo.* The residue was purified by Prep-HPLC(Neu: column: Waters Xbridge 150 * 25 mm * 5 um; mobile phase: [water(10 mM NH₄HCO₃) - ACN]; B%: 30% - 60%, 10 min) to give the title compound (40.0 mg, 56% yield) as a yellow solid. ¹H NMR (400MHz, DMSO-d₆) δ 9.39 (s, 1H), 8.78 (d, *J* = 7.6 Hz, 1H), 8.35 (s, 1H), 8.28 (s, 1H) 8.08 - 8.01 (m, 1H), 7.70 - 7.51 (m, 2H), 7.24 - 6.69 (m, 7H), 5.04 (d, *J* = 7.2 Hz, 1H), 4.33 (s, 2H), 3.77 (d, *J =* 4.8 Hz, 7H), 3.71 (s, 8H), 2.07 - 1.97 (m, 5H), 1.95 - 1.84 (m, 3H), 1.76 - 1.64 (m, 3H), 1.49 - 1.35 (m, 2H), 1.10 - 0.99 (m, 2H); LC-MS (ESI⁺) *m*/*z* 894.9 (M+H)⁺.

### Step 2 - N-(3-(difluoromethyl)-1-((1r,4r)-4-((((6-(3-((2-(2,6-dioxopiperidin-3-yl)-1,3 -dioxoisoindolin-4-yl)amino)propoxy)pyridin-3-yl)methyl)(methyl)amino)methyl)cyclohexyl)-1H-pyrazol-4-yl)-5-morpholinopyrazolo[1,5-a]pyrimidine-3-carboxamide

To a solution of N-[3-(difluoromethyl)-1-[4-[[[6-[3-[[2-(2,6-dioxo-3-piperidyl)-1,3-dioxo-isoindolin -4-yl]amino]propoxy]-3-pyridyl]methylamino]methyl]cyclohexyl]pyrazol-4-yl]-5-morpholino-pyrazolo[1,5-a]pyrimidine-3-carboxamide (30.0 mg, 33.5 umol) in DMF (0.5 mL) and THF (2 mL) was added AcOH (402 ug, 6.70 umol). Paraformaldehyde (10.0 mg, 335 umol) was added and the mixture was stirred at 0 °C for 0.2 hour. Next, NaBH(OAc)₃ (85.2 mg, 402 umol) was added and the mixture was stirred at 0 °C for 2 hours. On completion, the mixture was filtered and the filtrate was concentrated *in vacuo.* The residue was purified by prep-HPLC (Neu: column: Waters Xbridge 150 * 25 mm * 5 um; mobile phase: [water(10 mM NH₄HCO₃) - ACN]; B%: 55% - 75%, 10 min) to give the title compound (7.00 mg, 23% yield) as a yellow solid. ¹H NMR (400MHz, DMSO-d₆) δ 9.39 (s, 1H), 8.82 (d, *J* = 8.0 Hz, 1H), 8.36 (s, 1H), 8.28 (s, 1H), 8.02 (s, 1H), 7.61 - 7.54 (m, 2H), 7.13 - 6.82 (m, 6H), 5.07 (m, 1H), 4.36 - 4.33 (m, 2H), 4.16 (m, 1H), 3.79 - 3.71 (m, 8H), 3.48 - 3.38 (m, 5H), 2.60 (m, 1H), 2.57 - 2.55 (m, 2H), 2.13 - 2.11 (m, 5H), 2.04 - 2.01 (m, 5H), 1.93 - 1.90 (m, 2H), 1.73 (m, 2H), 1.60 (m, 1H), 1.01 - 0.92 (m, 2H); LC-MS (ESI⁺) *m*/*z* 908.9 (M+H)⁺.

### Examples 48. Synthesis of N-[3-(difluoromethyl)-1-[4-[[4-[3-[1-[(3S)-2,6-dioxo-3-piperidyl]-3-methyl-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (I-560) and N-[3- (difluoromethyl)-1-[4-[[4-[3-[1-[(3R)-2,6-dioxo-3-piperidyl]-3-methyl-2-oxo-benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (I-561)

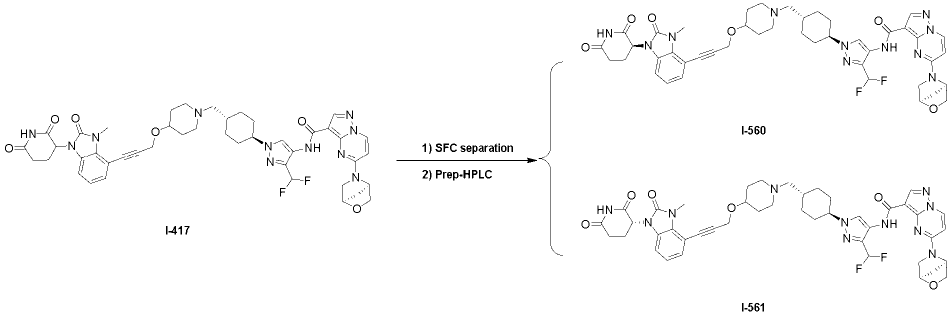

The compound N-[3-(difluoromethyl)-1-[4-[[4-[3-[1-(2,6-dioxo-3-piperidyl)-3-methyl-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (1.00 g, 1.15 mmol, **I-417)** was carried out on a super-fluid chromatography unit using Sample preparation (Add ACN+DCM+THF 80 mL into sample Instrument: Waters 80 SFC Mobile Phase: 80% IPA+ACN (0.1%NH₃•H₂O) in Supercritical CO₂ Flow Rate: 80 g/min Cycle Time: 3.25 min, total time: 422 min Single injetion volume: 0.8 mL Back Pressure: 100 bar to keep the CO₂ in Supercritical flow). This afforded two enantiomers: N-[3-(difluoromethyl)-1-[4-[[4-[3-[1-[(3S)-2,6-dioxo-3-piperidyl]-3-methyl-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (612 mg, 57% yield, 98% ee, FA salt, retention time of 4.96 min) as white solid and N-[3- (difluoromethyl)-1-[4-[[4-[3-[1-[(3R)-2,6-dioxo-3-piperidyl]-3-methyl-2-oxo-benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (552 mg, 51% yield, 95% ee, FA salt, retention time of 6.44 min) as white solid. The absolute configuration of the stereoisomers was randomly assigned. Characterization of N-[3-(difluoromethyl)-1-[4-[[4-[3-[1-[(3S)-2,6-dioxo-3-piperidyl]-3-methyl-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide: ¹H NMR (400 MHz, DMSO-*d₆*) δ 11.12 (s, 1H), 9.49 (d, *J =* 6.0 Hz, 1H), 8.77 (d, *J* = 7.6 Hz, 1H), 8.38 (d, *J* = 4.4 Hz, 1H), 8.25 (d, *J* = 5.6 Hz, 1H), 7.27 - 6.95 (m, 4H), 6.89 - 6.41 (m, 1H), 5.40 (dd, *J* = 5.2, 12.8 Hz, 1H), 5.30 - 5.04 (m, 1H), 4.76 (d, *J* = 16.0 Hz, 1H), 4.47 (s, 2H), 4.21 - 4.12 (m, 1H), 3.84 - 3.78 (m, 1H), 3.73 (d, *J =* 7.6 Hz, 1H), 3.64 (s, 3H), 3.62 - 3.58 (m, 2H), 3.58 - 3.41 (m, 1H), 2.94 - 2.83 (m, 1H), 2.78 - 2.67 (m, 3H), 2.65 - 2.59 (m, 1H), 2.15 - 1.85 (m, 13H), 1.80 - 1.67 (m, 2H), 1.63 - 1.44 (m, 3H), 1.09 - 0.95 (m, 2H); LC-MS (ESI+) m/z 866.5 (M+H)⁺. Characterization of N-[3- (difluoromethyl)-1-[4-[[4-[3-[1-[(3R)-2,6-dioxo-3-piperidyl]-3-methyl-2-oxo-benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide: ¹H NMR (400 MHz, DMSO-*d₆*) δ 11.12 (s, 1H), 9.50 (d, *J =* 6.0 Hz, 1H), 8.78 (d, *J =* 7.6 Hz, 1H), 8.38 (d, *J* = 4.4 Hz, 1H), 8.25 (d, *J* = 5.6 Hz, 1H), 7.26 - 6.95 (m, 4H), 6.88 - 6.42 (m, 1H), 5.40 (dd, *J* = 5.2, 12.8 Hz, 1H), 5.31 - 5.01 (m, 1H), 4.76 (d, *J* = 16.4 Hz, 1H), 4.47 (s, 2H), 4.21 - 4.11 (m, 1H), 3.84 - 3.78 (m, 1H), 3.74 (d, *J =* 7.6 Hz, 1H), 3.64 (s, 3H), 3.62 - 3.58 (m, 2H), 3.58 - 3.41 (m, 1H), 2.94 - 2.84 (m, 1H), 2.78 - 2.67 (m, 3H), 2.66 - 2.59 (m, 1H), 2.16 - 1.85 (m, 13H), 1.80 - 1.66 (m, 2H), 1.61 - 1.43 (m, 3H), 1.05 - 1.02 (m, 2H); LC-MS (ESI+) m/z 866.5 (M+H)⁺.

### Example 49. Synthesis of N-[3-(difluoromethyl)-1-[4-[[4-[[[2-(2,6-dioxo-3-piperidyl)-1,3-dioxo-isoindolin-4-yl]amino] methyl]-1-piperidyl]methyl]phenyl]pyrazol-4-yl]-5-[(1R)-2-oxa-5-azabicyclo[2.2.1]heptan-5-yl]pyrazolo[1,5-a]pyrimidine-3-carboxamide (I-439)

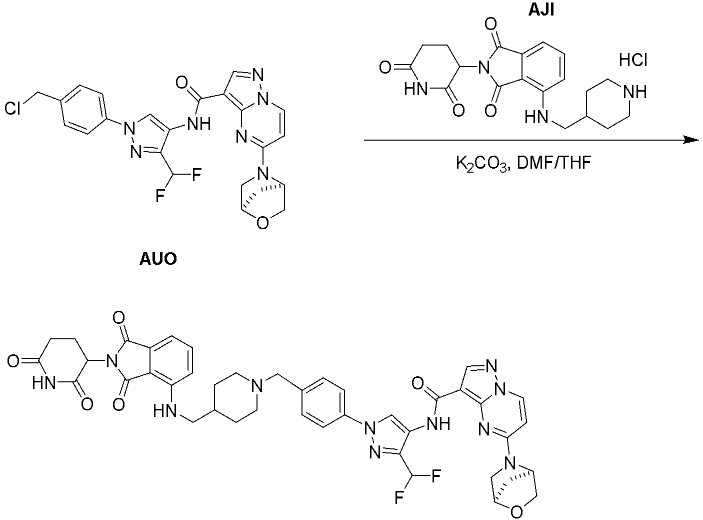

To a mixture of N-[1-[4-(chloromethyl)phenyl]-3-(difluoromethyl)pyrazol-4-yl]-5-[(1R)-2-oxa-5 -azabicyclo[2.2.1]heptan-5-yl]pyrazolo[1,5-a]pyrimidine-3-carboxamide (200 mg, 400 umol, Intermediate AUO) and 2-(2,6-dioxo-3-piperidyl)-4-(4-piperidylmethylamino)isoindoline-1,3-dione (162 mg, 400 umol, HCl salt, Intermediate AJI) in THF (5 mL) and DMF (1 mL) was added K₂CO₃ (165 mg, 1.20 mmol). The reaction mixture was stirred at 60 °C for 12 hours. On completion, the reaction mixture was filtered and concentrated *in vacuo.* The residue was purified by prep-HPLC (column: Phenomenex Synergi C18 150*25*10um;mobile phase: [water(0.225%FA)-ACN];B%: 19%-49%,10min) to give the title compound (35.1 mg, 9% yield) as yellow solid. ¹H NMR (400 MHz, DMSO-*d₆*) δ 11.09 (s, 1H), 9.61 (d, *J* = 4.4 Hz, 1H), 8.96 (d, *J =* 2.8 Hz, 1H), 8.80 (d, *J =* 7.6 Hz, 1H), 8.34 - 8.27 (m, 1H), 7.78 (d, *J* = 8.4 Hz, 2H), 7.61 - 7.50 (m, 1H), 7.44 (d, *J* = 8.4 Hz, 2H), 7.30 - 7.00 (m, 3H), 6.93 - 6.43 (m, 2H), 5.32 - 5.01 (m, 2H), 4.78 (d, *J =* 13.6 Hz, 1H), 3.83 (s, 1H), 3.76 - 3.61 (m, 2H), 3.53 - 3.45 (m, 4H), 3.25 - 3.18 (m, 2H), 2.94 - 2.79 (m, 3H), 2.63 - 2.53 (m, 2H), 2.08 - 1.86 (m, 4H), 1.73 - 1.62 (m, 2H), 1.62 - 1.52 (m, 1H), 1.33 - 1.18 (m, 2H); LC-MS (ESI⁺) *m*/*z* 834.4 (M+H)⁺.

### Example 50. Synthesis of N-[3-(difluoromethyl)-1-[4-[[[4-[3-(difluoromethyl)-4-[(5-morpholinopyrazolo[1,5-a] pyrimidine-3-carbonyl)amino]pyrazol-1-yl]cyclohexyl]methylamino]methyl]cyclohexyl]pyrazol-4-yl]-5-morpholino-pyrazolo[1,5-a]pyrimidine-3-carboxamide (I-408)

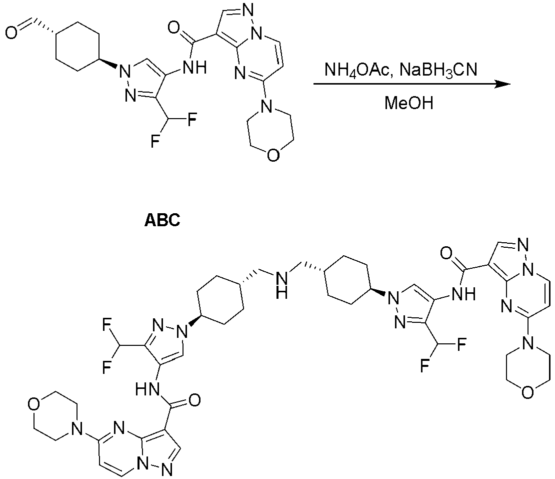

To a mixture of N-[3-(difluoromethyl)-1-(4-formylcyclohexyl)pyrazol-4-yl]-5-morpholino-pyrazolo [1,5-a]pyrimidine-3-carboxamide (0.50 g, 1.06 mmol, Intermediate ABC) in MeOH (100 mL) was added NH₄OAc (814 mg, 10.56 mmol). The mixture was stirred at 20 °C for 1 hour. Then NaBH₃CN (99.5 mg, 1.58 mmol) was added into the mixture. The mixture was stirred at 20 °C for 12 hours. On completion, the reaction mixture was quenched by water (5 mL) and filtered to give the filtrate. The filtrate was concentrated *in vacuo* to give the residue. The residue was purified by prep-HPLC (column: Shim-pack C18 150*25*10 um; mobile phase: [water (0.225%FA)-ACN]; B%: 22%-52%) to give the title compound (87.0 mg, 8% yield) as an off-white solid. ¹H NMR (400 MHz, DMSO-*d₆*) δ 9.40 (s, 2H), 8.82 (d, *J =* 8.0 Hz, 2H), 8.46 - 8.37 (m, 2H), 8.31 - 8.22 (m, 2H), 7.28 - 6.95 (m, 2H), 6.90 (d, *J =* 8.0 Hz, 2H), 4.26 - 4.15 (m, 2H), 3.79 (s, 8H), 3.74 - 3.70 (m, 8H), 2.67 (d, *J =* 2.8 Hz, 1H), 2.62 (d, *J =* 6.4 Hz, 3H), 2.11 - 2.03 (m, 4H), 1.93 (d, *J* = 12.0 Hz, 4H), 1.80 - 1.69 (m, 4H), 1.63 (s, 3H), 1.25 - 1.03 (m, 4H); LC-MS (ESI⁺) *m*/*z* 931.9 (M+H)⁺.

### Example 51. Synthesis of N-[1-[4-[[5-aminopent-3-ynyl(methyl)amino]methyl]cyclohexyl]-3-(difluoromethyl)pyrazol -4-yl]-5-morpholino-pyrazolo[1,5-a]pyrimidine-3-carboxamide (I-412)

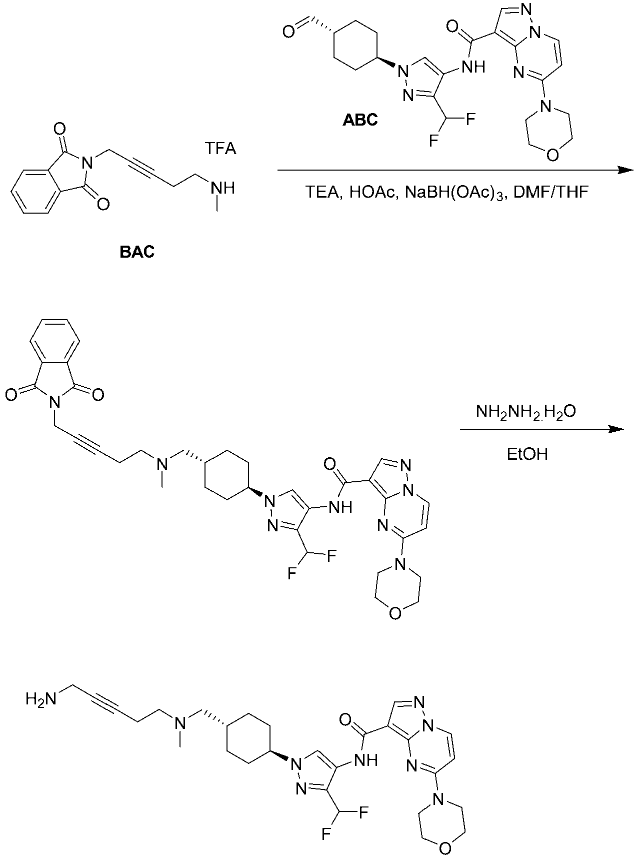

### Step 1 - N-[3-(difluoromethyl)-1-[4-[[5-(1,3-dioxoisoindolin-2-yl)pent-3-ynyl-methyl-amino] methyl] cyclohexyl]pyrazol-4-yl]-5-morpholino-pyrazolo[1,5-a]pyrimidine-3-carboxamide

To a mixture of 2-[5-(methylamino)pent-2-ynyl]isoindoline-1,3-dione (90.0 mg, 252 umol, TFA salt, Intermediate BAC) in a mixed solvent of THF (2 mL) and DMF (0.5 mL) was added TEA (51.1 mg, 505 umol) at 0 °C. The mixture was stirred at 0 °C for 30 minutes. Then N-[3-(difluoromethyl)-1-(4-formylcyclohexyl) pyrazol-4 -yl]-5-morpholino-pyrazolo[1,5-a]pyrimidine-3-carboxamide (119 mg, 252 umol, Intermediate ABC) and HOAc (30.3 mg, 505 umol) was added. The mixture was at 0 °C for 30 minutes. After that, NaBH(OAc)₃ (107 mg, 505 umol) was added. The mixture was stirred at 0 °C for 2 hours. On completion, the reaction mixture was quenched with water (0.2 mL) at 0 °C and concentrated *in vacuo.* The residue was purified by prep-HPLC (column: Phenomenex Gemini-NX C18 75*30mm*3um; mobile phase: [water (0.1%TFA)-ACN]; B%: 20%-50%, 7 min) to afford the title compound (55 mg, 31% yield) as a white solid. ¹H NMR (400 MHz, DMSO-*d*₆) δ 9.41 (s, 1H), 9.14 (d, *J* = 3.2 Hz, 1H), 8.84 (d, *J* = 8.0 Hz, 1H), 8.39 (s, 1H), 8.30 (s, 1H), 7.96 - 7.85 (m, 4H), 7.27 - 6.95 (m, 1H), 6.92 (d, *J* = 8.0 Hz, 1H), 4.40 (s, 2H), 4.23 - 4.12 (m, 1H), 3.80 (s, 4H), 3.73 (d, *J* = 4.4 Hz, 4H), 3.34 - 3.16 (m, 2H), 3.06 (d, *J =* 4.0 Hz, 1H), 2.99 - 2.88 (m, 1H), 2.81 (d, *J =* 4.0 Hz, 3H), 2.72 (d, *J =* 7.2 Hz, 2H), 2.07 - 1.72 (m, 7H), 1.22 - 1.05 (m, 2H). LC-MS (ESI⁺) m/z 700.4 (M+H)⁺.

### Step 2 - N-[1-[4-[[5-aminopent-3-ynyl(methyl)amino]methyl]cyclohexyl]-3-(difluoromethyl)pyrazol -4-yl]-5-morpholino-pyrazolo[1,5-a]pyrimidine-3-carboxamide

To a mixture of N-[3-(difluoromethyl)-1-[4-[[5-(1,3-dioxoisoindolin-2-yl)pent-3-ynyl-methyl-amino] methyl]cyclohexyl]pyrazol-4-yl]-5-morpholino-pyrazolo[1,5-a]pyrimidine-3-carboxamide (40.0 mg, 57.2 umol) in EtOH (2.0 mL) was added NH₂NH₂·H₂O (14.3 mg, 285 umol) at 25 °C. The mixture was stirred at 80 °C for 2 hours. On completion, the mixture was filtered and the filtrate was concentrated *in vacuo.* The residue was purified by prep-HPLC (column: Phenomenex Gemini-NX C18 75*30mm*3um; mobile phase: [water (0.1%TFA)-ACN]; B%: 8%-38%, 7 min) to afford the title compound (30.6 mg, 94% yield) as a yellow solid. ¹H NMR (400 MHz, DMSO-*d*₆) δ 9.41 (s, 1H), 8.83 (d, *J* = 8.0 Hz, 1H), 8.40 (s, 1H), 8.28 (s, 1H), 8.21 - 8.10 (m, 2H), 7.23 - 6.95 (m, 1H), 6.92 (d, *J =* 8.0 Hz, 1H), 4.28 - 4.18 (m, 1H), 3.79 (d, *J* = 3.2 Hz, 4H), 3.74 - 3.70 (m, 5H), 3.09 - 2.97 (m, 2H), 2.87 - 2.74 (m, 5H), 2.10 - 2.07 (m, 3H), 1.98 - 1.74 (m, 6H), 1.29 - 1.07 (m, 3H). LC-MS (ESI⁺) m/z 570.2 (M+H)⁺.

### Example 52. Synthesis of N-[3-(difluoromethyl)-1-[4-[[4-[3-[3-methyl-1-(1-methyl-2,6-dioxo-3-piperidyl)-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (I-563)

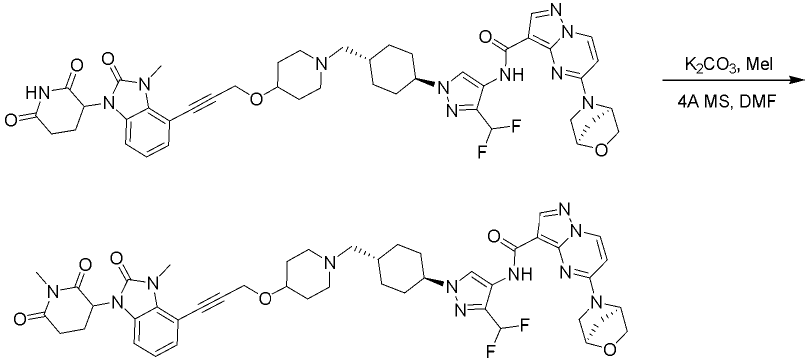

To a solution of N-[3-(difluoromethyl)-1-[4-[[4-[3-[1-(2,6-dioxo-3-piperidyl)-3-methyl-2-oxo- benzimidazol-4-yl]prop-2-ynoxy]-1-piperidyl]methyl]cyclohexyl]pyrazol-4-yl]-5-[(1R,4R)-2-oxa-5-azabicyclo[2.2.1]heptan-5-yl]pyrazolo[1,5-a]pyrimidine-3-carboxamide (300 mg, 346 umol, **I-417)** and 4Å molecular sieves (60 mg) in DMF (1 mL) was added K₂CO₃ (95.8 mg, 693 umol) at 0 °C. Thirty minutes later, MeI (54.1 mg, 381 umol) was added and the reaction mixture was stirred at 20 °C for 1 hr. On completion, the reaction mixture was filtered and the filtrate was concentrated *in vacuo.* The residue was purified by prep-HPLC (column: Waters Xbridge C18 150*50mm* 10um; mobile phase: [water (10mM NH₄HCO₃)-ACN]; B%: 32%-62%, 10 min) to give the title compound (29.0 mg, 9.5% yield) as a white solid. ¹H NMR (400 MHz, DMSO-*d₆*) 9.50 (d, *J* = 6.0 Hz, 1H), 8.78 (d, *J* = 7.6 Hz, 1H), 8.38 (d, *J* = 4.4 Hz, 1H), 8.26 (d, *J* = 5.6 Hz, 1H), 7.26 - 6.96 (m, 4H), 6.90 - 6.43 (m, 1H), 5.47 (dd, *J* = 5.2, 12.8 Hz, 1H), 5.31 - 5.04 (m, 1H), 4.77 (d, *J* = 17.2 Hz, 1H), 4.48 (s, 2H), 4.25 - 4.09 (m, 1H), 3.88 - 3.72 (m, 2H), 3.65 (s, 3H), 3.63 - 3.42 (m, 3H), 3.04 (s, 3H), 3.01 - 2.93 (m, 1H), 2.82 - 2.64 (m, 4H), 2.12 - 1.86 (m, 13H), 1.80 - 1.67 (m, 2H), 1.59 - 1.39 (m, 3H), 1.11 - 0.92 (m, 2H); LC-MS (ESI⁺) *m*/*z* 880.5 (M+H)⁺.

### Example 53. IMQ (imiquimod) induced psoriasis

A model of psoriasis includes the effects of degrader on epidermal hyperplasia development induced by daily topical application of 5% imiquimod in C57BL/6 mice. On each of Days 0 to 6, mice had 5% imiquimod cream administered topically on left ear and shaved back skin. Control mice (no imiquimod group) received control cream without imiquimod. Mice were dosed orally with degrader **I-417** 2 to 4 hours before the imiquimod application that day. The last dosing was on Day 6 for all mice. All dosing was at the same time (+/- 1 hour) each day. For the BID groups there was no less than 9 and no more than 15 hours between doses. Readouts were body weight, PASI score, and skin thickness. Body weight was measured 3 times/week, starting on Day -3. Once on each of Days 0, 3, 5, 6, and 7, back and ear thickness was measured with calipers. Each measurement was performed blind, by a person unaware of the group or previous measurement. On Day 7, approximately 24 hours after the last imiquimod application, all mice were euthanized and blood collected via cardiac bleed into EDTA tubes. Plasma was isolated, and frozen. Tissues of interest were removed, weighed, recorded and frozen.

FIG. 36 shows that **I-417** significantly reduced skin thickening in mice treated with topical imiquimod.

### Example 54. MSU Peritonitis

C57BL/6 mice were dosed with vehicle, positive control or **I-30** 30 minutes prior to intraperitoneal injection of 3 mg of monosodium urate crystals in 0.5-ml sterile PBS. After 4 hours, mice were sacrificed and plasma taken by cardiac puncture, peritoneal cavities were washed with PBS and the lavage Fluid collected. Plasma and lavage fluid were analyzed for IL-1beta via ELISA ( in duplicate). Lavage fluid was also analyzed for PMN recruitment by FACS using Ly6G, CD45 and L/D dye.

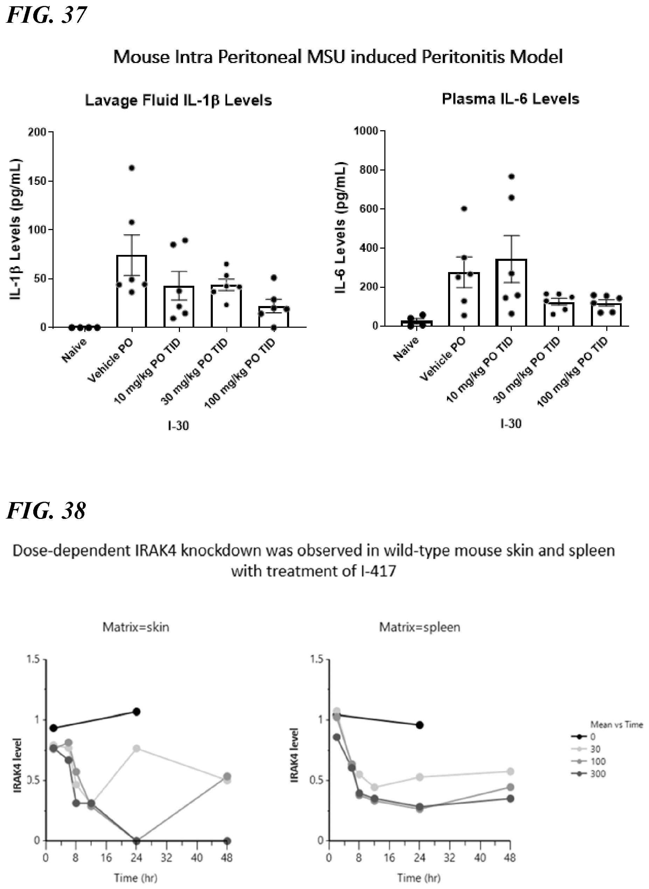

FIG. 37 shows that compounds of the invention significantly reduce IL-1b in peritoneal lavage and plasma IL-6 levels following IP injection of MSU crystals.

### Example 55. PD study of I-417 in wild type mice

Based on the body weight, mice were randomly assigned to respective groups such that the body weights for each treatment groups are the same. All mice had starting body weight greater than 18 grams. For routine monitoring, all study animals were monitored behavior such as mobility, food and water consumption (by cage side checking only), body weight (BW), eye/hair matting and any other abnormal effect. Any mortality and/or abnormal clinical signs were recorded. Body weights of all animals were measured daily throughout the study. Body weight change, expressed in %, was calculated using the following formula: $BW change \left(%\right) = \left({BW}_{Day X}/{BW}_{Day 1}\right) \times 100 ,$ where BW_{DayX} is BW on a given day, and
BW_{Day1} was BW on Day 1 (initiation of treatment).

Animals were dosed orally with 30, 100 or 300 mg/kg compound formulated in 20%HPBCD in water. At termination plasma skin and spleen were collected for analysis. Whole blood was harvested plasma by terminal bleeding into tubes containing anticoagulant, plasma harvest by centrifugation at 4500 rpm @ 5 min and store at -80°C until analysis. Skin and Spleen were snap frozen for PD analysis.

FIG. 38 shows that dose-dependent IRAK4 knockdown was observed in wild-type mouse skin and spleen with treatment of **I-417**.

All of the U.S. patents, U.S. patent application publications, U.S. patent applications, foreign patents, foreign patent applications, and non-patent publications referred to in this specification are incorporated herein by reference in their entireties.

While we have described a number of embodiments of this invention, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of example.

## Claims

1. A compound of Formula **II':** 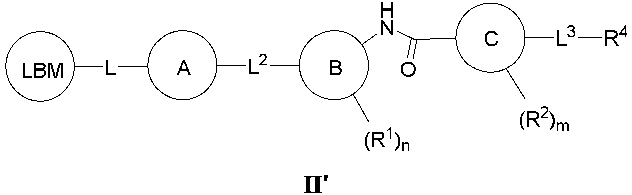 or a pharmaceutically acceptable salt thereof, wherein:
Ring A is an optionally substituted 4-10 membered saturated mono- or bicyclic carbocyclic or heterocyclic ring having 0-2 heteroatoms independently selected from nitrogen, oxygen, and sulfur;
Ring B is phenyl, a 4-7 membered saturated or partially unsaturated carbocyclic ring or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
Ring C is phenyl, a 4-7 membered saturated or partially unsaturated carbocyclic ring or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each of L² and L³ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, -S(O)₂- or -CR=CR-;
each R¹ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CFR₂, - CF₂(R), -CF₃, -CR₂(OR), -CR₂(NR₂), -C(O)R, -C(O)OR, or -C(O)NR₂, or two R¹ on the same carbon together form =O or =S;
each R is independently hydrogen, deuterium, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same atom are optionally taken together with their intervening atom to form an optionally substituted 4-11 membered saturated or partially unsaturated carbocyclic or heterocyclic monocyclic, bicyclic, bridged bicyclic, spiro, or heteroaryl ring having 0-3 heteroatoms, in addition to the atom to which they are attached, independently selected from nitrogen, oxygen, and sulfur;
each R² is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R, or two R² on the same carbon together form =O or =S;
R⁴ is selected from hydrogen or 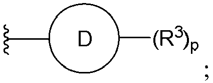
Ring D is phenyl, a 4-10 membered saturated or partially unsaturated mono- or bicyclic carbocyclic or heterocyclic ring having 1-3 heteroatoms independently selected from nitrogen, oxygen, and sulfur, or a 5-6 membered heteroaryl ring having 1-4 heteroatoms independently selected from nitrogen, oxygen, and sulfur;
each R³ is independently hydrogen, deuterium, -R⁵, halogen, -CN, -NO₂, -OR, - SR, -NR₂, -S(O)₂R, -S(O)₂NR₂, -S(O)R, -S(O)(NR)R, -P(O)(OR)₂, -P(O)(NR₂)₂, -CF₂(R), - CF₃, -CR₂(OR), -CR₂(NR₂), -C(O)R, -C(O)OR, - C(O)NR₂, -C(O)N(R)OR, -OC(O)R, -OC(O)NR₂, - N(R)C(O)OR, -N(R)C(O)R, -N(R)C(O)NR₂, or -N(R)S(O)₂R, or two R³ on the same carbon together form =O or =S;
each R⁵ is independently an optionally substituted group selected from C₁₋₆ aliphatic, phenyl, a 3-7 membered saturated or partially unsaturated carbocyclic or heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
each n is 0, 1, or 2;
each m is 0, 1, 2, 3 or 4;
each p is 0, 1, 2, 3 or 4;
L is a covalent bond or a bivalent, saturated or unsaturated, straight or branched C₁₋₅₀ hydrocarbon chain, wherein 0-6 methylene units of L are independently replaced by -C(D)(H)-, -C(D)₂-, -Cy-, -O-, -N(R)-, -Si(R)₂-, -Si(OH)(R)-, -Si(OH)₂-, -P(O)(OR)-, -P(O)(R)-, -P(O)(NR₂)-, -S-, - OC(O)-, -C(O)O-, -C(O)-, -S(O)-, -S(O)₂-, -N(R)S(O)₂-, -S(O)₂N(R)-, -N(R)C(O)-, -C(O)N(R)-, -OC(O)N(R)-, -N(R)C(O)O-, 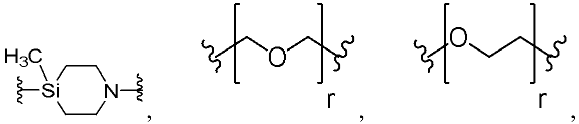 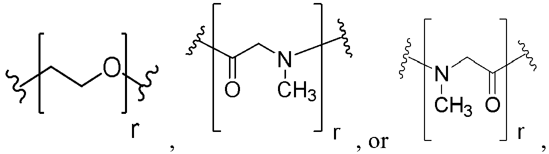 wherein
each -Cy- is independently an optionally substituted bivalent ring selected from phenylenyl, an 8-10 membered bicyclic arylenyl, a 4-7 membered saturated or partially unsaturated carbocyclylenyl, a 4-11 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-11 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur, and wherein r is 0, 1, 2, 3, 4, 5, 6, 7, 8, 9, or 10; and
LBM is a ligase binding moiety,
wherein the compound of formula **II'** is not compound **I-99** or **I-100** in **Table 1A.**

2. The compound according to claim 1, wherein said compound is formula **II-a, II-b, II-c,** or **II-d:** 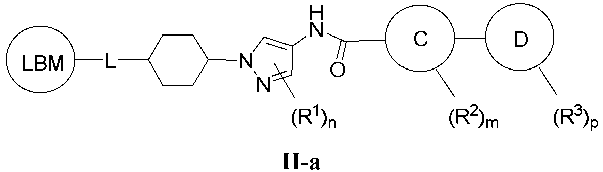 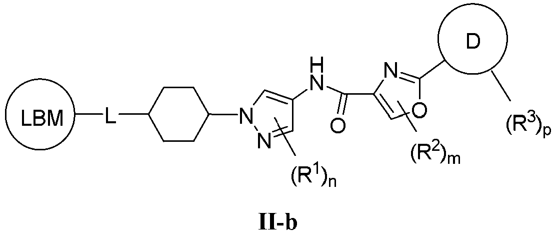 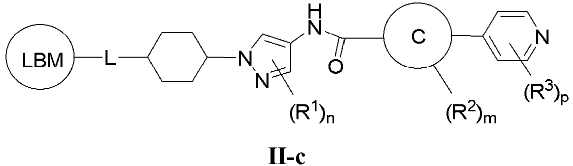 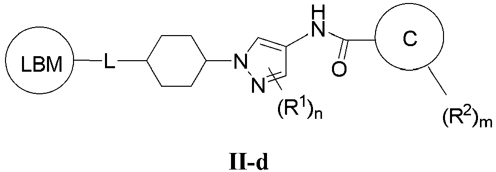 or a pharmaceutically acceptable salt thereof, or:

3. The compound according to either of claim 1 or claim 2, wherein LBM is cereblon E3 ubiquitin ligase binding moiety 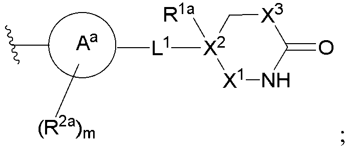 wherein:
X¹ is a bivalent moiety selected from a covalent bond, -CH₂-, -C(O)-, -C(S)-, or 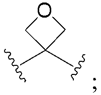
X² is a carbon atom or silicon atom;
X³ is a bivalent moiety selected from -CH₂- or -Si(R₂)-;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur;
R^{1a} is hydrogen, deuterium, halogen, -CN, -OR, -SR, -S(O)R, -S(O)₂R, -N(R)₂, -Si(R)₃, or an optionally substituted C₁₋₄ aliphatic;
each R^{2a} is independently hydrogen, deuterium, -R^{6a}, halogen, -CN, -NO₂, -OR, -SR, -NR₂, -Si(R)₃, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring A^{a} is a bi- or tricyclic ring selected from 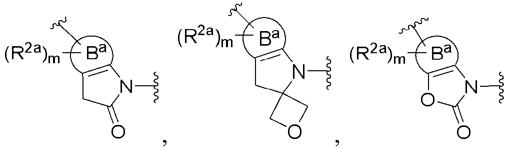 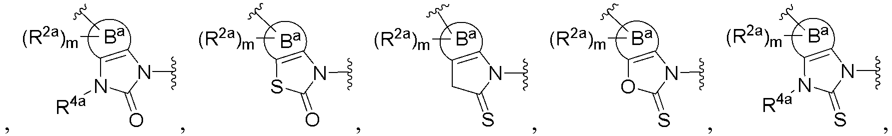 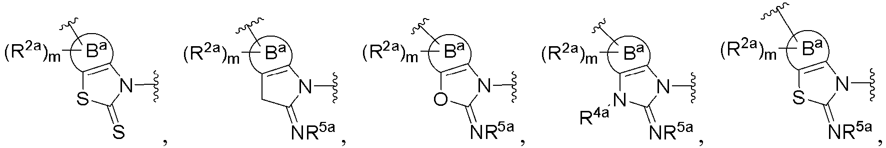 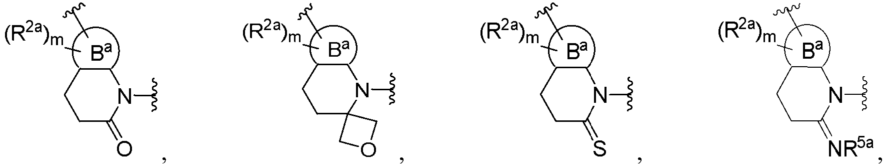 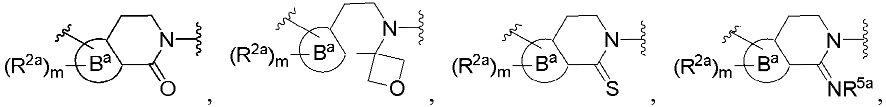 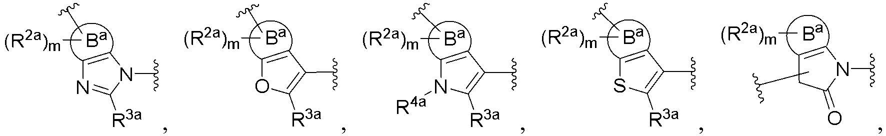 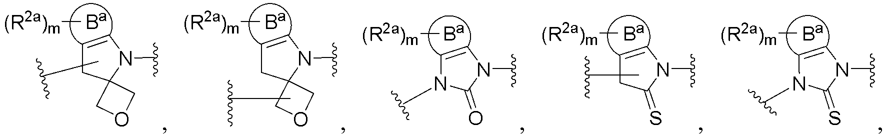 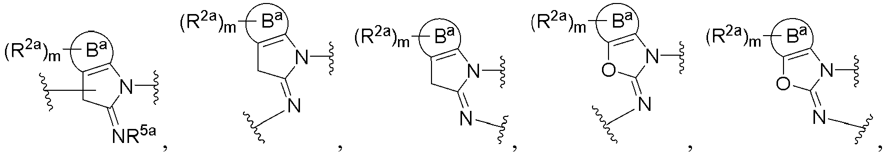 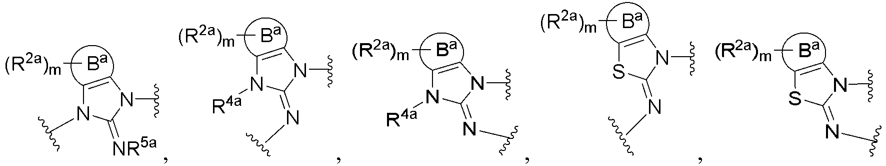 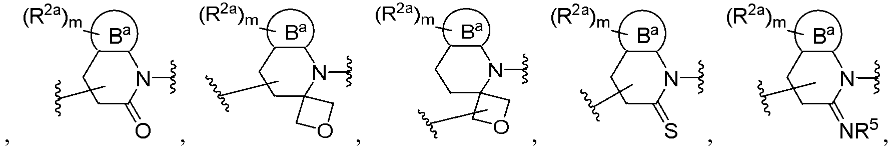 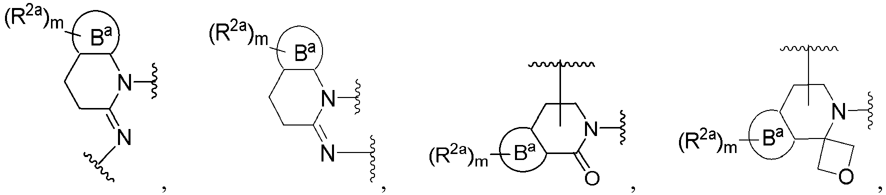 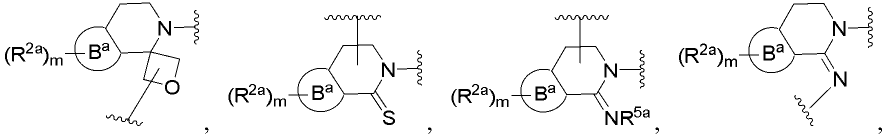 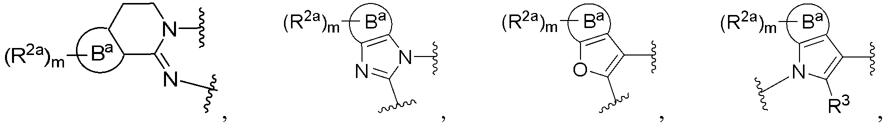 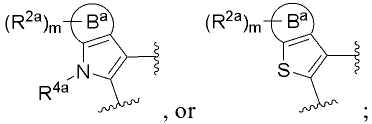
Ring B^{a} is a fused ring selected from 6-membered aryl containing 0-2 nitrogen atoms, 5 to 7-membered partially saturated carbocyclyl, 5 to 7-membered partially saturated heterocyclyl with 1-2 heteroatoms independently selected from boron, nitrogen, oxygen, silicon, or sulfur, or 5-membered heteroaryl with 1-3 heteroatoms independently selected from nitrogen, oxygen or sulfur;
R^{3a} is selected from hydrogen, halogen, -OR, -N(R)₂, or -SR;
each R^{4a} is independently hydrogen, -R^{6a}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R⁵ is hydrogen, C₁₋₄ aliphatic, or -CN;
each R^{6a} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, - S(O)₂- or -CR=CR-; and
m is 0, 1, 2, 3 or 4, or:
wherein said compound is any one of formula **II-h, II-i, II-j, II-k,** or **II-l:** 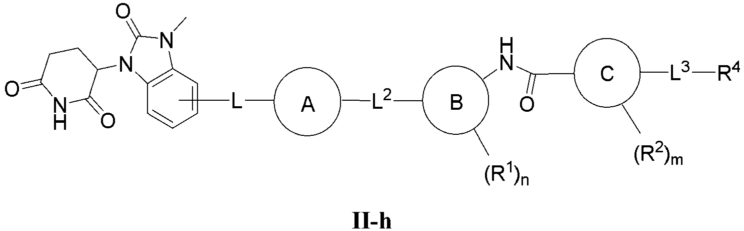 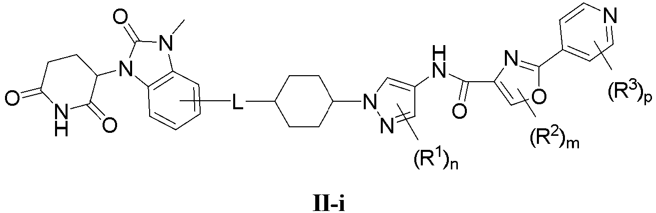 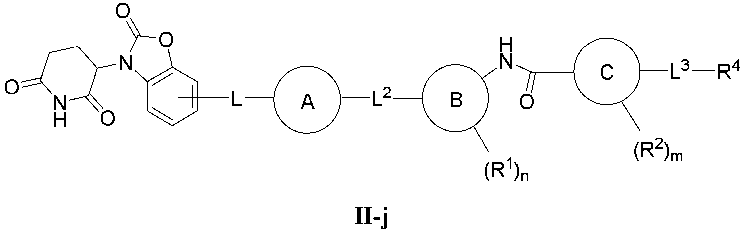 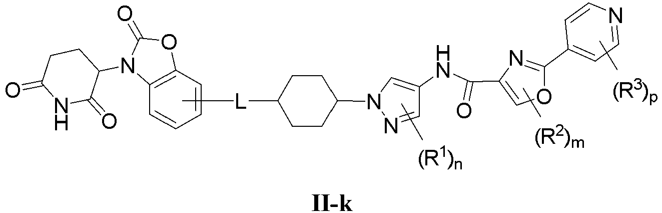 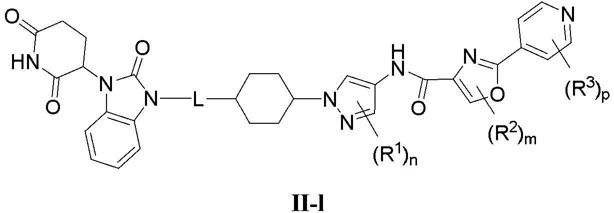
or a pharmaceutically acceptable salt thereof.

4. The compound according to either of claim 1 or claim 2, wherein (I) LBM is E3 ubiquitin ligase binding moiety 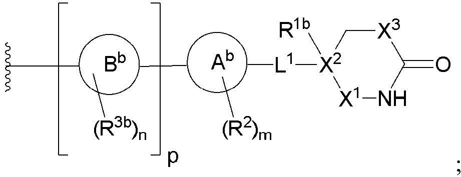 wherein:
X¹ is a bivalent moiety selected from a covalent bond, -CH₂-, -C(O)-, -C(S)-, or 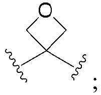
X² is a carbon atom or silicon atom;
X³ is a bivalent moiety selected from -CH₂- or -Si(R₂)-; each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur;
R^{1b} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{b} is a mono- or bicyclic ring selected from 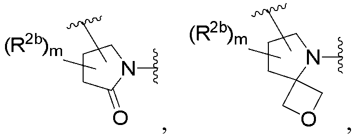 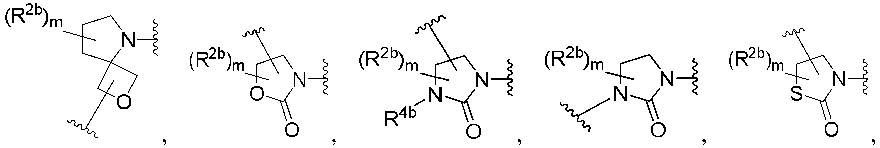 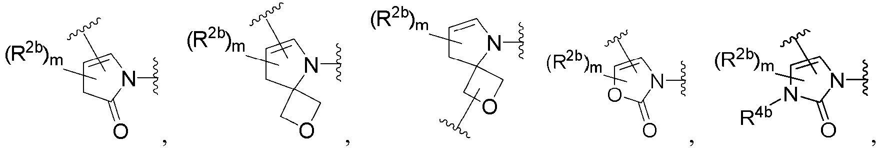 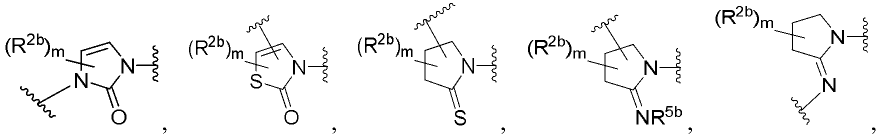 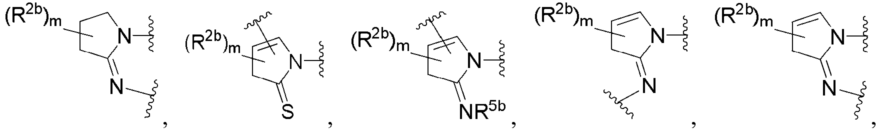 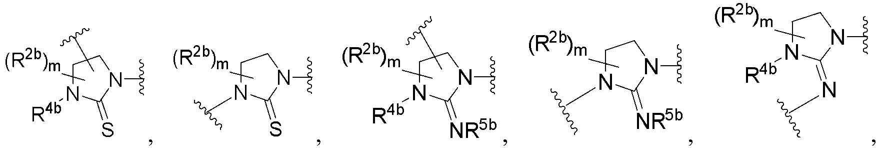 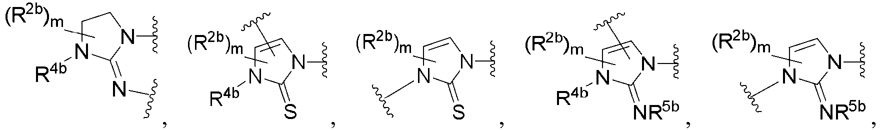 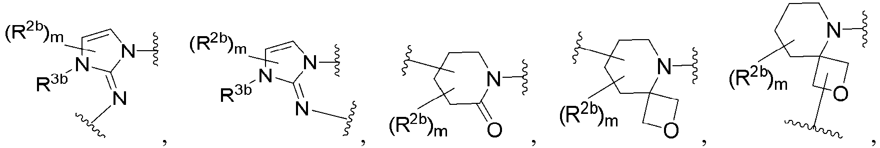 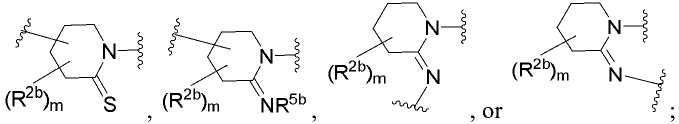
each R^{2b} is independently hydrogen, -R^{6b}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{b} is selected from a 6-membered aryl containing 0-2 nitrogen atoms or a 5-membered heteroaryl with 1-3 heteroatoms independently selected from nitrogen, oxygen or sulfur;
each of R^{3b} and R^{4b} is independently hydrogen, -R^{6b}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5b} is hydrogen, C₁₋₄ aliphatic, or -CN;
each R^{6b} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, - S(O)₂- or -CR=CR-;
m is 0, 1, or 2;
n is 0, 1, 2, 3, or 4; and
p is 0 or 1, or:
wherein (II) LBM is E3 ubiquitin ligase binding moiety 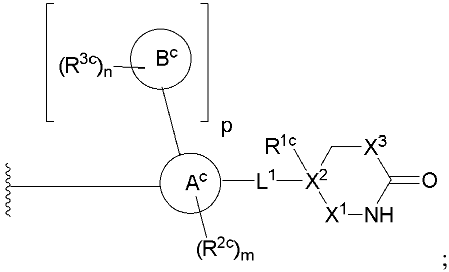 wherein:
X¹ is a bivalent moiety selected from a covalent bond, -CH₂-, -C(O)-, -C(S)-, or 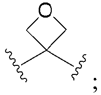
X² is a carbon atom or silicon atom;
X³ is a bivalent moiety selected from -CH₂- or -Si(R₂)-;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are optionally taken together with their intervening atoms to form a 4-7 membered saturated, partially unsaturated, or heteroaryl ring having 0-3 heteroatoms, in addition to the nitrogen, independently selected from nitrogen, oxygen, and sulfur;
R^{1c} is hydrogen, deuterium, halogen, -CN, -OR, -SR, -S(O)R, -S(O)₂R, -N(R)₂, -Si(R)₃, or an optionally substituted C₁₋₄ aliphatic;
Ring A^{c} is a mono- or bicyclic ring selected from 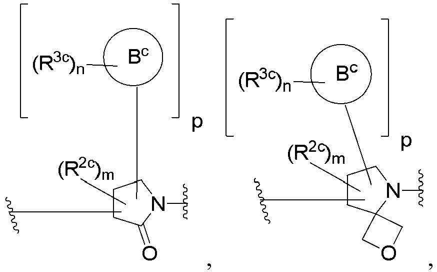 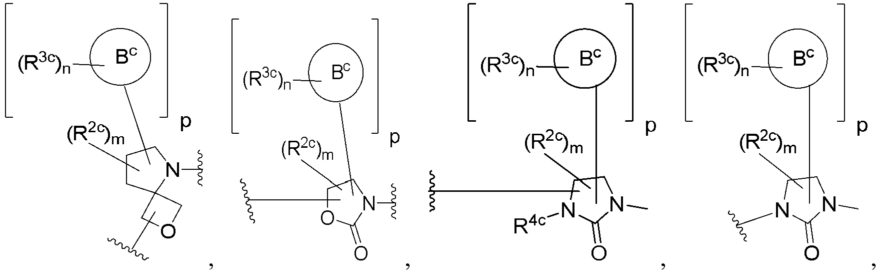 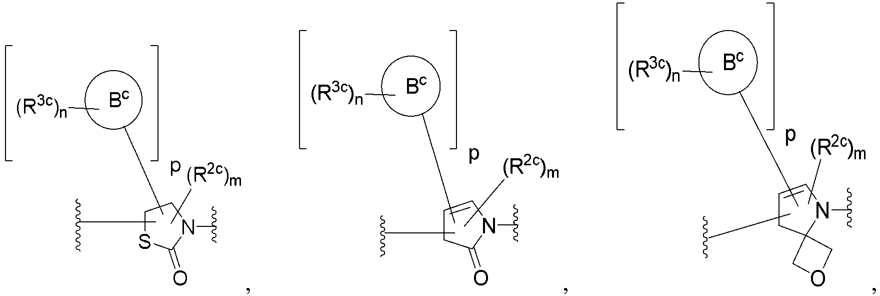 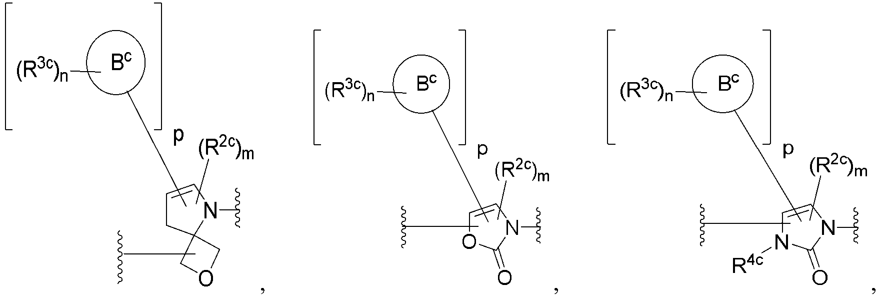 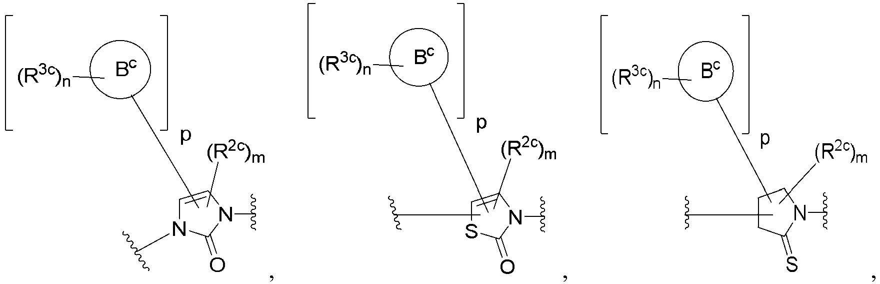 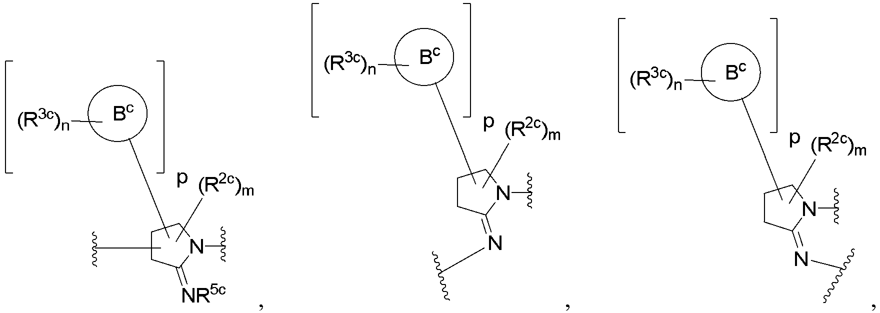 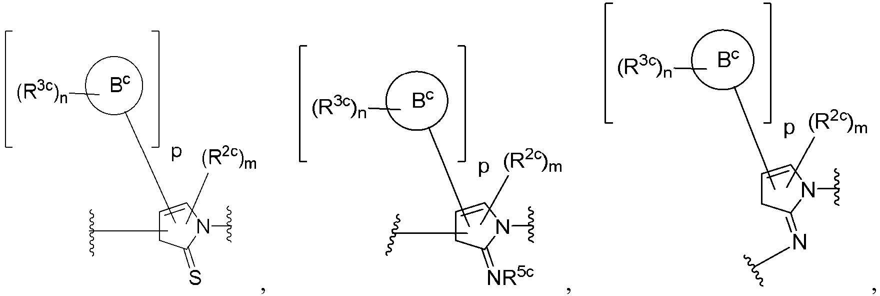 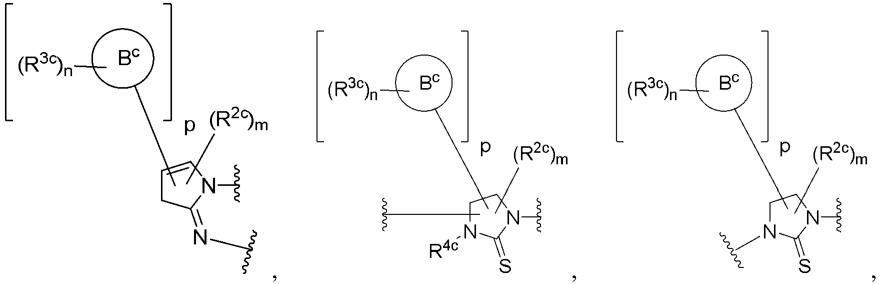 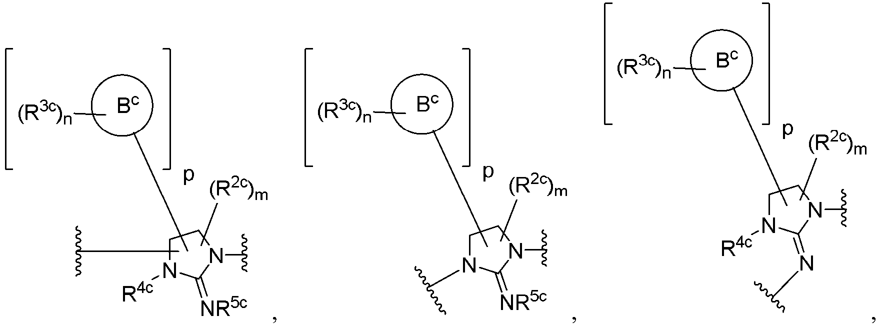 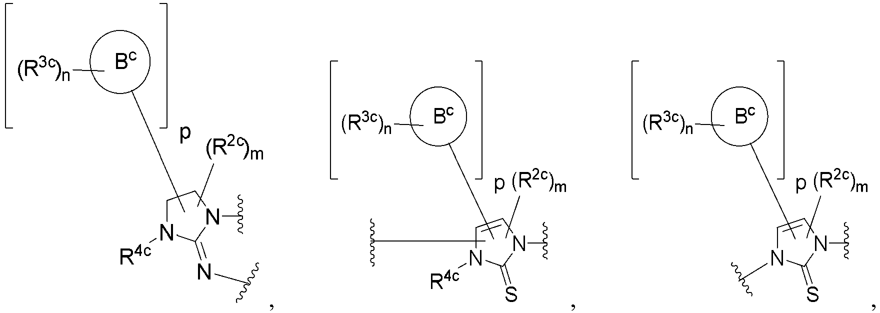 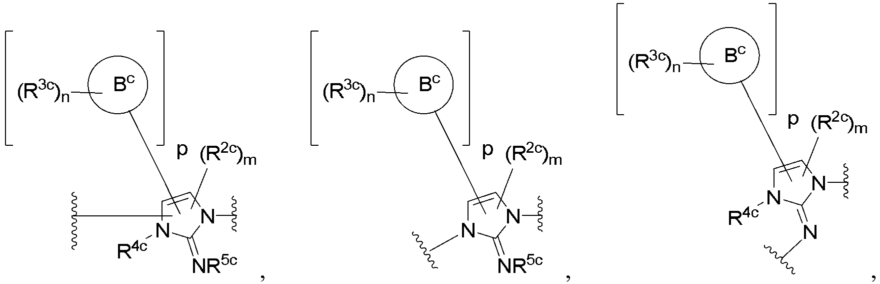 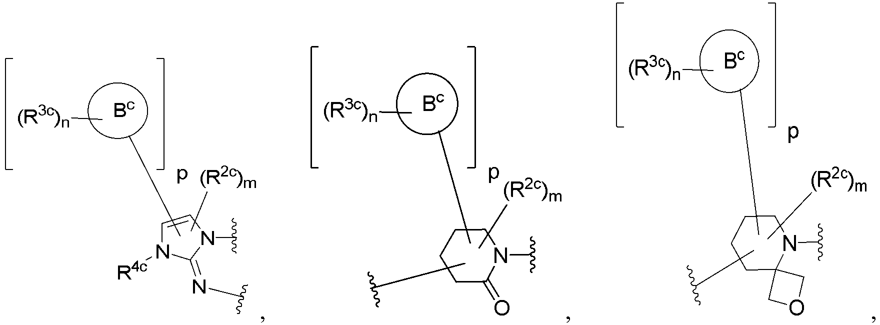 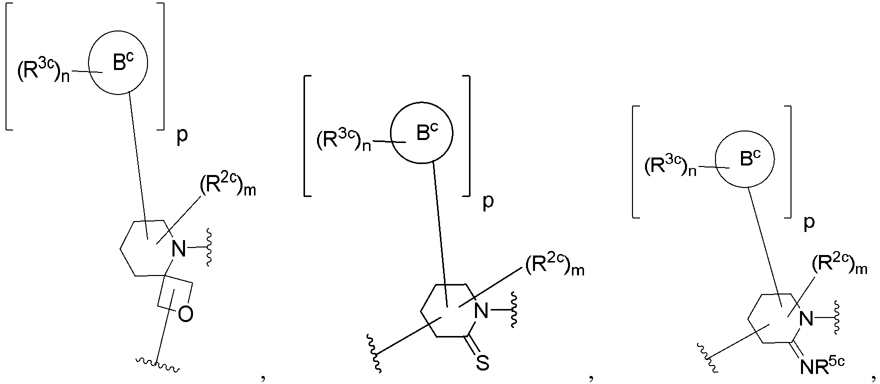 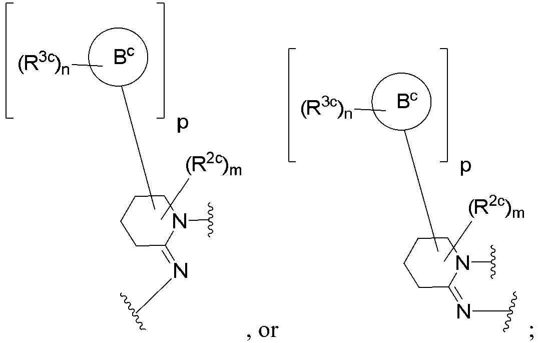
each R^{2c} is independently hydrogen, -R^{6c}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
Ring B^{c} is selected from a 6-membered aryl containing 0-2 nitrogen atoms or a 5-membered heteroaryl with 1-3 heteroatoms independently selected from nitrogen, oxygen or sulfur;
each of R^{3c} and R^{4c} is independently hydrogen, -R^{6c}, halogen, -CN, -NO₂, -OR, -SR, -NR₂, -S(O)₂R, -S(O)₂NR₂, -S(O)R, -C(O)R, -C(O)OR, -C(O)NR₂, -C(O)N(R)OR, -OC(O)R, -OC(O)NR₂, -N(R)C(O)OR, -N(R)C(O)R, -N(R)C(O)NR₂, or -N(R)S(O)₂R;
R^{5c} is hydrogen, C₁₋₄ aliphatic, or -CN;
each R^{6c} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
L¹ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, - S(O)₂- or -CR=CR-;
m is 0, 1, or 2;
n is 0, 1, 2, 3, or 4; and
p is 0 or 1, optionally
wherein said compound is of formula II-m: 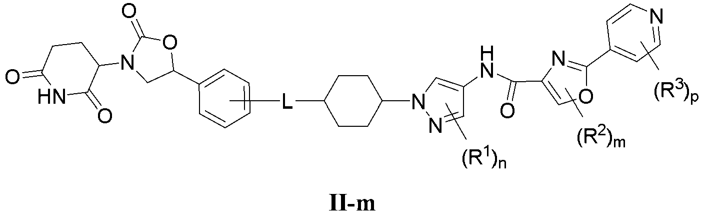
or a pharmaceutically acceptable salt thereof.

5. The compound according to either of claim 1 or claim 2, wherein (I) LBM is E3 ubiquitin ligase binding moiety 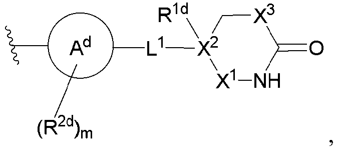 wherein:
X¹ is a bivalent moiety selected from a covalent bond, -CH₂-, -C(O)-, -C(S)-, or 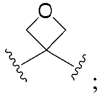
X² is a carbon atom or silicon atom;
X³ is a bivalent moiety selected from -CH₂- or -Si(R₂)-;
R^{1d} is hydrogen, deuterium, halogen, -CN, -OR, -SR, -S(O)R, -S(O)₂R, -N(R)₂, -Si(R)₃, or an optionally substituted C₁₋₄ aliphatic;
each R is independently hydrogen, or an optionally substituted group selected from C₁₋₆ aliphatic, phenyl, a 4-7 membered saturated or partially unsaturated heterocyclic having 1-2 heteroatoms independently selected from nitrogen, oxygen, and sulfur, and a 5-6 membered heteroaryl ring having 1-4 heteroatoms independently selected from nitrogen, oxygen, and sulfur, or:
two R groups on the same nitrogen are taken together with their intervening atoms to form a 4-7 membered saturated, partially unsaturated, or heteroaryl ring having 0-3 heteroatoms, in addition to the nitrogen, independently selected from nitrogen, oxygen, and sulfur;
each R^{2d} is independently hydrogen, -R^{3d}, halogen, -CN, -NO₂, -OR, -SR, -N(R)₂, -Si(R)₃, -S(O)₂R, -S(O)₂N(R)₂, -S(O)R, -C(O)R, -C(O)OR, -C(O)N(R)₂, -C(O)N(R)OR, -C(R)₂N(R)C(O)R, -C(R)₂N(R)C(O)N(R)₂, -OC(O)R, -OC(O)N(R)₂, - N(R)C(O)OR, -N(R)C(O)R, -N(R)C(O)N(R)₂, or -N(R)S(O)₂R, or two R^{2d} on the same carbon together form =O or =S;
each R^{3d} is independently an optionally substituted group selected from C₁₋₆ aliphatic, phenyl, a 4-7 membered saturated or partially unsaturated heterocyclic ring having 1-2 heteroatoms independently selected from nitrogen, oxygen, and sulfur, and a 5-6 membered heteroaryl ring having 1-4 heteroatoms independently selected from nitrogen, oxygen, and sulfur;
Ring A^{d} is a tricyclic ring selected from 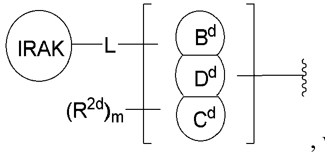
wherein
each of Ring B^{d}, Ring C^{d}, and Ring D^{d} is independently a fused ring selected from 6-membered aryl containing 0-3 nitrogens, 5 to 7-membered saturated or partially unsaturated carbocyclyl, 5 to 7-membered saturated or partially unsaturated heterocyclyl ring with 1-3 heteroatoms independently selected from boron, nitrogen, oxygen, silicon, or sulfur, or 5-membered heteroaryl with 1-3 heteroatoms independently selected from nitrogen, oxygen or sulfur; and
L¹ is independently a covalent bond or a C₁₋₃ bivalent straight or branched saturated or unsaturated hydrocarbon chain wherein 1-3 methylene units of the chain are independently and optionally replaced with -O-, -C(O)-, -C(S)-, -C(R)₂-, -CH(R)-, -C(F)₂-, -N(R)-, -S-, - S(O)₂- or -CR=CR-; and
m is 0, 1, 2, 3, 4, 5, 6, 7, 8, 9, 10, 11, 12, 13, 14, 15, or 16; optionally:
wherein said compound is of formula **II-r** or **II-s**: 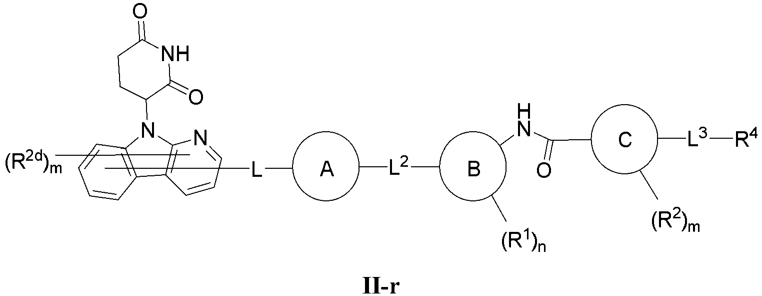 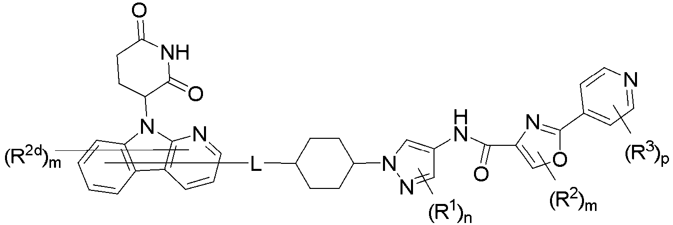 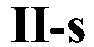
or a pharmaceutically acceptable salt thereof, or
wherein (II) LBM is cereblon E3 ubiquitin ligase binding moiety 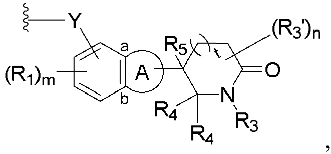 or a pharmaceutically acceptable salt thereof, wherein: 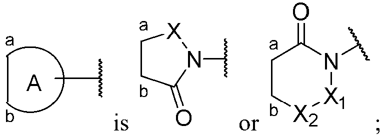
Y is a bond, Y₁, O, NH, NR₂, C(O)O, OC(O), C(O)NR₂', NR₂'C(O), Y₁-O, Y₁-NH, Y₁-NR₂, Yi-C(O), Y₁-C(O)O, Y₁-OC(O), Y₁-C(O)NR₂', or Y₁-NR₂'C(O), wherein Y₁ is C₁₋₆ alkylene, C₂-₆ alkenylene, or C₂-C₆ alkynylene;
X is C(O) or C(R₃)₂;
X₁-X₂ is C(R₃)=N or C(R₃)₂-C(R₃)₂;
each R¹ is independently halogen, nitro, NH₂, OH, C(O)OH, C₁₋₆ alkyl, or C₁₋₆ alkoxy;
R² is C₁₋₆ alkyl, C₂-₆ alkenyl, C₃-₈ cycloalkyl, 3- to 8-membered heterocycloalkyl, C(O)-C₁-₆ alkyl, C(O)-C₂-₆ alkenyl, C(O)-C₃-₈ cycloalkyl, or C(O)-3- to 8-membered heterocycloalkyl, and R² is optionally substituted with one or more of halogen, N(Rₐ)₂, NHC(O)Rₐ, NHC(O)ORₐ, OR_{b}, C₃-₈ cycloalkyl, 3- to 8-membered heterocycloalkyl, C₆₋₁₀ aryl, or 5- to 10-membered heteroaryl, wherein each of the C₃-₈ cycloalkyl, 3- to 8-membered heterocycloalkyl, C₆-₁₀ aryl or 5- to 10-membered heteroaryl is optionally further substituted with one or more of halogen, NH₂, CN, nitro, OH, C(O)OH, C₁₋₆ alkyl, C₁₋₆ haloalkyl, C₁₋₆ alkoxy, or C₁-₆ haloalkoxy;
R₂' is H, C₁₋₆ alkyl, C₂-₆ alkenyl, C₃-₈ cycloalkyl, or 3- to 8-membered heterocycloalkyl, and R₂', when not being H, is optionally substituted with one or more of halogen, N(Rₐ)₂, NHC(O)Rₐ, NHC(O)ORₐ, OR_{b}, C₃-₈ cycloalkyl, 3- to 8-membered heterocycloalkyl, C₆-₁₀ aryl, or 5- to 10-membered heteroaryl, wherein each of the C₃-₈ cycloalkyl, 3- to 8-membered heterocycloalkyl, C₆-₁₀ aryl or 5- to 10-membered heteroaryl is optionally further substituted with one or more of halogen, NH₂, CN, nitro, OH, C(O)OH, C₁₋₆ alkyl, C₁₋₆ haloalkyl, C₁₋₆ alkoxy, or C₁₋₆ haloalkoxy;
each R₃ is independently H or C₁₋₃ alkyl optionally substituted with C₆-₁₀ aryl or 5- to 10-membered heteroaryl;
each R₃' is independently C₁₋₃ alkyl;
each R₄ is independently H or C₁₋₃ alkyl; or two R₄, together with the carbon atom to which they are attached, form C(O), a C₃-₆ carbocycle, or a 4-, 5-, or 6-membered heterocycle comprising 1 or 2 heteroatoms selected from N and O;
R₅ is H, C₁₋₃ alkyl, F, or Cl;
each Rₐ independently is H or C₁₋₆ alkyl;
R_{b} is H or tosyl;
t is 0 or 1;
m is 0, 1, 2 or 3; and
n is 0, 1 or 2, optionally:
wherein said compound is of formula **II-n, II-o, II-p,** or **II-q:** 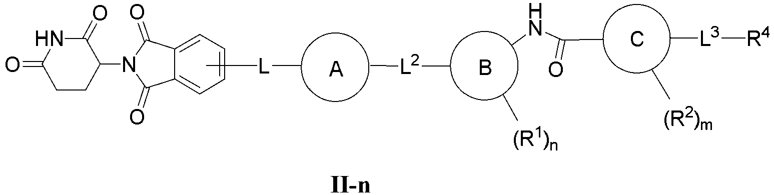 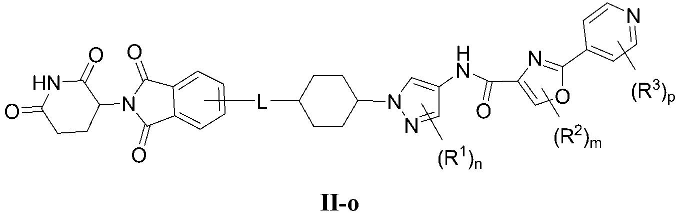 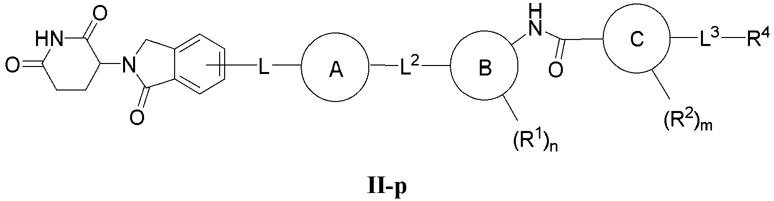 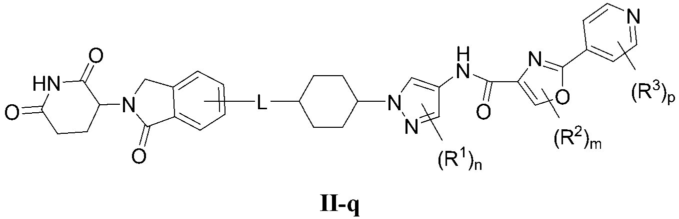
or a pharmaceutically acceptable salt thereof, or:
wherein (III) LBM is IAP E3 ubiquitin ligase binding moiety 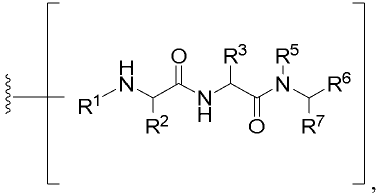 or a pharmaceutically acceptable salt thereof, wherein:
R¹ is selected from hydrogen or alkyl;
R² is selected from hydrogen or alkyl;
R³ is selected from hydrogen, alkyl, cycloalkyl and heterocycloalkyl;
R⁵ and R⁶ are independently selected from hydrogen, alkyl, cycloalkyl, heterocycloalkyl, or
R⁵ and R⁶ are taken together to form a pyrrolidine or a piperidine ring further optionally fused to 1-2 cycloalkyl, heterocycloalkyl, aryl or heteroaryl rings, each of which can then be further fused to another cycloalkyl, heterocycloalkyl, aryl or heteroaryl ring, or
R³ and R⁵ are taken together to form a 5-8-membered ring further optionally fused to 1-2 cycloalkyl, heterocycloalkyl, aryl or heteroaryl ring;
R⁷ is selected from cycloalkyl, cycloalkylalkyl, heterocycloalkyl, heterocycloalkylalkyl, aryl, arylalkyl, heteroaryl or heteroarylalkyl, each one further optionally substituted with 1-3 substituents selected from halogen, alkyl, haloalkyl, hydroxyl, alkoxy, cyano, (hetero)cycloalkyl or (hetero)aryl, or R⁷ is C(O)NHR⁴; and
R⁴ is selected from alkyl, cycloalkyl, heterocycloalkyl, cycloalkylalkyl, heterocycloalkylalkyl, aryl, arylalkyl, heteroaryl, heteroarylalkyl, further optionally substituted with 1-3 substituents selected from halogen, alkyl, haloalkyl, hydroxyl, alkoxy, cyano, (hetero)cycloalkyl or (hetero)aryl, or R⁷ is C(O)NHR⁴, optionally:
wherein said compound is of formula II-vv, II-ww, II-zz, or II-aaa: 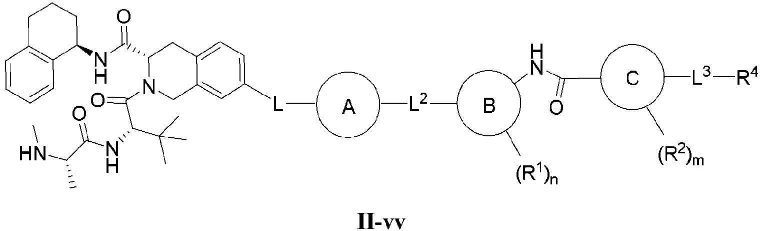 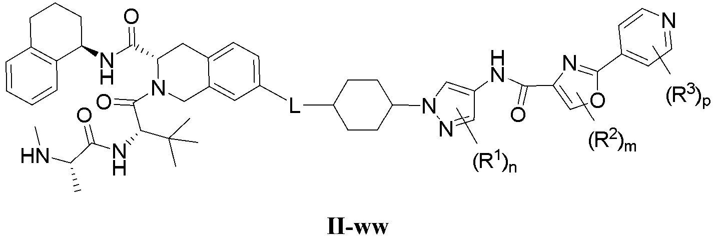 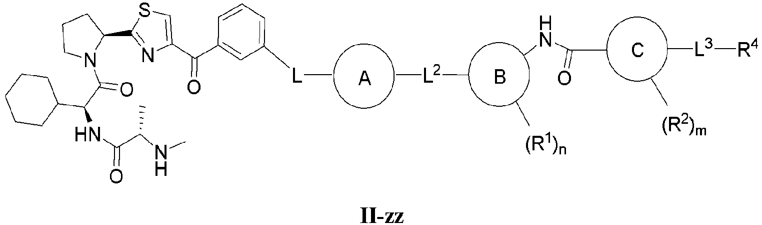 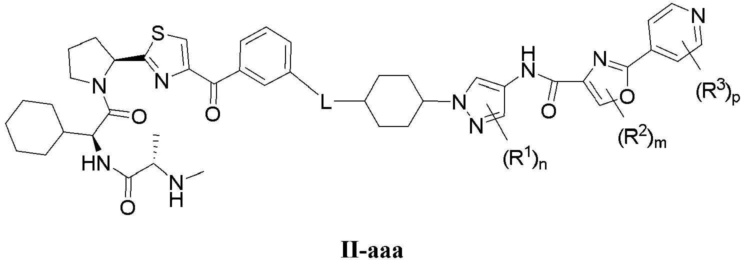
or a pharmaceutically acceptable salt thereof, or
wherein (IV) LBM is a MDM2 E3 ligase binding moiety 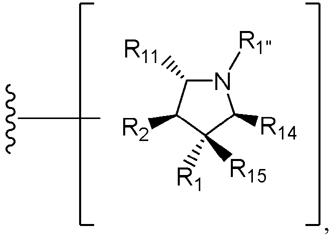 or a pharmaceutically acceptable salt thereof, wherein:
R₁ and R₂ are independently selected from the group consisting of an aryl or heteroaryl group, a heteroaryl group having one or two heteroatoms independently selected from sulfur or nitrogen, wherein the aryl or heteroaryl group can be mono-cyclic or bi-cyclic, or unsubstituted or substituted with one to three substituents independently selected from the group consisting of: halogen, -CN, C₁₋₆ alkyl group, C₃₋₆ cycloalkyl, -OH, alkoxy with 1 to 6 carbons, fluorine substituted alkoxy with 1-6 carbons, sulfoxide with 1-6 carbons, sulfone with 1-6 carbons, ketone with 2-6 carbons, amides with 2-6 carbons, and dialkyl amine with 2-6 carbons;
R₁₁ is -C(O)-N(R^{h})(Rⁱ), wherein R^{h} and Rⁱ are selected from groups consisting of the following: H, C1 to C6 alkyl, alkoxy substituted alkyl, sulfone substituted alkyl, aryl, heterol aryl, mono-, bis- or tri-substituted aryl or hetero aryl, alkyl carboxylic acid, heteroaryl carboxylic acid, alkyl carboxylic acid, fluorine substituted alkyl carboxylic acid, aryl substituted cycloalkyl, hetero aryl substituted cycloalkyl; wherein R^{h} and Rⁱ are independently selected from the group consisting of H, connected to form a ring, 4-hydroxycyclohehexane; mono- and di-hydroxy substituted alkyl (C₃₋₆); 3-hydroxycyclobutane; phenyl-4-carboxylic acid, and substituted phenyl-4-carboxylic acid;
R₁₄ is selected from the group consisting of alkyl, substituted alkyl, alkenyl, substituted alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₁₅ is CN;
R_{1"} is selected from the group consisting of alkyl, aryl substituted alkyl, alkoxy substituted alkyl, cycloalkyl, aryl-substituted cycloalkyl, and alkoxy substituted cycloalkyl, optionally
wherein said compound is of formula **II-xx** or **II-yy:** 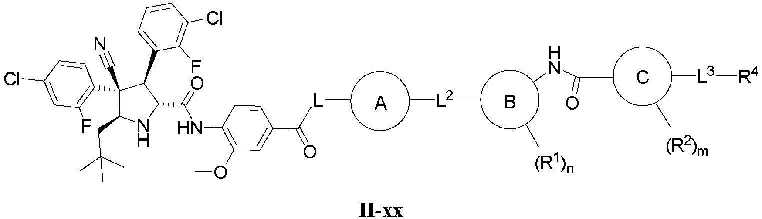 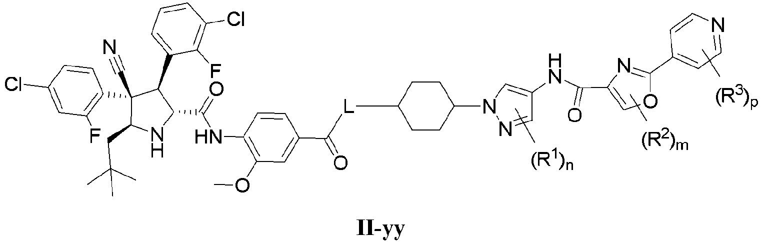
or a pharmaceutically acceptable salt thereof.

6. The compound according to either of claim 1 or claim 2, wherein LBM is VHL E3 ubiquitin ligase binding moiety 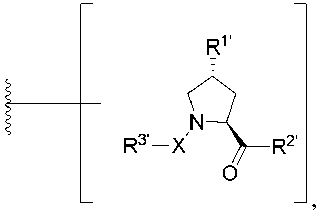 or a pharmaceutically acceptable salt thereof, wherein:
R^{1'} is an optionally substituted C₁₋₆ alkyl group, an optionally substituted -(CH₂)ₙOH, an optionally substituted -(CH₂)ₙSH, an optionally substituted (CH₂)ₙ-O-(C₁-C₆)alkyl group, an optionally substituted (CH₂)ₙ-WCOCW-(C₀₋₆) alkyl group containing an epoxide moiety WCOCW where each W is independently H or a C₁₋₃ alkyl group, an optionally substituted -(CH₂)ₙCOOH, an optionally substituted -(CH₂)ₙC(O)-(C₁-₆ alkyl), an optionally substituted -(CH₂)ₙNHC(O)-R₁, an optionally substituted -(CH₂)ₙC(O)-NR₁R₂, an optionally substituted -(CH₂)ₙOC(O)-NR₁R₂, -(CH₂O)ₙH, an optionally substituted - (CH₂)ₙOC(O)-(C₁-₆ alkyl), an optionally substituted -(CH₂)ₙC(O)-O-(C₁-₆ alkyl), an optionally substituted -(CH₂O)ₙCOOH, an optionally substituted -(OCH₂)ₙO-(C₁-C₆ alkyl), an optionally substituted -(CH₂O)ₙC(O)-(C₁-₆ alkyl), an optionally substituted - (OCH₂)ₙNHC(O)-R₁, an optionally substituted -(CH₂O)ₙC(O)-NR₁R₂, -(CH₂CH₂O)ₙH, an optionally substituted -(CH₂CH₂O)ₙCOOH, an optionally substituted -(OCH₂CH₂)ₙO-(C₁-₆ alkyl), an optionally substituted -(CH₂CH₂O)ₙC(O)-(C₁-C₆ alkyl), an optionally substituted -(OCH₂CH₂)ₙNHC(O)-R₁, an optionally substituted -(CH₂CH₂O)ₙC(O)-NR₁R₂, an optionally substituted -SO₂R_{S}, an optionally substituted S(O)R_{S}, NO₂, CN, or halogen;
R₁ and R₂ are each independently H or a C₁₋₆ alkyl group which may be optionally substituted with one or two hydroxyl groups or up to three halogen groups;
R_{S} is a C₁₋₆ alkyl group, an optionally substituted aryl, heteroaryl or heterocycle group or a - (CH₂)ₘNR₁R₂ group;
X and X' are each independently C=O, C=S, -S(O), S(O)₂;
R is an optionally substituted -(CH₂)ₙ-(C=O)ᵤ(NR₁)ᵥ(SO₂)_{w}alkyl group, an optionally substituted -(CH₂)ₙ-(C=O)ᵤ(NR₁)ᵥ(SO₂)_{w}NR_{1N}R_{2N} group, an optionally substituted -(CH₂)ₙ-(C=O)ᵤ(NR₁)ᵥ(SO₂)_{w}-Aryl, an optionally substituted -(CH₂)ₙ-(C=O)ᵤ(NR₁)ᵥ(SO₂)_{w}-heteroaryl, an optionally substituted -(CH₂)ₙ-(C=O)ᵥNR₁(SO₂)_{w}-heterocycle, an optionally substituted -NR¹-(CH₂)ₙ-C(O)ᵤ(NR₁)ᵥ(SO₂)_{w}-alkyl, an optionally substituted -NR¹-(CH₂)ₙ-C(O)ᵤ(NR₁)ᵥ(SO₂)_{w}-NR_{1N}R_{2N}, an optionally substituted -NR¹-(CH₂)ₙ-C(O)ᵤ(NR₁)ᵥ(SO₂)_{w}-NR₁C(O)R_{1N}, an optionally substituted -NR¹-(CH₂)ₙ-(C=O)ᵤ(NR₁)ᵥ(SO₂)_{w}-aryl, an optionally substituted -NR¹-(CH₂)ₙ-(C=O)ᵤ(NR₁)ᵥ(SO₂)_{w}-heteroaryl or an optionally substituted -NR¹-(CH₂)ₙ-(C=O)ᵥNR₁(SO₂)_{w}-heterocycle, an optionally substituted -X^{R2'}-alkyl group; an optionally substituted -X^{R2'}-aryl group; an optionally substituted -X^{R2'}-heteroaryl group; an optionally substituted -X^{R2'}-heterocycle group;
R^{3'} is an optionally substituted alkyl, an optionally substituted -(CH₂)ₙ-(O)ᵤ(NR₁)ᵥ(SO₂)_{w}-alkyl, an optionally substituted -(CH₂)ₙ-C(O)ᵤ(NR₁)ᵥ(SO₂)_{w}-NR_{1N}R_{2N}, an optionally substituted - (CH₂)ₙ-C(O)ᵤ(NR₁)ᵥ(SO₂)_{w}-NR₁C(O)R_{1N}, an optionally substituted -(CH₂)ₙ-C(O)ᵤ(NR₁)ᵥ(SO₂)_{w}-C(O)NR₁R₂, an optionally substituted -(CH₂)ₙ-C(O)ᵤ(NR₁)ᵥ(SO₂)_{w}-Aryl, an optionally substituted -(CH₂)ₙ-C(O)ᵤ(NR₁)ᵥ(SO₂)_{w}-heteroaryl, an optionally substituted -(CH₂)ₙ-C(O)ᵤ(NR₁)ᵥ(SO₂)_{w}-heterocycle, an optionally substituted -NR¹-(CH₂)ₙ-C(O)ᵤ(NR₁)ᵥ(SO₂)_{w}-alkyl, an optionally substituted -NR¹-(CH₂)ₙ-C(O)ᵤ(NR₁)ᵥ(SO₂)_{w}-NR_{1N}R_{2N}, an optionally substituted -NR¹-(CH₂)ₙ-C(O)ᵤ(NR₁)ᵥ(SO₂)_{w}-NR₁C(O)R_{1N}, an optionally substituted -NR¹-(CH₂)ₙ-C(O)ᵤ(NR₁)ᵥ(SO₂)_{w}-Aryl, an optionally substituted -NR¹-(CH₂)ₙ-C(O)ᵤ(NR₁)ᵥ(SO₂)_{w}-heteroaryl, an optionally substituted -NR¹-(CH₂)ₙ-C(O)ᵤ(NR₁)ᵥ(SO₂)_{w}-heterocycle, an optionally substituted -O-(CH₂)n-(C=O)ᵤ(NR₁)ᵥ(SO₂)_{w}-alkyl, an optionally substituted -O-(CH₂)n-(C=O)ᵤ(NR₁)ᵥ(SO₂)_{w}-NR_{1N}R_{2N}, an optionally substituted -O-(CH₂)n-(C=O)ᵤ(NR₁)ᵥ(SO₂)_{w}-NR₁C(O)R_{1N}, an optionally substituted -O-(CH₂)n-(C=O)ᵤ(NR₁)ᵥ(SO₂)_{w}-Aryl, an optionally substituted -O-(CH₂)ₙ-(C=O)ᵤ(NR₁)ᵥ(SO₂)_{w}-heteroaryl or an optionally substituted -O-(CH₂)ₙ-(C=O)ᵤ(NR₁)ᵥ(SO₂)_{w}-heterocycle; - (CH₂)ₙ-(V)_{n'}-(CH₂)ₙ-(V)_{n'}-alkyl group, an optionally substituted -(CH₂)ₙ-(V)_{n'}-(CH₂)ₙ-(V)_{n'}-aryl group, an optionally substituted -(CH₂)ₙ-(V)_{n'}-(CH₂)ₙ-(V)_{n'}-heteroaryl group, an optionally substituted -(CH₂)ₙ-(V)_{n'}-(CH₂)ₙ-(V)_{n'}-heterocycle group, an optionally substituted -(CH₂)ₙ-N(R_{1'})(C=O)_{m'}-(V)_{n'}-alkyl group, an optionally substituted -(CH₂)-N(R_{1'})(C=O)_{m'}-(V)_{n'}-aryl group, an optionally substituted -(CH₂)ₙ-N(R₁')(C=O)_{m'}-(V)_{n'}-heteroaryl group, an optionally substituted -(CH₂)ₙ-N(R₁')(C=O)_{m'}-(V)_{n'}-heterocycle group, an optionally substituted -X^{R3'}-alkyl group, an optionally substituted -X^{R3'}-aryl group, an optionally substituted -X^{R3'}-heteroaryl group, an optionally substituted -X^{R3'}-heterocycle group, wherein R_{1N} and R_{2N} are each independently H, C₁-₆ alkyl which is optionally substituted with one or two hydroxyl groups and up to three halogen groups or an optionally substituted -CH₂)ₙ-aryl, -(CH₂)ₙ-heteroaryl or -(CH₂)ₙ-heterocycle group;
V is O, S or NR₁;
R¹ is the same as above;
R^{h} and R_{1'} are each independently H or a C₁-C₃ alkyl group;
X^{R2'} and X^{R3'} are each independently an optionally substituted -CH₂)ₙ-, -CH₂)ₙ-CH(Xᵥ)=CH(Xᵥ)-, -CH₂)ₙ-CH≡CH-, -(CH₂CH₂O)ₙ- or a C₃-C₆ cycloalkyl group, where Xᵥ is H, a halo or a C₁-C₃ alkyl group which is optionally substituted;
each m is independently 0, 1, 2, 3, 4, 5, 6;
each m' is independently 0 or 1;
each n is independently 0, 1, 2, 3, 4, 5, 6;
each n' is independently 0 or 1;
each u is independently 0 or 1;
each v is independently 0 or 1; and
each w is independently 0 or 1, optionally:
wherein said compound is of formula II-bbb or II-ccc: 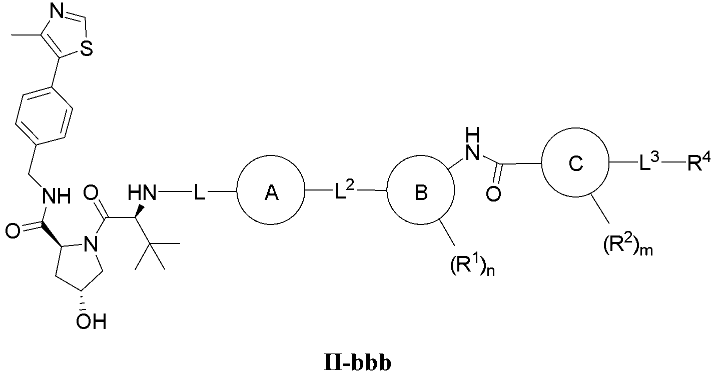 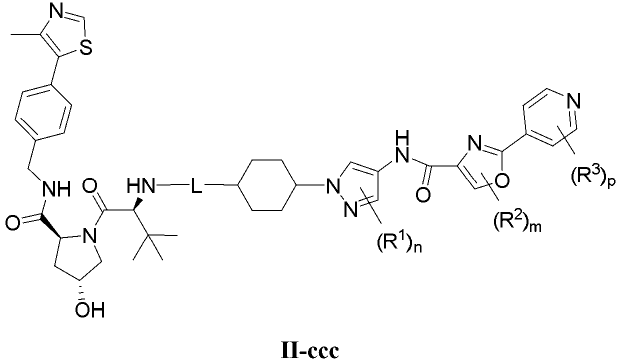
or a pharmaceutically acceptable salt thereof.

7. The compound according to any one of claims 1-6, wherein L is a covalent bond or a bivalent, saturated or unsaturated, straight or branched C₁₋₅₀ hydrocarbon chain, wherein 0-6 methylene units of L are independently replaced by -Cy-, -O-, -N(R)-, -C(O)-, -S(O)-, -S(O)₂-, - N(R)S(O)₂-, -S(O)₂N(R)-, -N(R)C(O)-, -C(O)N(R)-, -OC(O)N(R)-, -N(R)C(O)O-, wherein
each -Cy- is independently an optionally substituted bivalent ring selected from phenylenyl, an 8-10 membered bicyclic arylenyl, a 4-7 membered saturated or partially unsaturated carbocyclylenyl, a 4-11 membered saturated or partially unsaturated spiro carbocyclylenyl, an 8-10 membered bicyclic saturated or partially unsaturated carbocyclylenyl, a 4-7 membered saturated or partially unsaturated heterocyclylenyl having 1-2 heteroatoms independently selected from nitrogen, oxygen, and sulfur, a 4-11 membered saturated or partially unsaturated spiro heterocyclylenyl having 1-2 heteroatoms independently selected from nitrogen, oxygen, and sulfur, an 8-10 membered bicyclic saturated or partially unsaturated heterocyclylenyl having 1-2 heteroatoms independently selected from nitrogen, oxygen, and sulfur, a 5-6 membered heteroarylenyl having 1-4 heteroatoms independently selected from nitrogen, oxygen, and sulfur, or an 8-10 membered bicyclic heteroarylenyl having 1-5 heteroatoms independently selected from nitrogen, oxygen, or sulfur.

8. The compound of any of one of claims 1-7, wherein said compound is selected from any one of those depicted in **Table 1,** or a pharmaceutically acceptable salt thereof.

9. A pharmaceutical composition comprising a compound according any of one claims claims 1-8, and a pharmaceutically acceptable carrier, adjuvant, or vehicle.

10. A method of degrading and/or inhibiting an IRAK protein kinase in a patient or biological sample comprising administering to said patient, or contacting said biological sample with a compound according to any one of claims 1-8, or a pharmaceutical composition thereof, optionally
wherein the IRAK protein kinase is an IRAK-4 protein kinase or wherein the IRAK protein kinase is an IRAK-1, IRAK-2, or IRAK-3 protein kinase.

11. A method of treating an IRAK-mediated disorder, disease, or condition in a patient comprising administering to said patient a compound according to any of one claims claims 1-8, or a pharmaceutical composition thereof.

12. The method of claim 11, wherein the IRAK-mediated disorder, disease or condition is selected from the group consisting of a cancer, a neurodegenerative disease, a viral disease, an autoimmune disease, an inflammatory disorder, a hereditary disorder, a hormone-related disease, a metabolic disorder, a condition associated with organ transplantation, an immunodeficiency disorder, a destructive bone disorder, a proliferative disorder, an infectious disease, a condition associated with cell death, thrombin-induced platelet aggregation, liver disease, a pathologic immune condition involving T cell activation, a cardiovascular disorder, and a CNS disorder.

13. The method of claim 12, wherein the cancer or proliferative disorder is selected from the group consisting of a benign or malignant tumor, solid tumor, carcinoma of the brain, kidney, liver, adrenal gland, bladder, breast, stomach, gastric tumors, ovaries, colon, rectum, prostate, pancreas, lung, vagina, cervix, testis, genitourinary tract, esophagus, larynx, skin, bone or thyroid, sarcoma, glioblastoma, neuroblastoma, multiple myeloma, gastrointestinal cancer, colon carcinoma, colorectal adenoma, a tumor of the neck and head, an epidermal hyperproliferation, psoriasis, prostate hyperplasia, a neoplasia, a neoplasia of epithelial character, adenoma, adenocarcinoma, keratoacanthoma, epidermoid carcinoma, large cell carcinoma, non-small-cell lung carcinoma, lymphoma, Hodgkin's or Non-Hodgkin's lymphoma, a mammary carcinoma, follicular carcinoma, undifferentiated carcinoma, papillary carcinoma, seminoma, melanoma, an IL-1 driven disorder, a MyD88 driven disorder, smoldering or indolent multiple myeloma, and a hematological malignancy selected from leukemia, diffuse large B-cell lymphoma (DLBCL), ABC DLBCL, chronic lymphocytic leukemia (CLL), chronic lymphocytic lymphoma, primary effusion lymphoma, Burkitt lymphoma/leukemia, acute lymphocytic leukemia, B-cell prolymphocytic leukemia, lymphoplasmacytic lymphoma, Waldenström's macroglobulinemia (WM), splenic marginal zone lymphoma, multiple myeloma, plasmacytoma, or intravascular large B-cell lymphoma.

14. The method of claim 13, wherein the MyD88 driven disorder is selected from the group consisting of ABC DLBCL, Waldenström's macroglobulinemia, Hodgkin's lymphoma, primary cutaneous T-cell lymphoma, and chronic lymphocytic leukemia, or:
wherein the IL-1 driven disorder is smoldering or indolent multiple myeloma, optionally:

15. The method of claim 12, wherein the neurodegenerative disease is selected from the group consisting of Alzheimer's disease, Parkinson's disease, amyotrophic lateral sclerosis, Huntington's disease, cerebral ischemia, and a neurodegenerative disease caused by traumatic injury, glutamate neurotoxicity, hypoxia, epilepsy, treatment of diabetes, metabolic syndrome, obesity, organ transplantation and graft versus host disease, or:
wherein the inflammatory disorder is selected from the group consisting of ocular allergy, conjunctivitis, keratoconjunctivitis sicca, vernal conjunctivitis; allergic rhinitis, hemolytic anemia, aplastic anemia, pure red cell anemia, idiopathic thrombocytopenia or another inflammatory disease in which autoimmune reactions are implicated or which have an autoimmune component or etiology, systemic lupus erythematosus, rheumatoid arthritis, polychondritis, scleroderma, Wegener granulamatosis, dermatomyositis, chronic active hepatitis, myasthenia gravis, Steven-Johnson syndrome, idiopathic sprue, ulcerative colitis, Crohn's disease or another autoimmune inflammatory bowel disease, irritable bowel syndrome, celiac disease, periodontitis, hyaline membrane disease, kidney disease, glomerular disease, alcoholic liver disease, endocrine opthalmopathy, Grave's disease, sarcoidosis, alveolitis, chronic hypersensitivity pneumonitis, multiple sclerosis, primary biliary cirrhosis, uveitis (anterior and posterior), Sjogren's syndrome, vernal keratoconjunctivitis, interstitial lung fibrosis, psoriatic arthritis, systemic juvenile idiopathic arthritis, nephritis, diverticulitis, interstitial cystitis, glomerulonephritis (with and without nephrotic syndrome, optionally including idiopathic nephrotic syndrome or minal change nephropathy), chronic granulomatous disease, endometriosis, leptospiriosis renal disease, glaucoma, retinal disease, aging, headache, pain, complex regional pain syndrome, cardiac hypertrophy, muscle wasting, catabolic disorders, obesity, fetal growth retardation, hyperchlolesterolemia, heart disease, chronic heart failure, mesothelioma, anhidrotic ecodermal dysplasia, Behcet's disease, incontinentia pigmenti, Paget's disease, pancreatitis, hereditary periodic fever syndrome, asthma (allergic, non-allergic, mild, moderate, severe, bronchitic, or exercise-induced), acute lung injury, acute respiratory distress syndrome, eosinophilia, hypersensitivities, anaphylaxis, nasal sinusitis, silica induced diseases, COPD (reduction of damage, airways inflammation, bronchial hyperreactivity, remodeling or disease progression), pulmonary disease, cystic fibrosis, acid-induced lung injury, pulmonary hypertension, polyneuropathy, cataracts, muscle inflammation in conjunction with systemic sclerosis, inclusion body myositis, myasthenia gravis, thyroiditis, Addison's disease, lichen planus, Type 1 diabetes, Type 2 diabetes, appendicitis, atopic dermatitis, allergy, blepharitis, bronchiolitis, bronchitis, bursitis, cervicitis, cholangitis, cholecystitis, chronic graft rejection, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonia, polymyositis, proctitis, prostatitis, pyelonephritis, rhinitis, salpingitis, sinusitis, stomatitis, synovitis, tendonitis, tonsillitis, vaginitis, vasculitis, vulvitis, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acute and chronic gout, chronic gouty arthritis, psoriasis, psoriatic arthritis, rheumatoid arthritis, Juvenile rheumatoid arthritis, Cryopyrin Associated Periodic Syndrome (CAPS), and osteoarthritis.
